# EUROPEAN PATENT APPLICATION

(11) **EP 4 606 803 A1**
(43) Date of publication of application: **27.08.2025**
(21) Application number: 23879243.6
(22) Date of filing: 23.10.2023
(51) Int. Cl.: C07D 519/00, C07D 239/22, C07D 283/02, A61K 31/519, A61P 35/00

(54) **KRAS G12D DEGRADATION AGENT, AND PREPARATION METHOD AND USE THEREFOR**

(30) Priority: 21.10.2022 CN 202211297363; 20.01.2023 CN 202310056399; 11.07.2023 CN 202310846875; 19.07.2023 CN 202310886982; 17.10.2023 CN 202311347534
(71) Applicant: Leadingtac Pharmaceutical (Shaoxing) Co., Ltd., Shaoxing, Zhejiang 312030 (CN); Shanghai Leadingtac Qifan Pharmaceutical Co., Ltd., Shanghai 201203 (CN)
(72) Inventor: YE, Zhengqing, Shanghai 201203 (CN); FENG, Yan, Shanghai 201203 (CN); DING, Chenli, Shanghai 201203 (CN)
(74) Representative: Murgitroyd & Company
(86) International application number: PCT/CN2023/125979
(87) International publication number: WO 2024/083258

(57) **Abstract**

Disclosed in the present invention are a KRAS G12D degradation agent, and a preparation method and use therefor. The KRAS G12D degradation agent in the present invention is a compound as shown in formula I or I', and/or a stereoisomer, enantiomer, diastereomer, atropisomer, deuterated compound, hydrate, solvate, prodrug, and/or pharmaceutically acceptable salt thereof. The compound provided by the invention can effectively degrade and/or inhibit KRAS G12D protein in cells, and can be used for preparing a drug for treating and/or preventing related diseases or conditions caused by KRAS G12D mediation.

G-L-E I

G'-L-E I'

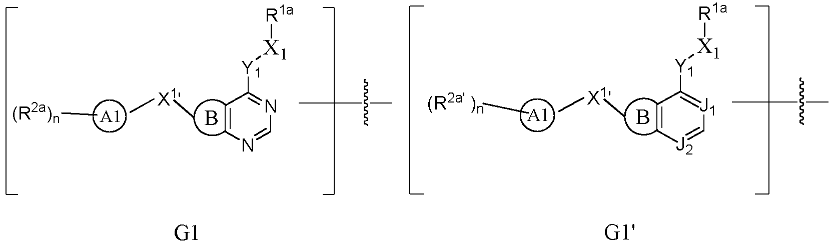

## Description

The present application claims priority to the Chinese patent application 2022112973637 filed on Oct. 21, 2022, the Chinese patent application 2023100563994 filed on Jan. 20, 2023, the Chinese patent application 2023108468752 filed on Jul. 11, 2023, the Chinese patent application 2023108869828 filed on Jul. 19, 2023, and the Chinese patent application 2023113475347 filed on Oct. 17, 2023, which are incorporated herein by reference in their entirety.

### TECHNICAL FIELD

The present disclosure relates to the technical field of pharmaceuticals, and particularly relates to a KRAS G12D degrader, a preparation method therefor, and use thereof.

### BACKGROUND

RAS (rat sarcoma) is one of the oncogenes with the highest mutation rate in tumors, and its mutations are present in about 30% of human malignancies. The RAS family includes KRAS, NRAS, and HRAS, among which KRAS (kirsten rat sarcoma viral oncogene) is more prone to mutations compared to the other two RAS subtypes. KRAS mutations account for approximately 85% of all RAS mutations and are particularly common in solid tumors (Cancer Res. 2020, 80, 2969-2974). The mutation of the KRAS gene exists in 30%-40% of colorectal cancers, 90% of pancreatic cancers, and 15%-20% of lung cancers. After KRAS is activated, it regulates the functions such as the proliferation, differentiation, and survival of cells through downstream signaling pathways such as RAF-MEK-ERK and PI3K-AKT-mTOR. After the mutation of the KRAS gene, the protein is continuously activated, resulting in continuous activation of downstream signaling pathways, and thus promoting tumorigenesis (Nat. Rev. Cancer 2015, 15, 290-301). Therefore, KRAS is an important therapeutic target in cancer treatment, with efforts focused on targeting the KRAS protein itself, its post-translational modifications, membrane localization, protein-protein interactions, and the downstream signaling pathways of RAS.

Due to the ultra-high affinity of KRAS for binding with GTP or GDP (in the picomolar concentration range) and the smooth surface of the RAS protein, which lacks ideal binding sites for small molecules, the development of competitive inhibitors directly targeting RAS proteins is considered highly challenging *(*Nat. Rev. Drug Discov. 2020, 19, 533-552).

At present, according to action modes of inhibitors, KRAS inhibitors *(*Cancer Discov. 2022, 12, 924-937) may be:
inhibitors directly targeting KRAS, such as KRAS G12C, KRAS G12D, KRAS G12R *(*J. Am. Chem. Soc. 2022, 144, 35, 15916-15921), and KRAS G12S *(*Nat. Chem. Biol. 2022, 18, 1177-1183); or
inhibitors indirectly acting on KRAS, such as SHP2 (SHP 099, Nature 2016, 535, 148-152 & J. Med. Chem. 2016, 59, 7773-7782; RMC4550, Nat. Cell Biol. 2018, 20, 1064-1073; TNO155, J. Med. Chem. 2020, 63, 22, 13578-13594; RMC4630, WO 2021142026A1; JAB-3068, WO2017211303A1, etc.), SOS1 inhibitors (Bay-293, Proc. Natl. Acad. Sci. U S A. 2019, 116, 2551-2560; BI-3406, Cancer Discov. 2021, 11, 142-157; MRTX0902, J. Med. Chem. 2022, 65, 678-9690, etc.), and KRAS (on) inhibitors (WO2021091982A1, WO2022060836A1).

In recent years, with the continuous advancement in KRAS research, significant progress has been made in the development of KRAS inhibitors *(*Cancer Discov. 2022, 12, 924-937). In the design of KRAS-G12C inhibitors, the cysteine residue at codon 12 is primarily utilized due to its ability to form covalent bonds. Covalent targeting of this cysteine residue has become a significant breakthrough in the development of KRAS drugs. Shokat discovered a new allosteric binding pocket in Switch-II, called the Switch-II pocket, and developed the first batch of KRAS-G12C covalent inhibitors *(*Nature 2013, 503, 548-551; Nat. Rev. Drug Discov. 2016, 15, 771-785). On May 28, 2021, the FDA granted accelerated approval to Lumakras (Sotorasib, AMG510) *(*Nature 2019, 575, 217-223; J. Med. Chem. 2020, 63, 52-65), developed by Amgen, for the treatment of patients with non-small cell lung cancer harboring the KRAS-G12C mutation *(N*. Engl. J. Med. 2021, 384, 2371-2381). Currently, multiple small molecule drugs targeting KRAS-G12C are undergoing clinical development worldwide. On Feb. 16, 2022, the FDA accepted the New Drug Application submitted by Mirati Therapeutics for its second KRAS-G12C inhibitor, Adagrasib (MRTX849) *(*Cancer Discov. 2020, 10, 54-71 & J. Med. Chem. 2020, 63, 6679-6693). This inhibitor is intended for the treatment of patients with non-small cell lung cancer harboring the KRAS G12C mutation who have received at least one systemic therapy *(*N. Engl. J. Med. 2022, 387, 120-131). Adagrasib is expected to become the second KRAS-targeted drug worldwide.

Unlike the KRAS G12C mutation, the most common mutation in patients with non-small cell lung cancer (NSCLC), KRAS G12D is the most common mutation in colorectal cancer and pancreatic cancer. The KRAS G12C mutant contains a cysteine (Cys) residue at codon 12, which facilitates the formation of covalent bonds. This structural characteristic enables the covalent binding of the mutant to small molecule inhibitors. In contrast, the KRAS G12D mutant has aspartic acid (Asp) at codon 12, which precludes the use of this covalent binding strategy.

The activation of KRAS-G12D is primarily mediated by protein-protein interactions. Considering the lack of distinct binding pockets on the surface of KRAS proteins and the high binding affinity of cyclic peptides for protein surfaces, peptide molecules are used as the first choice for targeting KRAS G12D *(*Biochem. Biophys. Res. Commun. 2017, 484, 605-611; Bioorg. Med. Chem. Lett. 2017, 27, 2757-2761; ACS Med. Chem. Lett. 2017, 8, 732-736; Sci. Rep. 2020, 10, 21671). At present, several potent cyclic peptides targeting KRAS G12D have been reported *(*MedChemComm 2013, 4, 378-382; Angew. Chem., Int. Ed. 2015, 54, 7602-7606; J. Med. Chem. 2021, 64, 13038), but they have relatively poor cell membrane permeability. The ability of cells to uptake peptides depends on the regulation of the cell surface receptor, neuropilin 1 (NRP1), and this protein is overexpressed on the cell membrane of human lung cancer cells. Therefore, cyclic peptides targeting NRP1 and KRAS G12D are picked out, and in this way, the cyclic peptides not only have relatively high cell uptake ability, but also act on KRAS G12D to exert an anti-tumor effect (J. Am. Chem. Soc. 2022, 144, 7117-7128).

On Dec. 10, 2021, the first non-covalent, potent, and highly selective KRAS-G12D inhibitor MRTX1133 *(*J. Med. Chem. 2022, 65, 3923-3942; WO2021041671A1) was reported by Mirati Therapeutics. MRTX1133 can inhibit KRAS-G12D mutant cells in an activated or inactivated state, but does not inhibit wild-type tumor cells, with a specificity of more than 1000 times. Dose-dependent inhibition was shown in *in vivo* xenograft tumor models of both pancreatic cancer and colorectal cancer.

Patents such as WO2021041671A1, WO2022015375A1, WO2022031678A1, WO2022066646A1, WO2022098625A1, WO2022192790A1, WO2022192794A1, WO2021106231A1, WO2021107160A1, and WO2022173870A1 disclose several classes of KRAS G12D inhibitors.

Targeted protein degraders have become a globally popular research and development technology in recent years *(*Nat. Rev. Drug Discov. 2017, 16, 101-114; Nat. Rev. Drug Discov. 2019, 18, 949-963; Nat. Rev. Drug Discov. 2022, 21, 181-200). The technology has the following characteristics: the development of the "druggable" target encounters a bottleneck, the potential of the "non-druggable" target is infinite, and the PROTAC technology is expected to solve the development problem of the "non-druggable" target; the PROTAC drugs have the advantages of good druggability, high selectivity, capability of overcoming drug resistance, multiple administration routes, small dose, low toxicity, etc.

Therefore, there is an unmet clinical need for KRAS G12D targeted protein degraders.

### SUMMARY

The technical problem to be solved in the present disclosure is for overcoming the defect of limited types of KRAS G12D degraders, and thus, the present disclosure provides a KRAS G12D degrader, a preparation method therefor, and use thereof. The compound provided by the present disclosure can effectively degrade and/or inhibit KRAS G12D protein in cells.

The present disclosure provides a compound of formula I or I', and/or a stereoisomer, an enantiomer, a diastereomer, an atropisomer, a deuteride, a hydrate, a solvate or a prodrug thereof and/or a pharmaceutically acceptable salt thereof: 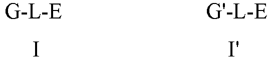 wherein:
G is G1: 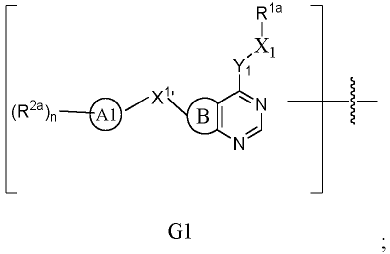
G' is G1': 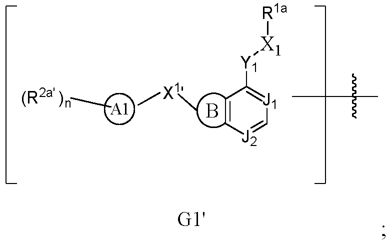
each Y₁ is independently a bond, O, -C(R^{3a})₂, or -NR^{3a};
each X₁ is independently a bond or -(CH₂)ₘ-;
each X¹' is independently a bond or -C(O)-;
each R^{1a} is independently -OH, -N(R^{3a})₂, C₃-C₁₂ cycloalkyl, or 3- to 12-membered heterocycloalkyl, wherein the C₃-C₁₂ cycloalkyl or 3- to 12-membered heterocycloalkyl may be optionally substituted with 1 or more R^{a};
each R^{2a} is independently halogen, deuterium, cyano, amino, hydroxy, C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, triazolyl, -O-C₁-C₆ alkyl, -O-C₃-C₈ cycloalkyl, -CH₂C(=O)N(R^{3a})₂, -N(R^{3a})₂, C₁-C₃ alkyl-O-C₁-C₃ alkyl-, HC(=O)-, -CO₂R^{3a}, -CON(R^{3a})₂, or 5- to 6-membered heteroaryl, wherein the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, O-C₁-C₆ alkyl, -O-C₃-C₈ cycloalkyl, or 5- to 6-membered heteroaryl is optionally substituted with 1 or more deuterium, halogen, hydroxy, cyano, amino, nitro, C₁-C₃ alkoxy, or C₁-C₃ alkyl;
each R^{3a} is independently H, C₁-C₆ alkyl, C₃-C₈ cycloalkyl, C₃-C₆ alkenyl, or C₃-C₆ alkynyl;
or two R^{3a}, together with the atom linked thereto, form 4- to 12-membered heterocycloalkyl, wherein the 4- to 12-membered heterocycloalkyl is optionally substituted with 1 or more deuterium, halogen, hydroxy, cyano, amino, C₁-C₃ alkoxy, or C₁-C₃ alkyl;
or R^{1a} and R^{3a} (R^{3a} here is R^{3a} in -C(R^{3a})₂ and -NR^{3a} in group Y₁), together with the atoms to which they are each linked, form 4- to 12-membered heterocycloalkyl, wherein the 4- to 12-membered heterocycloalkyl contains at least one heteroatom selected from N, O, and S (when R^{1a} and R^{3a} in -NR^{3a}, together with the atoms to which they are each linked, form 4- to 12-membered heterocycloalkyl, the 4- to 12-membered heterocycloalkyl contains at least 1 N atom), and is optionally substituted with 1 or more R^{b};
each ring A1 is independently absent, -L'-(6- to 15-membered aryl), -L'-(5- to 15-membered heteroaryl), or -L'-(5- to 15-membered heterocycloalkyl);
each ring B is independently 6- to 15-membered aryl, 5- to 15-membered heteroaryl, C₅-C₁₅ cycloalkyl, or 5- to 16-membered heterocycloalkyl, wherein the 6- to 15-membered aryl, 5- to 15-membered heteroaryl, C₅-C₁₅ cycloalkyl, or 5- to 16-membered heterocycloalkyl is optionally substituted with 1 or more R^{a};
each L' is independently a bond or C₁-C₄ alkylene, wherein the C₁-C₄ alkylene is optionally substituted with 1 or more deuterium, hydroxy, C₁-C₄ hydroxyalkyl, or 5- to 10-membered heteroaryl;
each R^{a} and each R^{b} are independently hydrogen, hydroxy, halogen, cyano, -N(R^{3a})₂, -CH₂N(R^{3a})₂, 3- to 8-membered heterocycloalkyl, C₁-C₆ alkyl, HC(=O)-, -CO₂R^{4a}, -CO₂N(R^{4a})₂, C₁-C₃ alkoxy, (C₁-C₃ alkoxy)-C₁-C₃ alkyl-, C₁-C₃ alkyl-N(R^{4a})₂, C₂-C₄ alkenyl, C₃-C₈ cycloalkyl, 3- to 8-membered heterocycloalkyl, or 5- to 6-membered heteroaryl, wherein the C₁-C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more deuterium, halogen, cyano, hydroxy, amino, nitro, C₁-C₃ alkyl, or C₁-C₃ alkoxy;
each R^{4a} is independently hydrogen, C₁-C₃ alkyl, or C₁-C₃ hydroxyalkyl;
or two R^{4a}, together with the atom linked thereto, form heterocycloalkyl, wherein the heterocycloalkyl is optionally substituted with 1 or more deuterium, -OH, -NH₂, C₁-C₃ alkyl, or C₁-C₃ alkoxy;
m is 0, 1, 2, 3, or 4;
n is 0, 1, 2, 3, or 4;
J₁ and J₂ are independently CR' or N, provided that J₁ and J₂ are not both CR'; R' is hydrogen, halogen, deuterium, cyano, amino, hydroxy, or C₁-C₆ alkyl;
each R^{2a'} is independently halogen, deuterium, cyano, amino, hydroxy, C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, triazolyl, -O-C₁-C₆ alkyl, -O-C₃-C₈ cycloalkyl, -CH₂C(=O)N(R^{3a})₂, N(R^{3a})₂, C₁-C₃ alkyl-O-C₁-C₃ alkyl-, HC(=O)-, -CO₂R^{3a}, -CON(R^{3a})₂, 5- to 6-membered heteroaryl, or -O-(3- to 8-membered heterocycloalkyl), wherein the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, O-C₁-C₆ alkyl, -O-C₃-C₈ cycloalkyl, 5- to 6-membered heteroaryl, or -O-(3- to 8-membered heterocycloalkyl) is optionally substituted with 1 or more deuterium, halogen, hydroxy, cyano, amino, nitro, C₁-C₃ alkoxy, or C₁-C₃ alkyl;
when J₁ and J₂ are both N, at least one R^{2a'} is -O-(3- to 8-membered heterocycloalkyl);
L is a linking chain, which links G and E by means of covalent bonds;
L is a linking chain, which links G' and E by means of covalent bonds;
each E is independently E1, E2, or E3: 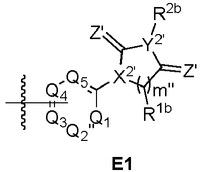
wherein in E1:
   Z' is O, S, or CH₂;
   X²' is CH or N;
   Y²' is CH, N, O, or S;
   Q₁, Q₂, Q₃, Q₄, and Q₅ are each independently CR^{3b} or N;
   each R^{3b} is independently hydrogen, deuterium, hydroxy, amino, cyano, halogen, nitro, sulfhydryl, C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, 6- to 10-membered aryl, 5- to 10-membered heteroaryl, -O-(C₁-C₆ alkyl), -O-(C₁-C₆ heteroalkyl), -O-(C₃-C₈ cycloalkyl), -O-(3- to 8-membered heterocycloalkyl), -S-(C₁-C₆ alkyl), -S-(C₁-C₆ heteroalkyl), -S-(C₃-C₈ cycloalkyl), -S-(3- to 8-membered heterocycloalkyl), -N(C₁-C₆ alkyl)₁₋₂, -N(C₁-C₆ heteroalkyl)₁₋₂, -N(C₃-C₈ cycloalkyl)₁₋₂, -N(3- to 8-membered heterocycloalkyl)₁₋₂, -O-(C₆-C₁₀ aryl), or -O-(5- to 10-membered heteroaryl), wherein the C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, or 5- to 10-membered heteroaryl is optionally substituted with 1-3 groups independently selected from deuterium, hydroxy, halogen, cyano, amino, O(C₁-C₆ alkyl), O-(C₃-C₈ cycloalkyl), -O-(3- to 8-membered heterocycloalkyl), N(C₁-C₆ alkyl)₁₋₂, - NH(C₃-C₈ cycloalkyl), -NH(3- to 8-membered heterocycloalkyl), -O-(C₆-C₁₀ aryl), and -O-(5- to 10-membered heteroaryl); or two adjacent R^{3b}, together with the atoms linked thereto, form cycloalkyl, heterocycloalkyl (e.g., 5-, 6-, 7-, or 8-membered heterocycloalkyl containing 1, 2, or 3 heteroatoms being one or more of N, S, and O), heteroaryl, or aryl;
   m" is 1, 2, or 3;
   each R^{1b} is independently hydrogen, deuterium, hydroxy, amino, cyano, halogen, C₁-C₆ alkyl, C₁-C₆ heteroalkyl, C₃-C₈ cycloalkyl, 3- to 8-membered heterocycloalkyl, C₆-C₁₀ aryl, 5- to 10-membered heteroaryl, -O(C₁-C₆ alkyl), -O-(C₃-C₈ cycloalkyl), -O-(3- to 8-membered heterocycloalkyl), -N(C₁-C₆ alkyl)₁₋₂, -NH(C₃-C₈ cycloalkyl), -NH(3- to 8-membered heterocycloalkyl), -O-(C₆-C₁₀ aryl), or -O-(5- to 10-membered heteroaryl), wherein the alkyl, cycloalkyl, heterocycloalkyl, aryl, or heteroaryl is optionally substituted with 1-3 groups independently selected from hydroxy, halogen, cyano, hydroxy, and amino;
   R^{2b} is absent, hydrogen, deuterium, C₁-C₆ alkyl, or C₃-C₆ cycloalkyl, wherein the C₁-C₆ alkyl and C₃-C₆ cycloalkyl are optionally substituted with 1-3 groups independently selected from hydroxy, halogen, cyano, hydroxy, amino, and -OC(O)(C₁-C₆ alkyl); 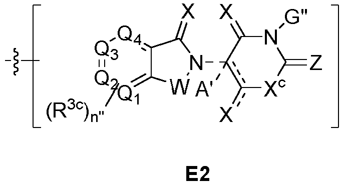
   wherein in E2:
      Q₁, Q₂, Q₃, and Q₄ are each independently CR^{3b} or N;
      W is CR^{1c}R^{2c}, C(S), C(O), SO₂, -OC=R^{4c}-, -SC=R^{4c}-, -C=R^{4c}NR^{5c}-, or -N=CA'-;
      X is CH₂, O, or S;
      X^{c}is -CH₂- or -NG'-;
      Z is CH₂, O, or S;
      G' and G" are each independently hydrogen, deuterium, C₁-C₆ alkyl, OH, C₃-C₆ cycloalkyl, -CH₂-heterocycloalkyl, or -CH₂-phenyl, wherein the C₁-C₆ alkyl, C₃-C₆ cycloalkyl, -CH₂-heterocycloalkyl, or - CH₂-phenyl is optionally substituted with 1 or more hydroxy, halogen, cyano, and amino;
      A' is hydrogen, deuterium, C₁-C₆ alkyl, C₃-C₈ cycloalkyl, or halogen;
      R^{1c}, R^{2c}, and R^{3c} are each independently hydrogen, deuterium, hydroxy, halogen, -NH₂, -N(C₁-C₆ alkyl), C₁-C₆ alkyl, C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, - CR_{'}R_{"}, -CR_{'}NR_{'}R_{"}, C₆-C₁₀ aryl, 5- to 10-membered heteroaryl, C₃-C₈ cycloalkyl, 3- to 8-membered heterocycloalkyl, -P(O)(OR_{'})R_{"}, -P(O)R_{'}R_{"}, -OP(O)(OR_{'})R_{"}, CN, -NR_{'}SO₂NR_{'}R_{"}, -NR_{'}C(O)NR_{'}R_{"}, - C(O)NR_{'}C(O)R_{"}, -NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, - SO₂NR_{'}COR_{"}, -NO₂, -COR_{'}, -C(C=N-OR_{'})R_{"}, -CR_{'}=CR_{'}R_{"}, -CCR_{'}, -S(C=O)(C=N-R_{'})R_{"}, -SF₅, or -OCF₃;
      R^{4c} is O or S;
      R^{5c} is H, C₁-C₆ alkyl, C₆-C₁₀ aryl, 5- to 12-membered heteroaryl, C₃-C₈ cycloalkyl, or 3- to 8-membered heterocycloalkyl;
      R_{'} and R_{"} are each independently a bond, hydrogen, deuterium, C₁-C₆ alkyl, C₃-C₈ cycloalkyl, C₆-C₁₀ aryl, 5- to 10-membered heteroaryl, or 3- to 8-membered heterocycloalkyl;
      n" is 0, 1, 2, 3, or 4;
      -̅ -̅ -̅ is a single bond or a double bond;
      ----- is a bond, which may be an R stereoisomer, an S stereoisomer, or a non-stereoisomer; 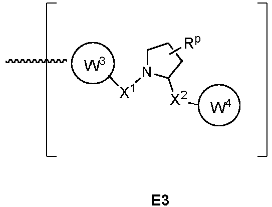
      wherein in E3:
         X¹ and X² are each independently a bond, O, C(O), C(S), NR^{1d}, or CR^{1d}R^{2d};
         R^{1d} and R^{2d} are each independently H, deuterium, or C₁-C₆ alkyl, wherein the C₁-C₆ alkyl is optionally substituted with 1 or more halogen or C₁-C₆ alkoxy;
         R^{P} is independently H, deuterium, halogen, -OH, or C₁-C₃ alkyl, wherein the C₁-C₃ alkyl is optionally substituted with 1 or more halogen, hydroxy, or C₁-C₃ alkoxy;
         W³ is C₁-C₆ alkyl, -T-N(R^{3d}R^{4d}), -T-N(R^{3d}R^{4d})X³, -T-C₆-C₁₀ aryl, -T-(5- to 10-membered heteroaryl), -T-(3- to 8-membered heterocyclyl), -NR^{5d}-T-C₆-C₁₀ aryl, -NR^{5d}-T-(5- to 10-membered heteroaryl), or -NR^{5d}-T-(3- to 8-membered heterocyclyl), wherein the C₁-C₆ alkyl, -T-N(R^{3d}R^{4d}), -T-N(R^{3d}R^{4d})X³, -T-C₆-C₁₀ aryl, -T-(5- to 10-membered heteroaryl), -T-(3- to 8-membered heterocyclyl), -NR^{5d}-T-C₆-C₁₀ aryl, -NR^{5d}-T-(5- to 10-membered heteroaryl), or -NR^{5d}-T-(3- to 8-membered heterocyclyl) is optionally substituted;
         X³ is C(O), R^{3d}, R^{4d}, or R^{5d};
         R^{3d}, R^{4d}, or R^{5d} is each independently selected from H, deuterium, and C₁-C₆ alkyl, wherein the C₁-C₆ alkyl is optionally substituted with 1 or more halogen, -OH, R^{1d}C(O), R^{1d}C(S), R^{1d}SO, R^{1d}SO₂, NR^{1d}R^{2d}C(O), NR^{1d}R^{2d}C(S), NR^{1d}R^{2d}SO, or NR^{1d}R^{2d}SO₂;
         T is C₁-C₆ alkyl or -(CH₂)ₙ-, wherein 1 or more methylene groups in the -(CH₂)ₙ- are optionally substituted with groups selected from deuterium, halogen, and C₁-C₆ alkyl, and the C₁-C₆ alkyl is optionally substituted with halogen, -OH, or amino;
         n is 0, 1, 2, 3, 4, 5, or 6;
         W⁴ is 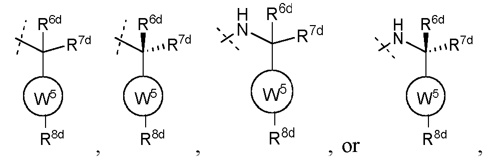 wherein the 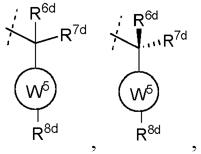 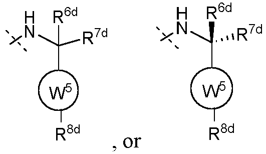 is optionally substituted;
         R^{6d} and R^{7d} are each independently H, deuterium, C₃-C₈ cycloalkyl, or C₁-C₆ alkyl, wherein the C₃-C₈ cycloalkyl or C₁-C₆ alkyl is optionally substituted with halogen, -OH, CN, NO₂, or amino;
         W⁵ is 6- to 10-membered aryl or 5- to 10-membered heteroaryl;
         R^{8d} is H, deuterium, halogen, CN, OH, NO₂, NR^{6d}R^{7d}, OR^{6d}, COR^{6d}R^{7d}, NR^{6d}COR^{7d}, SO₂R^{6d}R^{7d}, R^{6d}SO₂R^{7d}, C₁-C₆ alkyl, C₁-C₆ alkoxy, C₆-C₁₀ aryl, 5- to 10-membered heteroaryl, C₃-C₈ cycloalkyl, or 3- to 8-membered heterocycloalkyl, wherein the C₁-C₆ alkyl or C₁-C₆ alkoxy is optionally substituted with deuterium, halogen, -OH, CN, NO₂, or amino.

Preferably, in certain embodiments of the present disclosure, 1, 2, 3, or 4 methylene groups in each C₁-C₆ heteroalkyl described above are replaced by a heteroatom and/or a heteroatom group, wherein the heteroatom is one or more of O, S, and N; and the heteroatom group is one or more of -C(O)-, -S(O)-, - S(O)₂-,-C(O)O-, -OC(O)-, -C(O)NH-, and -NHC(O)-.

Preferably, in certain embodiments of the present disclosure, the heteroatoms or heteroatom groups in each 3- to 8-membered heterocycloalkyl described above, each 4- to 12-membered heterocycloalkyl described above, each 5- to 16-membered heterocycloalkyl described above, and each heterocycloalkyl described above may each independently be one or more of O, S, -S(O)-, -S(O)₂-, and N, the number of the heteroatoms or heteroatom groups being 1, 2, 3, 4, or 5. Preferably, when the heteroatom is a nitrogen atom, it is not quaternized or oxidized.

Preferably, in certain embodiments of the present disclosure, the heteroatoms in each 5- to 6-membered heteroaryl described above, each 5- to 10-membered heteroaryl described above, each 5- to 12-membered heteroaryl described above, each 5- to 15-membered heteroaryl described above, and each heteroaryl described above may each independently be one or more of O, S, -S(O)-, -S(O)₂-, and N, the number of the heteroatoms being 1, 2, 3, or 4.

Preferably, in certain embodiments of the present disclosure, in formula I, the definitions of one or more of the groups R^{2a}, R^{3a}, C₁-C₄ alkylene in L', R^{a}, and R^{b} may also be replaced by the following definitions, provided that L is linked to E via -C(O)-;
R^{2a} and R^{2a'} are independently -O-C₁-C₆ heteroalkyl, -NH(C₁-C₆ heteroalkyl), -N(C₁-C₆ heteroalkyl)(C₁-C₆ heteroalkyl), or -C₃-C₄ alkynyl-N(R^{5a'})₂;
in R^{2a} and R^{2a'}, the hydroxy is independently optionally substituted with 1 or more deuterium, halogen, hydroxy, cyano, amino, nitro, C₁-C₃ alkoxy, C₁-C₃ alkyl, C₃-C₈ cycloalkyl, -Si-C₁-C₃ alkyl, 3- to 8-membered heterocycloalkyl, or -CON(R^{3a})₂;
in R^{2a} and R^{2a'}, the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, -O-C₁-C₆ alkyl, -O-C₃-C₈ cycloalkyl, and 5-to 6-membered heteroaryl are independently optionally substituted with 1 or more C₃-C₈ cycloalkyl, -Si-C₁-C₃ alkyl, 3- to 8-membered heterocycloalkyl, or -CON(R^{3a})₂;
each R^{3a} is deuterium;
C₁-C₄ alkylene in L' may also be optionally substituted with 1 or more halogen;
R^{a} and R^{b} may be independently a bond, deuterium, oxo, cyanomethyl, -N(R^{3a})₂, -CH₂N(R^{3a})₋₂, - O(C₁-C₆ alkyl), -O(C₃-C₈ cycloalkyl), -O(C₁-C₆ heteroalkyl), -O(3- to 8-membered heterocycloalkyl), -S(C₁-C₆ alkyl), -S(C₃-C₈ cycloalkyl), -O-phenyl, -O-pyridyl, C₂-C₄ alkynyl, triazolyl, -CH₂C(=O)N(R^{3a})₂, -C₃-C₄ alkynyl-N(R^{3a})₂, or 3- to 8-membered heterocycloalkyl;
or two adjacent R^{a}, together with the atoms linked thereto, form 6- to 10-membered aryl, 5- to 10-membered heteroaryl, 5- to 8-membered cycloalkyl, or 5- to 8-membered heterocycloalkyl, wherein the 6-to 10-membered aryl, 5- to 10-membered heteroaryl, 5- to 8-membered cycloalkyl, or 5- to 8-membered heterocycloalkyl is optionally substituted with 1 or more hydroxy, amino, halogen, cyano, or nitro;
in R^{a} and R^{b}, the C₁-C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more C₁-C₃ haloalkyl;
in R^{a} and R^{b}, the C₁-C₆ heteroalkyl, phenyl, pyridyl, C₂-C₄ alkynyl, triazolyl, -CH₂C(=O)N(R^{3a})₁₋₂, or -C₃-C₄ alkynyl-N(R^{3a})₂ is optionally substituted with 1 or more deuterium, halogen, cyano, hydroxy, amino, nitro, C₁-C₃ haloalkyl, C₁-C₃ alkyl, or C₁-C₃ alkoxy;
each R^{4a} is independently C₄-C₆ alkyl.

Preferably, in certain embodiments of the present disclosure, in each R^{1a}, the C₃-C₁₂ cycloalkyl is C₃-C₆ cycloalkyl.

Preferably, in certain embodiments of the present disclosure, in each R^{1a}, the 3- to 12-membered heterocycloalkyl is 5- to 6-membered monocyclic saturated heterocycloalkyl or 7-, 8-, or 9-membered bridged heterocycloalkyl containing 1 or 2 heteroatoms of N, such as piperidinyl,

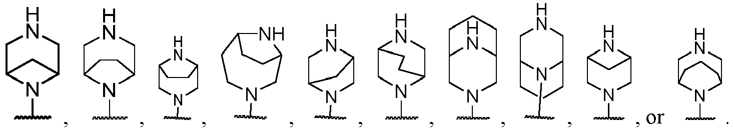

Preferably, in certain embodiments of the present disclosure, in each R^{2a} and each R^{2a'}, the halogen is F, Cl, Br, or I, such as F or Cl.

Preferably, in certain embodiments of the present disclosure, in each R^{2a} and each R^{2a'}, the C₁-C₆ alkyl, C₁-C₆ alkyl in the -S-C₁-C₆ alkyl, and C₁-C₆ alkyl in the -O-C₁-C₆ alkyl are independently C₁-C₄ alkyl, and may also be C₁-C₃ alkyl, such as methyl, ethyl, n-propyl, or isopropyl.

Preferably, in certain embodiments of the present disclosure, in each R^{2a} and each R^{2a'}, the C₁-C₆ alkyl or -O-C₁-C₆ alkyl may be optionally substituted with 1 or more halogen or C₁-C₃ alkoxy.

Preferably, in certain embodiments of the present disclosure, in each R^{2a} and each R^{2a'}, the C₁-C₆ heteroalkyl is C₃ heteroalkyl, wherein in the heteroalkyl, 2 methylene groups are replaced by O and - C(O)NH-, such as

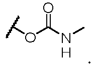

Preferably, in certain embodiments of the present disclosure, in each R^{2a} and each R^{2a'}, the C₃-C₈ cycloalkyl is C₃-C₆ cycloalkyl, such as cyclopropyl, cyclobutyl, cyclopentyl, or cyclohexyl.

Preferably, in certain embodiments of the present disclosure, in each R^{2a'}, the -O-(3- to 8-membered heterocycloalkyl) is -O-(3-, 4-, 5-, or 6-membered monocyclic saturated heterocycloalkyl) containing 1, 2, or 3 heteroatoms being one or more of N, S, and O, such as

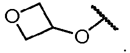

Preferably, in certain embodiments of the present disclosure, in each R^{2a} and each R^{2a'}, the C₃-C₈ may be optionally substituted with 1 or more C₁-C₃ alkyl.

Preferably, in certain embodiments of the present disclosure, in each R^{2a} and each R^{2a'}, the 3- to 8-membered heterocycloalkyl is 3- to 6-membered heterocycloalkyl containing 1, 2, or 3 heteroatoms being one or more of N, S, and O.

Preferably, in certain embodiments of the present disclosure, in each R^{2a} and each R^{2a'}, the C₂-C₆ alkynyl is C₂-C₄ alkynyl, such as ethynyl.

Preferably, in certain embodiments of the present disclosure, each R^{2a} and each R^{2a'} are optionally substituted with substituents, wherein in the substituents, the halogen is F, Cl, Br, or I, such as F.

Preferably, in certain embodiments of the present disclosure, each R^{2a} and each R^{2a'} are optionally substituted with substituents, wherein in the substituents, the C₁-C₃ alkoxy is methoxy, ethoxy, n-propoxy, or isopropoxy, such as methoxy.

Preferably, in certain embodiments of the present disclosure, each R^{2a} and each R^{2a'} are optionally substituted with substituents, wherein in the substituents, the C₁-C₃ alkyl is methyl, ethyl, n-propyl, or isopropyl, such as methyl.

Preferably, in certain embodiments of the present disclosure, in each R^{3a}, the C₁-C₆ alkyl, C₁-C₆ alkyl in the -S-C₁-C₆ alkyl, and C₁-C₆ alkyl in the -O-C₁-C₆ alkyl are independently C₁-C₄ alkyl, and may also be C₁-C₃ alkyl, such as methyl, ethyl, n-propyl, or isopropyl.

Preferably, in certain embodiments of the present disclosure, in each ring A1, 6- to 15-membered aryl in the -L'-(6- to 15-membered aryl) is 6- to 10-membered aryl, such as phenyl or naphthyl.

Preferably, in certain embodiments of the present disclosure, in each ring A1, 5- to 15-membered heteroaryl in the -L'-(5- to 15-membered heteroaryl) is 5- to 10-membered heteroaryl, and may also be 6-, 7-, 8-, or 9-membered monocyclic or bicyclic heteroaryl, containing 1 or 2 heteroatoms being one or more of N, S, and O, such as pyridyl, 1*H*-indazolyl, phenyl[*d*]thiazole, or oxazolophenyl.

Preferably, in certain embodiments of the present disclosure, in each ring A1, 5- to 15-membered heterocycloalkyl in the -L'-(5- to 15-membered heterocycloalkyl) is 5- to 10-membered heterocycloalkyl containing 1 or 2 heteroatoms being one or more of N, S, and O.

Preferably, in certain embodiments of the present disclosure, in each ring B, the 6- to 15-membered aryl is 6- to 10-membered aryl, such as phenyl or naphthyl.

Preferably, in certain embodiments of the present disclosure, in each ring B, the 5- to 15-membered heteroaryl is 5- to 10-membered heteroaryl, and may also be 5- to 6-membered heteroaryl, containing 1 or 2 heteroatoms of N, such as pyridyl.

Preferably, in certain embodiments of the present disclosure, in each ring B, the C₅-C₁₅ cycloalkyl is C₅-C₇ monocyclic saturated cycloalkyl, C₅-C₇ cycloalkenyl, or C₈-C₁₅ tricyclic cycloalkyl, such as 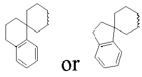 , and 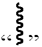 indicates that the cycloalkyl is fused to a ring linked thereto via the bond herein.

Preferably, in certain embodiments of the present disclosure, in each ring B, the 5- to 16-membered heterocycloalkyl is 5- to 7-membered monocyclic heterocycloalkyl containing 1 or 2 heteroatoms of N and/or O, such as pyrrolidinyl, piperidinyl, or tetrahydro-2H-pyranyl.

Preferably, in certain embodiments of the present disclosure, in each R^{a} and each R^{b}, the halogen is F, Cl, Br, or I, such as F or Cl.

Preferably, in certain embodiments of the present disclosure, in each R^{a} and each R^{b}, the C₁-C₆ alkyl is C₁-C₃ alkyl, such as methyl, ethyl, n-propyl, or isopropyl.

Preferably, in certain embodiments of the present disclosure, in each R^{a} and each R^{b}, the C₁-C₃ alkoxy and C₁-C₃ alkoxy in the (C₁-C₃ alkoxy)-C₁-C₃ alkyl- are independently methoxy, ethoxy, n-propoxy, or isopropoxy.

Preferably, in certain embodiments of the present disclosure, G1 is G1-I or G1-II: 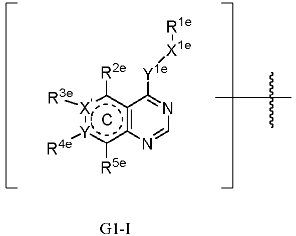 wherein in G1-I:
R^{1e} is hydroxy, -N(R^{a"})₂, C₃-C₁₂ cycloalkyl, or 3- to 12-membered heterocycloalkyl (e.g., 6-, 7-, 8-, 9-, or 10-membered monocyclic or bridged heterocycloalkyl containing 1, 2, or 3 heteroatoms being one or more of N, S, or O), wherein the C₃-C₁₂ cycloalkyl or 3- to 12-membered heterocycloalkyl is optionally substituted with one or more R^{x};
X^{1e} is a bond or C₁-C₄ alkylene;
Y^{1e} is a bond, O, or NR^{a"};
ring C is C₆-C₁₀ aryl (e.g., phenyl), 5- to 10-membered heteroaryl (e.g., 5- or 6-membered heteroaryl containing 1 or 2 heteroatoms of N), or 5- to 10-membered heterocycloalkyl (e.g., 5- or 6-membered heterocycloalkyl containing 1 or 2 heteroatoms of N);
X' is a bond, C, or N (e.g., X' is a bond, C, or N);
Y' is C or N;
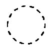 is one or more double bonds that are optionally present;
R^{2e} is H, deuterium, halogen, hydroxy, amino, C₁-C₆ alkyl, C₃-C₈ cycloalkyl, -O(C₁-C₆ alkyl), or - O(C₃-C₈ cycloalkyl);
R^{3e} is a bond, H, deuterium, halogen, cyano, oxo, -O(C₁-C₆ alkyl) (e.g., methoxy or ethoxy), - O(C₁-C₆ heteroalkyl), -O(C₃-C₈ cycloalkyl), -O(3- to 8-membered heterocycloalkyl), -S(C₁-C₆ alkyl), -S(C₃-C₈ cycloalkyl), -O-phenyl, or -O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is -L₁-(6- to 10-membered aryl) (e.g., phenyl or naphthyl) or -L₁-(5- to 10-membered heteroaryl) (e.g., 6-, 7-, 8-, or 9-membered monocyclic or bicyclic heteroaryl containing 1 or 2 heteroatoms being one or two of N, S, or O), wherein the 6- to 10-membered aryl or 5- to 10-membered heteroaryl is optionally substituted with 1 or more R^{a'};
or R^{4e}, together with the carbon atom linked thereto, forms C₈-C₁₀ aryl or 8- to 10-membered heteroaryl (i.e., R^{4e}, together with Y' linked thereto, forms a spiro ring structure), wherein the C₈-C₁₀ aryl or 8- to 10-membered heteroaryl is optionally substituted with halogen, hydroxy, cyano, -O(C₁-C₆ alkyl), - S(C₁-C₆ alkyl), and C₁-C₆ alkyl;
R^{5e} is hydrogen, deuterium, halogen (e.g., F, Cl, or Br), cyano, C₁-C₆ alkyl, C₃-C₈ cycloalkyl, - O(C₁-C₆ alkyl), or -O(C₃-C₈ cycloalkyl);
L₁ is a bond or C₁-C₄ alkylene, wherein the C₁-C₄ alkylene is optionally substituted with halogen, deuterium, hydroxy, C₁-C₄ hydroxyalkyl, or 5- to 10-membered heteroaryl;
each R^{a"} is independently hydrogen, deuterium, or C₁-C₆ alkyl;
each R^{x} is independently H, deuterium, -OH, halogen, C₁-C₃ alkyl (e.g., methyl), -N(R^{a"})₁₋₂ (e.g., - N(CH₃)₂), -CH₂N(R^{a"})₁₋₂, C₁-C₃ alkoxy, (C₁-C₃ alkoxy)-C₁-C₃ alkyl, C₁-C₃ alkyl-N(R^{a"})₁₋₂, cyano, C₂-C₄ alkenyl, HC(=O), -CO₂R^{a"}, -CON(R^{a"})₂, 3- to 8-membered heterocycloalkyl, or 5- to 6-membered heteroaryl, wherein the C₁-C₃ alkyl, C₁-C₃ alkoxy, or C₂-C₄ alkenyl is optionally substituted with deuterium, halogen, cyano, hydroxy, nitro, or amino;
R^{a'} is independently halogen (e.g., F or Cl), cyano, hydroxy, C₁-C₆ alkyl (e.g., methyl, ethyl, or isopropyl), C₁-C₆ heteroalkyl, -S-C₁-C₆ alkyl (e.g., -S-methyl), C₂-C₆ alkenyl, C₂-C₆ alkynyl (e.g., ethynyl), triazolyl, -O-C₁-C₆ alkyl (e.g., ethoxy), -O-C₁-C₆ heteroalkyl, -N(C₁-C₆ alkyl)₁₋₂, -N(C₁-C₆ heteroalkyl)₁₋₂, - CH₂C(=O)N(R^{5a'})₂, -C₃-C₄ alkynyl(NR^{5a'})₁₋₂, -N(R^{5a'})₁₋₂, (C₁-C₃ alkoxy)C₁-C₃ alkyl-, C₃-C₆ cycloalkyl (e.g., cyclopropyl), or 3- to 6-membered heterocycloalkyl, wherein the C₁-C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-C₁-C₃ alkyl;
each R^{5a'} is independently H, deuterium, or C₁-C₆ alkyl; 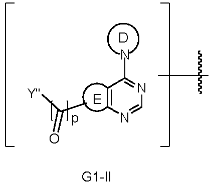
wherein in G1-II:
   ring D is 6- to 10-membered heterocycloalkyl, wherein the 6- to 10-membered heterocycloalkyl is optionally substituted with R^{b'}, and the 6- to 10-membered heterocycloalkyl contains at least one heteroatom selected from N, S, and O;
   ring E is C₅-C₇ cycloalkyl, 5- to 7-membered heterocycloalkyl (e.g., 5-membered monocyclic heterocycloalkyl containing 1 or 2 heteroatoms of N), C₆-C₁₀ aryl, 5- to 10-membered heteroaryl, C₅-C₇ cycloalkenyl, or a 8- to 10-membered spiro ring, and a fused ring is formed between ring E and a pyrimidine ring, wherein the C₅-C₇ cycloalkyl, 5- to 7-membered heterocycloalkyl, C₆-C₁₀ aryl, 5- to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
   Y" is 6- to 10-membered heterocycloalkyl, 6- to 10-membered aryl (e.g., phenyl or naphthyl), or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
   R^{b'} is C₁-C₃ alkyl, hydroxy, -O(C₁-C₃ alkyl), cyano, halogen, -N(R^{x'})₁₋₂, -CH₂N(R^{x'})₁₋₂, cyanomethyl, or 3- to 8-membered heterocycloalkyl;
   R^{b"} is halogen (e.g., F), deuterium, cyano, hydroxy, C₁-C₄ alkyl, -S-C₁-C₃ alkyl, C₂-C₄ alkenyl, C₂-C₄ alkynyl (e.g., ethynyl), triazolyl, -O-C₁-C₃ alkyl, -CH₂C(=O)N(R^{x'})₁₋₂, -C₃-C₄ alkynyl(NR^{x'})₁₋₂, -N(R^{x'})₁₋₂, or C₁-C₃ alkoxy-C₁-C₃ alkyl-, wherein the C₁-C₄ alkyl, -S-C₁-C₃ alkyl, C₂-C₄ alkenyl, C₂-C₄ alkynyl, or C₁-C₃ alkoxy is optionally substituted with deuterium, halogen, hydroxy, cyano, amino, nitro, C₁-C₃ alkoxy, or C₁-C₃ alkyl;
   R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁-C₆ alkyl is optionally substituted with halogen, hydroxy, cyano, or amino;
   each R^{x'} is independently H or C₁-C₃ alkyl;
   p is 0 or 1.

Preferably, in certain embodiments of the present disclosure, G1 is G1-I-a, G1-I-b, or G1-II-a: 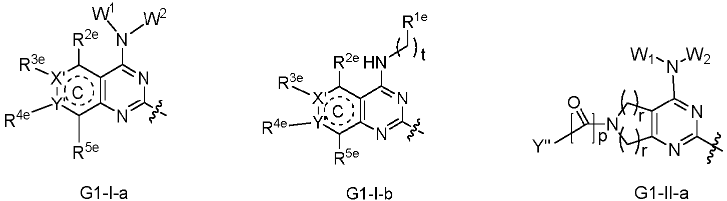 wherein:
r is 1 or 2;
t is 1, 2, or 3;
W¹ and W², together with the N atom linked thereto, form 6- to 10-membered heterocycloalkyl, wherein the 6- to 10-membered heterocycloalkyl contains at least one heteroatom selected from N, S, and O (e.g., 6-, 7-, 8-, 9-, or 10-membered monocyclic or bridged heterocycloalkyl containing 1, 2, or 3 heteroatoms being one or more of N, S, or O, and containing at least 1 N);
ring C, R^{1e}, R^{2e}, R^{3e}, R^{4e}, R^{5e}, X', Y', Y", and p in G1-I-a, G1-I-b, or G1-II-a are as defined and described in the present disclosure.

Preferably, in certain embodiments of the present disclosure, G1 is G1-I-a1, G1-I-a1', G1-I-a1", G1-I-a1‴, G1-I-a2, G1-I-a2', G1-I-b1, G1-I-b2, or G1-II-a1: 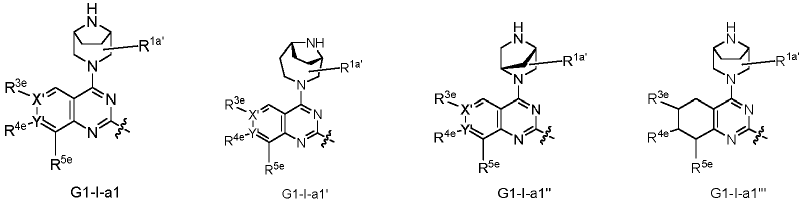 wherein in G1-I-a1, G1-I-a1', G1-I-a1", and G1-I-a1‴:
R^{1a'} is hydrogen, hydroxy, halogen, cyano, C₁-C₆ alkyl (e.g., methyl), C₃-C₈ cycloalkyl, HC(=O)-, -CO₂R^{5a'}, -CO₂N(R^{5a'})₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl;
R^{3e}, R^{4e}, R^{5e}, X', and Y' are as defined and described in G1-I-a; 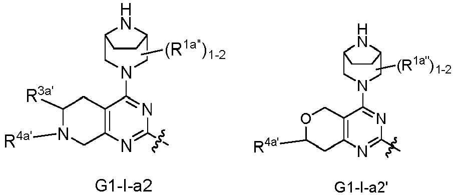
wherein in G1-I-a2 and G1-I-a2':
   each R^{1a"} is independently hydrogen, hydroxy, halogen, C₁-C₃ alkyl, C₁-C₃ alkoxy, (C₁-C₃ alkoxy)-C₁-C₃ alkyl-, C₁-C₃ alkyl-N(R^{5a'})₁₋₂, cyano, C₂-C₄ alkenyl, HC(=O)-, -CO₂R^{5a'}, or -CO₂N(R^{5a'})₂, wherein the C₁-C₃ alkyl or C₂-C₄ alkenyl is optionally substituted with halogen, hydroxy, cyano, or amino;
   each R^{4a'} is -L₂-(6- to 10-membered aryl), 6- to 10-membered aryl (e.g., phenyl or naphthyl), -L₂-(5- to 10-membered heteroaryl), or 5- to 10-membered heteroaryl, wherein the 6- to 10-membered aryl or 5-to 10-membered heteroaryl may be optionally substituted with one or more R^{8a'};
   each L₂ is C₁-C₄ alkylene, wherein the C₁-C₄ alkylene is optionally substituted with hydroxy, halogen, cyano, amino, C₁-C₄ hydroxyalkyl, or 5- to 10-membered heteroaryl;
   each R^{5a'} is independently H or C₁-C₃ alkyl;
   each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, N(R^{5a'})₁₋₂, or -O-C₁-C₆ alkyl, wherein the C₁-C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
   R^{3a'} is hydrogen or oxo.

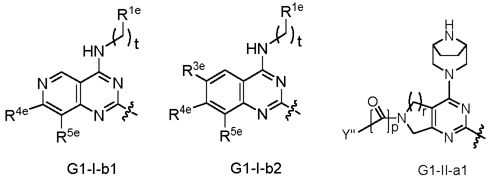

In G1-I-b1, G1-I-b2, and G1-II-a1:
R^{1e}, R^{3e}, R^{4e}, R^{5e}, t, r, Y", and p are as defined and described in the present disclosure.

Preferably, in certain embodiments of the present disclosure, G1' is G1'-I or G1'-II: 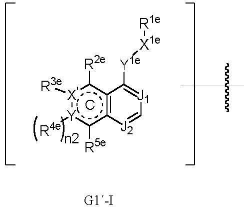 wherein in G1'-I:
n2 is 1 or 2;
J₁ and J₂ are independently selected from CR' and N, and J₁ and J₂ are not both CR';
R' is hydrogen, halogen, deuterium, cyano, amino, hydroxy, or C₁-C₆ alkyl;
R^{1e} is hydroxy, -N(R^{a"})₂, C₃-C₁₂ cycloalkyl, or 3- to 12-membered heterocycloalkyl, wherein the C₃-C₁₂ cycloalkyl or 3- to 12-membered heterocycloalkyl is optionally substituted with one or more R^{x};
X^{1e} is a bond or C₁-C₄ alkylene;
Y^{1e} is a bond, O, or NR^{a"};
ring C is C₆-C₁₀ aryl, 5- to 10-membered heteroaryl, or 5- to 10-membered heterocycloalkyl;
X' is a bond, O, C, or N;
Y' is C or N; 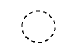
is one or more double bonds that are optionally present;
R^{2e} is H, deuterium, halogen, hydroxy, amino, C₁-C₆ alkyl, C₃-C₈ cycloalkyl, -O(C₁-C₆ alkyl), or - O(C₃-C₈ cycloalkyl);
R^{3e} is H, deuterium, halogen, cyano, oxo, -O(C₁-C₆ alkyl), -O(C₁-C₆ heteroalkyl), -O(C₃-C₈ cycloalkyl), -O(3- to 8-membered heterocycloalkyl), -S(C₁-C₆ alkyl), -S(C₃-C₈ cycloalkyl), -O-phenyl, or - O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is -L₁-(6- to 10-membered aryl) or -L₁-(5- to 10-membered heteroaryl), wherein the 6- to 10-membered aryl or 5- to 10-membered heteroaryl is optionally substituted with 1 or more R^{a'};
or when n2 is 2, two R^{4e}, together with the carbon atom linked thereto, form C₈-C₁₀ aryl or 8- to 10-membered heteroaryl, wherein the C₈-C₁₀ aryl or 8- to 10-membered heteroaryl is optionally substituted with halogen, hydroxy, cyano, -O(C₁-C₆ alkyl), -S(C₁-C₆ alkyl), or C₁-C₆ alkyl;
R^{5e} is hydrogen, deuterium, halogen, cyano, C₁-C₆ alkyl, C₃-C₈ cycloalkyl, -O(C₁-C₆ alkyl), or - O(C₃-C₈ cycloalkyl);
L₁ is a bond or C₁-C₄ alkylene, wherein the C₁-C₄ alkylene is optionally substituted with halogen, deuterium, hydroxy, C₁-C₄ hydroxyalkyl, or 5- to 10-membered heteroaryl;
each R^{a"} is independently hydrogen, deuterium, or C₁-C₆ alkyl;
each R^{x} is independently H, deuterium, -OH, halogen, cyano, C₁-C₆ alkyl, -N(R^{a"})₁₋₂, -CH₂N(R^{a"})₁₋₂, C₁-C₃ alkoxy, (C₁-C₃ alkoxy)-C₁-C₃ alkyl, C₁-C₃ alkyl-N(R^{a"})₁₋₂, cyano, C₂-C₄ alkenyl, HC(=O), -CO₂R^{a"}, -CON(R^{a"})₂, C₃-C₈ cycloalkyl, 3- to 8-membered heterocycloalkyl, or 5- to 6-membered heteroaryl, wherein the C₁-C₆ alkyl, C₃-C₈ cycloalkyl, C₁-C₃ alkoxy, or C₂-C₄ alkenyl is optionally substituted with deuterium, halogen, cyano, hydroxy, nitro, or amino;
R^{a'} is independently halogen, deuterium, cyano, hydroxy, C₁-C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -NH(C₁-C₆ heteroalkyl), -N(C₁-C₆ heteroalkyl)₂, -CH₂C(=O)N(R^{5a'})₂, -C₃-C₄ alkynyl(NR^{5a'})₂, -N(R^{5a'})₂ (e.g., -N(C₁-C₆ alkyl)₂, - NH(C₁-C₆ heteroalkyl)), (C₁-C₃ alkoxy)C₁-C₃ alkyl-, C₃-C₆ cycloalkyl, or 3- to 6-membered heterocycloalkyl, wherein the C₁-C₆ alkyl, C₁-C₆ heteroalkyl, -S-C₁-C₆ alkyl, C₂-C₆ alkenyl, C₂-C₆ alkynyl, - O-C₁-C₆ alkyl, -O-C₁-C₆ heteroalkyl, -NH(C₁-C₆ alkyl), -N(C₁-C₆ alkyl)₂, -NH(C₁-C₆ heteroalkyl), -N(C₁-C₆ heteroalkyl)₂, -, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-C₁-C₃ alkyl;
each R^{5a'} is independently H, deuterium, or C₁-C₆ alkyl; 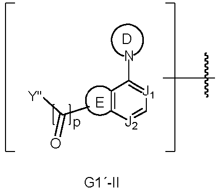
wherein in G1'-II:
   J₁ and J₂ are independently CR' or N, and J₁ and J₂ are not both CR';
   R' is hydrogen, halogen, deuterium, cyano, amino, hydroxy, or C₁-C₆ alkyl;
   ring D is 6- to 10-membered heterocycloalkyl, wherein the 6- to 10-membered heterocycloalkyl is optionally substituted with R^{b'}, and the 6- to 10-membered heterocycloalkyl contains at least one heteroatom selected from N, S, and O;
   ring E is C₅-C₁₅ cycloalkyl, 5- to 7-membered heterocycloalkyl, C₆-C₁₀ aryl, 5- to 10-membered heteroaryl, C₅-C₇ cycloalkenyl 8- to 10-membered spiro ring, wherein the C₅-C₇ cycloalkyl, 5- to 7-membered heterocycloalkyl, C₆-C₁₀ aryl, 5- to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
   Y" is 6- to 10-membered heterocycloalkyl, 6- to 10-membered aryl, or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
   R^{b'} is hydrogen, C₁-C₆ alkyl, hydroxy, -O(C₁-C₃ alkyl), cyano, halogen, -N(R^{x'})₂, -CH₂N(R^{x'})₂, - CO₂R^{x'}, -CO₂N(R^{x'})₂ cyanomethyl 5- to 6-membered heteroaryl, or 3- to 8-membered heterocycloalkyl, wherein the C₁-C₆ alkyl or C₃-C₈ cycloalkyl is optionally substituted with halogen, hydroxy, cyano, or amino;
   R^{b"} is halogen, deuterium, cyano, hydroxy, C₁-C₄ alkyl, -S-C₁-C₃ alkyl, C₂-C₄ alkenyl, C₂-C₄ alkynyl, triazolyl, -O-C₁-C₃ alkyl, -CH₂C(=O)N(R^{x'})₋₂, -C₃-C₄ alkynyl(NR^{x'})₂, -N(R^{x'})₂, or C₁-C₃ alkoxy-C₁-C₃ alkyl-, wherein the C₁-C₄ alkyl, -S-C₁-C₃ alkyl, C₂-C₄ alkenyl, C₂-C₄ alkynyl, or C₁-C₃ alkoxy is optionally substituted with deuterium, halogen, hydroxy, cyano, amino, nitro, C₁-C₃ alkoxy, or C₁-C₃ alkyl;
   R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁-C₆ alkyl is optionally substituted with halogen, hydroxy, cyano, or amino;
   each R^{x'} is independently H or C₁-C₆ alkyl;
   p is 0 or 1.

Preferably, in certain embodiments of the present disclosure, G1'-I is G1'-I-a or G1'-I-b; and G1'-II is G1'-II-a: 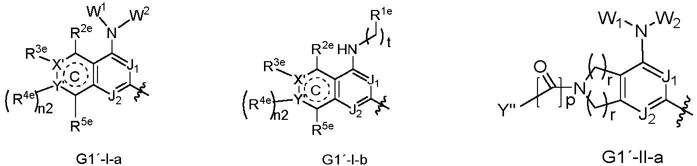 wherein:
r is 1 or 2;
t is 1, 2, or 3;
W¹ and W², together with the N atom linked thereto, form 6- to 10-membered heterocycloalkyl, wherein the 6- to 10-membered heterocycloalkyl contains at least one heteroatom selected from N, S, and O;
ring C, J₁, J₂, R^{1e}, R^{2e}, R^{3e}, R^{4e}, R^{5e}, X', Y', Y", and p in G1'-I-a, G1'-I-b, or G1'-II-a are as defined and described in the present disclosure.

Preferably, in certain embodiments of the present disclosure, G1' is G1'-I-a1, G1'-I-a1', G1'-I-a1", G1'-I-a1‴, G1'-I-a2, G1'-I-b1, G1'-I-b2, or G1'-II-a1: 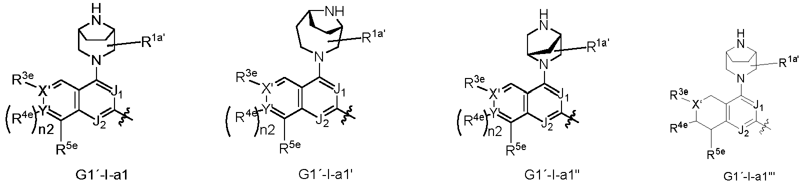 wherein in G1'-I-a1, G1'-I-a1', G1'-I-a1", and G1'-I-a1‴:
R^{1a'} is hydrogen, hydroxy, halogen, cyano, C₁-C₆ alkyl, C₃-C₈ cycloalkyl, HC(=O)-, -CO₂R^{5a'}, - CO₂N(R^{5a'})₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl;
J₁, J₂, R^{3e}, R^{4e}, R^{5e}, X', and Y' are as defined and described in G1'-I-a of the present disclosure; 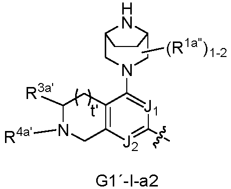
wherein in G1'-I-a2:
   J₁ and J₂ are independently CR' or N, and J₁ and J₂ are not both CR';
   R' is hydrogen, halogen, deuterium, cyano, amino, hydroxy, or C₁-C₆ alkyl;
   t' is 0 or 1;
   each R^{1a"} is independently hydrogen, hydroxy, halogen, C₁-C₃ alkyl, C₁-C₃ alkoxy, -(C₁-C₃ alkoxy)-C₁-C₃ alkyl-, -C₁-C₃ alkyl-N(R^{5a'})₋₂, cyano, C₂-C₄ alkenyl, HC(=O)-, -CO₂R^{5a'}, or -CO₂N(R^{5a'})₂, wherein the C₁-C₃ alkyl or C₂-C₄ alkenyl is optionally substituted with halogen, hydroxy, cyano, or amino;
   R^{4a'} is -L₂-(6- to 10-membered aryl), 6- to 10-membered aryl, -L₂-(5- to 10-membered heteroaryl), or 5- to 10-membered heteroaryl, wherein the 6- to 10-membered aryl or 5- to 10-membered heteroaryl may be optionally substituted with one or more R^{8a'};
   L₂ is C₁-C₄ alkylene, wherein the C₁-C₄ alkylene is optionally substituted with hydroxy, halogen, cyano, amino, C₁-C₄ hydroxyalkyl, or 5- to 10-membered heteroaryl;
   R^{5a'} is independently H or C₁-C₃ alkyl;
   each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, N(R^{5a'})₁₋₂, or -O-C₁-C₆ alkyl, wherein the C₁-C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
   R^{3a'} is hydrogen or oxo; 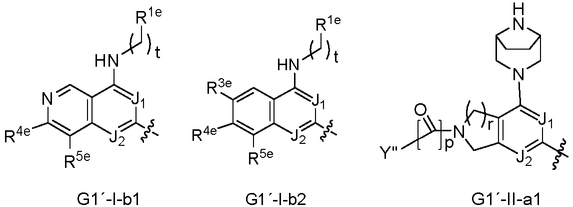
   In G1'-I-b1, G1'-I-b2, and G1'-II-al:
      J₁, J₂, R^{1e}, R^{3e}, R^{4e}, R^{5e}*,* t, r, Y", and p are as defined and described in the present disclosure.

Preferably, in certain embodiments of the present disclosure, in G1':
Y₁ is a bond; X₁ is a bond; X^{1'} is a bond;
R^{1a} is 3- to 12-membered heterocycloalkyl, wherein the 3- to 12-membered heterocycloalkyl contains 1, 2, or 3 heteroatoms being one or more of O, S, and N;
ring A1 is -L'-(6- to 15-membered aryl); L' is a bond;
ring B is 5- to 15-membered heteroaryl, wherein the 5- to 15-membered heteroaryl contains 1, 2, or 3 heteroatoms being one or more of O, S, and N;
each R^{2a'} is independently hydroxy, C₁-C₆ alkyl, C₂-C₆ alkynyl, or -O-(3- to 8-membered heterocycloalkyl).

Preferably, G1' is G1'-I-a1 or G1'-III:

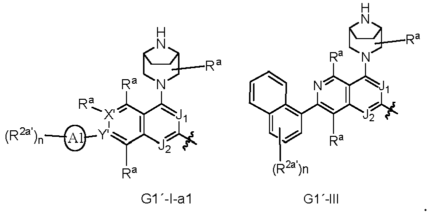

Preferably, in certain embodiments of the present disclosure, each ring B is independently 6- to 15-membered aryl, 5- to 15-membered heteroaryl, C₅-C₁₅ cycloalkyl, or 5- to 16-membered heterocycloalkyl, and each R^{a} is independently halogen, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -NH(C₁-C₆ alkyl)₂-N(C₁-C₆ alkyl)₂, -NH(C₃-C₈ cycloalkyl), or -N(C₃-C₈ cycloalkyl)₂.

Preferably, each ring B is independently 6- to 15-membered aryl, 5- to 15-membered heteroaryl, or 5- to 16-membered heterocycloalkyl, and each R^{a} is independently halogen, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -C₃-C₈ heterocycloalkyl (e.g., 3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂ substitution.

More preferably, each ring B is independently 6- to 15-membered aryl, 5- to 15-membered heteroaryl, or 5- to 16-membered heterocycloalkyl, and each R^{a} is independently halogen, hydroxy, amino, cyano, -C₁-C₆ alkyl, -C₁-C₆ heteroalkyl, -C₃-C₆ alkenyl, -C₃-C₆ alkynyl, -C₃-C₈ heterocycloalkyl (e.g., 3- to 8-membered heterocycloalkyl), -O(C₁-C₆ alkyl), -S(C₁-C₆ alkyl), or -N(C₁-C₆ alkyl)₁₋₂ substitution (e.g., F, Cl, hydroxy, cyano, or methyl).

Preferably, in certain embodiments of the present disclosure, the structural unit 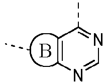 is 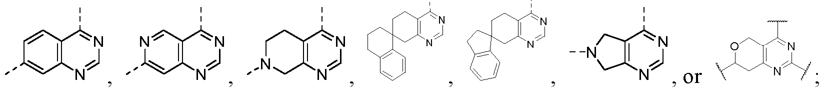 the structural unit 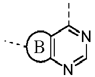 (i.e., a structural unit as defined in this scheme) is optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂.

Preferably, in certain embodiments of the present disclosure, the structural unit 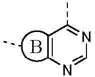 is 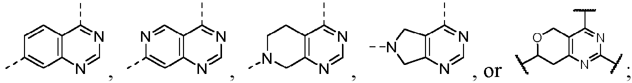 the structural unit 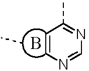 (i.e., a structural unit as defined in this scheme) is optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -C₃-C₈ heterocycloalkyl (e.g., 3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂.

More preferably, in certain embodiments of the present disclosure, the structural unit 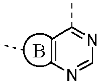 is 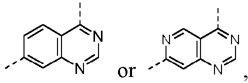 and the structural unit 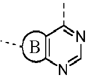 (i.e., a structural unit as defined in this scheme) is optionally substituted with F, Cl, Br, I, hydroxy, amino, cyano, -C₁-C₆ alkyl, -C₁-C₆ heteroalkyl, -C₃-C₆ alkenyl, -C₃-C₆ alkynyl, -C₃-C₈ heterocycloalkyl (e.g., 3- to 8-membered heterocycloalkyl), -O(C₁-C₆ alkyl), -S(C₁-C₆ alkyl), or -N(C₁-C₆ alkyl)₁₋₂.

Preferably, in certain embodiments of the present disclosure, the structural unit 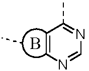 is

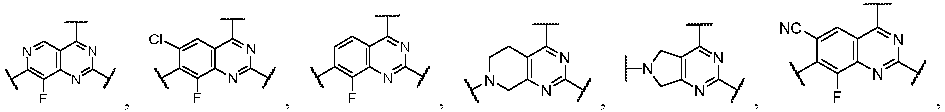

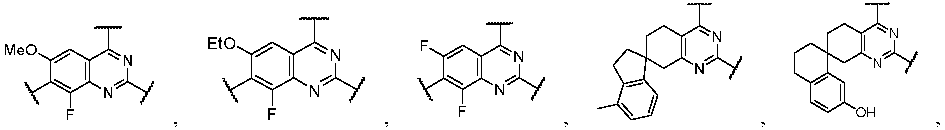

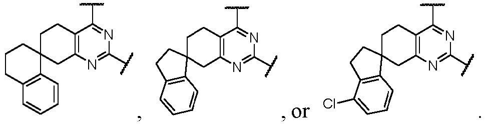

Preferably, in certain embodiments of the present disclosure, the structural unit 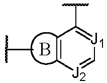 is 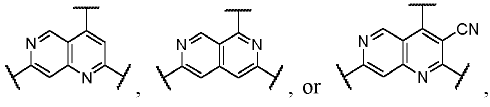 and the structural unit 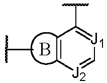 is optionally substituted with F, Cl, Br, I, hydroxy, amino, cyano, -C₁-C₆ alkyl, -C₁-C₆ heteroalkyl, -C₃-C₈ cycloalkyl, - C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -NH(C₁-C₆ alkyl), -N(C₁-C₆ alkyl)₂, -NH(C₃-C₈ cycloalkyl), or -N(C₃-C₈ cycloalkyl)₂.

Preferably, in certain embodiments of the present disclosure, the structural unit

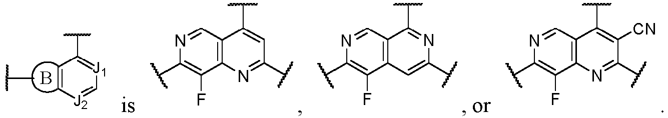

Preferably, in certain embodiments of the present disclosure, each ring A1 is C₆-C₁₅ aryl or 5- to 15-membered heteroaryl; R^{2a} and R^{2a'} are independently hydroxy, amino, halogen, C₁-C₆ alkyl, C₁-C₆ heteroalkyl, -S-C₁-C₆ alkyl, -O-C₁-C₆ alkyl, C₃-C₈ cyclopropyl, C₂-C₆ alkynyl, or -O-(3- to 8-membered heterocycloalkyl), wherein the C₁-C₆ alkyl, -S-C₁-C₆ alkyl, and C₃-C₈ cyclopropyl are optionally substituted with halogen, CN, -OH, -NH₂, or C₁-C₃ alkyl.

Preferably, R^{2a} and R^{2a'} are independently hydroxy, amino, halogen, C₁-C₆ alkyl, C₁-C₆ heteroalkyl, -S-C₁-C₆ alkyl, -O-C₁-C₆ alkyl, C₃-C₈ cyclopropyl, or C₂-C₆ alkynyl.

Preferably, in certain embodiments of the present disclosure, each ring A1 is C₆-C₁₅ aryl or 5- to 15-membered heteroaryl, wherein the C₆-C₁₅ aryl or 5- to 15-membered heteroaryl is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 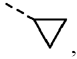 or -SCH₃, the -CH₃, -CH₂CH₃, -CH₂CH₂CH₃, -CH(CH₃)₂-, 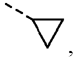 or -SCH₃ being optionally substituted with halogen, CN, -OH, -NH₂, or C₁-C₃ alkyl.

Preferably, R^{2a} and R^{2a'} are independently hydroxy, amino, F, Cl, methyl, ethyl, isopropyl, trifluoromethyl, 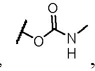 -SCH₃, 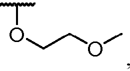 cyclopropyl, or

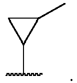

More preferably, in certain embodiments of the present disclosure, ring A1 is phenyl, pyridyl, naphthyl, 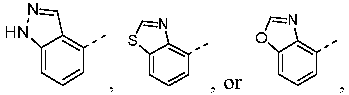 and ring A1 (i.e., a group as defined in this scheme) is optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH , 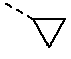 , or -SCH₃, the -CH₃, -CH₂CH₃, -CH₂CH₂CH₃, -CH(CH₃)₂-, 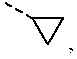 or - SCH₃ being optionally substituted with halogen, CN, -OH, -NH₂, or C₁-C₃ alkyl.

Preferably, in certain embodiments of the present disclosure, 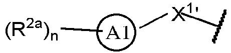 is absent,

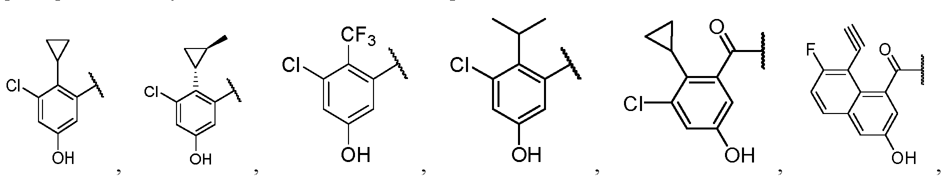

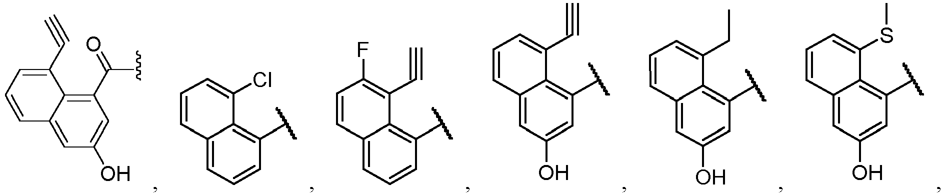

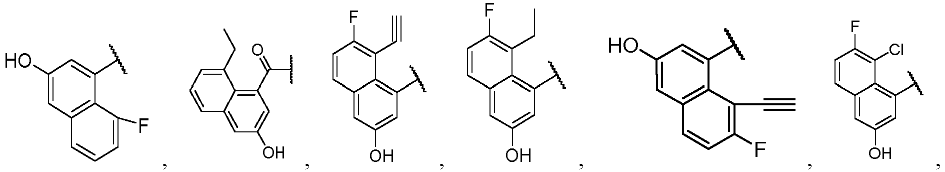

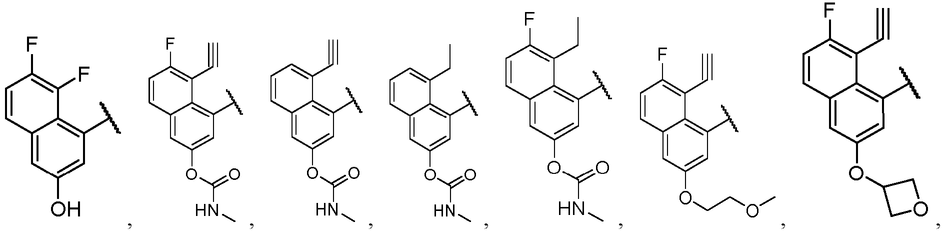

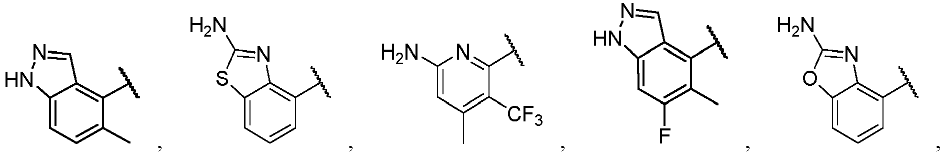

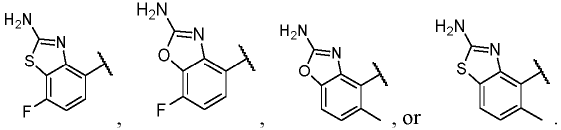

Preferably, in certain embodiments of the present disclosure, the structural unit 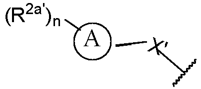 is

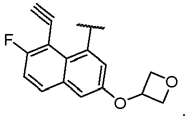

Preferably, in certain embodiments of the present disclosure, the structural unit 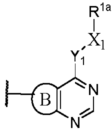 is 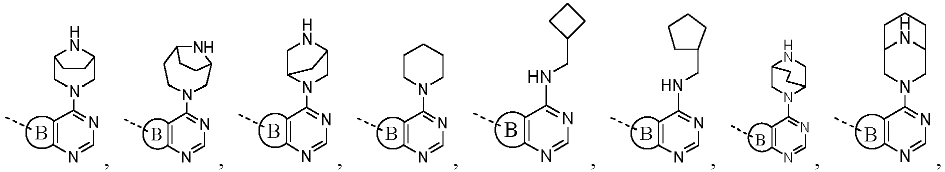 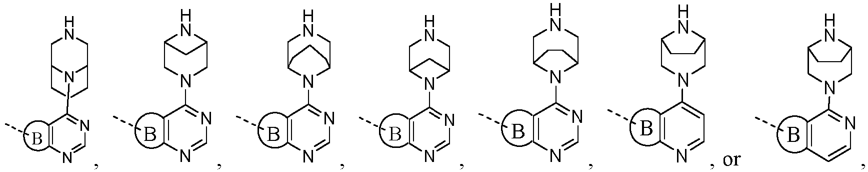 and the structural unit 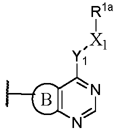 (i.e., a structural unit as defined in this scheme) is optionally substituted with 1, 2, or 3 R^{a}.

More preferably, in certain embodiments of the present disclosure, the structural unit 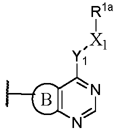 is 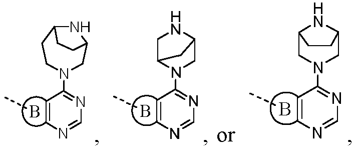 and the structural unit 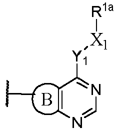 (i.e., a structural unit as defined in this scheme) is optionally substituted with 1 or 2 R^{a}.

Preferably, in certain embodiments of the present disclosure, the structural unit 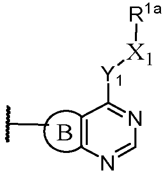 is

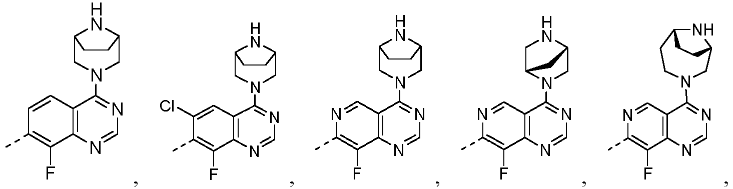

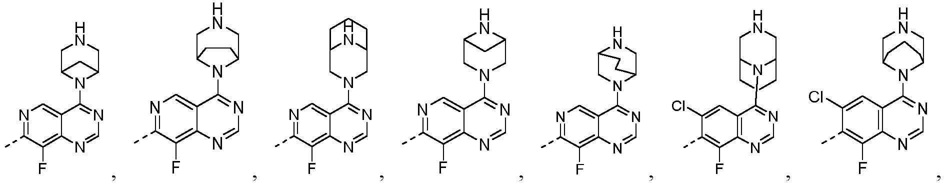

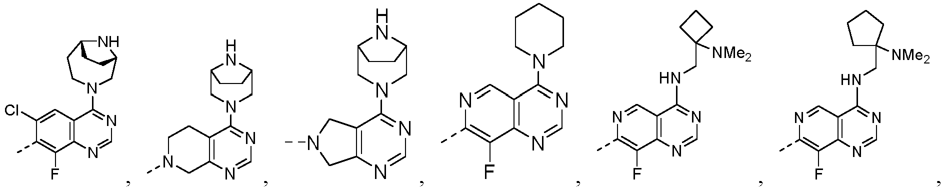

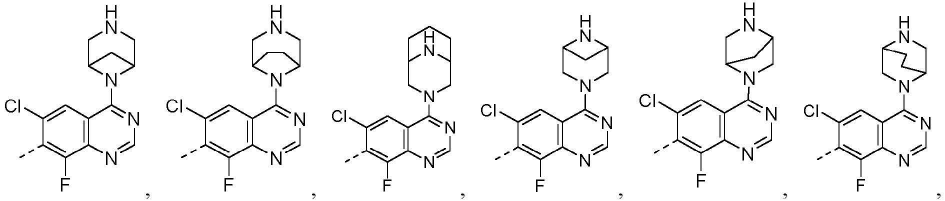

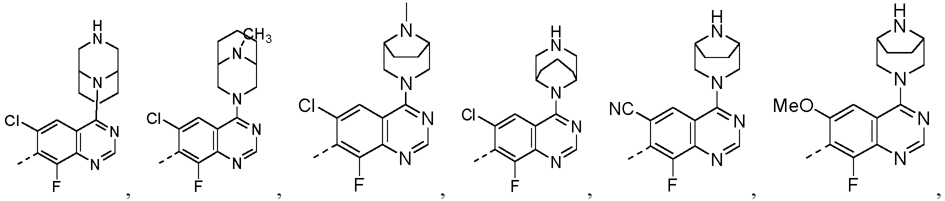

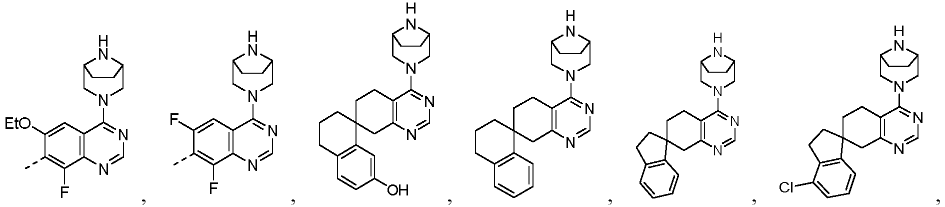

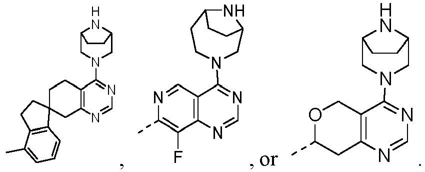

Preferably, in certain embodiments of the present disclosure, the structural unit 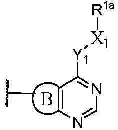 is

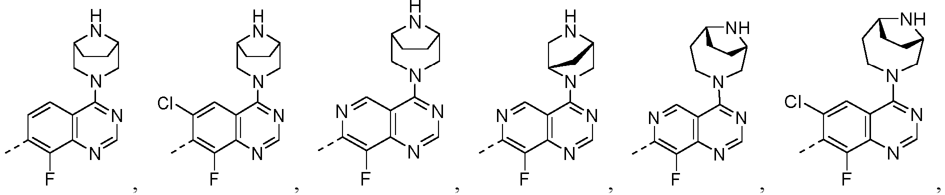

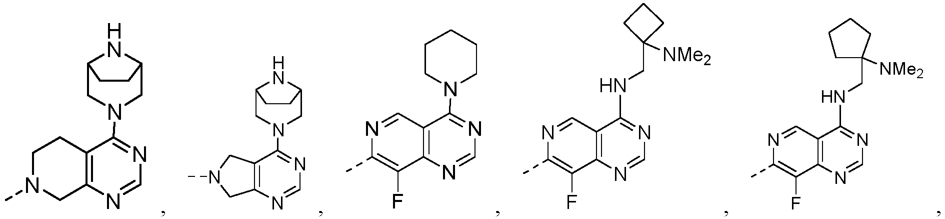

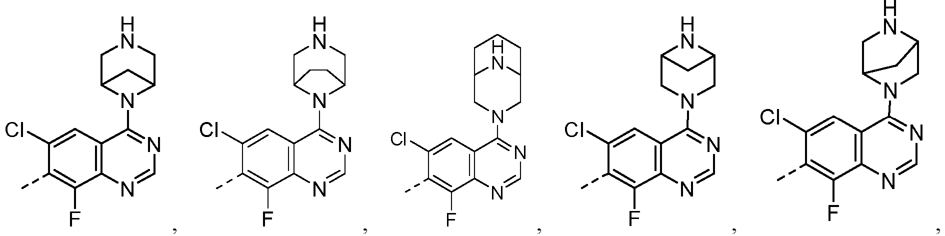

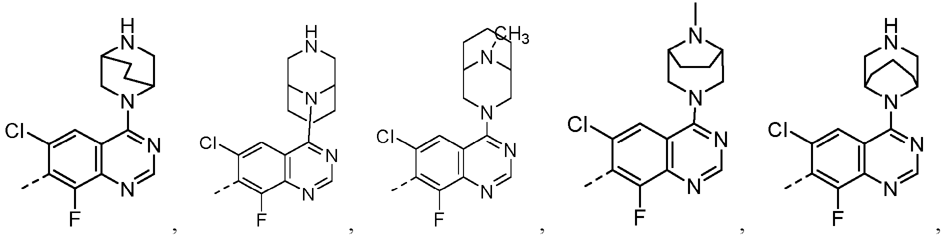

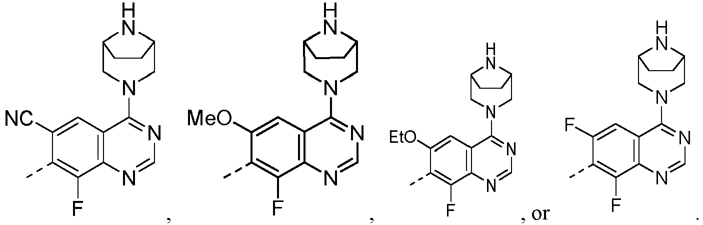

More preferably, in certain embodiments of the present disclosure, the structural unit

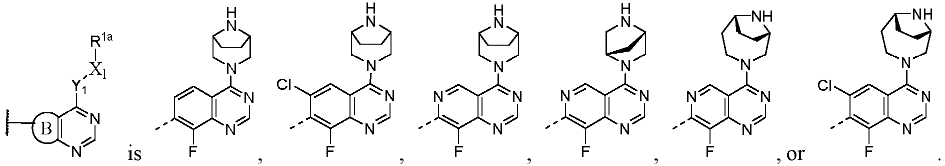

Preferably, in certain embodiments of the present disclosure, the structural unit 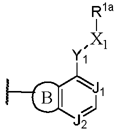 is

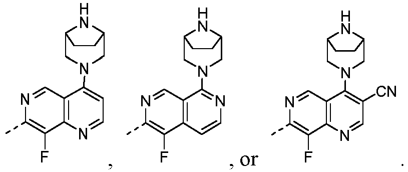

Preferably, in certain embodiments of the present disclosure, the structural unit 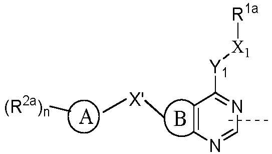 is: 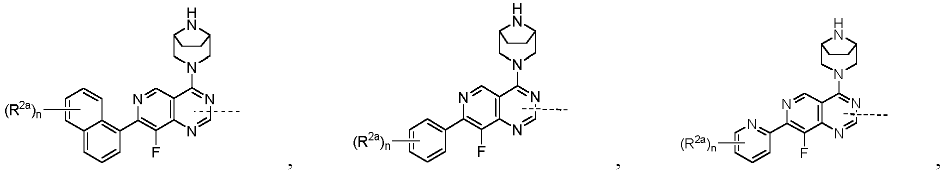 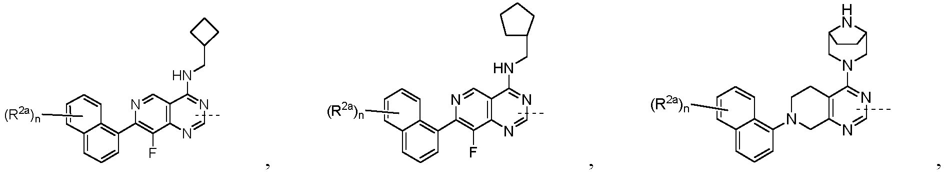 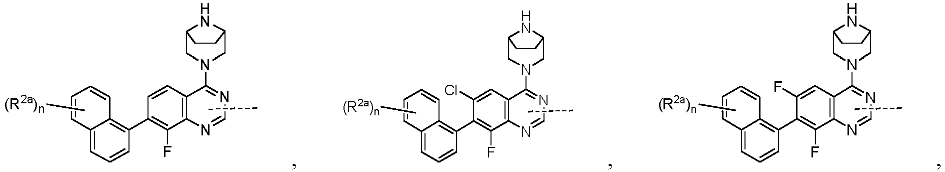 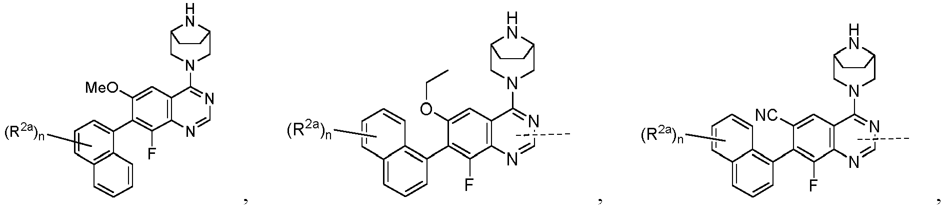 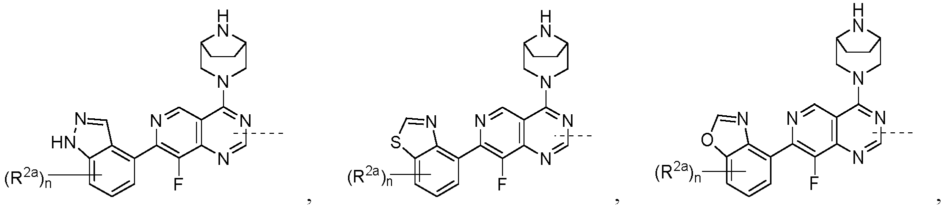 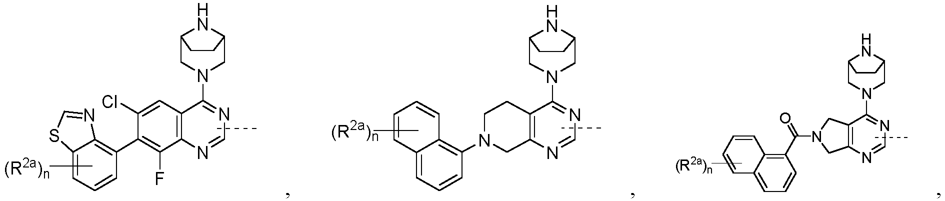 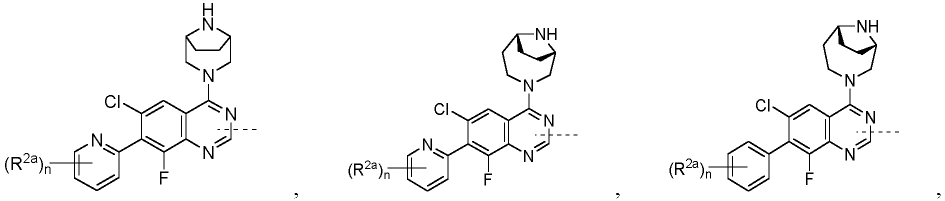 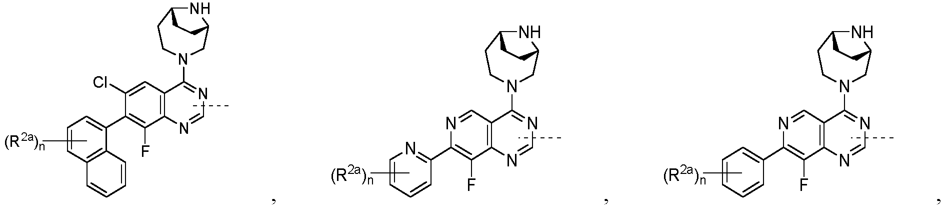 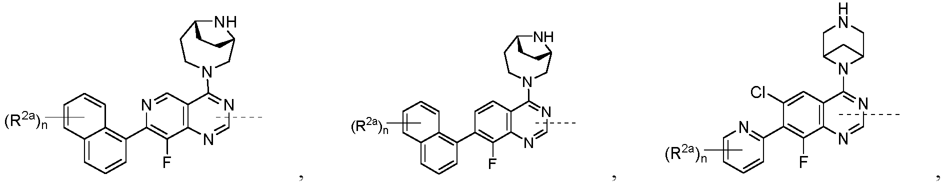 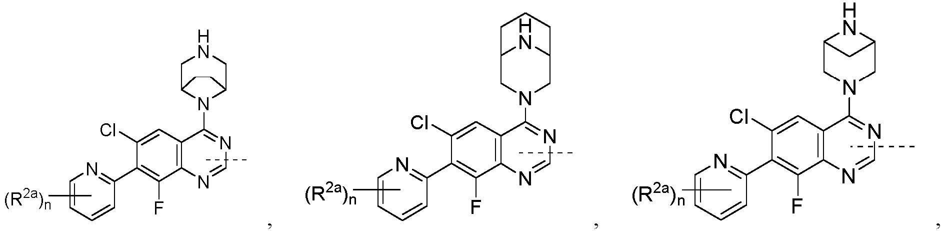 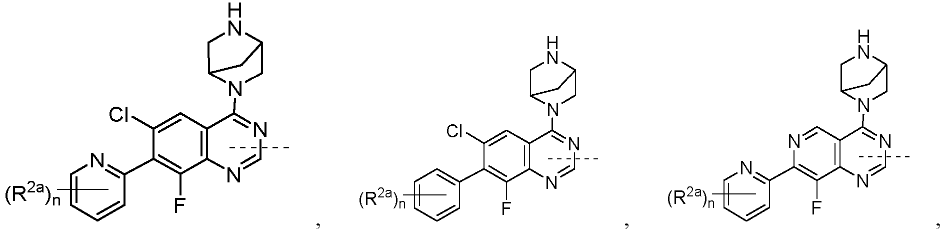 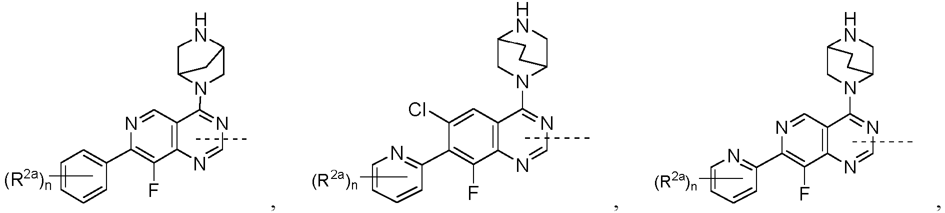 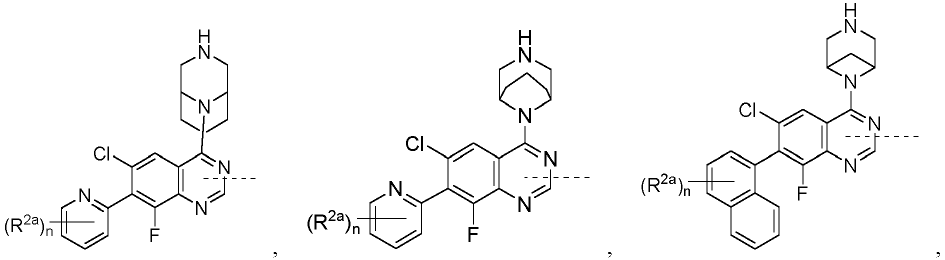 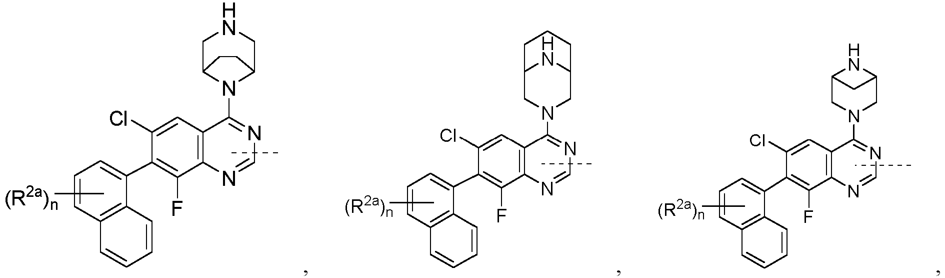 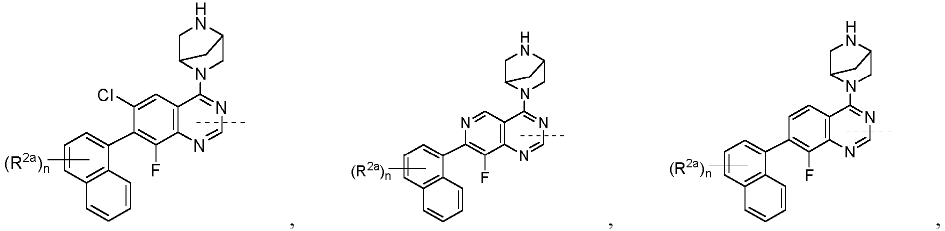 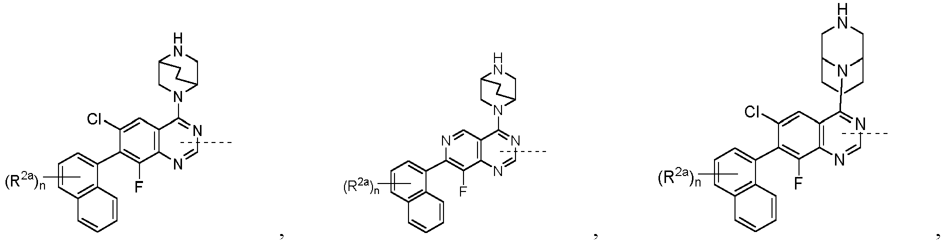 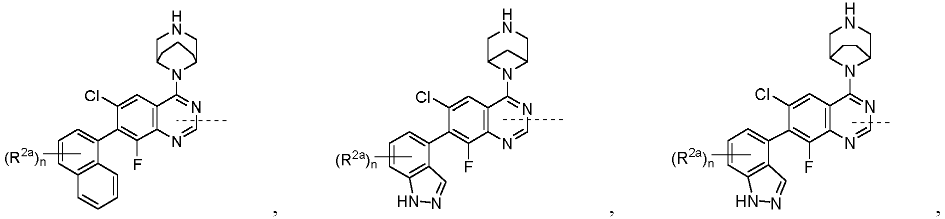 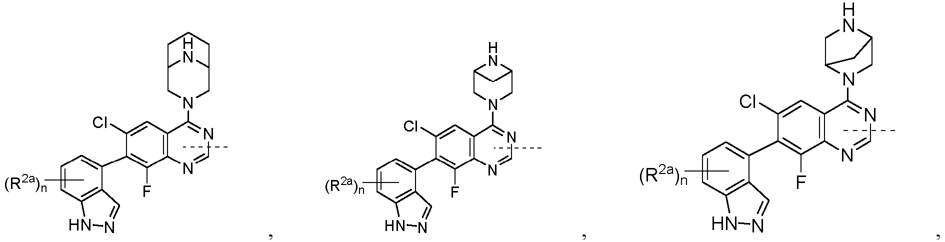 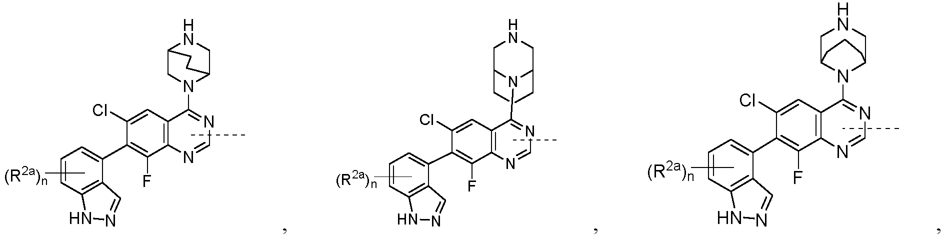 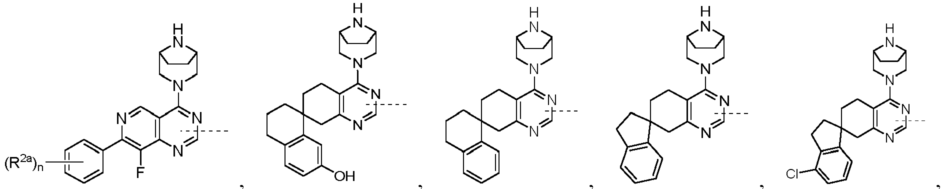 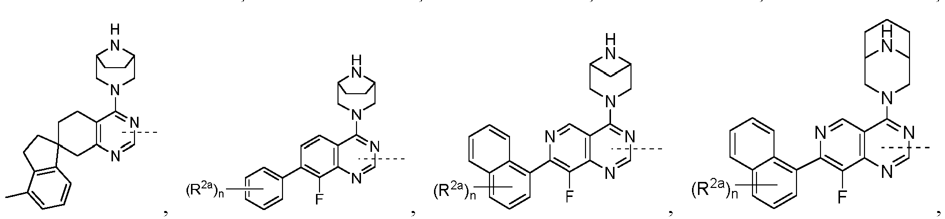 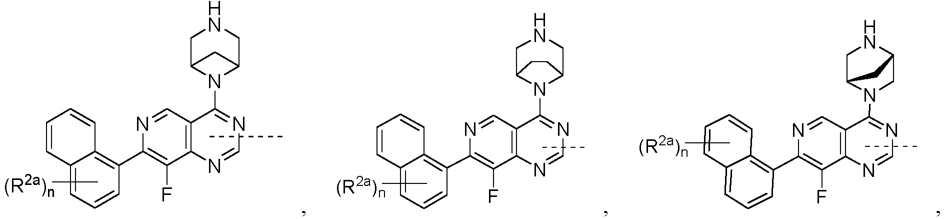 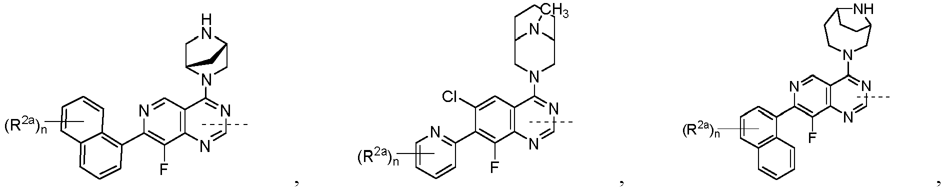 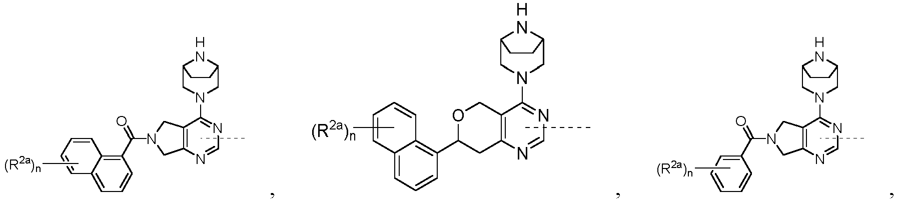 or 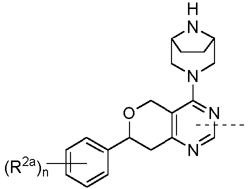 , and the structural unit 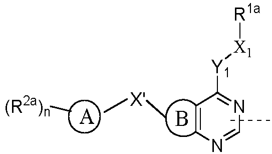 (i.e., a structural unit as defined in this scheme) is optionally substituted with 1, 2, or 3 R^{a}.

Preferably, in certain embodiments of the present disclosure, the structural unit 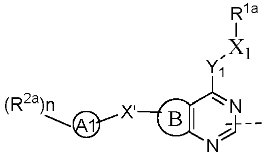 is: 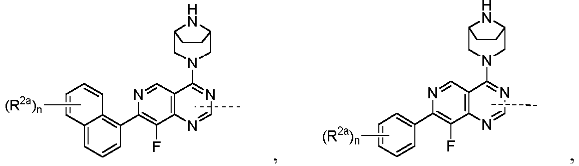 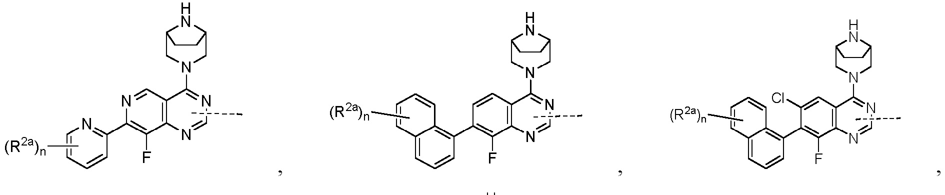 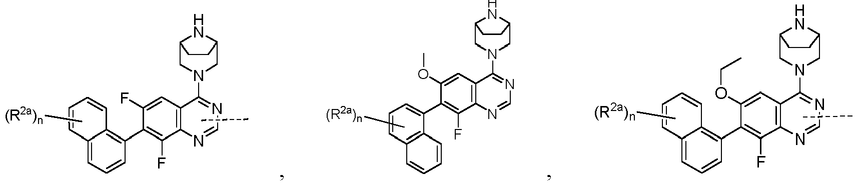 , or 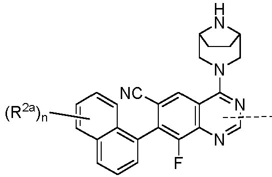 , and the structural unit 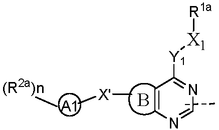 (i.e., a structural unit as defined in this scheme) is optionally substituted with 1 or 2 R^{a}. Preferably, R^{a} is C₁-C₆ alkyl optionally substituted with 1 or more hydroxy, such as -CH₂OH.

Preferably, in certain embodiments of the present disclosure, the structural unit 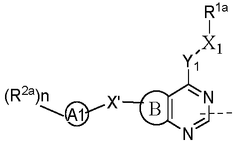 is:

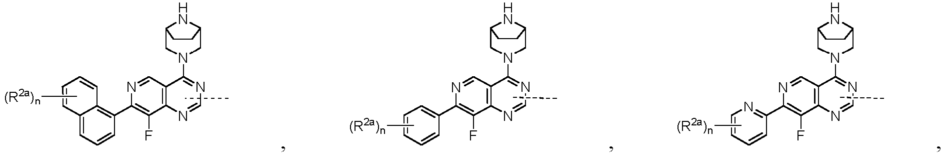

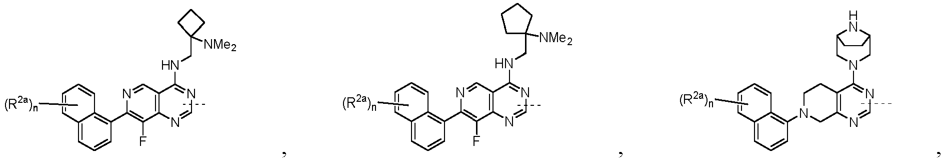

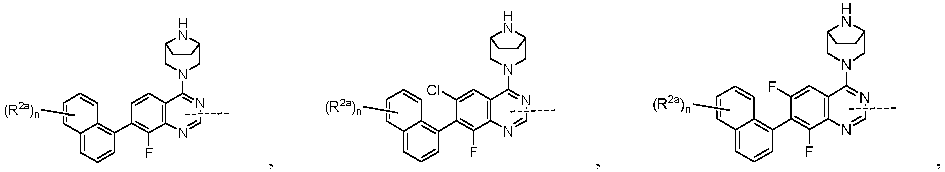

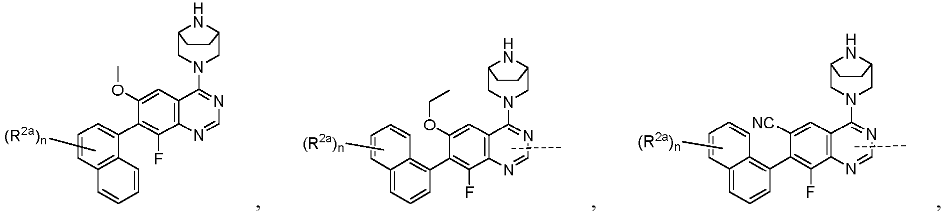

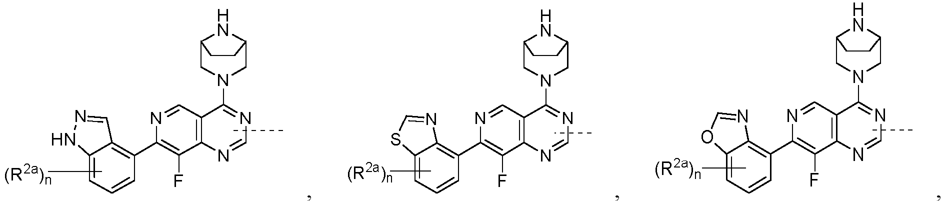

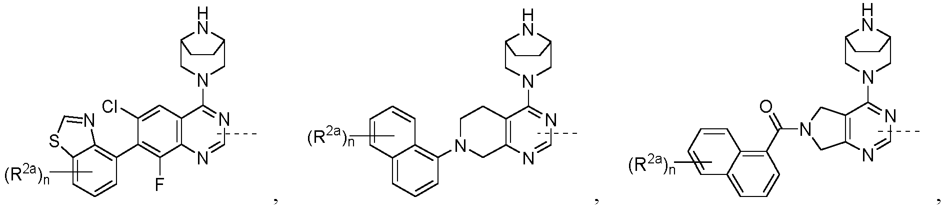

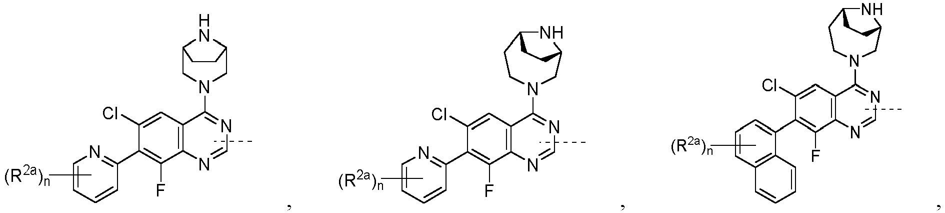

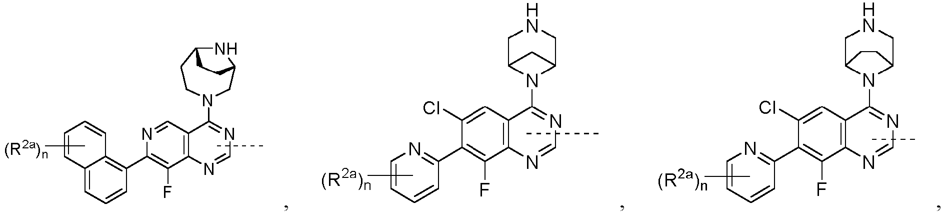

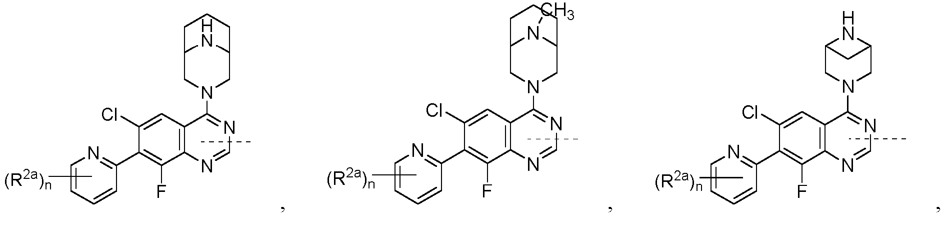

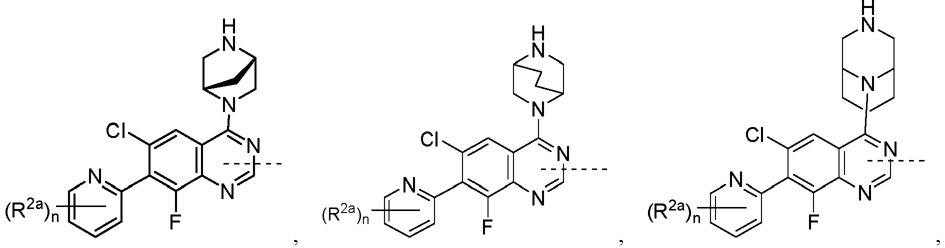

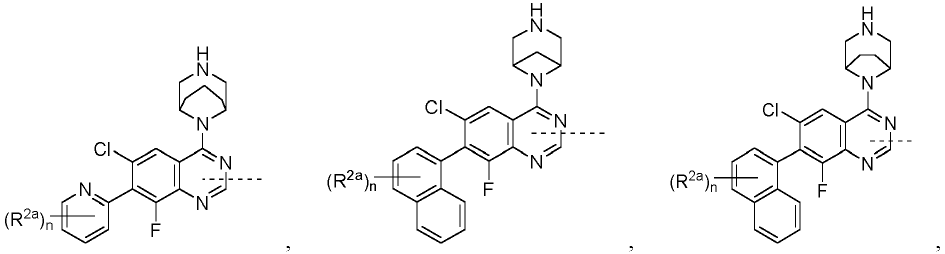

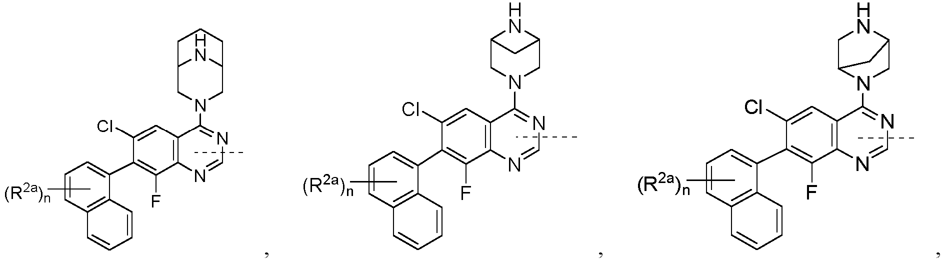

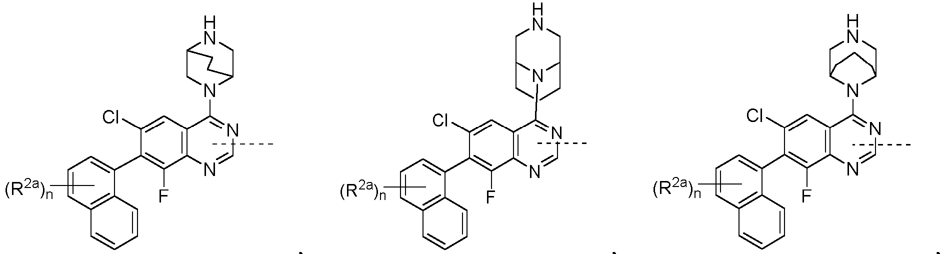

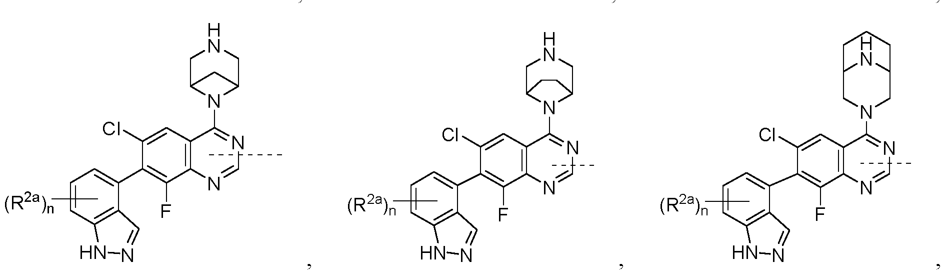

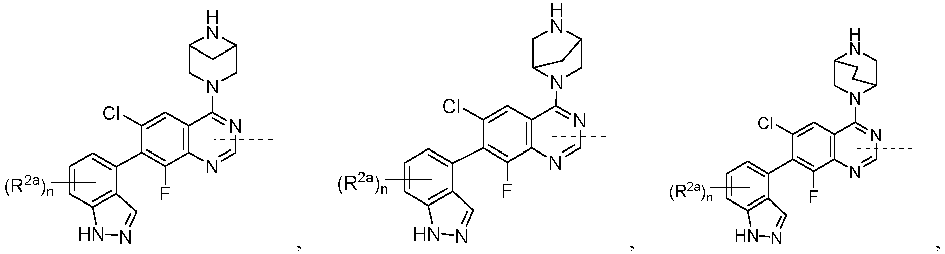

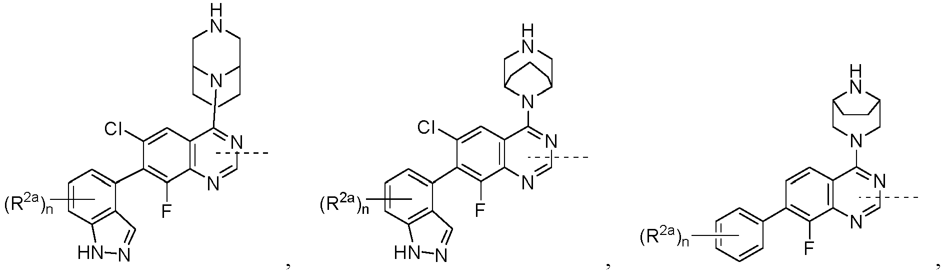

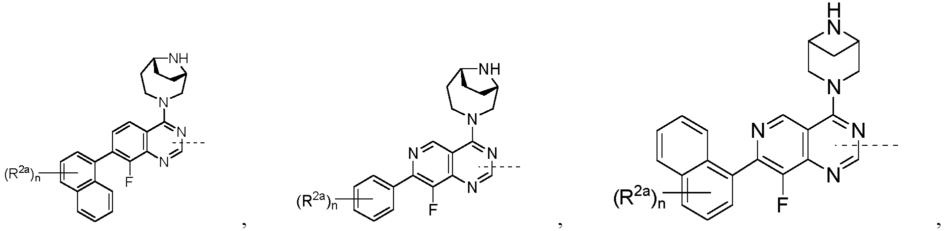

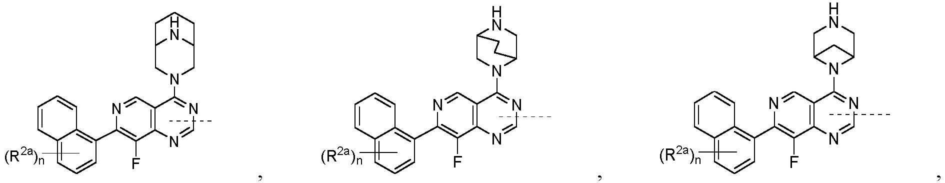

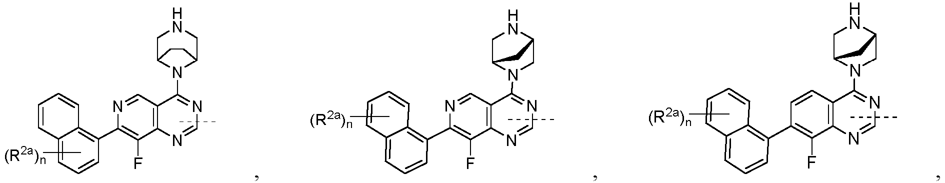

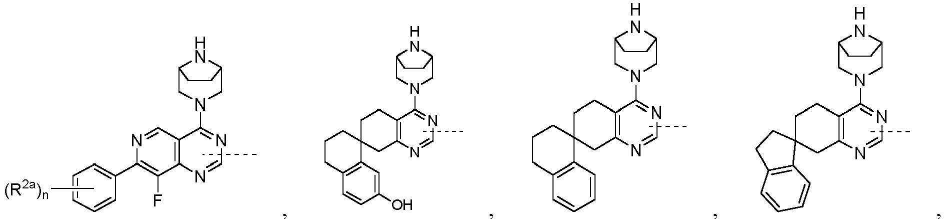

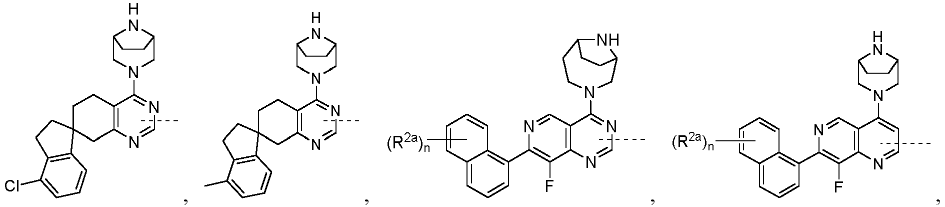

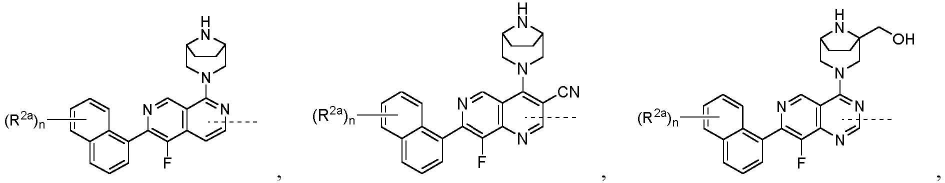

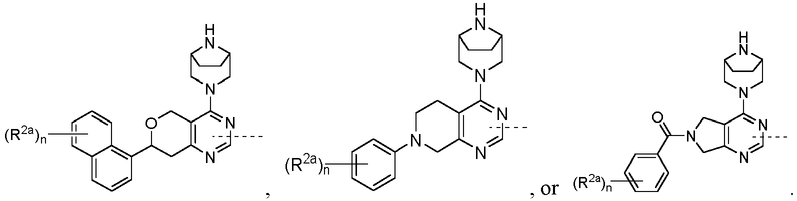

Preferably, in certain embodiments of the present disclosure, the structural unit 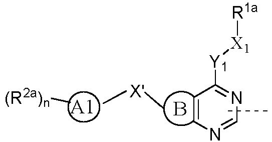 is: 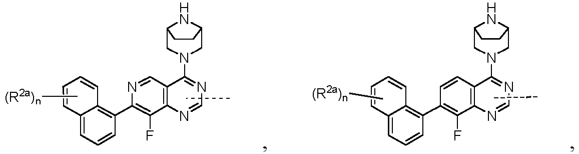 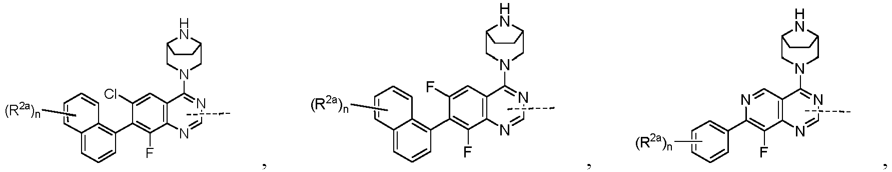 or

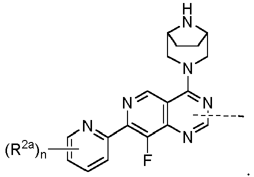

Preferably, in certain embodiments of the present disclosure, the structural unit

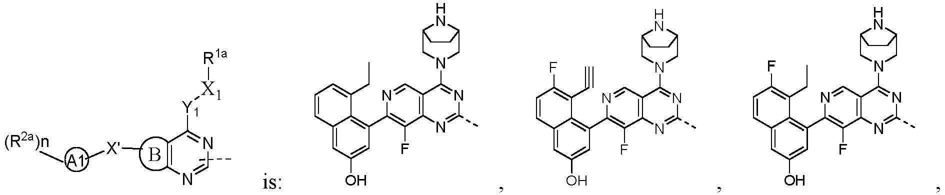

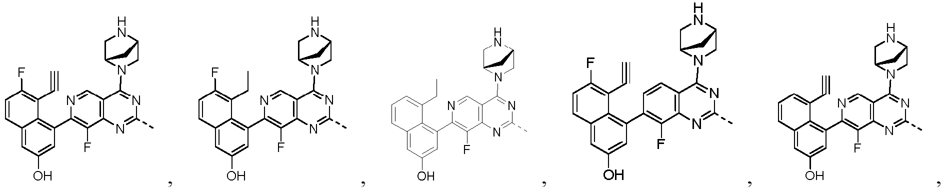

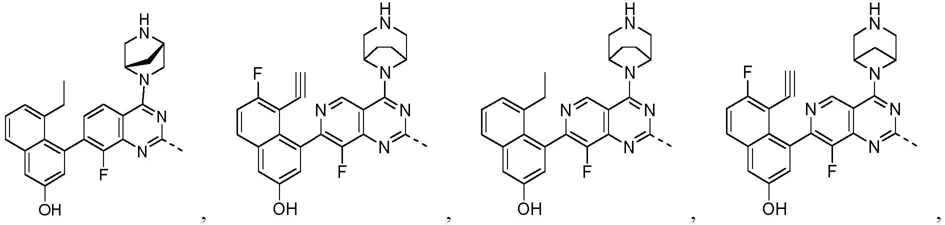

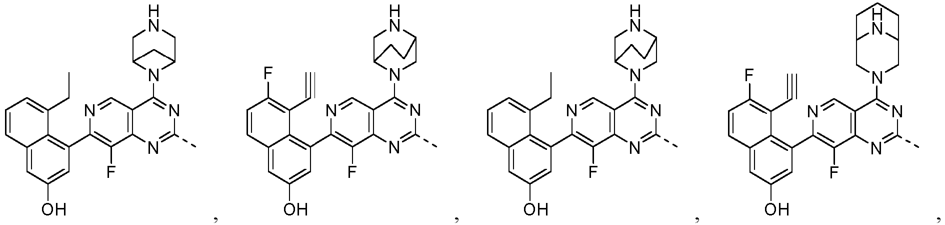

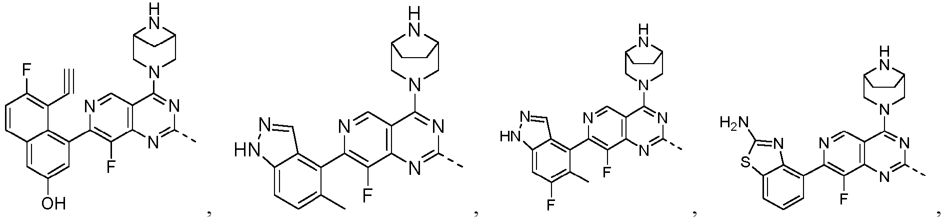

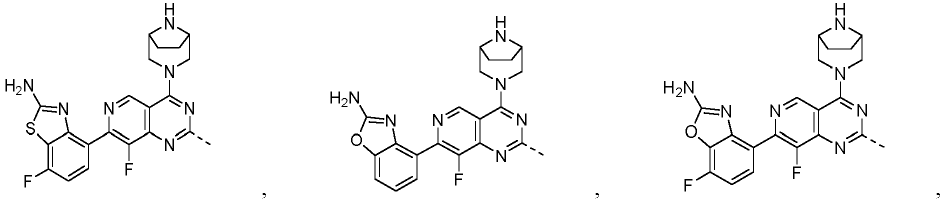

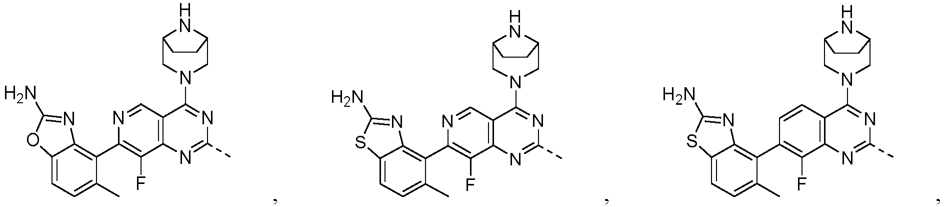

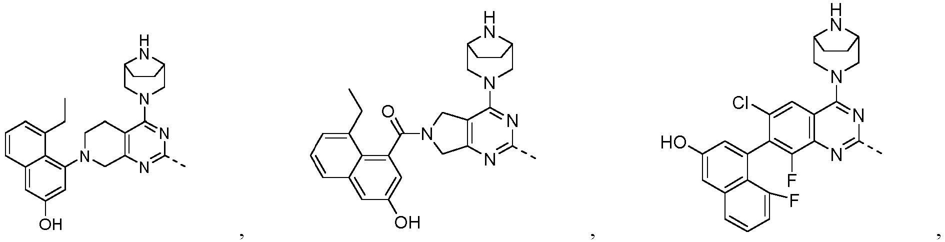

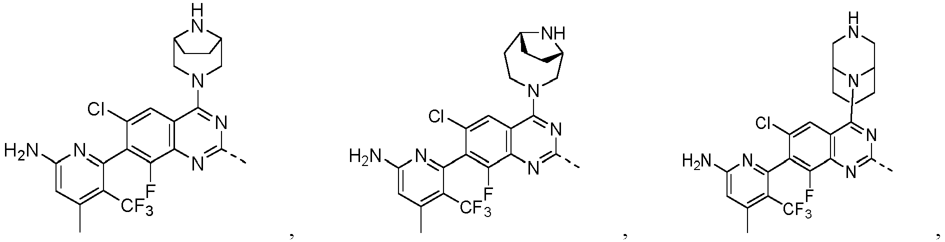

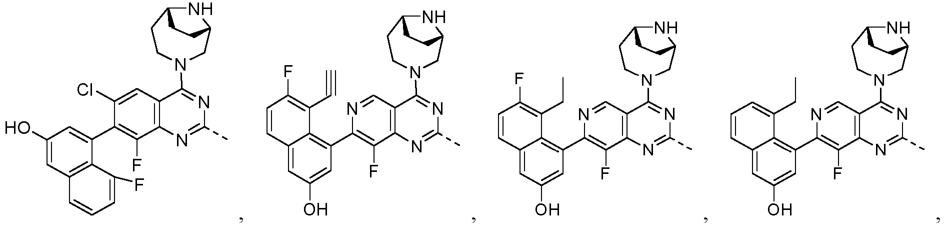

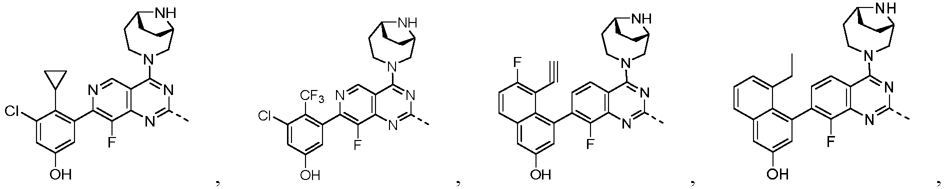

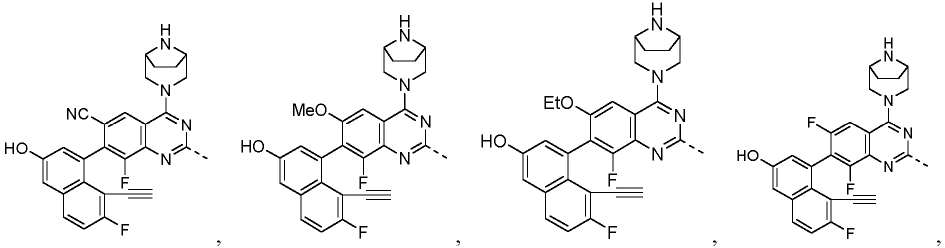

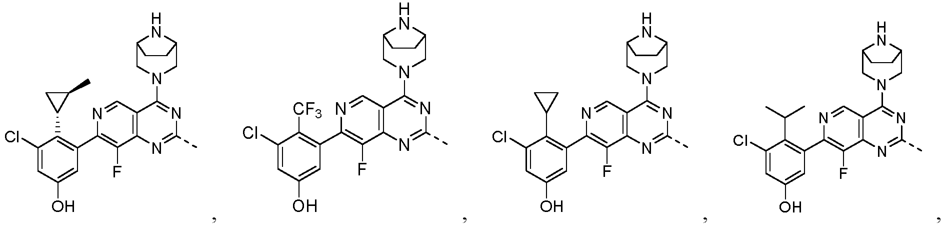

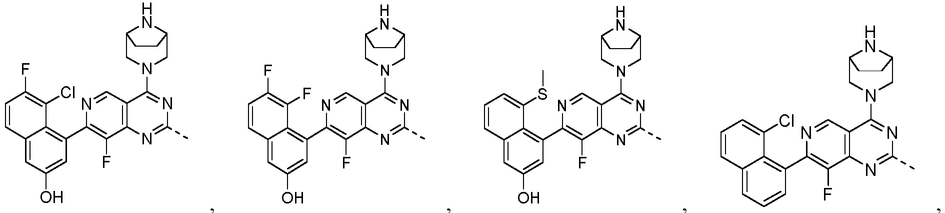

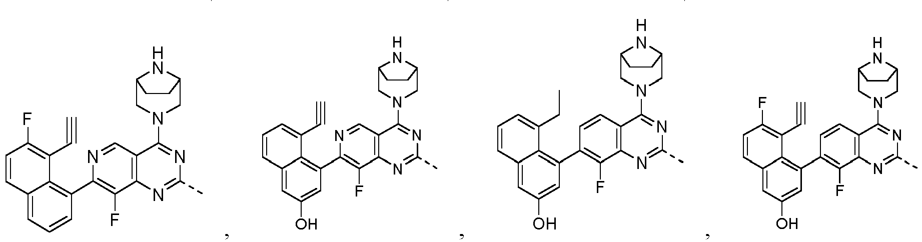

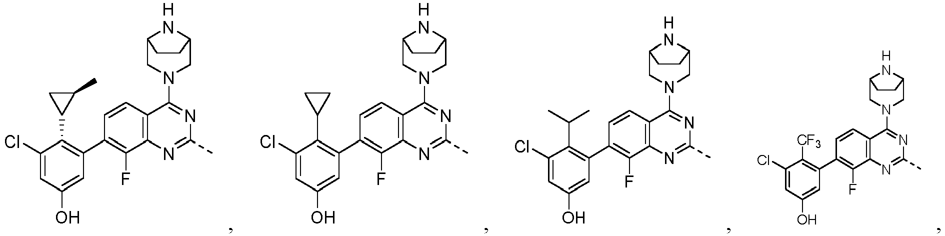

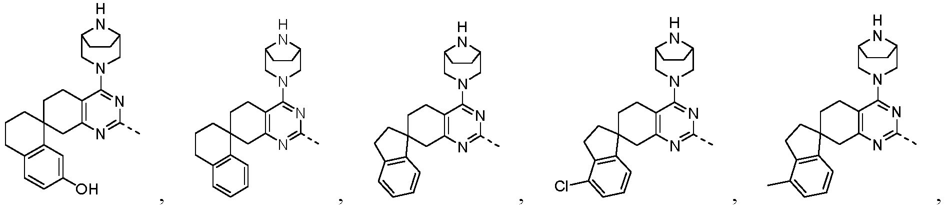

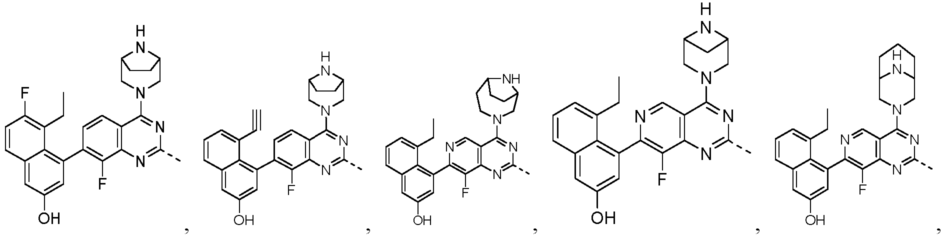

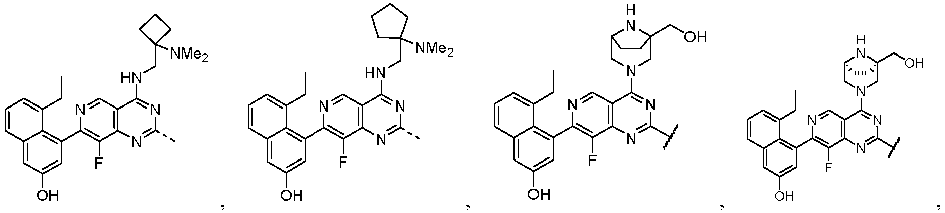

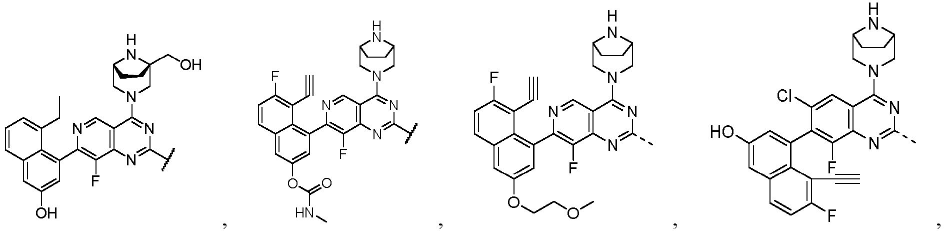

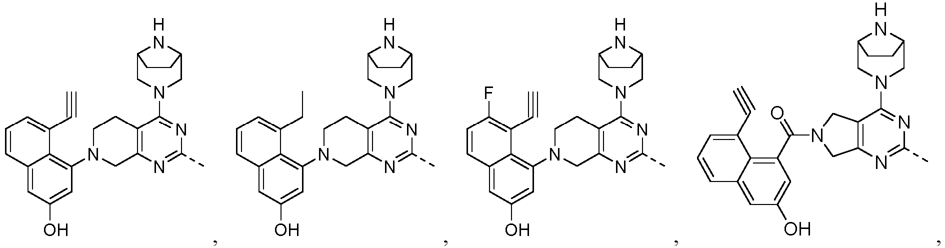

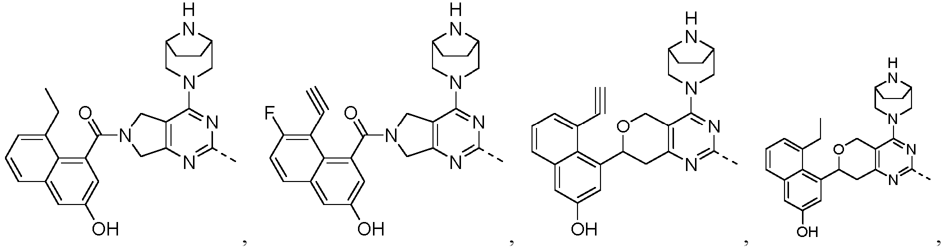

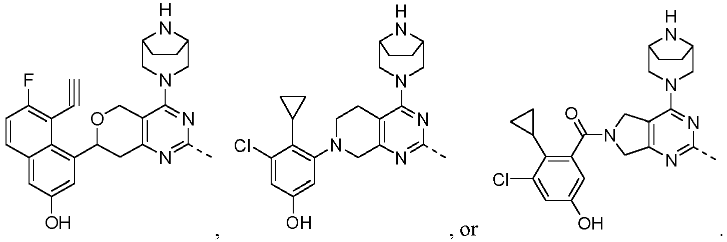

Preferably, in certain embodiments of the present disclosure, the structural unit 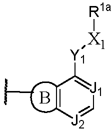 is 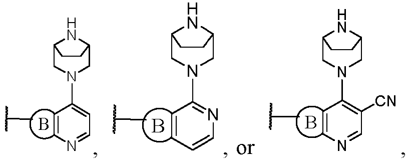 wherein the 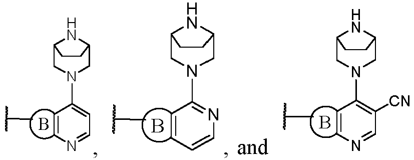 are independently optionally substituted with 1, 2, or 3 R^{a}.

Preferably, in certain embodiments of the present disclosure, the structural unit 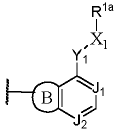 is

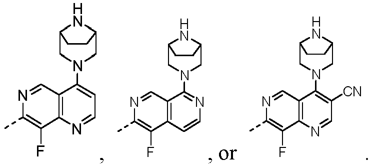

Preferably, in certain embodiments of the present disclosure, the structural unit 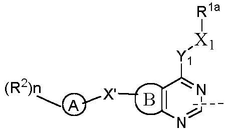 is

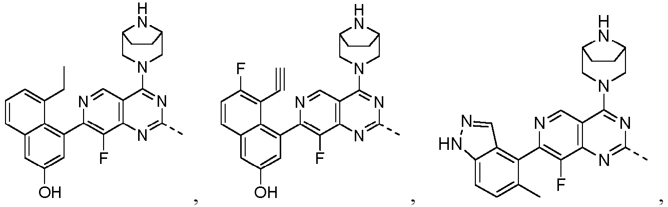

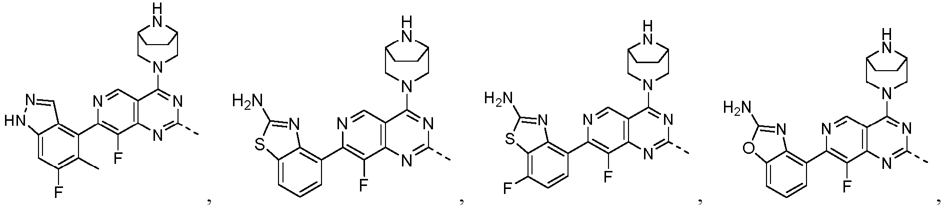

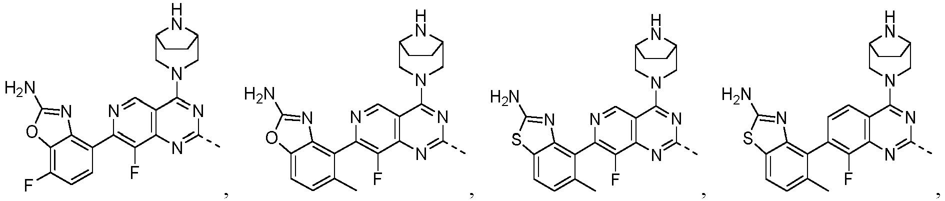

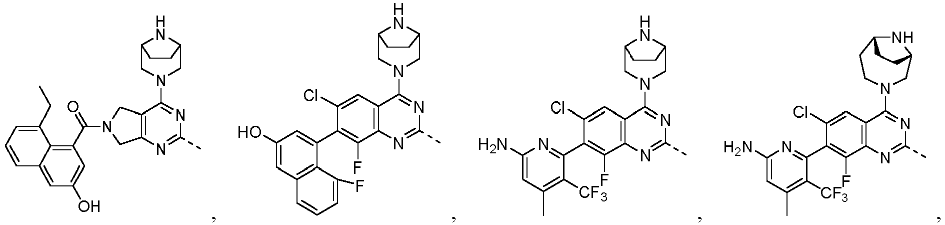

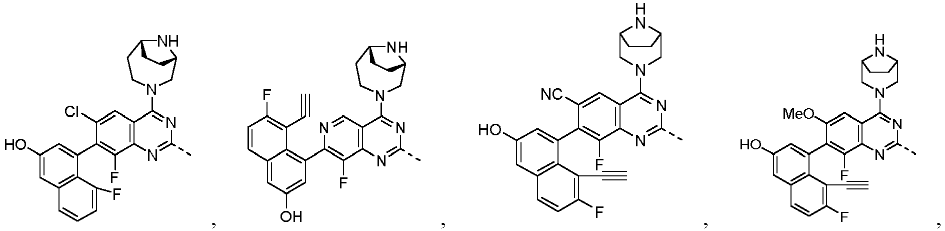

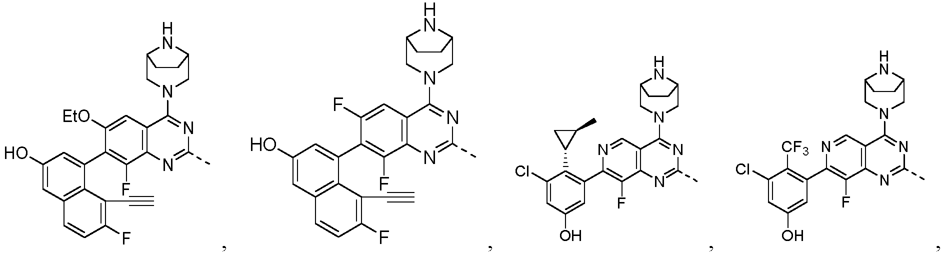

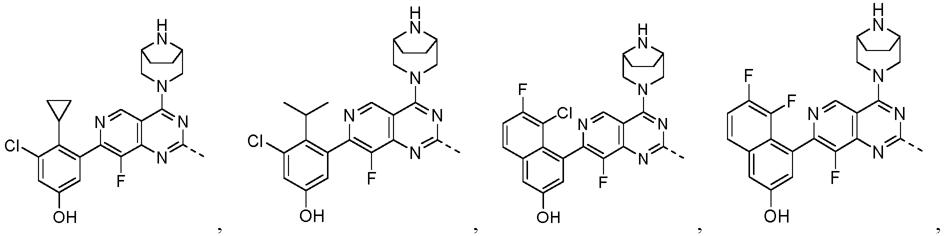

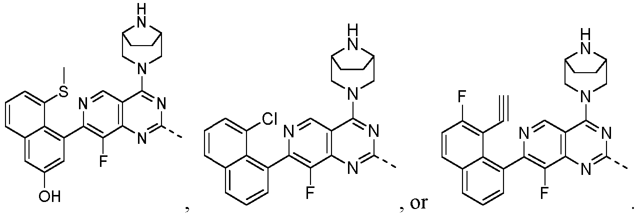

Preferably, in certain embodiments of the present disclosure, the structural unit 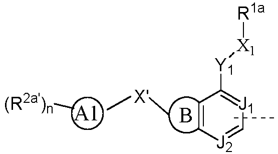 is 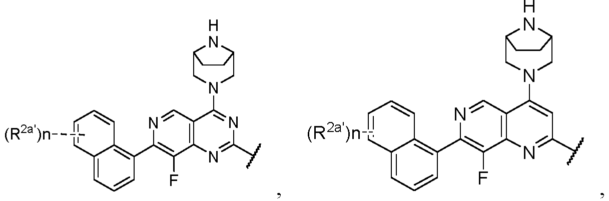 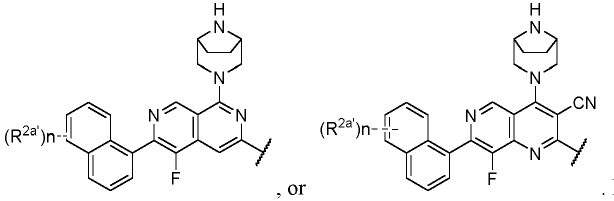 . Preferably, each R^{2a'} is independently halogen, C₁-C₆ alkyl, C₂-C₆ alkynyl, or -O-(3- to 8-membered heterocycloalkyl).

Preferably, in certain embodiments of the present disclosure, the structural unit 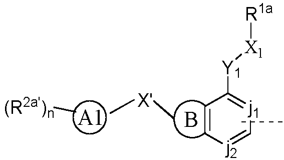 is 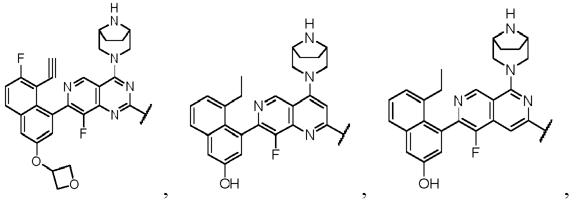 or

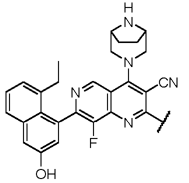

Preferably, in certain embodiments of the present disclosure, L is -(CH₂)ⱼ-, wherein 1 or more methylene groups in the -(CH₂)ⱼ- are optionally replaced by a group selected from -NR^{3'}-, -O-, -CR^{1'}R^{2'}-, - C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8- to 10-membered bicyclic arylene, saturated or partially unsaturated 3- to 7-membered cycloalkylene, saturated or partially unsaturated 5- to 11-membered spirocycloalkylene, saturated or partially unsaturated 5- to 11-membered fused cycloalkylene, saturated or partially unsaturated 8- to 10-membered bicyclic cycloalkylene, saturated or partially unsaturated 4- to 7-membered heterocycloalkylene having 1-2 heteroatoms independently selected from nitrogen, oxygen, and sulfur, saturated or partially unsaturated 5-to 11-membered spiroheterocycloalkylene having 1-2 heteroatoms independently selected from nitrogen, oxygen, and sulfur, saturated or partially unsaturated 5- to 11-membered fused heterocycloalkylene having 1-2 heteroatoms independently selected from nitrogen, oxygen, and sulfur, saturated or partially unsaturated 8- to 10-membered bicyclic heterocycloalkylene having 1-2 heteroatoms independently selected from nitrogen, oxygen, and sulfur, 5- to 6-membered heteroarylene having 1-4 heteroatoms independently selected from nitrogen, oxygen, and sulfur, and 8- to 10-membered bicyclic heteroaryl having 1-5 heteroatoms selected from nitrogen, oxygen, and sulfur, wherein the ethenylene, ethynylene, cycloalkylene, heterocycloalkylene, phenyl, spiroheterocycloalkylene, fused heterocycloalkylene, spirocycloalkylene, fused cycloalkylene, and heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl, and C₃-C₁₀ heterocycloalkyl, the alkyl, cycloalkyl, or heterocycloalkyl being optionally substituted with 1 or more substituents selected from halogen, -OH, -NH₂, -CN, C₁-C₄ alkyl, and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), - NH(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl), or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl; j is 2, 3, 4, 5, 6, 7, 8, 9, 10, 11, or 12.

Preferably, in certain embodiments of the present disclosure, L is:

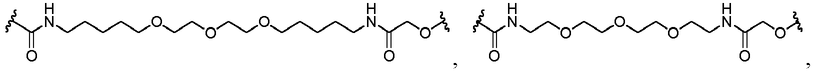

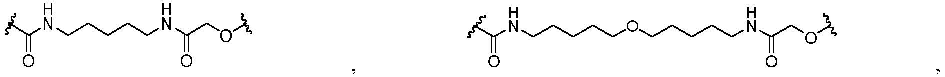

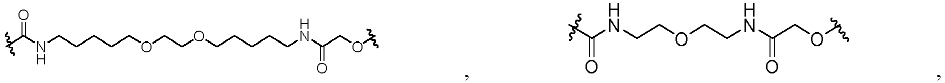

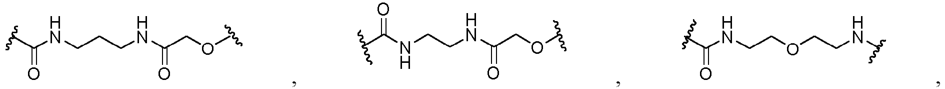

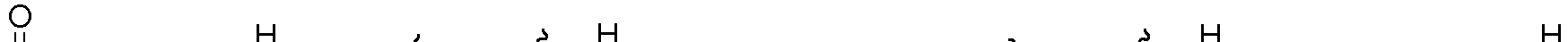

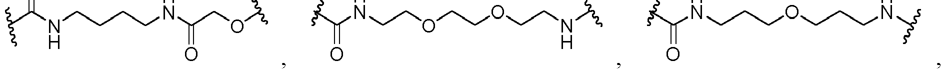

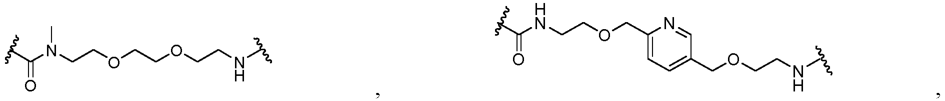

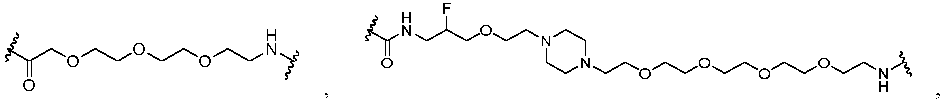

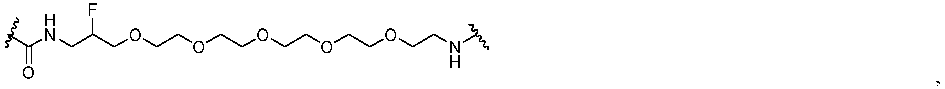

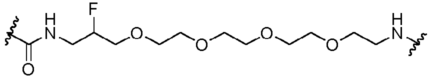

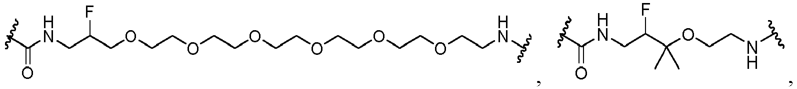

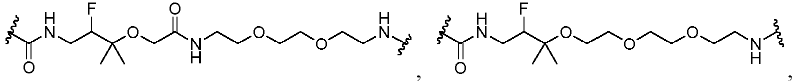

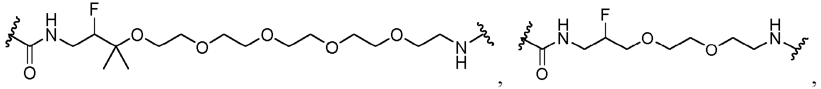

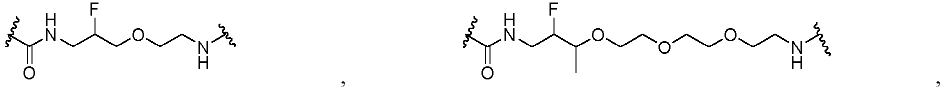

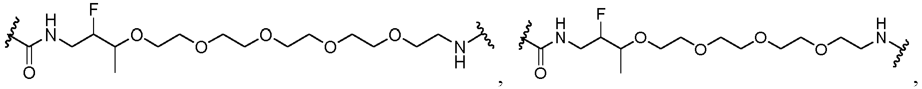

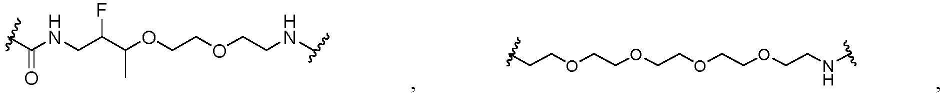

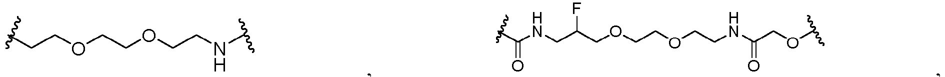

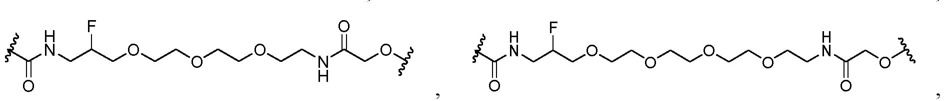

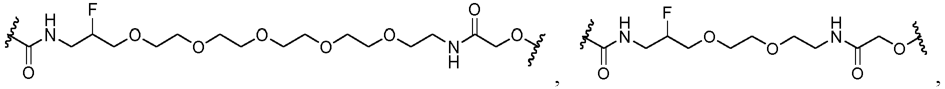

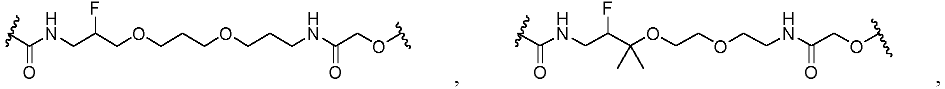

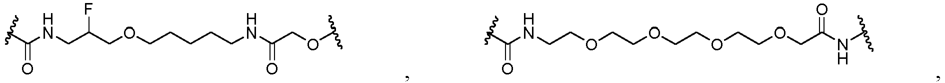

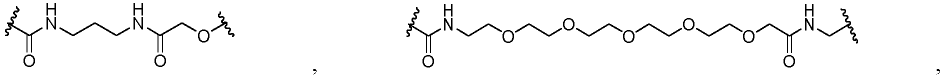

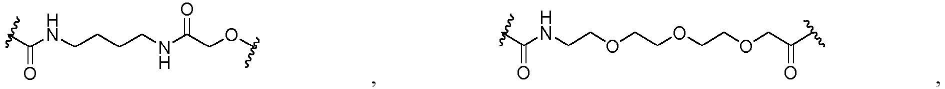

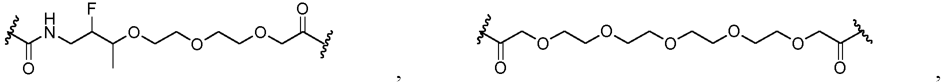

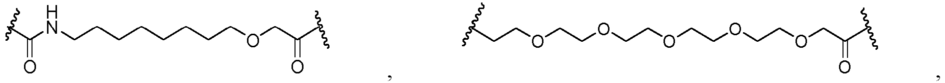

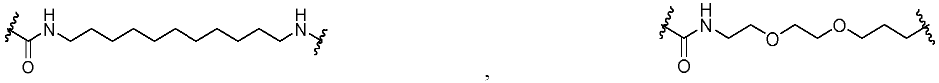

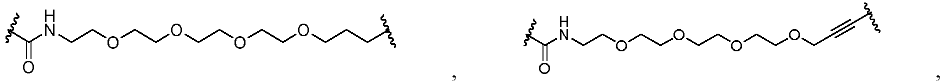

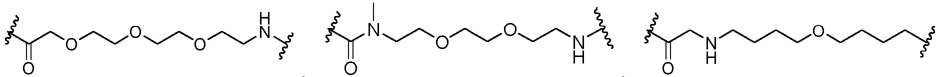

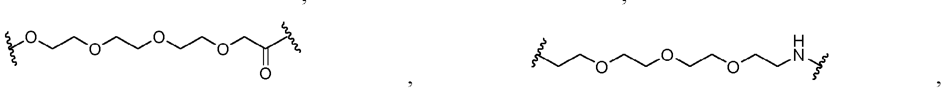

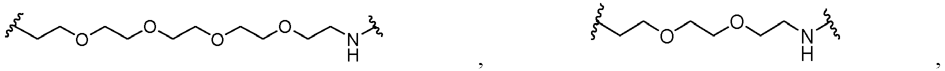

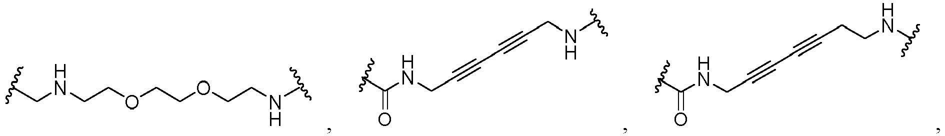

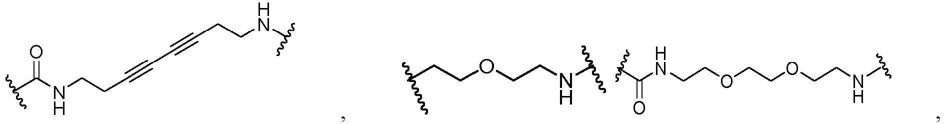

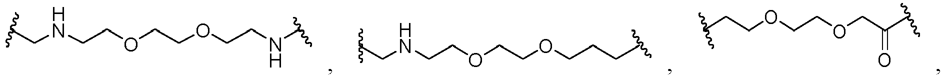

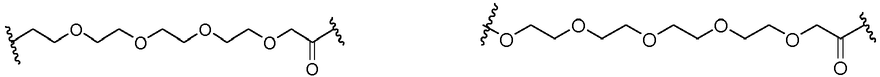

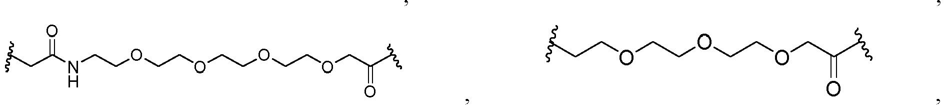

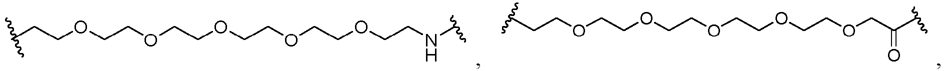

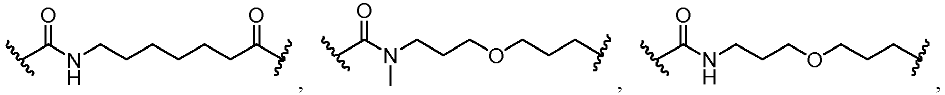

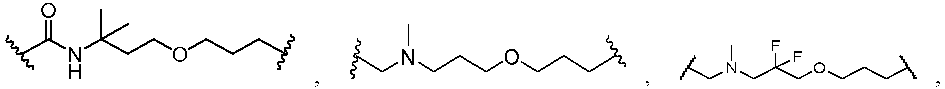

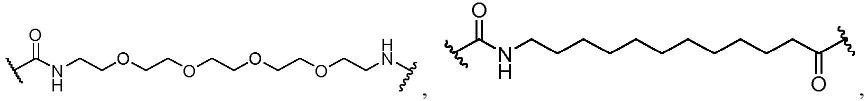

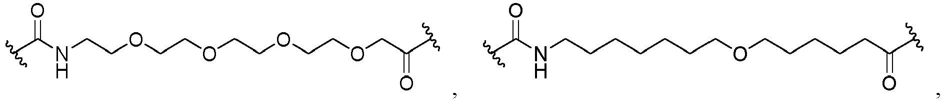

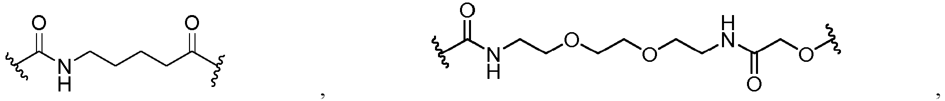

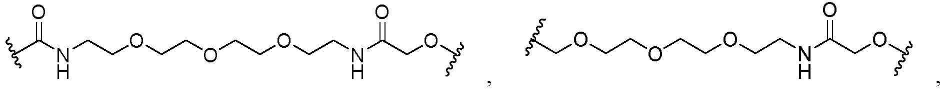

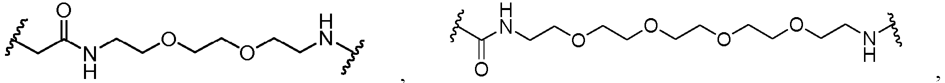

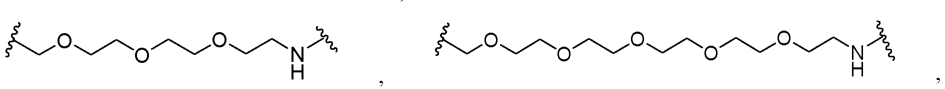

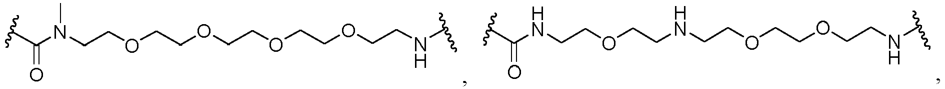

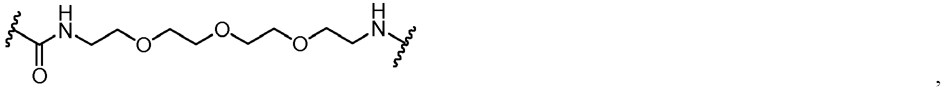

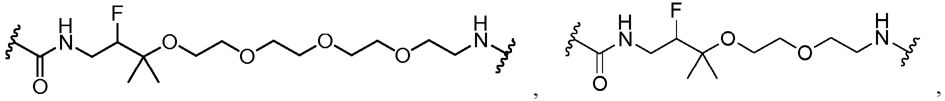

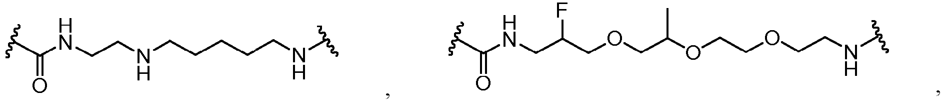

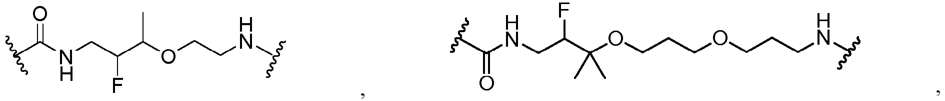

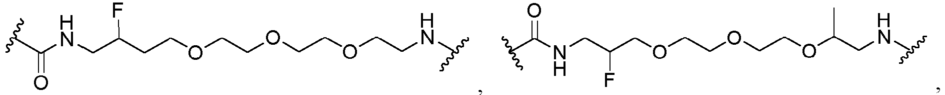

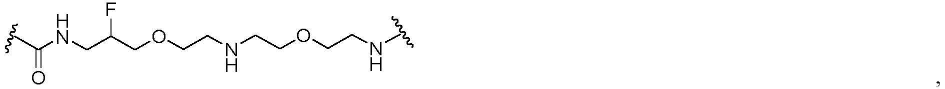

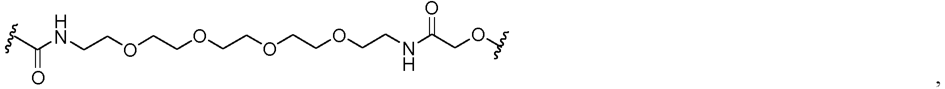

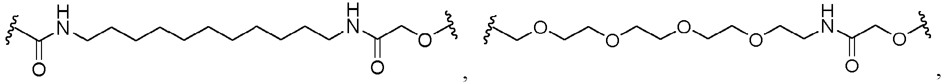

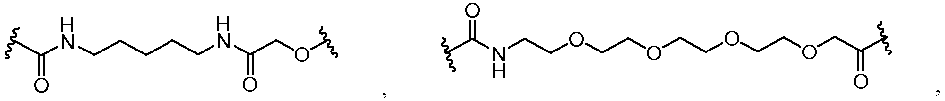

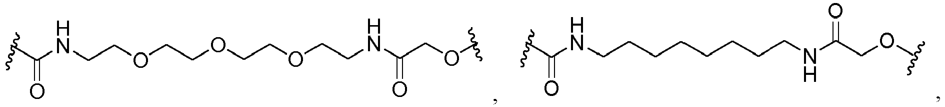

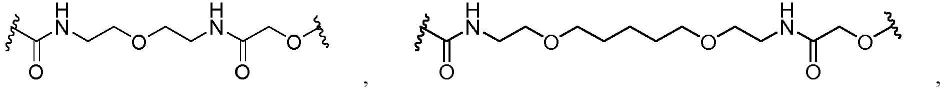

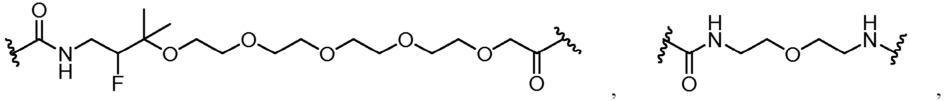

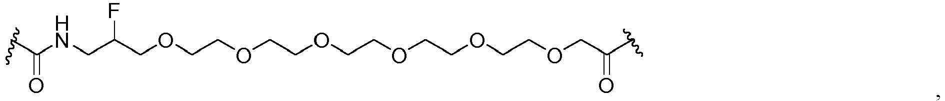

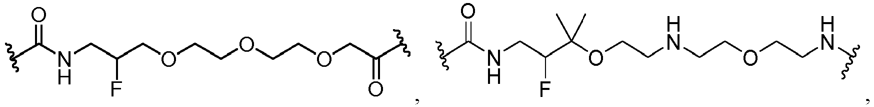

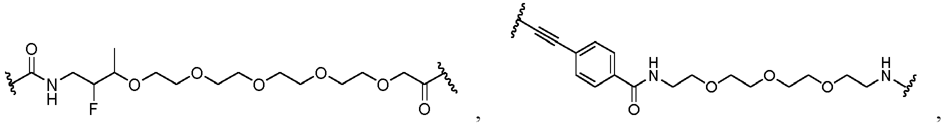

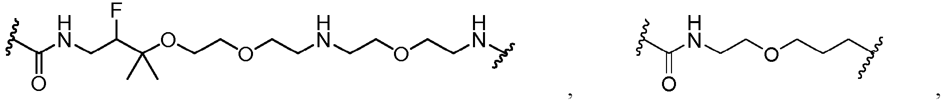

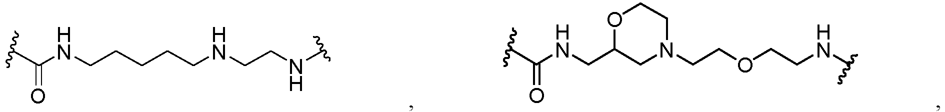

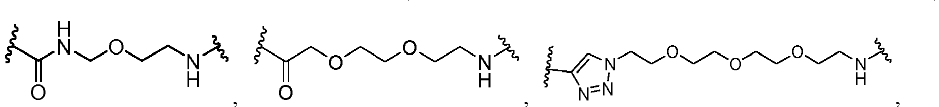

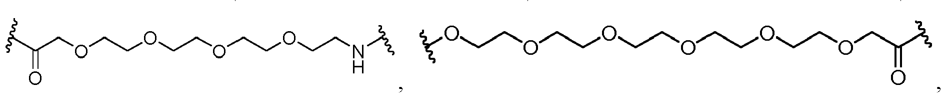

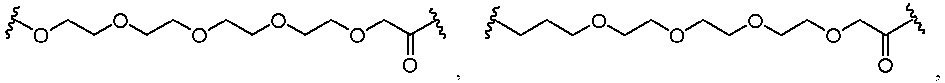

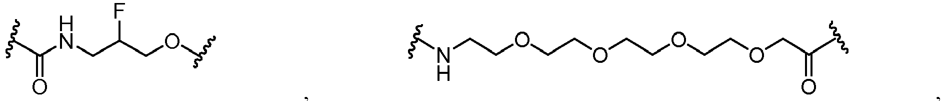

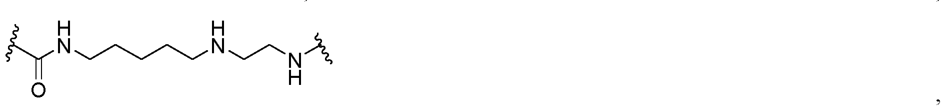

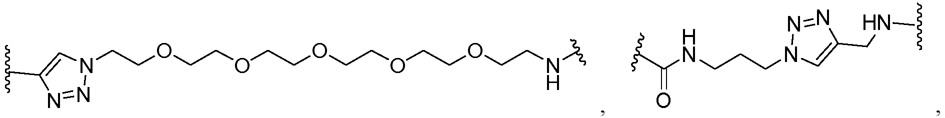

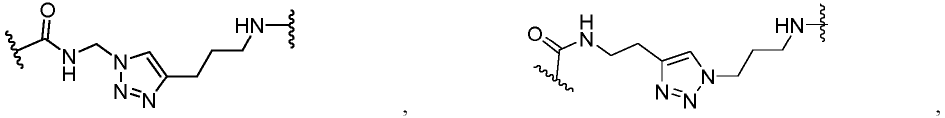

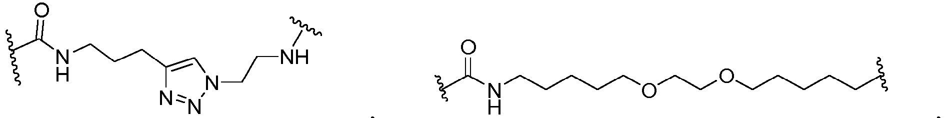

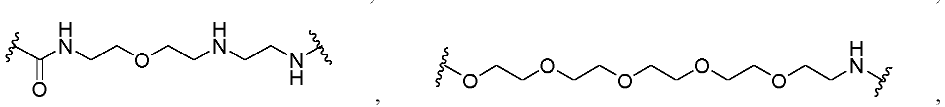

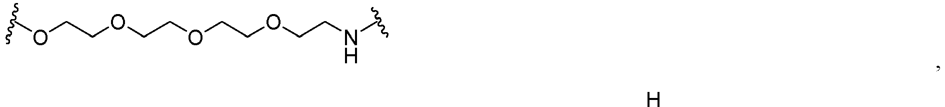

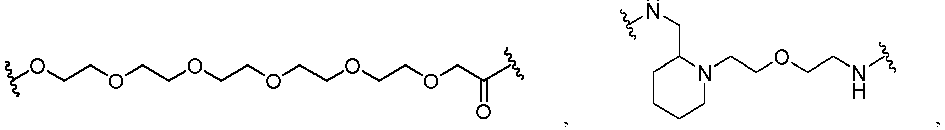

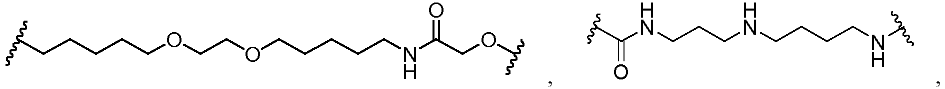

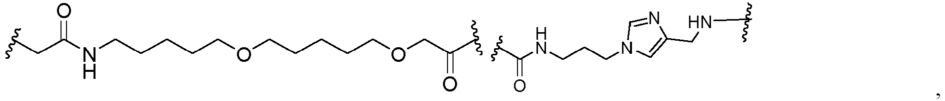

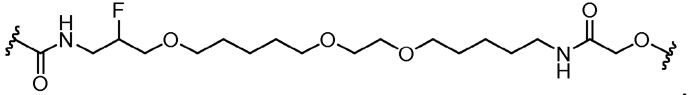

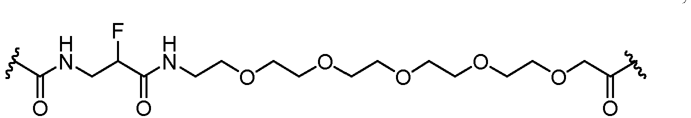

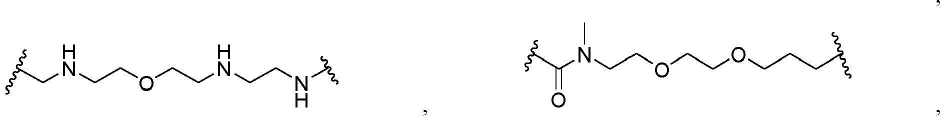

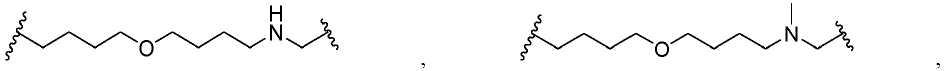

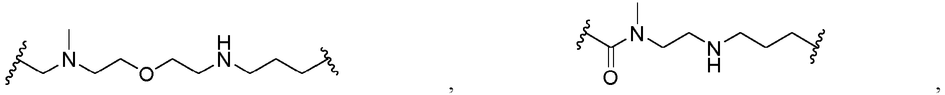

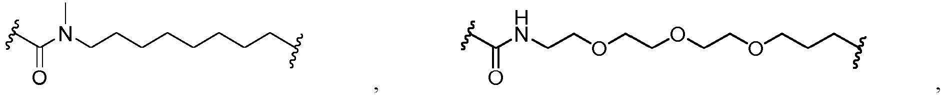

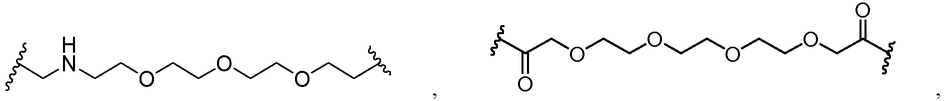

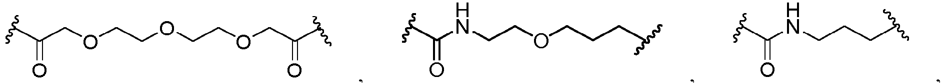

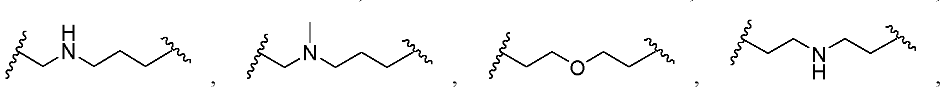

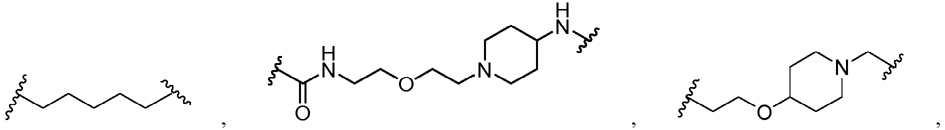

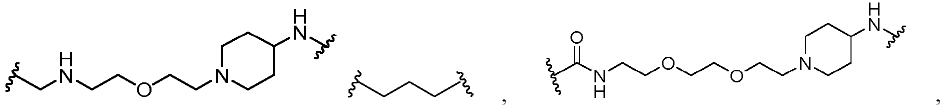

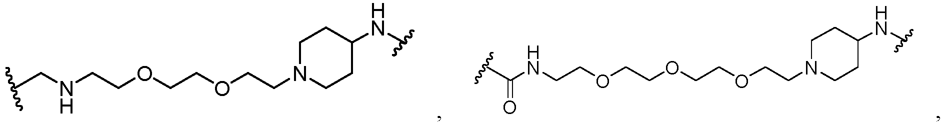

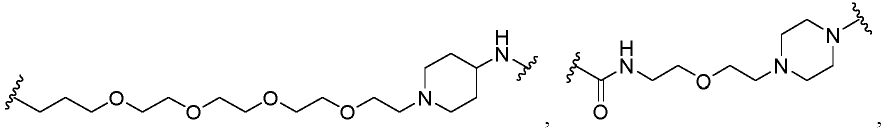

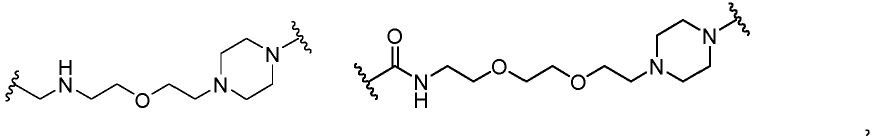

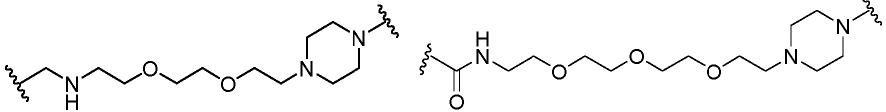

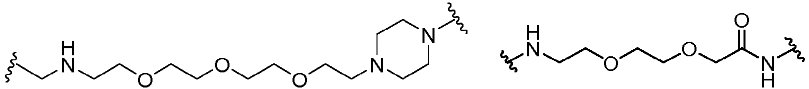

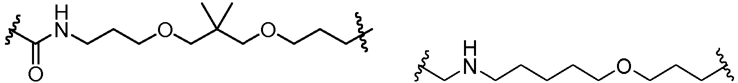

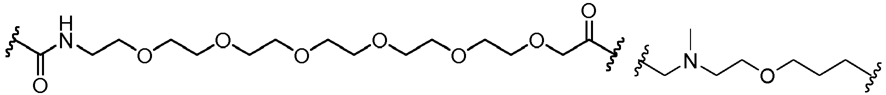

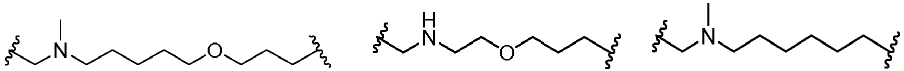

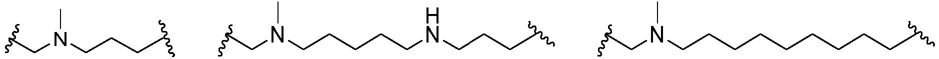

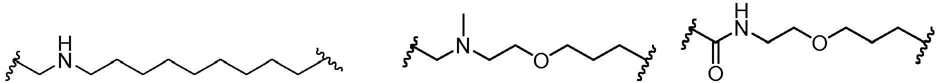

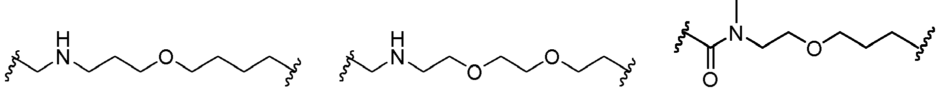

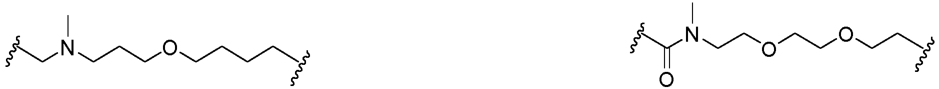

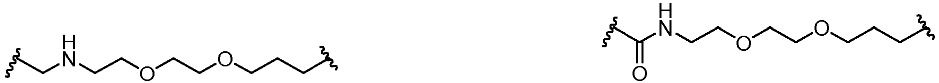

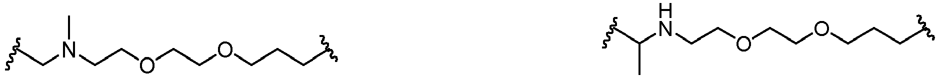

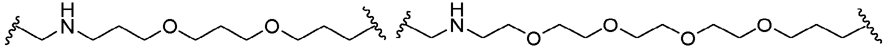

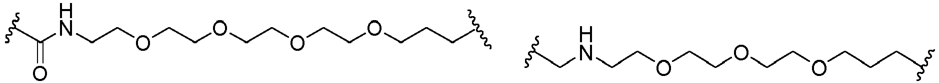

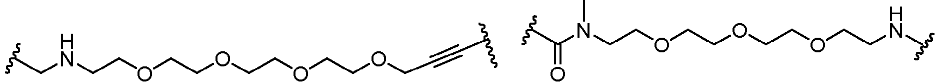

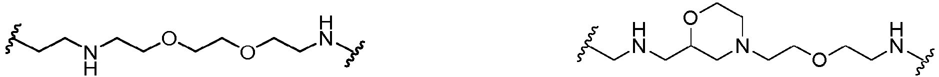

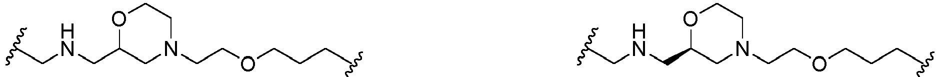

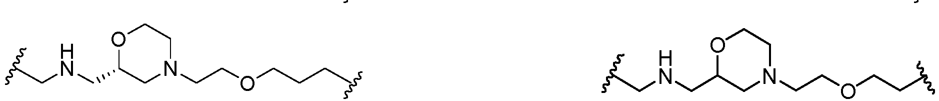

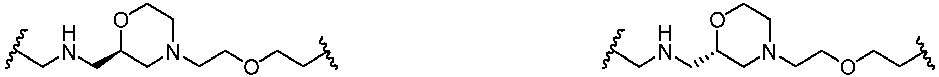

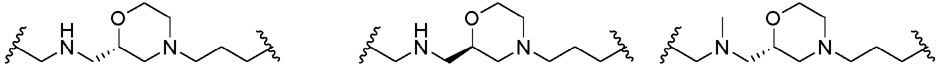

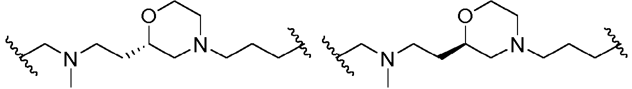

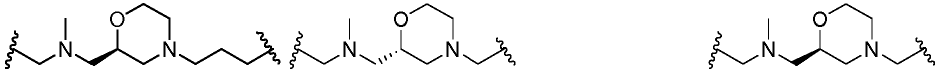

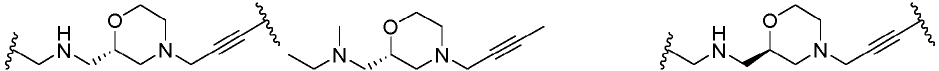

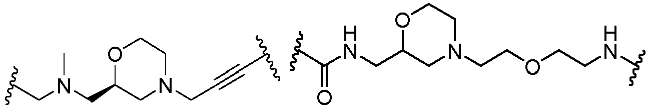

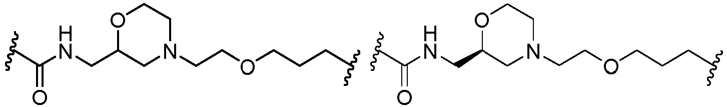

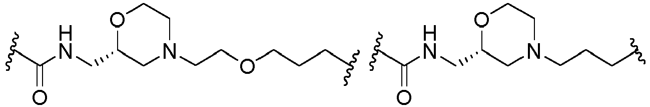

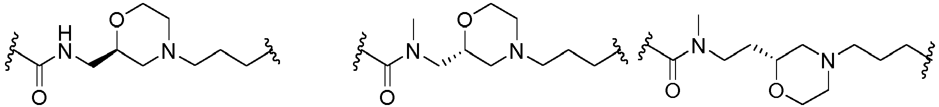

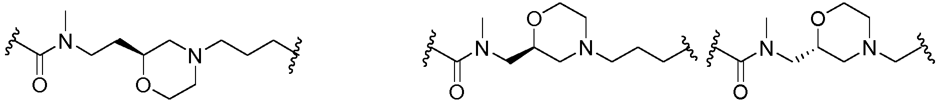

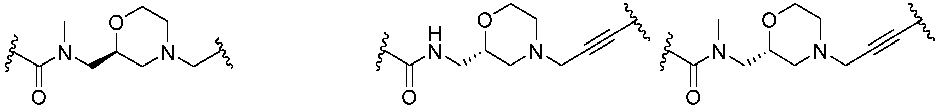

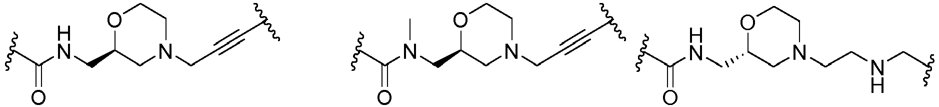

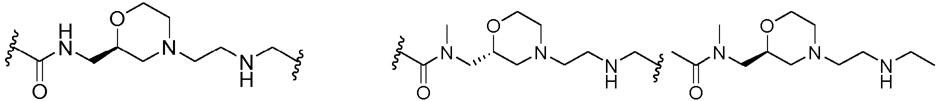

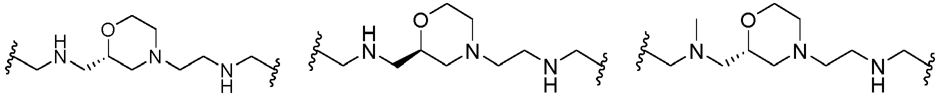

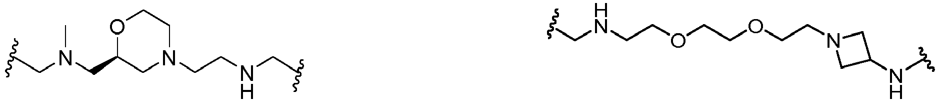

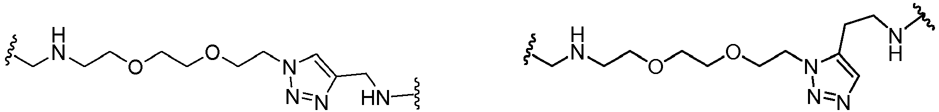

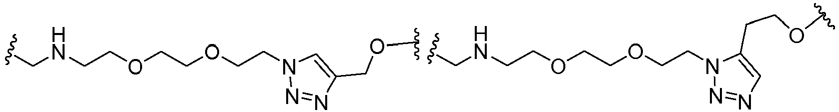

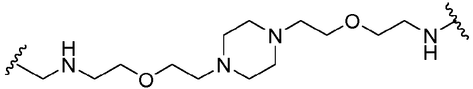

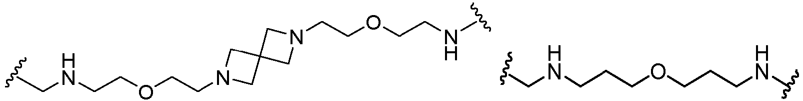

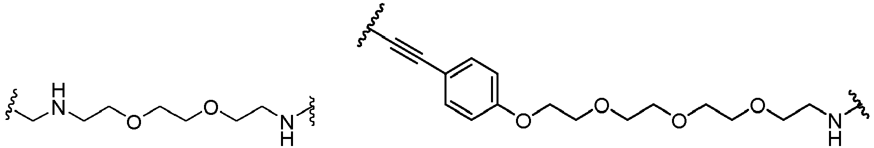

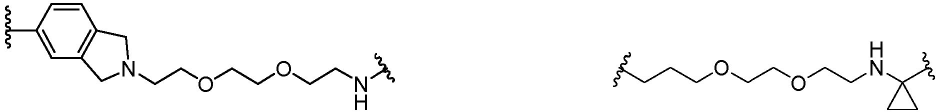

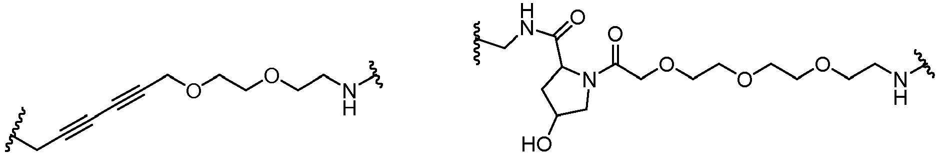

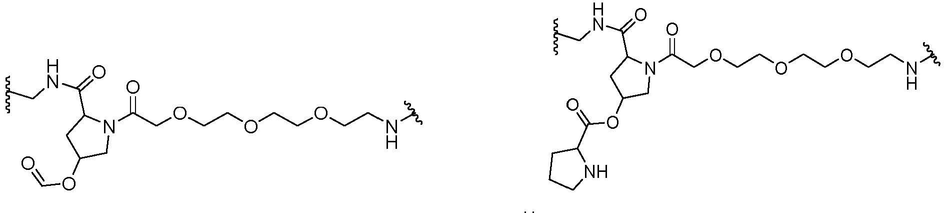

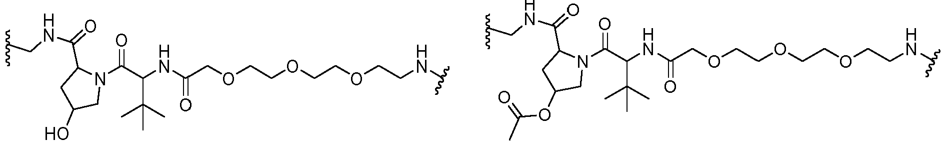

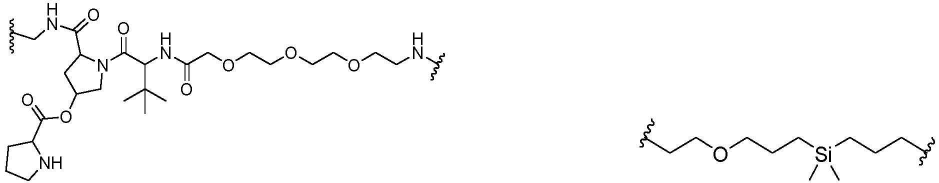

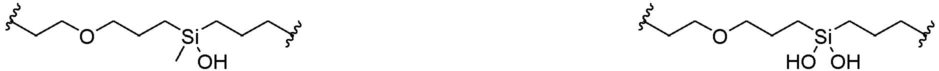

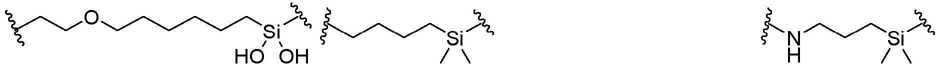

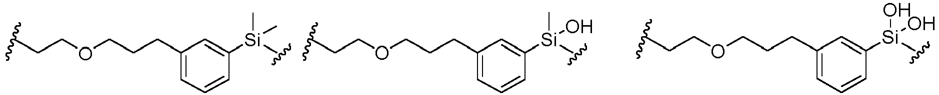

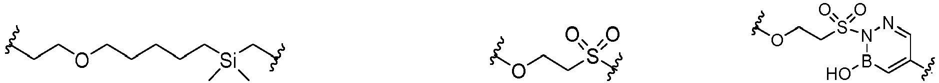

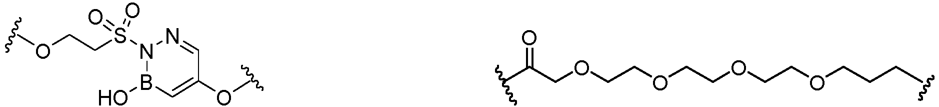

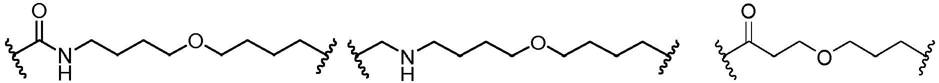

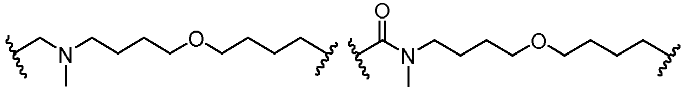

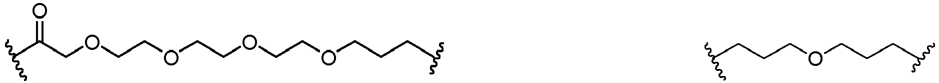

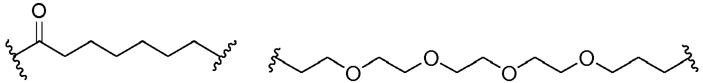

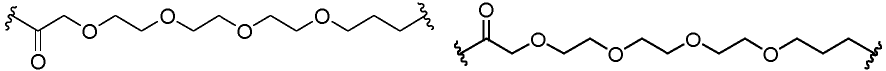

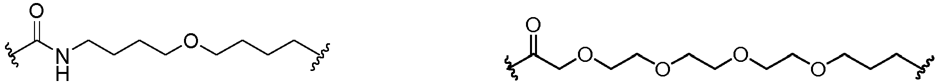

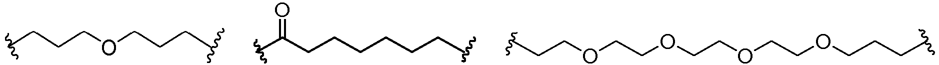

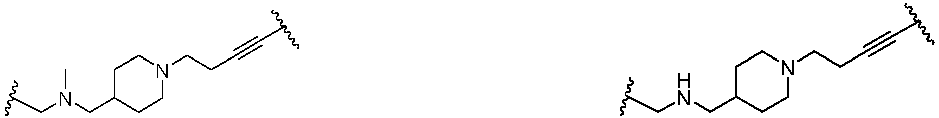

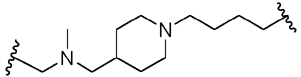

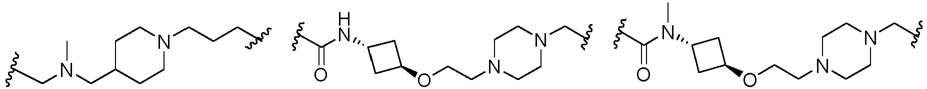

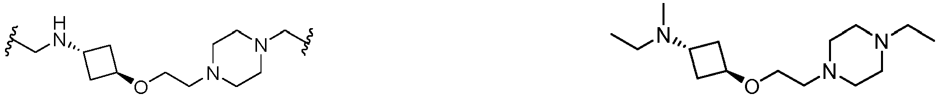

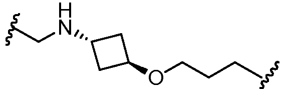

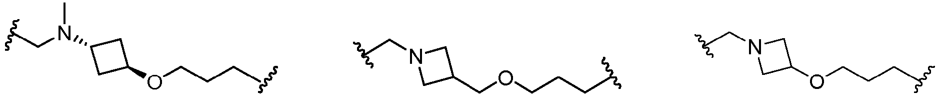

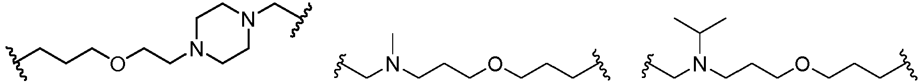

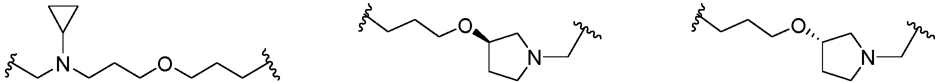

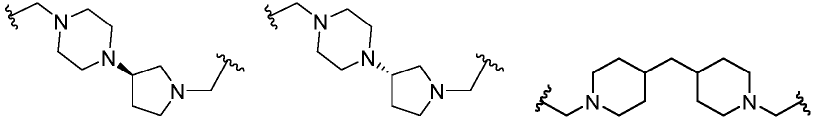

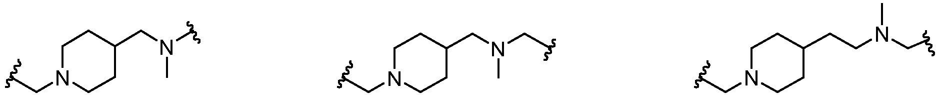

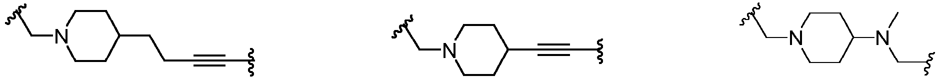

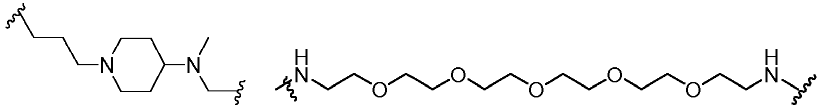

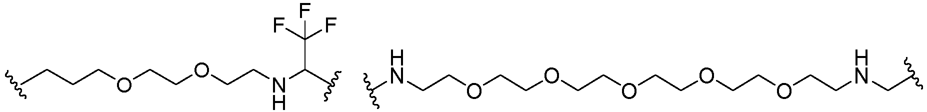

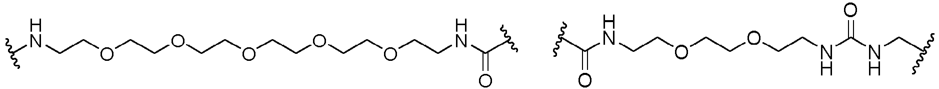

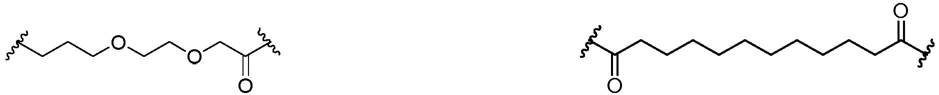

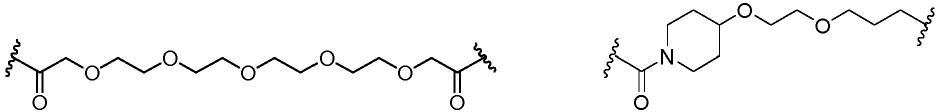

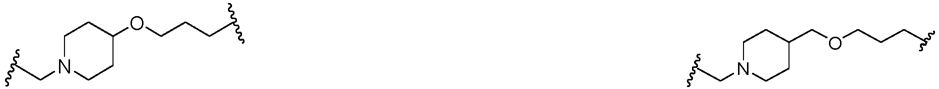

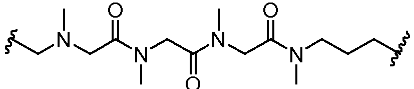

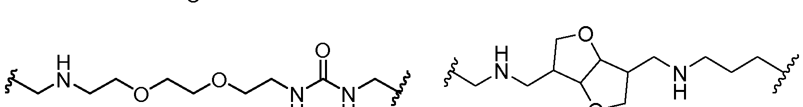

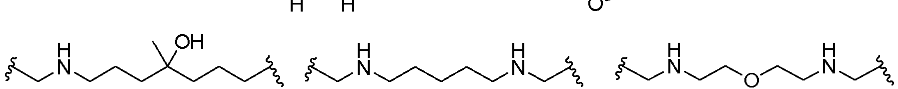

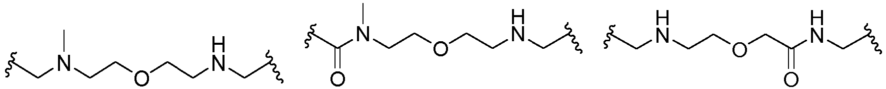

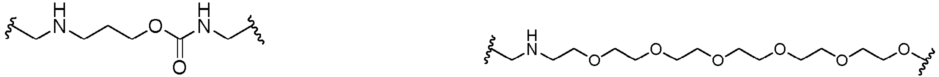

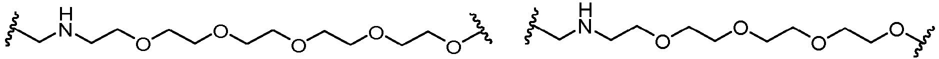

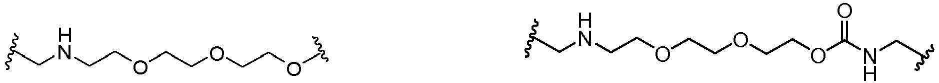

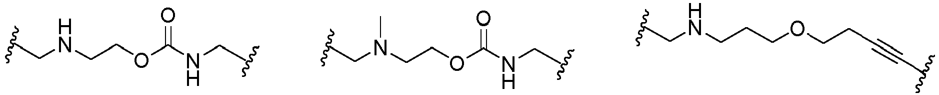

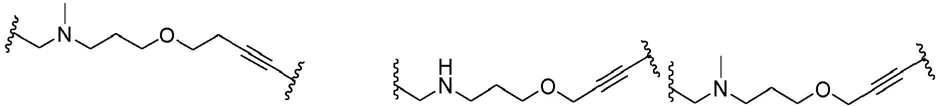

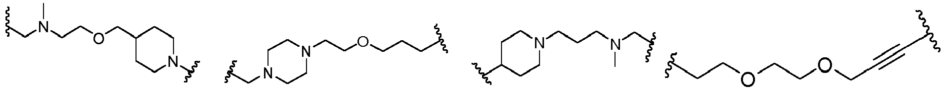

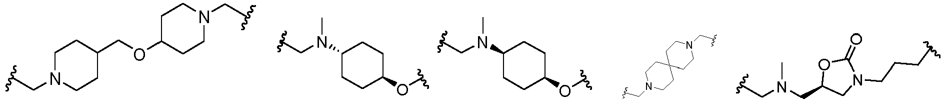

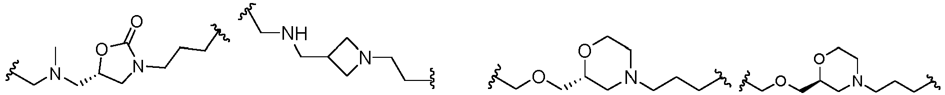

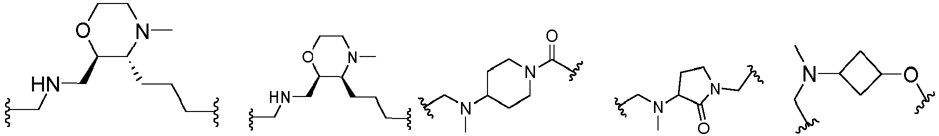

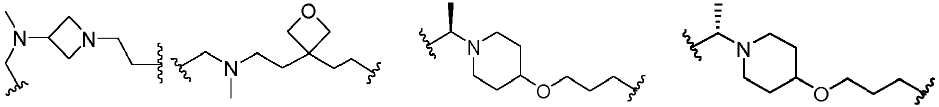

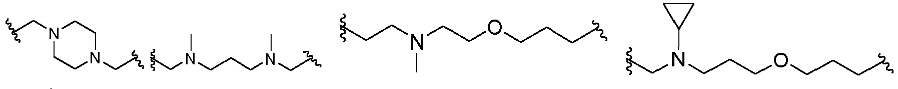

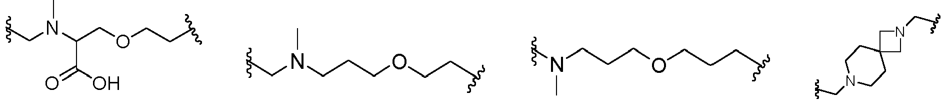

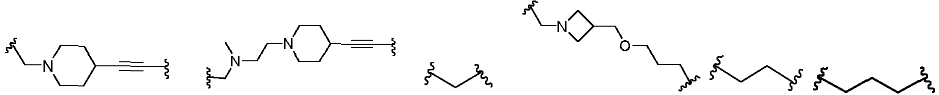

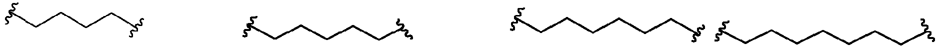

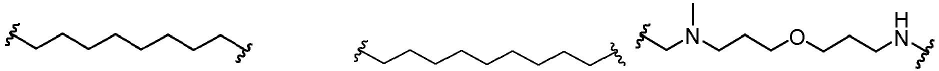

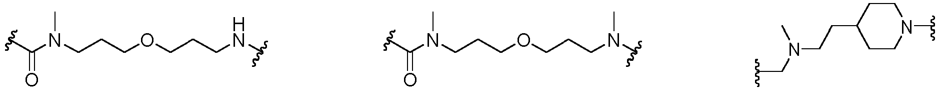

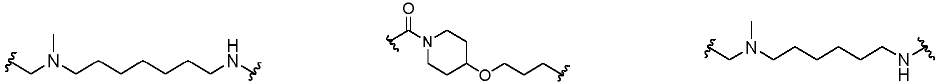

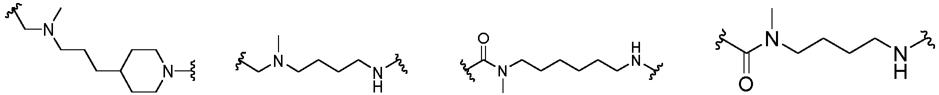

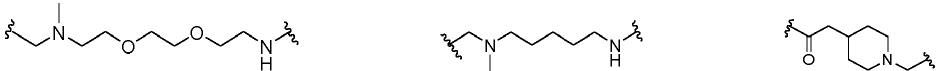

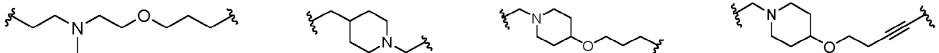

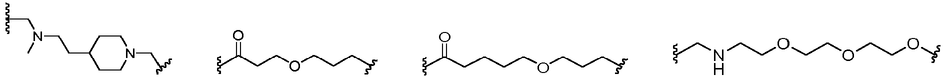

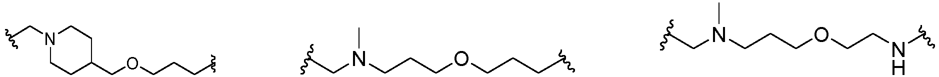

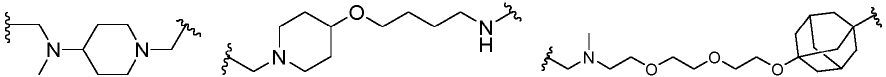

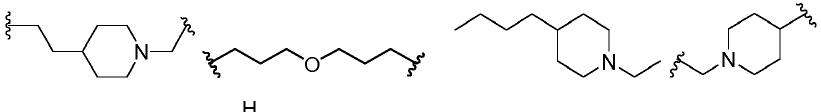

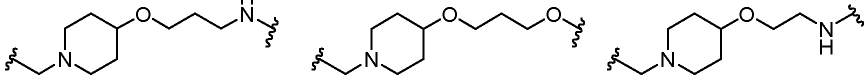

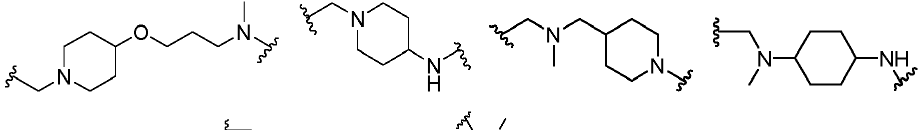

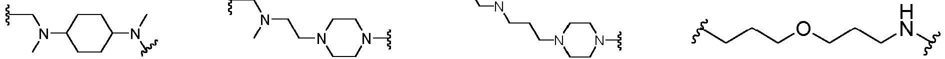

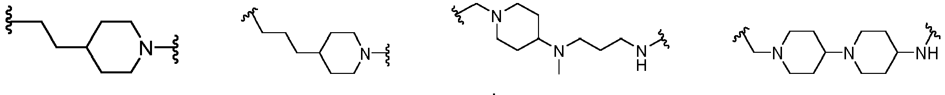

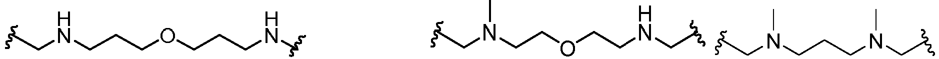

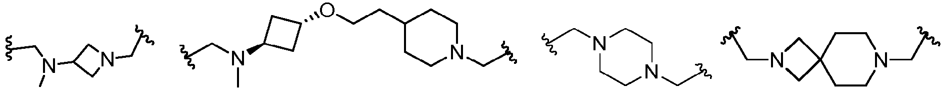

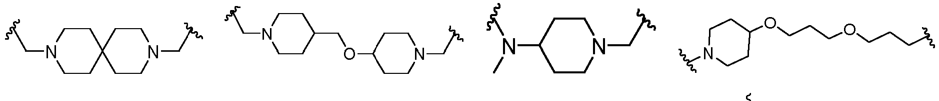

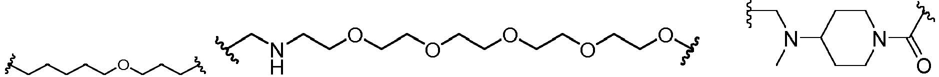

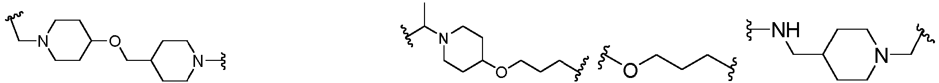

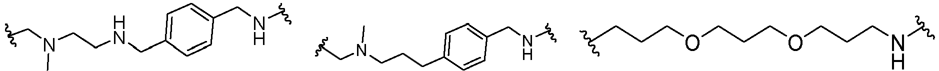

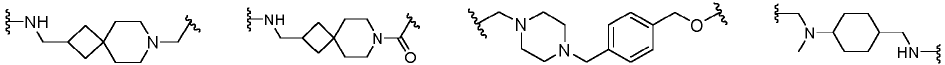

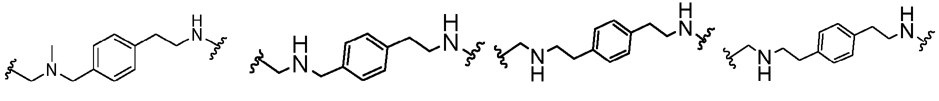

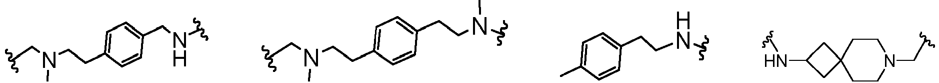

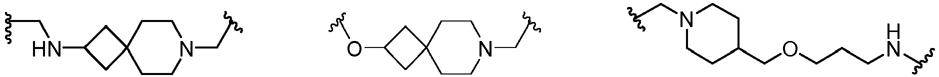

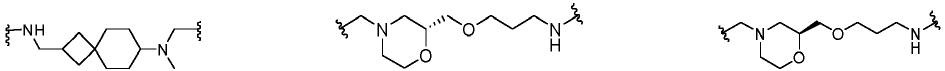

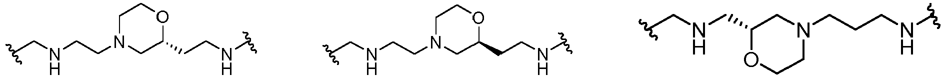

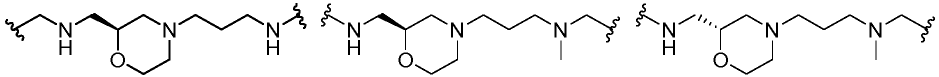

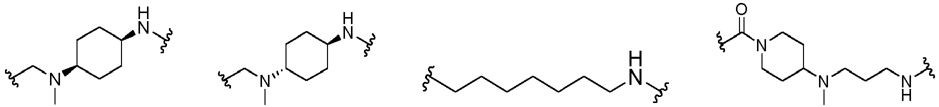

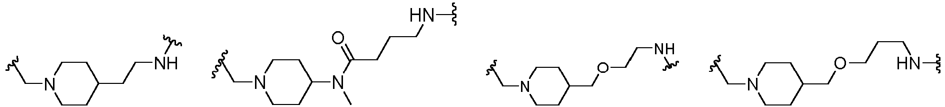

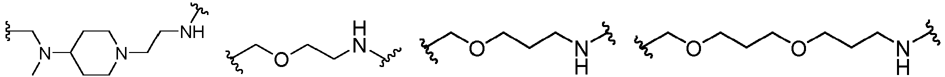

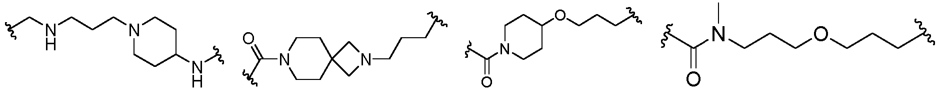

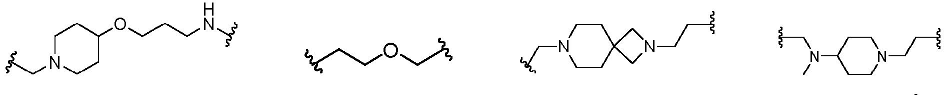

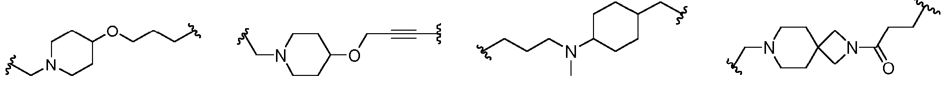

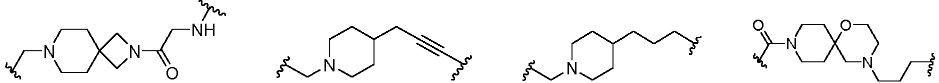

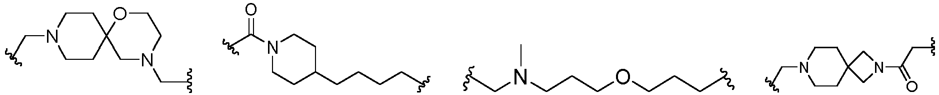

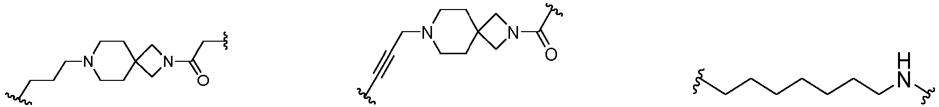

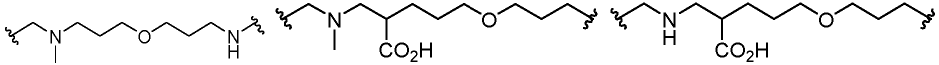

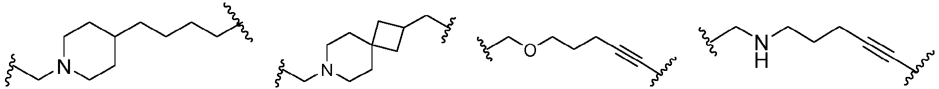

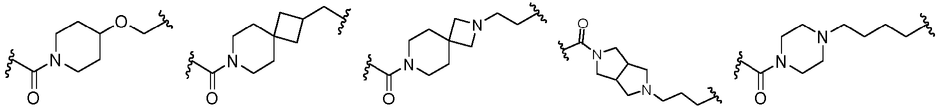

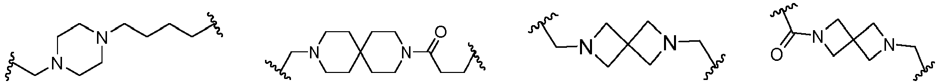

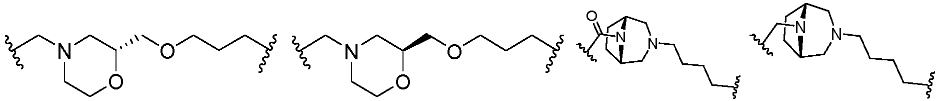

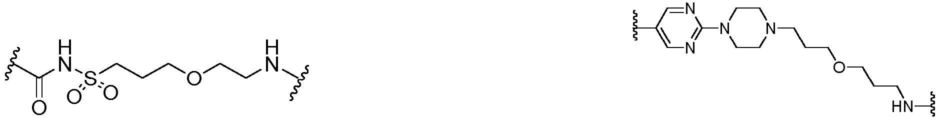

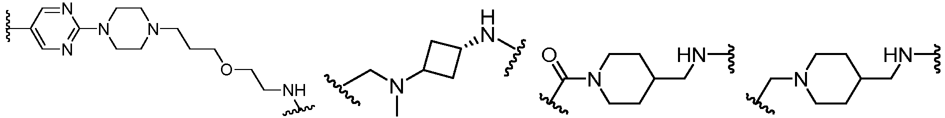

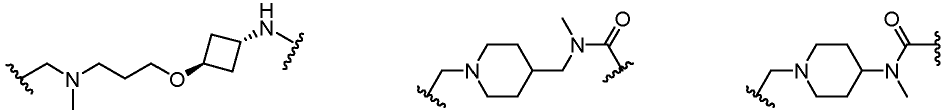

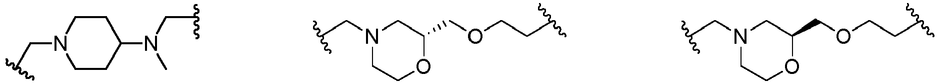

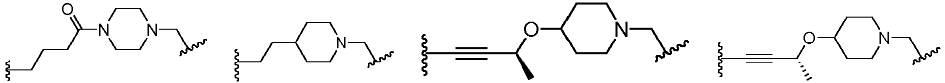

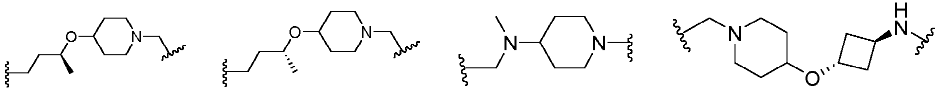

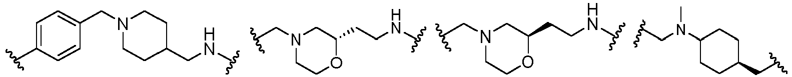

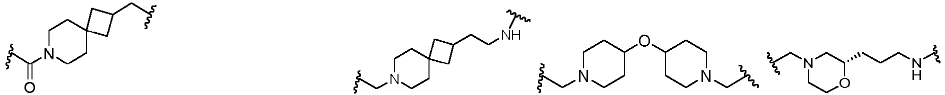

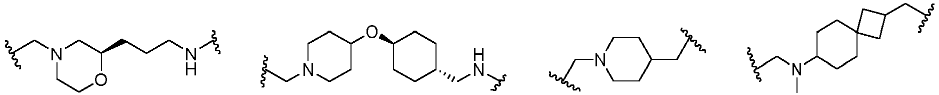

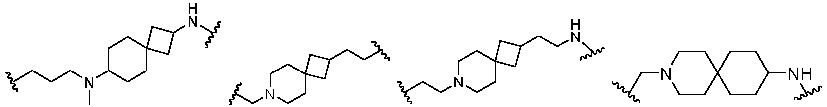

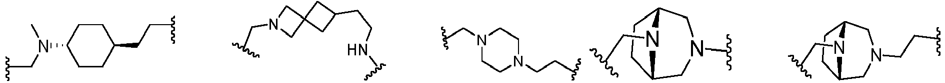

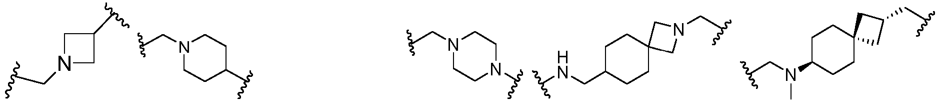

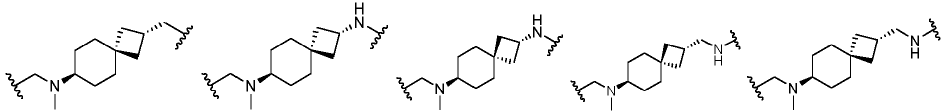

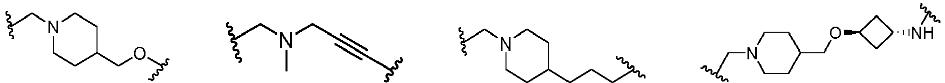

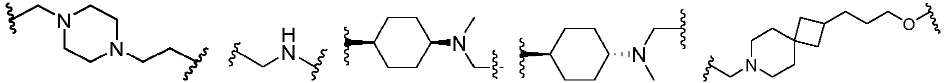

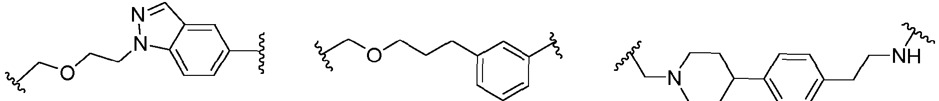

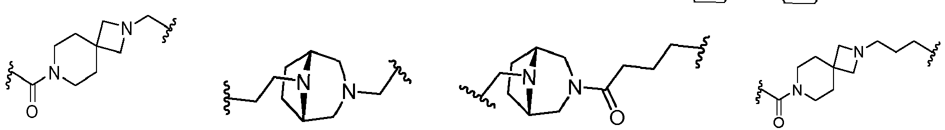

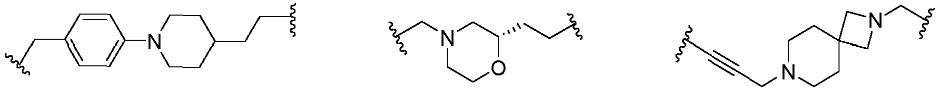

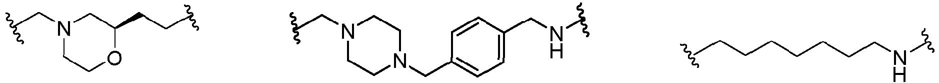

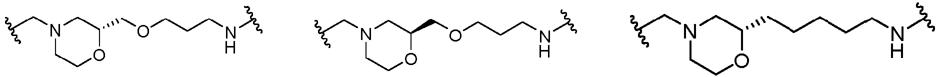

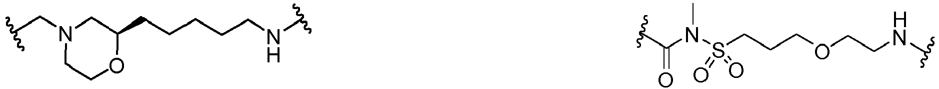

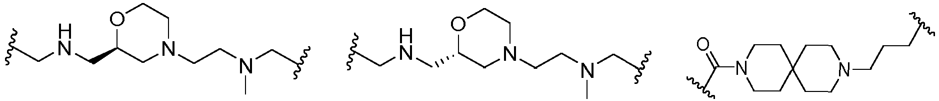

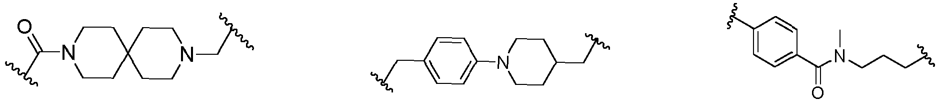

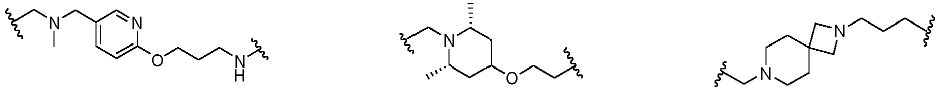

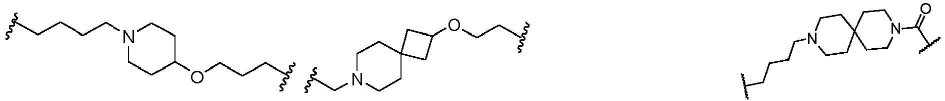

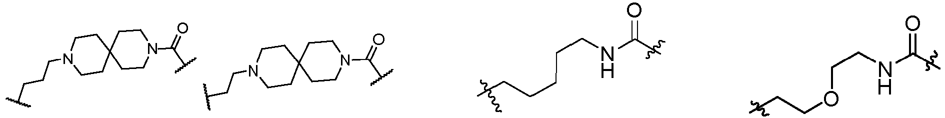

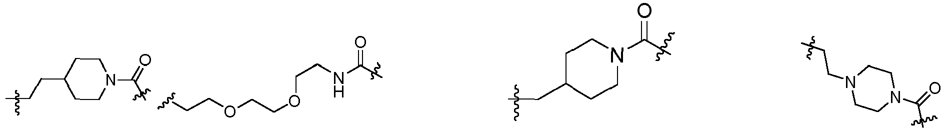

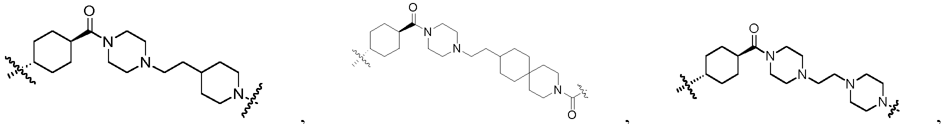

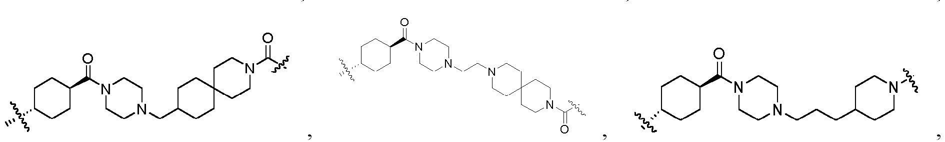

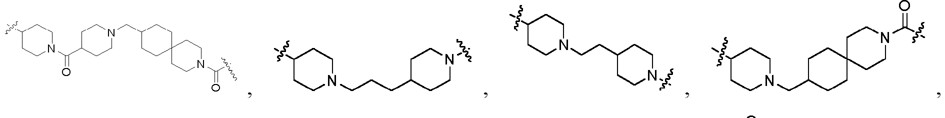

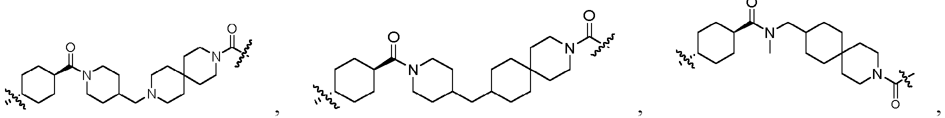

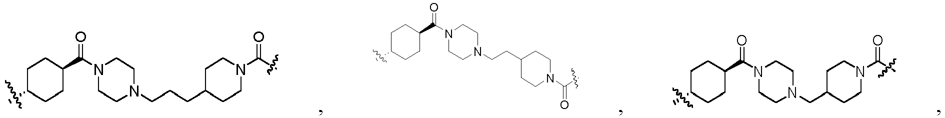

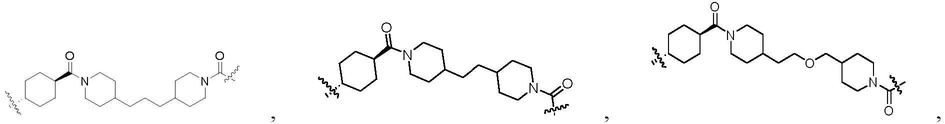

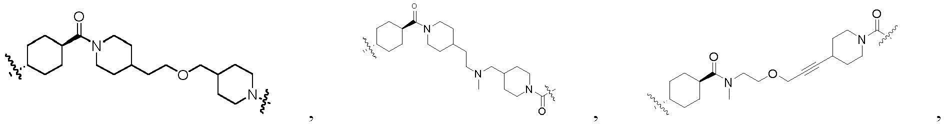

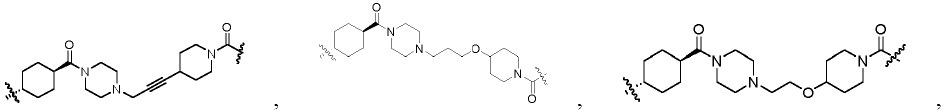

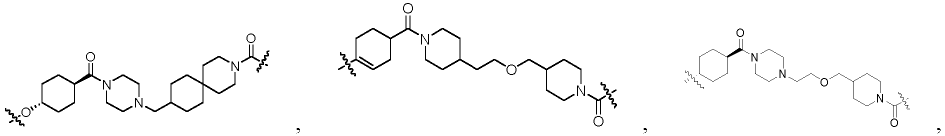

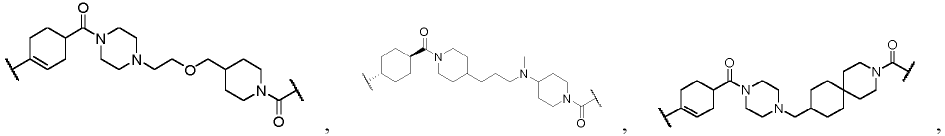

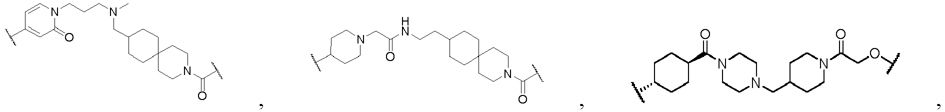

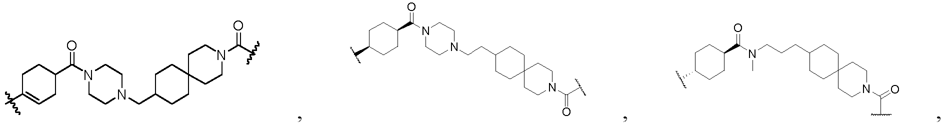

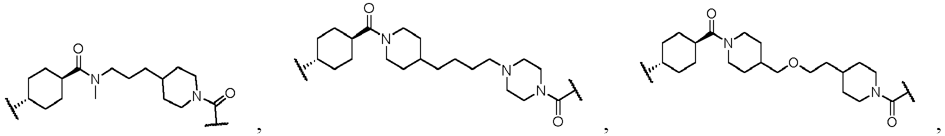

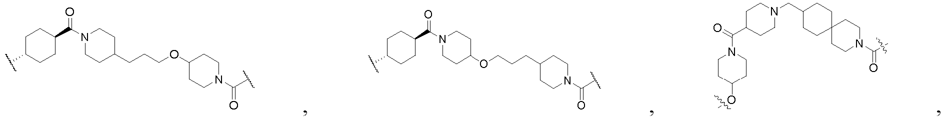

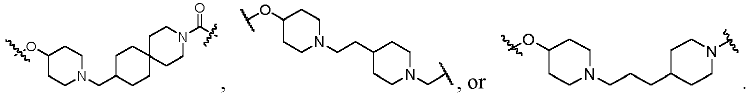

Preferably, in certain embodiments of the present disclosure, the methylene group in L is substituted with a group selected from -O-, C(O)-, ethenylene, saturated or partially unsaturated 4- to 7-membered heterocycloalkylene having 1-2 heteroatoms independently selected from nitrogen, oxygen, and sulfur, and saturated or partially unsaturated 5- to 11-membered spiroheterocycloalkylene having 1-2 heteroatoms independently selected from nitrogen, oxygen, and sulfur.

Preferably, in certain embodiments of the present disclosure, j is 8.

Preferably, in certain embodiments of the present disclosure, L is -O-CH₂-ethenylene-CH₂-(saturated or partially unsaturated 4- to 7-membered heterocycloalkylene having 1-2 heteroatoms independently selected from nitrogen, oxygen, and sulfur)-CH₂-(saturated or partially unsaturated 5- to 11-membered spiroheterocycloalkylene having 1-2 heteroatoms independently selected from nitrogen, oxygen, and sulfur)-C(O)-, such as

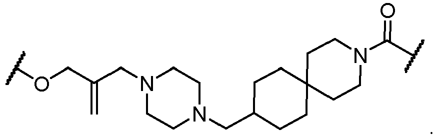

Preferably, in certain embodiments of the present disclosure, L is LA: 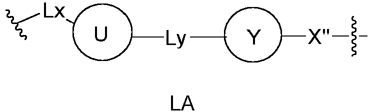 wherein in LA:
case I:
   ring U is C₃-C₁₂ cycloalkylene or 3- to 12-membered heterocycloalkylene containing 1-2 heteroatoms selected from N, O, and S, wherein the C₃-C₁₂ cycloalkylene and 3- to 12-membered heterocycloalkylene are optionally substituted with substituents selected from halogen, oxo, cyano, amino, hydroxy, CO₂Bn, C₁-C₆ alkyl, C₁-C₆ haloalkyl, C₁-C₆ hydroxyalkyl, and -O-(C₁-C₆ alkyl);
   ring Y is a bond, C₃-C₁₂ cycloalkylene, or 3- to 12-membered heterocycloalkylene containing 1-2 heteroatoms selected from N, O, and S (the heterocycloalkylene may be monocyclic heterocycloalkylene or bispiroheterocycloalkylene), wherein the C₃-C₁₂ cycloalkylene and 3- to 12-membered heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
   X" is a bond, -C(O)-, -NH-, -NCH₃-, -O-, -C(CH₃)₂-, -S-, -C=C-, -C≡C-, -CHF-, -CHCF₃-, - (CH₂)_{q}C(O)-, -S(O)-, -S(O)₂-, -C(O)O-, -OC(O)-, -(CH₂)_{q}C(O)NH-, -C(O)NCH₃-, -NHC(O)-, -NCH₃C(O)-, or -C(O)CH₂O-;
   q is 1 or 2;
   Lx is -(CH₂)ᵥ-, wherein one or two methylene groups in Lx are optionally replaced by a group selected from -O-, -S-, -NH-, -C ≡ C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CR_{d}Rₑ-; v is 1, 2, 3, 4, 5, 6, or 7;
   R_{d} and Rₑ are each independently H, -OH, C₁-C₆ alkyl, or C₁-C₆ alkoxy;
   or R_{d} and Rₑ, together with the C atom linked thereto, form -C=C(R_{f})₂, C₃-C₈ cycloalkyl, or 3- to 8-membered heterocycloalkyl;
   R_{f} is hydrogen or C₁-C₃ alkyl;
   Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8;
case II:
   ring U is 3- to 12-membered heterocycloalkylene containing 1-2 heteroatoms selected from N, O, and S (e.g., 4- to 8-membered saturated monocyclic heterocycloalkylene containing 1 or 2 nitrogen heteroatoms), wherein the 3- to 12-membered heterocycloalkylene is optionally substituted with the following substituent: C₁-C₆ alkyl substituted with 1, 2, or 3 hydroxy (e.g., -CH₂OH);
   ring Y is 3- to 12-membered heterocycloalkylene containing 1-2 heteroatoms selected from N, O, and S (e.g., bispiroheterocycloalkylene);
   X" is -C(O)-;
   Lx is -(CH₂)ᵥ-, wherein 1 or two methylene groups in Lx are optionally replaced by a group selected from -O- and -CR_{d}Rₑ-; v is 1, 2, 3, 4, 5, 6, or 7; R_{d} and Rₑ are each independently H, NH₂, or C₁-C₆ alkyl substituted with 1, 2, or 3 halogen (e.g., fluorine) (e.g., -CH₂CHF₂ or -CH₂CH₂CF₃), or R_{d} and Rₑ, together with the C atom linked thereto, form C₃-C₈ cycloalkyl;
   Ly is -(CH₂)ₖ-, wherein 1 or more (e.g., 1 or 2) hydrogens on the methylene group in Ly are optionally substituted with deuterium; each k is independently 1, 2, 3, 4, 5, 6, 7, or 8.

Preferably, in certain embodiments of the present disclosure, L is LA: 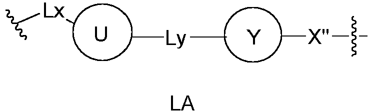 wherein in LA:
ring U is C₃-C₁₂ cycloalkylene or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring Y is a bond, C₃-C₁₂ cycloalkylene, or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
X" is a bond, -C(O)-, -NH-, -NCH₃-, -O-, -C(CH₃)₂-, -S-, -C=C-, -C≡C-, -CHF-, -CHCF₃-, - (CH₂)_{q}C(O)-, -S(O)-, -S(O)₂-, -C(O)O-, -OC(O)-, -(CH₂)_{q}C(O)NH-, -C(O)NCH₃-, -NHC(O)-, -NCH₃C(O)-, or -C(O)CH₂O-;
q is 1 or 2;
Lx is -(CH₂)ᵥ-, wherein one or two methylene groups in Lx are optionally replaced by a group selected from -O-, -S-, -NH-, -C ≡ C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CR_{d}Rₑ-; v is 1, 2, 3, 4, 5, 6, or 7;
R_{d} and Rₑ are each independently H, -OH, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8.

Preferably, in certain embodiments of the present disclosure, in ring U, the C₃-C₁₂ cycloalkylene is C₃-C₆ monocyclic saturated cycloalkylene or C₆-C₁₀ (e.g., 7-, 8-, or 9-membered bicyclic bridged ring) bridged cycloalkylene, such as cyclohexylene or bicyclo[2.2.2]octanyl.

Preferably, in certain embodiments of the present disclosure, in ring U, the 3- to 12-membered heterocycloalkylene is 3- to 8-membered (e.g., 3-, 4-, 5-, or 6-membered heterocycloalkylene) saturated monocyclic heterocycloalkylene, 6- to 10-membered (e.g., 7-, 8-, or 9-membered fused ring) fused heterocycloalkylene, or 6- to 10-membered (e.g., 7-, 8-, or 9-membered fused ring) bridged heterocycloalkylene, containing 1 or 2 heteroatoms of N, e.g., azetidinylene, pyrrolidinylene, piperidinylidene, piperazinylidene, hexahydro-1*H*-pyrrolizinylene, 2,5-diazabicyclo[2.2.1]heptanylene, 3,8-diazabicyclo[3.2.1]octanylene, or 3,6-diazabicyclo[3.1.1]heptanylene. Alternatively, the 3- to 12-membered heterocycloalkylene may also be replaced by 8-, 9-, or 10-membered bridged heterocycloalkylene containing 1, 2, or 3 heteroatoms of N and/or O, e.g.,

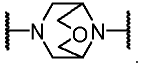

Preferably, in certain embodiments of the present disclosure, in the substituents of ring U, the halogen is F, Cl, Br, or I.

Preferably, in certain embodiments of the present disclosure, in the substituents of ring U, the C₁-C₆ alkyl and C₁-C₆ alkyl in the -O-(C₁-C₆ alkyl) are independently methyl, ethyl, n-propyl, isopropyl, n-butyl, isobutyl, sec-butyl, or *tert-butyl.*

Preferably, in certain embodiments of the present disclosure, 1, 2, 3, or 4 hydrogen atoms on ring U are optionally substituted with substituents selected from halogen, amino, hydroxy, -O-(C₁-C₆ alkyl), and C₁-C₆ alkyl. Alternatively, 1, 2, 3, or 4 hydrogen atoms on ring U may also be substituted with C₁-C₆ alkyl that is substituted with 1, 2, or 3 hydroxy.

More preferably, in certain embodiments of the present disclosure, 1, 2, 3, or 4 hydrogen atoms in ring U are optionally substituted with a group selected from F, -OH, -OCH₃, -NH₂, and methyl. Alternatively, 1, 2, 3, or 4 hydrogen atoms on ring U may also be substituted with -CH₂OH.

Preferably, in certain embodiments of the present disclosure, ring U is 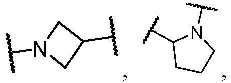 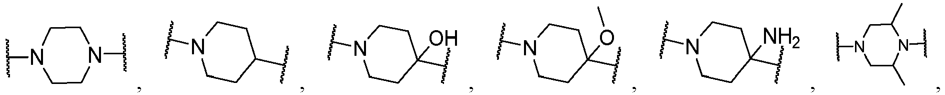 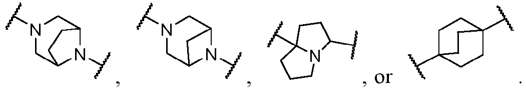 Alternatively, ring U is preferably replaced by

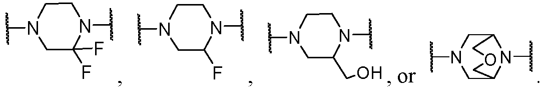

Preferably, in certain embodiments of the present disclosure, in ring Y, the C₃-C₁₂ cycloalkylene is C₃-C₆ monocyclic cycloalkylene, such as cyclohexylene.

Preferably, in certain embodiments of the present disclosure, in ring Y, the 3- to 12-membered heterocycloalkylene is 6- to 8-membered (e.g., 6-, 7-, or 8-membered) monocyclic heterocycloalkylene, 7-to 11-membered (e.g., 7-, 8-, 9-, 10-, or 11-membered) spiroheterocycloalkylene, or 7- to 11-membered (e.g., 7-, 8-, 9-, 10-, or 11-membered) fused heterocycloalkylene, containing 1 or 2 heteroatoms of N, e.g., piperidinylidene, piperazinylidene, 3-azaspiro[5.5]undecylene, 7-azaspiro[3.5]nonanylene, 2,7-diazaspiro[3.5]nonanylene, 3,9-diazaspiro[5.5]undecylene, 2,6-diazaspiro[3.3]heptanylene, or 2-azaspiro[3.3]heptanylene. Alternatively, the 3- to 12-membered heterocycloalkylene may also be replaced by 7-, 8-, 9-, 10-, or 11-membered spiroheterocycloalkylene containing 1 or 2 heteroatoms of N and/or O, e.g., 2-oxa-9-azaspiro[5.5]undecane.

Preferably, in certain embodiments of the present disclosure, in the substituents of ring Y, C₁-C₆ alkyl in the -O-(C₁-C₆ alkyl) is independently methyl, ethyl, *n*-propyl, isopropyl, *n*-butyl, isobutyl, *sec*-butyl, or *tert*-butyl.

Preferably, in certain embodiments of the present disclosure, ring Y is 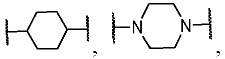 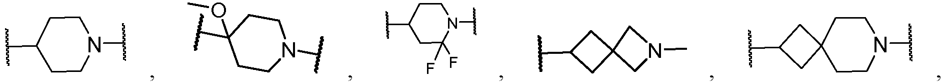 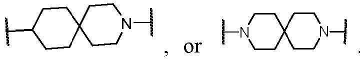 Alternatively, ring Y may also be preferably replaced by

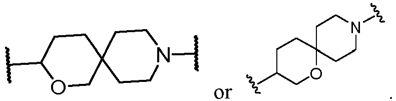

Preferably, in certain embodiments of the present disclosure, ring U in LA is 4- to 8-membered saturated monocyclic heterocycloalkylene, 6- to 10-membered fused heterocycloalkylene, 6- to 10-membered bridged heterocycloalkylene, or C₆-C₁₀ bridged cycloalkylene, containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; Lx is -(CH₂)ᵥ-, and v is 1, 2, 3, 4, or 5; Ly is -(CH₂)ₖ-, and k is 1, 2, 3, 4, or 5. In LA, ring Y may also be replaced by 7-, 8-, 9-, 10-, or 11-membered spiroheterocycloalkylene containing 1 or 2 heteroatoms of N and/or O.

Preferably, in certain embodiments of the present disclosure, ring U in LA is 4- to 8-membered saturated monocyclic heterocycloalkylene or 6- to 10-membered fused heterocycloalkylene, containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; Lx is -(CH₂)ᵥ-, and v is 1, 2, 3, 4, or 5; Ly is -(CH₂)ₖ-, and k is 1, 2, 3, 4, or 5. In LA, ring Y may also be replaced by 7-, 8-, 9-, 10-, or 11-membered spiroheterocycloalkylene containing 1 or 2 heteroatoms of N and/or O.

Preferably, in certain embodiments of the present disclosure, LA is LA-1 or LA-2: 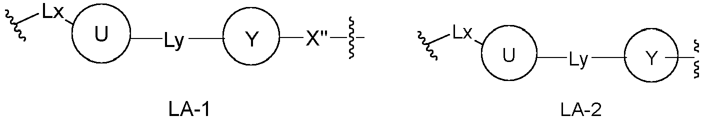
X" is -C(O)- or -C(O)NH-;
in LA-1 and LA-2, ring U, ring Y, Lx, Ly, and q are as defined and described in the present disclosure.

Preferably, in certain embodiments of the present disclosure, LA is LA-3, LA-4, LA-5, or LA-6: 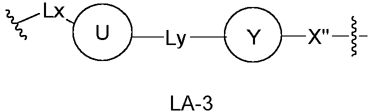 wherein in LA-3:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 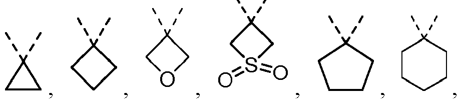 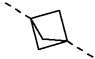 , -C(CH₃)₂-, -CH(CH₃)-, -CH(OH)-, -CH(OCH₃)-, C(CH₃)(OH)-, or -C=CH₂; v is 1, 2, 3, 4, 5, or 6;
ring U is 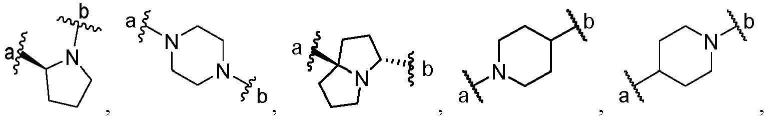 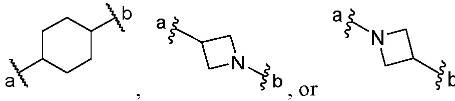 , wherein end a is linked to Lx, and end b is linked to Ly; 1, 2, 3, or 4 hydrogen atoms in ring U are optionally substituted with F, -OH, -OCH₃, or -NH₂;
ring Y is a bond, 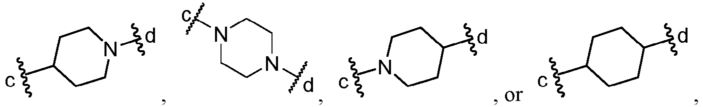 wherein end c is linked to Ly, and end d is linked to X"; 1, 2, 3, or 4 hydrogen atoms in ring Y are optionally substituted with F, -OH, or -OCH₃;
Ly is -(CH₂)ₖ-, wherein one or two CH₂ contained in Ly are each independently optionally replaced by -O-, -C≡C-, -C(O)-, or -N(C₁-C₆ alkyl)-; k is 1, 2, 3, 4, 5, or 6;
X" is a bond, -C(O)-, -(CH₂)₁₋₂C(O)-, or -(CH₂)₁₋₂C(O)NH-; 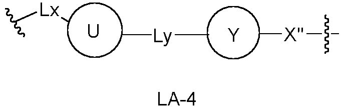
wherein in LA-4:
   Lx is -(CH₂)ᵥ-, wherein one or two CH₂ contained in Lx are each independently optionally replaced by -O-, -S-, -NH-, or -CR_{d}Rₑ-; -CR_{d}Rₑ- is 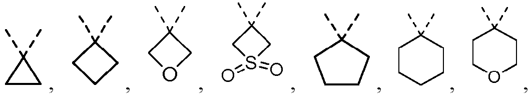 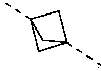 -C(CH₃)₂-, -CH(CH₃)-, -CH(CH₃)-, -CH(OCH₃)-, -CH(OH)-, -C(CH₃)(OH)-, or -C=CH₂; v is 1, 2, 3, 4, 5, or 6;
   ring U is 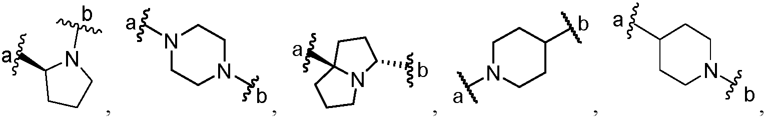 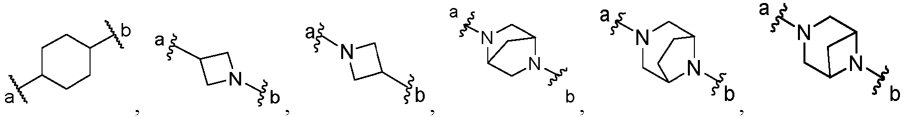 , or 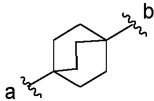 , wherein end a is linked to Lx, and end b is linked to Ly; 1, 2, 3, or 4 hydrogen atoms in ring U are optionally substituted with F (e.g., 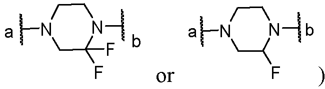 ; ring Y is 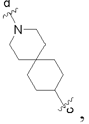 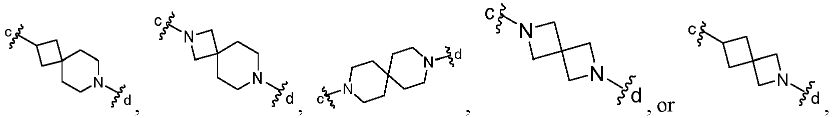 wherein end c is linked to Ly, and end d is linked to X"; 1, 2, 3, or 4 hydrogen atoms in ring Y are optionally substituted with F;
   Ly is -(CH₂)ₖ-, wherein one or two CH₂ contained in Ly are each independently optionally replaced by -O- or -N(C₁-C₆ alkyl)-; k is 1, 2, 3, 4, 5, or 6;
   X" is -C(O)-;
   or ring Y may also be replaced by 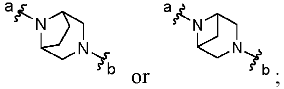
   case b:
      Lx is -(CH₂)ᵥ-, wherein one or two CH₂ contained in Lx are each independently optionally replaced by -O-, -S-, -NH-, or -CR_{d}Rₑ-; -CR_{d}Rₑ- is 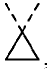 -CH(NH₂)-, -CH(CH₂CHF₂)-, -CH(CH₂CH₂CF₃)-, or - CH(CH₃)₂-; v is 1, 2, 3, 4, 5, or 6;
      ring U is 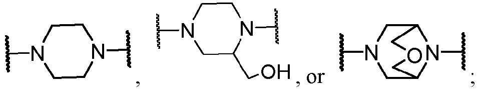
      ring Y is 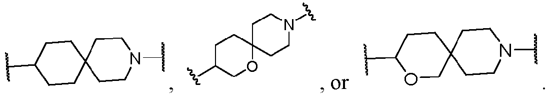
      Ly is -(CH₂)ₖ-, wherein 1 or more (e.g., 1 or 2) hydrogens on the methylene group in Ly are optionally substituted with deuterium; k is 1, 2, 3, 4, 5, or 6;
      X" is -C(O)-; 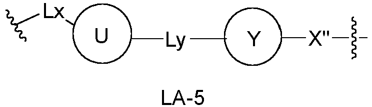
      wherein in LA-5:
         Lx is -(CH₂)ᵥ-, wherein one or two CH₂ contained in Lx are each independently optionally replaced by -O-; v is 1, 2, 3, or 4;
         ring U is 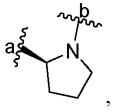 wherein end a is linked to Lx, and end b is linked to Ly; 1, 2, 3, or 4 hydrogen atoms in ring U are optionally substituted with F;
         ring Y is a bond;
         Ly is -(CH₂)ₖ-, wherein one or two CH₂ contained in Ly are each independently optionally replaced by -O-, -C(O)-, or -NH-; k is 1, 2, 3, 4, 5, 6, 7, or 8;
         X" is -C(O)NH-; 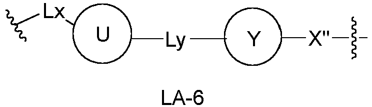
         wherein in LA-6:
            Lx is -(CH₂)ᵥ-, wherein one or two CH₂ contained in Lx are each independently optionally replaced by -O- or -CR_{d}Rₑ-; -CR_{d}Rₑ- is 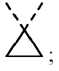 v is 1, 2, 3, or 4;
            ring U is 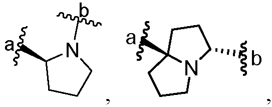 or 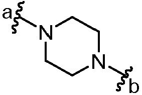 , wherein end a is linked to Lx, and end b is linked to Ly;
            ring Y is 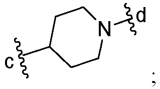
            Ly is -(CH₂)ₖ-, wherein one or two CH₂ contained in Ly are each independently optionally replaced by -O-, -C(O)-, or -NH-; k is 1, 2, 3, 4, 5, or 6;
            X" is a bond.

Preferably, LA is of the following structure: 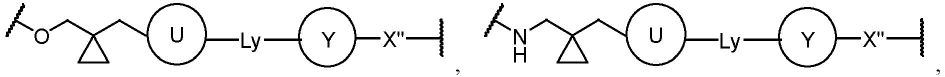 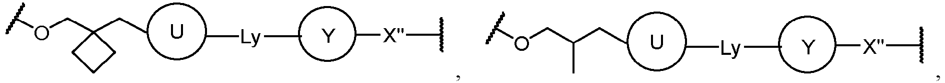 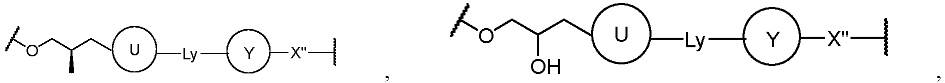 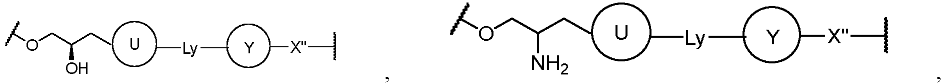 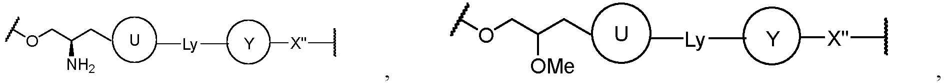 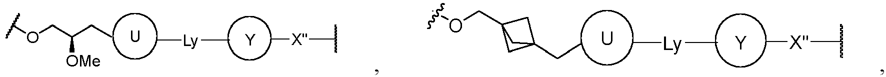 or

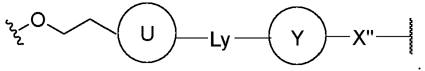

Preferably, each ring U is independently 4- to 8-membered saturated monocyclic heterocycloalkylene or 6- to 10-membered bridged heterocycloalkylene containing 1 or 2 nitrogen heteroatoms;
each ring Y is independently 3- to 12-membered heterocycloalkylene containing 1-2 heteroatoms selected from N, O, and S, wherein the heterocycloalkyl is monocyclic heterocycloalkylene or bispiroheterocycloalkylene;
X" is -C(O)-.

Most preferably, LA is of any one of the following structures:

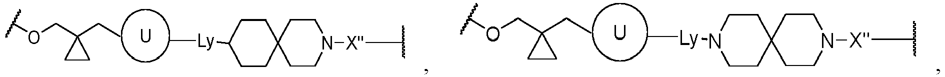

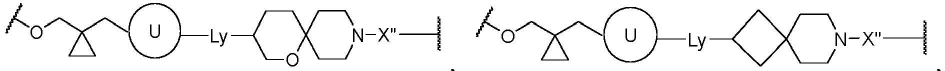

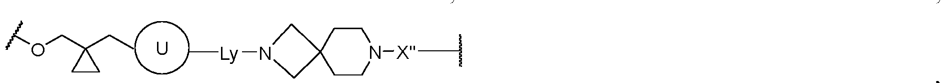

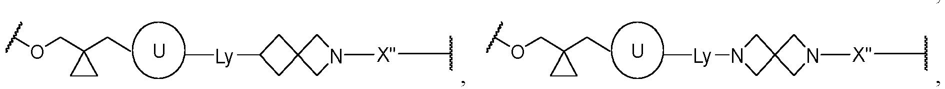

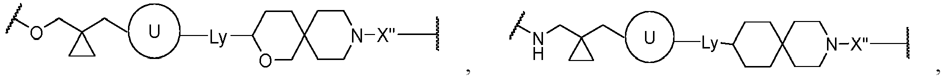

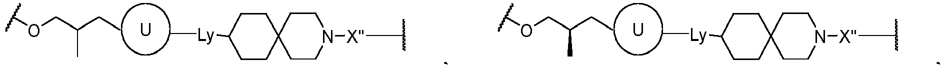

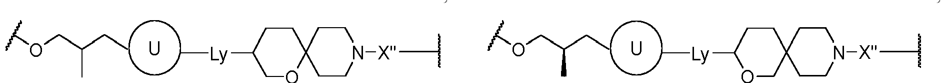

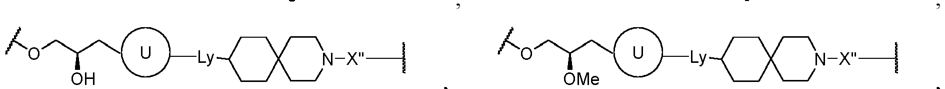

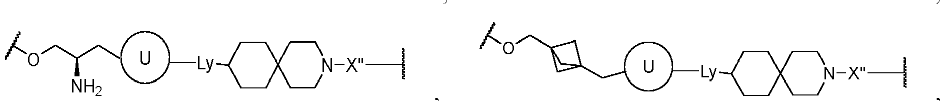

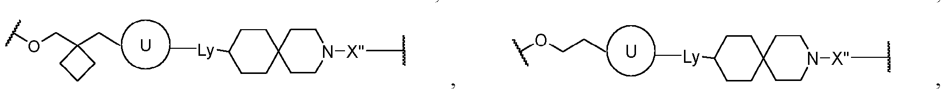

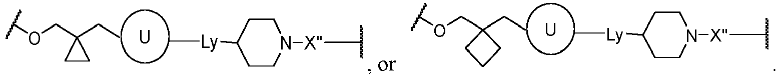

Preferably, in certain embodiments of the present disclosure, LA is of any one of the following structures:

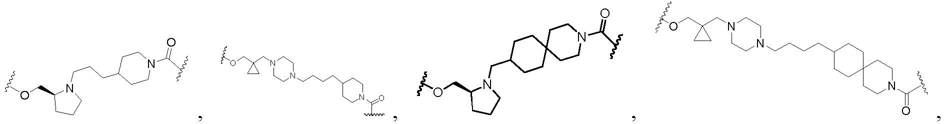

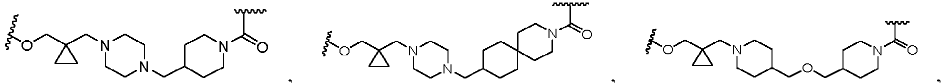

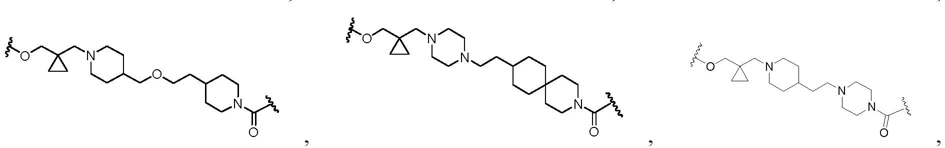

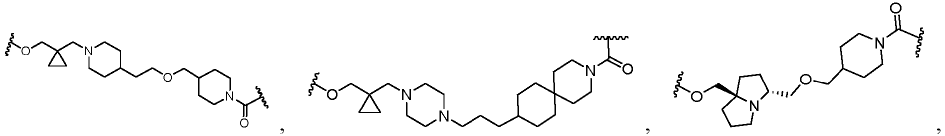

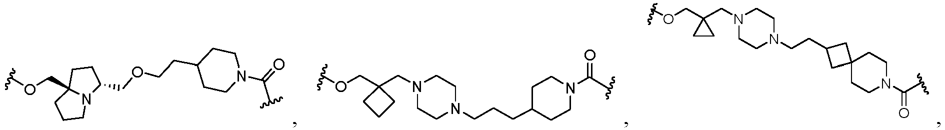

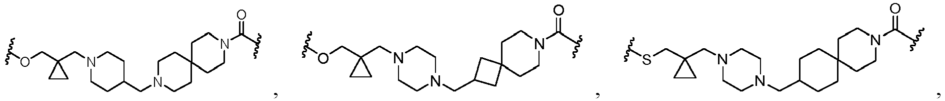

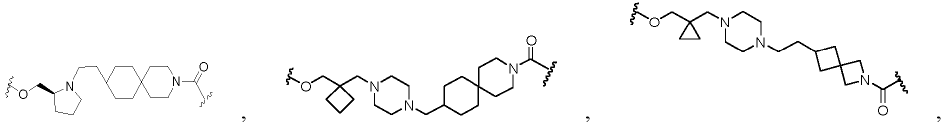

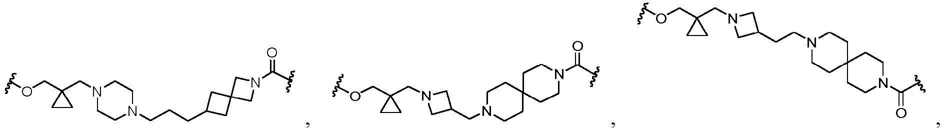

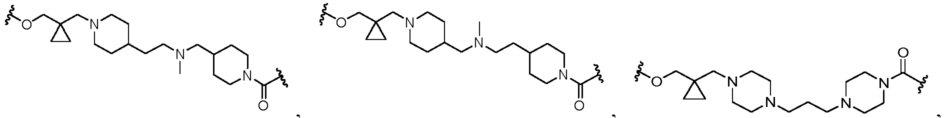

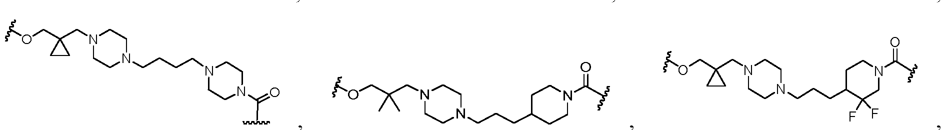

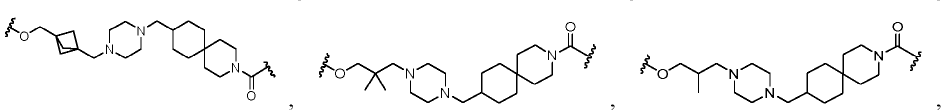

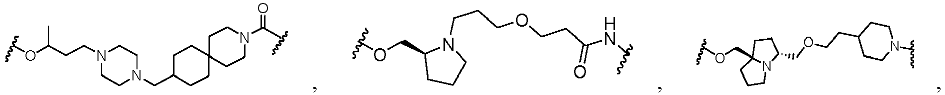

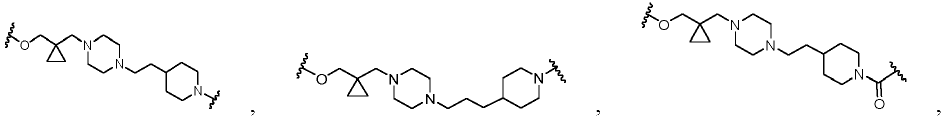

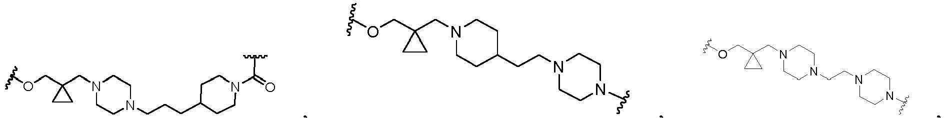

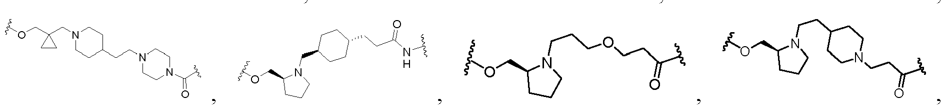

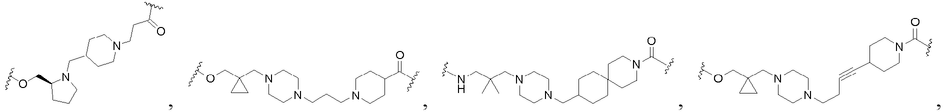

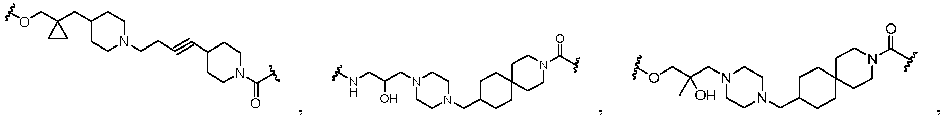

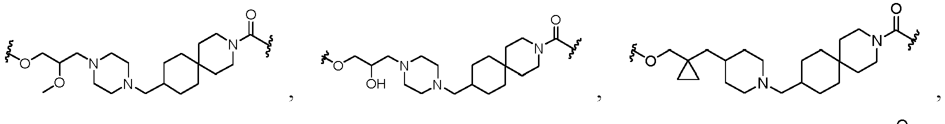

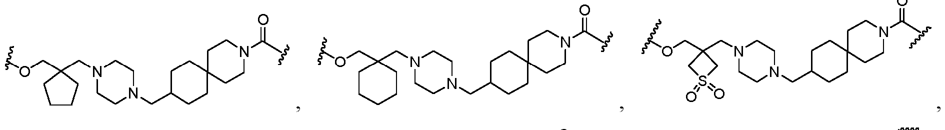

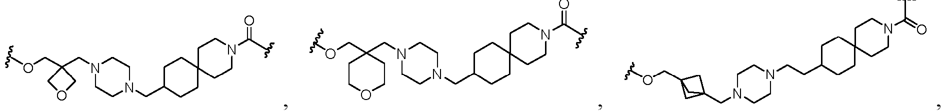

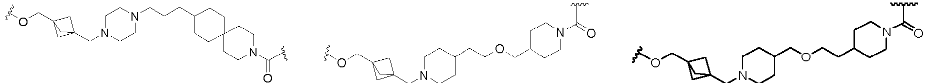

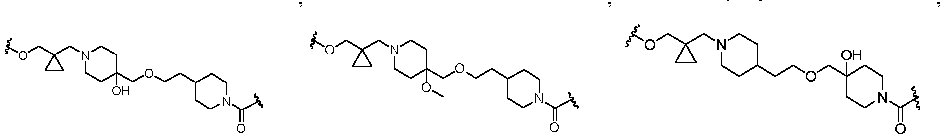

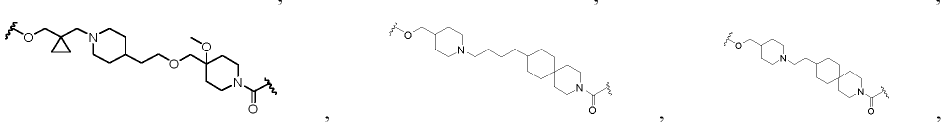

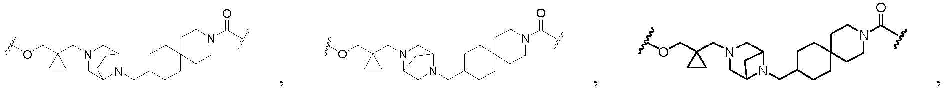

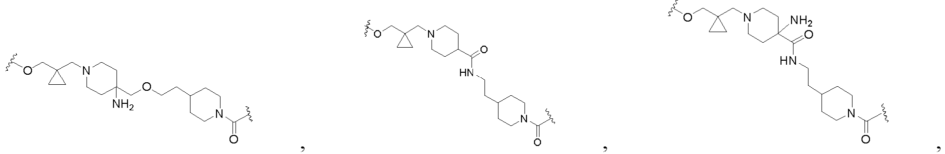

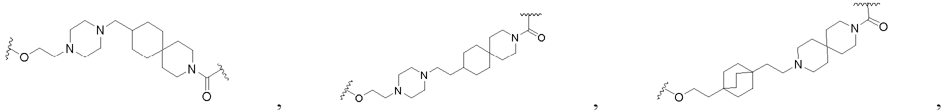

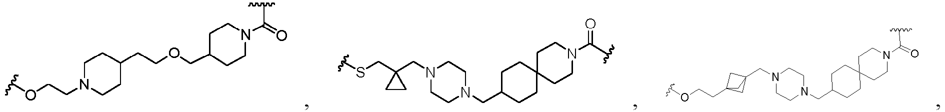

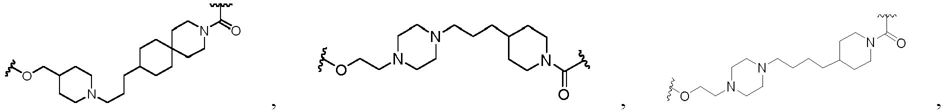

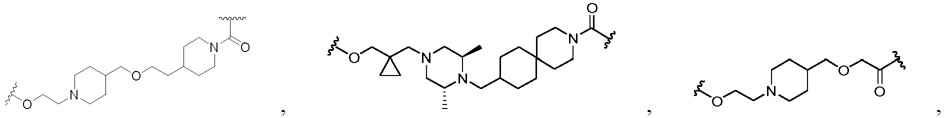

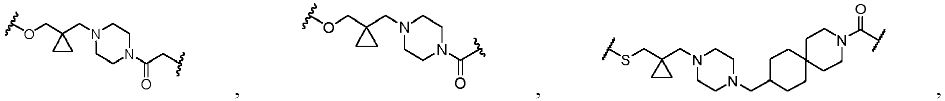

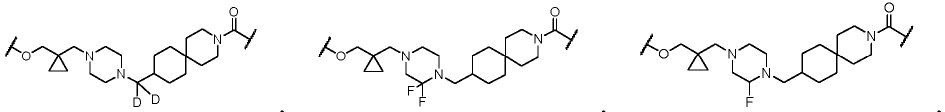

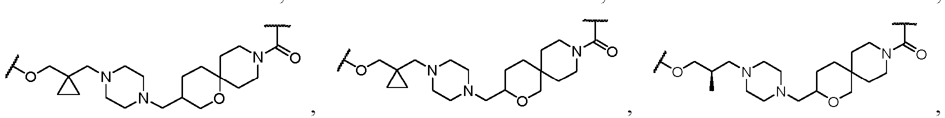

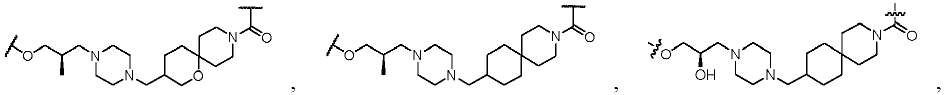

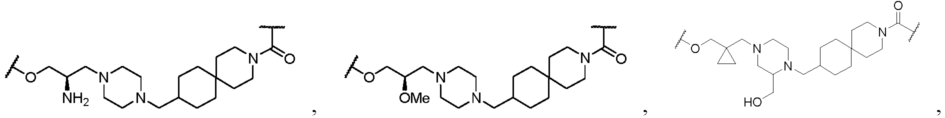

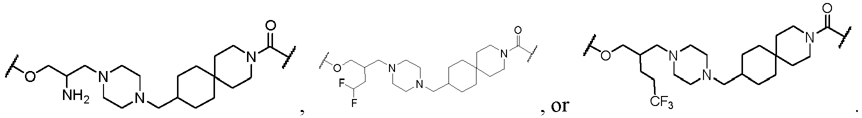

Preferably, in certain embodiments of the present disclosure, E1 has a structure of formula E1-1a, E1-1b, E1-1c, El-1d, E1-1e, E1-1f, E1-1g, E1-1aa, E E1-1h, 1-1bb, E1-1cc, E1-1dd, E1-1ee, E1-1ff, E1-1gg, or E1-1hh:

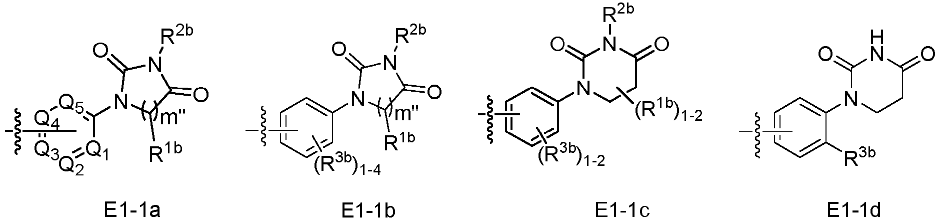

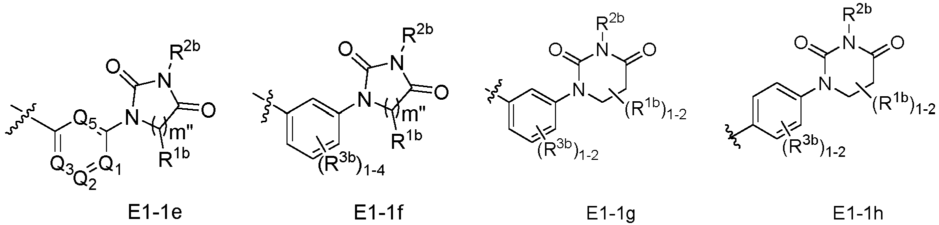

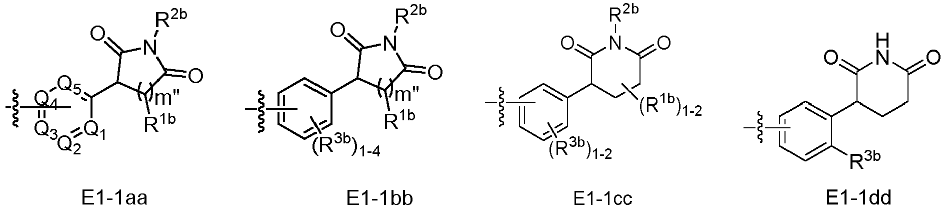

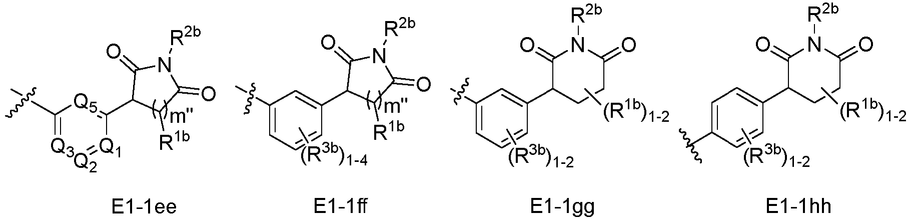

wherein Q₁, Q₂, Q₃, Q₄, Q₅, R₁", R₂", R₃", and m" are as defined and described in the present disclosure.

Preferably, in certain embodiments of the present disclosure, E1 has a structure of formula E1-1h", E1-1i', E1-1j', or E1-1h"h": 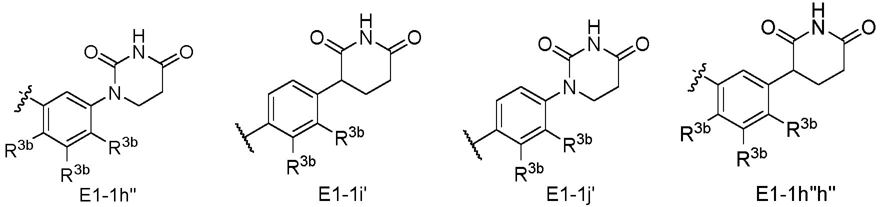 wherein R^{3b} is as defined and described in the present disclosure.

Preferably, in certain embodiments of the present disclosure, R^{3b} is hydrogen, halogen, cyano, - OH, -NH₂, C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl), wherein the C₁-C₆ alkyl, C₃-C₈ cycloalkyl, 3- to 8-membered heterocycloalkyl, -O(C₁-C₆ alkyl), - O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl) is optionally substituted with 1-3 halogen, cyano, -OH, and -NH₂. Alternatively, the C₁-C₆ alkyl, C₃-C₈ cycloalkyl, 3- to 8-membered heterocycloalkyl, -O(C₁-C₆ alkyl), -O(C₃-C₈ cycloalkyl), -O(3-to 8-membered heterocycloalkyl), -N(C₁-C₆ alkyl)₁₋₂, -N(C₃-C₈ cycloalkyl)₁₋₂, and -S(C₁-C₆ alkyl) may also be optionally substituted with 1, 2, or 3 deuterium.

Preferably, in certain embodiments of the present disclosure, E1 is:

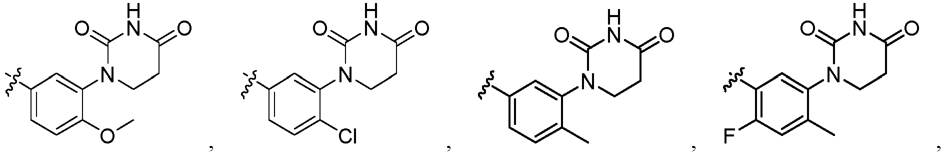

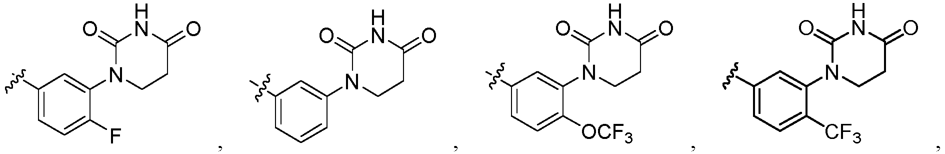

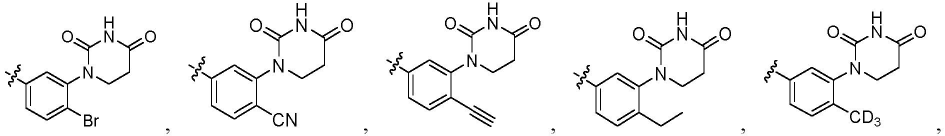

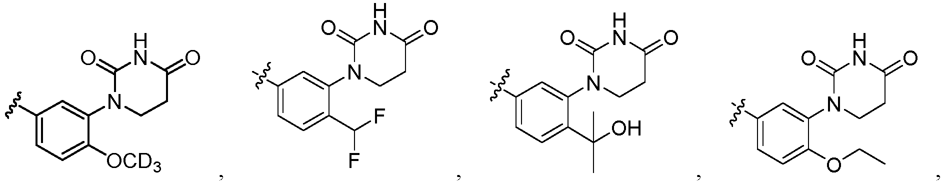

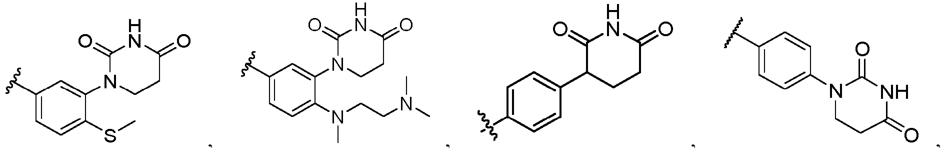

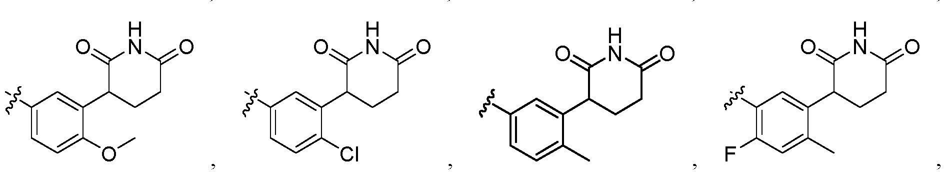

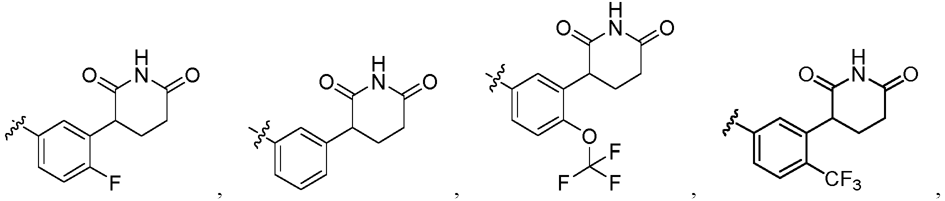

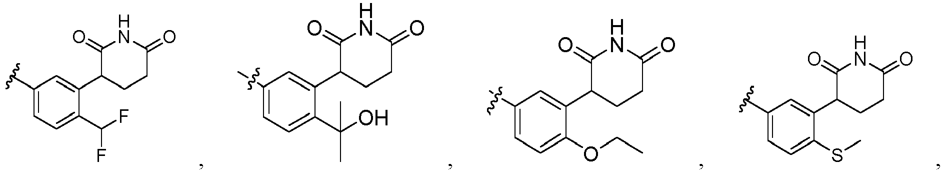

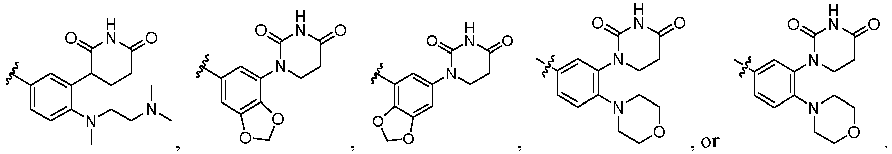

Preferably, in certain embodiments of the present disclosure, E2 has a structure of formula E2-1a, E2-1b, E2-1c, E2-1d, E2-1e, or E2-1f: 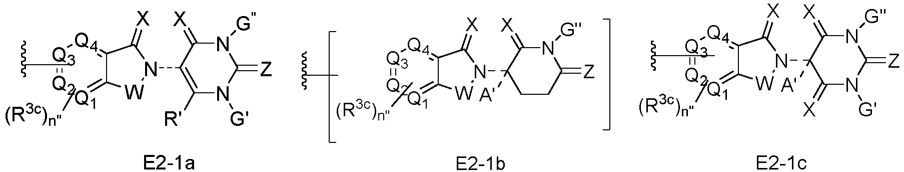 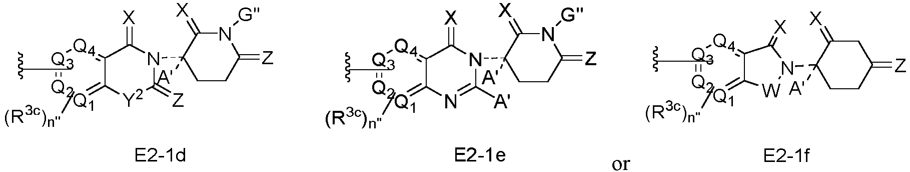 wherein in E2-1a, E2-1b, E2-1c, E2-1d, E2-1e, and E2-1f:
W is CR^{1c}R^{2c}, C(S), C(O), or SO₂;
X is CH₂, O, or S;
Y² is NH, -N-C₁-C₆ alkyl, -N-C₆-C₁₀ aryl, -N-(5- to 10-membered heteroaryl), -N-C₃-C₈ cycloalkyl, -N-(3- to 8-membered heterocycloalkyl), O, or S;
Z is CH₂, O, or S;
G" and G' are each independently selected from hydrogen, deuterium, C₁-C₆ alkyl, OH, C₃-C₆ cycloalkyl, -CH₂-heterocycloalkyl, and -CH₂-phenyl, wherein the C₁-C₆ alkyl, C₃-C₆ cycloalkyl, -CH₂-heterocycloalkyl, or -CH₂-phenyl is optionally substituted with 1-3 groups selected from hydroxy, halogen, cyano, and amino;
Q₁, Q₂, Q₃, and Q₄ are each independently CR^{3b} or N;
A' is hydrogen, deuterium, C₁-C₆ alkyl, C₃-C₆ cycloalkyl, or halogen;
R^{1c}, R^{2c}, and R^{3c} are each independently selected from hydrogen, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, -CR_{'}R_{"}, -CR_{'}NR_{'}R_{"}, aryl, heteroaryl, C₃-C₈ cycloalkyl, 3- to 8-membered heterocycloalkyl, -P(O)(OR_{'})R_{"}, - P(O)R_{'}R_{"}, -OP(O)(OR_{'})R_{"}, -Cl, -F, -Br, -I, -CF₃, -CN, -NR_{'}SO₂NR_{'}R_{"}, -NR_{'}C(O)NR_{'}R_{"}, -C(O)NR_{'}C(O)R_{"}, - NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, -SO₂NR_{'}COR_{"}, -NO₂, - COR_{'}, -C(C=N-OR_{'})R_{"}, -CR_{'}=CR_{'}R_{"}, -CCR_{'}, -S(C=O)(C=N-R_{'})R_{"}, -SF₅, and -OCF₃;
R_{'} and R_{"} are each independently a bond, hydrogen, deuterium, C₁-C₆ alkyl, C₃-C₈ cycloalkyl, C₆-C₁₀ aryl, 5- to 10-membered heteroaryl, or 3- to 8-membered heterocycloalkyl;
n" is 0, 1, 2, or 3;
----- is a bond, which may be an R stereoisomer, an S stereoisomer, or a non-stereoisomer;
R^{3b} is as described and defined in the present disclosure.

Preferably, in certain embodiments of the present disclosure, E2 has a structure of formula E2-1bb, E2-1aa, or E2-1cc: 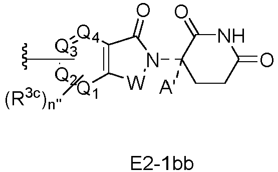 wherein in E2-1bb:
Q₁, Q₂, Q₃, and Q₄ are each independently CR^{3b} or N;
W is C(O) or CH₂;
A' is hydrogen, deuterium, C₁-C₆ alkyl, or halogen;
R^{3c} is hydrogen, deuterium, hydroxy, halogen, -NH₂, -N(C₁-C₆ alkyl)₁₋₂, C₁-C₆ alkyl, C₁-C₆ alkoxy, or C₁-C₆ haloalkyl;
n" is 0, 1, 2, or 3;
----- is a bond, which may be an R stereoisomer, an S stereoisomer, or a non-stereoisomer;
R^{3b} is as described and defined in the present disclosure; 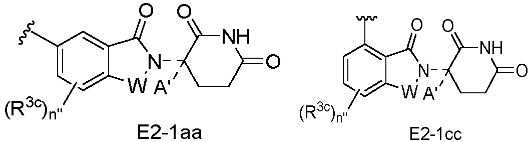
wherein in E2-1aa or E2-1cc:
   W is CH₂ or C(O);
   A' is hydrogen, methyl, Cl, or F;
   each R^{3c} is independently hydrogen, halogen, cyano, hydroxy, NH₂, C₁-C₆ alkyl, or C₁-C₆ alkoxy;
   n" is 0, 1, 2, or 3;
   ----- is a bond, which may be an R stereoisomer, an S stereoisomer, or a non-stereoisomer.

Preferably, in certain embodiments of the present disclosure, E2 is:

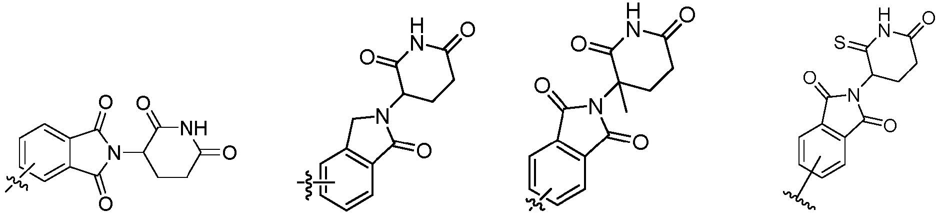

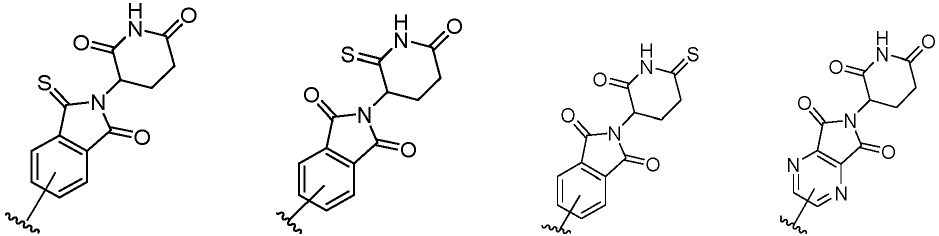

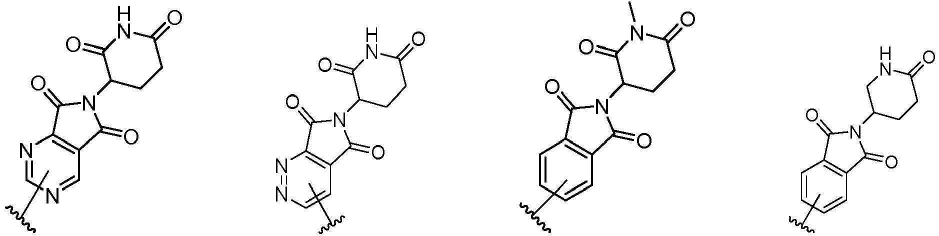

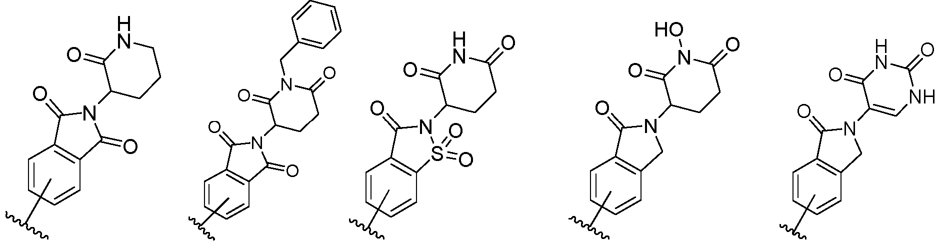

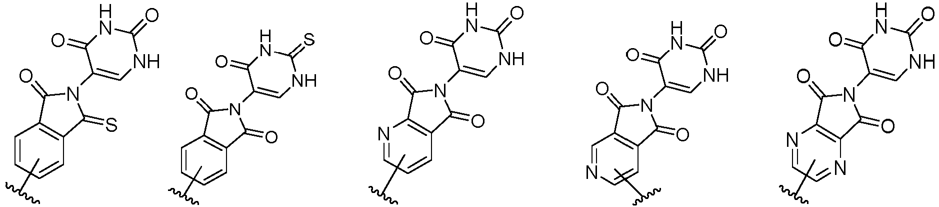

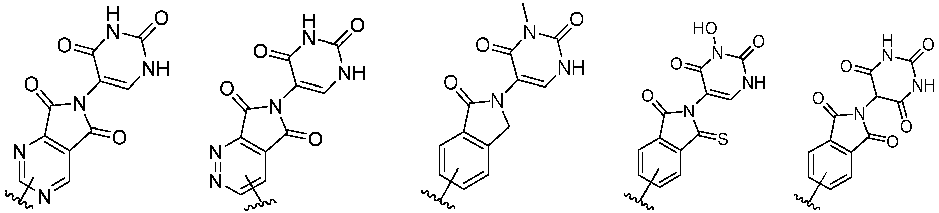

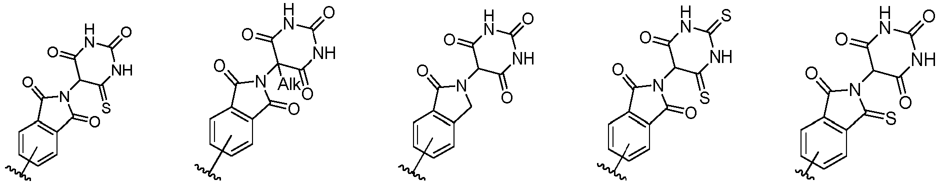

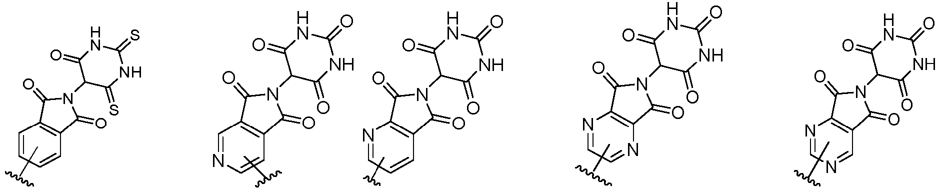

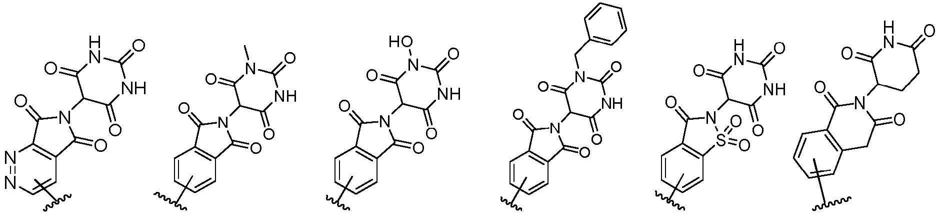

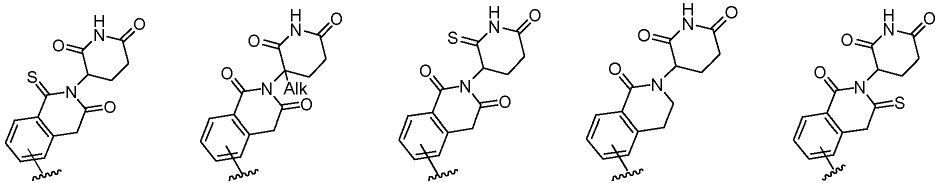

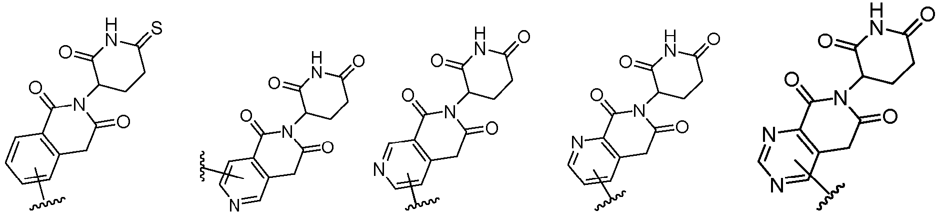

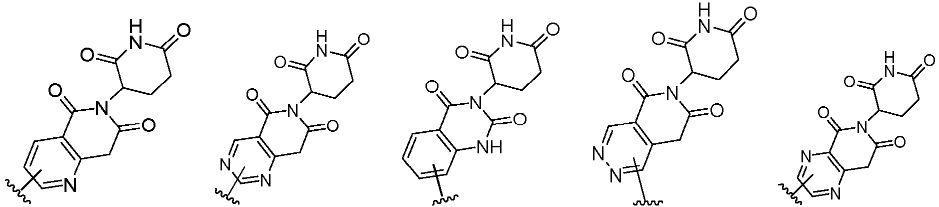

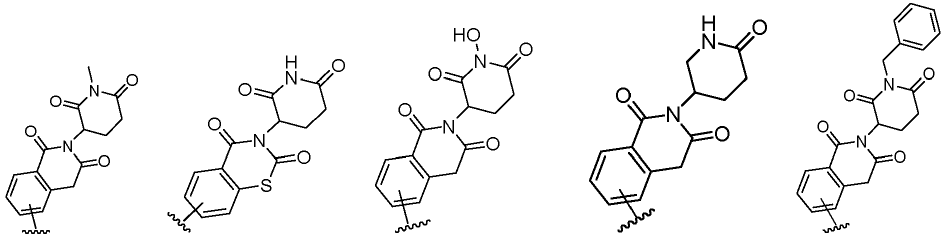

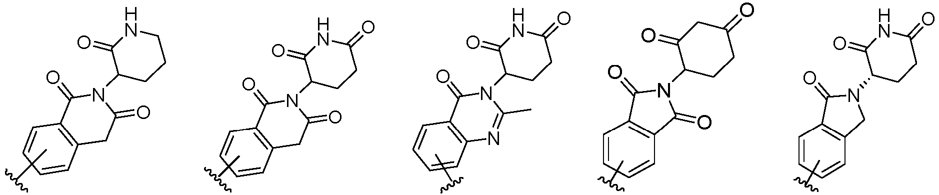

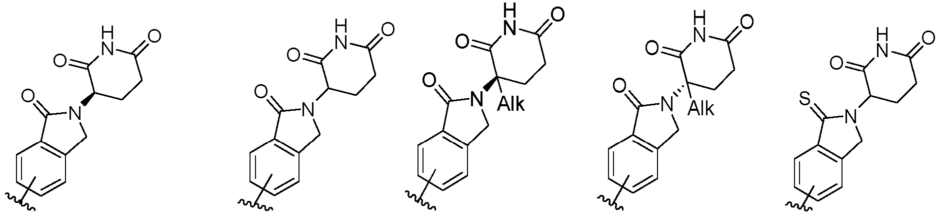

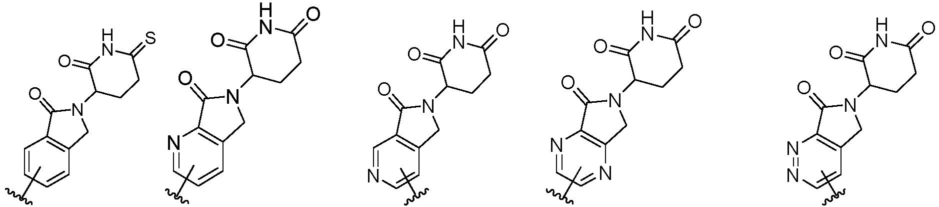

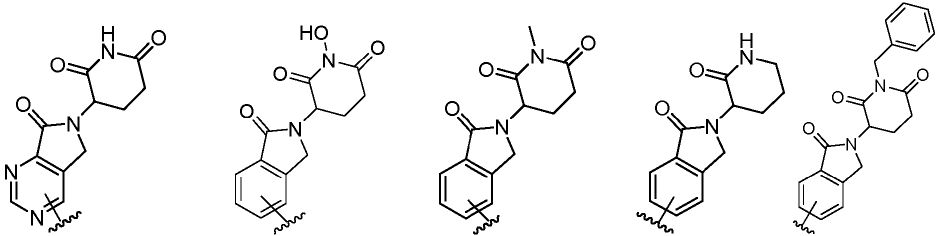

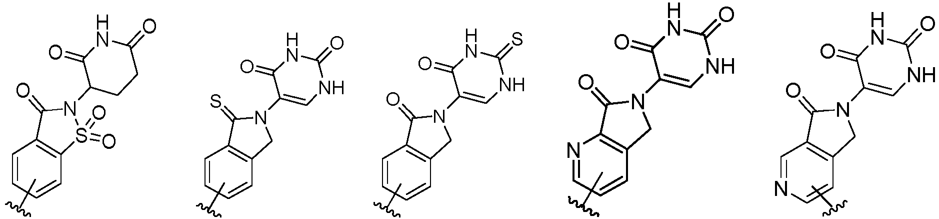

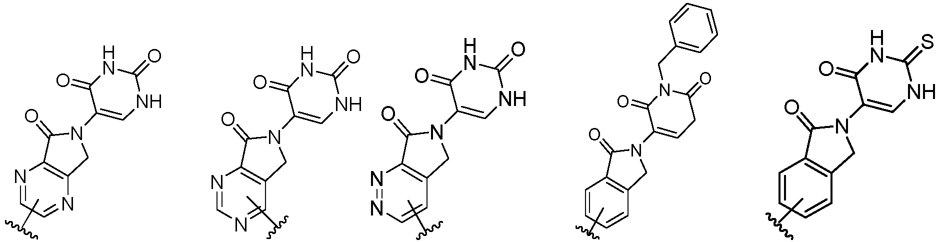

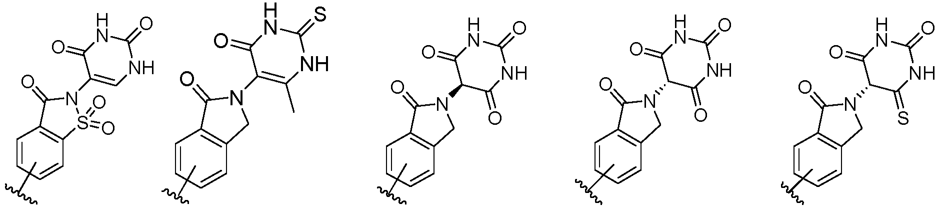

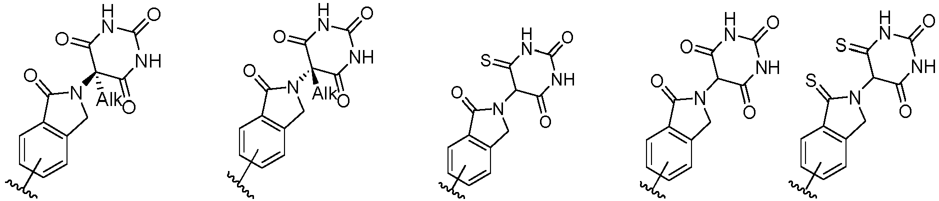

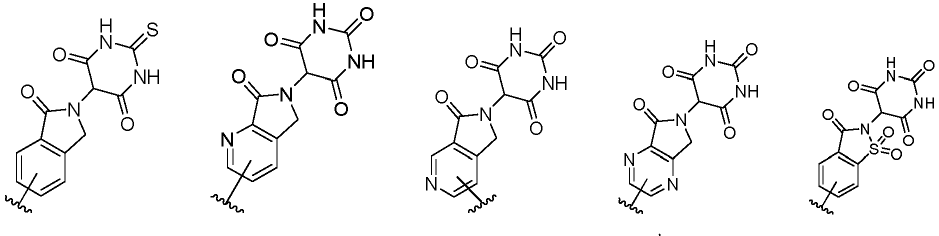

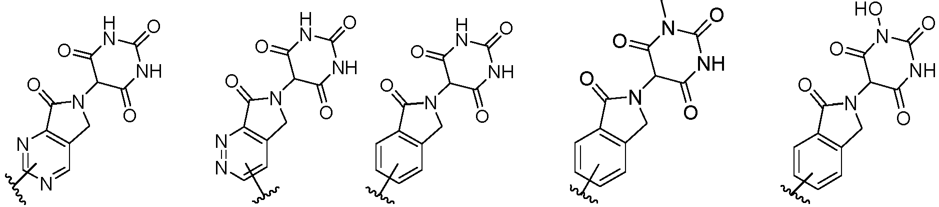

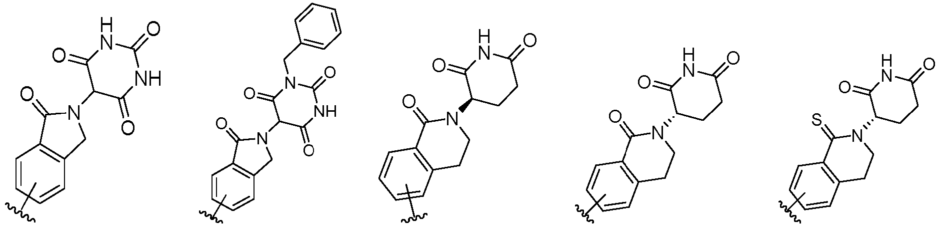

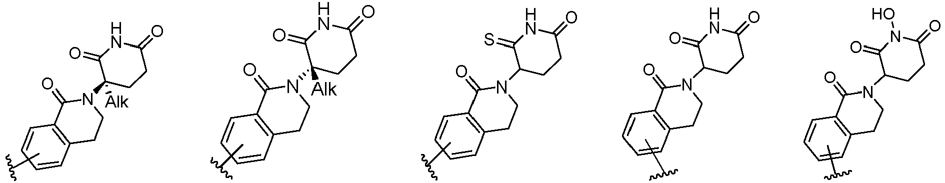

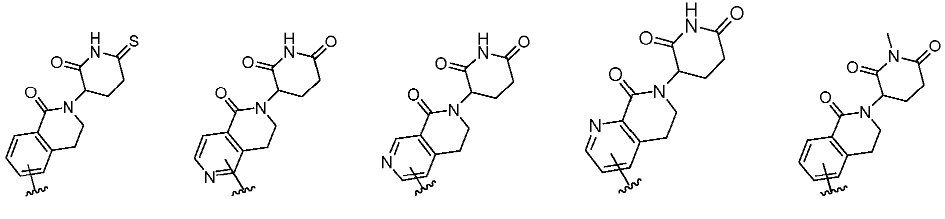

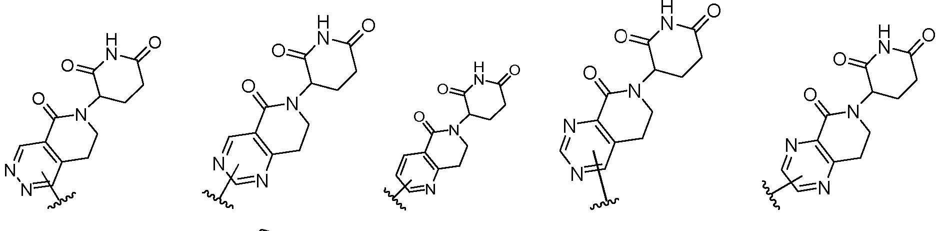

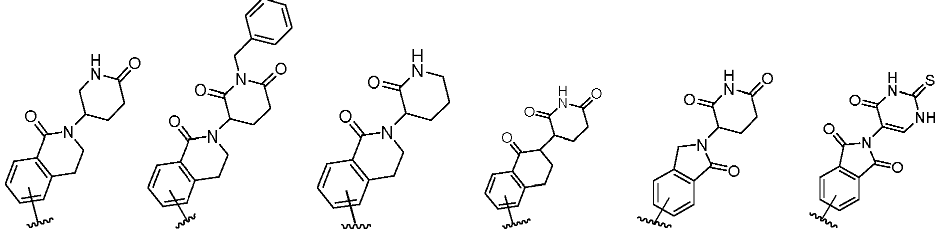

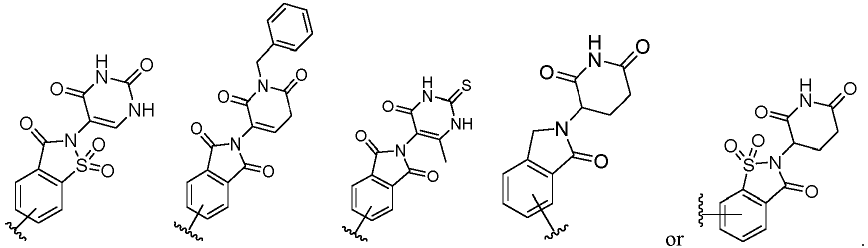

Preferably, in certain embodiments of the present disclosure, E3 has a structure of formula E3-1: 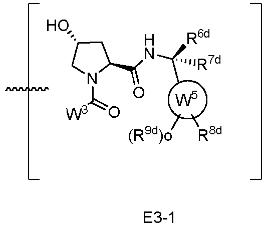
W³ is C₆-C₁₀ aryl, 5- to 10-membered heteroaryl, or 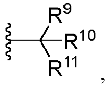 wherein the aryl or heteroaryl is optionally substituted;
R⁹ and R¹⁰ are each independently selected from hydrogen, C₁-C₆ alkyl, C₃-C₈ cycloalkyl, and 5-to 10-membered heteroaryl, wherein the C₁-C₆ alkyl, C₃-C₈ cycloalkyl, or 5- to 10-membered heteroaryl is optionally substituted with 1 or more -OH, halogen, or -NH₂;
or R⁹ and R¹⁰, together with the carbon atom linked thereto, form C₃-C₈ cycloalkyl, wherein the C₃-C₈ cycloalkyl is optionally substituted with -OH, halogen, -NH₂, or C₁-C₃ alkyl;
R¹¹ is selected from C₁-C₆ alkyl, C₁-C₆ alkoxy, 3- to 8-membered heterocycloalkyl, C₆-C₁₀ aryl, 5-to 10-membered heteroaryl, and 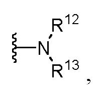 , wherein the C₁-C₆ alkyl, C₁-C₆ alkoxy, 3- to 8-membered heterocycloalkyl, C₆-C₁₀ aryl, or 5- to 10-membered heteroaryl is optionally substituted with 1 or more -OH, halogen, or -NH₂;
R¹² is selected from H, C(O), and substituted alkyl;
R¹³ is selected from H, C₁-C₆ alkyl, -alkyl CO-, -(cycloalkyl)alkyl CO-, -aralkyl CO-, -aryl CO-, -(heterocycloalkyl)CO-, and arylalkyl, wherein the alkyl, -alkyl CO-, -(cycloalkyl)alkyl CO-, -aralkyl CO-, -aryl CO-, -(heterocycloalkyl)CO-, or arylalkyl is optionally substituted;
R¹⁶ is H, halogen, -OH, C₁-C₆ alkyl, or C₁-C₆ alkoxy, wherein the C₁-C₆ alkyl or C₁-C₆ alkoxy is optionally substituted with halogen;
   i.is 1, 2, 3, or 4;
R^{8d}, R^{14a}, R^{14b}, and R¹⁵ are as described and defined in the present disclosure.

Preferably, in certain embodiments of the present disclosure, E3 has a structure of formula E3-1a, E3-1b, or E3-1c: 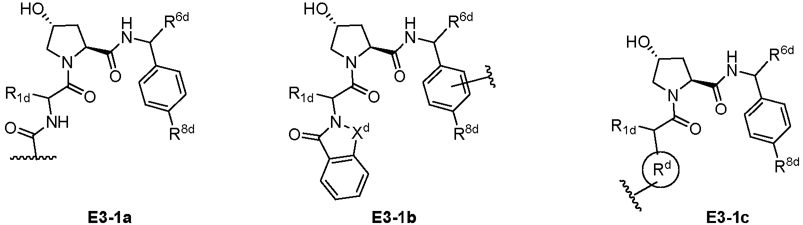 wherein,
R_{1d} is H, ethyl, isopropyl, *tert-*butyl, *sec*-butyl, cyclopropyl, cyclobutyl, cyclopentyl, cyclohexyl, alkyl, hydroxyalkyl, heteroaryl, or haloalkyl, wherein the alkyl, hydroxyalkyl, or heteroaryl is optionally substituted;
R^{6d} is H, -CH₃, -CH₂F, -CH₂OH, ethyl, isopropyl, or cyclopropyl;
R^{8d} is H, halogen, CN, OH, NO₂, C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl, wherein the C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl is optionally substituted with halogen, -OH, CN, NO₂, or amino;
X^{d} is CH₂ or C(O);
R^{d} is 5- to 6-membered heteroaryl, wherein the 5- to 6-membered heteroaryl is optionally substituted.

Preferably, in certain embodiments of the present disclosure, E3 has a structure of formula E3-1aa: 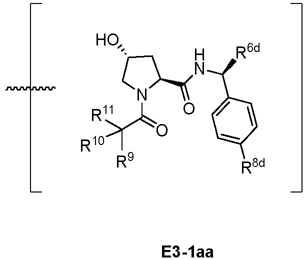 wherein,
R^{6d} is H, -CH₃, -CH₂F, -CH₂OH, ethyl, isopropyl, or cyclopropyl;
R⁹ is H;
R¹⁰ is H, ethyl, isopropyl, *tert-*butyl*, sec*-butyl, cyclopropyl, cyclobutyl, cyclopentyl, or cyclohexyl;
R¹¹ is 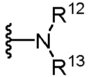 or 5- to 10-membered heteroaryl, wherein the 5- to 10-membered heteroaryl is optionally substituted with 1 or more -OH, halogen, or -NH₂;
R¹² is H or C(O);
R¹³ is H, alkyl, -alkyl CO-, -(cycloalkyl)alkyl CO-, -aralkyl CO-, -aryl CO-, - (heterocycloalkyl)CO-, or arylalkyl, wherein the alkyl, -alkyl CO-, -(cycloalkyl)alkyl CO-, -aralkyl CO-, - aryl CO-, -(heterocycloalkyl)CO-, or arylalkyl is optionally substituted;
R^{8d} is H, halogen, CN, OH, NO₂, nn,

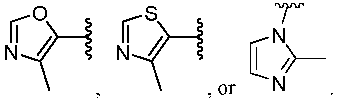

More preferably, formula E3-1aa is linked to L via R¹².

Preferably, in certain embodiments of the present disclosure, E3 is:

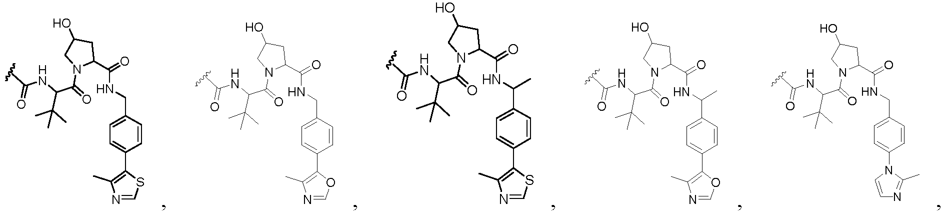

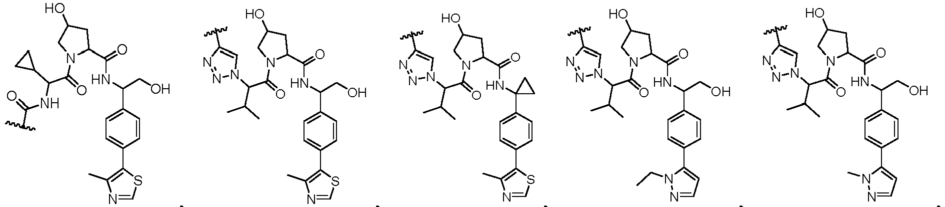

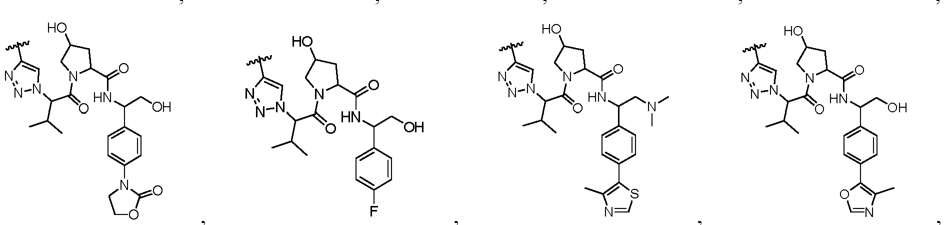

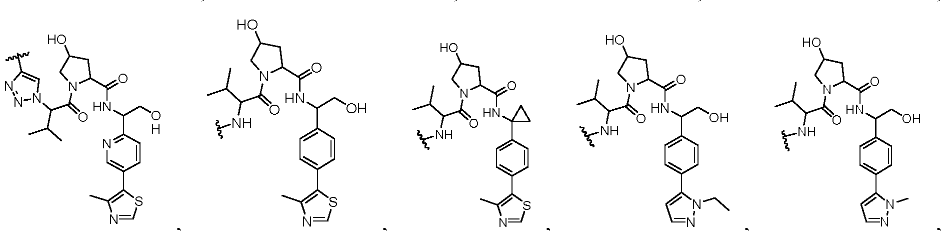

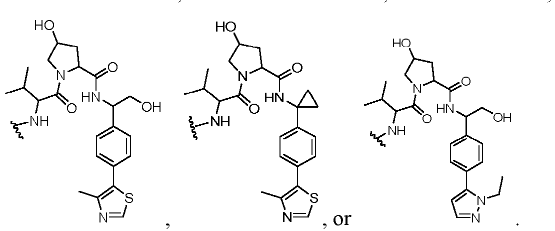

Preferably, in certain embodiments of the present disclosure, E3 is:

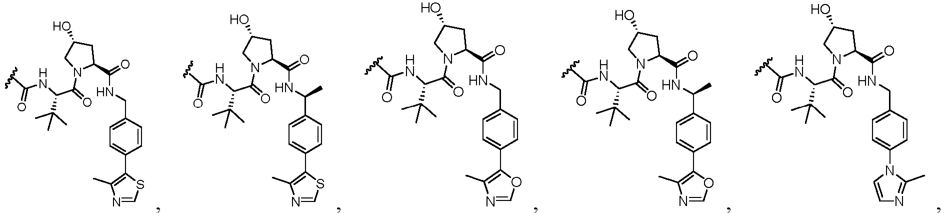

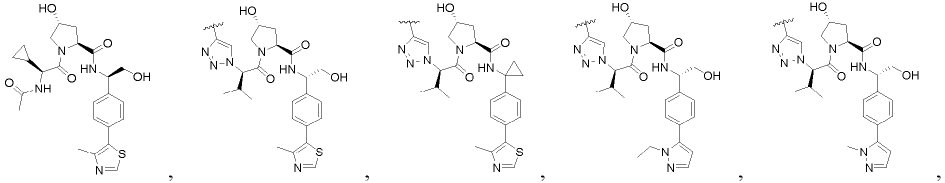

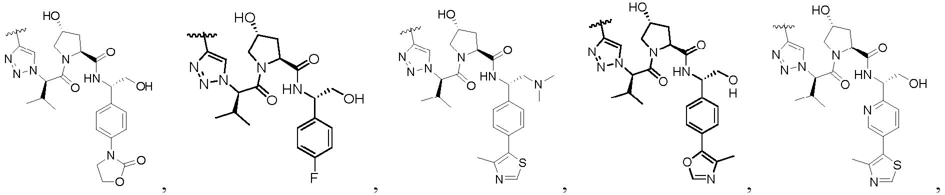

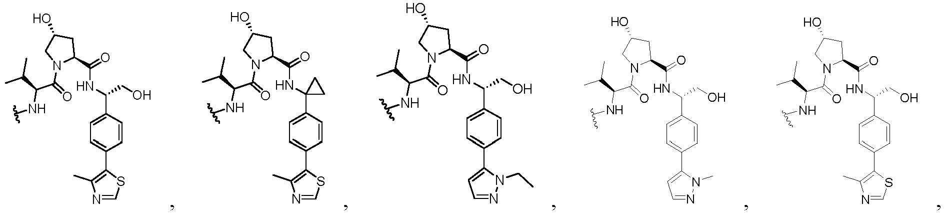

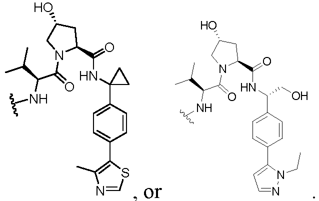

In one preferred embodiment of the present disclosure, in the compound of formula I or I', 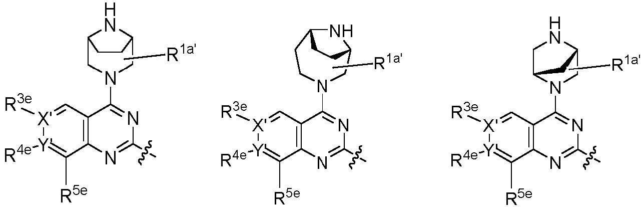
G1 is 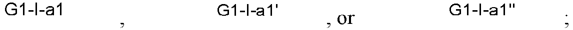
G1' is 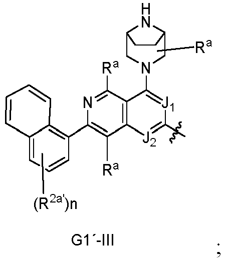
L is 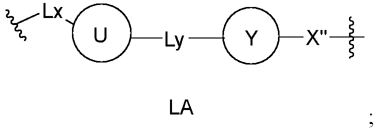
E is independently E1: 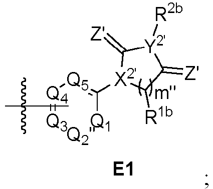
R^{1a}', R^{3e}, R^{4e}, R^{5e}*,* X', Y', each R^{a}, R^{2a}', J₁, J₂, Lx, Ly, ring U, ring Y, X", Q₁, Q₂, Q₃, Q₄, Q₅, X^{2'}, Y^{2'}, R^{1b}, R^{2b}, Z', and m" are as defined above.

In one preferred embodiment of the present disclosure,
in the compound of formula II or I', G1 is 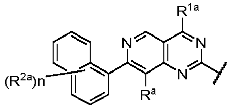 or 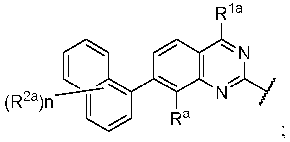 G1' is 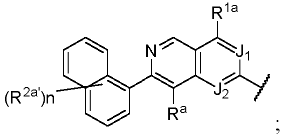
R^{1a} is 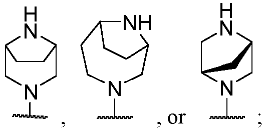
L is 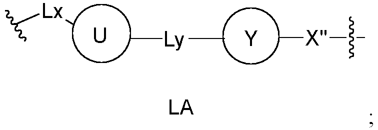
Lx is -(CH₂)ᵥ-, wherein two methylene groups in Lx are replaced by a group selected from -O- and -CR_{d}Rₑ-;
R_{d} and Rₑ are each independently H, C₁-C₆ alkyl, C₁-C₆ alkoxy, NH₂, or C₁-C₆ alkyl substituted with 1, 2, or 3 halogen, or R_{d} and Rₑ, together with the C atom linked thereto, form C₃-C₈ cycloalkyl;
v is 1, 2, 3, 4, 5, 6, or 7;
ring U is 3- to 12-membered heterocycloalkylene containing 1-2 N heteroatoms;
Ly is -(CH₂)ₖ-, wherein one or two methylene groups in Ly are optionally replaced by -O-; k is 1, 2, 3, 4, 5, 6, 7, or 8;
ring Y is 3- to 12-membered heterocycloalkylene containing 1, 2, or 3 heteroatoms selected from N and O;
X" is -C(O)-;
E is 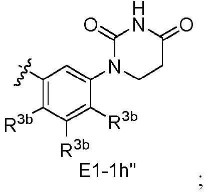
each R^{a}, R^{2a}, R^{2a}', J₁, J₂, n, and each R^{3b} are as defined above.

Preferably:
R^{2a} and R^{2a'} are each independently halogen, hydroxy, C₁-C₆ alkyl, or C₂-C₆ alkynyl; R^{a} is halogen;
L is of any one of the following structures: 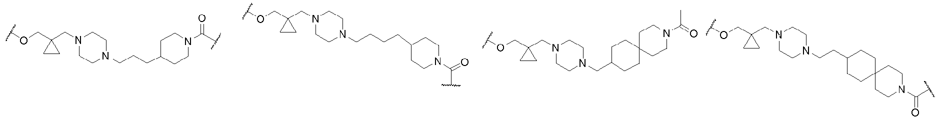 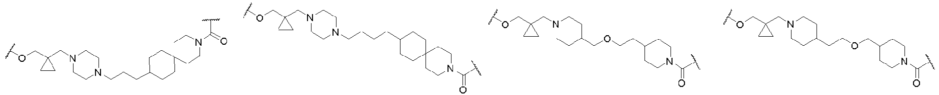 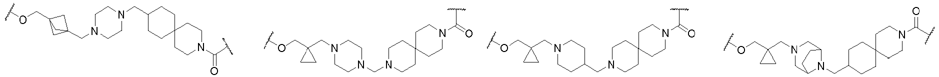 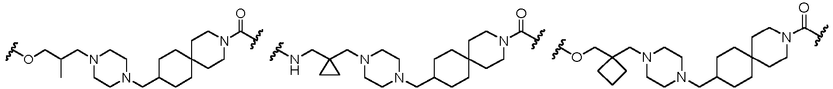 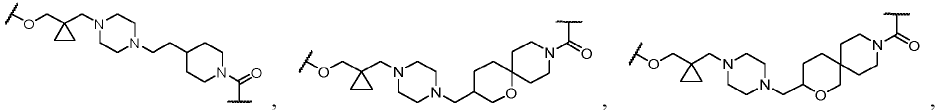 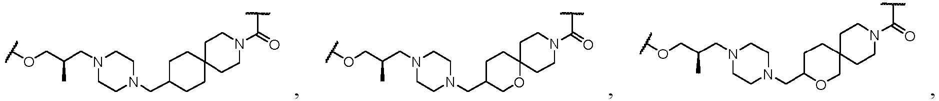 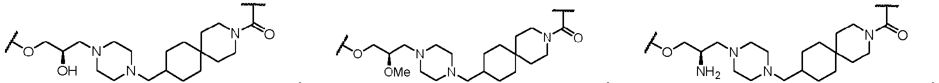 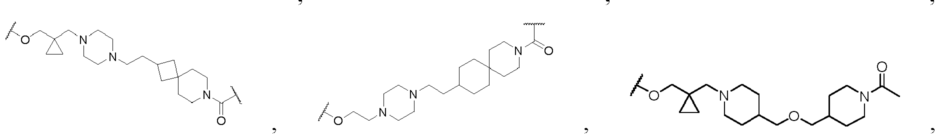 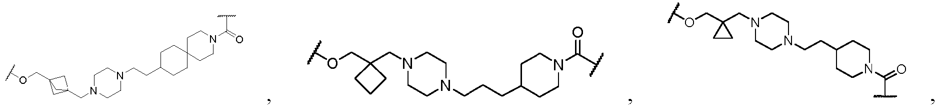 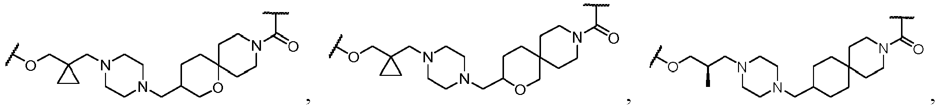 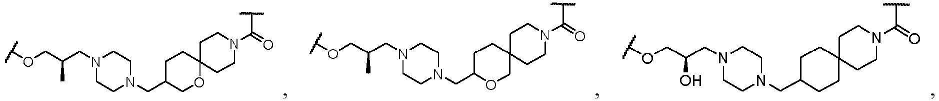 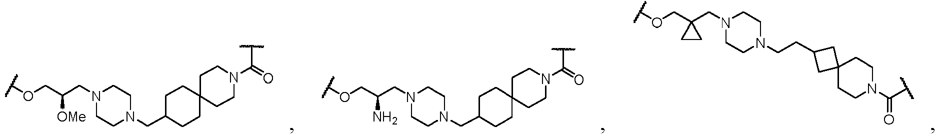 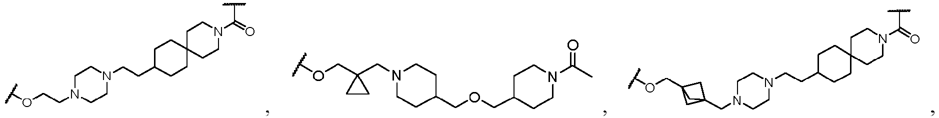 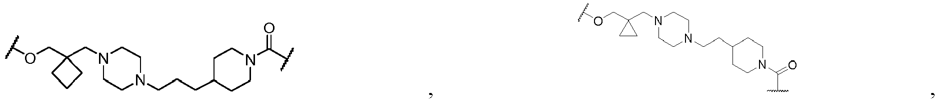 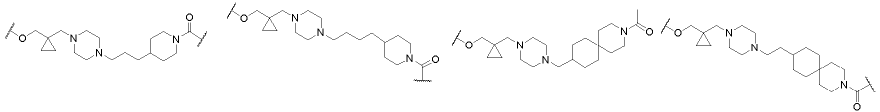 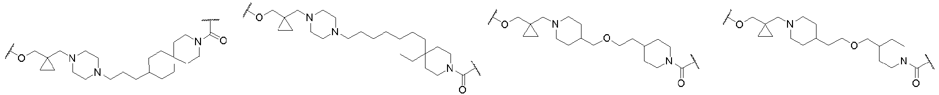 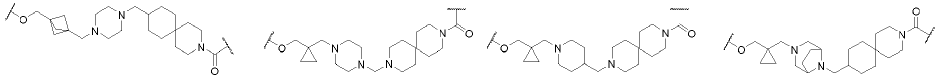 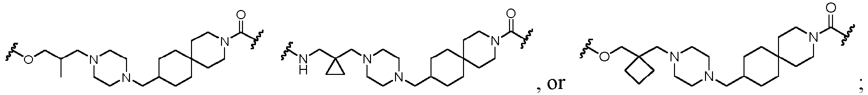
R^{3b} is halogen, C₁-C₆ alkyl, or -O-(C₁-C₆ alkyl), wherein the C₁-C₆ alkyl or -O-(C₁-C₆ alkyl) is optionally substituted with 1-3 groups independently selected from deuterium and halogen. Preferably, in one preferred embodiment of the present disclosure, in the compound of formula I or I', G1 is 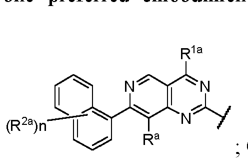 G1' is 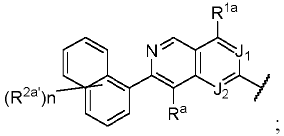
R^{1a} is 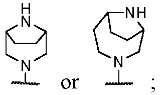
L is 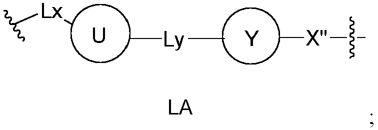
Lx is -(CH₂)ᵥ-, wherein two methylene groups in Lx are replaced by a group selected from -O- and -CR_{d}Rₑ-;
R_{d} and Rₑ are each independently C₁-C₆ alkyl, or R_{d} and Rₑ, together with the C atom linked thereto, form C₃-C₈ cycloalkyl;
v is 1, 2, 3, 4, 5, 6, or 7;
ring U is 3- to 12-membered heterocycloalkylene containing 1-2 N heteroatoms;
Ly is -(CH₂)ₖ-, wherein one or two methylene groups in Ly are optionally replaced by -O-; k is 1, 2, 3, 4, 5, 6, 7, or 8;
ring Y is 3- to 12-membered heterocycloalkylene containing 1-2 heteroatoms selected from N;
X" is -C(O)-;
E is

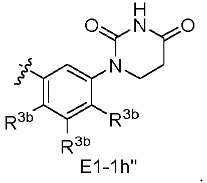

More preferably,
R^{2a} and R^{2a'} are each independently halogen, hydroxy, C₁-C₆ alkyl, or C₂-C₆ alkynyl; R^{a} is halogen;
L is a compound with any one of the following structures: 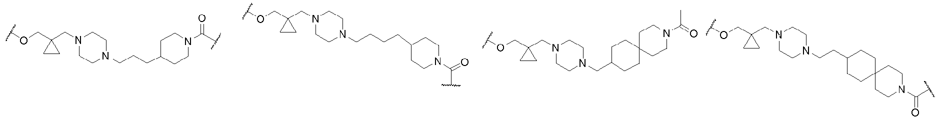 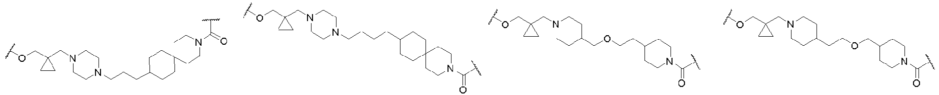 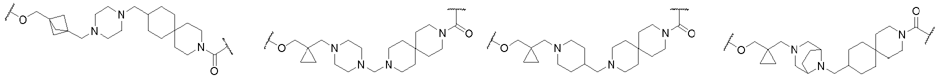 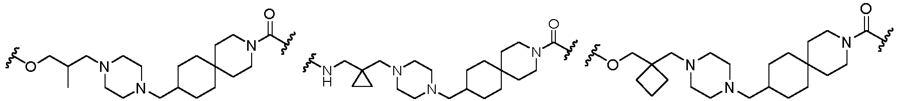 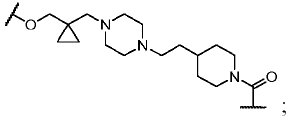
each R^{3b} is independently hydrogen, C₁-C₆ alkyl, or -O-(C₁-C₆ alkyl).

Preferably, in certain embodiments of the present disclosure, the compound of formula I or I' is: 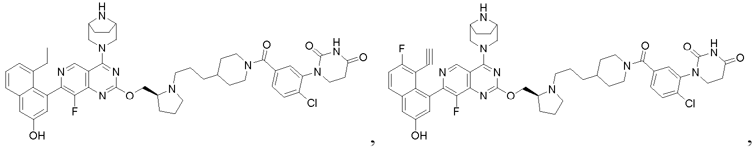 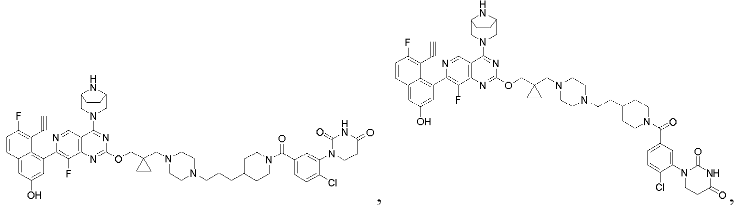 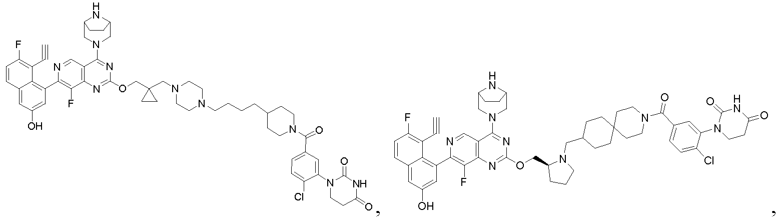 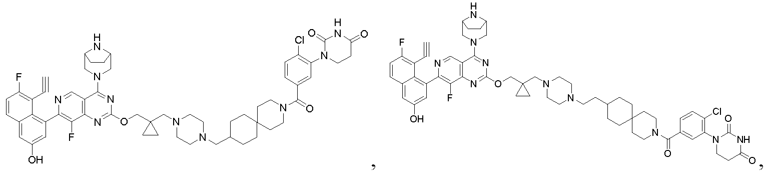 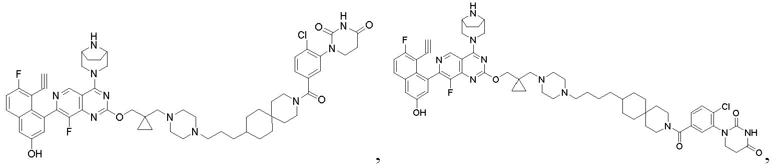 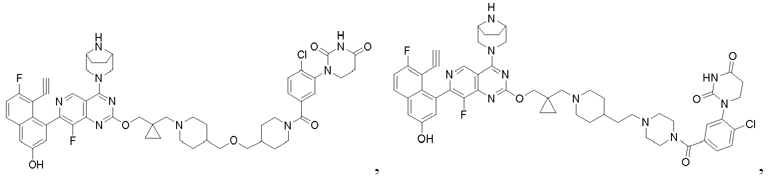 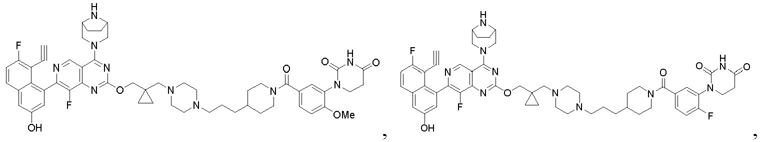 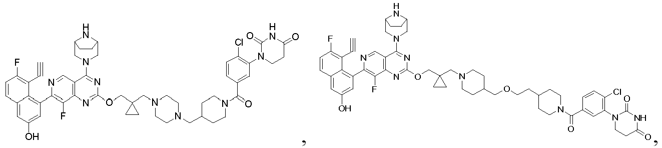 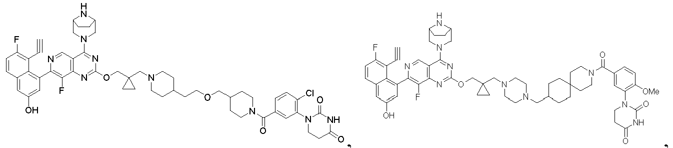 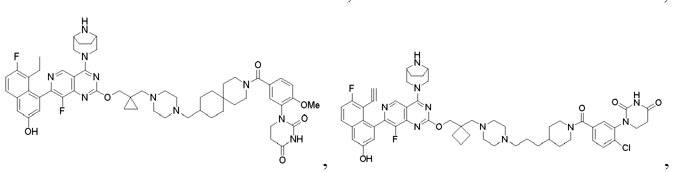 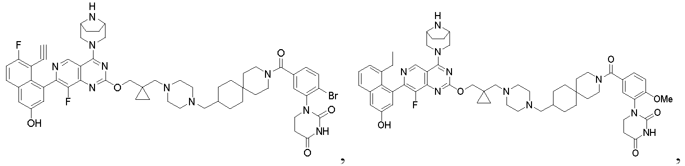 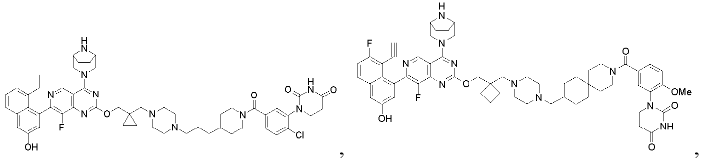 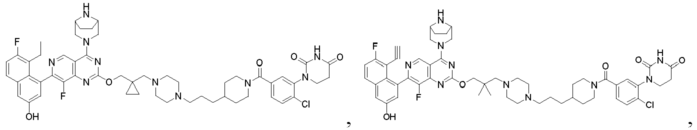 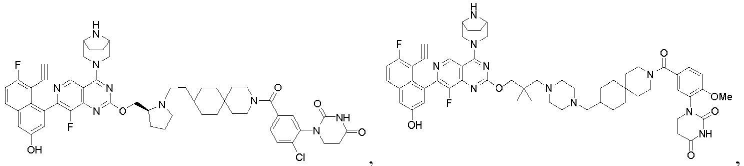 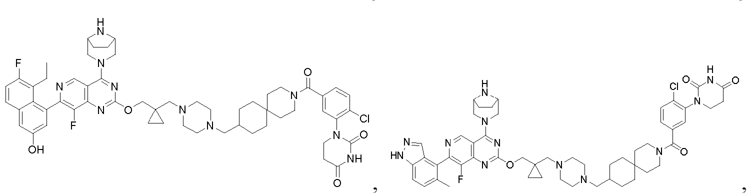 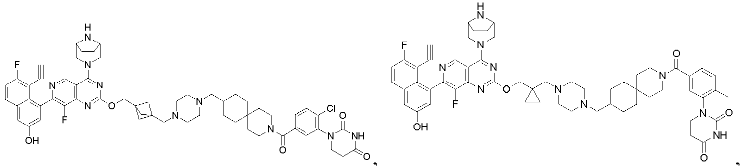 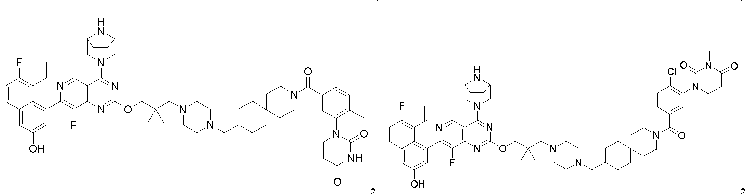 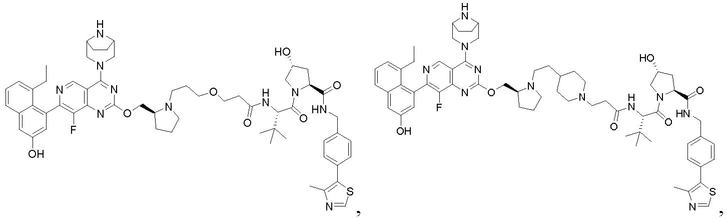 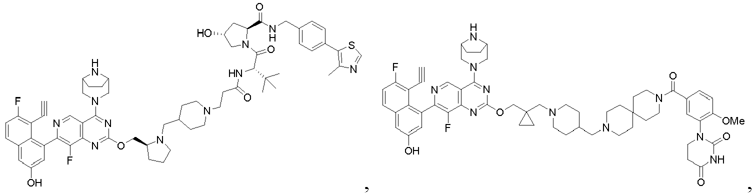 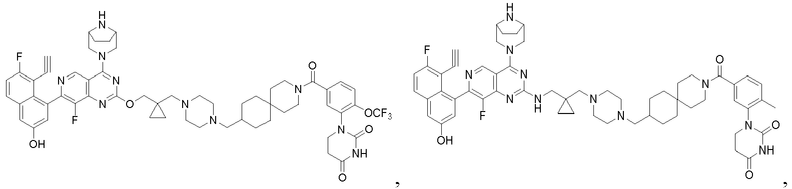 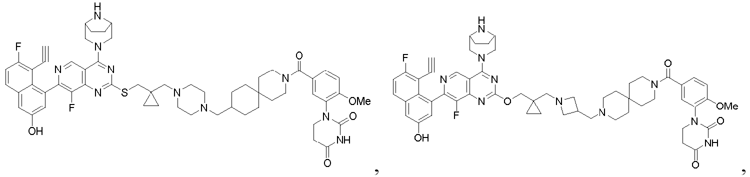 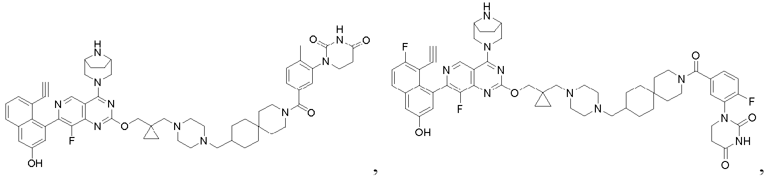 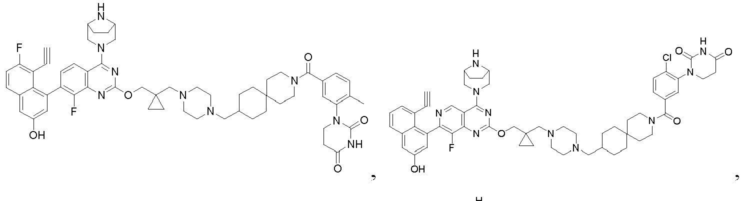 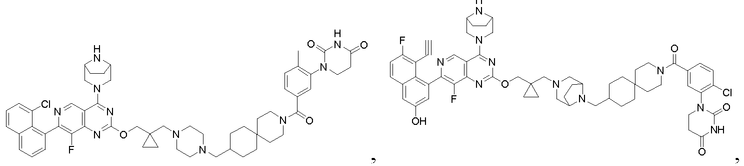 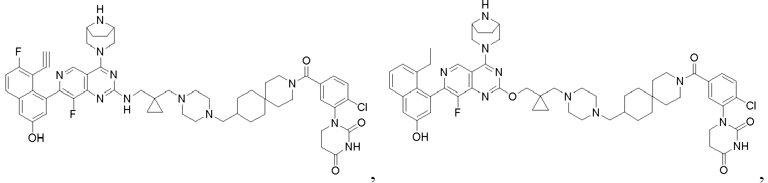 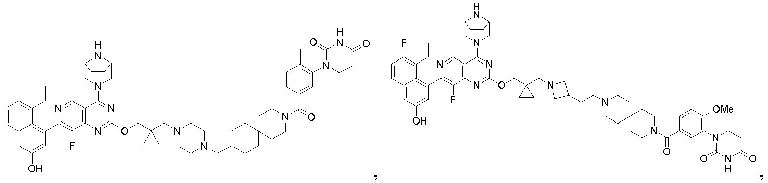 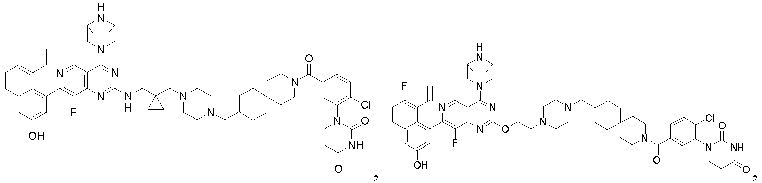 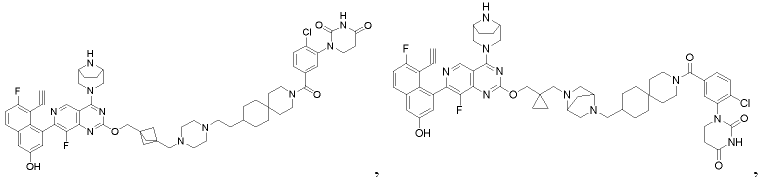 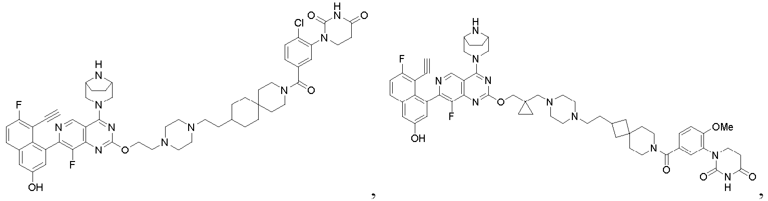 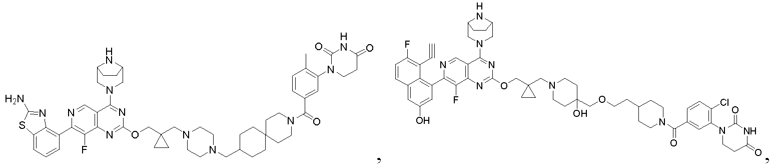 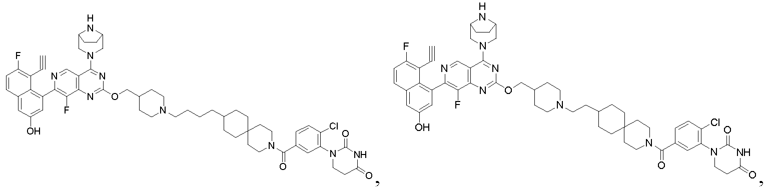 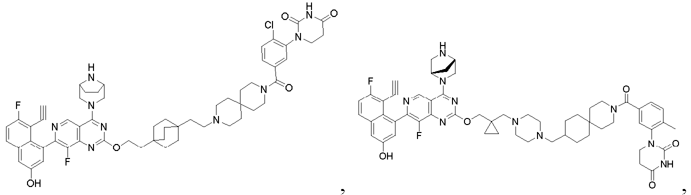 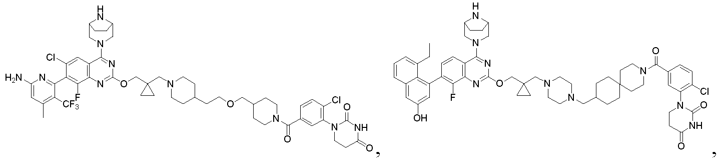 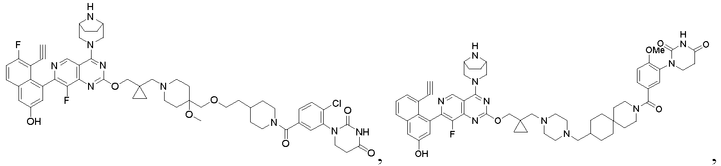 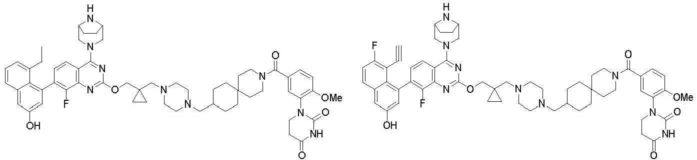 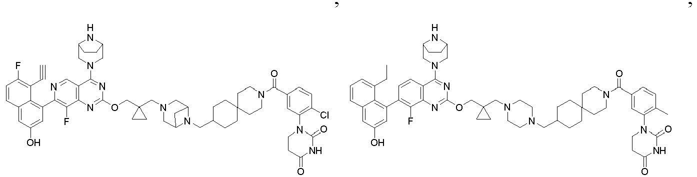 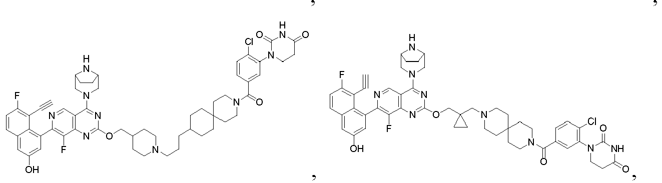 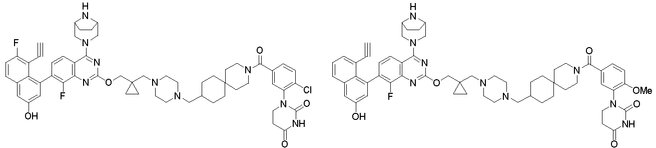 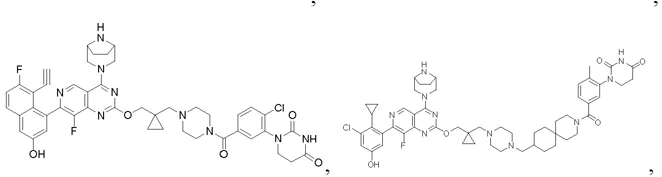 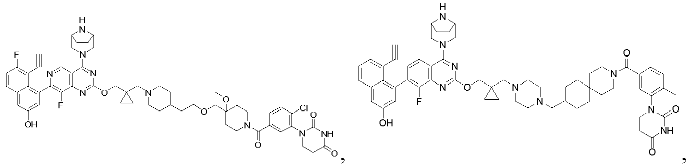 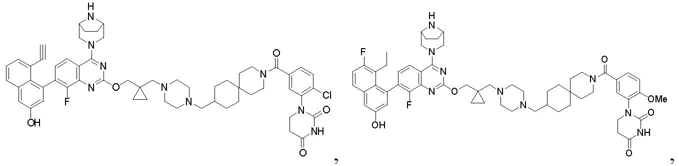 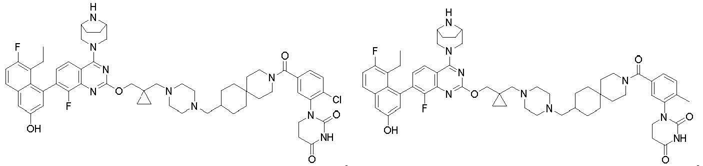 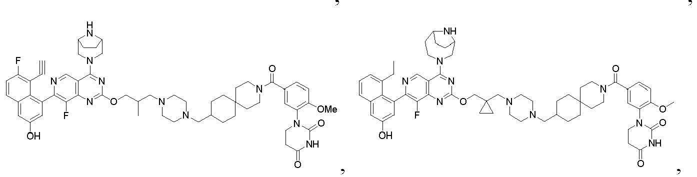 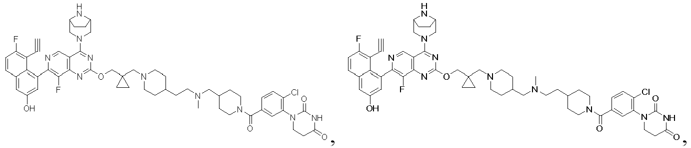 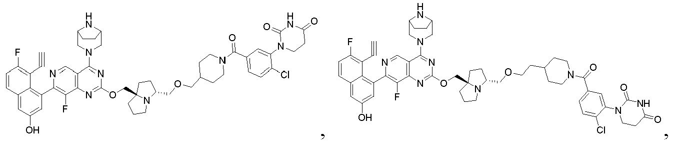 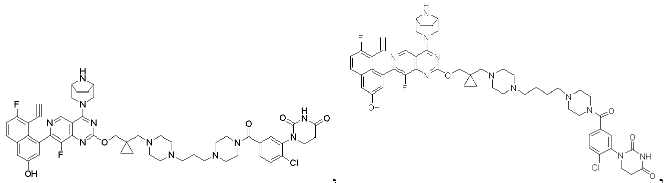 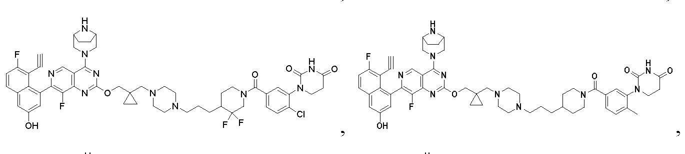 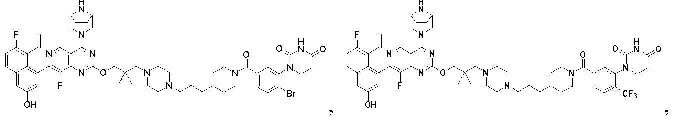 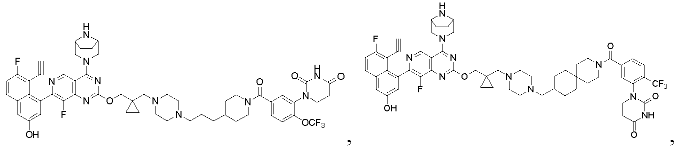 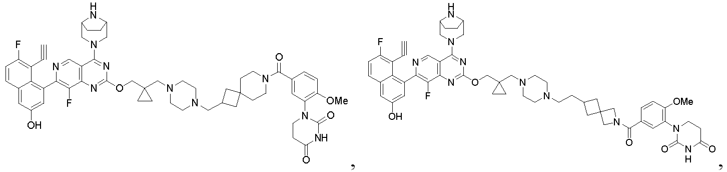 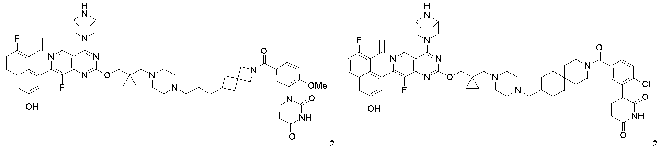 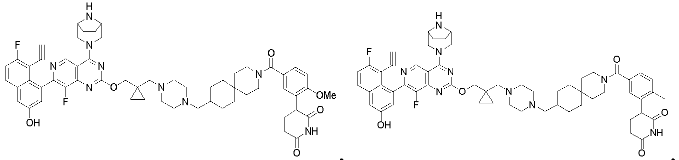 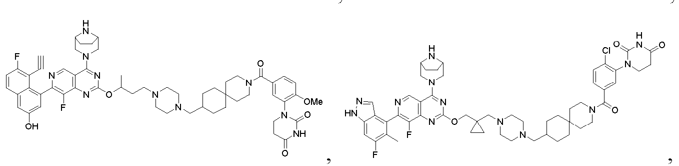 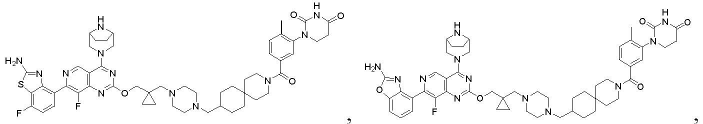 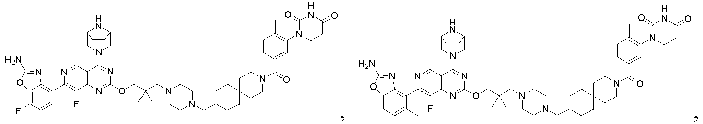 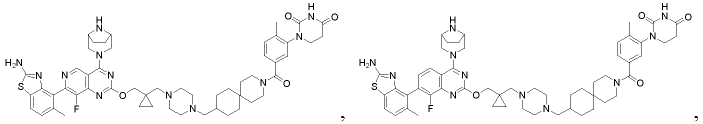 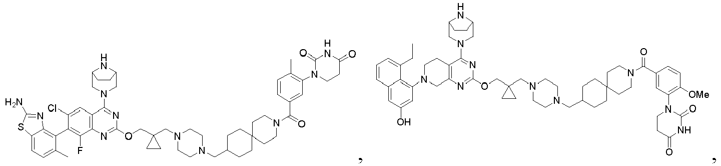 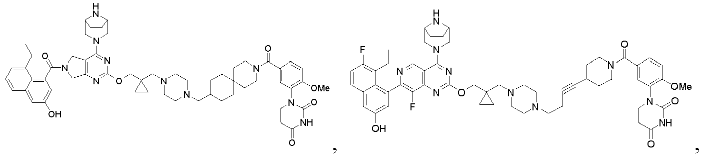 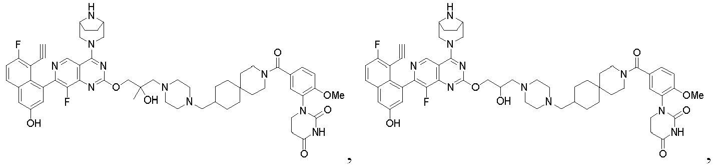 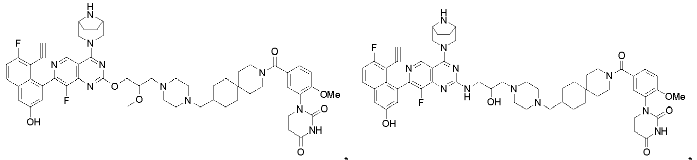 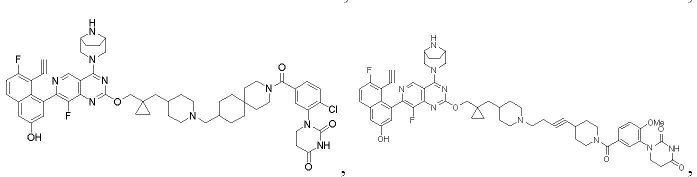 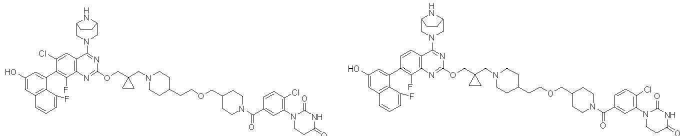 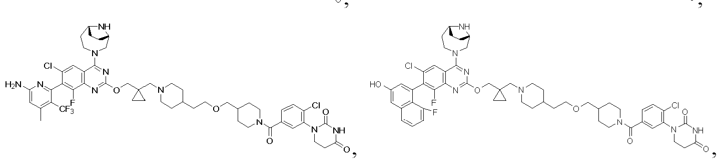 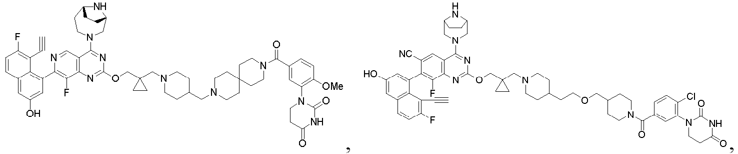 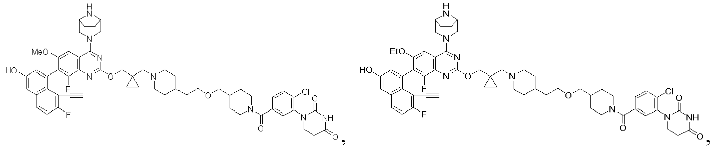 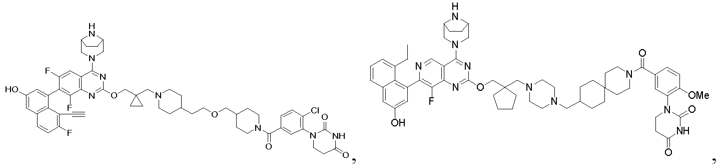 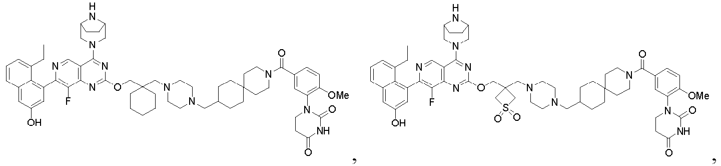 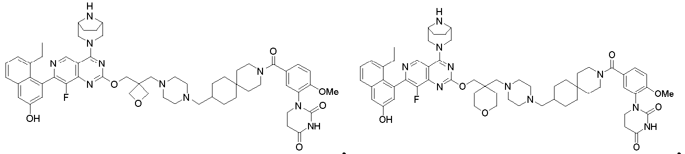 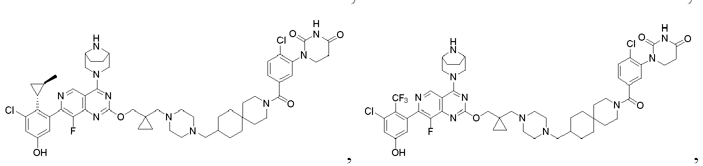 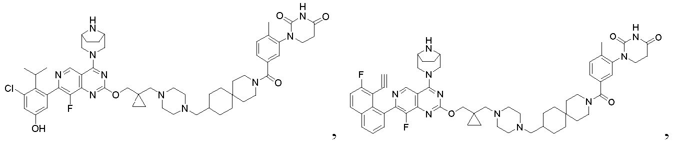 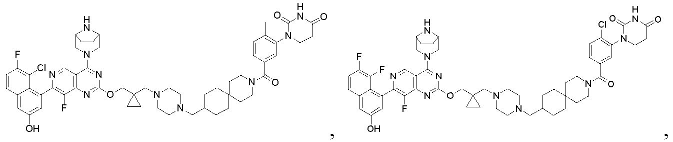 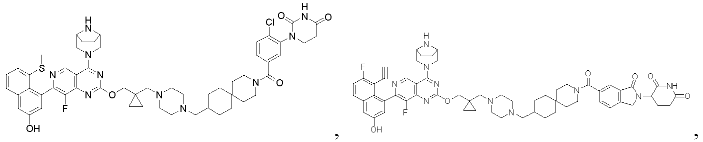 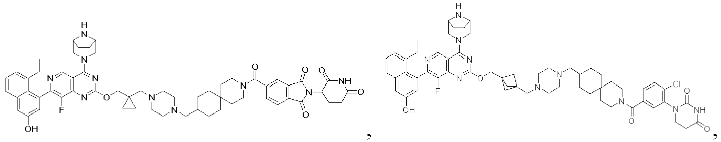 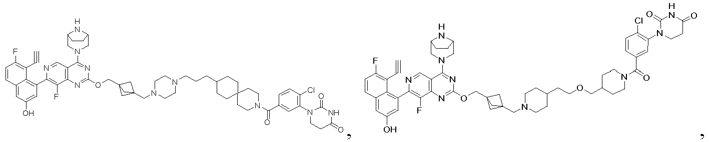 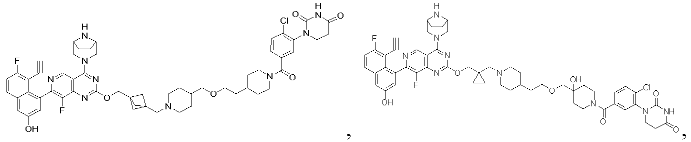 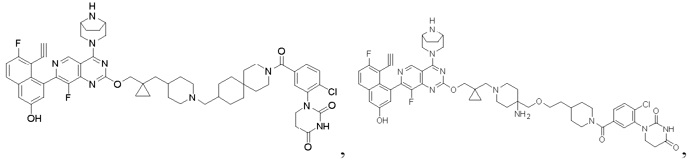 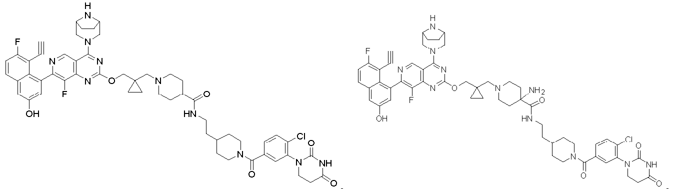 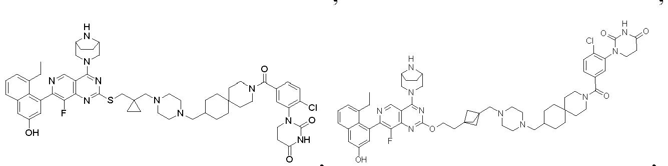 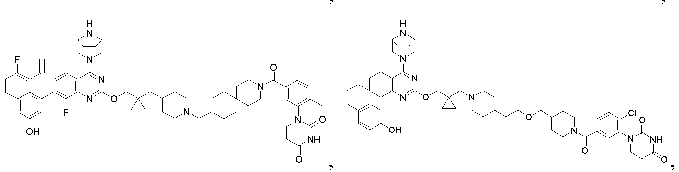 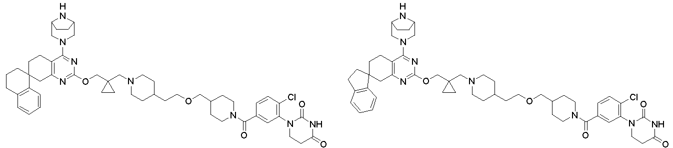 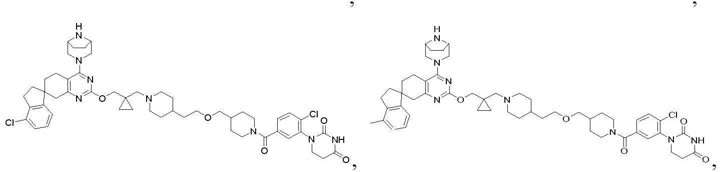 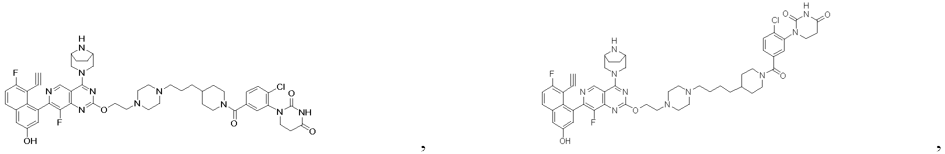 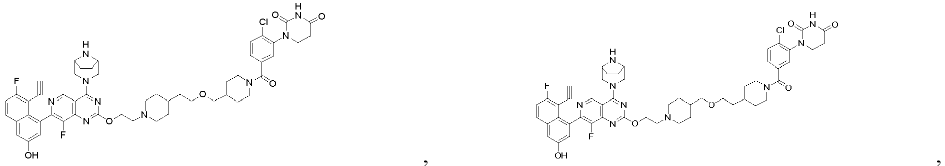 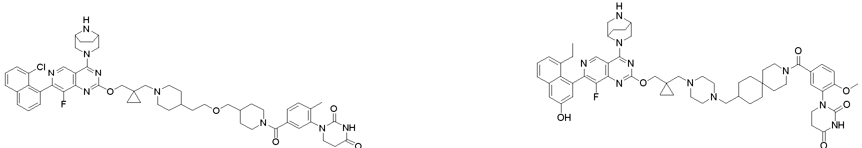 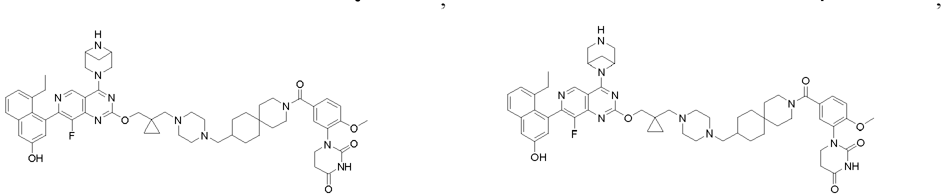 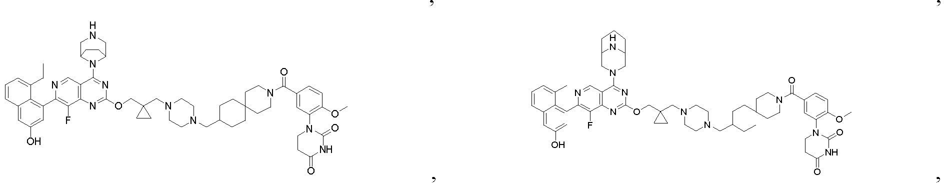 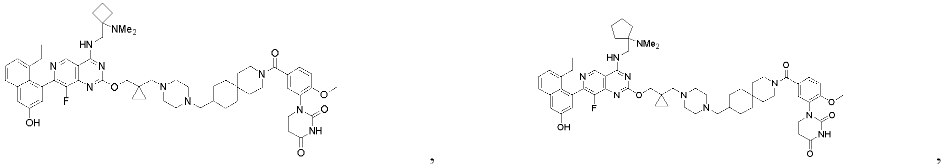 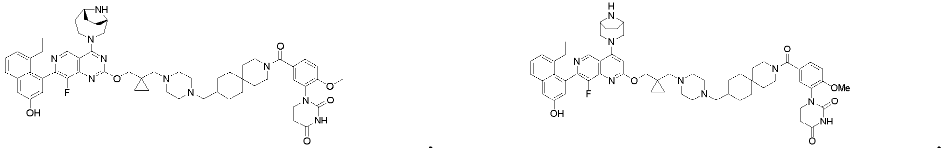 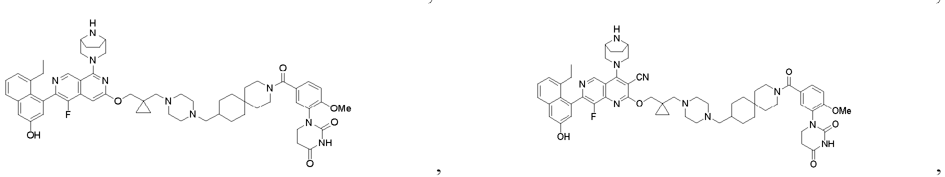 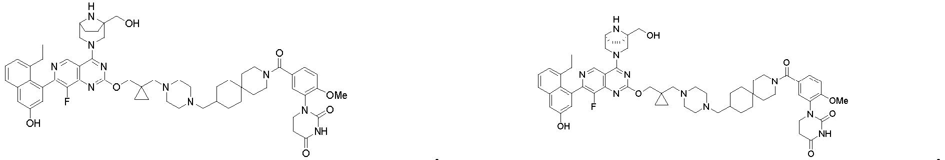 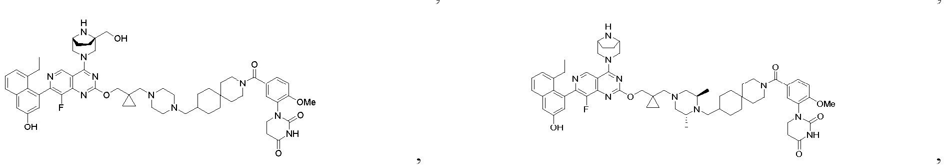 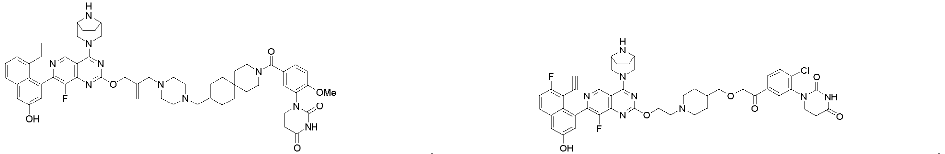 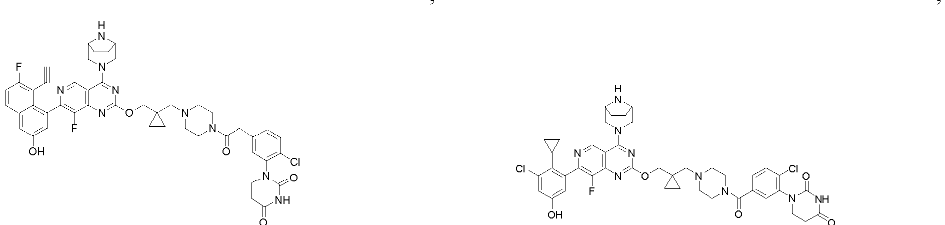 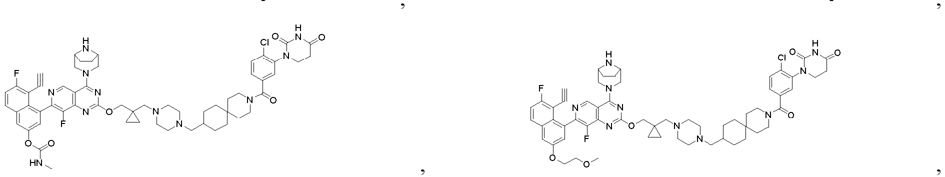 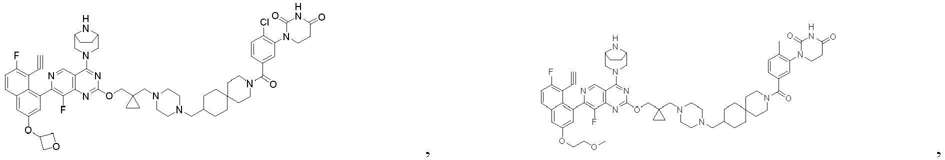 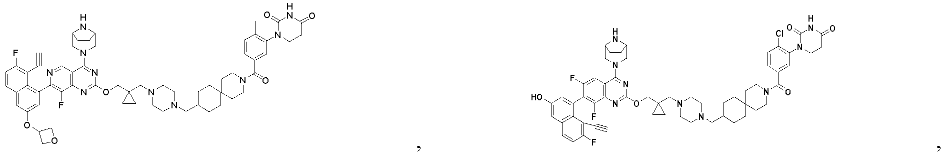 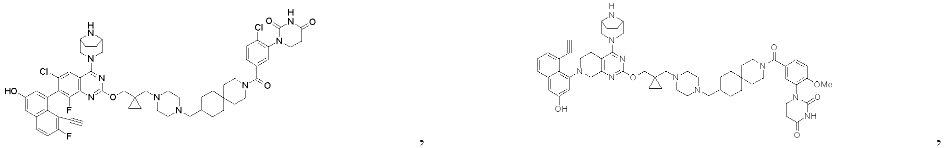 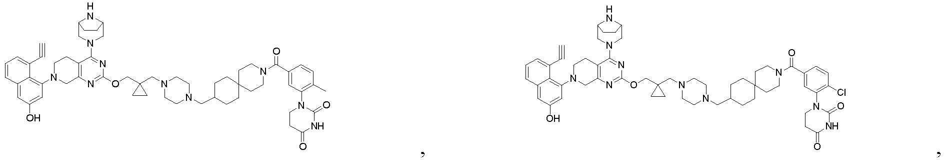 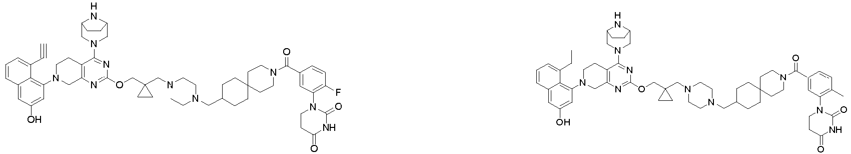 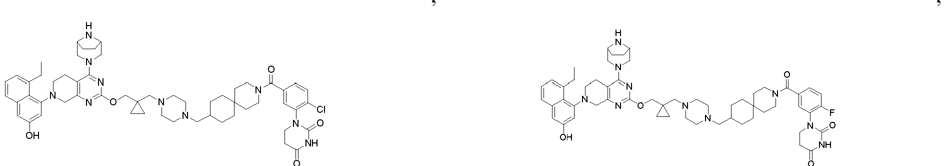 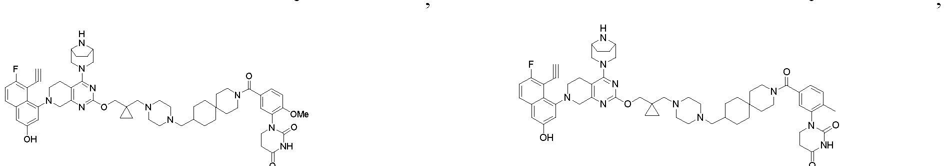 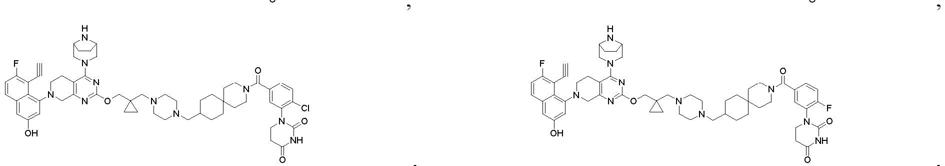 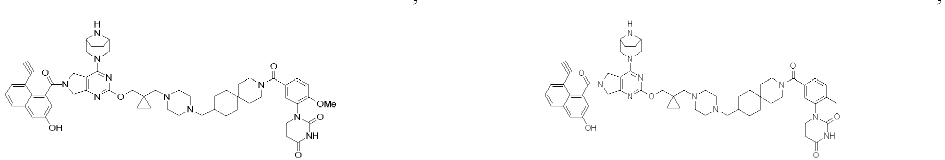 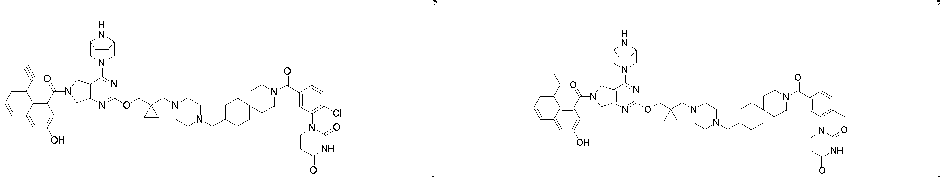 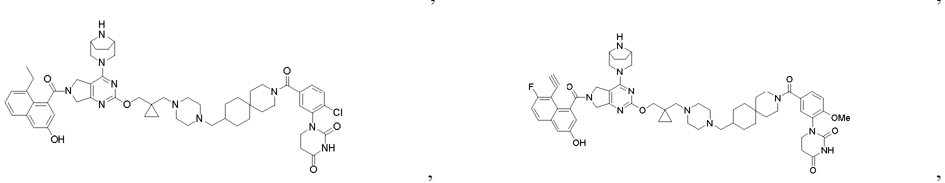 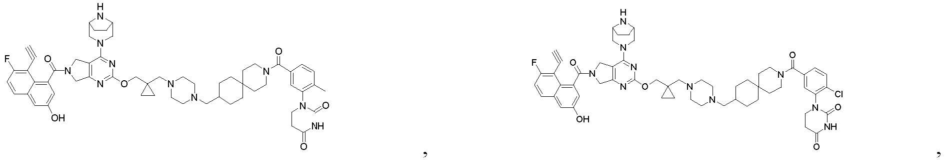 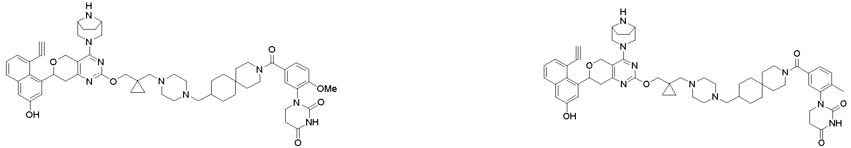 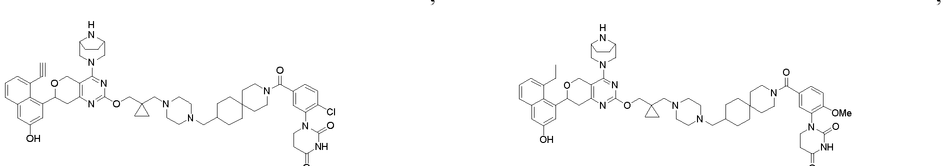 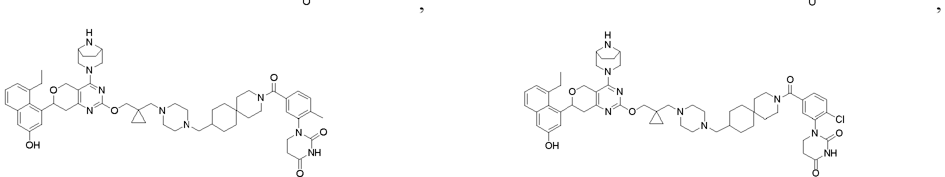 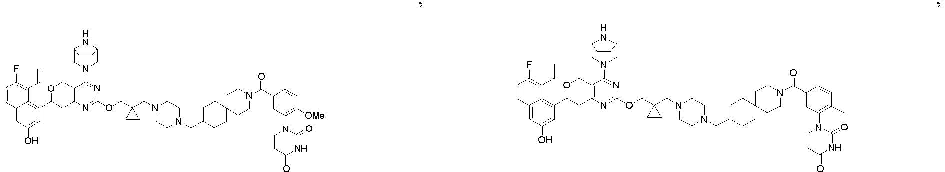 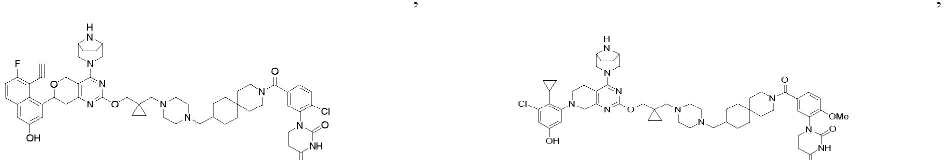 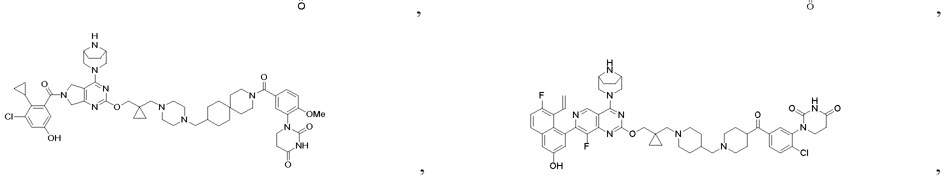 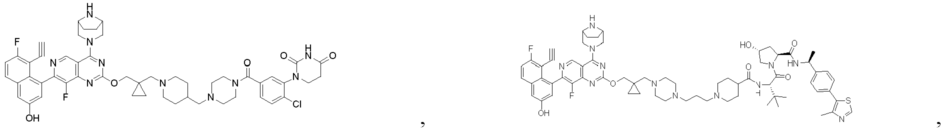 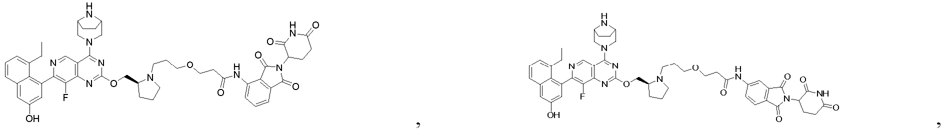 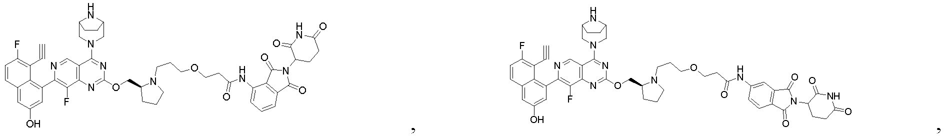 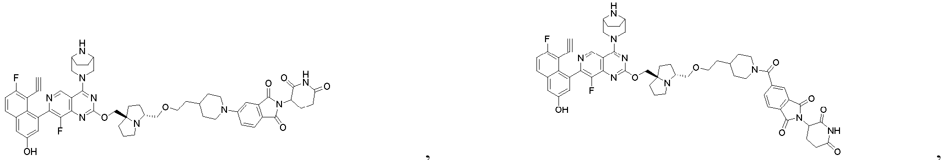 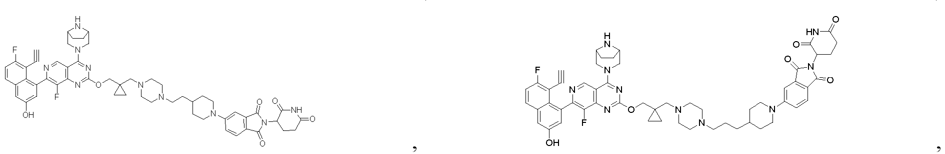 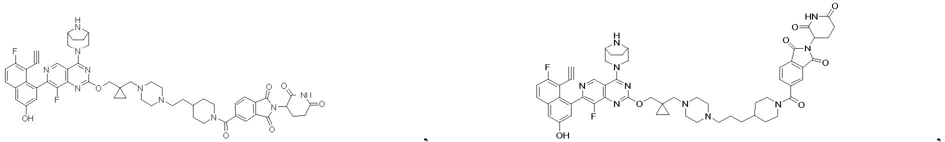 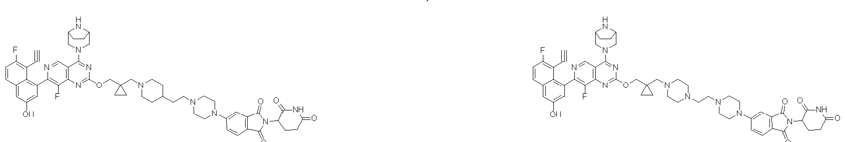 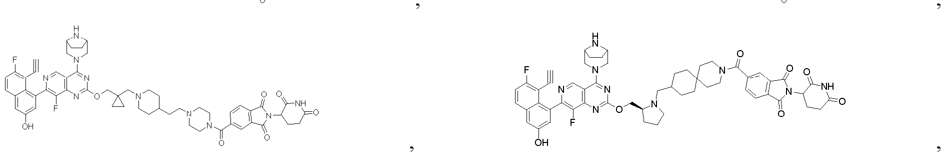 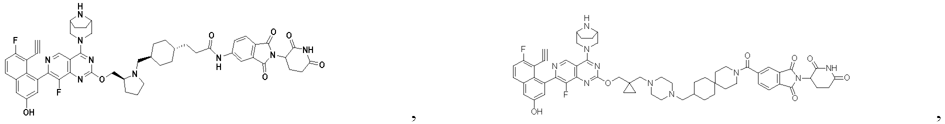 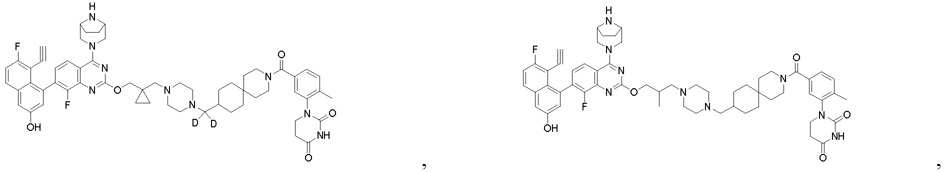 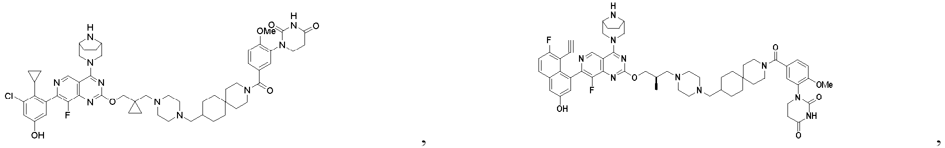 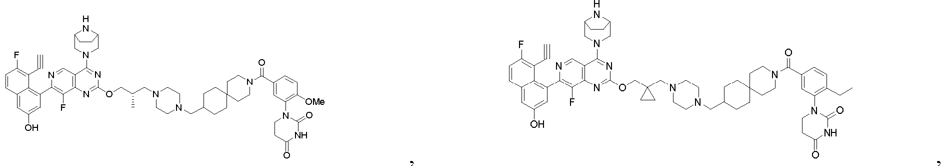 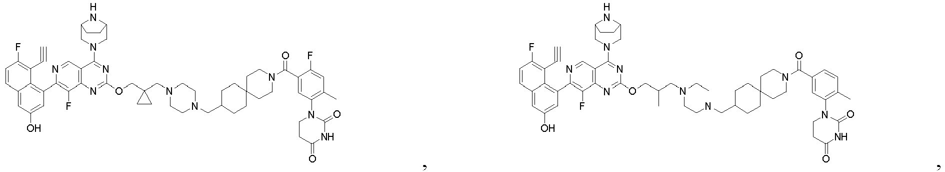 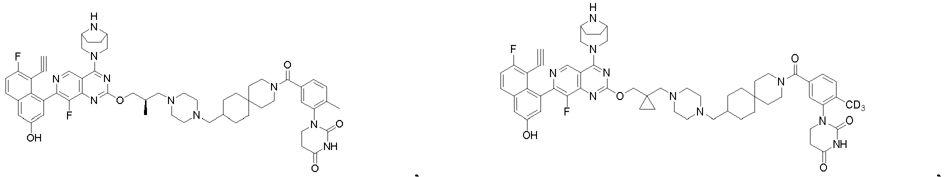 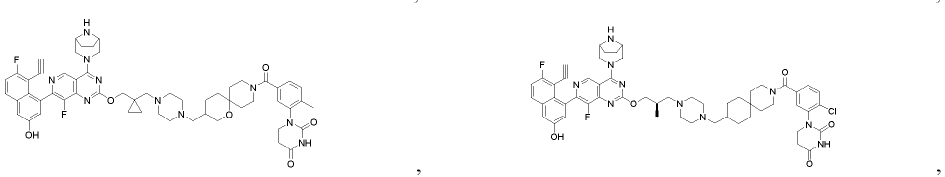 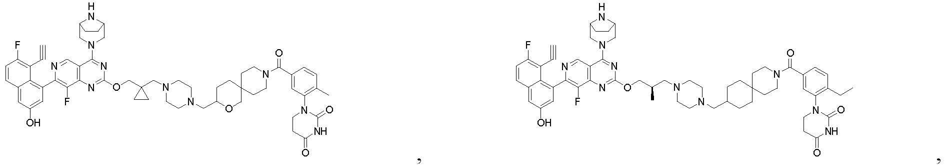 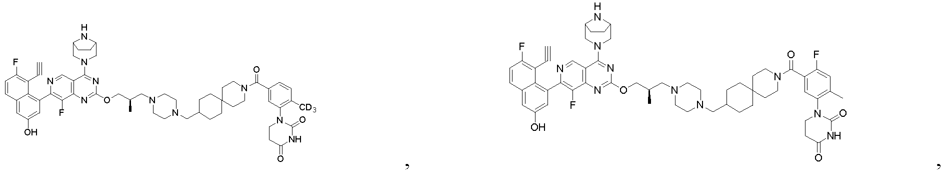 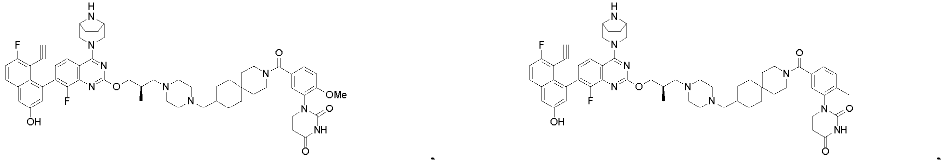 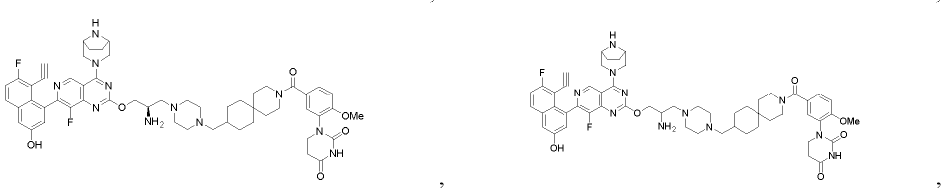 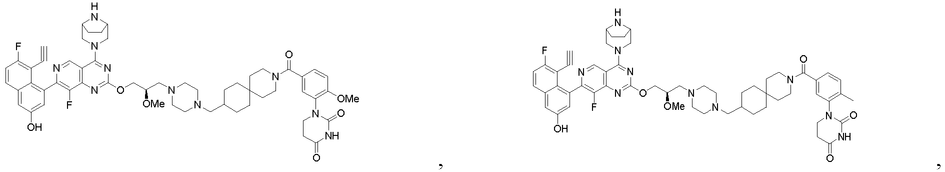 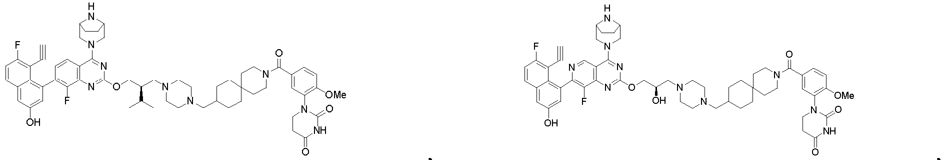 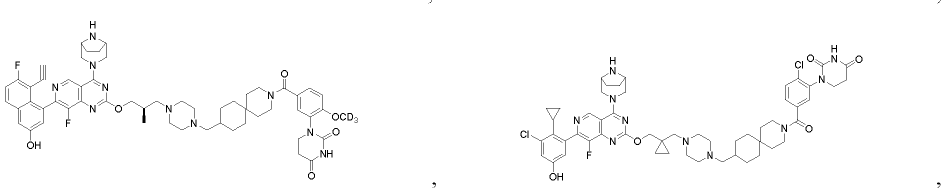 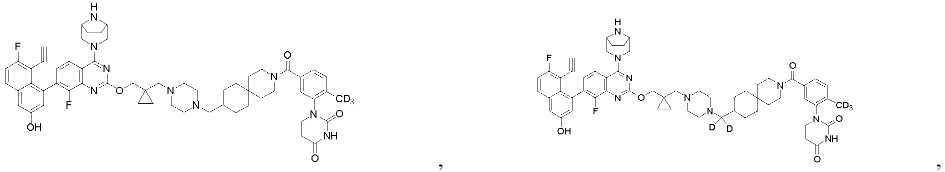 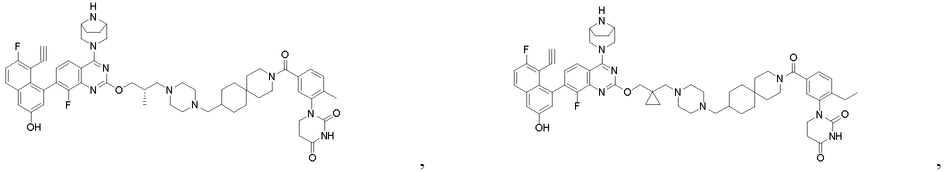 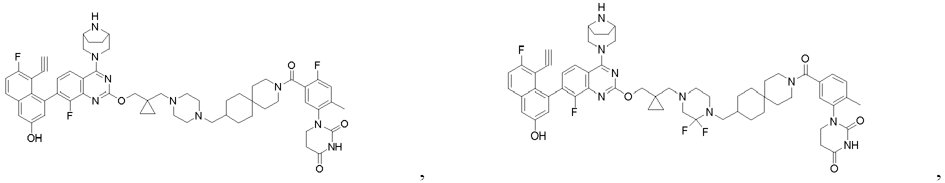 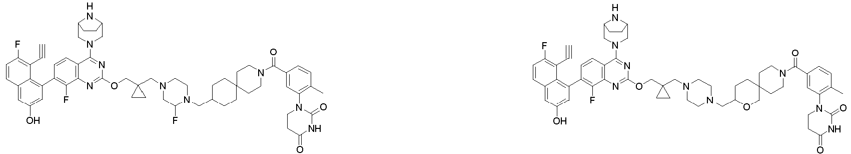 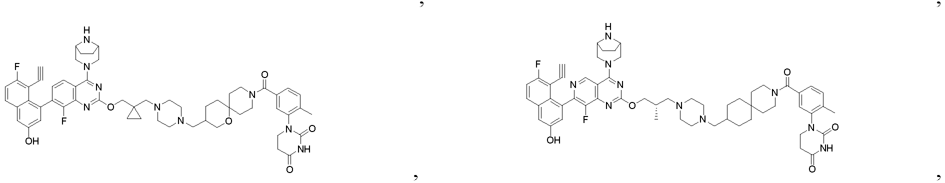 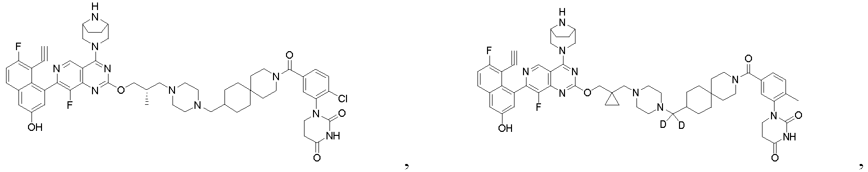 or

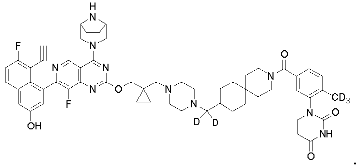

The present disclosure further provides a compound of formula W-1, W-2, W-3, W-4, W-5, INTA, INTA', INTC, or INTC': 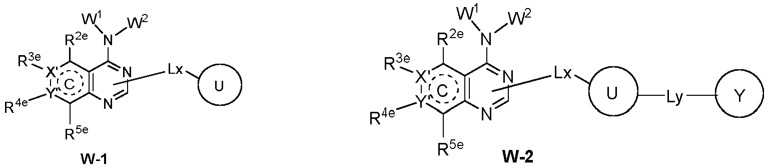 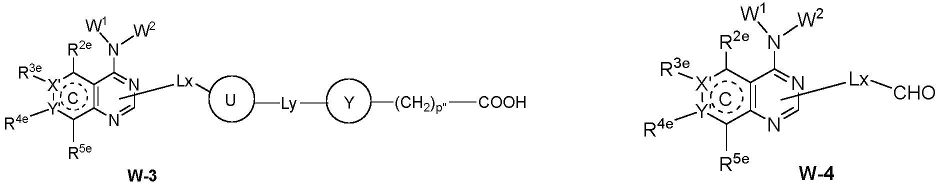 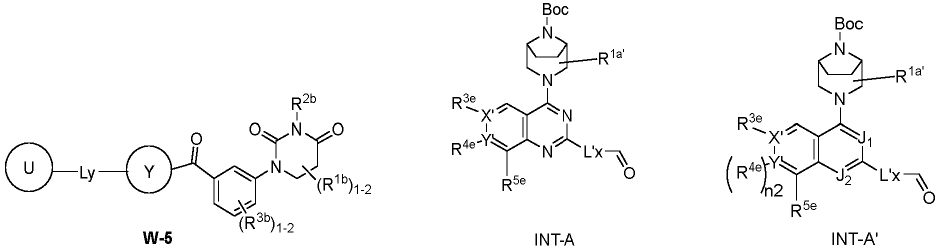 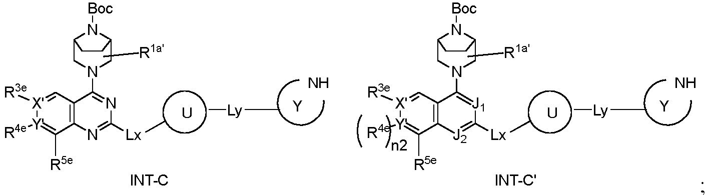 wherein ring C, R^{2e}, R^{3e}, R^{4e}, R^{5e}*,* R^{1b}, R^{2b}, R^{3b}, W¹, W², X', Y', J₁, J₂, L, and E are as defined and described in the present disclosure; Lx, ring U, Ly, and ring Y, as well as the linking relationships among them are as defined and described in the present disclosure; p" is 1, 2, 3, or 4; n2 is 1 or 2.

Preferably, formula W-1 is formula W-1-1, formula W-2 is formula W-2-1, formula W-3 is formula W-3-1, formula W-4 is formula W-4-1, or formula W-5 is formula W-5-1: 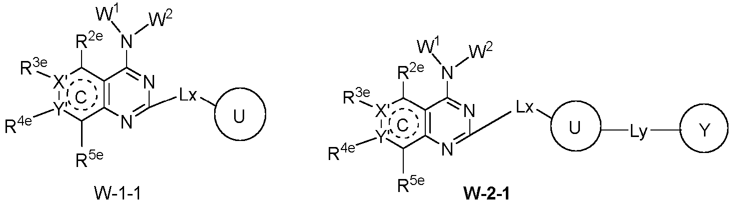 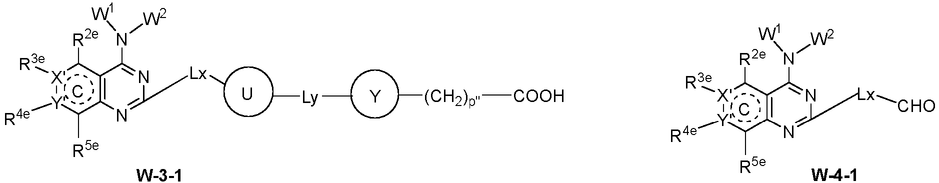 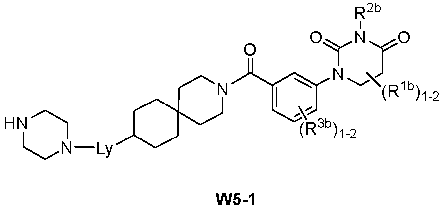 wherein ring C, R^{2e}, R^{3e}, R^{4e}, R^{5e}*,* R^{1b}, R^{2b}, R^{3b}, W¹, W², Lx, ring U, Ly, ring Y, and p" are as defined and described in the present disclosure.

Preferably, the compound of formula W-1, W-2, W-3, W-4, or W-5 is selected from:

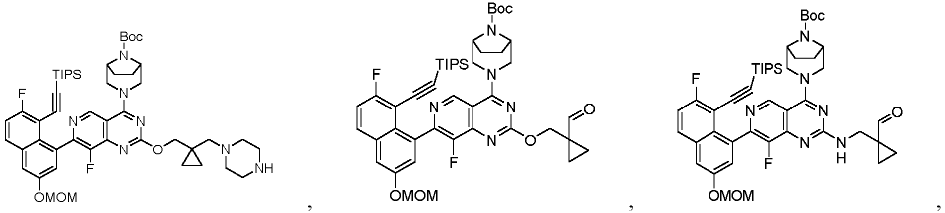

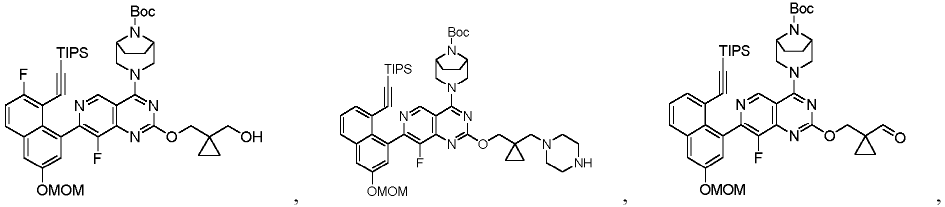

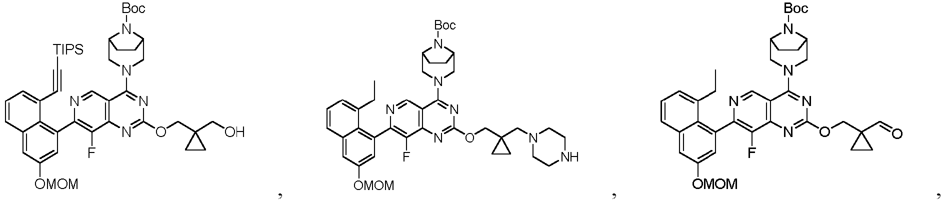

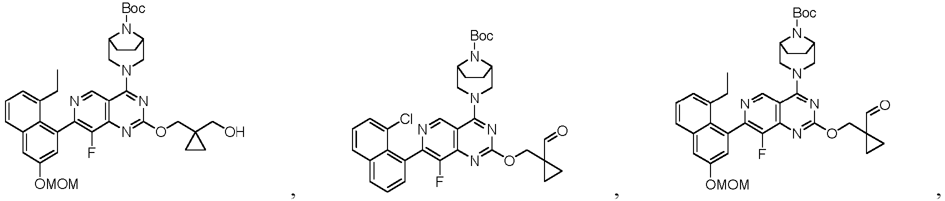

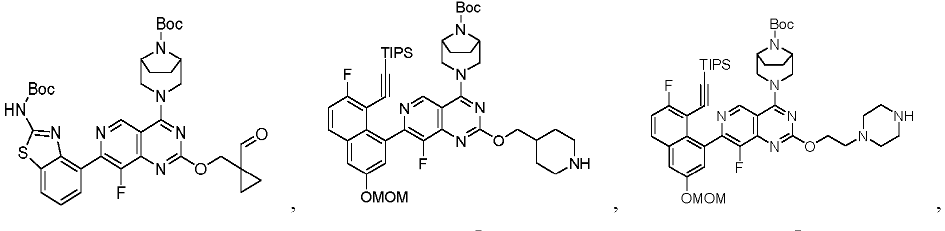

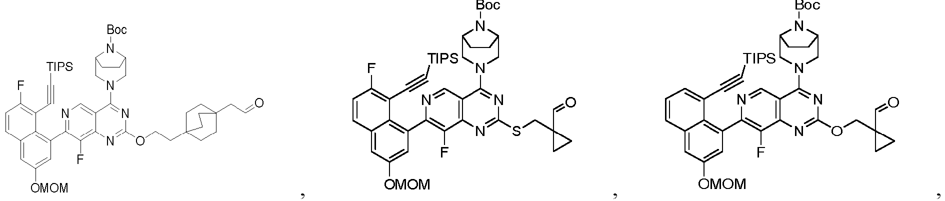

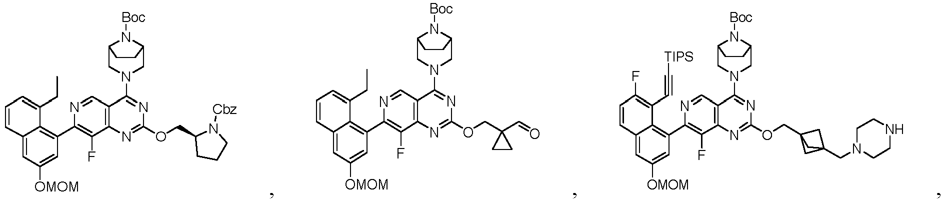

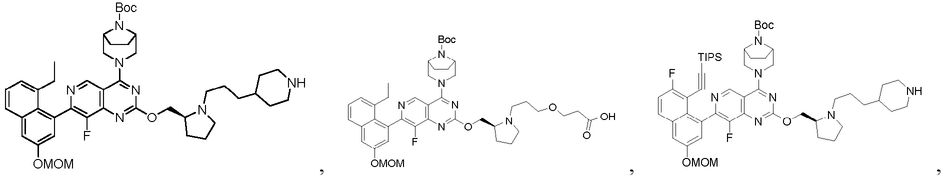

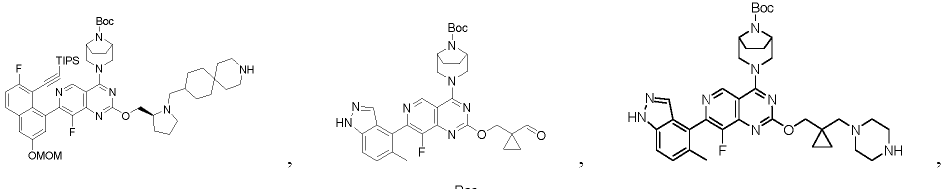

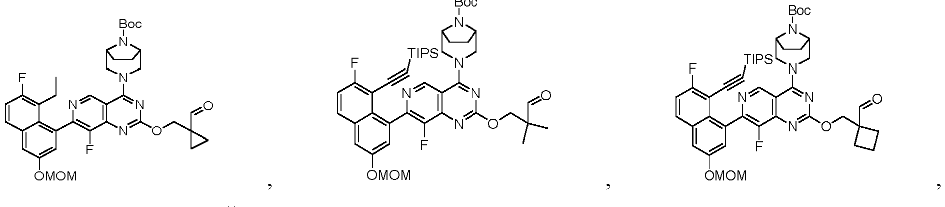

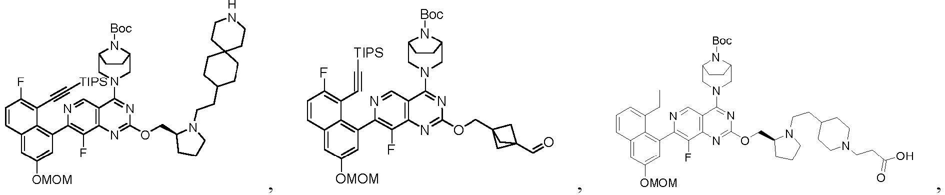

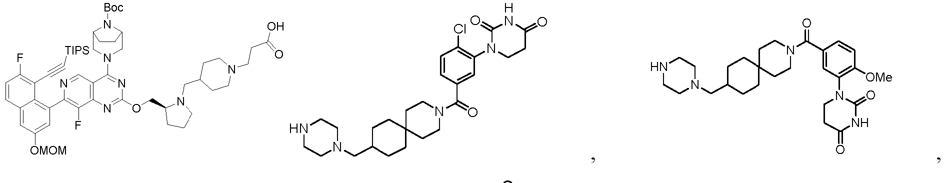

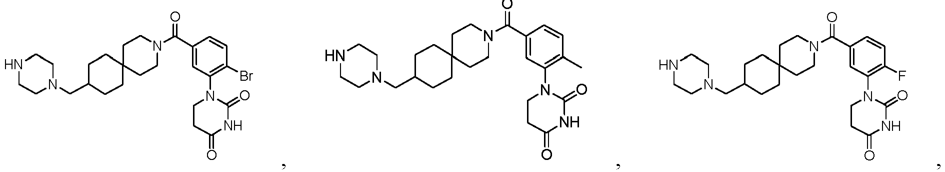

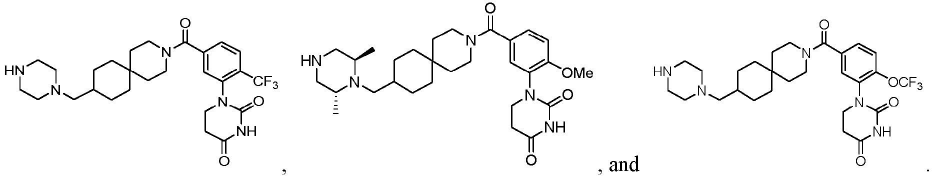

The present disclosure further provides a compound of formula II-1, formula II-2, or formula II-3: 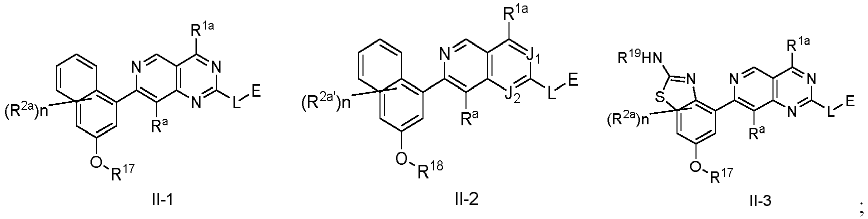 wherein each R^{1a} is independently 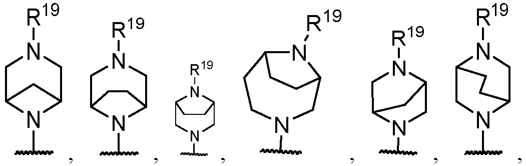 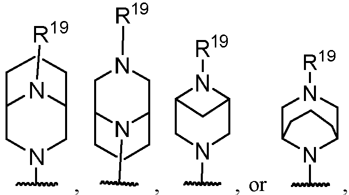 and each R¹⁹ is independently an amino-protecting group (e.g., - Boc); R¹⁷ and R¹⁸ are each independently a hydroxy-protecting group (e.g., -MOM); J1, J2, L, and E are as defined above.

The compound of formula II-1, formula II-2, or formula II-3 is preferably any one of the following compounds:

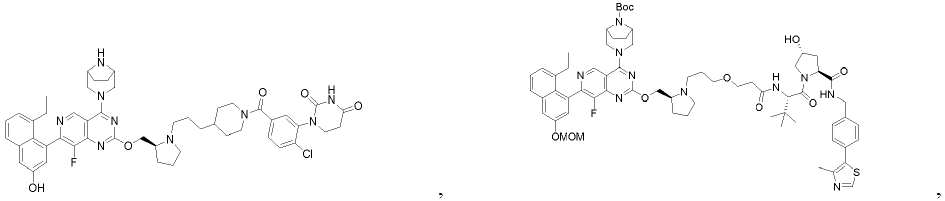

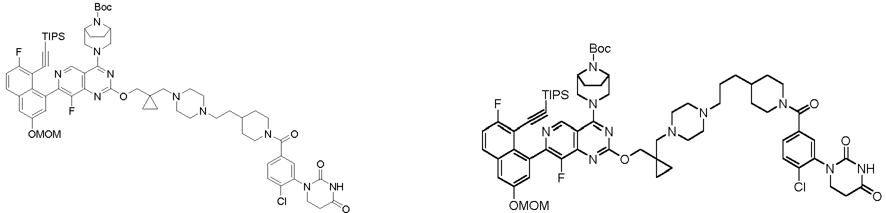

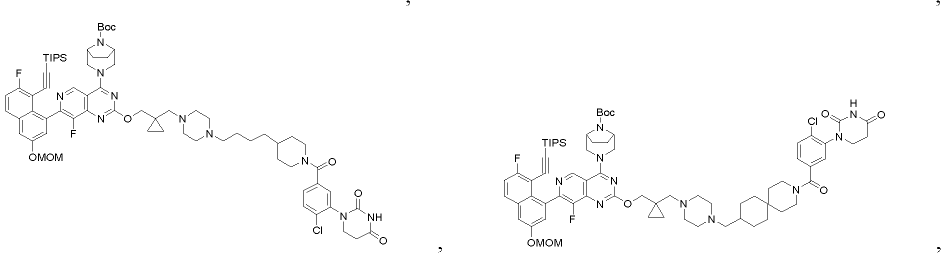

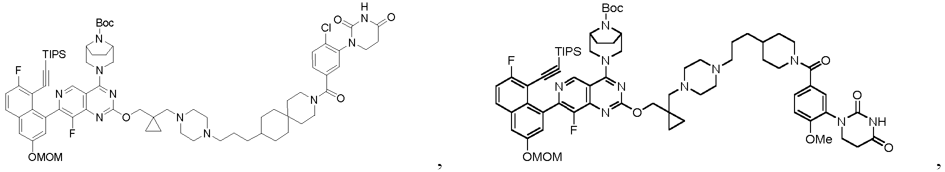

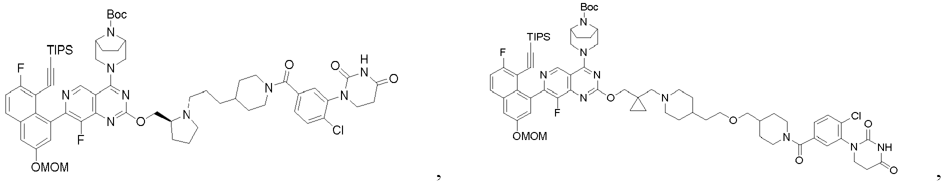

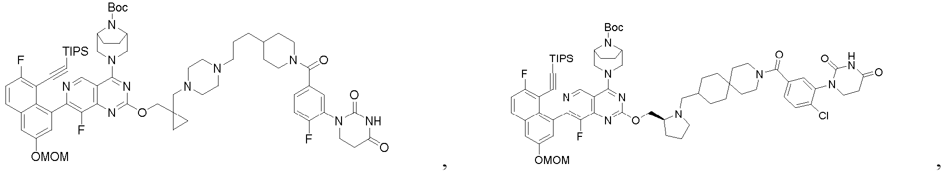

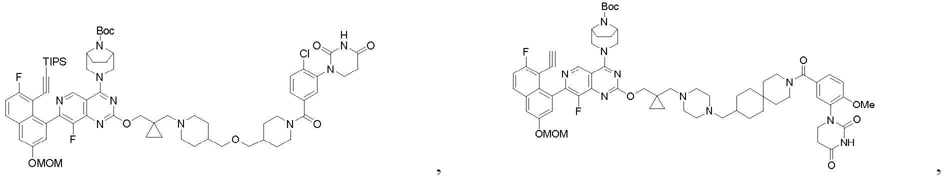

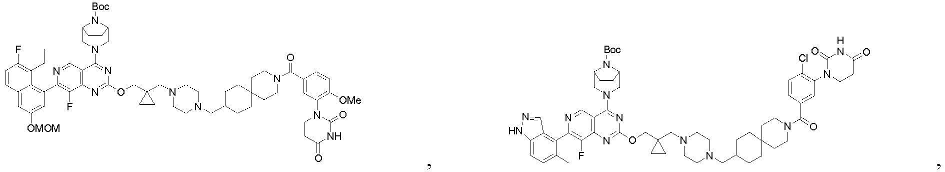

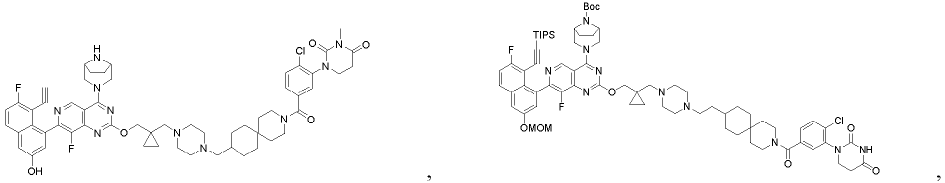

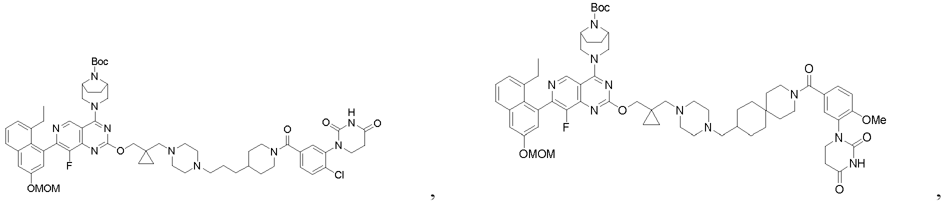

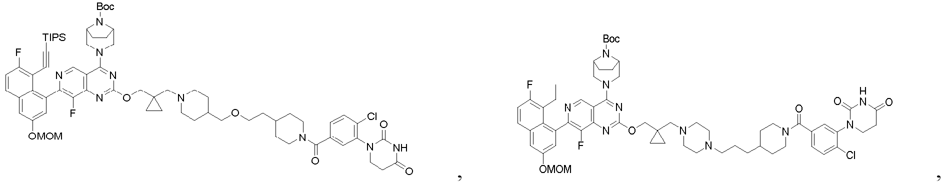

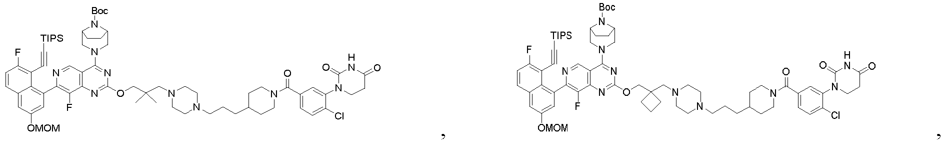

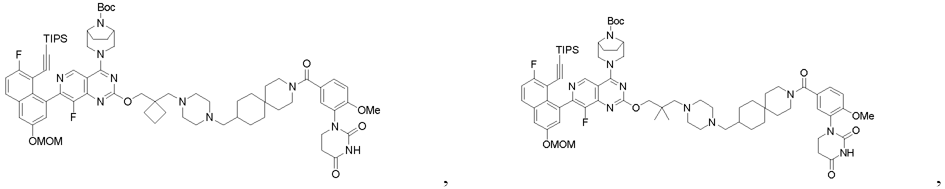

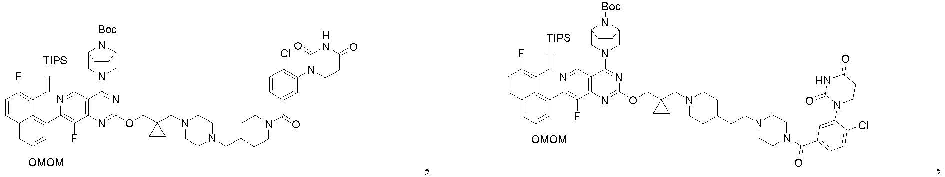

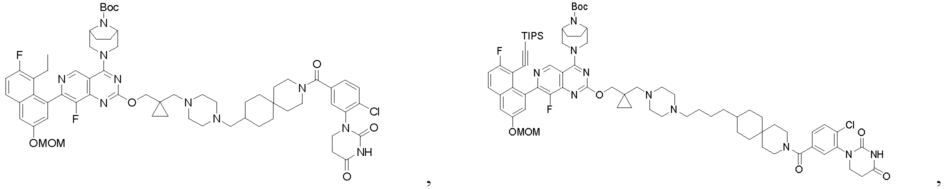

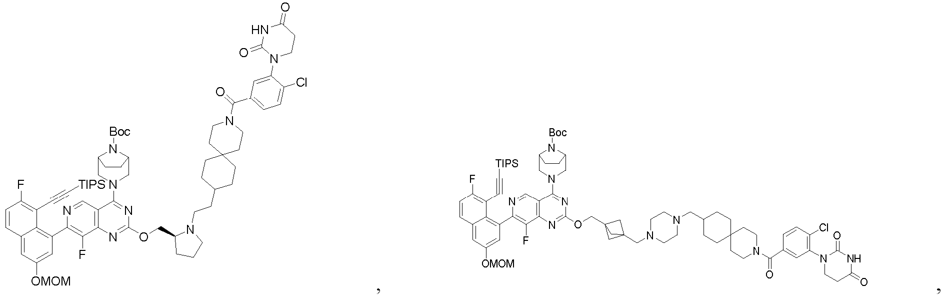

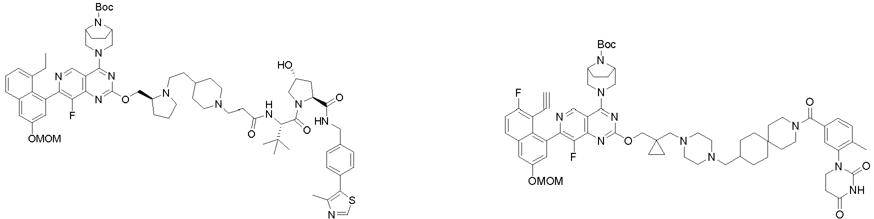

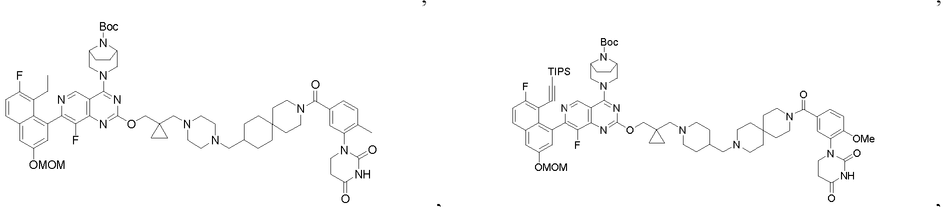

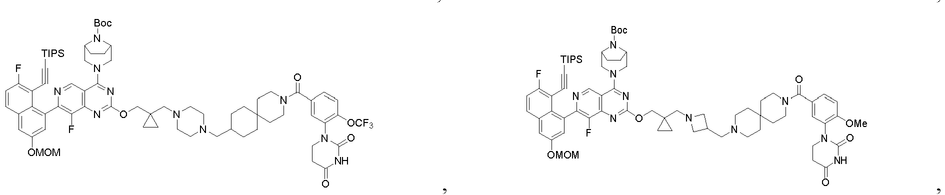

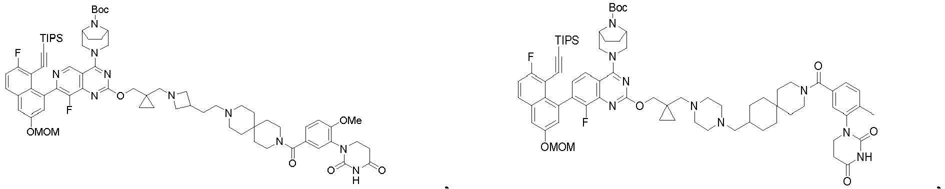

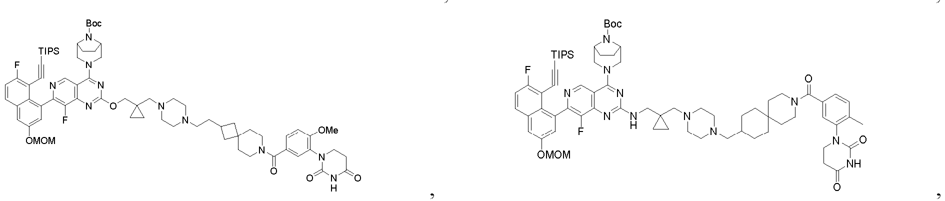

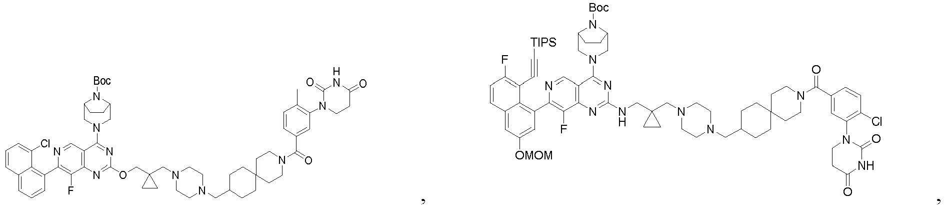

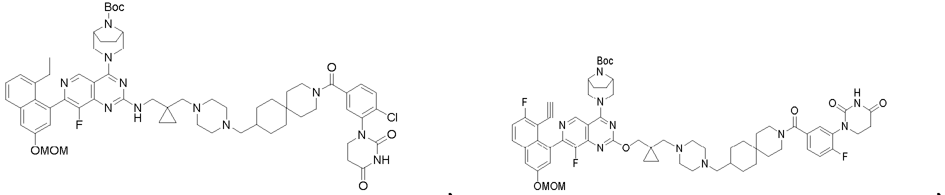

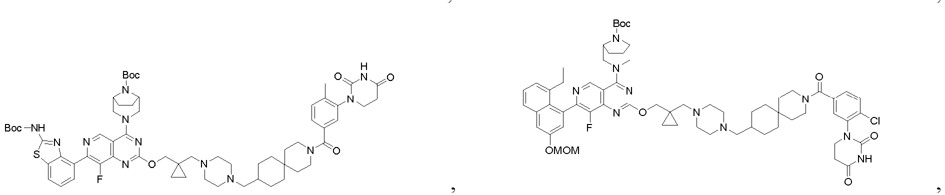

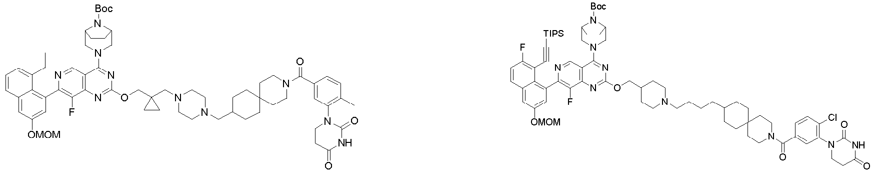

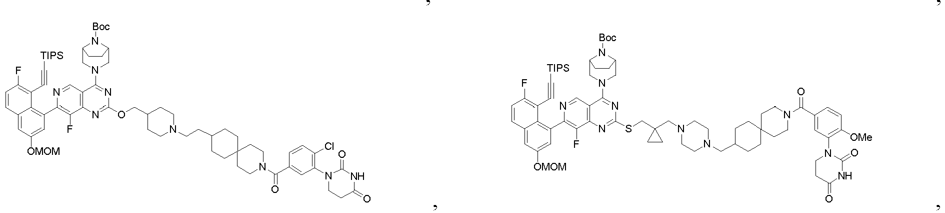

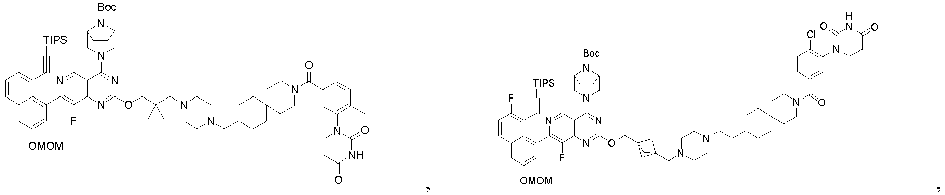

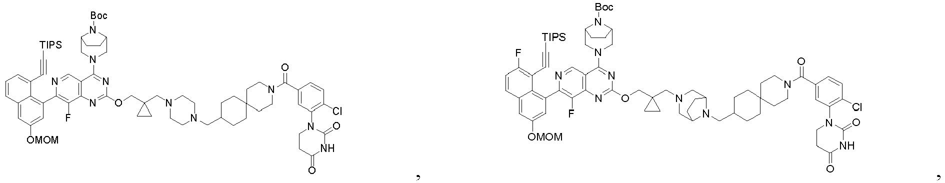

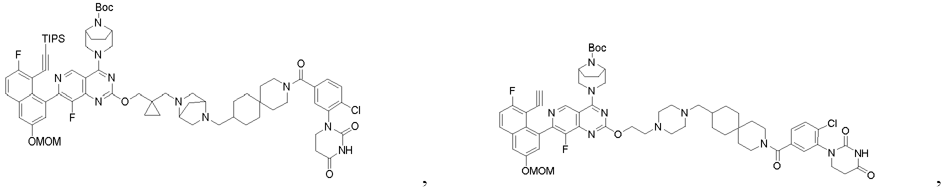

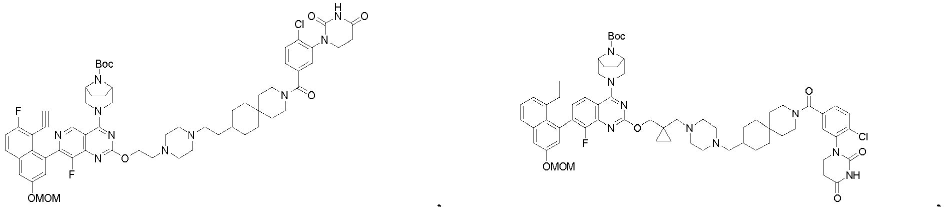

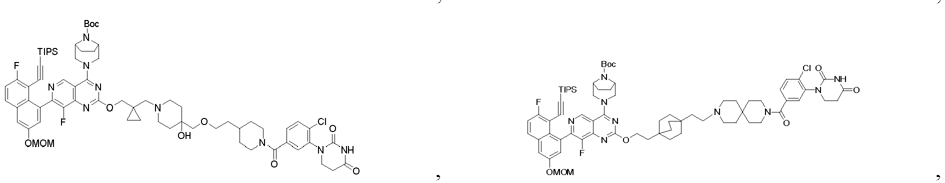

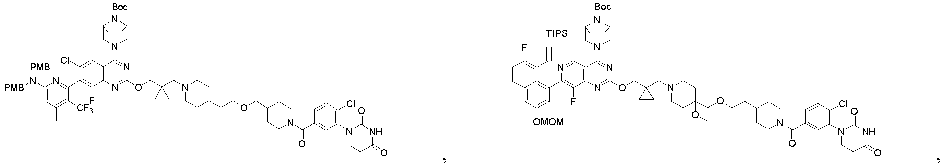

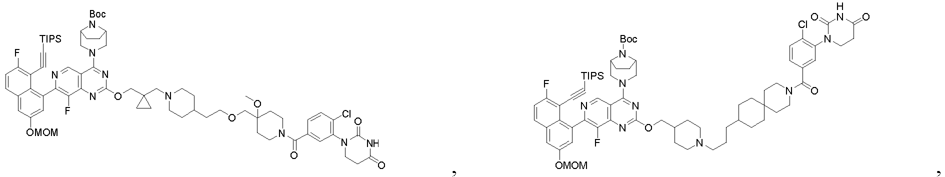

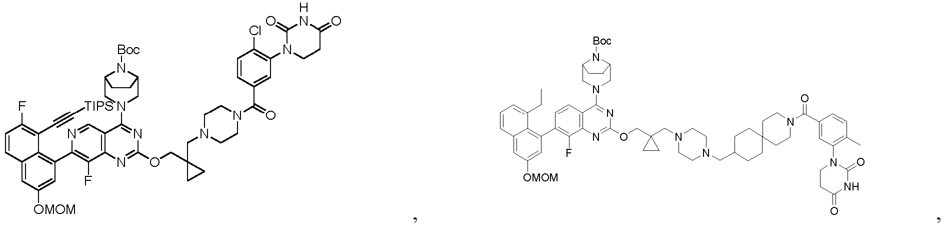

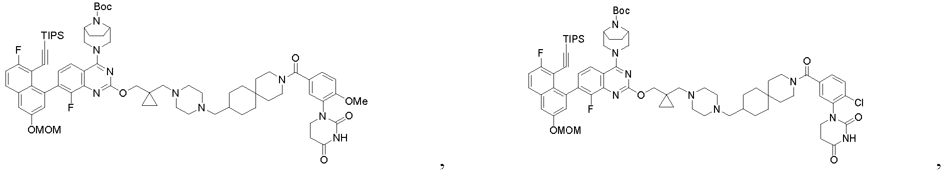

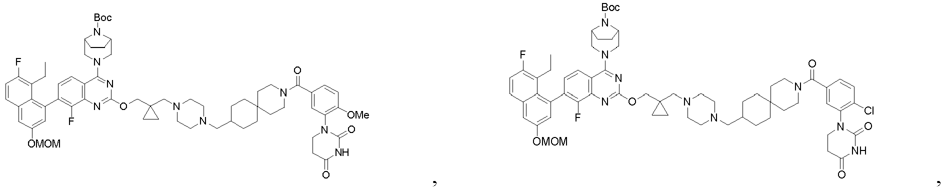

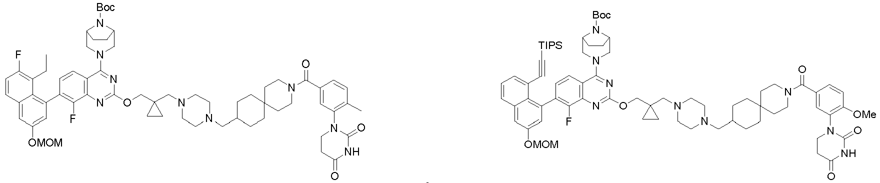

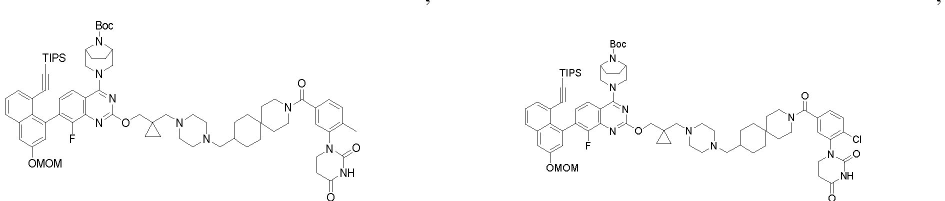

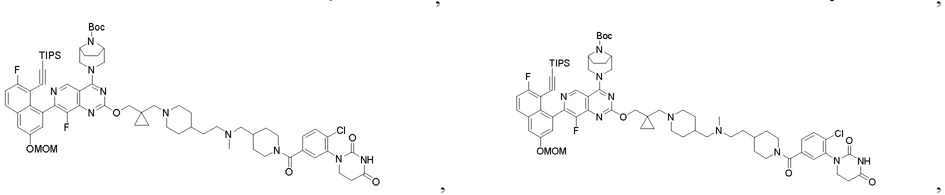

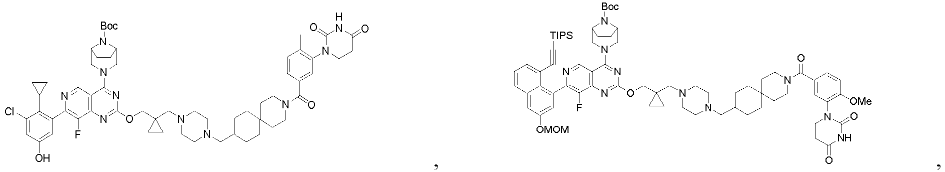

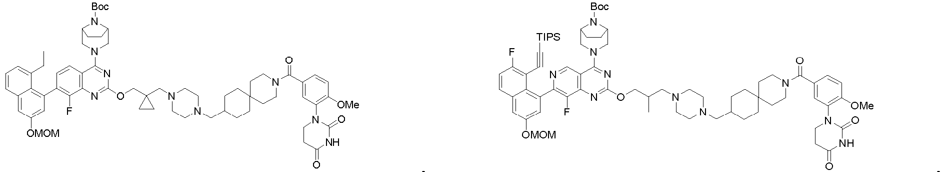

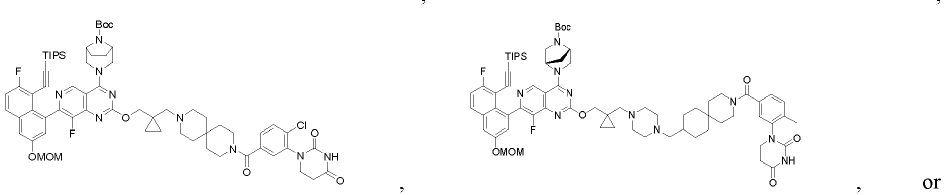

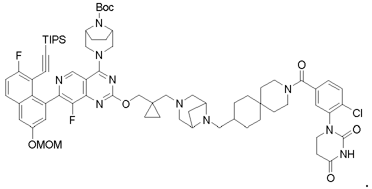

The present disclosure provides a method for preparing the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof.

The present disclosure provides a pharmaceutical composition comprising a therapeutically effective amount of the compound of formula I or I', and/or the stereoisomer, the enantiomer, the diastereomer, the atropisomer, the deuteride, the hydrate, the solvate or the prodrug thereof and/or the pharmaceutically acceptable salt thereof, and a pharmaceutically acceptable carrier, diluent, or excipient.

The present disclosure provides a method for degrading KRAS G12D protein, comprising contacting the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof with the KRAS G12D protein.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof as a medicament for the treatment or prevention of a KRAS G12D-mediated disease or disorder.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof as a medicament for the treatment or prevention of a disease or disorder mediated by a KRAS mutation.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of a disease or disorder mediated by a KRAS mutation.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of a disease or disorder (cancer) caused by the interaction of KRAS G12D with SOS1 or SHP2 protein.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of cancer.

The present disclosure provides use of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof in preparing a medicament for the treatment or prevention of pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer, and sarcoma (e.g., the disease described above is a disease or disorder caused by the interaction of KRAS G12D with SOS1 or SHP2 protein).

The present disclosure provides a method for treating or preventing a KRAS G12D-mediated disease or disorder, comprising administering to a patient in need thereof a therapeutically effective amount of the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof.

The present disclosure provides a method for treating or preventing a disease or disorder (cancer) regulated by the interaction of KRAS G12D with SOS1 or SHP2 protein, comprising administering to a patient in need thereof a therapeutically effective amount of the compound of formula I or I' or I', and/or the stereoisomer, the enantiomer, the diastereomer, the atropisomer, the deuteride, the hydrate, the solvate, the metabolite or the prodrug thereof and/or the pharmaceutically acceptable salt thereof, or the pharmaceutical composition thereof.

In certain embodiments of the present disclosure, the cancer is selected from:
heart: sarcoma (angiosarcoma, fibrosarcoma, rhabdomyosarcoma and liposarcoma), myxoma, rhabdomyoma, fibroma, lipoma and teratoma;
lung: bronchial cancer (squamous cell carcinoma, undifferentiated small cell carcinoma, undifferentiated large cell carcinoma and adenocarcinoma), bronchioloalveolar carcinoma (bronchiolar carcinoma), bronchial adenoma, sarcoma, lymphoma, chondroma, hamartoma and mesothelioma;
gastrointestinal tract: esophagus cancer (squamous cell carcinoma, adenocarcinoma, leiomyosarcoma, and lymphoma), stomach (cancer, lymphoma, and leiomyosarcoma), pancreas (ductal adenocarcinoma, insulinoma, glucagonoma, gastrinoma, carcinoid tumor, and vipoma), small intestine (adenocarcinoma, lymphoma, carcinoid tumor, Kaposi's sarcoma, leiomyoma, hemangioma, lipoma, neurofibroma, and fibroma), and large intestine (adenocarcinoma, tubular adenoma, villous adenoma, hamartoma, and leiomyoma);
genitourinary system: kidney (adenocarcinoma, embryonal carcinosarcoma (Wilms' tumor), lymphoma, and leukemia), bladder and urinary tract (squamous cell carcinoma, transitional cell carcinoma, and adenocarcinoma), prostate (adenocarcinoma and sarcoma), and testis (seminoma, teratoma, embryonal carcinoma, teratoma, choriocarcinoma, sarcoma, interstitial cell carcinoma, fibroma, fibroadenoma, adenomatoid tumor, and lipoma);
liver: liver cancer (hepatocellular carcinoma), intrahepatic cholangiocarcinoma, hepatoblastoma, malignant hemangioendothelioma, hepatocellular adenoma, and hemangioma;
biliary tract: cholangiocarcinoma, gallbladder cancer, ampullary cancer, and intrahepatic cholangiocarcinoma;
bone: osteogenic sarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma, and giant cell tumor;
nervous system: skull (osteoma, hemangioma, granuloma, xanthoma, and osteitis deformans), meninges (meningioma, meningosarcoma, and glioma), brain (astrocytoma, medulloblastoma, glioma, ependymoma, germinal cell tumor (pinealoma), glioblastoma multiforme, oligodendroglioma, neurilemmoma, eye cancer, and congenital tumor), spinal neurofibroma, meningioma, glioma, and sarcoma);
gynaecology and obstetrics: uterus (endometrial cancer (serous cystadenocarcinoma, mucinous cystadenocarcinoma, and unclassified cancer), granulosa theca cell tumor, ovarian Sertoli-Leydig cell tumor, dysgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, botulinum sarcoma (embryonal rhabdomyosarcoma), and fallopian tube (cancer);
hematology: blood (myeloid leukemia (acute and chronic), acute lymphoblastic leukemia, chronic lymphocytic leukemia, myeloproliferative disease, multiple myeloma, and myelodysplastic syndrome), Hodgkin's disease, and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid, and psoriasis; and
adrenal gland: neuroblastoma.

In certain embodiments of the present disclosure, the cancer is a KRAS G12D-associated cancer.

In certain embodiments of the present disclosure, the cancer is non-small cell lung cancer, small cell lung cancer, colorectal cancer, rectal cancer, or pancreatic cancer.

The present disclosure further provides the compound of formula I or I' or the stereoisomer thereof, the atropisomer thereof, the pharmaceutically acceptable salt thereof, the pharmaceutically acceptable salt of the stereoisomer thereof, or the pharmaceutically acceptable salt of the atropisomer thereof described above for use in the treatment of a disease or disorder associated with KRAS G12D mutant protein.

The present disclosure further provides the compound of formula I or I' described above, wherein the disease or disorder associated with the KRAS G12D mutant protein is a cancer associated with the KRAS G12D mutant protein.

In certain embodiments of the present disclosure, the cancer is selected from pancreatic cancer, colorectal cancer, endometrial cancer, and lung cancer.

In certain embodiments of the present disclosure, the lung cancer is selected from non-small cell lung cancer and small cell lung cancer.

The present disclosure provides a method for preparing the compound of formula I or I', and/or the stereoisomer, the enantiomer, the diastereomer, the atropisomer, the deuteride, the hydrate, the solvate, the metabolite or the prodrug thereof and/or the pharmaceutically acceptable salt thereof: when G is 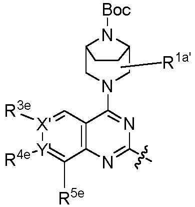 , L is LA 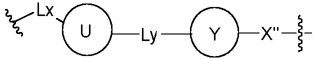 , and ring U contains an NH group, the preparation method is: 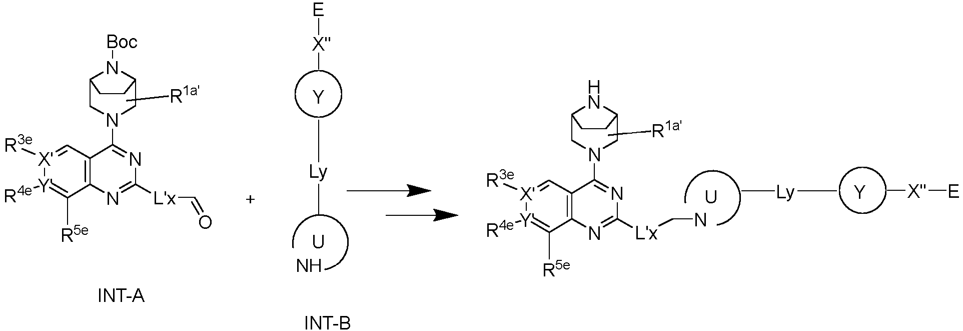
when G1' is 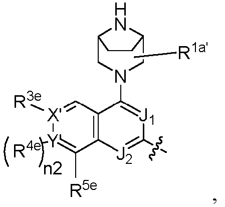 L is LA 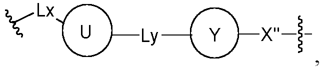 and ring U contains an NH group, the preparation method is: 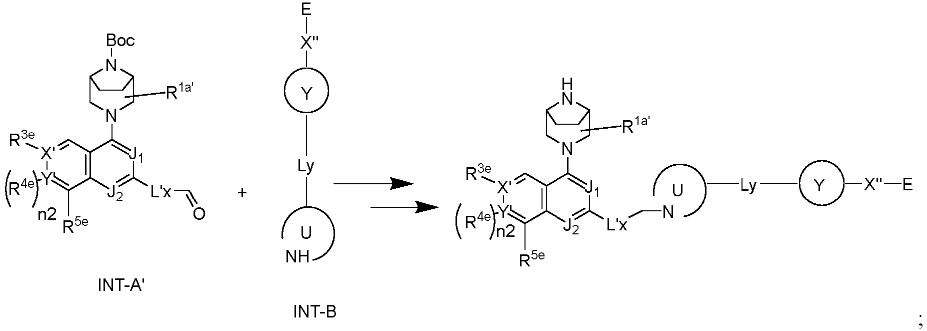
subjecting INT-A (INT-A') and INT-B to a reductive amination reaction to give the target compound, wherein the reductant for the reductive amination includes, but is not limited to, Pd/C, sodium borohydride, sodium cyanoborohydride, borane, and sodium triacetoxyborohydride, wherein 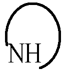 refers to ring U containing an NH group; R^{3e}, R^{4e}, R^{5e}*,* X', Y', n2, and R^{1a'} are as defined and described in G1-I-a1; 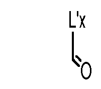 refers to Lx containing an aldehyde group; ring U, ring Y, X", Lx, and Ly are as defined in LA; E is as defined and described in the present disclosure, and preferably, E is E1-1a, E1-1b, E1-1c, E1-1d, E1-1e, E1-1f, E1-1g, E1-1aa, E E1-1h, 1-1bb, E1-1cc, E1-1dd, E1-1ee, E1-1ff, E1-1gg, or E1-1hh.

When G is 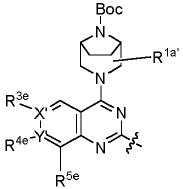 , L is LA 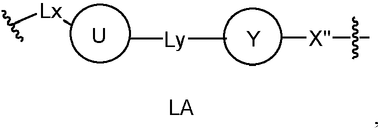 ring Y contains an NH group, and X" is C(O), the preparation method is: 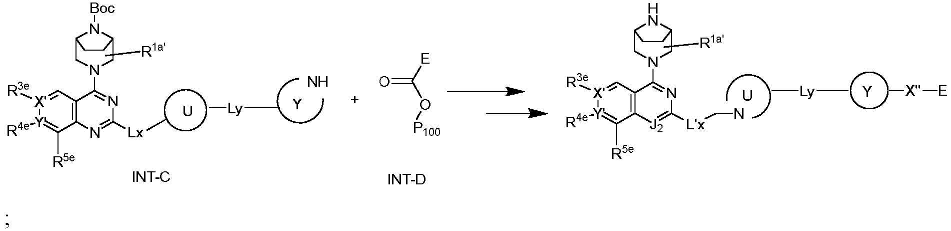
when G1' is 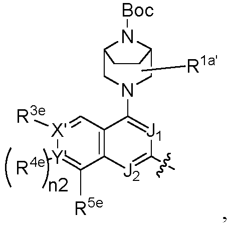 L is LA 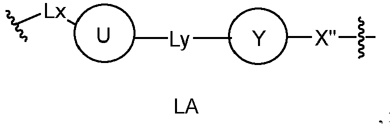 ring Y contains an NH group, and X" is C(O), the preparation method is: 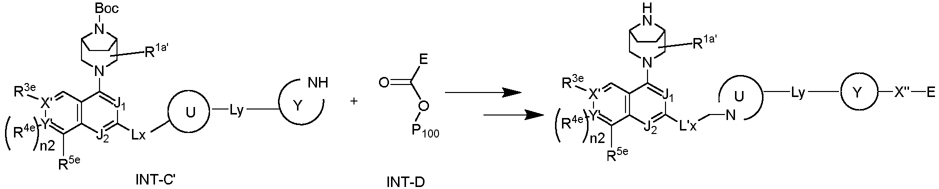
subjecting INT-C (or INT-C') and INT-D to a substitution reaction under an alkaline condition to give the target compound, wherein the base includes, but is not limited to, triethylamine, *N,N-*diisopropylethylamine, potassium carbonate, sodium carbonate, and sodium bicarbonate, wherein 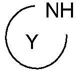 refers to ring Y containing an NH group; P₁₀₀ is pentafluorophenyl or *p*-nitrophenyl; R^{3e}, R^{4e}, R^{5e}*,* X', Y', n2, and R^{1a'} are as defined and described in G1-I-a1; ring U, ring Y, X", Lx, and Ly are as defined in LA; E is as defined and described in the present disclosure, and preferably, E is E1-1a, E1-1b, E1-1c, E1-1d, E1-1e, E1-1f, E1-1g, E1-1aa, E E1-1h, 1-1bb, E1-1cc, E1-1dd, E1-1ee, E1-1ff, E1-1gg, or E1-1hh.

When G is of other structures in the specification and L is LA 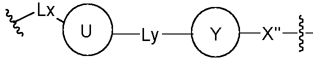 , the preparation method is the same as above.

Detailed description: Unless otherwise stated, the following terms used in the specification and claims have the following meanings.

"Alkyl" refers to a saturated aliphatic hydrocarbon group, including linear or branched alkyl; for example, C₁-C₈ alkyl refers to an alkyl group containing 1-8 carbon atoms, and the C₁-C₈ alkyl includes C₁-C₂, C₁-C₃, C₁-C₄, C₁-C₅, C₂-C₃, C₂-C₄ alkyl, etc., such as methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, or various branched isomers thereof, preferably C₁-C₆ alkyl, and more preferably C₁-C₄ alkyl. The alkyl may be substituted or unsubstituted. In some embodiments, the alkyl is C₁, C₂, C₃, C₄, C₅, C₆, C₇, or C₈ alkyl.

"Heteroalkyl" means that the methylene group (-CH₂-) in a saturated aliphatic hydrocarbon group is substituted with a heteroatom (e.g., O, S, or N), a heteroatom group (e.g., -C(O)-, -S(O)-, -S(O)₂-, -C(O)O-, or -OC(O)-), C(O)NH-, -NHC(O)-, ethenylene, or ethynylene, and includes linear or branched heteroalkyl; C₁-C₈ heteroalkyl means that at least one methylene group in an alkyl group containing 1-8 carbon atoms is substituted with a heteroatom or a heteroatom group, such as -C(O)-CH₃, -CH₂-O-CH₃, -CH₂-S-CH₃, -CH₂-S(O)-CH₃, -CH₂-S(O)₂-CH₃, -CH₂-S-CH₂-CH₃, -CH₂-O-CH₂-CH₃, -CH₂-O-CH₂-CH₃, -CH₂-S(O)-CH₂-CH₃, or various branched isomers thereof, preferably C₁-C₆ heteroalkyl, and more preferably C₁-C₄ heteroalkyl. The heteroalkyl may be substituted or unsubstituted. In some embodiments, the heteroalkyl is C₁, C₂, C₃, C₄, C₅, C₆, C₇, or C₈ heteroalkyl.

Unless otherwise specified, the number of atoms on a ring is generally defined as the member number of the ring. For example, "5- to 7-membered ring" refers to a "ring" on which 5 to 7 atoms are arranged in a circle.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁, and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. Also, any range within n to n+m may be included. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

"Cycloalkyl" refers to a saturated or partially unsaturated, monocyclic or polycyclic hydrocarbon substituent; "C₃-C₁₁ cycloalkyl" refers to a cycloalkyl group containing 3 to 11 carbon atoms, and the C₃-C₁₁ cycloalkyl includes C₃-C₁₁, C₃-C₁₀, C₃-C₈, C₄-C₁₁, C₄-C₁₀, C₄-C₈, C₄-C₇, C₄-C₆, C₄-C₅, etc.; "C₃-C₈ cycloalkyl" refers to a cycloalkyl group containing 3 to 8 carbon atoms, and the C₃-C₈ cycloalkyl includes C₃-C₈, C₃-C₆, C₃-C₅, C₄-C₈, C₄-C₇, C₄-C₆, C₄-C₅, etc.

Non-limiting examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, etc., preferably cyclopropyl, cyclobutyl, cyclopentyl, and cyclohexyl, preferably C₃-C₈ cycloalkyl, and more preferably C₃-C₆ cycloalkyl.

Polycyclic cycloalkyl includes spiro, fused, and bridged cycloalkyl. "Spirocycloalkyl" refers to a polycyclic group in which a carbon atom (referred to as a spiro atom) is shared between monocyclic rings; they may contain one or more double bonds, but none of the rings has a fully conjugated π-electron system. According to the number of spiro atoms shared between rings, the spirocycloalkyl may include monospirocycloalkyl, bispirocycloalkyl, or polyspirocycloalkyl, and is preferably 7- to 12-membered bispirocycloalkyl. Non-limiting examples of the spirocycloalkyl include: 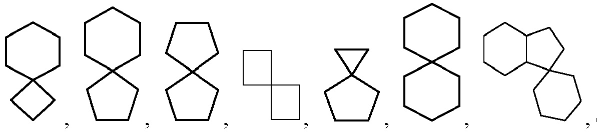 etc.

"Fused cycloalkyl" refers to an all-carbon polycyclic group in which each ring shares a pair of adjacent carbon atoms with the other rings in the system, wherein one or more of the rings may contain one or more double bonds, but none of the rings has a fully conjugated π-electron system. According to the number of constituent rings, the fused cycloalkyl may include bicyclic, tricyclic, tetracyclic, or polycyclic fused cycloalkyl, and is preferably bicyclic fused cycloalkyl. Non-limiting examples of the fused cycloalkyl include: 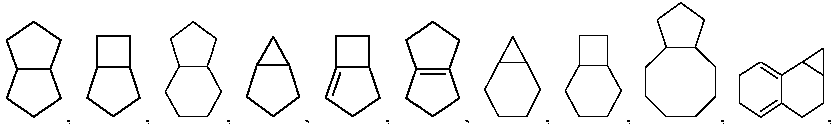 etc.

"Bridged cycloalkyl" refers to an all-carbon polycyclic group in which any two rings share two carbon atoms not directly connected; they may contain one or more double bonds, but none of the rings has a fully conjugated π-electron system. According to the number of constituent rings, the bridged cycloalkyl may include bicyclic, tricyclic, tetracyclic, or polycyclic bridged cycloalkyl. Non-limiting examples of the bridged cycloalkyl include: 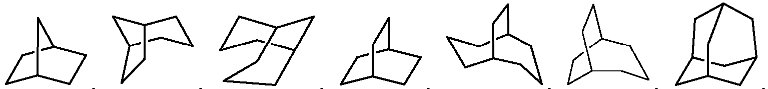 etc.

The cycloalkyl ring may be fused to an aryl, heteroaryl, or heterocycloalkyl ring, wherein the ring attached to the parent structure is cycloalkyl; non-limiting examples include indanyl, tetrahydronaphthyl, benzocycloheptyl, etc. The cycloalkyl may be optionally substituted or unsubstituted.

In some embodiments, the cycloalkyl is C₃, C₄, C₅, C₆, C₇, C₈, C₉, C₁₀, C₁₁ or C₁₂ monocyclic or polycyclic (e.g., spiro, fused or bridged) cycloalkyl.

"Heterocycloalkyl" refers to a saturated or partially unsaturated, monocyclic or polycyclic hydrocarbon substituent, wherein one or more (e.g., 2, 3, 4, or 5) of the ring atoms are selected from nitrogen, oxygen, and S(O)ᵣ (wherein r is an integer of 0, 1, or 2), but a ring portion of -O-O-, -O-S-, or -S-S- is not included, and the remaining ring atoms are carbon atoms, wherein the nitrogen atom is optionally quaternized, and the nitrogen and sulfur heteroatoms may optionally be oxidized (i.e., NO and S(O)p, and p is 1 or 2). "3- to 11-membered heterocycloalkyl" refers to a cyclic group containing 3 to 11 ring atoms, "5-to 10-membered heterocycloalkyl" refers to a cyclic group containing 5 to 10 ring atoms, and "3- to 8-membered heterocycloalkyl" refers to a cyclic group containing 3 to 8 ring atoms. "3- to 11-membered heterocycloalkyl" containing 1-2 heteroatoms selected from N, O, and S is preferred, and 3- to 11-membered heterocycloalkyl containing 1 or 2 N atoms is more preferred, such as 3- to 10-membered heterocycloalkyl, including 3- to 8-membered, 3- to 6-membered, 3- to 5-membered, 4- to 6-membered, 5- to 6-membered, 4-membered, 5-membered, and 6-membered heterocycloalkyl, etc. Examples of the 3- to 10-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, etc.

Monocyclic heterocycloalkyl is preferably 3- to 8-membered monocyclic heterocycloalkyl containing 1-2 N heteroatoms; non-limiting examples of the monocyclic heterocycloalkyl include pyrrolidinyl, piperidinyl, piperazinyl, morpholinyl, thiomorpholinyl, homopiperazinyl, etc., preferably piperidinyl and piperazinyl.

The polycyclic heterocycloalkyl includes spiro, fused, and bridged heterocycloalkyl. "Spiroheterocycloalkyl" refers to a polycyclic heterocycloalkyl group in which an atom (referred to as a spiro atom) is shared between monocyclic rings, wherein one or more of the ring atoms are selected from nitrogen, oxygen, and S(O)ᵣ (wherein r is an integer of 0, 1, or 2), and the remaining ring atoms are carbon atoms. They may contain one or more double bonds, but none of the rings has a fully conjugated π-electron system. According to the number of spiro atoms shared between rings, the spirocycloalkyl may include monospiroheterocycloalkyl, bispiroheterocycloalkyl, or polyspiroheterocycloalkyl, and is preferably saturated "3- to 11-membered bispiroheterocycloalkyl" containing 1-2 heteroatoms selected from N, O, and S, and more preferably saturated "7- to 11-membered bispiroheterocycloalkyl" containing 1 or 2 N atoms. Non-limiting examples of the spiroheterocycloalkyl include: 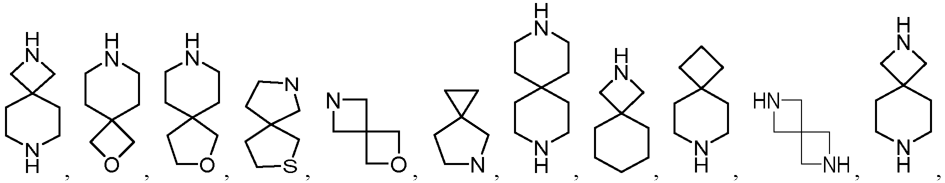 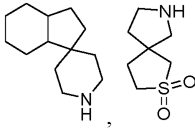 , etc.

"Fused heterocycloalkyl" refers to a polycyclic heterocycloalkyl group in which each ring shares a pair of adjacent atoms with the other rings in the system, wherein one or more of the rings may contain one or more double bonds, but none of the rings has a fully conjugated π-electron system, wherein one or more of the ring atoms are selected from nitrogen, oxygen, and S(O)ᵣ (wherein r is an integer of 0, 1, or 2), and the remaining ring atoms are carbon atoms. According to the number of constituent rings, the fused heterocycloalkyl may include bicyclic, tricyclic, tetracyclic, or polycyclic fused heterocycloalkyl, and is preferably "3- to 11-membered bicyclic fused heterocycloalkyl" containing 1-3 heteroatoms selected from N, O, and S, and more preferably saturated "3- to 11-membered bicyclic fused heterocycloalkyl" containing 1 or 2 N atoms. Non-limiting examples of the fused heterocycloalkyl include: 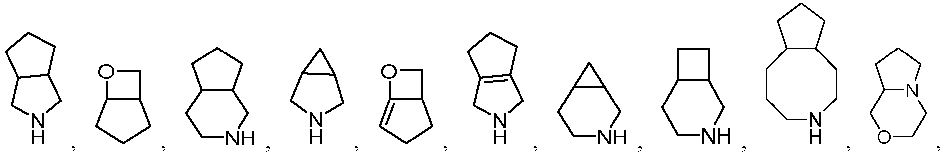 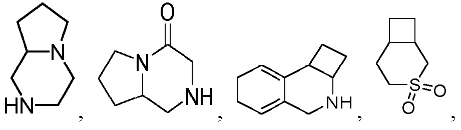 etc.

"Bridged heterocycloalkyl" refers to a polycyclic heterocycloalkyl group in which any two rings share two atoms not directly attached, and they may contain one or more double bonds, but none of the rings has a fully conjugated π-electron system, wherein one or more of the ring atoms are selected from nitrogen, oxygen, and S(O)ᵣ (wherein r is an integer of 0, 1, or 2), and the remaining ring atoms are carbon atoms. According to the number of constituent rings, the bridged heterocycloalkyl may include bicyclic, tricyclic, tetracyclic, or polycyclic bridged cycloalkyl; non-limiting examples of the bridged heterocycloalkyl include: 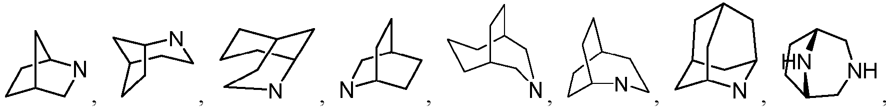 etc.

The heterocycloalkyl ring may be fused to an aryl, heteroaryl, or cycloalkyl ring, wherein the ring attached to the parent structure is heterocycloalkyl; non-limiting examples include: 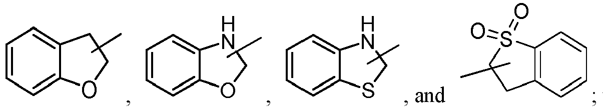 the heterocycloalkyl may be optionally substituted or unsubstituted.

In some embodiments, the heterocycloalkyl is 3-, 4-, 5-, 6-, 7-, 8-, 9-, 10-, 11-, or 12-membered monocyclic or polycyclic (e.g., spiro, fused, or bridged) heterocycloalkyl, wherein the number of the heteroatoms may be 1, 2, 3, 4, or 5, each heteroatom being independently nitrogen, oxygen, or S(O)ᵣ (wherein r is an integer of 0, 1, or 2).

"Aryl" refers to an all-carbon monocyclic or fused polycyclic group (i.e., rings that share pairs of adjacent carbon atoms) and a polycyclic group having a conjugated π-electron system; "C₆-C₁₀ aryl" or "6-to 10-membered aryl" refers to an all-carbon aryl group containing 6-10 carbon atoms, such as phenyl and naphthyl, preferably phenyl. The aryl ring may be fused to a heteroaryl, heterocycloalkyl, or cycloalkyl ring, wherein the ring attached to the parent structure is an aryl ring; non-limiting examples include: 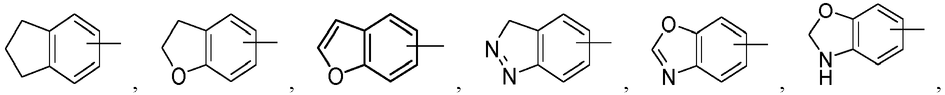 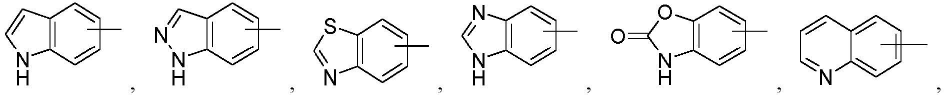 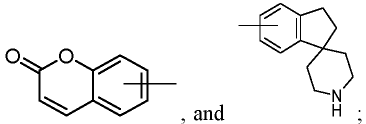 the aryl may be optionally substituted or unsubstituted. In some embodiments, the aryl is C₆-C₁₀ aryl.

"Heteroaryl" refers to a heteroaromatic system containing 1 to 4 heteroatoms, and the heteroatoms include nitrogen, oxygen, or S(O)ᵣ (wherein r is an integer of 0, 1, or 2); 5- to 6-membered heteroaryl refers to a heteroaromatic system containing 5-6 ring atoms; 5- to 10-membered heteroaryl refers to a heteroaromatic system containing 5-10 ring atoms, preferably 5- to 6-membered heteroaryl, and more preferably 5- to 6-membered heteroaryl containing 1 or 2 N atoms; non-limiting examples include furanyl, thienyl, pyridyl, pyrrolyl, *N*-alkylpyrrolyl, pyrimidinyl, pyrazinyl, pyrazole, imidazolyl, triazolyl, tetrazolyl, etc., preferably pyridyl. The heteroaryl ring may be fused to an aryl, heterocycloalkyl, or cycloalkyl ring, wherein the ring attached to the parent structure is a heteroaryl ring; non-limiting examples include: 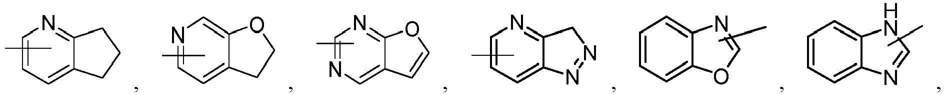 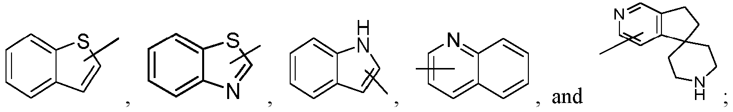 the heteroaryl may be optionally substituted or unsubstituted. In some embodiments, the heteroaryl is 5-, 6-, 7-, 8-, 9-, or 10-membered heteroaryl, wherein the number of the heteroatoms may be 1, 2, 3, 4, or 5, each heteroatom being independently nitrogen, oxygen, or S.

"Alkenyl" refers to an alkyl group as defined above that consists of at least two carbon atoms and at least one carbon-carbon double bond; "C₂₋₈ alkenyl" refers to a linear or branched alkenyl group containing 2-8 carbon atoms, including but not limited to, ethenyl, 1-propenyl, 2-propenyl, 1-, 2- or 3-butenyl, etc., preferably "C₂₋₆ alkenyl", and more preferably "C₂₋₄ alkenyl". The alkenyl may be substituted or unsubstituted. In some embodiments, the alkenyl is C₂, C₃, C₄, C₅, C₆, C₇ or C₈ alkenyl.

"Alkynyl" refers to an alkyl group as defined above that consists of at least two carbon atoms and at least one carbon-carbon triple bond; "C₂₋₈ alkynyl" refers to a linear or branched alkynyl group containing 2-8 carbon atoms, including but not limited to, ethynyl, 1-propynyl, 2-propynyl, 1-, 2-, or 3-butynyl, preferably "C₂₋₆ alkynyl", and more preferably "C₂₋₄ alkynyl". The alkynyl may be substituted or unsubstituted. In some embodiments, the alkynyl is C₂, C₃, C₄, C₅, C₆, C₇ or C₈ alkynyl.

"-ylene" refers to a divalent group. For example, alkylene refers to a divalent alkyl group; alkenylene refers to a divalent alkenyl group; alkynylene refers to a divalent alkynyl group; cycloalkylene refers to a divalent cycloalkyl group; heterocycloalkylene refers to a divalent heterocycloalkyl group; arylene refers to a divalent aryl group; heteroarylene refers to a divalent heteroaryl group; the alkyl, alkenyl, alkynyl, cycloalkyl, heterocycloalkyl, aryl, and heteroaryl groups are as defined above; the "-ylene" groups may be optionally substituted or unsubstituted.

"Haloalkyl" refers to an alkyl group optionally substituted with one or more fluorine, chlorine, bromine, or iodine atoms, wherein the alkyl group is as defined above; non-limiting examples include difluoromethyl, dichloromethyl, dibromomethyl, trifluoromethyl, trichloromethyl, tribromomethyl, etc.

"Hydroxyalkyl" refers to an alkyl group optionally substituted with one or more -OH, wherein the alkyl group is as defined above; non-limiting examples include hydroxymethyl, hydroxyethyl, hydroxypropyl, and hydroxyisopropyl.

"Alkoxy" refers to -O-alkyl, wherein the alkyl is as defined above; non-limiting examples include methoxy, ethoxy, isopropoxy, tert-butoxy, etc.

"Cycloalkoxy" refers to -O-cycloalkyl, wherein the cycloalkyl is as defined above; non-limiting examples include cyclopropaneoxy, cyclobutaneoxy, cyclopentanyloxy, cyclohexyloxy, etc.

"Heterocycloalkoxy" refers to -O-heterocycloalkyl, wherein the heterocycloalkyl is as defined above; non-limiting examples include azetidinyloxy, azacyclopentyloxy, piperidinyloxy, piperazinyloxy, oxolanyloxy, oxanyloxy, etc.

"-C(O)C₁-C₃ alkyl" refers to -C(O)-CH₃, -C(O)-CH₂CH₃, etc.

"Heteroatom or heteroatom group" refers to N, O, S, S(=O)₂, S(O), etc.

"Cyano" refers to -CN.

"Hydroxy" refers to -OH.

"Sulfonyl" refers to -S(O)₂-.

"Carboxyl" or "carboxylic acid" refers to -COOH.

"Oxo" refers to the =O group.

"Halogen" refers to fluorine, chlorine, bromine, or iodine.

"EA or EtOAc" refers to ethyl acetate.

"THF" refers to tetrahydrofuran.

"DCM" refers to dichloromethane.

"cataCXium A Pd-G3" refers to mesylate[n-butyl di(1-adamantyl)phosphine](2-amino-1,1'-biphenyl-2-yl)palladium(II).

"POCl₃" refers to phosphorus oxychloride.

"NaHCO₃" refers to sodium bicarbonate.

"NaCl" refers to sodium chloride.

"Na₂SO₄" refers to sodium sulfate.

"NH₄Cl" refers to ammonium chloride.

"Cs₂CO₃" refers to cesium carbonate.

"NaBH(OAc)₃" refers to sodium triacetoxyborohydride.

"Triton B" refers to benzyltrimethylammonium hydroxide.

"NaBH₃CN" refers to sodium cyanoborohydride.

"CsF" refers to cesium fluoride.

"Na₂CO₃" refers to sodium carbonate.

"HOAc" refers to acetic acid.

"TPAP" refers to tetrapropylammonium perruthenate.

"NMO" refers to N-methylmorpholine oxide.

"Cs₂CO₃" refers to cesium carbonate.

"NaH" refers to sodium hydride.

"i-PrOH" refers to isopropanol.

"DMF" refers to N,N-dimethylformamide.

"MeOH" refers to methanol.

"Pd/C" refers to palladium/carbon.

"DMSO" refers to dimethyl sulfoxide.

"TFA" refers to trifluoroacetic acid.

"DIEA" refers to *N,N*-diisopropylethylamine.

"Dess-Martin" refers to Dess-Martin reagent.

"PBS" refers to phosphate-buffered saline.

"SDS-PAGE" refers to sodium dodecyl sulfate-polyacrylamide gel electrophoresis.

"PVDF" refers to polyvinylidene fluoride.

"PE" refers to petroleum ether.

"AcOH" refers to acetic acid.

"HCl" refers to hydrochloric acid.

"dioxane" refers to 1,4-dioxane.

"ACN" refers to acetonitrile.

"DMP" refers to dimethyl phthalate.

"Pd-118" refers to 1,1'-bis(di-*tert*-butylphosphino)ferrocene palladium dichloride.

"BH₃" refers to borane.

"PdCl₂(dtbpf)" refers to [1,1'-bis(di-*tert*-butylphosphino)ferrocene]palladium dichloride.

"HATU" refers to 2-(7-azabenzotriazol-1-yl)-*N,N,N',N*'-tetramethyluronium hexafluorophosphate.

"EtOH" refers to ethanol.

"DMAP" refers to 4-dimethylaminopyridine.

"TBAF" refers to tetrabutylammonium fluoride.

"EDCI" refers to 1-ethyl-(3-dimethylaminopropyl)carbodiimide hydrochloride.

"HOBT" refers to 1-hydroxybenzotriazole.

"LiAlH₄" refers to lithium aluminum hydride.

"RuPhos Pd G3" refers to (2-dicyclohexylphosphino-2',6'-diisopropoxy-1,1'-biphenyl)[2-(2'-amino-1,1'-biphenyl)]palladium (II) methanesulfonate.

"LAH" refers to lithium aluminum hydride.

"TsCl" refers to p-toluenesulfonyl chloride.

"Na₂S₂O₃" refers to sodium thiosulfate.

"NaNO₂" refers to sodium nitrite.

"BBr₃" refers to boron tribromide.

"K₃PO₄" refers to potassium phosphate.

"AcOK" refers to potassium acetate.

"DIBAL-H" refers to diisobutylaluminum hydride.

"TEA" refers to triethylamine.

"GTP" refers to guanosine triphosphate.

"Prep-HPLC" or "pre-HPLC" refers to preparative high-performance liquid chromatography.

"sat." refers to a saturated solution.

"aq" refers to an aqueous solution.

In the chemical structure herein, "-" as a linking bond represents a single bond, and "=" represents a double bond (in the case of an undefined configuration, it may be *trans* or *cis*)*.*

"Optional" or "optionally" means that the event or circumstance subsequently described may, but does not necessarily, occur; this description includes instances where the event or circumstance occurs or does not occur. For example, "a heterocycloalkyl group optionally substituted with an alkyl group" means that the alkyl group may, but does not necessarily, exist; this description includes instances where the heterocycloalkyl group is substituted with the alkyl group and instances where it is not.

"Substituted" means that one or more, preferably up to 5, and more preferably 1-3, hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when amino or hydroxy having a free hydrogen is bound to a carbon atom having an unsaturated (such as olefin) bond.

As used herein, unless otherwise indicated, the term "optionally substituted" may be unsubstituted or substituted; when it is substituted, the substituent may be one or more (e.g., 2, 3, 4, 5, or 6) groups independently selected from alkyl, alkenyl, alkynyl, hydroxy, hydroxyalkyl, haloalkyl, alkoxy, amino, aminoalkyl, cyano, halogen, oxo, cycloalkyl, heterocycloalkyl, aryl, and heteroaryl, and the alkyl, alkenyl, alkynyl, hydroxy, hydroxyalkyl, haloalkyl, alkoxy, amino, and aminoalkyl are optionally substituted with one or more (e.g., 2, 3, 4, 5, or 6) cycloalkyl, heterocycloalkyl, aryl, or heteroaryl; the cycloalkyl, heterocycloalkyl, aryl, and heteroaryl are optionally substituted with one or more groups selected from alkyl, alkenyl, alkynyl, hydroxy, hydroxyalkyl, haloalkyl, alkoxy, amino, aminoalkyl, cyano, halogen, and oxo.

Unless otherwise indicated, the following terms and phrases used herein are intended to have the following meanings. A specific term or phrase should not be considered indefinite or unclear in the absence of a specific definition, but should be understood in the conventional sense. When a trade name appears herein, it is intended to refer to its corresponding commodity or active ingredient thereof.

"Pharmaceutical composition" refers to a mixture containing one or more of the compounds or the physiologically/pharmaceutically acceptable salts or prodrugs thereof described herein and other chemical components, as well as other components such as physiologically/pharmaceutically acceptable carriers and excipients. The pharmaceutical composition is intended to promote the administration to an organism, so as to facilitate the absorption of the active ingredient, thereby exerting biological activity.

The present disclosure further provides a pharmaceutically acceptable salt of the compound of formula (I). The term "pharmaceutically acceptable salt" refers to a relatively non-toxic acid addition salt or base addition salt of the compound of the present disclosure. The acid addition salt is a salt formed from the compound of formula (I) of the present disclosure and a suitable inorganic acid or organic acid. These salts may be prepared during the final isolation and purification of the compound or by reacting the purified compound of formula (I) in its free base form with a suitable organic acid or inorganic acid. The representative acid addition salt includes hydrochloride, tartrate, hydrobromide, sulfate, bisulfate, sulfite, acetate, oxalate, valerate, oleate, palmitate, stearate, laurate, borate, benzoate, lactate, phosphate, biphosphate, carbonate, bicarbonate, toluate, citrate, maleate, fumarate, succinate, methanesulfonate, p-toluenesulfonate, gluconate, lactobionate, lauryl sulfonate, and the like. The base addition salt is a salt formed from the compound of formula (I) and a suitable inorganic base or organic base, including, for example, salts formed with alkali metal, alkaline earth metal, and quaternary ammonium cations, such as sodium salts, lithium salts, potassium salts, calcium salts, magnesium salts, tetramethyl quaternary ammonium salts, tetraethyl quaternary ammonium salts, and the like; amine salts include salts formed with ammonia (NH₃) and primary, secondary, or tertiary amines, such as methylamine salts, dimethylamine salts, trimethylamine salts, triethylamine salts, ethylamine salts, and the like.

The pharmaceutically acceptable salts of the present disclosure can be synthesized from a parent compound containing an acidic or basic group using conventional chemical methods. In general, such salts are prepared by subjecting the compounds in a free acid or base form to a reaction with a stoichiometric amount of appropriate base or acid in water or an organic solvent or a mixture thereof.

The term "pharmaceutically acceptable excipient" refers to an inert substance administered with an active ingredient to facilitate administration of the active ingredient, including but not limited to, any glidant, sweetener, diluent, preservative, dye/coloring agent, flavor enhancer, surfactant, wetting agent, dispersant, disintegrant, suspending agent, stabilizer, isotonizing agent, solvent, or emulsifier acceptable for use in humans or animals (e.g., domesticated animals) as permitted by the National Medical Products Administration, PRC.

As used herein and as is familiar in the art, "treatment" or "treating" is a method for obtaining beneficial or desired results, including clinical results. The beneficial or desired clinical results may include, but are not limited to, reduction in the tumor progression, reduction in the tumor size, decrease in the tumor growth rate, reduction in tumor invasion and metastatic potential, alleviation or amelioration of one or more symptoms or conditions, decrease in the extent of disease, stabilized (i.e., not worsening) disease state, prevention of disease spread, delay or slowing down of the disease progression, amelioration or mitigation of the disease state, and alleviation (whether partial or total), whether detectable or undetectable. "Treatment" or "treating" may also refers to prolonging survival as compared to the expected survival if not receiving treatment.

The therapeutic dose of the compound of the present application may be determined, for example, according to: the specific use of the treatment, the route of administration of the compound, the health and state of the patient, and the judgment of the prescriber. The proportion or concentration of the compound of the present application in a pharmaceutical composition may not be constant and depends on a variety of factors including doses, chemical properties (e.g., hydrophobicity), and administration routes.

In the present disclosure, the expression -N(group)₁₋₂ refers to -NH(group) or -N(group)₂.

The term "treatment" or "treating" refers to administering the compound or formulation described herein to ameliorate or eliminate a disease or one or more symptoms related to the disease, and includes:
(i) inhibiting a disease or disease state, i.e., arresting its progression; and
(ii) alleviating a disease or disease state, i.e., causing regression of the disease or the disease state.

The term "therapeutically effective amount" refers to an amount of the compound of the present application for (i) treating a specific disease, condition, or disorder, (ii) alleviating, ameliorating, or eliminating one or more symptoms of a specific disease, condition, or disorder, or (iii) preventing or delaying onset of one or more symptoms of the specific disease, condition, or disorder described herein. The amount of the compound of the present application composing the "therapeutically effective amount" varies depending on the compound, the disease state, and its severity, the administration mode, and the age of the mammal to be treated, but may be determined routinely by those skilled in the art in accordance with their knowledge and the present disclosure.

Unless otherwise required, the word "comprise" and variations thereof such as "comprises" and "comprising", used in the specification and the claims which follow, should be understood in an open-ended and non-exclusive sense, i.e., "including, but not limited to".

"In some embodiments", "in an embodiment", "in another embodiment", or "in certain embodiments" used in the specification means that a specific reference element, structure, or feature described in connection with the embodiment is included in at least one embodiment. Thus, the phrase "in some embodiments", "in an embodiment", "in another embodiment", or "in certain embodiments" in various places throughout the specification are not necessarily all referring to the same embodiment. Furthermore, the specific elements, structures, or features may be combined in any suitable manner in one or more embodiments.

Unless otherwise indicated, the term "isomer" is intended to include geometric isomers, *cis-trans* isomers, stereoisomers, enantiomers, optical isomers, diastereomers, and tautomers.

The compounds of the present disclosure may be in the form of a specific geometric isomer or stereoisomer. All such compounds are contemplated herein, including *cis* and *trans* isomers, (-)- and (+)-enantiomers, (R)- and (S)-enantiomers, diastereomers, (D)-isomers, (L)-isomers, and racemic mixtures and other mixtures thereof, such as enantiomer or diastereomer enriched mixtures, all of which are encompassed within the scope of the present disclosure. Additional asymmetric carbon atoms may be present in substituents such as alkyl. All these isomers and mixtures thereof are encompassed within the scope of the present disclosure.

Unless otherwise indicated, the term "enantiomer" or "optical isomer" refers to stereoisomers that are mirror images of each other.

Unless otherwise indicated, the term *"cis-trans* isomer" or "geometric isomer" results from the inability of a double bond or a single bond of a ring carbon atom to rotate freely.

Unless otherwise indicated, the term "diastereomer" refers to stereoisomers whose molecules have two or more chiral centers and are not mirror images of each other.

Unless otherwise indicated, the absolute configuration of a stereogenic center is represented by a wedged solid bond and a wedged dashed bond, and the relative configuration of a stereogenic center is represented by a straight solid bond and a straight dashed bond. A wavy line represents a wedged solid bond or a wedged dashed bond, or a wavy line represents a straight solid bond or a straight dashed bond.

Unless otherwise indicated, the term "diastereomer" refers to stereoisomers whose molecules have two or more chiral centers and are not mirror images of each other.

Unless otherwise indicated, "(+)" represents dextrorotation, "(-)" represents levorotation, and "(±)" represents racemization.

"Optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents, wherein the substituents may include deuterium and hydrogen variants, as long as the valence of the specific atom is normal and the substituted compound is stable. When the substituent is oxygen (i.e., =O), it means that two hydrogen atoms are substituted. Substitution with oxygen does not occur on aromatic groups.

The term "optionally substituted" means that an atom may or may not be substituted with a substituent. Unless otherwise specified, the type and number of the substituent may be arbitrary as long as being chemically achievable.

The term "prodrug" refers to a chemical derivative of the compound of the present disclosure that, if chemically reacted *in vivo*, is converted to a compound of general formula I.

When any variable (e.g., R) occurs once or more in the constitution or structure of a compound, the definition of the variable in each case is independent. Thus, for example, if a group is substituted with 0-2 R, the group may be optionally substituted with up to two R, and the definition of R in each case is independent. Furthermore, a combination of the substituent and/or the variant thereof is permissible only if the combination can result in a stable compound.

When the number of a linking group is 0, for example, -(CRR)0-, it means that the linking group is a single bond.

When one of the variables is selected from a single bond, it means that the two groups connected to it are linked directly. For example, in A-L-Z, when L represents a single bond, it means that the structure is actually A-Z.

When a substituent is absent, it means that there is no such a substituent. For example, when X in A-X is absent, the structure is actually A. When it is not specified by which atom the listed substituent is linked to the group to be substituted, the substituent may be linked via any atom of the group. For example, pyridyl as a substituent may be linked to the group to be substituted via any carbon atom on the pyridine ring.

When the direction for linkage of the listed linking group is not specified, the direction for linkage is arbitrary. For example, when the linking group L is -M-W-, -M-W- may either link ring A to ring B in a direction same as left-to-right reading order to form, or link ring A to ring B in a direction opposite to the left-to-right reading order to form. A combination of the linking group, the substituent, and/or the variant thereof is permissible only if the combination can result in a stable compound.

Unless otherwise specified, when a group has one or more linkable sites, any one or more of the sites of the group may be linked to other groups by chemical bonds. If there is no designated linking mode for chemical bonds and H atoms are present at a linkable site, when the linkable site is linked to chemical bonds, the number of the H atoms at the linkable site is correspondingly reduced based on the number of the linked chemical bonds, so that the resulting groups have corresponding valences. The chemical bond that links the site to another group may be represented by a straight solid bond, a straight dashed bond, or a wavy line. For example, the straight solid bond in -OCH₃ indicates that the group is linked to another group through the oxygen atom; the straight dashed bond indicates that the group is linked to another group through the two ends of the nitrogen atom; the wavy line indicates that the phenyl group is linked to another group through the carbon atoms on positions 1 and 2.

Unless otherwise specified, 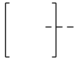 is used to indicate that a hydrogen atom at any position of a group within 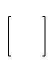 may be substituted.

Unless otherwise indicated, the term "enriched with one isomer", "isomer enriched", "enriched with one enantiomer", or "enantiomer enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise indicated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee) is 80%.

Optically active (R)- and (S)-enantiomers, as well as D- and L-isomers may be prepared by chiral synthesis, chiral reagents, or other conventional techniques. If one enantiomer of a certain compound of the present disclosure is to be obtained, the desired pure enantiomer may be prepared by asymmetric synthesis or derivatization using a chiral auxiliary, wherein the resulting diastereoisomeric mixture is separated and the auxiliary group is cleaved. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen may be substituted with deuterium to form a deuterated drug, and the bond formed by deuterium and carbon is firmer than that formed by common hydrogen and carbon. Compared with an un-deuterated drug, the deuterated drug has the advantages of reduced toxic and side effects, increased pharmaceutical stability, enhanced efficacy, prolonged biological half-life, and the like. All isotopic variations of the compounds of the present disclosure, whether radioactive or not, are encompassed within the scope of the present disclosure.

The compounds of the present disclosure may be prepared by a variety of synthetic methods well known to those skilled in the art, including the specific embodiments listed below, embodiments formed by combinations thereof with other chemical synthetic methods, and equivalents thereof well known to those skilled in the art. Preferred embodiments include, but are not limited to, the examples of the present disclosure.

The structures of the compounds of the present disclosure may be confirmed by conventional methods well known to those skilled in the art, and if the present disclosure relates to an absolute configuration of the compound, the absolute configuration may be confirmed by means of conventional techniques in the art. For example, in single crystal X-ray diffraction (SXRD), intensity data of diffraction of the single crystal grown are collected with a Bruker D8 venture diffractometer, with the light source being Cu-Kα radiation and the scanning mode being scanning; after related data are collected, a direct method (Shelxs97) is further employed to analyze the crystal structure, and thus the absolute configuration can be confirmed.

The compound or the pharmaceutically acceptable salt thereof of the present disclosure may be administered to mammals including humans, and may be administered orally, rectally, parenterally (intravenously, intramuscularly, or subcutaneously), topically (powders, ointments, or drops), or intratumorally.

The compound of the present disclosure may be administered at a dose of about 0.05-300 mg/kg body weight/day, preferably 10-300 mg/kg body weight/day, and more preferably 10-200 mg/kg body weight/day.

The compound or the pharmaceutically acceptable salt thereof of the present disclosure may be formulated into a solid dosage form for oral administration, including but not limited to, capsules, tablets, pills, pulvis, granules, and the like. In these solid dosage forms, the compound of formula (I) of the present disclosure, as an active ingredient, is mixed with at least one conventional inert excipient (or carrier), such as sodium citrate or dicalcium phosphate, or with the following ingredients: (1) fillers or extenders, such as starch, lactose, sucrose, glucose, mannitol, silicic acid, and the like; (2) binders, such as hydroxymethyl cellulose, alginate, gelatin, polyvinylpyrrolidone, sucrose, acacia, and the like; (3) humectants, such as glycerol and the like; (4) disintegrants, such as agar, calcium carbonate, potato or tapioca starch, alginic acid, certain complex silicates, sodium carbonate, and the like; (5) solution retarders, such as paraffin and the like; (6) absorption accelerators, such as quaternary ammonium compounds and the like; (7) wetting agents, such as cetyl alcohol, glycerol monostearate, and the like; (8) adsorbents, such as kaolin and the like; and (9) lubricants, such as talc, calcium stearate, magnesium stearate, solid polyethylene glycol, sodium lauryl sulfate, and the like, or mixtures thereof. Capsules, tablets, and pills may further comprise buffers.

The solid dosage forms such as tablets, dragees, capsules, pills, and granules may be coated or microencapsulated using coatings and shells such as enteric coatings and other materials well known in the art. They may comprise opacifying agents, and the active ingredient in such a composition may be released in a certain part of the digestive tract in a delayed manner. Examples of embedding components that can be used are polymeric substances and wax-based substances. If necessary, the active ingredient can also be in microcapsule form with one or more of the above-mentioned excipients.

The compound or the pharmaceutically acceptable salt thereof of the present disclosure may be formulated into a liquid dosage form for oral administration, including but not limited to, pharmaceutically acceptable emulsions, solutions, suspensions, syrups, tinctures, and the like. In addition to the compound of formula (I) or the pharmaceutically acceptable salt thereof as an active ingredient, the liquid dosage form may comprise inert diluents commonly used in the art, such as water and other solvents, solubilizers, and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, sesame oil, and the like, or mixtures of these substances. In addition to these inert diluents, the liquid dosage form of the present disclosure may further comprise conventional adjuvants, such as wetting agents, emulsifiers and suspending agents, sweeteners, flavoring agents, perfuming agents, and the like.

The suspending agents include, for example, ethoxylated isostearyl alcohols, polyoxyethylene sorbitol and sorbitan esters, microcrystalline cellulose, aluminum methylate, agar, and the like, or mixtures of these substances.

The compound or the pharmaceutically acceptable salt thereof of the present disclosure may be formulated into a dosage form for parenteral injection, including but not limited to, physiologically acceptable sterile aqueous or water-free solutions, dispersions, suspensions, or emulsions, and sterile powders for re-dissolution to form sterile injectable solutions or dispersions. Suitable carriers, diluents, solvents, or excipients include water, ethanol, polyols, and suitable mixtures thereof.

The compound or the pharmaceutically acceptable salt thereof of the present disclosure may also be formulated into a dosage form for topical administration, including, for example, ointments, pulvis, suppositories, drops, sprays, inhalants, and the like. The compound of formula (I) or the pharmaceutically acceptable salt thereof of the present disclosure as an active ingredient is mixed under sterile conditions with a physiologically acceptable carrier and optional preservatives, buffers, or propellants that may be required if necessary.

The present disclosure further provides a pharmaceutical composition comprising the compound of formula (I) or the pharmaceutically acceptable salt thereof of the present disclosure as an active ingredient, and a pharmaceutically acceptable carrier, excipient, or diluent. In the preparation of the pharmaceutical composition, the compound of formula (I) or the pharmaceutically acceptable salt thereof of the present disclosure is typically mixed with the pharmaceutically acceptable carrier, excipient, or diluent.

The composition of the present disclosure may be formulated into a conventional pharmaceutical formulation according to a conventional preparation method. Examples include tablets, pills, capsules, pulvis, granules, emulsions, suspensions, dispersions, solutions, syrups, elixirs, ointments, drops, suppositories, inhalants, sprays, and the like.

The compound or the pharmaceutically acceptable salt thereof described herein may be administered alone or, if desired, in combination with other pharmaceutically acceptable therapeutic agents, such as other anti-tumor medicaments. The ingredients to be combined may be administered simultaneously or sequentially, and administered in a single formulation form or in different formulation forms. The combination may include not only a combination of the compound of the present disclosure and one additional active agent but also a combination of the compound of the present disclosure and two or more additional active agents.

In the present disclosure, other pharmaceutically acceptable therapeutic agents that may be used together with or in combination with the KRAS G12D degrader, the compound of formula (I), may be: EGFR and/or a mutant inhibitor thereof, ErbB2(Her2) and/or a mutant inhibitor thereof, ALK and/or a mutant inhibitor thereof, MEK and/or a mutant inhibitor thereof, KRAS and/or a mutant inhibitor thereof, BCR-ABL and/or a mutant inhibitor thereof, FGFR1/FGFR2/FGFR3 and/or a mutant inhibitor thereof, ROS1 and/or a mutant inhibitor thereof, c-MET and/or a mutant inhibitor thereof, AXL and/or a mutant inhibitor thereof, NTRK1 and/or a mutant inhibitor thereof, RET and/or a mutant inhibitor thereof, taxane, a platinum-containing compound, an antimetabolite, a mitotic kinase inhibitor, an immunotherapeutic agent, an anti-angiogenic drug, a topoisomerase inhibitor, A-Raf/B-Raf/C-RAf and/or a mutant inhibitor thereof, ERK and/or a mutant inhibitor thereof, an apoptosis inhibitor, AKT and/or a mutant inhibitor thereof, an mTOR inhibitor, an epigenetic modulator, an IGF1/2 and/or IGF1-R inhibitor, Ras GEF and/or a mutant inhibitor thereof, SOS1 and/or a mutant inhibitor thereof, SHP2 and/or a mutant inhibitor thereof, PI3K and/or a mutant inhibitor thereof, or a PD-1/PD-L1 inhibitor; EGFR and/or a mutant degrader thereof, ErbB2(Her2) and/or a mutant degrader thereof, ALK and/or a mutant degrader thereof, MEK and/or a mutant degrader thereof, KRAS and/or a mutant degrader thereof, BCR-ABL and/or a mutant degrader thereof, FGFR1/FGFR2/FGFR3 and/or a mutant degrader thereof, ROS1 and/or a mutant degrader thereof, c-MET and/or a mutant degrader thereof, AXL and/or a mutant degrader thereof, NTRK1 and/or a mutant degrader thereof, RET and/or a mutant degrader thereof, A-Raf/B-Raf/C-RAf and/or a mutant degrader thereof, ERK and/or a mutant degrader thereof, AKT and/or a mutant degrader thereof, an IGF1/2 and/or IGF1-R degrader, Ras GEF and/or a mutant degrader thereof, SOS1 and/or a mutant degrader thereof, SHP2 and/or a mutant degrader thereof, or PI3K and/or a mutant degrader thereof; EGFR and/or a mutant monoclonal antibody thereof, ErbB2(Her2) and/or a mutant monoclonal antibody thereof, a PD-1/PD-L1 monoclonal antibody, or a CTLA-4 monoclonal antibody; a PD-L1/TIGHT bispecific antibody, a PD-L1/CTLA-4 bispecific antibody, an EGFR/MET bispecific antibody, an EGFR/CD3 bispecific antibody, an EGFR/4-IBB bispecific antibody, a PD-L1/4-IBB bispecific antibody, or an HER2/CD3 bispecific antibody.

In the present disclosure, other pharmaceutically acceptable therapeutic agents that may be used together with or in combination with the KRAS G12D degrader, the compound of formula (I), may be: afatinib, erlotinib, gefitinib, lapatinib, cetuximab, panitumumab, osimertinib, olmutinib, EGF-816, trastuzumab, pertuzumab, crizotinib, alectinib, entrectinib, brigatinib, trametinib, cobimetinib, binimetinib, selumetinib, refametinib, imatinib, dasatinib, nilotinib, nintedanib, crizotinib, lorlatinib, ceritinib, merestinib, paclitaxel, nab-paclitaxel, docetaxel, cisplatin, carboplatin, oxaliplatin, 5-fluorouracil, capecitabine, floxuridine, cytarabine, gemcitabine, a combination of trifluridine and tipiracil (=TAS102), palbociclib, ribociclib, abemaciclib, ipilimumab, nivolumab, pembrolizumab, atezolizumab, avelumab, durvalumab, pidilizumab, PDR-001 (=spartalizumab), bevacizumab, irinotecan, liposomal irinotecan, topotecan, ulixertinib, rapamycin, temsirolimus, everolimus, ridaforolimus, JQ-1, GSK 525762, OTX 015 (=MK8628), CPI 0610, TEN-010 (=RO6870810), xentuzumab (antibody 60833 in WO 2010/066868), or MEDI-573 (=dusigitumab).

On the basis of the general knowledge in the art, the preferred conditions described above may be combined arbitrarily to obtain preferred embodiments of the present disclosure.

The reagents and starting materials used in the present disclosure are commercially available.

Compounds are named according to conventional nomenclature rules in the art, and supplier's catalog names are given for commercially available compounds.

According to the present disclosure, KRAS G12D kinase activity tests prove that the compound of formula I described herein is able to effectively bind to the KRAS G12D target protein or have an inhibitory effect, and it is proved that the compound of formula I described herein is able to effectively and specifically degrade the KRAS G12D protein in A427 cells by means of Western-Blot. The compound of formula I, and/or the stereoisomer, the enantiomer, the diastereomer, the deuteride, the hydrate, the solvate, the metabolite or the prodrug thereof and/or the pharmaceutically acceptable salt thereof described herein can effectively degrade the KRAS G12D protein, thereby achieving the effect of preventing or treating a disease or disorder associated with KRAS G12D or caused by the interaction of KRAS G12D with SOS1 or SHP2 protein.

### DETAILED DESCRIPTION

The following examples further illustrate the present disclosure, but the present disclosure is not limited thereto. Experimental procedures without specified conditions in the following examples were performed in accordance with conventional procedures and conditions, or in accordance with product instructions.

The present disclosure is further explained in detail below with reference to examples; however, the examples are not intended to limit the present disclosure, and the present disclosure is not limited to the contents of the examples. The starting materials in the examples of the present disclosure are known and commercially available, or may be synthesized by using or following methods known in the art. Unless otherwise stated, experimental methods without specific conditions indicated in the examples of the present disclosure were generally conducted under conventional conditions or conditions recommended by the manufacturers of the starting materials or commercial products.

### I. Compound Preparation Examples

### Intermediate 1: tert-butyl 3-(8-fluoro-7-(7-fluoro-3-(methoxymethoxy)-8-((triisopropylsilyl)ethynyl) naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate

### Step 1: preparation of 1-((4-benzylpiperazin-1-yl)methyl)cyclopropyl)methanol

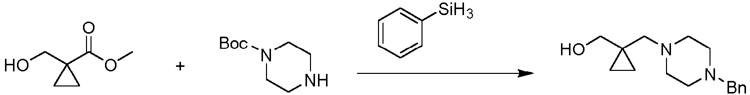

*tert*-Butyl piperazine-1-carboxylate (14.7 g, 102.0 mmol) was dissolved in toluene (120 mL), and the mixture was heated to 120 °C. Then, methyl 1-(hydroxymethyl)cyclopropane-1-carboxylate (12 g, 68.0 mmol) and phenylsilane (5.5 g, 51.0 mmol) were added. The mixture was reacted at 120 °C for 16 h, and then phenylsilane (14.7 g, 136.0 mmol) and zinc acetate (1.2 g, 6.8 mmol) were added. The mixture was reacted for another 2 h. After the reaction was completed, the reaction mixture was cooled to room temperature. 2 M aqueous hydrochloric acid solution (120 mL) was added to quench the reaction, and the mixture was extract with dichloromethane (10 mL × 3). The aqueous phase was adjusted to pH 12 with a sodium oxide solution (4 M, 60 mL) and extract with dichloromethane (100 mL × 3). The organic phases were combined, dried over anhydrous sodium sulfate, and concentrated under reduced pressure. The resulting crude product was purified by silica gel column chromatography to give 1-((4-benzylpiperazin-1-yl)methyl)cyclopropyl)meth*a*nol.

LC-MS: (ESI, m/z): [M+H]⁺ = 261.2.

### Step 2: preparation of tert-butyl 3-(2-((1-((4-benzylpiperazin-1-yl)methyl)cyclopropyl)methoxy)-7-chloro-8-fluoropyrido[4,3-d]pyrimidin-4-yl)-3,8-diazabicyclo[3.2.1]octane-8-carboxylate

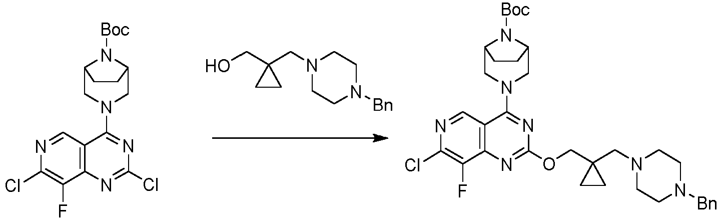

*tert*-Butyl 3-(2,7-dichloro-8-fluoropyrido[4,3-*d*]pyrimidin-4-yl)-3,8-diazabicyclo[3.2.1]octane-8-carboxylate (5.00 g, 11.70 mmol) and 1-((4-benzylpiperazin-1-yl)methyl)cyclopropyl)methanol (3.30 g, 12.80 mmol) were dissolved in THF, and then cesium carbonate was added. The mixture was stirred at 90 °C for 16 h. After the reaction was completed, the reaction mixture was cooled to room temperature. A saturated ammonium chloride solution (100 mL) was added to quench the reaction, and the mixture was extracted with ethyl acetate (100 mL × 3). The organic phases were combined, dried over anhydrous sodium sulfate, and concentrated under reduced pressure. The concentrate was purified by silica gel column chromatography (PE:EtOAc = 10:1 to 3:1) to give *tert*-butyl 3-(2-((1-((4-benzylpiperazin-1-yl)methyl)cyclopropyl)methoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 652.3.

### Step 3: preparation of tert-butyl 3-(7-chloro-8-fluoro-2-((1-(piperazin-1-ylmethyl)cyclopropyl)methoxy) pyrido[4,3-d]pyrimidin-4-yl)-3,8-diazabicyclo[3.2.1]octane-8-carboxylate

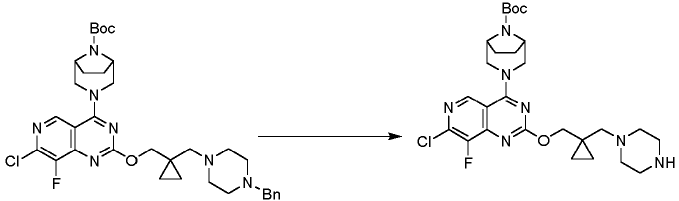

*tert*-Butyl 3-(2-((1-((4-benzylpiperazin-1-yl)methyl)cyclopropyl)methoxy)-7-chloro-8-fluoropyrido[4,3-*d*]pyrimidin-4-yl)-3,8-diazabicyclo[3.2.1]octane-8-carboxylate (1.5 g, 2.4 mmol) was dissolved in DCM (15 mL), and 1-chloroethyl chloroformate (1.03 g, 7.2 mmol) and DIEA (0.62 g, 4.8 mmol) were added. The mixture was reacted at room temperature for 30 min and concentrated under reduced pressure. The concentrate was dissolved in MeOH (15 mL), and the mixture was heated to 50 °C and reacted for 30 min. After the reaction was completed, the mixture was concentrated and purified by silica gel column chromatography (DCM:MeOH = 100:1 to 10:1) to give *tert*-butyl 3-(7-chloro-8-fluoro-2-((1-(piperazin-1-ylmethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M/2+H]⁺ = 562.2.

### Step 4: preparation of tert-butyl 3-(8-fluoro-7-(7-fluoro-3-(methoxymethoxy)-8-((triisopropylsilyl)ethynyl) naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate

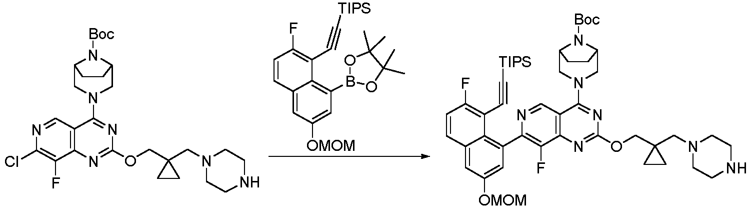

cataCXium A Pd-G3 (262 mg, 0.36 mmol) and cesium carbonate (1.74 g, 5.34 mmol) were added to a mixed solution of *tert*-butyl 3-(7-chloro-8-fluoro-2-((1-(piperazin-1-ylmethyl)cyclopropyl)methoxy)pyrido[4,3*-d*]pyrimidin-4-yl)-3,8-diazabicyclo[3.2.1]octane-8-carboxylate (1.00 g, 1.78 mmol) and ((2-fluoro-6-((methoxymethyl)oxy)-8-(4,4,5,5-tetramethyl-1,3,2-dioxaborolan-2-yl)naphthalen-1-yl)ethynyl)triisopropylsilane (1.1 g, 2.14 mmol) in 1,4-dioxane (9 mL) and H₂O (1 mL). The mixture was reacted at 80 °C for 16 h under nitrogen atmosphere. Water (100 mL) was added to the reaction liquid, and the mixture was extracted with ethyl acetate (100 mL × 3). The organic phases were combined, dried over anhydrous sodium sulfate, and concentrated under reduced pressure. The concentrate was purified by column chromatography (DCM:MeOH = 100:1 to 7:1) to give *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 912.4.

¹H NMR (400 MHz, DMSO-*d*₆) δ 9.17 (s, 1H), 8.11 (dd, *J* = 9.2 Hz, 6.0 Hz, 1H), 7.75 (d, *J* = 2.4 Hz, 1H), 7.59-7.53 (m, 1H), 7.33 (d, *J* = 2.4 Hz, 1H), 5.37 (s, 2H), 4.77-4.70 (d, *J* = 12 Hz, 1H), 4.40-4.15 (m, 6H), 3.73 (d, *J* = 11.6 Hz, 1H), 3.43 (s, 1H), 3.38-3.32 (m, 2H), 2.95-2.85 (m, 4H), 2.52-2.51 (m, 2H), 2.47-2.29 (m, 2H), 1.96-1.77 (m, 3H), 1.74-1.57 (m, 2H), 1.46 (s, 9H), 0.81 (t, *J* = 7.2 Hz, 18H), 0.69-0.60 (m, 2H), 0.53-0.37 (m, 5H).

### Intermediate 2: tert-butyl 3-(2-(((S)-1-((benzyloxy)carbonyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

### Step 1: preparation of 2,4,7-trichloro-8-fluoropyrido[4,3-d]pyrimidine

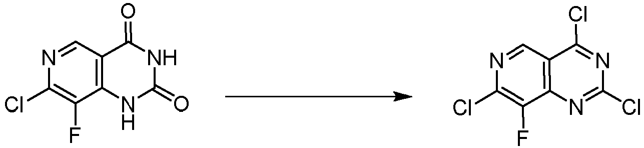

7-Chloro-8-fluoro-1,2,3,4-tetrahydropyrido[4,3-*d*]pyrimidine-2,4-dione (9.30 g, 43.14 mol) was dissolved in POCl₃ (100 mL). The mixture was cooled to 0 °C, and DIEA (16.69 g, 129.42 mol) was slowly added dropwise with stirring. The reaction liquid was heated to 120 °C and reacted for 16 h. After the reaction was completed, the reaction liquid was concentrated, and the concentrate was diluted with ethyl acetate. The mixture was slowly poured into ice water to quench the reaction and extracted with ethyl acetate (100 mL × 3), and the organic phase was separated. The organic phase was sequentially washed with a cooled aqueous NaHCO₃ solution and a saturated NaCl solution, dried over anhydrous Na₂SO₄, filtered, and concentrated under reduced pressure to give 2,4,7-trichloro-8-fluoropyrido[4,3-*d*]pyrimidine.

### Step 2: preparation of tert-butyl 3-(2,7-dichloro-8-fluoropyrido[4,3-d]pyrimidin-4-yl)-3,8-diazabicyclo[3.2.1] octane-8-carboxylate

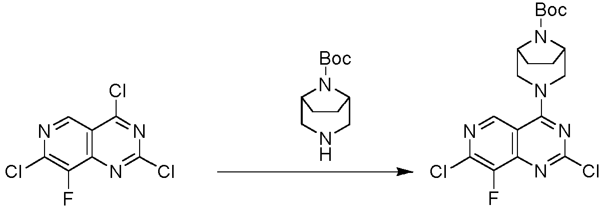

2,4,7-Trichloro-8-fluoropyrido[4,3-*d*]pyrimidine (1.00 g, 3.97 mmol) and TEA (1.20 g, 11.90 mmol) were dissolved in THF (15 mL), and the mixture was cooled to -60 °C. A solution of *tert*-butyl 3,8-diazabicyclo[3.2.1]octane-8-carboxylate (0.71 g, 3.37 mmol) in THF (5 mL) was slowly added dropwise, and the mixture was reacted at -60 °C for 30 min. After the reaction was completed, the reaction liquid was poured into an aqueous NH₄Cl solution (20 mL), and the mixture was extracted with ethyl acetate (30 mL × 3). The organic phases were combined, washed with a saturated NaCl solution, dried over anhydrous Na₂SO₄, and filtered. The filtrate was concentrated to give a crude product, which was purified by silica gel column chromatography (PE:EtOAc = 3:1) to give *tert*-butyl 3-(2,7-dichloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 428.2.

### Step 3: preparation of tert-butyl 3-(2-(((S)-1-((benzyloxy)carbonyl)pyrrolidin-2-yl)methoxy)-7-chloro-8-fluoropyrido[4,3-d]pyrimidin-4-yl)-3,8-diazabicyclo[3.2.1]octane-8-carboxylate

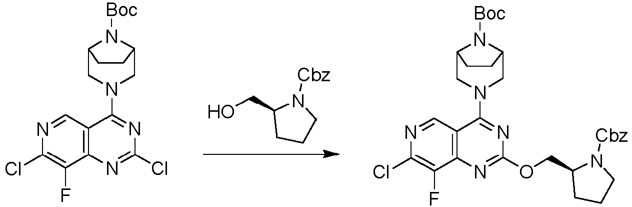

*tert*-Butyl 3-(2,7-dichloro-8-fluoropyrido[4,3*-d*]pyrimidin-4-yl)-3,8-diazabicyclo[3.2.1]octane-8-carboxylate (1.20 g, 3.27 mmol) and benzyl (S)-2-(hydroxymethyl)pyrrolidine-1-carboxylate (1.15 g, 4.91 mmol) were dissolved in THF (25 mL), and Cs₂CO₃ (2.13 g, 6.54 mmol) was added. The reaction liquid was heated to 85 °C and reacted for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered. The filtrate was concentrated to give a crude product, which was purified by silica gel column chromatography (DCM:MeOH = 100:1) to give *tert*-butyl 3-(2-(((5)-1-((benzyloxy)carbonyl)pyrrolidin-2-yl)methoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 627.2.

### Step 4: preparation of tert-butyl 3-(2-(((S)-1-((benzyloxy)carbonyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

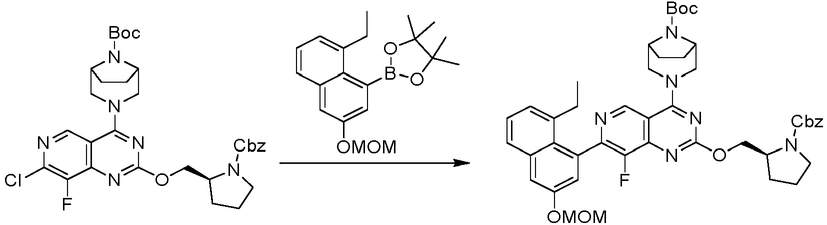

*tert*-Butyl 3-(2-(((*S*)-1-((benzyloxy)carbonyl)pyrrolidin-2-yl)methoxy)-7-chloro-8-fluoropyrido[4,3-*d*]pyrimidin-4-yl)-3,8-diazabicyclo[3.2.1]octane-8-carboxylate (540 mg, 0.86 mmol), 2-(8-ethyl-3-((methoxymethyl)oxy)naphthalen-1-yl)-4,4,5,5-tetramethyl-1,3,2-dioxaborolane (353 mg, 1.03 mmol) and potassium phosphate (364 mg, 1.72 mmol) were added to dioxane/H₂O (10 mL/2 mL), and the catalyst cataCXium A Pd-G3 (62 mg, 0.086 mmol) was added. The reaction liquid was mixed uniformly, purged with nitrogen three times, heated to 100 °C, and reacted for 16 h. After the reaction was completed, the reaction liquid was concentrated and purified by silica gel column chromatography (DCM:MeOH = 80:1) to give *tert*-butyl 3-(2-(((*S*)-1-((benzyloxy)carbonyl)pyrrolidin-2-yl)methoxy)-7-(8-ethyl-3-(methoxymethoxy) naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 806.9.

### Intermediate 3: 3-(4-chloro-3-(2,4-dioxotetrahydropyrimidin-1(2H)-yl)benzoyl)-3-azaspiro [5 .5]undecane-9-carbaldehyde

### Step 1: preparation of 3-azaspiro[5.5]undecane-9-carbaldehyde

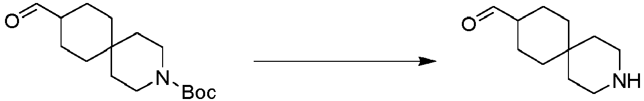

*tert*-Butyl 9-formyl-3-azaspiro[5.5]undecane-3-carboxylate (400 mg, 1.16 mmol) was dissolved in DCM (4 mL), and then TFA (2 mL) was added. The mixture was stirred at room temperature for 1 h and concentrated to give 3-azaspiro[5.5]undecane-9-carbaldehyde, which was directly used in the next step.

### Step 2: preparation of 3-(4-chloro-3-(2,4-dioxotetrahydropyrimidin-1(2H)-yl)benzoyl)-3-azaspiro[5.5] undecane-9-carbaldehyde

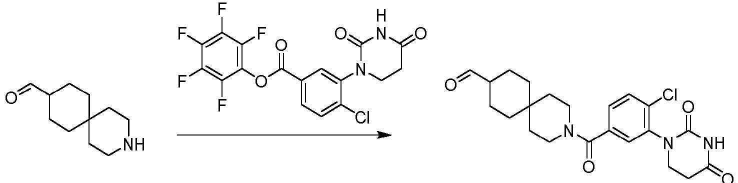

DIEA (720 mg, 5.61 mmol) and pentafluorophenyl 4-chloro-3-(2,4-dioxotetrahydropyrimidin-1(2H)-yl)benzoate (850 mg, 1.96 mmol) were added to a solution of 3-azaspiro[5.5]undecane-9-carbaldehyde (500 mg, 1.87 mmol) in DMSO (5 mL), and the reaction liquid was stirred at room temperature for 1 h. Water was added, and the mixture was extracted with ethyl acetate. The organic phases were combined, dried over anhydrous sodium sulfate, and filtered. The filtrate was concentrated, and the resulting crude product was purified by silica gel column chromatography (DCM:MeOH = 100:1 to 5:1) to give 3-(4-chloro-3-(2,4-dioxotetrahydropyrimidin-1(2*H*)-yl)benzoyl)-3-azaspiro[5.5]undecane-9-carbaldehyde.

LC-MS: (ESI, m/z): [M+H]⁺ = 432.0.

### Intermediate 4: tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

### Step 1: preparation of tert-butyl 9-((4-((benzyloxy)carbonyl)piperazin-1-yl)methyl)-3-azaspiro[5.5]undecane-3-carboxylate

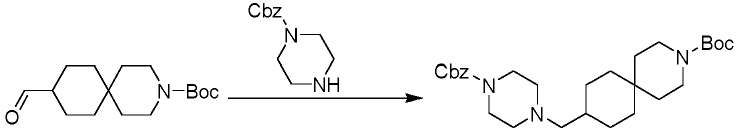

AcOH (0.2 mL) was added to a mixed solution of *tert-butyl* 9-formyl-3-azaspiro[5.5]undecane-3-carboxylate (1.30 g, 4.62 mmol) and *tert*-butyl piperazine-1-carboxylate (1.82 g, 8.28 mmol) in DCM/MeOH (15 mL/5 mL), and the mixture was stirred at room temperature for 0.5 h. NaBH(OAc)₃ (2.25 g, 10.60 mmol) was then added in batches to the above mixture at 0-5 °C, and the resulting mixture was stirred for another 0.5 h. The reaction liquid was directly concentrated under reduced pressure, and the resulting crude product was purified by silica gel column chromatography (DCM:MeOH = 150:1 to 20:1) to give *tert*-butyl 9-((4-((benzyloxy)carbonyl)piperazin-1-yl)methyl)-3-azaspiro[5.5]undecane-3-carboxylate.

LC-MS: (ESI, m/z): [M+H]⁺ = 486.3.

### Step 2: preparation of benzyl 4-((3-azaspiro[5.5]undecan-9-yl)methyl)piperazine-1-carboxylate

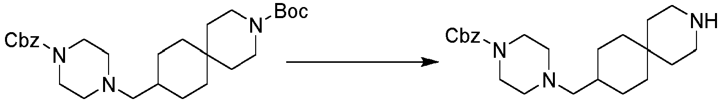

HCl/1,4-dioxane (6 N, 5 mL) was added to a solution of *tert*-butyl 9-((4-((benzyloxy)carbonyl)piperazin-1-yl)methyl)-3-azaspiro[5.5]undecane-3-carboxylate (750 mg, 4.54 mmol) in 1,4-dioxane (5 mL). The mixture was stirred at room temperature for 1 h, and the reaction liquid was directly concentrated under reduced pressure to give benzyl 4-((3-azaspiro[5.5]undecan-9-yl)methyl)piperazine-1-carboxylate. The resulting crude product was directly used in the next step without purification.

### Step 3: preparation of benzyl 4-((3-(3-(2,4-dioxotetrahydropyrimidin-1(2H)-yl)-4-methoxybenzoyl)-3-azaspiro[5.5]undecan-9-yl)methyl)piperazine-1-carboxylate

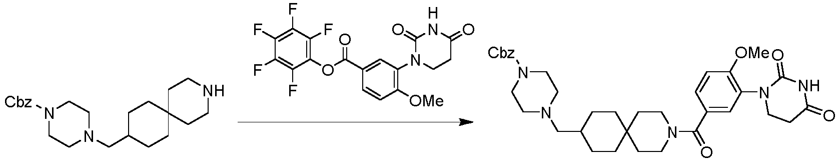

Pentafluorophenyl 3-(2,4-dioxotetrahydropyrimidin-1(2*H*)-yl)-4-methoxybenzoate (430 mg, 1.00 mmol) and DIEA (516 mg, 4.00 mmol) were added to a solution of benzyl 4-((3-azaspiro[5.5]undecan-9-yl)methyl)piperazine-1-carboxylate (632 mg, crude) in DMSO (10 mL). The mixture was stirred at room temperature for 1 h and then purified by pre-HPLC to give benzyl 4-((3-(3-(2,4-dioxotetrahydropyrimidin-1(2*H*)-yl)-4-methoxybenzoyl)-3-azaspiro[5.5]undecan-9-yl)methyl)piperazine-1-carboxylate.

LC-MS: (ESI, m/z): [M+H]⁺ = 632.3.

### Step 4: preparation of 1-(2-methoxy-5-(9-(piperazin-1-ylmethyl)-3-azaspiro[5.5]undecane-3-carbonyl) phenyl)dihydropyrimidine-2,4(1H,3H)-dione

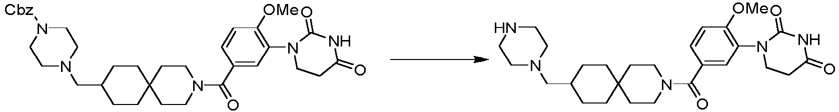

Pd/C (300 mg) was added to a solution of benzyl 4-((3-(3-(2,4-dioxotetrahydropyrimidin-1(2*H*)-yl)-4-methoxybenzoyl)-3-azaspiro[5.5]undecan-9-yl)methyl)piperazine-1-carboxylate (586 mg, 0.93 mmol) in ethyl acetate (20 mL), and the mixture was stirred at room temperature for 16 h. The reaction liquid was filtered through celite, and the filtrate was concentrated under reduced pressure to give 1-(2-methoxy-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 498.2.

### Step 5: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

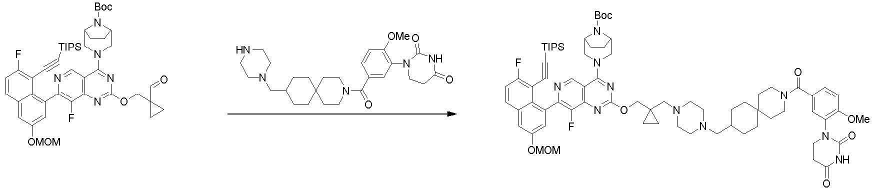

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (68 mg, 0.17 mmol) and tetraisopropyl titanate (485 mg, 1.71 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.114 mmol) in THF (0.8 mL), and the mixture was stirred at 70 °C for 2 h. At room temperature, MeOH (0.3 mL) was first added to the mixture, and then NaBH(OAc)₃ (72 mg, 0.34 mmol) was added in batches. The mixture was stirred for 4 h. The reaction liquid was directly concentrated under reduced pressure, and the resulting crude product was purified by silica gel column chromatography (DCM:MeOH = 100:1 to 20:1) to give *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 662.4.

### Step 6: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

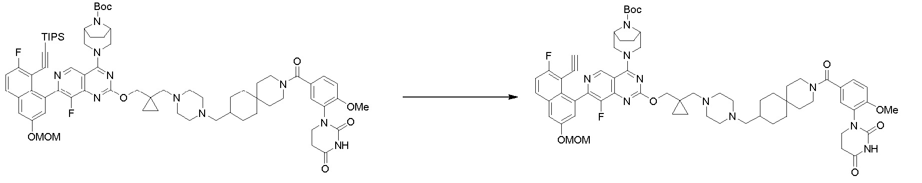

Cesium fluoride (40 mg, 0.27 mmol) was added to a solution of *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate (100 mg, 0.0755 mmol) in DMF (3 mL), and the mixture was stirred at room temperature for 30 min. Water was added to the above mixture, and the resulting mixture was extracted with ethyl acetate. The organic phase was dried over anhydrous sodium sulfate, and the filtrate was concentrated under reduced pressure to give *tert-butyl* 3-(2-((1-((4-((3-(3-(2,4-dioxotetrahydropyrimidin-1(2*H*)-yl)-4-methoxybenzoyl)-3-azaspiro[5.5]undecan-9-yl)methyl) piperazin-1-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1167.3.

### Intermediate 5: tert-butyl 3-(8-fluoro-2-((1-(hydroxymethyl)cyclopropyl)methoxy)-7-(5-methyl-1H-indazol-4-yl)pyrido[4,3-d]pyrimidin-4-yl)-3,8-diazabicyclo[3.2.1]octane-8-carboxylate

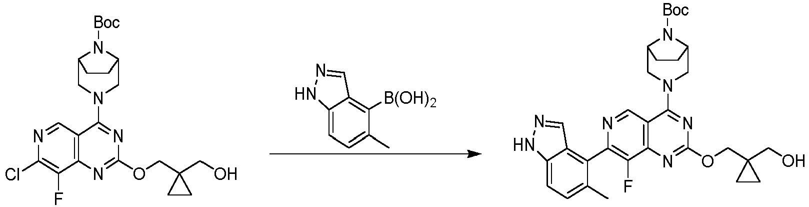

(5-Methyl-1*H*-indazol-4-yl)boronic acid (257 mg, 1.46 mmol), cesium carbonate (792 mg, 2.43 mmol), and 1,1"-bis(di-*tert*-butylphosphine)ferrocene palladium dichloride (59 mg, 0.08 mmol) were added to a solution of *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (400 mg, 0.81 mmol) in 1,4-dioxane/H₂O (5.0 mL/1.0 mL), and the mixture was reacted at 75 °C overnight under N₂ atmosphere. The reaction mixture was filtered, the filtrate was concentrated under reduced pressure, and the resulting crude product was purified by silica gel column chromatography (MeOH:DCM = 100:1 to 20:1) to give *tert*-butyl 3-(8-fluoro-2-((1-(hydroxymethyl)cyclopropyl)methoxy)-7-(5-methyl-1*H*-indazol-4-yl)pyrido[4,3-*d*]pyrimidin-4-yl)-3,8-diazabicyclo[3.2.1]octane-8-carboxylate.

LC-MS: (ESI, m/z): [M+H]⁺ = 590.2.

### Intermediate 6: tert-butyl 3-(7-(8-ethyl-3-(methoxymethoxy)naphthalen-1-yl)-8-fluoro-2-((1-formylcyclopropyl) methoxy)pyrido[4,3-d]pyrimidin-4-yl)-3,8-diazabicyclo[3.2.1]octane-8-carboxylate

### Step 1: preparation of tert-butyl 3-(7-(8-ethyl-3-(methoxymethoxy)naphthalen-1-yl)-8-fluoro-2-((1-(hydroxymethyl)cyclopropyl)methoxy)pyrido[4,3-d]pyrimidin-4-yl)-3,8-diazabicyclo[3.2.1]octane-8-carboxylate

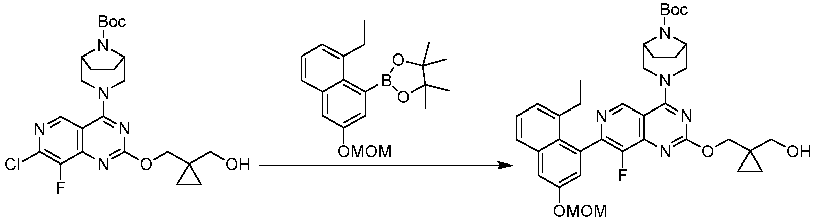

cataCXium A Pd-G3 (146 mg, 0.2 mmol) and cesium carbonate (981 mg, 3.0 mmol) were added to a mixed solution of *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (500 mg, 1.0 mmol) and 2-(8-ethyl-3-(methoxymethoxy)naphthalen-1-yl)-4,4,5,5-tetramethyl-1,3,2-dioxaborolane (411 mg, 1.2 mmol) in 1,4-dioxane/H₂O (5 mL/1 mL). The mixture was reacted at 85 °C for 16 h under nitrogen atmosphere. Water (10 mL) was added to the reaction liquid, and the mixture was extracted with ethyl acetate (10 mL × 3). The organic phases were combined, dried over anhydrous sodium sulfate, and filtered, and the filtrate was concentrated under reduced pressure. The resulting crude product was purified by silica gel column chromatography (DCM:MeOH = 50:1) to give *tert*-butyl 3-(7-(8-ethyl-3-(methoxymethoxy)naphthalen-1-yl)-8-fluoro-2-((1-(hydroxymethyl)cyclopropyl)methoxy)pyrido [4,3-*d*]pyrimidin-4-yl)-3,8-diazabicyclo[3.2.1]octane-8-carboxylate.

LC-MS: (ESI, m/z): [M/2+H]⁺ = 674.2.

### Step 2: preparation of tert-butyl 3-(7-(8-ethyl-3-(methoxymethoxy)naphthalen-1-yl)-8-fluoro-2-((1-formylcyclopropyl)methoxy)pyrido[4,3-d]pyrimidin-4-yl)-3,8-diazabicyclo[3.2.1]octane-8-carboxylate

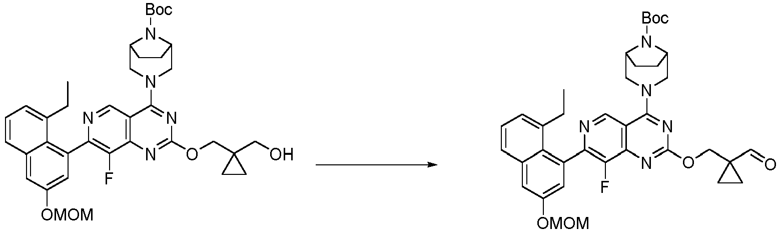

Dess-Martin oxidant (242 g, 0.57 mmol) was added to a solution of *tert*-butyl 3-(7-(8-ethyl-3-(methoxymethoxy)naphthalen-1-yl)-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (320 mg, 0.47 mmol) in DCM (5 mL) at 0 °C, and the mixture was reacted at room temperature for 1 h. A sodium thiosulfate solution (5 mL) and a sodium bicarbonate solution (5 mL) were added to the reaction liquid to quench the reaction, and the mixture was extracted with dichloromethane (5 mL × 3). The organic phases were combined, dried over anhydrous sodium sulfate, and filtered, and the filtrate was concentrated under reduced pressure. The concentrate was purified by silica gel column chromatography (DCM:MeOH = 30:1) to give *tert*-butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate.

LC-MS: (ESI, m/z): [M/2+H]⁺ = 672.3.

### Intermediate 7: tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

### Step 1: preparation of benzyl 4-((3-(3-(2,4-dioxotetrahydropyrimidin-1(2H)-yl)-4-methylbenzoyl)-3-azaspiro[5 .5]undecan-9-yl)methyl)piperazine-1-carboxylate

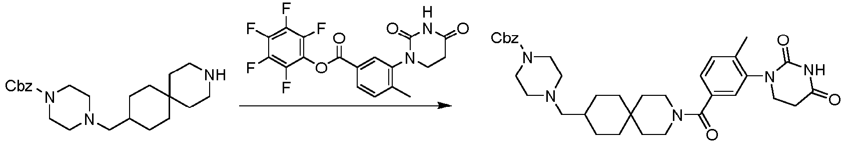

Pentafluorophenyl 3-(2,4-dioxotetrahydropyrimidin-1(2*H*)-yl)-4-methylbenzoate (921 mg, 2.3 mmol) and DIEA (995 mg, 7.7 mmol) were added to a solution of benzyl 4-((3-azaspiro[5.5]undecan-9-yl)methyl)piperazine-1-carboxylate hydrochloride (930 mg, 2.2 mmol) in DMSO (20 mL). The mixture was stirred at room temperature for 1 h and purified by Pre-HPLC to give benzyl 4-((3-(3-(2,4-dioxotetrahydropyrimidin-1(2*H*)-yl)-4-methylbenzoyl)-3-azaspiro[5.5]undecan-9-yl)methyl)piperazine-1-carboxylate.

LC-MS: (ESI, m/z): [M+H]⁺ = 616.3.

### Step 2: preparation of 1-(2-methyl-5-(9-(piperazin-1-ylmethyl)-3-azaspiro[5.5]undecane-3-carbonyl)phenyl) dihydropyrimidine-2,4(1H,3H)-dione

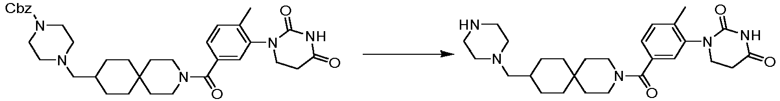

Pd/C (100 mg) was added to a solution of benzyl 4-((3-(3-(2,4-dioxotetrahydropyrimidin-1(2H)-yl)-4-methylbenzoyl)-3-azaspiro[5.5]undecan-9-yl)methyl)piperazine-1-carboxylate (900 mg, 1.46 mmol) in ethyl acetate (20 mL), and the mixture was stirred at room temperature for 16 h. The reaction liquid was filtered through celite, and the filtrate was concentrated to give 1-(2-methyl-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 482.2.

### Step 3: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

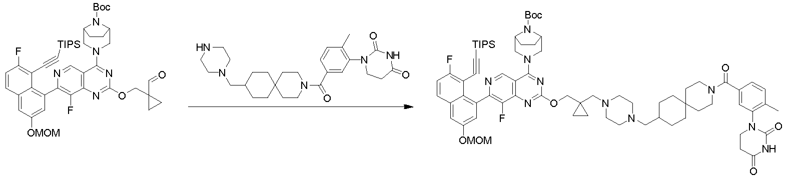

1-(2-Methyl-5-(9-(piperazin-1-ylmethyl)-3-azaspiro[5.5]undecane-3-carbonyl)phenyl)dihydropyrimidine-2,4(1*H*,3*H*)-dione (66 mg, 0.17 mmol) and tetraisopropyl titanate (485 mg, 1.71 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido [4,3-*d*]pyrimidin-4-yl)-3,8-diazabicyclo[3.2.1]octane-8-carboxylate (100 mg, 0.114 mmol) in THF (5 mL), and the mixture was stirred at 70 °C for 2 h. At room temperature, MeOH (5 mL) was first added to the mixture, and then NaBH(OAc)₃ (72 mg, 0.34 mmol) was added in batches. The mixture was stirred for 2 h. The mixture was concentrated, and the resulting crude product was purified by silica gel column chromatography (DCM:MeOH = 100:1 to 20:1) to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate.

LC-MS: (ESI, m/z): [M/2+H]⁺ = 654.9.

### Step 4: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

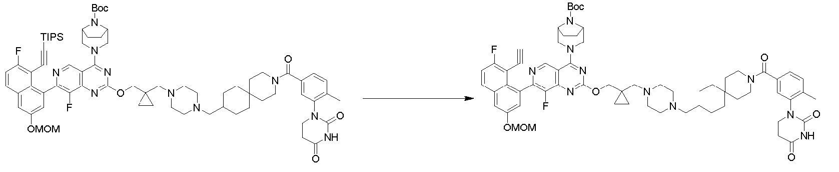

Cesium fluoride (40 mg, 0.27 mmol) was added to a solution of *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-y1)-3,8-diazabicyclo[3.2.1]octane-8-carboxylate (100 mg, 0.076 mmol) in DMF (3 mL), and the mixture was stirred at room temperature for 30 min. Water was added, and the mixture was extracted with ethyl acetate. The organic phase was dried over anhydrous sodium sulfate and concentrated to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1151.5.

### Intermediate 8: tert-butyl 3-(8-fluoro-7-(7-fluoro-3-(methoxymethoxy)-8-((triisopropylsilyl)ethynyl) naphthalen-1-yl)-2-(((1-formylcyclopropyl)methyl)amino)pyrido[4,3-d]pyrimidin-4-yl)-3,8-diazabicyclo [3.2.1]octane-8-carboxylate

### Step 1: preparation of tert-butyl 3-(7-chloro-8-fluoro-2-(((1-(hydroxymethyl)cyclopropyl)methyl)amino) pyrido[4,3-d]pyrimidin-4-yl)-3,8-diazabicyclo[3.2.1]octane-8-carboxylate

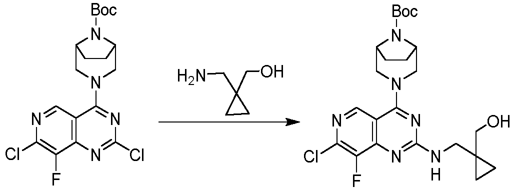

*tert*-Butyl 3-(2,7-dichloro-8-fluoropyrido[4,3-*d*]pyrimidin-4-yl)-3,8-diazabicyclo[3.2.1]octane-8-carboxylate (0.5 g, 1.17 mmol) and 1-(aminomethyl)cyclopropyl]methanol (0.16 g, 1.52 mmol) were added to THF (8 mL), and then cesium carbonate (0.76 g, 2.34 mmol) was added. The mixture was reacted at 25 °C for 16 h. After the reaction was completed, water (30 mL) was added, and the mixture was extracted with ethyl acetate (30 mL × 2). The organic phases were combined, dried over anhydrous sodium sulfate, and concentrated under reduced pressure. The concentrate was purified by silica gel column chromatography (PE:EA = 5:1) to give *tert*-butyl 3-(7-chloro-8-fluoro-2-(((1-(hydroxymethyl)cyclopropyl)methyl)amino)pyrido[4,3-*d*]pyrimidin-4-yl)-3,8-diazabicyclo[3.2.1]octane-8-carboxylate.

LC-MS: (ESI, m/z): [M+H]⁺ = 493.1.

¹H NMR (300 MHz, DMSO-*d*₆) δ 8.65 (d, *J* = 8.6 Hz, 1H), 7.89 - 7.57 (m, 1H), 4.78 (t, *J* = 6.0 Hz, 1H), 4.54 - 4.35 (m, 2H), 4.23 (s, 2H), 3.58 - 3.43 (m, 3H), 3.42 - 3.37 (m, 1H), 3.33 - 3.28 (m, 1H), 1.87 -1.65 (m, 4H), 1.49 (s, 9H), 0.52 - 0.35 (m, 4H).

### Step 2: preparation of tert-butyl 3-(8-fluoro-7-(7-fluoro-3-(methoxymethoxy)-8-((triisopropylsilyl)ethynyl) naphthalen-1-yl)-2-(((1-(hydroxymethyl)cyclopropyl)methyl)amino)pyrido[4,3-d]pyrimidin-4-yl)-3,8-diazabicyclo[3.2.1]octane-8-carboxylate

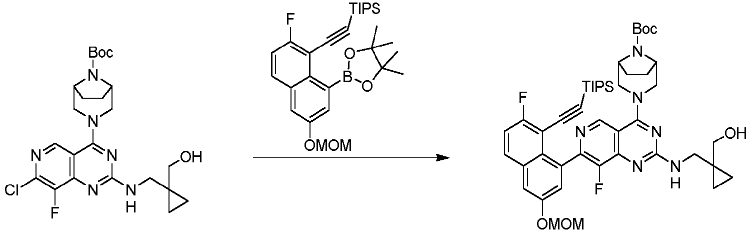

cataCXium A Pd G3 (60 mg, 0.081 mmol) and cesium carbonate (660 mg, 2.03 mmol) were added to a mixed solution of *tert*-butyl 3-(7-chloro-8-fluoro-2-(((1-(hydroxymethyl)cyclopropyl)methyl)amino)pyrido[4,3-*d*]pyrimidin-4-yl)-3,8-diazabicyclo[3.2.1]octane-8-carboxylate (400 mg, 0.81 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (540 mg, 1.05 mmol) in 1,4-dioxane (15 mL) and H₂O (3 mL). The mixture was reacted at 85 °C for 6 h under nitrogen atmosphere. The reaction liquid was directly filtered and concentrated under reduced pressure. The concentrate was purified by silica gel column chromatography (DCM:MeOH = 100:1) to give *tert*-butyl 3-(8-fluoro-7-(7-fluoro-3-(methoxymethoxy)-8-((triisopropylsilyl)ethynyl)naphthalen-1-yl)-2-(((1-(hydroxymethyl)cyclopropyl)methyl)amino)pyrido[4,3-*d*]pyrimidin-4-yl)-3,8-diazabicyclo [3.2.1]octane-8-carboxylate.

LC-MS: (ESI, m/z): [M+H]⁺ = 843.4.

### Step 3: preparation of tert-butyl 3-(8-fluoro-7-(7-fluoro-3-(methoxymethoxy)-8-((triisopropylsilyl)ethynyl) naphthalen-1-yl)-2-(((1-formylcyclopropyl)methyl)amino)pyrido[4,3-d]pyrimidin-4-yl)-3,8-diazabicyclo [3.2.1]octane-8-carboxylate

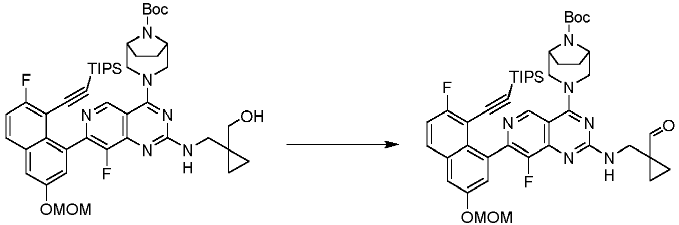

Dess-Martin reagent (122 mg, 0.29 mmol) was added to a solution of *tert*-butyl 3-(8-fluoro-7-(7-fluoro-3-(methoxymethoxy)-8-((triisopropylsilyl)ethynyl)naphthalen-1-yl)-2-(((1-(hydroxymethyl)cyclopropyl)methyl) amino)pyrido[4,3-*d*]pyrimidin-4-yl)-3,8-diazabicyclo[3.2.1]octane-8-carboxylate (200 mg, 0.24 mmol) in dichloromethane (1 mL). The mixture was stirred at room temperature for 2 h. After the reaction was completed, a sodium thiosulfate solution (3 mL) and a sodium bicarbonate solution (3 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (PE:EA = 1:1) to give *tert*-butyl 3-(8-fluoro-7-(7-fluoro-3-(methoxymethoxy)-8-((triisopropylsilyl)ethynyl)naphthalen-1-yl)-2-(((1-formylcyclopropyl)methyl)amino) pyrido[4,3-*d*]pyrimidin-4-yl)-3,8-diazabicyclo[3.2.1]octane-8-carboxylate.

LC-MS: (ESI, m/z): [M+H]⁺ = 841.4.

### Intermediate 9: tert-butyl 3-(8-fluoro-7-(7-fluoro-3-(methoxymethoxy)-8-((triisopropylsilyl)ethynyl) naphthalen-1-yl)-2-(piperidin-4-ylmethoxy)pyrido[4,3-d]pyrimidin-4-yl)-3,8-diazabicyclo[3.2.1]octane-8-carboxylate

### Step 1: preparation of tert-butyl 3-(2-((1-benzylpiperidin-4-yl)methoxy)-7-chloro-8-fluoropyrido[4,3-d]pyrimidin-4-yl)-3,8-diazabicyclo[3.2.1]octane-8-carboxylate

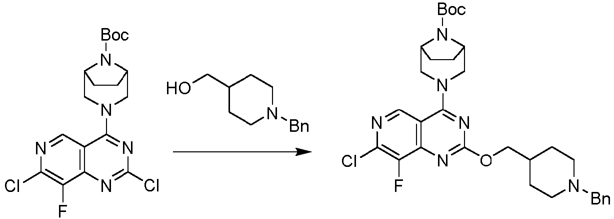

*tert*-Butyl 3-(2,7-dichloro-8-fluoropyrido[4,3-*d*]pyrimidin-4-yl)-3,8-diazabicyclo[3.2.1]octane-8-carboxylate (1.00 g, 2.33 mmol) and (1-benzylpiperidin-4-yl)methanol (0.96 g, 2.67 mmol) were dissolved in THF (10 mL), and Cs₂CO₃ (2.28 g, 7.00 mmol) was added. The reaction liquid was heated to 90 °C and reacted for 16 h. After the reaction was completed, the mixture was cooled to room temperature. Water (50 mL) was added, and the mixture was extracted with ethyl acetate (30 mL × 3). The organic phases were combined, washed with a saturated NaCl solution, dried over anhydrous Na₂SO₄, and filtered, and the filtrate was concentrated to give a crude product, which was purified by silica gel column chromatography (PE:EA = 3:1) to give *tert*-butyl 3-(2-((1-benzylpiperidin-4-yl)methoxy)-7-chloro-8-fluoropyrido[4,3-d]pyrimidin-4-yl)-3,8-diazabicyclo
[3.2.1]octane-8-carboxylate.

LC-MS: (ESI, m/z): [M+H]⁺ = 597.3.

¹H NMR (400 MHz, CDCl₃) δ 8.71 (s, 1H), 7.36 - 7.26 (m, 5H), 4.53 - 4.25 (m, 6H), 3.77 - 3.57 (m, 2H), 3.51 (s, 2H), 2.95 (d, *J* = 11.2 Hz, 2H), 2.05 - 1.93 (m, 4H), 1.86 - 1.80 (m, 2H), 1.73 - 1.68 (m, 2H), 1.51 (s, 9H), 1.47 - 1.37 (m, 2H), 1.28 - 1.22 (m, 1H).

### Step 2: preparation of tert-butyl 3-(7-chloro-8-fluoro-2-(piperidin-4-ylmethoxy)pyrido[4,3-d]pyrimidin-4-yl)-3,8-diazabicyclo[3.2.1]octane-8-carboxylate

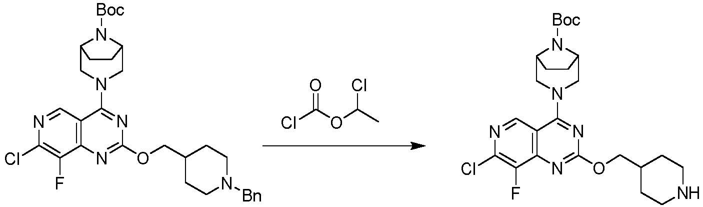

*tert*-Butyl 3-(2-((1-benzylpiperidin-4-yl)methoxy)-7-chloro-8-fluoropyrido[4,3-*d*]pyrimidin-4-yl)-3,8-diazabicyclo[3.2.1]octane-8-carboxylate (1.02 g, 1.71 mmol) was dissolved in DCM (10 mL), and 1-chloroethyl chloroformate (733 mg, 5.12 mmol) and DIEA (441 mg, 3.42 mmol) were added. The mixture was reacted at room temperature for 1 h and concentrated under reduced pressure. MeOH (3 mL) was added to the concentrate for dissolution, and the mixture was heated to 50 °C and reacted for 10 min. After the reaction was completed, the mixture was concentrated under reduced pressure. The concentrate was purified by silica gel column chromatography (DCM:MeOH (NH₃) = 7:93) to give *tert*-butyl 3-(7-chloro-8-fluoro-2-(piperidin-4-ylmethoxy)pyrido[4,3-*d*]pyrimidin-4-yl)-3,8-diazabicyclo[3.2.1]octane-8-carboxylate.

LC-MS: (ESI, m/z): [M+H]⁺ = 507.0.

¹H NMR (400 MHz, CD₃OD) δ 8.86 (s, 1H), 4.61 (d, *J* = 12.4 Hz, 2H), 4.42 - 4.32 (m, 4H), 3.72 (d, *J=* 12.4 Hz, 2H), 3.41 (d, *J* = 12.4 Hz, 2H), 3.03 = 2.90 (m, 2H), 2.19 - 2.16 (m, 1H), 2.06 (d, *J* = 13.6 Hz, 2H),1.97 - 1.89 (m, 2H), 1.78 - 1.71 (m, 2H), 1.67 - 1.57 (m, 2H), 1.53 (s, 9H), 1.54 - 1.24 (m, 1H).

### Step 3: preparation of tert-butyl 3-(8-fluoro-7-(7-fluoro-3-(methoxymethoxy)-8-((triisopropylsilyl)ethynyl) naphthalen-1-yl)-2-(piperidin-4-ylmethoxy)pyrido[4,3-d]pyrimidin-4-yl)-3,8-diazabicyclo[3.2.1]octane-8-carboxylate

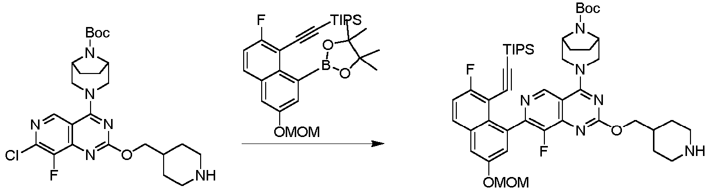

cataCXium A Pd G3 (58 mg, 0.08 mmol) and cesium carbonate (771 mg, 2.37 mmol) were added to a mixed solution of *tert*-butyl 3-(7-chloro-8-fluoro-2-(piperidin-4-ylmethoxy)pyrido[4,3-*d*]pyrimidin-4-yl)-3,8-diazabicyclo[3.2.1]octane-8-carboxylate (400 mg, 0.79 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (485 mg, 0.95 mmol) in 1,4-dioxane (5 mL) and H₂O (1 mL). The mixture was reacted at 85 °C for 16 h under nitrogen atmosphere. Water (10 mL) was added to quench the reaction, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH (NH₃) = 7%) to give *tert*-butyl 3-(8-fluoro-7-(7-fluoro-3-(methoxymethoxy)-8-((triisopropylsilyl)ethynyl)naphthalen-1-yl)-2-(piperidin-4-ylmethoxy)pyrido[4,3-*d*]pyrimidin-4-yl)-3,8-diazabicyclo[3.2.1]octane-8-carboxylate.

LC-MS: (ESI, m/z): [M/2+H]⁺ = 429.3.

### Intermediate 10: tert-butyl 3-(8-fluoro-7-(7-fluoro-3-(methoxymethoxy)-8-((triisopropylsilyl)ethynyl) naphthalen-1-yl)-2-(2-(piperazin-1-yl)ethoxy)pyrido[4,3-d]pyrimidin-4-yl)-3,8-diazabicyclo[3.2.1]octane-8-carboxylate

### Step 1: preparation of 2-(4-benzylpiperazin-1-yl)ethanol

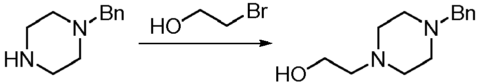

Anhydrous potassium carbonate (1180 mg, 8.510 mmol) was added to a solution of 1-benzylpiperazine (1000 mg, 5.673 mmol) and 2-bromoethan-1-ol (851 mg, 6.808 mmol) in anhydrous acetonitrile (25 mL) at room temperature, and the mixture was stirred at 90 °C for 16 h. After the reaction was completed, the mixture was filtered to remove the precipitate, and concentrated under reduced pressure at 40 °C. The resulting crude product was purified by silica gel column chromatography (dichloromethane:methanol = 100:1 to 10:1, 0.1% NH₃) to give 2-(4-benzylpiperazin-1-yl)ethanol.

LC-MS: (ESI, m/z): [M+H]⁺ = 221.2.

### Step 2: preparation of tert-butyl 3-(2-(2-(4-benzylpiperazin-1-yl)ethoxy)-7-chloro-8-fluoropyrido[4,3-d]pyrimidin-4-yl)-3,8-diazabicyclo[3.2.1]octane-8-carboxylate

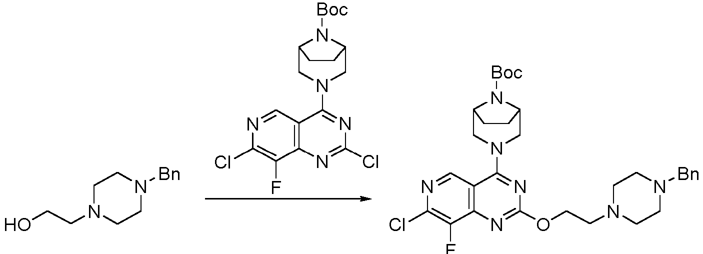

Anhydrous cesium carbonate (1217 mg, 3.736 mmol) and 1,4-diazabicyclo[2.2.2]octane (105 mg, 0.934 mmol) were added to a solution of 2-(4-benzylpiperazin-1-yl)ethanol (823 mg, 3.735 mmol) and *tert-*butyl 3-(2,7-dichloro-8-fluoropyrido[4,3-*d*]pyrimidin-4-yl)-3,8-diazabicyclo[3.2.1]octane-8-carboxylate (800 mg, 1.868 mmol) in anhydrous acetonitrile (25 mL) at room temperature, and the mixture was stirred at 25 °C for 4 h. After the reaction was completed, the mixture was diluted with tetrahydrofuran (50 mL) and filtered to remove the solid. The filtrate was concentrated under reduced pressure at 40 °C, and the resulting crude product was purified by silica gel column chromatography (petroleum ether:tetrahydrofuran = 10:1 to 2:1) to give *tert*-butyl 3-(2-(2-(4-benzylpiperazin-1-yl)ethoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 612.1.

### Step 3: preparation of tert-butyl 3-(7-chloro-8-fluoro-2-(2-(piperazin-1-yl)ethoxy)pyrido[4,3-d]pyrimidin-4-yl)-3,8-diazabicyclo[3.2.1]octane-8-carboxylate

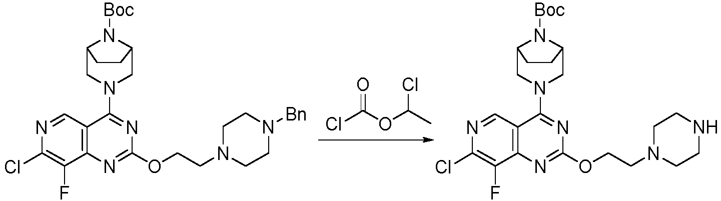

1-Chloroethyl chloroformate (1217 mg, 3.736 mmol) was added to a solution of *tert*-butyl 3-(2-(2-(4-benzylpiperazin-1-yl)ethoxy)-7-chloro-8-fluoropyrido[4,3-*d*]pyrimidin-4-yl)-3,8-diazabicyclo[3.2.1]octane-8-carboxylate (840 mg, 1.372 mmol) in dichloromethane (40 mL) at room temperature, and the mixture was stirred at 30 °C for 1 h. After the reaction was completed, the mixture was concentrated under reduced pressure at 40 °C to give *tert*-butyl 3-(7-chloro-8-fluoro-2-(2-(piperazin-1-yl)ethoxy)pyrido[4,3-*d*]pyrimidin-4-yl)-3,8-diazabicyclo[3.2.1]octane-8-carboxylate.

LC-MS: (ESI, m/z): [M+H]⁺ = 522.1.

### Step 4: preparation of tert-butyl 3-(8-fluoro-7-(7-fluoro-3-(methoxymethoxy)-8-((triisopropylsilyl)ethynyl) naphthalen-1-yl)-2-(2-(piperazin-1-yl)ethoxy)pyrido[4,3-d]pyrimidin-4-yl)-3,8-diazabicyclo[3.2.1]octane-8-carboxylate

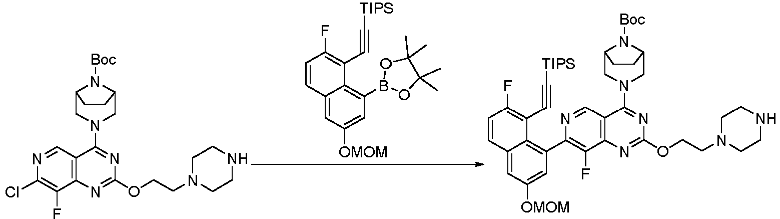

Anhydrous cesium carbonate (1608 mg, 4.938 mmol) and cataCXium(R)A Pd G3 (240 mg, 0.329 mmol) were added to a solution of *tert*-butyl 3-(7-chloro-8-fluoro-2-(2-(piperazin-1-yl)ethoxy)pyrido[4,3-*d*]pyrimidin-4-yl)-3,8-diazabicyclo[3.2.1]octane-8-carboxylate (714 mg, 1.372 mmol) and (2-fluoro-6-methoxymethoxy)-8-(4,4,5,5-tetramethyl-1,3,2-dioxaborolan-2-yl)naphthalen-1-yl)ethynyl)triisopropylsilane (845 mg, 1.646 mmol) in 1,4-dioxane (20 mL) and water (4 mL) at room temperature. After the system was degassed, the mixture was stirred at 85 °C for 16 h under nitrogen atmosphere. After the reaction was completed, the mixture was concentrated under reduced pressure at 40 °C, and the resulting crude product was purified by silica gel column chromatography (dichloromethane:methanol = 100:1 to 10:1, 0.1% NH₃) to give *tert*-butyl 3-(8-fluoro-7-(7-fluoro-3-(methoxymethoxy)-8-((triisopropylsilyl)ethynyl)naphthalen-1-yl)-2-(2-(piperazin-1-yl)ethoxy)pyrido[4,3-*d*]pyrimidin-4-yl)-3,8-diazabicyclo[3.2.1]octane-8-carboxylate.

LC-MS: (ESI, m/z): [M+H]⁺ = 872.2.

### Intermediate 11: 1-(2-methoxy-5-(3,9-diazaspiro[5.5]undecane-3-carbonyl)phenyl)dihydropyrimidine-2,4(1H,3H)-dione

### Step 1: preparation of tert-butyl 9-(3-(2,4-dioxotetrahydropyrimidin-1(2H)-yl)-4-methoxybenzoyl)-3,9-diazaspiro[5.5]undecane-3-carboxylate

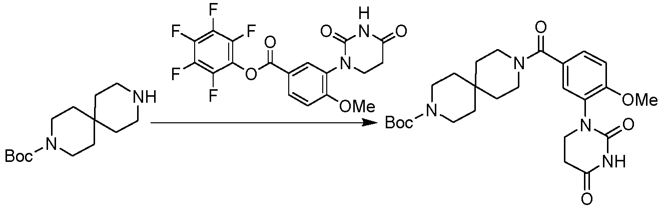

DIEA (721 mg, 5.58 mmol) and pentafluorophenyl 3-(2,4-dioxotetrahydropyrimidin-1(2*H*)-yl)-4-methoxybenzoate (800 mg, 1.86 mmol) were added to a solution of *tert*-butyl 3,9-diazaspiro[5.5]undecane-3-carboxylate (473 mg, 1.86 mmol) in dimethyl sulfoxide (8 mL). The mixture was stirred at 30 °C for 1 h. After the reaction was completed, the mixture was extracted with water (10 mL) and ethyl acetate (10 mL × 3). The organic phase was washed with brine (20 mL), dried over anhydrous Na₂SO₄, filtered, and concentrated in vacuum. The resulting crude product was purified by silica gel column chromatography (DCM:MeOH = 20:1) to give *tert*-butyl 9-(3-(2,4-dioxotetrahydropyrimidin-1(2*H*)-yl)-4-methoxybenzoyl)-3,9-diazaspiro[5.5]
undecane-3-carboxylate.

LC-MS: (ESI, m/z): [M-tBu+H]⁺ = 445.1.

### Step 2: preparation of 1-(2-methoxy-5-(3,9-diazaspiro[5.5]undecane-3-carbonyl)phenyl)dihydropyrimidine-2,4(1H,3H)-dione

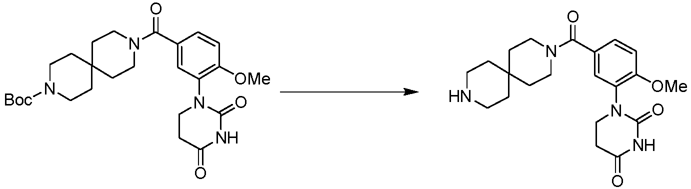

HCl/1,4-dioxane (2 mL) was added to a solution of *tert*-butyl 9-(3-(2,4-dioxotetrahydropyrimidin-1(2*H*)-yl)-4-methoxybenzoyl)-3,9-diazaspiro[5.5]undecane-3-carboxylate (920 mg, 1.84 mmol) in 1,4-dioxane (4 mL), and the mixture was stirred at 30 °C for 1 h. After the reaction was completed, the mixture was concentrated in vacuum to give 1-(2-methoxy-5-(3,9-diazaspiro[5.5]undecane-3-carbonyl)phenyl)dihydropyrimidine-2,4(1*H*,3*H*)-dione. The crude product was directly used in the next step without further purification.

LC-MS: (ESI, m/z): [M+H]⁺ = 401.1.

### Intermediate 12: preparation of 1-(2-chloro-5-(9-(piperazin-1-ylmethyl)-3-azaspiro[5.5]undecane-3-carbonyl) phenyl)dihydropyrimidine-2,4(1H,3H)-dione

### Step 1: preparation of tert-butyl 9-((4-benzylpiperazin-1-yl)methyl)-3-azaspiro[5.5]undecane-3-carboxylate

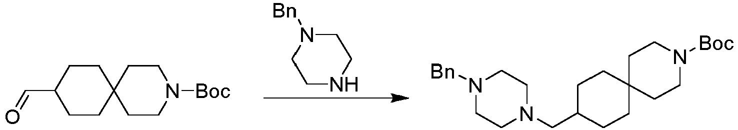

Acetic acid (0.1 mL) was added to a solution of *tert*-butyl 9-formyl-3-azaspiro[5.5]undecane-3-carboxylate (0.5 g, 1.77 mmol) and 1-benzylpiperazine (0.38 g, 2.14 mmol) in 1,2-dichloroethane (10 mL). The mixture was stirred at 30 °C for half an hour. The above mixture was cooled to 0 °C, NaBH(OAc)₃ (0.75 g, 3.56 mmol) was added, and then the mixture was warmed to room temperature and reacted for 3 h. After the reaction was completed, H₂O (10 mL) was added to quench the reaction, and the mixture was extracted with ethyl acetate (50 mL × 3). The combined organic phase was dried over anhydrous sodium sulfate, filtered, and concentrated under reduced pressure. The crude product was purified by silica gel column chromatography (DCM/MeOH = 20:1) to give *tert*-butyl 9-((4-benzylpiperazin-1-yl)methyl)-3-azaspiro[5.5]undecane-3-carboxylate.

LC-MS: (ESI, m/z): [M+H]⁺ = 442.2.

### Step 2: preparation of 9-((4-benzylpiperazin-1-yl)methyl)-3-azaspiro[5.5]undecane

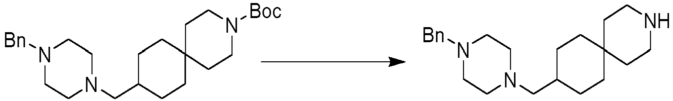

Hydrochloric acid/1,4-dioxane (1 mL) was added to a solution of *tert*-butyl 9-((4-benzylpiperazin-1-yl)methyl)-3-azaspiro[5.5]undecane-3-carboxylate (200 mg, 0.45 mmol) in 1,4-dioxane (3 mL). The mixture was stirred at room temperature for 1 h. The reaction liquid was directly concentrated under reduced pressure to give 9-((4-benzylpiperazin-1-yl)methyl)-3-azaspiro[5.5]undecane, which was directly used in the next step without further purification.

LC-MS: (ESI, m/z): [M+H]⁺ = 342.3.

### Step 3: preparation of 1-(5-(9-((4-benzylpiperazin-1-yl)methyl)-3-azaspiro[5.5]undecane-3-carbonyl)-2-chlorophenyl)dihydropyrimidine-2,4(1H,3H)-dione

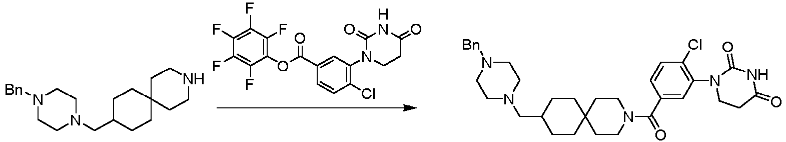

9-((4-Benzylpiperazin-1-yl)methyl)-3-azaspiro[5.5]undecane (150 mg, 0.45 mmol) was dissolved in DMF (3 mL), and pentafluorophenyl 4-chloro-3-(2,4-dioxotetrahydropyrimidin-1(2*H*)-yl)benzoate (205 mg, 0.47 mmol) and DIEA (175 mg, 1.35 mmol) were added. The reaction liquid was stirred at room temperature for 1 h. Water (20 mL) was added to the reaction liquid, and the mixture was extracted with ethyl acetate (20 mL × 2). The organic phases were combined, washed with saturated brine (30 mL × 3), dried over anhydrous sodium sulfate, and filtered. The filtrate was concentrated, and the resulting crude product was purified by silica gel column chromatography (DCM:MeOH = 100:1 to 5:1) to give 1-(5-(9-((4-benzylpiperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 592.2.

### Step 4: preparation of 1-(2-chloro-5-(9-(piperazin-1-ylmethyl)-3-azaspiro[5.5]undecane-3-carbonyl)phenyl) dihydropyrimidine-2,4(1H,3H)-dione

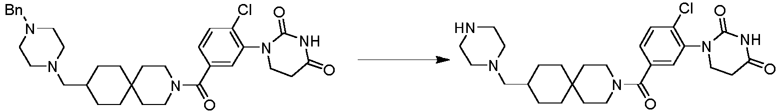

1-(5-(9-((4-Benzylpiperazin-1-yl)methyl)-3-azaspiro[5.5]undecane-3-carbonyl)-2-chlorophenyl)dihydropyrimidine-2,4(1*H*,3*H*)-dione (0.16 g, 0.27 mmol) was dissolved in DCM (3 mL), and then 1-chloroethyl chloroformate (77 mg, 0.54 mmol) and DIEA (70 mg, 0.54 mmol) were added. The mixture was reacted at room temperature for 30 min, and MeOH (3 mL) was added. The reaction liquid was reacted at 50 °C for 30 min. After the reaction was completed, water was added, and the mixture was extracted with ethyl acetate (50 mL × 3). The organic phase was washed with a saturated sodium chloride solution, dried over anhydrous sodium sulfate, and filtered, and the filtrate was concentrated. The resulting crude product was purified by silica gel column chromatography (DCM:MeOH = 20:1) to give 1-(2-chloro-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 502.2.

### Intermediate 13: 1-(2-chloro-5-(3,9-diazaspiro[5.5]undecane-3-carbonyl)phenyl)dihydropyrimidine-2,4(1H,3H)-dione hydrochloride

### Step 1: preparation of tert-butyl 9-(4-chloro-3-(2,4-dioxotetrahydropyrimidin-1(2H)-yl)benzoyl)-3,9-diazaspiro[5.5]undecane-3-carboxylate

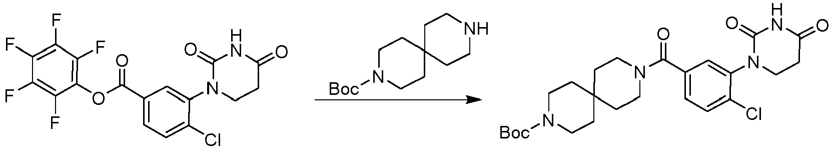

Pentafluorophenyl 4-chloro-3-(2,4-dioxotetrahydropyrimidin-1(2*H*)-yl)benzoate (500 mg, 1.15 mmol) and DIEA (387 mg, 3.00 mmol) were added to a solution of *tert*-butyl 3,9-diazaspiro[5.5]undecane-3-carboxylate (294 mg, 1.16 mmol) in DMF (5 mL), and the mixture was reacted at room temperature for 2 h. Water (50 mL) was added to the reaction liquid, and the mixture was extracted with ethyl acetate (30 mL × 2). The organic phase was washed with saturated brine, dried over anhydrous sodium sulfate, and filtered. The filtrate was concentrated and purified by silica gel column chromatography (DCM:MeOH = 100:1 for elution) to give *tert*-butyl 9-(4-chloro-3-(2,4-dioxotetrahydropyrimidin-1(2*H*)-yl)benzoyl)-3,9-diazaspiro [5.5]undecane-3-carboxylate.

LC-MS: (ESI, m/z): [M-tBu+H]⁺ = 449.2.

### Step 2: preparation of 1-(2-chloro-5-(3,9-diazaspiro[5.5]undecane-3-carbonyl)phenyl)dihydropyrimidine-2,4(1H,3H)-dione hydrochloride

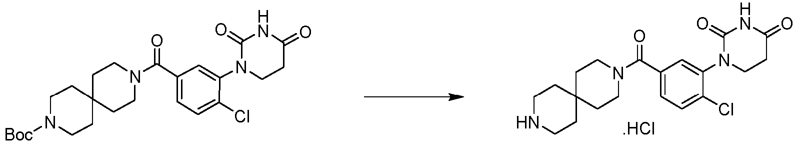

HCl/1,4-dioxane (6 N, 5.0 mL) was added to a stirred solution of *tert*-butyl 9-(4-chloro-3-(2,4-dioxotetrahydropyrimidin-1(2*H*)-yl)benzoyl)-3,9-diazaspiro[5.5]undecane-3-carboxylate (400 mg, 0.79 mmol) in 1,4-dioxane (5.0 mL), and the mixture was reacted at room temperature for 1 h. The reaction liquid was directly concentrated under reduced pressure to give 1-(2-chloro-5-(3,9-diazaspiro[5.5]undecane-3-carbonyl)phenyl)dihydropyrimidine-2,4(1*H*,3*H*)-dione hydrochloride. The resulting product was directly used in the next step.

### Intermediate 14: tert-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate

### Step 1: preparation of tert-butyl 3-(7-(8-ethynyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate

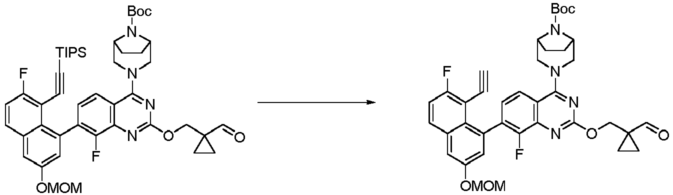

Cesium fluoride (1.35 g, 8.92 mmol) was added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate (1.5 g, 1.78 mmol) in DMF (6 mL). The mixture was reacted at 25 °C for 16 h, and water (10 mL) was added to quench the reaction. The mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 30:1) to give *tert*-butyl 3-(7-(8-ethynyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate.

LC-MS: (ESI, m/z): [M+H]⁺ = 685.3.

### Step 2: preparation of tert-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate

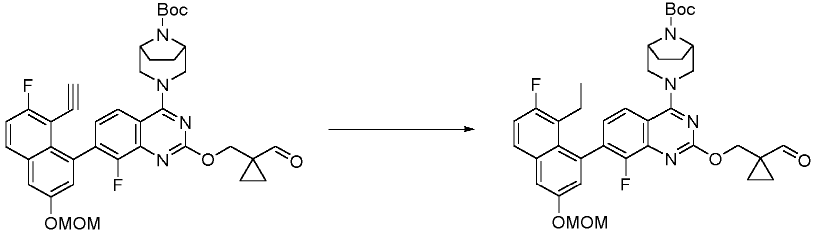

Pd/C (124 mg, 0.117 mmol) was added to a solution of *tert-butyl* 3-(7-(8-ethynyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (800 mg, 1.17 mmol) in ethyl acetate (6 mL), and the mixture was reacted at 30 °C for 16 h. After the reaction was completed, the reaction liquid was concentrated under reduced pressure to give *tert*-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate.

LC-MS: (ESI, m/z): [M+H]⁺ = 688.9.

### Intermediate 15: tert-butyl 3-(8-fluoro-2-((1-formylcyclopropyl)methoxy)-7-(3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

### Step 1: preparation of tert-butyl 3-(8-fluoro-2-((1-(hydroxymethyl)cyclopropyl)methoxy)-7-(3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

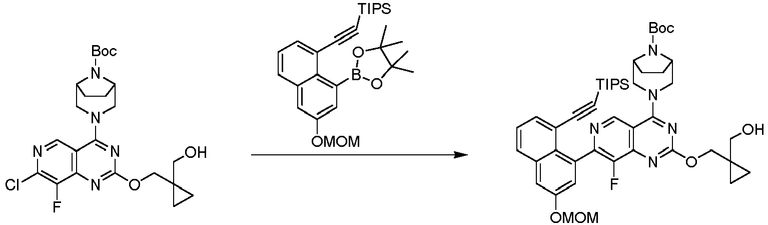

cataCXium A Pd G3 (44 mg, 0.061 mmol) and cesium carbonate (398 mg, 1.22 mmol) were added to a mixed solution of *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (300 mg, 0.61 mmol) and triisopropyl((6-(methoxymethoxy)-8-(4,4,5,5-tetramethyl-1,3,2-dioxaborolan-2-yl)naphthalen-1-yl)ethynyl)silane (361 mg, 0.73 mmol) in 1,4-dioxane (6 mL) and H₂O (1.2 mL). The mixture was reacted at 85 °C for 6 h under nitrogen atmosphere. The reaction liquid was directly filtered and concentrated under reduced pressure. The concentrate was purified by silica gel column chromatography (DCM:MeOH = 100:1) to give *tert*-butyl 3-(8-fluoro-2-((1-(hydroxymethyl)cyclopropyl)methoxy)-7-(3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate.

LC-MS: (ESI, m/z): [M+H]⁺ = 826.1.

### Step 2: preparation of tert-butyl 3-(8-fluoro-2-((1-formylcyclopropyl)methoxy)-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

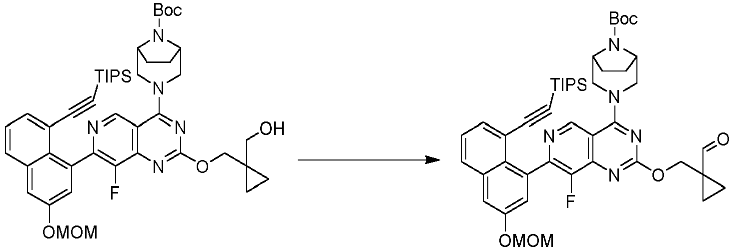

Dess-Martin reagent (200 mg, 0.47 mmol) was added to a solution of *tert*-butyl 3-(8-fluoro-2-((1-(hydroxymethyl)cyclopropyl)methoxy)-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (260 mg, 0.31 mmol) in dichloromethane (5 mL). The mixture was stirred at room temperature for 2 h. After the reaction was completed, a sodium thiosulfate solution (5 mL) and a sodium bicarbonate solution (5 mL) were added to quench the reaction, and the mixture was extracted with dichloromethane (10 mL × 3). The organic phases were combined, dried over anhydrous sodium sulfate, and concentrated under reduced pressure. The concentrate was purified by silica gel column chromatography (PE:EA = 1:1) to give *tert*-butyl 3-(8-fluoro-2-((1-formylcyclopropyl)methoxy)-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 824.1.

### Intermediate 16: 1-(2-chloro-5-(4-(2-(methyl(piperidin-4-ylmethyl)amino)ethyl)piperidine-1-carbonyl) phenyl)dihydropyrimidine-2,4(1H,3H)-dione

### Step 1: preparation of benzyl 4-(2-oxoethyl)piperidine-1-carboxylate

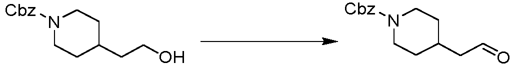

Dess-Martin oxidant (10.5 g, 24.69 mmol) was added to a solution of benzyl 4-(2-hydroxyethyl)piperidine-1-carboxylate (5 g, 18.99 mmol) in DCM (50 mL), and the mixture was stirred at room temperature for 2 h. After the reaction was completed, a sodium thiosulfate solution (50 mL) and a sodium bicarbonate solution (50 mL) were added to quench the reaction, and the mixture was extracted with dichloromethane (50 mL × 3). The organic phases were combined, dried over anhydrous sodium sulfate, and concentrated under reduced pressure to give benzyl 4-(2-oxoethyl)piperidine-1-carboxylate, which was directly used in the next step.

### Step 2: preparation of benzyl 4-(2-(((1-(tert-butoxycarbonyl)piperidin-4-yl)methyl)(methyl)amino)ethyl) piperidine-1-carboxylate

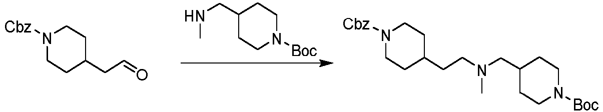

*tert*-Butyl 4-((methylamino)methyl)piperidine-1-carboxylate (2.8 g, 12.3 mmol) and acetic acid (1.5 mL) were added to a solution of benzyl 4-(2-oxoethyl)piperidine-1-carboxylate (2.66 g, 10.2 mmol) in DCM/MeOH (30 mL/15 mL). The mixture was stirred at room temperature for 2 h. NaBH(OAc)₃ (4.3 g, 20.4 mmol) was then added at 0 °C. The mixture was diluted with water (30 mL) and extracted with DCM (30 mL × 3). The combined organic phase was dried over anhydrous sodium sulfate and filtered, and the filtrate was concentrated and purified by silica gel column chromatography (DCM:MeOH = 20:1) to give benzyl 4-(2-(((1-(*tert*-butoxycarbonyl)piperidin-4-yl)methyl)(methyl)amino)ethyl)piperidine-1-carboxylate.

LC-MS: (ESI, m/z): [M+H]⁺ = 474.1.

### Step 3: preparation of tert-butyl 4-((methyl(2-(piperidin-4-yl)ethyl)amino)methyl)piperidine-1-carboxylate

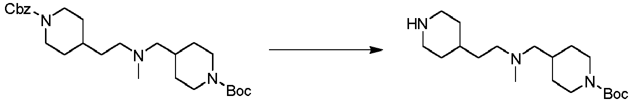

Pd/C (447 mg) was added to a solution of benzyl 4-(2-(((1-(*tert*-butoxycarbonyl)piperidin-4-yl)methyl)(methyl)amino)ethyl)piperidine-1-carboxylate (2 g, 4.2 mmol) in methanol (15 mL), and the mixture was stirred at 30 °C for 16 h under hydrogen atmosphere. The mixture was filtered and concentrated to give *tert*-butyl 4-((methyl(2-(piperidin-4-yl)ethyl)amino)methyl)piperidine-1-carboxylate. The crude product was directly used in the next step without further purification.

LC-MS: (ESI, m/z): [M+H]⁺ = 340.3.

### Step 4: preparation of tert-butyl 4-(((2-(1-(4-chloro-3-(2,4-dioxotetrahydropyrimidin-1(2H)-yl)benzoyl) piperidin-4-yl)ethyl)(methyl)amino)methyl)piperidine-1-carboxylate

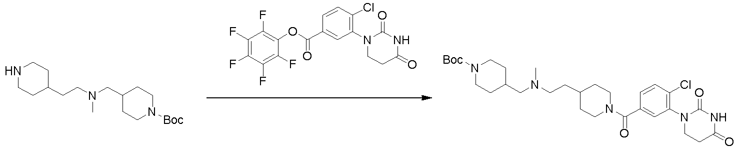

Pentafluorophenyl 4-chloro-3-(2,4-dioxotetrahydropyrimidin-1(2H)-yl)benzoate (769 mg, 1.77 mmol) and DIEA (686 mg, 5.31 mmol) were added to a solution of *tert*-butyl 4-((methyl(2-(piperidin-4-yl)ethyl)amino)methyl)piperidine-1-carboxylate (600 mg, crude) in DMF (8 mL). The mixture was stirred at room temperature for 2 h. After the reaction was completed, water (10 mL) was added to quench the reaction, and the mixture was extracted with ethyl acetate (30 mL × 3). The organic phase was dried, concentrated, and purified by silica gel column chromatography (DCM:MeOH = 20:1) to give *tert*-butyl 4-(((2-(1-(4-chloro-3-(2,4-dioxotetrahydropyrimidin-1(2*H*)-yl)benzoyl)piperidin-4-yl)ethyl)(methyl)amino)methyl)piperidine-1-carboxylate.

LC-MS: (ESI, m/z): [M+H]⁺ = 590.3.

### Step 5: preparation of 1-(2-chloro-5-(4-(2-(methyl(piperidin-4-ylmethyl)amino)ethyl)piperidine-1-carbonyl) phenyl)dihydropyrimidine-2,4(1H,3H)-dione

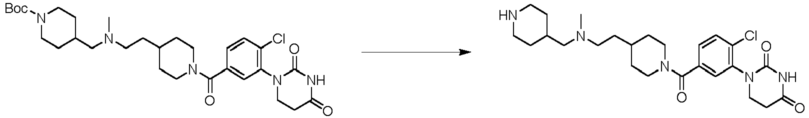

HCl/1,4-dioxane (2 mL) was added to a solution of *tert*-butyl 4-(((2-(1-(4-chloro-3-(2,4-dioxotetrahydropyrimidin-1(2*H*)-yl)benzoyl)piperidin-4-yl)ethyl)(methyl)amino)methyl)piperidine-1-carboxylate (900 mg, 1.5 mmol) in 1,4-dioxane (3 mL), and the mixture was stirred at 30 °C for 1 h. After the reaction was completed, an aqueous Na₂CO₃ solution (5 mL) was added to adjust the pH > 7, and the mixture was extracted with ethyl acetate (5 mL × 3). The combined organic phase was dried over Na₂SO₄, filtered, concentrated under reduced pressure, and purified by silica gel column chromatography (DCM:MeOH (NH₃) = 20:1) to give 1-(2-chloro-5-(4-(2-(methyl(piperidin-4-ylmethyl)amino)ethyl)piperidine-1-carbonyl)phenyl)
dihydropyrimidine-2,4(1*H*,3*H*)-dione.

LC-MS: (ESI, m/z): [M+H]⁺ = 490.3.

### Intermediate 17: tert-butyl 3-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-d]pyrimidin-4-yl)-3,8-diazabicyclo[3.2.1] octane-8-carboxylate

### Step 1: preparation of tert-butyl 3-(7-chloro-8-fluoro-2-((1-(hydroxymethyl)cyclopropyl)methoxy)pyrido[4,3-d]pyrimidin-4-yl)-3,8-diazabicyclo[3.2.1]octane-8-carboxylate

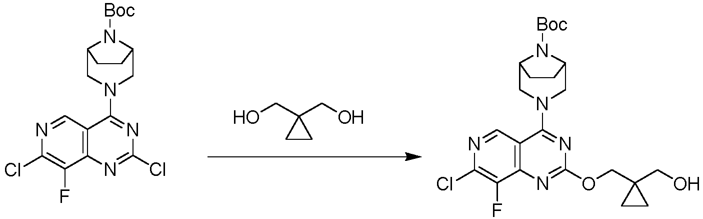

Cs₂CO₃ (9.1 g, 28.0 mmol) was added to a solution of *tert*-butyl 3-(2,7-dichloro-8-fluoropyrido[4,3-*d*]pyrimidin-4-yl)-3,8-diazabicyclo[3.2.1]octane-8-carboxylate (4.0 g, 9.35 mmol) and cyclopropane-1,1-diyldimethanol (1.9 g, 18.7 mmol) in THF (50 mL), and the mixture was stirred at room temperature for 16 h. H₂O (50 mL) was added to the reaction liquid, and the mixture was extracted with EtOAc (50 mL × 3). The organic phases were combined, dried over anhydrous sodium sulfate, and concentrated under reduced pressure, and the concentrate was purified by silica gel column chromatography (DCM:MeOH = 50:1) to give *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 494.2.

### Step 2: preparation of tert-butyl 3-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-d]pyrimidin-4-yl)-3,8-diazabicyclo [3.2.1]octane-8-carboxylate

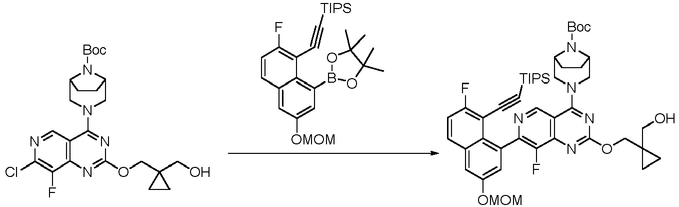

cataCXium A Pd G3 (1.8 g, 2.4 mmol) and cesium carbonate (11.9 g, 36.4 mmol) were added to a mixed solution of *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (6.0 g, 12.2 mmol) and (2-fluoro-6-(methoxymethoxy)-8-(4,4,5,5-tetramethyl-1,3,2-dioxaborolan-2-yl)naphthalen-1-yl)ethynyl)triisopropylsilane (7.5 g, 14.6 mmol) in 1,4-dioxane (60 mL) and H₂O (12 mL). The mixture was reacted at 85 °C for 3 h under nitrogen atmosphere. Water (100 mL) was added to quench the reaction, and the mixture was extracted with ethyl acetate (100 mL × 3). The organic phases were combined, dried over anhydrous sodium sulfate, and concentrated under reduced pressure. The concentrate was purified by silica gel column chromatography (DCM:MeOH = 100:1) to give *tert*-butyl 3-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 844.4.

### Step 3: preparation of tert-butyl 3-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-d]pyrimidin-4-yl)-3,8-diazabicyclo[3.2.1] octane-8-carboxylate

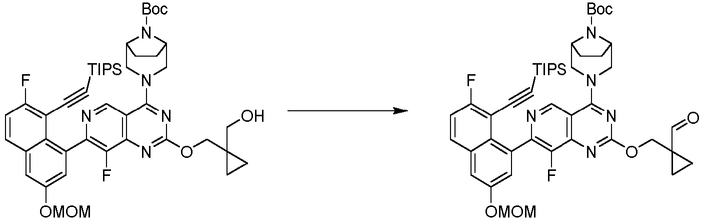

A solution of *tert*-butyl 3-(8-fluoro-7-(7-fluoro-3-(methoxymethoxy)-8-((triisopropylsilyl)ethynyl) naphthalen-1-yl)-2-((1-(hydroxymethyl)cyclopropyl)methoxy)pyrido[4,3-*d*]pyrimidin-4-yl)-3,8-diazabicyclo [3.2.1]octane-8-carboxylate (4.0 g, 4.7 mmol) in DCM (40 mL) was cooled to 0 °C, and then Dess-Martin (2.4 g, 5.7 mmol) was added. The mixture was reacted at room temperature for 0.5 h. Water (30 mL) was added to the reaction liquid to quench the reaction, and the mixture was extracted with dichloromethane (30 mL × 3). The organic phases were combined, dried over anhydrous sodium sulfate, and concentrated under reduced pressure. The concentrate was purified by silica gel column chromatography (DCM:MeOH (NH₃) = 30:1) to give *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 842.4.

¹H NMR (400 MHz, CDCl₃) δ 9.27 (s, 1H), 9.07 (s, 1H), 7.79 (dd, *J* = 9.2 Hz, 5.6 Hz, 1H), 7.51 (d, *J =* 2.8 Hz, 1H), 7.35-7.28 (m, 2H), 5.35 - 5.24 (m, 2H), 4.86 - 4.69 (m, 2H), 4.64 - 4.55 (m, 1H), 4.50 - 4.28 (m, 2H), 4.21 - 4.10 (m, 1H), 3.90 - 3.68 (m, 1H), 3.62 - 3.34 (m, 4H), 2.05 - 1.98 (m, 3H), 1.79 - 1.64 (m, 1H), 1.52 (s, 9H), 1.39 - 1.26 (m, 4H), 0.95 - 0.78 (m, 18H), 0.61 - 0.47 (m, 3H).

### Intermediate 18: preparation of 1-(2-chloro-5-(4-(3-(piperazin-1-yl)propyl)piperidine-1-carbonyl)phenyl) dihydropyrimidine-2,4(1H,3H)-dione

### Step 1: preparation of benzyl 4-(3-hydroxypropyl)piperidine-1-carboxylate

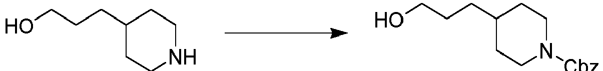

A sodium bicarbonate solution (42 mL) was added to a solution of 3-(piperidin-4-yl)propan-1-ol (3 g, 20.9 mmol) in tetrahydrofuran (12 mL). After the mixture was cooled to 0 °C, benzyl chloroformate (3.9 g, 23.0 mmol) was slowly added dropwise. The mixture was reacted at room temperature for 2 h and then extracted with ethyl acetate (30 mL × 3). The organic phase was dried, concentrated, and purified by silica gel column chromatography (PE:EA = 30:1) to give benzyl 4-(3-hydroxypropyl)piperidine-1-carboxylate.

LC-MS: (ESI, m/z): [M+H]⁺ = 278.1.

### Step 2: preparation of benzyl 4-(3-oxopropyl)piperidine-1-carboxylate

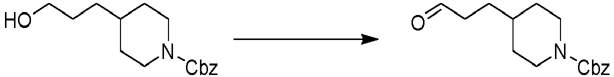

Dess-Martin (7 g, 17.3 mmol) was added to a solution of benzyl 4-(3-hydroxypropyl)piperidine-1-carboxylate (4 g, 14.4 mmol) in dichloromethane (50 mL). The mixture was stirred at room temperature for 2 h. After the reaction was completed, a sodium thiosulfate solution (50 mL) and a sodium bicarbonate solution (50 mL) were added to quench the reaction, and the mixture was extracted with dichloromethane (50 mL × 3). The organic phases were combined, dried over anhydrous sodium sulfate, and concentrated under reduced pressure. The concentrate was purified by silica gel column chromatography (PE:EA = 2:1) to give benzyl 4-(3-oxopropyl)piperidine-1-carboxylate.

LC-MS: (ESI, m/z): [M+H]⁺ = 276.1.

### Step 3: preparation of tert-butyl 4-(3-(1-((benzyloxy)carbonyl)piperidin-4-yl)propyl)piperazine-1-carboxylate

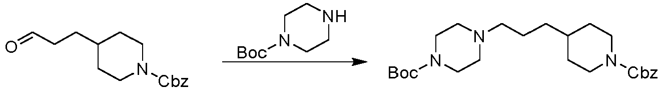

*tert*-Butyl piperazine-1-carboxylate (6.7 g, 36.0 mmol) and acetic acid (2 mL) were added to a solution of benzyl 4-(3-oxopropyl)piperidine-1-carboxylate (3.3 g, 12.0 mmol) in 1,2-dichloroethane (60 mL), and the mixture was reacted at room temperature for 2 h. After the mixture was cooled to 0 °C sodium triacetoxyborohydride (5.3 g, 24.0 mmol) was slowly added. The mixture was reacted at room temperature for 16 h. After the reaction was completed, water (60 mL) was added to quench the reaction, and the mixture was extracted with dichloromethane (60 mL × 3). The organic phase was dried, concentrated, and purified by silica gel column chromatography (PE:EA = 1:1) to give *tert*-butyl 4-(3-(1-((benzyloxy)carbonyl)piperidin-4-yl)propyl)piperazine-1-carboxylate.

LC-MS: (ESI, m/z): [M+H]⁺ = 446.2.

### Step 4: preparation of tert-butyl 4-(3-(piperidin-4-yl)propyl)piperazine-1-carboxylate

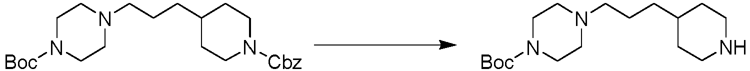

Pd/C (597 mg) was added to a solution of *tert*-butyl 4-(3-(1-((benzyloxy)carbonyl)piperidin-4-yl)propyl)piperazine-1-carboxylate (2.5 g, 5.6 mmol) in methanol (40 mL), and the mixture was stirred at 30 °C for 16 h. The mixture was filtered and concentrated to give *tert*-butyl 4-(3-(piperidin-4-yl)propyl)piperazine-1-carboxylate.

LC-MS: (ESI, m/z): [M+H]⁺ = 312.2.

### Step 5: preparation of tert-butyl 4-(3-(1-(4-chloro-3-(2,4-dioxotetrahydropyrimidin-1(2H)-yl)benzoyl) piperidin-4-yl)propyl)piperazine-1-carboxylate

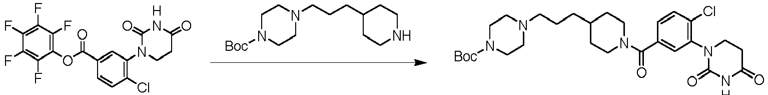

Pentafluorophenyl 4-chloro-3-(2,4-dioxotetrahydropyrimidin-1(2H)-yl)benzoate (348 mg, 0.8 mmol) and DIEA (310 mg, 2.4 mmol) were added to a solution of *tert*-butyl 4-(3-(piperidin-4-yl)propyl)piperazine-1-carboxylate (250 mg, 0.8 mmol) in DMF (3 mL). The mixture was stirred at room temperature for 1 h. After the reaction was completed, water (5 mL) was added to quench the reaction, and the mixture was extracted with ethyl acetate (5 mL × 3). The organic phase was dried, concentrated, and purified by silica gel column chromatography (DCM:MeOH = 20:1) to give *tert*-butyl 4-(3-(1-(4-chloro-3-(2,4-dioxotetrahydropyrimidin-1(2*H*)-yl)benzoyl)piperidin-4-yl)propyl)piperazine-1-carboxylate.

LC-MS: (ESI, m/z): [M+H]⁺ = 562.2.

### Step 6: preparation of 1-(2-chloro-5-(4-(3-(piperazin-1-yl)propyl)piperidine-1-carbonyl)phenyl) dihydropyrimidine-2,4(1H,3H)-dione

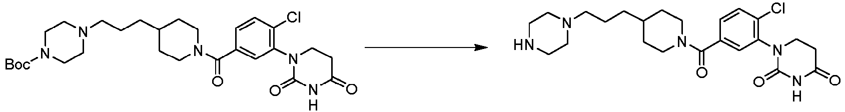

HCl/1,4-dioxane (6 N, 1 mL) was added to a solution of *tert*-butyl 4-(3-(1-(4-chloro-3-(2,4-dioxotetrahydropyrimidin-1(2*H*)-yl)benzoyl)piperidin-4-yl)propyl)piperazine-1-carboxylate (400 mg, 0.71 mmol) in 1,4-dioxane (2 mL). The mixture was stirred at room temperature for 1 h, concentrated, and purified by silica gel column chromatography (DCM:MeOH (NH₃) = 20:1) to give 1-(2-chloro-5-(4-(3-(piperazin-1-yl)propyl)piperidine-1-carbonyl)phenyl)dihydropyrimidine-2,4(1*H*,3*H*)-dione.

LC-MS: (ESI, m/z): [M+H]⁺ = 462.2.

### Intermediate 19: tert-butyl 9-(3-hydroxypropyl)-3-azaspiro[5.5]undecane-3-carboxylate

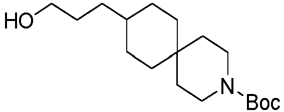

The intermediate was prepared with reference to Example 21 in the patent WO2022223034A1.

### Intermediate 20: tert-butyl 9-(2-hydroxyethyl)-3-azaspiro[5.5]undecane-3-carboxylate

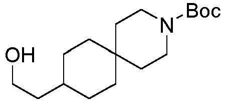

The intermediate was prepared with reference to Example 22 in the patent WO2022223034A1.

### Intermediate 21: 1-(2-chloro-5-(piperazine-1-carbonyl)phenyl)dihydropyrimidine-2,4(1H,3H)-dione

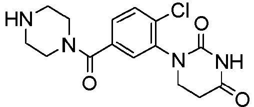

The intermediate was prepared with reference to Example 40 in the patent WO2023025159A1.

### Intermediate 22: 3-(3-(2,4-dioxotetrahydropyrimidin-1(2H)-yl)-4-methylbenzoyl)-3-azaspiro[5.5]undecane-9-carbaldehyde

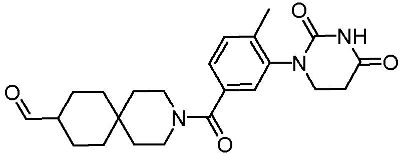

The intermediate was prepared with reference to Example 32 in the patent WO2023025159A1.

### Intermediate 23: 1-(2-chloro-5-(3,9-diazaspiro[5.5]undecane-3-carbonyl)phenyl)dihydropyrimidine-2,4(1H,3H)-dione

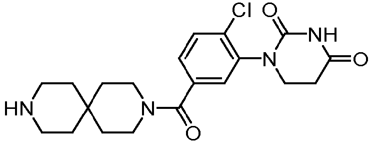

The intermediate was prepared with reference to Example 48 in the patent WO2023025159A1.

### Intermediate 24: 3-(3-(2,4-dioxotetrahydropyrimidin-1(2H)-yl)-4-methoxybenzoyl)-3-azaspiro[5.5]undecane-9-carbaldehyde

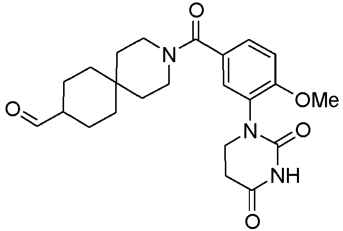

Reference was made to the preparation method for intermediate 3.

### Intermediate 25: tert-butyl 3-(8-fluoro-7-(7-fluoro-3-(methoxymethoxy)-8-((triisopropylsilyl)ethynyl) naphthalen-1-yl)-2-((R)-2-methyl-3-(piperazin-1-yl)propoxy)pyrido[4,3-d]pyrimidin-4-yl)-3,8-diazabicyclo [3.2.1]octane-8-carboxylate

### Step 1: preparation of methyl (S)-3-((tert-butyldimethylsilyl)oxy)-2-methylpropanoate

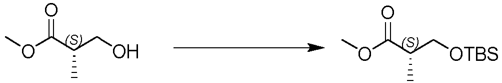

Methyl (*S*)-3-hydroxy-2-methylpropanoate (30 g, 254 mmol) and imidazole (34.5 g, 508 mmol) were dissolved in dichloromethane (120 mL), and *tert*-butyldimethylchlorosilane (46 g, 305 mmol) was added. The mixture was reacted at room temperature for 3 h. After the reaction was completed, the reaction liquid was poured into a saturated aqueous ammonium chloride solution (20 mL), and the mixture was extracted with ethyl acetate (200 mL × 3). The organic phases were combined, washed with saturated brine, dried over anhydrous sodium sulfate, and filtered. The filtrate was concentrated, and the resulting crude product was purified by silica gel column chromatography (PE:EtOAc = 30:1) to give methyl (*S*)-3-((*tert*butyldimethylsilyl)oxy)-2-methylpropanoate.

¹H NMR (400 MHz, CDCl₃) δ3.80 - 3.73 (m, 1H), 3.70 - 3.58 (m, 4H), 2.69 - 2.57(m, 1H), 1.14 (d, *J* = 7.0 Hz, 3H), 0.87 (s, 9H), 0.03 (d, *J* = 1.4 Hz, 6H).

### Step 2: preparation of (R)-3-((tert-butyldimethylsilyl)oxy)-2-methylpropan-1-ol

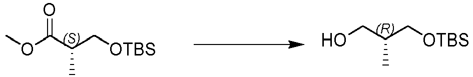

Methyl (*S*)-3-((*tert*-butyldimethylsilyl)oxy)-2-methylpropanoate (49 g, 211 mmol) was dissolved in THF/MeOH (500 mL/100 mL), and the mixture was cooled to 0-5 °C. Lithium borohydride (7 g, 317 mmol) was added, and the mixture was reacted at room temperature for 16 h. An aqueous ammonium chloride solution was first added to quench the reaction, and the mixture was then extracted with ethyl acetate (200 mL × 3). The organic phases were combined, washed with saturated brine, dried over anhydrous sodium sulfate, and filtered, and the filtrate was concentrated to give (*R*)-3-((*tert*-butyldimethylsilyl)oxy)-2-methylpropan-1-ol. The resulting crude product was directly used in the next step.

¹H NMR (400 MHz, CDCl₃) δ 3.77 - 3.69 (m, 1H), 3.68 - 3.49 (m, 3H), 2.82 (brs, 1H), 2.00 - 1.87 (m, 1H), 0.89 (s, 9H), 0.83 (d, *J* = 7.0 Hz, 3H), 0.07 (s, 6H).

### Step 3: preparation of (S)-3-((tert-butyldimethylsilyl)oxy)-2-methylpropyl methanesulfonate

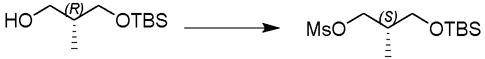

(*R*)-3-((*tert*-Butyldimethylsilyl)oxy)-2-methylpropan-1-ol (35 g, 172 mmol) and triethylamine (34.7 g, 344 mmol) were dissolved in DCM, and the mixture was cooled to 0-5 °C. Methanesulfonyl chloride (23.6 g, 206 mmol) was added, and the mixture was reacted at room temperature for 2 h. An aqueous ammonium chloride solution was added, and the mixture was extracted with DCM. The organic phase was washed with a saturated aqueous sodium chloride solution, dried over anhydrous sodium sulfate, and filtered, and the filtrate was concentrated to give a crude product (*S*)-3-((*tert*-butyldimethylsilyl)oxy)-2-methylpropyl methanesulfonate. The resulting crude product was directly used in the next step.

### Step 4: preparation of (R)-1-benzyl-4-(3-((tert-butyldimethylsilyl)oxy)-2-methylpropyl)piperazine

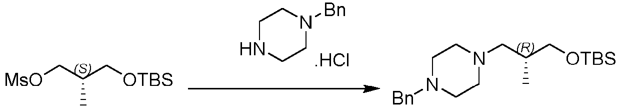

(*S*)-3-((*tert*-Butyldimethylsilyl)oxy)-2-methylpropyl methanesulfonate (48 g, crude) was added to a mixed solution of 1-benzylpiperazine hydrochloride (73 g, 344 mmol) and potassium carbonate (95 g, 688 mmol), and the mixture was heated at 60 °C and reacted for 16 h. The reaction liquid was filtered, and the filtrate was concentrated and purified by column chromatography (PE:EtOAc = 20:1 to 3:1) to give (R)-1-benzyl-4-(3-((*tert*-butyldimethylsilyl)oxy)-2-methylpropyl)piperazine.

LC-MS: (ESI, m/z): [M+H]⁺ = 363.4.

### Step 5: preparation of (R)-3-(4-benzylpiperazin-1-yl)-2-methylpropan-1-ol

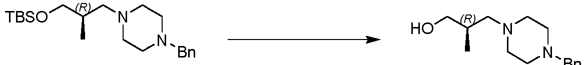

(*R*)-1-Benzyl-4-(3-((*tert*-butyldimethylsilyl)oxy)-2-methylpropyl)piperazine (22 g, 60 mmol) was dissolved in ethanol (300 mL), and the mixture was cooled to 0-5 °C. Concentrated hydrochloric acid (80 mL) was added, and the mixture was reacted at room temperature for 1 h. The reaction liquid was concentrated to remove ethanol, and water (100 mL) was added to the residue. The mixture was then extracted with methyl *tert*-butyl ether (100 mL). The aqueous phase was adjusted to pH = 8-9 with an aqueous sodium carbonate solution, and the mixture was extracted with ethyl acetate (100 mL × 3). The combined ethyl acetate organic phase was dried over anhydrous sodium sulfate and filtered, and the filtrate was concentrated to give (*R*)-3-(4-benzylpiperazin-1-yl)-2-methylpropan-1-ol (*e.e.* = 98.8%).

¹H NMR (400 MHz, CDCl₃) δ 7.34 - 7.28 (m, 4H), 7.27 - 7.22 (m, 1H), 3.70 - 3.59 (m, 1H), 3.54 - 3.39 (m, 3H), 2.86 - 2.25 (m, 10H), 2.18 - 2.10 (m, 1H), 0.73 (d, *J =* 6.8 Hz, 3H).

LC-MS: (ESI, m/z): [M+H]⁺ = 249.2.

### Step 6: preparation of tert-butyl 3-(2-((R)-3-(4-benzylpiperazin-1-yl)-2-methylpropoxy)-7-chloro-8-fluoropyrido[4,3-d]pyrimidin-4-yl)-diazabicyclo[3.2.1]octane-8-carboxylate

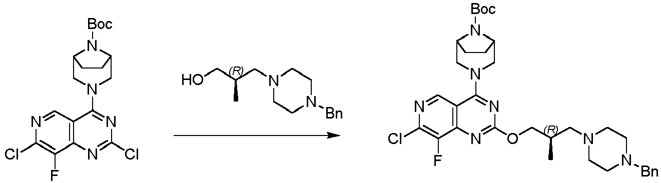

*tert*-Butyl 3-(2,7-dichloro-8-fluoropyrido[4,3-*d*]pyrimidin-4-yl)-diazabicyclo[3.2.1]octane-8-carboxylate (1.50 g, 3.50 mmol) and (*R*)-3-(4-benzylpiperazin-1-yl)-2-methylpropan-1-ol (1.30 g, 5.26 mmol) were dissolved in THF (100 mL) in a sealed tube reactor, and Cs₂CO₃ (3.42 g, 10.5 mmol) was added. The reaction liquid was heated to 90 °C and reacted for 16 h. After the reaction was completed, the mixture was cooled to room temperature. Water (30 mL) was added, and then the mixture was extract with ethyl acetate (30 mL × 3). The organic phases were combined, washed with a saturated NaCl solution, dried over anhydrous Na₂SO₄, and filtered. The filtrate was concentrated, and the resulting crude product was purified by silica gel column chromatography (DCM:MeOH = 20:1) to give *tert*-butyl 3-(2-((*R*)-3-(4-benzylpiperazin-1-yl)-2-methylpropoxy)-7-chloro-8-fluoropyrido[4,3-*d*]pyrimidin-4-yl)-diazabicyclo[3.2.1]octane-8-carboxylate.

LC-MS: (ESI, m/z): [M+H]⁺ = 640.4.

### Step 7: preparation of tert-butyl 3-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-diazabicyclo[3 .2.1]octane-8-carboxylate

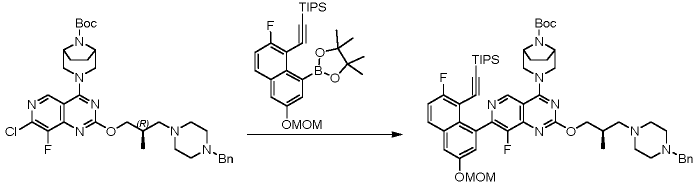

Ruphos Pd G3 (0.13 g, 0.16 mmol) and potassium phosphate (0.99 g, 4.68 mmol) were added to a mixed solution of *tert*-butyl 3-(2-((*R*)-3-(4-benzylpiperazin-1-yl)-2-methylpropoxy)-7-chloro-8-fluoropyrido[4,3-*d*]pyrimidin-4-yl)-diazabicyclo[3.2.1]octane-8-carboxylate (1.00 g, 1.56 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (0.96 g, 1.81 mmol) in 1,4-dioxane/water (6 mL/1.2 mL). The mixture was reacted at 85 °C for 16 h under nitrogen atmosphere. Water (20 mL) was added, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 20:1) to give *tert-butyl* 3-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-diazabicyclo[3.2.1]octane-8-carboxylate.

LC-MS: (ESI, m/z): [M+H]⁺ = 990.6.

### Step 8: preparation of tert-butyl 3-(8-fluoro-7-(7-fluoro-3-(methoxymethoxy)-8-((triisopropylsilyl) ethynyl)naphthalen-1-yl)-2-((R)-2-methyl-3-(piperazin-1-yl)propoxy)pyrido[4,3-d]pyrimidin-4-yl)-3,8-diazabicyclo[3.2.1]octane-8-carboxylate

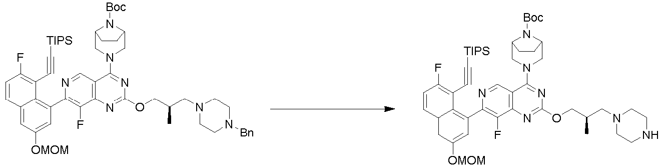

*tert*-Butyl 3-(2-((*R*)-3-(4-benzylpiperazin-1-yl)-2-methylpropoxy)-8-fluoro-7-(7-fluoro-3-(methoxymethoxy)- 8-((triisopropylsilyl)ethynyl)naphthalen-1-yl)pyrido[4,3-*d*]pyrimidin-4-yl)-diazabicyclo[3.2.1]octane-8-carboxylate (300 mg, 0.30 mmol) was dissolved in DCM (3 mL), and 1-chloroethyl chloroformate (130 mg, 0.91 mmol) and DIEA (117 mg, 0.91 mmol) were added. The mixture was reacted at room temperature for 1 h. The reaction liquid was directly concentrated under reduced pressure, and MeOH (5 mL) was added to the concentrate. The mixture was then heated to 50 °C and reacted for 1 h. After the reaction was completed, the mixture was concentrated under reduced pressure. The concentrate was purified by silica gel column chromatography (DCM:MeOH (NH₃) = 20:1) to give *tert-*butyl 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate.

LC-MS: (ESI, m/z): [M+H]⁺ = 900.5.

### Intermediate 26: 3-(3-(2,4-dioxotetrahydropyrimidin-1(2H)-yl)-4-(methyl-d3)benzoyl)-3-azaspiro[5.5] undecane-9-carbaldehyde

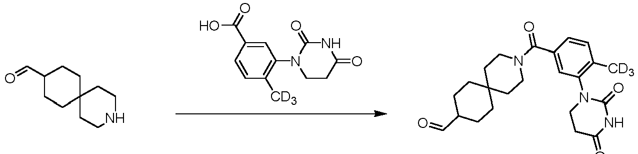

HATU (76 mg, 0.2 mmol) and DIEA (130 mg, 1 mmol) were added to a solution of 3-(2,4-dioxotetrahydropyrimidin-1(2*H*)-yl)-4-(methyl-d3)benzoic acid (50 mg, 0.2 mmol) in DMF (5 mL), and then 3-azaspiro[5.5]undecane-9-carbaldehyde (47 mg, 0.17 mmol) was added. The mixture was reacted at room temperature overnight. After the reaction was completed, H₂O (15 mL) was added to the reaction liquid, and then the mixture was extracted with ethyl acetate (15 mL × 3). The combined organic phase was dried over anhydrous sodium sulfate, filtered, and concentrated in vacuum under reduced pressure. The crude product was purified by silica gel column chromatography (DCM:MeOH = 20:1) to give 3-(3-(2,4-dioxotetrahydropyrimidin-1(2*H*)-yl)-4-(methyl-*d*3)benzoyl)-3-azaspiro[5.5]undecane-9-carbaldehyde.

LC-MS: (ESI, m/z): [M+H]⁺ = 415.3.

### Intermediate 27: 3-(5-(2,4-dioxotetrahydropyrimidin-1(2H)-yl)-2-fluoro-4-methylbenzoyl)-3-azaspiro[5.5] undecane-9-carbaldehyde

### Step 1: preparation of 5-amino-2-fluoro-4-methylbenzoic acid

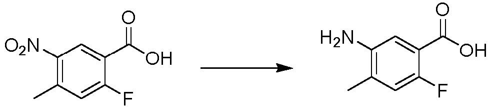

Pd/C (700 mg, 10%) was added to a solution of 2-fluoro-4-methyl-5-nitrobenzoic acid (4.8 g, 24.1 mmol) in MeOH (150 mL). The mixture was stirred at 50 °C overnight under H₂ atmosphere. The reaction liquid was filtered through celite, and the filtrate was concentrated to give 5-amino-2-fluoro-4-methylbenzoic acid.

LC-MS: (ESI, m/z): [M+H]⁺ = 170.1.

### Step 2: preparation of 5-(2,4-dioxotetrahydropyrimidin-1(2H)-yl)-2-fluoro-4-methylbenzoic acid

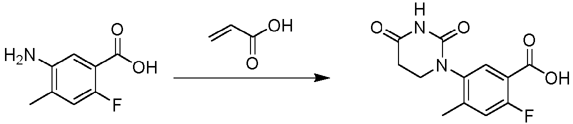

Acrylic acid (6.6 mL, 7 g, 96.8 mmol) and HOAc (30 mL) were added to 5-amino-2-fluoro-4-methylbenzoic acid (4.1 g, 24.2 mmol), and the mixture was stirred at 100 °C for 3 h. After the reaction was completed, urea (9 g, 150 mmol) was added, and the mixture was stirred at 120 °C overnight. The reaction liquid was poured into ice water, and concentrated hydrochloric acid was added while stirring. The mixture was left to stand at low temperature overnight, filtered, and washed with water. The filter cake was dried to give 5-(2,4-dioxotetrahydropyrimidin-1(2*H*)-yl)-2-fluoro-4-methylbenzoic acid.

LC-MS: (ESI, m/z): [M+H]⁺ = 267.1.

### Step 3: preparation of 3-(5-(2,4-dioxotetrahydropyrimidin-1(2H)-yl)-2-fluoro-4-methylbenzoyl)-3-azaspiro[5.5]undecane-9-carbaldehyde

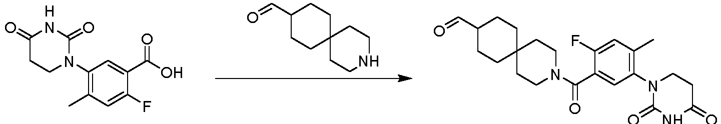

HATU (502 mg, 1.3 mmol) and DIEA (430 mg, 3.3 mmol) were added to a solution of 5-(2,4-dioxotetrahydropyrimidin-1(2*H*)-yl)-2-fluoro-4-methylbenzoic acid (350 mg, 1.3 mmol) in DMF (10 mL), and then 3-azaspiro[5.5]undecane-9-carbaldehyde (200 mg, 1.1 mmol) was added. The mixture was stirred at room temperature overnight. After the reaction was completed, H₂O (50 mL) was added, and the mixture was extracted with ethyl acetate (50 mL × 3). The combined organic phase was dried over anhydrous sodium sulfate, filtered, and concentrated in vacuum under reduced pressure. The crude product was purified by silica gel column chromatography (DCM:MeOH = 20:1) to give 3-(5-(2,4-dioxotetrahydropyrimidin-1(2H)-yl)-2-fluoro-4-methylbenzoyl)-3-azaspiro[5.5]undecane-9-carbaldehyde.

LC-MS: (ESI, m/z): [M+H]⁺ = 430.2.

### Intermediate 28: tert-butyl 3-(8-fluoro-7-(7-fluoro-3-(methoxymethoxy)-8-((triisopropylsilyl)ethynyl) naphthalen-1-yl)-2-((1-formylcyclopropyl)methoxy)quinazolin-4-yl)-3,8-diazabicyclo[3.2.1]octane-8-carboxylate

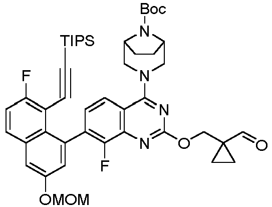

Reference was made to the preparation method for intermediate 17.

### Intermediate 29: 3-(3-(2,4-dioxotetrahydropyrimidin-1(2H)-yl)-4-ethylbenzoyl)-3-azaspiro[5.5]undecane-9-carbaldehyde

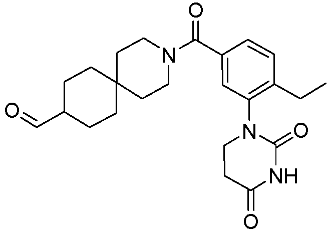

Reference was made to the preparation method for intermediate 26.

Intermediate 30: (R)-3-(4-benzylpiperazin-1-yl)-2-methoxypropan-1-ol

### Step 1: preparation of (R)-1-(4-benzylpiperazin-1-yl)-3-((tert-butyldimethylsilyl)oxy)propan-2-ol

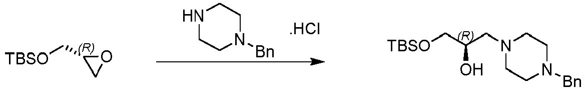

A mixed solution of (R)-tert-butyldimethyl(oxiran-2-ylmethoxy)silane (20 g, 106 mmol), N-benzylpiperazine hydrochloride (25 g, 117 mmol), and potassium carbonate (29 g, 212 mmol) in THF (15 mL) was heated at reflux for 3 h. After the reaction was completed, the mixture was filtered to remove the solid, and the filtrate was concentrated to give a crude product. The resulting crude product was purified by silica gel column chromatography (PE:EA = 2:1) to give (R)-1-(4-benzylpiperazin-1-yl)-3-((tertbutyldimethylsilyl)oxy)
propan-2-ol.

LC-MS: (ESI, m/z): [M+H]⁺ = 365.3.

### Step 2: preparation of (R)-1-benzyl-4-(3-((tert-butyldimethylsilyl)oxy)-2-methoxypropyl)piperazine

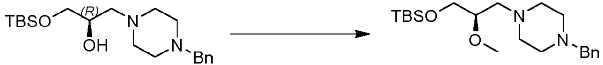

(*R*)-1-(4-Benzylpiperazin-1-yl)-3-((tert-butyldimethylsilyl)oxy)propan-2-ol (20.0 g, 55 mmol) was dissolved in THF (200 mL), and NaH (2.6 g, 110 mmol) was added at 0 °C. The mixture was reacted at 25 °C for 30 min and then cooled to 0 °C. Iodomethane (9.7 g, 69 mmol) was added, and the mixture was reacted at room temperature for 3 h. After the reaction was completed, the reaction liquid was slowly poured into a cooled aqueous NH₄Cl solution (200 mL), and then the mixture was extracted with ethyl acetate (200 mL × 3). The organic phases were combined, washed with a saturated NaCl solution, dried over anhydrous Na₂SO₄, and filtered. The filtrate was concentrated, and the resulting crude product was purified by silica gel column chromatography (PE:EA = 5:1) to give (R)-1-benzyl-4-(3-((tert-butyldimethylsilyl)oxy)-2-methoxypropyl)piperazine (e.e. = 99.3%).

Chiral resolution: chromatography column: IG 5 µm; column size: 4.6 × 250 mm; mobile phase: Hex:EtOH:DEA = 90:2:0.2; flow rate: 1 mL/min 254 nm; column temperature: 30 °C.

LC-MS: (ESI, m/z): [M+H]⁺ = 379.2.

### Step 3: preparation of (R)-3-(4-benzylpiperazin-1-yl)-2-methoxypropan-1-ol

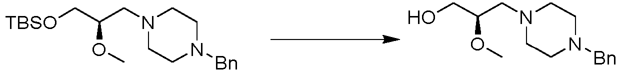

(R)-1-Benzyl-4-(3-((tert-butyldimethylsilyl)oxy)-2-methoxypropyl)piperazine (15.0 g, 40 mmol) was dissolved in ethanol (100 mL). The mixture was cooled to 0-5 °C, and concentrated hydrochloric acid (40 mL) was added. The mixture was reacted at room temperature for 1 h. After the reaction was completed, the reaction liquid was concentrated to remove ethanol, and water (100 mL) was added to the concentrate. The mixture was extracted with methyl *tert-butyl* ether (100 mL). The aqueous phase was adjusted to pH = 8-9 with an aqueous sodium carbonate solution, and the mixture was extracted with ethyl acetate (100 mL × 3). The combined EA phase was dried over anhydrous sodium sulfate and filtered, and the filtrate was concentrated to give (R)-3-(4-benzylpiperazin-1-yl)-2-methoxypropan-1-ol, which was directly used in the next step.

Intermediate 31: *tert-butyl* 3-(8-fluoro-7-(7-fluoro-3-(methoxymethoxy)-8-((triisopropylsilyl)ethynyl) naphthalen-1-yl)-2-((R)-2-methoxy-3-(piperazin-1-yl)propoxy)pyrido[4,3-*d*]pyrimidin-4-yl)-3,8-diazabicyclo[3.2.1]octane-8-carboxylate

### Step 1: preparation of tert-butyl 3-(2-((R)-3-(4-benzylpiperazin-1-yl)-2-methoxypropoxy)-7-chloro-8-fluoropyrido[4,3-djpyrimidin-4-yl)-3,8-diazabicyclo[3.2.1]octane-8-carboxylate

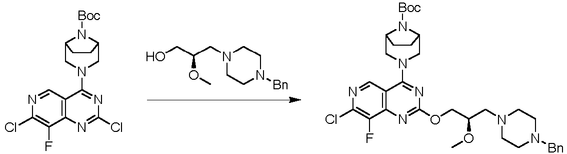

*tert*-Butyl 3-(2,7-dichloro-8-fluoropyrido[4,3-djpyrimidin-4-yl)-3,8-diazabicyclo[3.2.1]octane-8-carboxylate (1.5 g, 3.50 mmol) and (R)-3-(4-benzylpiperazin-1-yl)-2-methoxypropan-1-ol (1.11 g, 4.20 mmol) were dissolved in ACN (15 mL), and Cs₂CO₃ (3.42 g, 10.5 mmol) and DABCO (79 mg, 0.70 mmol) were added. The mixture was reacted at room temperature for 1 h. After the reaction was completed, the mixture was filtered to remove the solid, and the filter cake was washed twice with dichloromethane. The filtrate was concentrated, and the resulting crude product was purified by silica gel column chromatography (PE:EA = 2:1) to give *tert-butyl* 3-(2-((R)-3-(4-benzylpiperazin-1-yl)-2-methoxypropoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 656.3.

### Step 2: preparation of tert-butyl 3-(2-((R)-3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

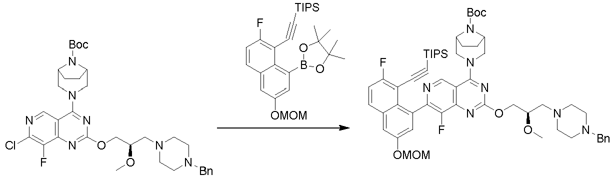

Cata CXium Pd G₃ (267 mg, 0.366 mmol) and cesium carbonate (1.79 g, 5.49 mmol) were added to a mixed solution of *tert-*butyl 3-(2-((*R*)-3-(4-benzylpiperazin-1-yl)-2-methoxypropoxy)-7-chloro-8-fluoropyrido[4,3-*d*]pyrimidin-4-yl)-3,8-diazabicyclo[3.2.1]octane-8-carboxylate (1.2 g, 1.83 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.13 g, 2.20 mmol) in 1,4-dioxane (8 mL) and H₂O (1.6 mL). The mixture was reacted at 85 °C for 16 h under nitrogen atmosphere. Water (20 mL) was added to the reaction liquid, and then the mixture was extracted with ethyl acetate (30 mL × 3). The combined organic phase was dried over anhydrous sodium sulfate and concentrated under reduced pressure. The concentrate was purified by silica gel column chromatography (DCM:MeOH = 20:1) to give *tert*-butyl 3-(2-((R)-3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate.

LC-MS: (ESI, m/z): [M+H]⁺ = 1006.6.

### Step 3: preparation of tert-butyl 3-(8-fluoro-7-(7-fluoro-3-(methoxymethoxy)-8-((triisopropylsilyl) ethynyl)naphthalen-1-yl)-2-((R)-2-methoxy-3-(piperazin-1-yl)propoxy)pyrido[4,3-d]pyrimidin-4-yl)-3,8-diazabicyclo[3.2.1]octane-8-carboxylate

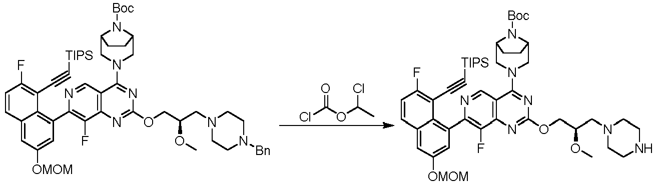

*tert*-Butyl 3-(2-((R)-3-(4-benzylpiperazin-1-yl)-2-methoxypropoxy)-8-fluoro-7-(7-fluoro-3-(methoxymethoxy)-8-((triisopropylsilyl)ethynyl )naphthalen-1-yl)pyrido[4,3-d]pyrimidin-4-y1)-3,8-diazabicyclo[3.2.1]octane-8-carboxylate (1.3 g, 1.29 mmol) was dissolved in DCM (5 mL), and 1-chloroethyl carbonochloridate (553 mg, 3.87 mmol) and DIEA (500 mg, 3.87 mmol) were added. The mixture was reacted at room temperature for 1 h. The reaction liquid was directly concentrated under reduced pressure, and the concentrate was dissolved in MeOH (5 mL). The mixture was then heated to 50 °C and reacted for 30 min. After the reaction was completed, the mixture was concentrated under reduced pressure, and the concentrate was purified by silica gel column chromatography (DCM:MeOH (NH₃) = 15:1) to give *tert-butyl* 3-(8-fluoro-7-(7-fluoro-3-(methoxymethoxy)-8-((triisopropylsilyl)ethynyl)naphthalen-1-yl)-2-((*R*)-2-methoxy-3-(piperazin-1-yl)propoxy)pyrido[4,3-*d*]pyrimidin-4-yl)-3,8-diazabicyclo[3.2.1]octane-8-carboxylate.

LC-MS: (ESI, m/z): [M+H]⁺ = 916.5.

### Intermediate 32: tert-butyl 3-(8-fluoro-7-(7-fluoro-3-(methoxymethoxy)-8-((triisopropylsilyl)ethynyl) naphthalen-1-yl)-2-((R)-2-methyl-3-(piperazin-1-yl)propoxy)quinazolin-4-yl)-3,8-diazabicyclo[3.2.1]octane-8-carboxylate

### Step 1: preparation of tert-butyl 3-(7-bromo-2-chloro-8-fluoroquinazolin-4-yl)-3,8-diazabicyclo[3.2.1]octane-8-carboxylate

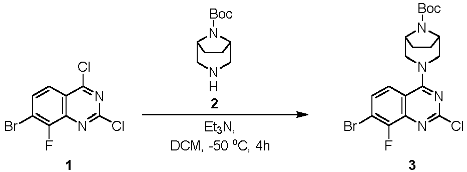

Triethylamine (5.129 g, 50.689 mmol) and *tert*-butyl 3,8-diazabicyclo[3.2.1]octane-8-carboxylate (3.659 g, 17.234 mmol) were added to a solution of 7-bromo-2,4-dichloro-8-fluoroquinazoline (5.000 g, 16.896 mmol) in dichloromethane (150 mL) at -50 °C, and the mixture was stirred at -50 °C for 4 h. Anhydrous tetrahydrofuran (60 mL) was added, and the mixture was stirred for 30 min and filtered to remove the insoluble substances. The filtrate was concentrated, and petroleum ether/tetrahydrofuran (10/1, 50 mL) was added to the concentrate. The mixture was stirred and filtered to give *tert*-butyl 3-(7-bromo-2-chloro-8-fluoroquinazolin-4-yl)-3,8-diazabicyclo[3.2.1]octane-8-carboxylate.

LC-MS: (ESI, m/z): [M+H]⁺ = 471.1.

### Step 2: preparation of tert-butyl 3-(2-((R)-3-(4-benzylpiperazin-1-yl)-2-methylpropoxy)-7-bromo-8-fluoroquinazolin-4-yl)-3,8-diazabicyclo[3.2.1]octane-8-carboxylate

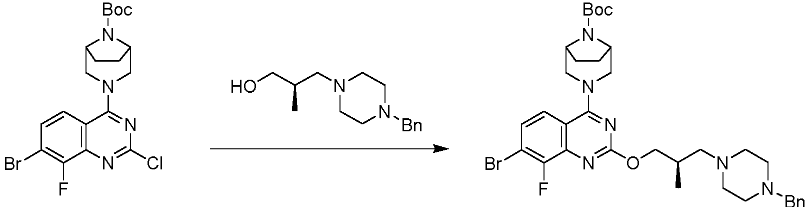

Triethylenediamine (0.143 g, 1.272 mmol) and cesium carbonate (4.143 g, 12.717 mmol) were added to a stirred solution of *tert-butyl* 3-(7-bromo-2-chloro-8-fluoroquinazolin-4-yl)-3,8-diazabicyclo[3.2.1]octane-8-carboxylate (2.000 g, 4.239 mmol) and (R)-3-(4-benzylpiperazin-1-yl)-2-methylpropan-1-ol (1.369 g, 5.511 mmol) in anhydrous acetonitrile (50 mL) at 25 °C, and the mixture was stirred at 25 °C for 18 h. After the reaction was completed, the reaction liquid was poured into water (150 mL), and the mixture was extracted with ethyl acetate (50 mL × 3). The organic phase was washed with saturated brine (150 mL), and the combined organic phase was dried over anhydrous sodium sulfate, filtered, and concentrated under reduced pressure. The resulting crude product was purified by silica gel column chromatography (petroleum ether:tetrahydrofuran = 20:1 to 4:1) to give *tert-butyl* 3-(2-((R)-3-(4-benzylpiperazin-1-yl)-2-methylpropoxy)-7-bromo-8-fluoroquinazolin-4-yl)-3,8-diazabicyclo[3.2.1]octane-8-carboxylate.

LC-MS: (ESI, m/z): [M+H]⁺ = 683.2.

### Step 3: preparation of tert-butyl 3-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

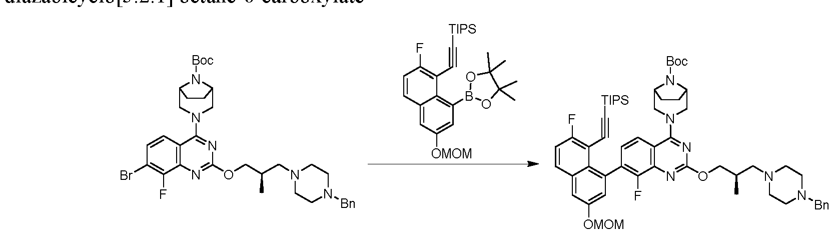

Cesium carbonate (3.366 g, 10.330 mmol) and 1,1'-bis(di-*tert*-butylphosphine)ferrocene palladium dichloride (0.449 g, 0.689 mmol) were added to a solution of *tert-butyl* 3-(2-((R)-3-(4-benzylpiperazin-1-yl)-2-methylpropoxy)-7-bromo-8-fluoroquinazolin-4-yl)-3,8-diazabicyclo[3.2.1]octane-8-carboxylate (2.354 g, 3.443 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (2.118 g, 4.132 mmol) in 1,4-dioxane (25 mL) and water (5 mL) at 25 °C, and the mixture was stirred at 85 °C for 16 h. After the reaction was completed, the reaction liquid was poured into water (100 mL), and the mixture was extracted with ethyl acetate (20 mL × 3). The organic phase was washed with saturated brine (100 mL), and the combined organic phase was dried over anhydrous sodium sulfate, filtered, and concentrated under reduced pressure. The resulting crude product was purified by silica gel column chromatography (dichloromethane:methanol = 600:1 to 30:1) to give *tert-butyl* 3-(2-((R)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 989.5.

### Step 4: preparation of tert-butyl 3-(8-fluoro-7-(7-fluoro-3-(methoxymethoxy)-8-((triisopropylsilyl) ethynyl)naphthalen-1-yl)-2-((R)-2-methyl-3-(piperazin-1-yl)propoxy)quinazolin-4-yl)-3,8-diazabicyclo [3.2.1]octane-8-carboxylate

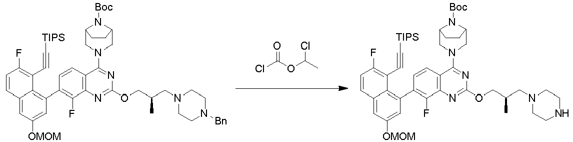

1-Chloroethyl chloroformate (1.200 g, 8.391 mmol) was added to a stirred solution of *tert*-butyl 3-(2-((R)-3-(4-benzylpiperazin-1-yl)-2-methylpropoxy)-8-fluoro-7-(7-fluoro-3-(methoxymethoxy)-8-((triisopropylsilyl) ethynyl)naphthalen-1-yl)quinazolin-4-yl)-3,8-diazabicyclo[3.2.1]octane-8-carboxylate (2.767 g, 2.797 mmol) and *N,N*-diisopropylethylamine (1.085 g, 8.391 mmol) in dichloromethane (80 mL) at 25 °C. The mixture was stirred at 25 °C for 1 h and concentrated under reduced pressure, and methanol (80 mL) was added to the concentrate for dissolution. The mixture was stirred at 50 °C for 1 h and concentrated under reduced pressure again. The crude product was purified by silica gel column chromatography (petroleum ether:tetrahydrofuran:methanol = 1:1:0.01 to 1:1:0.1) to give *tert-butyl* 3-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)quinazolin-4-yl)-3,8-diazabicyclo[3.2.1]octane-8-carboxylate.

### LC-MS: (ESI, m/z): [M+H]⁺ = 899.4.

### Example 1: 1-(5-( 4-(3-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1H,3H)-dione

### Step 1: preparation of benzyl 4-(3-oxopropyl)piperidine-1-carboxylate

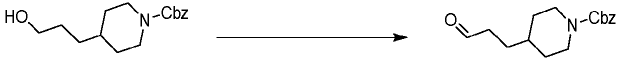

Dess-Martin reagent (5.5 g, 13.0 mmol) was added in batches to a solution of benzyl 4-(3-hydroxypropyl)piperidine-1-carboxylate (3.0 g, 10.8 mmol) in DCM (60 mL) at 0 °C, and the mixture was reacted for 1 h. After the reaction was completed, the reaction liquid was quenched with a saturated aqueous Na₂S₂O₃ solution (50 mL) and an aqueous NaHCO₃ solution (50 mL) and extracted with DCM (100 mL × 3). The organic phases were combined, washed with saturated brine (100 mL), dried over sodium sulfate, filtered, and concentrated under reduced pressure. The crude product was purified by silica gel column chromatography (EA:PE = 3:17 to 3:7) to give benzyl 4-(3-oxopropyl)piperidine-1-carboxylate.

¹H NMR (400 MHz, CDCl₃) δ 9.78 (s, 1H), 7.38-7.28 (m, 5H), 5.12 (s, 2H), 4.28-4.02 (m, 2H), 2.90-2.65 (m, 2H), 2.51-2.42 (m, 2H), 1.74-1.54 (m, 4H), 1.51-1.37 (m, 1H), 1.21-1.03 (m, 2H).

### Step 2: preparation of tert-butyl 3-(2-(((S)-1-(3-(1-((benzyloxy)carbonyl)piperidin-4-yl)propyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

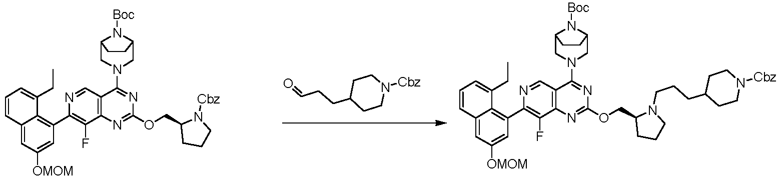

*tert-Butyl* 3-(2-(((*S*)-1-((benzyloxy)carbonyl)pyrrolidin-2-yl)methoxy)-7-(8-ethyl-3-(methoxymethoxy) naphthalen-1-yl)-8-fluoropyrido[4,3-*d*]pyrimidin-4-yl)-3,8-diazabicyclo[3.2.1]octane-8-carboxylate (360 mg, 0.45 mmol) was dissolved in ethanol (10 mL) and acetic acid (1 mL), and palladium on carbon (100 mg) was added. The reaction liquid was purged three times under hydrogen atmosphere and reacted at 15 °C for 1 h. After the reaction was completed, the mixture was filtered, and the filtrate was directly used in the next step. The above filtrate was cooled to 0-5 °C, NaBH(OAc)₃ (243.80 mg, 1.15 mmol) was added, and then benzyl 4-(3-oxopropyl)piperidine-1-carboxylate was added. The mixture was reacted at 15 °C for another 30 min. An aqueous sodium bicarbonate solution was added to quench the reaction, and the mixture was extract with EtOAc (30 mL × 2). The organic phase was washed with a saturated NaCl solution, dried over anhydrous Na₂SO₄, and filtered, and the filtrate was concentrated to give a crude product, which was purified by silica gel column chromatography (DCM:MeOH = 80:1) to give *tert-butyl* 3-(2-(((*S*)-1-(3-(1-((benzyloxy)carbonyl)piperidin-4-yl)propyl)pyrrolidin-2-yl)methoxy)-7-(8-ethyl-3-(methoxymethoxy) naphthalen-1-yl)-8-fluoropyrido[4,3-*d*jpyrimidin-4-yl)-3,8-diazabicyclo[3.2.1]octane-8-carboxylate.

LC-MS: (ESI, m/z): [M+H]⁺= 932.1.

### Step 3: preparation of tert-butyl 3-(7-(8-ethyl-3-(methoxymethoxy)naphthalen-1-yl)-8-fluoro-2-((((S)-1-(3-(piperidin-4-yl)propyl]pyrrolidin-2-yl)methoxy)pyrido[4,3-d]pyrimidin-4-yl)-3,8-diazabicyclo[3.2.1]octane-8-carboxylate

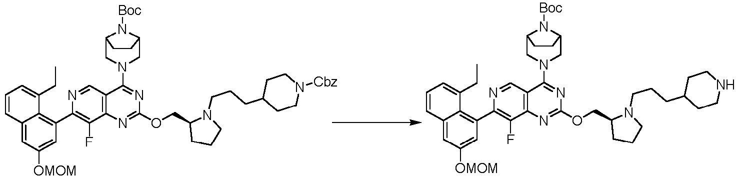

*tert-Butyl* 3-(2-(((*S*)-1-(3-(1-((benzyloxy)carbonyl)piperidin-4-yl)propyl)pyrrolidin-2-yl)methoxy)-7-(8-ethyl-3-(methoxymethoxy)naphthalen-1-yl)-8-fluoropyrido[4,3-*d*jpyrimidin-4-yl)-3,8-diazabicyclo[3.2.1] octane-8-carboxylate (230 mg, 0.25 mmol) was dissolved in ethanol (10 mL), and palladium on carbon (80 mg) was added. The reaction liquid was purged three times under hydrogen atmosphere and reacted at 15 °C for 3 h. After the reaction was completed, the mixture was filtered, and the filtrate was concentrated under reduced pressure to give *tert-butyl* 3-(7-(8-ethyl-3-(methoxymethoxy)naphthalen-1-yl)-8-fluoro-2-((((*S*)-1-(3-(piperidin-4-yl)propyl]pyrrolidin-2-yl)methoxy)pyrido[4,3-*d*]pyrimidin-4-yl)-3,8-diazabicyclo[3.2.1]octane-8-carboxylate, which was directly used in the next step without purification.

LC-MS: (ESI, m/z): [M+H]⁺= 798.3.

### Step 4: preparation of tert-butyl 3-(2-(((S)-1-(3-(1-(4-chloro-3-(2,4-dioxotetrahydropyrimidin-1(2H)-yl)benzoyl)piperidin-4-yl)propyl)pyrrolidin-2-yl)methoxy)-7-(8-ethyl-3-(methoxymethoxy)naphthalen-1-yl)-8-fluoropyrido[4,3-dj]yrimidin-4-yl)-3,8-diazabicyclo[3.2.1]octane-8-carboxylate

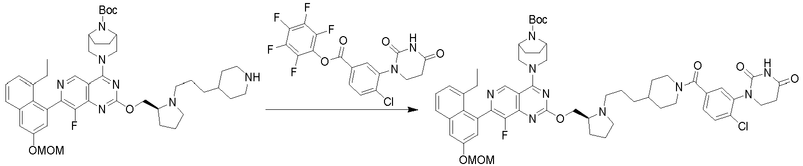

*tert-Butyl* 3-(7-(8-ethyl-3-(methoxymethoxy)naphthalen-1-yl)-8-fluoro-2-((((*S*)-1-(3-(piperidin-4-yl)propyl] pyrrolidin-2-yl)methoxy)pyrido[4,3-d]pyrimidin-4-yl)-3,8-diazabicyclo[3.2.1]octane-8-carboxylate (160 mg, 0.20 mmol) and DIEA (51.60 mg, 0.40 mmol) were dissolved in DMSO (2 mL), and pentafluorophenyl 4-chloro-3-(2,4-dioxotetrahydropyrimidin-1-yl)benzoate (87 mg, 0.20 mmol) was added. The mixture was reacted at room temperature for 1 h. Water (30 mL) was added, and the mixture was extracted with EtOAc (30 mL × 2). The organic phase was washed with a saturated NaCl solution (50 mL × 3), dried over anhydrous Na₂SO₄, and filtered, and the filtrate was concentrated to give a crude product, which was purified by silica gel column chromatography (DCM:MeOH = 20:1) to give *tert-butyl* 3-(2-(((S)-1-(3-(1-(4-chloro-3-(2,4-dioxotetrahydropyrimidin-1(2H)-yl)benzoyl)piperidin-4-yl)propyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate.

LC-MS: (ESI, m/z): [M+H]⁺= 1048.6.

### Step 5: preparation of 1-(5-(4-(3-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1H,3H)-dione

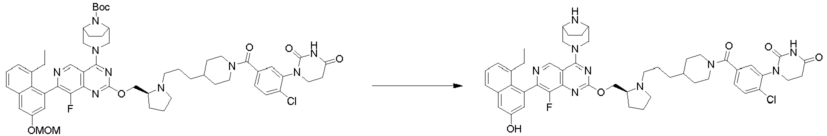

*tert-Butyl* 3-(2-(((*S*)-1-(3-(1-(4-chloro-3-(2,4-dioxotetrahydropyrimidin-1(2H)-yl)benzoyl)piperidin-4-yl)propyl)pyrrolidin-2-yl)methoxy)-7-(8-ethyl-3-(methoxymethoxy)naphthalen-1-yl)-8-fluoropyrido[4,3-djpyrimidin-4-yl)-3,8-diazabicyclo[3.2.1]octane-8-carboxylate (100 mg, 0.095 mmol) was dissolved in DCM (2 mL), and TFA (1 mL) was added. The mixture was reacted at 15 °C for 1 h. The reaction liquid was concentrated, diluted with DCM (20 mL), and adjusted to pH > 7 with an aqueous NaHCO₃ solution. The organic phase was separated, washed with a saturated NaCl solution, dried over anhydrous Na₂SO₄, and filtered, and the filtrate was concentrated to give a crude product, which was purified by preparative high-performance liquid chromatography to give 1-(5-(4-(3-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1*H*,3*H*)-dione.

LC-MS: (ESI, m/z): [M+H]⁺= 904.3.

¹H NMR (400 MHz, CD₃OD) δ 9.04 (s, 1H), 7.64-7.56 (m, 2H), 7.46 (s, 1H), 7.38-7.31 (m, 2H), 7.27 (s, 1H), 7.15 (d, *J=* 7.2 Hz, 1H), 7.00 (d, *J=* 2.8 Hz, 1H), 4.66-4.34 (m, 5H), 3.84-3.59 (m, 7H), 3.20-3.15 (m, 1H), 3.04-2.66 (m, 6H), 2.45-2.20 (m, 4H), 2.09-2.00 (m, 1H), 1.90-1.49 (m, 12H), 1.33-0.98 (m, 4H), 0.89 (t, *J=* 7.2 Hz, 3h).

### Example 2: 1-((S)-2-(3-(3-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)propoxy)propanamido)-3,3-dimethylbutanoyl)-4-hydroxy-N-(4-(4-methylthiazol-5-yl)phenyl)pyrrolidine-2-carboxamide

### Step 1: preparation of benzyl 3-(3-hydroxypropoxy)propanoate

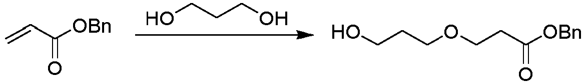

Triton B (82.00 g, 0.123 mol, 25% aqueous solution) was added to a solution of benzyl acrylate (20.0 g, 0.123 mol) and propane-1,3-diol (46.89 g, 0.617 mol) in acetonitrile (250 mL) at room temperature. The reaction liquid was stirred at room temperature overnight. The reaction mixture was added to brine, and the mixture was extracted with EtOAc (200 mL × 2). The organic phase was dried over Na₂SO₄ and filtered, and the filtrate was concentrated to give a crude product, which was purified by silica gel column chromatography (DCM:MeOH = 5:1) to give benzyl 3-(3-hydroxypropoxy)propanoate.

LC-MS: (ESI, m/z): [M+H]⁺= 239.1.

### Step 2: preparation of benzyl 3-(3-oxopropoxy)propanoate

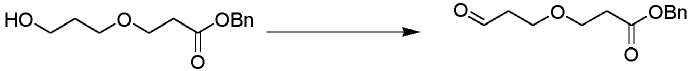

A solution of benzyl 3-(3-hydroxypropoxy)propanoate (1.90 g, 7.98 mmol) in dichloromethane (30 mL) was cooled to 0-10 °C, and Dess-Martin oxidant (4.06 g, 9.58 mmol) was added in batches. The resulting reaction liquid was stirred at room temperature for 1.5 h. An aqueous sodium thiosulfate solution and an aqueous sodium bicarbonate solution were added to quench the reaction, and the mixture was extracted with dichloromethane (30 mL × 2). The combined organic phase was washed with saturated brine, dried over anhydrous sodium sulfate, and filtered. The filtrate was concentrated under reduced pressure, and the resulting crude product was purified by silica gel column chromatography (PE:EtOAc = 5:1) to give benzyl 3-(3-oxopropoxy)propanoate.

¹H NMR (400 MHz, CDCl₃) δ 9.76 (t, *J* = 1.6 Hz, 1H), 7.37-7.28 (m, 5H), 4.52 (s, 2H), 4.44 (t, *J* = 6.0 Hz, 2H), 3.73 (t, *J* = 6.0 Hz, 2H), 2.79-2.73 (m, 2H), 2.63-2.58 (m, 2H).

### Step 3: preparation of tert-butyl 3-(2-(((S)-1-(3-(3-(benzyloxy)-3-oxopropoxy)propyl)pyrrolidin-2-yl)methoxy)-7-(8-ethyl-3-(methoxymethoxy)naphthalen-1-yl)-8-fluoropyrido [4,3-d]pyrimidin-4-yl)-3,8-diazabicyclo[3.2.1]octane-8-carboxylate

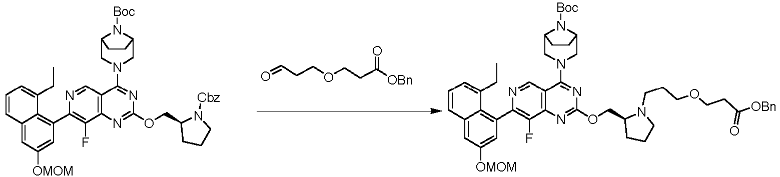

*tert-Butyl* 3-(2-(((S)-1-((benzyloxy)carbonyl)pyrrolidin-2-yl)methoxy)-7-(8-ethyl-3-(methoxymethoxy) naphthalen-1-yl)-8-fluoropyrido[4,3-d]pyrimidin-4-yl)-3,8-diazabicyclo[3.2.1]octane-8-carboxylate (300 mg, 0.37 mmol) was dissolved in ethanol (9 mL)/acetic acid (1 mL), and palladium on carbon (100 mg) was added. The mixture was purged three times under hydrogen atmosphere and reacted at 10 °C for 1 h. The reaction liquid was filtered through celite, and the filtrate was directly used in the next step. The filtrate was cooled to 0-5 °C, NaBH(OAc)₃ (196.10 mg, 0.93 mmol) was added, and then benzyl 3-(3-oxopropoxy)propanoate (174.64 mg, 0.74 mmol) was added. The mixture was stirred at 15 °C for 30 min. After the reaction was completed, an aqueous sodium bicarbonate solution was added, and the mixture was extracted with ethyl acetate (30 mL × 2). The combined organic layer was washed with brine, dried over anhydrous sodium sulfate, and filtered. The filtrate was concentrated under reduced pressure to give a crude product, which was purified by a preparative thin-layer plate (DCM:MeOH = 10:1) to give tert-butyl 3-(2-(((S)-1-(3-(3-(benzyloxy)-3-oxopropoxy)propyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate.

LC-MS: (ESI, m/z): [M/2+H]⁺= 447.3.

### Step 4: preparation of 3-(3-((S)-2-(((4-(8-(tert-butoxycarbonyl)-3,8-diazabicyclo[3.2.1]octan-3-yl)-7-(8-ethyl-3-(methoxymethoxy)naphthalen-1-yl)-8-fluoropyrido[4,3-d]pyrimidin-2-yl)oxy)methyl)pyrrolidin-1-yl)propoxy)propanoic acid

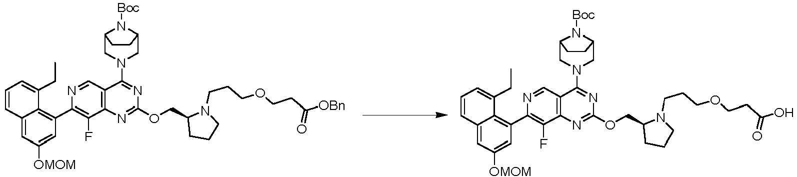

*tert-Butyl* 3-(2-(((S)-1-(3-(3-(benzyloxy)-3-oxopropoxy)propyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate (50.0 mg, 0.06 mmol) was dissolved in ethyl acetate (3 mL), and palladium on carbon (20 mg) was added. The mixture was purged three times under hydrogen atmosphere and stirred at 30 °C for 2 h. The reaction liquid was filtered through celite, and the filtrate was concentrated under reduced pressure. The resulting crude product was purified by a preparative thin-layer plate (DCM:MeOH = 5:1) to give 3-(3-((*S*)-2-(((4-(8-(tert-butoxycarbonyl)-3,8-diazabicyclo[3.2.1]octan-3-yl)-7-(8-ethyl-3-(methoxymethoxy) naphthalen-1-yl)-8-fluoropyrido[4,3-d]pyrimidin-2-yl)oxy)methyl)pyrrolidin-1-yl)propoxy)propanoic acid.

LC-MS: (ESI, m/z): [M/2+H]⁺= 402.3.

### Step 5: preparation of tert-butyl 3-(7-(8-ethyl-3-(methoxymethoxy)naphthalen-1-yl)-8-fluoro-2-(((S)-1-(3-(3-(((S)-1-((2S,4R)-4-hydroxy-2-((4-(4-methylthiazol-5-yl)phenyl)carbamoyl)pyrrolidin-1-yl)-3,3-dimethyl-1-oxobutan-2-yl)amino)-3-oxopropoxy)propyl)pyrrolidin-2-yl)methoxy)pyrido[4,3-d]pyrimidin-4-yl)-3,8-diazabicyclo[3.2.1]octane-8-carboxylate

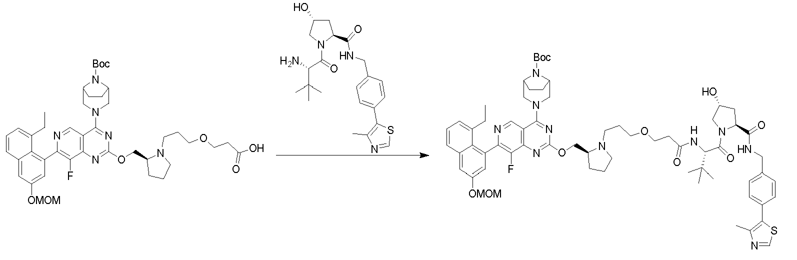

3-(3-((*S*)-2-(((4-(8-(tert-Butoxycarbonyl)-3,8-diazabicyclo[3.2.1]octan-3-yl)-7-(8-ethyl-3-(methoxymethoxy) naphthalen-1-yl)-8-fluoropyrido[4,3-*d*]pyrimidin-2-yl)oxy)methyl)pyrrolidin-1-yl)propoxy)propanoic acid (33 mg, 0.041 mmol) and HATU (18.62 mg, 0.049 mmol) were dissolved in DMF (1 mL). The mixture was stirred for 3 min, and DIEA (10.58 mg, 0.82 mmol) and (2S,4R)-1-((S)-2-amino-3,3-dimethylbutanoyl)-4-hydroxy-*N*-(4-(4-methylthiazol-5-yl)phenyl)pyrrolidine-2-carboxamide (21.18 mg, 0.049 mmol) were added sequentially. The reaction liquid was stirred at 25 °C for 1 h. The reaction liquid was diluted with water (30 mL) and extracted with ethyl acetate (10 mL × 2). The organic layer was washed with brine, dried over anhydrous sodium sulfate, and filtered. The filtrate was concentrated under reduced pressure, and the resulting crude product was purified by a preparative thin-layer plate (DCM:MeOH = 5:1) to give *tert-butyl* 3-(7-(8-ethyl-3-(methoxymethoxy)naphthalen-1-yl)-8-fluoro-2-(((*S*)-1-(3-(3-(((S)-1-((2S,4R)-4-hydroxy-2-((4-(4-methylthiazol-5-yl)phenyl)carbamoyl)pyrrolidin-1-yl)-3,3-dimethyl-1-oxobutan-2-yl)amino)-3-oxopropoxy) propyl)pyrrolidin-2-yl)methoxy)pyrido [4,3-d]pyrimidin-4-yl)-3,8-diazabicyclo[3.2.1]octane-8-carboxylate.

LC-MS: (ESI, m/z): [M/2+H]⁺= 608.5.

### Step 6: preparation of 1-((S)-2-(3-(3-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)propoxy) propanamido)-3,3-dimethylbutanoyl)-4-hydroxy-N-(4-(4-methylthiazol-5-yl)phenyl)pyrrolidine-2-carboxamide

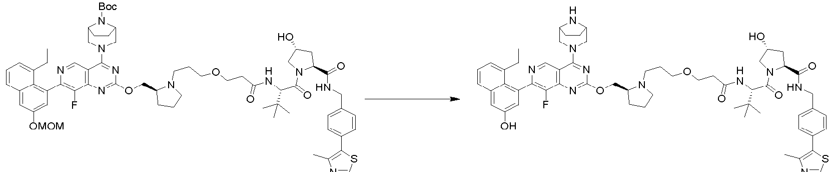

*tert-Butyl* 3-(7-(8-ethyl-3-(methoxymethoxy)naphthalen-1-yl)-8-fluoro-2-(((S)-1-(3-(3-(((S)-1-((2S,4R)-4-hydroxy-2-((4-(4-methylthiazol-5-yl)phenyl)carbamoyl)pyrrolidin-1-yl)-3,3-dimethyl-1-oxobutan-2-yl) amino)-3-oxopropoxy)propyl)pyrrolidin-2-yl)methoxy)pyrido[4,3-d]pyrimidin-4-yl)-3,8-diazabicyclo [3.2.1]octane-8-carboxylate (30 mg, 0.0247 mmol) was dissolved in DCM (1 mL), and TFA (0.5 mL) was added. The mixture was stirred at 15 °C for 1 h. The reaction liquid was concentrated under reduced pressure, then dissolved in DCM (20 mL), and adjusted to pH > 7 with an aqueous NaHCO₃ solution. The organic phase was separated, washed with a saturated NaCl solution, dried over anhydrous Na₂SO₄, and filtered, and the filtrate was concentrated to give a crude product, which was purified by Pre-HPLC to give 1-((S)-2-(3-(3-((S)-2-(((4-(3,8-diazabicyclo[3.2.1]octan-3-yl)-7-(8-ethyl-3-hydroxynaphthalen-l-yl)-8-fluoropyrido [4,3-dJpyrimidin-2-yl)oxy)methyl)pyrrolidin-1-yl)propoxy)propanatnido)-3,3-dimethylbutanoyl)-4-hydroxy-N-(4-(4-methylthiazol-5-yl)phenyl)pyrrolidine-2-carboxamide.

LC-MS: (ESI, m/z): [M+H]⁺= 1071.3.

¹H NMR (400 MHz, CD₃OD) δ 9.03 (s, 1H), 8.85 (s, 1H), 7.62 (d*, J* = 8.4 Hz, 1H), 7.47-7.32 (m, 5H), 7.28 (d, *J=* 2.4 Hz, 1H), 7.15 (d, *J=* 7.2 Hz, 1H), 7.03-7.00 (m, 1H), 4.65-4.31 (m, 8H), 3.89-3.44 (m, 10H), 3.22-2.99 (m, 3H), 2.56-2.00 (m, 11H), 1.88-1.74 (m, 8H), 1.35-1.27 (m, 3H), 1.00 (s, 9H), 0.88 (*J* = 7.6 Hz, 3H).

### Example 3: 1-(5-(4-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)piperidine-1-carbonyl)-2-chlorophenyl)dihydropyrimidine-2,4(1H,3H)-dione

### Step 1: preparation of 2-(piperidin-4-yl)acetaldehyde

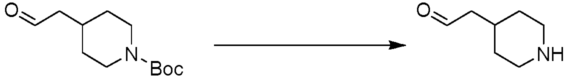

### TFA (2.0 mL) was added to a solution of tert-butyl 4-(2-oxoethyl)piperidine-1-carboxylate (400 mg, 1.76 mmol) in DCM (4.0 mL) at 0 °C, and the mixture was stirred at room temperature for 1 h. The reaction liquid was concentrated under reduced pressure to give a crude product of 2-(piperidin-4-yl)acetaldehyde, which was directly used in the next step without purification.

### Step 2: preparation of 2-(1-(4-chloro-3-(2,4-dioxotetrahydropyrimidin-1(2H)-yl)benzoyl)piperidin-4-yl)acetaldehyde

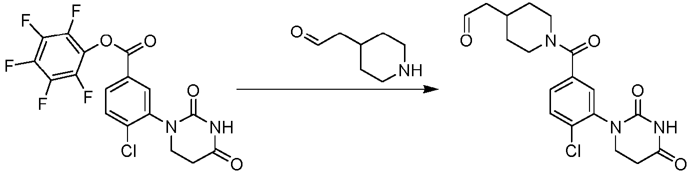

2-(Piperidin-4-yl)acetaldehyde (180 mg, 1.41 mmol) was added to a solution of pentafluorophenyl 4-chloro-3-(2,4-dioxotetrahydropyrimidin-1(2H)-yl)benzoate (477 mg, 1.10 mmol) and DIEA (568 mg, 4.40 mmol) in DMF (3 mL). The mixture was stirred at room temperature for 1 h and purified by reversed-phase silica gel column chromatography (C18, ACN:water = 0-100%) to give 2-(1-(4-chloro-3-(2,4-dioxotetrahydropyrimidin-1(2H)-yl)benzoyl)piperidin-4-yl)acetaldehyde.

LC-MS: (ESI, m/z): [M+H]⁺= 378.1.

### Step 3: preparation of tert-butyl 3-(2-((1-((4-(2-(1-(4-chloro-3-(2,4-dioxotetrahydropyrimidin-1(2H)-yl)benzoyl)piperidin-4-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

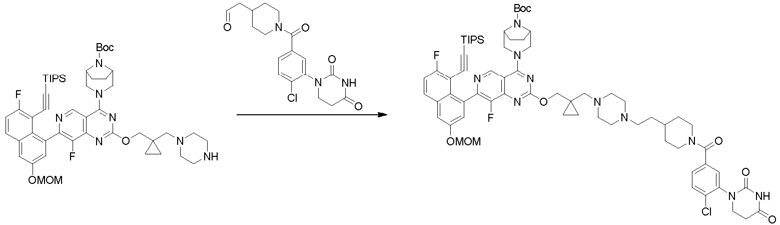

*tert*-Butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (62 mg, 0.068 mmol) and 2-(1-(4-chloro-3-(2,4-dioxotetrahydropyrimidin-1(2H)-yl)benzoyl)piperidin-4-yl)acetaldehyde (51 mg, 0.14 mmol) were dissolved in DCM/MeOH/AcOH (0.3 mL/0.2 mL/0.1 mL). The mixture was stirred at room temperature for 30 min. NaBH(OAc)₃ (19 mg, 0.22 mmol) was added, and the mixture was stirred at room temperature for 2 h. An aqueous NaHCO₃ solution was added, and the mixture was extracted with DCM (20 mL × 2). The organic phases were combined, washed with brine, dried, and concentrated. The crude product was purified by silica gel column chromatography (dichloromethane:methanol = 20:1) to give tert-butyl 3-(2-((1-((4-(2-(1-(4-chloro-3-(2,4-dioxotetrahydropyrimidin-1(2H)-yl)benzoyl)piperidin-4-yl)ethyl)piperazin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate.

LC-MS: (ESI, m/z): [M/2+H]⁺ = 637.2.

### Step 4: preparation of 1-(5-(4-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)piperidine-1-carbonyl)-2-chlorophenyl)dihydropyrimidine-2,4(1H,3H)-dione

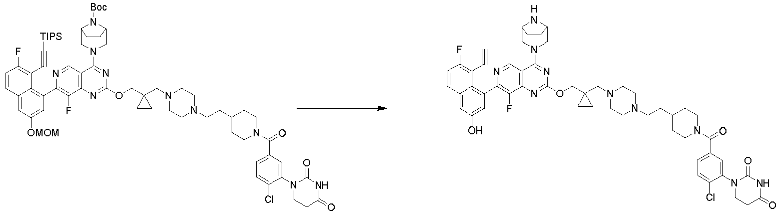

*tert*-Butyl 3-(2-((1-((4-(2-(1-(4-chloro-3-(2,4-dioxotetrahydropyrimidin-1(2H)-yl)benzoyl)piperidin-4-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate (54 mg, 0.042 mmol) was dissolved in DMF (2 mL), and cesium fluoride (32 mg, 0.21 mmol) was added. The mixture was reacted at room temperature for 30 min. Water (10 mL) was added, and the mixture was extracted with EtOAc (20 mL × 2). The organic phase was washed with brine, dried, and concentrated. The residue was dissolved in 1,4-dioxane (2 mL), and an HCl/dioxane solution (6 N, 1 mL) was added. The mixture was stirred at room temperature for 0.5 h and concentrated under reduced pressure. The resulting crude product was purified by Pre-HPLC to give the compound 1-(5-(4-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl) cyclopropyl)methyl)piperazin-1-yl)ethyl)piperidine-1-carbonyl)-2-chlorophenyl)dihydropyrimidine-2,4(1*H*,3*H*)-dione.

LC-MS: (ESI, m/z): [M+H]⁺= 973.2.

¹H NMR (400 MHz, CD₃OD) δ 8.99 (s, 1H), 7.88-7.81 (m, 1H), 7.63 (d, *J =* 8.4 Hz, 1H), 7.52 (s, 1H), 7.39 (d, *J=* 8.4 Hz, 1H), 7.36-7.27 (m, 2H), 7.19 (s, 1H), 4.70-4.47 (m, 4H), 4.41-4.32 (m, 1H), 3.81-3.62 (m, 7H), 3.31 (s, 1H), 3.10-3.00 (m, 1H), 2.96-2.73 (m, 4H), 2.67-2.27 (m, 11H), 1.89-1.76 (m, 4H), 1.67-1.54 (m, 2H), 1.49-1.42 (m, 2H), 1.35-1.11 (m, 3H), 0.76-0.66 (m, 2H), 0.55-0.43 (m, 2H).

### Example 4: 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione

### Step 1: preparation of 1-(2-chloro-5-(4-(3-hydroxypropyl)piperidine-1-carbonyl)phenyl)dihydropyrimidine-2,4(1H,3H)-dione

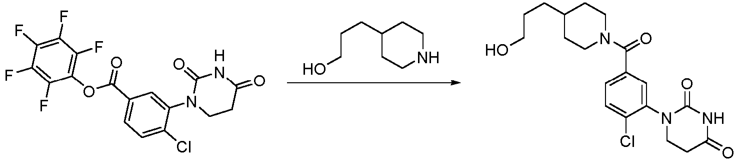

Pentafluorophenyl 4-chloro-3-(2,4-dioxotetrahydropyrimidin-1(2H)-yl)benzoate (1.6 g, 3.66 mmol) and DIEA (1.35 g, 10.47 mmol) were added to a solution of 3-(piperidin-4-yl)propan-1-ol (500 mg, 3.49 mmol) in DMSO (5 mL). The mixture was stirred at room temperature for 1 h and purified by Pre-HPLC (acetonitrile/water = 0-100%) to give 1-(2-chloro-5-(4-(3-hydroxypropyl)piperidine-1-carbonyl)phenyl)dihydropyrimidine-2,4(1H,3H)-dione.

LC-MS: (ESI, m/z): [M+H]⁺= 394.0.

### Step 2: preparation of 3-(1-(4-chloro-3-(2,4-dioxotetrahydropyrimidin-1(2H)-yl)benzoyl)piperidin-4-yl)propanal

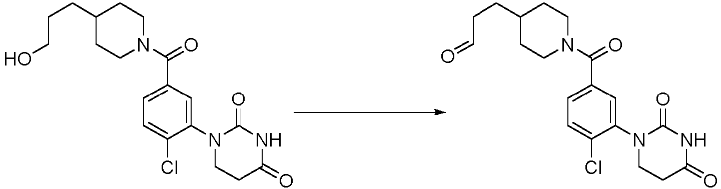

Dess-Martin reagent (452 mg, 1.07 mmol) was added to a solution of 1-(2-chloro-5-(4-(3-hydroxypropyl)piperidine-1-carbonyl)phenyl)dihydropyrimidine-2,4(1H,3H)-dione (350 mg, 0.89 mmol) in DCM (10 mL), and the mixture was reacted for one hour. The reaction liquid was filtered, the filtrate was concentrated under reduced pressure, and the resulting crude product was purified by silica gel column chromatography (DCM:MeOH = 200:1 to 20:1) to give 3-(1-(4-chloro-3-(2,4-dioxotetrahydropyrimidin-1(2H)-yl)benzoyl)piperidin-4-yl)propanal.

LC-MS: (ESI, m/z): [M+H]⁺= 392.0.

### Step 3: preparation of tert-butyl 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

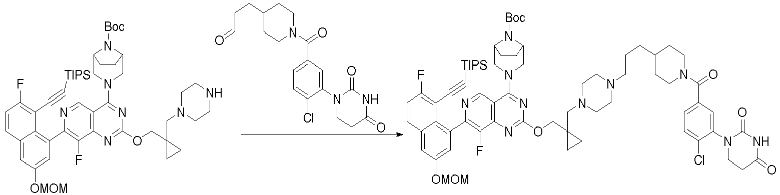

Acetic acid (0.1 mL) and 3-(1-(4-chloro-3-(2,4-dioxotetrahydropyrimidin-1(2H)-yl)benzoyl)piperidin-4-yl)propanal (65 mg, 0.16 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl) methoxy)pyrido[4,3-d]pyrimidin-4-yl)-3,8-diazabicyclo[3.2.1]octane-8-carboxylate (100 mg, 0.11 mmol) in DCM/MeOH (3 mL/3 mL). The mixture was stirred at room temperature for 0.5 h. NaBH₃CN (19 mg, 0.22 mmol) was added to the mixture at room temperature, and the resulting mixture was stirred for 0.5 h. The mixture was concentrated and purified by silica gel column chromatography (MeOH:DCM = 200:1 to 20:1) to give *tert-butyl* 3-(2-((1-((4-(3-(1-(4-chloro-3-(2,4-dioxotetrahydropyrimidin-1(2*H*)-yl)benzoyl)piperidin-4-yl)propyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate.

LC-MS: (ESI, m/z): [M/2+H]⁺ = 644.6.

### Step 4: preparation of 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H3H)-dione

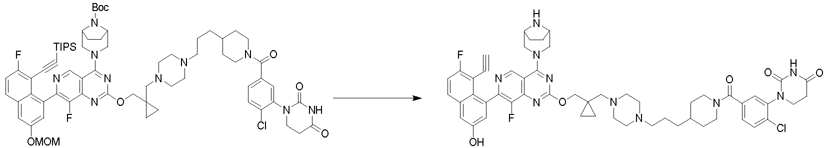

Cesium fluoride (80 mg, 0.5 mmol) was added to a solution of *tert-butyl* 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate (130 mg, 0.1 mmol) in DMF (3 mL). The mixture was stirred at room temperature for 30 min and extracted with EA and water. The organic phase was concentrated, and a mixture of 1,4-dioxane (2 mL) and HCl/1,4-dioxane (6 N, 1 mL) was added to the residue at room temperature. The reaction mixture was reacted for 0.5 h, and then concentrated under reduced pressure. The resulting crude product was purified by Pre-HPLC to give 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione.

LC-MS: (ESI, m/z): [M+H]⁺= 987.2.

¹H NMR (400 MHz, CD₃OD) δ 8.99 (s, 1H), 7.85 (dd, *J* = 9.1, 5.7 Hz, 1H), 7.63 (d, *J* = 8.2 Hz, 1H), 7.51 (s, 1H), 7.40 (d, *J* = 8.1 Hz, 1H), 7.34-7.27 (m, 2H), 7.20 (d, *J* = 2.5 Hz, 1H), 4.69-4.34 (m, 6H), 3.81-3.64 (m, 7H), 3.36 (s, 1H), 3.12-3.00 (m, 1H), 2.90 -2.77 (m, 3H), 2.66-2.35 (m, 9H), 2.32-2.26 (m, 2H), 1.90-1.76 (m, 5H), 1.57-1.47 (m, 3H), 1.30-1.12 (m, 5H), 0.71 (t, *J* = 5.2 Hz, 2H), 0.49 (t, *J* = 5.2 Hz, 2H).

### Example 5: 1-(5-(4-(4-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)butyl)piperidine-1-carbonyl)-2-chlorophenyl)dihydropyrimidine-2,4(1H,3H)-dione

### Step 1: preparation of 4-(piperidin-4-yl)butan-1-ol

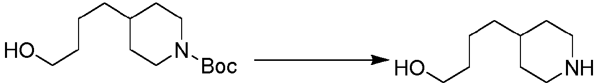

*tert-Butyl* 4-(4-hydroxybutyl)piperidine-1-carboxylate (500 mg, 1.94 mmol) was dissolved in a solution of hydrochloric acid/1,4-dioxane (8 M, 1.5 mL), and the mixture was stirred at 30 °C for 16 h. After the reaction was completed, the mixture was concentrated under reduced pressure to give 4-(piperidin-4-yl)butan-1-ol. The crude product was directly used in the next step without further purification.

### Step 2: preparation of 1-(2-chloro-5-(4-(4-hydroxybutyl)piperidine-1-carbonyl)phenyl)dihydropyrimidine-2,4(1H,3H)-dione

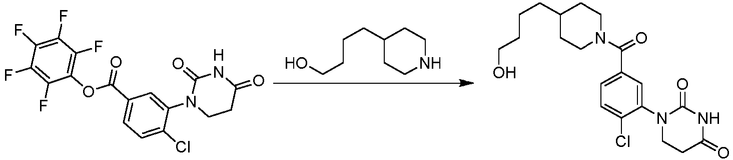

DIEA (1.3 mL) and pentafluorophenyl 4-chloro-3-(2,4-dioxotetrahydropyrimidin-1(2H)-yl)benzoate (746 mg, 1.72 mmol) were added to a solution of 4-(piperidin-4-yl)butan-1-ol (257 mg, 1.63 mmol) in dimethyl sulfoxide (3 mL). The mixture was stirred at 30 °C for 16 h. After the reaction was completed, the reaction liquid was diluted with water (10 mL) and extracted with ethyl acetate (100 mL × 3). The organic phase was washed with brine (50 mL), dried over anhydrous Na₂SO₄, filtered, and concentrated in vacuum. The crude product was purified by silica gel column (DCM:MeOH (NH₃) = 20:1) to give 1-(2-chloro-5-(4-(4-hydroxybutyl)piperidine-1-carbonyl)phenyl)dihydropyrimidine-2,4(1H,3H)-dione.

LC-MS: (ESI, m/z): [M+H]⁺= 408.1.

### Step 3: preparation of 4-(1-(4-chloro-3-(2,4-dioxotetrahydropyrimidin-1(2H)-yl)benzoyl)piperidin-4-yl)butanal

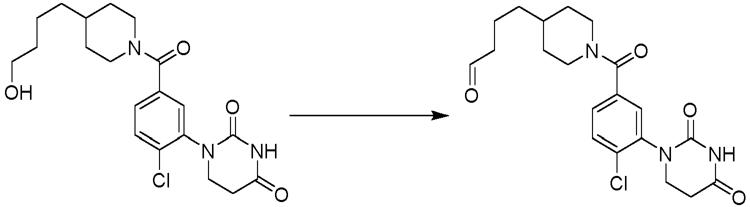

Dess Martin reagent (305 mg, 0.72 mmol) was added to a solution of 1-(2-chloro-5-(4-(4-hydroxybutyl)piperidine-1-carbonyl)phenyl)dihydropyrimidine-2,4(1H,3H)-dione (245 mg, 0.60 mmol) in dichloromethane (5 mL). The mixture was stirred at 30 °C for 1 h. After the reaction was completed, the reaction liquid was diluted with water (10 mL) and extracted with ethyl acetate (20 mL × 3). The organic phase was washed with brine (20 mL), dried over anhydrous Na₂SO₄, and filtered, and the filtrate was concentrated under reduced pressure to give a crude product of 4-(1-(4-chloro-3-(2,4-dioxotetrahydropyrimidin-1(2H)-yl)benzoyl)piperidin-4-yl)butanal. The crude product was used in the next step without further purification.

LC-MS: (ESI, m/z): [M+H]⁺= 406.1.

### Step 4: preparation of tert-butyl 3-(2-((1-((4-(4-(1-(4-chloro-3-(2,4-dioxotetrahydropyrimidin-1(2H)-yl)benzoyl)piperidin-4-yl)butyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

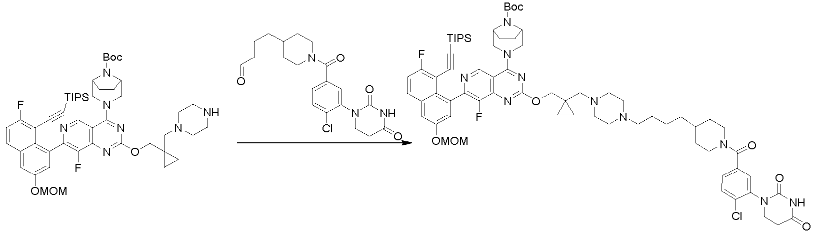

Acetic acid (0.01 mL) and 4-(1-(4-chloro-3-(2,4-dioxotetrahydropyrimidin-1(2H)-yl)benzoyl)piperidin-4-yl)butanal (134 mg, 0.33 mmol) were added to a mixed solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl) methoxy)pyrido[4,3-djpyrimidin-4-yl)-3,8-diazabicyclo[3.2.1]octane-8-carboxylate (100 mg, 0.11 mmol) in 1,2-dichloroethane (0.5 mL) and methanol (5 mL). The mixture was stirred at 30 °C for 1 h, and then NaBH₃CN (21 mg, 0.33 mmol) was added. After the reaction was completed, H₂O (10 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM:MeOH = 20:1) to give *tert-butyl* 3-(2-((1-((4-(4-(1-(4-chloro-3-(2,4-dioxotetrahydropyrimidin-1(2H)-yl)benzoyl)piperidin-4-yl)butyl)piperazin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate.

LC-MS: (ESI, m/z): [M/2+H]⁺ = 651.3

### Step 5: preparation of 1-(5-(4-(4-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)butyl)piperidine-1-carbonyl)-2-chlorophenyl)dihydropyrimidine-2,4(1H,3H)-dione

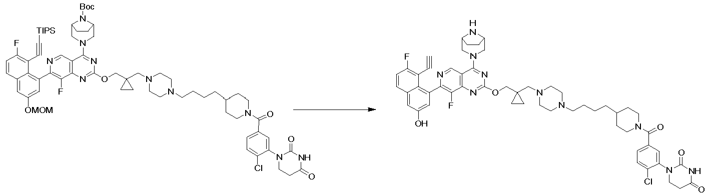

*tert-Butyl* 3-(2-((1-((4-(4-(1-(4-chloro-3-(2,4-dioxotetrahydropyrimidin-1(2H)-yl)benzoyl)piperidin-4-yl)butyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate (110 mg, 0.08 mmol) was dissolved in DMF (1 mL), and CsF (60 mg, 0.4 mmol) was added. The mixture was reacted at room temperature for 2 h. After the reaction was completed, the mixture was diluted with H₂O (5 mL) and extracted with ethyl acetate (5 mL × 3). The combined organic layer was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and then extracted with ethyl acetate (5 mL × 3). The combined organic phase was dried over Na₂SO₄, filtered, and concentrated under reduced pressure. The crude product was purified by Pre-HPLC to give 1-(5-(4-(4-(4-((1-(((4-(3,8-diazabicyclo[3.2.1]octan-3-yl)-7-(8-cthynyl-7-fluoro-3-hydroxynaphthalen-1-y1)-8-fluoropyrido [4,3-d]pyrimidin-2-yl)oxy)methyl)cyclopropyl)methyl)piperazin-1-yl)butyl)piperidine-1-carbonyl)-2-chlorophenyl)dihydropyrimidine-2,4(1H,3H)-dione.

LC-MS: (ESI, m/z): [M+H]⁺= 1001.1.

¹H NMR (400 MHz, CD₃OD) δ 8.99 (s, 1H), 7.85 (dd, *J* = 9.2, 4.2 Hz, 1H), 7.63 (d, *J* = 8.4 Hz, 1H), 7.52 (s, 1H), 7.40 (d, *J=* 8.4 Hz, 1H), 7.35-7.27 (m, 2H), 7.20 (d, *J=* 2.8 Hz, 1H), 4.71-4.30 (m, 6H), 3.82-3.62 (m, 7H), 3.31 (s, 1H), 3.12-3.03 (m, 1H), 2.91-2.78 (m, 3H), 2.62-2.24 (m, 11H), 1.91-1.74 (m, 5H), 1.66-1.43 (m, 4H), 1.32-1.09 (m, 6H), 0.71 (t, *J* = 6.0 Hz, 2H), 0.49 (t, *J* = 6.0 Hz, 2H).

### Example 6: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

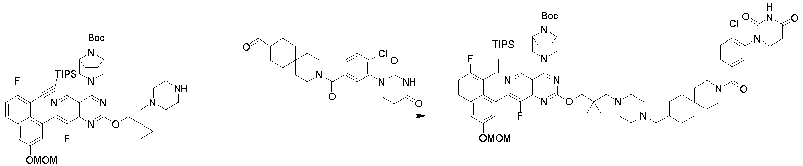

HOAc (0.1 mL) and 3-(4-chloro-3-(2,4-dioxotetrahydropyrimidin-1(2H)-yl)benzoyl)-3-azaspiro[5.5] undecane-9-carbaldehyde (64 mg, 0.15 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl) methoxy)pyrido[4,3-d]pyrimidin-4-yl)-3,8-diazabicyclo[3.2.1]octane-8-carboxylate (90 mg, 0.1 mmol) in DCM/methanol (3 mL/3 mL), and the mixture was stirred at room temperature for 0.5 h. NaBH₃CN (13 mg, 0.20 mmol) was added to the mixture at 0-5 °C, and the resulting mixture was stirred for 0.5 h. The mixture was concentrated and purified by silica gel column chromatography (DCM:MeOH = 100:1 to 5:1) to give *tert-butyl* 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 664.2.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

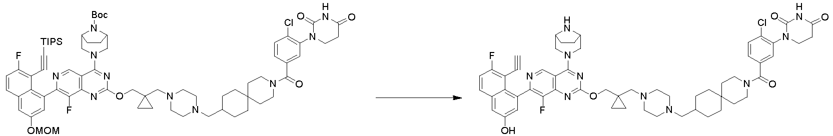

CsF (75 mg, 0.49 mmol) was added to a solution of *tert-butyl* 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (120 mg, 0.09 mmol) in DMF (3.0 mL), and the mixture was reacted at room temperature for 30 min. Water (10 mL) was added, and the mixture was extracted with ethyl acetate (10 mL × 2). The organic phases were combined, washed with saturated brine, dried over anhydrous sodium sulfate, and filtered, and the filtrate was concentrated to give a crude product of the intermediate. The crude product was dissolved in 1,4-dioxane (2 mL), and HCl/1,4-dioxane (6 N, 1.0 mL) was added. The mixture was reacted at room temperature for 0.5 h. An aqueous Na₂CO₃ solution was added to the reaction liquid, and the mixture was adjusted to pH > 7, and then extracted with EtOAc/i-PrOH (v/v = 5/1, 10 mL × 2). The organic phase was washed with brine, dried over anhydrous Na₂SO₄, and concentrated. The crude product was purified by Pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M+H]⁺= 1027.2.

¹H NMR (400 MHz, CD₃OD) δ 8.99 (s, 1H), 7.85 (dd, *J* = 9.2, 5.6 Hz, 1H), 7.63 (d, *J* = 8.2 Hz, 1H), 7.53 *(d, J =* 1.6 Hz, 1H), 7.41 (dd, *J* = 8.2, 2.0 Hz, 1H), 7.36-7.28 (m, 2H), 7.20 (d, *J* = 2.4 Hz, 1H), 4.85-4.33 (m, 5H), 3.82-3.64 (m, 8H), 3.45-3.34 (m, 3H), 2.88-2.82 (m, 2H), 2.57-2.36 (m, 8H), 2.19-2.11 (m, 2H), 1.91-1.73 (m, 6H), 1.61-1.29 (m, 8H), 1.21-1.04 (m, 4H), 0.75-0.66 (m, 2H), 0.54-0.45 (m, 2H).

### Example 7: 1-(5-(9-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)-3-azaspiro[5.5]undecane-3-carbonyl)-2-chlorophenyl)dihydropyrimidine-2,4(1H,3H)-dione

### Step 1: preparation of 3-(3-azaspiro[5.5]undecan-9-yl)propan-1-ol

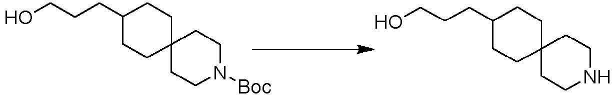

*tert*-Butyl 9-(3-hydroxypropyl)-3-azaspiro[5.5]undecane-3-carboxylate (360 mg, 1.16 mmol) was dissolved in DCM (4 mL), and then TFA (2 mL) was added. The mixture was stirred at room temperature for 1 h and concentrated to give 3-(3-azaspiro[5.5]undecan-9-yl)propan-1-ol. The crude product was directly used in the next step without further purification.

### Step 2: preparation of 1-(2-chloro-5-(9-(3-hydroxypropyl)-3-azaspiro[5.5]undecane-3-carbonyl)phenyl)dihydropyrimidine-2,4(1H,3H)-dione

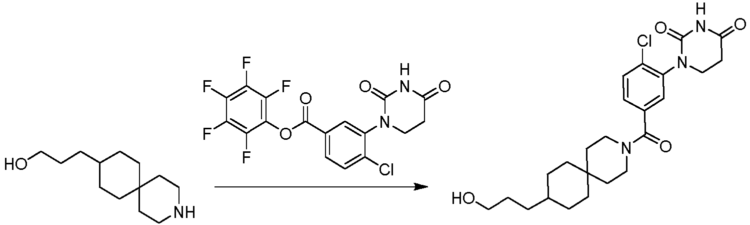

DIEA (736 mg, 5.70 mmol) and pentafluorophenyl 4-chloro-3-(2,4-dioxotetrahydropyrimidin-1(2H)-yl)benzoate (496 mg, 1.14 mmol) were added to a solution of 3-(3-azaspiro[5.5]undecan-9-yl)propan-1-ol (240 mg, 1.14 mmol) in DMF (5 mL), and the reaction liquid was stirred at room temperature for 1 h. Water was added to the reaction liquid, and then the mixture was extracted with ethyl acetate. The organic phases were combined, dried over anhydrous sodium sulfate, and filtered. The filtrate was concentrated, and the resulting crude product was purified by silica gel column chromatography (DCM:MeOH = 100:1 to 5:1) to give 1-(2-chloro-5-(9-(3-hydroxypropyl)-3-azaspiro[5.5]undecane-3-carbonyl)phenyl)dihydropyrimidine-2,4(1H,3H)-dione.

LC-MS: (ESI, m/z): [M+H]⁺= 462.2.

### Step 3: preparation of 3-(3-(4-chloro-3-(2,4-dioxotetrahydropyrimidin-1(2H)-yl)benzoyl)-3-azaspiro[5.5] undecan-9-yl)propanal

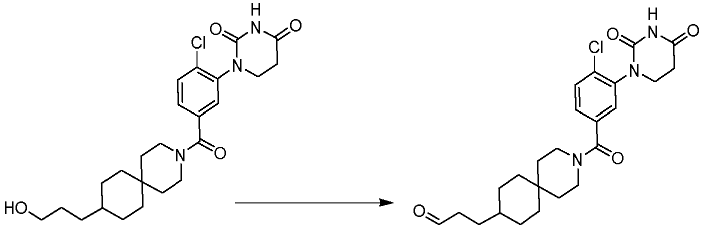

TPAP (101 mg, 0.87 mmol) and NMO (30 mg, 0.09 mmol) were added to a solution of 1-(2-chloro-5-(9-(3-hydroxypropyl)-3-azaspiro[5.5]undecane-3-carbonyl)phenyl)dihydropyrimidine-2,4(1H,3H)-dione (200 mg, 0.43 mmol) in DCM (5 mL), and the reaction liquid was stirred at room temperature for 1 h. After the reaction was completed, water was added to the reaction liquid, and then the mixture was extracted with ethyl acetate. The organic phases were combined, dried over anhydrous sodium sulfate, and filtered. The filtrate was concentrated, and the resulting crude product was purified by silica gel column chromatography (DCM:MeOH = 100:1 to 5:1) to give 3-(3-(4-chloro-3-(2,4-dioxotetrahydropyrimidin-1(2H)-yl)benzoyl)-3-azaspiro[5.5] undecan-9-yl)propanal.

LC-MS: (ESI, m/z): [M+H]⁺= 460.2.

### Step 4: preparation of tert-butyl 3-(2-((1-((4-(3-(3-(4-chloro-3-(2,4-dioxotetrahydropyrimidin-1(2H)-yl)benzoyl)-3-azaspiro[5.5]undecan-9-yl)propyl)piperazin-l-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

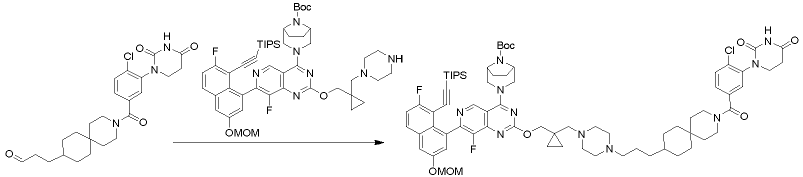

Acetic acid (0.1 mL) and 3-(3-(4-chloro-3-(2,4-dioxotetrahydropyrimidin-1(2H)-yl)benzoyl)-3-azaspiro[5.5] undecan-9-yl)propanal (76 mg, 0.17 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (100 mg, 0.11 mmol) in ethanol (2 mL), and the mixture was stirred at room temperature for 15 min. NaBH₃CN (21 mg, 0.33 mmol) was added to the mixture at 0-5 °C, and the resulting mixture was stirred for 0.5 h. The mixture was concentrated, and the resulting crude product was purified by silica gel column chromatography (DCM:MeOH = 100:1 to 5:1) to give *tert-butyl* 3-(2-((1-((4-(3-(3-(4-chloro-3-(2,4-dioxotetrahydropyrimidin-1(2H)-yl)benzoyl)-3-azaspiro[5.5]undecan-9-yl)propyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 678.7.

### Step 5: preparation of 1-(5-(9-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)-3-azaspiro[5.5]undecane-3-carbonyl)-2-chlorophenyl)dihydropyrimidine-2,4(1H,3H)-dione

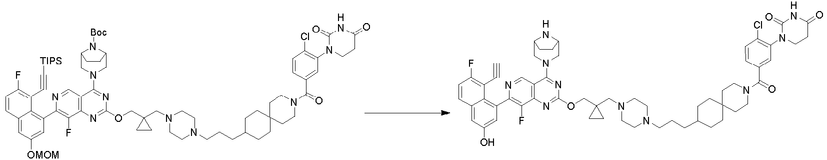

CsF (56 mg, 0.37 mmol) was added to a solution of *tert-butyl* 3-(2-((1-((4-(3-(3-(4-chloro-3-(2,4-dioxotetrahydropyrimidin-1(2H)-yl)benzoyl)-3-azaspiro[5.5]undecan-9-yl)propyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (100 mg, 0.07 mmol) in DMF (2.0 mL), and the mixture was reacted at room temperature for 2 h. Water (10 mL) was added to the reaction liquid, and the mixture was extracted with ethyl acetate (10 mL × 2). The organic phases were combined, washed with saturated brine, dried over anhydrous sodium sulfate, and filtered, and the filtrate was concentrated to give a crude product. The resulting crude product was dissolved in 1,4-dioxane (2.0 mL), and then HCl/1,4-dioxane (6 N, 1.0 mL) was added. The mixture was reacted at room temperature for 0.5 h. An aqueous Na₂CO₃ solution was added to the reaction liquid, and the mixture was adjusted to pH > 7, and then extracted with ethyl acetate (10 mL × 2). The organic phase was washed with brine, dried over anhydrous Na₂SO₄, and concentrated. The crude product was purified by Pre-HPLC to give 1-(5-(9-(3-(4-((1-(((4-(3,8-diazabicyclo[3.2.1]octan-3-yl)-7-(8-ethynyl-7-fluoro-3-hydroxynaphthalen-1-yl)-8-fluoropyrido [4,3-d]pyrimidin-2-yl)oxy)methyl)cyclopropyl)methyl)piperazin-1-yl)propyl)-3-azaspiro[5.5]undecane-3-carbonyl)-2-chlorophenyl)dihydropyrimidine-2,4(1H,3H)-dione.

LC-MS: (ESI, m/z): [M+H]⁺= 1055.1.

¹H NMR (400 MHz, DMSO-d₆) δ 10.51 (s, 1H), 10.23 (s, 1H), 9.02 (s, 1H),7.99 - 7.95 (m, 1H), 7.63 (d, *J* = 8.0 Hz, 1H), 7.54 - 7.35 (m, 4H), 7.16 (d, *J* = 2.4 Hz, 1H), 4.48 (d, *J* = 10.4 Hz, 1H), 4.34 - 4.18 (m, 3H), 3.92 (s, 1H), 3.77 - 3.71 (m, 1H), 3.62 - 3.48 (m, 7H), 3.18 - 3.27 (m, 2H), 2.76 - 2.69 (m, 2H), 2.43 - 2.11 (m, 13H), 1.73 - 1.60 (m, 5H), 1.48 - 0.97 (m, 16H), 0.66 - 0.56 (m, 2H), 0.43 - 0.36 (m, 2H).

### Example 8: 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-methoxyphenyl)dihydropyrimidine-2,4(1H,3H)-dione

### Step 1: preparation of 1-(5-(4-(3-hydroxypropyl)piperidine-1-carbonyl)-2-methoxyphenyl)dihydropyrimidine-2,4(1H,3H)-dione

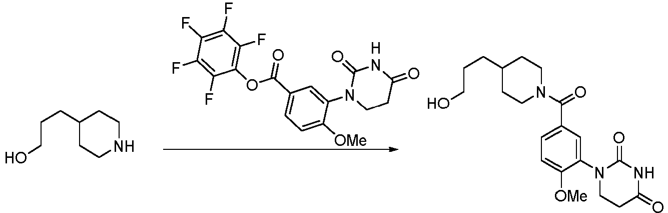

Pentafluorophenyl 3-(2,4-dioxotetrahydropyrimidin-1(2H)-yl)-4-methoxybenzoate (0.95 g, 2.20 mmol) and DIEA (0.81 g, 6.27 mmol) were added to a solution of 3-(piperidin-4-yl)propan-1-ol (300 mg, 2.09 mmol) in DMSO (3 mL). The mixture was stirred at room temperature for 1 h. After the reaction was completed, water was added to the reaction liquid, and then the mixture was extracted with ethyl acetate. The organic phase was washed with saturated brine, dried over anhydrous sodium sulfate, and concentrated under reduced pressure. The resulting crude product was purified by silica gel column chromatography to give 1-(5-(4-(3-hydroxypropyl)piperidine-1-carbonyl)-2-methoxyphenyl)dihydropyrimidine-2,4(1H,3H)-dione.

LC-MS: (ESI, m/z): [M+H]⁺= 390.1.

### Step 2: preparation of 3-(1-(3-(2,4-dioxotetrahydropyrimidin-1(2H)-yl)-4-methoxybenzoyl)piperidin-4-yl)propanal

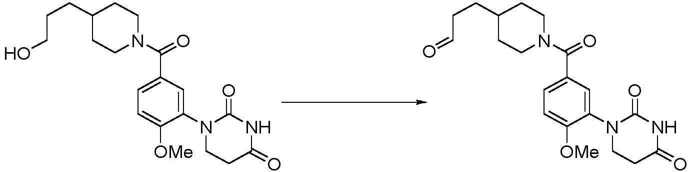

Dess-Martin reagent (353 mg, 0.83 mmol) was added to a solution of 1-(5-(4-(3-hydroxypropyl)piperidine-1-carbonyl)-2-methoxyphenyl)dihydropyrimidine-2,4(1H,3H)-dione (270 mg, 0.69 mmol) in DCM (5 mL), and then the mixture was reacted for 1 h. The reaction mixture was filtered, the filtrate was concentrated, and the resulting crude product was purified by silica gel column chromatography (MeOH:DCM = (0-1):4) to give 3-(1-(3-(2,4-dioxotetrahydropyrimidin-1(2H)-yl)-4-methoxybenzoyl)piperidin-4-yl)propanal.

LC-MS: (ESI, m/z): [M+H]⁺= 388.1.

### Step 3: preparation of tert-butyl 3-(2-((1-((4-(3-(1-(3-(2,4-dioxotetrahydropyrimidin-1(2H)-yl)-4-methoxybenzoyl)piperidin-4-yl)propyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

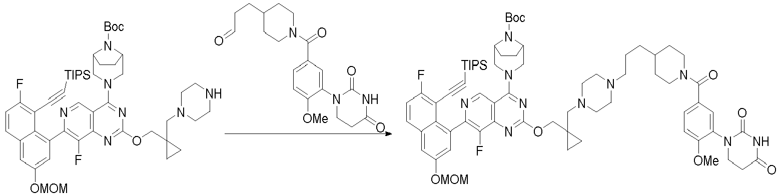

HOAc (0.1 mL) and 3-(1-(3-(2,4-dioxotetrahydropyrimidin-1(2H)-yl)-4-methoxybenzoyl)piperidin-4-yl)propanal (51 mg, 0.13 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl) methoxy)pyrido[4,3-d]pyrimidin-4-yl)-3,8-diazabicyclo[3.2.1]octane-8-carboxylate (100 mg, 0.11 mmol) in DCM/MeOH (3 mL/3 mL), and the mixture was stirred at room temperature for 0.5 h. NaBH₃CN (14 mg, 0.22 mmol) was added to the mixture at room temperature, and the resulting mixture was stirred for 0.5 h. The mixture was concentrated and purified by column chromatography to give *tert-*butyl 3-(2-((1-((4-(3-(1-(3-(2,4-dioxotetrahydropyrimidin-1(2H)-yl)-4-methoxybenzoyl)piperidin-4-yl)propyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 642.5.

### Step 4: preparation of 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-methoxyphenyl)dihydropyrimidine-2,4(1H,3H)-dione

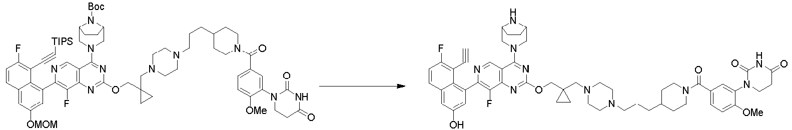

Cesium fluoride (52 mg, 0.34 mmol) was added to a solution of *tert*-butyl 3-(2-((1-((4-(3-(1-(3-(2,4-dioxotetrahydropyrimidin-1(2H)-yl)-4-methoxybenzoyl)piperidin-4-yl)propyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (90 mg, 0.07 mmol) in DMF (3 mL). The mixture was stirred at room temperature for 30 min. EA and water were added to the reaction liquid, and the organic phase was separated, dried, and concentrated under reduced pressure. A mixture of 1,4-dioxane (2 mL) and HCl/1,4-dioxane (6 N, 1 mL)) was then added to the resulting mixture at room temperature. The reaction mixture was reacted for 0.5 h and purified by Pre-HPLC to give 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-methoxyphenyl)dihydropyrimidine-2,4(1H,3H)-dione.

LC-MS: (ESI, m/z): [M+H]⁺= 983.1.

¹H NMR (400 MHz, DMSO-*d*₆) δ 10.33 (s, 1H), 10.15 (s, 1H), 9.02 (s, 1H), 8.01-7.92 (m, 1H), 7.52-7.41 (m, 1H), 7.39-7.29 (m, 3H), 7.19-7.10 (m, 2H), 4.48 (d, *J* = 11.2 Hz, 1H), 4.31-4.23 (m, 3H), 3.92 (s, 1H), 3.84 (s, 3H), 3.66-3.50 (m, 7H), 3.01-2.59 (m, 6H), 2.44-2.03 (m, 12H), 1.72-1.60 (m, 4H), 1.51-1.36 (m, 3H), 1.29-1.14 (m, 4H), 1.11-0.98 (m, 2H), 0.68-0.58 (m, 2H), 0.45-0.35 (m, 2H).

### Example 9: 1-(5-(4-(3-((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1H,3H)-dione

### Step 1: preparation of (9H-fluoren-9-yl)methyl 4-(3-hydroxypropyl)piperidine-1-carboxylate

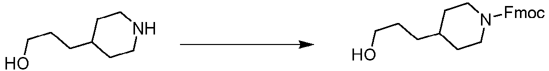

3-(Piperidin-4-yl)propan-1-ol (2.5 mg, 17.48 mmol) was dissolved in a solution of 1,4-dioxane/water (10 mL/10 mL), and then sodium bicarbonate (2.93 g, 34.88 mmol) and N-(9-fluorenylmethoxycarbonyloxy)succinimide (6.50 g, 19.29 mmol) were added. The mixture was stirred at room temperature for 16 h, extracted with ethyl acetate (20 mL × 2), and concentrated under reduced pressure. The resulting crude product was purified by silica gel column chromatography to give (9H-fluoren-9-yl)methyl 4-(3-hydroxypropyl)piperidine-1-carboxylate.

LC-MS: (ESI, m/z): [M+H]⁺= 366.0.

### Step 2: preparation of (9H-fluoren-9-yl)methyl 4-(3-oxopropyl)piperidine-1-carboxylate

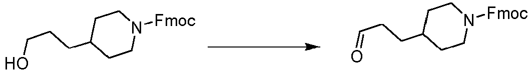

Dess-Martin reagent (696 mg, 1.64 mmol) was added to a solution of (9H-fluoren-9-yl)methyl 4-(3-hydroxypropyl)piperidine-1-carboxylate (500 mg, 1.37 mmol) in DCM (10 mL). The mixture was reacted for 1 h and filtered, and the filtrate was concentrated and purified by silica gel column chromatography (PE:EA = 10:1 to 3:1) to give (9H-fluoren-9-yl)methyl 4-(3-oxopropyl)piperidine-1-carboxylate.

¹H NMR (400 MHz, CDCl₃) δ 9.84(s, 1H), 7.81 (d, *J* = 10.0 Hz, 2H), 7.62 (d, *J* = 8.8 Hz, 2H), 7.49 - 7.41 (m, 2H), 7.40 - 7.33 (m, 2H), 4.54 - 4.89 (m, 2H), 4.33 - 4.05 (m, 3H), 2.87 - 2.70 (m, 2H), 2.58 - 2.48 (m, 2H), 1.82 - 1.65 (m, 4H), 1.51 - 1.39 (m, 1H), 1.20-1.01 (m, 2H).

LC-MS: (ESI, m/z): [M+Na]⁺ = 386.0.

### Step 3: preparation of tert-butyl 3-(2-(((S)-1-benzylpyrrolidin-2-yl)methoxy)-7-chloro-8-fluoropyrido[4,3-d]pyrimidin-4-yl)-3,8-diazabicyclo[3.2.1]octane-8-carboxylate

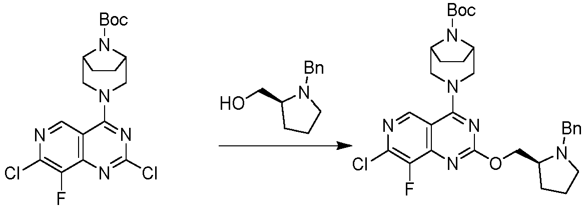

*tert*-Butyl 3-(2,7-dichloro-8-fluoropyrido[4,3-d]pyrimidin-4-yl)-3,8-diazabicyclo[3.2.1]octane-8-carboxylate (5.00 g, 11.67 mmol) and (*S*)-(1-benzylpyrrolidin-2-yl)methanol (3.35 g, 17.51 mmol) were dissolved in THF (50 mL), and Cs₂CO₃ (11.44 g, 35.01 mmol) was added. The reaction liquid was heated to 85 °C and reacted for 16 h. After the reaction was completed, the mixture was cooled to room temperature. Water (50 mL) was added, and the mixture was extracted with ethyl acetate (50 mL × 3). The organic phases were combined, washed with a saturated NaCl solution, dried over anhydrous Na₂SO₄, and filtered. The filtrate was concentrated, and the resulting crude product was purified by silica gel column chromatography (PE:EA = 5:2) to give *tert-butyl* 3-(2-(((S)-1-benzylpyrrolidin-2-yl)methoxy)-7-chloro-8-fluoropyrido[4,3-d]pyrimidin-4-yl)-3,8-diazabicyclo[3.2.1]octane-8-carboxylate.

LC-MS: (ESI, m/z): [M+H]⁺= 583.2.

### Step 4: preparation of tert-butyl 3-(2-(((5)-1-(3-(1-(((9H-fluoren-9-yl)methoxy)carbonyl)piperidin-4-yl)propyl)pyrrolidin-2-yl)methoxy)-7-chloro-8-fluoropyrido[4,3-d]pyrimidin-4-yl)-3,8-diazabicyclo[3.2.1]octane-8-carboxylate

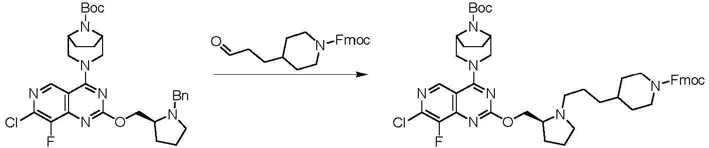

*tert*-Butyl 3-(2-(((S)-1-benzylpyrrolidin-2-yl)methoxy)-7-chloro-8-fluoropyrido[4,3-d]pyrimidin-4-yl)-3,8-diazabicyclo[3.2.1]octane-8-carboxylate (2.0 g, 3.4 mmol) was dissolved in DCM (20 mL), and 1-chloroethyl chloroformate (1.46 g, 1.02 mmol) and DIEA (1.32 g, 1.02 mmol) were added. The reaction mixture was reacted at room temperature for 30 min and concentrated under reduced pressure, and MeOH (20 mL) was added to the resulting concentrate. The mixture was heated to 50 °C and reacted for 30 min. After the reaction was completed, the mixture was concentrated to one-third volume, (9H-fluoren-9-yl)methyl 4-(3-oxopropyl)piperidine-1-carboxylate (2.12 g, 5.8 mmol) was added, and then the mixture was stirred at room temperature for 2 min. NaBH(OAc)₃ (1.84 g, 8.7 mmol) was added at 0-5 °C, and then the reaction liquid was warmed to room temperature and reacted for 30 min. An aqueous sodium carbonate solution was added to the reaction liquid to adjust pH > 7, and the mixture was extracted with ethyl acetate (50 mL × 3). The organic phase was washed with a saturated sodium chloride solution, dried over anhydrous sodium sulfate, and filtered, and the filtrate was concentrated. The resulting crude product was purified by silica gel column chromatography (PE:EA = 2:1) to give *tert-butyl* 3-(2-(((S)-1-(3-(1-(((9H-fluoren-9-yl)methoxy)carbonyl)piperidin-4-yl)propyl)pyrrolidin-2-yl)methoxy)-7-chloro-8-fluoropyrido[4,3-d]pyrimidin-4-yl)-3,8-diazabicyclo[3.2.1]octane-8-carboxylate.

LC-MS: (ESI, m/z): [M+H]⁺= 840.4.

### Step 5: preparation of tert-butyl 3-(7-chloro-8-fluoro-2-(((S)-1-(3-(piperidin-4-yl)propyl)pyrrolidin-2-yl)methoxy)pyrido[4,3-d]pyrimidin-4-yl)-3,8-diazabicyclo[3.2.1]octane-8-carboxylate

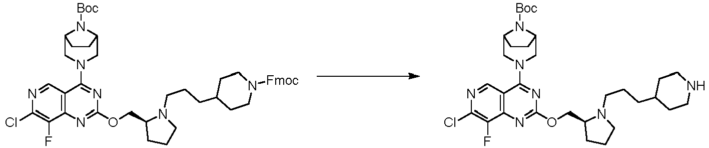

*tert-Butyl* 3-(2-(((S)-1-(3-(1-(((9H-fluoren-9-yl)methoxy)carbonyl)piperidin-4-yl)propyl)pyrrolidin-2-yl)methoxy)-7-chloro-8-fluoropyrido[4,3-d]pyrimidin-4-yl)-3,8-diazabicyclo[3.2.1]octane-8-carboxylate (1.00 g, 1.19 mmol) was dissolved in dichloromethane (2 mL) and methanol (10 mL), and piperidine (2 mL) was added. The reaction mixture was stirred at room temperature overnight. The reaction liquid was directly concentrated under reduced pressure, and the resulting crude product was purified by silica gel column chromatography (DCM:MeOH = 100:1 to 20:1) to give *tert-butyl* 3-(7-chloro-8-fluoro-2-(((S)-1-(3-(piperidin-4-yl)propyl)pyrrolidin-2-yl)methoxy)pyrido[4,3-d]pyrimidin-4-yl)-3,8-diazabicyclo[3.2.1]octane-8-carboxylate.

LC-MS: (ESI, m/z): [M+H]⁺= 618.3.

### Step 6: preparation of tert-butyl 3-(8-fluoro-7-(7-fluoro-3-(methoxymethoxy)-8-((triisopropylsilyl)ethynyl) naphthalen-1-yl)-2-(((S)-1-(3-(piperidin-4-yl)propyl)pyrrolidin-2-yl)methoxy)pyrido[4,3-d]pyrimidin-4-yl)-3,8-diazabicyclo[3.2.1]octane-8-carboxylate

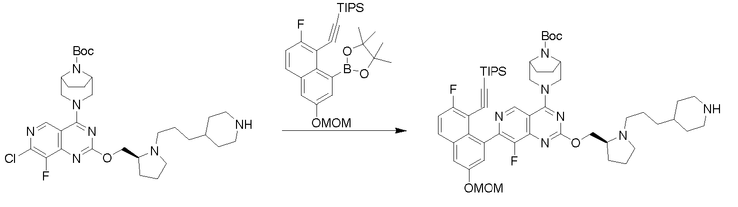

((2-Fluoro-6-(methoxymethoxy)-8-(4,4,5,5-tetramethyl-1,3,2-dioxaborolan-2-yl)naphthalen-1-yl)ethynyl) triisopropylsilane, potassium phosphate (360 mg, 1.7 mmol), and cataXium A Pd G3 (41 mg, 0.057 mmol) were added to a solution of *tert-butyl* 3-(7-chloro-8-fluoro-2-(((S)-1-(3-(piperidin-4-yl)propyl)pyrrolidin-2-yl)methoxy)pyrido[4,3-d]pyrimidin-4-yl)-3,8-diazabicyclo[3.2.1]octane-8-carboxylate (350 mg, 0.57 mmol) in 1,4-dioxane/H₂O (4.0 mL/0.8 mL). The mixture was reacted at 100 °C overnight under N₂ atmosphere. The reaction mixture was filtered and concentrated, and the resulting crude product was purified by silica gel column chromatography (DCM:MeOH = 100:1 to 20:1) to give *tert-butyl* 3-(8-fluoro-7-(7-fluoro-3-(methoxymethoxy)-8-((triisopropylsilyl)ethynyl)naphthalen-1-yl)-2-(((S)-1-(3-(piperidin-4-yl)propyl)pyrrolidin-2-yl)methoxy)pyrido[4,3-*d*]pyrimidin-4-yl)-3,8-diazabicyclo[3.2.1]octane-8-carboxylate.

LC-MS: (ESI, m/z): [M/2+H]⁺ = 484.7.

### Step 7: preparation of tert-butyl 3-(2-(((S)-1-(3-(1-(4-chloro-3-(2,4-dioxotetrahydropyrimidin-1(2H)-yl)benzoyl)piperidin-4-yl)propyl)pyrrolidin-2-yl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

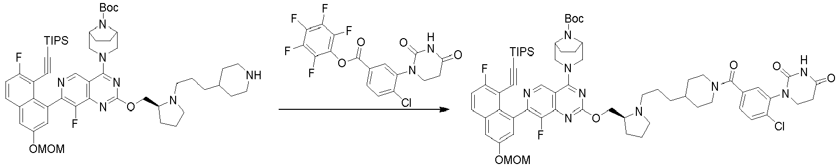

**Pentafluorophenyl** 4-chloro-3-(2,4-dioxotetrahydropyrimidin-1(2H)-yl)benzoate (74 mg, 0.17 mmol) and DIEA (60 mg, 0.47 mmol) were added to a solution of *tert-butyl* 3-(8-fluoro-7-(7-fluoro-3-(methoxymethoxy)-8-((triisopropylsilyl)ethynyl)naphthalen-1-yl)-2-(((S)-1-(3-(piperidin-4-yl)propyl)pyrrolidin-2-yl)methoxy) pyrido[4,3-d]pyrimidin-4-yl)-3,8-diazabicyclo[3.2.1]octane-8-carboxylate (150 mg, 0.16 mmol) in DMSO (3 mL), and then the reaction liquid was reacted at room temperature for 2 h. Water (10 mL) was added to the reaction liquid, and the mixture was extracted with ethyl acetate (10 mL × 2). The organic layer was washed with saturated brine, dried over anhydrous sodium sulfate, and filtered. The filtrate was concentrated, and the resulting crude product was purified by silica gel column chromatography (DCM:MeOH = 100:1 to 20:1) to give *tert-butyl* 3-(2-(((*S*)-1-(3-(1-(4-chloro-3-(2,4-dioxotetrahydropyrimidin-1(2*H*)-yl)benzoyl)piperidin-4-yl)propyl)pyrrolidin-2-yl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 609.7.

### Step 8: preparation of 1-(5-(4-(3-((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1H,3H)-dione

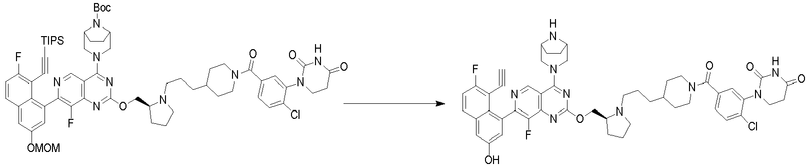

CsF (90 mg, 0.6 mmol) was added to a solution of *tert*-butyl 3-(2-(((*S*)-1-(3-(1-(4-chloro-3-(2,4-dioxotetrahydropyrimidin-1(2*H*)-yl)benzoyl)piperidin-4-yl)propyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-*d*jpyrimidin-4-yl)-3,8-diazabicyclo[3.2.1]octane-8-carboxylate (140 mg, 0.11 mmol) in DMF (3 mL) at room temperature. The mixture was stirred for 30 min and extracted with EA and water. The organic phase was concentrated. A mixture of 1,4-dioxane (2 mL) and HCl/1,4-dioxane (6 N, 1 mL) was then added to the resulting mixture at room temperature. The reaction liquid was stirred for 0.5 h and directly concentrated under reduced pressure, and the resulting crude product was purified by pre-HPLC to give 1-(5-(4-(3-((5)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)propyl)piperidine-1-carbonyl)-2-chlorophenyl)dihydropyrimidine-2,4(1*H*,3*H*)-dione.

LC-MS: (ESI, m/z): [M+H]⁺ = 918.2.

¹H NMR (400 MHz, CD₃OD) δ 9.11 (d, *J* = 5.4 Hz, 1H), 7.90 - 7.82 (m, 1H), 7.61 (dd, *J =* 8.3, 2.5 Hz, 1H), 7.53 - 7.46 (m, 1H), 7.39 - 7.28 (m, 3H), 7.23 - 7.19 (m, 1H), 4.56 - 4.41 (m, 2H), 4.31 - 4.24 (m, 2H), 4.10 - 3.96 (m, 3H), 3.81 - 3.73 (m, 3H), 3.56 - 3.47 (m, 1H), 3.16 - 3.11 (m, 1H), 2.89 - 2.81 (m, 2H), 2.44 - 2.34 (m, 1H), 2.20 - 2.11 (m, 6H), 1.86 - 1.78 (m, 2H), 1.65 - 1.55 (m, 2H), 1.37 - 1.27 (m, 12H), 1.16 - 1.03 (m, 2H).

### Example 10: 1-(5-(4-((2-(1-((1-(((4-(3,8-diazabicyclo[3.2.1]octan-3-yl)-7-(8-ethynyl-7-fluoro-3-hydroxynaphthalen-1-yl)-8-fluoropyrido[4.3-d]pyrimidin-2-yl)oxy)methyl)cyclohexyl)methyl)piperidin-4-yl)ethoxy)methyl)piperidine-1-carbonyl)-2-chlorophenyl)dihydropyrimidine-2,4(1H,3H)-dione

### Step 1: preparation of tert-butyl 4-(2-(pyridin-4-ylmethoxy)ethyl)piperidine-1-carboxylate

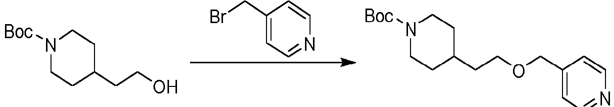

NaH (1.75 g, 43.6 mmol, 60%) was added to a solution of *tert*-butyl 4-(2-hydroxyethyl)piperidine-1-carboxylate (2 g, 8.72 mmol) in THF (10 mL) at 0 °C. The mixture was stirred at room temperature for 0.5 h, and then 4-(bromomethyl)pyridine (3.4 g, 13.08 mmol) was added. The reaction liquid was stirred at room temperature for 12 h. An aqueous ammonium chloride solution was added to quench the reaction liquid, and the mixture was extracted with ethyl acetate. The organic phase was dried and concentrated, and the resulting crude product was purified by silica gel column chromatography (DCM:MeOH = 100:1) to give *tert-butyl* 4-(2-(pyridin-4-ylmethoxy)ethyl)piperidine-1-carboxylate.

LC-MS: (ESI, m/z): [M+H]⁺= 321.2.

### Step 2: preparation of tert-butyl 4-(2-(piperidin-4-ylmethoxy)ethyl)piperidine-1-carboxylate

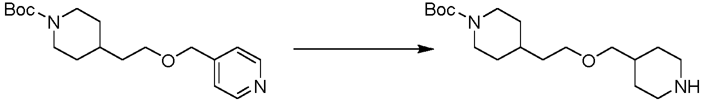

Pd/C (500 mg) was added to a solution of *tert*-butyl 4-(2-(pyridin-4-ylmethoxy)ethyl)piperidine-1-carboxylate (2.4 g, 7.49 mmol) in *i*-PrOH/H₂O (20 mL/20 mL), and the mixture was stirred at 75 °C for 16 h under hydrogen atmosphere. The reaction liquid was filtered through celite, and the filtrate was concentrated under reduced pressure. The resulting crude product was purified by silica gel column chromatography (MeOH:DCM = (0-1):4) to give *tert*-butyl 4-(2-(piperidin-4-ylmethoxy)ethyl)piperidine-1-carboxylate.

¹H NMR (400 MHz, CDCl₃) δ 4.05 (s, 1H), 3.44 (t, *J* = 6.3 Hz, 2H), 3.23 (d, *J =* 6.0 Hz, 2H), 3.11 - 3.04 (m, 2H), 2.74 - 2.55 (m, 4H), 1.93 (s, 2H), 1.75 - 1.62 (m, 5H), 1.59 - 1.42 (m, 12H), 1.19 - 1.04 (m, 4H).

### Step 3: preparation of tert-butyl 4-(2-((1-(4-chloro-3-(2,4-dioxotetrahydropyrimidin-1(2H)-yl)benzoyl)piperidin-4-yl)methoxy)ethyl)piperidine-1-carboxylate

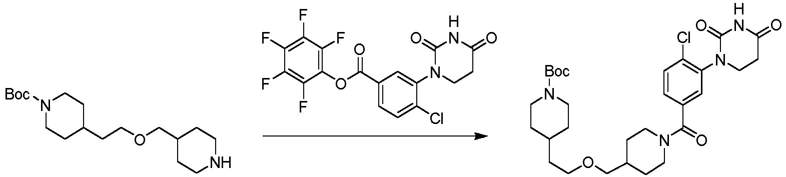

Pentafluorophenyl 4-chloro-3-(2,4-dioxotetrahydropyrimidin-1(2*H*)-yl)benzoate (0.28 g, 0.64 mmol) and DIEA (0.24 g, 1.84 mmol) were added to a solution of *tert-butyl* 4-(2-(piperidin-4-ylmethoxy)ethyl)piperidine-1-carboxylate (200 mg, 0.61 mmol) in DMSO (5 mL). The mixture was stirred at room temperature for 1 h. The crude product was purified by Pre-HPLC (ACN/H₂ = 0%-100%) to give *tert-butyl* 4-(2-((1-(4-chloro-3-(2,4-dioxotetrahydropyrimidin-1(2H)-yl)benzoyl)piperidin-4-yl)methoxy)ethyl)piperidine-1-carboxylate.

LC-MS: (ESI, m/z): [M+Na]⁺ = 599.2.

### Step 4: preparation of 1-(2-chloro-5-(4-((2-(piperidin-4-yl)ethoxy)methyl)piperidine-1-carbonyl)phenyl)dihydropyrimidine-2,4(1H,3H)-dione

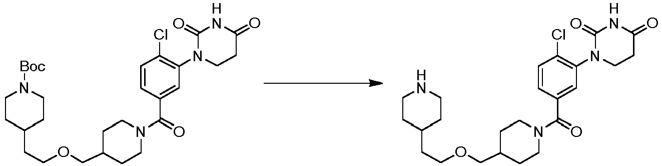

TFA (2 mL) was added to a solution of *tert-butyl* 4-(2-((1-(4-chloro-3-(2,4-dioxotetrahydropyrimidin-1(2*H*)-yl)benzoyl)piperidin-4-yl)methoxy)ethyl)piperidine-1-carboxylate (120 mg, 0.21 mmol) in DCM (4 mL). The mixture was stirred at room temperature for 1 h. The reaction liquid was directly concentrated under reduced pressure to give 1-(2-chloro-5-(4-((2-(piperidin-4-yl)ethoxy)methyl)piperidine-1-carbonyl)phenyl) dihydropyrimidine-2,4(1H,3H)-dione. The resulting crude product was directly used in the next step.

LC-MS: (ESI, m/z): [M+H]⁺= 477.2.

### Step 5: preparation of tert-butyl 3-(2-((1-((4-(2-((1-(4-chloro-3-(2,4-dioxotetrahydropyrimidin-1(2H)-yl)benzoyl)piperidin-4-yl)methoxy)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

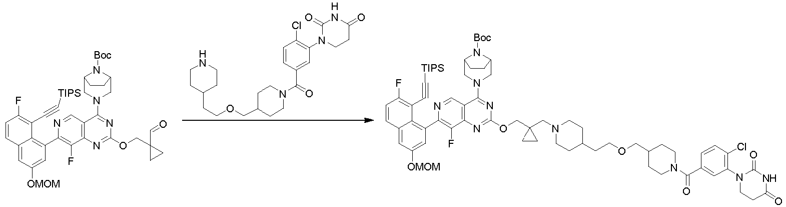

1-(2-Chloro-5-(4-((2-(piperidin-4-yl)ethoxy)methyl)piperidine-1-carbonyl)phenyl)dihydropyrimidine-2,4(1H,3H)-dione (54 mg, 0.11 mmol) and tetraisopropyl titanate (405 mg, 1.43 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido [4,3-d]pyrimidin-4-yl)-3,8-diazabicyclo[3.2.1]octane-8-carboxylate (80 mg, 0.095 mmol) in THF/MeOH (3 mL/3 mL), and the mixture was stirred at 70 °C for 2 h. NaBH(OAc)₃ (61 mg, 0.28 mmol) was then added at room temperature, and the mixture was stirred for another 2 h. The reaction liquid was directly concentrated, and the resulting crude product was purified by silica gel column chromatography (DCM:MeOH = 100:1 to 20:1) to give *tert-*butyl 3-(2-((1-((4-(2-((1-(4-chloro-3-(2,4-dioxotetrahydropyrimidin-1(2H)-yl)benzoyl)piperidin-4-yl)methoxy)ethyl)piperidin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-djpyrimidin-4-yl)-3,8-diazabicyclo[3.2.1]octane-8-carboxylate.

LC-MS: (ESI, m/z): [M/2+H]⁺ = 652.0.

### Step 6: preparation of 1-(5-(4-((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclohexyl)methyl)piperidin-4-yl)ethoxy)methyl)piperidine-1-carbonyl)-2-chlorophenyl)dihydropyrimidine-2,4(1H,3H)-dione

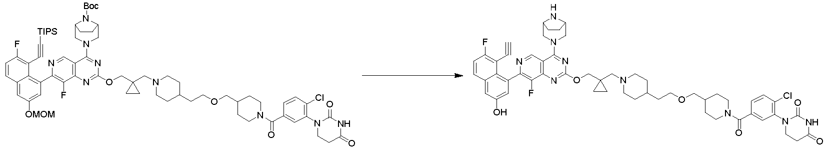

Cesium fluoride (40 mg, 0.27 mmol) was added to a solution of *tert*-butyl 3-(2-((1-((4-(2-((1-(4-chloro-3-(2,4-dioxotetrahydropyrimidin-1(2H)-yl)benzoyl)piperidin-4-yl)methoxy)ethyl)piperidin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate (70 mg, 0.054 mmol) in DMF (2 mL), and the mixed solution was stirred at room temperature for 30 min. Water was added to the mixed solution, and the mixture was extracted with ethyl acetate. The organic phase was concentrated. A mixture of 1,4-dioxane (2 mL) and HCl/1,4-dioxane (6 N, 1 mL) was then added to the resulting mixture at room temperature. The reaction mixture was reacted for another 0.5 h. The crude product was purified by Pre-HPLC to give 1-(5-(4-((2-(1-((1-(((4-(3,8-diazabicyclo[3.2.1]octan-3-yl)-7-(8-ethynyl-7-fluoro-3-hydroxynaphthalen-1-y1)-8-fluoropyrido[4.3-*d*]pyrimidin-2-yl)oxy)methyl)cyclohexyl)methyl)piperidin-4-yl)ethoxy)methyl)piperidine-1-carbonyl)-2-chlorophenyl)dihydropyrimidine-2,4(1H,3H)-dione.

LC-MS: (ESI) m/z: [M+H]⁺ = 1002.1.

¹H NMR (400 MHz, CD₃OD) δ 8.99 (s, 1H), 7.88 - 7.81 (m, 1H), 7.62 (d, *J* = 8.4 Hz, 1H), 7.52 (d, *J=* 1.6 Hz, 1H), 7.43 - 7.37 (m, 1H), 7.35 - 7.28 (m, 2H), 7.20 (d, *J =* 2.4 Hz, 1H), 4.67 - 4.31 (m, 6H), 3.81 - 3.63 (m, 7H), 3.44 (t, *J* = 6.2 Hz, 2H), 3.34 (s, 1H), 3.30 - 3.23 (m, 3H), 3.14 - 3.02 (m, 3H), 2.90 - 2.79 (m, 3H), 2.52 - 2.36 (m, 2H), 2.00 - 1.91 (m, 2H), 1.88 - 1.77 (m, 5H), 1.66 (d, *J =* 11.7 Hz, 2H), 1.49 - 1.42 (m, 2H), 1.34 - 1.26 (m, 5H), 0.75 - 0.66 (m, 2H), 0.56 - 0.44 (m, 2H).

### Example 11: 1-(5-(4-(3-(4-((1-(((4-(3,8-diazabicyclo[3.2.1]octan-3-yl)-7-(8-ethynyl-7-fluoro-3-cyanonaphthalen-1-yl)-8-fluoropyrido[4,3-d]pyrimidin-2-yl)oxy)methyl)cyclohexyl)methyl)piperazin-1-yl)propyl)piperidine-1-carbonyl)-2-fluorophenyl)dihydropyrimidine-2,4(1H,3H)-dione

### Step 1: preparation of 1-(2-fluoro-5-(4-(3-hydroxypropyl)piperidine-1-carbonyl)phenyl)dihydropyrimidine-2,4(1H,3H)-dione

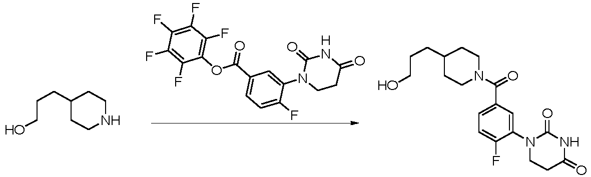

Pentafluorophenyl 4-fluoro-3-(2,4-dioxotetrahydropyrimidin-1(2H)-yl)benzoate (797 mg, 1.91 mmol) and DIEA (702 mg, 5.43 mmol) were added to a solution of 3-(piperidin-4-yl)propan-1-ol (260 mg, 1.81 mmol) in DMSO (5 mL). The mixture was stirred at room temperature for 1 h and purified by reversed-phase column chromatography to give 1-(2-fluoro-5-(4-(3-hydroxypropyl)piperidine-1-carbonyl)phenyl)dihydropyrimidine-2,4(1H,3H)-dione.

LC-MS: (ESI, m/z): [M+H]⁺= 378.1.

### Step 2: preparation of 3-(1-(3-(2,4-dioxotetrahydropyrimidin-1(2H)-yl)-4-fluorobenzoyl)piperidin-4-yl)propanal

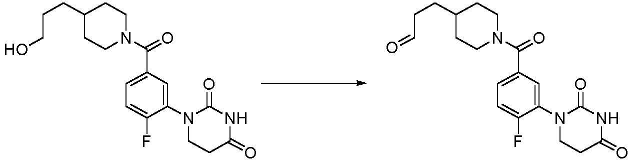

DMP (338 mg, 0.79 mmol) was added to a solution of 1-(2-fluoro-5-(4-(3-hydroxypropyl)piperidine-1-carbonyl)phenyl)dihydropyrimidine-2,4(1H,3H)-dione (250 mg, 0.66 mmol) in DCM (5 mL). The mixture was reacted for 1 h and filtered, and the filtrate was concentrated. The resulting crude product was purified by silica gel column chromatography (DCM:MeOH = 20:1) to give 3-(1-(3-(2,4-dioxotetrahydropyrimidin-1(2*H*)-yl)-4-fluorobenzoyl)piperidin-4-yl)propanal.

LC-MS: (ESI, m/z): [M+H]⁺= 376.1.

### Step 3: preparation of tert-butyl 3-(2-((1-((4-(3-(1-(3-(2,4-dioxotetrahydropyrimidin-1(2H)-yl)-4-fluorobenzoyl)piperidin-4-yl)propyl)piperazin-1-yl)methyl)cyclohexyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

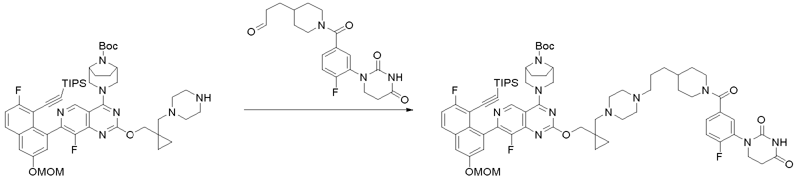

HOAc (0.1 mL) and 3-(1-(3-(2,4-dioxotetrahydropyrimidin-1(2H)-yl)-4-fluorobenzoyl)piperidin-4-yl)propanal (50 mg, 0.13 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (100 mg, 0.11 mmol) in DCM/MeOH (3 mL/3 mL). The mixture was stirred at room temperature for 0.5 h, and then cooled to 0 °C. NaBH₃CN (14 mg, 0.22 mmol) was added, and the mixture was stirred for 0.5 h. The reaction liquid was directly concentrated, and the resulting crude product was purified by silica gel column chromatography (DCM:MeOH = 20:1) to give *tert-butyl* 3-(2-((1-((4-(3-(1-(3-(2,4-dioxotetrahydropyrimidin-1(2H)-yl)-4-fluorobenzoyl)piperidin-4-yl)propyl)piperazin-1-yl)methyl)cyclohexyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 636.6.

### Step 4: preparation of 1-(5-(4-(3-(4-((1-(((4-(3,8-diazabicyclo[3.2.1]octan-3-yl)-7-(8-ethynyl-7-fluoro-3-cyanonaphthalen-1-yl)-8-fluoropyrido [4,3-d]pyrimidin-2-yl)oxy)methyl)cyclohexyl)methyl)piperazin-1-yl)propyl)piperidine-1-carbonyl)-2-fluorophenyl)dihydropyrimidine-2,4(1H,3H)-dione

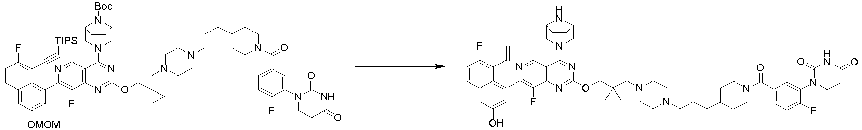

*tert-Butyl* 3-(2-((1-((4-(3-(1-(3-(2,4-dioxotetrahydropyrimidin-1(2*H*)-yl)-4-fluorobenzoyl)piperidin-4-yl)propyl) piperazin-1-yl)methyl)cyclohexyl)methoxy)-8-fluoro-7-(7-fluoro-3-(methoxymethoxy)-8-((triisopropylsilyl) ethynyl)naphthalen-1-yl)pyrido[4,3-d]pyrimidin-4-yl)-3,8-diazabicyclo[3.2.1]octane-8-carboxylate (110 mg, 0.086 mmol) was dissolved in DMF (3 mL), and cesium fluoride (66 mg, 0.43 mmol) was added. The mixture was stirred at room temperature for 30 min. Water (10 mL) was added to the reaction liquid, and the mixture was extracted with ethyl acetate. The organic phase was concentrated. The concentrate was dissolved in 1,4-dioxane (2 mL), a mixture of HCl/1,4-dioxane (6 N, 1 mL) was added, and the resulting mixture was reacted for 0.5 h. The reaction liquid was directly concentrated under reduced pressure, and the resulting crude product was purified by Pre-HPLC to give 1-(5-(4-(3-(4-((1-(((4-(3,8-diazabicyclo[3.2.1]octan-3-yl)-7-(8-ethynyl-7-fluoro-3-cyanonaphthalen-1-yl)-8-fluoropyrido[4,3-d]pyrimidin-2-yl)oxy)methyl)cyclohexyl)methyl) piperazin-1-yl)propyl)piperidine-1-carbonyl)-2-fluorophenyl)dihydropyrimidine-2,4(1H,3H)-dione.

LC-MS: (ESI, m/z): [M+H]⁺= 971.1.

¹H NMR (400 MHz, DMSO-d₆) δ 10.52 (s, 1H), 10.15 (s, 1H), 9.02 (s, 1H), 8.08 - 7.86 (m, 1H), 7.50 - 7.43 (m, 2H), 7.41 - 7.34 (m, 3H), 7.16 (d, *J* = 2.4 Hz, 1H), 4.48 (d, *J =* 11.6 Hz, 1H), 4.32 - 4.22 (m, 3H), 3.92 (s, 1H), 3.75 (t, *J* = 2.4 Hz, 2H), 3.66 - 3.42 (m, 5H), 3.11 - 2.60 (m, 6H), 2.46 - 2.10 (m, 12H), 1.68 - 1.62 (m, 4H), 1.49 - 1.36 (m, 3H), 1.28 - 1.13 (m, 4H), 1.12 - 0.97 (m, 2H), 0.68 - 0.55 (m, 2H), 0.43 - 0.35 (m, 2H).

### Example 12: 1-(5-(9-(((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)methyl)-3-azaspiro[5.5]undecane-3-carbonyl)-2-chlorophenyl)dihydropyrimidine-2,4(1H,3H)-dione

### Step 1: preparation of tert-butyl (S)-9-((2-(hydroxymethyl)pyrrolidin-1-yl)methyl)-3-azaspiro[5.5]undecane-3-carboxylate

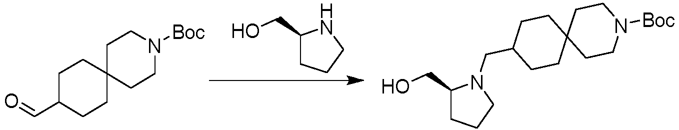

Acetic acid (0.5 mL) was added to a solution of *tert-butyl* 9-formyl-3-azaspiro[5.5]undecane-3-carboxylate (5.56 g, 19.77 mmol) and (5)-pyrrolidin-2-ylmethanol (3.00 g, 29.66 mmol) in 1,2-dichloroethane (150 mL). The mixture was stirred at 30 °C for half an hour. NaBH(OAc)₃ (8.42 g, 39.54 mmol) was added at 0 °C, and the mixture was reacted at room temperature for 2 h. After the reaction was completed, H₂O (100 mL) was added to the reaction liquid, and then the mixture was extracted with ethyl acetate (100 mL × 3). The combined organic phase was dried over anhydrous sodium sulfate, filtered, and concentrated under reduced pressure. The crude product was purified by silica gel column chromatography (DCM:MeOH = 20:1) to give *tert-butyl* (S)-9-((2-(hydroxymethyl)pyrrolidin-1-yl)methyl)-3-azaspiro [5.5 ]undecane-3-carboxylate.

LC-MS: (ESI, m/z): [M+H]⁺= 367.2.

### Step 2: preparation of (S)-(1-((3-azaspiro[5.5]undecan-9-yl)methyl)pyrrolidin-2-yl)methanol

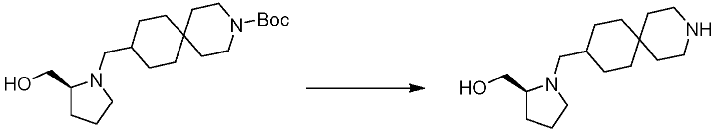

An HCl/1,4-dioxane solution (6 N, 10 mL) was added to a solution of *tert*-butyl (*S*)-9-((2-(hydroxymethyl)pyrrolidin-1-yl)methyl)-3-azaspiro[5.5]undecane-3-carboxylate (1.00 g, 2.73 mmol) in 1,4-dioxane (10 mL), and the mixture was reacted at room temperature for 2 h. The reaction liquid was concentrated to give (*S*)-(1-((3-azaspiro[5.5]undecan-9-yl)methyl)pyrrolidin-2-yl)methanol, and the resulting crude product was directly used in the next step.

### Step 3: preparation of (S)-(1-((3-benzyl-3-azaspiro[5.5]undecan-9-yl)methyl)pyrrolidin-2-yl)methanol

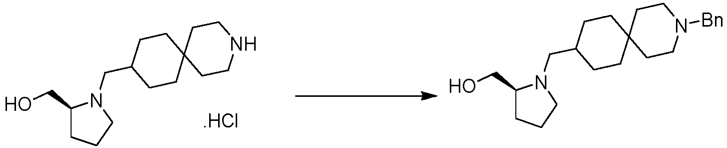

Triethylamine (0.67 g, 6.60 mmol) was added to a mixed solution of (S)-(1-((3-azaspiro[5.5]undecan-9-yl)methyl)pyrrolidin-2-yl)methanol hydrochloride (1.60 g, 10.73 mmol) in 1,2-dichloroethane/methanol (28 mL/14 mL), and the mixture was stirred for 3 min. Benzaldehyde (1.05 g, 9.90 mmol) and acetic acid (3 mL) were added. The mixture was stirred at 30 °C for 1 h. NaBH(OAc)₃ (0.52 g, 8.25 mmol) was added at 0 °C, and the mixture was reacted at room temperature for 16 h. After the reaction was completed, a sodium carbonate solution was added to adjust the pH > 7, and the mixture was extracted with ethyl acetate (30 mL × 3). The combined organic layer was dried over anhydrous sodium sulfate, filtered, and concentrated in vacuum. The crude product was purified by silica gel column chromatography (DCM:MeOH = 20:1) to give (S)-(1-((3-benzyl-3-azaspiro[5.5]undecan-9-yl)methyl)pyrrolidin-2-yl)methanol.

¹H NMR (400 MHz, CDCl₃) δ 7.33 - 7.29 (m, 4H), 7.20 - 7.25 (m, 1H), 3.60 (dd, *J* = 10.6, 3.4 Hz, 1H), 3.50 (s, 2H), 3.35 (d, *J* = 10.5 Hz, 1H), 3.19 - 3.09 (m, 1H), 2.58 - 2.47 (m, 1H), 2.45 - 2.33 (m, 5H), 2.22 - 2.12(m, 2H), 1.86 - 1.60 (m, 7H), 1.55 - 1.36 (m, 6H), 1.12 - 0.96 (m, 4H).

### Step 4: preparation of tert-butyl 3-(2-(((5)-1-((3-benzyl-3-azaspiro[5.5]undecan-9-yl)methyl)pyrrolidin-2-yl)methoxy)-7-chloro-8-fluoropyrido[4,3-d]pyrimidin-4-yl)-3,8-diazabicyclo[3.2.1]octane-8-carboxylate

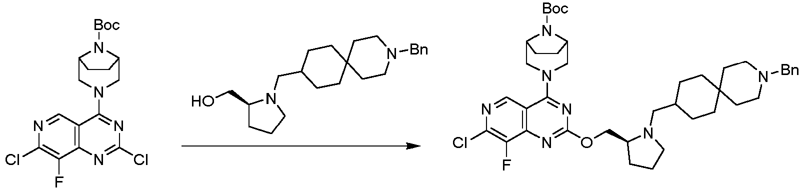

*tert*-Butyl 3-(2,7-dichloro-8-fluoropyrido[4,3-d]pyrimidin-4-yl)-3,8-diazabicyclo[3.2.1]octane-8-carboxylate (0.55 g, 1.28 mmol) and (S)-(1-((3-benzyl-3-azaspiro[5.5]undecan-9-yl)methyl)pyrrolidin-2-yl)methanol (0.75 g, 2.10 mmol) were dissolved in THF (10 mL), and Cs₂CO₃ (1.37 g, 4.20 mmol) was added. The reaction liquid was heated to 85 °C and reacted for 16 h. After the reaction was completed, the mixture was cooled to room temperature. Water (50 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was purified by silica gel column chromatography (PE:EA = 5:2) to give *tert-butyl* 3-(2-(((5)-1-((3-benzyl-3-azaspiro[5.5]undecan-9-yl)methyl)pyrrolidin-2-yl)methoxy)-7-chloro-8-fluoropyrido[4,3-d]pyrimidin-4-yl)-3,8-diazabicyclo[3.2.1]octane-8-carboxylate.

LC-MS: (ESI, m/z): [M+H]⁺= 748.3.

### Step 5: preparation of tert-butyl 3-(2-(((5)-1-((3-azaspiro[5.5]undecan-9-yl)methyl)pyrrolidin-2-yl)methoxy)-7-chloro-8-fluoropyrido[4,3-d]pyrimidin-4-yl)-3,8-diazabicyclo[3.2.1]octane-8-carboxylate

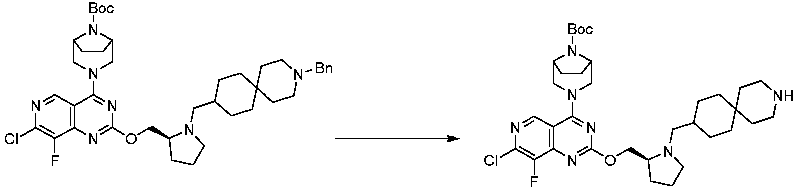

*tert-*Butyl 3-(2-(((S)-1-((3-benzyl-3-azaspiro[5.5]undecan-9-yl)methyl)pyrrolidin-2-yl)methoxy)-7-chloro-8-fluoropyrido[4,3-*d*]pyrimidin-4-yl)-3,8-diazabicyclo[3.2.1]octane-8-carboxylate (100 mg, 0.134 mmol) was dissolved in DCM (2 mL), and then 1-chloroethyl chloroformate (38 mg, 0.267 mmol) was added. The mixture was reacted at room temperature for 1 h, and then MeOH (2 mL) was added. The mixture was reacted for another 1 h. NH₃/MeOH was added to the reaction liquid, and the mixture was concentrated and purified by silica gel column chromatography (DCM:MeOH = 100:1) to give *tert*-butyl 3-(2-(((S)-1-((3-azaspiro[5.5]undecan-9-yl)methyl)pyrrolidin-2-yl)methoxy)-7-chloro-8-fluoropyrido[4,3-d]pyrimidin -4-yl)-3,8-diazabicyclo[3.2.1]octane-8-carboxylate.

LC-MS: (ESI, m/z): [M+H]⁺= 658.3.

### Step 6: preparation of tert-butyl 3-(2-(((S)-1-((3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

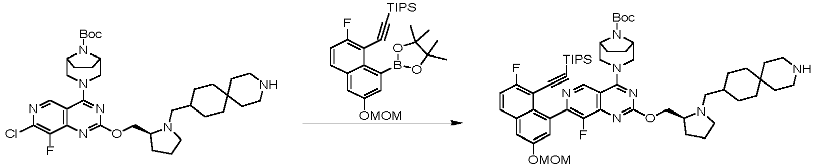

((2-Fluoro-6-(methoxymethoxy)-8-(4,4,5,5-tetramethyl-1,3,2-dioxaborolan-2-yl)naphthalen-1-yl)ethynyl)triisopropylsilane (70 mg, 0.14 mmol), cesium carbonate (77 mg, 0.23 mmol), and cataXium A Pd-G3 (7 mg, 0.0091 mmol) were added to a solution of *tert-butyl* 3-(2-(((5)-1-((3-azaspiro[5.5]undecan-9-yl)methyl)pyrrolidin-2-yl)methoxy)-7-chloro-8-fluoropyrido[4,3-d]pyrimidin-4-yl)-3,8-diazabicyclo[3.2.1]octane-8-carboxylate (60 mg, 0.091 mmol) in dioxane/H₂O (1.0 mL/0.2 mL). The mixture was reacted at 75 °C for 16 h under N₂ atmosphere and filtered, and the filtrate was concentrated and purified by silica gel column chromatography (DCM:MeOH = 100:1 to 20:1) to give *tert-butyl 3-(2-*(((S)-1-((3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 504.7.

### Step 7: preparation of tert-butyl 3-(2-(((S)-1-((3-(4-chloro-3-(2,4-dioxotetrahydropyrimidin-1(2H)-yl)benzoyl)-3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

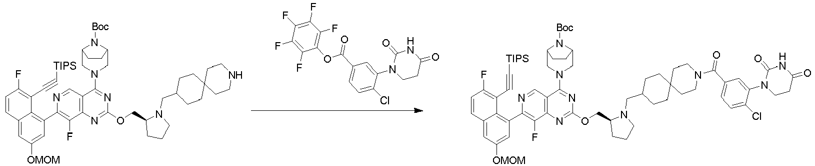

Pentafluorophenyl 4-chloro-3-(2,4-dioxotetrahydropyrimidin-1(2H)-yl)benzoate (28 mg, 0.017 mmol) and DIEA (19 mg, 0.015 mmol) were added to a solution of *tert-butyl* 3-(2-(((S)-1-((3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl) naphthalen-1-yl)pyrido[4,3-d]pyrimidin-4-yl)-3,8-diazabicyclo[3.2.1]octane-8-carboxylate (50 mg, 0.050 mmol) in DMSO (2 mL), and the mixture was reacted at room temperature for 1 h. Water (5 mL) was added to the reaction liquid, and the mixture was extracted with ethyl acetate (10 mL × 2). The organic phase was washed with saturated brine, dried over anhydrous sodium sulfate, and filtered. The filtrate was concentrated, and the resulting crude product was purified by silica gel column chromatography (DCM:MeOH = 100:1 to 20:1) to give *tert-butyl* 3-(2-(((S)-1-((3-(4-chloro-3-(2,4-dioxotetrahydropyrimidin-1(2H)-yl)benzoyl)-3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 629.7.

### Step 8: preparation of 1-(5-(9-(((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)methyl)-3-azaspiro[5.5]undecane-3-carbonyl)-2-chlorophenyl)dihydropyrimidine-2,4(1H,3H)-dione

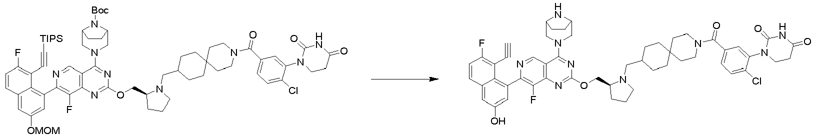

Cesium fluoride (30 mg, 0.20 mmol) was added to a solution of *tert-butyl* 3-(2-(((5)-1-((3-(4-chloro-3-(2,4-dioxotetrahydropyrimidin-1(2H)-yl)benzoyl)-3-azaspiro[5.5]undecan-9-yl)methyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (50 mg, 0.040 mmol) in DMF (1.5 mL), and the mixture was reacted at room temperature for 30 min. Water was added to the reaction liquid, and then the mixture was extracted with ethyl acetate. The organic phase was concentrated under reduced pressure. The concentrate was dissolved in 1,4-dioxane (2 mL), and an HCl/1,4-dioxane solution (6 N, 1 mL) was added. The mixture was reacted for 0.5 h and directly concentrated under reduced pressure. The resulting crude product was purified by Pre-HPLC to give 1-(5-(9-(((*S*)-2-(((4-(3,8-diazabicyclo[3.2.1]octan-3-y1)-7-(8-ethynyl-7-fluoro-3-hydroxynaphthalen-1-yl)-8-fluoropyrido[4,3-d]pyrimidin-2-yl)oxy)methyl)pyrrolidin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺= 958.3.

¹H NMR (400 MHz, CD₃OD) δ 9.05 (s, 1H), 8.52 (s, 1H), 7.91 - 7.82 (m, 1H), 7.76 - 7.47(m, 2H), 7.45 - 7.27 (m, 3H), 7.26 - 7.13 (m, 1H), 4.77-4.42 (m, 5H), 3.86 - 3.55 (m, 8H), 3.51 - 3.38 (m, 3H), 3.18 - 2.55 (m, 6H), 2.21-1.40 (m, 16H), 1.30 - 1.05 (s, 5H).

### Example 13: 1-(5-(4-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methoxy)methyl)piperidine-1-carbonyl)-2-chlorophenyl)dihydropyrimidine-2,4(1H,3H)-dione

### Step 1: preparation of tert-butyl 4-((pyridin-4-ylmethoxy)methyl)piperidine-1-carboxylate

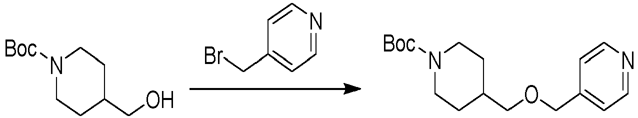

NaH (1.1 g, 27.9 mmol, 60%) was added to a solution of *tert*-butyl 4-(hydroxymethyl)piperidine-1-carboxylate (1 g, 4.65 mmol) in THF (10 mL) at 0 °C. The mixture was stirred at room temperature for 0.5 h, and then 4-(bromomethyl)pyridine (2.4 g, 9.3 mmol) was added. The reaction liquid was stirred at room temperature for 4 h. An aqueous ammonium chloride solution was added to quench the reaction liquid, and the mixture was extracted with ethyl acetate. The organic phase was dried and concentrated under reduced pressure, and the resulting crude product was purified by silica gel column chromatography (DCM:MeOH = 20:1) to give *tert-butyl* 4-((pyridin-4-ylmethoxy)methyl)piperidine-1-carboxylate.

LC-MS: (ESI, m/z): [M+H]⁺= 307.1.

### Step 2: preparation of tert-butyl 4-((piperidin-4-ylmethoxy)methyl)piperidine-1-carboxylate

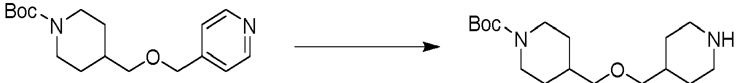

Pd/C (298 mg) was added to a mixed solution of *tert-butyl* 4-((pyridin-4-ylmethoxy)methyl)piperidine-1-carboxylate (850 mg, 2.77 mmol) in *i*-PrOH/H₂O (5 mL/5 mL), and the mixture was stirred at 75 °C for 16 h. The reaction liquid was filtered through celite, and the filtrate was concentrated under reduced pressure. The resulting crude product was purified by silica gel column chromatography (DCM:MeOH = 100:1 to 20:1) to give *tert-butyl* 4-((piperidin-4-ylmethoxy)methyl)piperidine-1-carboxylate.

LC-MS: (ESI, m/z): [M+H]⁺= 313.3.

### Step 3: preparation of tert-butyl 4-(((1-(4-chloro-3-(2,4-dioxotetrahydropyrimidin-1(2H)-yl)benzoyl)piperidin-4-yl)methoxy)methyl)piperidine-1-carboxylate

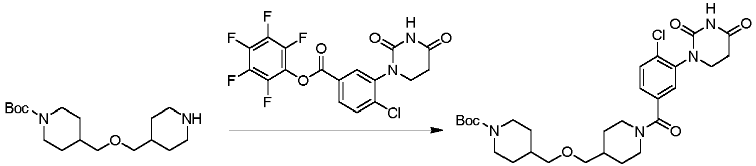

Pentafluorophenyl 4-chloro-3-(2,4-dioxotetrahydropyrimidin-1(2H)-yl)benzoate (139 mg, 0.32 mmol) and DIEA (124 g, 0.94 mmol) were added to a solution of *tert-butyl* 4-((piperidin-4-ylmethoxy)methyl)piperidine-1-carboxylate (100 mg, 0.32 mmol) in DMF (3 mL). The mixture was then stirred at room temperature for 3 h. The reaction liquid was directly purified by Pre-HPLC to give *tert-butyl* 4-(((1-(4-chloro-3-(2,4-dioxotetrahydropyrimidin-1(2H)-yl)benzoyl)piperidin-4-yl)methoxy)methyl)piperidine-1-carboxylate.

LC-MS: (ESI, m/z): [M-100+H]⁺ = 463.1.

### Step 4: preparation of 1-(2-chloro-5-(4-((piperidin-4-ylmethoxy)methyl)piperidine-1-carbonyl)phenyl)dihydropyrimidine-2,4(1H,3H)-dione

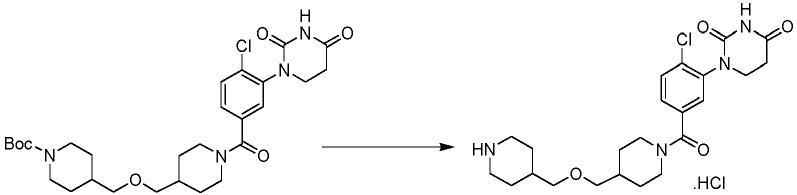

HCl/1,4-dioxane (6 N, 1 mL) was added to a solution of *tert-butyl* 4-(((1-(4-chloro-3-(2,4-dioxotetrahydropyrimidin-1(2*H*)-yl)benzoyl)piperidin-4-yl)methoxy)methyl)piperidine-1-carboxylate (160 mg, 0.28 mmol) in 1,4-dioxane (2 mL). The mixture was stirred at room temperature for 1 h. The reaction liquid was directly concentrated under reduced pressure to give 1-(2-chloro-5-(4-((piperidin-4-ylmethoxy)methyl)piperidine-1-carbonyl)phenyl)dihydropyrimidine-2,4(1H,3H)-dione.

LC-MS: (ESI, m/z): [M+H]⁺ = 463.2.

### Step 5: preparation of tert-butyl 3-(2-((1-((4-(((1-(4-chloro-3-(2,4-dioxotetrahydropyrimidin-1(2H)-yl)benzoyl)piperidin-4-yl)methoxy)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

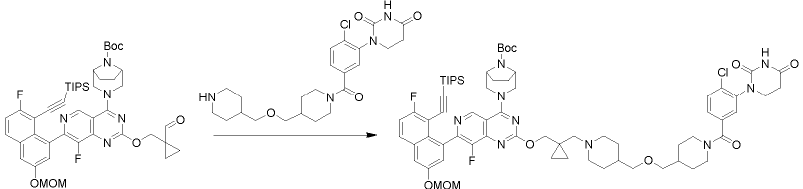

1-(2-Chloro-5-(4-((piperidin-4-ylmethoxy)methyl)piperidine-1-carbonyl)phenyl)dihydropyrimidine-2,4(1H,3H)-dione (80 mg, 0.17 mmol) and tetraisopropyl titanate (366 mg, 1.29 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-d]pyrimidin-4-yl)-3,8-diazabicyclo[3.2.1]octane-8-carboxylate (72 mg, 0.086 mmol) in THF (0.8 mL), and the mixture was stirred at 70 °C for 2 h. The above mixture was then cooled to room temperature. MeOH (0.3 mL) was first added, and then NaBH(OAc)₃ (54 mg, 0.26 mmol) was added in batches. The resulting reaction mixture was stirred for another 4 h. The reaction liquid was directly concentrated under reduced pressure, and the resulting crude product was purified by silica gel column chromatography (DCM:MeOH = 100:1 to 20:1) to give *tert-*butyl 3-(2-((1-((4-(((1-(4-chloro-3-(2,4-dioxotetrahydropyrimidin-1(2*H*)-yl)benzoyl)piperidin-4-yl)methoxy)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 644.8.

### Step 6: preparation of 1-(5-(4-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methoxy)methyl)piperidine-1-carbonyl)-2-chlorophenyl)dihydropyrimidine-2,4(1H,3H)-dione

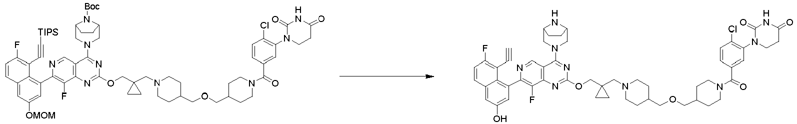

Cesium fluoride (40 mg, 0.27 mmol) was added to a solution of *tert-butyl* 3-(2-((1-((4-(((1-(4-chloro-3-(2,4-dioxotetrahydropyrimidin-1(2*H*)-yl)benzoyl)piperidin-4-yl)methoxy)methyl)piperidin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate (70 mg, 0.054 mmol) in DMF (2 mL), and the mixture was stirred at room temperature for 30 min. Water was added to the above mixture, and then the resulting mixture was extracted with ethyl acetate. The organic phase was concentrated under reduced pressure. 1,4-Dioxane (2 mL) and HCl/1,4-dioxane (6 N, 1 mL) were added to the resulting mixture at room temperature. The reaction liquid was stirred for 0.5 h and directly concentrated under reduced pressure, and the resulting crude product was purified by Pre-HPLC to give 1-(5-(4-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methoxy)methyl)piperidine-1-carbonyl)-2-chlorophenyl)dihydropyrimidine-2,4(1H,3H)-dione.

LC-MS: (ESI, m/z): [M+H]⁺= 988.3.

¹H NMR (400 MHz, DMSO-d₆) δ 10.50 (s, 1H), 10.14 (s, 1H), 9.02 (s, 1H), 7.97 (dd, *J=* 9.2, 6.0 Hz, 1H), 7.62 (d, *J =* 8.2 Hz, 1H), 7.53 (d, *J =* 1.9 Hz, 1H), 7.49 - 7.43 (m, 1H), 7.39 - 7.34 (m, 2H), 7.16 (d, *J=* 2.4 Hz, 1H), 4.54 - 4.36 (m, 2H), 4.31 - 4.20 (m, 3H), 3.92 (s, 1H), 3.79-3.69 (m, 1H), 3.67 - 3.45 (m, 6H), 3.22 - 3.10 (m, 5H), 3.09 - 2.86 (m, 4H), 2.79 - 2.70 (m, 3H), 2.34 - 2.21 (m, 3H), 1.84 - 1.76 (m, 3H), 1.67 - 1.55 (m, 6H), 1.48-1.41 (m, 1H), 1.16-1.04 (m, 4H), 0.66 - 0.60 (m, 2H), 0.42 - 0.35 (m, 2H).

### Example 14: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

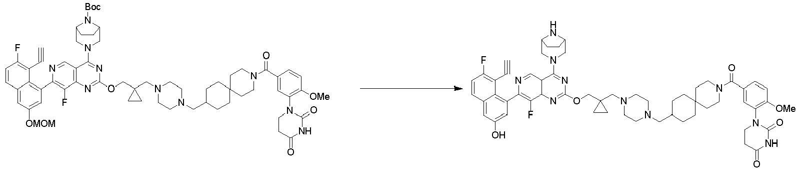

*tert-Butyl* 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (90 mg, crude) was dissolved in 1,4-dioxane (2 mL), and then HCl/1,4-dioxane (6 N, 1 mL) was added. The mixture was reacted at room temperature for 0.5 h. The reaction liquid was directly concentrated under reduced pressure, and the resulting crude product was purified by Pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, m/z): [M+H]⁺= 1023.7.

¹H NMR (400 MHz, DMSO-d₆) δ 10.32 (s, 1H), 10.13 (s, 1H), 9.02 (s, 1H), 7.97 (dd, *J=* 9.2, 5.9 Hz, 1H), 7.59 - 7.42 (m, 1H), 7.40 - 7.29 (m, 3H), 7.19-7.09 (m, 2H), 4.48 (d, *J =* 11.3 Hz, 1H), 4.34 - 4.18 (m, 3H), 3.92 (s, 1H), 3.84 (s, 3H), 3.71 - 3.35 (m, 10H), 2.68 (t, *J =* 6.6 Hz, 2H), 2.45 - 2.12 (m, 10H), 2.08 - 1.99 (m, 2H), 1.74 - 1.58 (m, 6H), 1.56 - 1.15 (m, 8H), 1.13 - 0.94 (m, 4H), 0.66 - 0.55 (m, 2H), 0.45 - 0.34 (m, 2H).

### Example 15: 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5|undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

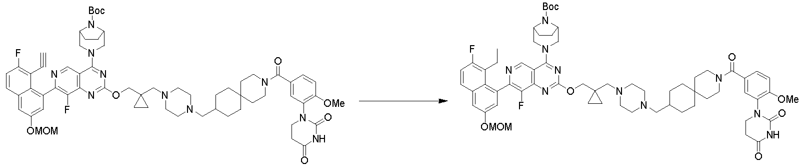

Pd/C (10 mg) was added to a solution of *tert-butyl* 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (40 mg, 0.034 mmol) in methanol (2 mL), and the mixture was reacted at room temperature for 2 h under hydrogen atmosphere. The reaction liquid was filtered through celite, and the filtrate was concentrated under reduced pressure to give *tert-butyl* 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate.

LC-MS: (ESI, m/z): [M+H]⁺= 586.4.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

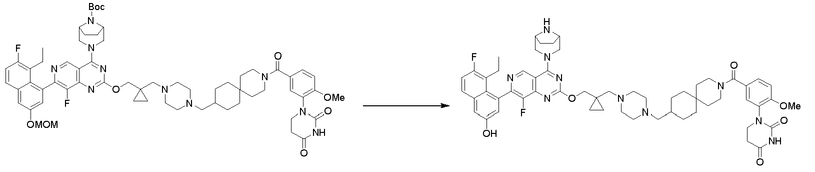

*tert*-Butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate (38 mg, crude) was dissolved in 1,4-dioxane (2 mL) and HCl/1,4-dioxane (6 N, 1 mL). The mixture was reacted at room temperature for 0.5 h. The reaction liquid was directly concentrated under reduced pressure, and the resulting crude product was purified by Pre-HPLC to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, m/z): [M+H]⁺= 1027.5.

¹H NMR (400 MHz, CD₃OD) δ 9.04 (s, 1H), 7.67 (dd, *J* = 9.0, 5.8 Hz, 1H), 7.43 (dd, *J* = 8.5, 2.0 Hz, 1H), 7.38 (d, *J=* 2.0 Hz, 1H), 7.29 (d, *J* = 2.6 Hz, 1H), 7.27 - 7.20 (m, 1H), 7.18 (d, *J* = 8.6 Hz, 1H), 7.05 (d, *J=* 2.5 Hz, 1H), 4.61 (t, *J* = 11.2 Hz, 2H), 4.52 - 4.33 (m, 2H), 3.91 (s, 3H), 3.76-3.40 (m, 10H), 2.80 (t, *J* = 6.7 Hz, 2H), 2.56 - 2.10 (m, 12H), 1.73 - 1.72 (m, 6H), 1.61 - 1.27 (m, 10H), 1.19 - 1.09 (m, 3H), 0.80 (t, *J=* 7.4 Hz, 3H), 0.74-0.64 (m, 2H), 0.55-0.43 (m, 2H).

### Example 16: 1-(5-(9-((4-((1-(((4-(3,8-diazabicyclo[3.2.1]octan-3-yl)-8-fluoro-7-(5-methyl-1H-indazol-4-yl)pyrido[4,3-d]pyrimidin-2-yl)oxy)methyl)cyclopropyl)methyl)piperazin-1-yl)methyl)-3-azaspiro[5.5] undecane-3-carbonyl)-2-chlorophenyl)dihydropyrimidine-2,4(1H,3H)-dione

### Step 1: preparation of tert-butyl 3-(8-fluoro-2-((1-formylcyclopropyl)methoxy)-7-(5-methyl-1H-indazol-4-yl)pyrido[4,3-d]pyrimidin-4-yl)-3,8-diazabicyclo[3.2.1]octane-8-carboxylate

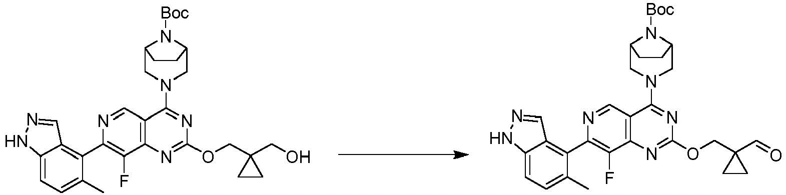

N-Methylmorpholine oxide (180 mg, 1.54 mmol) and tetrapropylammonium perruthenate (20 mg, 0.057 mmol) were added to a solution of *tert-butyl* 3-(8-fluoro-2-((1-(hydroxymethyl)cyclopropyl)methoxy)-7-(5-methyl-1*H*-indazol-4-yl)pyrido[4,3-d]pyrimidin-4-yl)-3,8-diazabicyclo[3.2.1]octane-8-carboxylate (400 mg, 0.68 mmol) in dichloromethane (5 mL), and the mixture was reacted at room temperature for 2 h. The reaction liquid was concentrated and purified by silica gel column chromatography (DCM/MeOH = 100/1 to 20:1) to give *tert-butyl* 3-(8-fluoro-2-((1-formylcyclopropyl)methoxy)-7-(5-methyl-1*H-*indazol-4-yl)pyrido[4,3-*d*]pyrimidin-4-yl)-3,8-diazabicyclo[3.2.1]octane-8-carboxylate.

LC-MS: (ESI, m/z): [M+H]⁺= 588.2.

### Step 2: preparation of tert-butyl 3-(2-((1-((4-((benzyloxy)carbonyl)piperazin-1-yl)methyl)cyclopropyl)methoxy)-8-fluoro-7-(5-methyl-1H-indazol-4-yl)pyrido[4,3-djpyrimidin-4-yl)-3,8-diazabicyclo[3.2.1]octane-8-carboxylate

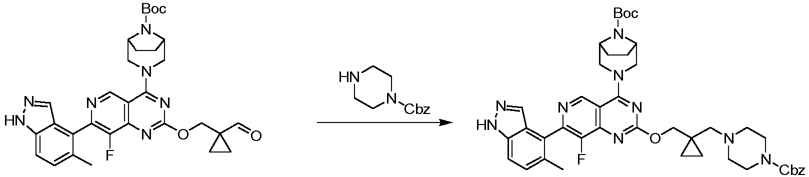

Tetraisopropyl titanate (191 mg, 0.87 mmol) was added to a mixed solution of *tert*-butyl 3-(8-fluoro-2-((1-formylcyclopropyl)methoxy)-7-(5-methyl-1*H*-indazol-4-yl)pyrido[4,3-*d*]pyrimidin-4-yl)-3,8-diazabicyclo [3.2.1]octane-8-carboxylate (170 mg, 0.29 mmol) and benzyl piperazine-1-carboxylate (191 mg, 0.87 mmol) in dichloromethane/acetic acid (2 mL/0.1 mL). The mixture was stirred at 30 °C for 2 h, and the reaction temperature was lowered to 0 °C. NaBH(OAc)₃ (184 mg, 0.87 mmol) was added, and the mixture was reacted at room temperature for another 16 h. After the reaction was completed, H₂O (10 mL) was added to the reaction liquid, and the mixture was extracted with ethyl acetate (30 mL × 3). The combined organic phase was dried over anhydrous sodium sulfate, filtered, and concentrated under reduced pressure. The crude product was purified by silica gel column chromatography (DCM:MeOH = 20:1) to give *tert-butyl* 3-(2-((1-((4-((benzyloxy)carbonyl)piperazin-1-yl)methyl)cyclopropyl)methoxy)-8-fluoro-7-(5-methyl-1H-indazol-4-yl)pyrido[4,3-*d*]pyrimidin-4-yl)-3,8-diazabicyclo[3.2.1]octane-8-carboxylate.

LC-MS: (ESI, m/z): [M+H]⁺= 792.3.

### Step 3: preparation of tert-butyl 3-(8-fluoro-7-(5-methyl-1H-indazol-4-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate

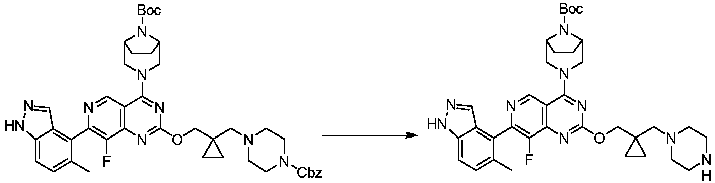

*tert-Butyl* 3-(2-((1-((4-((benzyloxy)carbonyl)piperazin-1-yl)methyl)cyclopropyl)methoxy)-8-fluoro-7-(5-methyl-1H-indazol-4-yl)pyrido[4,3-d]pyrimidin-4-yl)-3,8-diazabicyclo[3.2.1]octane-8-carboxylate (85 mg, 0.11 mmol) was dissolved in isopropanol (3 mL), Pd/C was added, and the mixture was reacted for 16 h under hydrogen atmosphere. The reaction liquid was filtered through celite, and the filtrate was concentrated under reduced pressure to give a crude product of *tert-butyl* 3-(8-fluoro-7-(5-methyl-1H-indazol-4-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate.

LC-MS: (ESI, m/z): [M+H]⁺= 658.3.

### Step 4: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(5-methyl-1H-indazol-4-yl)pyrido[4,3-d]pyrimidin-4-yl)-3,8-diazabicyclo[3.2.1]octane-8-carboxylate

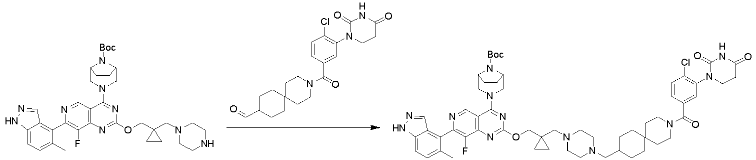

HOAc (0.05 mL) and 3-(4-chloro-3-(2,4-dioxotetrahydropyrimidin-1(2H)-yl)benzoyl)-3-azaspiro[5.5] undecane-9-carbaldehyde (62 mg, 0.14 mmol) were added to a solution of *tert-butyl* 3-(8-fluoro-7-(5-methyl-1H-indazol-4-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (80 mg, 0.12 mmol) in DCM/MeOH (1.0 mL/0.5 mL). The mixture was stirred at room temperature for 0.5 h, and then NaBH₃CN (76 mg, 0.36 mmol) was added to the mixture. The mixture was stirred for another 0.5 h. The reaction liquid was directly concentrated under reduced pressure, and the resulting crude product was purified by silica gel column chromatography (DCM:MeOH = 100:1 to 10:1) to give *tert-butyl* 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(5-methyl-1H-indazol-4-yl)pyrido[4,3-d]pyrimidin-4-yl)-3,8-diazabicyclo[3.2.1]octane-8-carboxylate.

LC-MS: (ESI, m/z): [M/2+H]⁺ = 1073.5.

### Step 5: preparation of 1-(5-(9-((4-((1-(((4-(3,8-diazabicyclo[3.2.1]octan-3-yl)-8-fluoro-7-(5-methyl-1H-indazol-4-yl)pyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

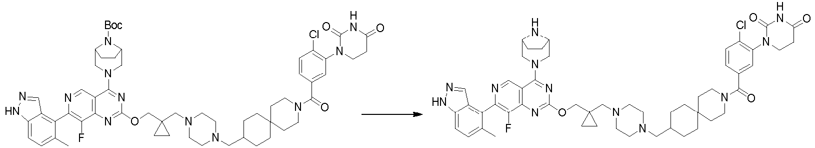

*tert-Butyl* 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(5-methyl-1H-indazol-4-yl)pyrido[4,3-d]pyrimidin-4-yl)-3,8-diazabicyclo[3.2.1]octane-8-carboxylate (60 mg, 0.056 mmol) was dissolved in 1,4-dioxane (2 mL), and then an HCl/1,4-dioxane solution (6 N, 1 mL) was added. The mixture was reacted for 0.5 h. The reaction liquid was directly concentrated under reduced pressure, and the resulting crude product was purified by Pre-HPLC to give 1-(5-(9-((4-((1-(((4-(3,8-diazabicyclo[3.2.1]octan-3-yl)-8-fluoro-7-(5-methyl-1H-indazol-4-yl)pyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M/2+H]⁺ = 487.2.

¹H NMR (400 MHz, DMSO-d₆) δ 13.09 (s, 1H), 10.50 (s, 1H), 9.16 (s, 1H), 7.69 - 7.50 (m, 4H), 7.41 - 7.30 (m, 2H), 4.56 - 4.34 (m, 2H), 4.30 (s, 2H), 3.82 - 3.68 (m, 1H), 3.67 - 3.43 (m, 7H), 3.30 - 3.15 (m, 3H), 2.78 - 2.69 (m, 2H), 2.44 - 2.10 (m, 13H), 2.05 - 1.97 (m, 2H), 1.71 - 1.57 (m, 6H), 1.50 - 1.24 (m, 7H), 1.11 - 1.90 (m, 4H), 0.69 - 0.56 (m, 2H), 0.44 - 0.33 (m, 2H).

### Example 17: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)-3-methyldihydropyrimidine-2,4(1H,3H)-dione

### Step 1: preparation of 3-(4-chloro-3-(3-methyl-2,4-dioxotetrahydropyrimidin-1(2H)-yl)benzoyl)-3-azaspiro[5.5 ]undecane-9-carbaldehyde

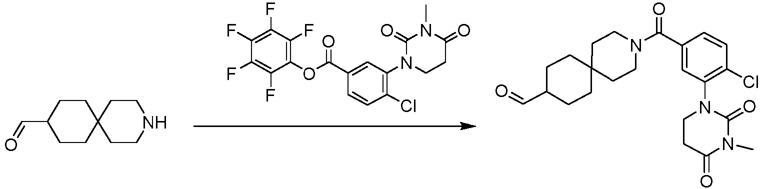

DIEA (411 mg, 3.18 mmol) and pentafluorophenyl 4-chloro-3-(3-methyl-2,4-dioxotetrahydropyrimidin-1(2H)-yl)benzoate (180 mg, 0.40 mmol) were added to a solution of 3-azaspiro[5.5]undecane-9-carbaldehyde (125 mg, crude) in DMF (3 mL), and the reaction liquid was stirred at room temperature for 1 h. Water was added to the reaction liquid, and the mixture was extracted with ethyl acetate. The organic phases were combined, dried over anhydrous sodium sulfate, and filtered. The filtrate was concentrated under reduced pressure, and the resulting crude product was purified by silica gel column chromatography (DCM:MeOH = 100:1 to 5:1) to give 3-(4-chloro-3-(3-methyl-2,4-dioxotetrahydropyrimidin-1(2*H*)-yl)benzoyl)-3-azaspiro[5.5]undecane-9-carbaldehyde.

LC-MS: (ESI, m/z): [M+H]⁺ = 446.2.

### Step 2: preparation of tert-butyl 3-(2-((1-((4-((3-(4-chloro-3-(3-methyl-2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

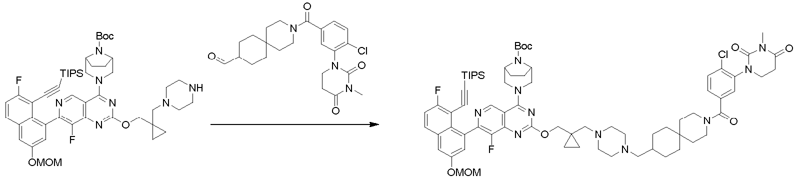

HOAc (0.1 mL) and 3-(4-chloro-3-(3-methyl-2,4-dioxotetrahydropyrimidin-1(2H)-yl)benzoyl)-3-azaspiro[5.5]undecane-9-carbaldehyde (76 mg, 0.17 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (100 mg, 0.11 mmol) in EtOH (2.0 mL), and the mixture was stirred at room temperature for 15 min, and then cooled to 0-5 °C. NaBH₃CN (21 mg, 0.33 mmol) was added, and the mixture was stirred for 0.5 h. The reaction liquid was directly concentrated under reduced pressure, and the resulting crude product was purified by silica gel column chromatography (DCM:MeOH = 100:1 to 5:1) to give *tert*-butyl 3-(2-((1-((4-((3-(4-chloro-3-(3-methyl-2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 671.4.

### Step 3: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)-3-methyldihydropyrimidine-2,4(1H,3H)-dione

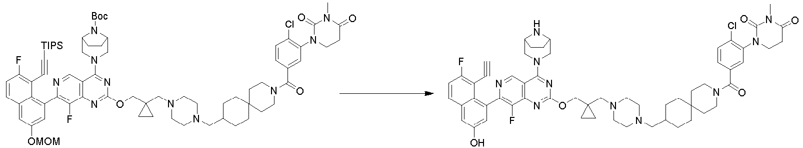

CsF (51 mg, 0.335 mmol) was added to a solution of *tert-butyl* 3-(2-((1-((4-((3-(4-chloro-3-(3-methyl-2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (90 mg, 0.067 mmol) in DMF (2.0 mL), and the mixture was reacted at room temperature for 1 h. Water (10 mL) was added to the above mixture, and the resulting mixture was extracted with ethyl acetate (10 mL × 2). The organic phases were combined, washed with saturated brine, dried over anhydrous sodium sulfate, and filtered, and the filtrate was checked and concentrated to give a crude product of the intermediate, which was dissolved in 1,4-dioxane (2.0 mL), and HCl/1,4-dioxane (6 N, 1.0 mL) was added. The mixture was reacted at room temperature for 0.5 h. An aqueous Na₂CO₃ solution was added to the reaction liquid to adjust the pH > 7, and then the mixture was extracted with EtOAc (10 mL × 2). The organic phase was washed with brine, dried over anhydrous Na₂SO₄, filtered, and concentrated under reduced pressure. The resulting crude product was purified by pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)-3-methyldihydropyrimidine-2,4(1*H*,3*H*)-dione.

LC-MS: (ESI, m/z): [M/2+H]⁺ = 521.2.
¹H NMR (400 MHz, DMSO-d₆) δ 10.15 (s, 1H), 9.03 (s, 1H), 7.97(dd, *J* = 9.2 Hz, 6.0 Hz, 1H), 7.63 (d, *J=* 8.4 Hz, 1H), 7.59 (d, *J* = 2.0 Hz, 1H), 7.49 - 7.35 (m, 3H), 7.17 (d, *J* = 2.4 Hz, 1H), 4.48 (d, *J* = 11.6 Hz, 1H), 4.33 - 4.20 (m, 3H), 3.92 (s, 1H), 3.78 - 3.45 (m, 9H), 3.29 - 3.21 (m, 2H), 3.15 (s, 3H), 2.92 - 2.80 (m, 2H), 2.45 - 2.17 (m, 9H), 2.10 - 1.95 (m, 1H), 1.73 - 1.58 (s, 6H), 1.55 - 1.37 (m, 5H), 1.35 - 1.19 (m, 3H), 1.11 - 0.92 (m, 4H), 0.71 - 0.54 (s, 2H), 0.45 - 0.31 (s, 2H).

### Example 18: 1-(5-(9-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)-3-azaspiro[5.5]undecane-3-carbonyl)-2-chlorophenyl)dihydropyrimidine-2,4(1H,3H)-dione

### Step 1: preparation of 2-(3-azaspiro[5.5]undecan-9-yl)acetaldehyde

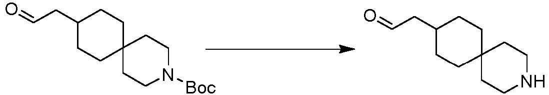

*tert-Butyl* 9-(2-oxoethyl)-3-azaspiro[5.5]undecane-3-carboxylate (250 mg, 0.85 mmol) was dissolved in DCM (2 mL), and then TFA (1 mL) was added. The mixture was stirred at room temperature for 1 h. The reaction liquid was directly concentrated under reduced pressure to give 2-(3-azaspiro[5.5]undecan-9-yl)acetaldehyde. The crude product was directly used in the next step without further purification.

LC-MS: (ESI, m/z): [M+H]⁺ = 196.2.

### Step 2: preparation of 2-(3-(4-chloro-3-(2,4-dioxotetrahydropyrimidin-1(2H)-yl)benzoyl)-3-azaspiro[5.5]undecan-9-yl)acetaldehyde

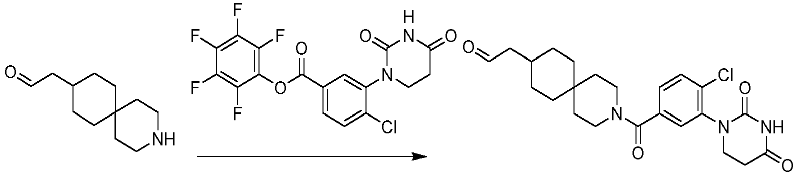

DIEA (496 mg, 3.84 mmol) and pentafluorophenyl 4-chloro-3-(2,4-dioxotetrahydropyrimidin-1(2H)-yl)benzoate (334 mg, 0.77 mmol) were added to a solution of 2-(3-azaspiro[5.5]undecan-9-yl)acetaldehyde (150 mg, 0.77 mmol) in DMF (5 mL), and the reaction liquid was stirred at room temperature for 1 h. Water was added to the reaction liquid, and the mixture was extracted with ethyl acetate. The organic phases were combined, dried over anhydrous sodium sulfate, and filtered. The filtrate was concentrated under reduced pressure, and the resulting crude product was purified by silica gel column chromatography (DCM:MeOH = 100:1 to 5:1) to give 2-(3-(4-chloro-3-(2,4-dioxotetrahydropyrimidin-1(2H)-yl)benzoyl)-3-azaspiro[5.5]undecan-9-yl)acetaldehyde.

LC-MS: (ESI, m/z): [M+H]⁺ = 446.2.

### Step 3: preparation of tert-butyl 3-(2-((1-((4-(2-(3-(4-chloro-3-(2,4-dioxotetrahydropyrimidin-1(2H)-yl)benzoyl)-3-azaspiro[5.5]undecan-9-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

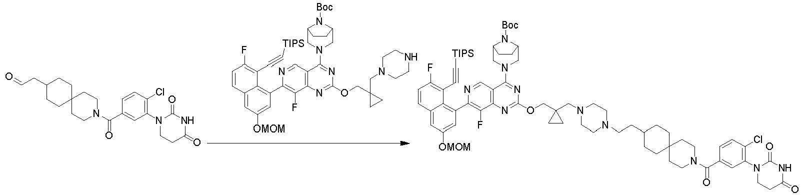

HOAc (0.1 mL) and 2-(3-(4-chloro-3-(2,4-dioxotetrahydropyrimidin-1(2*H*)-yl)benzoyl)-3-azaspiro[5.5] undecan-9-yl)acetaldehyde (73 mg, 0.17 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl) methoxy)pyrido[4,3-d]pyrimidin-4-yl)-3,8-diazabicyclo[3.2.1]octane-8-carboxylate (100 mg, 0.11 mmol) in EtOH (2.0 mL), and the mixture was stirred at room temperature for 15 min. The above mixture was then cooled to 0-5 °C. NaBH₃CN (21 mg, 0.33 mmol) was added, and the mixture was stirred for 0.5 h. The reaction liquid was directly concentrated under reduced pressure, and the resulting crude product was purified by silica gel column chromatography (DCM:MeOH = 100:1 to 5:1) to give *tert*-butyl 3-(2-((1-((4-(2-(3-(4-chloro-3-(2,4-dioxotetrahydropyrimidin-1(2H)-yl)benzoyl)-3-azaspiro[5.5]undecan-9-yl)ethyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 671.3.

### Step 4: preparation of 1-(5-(9-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)-3-azaspiro[5.5]undecane-3-carbonyl)-2-chlorophenyl)dihydropyrimidine-2,4(1H,3H)-dione

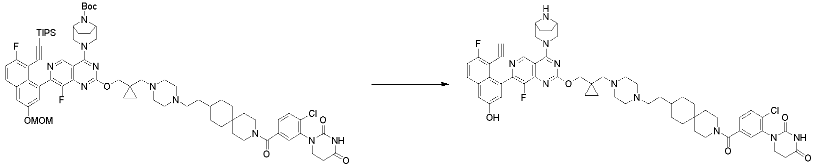

CsF (57 mg, 0.37 mmol) was added to a solution of *tert-butyl* 3-(2-((1-((4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (100 mg, 0.07 mmol) in DMF (2.0 mL), and the mixture was reacted at room temperature for 2 h. Water (10 mL) was added to the above mixture, and the resulting mixture was extracted with ethyl acetate (10 mL × 2). The organic phases were combined, washed with saturated brine, dried over anhydrous sodium sulfate, and filtered, and the filtrate was concentrated under reduced pressure to give a crude product of the intermediate, which was dissolved in dioxane (2.0 mL). HCl/dioxane (6 N, 1.0 mL) was added, and the mixture was reacted at room temperature for 0.5 h. An aqueous Na₂CO₃ solution was added to the reaction liquid to adjust the pH > 7, and the mixture was extracted with EtOAc (10 mL × 2). The organic phase was washed with brine, dried over anhydrous Na₂SO₄, and filtered, and the filtrate was concentrated under reduced pressure. The crude product was purified by Pre-HPLC to give 1-(5-(9-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 1041.0.

¹H NMR (400 MHz, DMSO-d₆) δ 10.51 (s, 1H), 10.15 (s, 1H), 9.02 (s, 1H), 7.97 (dd, *J* = 9.2 Hz, 6.0 Hz, 1H), 7.62 (d, *J =* 8.4 Hz, 1H), 7.55 - 7.35 (m, 4H), 7.17 (d, *J* = 2.4 Hz, 1H), 4.47 (d, *J =* 10.8 Hz, 1H), 4.33 - 4.19 (m, 3H), 3.91 (s, 1H), 3.79 - 3.71 (m, 1H), 3.66 - 3.49 (m, 7H), 3.27 - 3.21 (m, 2H), 2.80 - 2.68 (m, 2H), 2.48 - 2.12 (m, 13H), 1.65 - 1.59 (m, 5H), 1.52 - 0.95 (m, 14H), 0.69 - 0.56 (m, 2H), 0.46 - 0.32 (m, 2H).

### Example 19: 1-(5-(4-(3-(4-((1-(((4-(3,8-diazabicyclo[3.2.1]octan-3-yl)-7-(8-ethyl-3-hydroxynaphthalen-1-yl)-8-fluoropyrido[4.3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione

### Step 1: preparation of tert-butyl 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclopropyl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

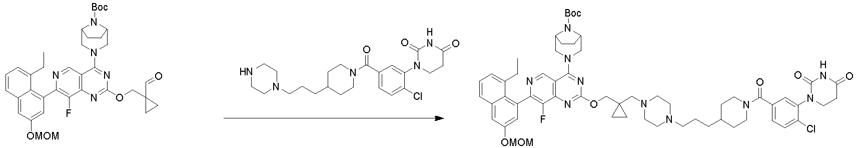

1-(2-Chloro-5-(4-(3-(piperazin-1-yl)propyl)piperidine-1-carbonyl)phenyl)dihydropyrimidine-2,4(1*H*,3*H*)-dione (69 mg, 0.15 mmol), AcOH (0.1 mL), and tetraisopropyl titanate (68 mg, 0.24 mmol) were added to a solution of *tert-butyl* 3-(7-(8-ethyl-3-(methoxymethoxy)naphthalen-1-yl)-8-fluoro-2-((1-formyleyclopropyl)methoxy)pyrido[4,3-d]pyrimidin-4-y1)-3,8-diazabicyclo[3.2.11]octane-8-carboxylate (80 mg, 0.12 mmol) in DCM (0.6 mL). The reaction liquid was stirred at room temperature for 2 h, and then cooled to 0 °C. NaBH(OAc)₃ (76 mg, 0.36 mmol) was added, and the mixture was reacted at room temperature for another 4 h. The reaction mixture was directly concentrated under reduced pressure, and the resulting crude product was purified by silica gel column chromatography (DCM:MeOH = 100:1 to 20:1) to give *tert-butyl* 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 559.2.

### Step 2: preparation of 1-(5-(4-(3-(4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione

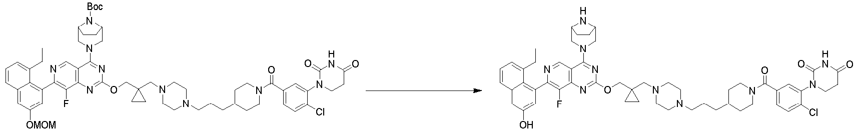

*tert-Butyl* 3-(2-((1-((4-(3-(1-(4-chloro-3-(2,4-dioxotetrahydropyrimidin-1(2H)-yl)benzoyl)piperidin-4-yl)propyl) piperazin-1-yl)methyl)cyclopropyl)methoxy)-7-(8-ethyl-3-(methoxymethoxy)naphthalen-1-yl)-8-fluoropyrido [4,3-d]pyrimidin-4-yl)-3,8-diazabicyclo[3.2.1]octane-8-carboxylate (50 mg, 0.045 mmol) was dissolved in 1,4-dioxane (2 mL), HCl/1,4-dioxane (6 N, 1 mL) was added, and the mixture was reacted at room temperature for 0.5 h. The reaction liquid was directly concentrated under reduced pressure, and the resulting crude product was purified by Pre-HPLC to give 1-(5-(4-(3-(4-((1-(((4-(3,8-diazabicyclo[3.2.1]octan-3-yl)-7-(8-ethyl-3-hydroxynaphthalen-1-yl)-8-fluoropyrido[4,3-*d*jpyrimidin-2-yl)oxy)methyl)cyclopropyl)methyl)piperazin-1-yl)propyl)piperidine-1-carbonyl)-2-chlorophenyl)dihydropyrimidine-2,4(1*H*,3*H*)-dione.

LC-MS: (ESI, m/z): [M+H]⁺ = 973.1.

¹H NMR (400 MHz, CD₃OD) δ 9.02 (s, 1H), 7.67 - 7.58 (m, 2H), 7.51 (s, 1H), 7.41 - 7.32 (m, 2H), 7.28 (d, *J=* 2.6 Hz, 1H), 7.16 (d, *J=* 7.0 Hz, 1H), 7.01 (d, *J =* 2.6 Hz, 1H), 4.66 - 4.44 (m, 4H), 4.37 (d, *J =* 11.2 Hz, 1H), 3.83 - 3.52 (m, 7H), 3.11 - 2.99 (m, 1H), 2.90 - 2.75 (m, 3H), 2.59 - 2.19 (m, 12H), 1.91 - 1.73 (m, 5H), 1.55 - 1.47 (m, 2H), 1.33 - 1.09 (m, 6H), 0.90 (t, *J=* 7.4 Hz, 3H), 0.75 - 0.66 (d, *J=* 4.5 Hz, 2H), 0.54 - 0.43 (s, 2H).

### Example 20: 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-d]pyrimidin-4-yl)-3,8-diazabicyclo[3.2.1] octane-8-carboxylate

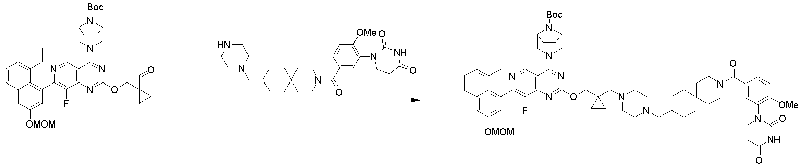

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (75 mg, 0.15 mmol), AcOH (0.1 mL), and tetraisopropyl titanate (68 mg, 0.24 mmol) were added to a solution of *tert-*butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate (80 mg, 0.12 mmol) in DCM (0.5 mL), and the mixture was stirred at room temperature for 2 h. The reaction liquid was cooled to 0 °C, and NaBH(OAc)₃ (76 mg, 0.36 mmol) was added. The reaction liquid was then reacted at room temperature for another 4 h. The reaction liquid was directly concentrated under reduced pressure, and the resulting crude product was purified by silica gel column chromatography (DCM:MeOH = 100:1 to 20:1) to give *tert-*butyl 3-(2-((1-((4-((3-(3-(2,4-dioxotetrahydropyrimidin-1(2*H*)-y1)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1153.3.

### Step 2: preparation of 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

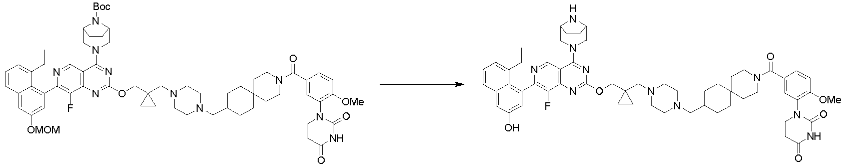

*tert-*Butyl 3-(2-((1-((4-((3-(3-(2,4-dioxotetrahydropyrimidin-1(2*H*)-yl)-4-methoxybenzoyl)-3-azaspiro[5.5] undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate (55 mg, 0.048 mmol) was dissolved in 1,4-dioxane (2 mL), and HCl/1,4-dioxane (6 N, 1 mL) was added. The mixture was reacted at room temperature for 0.5 h. The reaction liquid was directly concentrated under reduced pressure, and the resulting crude product was purified by Pre-HPLC to give 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1009.2.

¹H NMR (400 MHz, DMSO-*d*₆) δ 10.32 (s, 1H), 9.87 (s, 1H), 9.07 (s, 1H), 7.66 (d, *J =* 8.2 Hz, 1H), 7.40 - 7.33 (m, 2H), 7.31 (d, *J =* 2.0 Hz, 1H), 7.27 (d, *J* = 2.6 Hz, 1H), 7.17 - 7.09 (m, 2H), 6.96 (d, *J* = 2.6 Hz, 1H), 4.40 (d, *J* = 11.8 Hz, 2H), 4.35 - 4.21 (m, 2H), 3.84 (s, 3H), 3.68 - 3.38 (m, 9H), 2.68 (t, *J* = 6.5 Hz, 3H), 2.44 - 1.91 (m, 15H), 1.70 - 1.57 (m, 6H), 1.54 - 1.39 (m, 5H), 1.32 - 1.24 (m, 2H), 1.12 - 0.94 (m, 4H), 0.81 (t, *J =* 7.4 Hz, 3H), 0.67 - 0.58 (m, 2H), 0.42 - 0.33 (m, 2H).

### Example 21: 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methoxy)ethyl) piperidine-1-carbonyl)-2-chlorophenyl)dihydropyrimidine-2,4(1H,3H)-dione

### Step 1: preparation of tert-butyl 4-(2-((1-benzylpiperidin-4-yl)methoxy)ethyl)piperidine-1-carboxylate

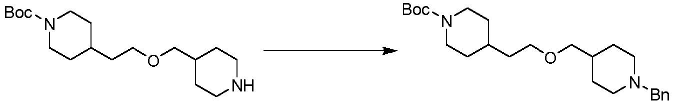

Acetic acid (0.01 mL) was added to a mixed solution of *tert-*butyl 4-(2-(piperidin-4-ylmethoxy)ethyl)piperidine-1-carboxylate (100 mg, 0.31 mmol) and benzaldehyde (39 mg, 0.37 mmol) in 1,2-dichloroethane/methanol (0.5 mL/0.5 mL). The mixture was stirred at 30 °C for 1 h. NaBH₃CN (39 mg, 0.62 mmol) was added at 0 °C, and the mixture was reacted at room temperature for 2 h. After the reaction was completed, H₂O (10 mL) was added, and the mixture was extracted with ethyl acetate (30 mL × 3). The combined organic layer was dried over anhydrous sodium sulfate, filtered, and concentrated in vacuum. The crude product was purified by silica gel column chromatography (DCM:MeOH = 20:1) to give *tert-*butyl 4-(2-((1-benzylpiperidin-4-yl)methoxy)ethyl)piperidine-1-carboxylate.

LC-MS: (ESI, m/z): [M+H]⁺ = 417.3.

### Step 2: preparation of 1-benzyl-4-((2-(piperidin-4-yl)ethoxy)methyl)piperidine

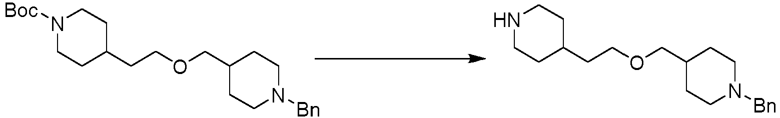

TFA (1 mL) was added to a solution of *tert-*butyl 4-(2-((1-benzylpiperidin-4-yl)methoxy)ethyl)piperidine-1-carboxylate (100 mg, 0.24 mmol) in DCM (2 mL). The mixture was stirred at room temperature for 1 h, and the reaction liquid was directly concentrated under reduced pressure to give 1-benzyl-4-((2-(piperidin-4-yl)ethoxy)methyl)piperidine, which was directly used in the next step.

### Step 3: preparation of 1-(5-(4-(2-((1-benzylpiperidin-4-yl)methoxy)ethyl)piperidine-1-carbonyl)-2-chlorophenyl)dihydropyrimidine-2,4(1H,3H)-dione

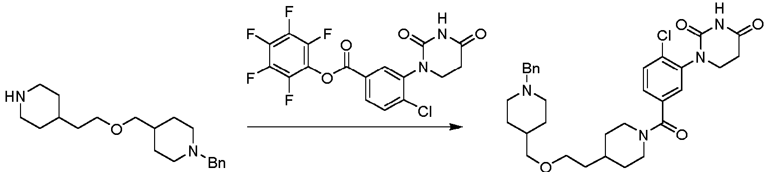

1-Benzyl-4-((2-(piperidin-4-yl)ethoxy)methyl)piperidine (100 mg, 0.32 mmol) was dissolved in DMSO (2 mL), and pentafluorophenyl 4-chloro-3-(2,4-dioxotetrahydropyrimidin-1(2*H*)-yl)benzoate (110 mg, 0.34 mmol) and DIEA (93 mg, 0.96 mmol) were added. The reaction liquid was stirred at room temperature for 2 h. Water (20 mL) was added, and the mixture was extracted with ethyl acetate (20 mL × 2). The organic phases were combined, washed with saturated brine (30 mL × 3), dried over anhydrous sodium sulfate, and filtered. The filtrate was concentrated and purified by silica gel column chromatography (DCM:MeOH = 100:1 to 5:1) to give 1-(5-(4-(2-((1-benzylpiperidin-4-yl)methoxy)ethyl)piperidine-1-carbonyl)-2-chlorophenyl)
dihydropyrimidine-2,4(1*H*,3*H*)-dione.

LC-MS: (ESI, m/z): [M+H]⁺ = 567.2.

### Step 4: preparation of 1-(2-chloro-5-(4-(2-(piperidin-4-ylmethoxy)ethyl)piperidine-1-carbonyl)phenyl)dihydropyrimidine-2,4(1H,3H)-dione

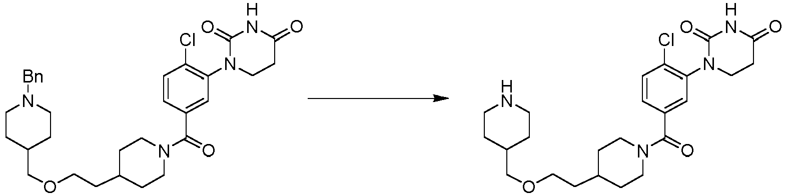

1-(5-(4-(2-((1-Benzylpiperidin-4-yl)methoxy)ethyl)piperidine-1-carbonyl)-2-chlorophenyl)dihydropyrimidine-2,4(1*H*,3*H*)-dione (200 mg, 0.35 mmol) was dissolved in DCM (2 mL), and 1-chloroethyl chloroformate (150 mg, 1.05 mmol) and DIEA (90 mg, 0.7 mmol) were added. The mixture was reacted at room temperature for 30 min and concentrated under reduced pressure. MeOH (2 mL) was added to the concentrate for dissolution, and the mixture was heated to 50 °C and reacted for 30 min. After the reaction was completed, the mixture was concentrated and purified by silica gel column chromatography (DCM:MeOH (NH₃) = 100:1 to 9:1) to give 1-(2-chloro-5-(4-(2-(piperidin-4-ylmethoxy)ethyl)piperidine-1-carbonyl)phenyl)dihydropyrimidine-2,4(1*H*,3*H*)-dione.

LC-MS: (ESI, m/z): [M+H]⁺ = 477.3.

### Step 5: preparation of tert-butyl 3-(2-((1-((4-((2-(1-(4-chloro-3-(2,4-dioxotetrahydropyrimidin-1(2H)-yl)benzoyl)piperidin-4-yl)ethoxy)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

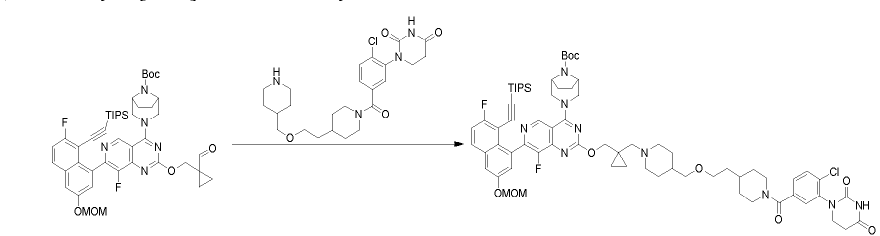

Tetraisopropyl titanate (270 mg, 0.95 mmol) and 1-(2-chloro-5-(4-(2-(piperidin-4-ylmethoxy)ethyl)piperidine-1-carbonyl)phenyl)dihydropyrimidine-2,4(1H,3H)-dione (54 mg, 0.11 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (80 mg, 0.095 mmol) in THF/MeOH (1 mL/1 mL), and the mixture was stirred at 70 °C for 2 h. NaBH(OAc)₃ (61 mg, 0.28 mmol) was then added to the mixture at room temperature, and the resulting mixture was stirred for 2 h. The mixture was concentrated and purified by silica gel column chromatography (DCM:MeOH = 100:1 to 20:1) to give *tert*-butyl 3-(2-((1-((4-((2-(1-(4-chloro-3-(2,4-dioxotetrahydropyrimidin-1(2*H*)-yl)benzoyl)piperidin-4-yl)ethoxy)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 651.7.

### Step 6: preparation of 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methoxy)ethyl)piperidine-1-carbonyl)-2-chlorophenyl)dihydropyrimidine-2,4(1H,3H)-dione

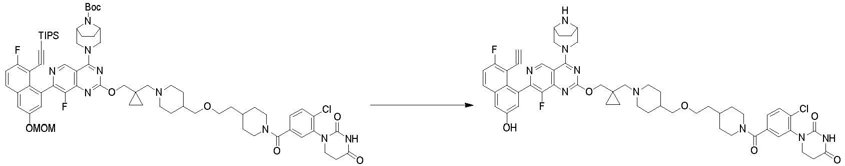

*tert-*Butyl *3-(*2-((1-((4-((2-(1-(4-chloro-3-(2,4-dioxotetrahydropyrimidin-1(2*H*)-yl)benzoyl)piperidin-4-yl)ethoxy)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (70 mg, 0.054 mmol) was dissolved in DMF (2 mL), and cesium fluoride (40 mg, 0.27 mmol) was added while stirring. The mixture was stirred at room temperature for 30 min and extracted with EA and water. The organic phase was concentrated. A mixture of 1,4-dioxane (2 mL) and HCl/1,4-dioxane (6 N, 1 mL) was then added to the residue at room temperature. The reaction liquid was reacted for another 0.5 h and directly concentrated under reduced pressure, and the crude product was purified by Pre-HPLC to give 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido
[4,3-*d*]pyrimidin-2-yl)oxy)methyl)cyclopropyl)methyl)piperidin-4-yl)methoxy)ethyl)piperidine-1-carbonyl)-2-chlorophenyl)dihydropyrimidine-2,4(1*H*,3*H*)-dione.

LC-MS: (ESI, m/z): [M/2+H]⁺ = 501.6.

¹H NMR (400 MHz, DMSO-*d*₆) δ 10.51 (s, 1H), 10.14 (s, 1H), 9.02 (s, 1H), 7.97 (dd, *J* = 9.1, 6.0 Hz, 1H), 7.62 (d, *J =* 8.2 Hz, 1H), 7.53 (d, *J =* 1.8 Hz, 1H), 7.46 (t, *J =* 9.0 Hz, 1H), 7.41 - 7.34 (m, 2H), 7.16 (d, *J =* 2.3 Hz, 1H), 4.54 - 4.33 (m, 2H), 4.31 - 4.22 (m, 3H), 3.92 (s, 1H), 3.78 - 3.68 (m, 1H), 3.67 - 3.58 (m, 2H), 3.57 - 3.47 (m, 4H), 3.39 - 3.32 (m, 2H), 3.14 (d, *J =* 6.4 Hz, 2H), 2.95 - 2.88 (m, 2H), 2.77 - 2.66 (m, 3H), 2.34 - 2.22 (m, 2H), 1.80 (t, J = 11.4 Hz, 2H), 1.70 - 1.51 (m, 9H), 1.47 - 1.39 (m, 3H), 1.28 - 1.19 (m, 2H), 1.16 - 1.01 (m, 4H), 0.76 - 0.66 (s, 2H), 0.55 - 0.45 (m, 2H).

### Example 22: 1-(5-(4-(3-(4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione

### Step 1: preparation of tert-butyl 3-(7-(8-ethynyl-7-fluoro-3-(methoxymethoxy)naphthalen-1-yl)-8-fluoro-2-((1-formylcyclopropyl)methoxy)pyrido[4,3-d]pyrimidin-4-yl)-3,8-diazabicyclo[3.2.1]octane-8-carboxylate

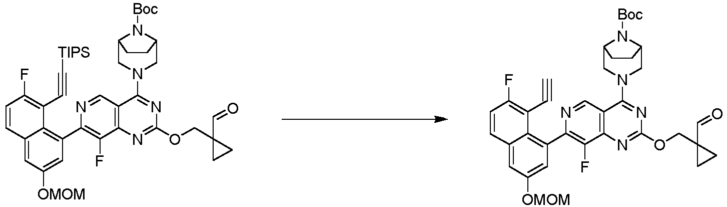

CsF (181 mg, 1.19 mmol) was added to a solution of *tert-*butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-d]pyrimidin-4-yl)-3,8-diazabicyclo[3.2.1]octane-8-carboxylate (200 mg, 0.24 mmol) in DMF (3 mL) at room temperature, and the mixture was reacted at room temperature for 30 min. After the reaction was completed, water was added, and the mixture was extracted with ethyl acetate. The organic phases were combined, washed with saturated brine, dried, and concentrated under reduced pressure. The crude product was purified by silica gel column chromatography (PE:EA = 10:1 to 5:2) to give *tert-*butyl 3-(7-(8-ethynyl-7-fluoro-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 686.3.

### Step 2: preparation of tert-butyl 3-(7-(8-ethyl-7-fluoro-3-(methoxymethoxy)naphthalen-1-yl)-8-fluoro-2-((1-formylcyclopropyl)methoxy)pyrido[4,3-d]pyrimidin-4-yl)-3,8-diazabicyclo[3.2.1]octane-8-carboxylate

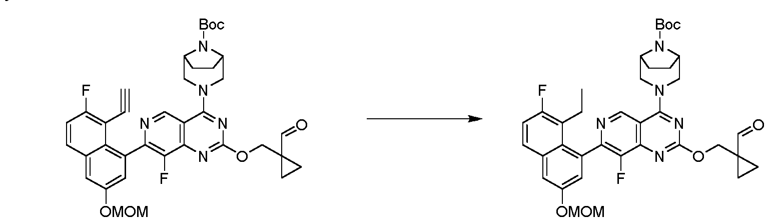

Pd/C (150 mg) was added to a solution of *tert-*butyl 3-(7-(8-ethynyl-7-fluoro-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]
octane-8-carboxylate (150 mg, 0.217 mmol) in EtOAc (2 mL). The mixture was stirred at 30 °C for 16 h and filtered, and the filtrate was concentrated under reduced pressure to give *tert*-butyl 3-(7-(8-ethyl-7-fluoro-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate. The crude product was directly used in the next step without purification.

LC-MS: (ESI, m/z): [M+H]⁺ = 690.3.

### Step 3: preparation of tert-butyl 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

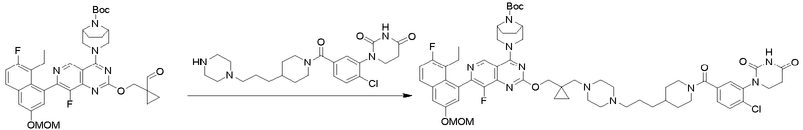

1-(2-Chloro-5-(4-(3-(piperazin-1-yl)propyl)piperidine-1-carbonyl)phenyl)dihydropyrimidine-2,4(1*H*,3*H*)-dione (80 mg, 0.174 mmol) and tetraisopropyl titanate (412 mg, 1.45 mmol) were added to a solution of *tert-*butyl 3-(7-(8-ethyl-7-fluoro-3-(methoxymethoxy)naphthalen-1-yl)-8-fluoro-2-((1-formylcyclopropyl) methoxy)pyrido[4,3-*d*]pyrimidin-4-yl)-3,8-diazabicyclo[3.2.1]octane-8-carboxylate (100 mg, 0.145 mmol) in THF/MeOH (1 mL/1 mL), and then the mixture was stirred at 80 °C for 2 h. The above mixture was cooled to room temperature, NaBH(OAc)₃ (97 mg, 0.435 mmol) was then added, and the mixture was stirred for 2 h. The mixture was concentrated and purified by silica gel column chromatography (DCM:MeOH = 100:1 to 9:1) to give *tert-*butyl 3-(2-((1-((4-(3-(1-(4-chloro-3-(2,4-dioxotetrahydropyrimidin-1(2*H*)-yl)benzoyl) piperidin-4-yl)propyl)piperazin-1-yl)methyl)cyclopropyl)methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1135.5.

### Step 4: preparation of 1-(5-(4-(3-(4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)propyl)piperidine-1-carbonyl)-2-chlorophenyl)dihydropyrimidine-2,4(1H,3H)-dione

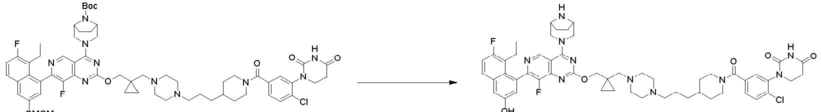

HCl/dioxane (6 N, 1.0 mL) was added to a solution of *tert-*butyl 3-(2-((1-((4-(3-(1-(4-chloro-3-(2,4-dioxotetrahydropyrimidin-1(2*H*)-yl)benzoyl)piperidin-4-yl)propyl)piperazin-1-yl)methyl)cyclopropyl) methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (100 mg, 0.088 mmol) in dioxane (2.0 mL), and the mixture was reacted at room temperature for 0.5 h. An aqueous Na₂CO₃ solution was added to the reaction liquid to adjust the pH > 7, and the mixture was extracted with EtOAc (10 mL × 2). The organic phase was washed with brine, dried over anhydrous Na₂SO₄, and concentrated under reduced pressure. The crude product was purified by Pre-HPLC to give 1-(5-(4-(3-(4-((1-(((4-(3,8-diazabicyclo[3.2.1]octan-3-yl)-7-(8-ethyl-7-fluoro-3-hydroxynaphthalen-1-yl)-8-fluoropyrido[4,3-*d*]pyrimidin-2-yl)oxy)methyl)cyclopropyl)methyl)piperazin-1-yl)propyl)piperidine-1-carbonyl)-2-chlorophenyl)dihydropyrimidine-2,4(1*H*,3*H*)-dione.

LC-MS: (ESI, m/z): [M/2+H]⁺ = 496.2.

¹H NMR (400 MHz, DMSO-*d*₆) δ 10.50 (s, 1H), 9.91 (s, 1H), 9.08 (s, 1H), 7.76 (dd, *J =* 9.1, 6.0 Hz, 1H), 7.62 (d, *J =* 8.2 Hz, 1H), 7.53 (d*, J* = 1.9 Hz, 1H), 7.40 - 7.28 (m, 3H), 7.01 (d, *J =* 2.4 Hz, 1H), 4.49 - 4.22 (m, 5H), 3.80 - 3.70 (s, 1H), 3.66 - 3.47 (m, 6H), 2.80 - 2.62 (m, 4H), 2.43 - 2.07 (m, 15H), 1.67 - 1.55 (m, 5H), 1.48 - 1.33 (m, 3H), 1.28 - 1.14 (m, 3H), 1.11 - 0.98 (m, 2H), 0.72 (t, *J =* 7.4 Hz, 3H), 0.66 - 0.58 (m, 2H), 0.45 - 0.33 (m, 2H).

### Example 23: 1-(5-(4-(3-(4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2,2-dimethylpropyl)piperazin-1-yl)propyl)piperidine-1-carbonyl)-2-chlorophenyl)dihydropyrimidine-2,4(1H,3H)-dione

### Step 1: preparation of 3-((tert-butyldiphenylsilyl)oxy)-2,2-dimethylpropan-1-ol

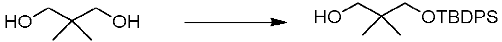

Imidazole (9.80 g, 144.0 mmol) was added to a solution of 2,2-dimethylpropyl-1,3-diol (5.00 g, 48.0 mmol) in dichloromethane (50 mL), and the mixture was cooled to 0 °C. tert-Butyldiphenylchlorosilane (7.92 g, 28.8 mmol) was slowly added dropwise, and the mixture was reacted at 0 °C for 1 h. After the reaction was completed, water (100 mL) was added to quench the reaction, and the mixture was extracted with dichloromethane (150 mL × 3). The organic phases were combined, dried over anhydrous sodium sulfate, and concentrated under reduced pressure. The concentrate was purified by silica gel column chromatography (PE:EA = 6:94) to give 3-((*tert*-butyldiphenylsilyl)oxy)-2,2-dimethylpropan-1-ol.

¹H NMR (400 MHz, CD₃OD) δ 7.72 - 7.61 (m, 4H), 7.47 - 7.32 (m, 6H), 3.48 - 3.34 (m, 4H), 1.09-0.98 (m, 9H), 0.94-0.79 (m, 6H).

### Step 2: preparation of tert-butyl 3-(2-(3-((tert-butyldiphenylsilyl)oxy)-2,2-dimethylpropoxy)-7-chloro-8-fluoropyrido[4,3-d]pyrimidin-4-yl)-3,8-diazabicyclo[3.2.1]octane-8-carboxylate

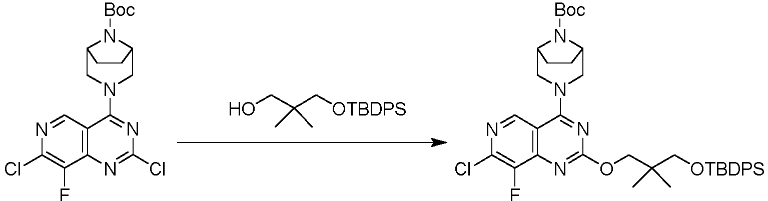

*tert*-Butyl 3-(2,7-dichloro-8-fluoropyrido[4,3-*d*]pyrimidin-4-yl)-3,8-diazabicyclo[3.2.1]octane-8-carboxylate (2.0 g, 4.7 mmol) and 3-((*tert*-butyldiphenylsilyl)oxy)-2,2-dimethylpropanol (3.22 g, 9.4 mmol) were added to THF (20 mL), and then cesium carbonate (4.60 g, 10.5 mmol) was added. The mixture was stirred at 90 °C for 16 h. After the reaction was completed, the mixture was cooled to room temperature. A saturated ammonium chloride solution (50 mL) was added to quench the reaction, and the mixture was extracted with ethyl acetate (100 mL × 3). The organic phases were combined, dried over anhydrous sodium sulfate, and concentrated under reduced pressure. The crude product was purified by silica gel column chromatography (PE:EA = 3:1) to give *tert-*butyl 3-(2-(3-((*tert*-butyldiphenylsilyl)oxy)-2,2-dimethylpropoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 734.3.

### Step 3: preparation of tert-butyl 3-(7-chloro-8-fluoro-2-(3-hydroxy-2,2-dimethylpropoxy)pyrido[4,3-d]pyrimidin-4-yl)-3,8-diazabicyclo[3.2.1]octane-8-carboxylate

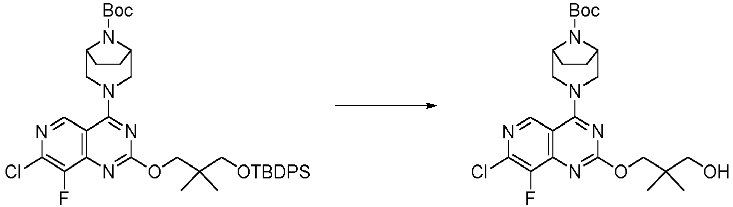

*tert-*Butyl 3-(2-(3-((*tert*-butyldiphenylsilyl)oxy)-2,2-dimethylpropoxy)-7-chloro-8-fluoropyrido[4,3-*d*]pyrimidin-4-yl)-3,8-diazabicyclo[3.2.1]octane-8-carboxylate (1.1 g, 1.5 mmol) was dissolved in DMF (15 mL), and cesium fluoride (684 mg, 4.5 mmol) was added. The mixture was heated to 50 °C and reacted for 6 h. After the reaction was completed, the mixture was cooled to room temperature. Water (50 mL) was added to quench the reaction, and the mixture was extracted with ethyl acetate (100 mL × 3). The organic phases were combined, dried over anhydrous sodium sulfate, and concentrated under reduced pressure. The crude product was purified by silica gel column chromatography (PE:EA = 2:1) to give *tert-*butyl *3-(7-*chloro-8-fluoro-2-(3-hydroxy-2,2-dimethylpropoxy)pyrido[4,3-*d*]pyrimidin-4-yl)-3,8-diazabicyclo[3.2.1]octane-8-carboxylate.

LC-MS: (ESI, m/z): [M+H]⁺ = 496.2.

### Step 4: preparation of tert-butyl 3-(8-fluoro-7-(7-fluoro-3-(methoxymethoxy)-8-((triisopropylsilyl)ethynyl)naphthalen-1-yl)-2-(3-hydroxy-2,2-dimethylpropoxy)pyrido[4,3-d]pyrimidin-4-yl)-3,8-diazabicyclo[3.2.1]octane-8-carboxylate

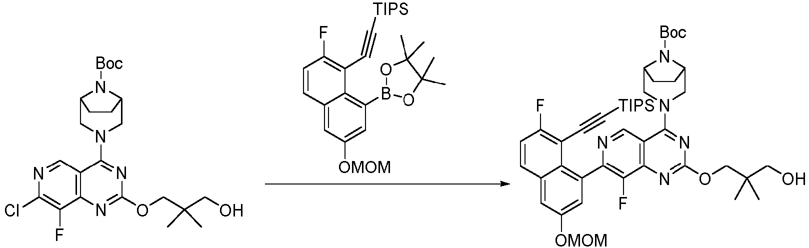

cataCXium A Pd G3 (117 mg, 0.161 mmol) and cesium carbonate (788 mg, 2.418 mmol) were added to a mixed solution of *tert*-butyl 3-(7-chloro-8-fluoro-2-(3-hydroxy-2,2-dimethylpropoxy)pyrido[4,3-*d*]pyrimidin-4-yl)-3,8-diazabicyclo[3.2.1]octane-8-carboxylate (400 mg, 0.806 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (496 mg, 0.967 mmol) in 1,4-dioxane/H₂O (5 mL/1 mL). The mixture was reacted at 80 °C for 16 h under nitrogen atmosphere. Water (10 mL) was added to quench the reaction, and the mixture was extracted with ethyl acetate (20 mL × 3). The organic phases were combined, dried over anhydrous sodium sulfate, and concentrated under reduced pressure. The crude product was purified by silica gel column chromatography (PE:EA = 2:1) to give *tert-*butyl 3-(8-fluoro-7-(7-fluoro-3-(methoxymethoxy)-8-((triisopropylsilyl)ethynyl)naphthalen-1-yl)-2-(3-hydroxy-2,2-dimethylpropoxy)pyrido[4,3-*d*]pyrimidin-4-yl)-3,8-diazabicyclo[3.2.1]octane-8-carboxylate.

LC-MS: (ESI, m/z): [M+H]⁺ = 846.4.

### Step 5: preparation of tert-butyl 3-(2-(2,2-dimethyl-3-oxopropyl)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

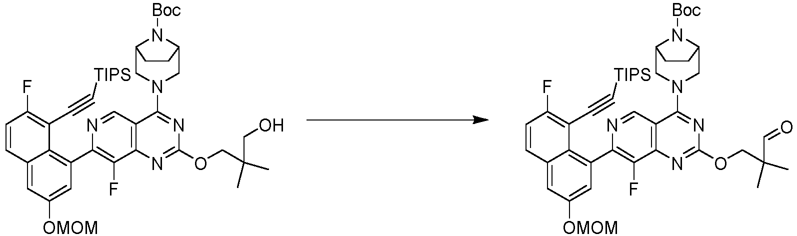

A solution of *tert-*butyl 3-(8-fluoro-7-(7-fluoro-3-(methoxymethoxy)-8-((triisopropylsilyl)ethynyl) naphthalen-1-yl)-2-(3-hydroxy-2,2-dimethylpropoxy)pyrido[4,3-*d*]pyrimidin-4-yl)-3,8-diazabicyclo[3.2.1] octane-8-carboxylate (400 mg, 0.473 mmol) in DCM (10 mL) was cooled to 0 °C, and Dess-Martin oxidant (401 mg, 0.946 mmol) was added. The mixture was reacted at room temperature for 0.5 h. After the reaction was completed, water (20 mL) was added to quench the reaction, and the mixture was extracted with dichloromethane (30 mL × 3). The organic phases were combined, dried over anhydrous sodium sulfate, and concentrated under reduced pressure. The crude product was purified by silica gel column chromatography (PE:EA = 2:1) to give *tert-*butyl 3-(2-(2,2-dimethyl-3-oxopropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 844.4.

¹H NMR (400 MHz, DMSO-*d*₆) δ 9.62 (s, 1H), 9.19 (s, 1H), 8.14 - 8.06 (m, 1H), 7.75 (d,*J* = 2.8 Hz, 1H), 7.60 - 7.53 (m, 1H), 7.34 (d, *J* = 2.8 Hz, 1H), 5.37 (s, 2H), 4.75 (d, *J =* 12.0 Hz, 1H), 4.49 - 4.18 (m, 5H), 3.76 (d, *J =* 12 Hz, 1H), 3.43 (s, 3H), 3.38-3.32 (m, 1H), 1.93-1.78 (m, 3H), 1.69-1.58 (m, 1H), 1.46 (s, 9H), 1.12 (d, *J =* 4.8 Hz, 6H), 0.81 (t, *J =* 7.6 Hz, 18H), 0.54 - 0.43 (m, 3H).

### Step 6: preparation of tert-butyl 3-(2-(3-(4-(3-(1-(4-chloro-3-(2,4-dioxotetrahydropyrimidin-1(2H)-yl)benzoyl)piperidin-4-yl)propyl)piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

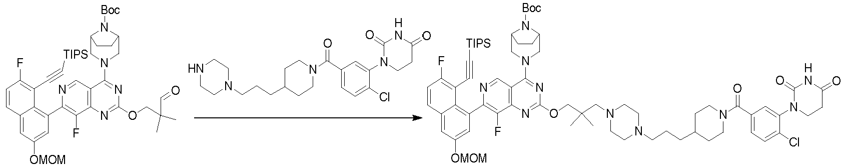

Tetraisopropyl titanate (335 mg, 1.18 mmol) was added to a stirred solution of *tert*-butyl 3-(2-(2,2-dimethyl-3-oxopropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (100 mg, 0.118 mmol) and 1-(2-chloro-5-(4-(3-(piperazin-1-yl)propyl)piperidine-1-carbonyl)phenyl)dihydropyrimidine-2,4(1*H*,3*H*)-dione (66 mg, 0.142 mmol) in DCM (2 mL), and the mixture was stirred at 30 °C for 2 h. NaBH(OAc)₃ (79 mg, 0.354 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-(3-(4-(3-(1-(4-chloro-3-(2,4-dioxotetrahydropyrimidin-1(2*H*)-yl)benzoyl)piperidin-4-yl)propyl) piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 645.4.

### Step 7: preparation of 1-(5-(4-(3-(4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2,2-dimethylpropyl)piperazin-1-yl)propyl)piperidine-1-carbonyl)-2-chlorophenyl)dihydropyrimidine-2,4(1H,3H)-dione

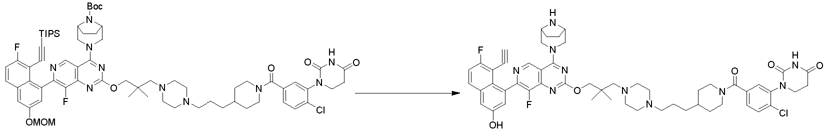

CsF (36 mg, 0.235 mmol) was added to a solution of *tert*-butyl 3-(2-(3-(4-(3-(1-(4-chloro-3-(2,4-dioxotetrahydropyrimidin-1(2*H*)-yl)benzoyl)piperidin-4-yl)propyl)piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (60 mg, 0.047 mmol) in DMF (2.0 mL). The mixture was reacted at room temperature for 1 h, diluted with water (10 mL), and extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (1.0 mL), and then HCl/1,4-dioxane (6 N, 0.5 mL) was added. The mixture was reacted at room temperature for 0.5 h. An aqueous Na₂CO₃ solution was added to the reaction liquid to adjust the pH > 7, and then the mixture was extracted with EtOAc (10 mL × 2). The organic phase was washed with brine dried over anhydrous Na₂SO₄, and concentrated The crude product was purified by Pre-HPLC to give 1-(5-(4-(3-(4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2,2-dimethylpropyl)piperazin-1-yl)propyl)piperidine-1-carbonyl)-2-chlorophenyl)dihydropyrimidine-2,4(1*H*,3*H*)-dione.

LC-MS: (ESI, m/z): [M+H]⁺ = 989.1.
¹H NMR (400 MHz, DMSO-*d*₆) δ 10.50 (s, 1H), 10.14 (s, 1H), 9.02 (s, 1H), 7.97(dd, *J* = 9.2 Hz, 6.0 Hz, 1H), 7.63 (d, *J =* 8.4 Hz, 1H), 7.53 (d, *J =* 2.0 Hz, 1H), 7.49 - 7.37 (m, 1H), 7.41 - 7.33 (m, 2H), 7.17 (d, *J* = 2.4 Hz, 1H), 4.45 (d, *J =* 11.6 Hz, 1H), 4.33 (d, *J =* 12.0 Hz, 1H), 4.19 - 4.05 (m, 2H), 3.90 (s, 1H), 3.79 - 3.71 (m,1H), 3.66 - 3.49 (m, 6H), 3.06 - 2.96(m, 1H), 2.82 - 2.64 (m, 4H), 2.49 - 2.42 (m, 4H), 2.37 - 2.13 (m, 9H), 1.70 - 1.60 (m, 4H), 1.54 - 1.32 (m, 4H), 1.28 - 1.14 (m, 3H), 1.10 - 1.00 (m, 2H), 0.94 (s, 6H).

### Example 24: 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclobutyl)methyl)piperazin-1-yl)propyl)piperidine-1-carbonyl)-2-chlorophenyl)dihydropyrimidine-2,4(1H,3H)-dione

### Step 1: preparation of (1-(((tert-butyldiphenylsilyl)oxy)methyl)cyclobutyl)methanol

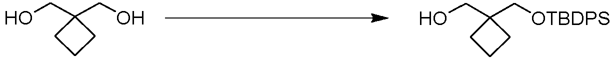

Imidazole (8.78 g, 129 mmol) was added to a solution of cyclobutane-1,1-diyldimethanol (5.0 g, 43 mmol) in dichloromethane (50 mL). The above mixture was cooled to 0 °C, and *tert-*butyldiphenylchlorosilane (7.1 g, 25.8 mmol) was slowly added dropwise. The mixture was reacted at 0 °C for 1 h. After the reaction was completed, water (100 mL) was added to quench the reaction, and the mixture was extracted with dichloromethane (150 mL × 3). The organic phases were combined, dried over anhydrous sodium sulfate, and concentrated under reduced pressure. The crude product was purified by silica gel column chromatography (PE:EA = 6:94) to give (1-(((*tert-*butyldiphenylsilyl)oxy)methyl)cyclobutyl)methanol.

LC-MS: (ESI, m/z): [M+Na]⁺ = 377.2.

¹H NMR (400 MHz, DMSO-*d*₆) δ 7.71 - 7.61 (m, 4H), 7.47 - 7.35 (m, 6H), 4.53 (t, *J =* 5.2 Hz, 1H), 3.59 (s, 2H), 4.53 (d, *J =* 5.2 Hz, 2H), 1.82 - 1.69(m, 6H),1.02(s, 9H).

### Step 2: preparation of tert-butyl 3-(2-((1-(((tert-butyldiphenylsilyl)oxy)methyl)cyclobutyl)methoxy)-7-chloro-8-fluoropyrido[4,3-d]pyrimidin-4-yl)-3,8-diazabicyclo[3.2.1]octane-8-carboxylate

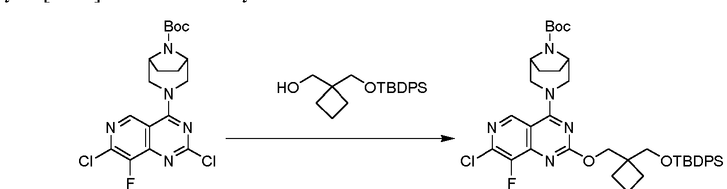

*tert*-Butyl 3-(2,7-dichloro-8-fluoropyrido[4,3-*d*]pyrimidin-4-yl)-3,8-diazabicyclo[3.2.1]octane-8-carboxylate (1.5 g, 3.5 mmol) and (1-(((*tert*-butyldiphenylsilyl)oxy)methyl)cyclobutyl)methanol (2.36 g, 6.65 mmol) were added to THF (20 mL), and then cesium carbonate (3.42 g, 10.5 mmol) was added. The mixture was stirred at 90 °C for 16 h. After the reaction was completed, the mixture was cooled to room temperature. A saturated ammonium chloride solution (50 mL) was added to quench the reaction, and the mixture was extracted with ethyl acetate (100 mL × 3). The organic phases were combined, dried over anhydrous sodium sulfate, and concentrated under reduced pressure. The crude product was purified by silica gel column chromatography (PE:EA = 4:1) to give *tert-*butyl 3-(2-((1-(((*tert-*butyldiphenylsilyl)oxy)methyl)cyclobutyl)methoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 746.3.

### Step 3: preparation of tert-butyl 3-(7-chloro-8-fluoro-2-((1-(hydroxymethyl)cyclobutyl)methoxy)pyrido[4,3-d]pyrimidin-4-yl)-3,8-diazabicyclo[3.2.1]octane-8-carboxylate

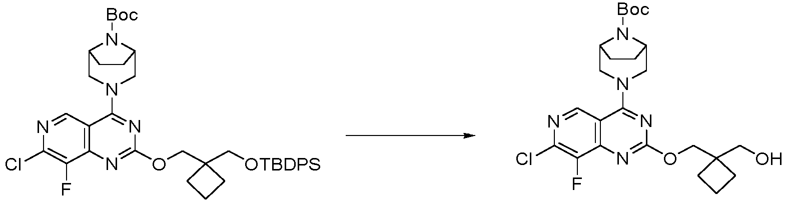

*tert-*Butyl 3-(2-((1-(((*tert*-butyldiphenylsilyl)oxy)methyl)cyclobutyl)methoxy)-7-chloro-8-fluoropyrido[4,3-*d*]pyrimidin-4-yl)-3,8-diazabicyclo[3.2.1]octane-8-carboxylate (1.2 g, 1.6 mmol) was dissolved in DMF (15 mL), and cesium fluoride (729 mg, 4.8 mmol) was added. The mixture was heated to 50 °C and reacted for 6 h. After the reaction was completed, the mixture was cooled to room temperature. Water (50 mL) was added to quench the reaction, and the mixture was extracted with ethyl acetate (100 mL × 3). The organic phases were combined, dried over anhydrous sodium sulfate, and concentrated under reduced pressure. The crude product was purified by silica gel column chromatography (PE:EtOAc = 2:1) to give *tert-*butyl 3-(7-chloro-8-fluoro-2-((1-(hydroxymethyl)cyclobutyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 508.2.

### Step 4: preparation of tert-butyl 3-(8-fluoro-7-(7-fluoro-3-(methoxymethoxy)-8-((triisopropylsilyl) ethynyl)naphthalen-1-yl)-2-((1-(hydroxymethyl)cyclobutyl)methoxy)pyrido[4,3-d]pyrimidin-4-yl)-3,8-diazabicyclo[3.2.1]octane-8-carboxylate

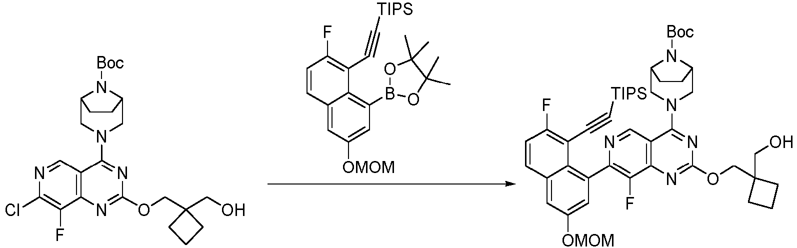

cataCXium A Pd G3 (143 mg, 0.20 mmol) and cesium carbonate (962 mg, 2.95 mmol) were added to a mixed solution of *tert-*butyl 3-(7-chloro-8-fluoro-2-((1-(hydroxymethyl)cyclobutyl)methoxy)pyrido[4,3-*d*]pyrimidin-4-yl)-3,8-diazabicyclo[3.2.1]octane-8-carboxylate (500 mg, 0.98 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (605 mg, 1.18 mmol) in 1,4-dioxane/H₂O (5 mL/1 mL). The mixture was reacted at 80 °C for 16 h under nitrogen atmosphere. Water (10 mL) was added to quench the reaction, and the mixture was extracted with ethyl acetate (20 mL × 3). The organic phases were combined, dried over anhydrous sodium sulfate, and concentrated under reduced pressure. The crude product was purified by silica gel column chromatography (PE:EA = 2:1) to give *tert-*butyl 3-(8-fluoro-7-(7-fluoro-3-(methoxymethoxy)-8-((triisopropylsilyl)ethynyl) naphthalen-1-yl)-2-((1-(hydroxymethyl)cyclobutyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 858.4.

### Step 5: preparation of tert-butyl 3-(8-fluoro-7-(7-fluoro-3-(methoxymethoxy)-8-((triisopropylsilyl)ethynyl) naphthalen-1-yl)-2-((1-formylcyclobutyl)methoxy)pyrido[4,3-d]pyrimidin-4-yl)-3,8-diazabicyclo[3.2.1] octane-8-carboxylate

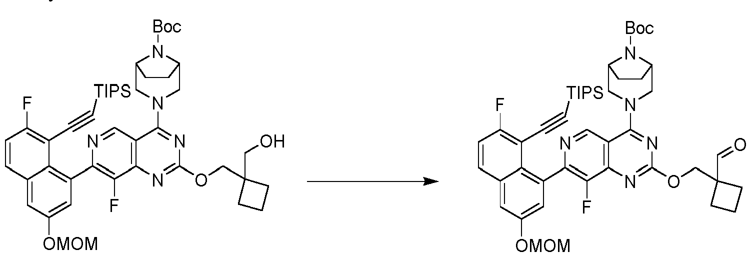

A solution of *tert-*butyl 3-(8-fluoro-7-(7-fluoro-3-(methoxymethoxy)-8-((triisopropylsilyl)ethynyl) naphthalen-1-yl)-2-((1-(hydroxymethyl)cyclobutyl)methoxy)pyrido[4,3-*d*]pyrimidin-4-yl)-3,8-diazabicyclo [3.2.1]octane-8-carboxylate (500 mg, 0.58 mmol) in DCM (10 mL) was cooled to 0 °C, and Dess-Martin oxidant (495 mg, 1.17 mmol) was added. The mixture was reacted at room temperature for 0.5 h. After the reaction was completed, water (20 mL) was added to quench the reaction, and the mixture was extracted with dichloromethane (30 mL × 3). The organic phases were combined, dried over anhydrous sodium sulfate, and concentrated under reduced pressure. The crude product was purified by silica gel column chromatography (PE:EA = 2:1) to give *tert-*butyl 3-(8-fluoro-7-(7-fluoro-3-(methoxymethoxy)-8-((triisopropylsilyl)ethynyl) naphthalen-1-yl)-2-((1-formylcyclobutyl)methoxy)pyrido[4,3-d]pyrimidin-4-yl)-3,8-diazabicyclo[3.2.1] octane-8-carboxylate.

LC-MS: (ESI, m/z): [M+H]⁺ = 856.4.

¹H NMR (400 MHz, DMSO-*d*₆) δ 9.73 (s, 1H), 9.19 (s, 1H), 8.14 - 8.06 (m, 1H), 7.75 (d, *J =* 2.4 Hz, 1H), 7.60 - 7.53 (m, 1H), 7.34 (d, *J* = 2.8 Hz, 1H), 5.37 (s, 2H), 4.79 - 4.63 (m, 3H), 4.37 - 4.18 (m, 3H), 3.75 (d, *J =* 12.0 Hz,1H), 3.44 (s, 3H), 3.36 (d, *J =* 12.4 Hz,1H), 2.40 - 2.25 (m, 2H), 2.10 - 1.98 (m, 3H), 1.99 - 1.78 (m, 4H), 1.65 - 1.58 (m, 1H), 1.46 (s, 9H), 0.81(t, *J* = 7.6 Hz, 18H), 0.54 - 0.43(m, 3H).

### Step 6: preparation of tert-butyl 3-(2-((1-((4-(3-(1-(4-chloro-3-(2,4-dioxotetrahydropyrimidin-1(2H)-yl)benzoyl)piperidin-4-yl)propyl)piperazin-1-yl)methyl)cyclobutyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

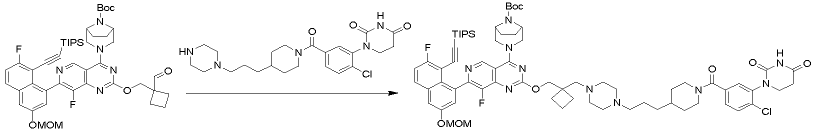

Tetraisopropyl titanate (333 mg, 1.17 mmol) was added to a solution of *tert-*butyl 3-(8-fluoro-7-(7-fluoro-3-(methoxymethoxy)-8-((triisopropylsilyl)ethynyl)naphthalen-1-yl)-2-((1-formylcyclobutyl)methoxy)pyrido[4,3-*d*]pyrimidin-4-yl)-3,8-diazabicyclo[3.2.1]octane-8-carboxylate (100 mg, 0.117 mmol) and 1-(2-chloro-5-(4-(3-(piperazin-1-yl)propyl)piperidine-1-carbonyl)phenyl)dihydropyrimidine-2,4(1*H*,3*H*)-dione (65 mg, 0.140 mmol) in DCM (2 mL), and the mixture was stirred at 30 °C for 2 h. NaBH(OAc)₃ (78 mg, 0.351 mmol) was added to the mixture at room temperature, and the resulting mixture was stirred for 2 h. The mixture was concentrated and purified by silica gel column chromatography (MeOH:DCM = (0-1):4) to give *tert-butyl* 3-(2-((1-((4-(3-(1-(4-chloro-3-(2,4-dioxotetrahydropyrimidin-1(2*H*)-yl)benzoyl)piperidin-4-yl)propyl)piperazin-1-yl)methyl)cyclobutyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 651.5.

### Step 7: preparation of 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclobutyl)methyl)piperazin-1-yl)propyl)piperidine-1-carbonyl)-2-chlorophenyl)dihydropyrimidine-2,4(1H,3H)-dione

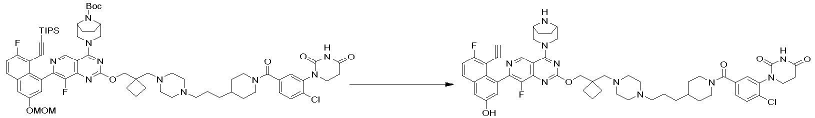

CsF (58 mg, 0.385 mmol) was added to a solution of *tert*-butyl 3-(2-((1-((4-(3-(1-(4-chloro-3-(2,4-dioxotetrahydropyrimidin-1(2*H*)-yl)benzoyl)piperidin-4-yl)propyl)piperazin-1-yl)methyl)cyclobutyl)methoxy)-8-fluoro-7-(7-fluoro-3-(methoxymethoxy)-8-((triissopropylsilyl)ethynyl)naphthalen-1-yl)pyrido[4,3-*d*]pyrimidin-4-yl)-3,8-diazabicyclo[3.2.1]octane-8-carboxylate (100 mg, 0.077 mmol) in DMF (2.0 mL). The mixture was stirred at room temperature for 1 h, diluted with water (10 mL), and extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (2.0 mL), and HCl/1,4-dioxane (6 N, 1.0 mL) was added. The mixture was reacted at room temperature for 0.5 h. An aqueous Na₂CO₃ solution was added to the reaction liquid to adjust the pH > 7, and then the mixture was extracted with EtOAc (10 mL × 2). The organic phase was washed with brine, dried over anhydrous Na₂SO₄, and concentrated. The crude product was purified by Pre-HPLC to give 1-(5-(4-(3-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclobutyl)methyl)piperazin-1-yl)propyl)piperidine-1-carbonyl)-2-chlorophenyl)dihydropyrimidine-2,4(1*H*,3*H*)-dione.

LC-MS: (ESI, m/z): [M+H]⁺ = 1001.4.
¹H NMR (400 MHz, DMSO-*d*₆) δ 10.50 (s, 1H), 10.14 (s, 1H), 9.03 (s, 1H), 7.97 (dd, *J =* 9.2 Hz, 6.0 Hz, 1H), 7.63 (d, *J =* 8.4 Hz, 1H), 7.53 (d, *J =* 2.0 Hz, 1H), 7.48 - 7.42 (m, 1H), 7.41-7.33 (m, 2H), 7.17 (d, *J =* 2.4 Hz, 1H), 4.53 - 4.35 (m, 4H), 4.31 (d, *J =* 12.0 Hz, 1H), 3.92 (s, 1H), 3.82 - 3.71 (m, 1H), 3.66 - 3.51 (m, 5H), 3.01 (s, 1H), 2.84 - 2.63 (m, 4H), 2.45 - 2.06 (m, 12H), 2.04 - 1.78 (m, 7H), 1.72-1.57 (m, 6H), 1.49 - 1.33 (m, 3H), 1.24 - 1.04 (m, 2H), 1.09 - 0.98 (m, 2H).

### Example 25: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclobutyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclobutyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

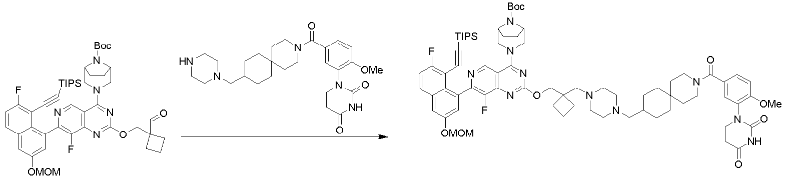

Tetraisopropyl titanate (333 mg, 1.17 mmol) was added to a solution of *tert-butyl* 3-(8-fluoro-7-(7-fluoro-3-(methoxymethoxy)-8-((triisopropylsilyl)ethynyl)naphthalen-1-yl)-2-((1-formylcyclobutyl)methoxy)pyrido[4,3-*d*]pyrimidin-4-yl)-3,8-diazabicyclo[3.2.1]octane-8-carboxylate (100 mg, 0.117 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (70 mg, 0.140 mmol) in DCM (2 mL), and the mixture was stirred at 30 °C for 2 h. NaBH(OAc)₃ (78 mg, 0.351 mmol) was added to the mixture at room temperature, and the resulting mixture was stirred for 2 h. The mixture was concentrated and purified by silica gel column chromatography (DCM:MeOH = 100:1 to 15:1) to give *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclobutyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 669.9.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclobutyl)methyl)piperazin-1-yl)methyl)-3-azaspiro[5.5]undecane-3-carbonyl)-2-methoxyphenyl)dihydropyrimidine-2,4(1H,3H)-dione

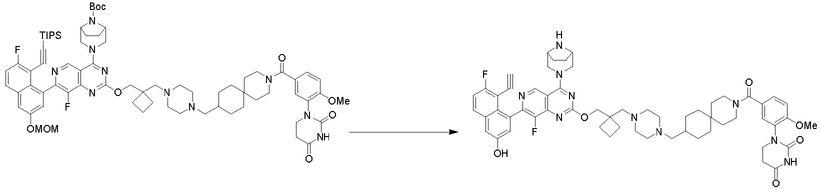

CsF (58 mg, 0.385 mmol) was added to a solution of *tert-butyl* 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclobutyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate (100 mg, 0.077 mmol) in DMF (2.0 mL), and the mixture was reacted at room temperature for 1 h. Water (10 mL) was added, and the mixture was extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (2.0 mL), and HCl/1,4-dioxane (6 N, 1.0 mL) was added. The mixture was reacted at room temperature for 0.5 h. An aqueous Na₂CO₃ solution was added to the reaction liquid to adjust the pH > 7, and the mixture was extracted with EtOAc (10 mL × 2). The organic phase was washed with brine, dried over anhydrous Na₂SO₄, and concentrated. The crude product was purified by Pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclobutyl)methyl) piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 519.2.

¹H NMR (400 MHz, DMSO-*d*₆) δ10.32 (s, 1H), 10.14 (s, 1H), 9.03 (s, 1H), 7.97 (dd, *J* = 9.1, 5.9 Hz, 1H), 7.51-7.42 (m, 1H), 7.41 - 7.27(m, 3H), 7.23 - 7.08 (m, 2H), 4.58 - 4.39 (m, 3H), 4.31 (d, *J =* 12.1 Hz, 1H), 3.92 (s, 1H), 3.84 (s, 3H), 3.67 - 3.37 (m, 9H), 2.68 (t, *J* = 6.4 Hz, 3H), 2.46 - 2.11 (m, 10H), 2.05 - 1.80 (m, 8H), 1.73 - 1.57 (m, 6H), 1.56 - 1.17 (m, 8H), 1.12 - 0.89 (m, 4H).

### Example 26: 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2,2-dimethylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-(3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

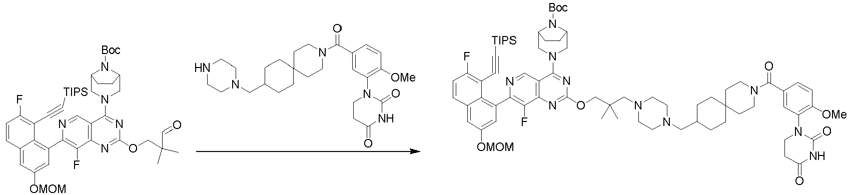

Tetraisopropyl titanate (335 mg, 1.18 mmol) was added to a solution of *tert-*butyl 3-(2-(2,2-dimethyl-3-oxopropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (100 mg, 0.118 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (71 mg, 0.142 mmol) in DCM (2 mL), and the mixture was stirred at 30 °C for 2 h. NaBH(OAc)₃ (79 mg, 0.354 mmol) was added to the mixture at room temperature, and the resulting mixture was stirred for 2 h. The mixture was concentrated and purified by silica gel column chromatography (MeOH:DCM = (0-1):4) to give *tert-*butyl 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 663.4.

### Step 2: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2,2-dimethylpropyl)piperazin-1-yl)methyl)-3-azaspiro[5.5]undecane-3-carbonyl)-2-methoxyphenyl)dihydropyrimidine-2,4(1H,3H)-dione

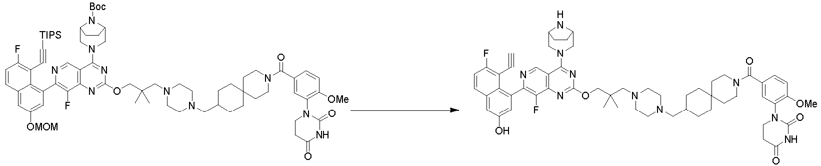

CsF (29 mg, 0.19 mmol) was added to a solution of *tert-butyl* 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2,2-dimethylpropyl)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (50 mg, 0.038 mmol) in DMF (2.0 mL), and the mixture was reacted at room temperature for 1 h. After the reaction was completed, water (10 mL) was added, and the mixture was extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (1.0 mL), and HCl/1,4-dioxane (6 N, 0.5 mL) was added. The mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by Pre-HPLC to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2,2-dimethylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1025.2.

¹H NMR (400 MHz, DMSO-*d*₆) δ 10.32 (s, 1H), 10.14 (s, 1H), 9.03 (s, 1H), 7.97 (dd, *J* = 9.2 Hz, 6.0 Hz, 1H), 7.48 - 7.42 (m, 1H), 7.39 - 7.29 (m, 3H), 7.19 - 7.12 (m, 2H), 4.46 (d, *J =* 11.4 Hz, 1H), 4.34 (d, *J* = 11.9 Hz, 1H), 4.20 - 4.14 (m, 2H), 3.91(s, 1H), 3.84 (s, 3H), 3.66 - 3.37 (m, 10H), 2.68 (t, *J* = 6.4 Hz, 2H), 2.48 - 2.36 (m, 5H), 2.33-2.19 (m, 6H), 2.08 - 1.98(m, 2H), 1.72 - 1.60 (m, 6H), 1.54 - 1.38 (m, 5H), 1.34 - 1.22 (m, 3H), 1.11 - 0.98 (m, 3H), 0.94 (s, 6H).

### Example 27: 1-(5-(4-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)piperidine-1-carbonyl)-2-chlorophenyl)dihydropyrimidine-2,4(1H,3H)-dione

### Step 1: preparation of piperidine-4-carbaldehyde

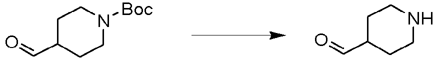

*tert*-Butyl 4-formylpiperidine-1-carboxylate (1 g, 4.7 mmol) was dissolved in a dichloromethane solution (4 mL), and trifluoroacetic acid (2 mL) was added. The mixture was stirred at 30 °C for 1 h. After the reaction was completed, the mixture was concentrated under reduced pressure to give piperidine-4-carbaldehyde. The crude product was directly used in the next step without further purification.

### Step 2: preparation of 1-(4-chloro-3-(2,4-dioxotetrahydropyrimidin-1(2H)-yl)benzoyl)piperidine-4-carbaldehyde

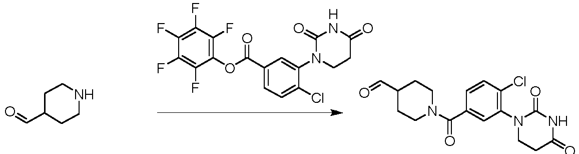

DIEA (2.8 mg, 22 mmol) and pentafluorophenyl 4-chloro-3-(2,4-dioxotetrahydropyrimidin-1(2*H*)-yl)benzoate (1.5 g, 3.5 mmol) were added to a solution of piperidine-4-carbaldehyde (500 mg, 4.4 mmol) in *N,N*-dimethylformamide (5 mL). The mixture was stirred at 30 °C for 16 h. After the reaction was completed, the mixture was diluted with water (10 mL) and extracted with ethyl acetate (20 mL × 3). The organic phase was washed with brine (50 mL), dried over anhydrous Na₂SO₄, filtered, and concentrated under reduced pressure. The crude product was purified by silica gel column chromatography (DCM:MeOH (NH₃) = 20:1) to give 1-(4-chloro-3-(2,4-dioxotetrahydropyrimidin-1(2*H*)-yl)benzoyl)piperidine-4-carbaldehyde.

LC-MS: (ESI, m/z): [M+H]⁺ = 364.1.

### Step 3: preparation of tert-butyl 3-(2-((1-((4-((1-(4-chloro-3-(2,4-dioxotetrahydropyrimidin-1(2H)-yl)benzoyl)piperidin-4-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

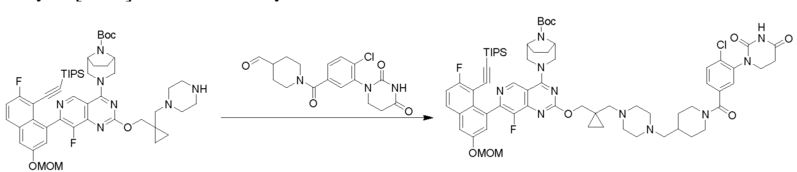

1-(4-Chloro-3-(2,4-dioxotetrahydropyrimidin-1(2*H*)-yl)benzoyl)piperidine-4-carbaldehyde (40 mg, 0.11 mmol) and tetraisopropyl titanate (1 g) were added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (151 mg, 0.17 mmol) in tetrahydrofuran (1 mL). The mixture was stirred at 70 °C for 2 h and then cooled to 0 °C, and anhydrous methanol (1 mL) and NaBH(OAc)₃ (98 mg, 0.44 mmol) were added. The mixture was stirred at 30 °C for 2 h. After the reaction was completed, H₂O (10 mL) was added for dilution, and the mixture was extracted with ethyl acetate (10 mL × 3). The combined organic phase was dried over anhydrous sodium sulfate, filtered, and concentrated under reduced pressure. The crude product was purified by silica gel column chromatography (DCM:MeOH = 20:1) to give *tert-*butyl 3-(2-((1-((4-((1-(4-chloro-3-(2,4-dioxotetrahydropyrimidin-1(2*H*)-yl)benzoyl)piperidin-4-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 630.3.

### Step 4: preparation of 1-(5-(4-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)piperidine-1-carbonyl)-2-chlorophenyl)dihydropyrimidine-2,4(1H,3H)-dione

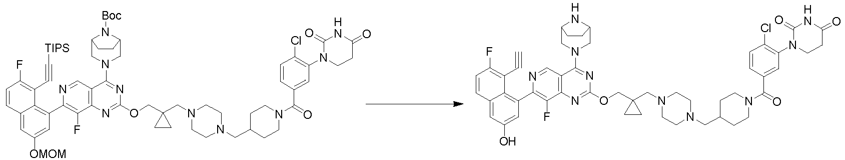

*tert-*Butyl 3-(2-((1-((4-((1-(4-chloro-3-(2,4-dioxotetrahydropyrimidin-1(2*H*)-yl)benzoyl)piperidin-4-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (100 mg, 0.08 mmol) was dissolved in DMF (1 mL), and CsF (61 mg, 0.4 mmol) was added. The mixture was reacted at room temperature for 2 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added for dilution, and the mixture was adjusted to pH > 7 with an aqueous Na₂CO₃ solution and then extracted with ethyl acetate (5 mL × 3). The combined organic phase was dried over Na₂SO₄, filtered, and concentrated under reduced pressure. The crude product was purified by Pre-HPLC to give 1-(5-(4-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)piperidine-1-carbonyl)-2-chlorophenyl)dihydropyrimidine-2,4(1*H*,3*H*)-dione.

LC-MS: (ESI, m/z): [M+H]⁺ = 959.1.

¹H NMR (400 MHz, CD₃OD) δ 8.99 (s, 1H), 7.85 (dd, *J =* 8.8 Hz, 5.6 Hz, 1H), 7.63 (m, *J =* 8.0 Hz, 1H), 7.52 (s, 1H), 7.40 (d, *J* = 8.0 Hz, 1H), 7.35 - 7.28 (m, 2H), 7.20 (d, *J* = 2.8 Hz, 1H), 4.70 - 4.44 (m, 4H), 4.38 - 4.33 (m, 1H), 3.85 - 3.61 (m, 7H), 3.36 (s, 1H), 2.93 - 2.77 (m, 2H), 2.55 - 2.33 (m, 7H), 2.24 - 2.11(m, 3H), 1.91 - 1.71(m, 7H), 1.36 - 1.27 (m, 4H), 1.24 - 1.04 (m, 2H), 0.76 - 0.64 (s, 2H), 0.56 - 0.43 (s, 2H).

### Example 28: 1-(5-(4-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)ethyl)piperazine-1-carbonyl)-2-chlorophenyl)dihydropyrimidine-2,4(1H,3H)-dione

### Step 1: preparation of benzyl 4-(2-(1-(tert-butoxycarbonyl)piperidin-4-yl)ethyl)piperazine-1-carboxylate

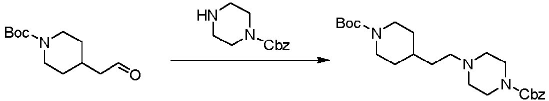

*tert-*Butyl 4-(2-oxoethyl)piperidine-1-carboxylate (2 g, 8.8 mmol) was dissolved in 1,2-dichloroethane (30 mL), and acetic acid (1 mL) and benzyl piperazine-1-carboxylate (5.8 g, 26.4 mmol) were added. The mixture was stirred at 30 °C for 2 h, and then NaBH₃CN (1.1 g, 17.6 mmol) was slowly added in an ice bath. The mixture was stirred at 30 °C for 16 h. After the reaction was completed, the mixture was diluted with water (30 mL) and extracted with ethyl acetate (50 mL × 3), dried over anhydrous Na₂SO₄, filtered, and concentrated under reduced pressure. The crude product was purified by silica gel column chromatography (DCM:MeOH = 20:1) to give benzyl 4-(2-(1-(tert-butoxycarbonyl)piperidin-4-yl)ethyl)piperazine-1-carboxylate.

LC-MS: (ESI, m/z): [M+H]⁺ = 432.2.

### Step 2: preparation of tert-butyl 4-(2-(piperazin-1-yl)ethyl)piperidine-1-carboxylate

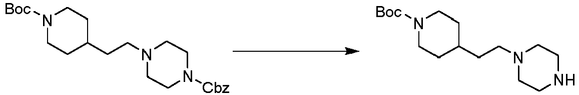

Pd/C (490 mg) was added to a solution of benzyl 4-(2-(1-(tert-butoxycarbonyl)piperidin-4-yl)ethyl)piperazine-1-carboxylate (2 g, 4.6 mmol) in methanol (10 mL), and the mixture was stirred at 30 °C for 16 h under hydrogen atmosphere. After the reaction was completed, the mixture was filtered through celite, and the filtrate was concentrated under reduced pressure to give *tert-*butyl 4-(2-(piperazin-1-yl)ethyl)piperidine-1-carboxylate. The crude product was directly used in the next step without further purification.

### Step 3: preparation of tert-butyl 4-(2-(4-(4-chloro-3-(2,4-dioxotetrahydropyrimidin-1(2H)-yl)benzoyl)piperazin-1-yl)ethyl)piperidine-1-carboxylate

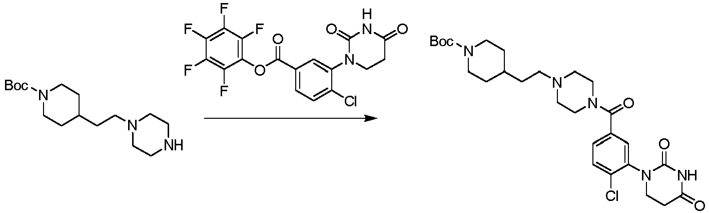

DIEA (520 mg, 4.02 mmol) was added to a solution of pentafluorophenyl 4-chloro-3-(2,4-dioxotetrahydropyrimidin-1(2H)-yl)benzoate (582 mg, 1.34 mmol) in DMF (3 mL), and a solution of *tert-*butyl 4-(2-(piperazin-1-yl)ethyl)piperidine-1-carboxylate (400 mg, 1.34 mmol) in DMF (3 mL) was added. The mixture was stirred at 30 °C for 2 h. After the reaction was completed, water (10 mL) was added, and the mixture was extracted with ethyl acetate (20 mL × 3). The organic phase was washed with saturated brine (30 mL), dried over anhydrous Na₂SO₄, filtered, and concentrated under reduced pressure. The crude product was purified by silica gel column chromatography (DCM:MeOH = 20:1) to give *tert*-butyl 4-(2-(4-(4-chloro-3-(2,4-dioxotetrahydropyrimidin-1(2*H*)-yl)benzoyl)piperazin-1-yl)ethyl)piperidine-1-carboxylate.

LC-MS: (ESI, m/z): [M+H]⁺ = 548.3.

### Step 4: preparation of 1-(2-chloro-5-(4-(2-(piperidin-4-yl)ethyl)piperazine-1-carbonyl)phenyl) dihydropyrimidine-2,4(1H,3H)-dione

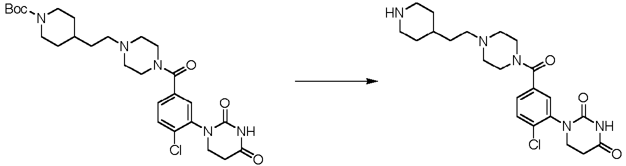

HCl/1,4-dioxane (1 mL) was added to a solution of *tert-*butyl 4-(2-(4-(4-chloro-3-(2,4-dioxotetrahydropyrimidin-1(2*H*)-yl)benzoyl)piperazin-1-yl)ethyl)piperidine-1-carboxylate (300 mg, 0.55 mmol) in 1,4-dioxane (2 mL), and the mixture was stirred at 30 °C for 1 h. After the reaction was completed, an Na₂CO₃ (5 mL) solution was added, and the mixture was extracted with ethyl acetate (10 mL × 3). The combined organic phase was dried over anhydrous sodium sulfate, filtered, and concentrated under reduced pressure to give 1-(2-chloro-5-(4-(2-(piperidin-4-yl)ethyl)piperazine-1-carbonyl)phenyl)dihydropyrimidine-2,4(1H,3H)-dione. The crude product was directly used in the next step without further purification.

### Step 5: preparation of tert-butyl 3-(2-((1-((4-(2-(4-(4-chloro-3-(2,4-dioxotetrahydropyrimidin-1(2H)-yl)benzoyl)piperazin-1-yl)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

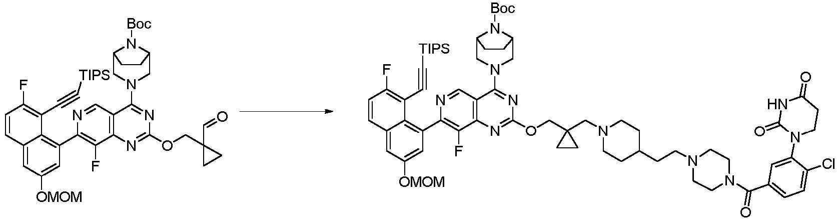

1-(2-Chloro-5-(4-(2-(piperidin-4-yl)ethyl)piperazine-1-carbonyl)phenyl)dihydropyrimidine-2,4(1*H*,3*H*)-dione (81 mg, 0.18 mmol) and tetraisopropyl titanate (1 g) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in tetrahydrofuran (1 mL). The mixture was stirred at 70 °C for 2 h and then cooled to 0 °C. Anhydrous methanol (1 mL) and NaBH(OAc)₃ (107 mg, 0.48 mmol) were added, and the mixture was stirred at 30 °C for 2 h. After the reaction was completed, H₂O (10 mL) was added, and the mixture was extracted with ethyl acetate (10 mL × 3). The combined organic phase was dried over anhydrous sodium sulfate, filtered, and concentrated under reduced pressure. The crude product was purified by silica gel column chromatography (DCM:MeOH = 20:1) to give *tert-*butyl 3-(2-((1-((4-(2-(4-(4-chloro-3-(2,4-dioxotetrahydropyrimidin-1(2*H*)-yl)benzoyl)piperazin-1-yl)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 637.4.

### Step 6: preparation of 1-(5-(4-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)ethyl)piperazine-1-carbonyl)-2-chlorophenyl)dihydropyrimidine-2,4(1H,3H)-dione

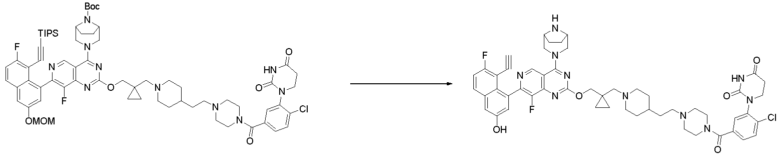

*tert-*Butyl 3-(2-((1-((4-(2-(4-(4-chloro-3-(2,4-dioxotetrahydropyrimidin-1(2*H*)-yl)benzoyl)piperazin-1-yl)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (110 mg, 0.086 mmol) was dissolved in DMF (1 mL), and CsF (65 mg, 0.43 mmol) was added. The mixture was reacted at room temperature for 1 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the product obtained above, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The resulting crude product was purified by Pre-HPLC to give 1-(5-(4-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)ethyl)piperazine-1-carbonyl)-2-chlorophenyl)dihydropyrimidine-2,4(1*H*,3*H*)-dione.

LC-MS: (ESI, m/z): [M+H]⁺ = 973.1.

¹H NMR (400 MHz, CD₃OD) δ 8.99 (s, 1H), 7.85 (dd, *J =* 9.2 Hz, 5.6 Hz, 1H), 7.64 (d, *J =* 8.4 Hz, 1H), 7.54 (d, *J =* 1.6 Hz, 1H), 7.46 - 7.38 (m,1H), 7.35 - 7.25 (m, 2H), 7.19 (d, *J =* 1.8 Hz, 1H), 4.66 - 4.43 (m, 4H), 4.42 - 4.29 (m, 1H), 3.94 - 3.60 (m, 7H), 3.35 (s, 1H), 2.90 - 2.81 (m, 2H), 2.53 -2.36 (m, 7H), 2.07 - 1.97 (m, 2H), 1.90 - 1.77 (m, 4H), 1.73 - 1.55 (m, 3H), 1.42 - 1.22 (m, 9H), 0.77 - 0.67(m, 2H), 0.59 - 0.44 (m, 2H).

### Example 29: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-bromophenyl)dihydropyrimidine-2,4(1H,3H)-dione

### Step 1: preparation of 3-azaspiro[5.5]undecane-9-carbaldehyde

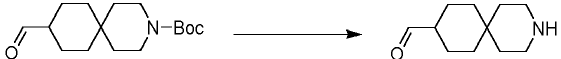

*tert-*Butyl 9-formyl-3-azaspiro[5.5]undecane-3-carboxylate (200 mg, 0.71 mmol) was dissolved in a dichloromethane solution (2 mL), and trifluoroacetic acid (1 mL) was added. The mixture was stirred at 30 °C for 1 h. After the reaction was completed, the mixture was concentrated under reduced pressure to give 3-azaspiro[5.5]undecane-9-carbaldehyde. The crude product was directly used in the next step without further purification.

### Step 2: preparation of 3-(4-bromo-3-(2,4-dioxotetrahydropyrimidin-1(2H)-yl)benzoyl)-3-azaspiro[5.5]undecane-9-carbaldehyde

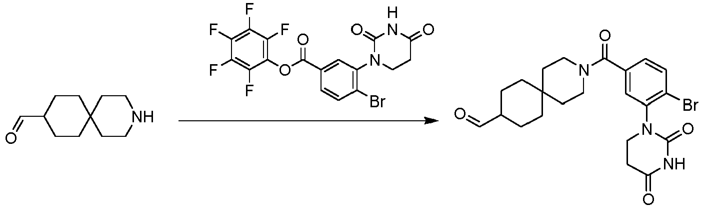

DIEA (459 mg, 3.55 mmol) and pentafluorophenyl 4-bromo-3-(2,4-dioxotetrahydropyrimidin-1(2*H*)-yl)benzoate (340 mg, 0.71 mmol) were added to a solution of 3-azaspiro[5.5]undecane-9-carbaldehyde (128 mg, 0.71 mmol) in dimethyl sulfoxide (3 mL). The mixture was stirred at 30 °C for 1 h. After the reaction was completed, the mixture was diluted with water (10 mL) and extracted with ethyl acetate (10 mL × 3). The organic phase was washed with brine (30 mL), dried over anhydrous Na₂SO₄, filtered, and concentrated under reduced pressure. The crude product was purified by silica gel column chromatography (DCM:MeOH (NH₃) = 20:1) to give 3-(4-bromo-3-(2,4-dioxotetrahydropyrimidin-1(2*H*)-yl)benzoyl)-3-azaspiro[5.5]undecane-9-carbaldehyde.

LC-MS: (ESI, m/z): [M+H]⁺ = 476.0.

### Step 3: preparation of tert-butyl 3-(2-((1-((4-((3-(4-bromo-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

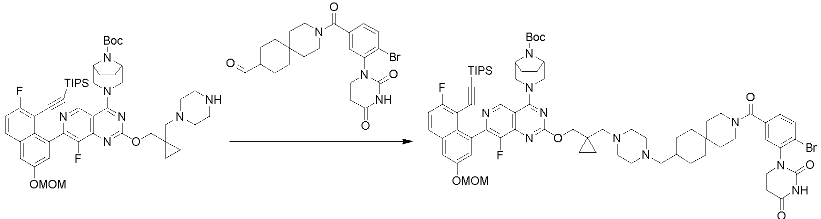

3-(4-Bromo-3-(2,4-dioxotetrahydropyrimidin-1(2*H*)-yl)benzoyl)-3-azaspiro[5.5]undecane-9-carbaldehyde (80 mg, 0.17 mmol) was added to a mixed solution of *tert-*butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-*d*]pyrimidin-4-yl)-3,8-diazabicyclo[3.2.1]octane-8-carboxylate (100 mg, 0.11 mmol) in ethanol/acetic acid (2 mL/0.02 mL). The mixture was stirred at 30 °C for 0.5 h. Then NaBH₃ CN (20 mg, 0.33 mmol) was added. After the reaction was completed, H₂O (10 mL) was added, and the mixture was extracted with ethyl acetate (10 mL × 3). The combined organic phase was dried over anhydrous sodium sulfate, filtered, and concentrated under reduced pressure. The crude product was purified by silica gel column chromatography (DCM:MeOH = 20:1) to give *tert-*butyl 3-(2-((1-((4-((3-(4-bromo-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 687.2.

### Step 4: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-bromophenyl)dihydropyrimidine-2,4(1H,3H)-dione

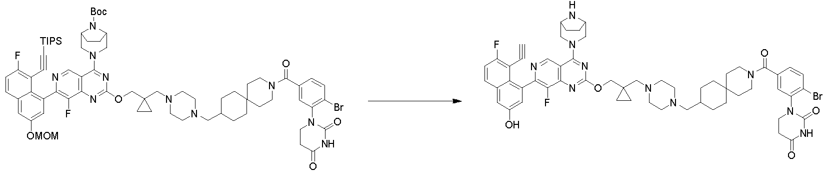

CsF (76 mg, 0.5 mmol) was added to a solution of *tert-*butyl 3-(2-((1-((4-((3-(4-bromo-3-(2,4-dioxotetrahydropyrimidin-1(2*H*)-yl)benzoyl)-3-azaspiro[5.5]undecan-9-yl)methyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (140 mg, 0.11 mmol) in DMF (1 mL), and the mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the product obtained above, and the mixture was stirred at room temperature for 0.5 h. After the reaction was completed, H₂O (5 mL) was added, and then the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by Pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-bromophenyl)dihydropyrimidine-2,4(1*H*,3*H*)-dione.

LC-MS: (ESI, m/z): [M/2+H]⁺ = 536.2.

¹H NMR (400 MHz, DMSO-*d*₆) δ 10.50 (s, 1H), 10.14 (s, 1H), 9.02 (s, 1H), 8.01 - 7.94 (m, 1H), 7.78 (d, *J =* 8.0 Hz, 1H), 7.53 (d, *J =* 8.0 Hz, 1H), 7.45 - 7.43 (m, 1H), 7.41 - 7.36 (m, 1H), 7.33 - 7.27 (m, 1H), 7.16 (d, *J =* 2.0 Hz,1H), 4.52 -4.43 (m, 1H), 4.32 - 4.20 (m, 3H), 3.93 (s, 1H), 3.79 - 3.69 (m, 1H), 3.68 - 3.47 (m, 7H), 3.27 - 3.18 (m, 2H), 2.80 - 2.65 (m, 2H), 2.37 - 2.19 (m, 9H), 2.10 - 1.95 (m, 3H), 1.72 - 1.58 (m, 6H), 1.56 - 1.22 (m, 9H), 1.14-0.89 (m, 3H), 0.66 - 0.55 (m, 2H), 0.44 - 0.33 (m, 2H).

### Example 30: 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

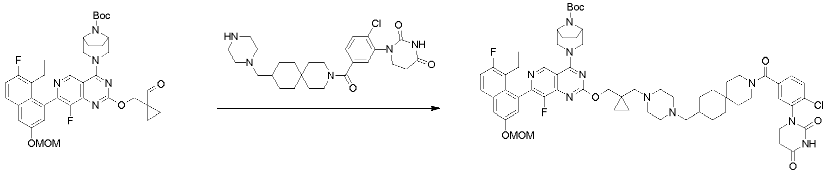

1-(2-Chloro-5-(9-(piperazin-1-ylmethyl)-3-azaspiro[5.5]undecane-3-carbonyl)phenyl)dihydropyrimidine-2,4(1*H*,3*H*)-dione (35 mg, 0.070 mmol) and tetraisopropyl titanate (50 mg) were added to a solution of *tert-*butyl 3-(7-(8-ethyl-7-fluoro-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate (40 mg, 0.058 mmol) in tetrahydrofuran (0.5 mL). The mixture was stirred at 70 °C for 2 h and then cooled to 0 °C. Anhydrous methanol (0.5 mL) and NaBH(OAc)₃ (52 mg, 0.23 mmol) were added, and the mixture was stirred at 30 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated under reduced pressure. The crude product was purified by silica gel column chromatography (DCM:MeOH = 20:1) to give *tert-*butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 588.4.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

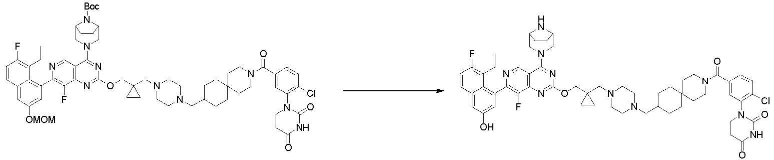

Hydrochloric acid/1,4-dioxane (6 N, 1 mL) was added to *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (30 mg, 0.026 mmol), and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by Pre-HPLC to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1*H,*3*H*)-dione.

LC-MS: (ESI, m/z): [M/2+H]⁺ = 516.3.

¹H NMR (400 MHz, CD₃OD) δ 9.04 (s, 1H), 7.69 - 7.62 (m, 2H), 7.53 (s, 1H), 7.41 (d, *J =* 8.2 Hz, 1H), 7.30 - 7.20 (m, 2H), 7.05 (d, *J=* 2.5 Hz, 1H), 4.61 (t, *J =* 11.3 Hz, 2H), 4.48 (d, *J =* 10.9 Hz, 1H), 4.38 (d, *J=* 11.1 Hz, 1H), 3.78 (t, *J =* 6.3 Hz, 2H), 3.74 - 3.63 (m, 7H), 3.46 - 3.36 (m, 2H), 2.90 - 2.82 (m, 2H), 2.55 - 2.35 (m, 9H), 2.24 - 2.08 (m, 4H), 1.90 - 1.72 (m, 7H), 1.64 - 1.58 (s, 3H), 1.53 - 1.40 (m, 3H), 1.35 - 1.26 (m, 2H), 1.18 - 1.11 (m, 2H), 0.80 (t, *J* = 7.4 Hz, 3H), 0.74 - 0.66 (m, 2H), 0.52 - 0.43 (m, 2H).

### Example 31: 1-(5-(9-(4-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)butyl)-3-azaspiro[5.5]undecane-3-carbonyl)-2-chlorophenyl)dihydropyrimidine-2,4(1H,3H)-dione

### Step 1: preparation of 4-(3-(tert-butoxycarbonyl)-3-azaspiro[5.5]undecan-9-yl)but-3-enoic acid

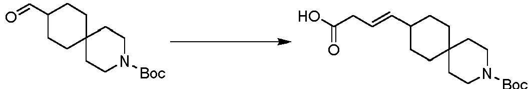

LiHMDS (10.8 mL, 10.8 mmol, 1 M/THF) was added dropwise to a solution of (2-carboxyethyl)triphenylphosphonium bromide (1.92 g, 4.60 mmol) in THF (10 mL) at -20 °C, and the mixture was stirred for 30 min. A solution of *tert*-butyl 9-formyl-3-azaspiro[5.5]undecane-3-carboxylate (1.00 g, 3.60 mmol) in THF (5 mL) was then added. The mixture was reacted at room temperature for 16 h. Water (50 mL) was added, and the mixture was extracted with ethyl acetate (40 mL). The aqueous phase was separated, adjusted to pH = 2-3 with an aqueous hydrochloric acid solution (1 N), and extracted with ethyl acetate (40 mL × 3). The combined organic phase was washed with brine (100 mL), dried over sodium sulfate, filtered, and concentrated under reduced pressure. The crude product was purified by silica gel column chromatography (PE:EtOAc = 100:1 to 4:1) to give 4-(3-(*tert*-butoxycarbonyl)-3-azaspiro[5.5]undecan-9-yl)but-3-enoic acid.

LC-MS: (ESI, m/z): [M+Na]⁺ = 360.1.

### Step 2: preparation of 4-(3-(tert-butoxycarbonyl)-3-azaspiro[5.5]undecan-9-yl)butanoic acid

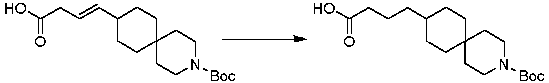

4-(3-(*tert*-Butoxycarbonyl)-3-azaspiro[5.5]undecan-9-yl)but-3-enoic acid (0.8 g, 2.37 mmol) was dissolved in MeOH (10 mL), and Pd/C (150 mg) was added. The mixture was purged three times under hydrogen atmosphere and stirred at room temperature overnight. The mixture was filtered and concentrated to give 4-(3-(tert-butoxycarbonyl)-3-azaspiro[5.5]undecan-9-yl)butanoic acid.

¹H NMR (400 MHz, CDCl₃) δ 3.39 - 3.30 (m, 4H), 2.33 (t, *J =* 7.2 Hz, 1H), 1.67 -1.60 (m, 4H), 1.57 - 1.52 (m, 2H), 1.49 - 1.43 (m, 11H), 1.30 - 1.22 (m, 5H), 1.10 - 1.04 (m, 4H).

### Step 3: preparation of tert-butyl 9-(4-hydroxybutyl)-3-azaspiro[5.5]undecane-3-carboxylate

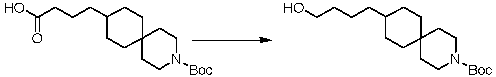

4-(3-(*tert*-Butoxycarbonyl)-3-azaspiro[5.5]undecan-9-yl)butanoic acid (0.75 g, 2.21 mmol) was dissolved in THF (5 mL), and a solution of BH₃/tetrahydrofuran (11.1 mmol, 1 M) was added dropwise at 0 °C. The mixture was reacted at room temperature for 2 h. MeOH (2 mL) was added at 0 °C to quench the reaction, and the mixture was concentrated to give *tert-*butyl 9-(4-hydroxybutyl)-3-azaspiro[5.5]undecane-3-carboxylate, which was directly used in the next step.

LC-MS: (ESI, m/z): [M-tBu+H]⁺ = 270.2.

### Step 4: preparation of tert-butyl 9-(4-oxobutyl)-3-azaspiro[5.5]undecane-3-carboxylate

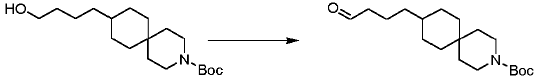

Dess-Martin oxidant (1.08 g, 2.54 mmol) was added in batches to a solution of *tert-*butyl 9-(4-hydroxybutyl)-3-azaspiro[5.5]undecane-3-carboxylate (0.55 g, 1.69 mmol) in DCM (10 mL). The mixture was stirred at room temperature for 1 h. The reaction liquid was concentrated, and the crude product was purified by silica gel column chromatography (PE:EA = 50:1 to 5:1) to give *tert-*butyl 9-(4-oxobutyl)-3-azaspiro[5.5]undecane-3-carboxylate.

¹H NMR (400 MHz, CDCl₃) δ 9.76 (s, 1H), 3.42 - 3.30 (m, 4H), 2.41 (t, *J* = 5.2 Hz, 1H), 1.70 - 1.60 (m, 4H), 1.48 - 1.40 (m, 12H), 1.30 - 1.20 (m, 6H), 1.12 - 1.02 (m, 4H).

### Step 5: preparation of 4-(3-(4-chloro-3-(2,4-dioxotetrahydropyrimidin-1(2H)-yl)benzoyl)-3-azaspiro[5.5]undecan-9-yl)butanal

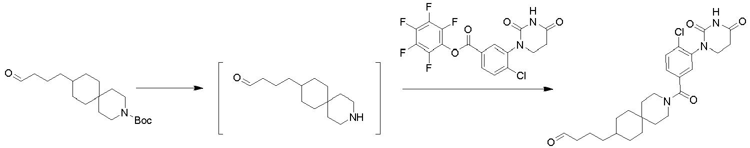

*tert-*Butyl 9-(4-oxobutyl)-3-azaspiro[5.5]undecane-3-carboxylate (320 mg, 0.99 mmol) was dissolved in DCM (4 mL), and then TFA (2 mL) was added. The mixture was stirred at room temperature for 1 h and concentrated to give 4-(3-azaspiro[5.5]undecan-9-yl)butanal trifluoroacetate (crude). The resulting crude product was dissolved in DMF (3 mL) and added to a solution of DIEA (516 mg, 4.00 mmol) and pentafluorophenyl 4-chloro-3-(2,4-dioxotetrahydropyrimidin-1(2*H*)-yl)benzoate (430 mg, 0.99 mmol) in DMF (3 mL). The reaction liquid was stirred at room temperature for 1 h. Water was added to the reaction liquid, and the mixture was extracted with ethyl acetate. The organic phases were combined, dried over anhydrous sodium sulfate, and filtered, and the filtrate was concentrated. The crude product was purified by silica gel column chromatography (DCM:MeOH = 100:1 to 30:1) to give 4-(3-(4-chloro-3-(2,4-dioxotetrahydropyrimidin-1(2*H*)-yl)benzoyl)-3-azaspiro[5.5]undecan-9-yl)butanal.

LC-MS: (ESI, m/z): [M+H]⁺ = 474.2.

### Step 6: preparation of tert-butyl 3-(2-((1-((4-(4-(3-(4-chloro-3-(2,4-dioxotetrahydropyrimidin-1(2H)-yl)benzoyl)-3-azaspiro[5.5]undecan-9-yl)butyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

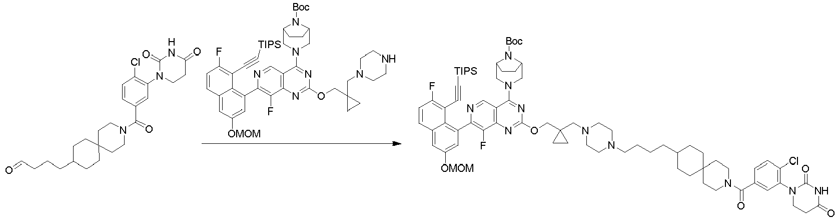

A solution of *tert-*Butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido [4,3-*d*]pyrimidin-4-yl)-3,8-diazabicyclo[3.2.1]octane-8-carboxylate (80 mg, 0.088 mmol) and 4-(3-(4-chloro-3-(2,4-dioxotetrahydropyrimidin-1(2*H*)-yl)benzoyl)-3-azaspiro[5.5]undecan-9-yl)butanal (83 mg, 0.175 mmol) in DCM/MeOH/AcOH (2.0 mL/0.5 mL/0.05 mL) was stirred at room temperature for 2 h. NaBH₃CN (47 mg, 0.22 mmol) was added at 0-5 °C, and the mixture was stirred at room temperature for 0.5 h and concentrated. The crude product was purified by silica gel column chromatography (DCM:MeOH = 100:1 to 5:1) to give *tert-*butyl 3-(2-((1-((4-(4-(3-(4-chloro-3-(2,4-dioxotetrahydropyrimidin-1(2*H*)-yl)benzoyl)-3-azaspiro[5.5]undecan-9-yl)butyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 685.0.

### Step 7: preparation of 1-(5-(9-(4-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)butyl)-3-azaspiro[5.5]undecane-3-carbonyl)-2-chlorophenyl)dihydropyrimidine-2,4(1H,3H)-dione

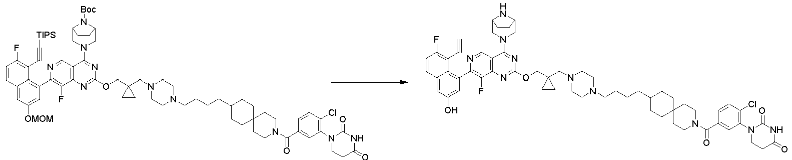

CsF (44 mg, 0.29 mmol) was added to a solution of *tert-*butyl 3-(2-((1-((4-(4-(3-(4-chloro-3-(2,4-dioxotetrahydropyrimidin-1(2*H*)-yl)benzoyl)-3-azaspiro[5.5]undecan-9-yl)butyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (80 mg, 0.058 mmol) in DMF (1.5 mL), and the mixture was reacted at room temperature for 1 h. Water (5 mL) was added to the reaction liquid, and the mixture was extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (2.0 mL), and HCl/1,4-dioxane (6 N, 1.0 mL) was added. The mixture was reacted at room temperature for 0.5 h. An aqueous Na₂CO₃ solution was added to the reaction liquid to adjust the pH> 7, and the mixture was extracted with ethyl acetate (10 mL × 2). The organic phase was washed with brine, dried over anhydrous Na₂SO₄, and concentrated. The crude product was purified by Pre-HPLC to give 1-(5-(9-(4-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)butyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 1069.3.

¹H NMR (400 MHz, CD₃OD) δ 8.99 (s, 1H), 7.88 - 7.80 (m, 1H), 7.63 (d, *J =* 8.4 Hz, 1H), 7.52 (s, 1H), 7.41 (dd, *J* = 8.0 Hz, 2.0 Hz, 1H), 7.35 - 7.28 (m, 2H), 7.20 (d, *J* = 1.8 Hz, 1H), 4.70 - 7.30 (m, 5H), 3.92 - 3.61 (m, 9H), 3.46 - 3.34 (m, 3H), 2.90 - 2.82 (m, 2H), 2.57 - 2.27 (m, 10H), 1.88 - 1.71 (m, 5H), 1.60 - 1.43 (m, 6H), 1.33 - 1.11 (m, 12H), 0.77 - 0.63 (m, 2H), 0.53 - 0.41 (m, 2H).

### Example 32: 1-(5-(9-(2-((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)ethyl)-3-azaspiro[5.5]undecane-3-carbonyl)-2-chlorophenyl)dihydropyrimidine-2,4(1H,3H)-dione

### Step 1: preparation of tert-butyl 9-(2-oxoethyl)-3-azaspiro[5.5]undecane-3-carboxylate

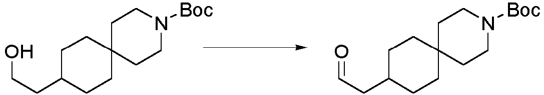

Dess-Martin reagent (860 mg, 2.02 mmol) was added to a solution of *tert-*butyl 9-(2-hydroxyethyl)-3-azaspiro[5.5]undecane-3-carboxylate (500 mg, 1.68 mmol) in DCM (10 mL). The mixture was reacted for 1 h and filtered. The filtrate was concentrated, and the resulting crude product was purified by silica gel column chromatography (PE:EA = 10:1 to 1:1) to give *tert-*butyl 9-(2-oxoethyl)-3-azaspiro[5.5]undecane-3-carboxylate.

LC-MS: (ESI, m/z): [M+H-tBu]⁺ = 240.2.

### Step 2: preparation of tert-butyl (S)-9-(2-(2-(hydroxymethyl)pyrrolidin-1-yl)ethyl)-3-azaspiro[5.5]undecane-3-carboxylate

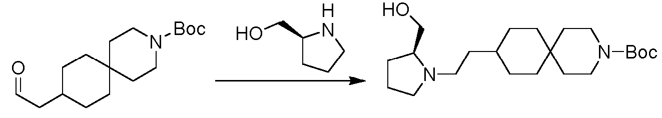

Acetic acid (0.14 mL) was added to a solution of *tert*-butyl 9-(2-oxoethyl)-3- azaspiro[5.5]undecane-3-carboxylate (0.35 g, 1.18 mmol) and (*S*)-pyrrolidin-2-ylmethanol (0.18 g, 1.78 mmol) in 1,2-dichloroethane (6 mL). The mixture was stirred at 30 °C for half an hour. NaBH(OAc)₃ (0.52 g, 2.36 mmol) was added at 0 °C, and then the mixture was reacted at room temperature for another 2 h. After the reaction was completed, H₂O (10 mL) was added, and the mixture was extracted with ethyl acetate (50 mL × 3). The combined organic phase was dried over anhydrous sodium sulfate and filtered, and the filtrate was concentrated under reduced pressure. The crude product was purified by silica gel column chromatography (DCM:MeOH = 20:1) to give *tert-*butyl (*S*)-9-(2-(2-(hydroxymethyl)pyrrolidin-1-yl)ethyl)-3-azaspiro[5.5]undecane-3-carboxylate.

LC-MS: (ESI, m/z): [M+H]⁺ = 381.2.

### Step 3: preparation of (S)-(1-(2-(3-azaspiro[5.5]undecan-9-yl)ethyl)pyrrolidin-2-yl)methanol

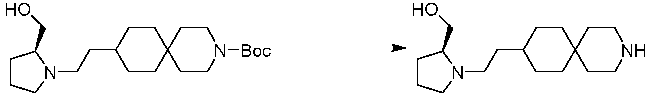

TFA (1 mL) was added to a solution of *tert-*butyl (*S*)-9-(2-(2-(hydroxymethyl)pyrrolidin-1-yl)ethyl)-3-azaspiro[5.5]undecane-3-carboxylate (210 mg, 0.55 mmol) in DCM (3 mL). The mixture was stirred at room temperature for 1 h. The reaction liquid was directly concentrated under reduced pressure to give (*S*)-(1-(2-(3-azaspiro[5.5]undecan-9-yl)ethyl)pyrrolidin-2-yl)methanol, which was directly used in the next step without further purification.

LC-MS: (ESI, m/z): [M+H]⁺ = 281.2.

### Step 4: preparation of (S)-(1-(2-(3-benzyl-3-azaspiro[5.5]undecan-9-yl)ethyl)pyrrolidin-2-yl)methanol

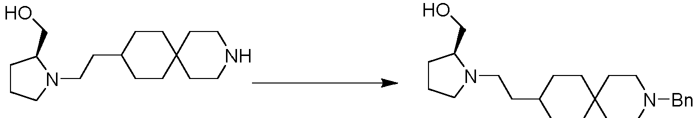

HOAc (0.1 mL) and benzaldehyde (88 mg, 0.83 mmol) were added to a solution of (*S*)-(1-(2-(3-azaspiro[5.5]undecan-9-yl)ethyl)pyrrolidin-2-yl)methanol (154 mg, 0.55 mmol) in DCM/MeOH (3 mL/3 mL), and the mixture was stirred at room temperature for 0.5 h. NaBH(OAc)₃ (233 mg, 1.65 mmol) was added to the mixture at room temperature, and the resulting mixture was stirred for 0.5 h. The mixture was concentrated and purified by silica gel column chromatography to give (*S*)-(1-(2-(3-benzyl-3-azaspiro[5.5]undecan-9-yl)ethyl)pyrrolidin-2-yl)methanol.

LC-MS: (ESI, m/z): [M+H]⁺ = 371.2.

### Step 5: preparation of tert-butyl 3-(2-(((S)-1-(2-(3-azaspiro[5.5]undecan-9-yl)ethyl)pyrrolidin-2-yl)methoxy)-7-chloro-8-fluoropyrido[4,3-d]pyrimidin-4-yl)-3,8-diazabicyclo[3.2.1]octane-8-carboxylate

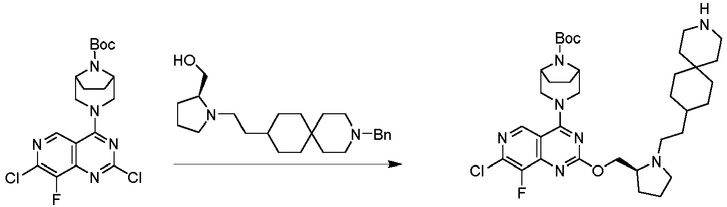

*tert*-Butyl 3-(2,7-dichloro-8-fluoropyrido[4,3-*d*]pyrimidin-4-yl)-3,8-diazabicyclo[3.2.1]octane-8-carboxylate (0.14 g, 0.32 mmol) and (S)-(1-(2-(3-benzyl-3-azaspiro[5.5]undecan-9-yl)ethyl)pyrrolidin-2-yl)methanol (0.18 g, 0.48 mmol) were dissolved in THF (10 mL), and then Cs₂CO₃ (0.32 g, 0.96 mmol) was added. The reaction liquid was heated to 80 °C and reacted for 16 h. After the reaction was completed, the mixture was cooled to room temperature. Water (50 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The crude product was purified by silica gel column chromatography (PE:EA = 1:1) to give *tert-*butyl 3-(2-(((S)-1-(2-(3-azaspiro[5.5]undecan-9-yl)ethyl)pyrrolidin-2-yl)methoxy)-7-chloro-8-fluoropyrido [4,3-*d*]pyrimidin-4-yl)-3,8-diazabicyclo[3.2.1]octane-8-carboxylate.

LC-MS: (ESI, m/z): [M+H]⁺ = 672.3.

### Step 6: preparation of tert-butyl 3-(2-(((S)-1-(2-(3-azaspiro[5.5]undecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

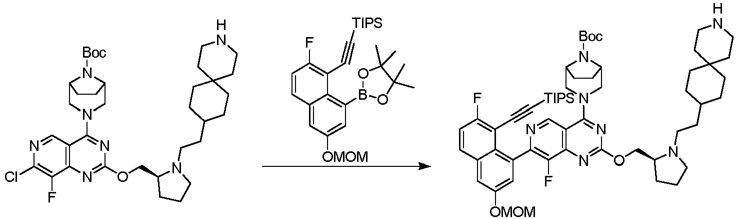

((2-Fluoro-6-(methoxymethoxy)-8-(4,4,5,5-tetramethyl-1,3,2-dioxaborolan-2-yl)naphthalen-1-yl)ethynyl)triisopropylsilane (57 mg, 0.112 mmol), cesium carbonate (73 mg, 0.22 mmol), and Pd-118 (5 mg, 0.012 mmol) were added to a solution of *tert-*butyl 3-(2-(((S)-1-(2-(3-azaspiro[5.5]undecan-9-yl)ethyl)pyrrolidin-2-yl)methoxy)-7-chloro-8-fluoropyrido[4,3-*d*]pyrimidin-4-yl)-3,8-diazabicyclo[3.2.1]octane-8-carboxylate (50 mg, 0.074 mmol) in dioxane/H₂O (1 mL/0.2 mL). The mixture was reacted at 100 °C overnight under N₂ atmosphere and filtered, and the filtrate was concentrated under reduced pressure. The resulting crude product was purified by silica gel column chromatography (DCM/MeOH = 100:1 to 20:1) to give *tert-*butyl 3-(2-(((S)-1-(2-(3-azaspiro[5.5]undecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 511.8.

### Step 7: preparation of tert-butyl 3-(2-(((S)-1-(2-(3-(4-chloro-3-(2,4-dioxotetrahydropyrimidin-1(2H)-yl)benzoyl)-3-azaspiro[5.5]undecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

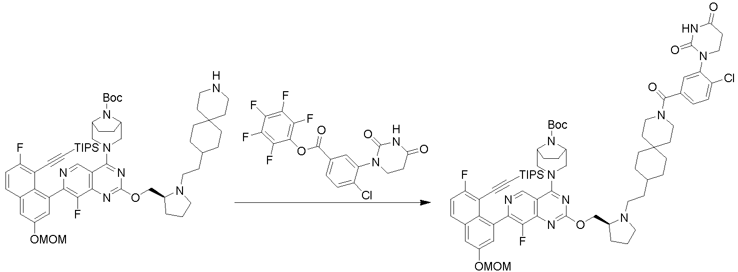

*tert-*Butyl 3-(2-(((*S*)-1-(2-(3-azaspiro[5.5 Jundecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-*d*|pyrimidin-4-yl)-3,8-diazabicyclo[3.2.1]octane-8-carboxylate (30 mg, 0.029 mmol) was dissolved in DMF (0.5 mL), and then pentafluorophenyl 4-chloro-3-(2,4-dioxotetrahydropyrimidin-1(2*H*)-yl)benzoate (16 mg, 0.035 mmol) and DIEA (12 mg, 0.087 mmol) were added. The reaction liquid was stirred at 50 °C overnight. Water (20 mL) was added to the reaction liquid, and the mixture was extracted with ethyl acetate (20 mL × 2). The organic phases were combined, washed with saturated brine (30 mL × 3), dried over anhydrous sodium sulfate, and filtered. The filtrate was concentrated, and the resulting crude product was purified by silica gel column chromatography (DCM:MeOH = 100:1 to 5:1) to give *tert*-butyl 3-(2-(((S)-1-(2-(3-(4-chloro-3-(2,4-dioxotetrahydropyrimidin-1(2*H*)-yl)benzoyl)-3-azaspiro[5.5]undecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺= 636.9.

### Step 8: preparation of 1-(5-(9-(2-((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)ethyl)-3-azaspiro [5.5]undecane-3-carbonyl)-2-chlorophenyl)dihydropyrimidine-2,4(1H,3H)-dione

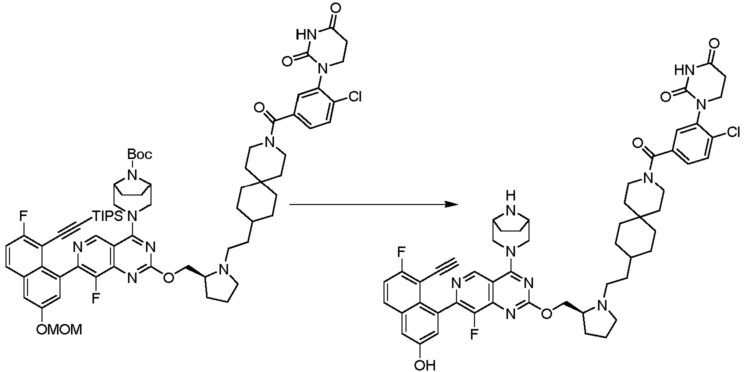

Cesium fluoride (12 mg, 0.078 mmol) was added to a solution of tert-butyl 3-(2-(((S)-1-(2-(3-(4-chloro-3-(2,4-dioxotetrahydropyrimidin-1(2*H*)-yl)benzoyl)-3-azaspiro[5.5]undecan-9-yl)ethyl)pyrrolidin-2-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (20 mg, 0.016 mmol) in DMF (1 mL), and the mixture was stirred at room temperature for 30 min. Ethyl acetate and water were added for dilution, and the organic phase was separated and concentrated. A mixture of 1,4-dioxane (2 mL) and HCl/1,4-dioxane (6 N, 1 mL) was added to the resulting product at room temperature. The reaction liquid was reacted for 0.5 h and directly concentrated under reduced pressure, and the resulting crude product was purified by Pre-HPLC to give 1-(5-(9-(2-((*S*)-2-(((4-(3,8-diazabicyclo[3.2.1]octan-3-yl)-7-(8-ethyny1-7-fluoro-3-hydroxynaphthalen-1-yl)-8-fluoropyrido[4,3-*d*]pyrimidin-2-yl)oxy)methyl)pyrrolidin-1-yl)ethyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 972.1.

¹H NMR (400 MHz, CD₃OD) δ 8.71 (s, 1H), 8.55 - 8.51 (m, 2H), 8.13 - 8.08 (m, 1H), 8.06 - 7.98 (m, 1H), 7.87 - 7.81 (m, 1H), 7.74 - 7.65 (m, 1H), 7.35 - 7.27 (m, 2H), 7.17 (s, 1H), 4.54 - 4.32 (m, 4H), 3.98 - 3.76 (m, 9H), 3.68 - 3.55 (m, 3H), 3.37 (s, 1H), 3.14 - 3.05 (m, 2H), 2.92 - 2.84 (m, 2H), 2.60 - 2.50 (m, 1H), 2.06 - 1.82 (m, 8H), 1.70 - 1.55 (m, 6H), 1.37 - 1.26 (m, 9H).

### Example 33: 1-(5-(9-((4-((3-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)bicyclo[1.1.1]pentan-1-yl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of methyl 3-(hydroxymethyl)bicyclo[1.1.1]pentane-1-carboxylate

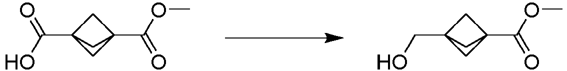

3-(Methoxycarbonyl)bicyclo[1.1.1]pentane-1-carboxylic acid (2 g, 11.75 mmol) was dissolved in THF (15 mL), and BH₃/THF (17.6 mL, 17.6 mmol, 1 M) was added. The reaction liquid was reacted at 0 °C for 3 h. After the reaction was completed, the reaction was quenched with methanol, and the mixture was directly concentrated under reduced pressure to give methyl 3-(hydroxymethyl)bicyclo[1.1.1]pentane-1-carboxylate.

¹H NMR (400 MHz, CDCl₃) δ 3.68 (s, 3H), 3.64 - 3.60 (m, 3H), 2.04 - 1.97 (m, 6H).

### Step 2: preparation of tert-butyl 3-(7-chloro-8-fluoro-2-((3-(methoxycarbonyl)bicyclo[1.1.1]pentan-1-yl)methoxy)pyrido[4,3-d]pyrimidin-4-yl)-3,8-diazabicyclo[3.2.1]octane-8-carboxylate

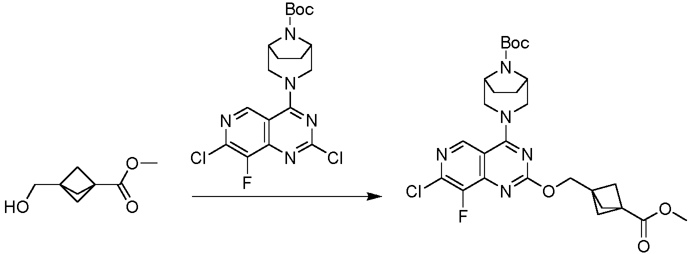

*tert*-Butyl 3-(2,7-dichloro-8-fluoropyrido[4,3-d]pyrimidin-4-yl)-3,8-diazabicyclo[3.2.1]octane-8-carboxylate (0.36 g, 0.85 mmol) and methyl 3-(hydroxymethyl)bicyclo[1.1.1]pentane-1-carboxylate (0.2 g, 1.28 mmol) were dissolved in THF (10 mL), and Cs₂CO₃ (0.73 g, 2.55 mmol) was added. The reaction liquid was heated to 80 °C and reacted for 16 h. After the reaction was completed, the mixture was cooled to room temperature. Water (50 mL) was added to quench the reaction, and the mixture was extracted with ethyl acetate (30 mL × 3). The organic phases were combined, washed with a saturated NaCl solution, dried over anhydrous Na₂SO₄, and filtered. The filtrate was concentrated to give a crude product, which was purified by silica gel column chromatography (PE:EA = 5:2) to give *tert-butyl* 3-(7-chloro-8-fluoro-2-((3-(methoxycarbonyl)bicyclo[1.1.1] pentan-1-yl)methoxy)pyrido[4,3-d]pyrimidin-4-yl)-3,8-diazabicyclo[3.2.1]octane-8-carboxylate.

LC-MS: (ESI, m/z): [M+H]⁺ = 548.1.

### Step 3: preparation of tert-butyl 3-(7-chloro-8-fluoro-2-((3-(hydroxymethyl)bicyclo[1.1.1]pentan-1-yl)methoxy)pyrido[4,3-d]pyrimidin-4-yl)-3,8-diazabicyclo[3.2.1]octane-8-carboxylate

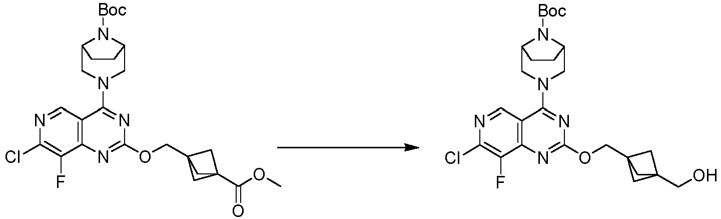

*tert-*Butyl 3-(7-chloro-8-fluoro-2-((3-(methoxycarbonyl)bicyclo[1.1.1]pentan-1-yl)methoxy)pyrido[4,3-*d*]pyrimidin-4-yl)-3,8-diazabicyclo[3.2.1]octane-8-carboxylate (300 mg, 0.55 mmol) was dissolved in THF (5 mL), and then LAH/THF (0.7 mL, 0.7 mmol, 1 M) was added. The reaction liquid was stirred at 0 °C to room temperature for 2 h. Potassium sodium tartrate tetrahydrate was added to quench the reaction, and the mixture was filtered. The filtrate was concentrated to give *tert-*butyl 3-7-chloro-8-fluoro-2-((3-(hydroxymethyl) bicyclo[1.1.1]pentan-1-yl)methoxy)pyrido[4,3-*d*]pyrimidin-4-yl)-3,8-diazabicyclo[3.2.1]octane-8-carboxylate.

LC-MS: (ESI, m/z): [M+H]⁺ = 520.1.

### Step 4: preparation of tert-butyl 3-(8-fluoro-7-(7-fluoro-3-(methoxymethoxy)-8-((triisopropylsilyl)ethynyl)naphthalen-1-yl)-2-((3-(hydroxymethyl)bicyclo[1.1.1]pentan-1-yl)methoxy)pyrido[4,3-d]pyrimidin-4-yl)-3,8-diazabicyclo[3.2.1]octane-8-carboxylate

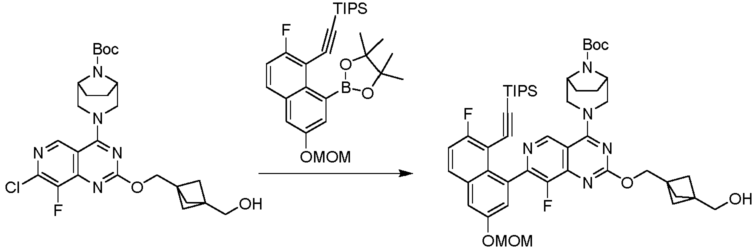

((2-Fluoro-6-(methoxymethoxy)-8-(4,4,5,5-tetramethyl-1,3,2-dioxaborolan-2-yl)naphthalen-1-yl)ethynyl)triisopropylsilane (71 mg, 0.14 mmol), cesium carbonate (117 mg, 0.36 mmol), and PdCl₂(dtbpf) (5 mg, 0.012 mmol) were added to a solution of *tert-*butyl 3-(7-chloro-8-fluoro-2-((3-(hydroxymethyl)bicyclo[1.1.1]pentan-1-yl)methoxy)pyrido[4,3-d]pyrimidin-4-yl)-3,8-diazabicyclo[3.2.1] octane-8-carboxylate (60 mg, 0.12 mmol) in 1,4-dioxane/H₂O (1 mL/0.2 mL). The mixture was reacted at 80 °C overnight under N₂ atmosphere. The reaction liquid was filtered. The filtrate was concentrated, and the resulting crude product was purified by silica gel column chromatography (DCM:MeOH = 100:1 to 20:1) to give *tert-*butyl 3-(8-fluoro-7-(7-fluoro-3-(methoxymethoxy)-8-((triisopropylsilyl)ethynyl)naphthalen-1-yl)-2-((3-(hydroxymethyl)bicyclo[1.1.1]pentan-1-yl)methoxy)pyrido[4,3-d]pyrimidin-4-yl)-3,8-diazabicyclo[3.2.1]octane-8-carboxylate.

LC-MS: (ESI, m/z): [M+H]⁺ = 870.4.

### Step 5: preparation of tert-butyl 3-(8-fluoro-7-(7-fluoro-3-(methoxymethoxy)-8-((triisopropylsilyl)ethynyl)naphthalen-1-yl)-2-((3-formylbicyclo[1.1.1]pentan-1-yl)methoxy)pyrido[4,3-d]pyrimidin-4-yl)-3,8-diazabicyclo[3.2.1]octane-8-carboxylate

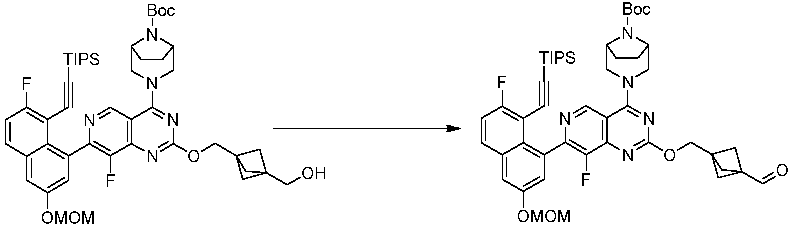

Dess-Martin reagent (30 mg, 0.068 mmol) was added to a solution of *tert*-butyl 3-(8-fluoro-7-(7-fluoro-3-(methoxymethoxy)-8-((triisopropylsilyl)ethynyl)naphthalen-1-yl)-2-((3-(hydroxymethyl)bicyclo[1.1.1]pentan-1-yl)methoxy)pyrido[4,3-*d*]pyrimidin-4-yl)-3,8-diazabicyclo[3.2.1]octane-8-carboxylate (50 mg, 0.057 mmol) in DCM (3 mL). The mixture was reacted for 1 h, filtered, and concentrated, and the resulting crude product was purified by silica gel column chromatography (DCM:MeOH = 100:1 to 5:1) to give *tert-butyl* 3-(8-fluoro-7-(7-fluoro-3-(methoxymethoxy)-8-((triisopropylsilyl)ethynyl)naphthalen-1-yl)-2-((3-formylbicyclo[1.1.1]pentan-1-yl)methoxy)pyrido[4,3-*d*]pyrimidin-4-yl)-3,8-diazabicyclo[3.2.1]octane-8-carboxylate.

LC-MS: (ESI, m/z): [M+H]⁺ = 868.2.

### Step 6: preparation of tert-butyl 3-(2-((3-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)blcyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

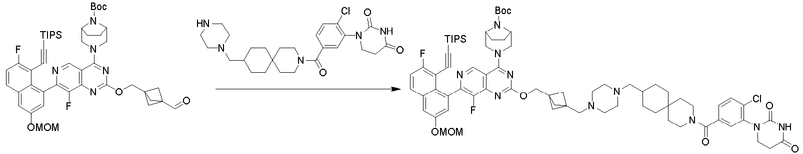

Tetraisopropyl titanate (50 mg, 0.18 mmol) was added to a mixed solution of *tert*-butyl 3-(8-fluoro-7-(7-fluoro-3-(methoxymethoxy)-8-((triisopropylsilyl)ethynyl)naphthalen-1-yl)-2-((3-formylbicyclo[1.1.1]pentan-1-yl)methoxy)pyrido[4,3-d]pyrimidin-4-yl)-3,8-diazabicyclo[3.2.1]octane-8-carboxylate (10 mg, 0.012 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (7 mg, 0.014 mmol) in tetrahydrofuran/methanol (0.5 mL/0.5 mL). The above mixture was first stirred at 70 °C for 2 h, and then cooled to 0 °C. NaBH(OAc)₃ (8 mg, 0.036 mmol) was added, and then the mixture was reacted at room temperature for 2 h. After the reaction was completed, H₂O (10 mL) was added to quench the reaction, and the mixture was extracted with ethyl acetate (30 mL × 3). The combined organic phase was dried over anhydrous sodium sulfate, filtered, and concentrated in vacuum. The crude product was purified by silica gel column chromatography (DCM:MeOH = 20:1) to give *tert-*butyl 3-(2-((3-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)bicyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 677.4.

### Step 7: preparation of 1-(5-(9-((4-((3-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)bicyclo[1.1.1]pentan-1-yl)methyl) piperazin-1-yl)methyl)-3-azaspiro[5.5]undecane-3-carbonyl)-2-chlorophenyl)dihydropyrimidine-2,4(1H,3H)-dione

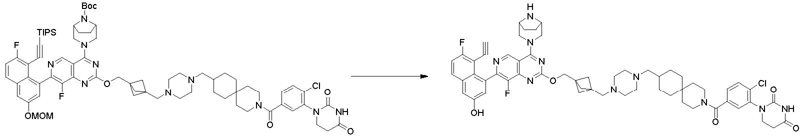

Cesium fluoride (12 mg, 0.078 mmol) was added to a solution of *tert-*butyl 3-(2-((3-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)bicyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate (15 mg, 0.011 mmol) in DMF (1 mL). The reaction liquid was stirred at room temperature overnight and diluted with ethyl acetate and water. The organic phase was separated and concentrated under reduced pressure. A mixture of 1,4-dioxane (2 mL) and HCl/1,4-dioxane (6 N, 1 mL) was added to the product obtained above at room temperature. The reaction liquid was reacted for another 0.5 h and directly concentrated under reduced pressure, and the resulting crude product was purified by Pre-HPLC to give 1-(5-(9-((4-((3-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)bicyclo[1.1.1]pentan-1-yl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 1053.1.

¹H NMR (400 MHz, CD₃OD) δ 9.03 (s, 1H), 8.61 - 8.31 (br, 1H), 7.87 (dd, *J =* 9.2, 5.7 Hz, 1H), 7.64 (d, *J =* 8.2 Hz, 1H), 7.53 (d, *J =* 1.8 Hz, 1H), 7.44 - 7.30 (m, 3H), 7.20 (d, *J* = 2.5 Hz, 1H), 4.73 (d, *J =* 13.4 Hz, 2H), 4.55 (d, *J =* 11.9 Hz, 1H), 4.46 (d, *J =* 11.9 Hz, 1H), 4.12 - 4.04 (m, 2H), 3.91 - 3.68 (m, 6H), 3.46 - 3.38 (m, 2H), 3.36 (s, 1H), 3.07 - 2.75 (m, 12H), 2.64 - 2.54 (m, 2H), 2.12 - 2.00 (m, 4H), 1.91 - 1.78 (m, 8H), 1.69 - 1.60 (m, 4H), 1.55 - 1.43 (m, 2H), 1.37 - 1.17 (m, 5H).

### Example 34: (2S,4R)-1-((S)-2-(3-(4-(2-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)ethyl)piperidin-1-yl)propanamido)-3,3-dimethylbutanoyl)-4-hydroxy-N-(4-(4-methylthiazol-5-yl)benzyl)pyrrolidine-2-carboxamide

### Step 1: preparation of benzyl 3-(4-(2-hydroxyethyl)piperidin-1-yl)propanoate

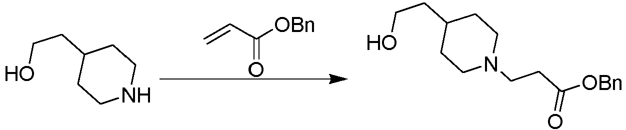

Benzyl acrylate (2.64 g, 15.5 mmol) was added to a solution of 2-(piperidin-4-yl)ethan-1-ol (2.00 g, 16.3 mmol) in acetonitrile (20 mL) at room temperature. The reaction liquid was stirred at room temperature overnight. The reaction mixture was added to brine, and the mixture was extracted with EtOAc (50 mL × 3). The organic phase was dried over Na₂SO₄ and filtered, and the filtrate was concentrated to give a crude product, which was purified by silica gel column chromatography (DCM:MeOH = 10:1) to give benzyl 3-(4-(2-hydroxyethyl)piperidin-1-yl)propanoate.

LC-MS: (ESI, m/z): [M+H]⁺ =292.2.

### Step 2: preparation of benzyl 3-(4-(2-oxoethyl)piperidin-1-yl)propanoate

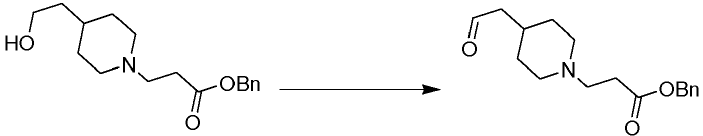

A solution of benzyl 3-(4-(2-hydroxyethyl)piperidin-1-yl)propanoate (2.00 g, 6.86 mmol) in dichloromethane (20 mL) was cooled to 0-10 °C, and Dess-Martin oxidant (5.8 g, 13.7 mmol) was added in batches. The resulting reaction liquid was stirred at room temperature for 1.5 h. An aqueous sodium thiosulfate solution and an aqueous sodium bicarbonate solution were added to quench the reaction, and the mixture was extracted with dichloromethane (20 mL × 3). The combined organic phase was washed with saturated brine, dried over anhydrous sodium sulfate, and filtered. The filtrate was concentrated in vacuum, and the crude product was purified by silica gel column chromatography (DCM:MeOH = 9:1) to give benzyl 3-(4-(2-oxoethyl)piperidin-1-yl)propanoate.

LC-MS: (ESI, m/z): [M+H]⁺ = 290.2.

### Step 3: preparation of tert-butyl 3-(2-(((S)-1-(2-(1-(3-(benzyloxy)-3-oxopropyl)piperidin-4-yl)ethyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

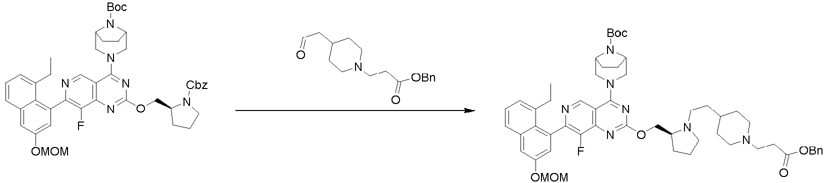

*tert-*Butyl 3-(2-(((*S*)-1-((benzyloxy)carbonyl)pyrrolidin-2-yl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate (200 mg, 0.248 mmol) was dissolved in ethanol/acetic acid (2 mL/0.1 mL), and then palladium on carbon (100 mg) was added. The mixture was purged three times under hydrogen atmosphere and reacted at 10 °C for 1 h. The reaction liquid was filtered through celite, and the filtrate was directly used in the next step. The filtrate was cooled to 0-5 °C, NaBH(OAc)₃ (158 mg, 0.744 mmol) was added, and then benzyl 3-(4-(2-oxoethyl)piperidin-1-yl)propanoate (79 mg, 0.273 mmol) was added. The mixture was stirred at 15 °C for 30 min. After the reaction was completed, an aqueous sodium bicarbonate solution was added, and the mixture was extracted with ethyl acetate (30 mL × 3). The combined organic layer was washed with brine, dried over anhydrous sodium sulfate, and filtered. The filtrate was concentrated under reduced pressure, and the crude product was purified by a preparative thin-layer plate (DCM:MeOH = 9:1) to give *tert-*butyl 3-(2-(((*S*)-1-(2-(1-(3-(benzyloxy)-3-oxopropyl)piperidin-4-yl)ethyl)pyrrolidin-2-yl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺=473.8.

### Step 4: preparation of 3-(4-(2-((S)-2-(((4-(8-(tert-butoxycarbonyl)-3,8-diazabicyclo[3.2.1]octan-3-yl)-7-(8-ethyl-3-(methoxymethoxy)naphthalen-1-yl)-8-fluoropyrido [4,3-d]pyrimidin-2-yl)oxy)methyl)pyrrolidin-1-yl)ethyl)piperidin-1-yl)propanoic acid

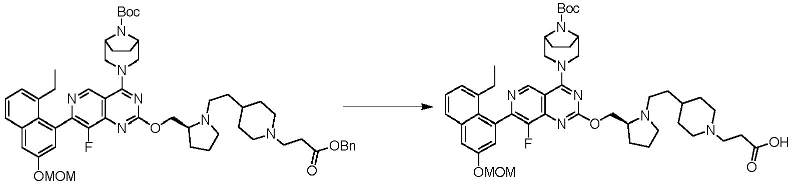

*tert-*Butyl 3-(2-(((*S*)-1-(2-(1-(3-(benzyloxy)-3-oxopropyl)piperidin-4-yl)ethyl)pyrrolidin-2-yl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate (130 mg, 0.137 mmol) was dissolved in ethanol (2 mL), and palladium on carbon (80 mg) was added. The mixture was purged three times under hydrogen atmosphere and stirred at 30 °C for 2 h. The reaction liquid was filtered through celite, and the filtrate was concentrated. The resulting crude product was purified by a preparative thin-layer plate (DCM:MeOH = 9:1) to give 3-(4-(2-((*S*)-2-(((4-(8-(*tert*-butoxycarbonyl)-3,8-diazabicyclo[3.2.1]octan-3-yl)-7-(8-ethyl-3-(methoxymethoxy)naphthalen-1-yl)-8-fluoropyrido[4,3-*d*]pyrimidin-2-yl)oxy)methyl)pyrrolidin-1-yl)ethyl)piperidin-1-yl)propanoic acid.

LC-MS: (ESI, m/z): [M-H]⁻ = 854.4.

### Step 5: preparation of tert-butyl 3-(7-(8-ethyl-3-(methoxymethoxy)naphthalen-1-yl)-8-fluoro-2-(((S)-1-(2-(1-(3-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)ethyl)pyrrolidin-2-yl)methoxy)pyrido[4,3-d]pyrimidin-4-yl)-3,8-diazabicyclo[3.2.1]octane-8-carboxylate

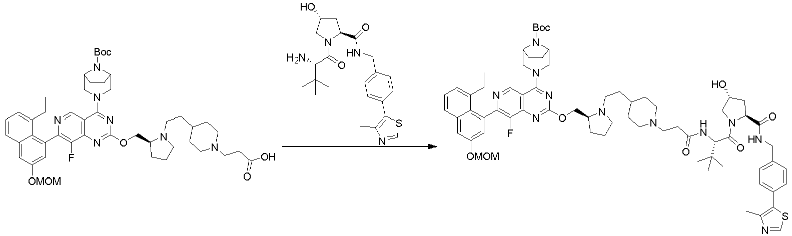

3-(4-(2-((S)-2-(((4-(8-(tert-Butoxycarbonyl)-3,8-diazabicyclo[3.2.1]octan-3-yl)-7-(8-ethyl-3-(methoxymethoxy)naphthalen-1-yl)-8-fluoropyrido[4,3-d]pyrimidin-2-yl)oxy)methyl)pyrrolidin-1-yl)ethyl)piperidin-1-yl)propanoic acid (100 mg, 0.058 mmol) and HATU (67 mg, 0.176 mmol) were dissolved in DMF (2 mL). The mixture was stirred for 3 min, and DIEA (45 mg, 0.351 mmol) and (2*S*,4*R*)-1-((*S*)-2-amino-3,3-dimethylbutanoyl)-4-hydroxy-*N*-(4-(4-methylthiazol-5-yl)benzyl)pyrrolidine-2-carboxamide (60 mg, 0.140 mmol) were added sequentially. The reaction liquid was stirred at 25 °C for 1 h. After the reaction was completed, the mixture was diluted with water (20 mL) and extracted with ethyl acetate (15 mL × 3). The organic phase was washed with brine, dried over anhydrous sodium sulfate, and filtered, and the filtrate was concentrated. The resulting crude product was purified by a preparative thin-layer plate (DCM:MeOH = 7:1) to give *tert*-butyl 3-(7-(8-ethyl-3-(methoxymethoxy)naphthalen-1-yl)-8-fluoro-2-(((*S*)-1-(2-(1-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)ethyl)pyrrolidin-2-yl)methoxy)pyrido[4,3-*d*]pyrimidin-4-yl)-3,8-diazabicyclo[3.2.1]octane-8-carboxylate.

LC-MS: (ESI, m/z): [M/2+H]⁺= 634.9.

### Step 6: preparation of (2S,4R)-1-((S)-2-(3-(4-(2-((S)-2-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)pyrrolidin-1-yl)ethyl)piperidin-1-yl)propanamido)-3,3-dimethylbutanoyl)-4-hydroxy-N-(4-(4-methylthiazol-5-yl)benzyl)pyrrolidine-2-carboxamide

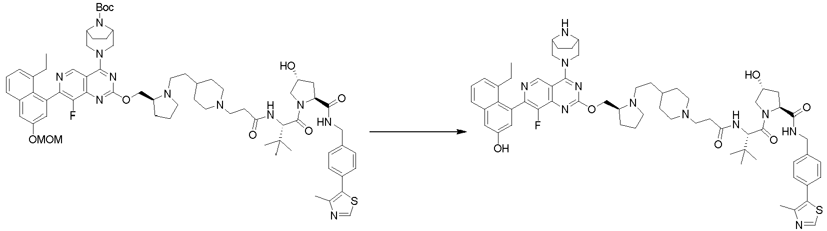

*tert-*Butyl 3-(7-(8-ethyl-3-(methoxymethoxy)naphthalen-1-yl)-8-fluoro-2-(((*S*)-1-(2-(1-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)ethyl)pyrrolidin-2-yl)methoxy)pyrido[4,3-d]pyrimidin-4-yl)-3,8-diazabicyclo[3.2.1]octane-8-carboxylate (100 mg, 0.082 mmol) was dissolved in DCM (2 mL), and then TFA (1 mL) was added. The mixture was stirred at 15 °C for 1 h. The reaction liquid was concentrated, and the residue was diluted with DCM (20 mL) and then adjusted to pH > 7 with an aqueous NaHCO₃ solution. The organic phase was separated, washed with a saturated NaCl solution, dried over anhydrous Na₂SO₄, and filtered. The filtrate was concentrated, and the resulting crude product was purified by Pre-HPLC to give (2*S*,4*R*)-1-((*S*)-2-(3-(4-(2-((*S*)-2-(((4-(3,8-diazabicyclo[3.2.1]octan-3-yl)-7-(8-ethyl-3-hydroxynaphthalen-1-yl)-8-fluoropyrido[4,3-*d*]pyrimidin-2-yl)oxy)methyl)pyrrolidin-1-yl)ethyl)piperidin-1-yl)propanamido)-3,3-dimethylbutanoyl)-4-hydroxy-*N*-(4-(4-methylthiazol-5-yl)benzyl)pyrrolidine-2-carboxamide.

LC-MS: (ESI, m/z): [M+H]⁺ = 1125.2.

¹H NMR (400 MHz, CD₃OD) δ 9.12 (s, 1H), 8.87 (s, 1H), 7.63 (d, *J* = 7.9 Hz, 1H), 7.47 -7.28 (m, 6H), 7.17 (d, *J* = 7.1 Hz, 1H), 7.01 (t, *J* = 2.5 Hz, 1H), 4.83 - 4.72(m, 3H), 4.59 - 4.44 (m, 4H), 4.37 - 4.31(m, 1H), 4.13 (s, 2H), 4.00 - 3.75 (m, 4H), 3.68 - 3.61 (m, 2H),3.41 - 3.33 (m, 2H), 3.23 - 3.07 (m, 3H), 2.78 - 2.61 (m, 4H),2.47 - 2.43 (m, 3H), 2.40 - 2.17 (m, 4H), 2.15 - 1.98 (m, 7H), 1.95 - 1.88 (m, 2H), 1.57 - 1.44 (m, 3H), 1.57 - 1.44 (m, 2H), 1.38 - 1.25 (m, 4H), 1.02 (d, J = 12.6 Hz, 9H), 0.93 - 0.84 (m, 3H).

### Example 35: (2S,4R)-1-((S)-2-(3-(4-(((S)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)methyl)piperidin-1-yl)propanamido)-3,3-dimethylbutanoyl)-4-hydroxy-N-(4-(4-methylthiazol-5-yl)benzyl)pyrrolidine-2-carboxamide

### Step 1: preparation of benzyl 3-(4-(hydroxymethyl)piperidin-1-yl)propanoate

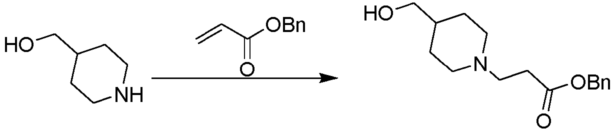

Benzyl acrylate (4.44 g, 0.027 mmol) was added to a solution of piperidin-4-ylmethanol (3.00 g, 0.026 mmol) in acetonitrile (30 mL) at room temperature. The reaction liquid was stirred at room temperature overnight. Brine was added to the reaction mixture, and the resulting mixture was extracted with EtOAc (50 mL × 3). The organic phase was dried over Na₂SO₄ and filtered. The filtrate was concentrated, and the resulting crude product was purified by silica gel column chromatography (DCM:MeOH = 9:1) to give benzyl 3-(4-(hydroxymethyl)piperidin-1-yl)propanoate.

LC-MS: (ESI, m/z): [M+H]⁺ = 278.2.

### Step 2: preparation of benzyl 3-(4-formylpiperidin-1-yl)propanoate

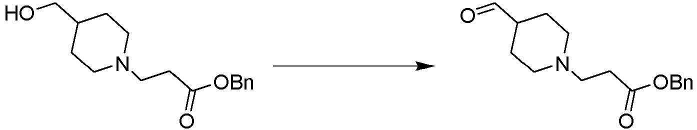

A solution of benzyl 3-(4-(hydroxymethyl)piperidin-1-yl)propanoate (3.0 g, 10.83 mmol) in dichloromethane (50 mL) was cooled to 0-10 °C, and Dess-Martin oxidant (9.18 g, 21.66 mmol) was added in batches. The resulting reaction liquid was stirred at room temperature for 1.5 h. An aqueous sodium thiosulfate solution and an aqueous sodium bicarbonate solution were added to the reaction liquid to quench the reaction, and the mixture was extracted with dichloromethane (30 mL × 2). The combined organic phase was washed with saturated brine, dried over anhydrous sodium sulfate, and filtered. The filtrate was concentrated in vacuum, and the crude product was purified by silica gel column chromatography (PE:EtOAc = 2:1) to give benzyl 3-(4-formylpiperidin-1-yl)propanoate.

LC-MS: (ESI, m/z): [M+H]⁺ = 276.1.

### Step 3: preparation of tert-butyl 3-(2-(((S)-1-((1-(3-(benzyloxy)-3-oxopropyl)piperidin-4-yl)methyl)pyrrolidin-2-yl)methoxy)-7-chloro-8-fluoropyrido[4,3-d]pyrimidin-4-yl)-3,8-diazabicyclo[3.2.1]octane-8-carboxylate

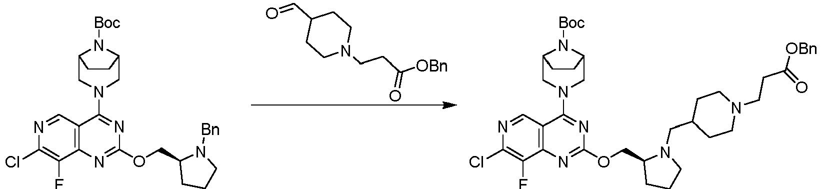

*tert*-Butyl 3-(2-(((*S*)-1-benzylpyrrolidin-2-yl)methoxy)-7-chloro-8-fluoropyrido[4,3-*d*]pyrimidin-4-yl)-3,8-diazabicyclo[3.2.1]octane-8-carboxylate (0.55 g, 0.94 mmol) was dissolved in DCM (10 mL), and 1-chloroethyl chloroformate (0.27 g, 1.87 mmol) and DIEA (0.24 g, 1.87 mmol) were added. The mixture was reacted at room temperature for 1 h and concentrated under reduced pressure to 5 mL. MeOH (3 mL) was added to the concentrate for dissolution, and the mixture was heated to 50 °C and reacted for 30 min. After the reaction was completed, the mixture was concentrated to two-thirds volume, and benzyl 3-(4-formylpiperidin-1-yl)propanoate (0.51 g, 1.87 mmol) was added. The mixture was stirred at room temperature for 2 min, and then NaBH(OAc)₃ (597 g, 2.82 mmol) was added at 0-5 °C. The reaction liquid was warmed to room temperature and reacted for 30 min. Water was first added, and then the mixture was extracted with ethyl acetate (50 mL × 3). The organic phase was washed with a saturated sodium chloride solution, dried over anhydrous sodium sulfate, and filtered, and the filtrate was concentrated. The resulting crude product was purified by silica gel column chromatography (PE:EA = 2:1) to give *tert*-butyl 3-(2-(((S)-1-((1-(3-(benzyloxy)-3-oxopropyl)piperidin-4-yl)methyl)pyrrolidin-2-yl)methoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 752.3.

### Step 4: preparation of 3-(4-(((S)-2-(((4-(8-(tert-butoxycarbonyl)-3,8-diazabicyclo[3.2.1]octan-3-yl)-7-chloro-8-fluoropyrido[4,3-d]pyrimidin-2-yl)oxy)methyl)pyrrolidin-1-yl)methyl)piperidin-1-yl)propanoic acid

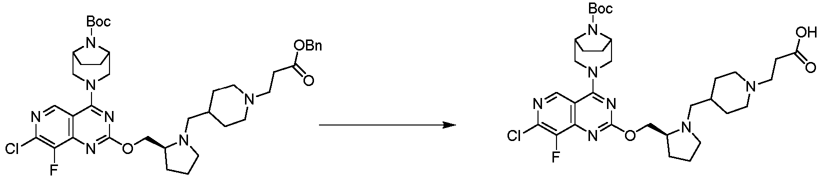

*tert-*Butyl 3-(2-(((*S*)-1-((1-(3-(benzyloxy)-3-oxopropyl)piperidin-4-yl)methyl)pyrrolidin-2-yl)methoxy)-7-chloro-8-fluoropyrido[4,3-*d*]pyrimidin-4-yl)-3,8-diazabicyclo[3.2.1]octane-8-carboxylate (300 mg, 0.40 mmol) was dissolved in 1,4-dioxane (3 mL), and an aqueous sodium hydroxide solution (1.05 mL, 2 N) was added. The mixture was stirred at room temperature for 2 h and extracted with ethyl acetate. The organic phase was concentrated under reduced pressure to give 3-(4-(((*S*)-2-(((4-(8-(*tert-*butoxycarbonyl)-3,8-diazabicyclo[3.2.1]octan-3-yl)-7-chloro-8-fluoropyrido[4,3-*d*]pyrimidin-2-yl)oxy)methyl)pyrrolidin-1-yl)methyl)piperidin-1-yl)propanoic acid, which was directly used in the next step.

LC-MS: (ESI, m/z): [M+H]⁺ = 662.3.

### Step 5: preparation of 3-(4-(((S)-2-(((4-(8-(tert-butoxycarbonyl)-3,8-diazabicyclo[3.2.1]octan-3-yl)-8-fluoro-7-(7-fluoro-3-(methoxymethoxy)-8-((triisopropylsilyl)ethynyl)naphthalen-1-yl)pyrido [4,3-d]pyrimidin-2-yl)oxy)methyl)pyrrolidin-1-yl)methyl)piperidin-1-yl)propanoic acid

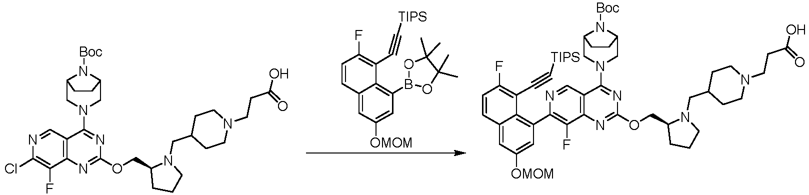

((2-Fluoro-6-(methoxymethoxy)-8-(4,4,5,5-tetramethyl-1,3,2-dioxaborolan-2-yl)naphthalen-1-yl)ethynyl)
triisopropylsilane (256 mg, 0.50 mmol), cesium carbonate (326 mg, 1.00 mmol), and cataXium A Pd G3 (29 mg, 0.04 mmol) were added to a solution of 3-(4-(((S)-2-(((4-(8-(*tert*-butoxycarbonyl)-3,8-diazabicyclo[3.2.1]octan-3-yl)-7-chloro-8-fluoropyrido[4,3-d]pyrimidin-2-yl)oxy)methyl)pyrrolidin-1-yl)methyl)piperidin-1-yl)propanoic acid (264 mg, 0.40 mmol) in 1,4-dioxane/H₂O (4.0 mL/1 mL). The mixture was reacted at 75 °C overnight under N₂ atmosphere and filtered. The filtrate was concentrated, and the resulting crude product was purified by silica gel column chromatography (DCM:MeOH = 100:1 to 20:1) to give 3-(4-(((*S*)-2-(((4-(8-(*tert*-butoxycarbonyl)-3,8-diazabicyclo[3.2.1]octan-3-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)pyrrolidin-1-yl)methyl)piperidin-1-yl)propanoic acid.

LC-MS: (ESI, m/z): [M+H]⁺ = 1012.9.

### Step 6: preparation of tert-butyl 3-(8-fluoro-7-(7-fluoro-3-(methoxymethoxy)-8-((triisopropylsilyl)ethynyl)naphthalen-1-yl)-2-(((S)-1-((1-(3-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)methyl)pyrrolidin-2-yl)methoxy)pyrido [4,3-d]pyrimidin-4-yl)-3,8-diazabicyclo[3.2.1]octane-8-carboxylate

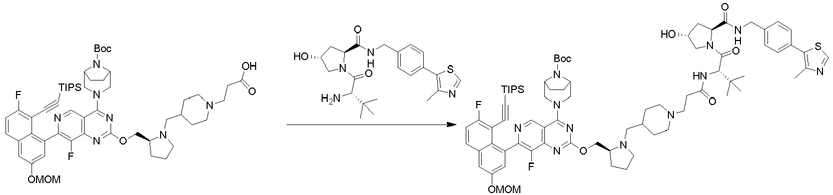

3-(4-(((*S*)-2-(((4-(8-(*tert*-Butoxycarbonyl)-3,8-diazabicyclo[3.2.1]octan-3-yl)-8-fluoro-7-(7-fluoro-3-(methoxymethoxy)-8-((triisopropylsilyl)ethynyl)naphthalen-1-yl)pyrido[4,3-*d*]pyrimidin-2-yl)oxy)methyl)pyrrolidin-1-yl)methyl)piperidin-1-yl)propanoic acid (220 mg, 0.22 mmol) and HATU (99 mg, 0.26 mmol) were dissolved in DMF (4 mL), and the mixture was stirred for 3 min. DIEA (85 mg, 0.66 mmol) and (2*S*,4*R*)-1-((*S*)-2-amino-3,3-dimethylbutanoyl)-4-hydroxy-N-(4-(4-methylthiazol-5-yl)benzyl)pyrrolidine-2-carboxamide (122 mg, 0.26 mmol) were added sequentially, and the reaction liquid was stirred at 25 °C for 1 h. Water (15 mL) was added to the reaction mixture for dilution, and the resulting mixture was extracted with ethyl acetate (10 mL × 2). The organic phase was washed with brine, dried over anhydrous sodium sulfate, and filtered, and the filtrate was concentrated. The resulting crude product was purified by silica gel column chromatography (DCM:MeOH = 20:1) to give *tert-butyl* 3-(8-fluoro-7-(7-fluoro-3-(methoxymethoxy)-8-((triisopropylsilyl)ethynyl)naphthalen-1-yl)-2-(((S)-1-((1-(3-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)methyl)pyrrolidin-2-yl)methoxy)pyrido[4,3-*d*]pyrimidin-4-yl)-3,8-diazabicyclo[3.2.1]octane-8-carboxylate.

LC-MS: (ESI, m/z): [M/2+H]⁺ = 713.1.

### Step 7: preparation of (2S,4R)-1-((S)-2-(3-(4-(((S)-2-(((4-3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)pyrrolidin-1-yl)methyl)piperidin-1-yl)propanamido)-3,3-dimethylbutanoyl)-4-hydroxy-N-(4-(4-methylthiazol-5-yl)benzyl)pyrrolidine-2-carboxamide

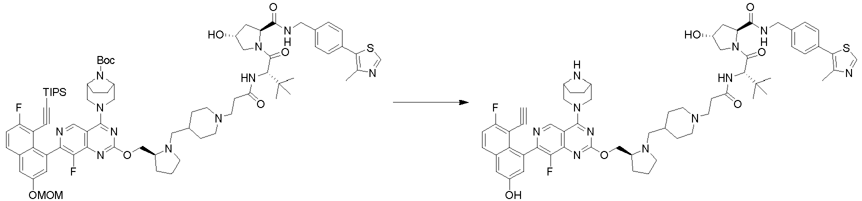

*tert*-Butyl 3-(8-fluoro-7-(7-fluoro-3-(methoxymethoxy)-8-((triisopropylsilyl)ethynyl)naphthalen-1-yl)-2-(((*S*)-1-((1-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)piperidin-4-yl)methyl)pyrrolidin-2-yl)methoxy)pyrido[4,3-*d*]pyrimidin-4-yl)-3,8-diazabicyclo[3.2.1]octane-8-carboxylate (160 mg, 0.11 mmol) was dissolved in DMF (2 mL), and CsF (83 mg, 0.55 mmol) was added at room temperature. The mixture was stirred for 30 min. Water was added, and the mixture was extracted with ethyl acetate. The organic phase was concentrated under reduced pressure. The concentrate was dissolved in 1,4-dioxane (2 mL), and then an HCl/1,4-dioxane solution (6 N, 1 mL) was added. The mixture was stirred for 0.5 h. The reaction liquid was directly concentrated under reduced pressure. The resulting crude product was purified by Pre-HPLC to give (2*S*,4*R*)-1-((*S*)-2-(3-(4-(((*S*)-2-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)pyrrolidin-1-yl)methyl)piperidin-1-yl)propanamido)-3,3-dimethylbutanoyl)-4-hydroxy-*N-*(4-(4-methylthiazol-5-yl)benzyl)pyrrolidine-2-carboxamide.

LC-MS: (ESI, m/z): [M+H]⁺ = 1124.3.

¹H NMR (400 MHz, CD₃OD) δ 9.08 (d, *J* = 2.4 Hz, 1H), 8.87 (d, *J* = 2.4 Hz, 1H), 7.91 -7.83 (m, 1H), 7.47 - 7.29 (m, 6H), 7.22 (d, *J* = 2.4 Hz, 1H), 4.81 - 4.75 (m, 3H), 4.57 - 4.30 (m, 6H), 4.28 - 4.20 (m, 2H), 4.03 - 3.86 (m, 3H), 3.78 - 3.68 (m, 1H), 3.58 - 3.40 (m, 4H), 3.27 - 3.18 (m, 3H), 3.10 - 2.64 (m, 6H), 2.48 - 2.41 (m, 3H), 2.31 - 1.85 (m, 14H), 1.67 - 1.45 (m, 2H), 1.05 - 0.96 (m, 9H).

### Example 36: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

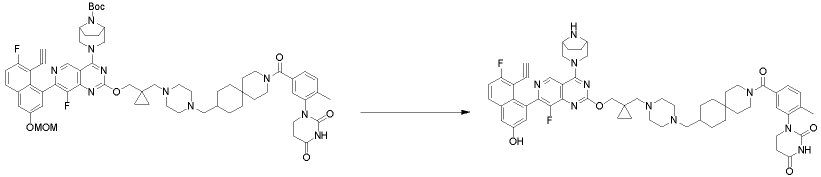

*tert-*Butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethynyl-7-fluoro-3-(methoxymethoxy) naphthalen-1-yl)-8-fluoropyrido[4,3-*d*]pyrimidin-4-yl)-3,8-diazabicyclo[3.2.1]octane-8-carboxylate (40 mg, 0.034 mmol) was dissolved in 1,4-dioxane (2 mL), and HCl/1,4-dioxane (6 N, 1 mL) was added. The mixture was reacted at room temperature for 0.5 h. The reaction liquid was concentrated, and the resulting crude product was purified by Pre-HPLC to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1008.2.

¹H NMR (400 MHz, CD₃OD) δ 9.02 (s, 1H), 8.70 - 8.34 (m, 2H), 7.90 - 7.83 (m, 1H), 7.41 (d, *J* = 7.7 Hz, 1H), 7.36 - 7.29 (m, 4H), 7.21 (d, *J=* 2.4 Hz, 1H), 4.65 (t, *J =* 13.9 Hz, 2H), 4.53 - 4.35 (m, 2H), 3.87 - 3.64 (m, 8H), 3.46 - 3.33 (m, 3H), 2.96 - 2.76 (m, 9H), 2.69 - 2.42 (m, 5H), 2.32 (s, 3H), 1.98 - 1.85 (m, 4H), 1.77 - 1.71 (m, 2H), 1.62 - 1.54 (m, 4H), 1.47 - 1.27 (m, 5H), 1.19 - 1.14 (m, 2H), 0.82 - 0.72 (m, 2H), 0.62 - 0.54 (m, 2H).

### Example 37: 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

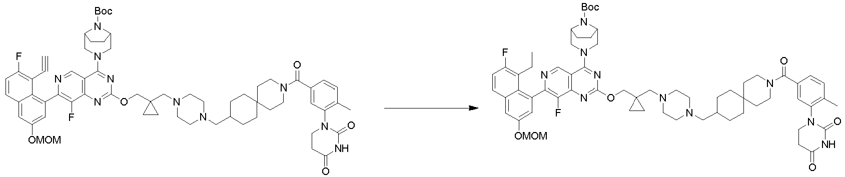

Pd/C (10 mg) was added to a solution of *tert-*butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (50 mg, 0.043 mmol) in methanol (2 mL), and the mixture was reacted at room temperature for 2 h under hydrogen atmosphere. The reaction liquid was filtered through celite, and the filtrate was concentrated to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 578.4.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

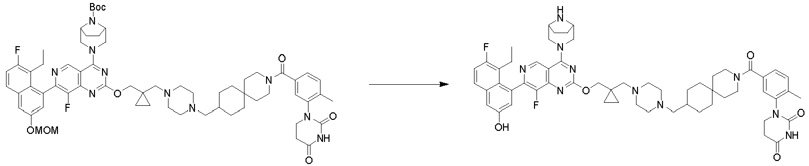

*tert-*Butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (36 mg, crude) was dissolved in 1,4-dioxane (2 mL) and HCl/1,4-dioxane (6 N, 1 mL). The mixture was reacted at room temperature for 0.5 h. The reaction liquid was directly concentrated under reduced pressure, and the resulting crude product was purified by Pre-HPLC to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 506.3.

### Example 38: 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methyl)-3,9-diazaspiro[5.5]undecane-3-carbonyl)-2-methoxyphenyl)dihydropyrimidine-2,4(1H,3H)-dione

### Step 1: preparation of tert-butyl 9-(3-(2,4-dioxotetrahydropyrimidin-1(2H)-yl)-4-methoxybenzoyl)-3,9-azaspiro[5.5]undecane-3-carboxylate

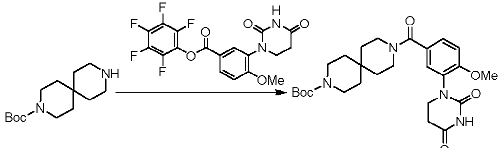

DIEA (721 mg, 5.58 mmol) and pentafluorophenyl 3-(2,4-dioxotetrahydropyrimidin-1(2*H*)-yl)-4-methoxybenzoate (800 mg, 1.86 mmol) were added to a solution of *tert*-butyl 3,9-diazaspiro[5.5]undecane-3-carboxylate (473 mg, 1.86 mmol) in dimethyl sulfoxide (8 mL). The mixture was stirred at 30 °C for 1 h. After the reaction was completed, the mixture was extracted with water (10 mL) and ethyl acetate (10 mL × 3). The organic phase was washed with brine (20 mL), dried over anhydrous Na₂SO₄, filtered, and concentrated in vacuum. The resulting crude product was purified by silica gel column chromatography (DCM:MeOH = 20:1) to give *tert-*butyl 9-(3-(2,4-dioxotetrahydropyrimidin-1(2*H*)-yl)-4-methoxybenzoyl)-3,9-azaspiro[5.5]undecane-3-carboxylate.

LC-MS: (ESI, m/z): [M+H]⁺ = 445.1.

### Step 2: preparation of 1-(2-methoxy-5-(3,9-azaspiro[5.5]undecane-3-carbonyl)phenyl)dihydropyrimidine-2,4(1H,3H)-dione

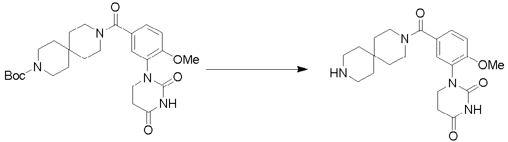

HCl/1,4-dioxane (2 mL) was added to a solution of *tert*-butyl 9-(3-(2,4-dioxotetrahydropyrimidin-1(2H)-yl)-4-methoxybenzoyl)-3,9-azaspiro[5.5]undecane-3-carboxylate (920 mg, 1.84 mmol) in 1,4-dioxane (4 mL), and the mixture was stirred at 30 °C for 1 h. After the reaction was completed, the mixture was concentrated in vacuum to give 1-(2-methoxy-5-(3,9-azaspiro[5.5]undecane-3-carbonyl)phenyl)dihydropyrimidine-2,4(1H,3H)-dione. The crude product was directly used in the next step without further purification.

LC-MS: (ESI, m/z): [M-tBu+H]⁺ = 401.1.

### Step 3: preparation of tert-butyl 4-((9-(3-(2,4-dioxotetrahydropyrimidin-1(2H)-yl)-4-methoxybenzoyl)-3,9-azaspiro[5.5]undecan-3-yl)methyl)piperidine-1-carboxylate

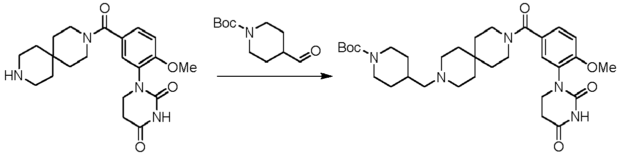

Triethylamine (51 mg, 0.5 mmol), acetic acid (0.1 mL), and *tert-*butyl 4-formylpiperidine-1-carboxylate (213 mg, 1.0 mmol) were added to a solution of 1-(2-methoxy-5-(3,9-azaspiro[5.5]undecane-3-carbonyl)phenyl)dihydropyrimidine-2,4(1H,3H)-dione (200 mg, 0.5 mmol) in 1,2-dichloromethane (3 mL). The mixture was stirred at 30 °C for 1 h, and then sodium triacetoxyborohydride (212 mg, 1.0 mmol) was added. The mixture was stirred at 30 °C for 1 h. After the reaction was completed, the mixture was extracted with water (5 mL) and ethyl acetate (5 mL × 3), dried over anhydrous Na₂SO₄, filtered, and concentrated in vacuum. The crude product was purified by silica gel column chromatography (DCM:MeOH = 10:1) to give *tert-*butyl 4-((9-(3-(2,4-dioxotetrahydropyrimidin-1(2*H*)-yl)-4-methoxybenzoyl)-3,9-azaspiro [5 .5]undecan-3-yl)methyl)piperidine-1-carboxylate.

LC-MS: (ESI, m/z): [M+H]⁺ = 598.4.

### Step 4: preparation of 1-(2-methoxy-5-(9-piperidin-4-ylmethyl)-3,9-azaspiro[5.5]undecane-3-carbonyl)phenyl)dihydropyrimidine-2,4(1H,3H)-dione

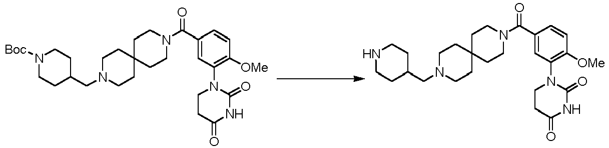

HCl/1,4-dioxane (1 mL) was added to a solution of *tert-*butyl 4-((9-(3-(2,4-dioxotetrahydropyrimidin-1(2H)-yl)-4-methoxybenzoyl)-3,9-azaspiro[5.5]undecan-3-yl)methyl)piperidine-1-carboxylate (250 mg, 0.42 mmol) in 1,4-dioxane (2 mL), and the mixture was stirred at 30 °C for 1 h. After the reaction was completed, the mixture was concentrated in vacuum to give 1-(2-methoxy-5-(9-piperidin-4-ylmethyl)-3,9-azaspiro[5.5]undecane-3-carbonyl)phenyl)dihydropyrimidine-2,4(1*H*,3*H*)-dione. The crude product was directly used in the next step without further purification.

LC-MS: (ESI, m/z): [M+H]⁺ = 498.2.

### Step 5: preparation of tert-butyl 3-(2-((1-((4-((9-(3-(2,4-dioxotetrahydropyrimidin-1(2H)-yl)-4-methoxybenzoyl)-3,9-azaspiro[5.5]undecan-3-yl)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

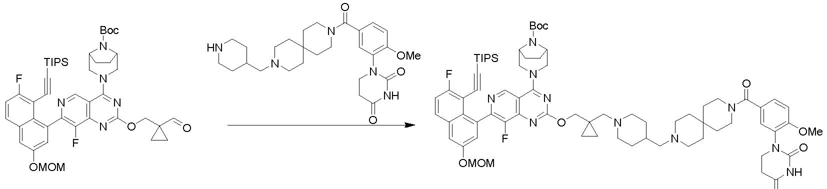

1-(2-Methoxy-5-(9-piperidin-4-ylmethyl)-3,9-azaspiro[5.5]undecane-3-carbonyl)phenyl)dihydropyrimidine-2,4(1*H*,3*H*)-dione (70 mg, 0.14 mmol) and tetraisopropyl titanate (171 mg, 0.6 mmol) were added to a solution of *tert-*butyl 3-(8-fluoro-7-(7-fluoro-3-(methoxymethoxy)-8-((triisopropylsilyl)ethynyl)naphthalen-1-yl)-2-((1-methoxycyclopropylmethoxy)pyridin[4,3-*d*]pyrimidin-4-yl)-3,8-diazabicyclo[3.2.1]octane-8-carboxylate (100 mg, 0.12 mmol) in tetrahydrofuran (1 mL) and anhydrous methanol (0.5 mL), and the mixture was stirred at 70 °C for 2 h. NaBH(OAc)₃ (100 mg, 0.48 mmol) was then added, and the mixture was stirred at 30 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert-*butyl 3-(2-((1-((4-((9-(3-(2,4-dioxotetrahydropyrimidin-1(2*H*)-yl)-4-methoxybenzoyl)-3,9-azaspiro[5.5]undecan-3-yl)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate.

LC-MS: (ESI, m/z): [M/2+H]⁺ = 662.9.

### Step 6: preparation of 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methyl)-3,9-diazaspiro[5.5]undecane-3-carbonyl)-2-methoxyphenyl)dihydropyrimidine-2,4(1H,3H)-dione

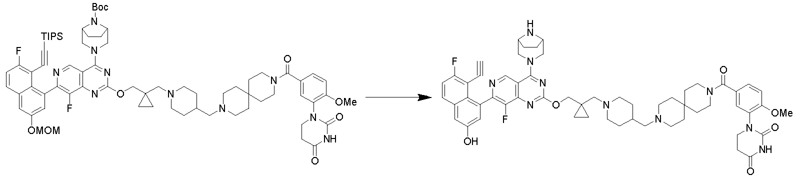

*tert*-Butyl 3-(2-((1-((4-((9-(3-(2,4-dioxotetrahydropyrimidin-1(2*H*)-yl)-4-methoxybenzoyl)-3,9-azaspiro[5.5]undecan-3-yl)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (125 mg, 0.095 mmol) was dissolved in DMF (1 mL), and CsF (73 mg, 0.48 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the residue, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)methyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 1023.5.

¹H NMR (400 MHz, DMSO-*d*₆) δ 10.32 (s, 1H), 9.03 (s, 1H), 8.19 (s, 1H), 7.99 - 7.95 (m, 1H), 7.46 (t, *J =* 9.0 Hz, 1H), 7.40 - 7.34 (m, 2H), 7.31 (d, *J* = 2.1 Hz, 1H), 7.17 - 7.13 (m, 2H), 4.50 (d, *J=* 12.2 Hz, 1H), 4.33 - 4.23 (m, 3H), 3.92 (s, 1H), 3.84 (s, 3H), 3.60 - 3.56 (m, 4H), 3.41 (s, 5H), 3.02 - 2.90 (m, 3H), 2.68 (t, *J* = 6.5 Hz, 2H), 2.30 (s, 7H), 2.09 (d, *J* = 6.3 Hz, 2H), 1.86 (t, *J =* 10.8 Hz, 2H), 1.73 - 1.67 (m, 4H), 1.63-1.57 (m, 2H), 1.47 - 1.37 (m, 9H), 1.05 - 0.99 (m, 2H), 0.65 - 0.62 (m, 2H), 0.42-0.37 (m, 2H).

### Example 39: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-(trifluoromethoxy)phenyl)dihydropyrimidine-2,4(1H,3H)-dione

### Step 1: preparation of benzyl 4-((3-(3-(2,4-dioxotetrahydropyrimidin-1(2H)-yl)-4-(trifluoromethoxy)benzoyl)-3-azaspiro[5.5]undecan-9-yl)methyl)piperazine-1-carboxylate

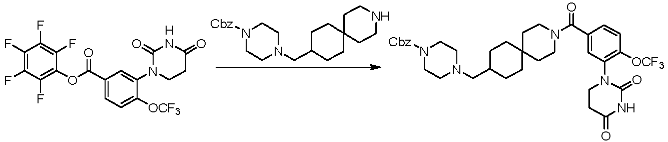

DIEA (256 mg, 1.98 mmol) and benzyl 4-((3-azaspiro[5.5]undecan-9-yl)methyl)piperazine-1-carboxylate (266 mg, 0.69 mmol) were added to a solution of pentafluorophenyl 3-(2,4-dioxotetrahydropyrimidin-1(2*H*)-yl)-4-(trifluoromethoxy)benzoate (320 mg, 0.66 mmol) in dimethyl sulfoxide (3 mL). The mixture was stirred at 30 °C for 1 h. After the reaction was completed, the mixture was extracted with water (10 mL) and ethyl acetate (10 mL × 3). The organic phase was washed with brine (20 mL), dried over anhydrous Na₂SO₄, filtered, and concentrated in vacuum. The crude product was purified by silica gel column chromatography (DCM:MeOH = 20:1) to give benzyl 4-((3-(3-(2,4-dioxotetrahydropyrimidin-1(2*H*)-yl)-4-(trifluoromethoxy)benzoyl)-3-azaspiro[5.5]undecan-9-yl)methyl)piperazine-1-carboxylate.

LC-MS: (ESI, m/z): [M+H]⁺ = 686.2.

### Step 2: preparation of 1-(5-(9-(piperazin-1-ylmethyl)-3-azaspiro[5.5]undecane-3-carbonyl)-2-(trifluoromethoxy)phenyl)dihydropyrimidine-2,4(1H,3H)-dione

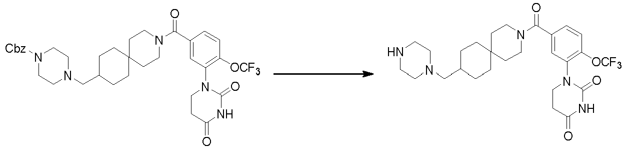

Pd/C (70 mg) was added to a solution of benzyl 4-((3-(3-(2,4-dioxotetrahydropyrimidin-1(2*H*)-yl)-4-(trifluoromethoxy)benzoyl)-3-azaspiro[5.5]undecan-9-yl)methyl)piperazine-1-carboxylate (450 mg, 0.66 mmol) in tetrahydrofuran (2 mL), and the mixture was stirred at 30 °C for 16 h under hydrogen atmosphere. The mixture was filtered and concentrated, and the crude product was purified by silica gel column chromatography (DCM:NH₃/MeOH = 10:1) to give 1-(5-(9-(piperazin-1-ylmethyl)-3-azaspiro[5.5]undecane-3-carbonyl)-2-(trifluoromethoxy)phenyl)dihydropyrimidine-2,4(1*H*,3*H*)-dione.

LC-MS: (ESI, m/z): [M+H]⁺ = 552.2.

### Step 3: preparation of tert-butyl 3-(2-((1-((4-((3-(3-(2,4-dioxotetrahydropyrimidin-1(2H)-yl)-4-(trifluoromethoxy)benzoyl)-3-azaspiro[5.5]undecan-9-yl)methyl)piperazin-1-yl)methyl)cyclopropyl) methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

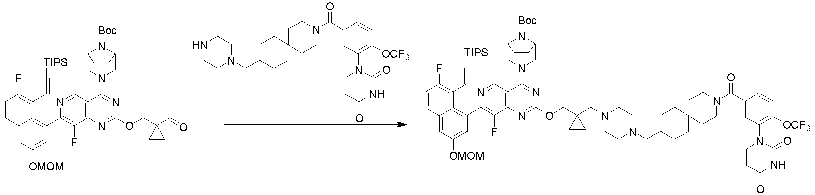

1-(5-(9-(Piperazin-1-ylmethyl)-3-azaspiro[5.5]undecane-3-carbonyl)-2-(trifluoromethoxy)phenyl)dihydropyrimidine-2,4(1*H*,3*H*)-dione (99 mg, 0.18 mmol) and tetraisopropyl titanate (171 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in tetrahydrofuran (1 mL) and anhydrous methanol (0.5 mL), and the mixture was stirred at 70 °C for 2 h. NaBH(OAc)₃ (100 mg, 0.48 mmol) was then added at 0 °C, and the mixture was stirred at 30 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert*-butyl 3-(2-((1-((4-((3-(3-(2,4-dioxotetrahydropyrimidin-1(2*H*)-yl)-4-(trifluoromethoxy)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate.

LC-MS: (ESI, m/z): [M/2+H]⁺ = 689.8.

### Step 4: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-(trifluoromethoxy)phenyl)dihydropyrimidine-2,4(1H,3H)-dione

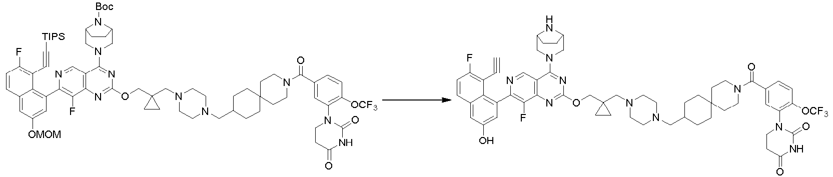

*tert*-Butyl 3-(2-((1-((4-((3-(3-(2,4-dioxotetrahydropyrimidin-1(2*H*)-yl)-4-(trifluoromethoxy)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (160 mg, 0.12 mmol) was dissolved in DMF (1 mL), and CsF (91 mg, 0.6 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-(trifluoromethoxy)phenyl)dihydropyrimidine-2,4(1*H*,3*H*)-dione.

LC-MS: (ESI, m/z): [M+H]⁺ = 1077.5.

¹H NMR (400 MHz, DMSO-*d*₆) δ 10.53 (s, 1H), 10.13 (s, 1H), 9.02 (s, 1H), 7.97 (dd, *J=* 9.2, 6.0 Hz, 1H), 7.59 (d, *J* = 1.9 Hz, 1H), 7.50 - 7.43 (m, 3H), 7.38 (d, *J* = 2.4 Hz, 1H), 7.16 (d, *J =* 2.4 Hz, 1H), 4.48 (d, *J* = 10.9 Hz, 1H), 4.27 - 4.26 (m, 3H), 3.92 (s, 1H), 3.72 - 3.54 (m, 8H), 3.30 - 3.24 (m, 3H), 2.69 - 2.67 (m, 3H), 2.38 - 2.27 (m, 9H), 2.04 (s, 2H), 1.70 - 1.62 (s, 6H), 1.53 - 1.50 (m, 3H), 1.44 - 1.26 (m, 4H), 1.14 - 0.96 (m, 4H), 0.67 - 0.59 (m, 2H), 0.44 - 0.34 (m, 2H).

### Example 40: 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)azetidin-3-yl)methyl)-3,9-diazaspiro[5.5]undecane-3-carbonyl)-2-methoxyphenyl)dihydropyrimidine-2,4(1H,3H)-dione

### Step 1: preparation of tert-butyl 3-((9-(3-(2,4-dioxotetrahydropyrimidin-1(2H)-yl)-4-methoxybenzoyl)-3,9-diazaspiro[5.5]undecan-3-yl)methyl)azetidine-1-carboxylate

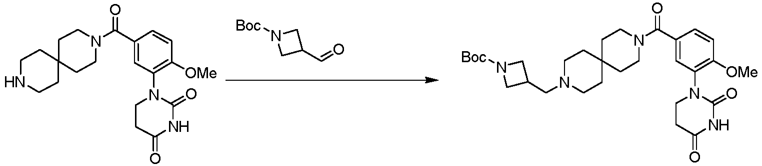

Triethylamine (51 mg, 0.5 mmol), acetic acid (0.1 mL), and *tert*-butyl 3-formylazetidine-1-carboxylate (185 mg, 1.0 mmol) were added to a solution of 1-(2-methoxy-5-(3,9-diazaspiro[5.5]undecane-3-carbonyl)phenyl)dihydropyrimidine-2,4(1*H*,3*H*)-dione (200 mg, 0.5 mmol) in 1,2-dichloromethane (3 mL). The mixture was stirred at 30 °C for 1 h, and then sodium triacetoxyborohydride (212 mg, 1.0 mmol) was added. The mixture was stirred at 30 °C for 1 h. After the reaction was completed, the mixture was extracted with water (5 mL) and ethyl acetate (5 mL × 3), dried over anhydrous Na₂SO₄, filtered, and concentrated in vacuum. The crude product was purified by silica gel column chromatography (DCM:MeOH = 10:1) to give *tert*-butyl 3-((9-(3-(2,4-dioxotetrahydropyrimidin-1(2*H*)-yl)-4-methoxybenzoyl)-3,9-diazaspiro[5.5]undecan-3-yl)methyl)azetidine-1-carboxylate.

LC-MS: (ESI, m/z): [M+H]⁺ = 570.2.

### Step 2: preparation of 1-(5-(9-(azetidin-3-ylmethyl)-3,9-diazaspiro[5.5]undecane-3-carbonyl)-2-methoxyphenyl)dihydropyrimidine-2,4(1H,3H)-dione

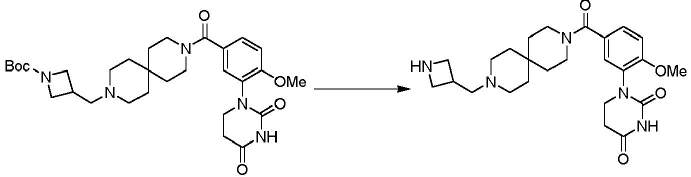

HCl/1,4-dioxane (1 mL) was added to a solution of *tert-butyl* 3-((9-(3-(2,4-dioxotetrahydropyrimidin-1(2*H*)-yl)-4-methoxybenzoyl)-3,9-diazaspiro[5.5]undecan-3-yl)methyl)azetidine-1-carboxylate (210 mg, 0.37 mmol) in 1,4-dioxane (2 mL), and the mixture was stirred at 30 °C for 1 h. After the reaction was completed, the mixture was concentrated in vacuum to give 1-(5-(9-(azetidin-3-ylmethyl)-3,9-diazaspiro[5.5]undecane-3-carbonyl)-2-methoxyphenyl)dihydropyrimidine-2,4(1H,3H)-dione. The crude product was directly used in the next step without further purification.

LC-MS: (ESI, m/z): [M+H]⁺ = 470.2.

### Step 3: preparation of tert-butyl 3-(2-((1-((3-((9-(3-(2,4-dioxotetrahydropyrimidin-1(2H)-yl)-4-methoxybenzoyl)-3,9-diazaspiro[5.5]undecan-3-yl)methyl)azetidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

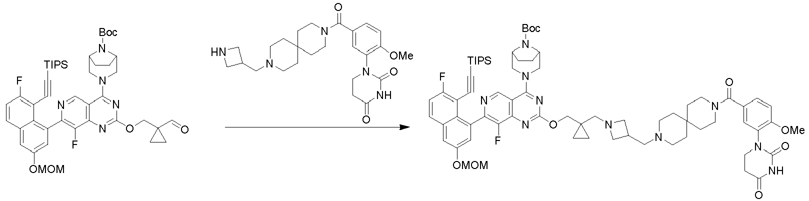

1-(5-(9-(Azetidin-3-ylmethyl)-3,9-diazaspiro[5.5]undecane-3-carbonyl)-2-methoxyphenyl)dihydropyrimidine-2,4(1*H*,3*H*)-dione (84 mg, 0.18 mmol) and tetraisopropyl titanate (171 mg, 0.6 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in tetrahydrofuran (1 mL) and anhydrous methanol (0.5 mL), and the mixture was stirred at 70 °C for 2 h. NaBH(OAc)₃ (100 mg, 0.48 mmol) was then added, and the mixture was stirred at 30 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert-butyl* 3-(2-((1-((3-((9-(3-(2,4-dioxotetrahydropyrimidin-1(2*H*)-yl)-4-methoxybenzoyl)-3,9-diazaspiro[5.5]undecan-3-yl)methyl)azetidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo [3.2.1]octane-8-carboxylate.

LC-MS: (ESI, m/z): [M/2+H]⁺ = 648.5.

### Step 4: preparation of 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)azetidin-3-yl)methyl)-3,9-diazaspiro[5.5]undecane-3-carbonyl)-2-methoxyphenyl)dihydropyrimidine-2,4(1H,3H)-dione

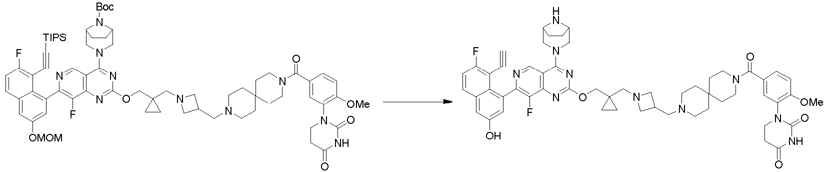

CsF (30 mg, 0.20 mmol) was added to a solution of *tert*-butyl 3-(2-((1-((3-((9-(3-(2,4-dioxotetrahydropyrimidin-1(2*H*)-yl)-4-methoxybenzoyl)-3,9-diazaspiro[5.5]undecan-3-yl)methyl)azetidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (50 mg, 0.039 mmol) in DMF (1 mL), and the mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)azetidin-3-yl)methyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 995.5.

¹H NMR (400 MHz, CD₃OD) δ 9.02 (s, 1H), 7.86 (dd, *J* = 9.2, 5.7 Hz, 1H), 7.45 - 7.41 (m, 1H), 7.39 - 7.37 (m, 1H), 7.36 - 7.30 (m, 2H), 7.22 - 7.17 (m, 2H), 4.65 - 4.57 (m, 2H), 4.34 (dd, *J* = 29.9, 11.3 Hz, 2H), 3.93 (s, 3H), 3.76 - 3.64 (m, 10H), 3.48 - 3.43 (m, 1H), 3.39 - 3.36 (m, 1H), 3.12 - 3.04 (m, 2H), 2.85 - 2.76 (m, 3H), 2.68 (s, 2H), 2.59 (d, *J* = 6.8 Hz, 2H), 2.46 - 2.36 (m, 4H), 1.90 - 1.78 (m, 4H), 1.59 - 1.52 (m, 5H), 1.38 - 1.28 (m, 4H), 0.70 - 0.65 (m, 2H), 0.61 - 0.57 (m, 2H).

### Example 41: 1-(5-(9-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)azetidin-3-yl)ethyl)-3,9-diazaspiro[5.5]undecane-3-carbonyl)-2-methoxyphenyl)dihydropyrimidine-2,4(1H,3H)-dione

### Step 1: preparation of tert-butyl 3-(2-oxoethyl)azetidine-1-carboxylate

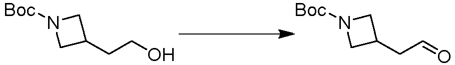

Dess-Martin (1.5 g, 3.58 mmol) was added to a solution of *tert*-butyl 3-(2-hydroxyethyl)azetidine-1-carboxylate (600 mg, 2.98 mmol) in dichloromethane (10 mL). The mixture was stirred at room temperature for 2 h. After the reaction was completed, a sodium thiosulfate solution (20 mL) and a sodium bicarbonate solution (20 mL) were added to quench the reaction, and the mixture was extracted with dichloromethane (20 mL × 3). The organic phases were combined, dried over anhydrous sodium sulfate, and concentrated under reduced pressure to give *tert*-butyl 3-(2-oxoethyl)azetidine-1-carboxylate. The crude product was directly used in the next step.

### Step 2: preparation of tert-butyl 3-(2-(9-(3-(2,4-dioxotetrahydropyrimidin-1(2H)-yl)-4-methoxybenzoyl)-3,9-diazaspiro[5.5]undecan-3-yl)ethyl)azetidine-1-carboxylate

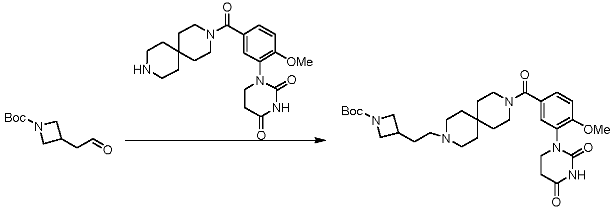

Triethylamine (63 mg, 0.62 mmol), acetic acid (0.1 mL), and *tert-butyl* 3-(2-oxoethyl)azetidine-1-carboxylate (247 mg, 1.24 mmol) were added to a solution of 1-(2-methoxy-5-(3,9-diazaspiro[5.5]undecane-3-carbonyl)phenyl)dihydropyrimidine-2,4(1*H*,3*H*)-dione (250 mg, 0.62 mmol) in 1,2-dichloromethane (3 mL), and the mixture was stirred at 30 °C for 1 h. Then sodium triacetoxyborohydride (263 mg, 1.24 mmol) was added. The mixture was stirred at 30 °C for 1 h. After the reaction was completed, the mixture was extracted with water (5 mL) and ethyl acetate (5 mL × 3), dried over anhydrous Na₂SO₄, filtered, and concentrated in vacuum. The mixture was purified by silica gel column chromatography (DCM:MeOH = 10:1) to give *tert*-butyl 3-(2-(9-(3-(2,4-dioxotetrahydropyrimidin-1(2H)-yl)-4-methoxybenzoyl)-3,9-diazaspiro[5.5]undecan-3-yl)ethyl)azetidine-1-carboxylate.

LC-MS: (ESI, m/z): [M+H]⁺ = 584.3.

### Step 3: preparation of 1-(5-(9-(2-(azetidin-3-yl)ethyl)-3,9-diazaspiro[5.5]undecane-3-carbonyl)-2-methoxyphenyl)dihydropyrimidine-2,4(1H,3H)-dione

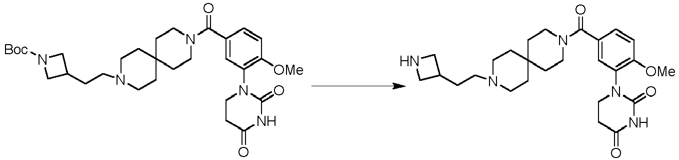

HCl/1,4-dioxane (1 mL) was added to a solution of *tert-butyl* 3-(2-(9-(3-(2,4-dioxotetrahydropyrimidin-1(2*H*)-yl)-4-methoxybenzoyl)-3,9-diazaspiro[5.5]undecan-3-yl)ethyl)azetidine-1-carboxylate (350 mg, 0.6 mmol) in 1,4-dioxane (2 mL), and the mixture was stirred at 30 °C for 1 h. After the reaction was completed, Na₂CO₃ (5 mL) was added, and the mixture was extracted with ethyl acetate (5 mL × 3), dried over anhydrous Na₂SO₄, filtered, and concentrated in vacuum. The concentrate was purified by silica gel column chromatography (DCM:MeOH (NH₃) = 5:1) to give 1-(5-(9-(2-(azetidin-3-yl)ethyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 484.2.

### Step 4: preparation of tert-butyl 3-(2-((1-((3-(2-(9-(3-(2,4-dioxotetrahydropyrimidin-1(2H)-yl)-4-methoxybenzoyl)-3,9-diazaspiro[5.5]undecan-3-yl)ethyl)azetidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

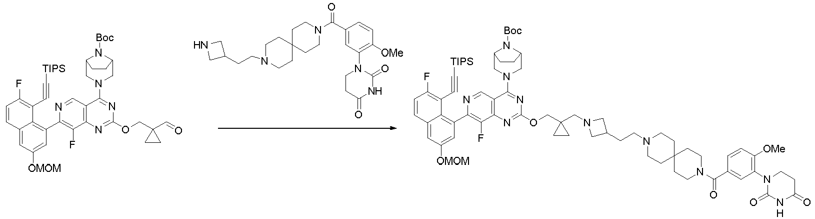

1-(5-(9-(2-(Azetidin-3-yl)ethyl)-3,9-diazaspiro[5.5]undecane-3-carbonyl)-2-methoxyphenyl)dihydropyrimidine-2,4(1H,3H)-dione (70 mg, 0.14 mmol) and tetraisopropyl titanate (171 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH (NH₃) = 10:1) to give *tert-butyl* 3-(2-((1-((3-(2-(9-(3-(2,4-dioxotetrahydropyrimidin-1(2*H*)-yl)-4-methoxybenzoyl)-3,9-diazaspiro[5.5]undecan-3-yl)ethyl)azetidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 655.9.

### Step 5: preparation of 1-(5-(9-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)azetidin-3-yl)ethyl)-3,9-diazaspiro[5.5]undecane-3-carbonyl)-2-methoxyphenyl)dihydropyrimidine-2,4(1H,3H)-dione

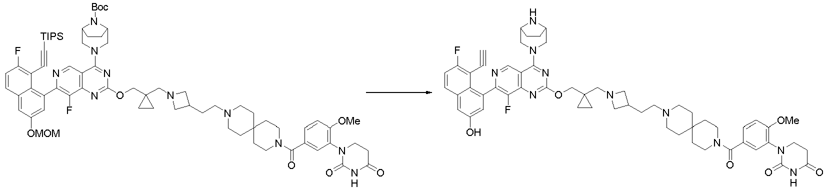

CsF (68 mg, 0.45 mmol) was added to a solution of *tert-butyl* 3-(2-((1-((3-(2-(9-(3-(2,4-dioxotetrahydropyrimidin-1(2*H*)-yl)-4-methoxybenzoyl)-3,9-diazaspiro[5.5]undecan-3-yl)ethyl)azetidin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate (120 mg, 0.09 mmol) in DMF (1 mL), and the mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-(2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)azetidin-3-yl)ethyl)-3,9-diazaspiro[5.5]undecane-3-carbonyl)-2-methoxyphenyl)dihydropyrimidine-2,4(1*H*,3*H*)-dione.

LC-MS: (ESI, m/z): [M+H]⁺ = 1009.2.

¹H NMR (400 MHz, CD₃OD) δ 8.89 - 8.76 (m, 1H), 8.53 - 8.47 (m, 2H), 8.01 - 7.65 (m, 1H), 7.47 - 7.10 (m, 6H), 4.73 - 4.28 (m, 7H), 3.95 (s, 7H), 3.82 - 3.63 (m, 7H), 3.59 - 3.36 (m, 9H), 3.16 - 2.88 (m, 2H), 2.82 (t, *J =* 6.3 Hz, 2H), 2.36 (s, 1H), 2.15 - 1.94 (m, 5H), 1.84 - 1.49 (m, 6H), 1.35 - 1.25 (m, 1H), 0.61 (s, 4H).

### Example 42: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of 7-bromo-2,4-dichloro-8-fluoroquinazoline

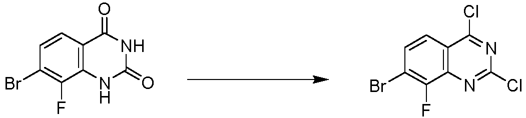

7-Bromo-8-fluoroquinazoline-2,4(1*H*,3*H*)-dione (20 g, 77.21 mol) was dissolved in POCl₃ (200 mL), and the mixture was cooled to 0 °C. DIEA (29.88 g, 231.63 mol) was slowly added dropwise with stirring. The reaction liquid was heated to 120 °C and reacted for 16 h. After the reaction was completed, the reaction liquid was concentrated, and the concentrate was diluted with ethyl acetate. The mixture was slowly poured into ice water to quench the reaction and extracted with dichloromethane (200 mL × 3), and the organic phase was separated. The organic phase was dried over anhydrous Na₂SO₄, filtered, and concentrated to give 7-bromo-2,4-dichloro-8-fluoroquinazoline. The crude product was directly used in the next step without purification.

### Step 2: preparation of tert-butyl 3-(7-bromo-2-chloro-8-fluoroquinazolin-4-yl)-3,8-diazabicyclo[3.2.1]octane-8-carboxylate

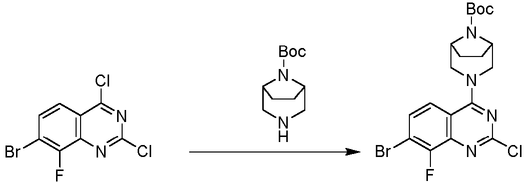

7-Bromo-2,4-dichloro-8-fluoroquinazoline (4.5 g, 0.0152 mmol) and TEA (4.6 g, 0.0456 mmol) were dissolved in THF (30 mL), and the mixture was cooled to -60 °C. A solution of *tert-butyl* 3,8-diazabicyclo[3.2.1]octane-8-carboxylate in THF (20 mL) was slowly added dropwise, and the mixture was reacted at -60 °C for 30 min. After the reaction was completed, the reaction liquid was poured into an aqueous NH₄Cl solution (50 mL), and the mixture was extracted with ethyl acetate (30 mL × 3). The organic phases were combined, dried over anhydrous Na₂SO₄, and filtered, and the filtrate was concentrated to give a crude product, which was purified by silica gel column chromatography (PE:EtOAc = 3:1) to give *tert-butyl* 3-(7-bromo-2-chloro-8-fluoroquinazolin-4-yl)-3,8-diazabicyclo[3.2.1]octane-8-carboxylate.

LC-MS: (ESI, m/z): [M+H]⁺ = 471.0, 473.0.

### Step 3: preparation of tert-butyl 3-(7-bromo-8-fluoro-2-((1-(hydroxymethyl)cyclopropyl)methoxy)quinazolin-4-yl)-3,8-diazabicyclo[3.2.1]octane-8-carboxylate

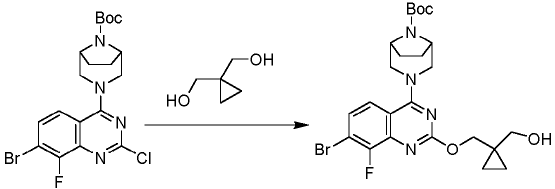

*tert*-Butyl 3-(7-bromo-2-chloro-8-fluoroquinazolin-4-yl)-3,8-diazabicyclo[3.2.1]octane-8-carboxylate (2.0 g, 4.24 mmol) was added to anhydrous acetonitrile (30 mL), and then cesium carbonate (4.14 g, 12.72 mmol) and cyclopropane-1,1-diyldimethanol (240 mg, 2.12 mmol) were added. The mixture was stirred at 25 °C for 1 h. After the reaction was completed, saturated brine (30 mL) was added to quench the reaction, and the mixture was extracted with ethyl acetate (50 mL × 3). The organic phases were combined, dried over anhydrous sodium sulfate, and concentrated under reduced pressure. The concentrate was purified by silica gel column chromatography (PE:EA = 2:1) to give *tert*-butyl 3-(7-bromo-8-fluoro-2-((1-(hydroxymethyl)cyclopropyl)methoxy)quinazolin-4-yl)-3,8-diazabicyclo[3.2.1]octane-8-carboxylate.

LC-MS: (ESI, m/z): [M+H]⁺ = 537.1.

### Step 4: preparation of tert-butyl 3-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)quinazolin-4-yl)-3,8-diazabicyclo[3.2.1]octane-8-carboxylate

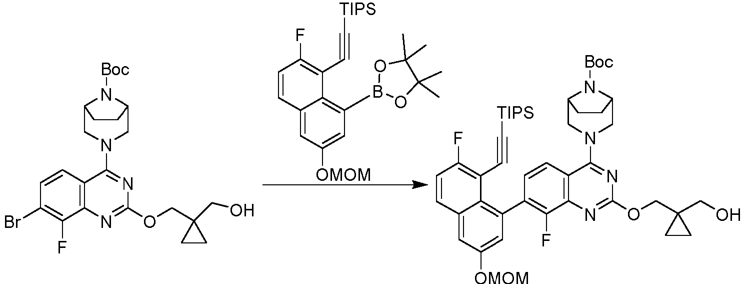

CataCXium A Pd G3 (81 mg, 0.112 mmol) and cesium carbonate (546 mg, 1.674 mmol) were added to a mixed solution of *tert-butyl* 3-(7-bromo-8-fluoro-2-((1-(hydroxymethyl)cyclopropyl)methoxy)quinazolin-4-yl)-3,8-diazabicyclo[3.2.1]octane-8-carboxylate (300 mg, 0.558 mmol) and (2-fluoro-6-(methoxymethoxy)-8-(4,4,5,5-tetramethyl-1,3,2-dioxaborolan-2-yl)naphthalen-1-yl)ethynyl)triisopropylsilane (284 mg, 0.67 mmol) in 1,4-dioxane (5 mL) and H₂O (1 mL). The mixture was reacted at 85 °C for 16 h under nitrogen atmosphere. Water (20 mL) was added to quench the reaction, and the mixture was extracted with ethyl acetate (20 mL × 3). The organic phases were combined, dried over anhydrous sodium sulfate, and concentrated under reduced pressure. The concentrate was purified by silica gel column chromatography (PE:EA = 2:1) to give *tert-butyl* 3-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)quinazolin-4-yl)-3,8-diazabicyclo[3.2.1]octane-8-carboxylate.

LC-MS: (ESI, m/z): [M+H]⁺ = 843.4.

### Step 5: preparation of tert-butyl 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate

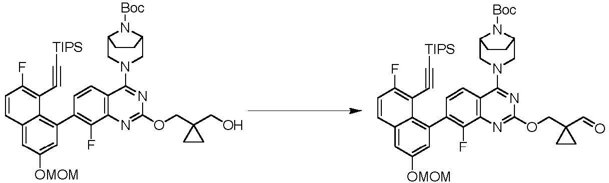

A solution of *tert-butyl* 3-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)quinazolin-4-yl)-3,8-diazabicyclo[3.2.1]octane-8-carboxylate (200 mg, 0.237 mmol) in DCM (4 mL) was cooled to 0 °C, and Dess-Martin reagent (201 mg, 5.7 mmol) was added. The mixture was reacted at room temperature for 0.5 h. After the reaction was completed, a saturated sodium thiosulfate solution (10 mL) was added to quench the reaction, and the mixture was extracted with dichloromethane (30 mL × 3). The organic phases were combined, dried over anhydrous sodium sulfate, and concentrated under reduced pressure. The concentrate was purified by silica gel column chromatography (PE:EA = 2:1) to give *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate.

LC-MS: (ESI, m/z): [M+H]⁺ = 841.4.

### Step 6: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

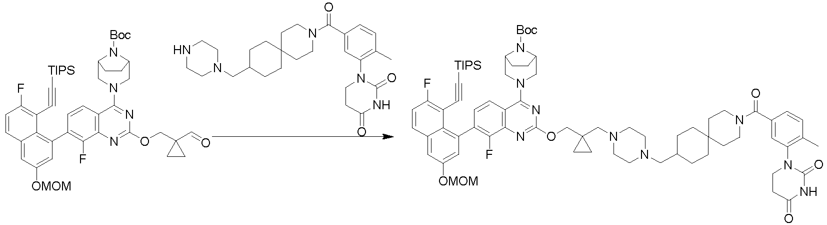

Tetraisopropyl titanate (169 mg, 0.595 mmol) was added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate (100 mg, 0.119 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (69 mg, 0.143 mmol) in DCM/AcOH (1.0 mL/0.01 mL), and the mixed solution was stirred at 30 °C for 2 h. NaBH(OAc)₃ (79 mg, 0.357 mmol) was then added at room temperature, and the mixture was stirred for 2 h. The mixture was concentrated and purified by silica gel column chromatography (MeOH:DCM = (0-1):4) to give *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1306.6.

### Step 7: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

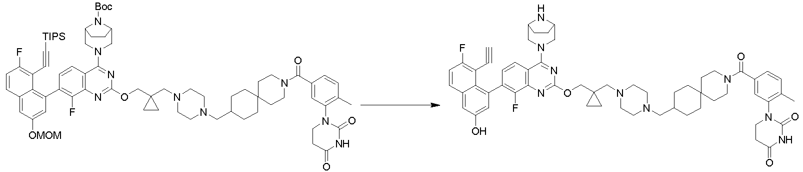

Cesium fluoride (58 mg, 0.385 mmol) was added to a stirred solution of *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (100 mg, 0.077 mmol) in DMF (2 mL), and the mixture was stirred at room temperature for 30 min and extracted with EA and water. The organic phase was concentrated. A mixture of 1,4-dioxane (1 mL) and HCl/1,4-dioxane (6 N, 0.5 mL) was then added to the mixture at room temperature, and the resulting mixture was reacted for 0.5 h. An aqueous Na₂CO₃ solution was added to the reaction liquid, and the mixture was adjusted to pH > 7 and extracted with ethyl acetate (10 mL × 2). The organic phase was washed with brine, dried over anhydrous Na₂SO₄, and concentrated. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1006.2.

¹H NMR (400 MHz, DMSO-*d*₆) δ 10.37 (s, 1H), 10.13 (s, 1H), 7.96 (dd, *J* = 9.2, 6.0 Hz, 1H), 7.72 (d, *J=* 8.6 Hz, 1H), 7.45 (t, *J =* 9.0 Hz, 1H), 7.36 - 7.29 (m, 3H), 7.24 (d, *J* = 7.6 Hz, 1H), 7.18 - 7.12 (m, 1H), 7.06 (d, *J* = 2.3 Hz, 1H), 4.29 (d, *J* = 11.8 Hz, 1H), 4.26 - 4.16 (m, 3H), 3.84 (s, 1H), 3.83 - 3.74 (m, 1H), 3.62 - 3.39 (m, 8H), 2.87 - 2.60 (m, 3H), 2.46 - 2.17 (m, 14H), 2.04 (s, 2H),1.78 - 1.63 (m, 6H), 1.54 - 1.37 (m, 5H), 1.34 - 1.32(m, 2H), 1.12 - 0.93(s, 4H), 0.61 (s, 2H), 0.38 (s, 2H).

### Example 43: 1-(5-(2-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)-7-azaspiro[3.5]nonane-7-carbonyl)-2-methoxyphenyl)dihydropyrimidine-2,4(1H,3H)-dione

### Step 1: preparation of tert-butyl 2-(2-ethoxy-2-oxoethylidene)-7-azaspiro[3.5]nonane-7-carboxylate

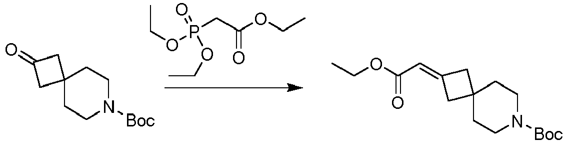

NaH (1.26 g, 31.4 mmol, 60%) was added to a solution of ethyl 2-(diethoxyphosphoryl)acetate (7.04 g, 31.4 mmol) in DMF (70 mL). The mixture was stirred at 0 °C for 30 min. *tert*-Butyl 2-oxo-7-azaspiro[3.5]nonane-7-carboxylate (5.00 g, 20.9 mmol) was then added, and the mixture was stirred at room temperature for 3 h. The reaction liquid was cooled to 0 °C. An aqueous NH₄Cl solution (100 mL) was added to quench the reaction, and the mixture was extracted with ethyl acetate (100 mL × 3). The combined organic phase was washed with brine (100 mL × 3), dried over anhydrous sodium sulfate, filtered, and concentrated, and the crude product was purified by silica gel column chromatography (PE:EA = 100:1 to 10:1) to give *tert*-butyl 2-(2-ethoxy-2-oxoethylidene)-7-azaspiro[3.5]nonane-7-carboxylate.

LC-MS: (ESI, m/z): [M-tBu+H]⁺ = 254.2.

### Step 2: preparation of tert-butyl 2-(2-ethoxy-2-oxoethyl)-7-azaspiro[3.5]nonane-7-carboxylate

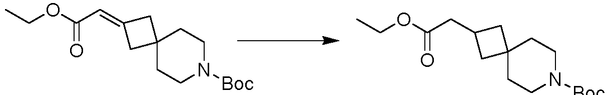

*tert*-Butyl 2-(2-ethoxy-2-oxoethylidene)-7-azaspiro[3.5]nonane-7-carboxylate (6.4 g, 20.7 mmol) was dissolved in EtOH (60 mL), and Pd/C (2.2 g, 2.07 mmol, 10%) was added. The mixture was stirred for 16 h under H₂ atmosphere. The mixture was filtered and concentrated to give *tert-butyl* 2-(2-ethoxy-2-oxoethyl)-7-azaspiro[3.5]nonane-7-carboxylate, which was directly used in the next step.

LC-MS: (ESI, m/z): [M-tBu+H]⁺ = 256.3.

### Step 3: preparation of tert-butyl 2-(2-hydroxyethyl)-7-azaspiro[3.5]nonane-7-carboxylate

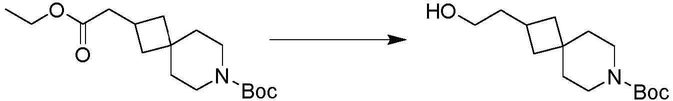

LAH (23.9 mL, 23.9 mmol, 1 M in THF) was added dropwise to a solution of *tert*-butyl 2-(2-ethoxy-2-oxoethyl)-7-azaspiro[3.5]nonane-7-carboxylate (6.2 g, 19.9 mmol) in THF (90 mL) at 0-5 °C. The mixture was reacted at room temperature for 1 h. The reaction liquid was cooled to 0-5 °C. Na₂SO₄·10H₂O was added to quench the reaction, and the mixture was stirred for 1 h. The mixture was filtered, and the filtrate was concentrated to give *tert*-butyl 2-(2-hydroxyethyl)-7-azaspiro[3.5]nonane-7-carboxylate.

LC-MS: (ESI, m/z): [M-tBu+H]⁺ = 214.2.

### Step 4: preparation of tert-butyl 2-(2-oxoethyl)-7-azaspiro[3.5]nonane-7-carboxylate

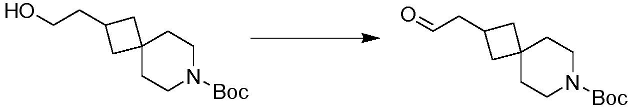

Dess-Martin reagent (3.8 g, 8.9 mmol) was added to a solution of *tert*-butyl 2-(2-hydroxyethyl)-7-azaspiro[3.5]nonane-7-carboxylate (2 g, 7.4 mmol) in DCM (20 mL), and the mixture was stirred at room temperature for 2 h. After the reaction was completed, a sodium thiosulfate solution (30 mL) and a sodium bicarbonate solution (30 mL) were added to quench the reaction, and the mixture was extracted with dichloromethane (30 mL × 3). The organic phases were combined, dried over anhydrous sodium sulfate, and concentrated under reduced pressure. The concentrate was purified by silica gel column chromatography (PE:EA = 5:1) to give *tert*-butyl 2-(2-oxoethyl)-7-azaspiro[3.5]nonane-7-carboxylate.

LC-MS: (ESI, m/z): [M-tBu+H]⁺ = 212.2.

### Step 5: preparation of tert-butyl 2-(2-(4-((benzyloxy)carbonyl)piperazin-1-yl)ethyl)-7-azaspiro [3.5]nonane-7-carboxylate

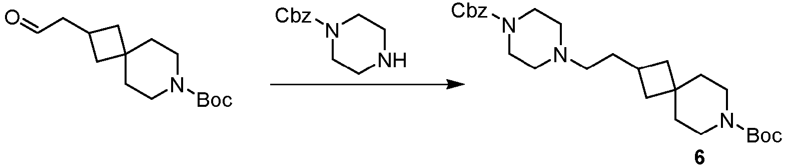

Benzyl piperazine-1-carboxylate (1.58 g, 7.18 mmol) and acetic acid (0.5 mL) were added to a solution of *tert-butyl* 2-(2-oxoethyl)-7-azaspiro[3.5]nonane-7-carboxylate (1.60 g, 5.98 mmol) in DCM/MeOH (10 mL/5 mL). The mixture was stirred at room temperature for 2 h. NaBH(OAc)₃ (2.53 g, 11.96 mmol) was then added at 0 °C. The mixture was diluted with an aqueous solution (10 mL) and extracted with DCM (10 mL × 3). The combined organic phase was dried over anhydrous sodium sulfate and filtered, and the filtrate was concentrated and purified by silica gel column chromatography (PE:EA = 3:1) to give *tert-butyl* 2-(2-(4-((benzyloxy)carbonyl)piperazin-1-yl)ethyl)-7-azaspiro[3.5]nonane-7-carboxylate.

LC-MS: (ESI, m/z): [M+H]⁺ = 472.2.

### Step 6: preparation of benzyl 4-(2-(7-azaspiro[3.5]nonan-2-yl)ethyl)piperazine-1-carboxylate

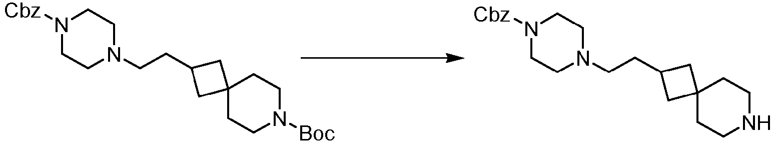

HCl/1,4-dioxane (2 mL) was added to a solution of *tert-butyl* 2-(2-(4-((benzyloxy)carbonyl)piperazin-1-yl)ethyl)-7-azaspiro[3.5]nonane-7-carboxylate (800 mg, 1.7 mmol) in 1,4-dioxane (4 mL), and the mixture was stirred at 30 °C for 1 h. After the reaction was completed, an aqueous Na₂CO₃ solution (5 mL) was added to adjust the pH > 7, and the mixture was extracted with ethyl acetate (5 mL × 3). The combined organic phase was dried over Na₂SO₄, filtered, and concentrated under reduced pressure to give benzyl 4-(2-(7-azaspiro[3.5]nonan-2-yl)ethyl)piperazine-1-carboxylate. The crude product was directly used in the next step without further purification.

LC-MS: (ESI, m/z): [M+H]⁺ = 372.2.

### Step 7: preparation of benzyl 4-(2-(7-(3-(2,4-dioxotetrahydropyrimidin-1(2H)-yl)-4-methoxybenzoyl)-7-azaspiro[3.5]nonan-2-yl)ethyl)piperazine-1-carboxylate

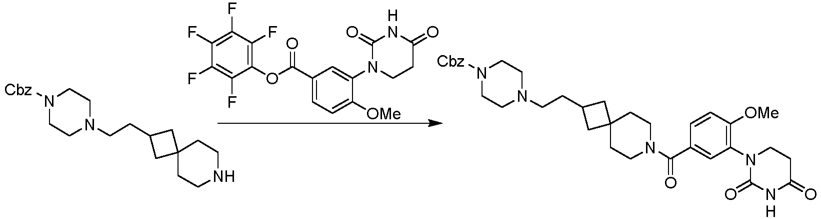

Pentafluorophenyl 3-(2,4-dioxotetrahydropyrimidin-1(2*H*)-yl)-4-methoxybenzoate (314 mg, 0.73 mmol) and DIEA (377 g, 2.92 mmol) were added to a solution of benzyl 4-(2-(7-azaspiro[3.5]nonan-2-yl)ethyl)piperazine-1-carboxylate (270 mg, crude) in DMF (4 mL). The mixture was stirred at room temperature for 2 h. After the reaction was completed, water (10 mL) was added to quench the reaction, and the mixture was extracted with ethyl acetate (10 mL × 3). The organic phase was dried, concentrated, and purified by silica gel column chromatography (DCM:MeOH = 20:1) to give benzyl 4-(2-(7-(3-(2,4-dioxotetrahydropyrimidin-1(2*H*)-yl)-4-methoxybenzoyl)-7-azaspiro[3.5]nonan-2-yl)ethyl)piperazine-1-carboxylate.

LC-MS: (ESI, m/z): [M+H]⁺ = 618.2.

### Step 8: preparation of 1-(2-methoxy-5-(2-(2-(piperazin-1-yl)ethyl)-7-azaspiro[3.5]nonane-7-carbonyl)phenyl)dihydropyrimidine-2,4(1H,3H)-dione

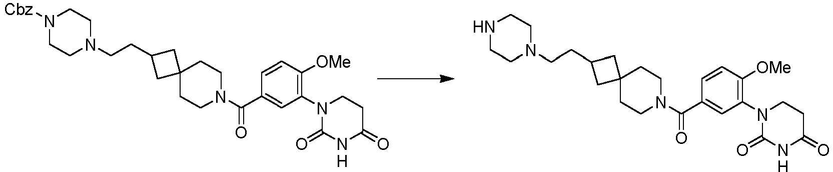

Pd/C (64 mg) was added to a solution of benzyl 4-(2-(7-(3-(2,4-dioxotetrahydropyrimidin-1(2*H*)-yl)-4-methoxybenzoyl)-7-azaspiro[3.5]nonan-2-yl)ethyl)piperazine-1-carboxylate (370 mg, 0.6 mmol) in isopropanol (3 mL) and tetrahydrofuran (1 mL), and the mixture was stirred at 30 °C for 16 h. The mixture was filtered and concentrated, and the resulting crude product was purified by silica gel column chromatography (DCM:MeOH (NH₃) = 15:1) to give 1-(2-methoxy-5-(2-(2-(piperazin-1-yl)ethyl)-7-azaspiro[3.5]nonane-7-carbonyl)phenyl)dihydropyrimidine-2,4(1*H*,3*H*)-dione.

LC-MS: (ESI, m/z): [M+H]⁺ = 484.3.

### Step 9: preparation of tert-butyl 3-(2-((1-((4-(2-(7-(3-(2,4-dioxotetrahydropyrimidin-1(2H)-yl)-4-methoxybenzoyl)-7-azaspiro[3.5]nonan-2-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

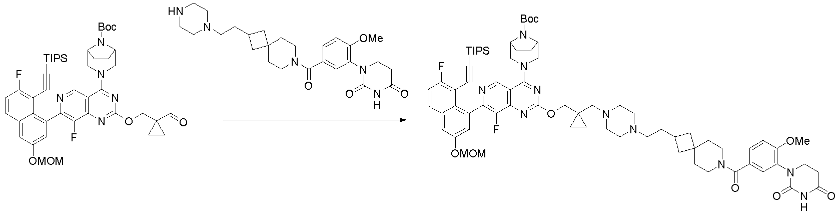

1-(2-Methoxy-5-(2-(2-(piperazin-1-yl)ethyl)-7-azaspiro[3.5]nonane-7-carbonyl)phenyl)dihydropyrimidine-2,4(1*H*,3*H*)-dione (70 mg, 0.14 mmol) and tetraisopropyl titanate (170 mg, 0.6 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert-butyl* 3-(2-((1-((4-(2-(7-(3-(2,4-dioxotetrahydropyrimidin-1(2*H*)-yl)-4-methoxybenzoyl)-7-azaspiro[3.5]nonan-2-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 655.9.

### Step 10: preparation of 1-(5-(2-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)ethyl)-7-azaspiro[3.5]nonane-7-carbonyl)-2-methoxyphenyl)dihydropyrimidine-2,4(1H,3H)-dione

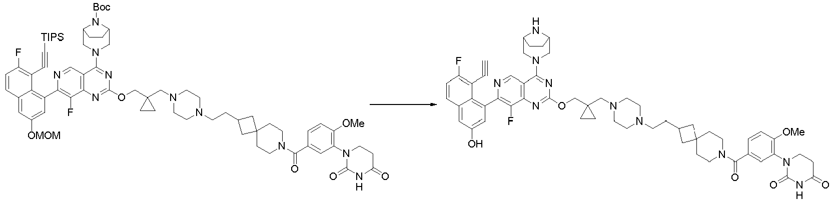

*tert*-Butyl 3-(2-((1-((4-(2-(7-(3-(2,4-dioxotetrahydropyrimidin-1(2*H*)-yl)-4-methoxybenzoyl)-7-azaspiro[3.5]nonan-2-yl)ethyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (140 mg, 0.11 mmol) was dissolved in DMF (1 mL), and CsF (84 mg, 0.55 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(2-(2-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)ethyl)-7-azaspiro[3.5]nonane-7-carbonyl)-2-methoxyphenyl)dihydropyrimidine-2,4(1*H*,3*H*)-dione.

LC-MS: (ESI, m/z): [M/2+H]⁺ = 505.2.

¹H NMR (400 MHz, DMSO-*d*₆) δ 10.32 (s, 1H), 10.14 (s, 1H), 9.02 (s, 1H), 7.97 (dd, *J* = 9.2, 5.9 Hz, 1H), 7.50 - 7.41 (m, 1H), 7.39 - 7.30 (m, 3H), 7.18 - 7.12 (m, 2H), 4.48 (d, *J* = 10.8 Hz, 1H), 4.31 - 4.24 (m, 3H), 3.92 (s, 1H), 3.84 (s, 3H), 3.67 - 3.49 (m, 7H), 3.46 - 3.46 - 3.34 (m, 2H), 2.69 - 2.60 (m, 3H), 2.44 - 2.13 (m, 11H), 2.12 - 2.06 (m, 2H), 1.95 - 1.87 (m, 2H), 1.69 - 1.62 (m, 4H), 1.62 - 1.41 (m, 7H), 1.39 - 1.32 (m, 2H), 0.65 - 0.59 (m, 2H), 0.42 - 0.36 (m, 2H).

### Example 44: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)amino)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-(((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

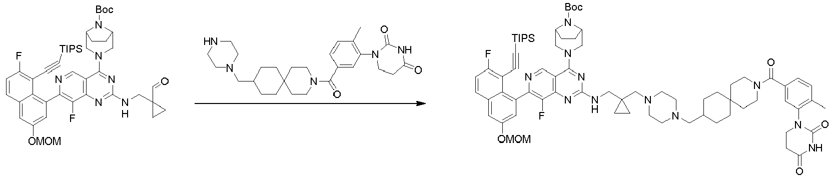

1-(2-Methyl-5-(9-(piperazin-1-ylmethyl)-3-azaspiro[5.5]undecane-3-carbonyl)phenyl)dihydropyrimidine-2,4(1*H*,3*H*)-dione (60 mg, 0.125 mmol) and tetraisopropyl titanate (170 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-(((1-formylcyclopropyl)methyl)amino)pyrido[4,3-*d*]pyrimidin-4-yl)-3,8-diazabicyclo[3.2.1]octane-8-carboxylate (80 mg, 0.096 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert-butyl* 3-(2-(((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 654.4.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)amino)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

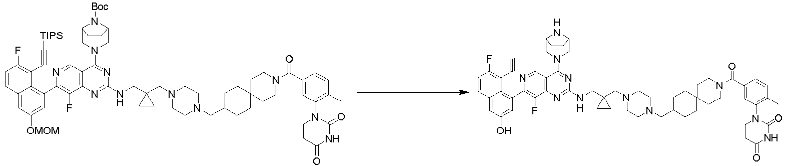

*tert*-Butyl 3-(2-(((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (70 mg, 0.067 mmol) was dissolved in DMF (2 mL), and CsF (102 mg, 0.67 mmol) was added. The mixture was reacted at room temperature for 1 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)amino)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 1006.2.

¹H NMR (400 MHz, DMSO-*d*₆) δ 10.36 (s, 1H), 10.09 (s, 1H), 8.73 (d, *J* = 6.7 Hz, 1H), 7.94 (dd, *J=* 9.2, 6.0 Hz, 1H), 7.88 - 7.81 (m, 0.5H), 7.48 - 7.38 (m, 1.5H), 7.36 - 7.29 (m, 3H), 7.24 (d, *J =* 7.9 Hz, 1H), 7.12 (s, 1H), 4.39 (dd, *J* = 23.8, 12.0 Hz, 1H), 4.28 - 4.10 (m, 1H), 3.92 (s, 1H), 3.85 - 3.75 (m, 1H), 3.69 - 3.33 (m, 10H), 2.87 - 2.57 (m, 3H), 2.47 - 2.14 (m, 13H), 2.12 - 1.93 (m, 3H), 1.74 - 1.60 (m, 6H), 1.55 - 1.22 (m, 8H), 1.11 - 1.01 (m, 3H), 0.56 - 0.46 (m, 2H), 0.32 - 0.17 (m, 2H).

Example 45: 1-(5-(9-((4-((1-(((4-(3,8-diazabicyclo[3.2.1]octan-3-yl)-7-(8-chloronaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione

### Step 1: preparation of tert-butyl 3-(7-(8-chloronaphthalen-1-yl)-8-fluoro-2-((1-(hydroxymethyl)cyclopropyl)methoxy)pyrido[4,3-d]pyrimidin-4-yl)-3,8-diazabicyclo[3.2.1]octane-8-carboxylate

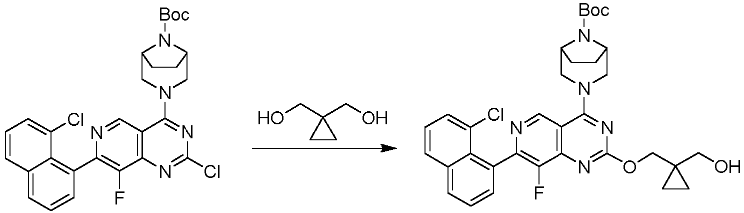

Cs₂CO₃ (235 mg, 0.72 mmol) and cyclopropane-1,1-diyldimethanol (49 mg, 0.48 mmol) were added to a solution of *tert*-butyl 3-(2-chloro-7-(8-chloronaphthalen-1-yl)-8-fluoropyrido[4,3-*d*]pyrimidin-4-yl)-3,8-diazabicyclo[3.2.1]octane-8-carboxylate (135 mg, 0.24 mmol) in tetrahydrofuran (1 mL). The mixture was stirred at 90 °C for 16 h. After the reaction was completed, the mixture was extracted with water (5 mL) and ethyl acetate (5 mL × 2), dried over anhydrous Na₂SO₄, filtered, and concentrated in vacuum. The crude product was purified by silica gel column chromatography (PE:EA = 1:1) to give *tert*-butyl 3-(7-(8-chloronaphthalen-1-yl)-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 620.2.

### Step 2: preparation of tert-butyl 3-(7-(8-chloronaphthalen-1-yl)-8-fluoro-2-((1-formylcyclopropyl)methoxy)pyrido[4,3-d]pyrimidin-4-yl)-3,8-diazabicyclo[3.2.1]octane-8-carboxylate

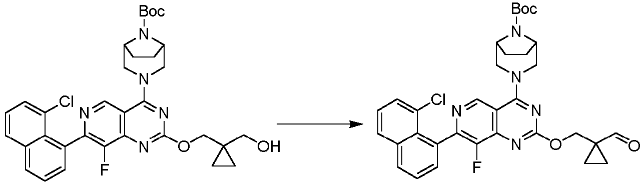

Dess-Martin reagent (98 mg, 0.23 mmol) was added to a solution of *tert-butyl* 3-(7-(8-chloronaphthalen-1-yl)-8-fluoro-2-((1-(hydroxymethyl)cyclopropyl)methoxy)pyrido[4,3-*d*]pyrimidin-4-yl)-3,8-diazabicyclo[3.2.1]
octane-8-carboxylate (115 mg, 0.19 mmol) in dichloromethane (1 mL). The mixture was stirred at room temperature for 2 h. After the reaction was completed, a sodium thiosulfate solution (3 mL) and a sodium bicarbonate solution (3 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (PE:EA = 1:1) to give *tert*-butyl 3-(7-(8-chloronaphthalen-1-yl)-8-fluoro-2-((1-formylcyclopropyl) methoxy)pyrido[4,3-*d*]pyrimidin-4-yl)-3,8-diazabicyclo[3.2.1]octane-8-carboxylate.

LC-MS: (ESI, m/z): [M+H]⁺ = 618.1.

### Step 3: preparation of tert-butyl 3-(7-(8-chloronaphthalen-1-yl)-2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoropyrido[4,3-d]pyrimidin-4-yl)-3,8-diazabicyclo[3.2.1]octane-8-carboxylate

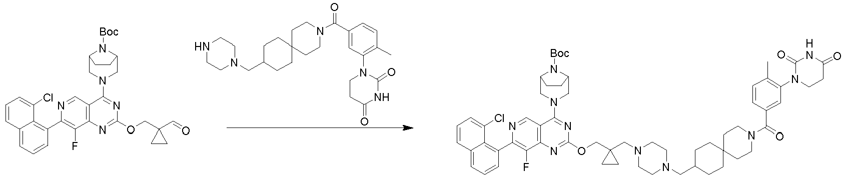

1-(2-Methyl-5-(9-(piperazin-1-ylmethyl)-3-azaspiro[5.5]undecane-3-carbonyl)phenyl)dihydropyrimidine-2,4(1H,3H)-dione (77 mg, 0.16 mmol) and tetraisopropyl titanate (185 mg, 0.65 mmol) were added to a solution of *tert-butyl* 3-(7-(8-chloronaphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate (80 mg, 0.13 mmol) in dichloromethane (1 mL) and acetic acid (0.1 mL), and the mixture was stirred at 25 °C for 2 h. Then NaBH(OAc)₃ (83 mg, 0.39 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(7-(8-chloronaphthalen-1-yl)-2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-3,8-diazabicyclo
[3.2.1]octane-8-carboxylate.

LC-MS: (ESI, m/z): [M+H]⁺ = 542.9.

### Step 4: preparation of 1-(5-(9-((4-((1-(((4-(3,8-diazabicyclo[3.2.1]octan-3-yl)-7-(8-chloronaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

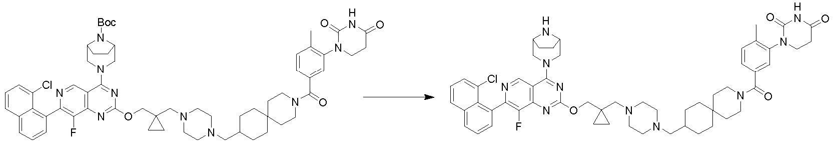

Hydrochloric acid/1,4-dioxane (6 M, 1 mL) was added to *tert*-butyl 3-(7-(8-chloronaphthalen-1-yl)-2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-3,8-diazabicyclo[3.2.1]octane-8-carboxylate (88 mg, 0.08 mmol), and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-chloronaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione.

LC-MS: (ESI, m/z): [M+H]⁺ = 983.4.

¹H NMR (400 MHz, DMSO-*d*₆) δ 10.36 (s, 1H), 9.07 (s, 1H), 8.19 (d, *J* = 7.6 Hz, 1H), 8.09 (d, *J* = 7.4 Hz, 1H), 7.75 - 7.68 (m, 1H), 7.66 - 7.54 (m, 3H), 7.35 - 7.29 (m, 2H), 7.25 - 7.21 (m, 1H), 4.46 - 4.36 (m, 2H), 4.32 - 4.24 (m, 2H), 3.84 - 3.74 (m, 1H), 3.68 - 3.43 (m, 8H), 2.82 - 2.65 (m, 3H), 2.42 - 2.23 (m, 10H), 2.21 (s, 3H), 2.09 -1.97 (m, 3H), 1.70 - 1.61 (mi, 6H), 1.54 - 1.45 (m, 3H), 1.42 - 1.36 (m, 2H), 1.27 - 1.21 (m, 1H), 1.12 - 0.93 (m, 5H), 0.64 - 0.59 (m, 2H), 0.41 - 0.36 (m, 2H).

### Example 46: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)amino)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-(((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

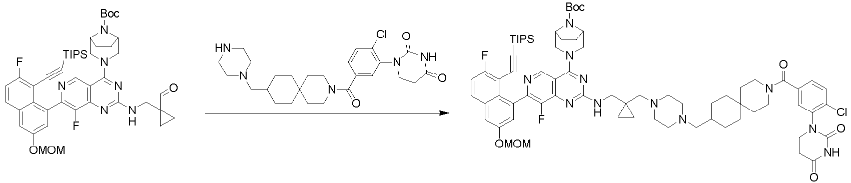

1-(2-Chloro-5-(9-(piperazin-1-ylmethyl)-3-azaspiro[5.5]undecane-3-carbonyl)phenyl)dihydropyrimidine-2,4(1*H*,3*H*)-dione (72 mg, 0.14 mmol) and tetraisopropyl titanate (170 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-(((1-formylcyclopropyl)methyl)amino)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-(((1-((4-((3-(4-chloro-3-(2,4-dioxotetrahydropyrimidin-1(2*H*)-yl)benzoyl)-3-azaspiro [5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo [3.2.1]octane-8-carboxylate.

LC-MS: (ESI, m/z): [M/2+H]⁺ = 664.3.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)amino)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

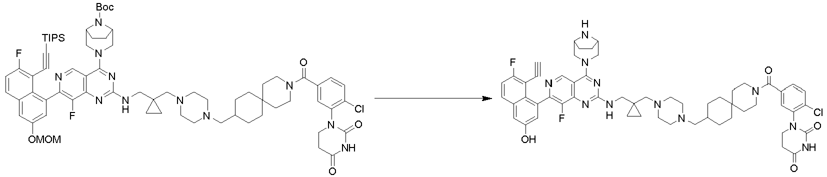

*tert*-Butyl 3-(2-(((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methyl)amino)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (45 mg, 0.03 mmol) was dissolved in DMF (1 mL), and CsF (23 mg, 0.15 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)amino)methyl)cyclopropyl)methyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 513.7.

¹H NMR (400 MHz, CD₃OD) δ 8.74 (s, 1H), 7.87 - 7.81 (m, 1H), 7.64 (d, *J* = 8.2 Hz, 1H), 7.55 - 7.52 (m, 1H), 7.44 -7.40 (m, 1H), 7.33 - 7.28 (m, 2H), 7.18 (d, *J* = 2.5 Hz, 1H), 4.55 - 4.35 (m, 2H), 3.83 - 3.76 (m, 2H), 3.74 -3.58 (m, 7H), 3.56 - 3.46 (m, 2H), 3.43 - 3.35 (m, 3H), 2.90 - 2.83 (m, 2H), 2.58 - 2.41 (m, 6H), 2.24 - 2.12 (m, 2H), 1.91 - 1.81 (m, 4H), 1.76 - 1.66 (m, 2H), 1.64 - 1.44 (m, 6H), 1.40 -1.26 (m, 3H), 1.24 - 0.86 (m, 5H), 0.66 - 0.54 (m, 2H), 0.46 - 0.32 (m, 2H).

### Example 47: 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)amino)methyl)cyclopropyl)methyl)piperazin-1-yl)methyl)-3-azaspiro [5.5]undecane-3-carbonyl)-2-chlorophenyl)dihydropyrimidine-2,4(1H,3H)-dione

### Step 1: preparation of tert-butyl 3-(7-(8-ethyl-3-(methoxymethoxy)naphthalen-1-yl)-8-fluoro-2-(((1-(hydroxymethyl)cyclopropyl)methyl)amino)pyrido[4,3-d]pyrimidin-4-yl)-3,8-diazabicyclo[3.2.1]octane-8-carboxylate

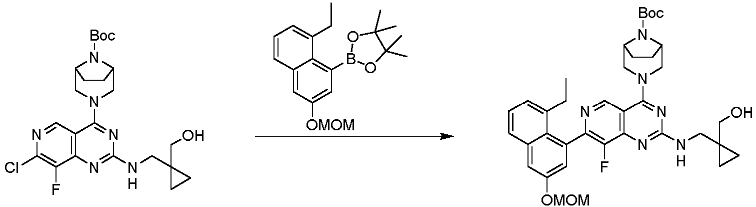

2-(8-Ethyl-3-(methoxymethoxy)naphthalen-1-yl)-4,4,5,5-tetramethyl-1,3,2-dioxaborolane (332 mg, 0.97 mmol) and Cs₂CO₃ (784 mg, 2.4 mmol) were added to a solution of *tert*-butyl 3-(7-chloro-8-fluoro-2-(((1-(hydroxymethyl)cyclopropyl)methyl)amino)pyrido[4,3-*d*]pyrimidin-4-yl)-3,8-diazabicyclo[3.2.1]octane-8-carboxylate (400 mg, 0.81 mmol) in 1,4-dioxane (5 mL) and water (1 mL), and CataCXium A Pd G3 (118 mg, 0.16 mmol) was added under N₂ atmosphere. The mixture was stirred at 85 °C for 16 h. After the reaction was completed, the mixture was extracted with water (5 mL) and ethyl acetate (5 mL × 3), dried over anhydrous Na₂SO₄, filtered, and concentrated in vacuum. The concentrate was purified by silica gel column chromatography (DCM:MeOH = 50:1) to give *tert-butyl* 3-(7-(8-ethyl-3-(methoxymethoxy)naphthalen-1-yl)-8-fluoro-2-(((1-(hydroxymethyl)cyclopropyl)methyl)amino)pyrido[4,3-*d*]pyrimidin-4-yl)-3,8-diazabicyclo [3.2.1]octane-8-carboxylate.

LC-MS: (ESI, m/z): [M+H]⁺ = 673.4.

### Step 2: preparation of tert-butyl 3-(7-(8-ethyl-3-(methoxymethoxy)naphthalen-1-yl)-8-fluoro-2-(((1-formylcyclopropyl)methyl)amino)pyrido[4,3-d]pyrimidin-4-yl)-3,8-diazabicyclo[3.2.1]octane-8-carboxylate

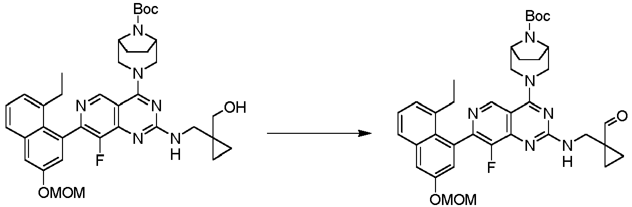

Dess-Martin reagent (304 mg, 0.71 mmol) was added to a solution of *tert-butyl* 3-(7-(8-ethyl-3-(methoxymethoxy)naphthalen-1-yl)-8-fluoro-2-(((1-(hydroxymethyl)cyclopropyl)methyl)amino)pyrido[4,3-*d*]pyrimidin-4-yl)-3,8-diazabicyclo[3.2.1]octane-8-carboxylate (400 mg, 0.59 mmol) in dichloromethane (3 mL). The mixture was stirred at room temperature for 2 h. After the reaction was completed, a sodium thiosulfate solution (3 mL) and a sodium bicarbonate solution (3 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (DCM:MeOH = 30:1) to give *tert-butyl* 3-(7-(8-ethyl-3-(methoxymethoxy)naphthalen-1-yl)-8-fluoro-2-(((1-formylcyclopropyl)methyl)amino)pyrido[4,3-*d*]pyrimidin-4-yl)-3,8-diazabicyclo[3.2.1]octane-8-carboxylate.

LC-MS: (ESI, m/z): [M+H]⁺ = 671.3.

### Step 3: preparation of tert-butyl 3-(2-(((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methyl)amino)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

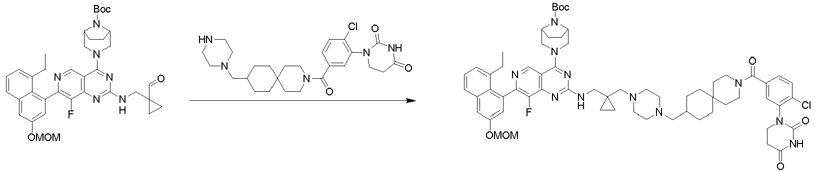

1-(2-Chloro-5-(9-(piperazin-1-ylmethyl)-3-azaspiro[5.5]undecane-3-carbonyl)phenyl)dihydropyrimidine-2,4(1*H*,3*H*)-dione (90 mg, 0.18 mmol) and tetraisopropyl titanate (213 mg, 0.75 mmol) were added to a solution of *tert*-butyl 3-(7-(8-ethyl-3-(methoxymethoxy)naphthalen-1-yl)-8-fluoro-2-(((1-formylcyclopropyl) methyl)amino)pyrido[4,3-*d*]pyrimidin-4-yl)-3,8-diazabicyclo[3.2.1]octane-8-carboxylate (100 mg, 0.15 mmol) in dichloromethane (1 mL) and acetic acid (0.1 mL), and the mixture was stirred at 25 °C for 2 h. Then NaBH(OAc)₃ (95 mg, 0.45 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-(((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methyl)amino)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 579.4.

### Step 4: preparation of 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)amino)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

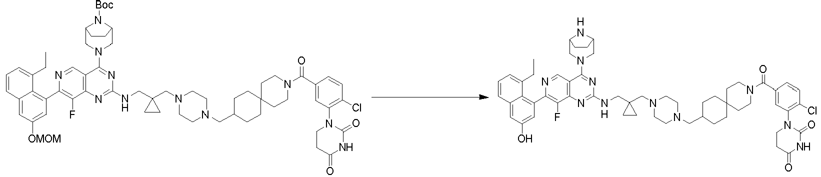

Hydrochloric acid/1,4-dioxane (8 M, 1 mL) was added to *tert*-butyl 3-(2-(((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methyl)amino)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate (100 mg, 0.086 mmol), and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-*d*]pyrimidin-2-yl)amino)methyl)cyclopropyl)methyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1012.2.

¹H NMR (400 MHz, DMSO-*d*₆) δ 10.51 (s, 1H), 9.84 (s, 1H), 8.79 (d, *J* = 6.4 Hz, 1H), 7.91-7.80 (m, 0.5H), 7.66 - 7.61 (m, 2H), 7.57 - 7.49 (m, 1.5H), 7.41 - 7.32 (m, 2H), 7.25 - 7.23 (m, 1H), 7.10 (d, *J* = 7.0 Hz, 1H), 6.95 - 7.91 (m, 1H), 4.36 - 4.27 (m, 2H), 3.80 - 3.70 (m, 1H), 3.65 -3.40 (m, 10H), 3.33 -3.18 (m, 3H), 2.81 - 2.66 (m, 3H), 2.47 - 2.16 (m, 10H), 2.11 - 2.01 (m, 2H), 1.70 - 1.62 (m, 5H), 1.60 - 1.22 (m, 8H), 1.16 - 0.94 (m, 4H), 0.87 - 0.80 (m, 3H), 0.56 - 0.46 (m, 2H), 0.33 - 0.15 (s, 1H).

### Example 48: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1H,3H)-dione

### Step 1: preparation of benzyl 4-((3-(3-(2,4-dioxotetrahydropyrimidin-1(2H)-yl)-4-fluorobenzoyl)-3 -azaspiro [5.5]undecan-9-yl)methyl)piperazine-1-carboxylate

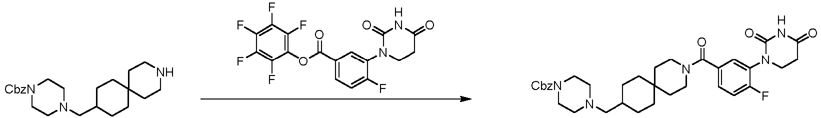

Pentafluorophenyl 3-(2,4-dioxotetrahydropyrimidin-1(2*H*)-yl)-4-fluorobenzoate (114 mg, 0.27 mmol) and DIEA (101 mg, 0.78 mmol) were added to a solution of benzyl 4-((3-azaspiro[5.5]undecan-9-yl)methyl)piperazine-1-carboxylate hydrochloride (100 mg, 0.26 mmol) in DMSO (3 mL). The mixture was stirred at room temperature for 1 h and purified by reversed-phase column chromatography to give benzyl 4-((3-(3-(2,4-dioxotetrahydropyrimidin-1(2*H*)-yl)-4-fluorobenzoyl)-3-azaspiro[5.5]undecan-9-yl)methyl)piperazine-1-carboxylate.

LC-MS: (ESI, m/z): [M+H]⁺ = 620.5.

### Step 2: preparation of 1-(2-fluoro-5-(9-(piperazin-1-ylmethyl)-3-azaspiro[5.5]undecane-3-carbonyl)phenyl)dihydropyrimidine-2,4(1H,3H)-dione

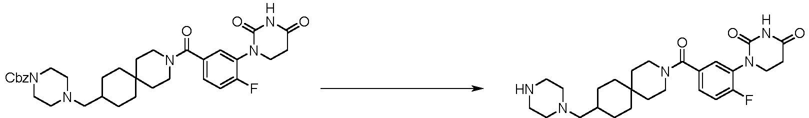

Pd/C (30 mg) was added to a solution of benzyl 4-((3-(3-(2,4-dioxotetrahydropyrimidin-1(2*H*)-yl)-4-fluorobenzoyl)-3-azaspiro[5.5]undecan-9-yl)methyl)piperazine-1-carboxylate (160 mg, 0.25 mmol) in THF (10 mL), and the mixture was stirred at room temperature for 16 h. The reaction liquid was filtered through celite, and the filtrate was concentrated to give 1-(2-fluoro-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 486.3.

### Step 3: preparation of tert-butyl 3-(2-((1-((4-((3-(3-(2,4-dioxotetrahydropyrimidin-1(2H)-yl)-4-fluorobenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

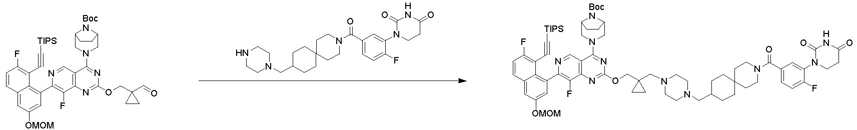

1-(2-Fluoro-5-(9-(piperazin-1-ylmethyl)-3-azaspiro[5.5]undecane-3-carbonyl)phenyl)dihydropyrimidine-2,4(1*H*,3*H*)-dione (69 mg, 0.14 mmol) and tetraisopropyl titanate (507 mg, 1.78 mmol) were added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.119 mmol) in THF (5 mL), and the mixture was stirred at 70 °C for 2 h. At room temperature, MeOH (5 mL) was added to the mixture, and NaBH(OAc)₃ (76 mg, 0.36 mmol) was added in batches. The mixture was stirred for 2 h. The mixture was concentrated and purified by silica gel column chromatography (DCM:MeOH = 102:1 to 20:1) to give *tert*-butyl 3-(2-((1-((4-((3-(3-(2,4-dioxotetrahydropyrimidin-1(2*H*)-yl)-4-fluorobenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 656.9.

### Step 4: preparation of tert-butyl 3-(2-((1-((4-((3-(3-(2,4-dioxotetrahydropyrimidin-1(2H)-yl)-4-fluorobenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo [3.2.1]octane-8-carboxylate

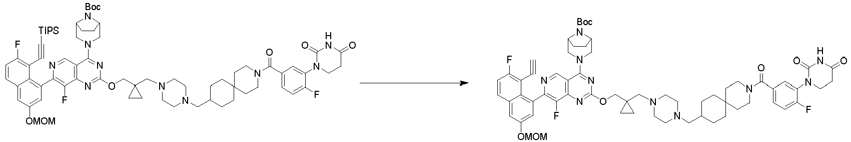

Cesium fluoride (75 mg, 0.49 mmol) was added to a solution of *tert*-butyl 3-(2-((1-((4-((3-(3-(2,4-dioxotetrahydropyrimidin-1(2*H*)-yl)-4-fluorobenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate (130 mg, 0.099 mmol) in DMF (3 mL), and the mixture was stirred at room temperature for 30 min. Water was added, and the mixture was extracted with ethyl acetate. The organic phase was dried over anhydrous sodium sulfate and concentrated to give *tert*-butyl 3-(2-((1-((4-((3-(3-(2,4-dioxotetrahydropyrimidin-1(2*H*)-yl)-4-fluorobenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1155.1.

### Step 5: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1H,3H)-dione

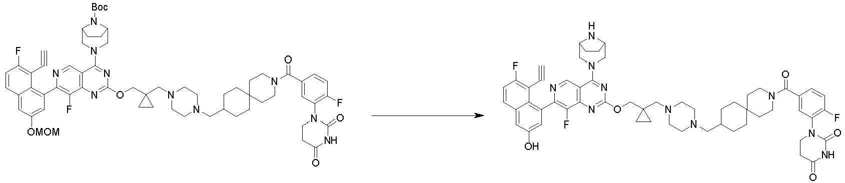

*tert*-Butyl 3-(2-((1-((4-((3-(3-(2,4-dioxotetrahydropyrimidin-1(2*H*)-yl)-4-fluorobenzoyl)-3-azaspiro[5.5]undecan-9-yl)methyl)piperazin-1-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (100 mg, 0.086 mmol) was dissolved in 1,4-dioxane (2 mL), and HCl/1,4-dioxane (6 N, 1 mL) was added. The mixture was reacted at room temperature for 0.5 h. The reaction liquid was concentrated and purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-fluorophenyl)dihydropyrimidine-2,4(1*H*,3*H*)-dione.

LC-MS: (ESI, m/z): [M/2+H]⁺ = 506.3.

¹H NMR (400 MHz, DMSO-*d*₆) δ 10.51 (s, 1H), 10.14 (s, 1H), 9.02 (s, 1H), 8.04 - 7.93 (m, 1H), 7.51 - 7.43 (m, 2H), 7.40 - 7.33 (m, 3H), 7.17 (d, *J* = 2.4 Hz, 1H), 4.49 (d, *J* = 11.3 Hz, 1H), 4.32 - 4.22 (m, 3H), 3.92 (s, 1H), 3.75 (t, *J =* 6.6 Hz, 2H), 3.67 - 3.49 (m, 7H), 2.75 - 2.65 (m, 3H), 2.43 - 2.16 (m, 11H), 2.04 (s, 2H), 1.67 (s, 6H), 1.52 - 1.27 (m, 7H), 1.12 - 0.96 (m, 4H), 0.67 - 0.59 (m, 2H), 0.44 - 0.36 (m, 2H).

### Example 49: 1-(5-(9-((4-((1-(((7-(2-aminobenzo[d]thiazol-4-yl)-4-(3,8-diazabicyclo[3.2.1]octan-3-yl)-8-fluoropyrido[4,3-d]pyrimidin-2-yl)oxy)methyl)cyclopropyl)methyl)piperazin-1-yl)methyl)-3-azaspiro[5.5] undecane-3-carbonyl)-2-methylphenyl)dihydropyrimidine-2,4(1H,3H)-dione formate

### Step 1: preparation of tert-butyl (4-bromobenzo[d]thiazol-2-yl)carbamate

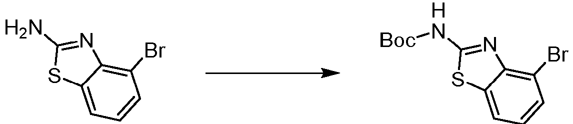

DMAP (214 mg, 1.75 mmol) was added to a solution of 4-bromobenzo[*d*]thiazol-2-amine (4.00 g, 17.46 mmol) in anhydrous dichloromethane (40 mL), and the mixture was cooled to 0 °C. Triethylamine (3.53 g, 34.92 mmol) and di-tert-butyl dicarbonate (5.72 g, 26.19 mmol) were added dropwise to the reaction liquid. The mixture was reacted at 25 °C for 4 h under nitrogen atmosphere. Water (50 mL) was added to quench the reaction, and the mixture was extracted with dichloromethane (50 mL × 3). The organic phases were combined, dried over anhydrous sodium sulfate, and concentrated under reduced pressure. The concentrate was stirred with petroleum ether and filtered to give *tert*-butyl (4-bromobenzo[*d*]thiazol-2-yl)carbamate.

LC-MS: (ESI, m/z): [M-tBu+H]⁺ = 272.9.

¹H NMR (400 MHz, CDCl₃) δ 8.68 (s, 1H), 7.71 (d, *J* = 8.0 Hz, 1H), 7.59 (d, *J* = 7.6 Hz, 1H), 7.15 - 7.10 (m, 1H), 1.54 (s, 9H).

### Step 2: preparation of tert-butyl (4-(1H-naphtho[1,8-de][1,3,2]diazaborinin-2(3H)-yl)benzo[d]thiazol-2-yl)carbamate

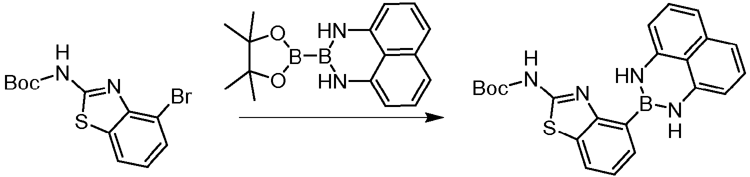

XPhos Pd G2 (104 mg, 1.06 mmol) and potassium acetate (3.13 g, 31.89 mmol) were added to a solution of *tert*-butyl (4-bromobenzo[*d*]thiazol-2-yl)carbamate (3.50 g, 10.63 mmol) and 2-(4,4,5,5-tetramethyl-1,3,2-dioxaborolan-2-yl)-2,3-dihydro-1*H*-naphtho[1,8-*de*][1,3,2]diazaborinine (4.06 g, 13.82 mmol) in 1,4-dioxane (35 mL). The mixture was reacted at 105 °C for 16 h under nitrogen atmosphere. Water (100 mL) was added to quench the reaction, and the mixture was extracted with ethyl acetate (100 mL × 3). The organic phases were combined, dried over anhydrous sodium sulfate, and concentrated under reduced pressure. The concentrate was purified by silica gel column chromatography (PE:EA = 10:1) to give *tert-*butyl (4-(1*H*-naphtho[1,8-*de*][1,3,2]diazaborinin-2(3*H*)-yl)benzo[*d*]thiazol-2-yl)carbamate.

LC-MS: (ESI, m/z): [M+H]⁺ = 417.1.

¹H NMR (400 MHz, CDCl₃) δ 8.32 (s, 1H), 7.87 (d, *J* = 7.6 Hz, 1H), 7.67 (d, *J* = 6.8 Hz, 1H), 7.36 - 7.29 (m, 1H), 7.19 (s, 2H), 7.12 (t, *J =* 8.0 Hz, 2H), 7.03 (d, *J =* 8.0 Hz, 2H), 6.43 (d, *J* = 7.2 Hz, 2H), 1.60 (s, 9H).

### Step 3: preparation of (2-((tert-butoxycarbonyl)amino)benzo[d]thiazol-4-yl)boronic acid

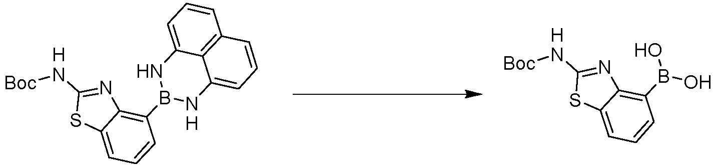

A solution of *tert*-butyl (4-(1*H*-naphtho[1,8-*de*][1,3,2]diazaborinin-2(3*H*)-yl)benzo[*d*]thiazol-2-yl)carbamate (2.50 g, 6.01 mmol) in THF (25 mL) was cooled to 0 °C, and a hydrochloric acid solution (5 N, 18 mL, 90.08 mmol) was added dropwise. The mixture was reacted at room temperature for 16 h. After the reaction was completed, water (100 mL) was added to quench the reaction, and the mixture was extracted with ethyl acetate (100 mL × 3). The organic phases were combined, dried over anhydrous sodium sulfate, and concentrated under reduced pressure. The concentrate was purified by silica gel column chromatography (PE:EA = 5:1) to give (2-((*tert*-butoxycarbonyl)amino)benzo[*d*]thiazol-4-yl)boronic acid.

LC-MS: (ESI, m/z): [M+H]⁺ = 295.0.

### Step 4: preparation of tert-butyl 3-(7-(2-((tert-butoxycarbonyl)amino)benzo[d]thiazol-4-yl)-8-fluoro-2-((1-(hydroxymethyl)cyclopropyl)methoxy)pyrido[4,3-d]pyrimidin-4-yl)-3,8-diazabicyclo[3.2.1]octane-8-carboxylate

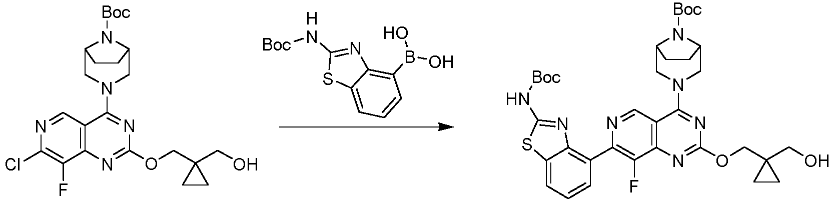

PdCl₂(dtbpf) (52 mg, 0.08 mmol) and cesium carbonate (528 mg, 1.62 mmol) were added to a mixed solution of *tert-butyl* 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (400 mg, 0.81 mmol) and (2-((*tert-*butoxycarbonyl)amino)
benzo[*d*]thiazol-4-yl)boronic acid (286 mg, 0.97 mmol) in 1,4-dioxane (5 mL) and H₂O (1 mL). The mixture was reacted at 85 °C for 16 h under nitrogen atmosphere. Water (10 mL) was added to quench the reaction, and the mixture was extracted with ethyl acetate (20 mL × 3). The organic phases were combined, dried over anhydrous sodium sulfate, and concentrated under reduced pressure. The concentrate was purified by silica gel column chromatography (PE:EA= 1:1) to give *tert*-butyl 3-(7-(2-((*tert*-butoxycarbonyl)amino)benzo [*d*]thiazol-4-yl)-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate.

LC-MS: (ESI, m/z): [M-H]⁻ = 706.0.

### Step 5: preparation of tert-butyl 3-(7-(2-((tert-butoxycarbonyl)amino)benzo[d]thiazol-4-yl)-8-fluoro-2-((1-formylcyclopropyl)methoxy)pyrido[4,3-d]pyrimidin-4-yl)-3,8-diazabicyclo[3.2.1]octane-8-carboxylate

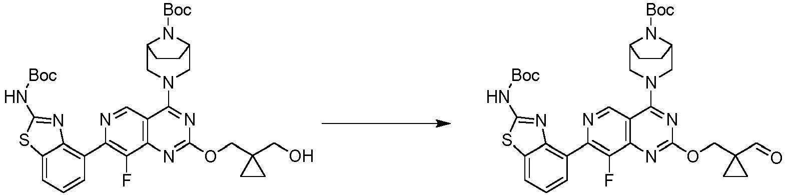

A solution of *tert*-butyl 3-(7-(2-((*tert*-butoxycarbonyl)amino)benzo[*d*]thiazol-4-yl)-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (300 mg, 0.42 mmol) in DCM (3 mL) was cooled to 0 °C, and Dess-Martin reagent (534 mg, 1.26 mmol) was added. The mixture was reacted at room temperature for 0.5 h. After the reaction was completed, water (10 mL) was added to quench the reaction, and the mixture was extracted with dichloromethane (10 mL × 3). The organic phases were combined, dried over anhydrous sodium sulfate, and concentrated under reduced pressure. The concentrate was purified by silica gel column chromatography (DCM:MeOH = 50:1) to give *tert*-butyl 3-(7-(2-((*tert*-butoxycarbonyl)amino)benzo[*d*]thiazol-4-yl)-8-fluoro-2-((1-formylcyclopropyl)
methoxy)pyrido[4,3-*d*]pyrimidin-4-yl)-3,8-diazabicyclo[3.2.1]octane-8-carboxylate.

LC-MS: (ESI, m/z): [M-H]⁻ = 704.1.

### Step 6: preparation of tert-butyl 3-(2-((1-((4-((benzyloxy)carbonyl)piperazin-1-yl)methyl)cyclopropyl)methoxy)-7-(2-((tert-butoxycarbonyl)amino)benzo[d]thiazol-4-yl)-8-fluoropyrido[4,3-d]pyrimidin-4-yl)-3,8-diazabicyclo[3.2.1]octane-8-carboxylate

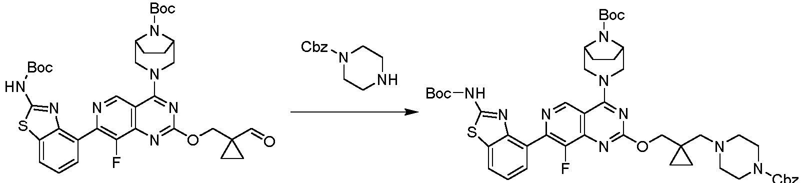

Tetraisopropyl titanate (403 mg, 1.42 mmol) and acetic acid (0.1 mL) were added to a stirred solution of *tert*-butyl 3-(7-(2-((*tert*-butoxycarbonyl)amino)benzo[*d*]thiazol-4-yl)-8-fluoro-2-((1-formylcyclopropyl)methoxy)pyrido[4,3-*d*]pyrimidin-4-yl)-3,8-diazabicyclo[3.2.1]octane-8-carboxylate (200 mg, 0.28 mmol) and benzyl piperazine-1-carboxylate (75 mg, 0.34 mmol) in DCM (1 mL), and the mixture was stirred at 30 °C for 2 h. NaBH(OAc)₃ (180 mg, 0.85 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-((1-((4-((benzyloxy)carbonyl)piperazin-1-yl)methyl)cyclopropyl)methoxy)-7-(2-((*tert*butoxycarbonyl)amino)benzo[*d*]thiazol-4-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 455.8.

### Step 7: preparation of tert-butyl 3-(7-(2-((tert-butoxycarbonyl)amino)benzo[d]thiazol-4-yl)-8-fluoro-2-((1-(piperazin-1-ylmethyl)cyclopropyl)methoxy)pyrido[4,3-d]pyrimidin-4-yl)-3,8-diazabicyclo[3.2.1]octane-8-carboxylate

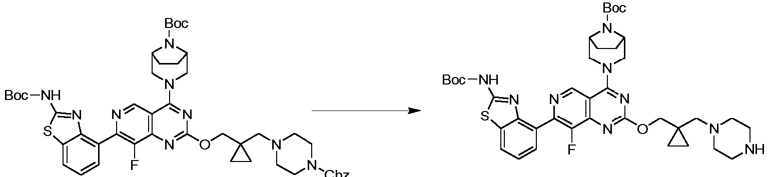

Palladium on carbon (200 mg) was added to a solution of *tert-butyl* 3-(2-((1-((4-((benzyloxy)carbonyl) piperazin-1-yl)methyl)cyclopropyl)methoxy)-7-(2-((*tert*-butoxycarbonyl)amino)benzo[*d*]thiazol-4-yl)-8-fluoropyrido[4,3-*d*]pyrimidin-4-yl)-3,8-diazabicyclo[3.2.1]octane-8-carboxylate (100 mg, 0.11 mmol) in methanol (1 mL) under H₂ atmosphere, and the mixture was reacted at room temperature for 16 h. The reaction liquid was filtered through celite, and the filtrate was concentrated to give *tert*-butyl 3-(7-(2-((*tert-*butoxycarbonyl)amino)benzo[*d*]thiazol-4-yl)-8-fluoro-2-((1-(piperazin-1-ylmethyl)cyclopropyl)methoxy) pyrido[4,3-*d*]pyrimidin-4-yl)-3,8-diazabicyclo[3.2.1]octane-8-carboxylate, which was directly used in the next step.

LC-MS: (ESI, m/z): [M+H]⁺ = 776.4.

### Step 8: preparation of tert-butyl 3-(7-(2-((tert-butoxycarbonyl)amino)benzo[d]thiazol-4-yl)-2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoropyrido[4,3-d]pyrimidin-4-yl)-3,8-diazabicyclo[3.2.1]octane-8-carboxylate

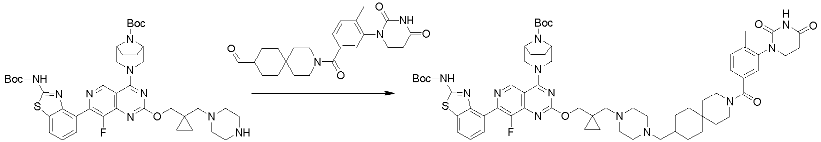

Tetraisopropyl titanate (119 mg, 0.418 mmol) and acetic acid (0.1 mL) were added to a stirred solution of *tert*-butyl 3-(7-(2-((*tert*-butoxycarbonyl)amino)benzo[*d*]thiazol-4-yl)-8-fluoro-2-((1-(piperazin-1-ylmethyl) cyclopropyl)methoxy)pyrido[4,3-*d*]pyrimidin-4-yl)-3,8-diazabicyclo[3.2.1]octane-8-carboxylate (65 mg, 0.083 mmol) and 3-(3-(2,4-dioxotetrahydropyrimidin-1(2*H*)-yl)-4-methylbenzoyl)-3-azaspiro[5.5]undecane-9-carbaldehyde (52 mg, 0.126 mmol) in DCM (1 mL), and the mixture was stirred at 30 °C for 2 h. NaBH(OAc)₃ (53 mg, 0.251 mmol) was added to the mixture at room temperature, and the resulting mixture was stirred for 2 h. The mixture was concentrated and purified by silica gel column chromatography (MeOH:DCM = (0-1):4) to give *tert*-butyl 3-(7-(2-((*tert*butoxycarbonyl)amino)benzo[*d*]thiazol-4-yl)-2-((1-((4-((3-(3-(2,4-dioxotetrahydropyrimidin-1(2*H*)-yl)-4-methylbenzoyl)-3-azaspiro[5.5]undecan-9-yl)methyl) piperazin-1-yl)methyl)cyclopropyl)methoxy)-8-fluoropyrido[4,3-*d*]pyrimidin-4-yl)-3,8-diazabicyclo[3.2.1] octane-8-carboxylate.

LC-MS: (ESI, m/z): [M/2+H]⁺ = 586.4.

### Step 9: preparation of 1-(5-(9-((4-((1-(((7-(2-aminobenzo[d]thiazol-4-yl)-4-(3,8-diazabicyclo[3.2.1]octan-3-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-methylphenyl)dihydropyrimidine-2,4(1H,3H)-dione formate

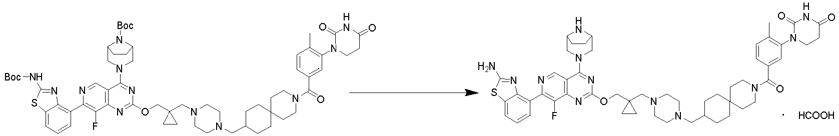

HCl/1,4-dioxane (6 N, 1.0 mL) was added to a stirred solution of *tert*-butyl 3-(7-(2-((*tert*butoxycarbonyl)amino)benzo[*d*]thiazol-4-yl)-2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-3,8-diazabicyclo[3.2.1]octane-8-carboxylate (48 mg, 0.041 mmol) in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((1-(((7-(2-aminobenzo[*d*]thiazol-4-yl)-4-(3,8-diazabicyclo[3.2.1]octan-3-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione formate.

LC-MS: (ESI, m/z): [M+H]⁺ = 971.2.

¹H NMR (400 MHz, CD₃OD) δ 9.05 (s, 1H), 8.47 (s, 1H), 7.77 (d, *J* = 7.6 Hz, 1H), 7.47 - 7.39 (m, 2H), 7.35 - 7.28 (m, 2H), 7.26 - 7.20 (m, 1H), 4.70 (d, *J =* 12.8 Hz, 2H), 4.46 (s, 2H), 3.89 - 3.82 (m, 3H), 3.80 - 3.74 (m, 2H), 3.73 - 3.63 (m, 3H), 3.48 - 3.37 (m, 3H), 2.98 - 2.78 (m, 9H), 2.66 (s, 2H), 2.59-2.47 (m, 2H), 2.32 (s, 3H), 1.99 - 1.86 (m, 4H), 1.81 - 1.72 (m, 2H), 1.66 - 1.55 (m, 4H), 1.50 - 1.41 (m, 2H), 1.34 - 1.28 (m, 1H), 1.24 - 1.09 (m, 4H), 0.82 - 0.76 (m, 2H), 0.62 - 0.56 (m, 2H).

### Example 50: 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

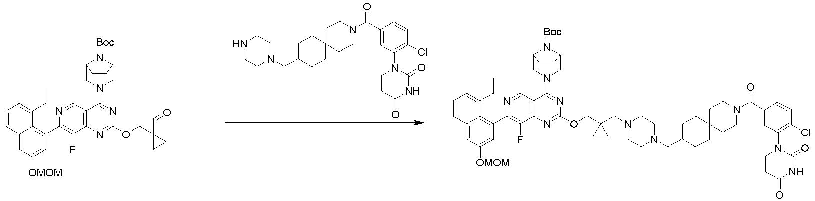

Tetraisopropyl titanate (211 mg, 0.744 mmol) was added to a stirred solution of *tert*-butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate (100 mg, 0.148 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (90 mg, 0.178 mmol) in DCM (1 mL), and the mixture was stirred at 30 °C for 2 h. NaBH(OAc)₃ (95 mg, 0.446 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 579.4.

### Step 2: preparation of 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

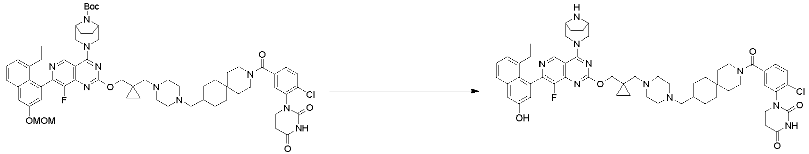

HCl/1,4-dioxane (6 N, 1.0 mL) was added to a stirred solution of *tert-butyl* 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate (103 mg, 0.088 mmol) in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 1013.2.
¹H NMR (400 MHz, DMSO-*d*₆) δ 10.50 (s, 1H), 9.87 (s, 1H), 9.07 (s, 1H), 7.69 - 7.60 (m, 2H), 7.54 (d, *J*= 1.6 Hz, 1H), 7.40 - 7.32 (m, 2H), 7.27 (d*, J* = 2.4 Hz, 1H), 7.12 (d, *J* = 6.8 Hz, 1H), 6.96 (d, *J* = 2.4 Hz, 1H), 4.42 (d, *J* = 12.4 Hz, 2H), 4.35 - 4.22 (m, 2H), 3.82 - 3.69 (m, 1H), 3.64 - 3.48 (m, 7H), 2.77 - 2.70 (m, 2H), 2.42 - 1.98 (m, 15H), 1.70 -1.57 (m, 6H), 1.56 - 1.16 (m, 9H), 1.11 - 0.91 (m, 4H), 0.81 (t, *J =* 7.2 Hz, 3H), 0.67 - 0.58 (m, 2H), 0.42 - 0.35 (m, 2H).

### Example 51: 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-d]pyrimidin-4-yl)-3,8-diazabicyclo[3.2.1]octane-8-carboxylate

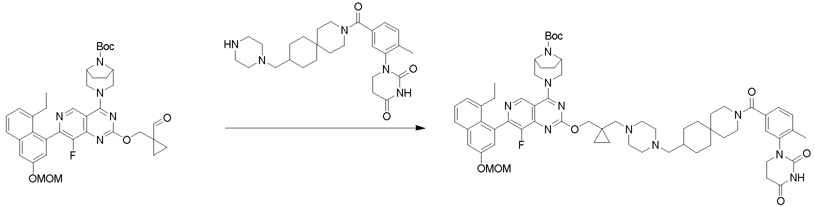

Tetraisopropyl titanate (211 mg, 0.744 mmol) was added to a stirred solution of *tert*-butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,8-diazabicyclo[3.2.1]octane-8-carboxylate (100 mg, 0.148 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (86 mg, 0.178 mmol) in DCM (2 mL), and the mixture was stirred at 30 °C for 2 h. NaBH(OAc)₃ (95 mg, 0.446 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 569.4.

### Step 2: preparation of 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

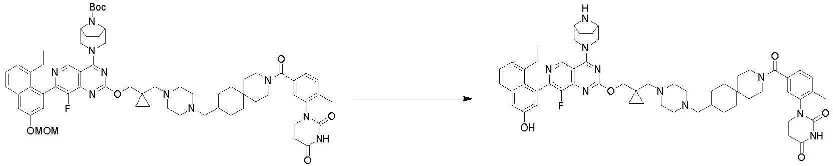

HCl/1,4-dioxane (6 N, 1.0 mL) was added to a stirred solution of *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,8-diazabicyclo[3.2.1]octane-8-carboxylate (107 mg, 0.094 mmol) in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 497.2.

¹H NMR (400 MHz, DMSO-*d*₆) δ 10.36 (s, 1H), 9.87 (s, 1H), 9.07 (s, 1H), 7.67 (d, *J =* 8.0 Hz, 1H), 7.39 - 7.20 (m, 5H), 7.12 (d, *J =* 6.8 Hz, 1H), 6.97 (d, *J* = 2.8 Hz, 1H), 4.42 (d, *J =* 11.6 Hz, 2H), 4.35 - 4.22 (m, 2H), 3.86 - 3.74 (m, 1H), 3.64 - 3.44 (m, 7H), 2.87 - 2.58 (m, 3H), 2.43 - 2.14 (m, 15H), 2.03 (s, 3H), 1.71-1.58 (m, 6H), 1.55 - 1.21 (m, 8H), 1.14 - 0.90 (m, 4H), 0.81 (t, *J =* 7.2 Hz, 3H), 0.65 - 0.58 (m, 2H), 0.43 - 0.34 (m, 2H).

### Example 52: 1-(5-(9-(4-(4-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)butyl)-3-azaspiro[5.5]undecane-3-carbonyl)-2-chlorophenyl)dihydropyrimidine-2,4(1H,3H)-dione

### Step 1: preparation of tert-butyl 3-(2-((1-(4-(3-(4-chloro-3-(2,4-dioxotetrahydropyrimidin-1(2H)-yl)benzoyl)-3-azaspiro[5.5]undecan-9-yl)butyl)piperidin-4-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

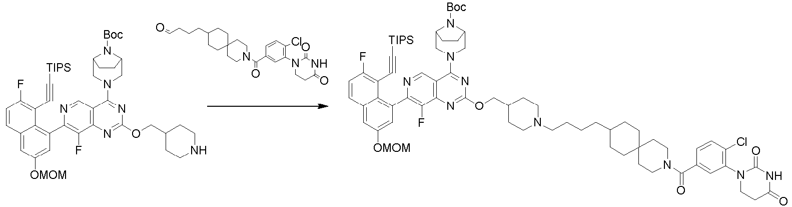

Acetic acid (0.1 mL) was added to a stirred solution of *tert-butyl* 3-(8-fluoro-7-(7-fluoro-3-(methoxymethoxy)-8-((triisopropylsilyl)ethynyl)naphthalen-1-yl)-2-(piperidin-4-ylmethoxy)pyrido[4,3-*d*]pyrimidin-4-yl)-3,8-diazabicyclo[3.2.1]octane-8-carboxylate (100 mg, 0.120 mmol) and 4-(3-(4-chloro-3-(2,4-dioxotetrahydropyrimidin-1(2*H*)-yl)benzoyl)-3-azaspiro[5.5]undecan-9-yl)butanal (68 mg, 0.144 mmol) in DCM/MeOH (0.5 mL/0.5 mL), and the mixture was stirred at 30 °C for 2 h. NaBH(OAc)₃ (76 mg, 0.360 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-((1-(4-(3-(4-chloro-3-(2,4-dioxotetrahydropyrimidin-1(2*H*)-yl)benzoyl)-3-azaspiro[5.5]undecan-9-yl)butyl)piperidin-4-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 658.0.

### Step 2: preparation of 1-(5-(9-(4-(4-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)butyl)-3-azaspiro[5.5]undecane-3-carbonyl)-2-chlorophenyl)dihydropyrimidine-2,4(1H,3H)-dione

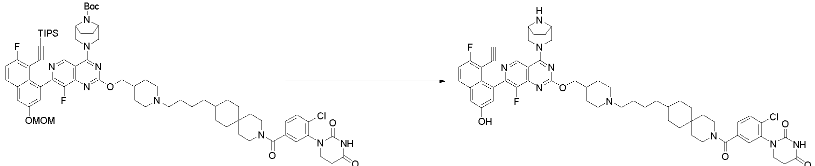

Cesium fluoride (80 mg, 0.525 mmol) was added to a stirred solution of *tert*-butyl 3-(2-((1-(4-(3-(4-chloro-3-(2,4-dioxotetrahydropyrimidin-1(2*H*)-yl)benzoyl)-3-azaspiro[5.5]undecan-9-yl)butyl)piperidin-4-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (138 mg, 0.088 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-(4-(4-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)piperidin-1-yl)butyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 508.3.

¹H NMR (400 MHz, DMSO-*d*₆) δ 10.50 (s, 1H), 10.13 (s, 1H), 9.03 (s, 1H), 8.00 - 7.93 (m, 1H), 7.64 (d, *J* = 8.4 Hz, 1H), 7.55 - 7.52 (m, 1H), 7.49 - 7.43 (m, 1H), 7.40 - 7.35 (m, 2H), 7.19 - 7.14 (m, 1H), 4.50 - 4.41 (m, 1H), 4.34 - 4.26 (m, 1H), 4.21 - 4.16 (m, 2H), 3.93 (s, 1H), 3.81 - 3.70 (m, 1H), 3.65 - 3.51 (m, 7H), 2.89 - 2.81 (m, 2H), 2.77 - 2.70 (m, 2H), 2.26 - 2.19 (m, 2H), 1.89 -1.80 (m, 2H), 1.75 - 1.63 (m, 9H), 1.52 - 1.45 (m, 3H), 1.43 - 1.35 (m, 3H), 1.34 - 1.14 (m, 12H), 1.10 - 0.96 (m, 4H).

### Example 53: 1-(5-(9-(2-(4-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)ethyl)-3-azaspiro[5.5]undecane-3-carbonyl)-2-chlorophenyl)dihydropyrimidine-2,4(1H,3H)-dione

### Step 1: preparation of tert-butyl 3-(2-((1-(2-(3-(4-chloro-3-(2,4-dioxotetrahydropyrimidin-1(2H)-yl)benzoyl)-3-azaspiro[5.5]undecan-9-yl)ethyl)piperidin-4-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

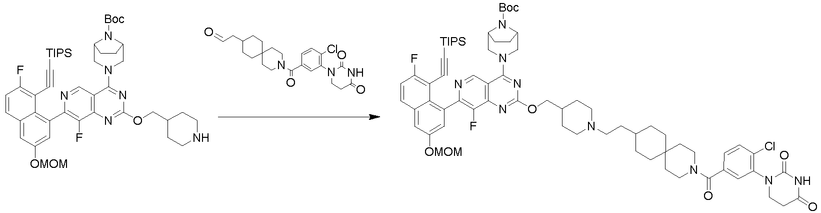

Tetraisopropyl titanate (166 mg, 0.583 mmol) and acetic acid (0.1 mL) were added to a stirred solution of *tert-butyl* 3-(8-fluoro-7-(7-fluoro-3-(methoxymethoxy)-8-((triisopropylsilyl)ethynyl)naphthalen-1-yl)-2-(piperidin-4-ylmethoxy)pyrido[4,3-*d*]pyrimidin-4-yl)-3,8-diazabicyclo[3.2.1]octane-8-carboxylate (100 mg, 0.117 mmol) and 2-(3-(4-chloro-3-(2,4-dioxotetrahydropyrimidin-1(2*H*)-yl)benzoyl)-3-azaspiro[5.5]undecan-9-yl)acetaldehyde (62 mg, 0.140 mmol) in DCM (1 mL), and the mixture was stirred at 30 °C for 2 h. NaBH(OAc)₃ (74 mg, 0.350 mmol) was added to the mixture at room temperature, and the resulting mixture was stirred for 2 h. The mixture was concentrated and purified by silica gel column chromatography (MeOH:DCM = (0-1):4) to give *tert*-butyl 3-(2-((1-(2-(3-(4-chloro-3-(2,4-dioxotetrahydropyrimidin-1(2*H*)-yl)benzoyl)-3-azaspiro[5.5]undecan-9-yl)ethyl)piperidin-4-yl)methoxy)-8-fluoro-7-(7-fluoro-3-(methoxymethoxy)-8-((triissopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 643.9.

### Step 2: preparation of 1-(5-(9-(2-(4-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)ethyl)-3-azaspiro[5.5]undecane-3-carbonyl)-2-chlorophenyl)dihydropyrimidine-2,4(1H,3H)-dione

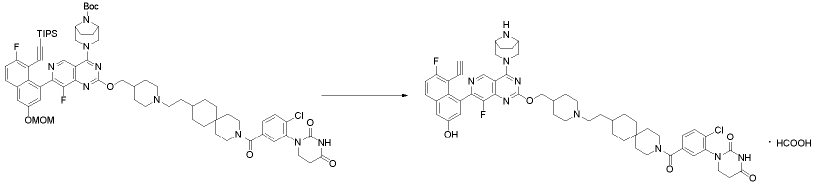

Cesium fluoride (46 mg, 0.303 mmol) was added to a stirred solution of *tert-butyl* 3-(2-((1-(2-(3-(4-chloro-3-(2,4-dioxotetrahydropyrimidin-1(2*H*)-yl)benzoyl)-3-azaspiro[5.5]undecan-9-yl)ethyl)piperidin-4-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (78 mg, 0.061 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-(2-(4-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)piperidin-1-yl)ethyl)-3-azaspiro[5.5] undecane-3-carbonyl)-2-chlorophenyl)dihydropyrimidine-2,4(1*H*,3*H*)-dione.

LC-MS: (ESI, m/z): [M/2+H]⁺ = 493.8.

¹H NMR (400 MHz, CD₃OD) δ 9.05 (s, 1H), 8.45 (s, 2H), 7.91 - 7.83 (m, 1H), 7.65 (d, *J* = 8.4 Hz, 1H), 7.55 - 7.51 (m, 1H), 7.46 - 7.39 (m, 1H), 7.38 - 7.29 (m, 2H), 7.24 - 7.18 (m, 1H), 4.79 - 4.70 (m, 2H), 4.51 - 4.35 (m, 2H), 4.16 - 4.05 (m, 2H), 3.95 - 3.86 (m, 2H), 3.82 - 3.76 (m, 2H), 3.75 - 3.66 (m, 2H), 3.65 - 3.55 (m, 2H), 3.46 - 3.35 (m, 3H), 3.17 - 3.08 (m, 2H), 3.07 - 2.95 (m, 2H), 2.92 - 2.78 (m, 2H), 2.22 - 2.00 (m, 7H), 1.83 - 1.71 (m, 4H), 1.70 - 1.42 (m, 7H), 1.40 - 1.31 (m, 2H), 1.28 - 1.11 (m, 4H).

### Example 54: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)thio)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of ((1-(hydroxymethyl)cyclopropyl)methyl) ethanethioate

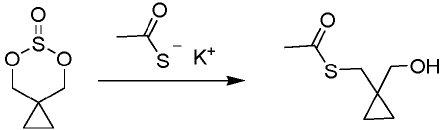

5,7-Dioxo-6-thio[2.5]octane 6-oxide (5.0 g, 33.8 mmol) was dissolved in DMSO (30 mL). The mixture was added to potassium thioacetate (7.7 g, 67.6 mmol). The reaction liquid was heated at 40 °C for 5 h. After the reaction was completed, water (100 mL) was added, and the mixture was extracted with ethyl acetate (50 mL × 2). The organic phase was washed with a saturated sodium chloride solution and concentrated to give ((1-(hydroxymethyl)cyclopropyl)methyl) ethanethioate.

¹H NMR (400 MHz, CDCl₃) δ 3.35 (s, 2H), 3.02 (s, 2H), 2.38 (s, 3H), 0.61 - 0.47 (d, *J =* 7.2 Hz, 4H).

### Step 2: preparation of ((1-(((tert-butyldimethylsilyl)oxy)methyl)cyclopropyl)methyl) ethanethioate

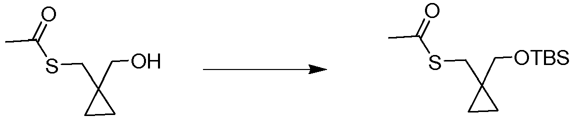

NaH (0.75 g, 18.7 mmol, 60%) was suspended in THF (30 mL), and the mixture was cooled to 0 °C. A solution of ((1-(hydroxymethyl)cyclopropyl)methyl) ethanethioate (2.0 g, 12.5 mmol) in THF (5 mL) was added dropwise. The mixture was reacted at room temperature for 2 h. After the reaction was completed, an aqueous ammonium chloride solution was added, and the mixture was extracted with ethyl acetate (50 mL × 2). The organic phase was washed with a saturated sodium chloride solution and concentrated to give ((1-(((tert-butyldimethylsilyl)oxy)methyl)cyclopropyl)methyl) ethanethioate.

¹H NMR (400 MHz, CDCl₃) δ 3.44 (s, 2H), 3.04 (s, 2H), 2.33 (s, 3H), 0.90 (s, 9H), 0.52 - 0.47 (s, 4H), 0.04 (s, 6H).

### Step 3: preparation of (1-(((tert-butyldimethylsilyl)oxy)methyl)cyclopropyl)methanethiol

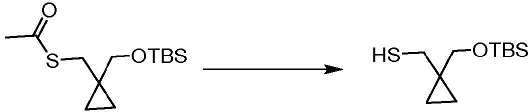

((1-(((tert-Butyldimethylsilyl)oxy)methyl)cyclopropyl)methyl) ethanethioate (1.5 g, 5.46 mmol) was dissolved in ethanol (20 mL), and an aqueous sodium hydroxide solution (2 N, 10 mL) was added. The mixture was reacted at room temperature for 1 h. The mixture was extracted with methyl *tert-butyl* ether to remove impurities, and the aqueous phase was adjusted to pH = 3 with an aqueous hydrochloric acid solution (1 N) and extracted with ethyl acetate (50 mL × 2). The ethyl acetate phase was washed with a saturated sodium chloride solution and concentrated to give (1-(((*tert*butyldimethylsilyl)oxy)methyl)cyclopropyl)methanethiol.

¹H NMR (400 MHz, DMSO-*d*₆) δ 3.53 (s, 2H), 2.54 (d, *J* = 7.9 Hz, 2H), 2.11 (t, *J* = 7.9 Hz, 1H), 0.87 (s, 9H), 0.48 - 0.39 (m, 4H), 0.04 (m, 6H).

### Step 4: preparation of tert-butyl 3-(2-(((1-(((tertbutyldimethylsllyl)oxy)methyl)cyclopropyl)methyl)thio)-7-chloro-8-fluoropyrido[4,3-d]pyrimidin-4-yl)-3,8-diazabicyclo[3.2.1]octane-8-carboxylate

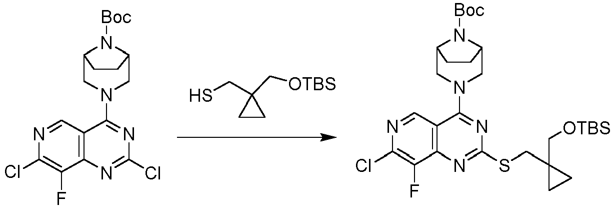

*tert*-Butyl 3-(2,7-dichloro-8-fluoropyrido[4,3-*d*]pyrimidin-4-yl)-3,8-diazabicyclo[3.2.1]octane-8-carboxylate (0.5 g, 1.17 mmol) was added to a mixed solution of (1-(((*tert*butyldimethylsilyl)oxy)methyl)cyclopropyl) methanethiol (0.41 g, 1.75 mmol) and cesium carbonate (0.76 g, 2.34 mmol) in THF (8 mL), and the mixture was reacted at 25 °C for 2 h. After the reaction was completed, water (30 mL) was added, and the mixture was extracted with ethyl acetate (30 mL × 2). The organic phases were combined, dried over anhydrous sodium sulfate, and concentrated under reduced pressure. The concentrate was purified by silica gel column chromatography (PE:EA = 5:1) to give *tert*-butyl 3-(2-(((1-(((*tert*butyldimethylsilyl)oxy)methyl)cyclopropyl) methyl)thio)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 624.1.

### Step 5: preparation of tert-butyl 3-(7-chloro-8-fluoro-2-(((1-(hydroxymethyl)cyclopropyl)methyl)thio)pyrido [4,3-d]pyrimidin-4-yl)-3,8-diazabicyclo[3.2.1]octane-8-carboxylate

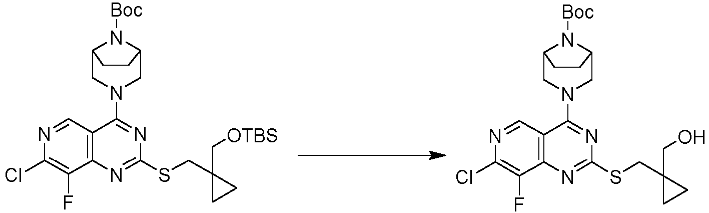

*tert*-Butyl 3-(2-(((1-(((tert-butyldimethylsilyl)oxy)methyl)cyclopropyl)methyl)thio)-7-chloro-8-fluoropyrido[4,3-*d*]pyrimidin-4-yl)-3,8-diazabicyclo[3.2.1]octane-8-carboxylate (450 mg, 0.72 mmol) was dissolved in THF (5 mL). TBAF (1 M, 1.3 mL) was added at 0 °C, and the mixture was reacted for half an hour. After the reaction was completed, water was added, and the mixture was extracted with ethyl acetate. The organic phase was dried over anhydrous sodium sulfate, filtered, concentrated, and purified by silica gel column chromatography (DCM:MeOH = 100:1) to give *tert*-butyl 3-(7-chloro-8-fluoro-2-(((1-(hydroxymethyl)cyclopropyl)methyl)thio)pyrido[4,3-*d*]pyrimidin-4-yl)-3,8-diazabicyclo[3.2.1]octane-8-carboxylate.

LC-MS: (ESI, m/z): [M+H]⁺ = 510.0.

### Step 6: preparation of tert-butyl 3-(8-fluoro-7-(7-fluoro-3-(methoxymethoxy)-8-((triisopropylsilyl)ethynyl) naphthalen-1-yl)-2-(((1-(hydroxymethyl)cyclopropyl)methyl)thio)pyrido[4,3-d]pyrimidin-4-yl)-3,8-diazabicyclo[3.2.1]octane-8-carboxylate

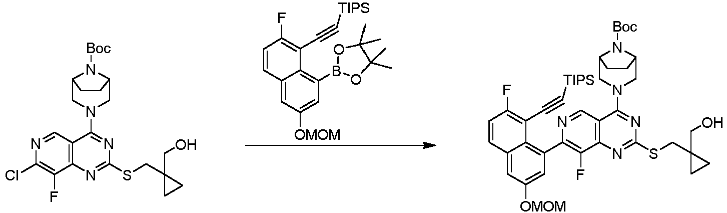

cataCXium A Pd G3 (43 mg, 0.059 mmol) and cesium carbonate (391 mg, 1.2 mmol) were added to a mixed solution of *tert-butyl* 3-(7-chloro-8-fluoro-2-(((1-(hydroxymethyl)cyclopropyl)methyl)thio)pyrido[4,3-*d*]pyrimidin-4-yl)-3,8-diazabicyclo[3.2.1]octane-8-carboxylate (300 mg, 0.59 mmol) and (2-fluoro-6-(methoxymethoxy)-8-(4,4,5,5-tetramethyl-1,3,2-dioxaborolan-2-yl)naphthalen-1-yl)ethynyl)triisopropylsilane (393 mg, 0.77 mmol) in 1,4-dioxane (5 mL) and H₂O (1 mL). The mixture was reacted at 85 °C for 6 h under nitrogen atmosphere. The reaction liquid was directly filtered and concentrated under reduced pressure. The concentrate was purified by silica gel column chromatography (DCM:MeOH = 100:1) to give *tert*-butyl 3-(8-fluoro-7-(7-fluoro-3-(methoxymethoxy)-8-((triisopropylsilyl)ethynyl)naphthalen-1-yl)-2-(((1-(hydroxymethyl) cyclopropyl)methyl)thio)pyrido[4,3-*d*]pyrimidin-4-yl)-3,8-diazabicyclo[3.2.1]octane-8-carboxylate.

LC-MS: (ESI, m/z): [M+H]⁺ = 860.0.

### Step 7: preparation of tert-butyl 3-(8-fluoro-7-(7-fluoro-3-(methoxymethoxy)-8-((triisopropylsilyl)ethynyl)naphthalen-1-yl)-2-(((1-formylcyclopropyl)methyl)thio)pyrido[4,3-d]pyrimidin-4-yl)-3,8-diazabicyclo[3.2.1]octane-8-carboxylate

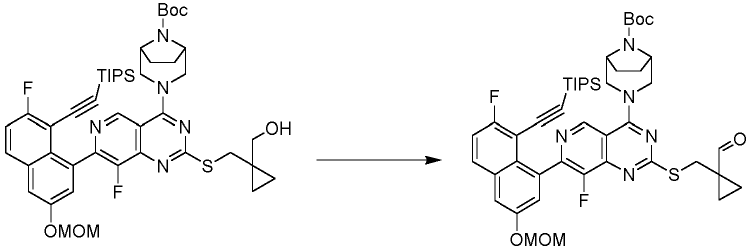

Dess-Martin reagent (207 mg, 0.48 mmol) was added to a solution of *tert*-butyl 3-(8-fluoro-7-(7-fluoro-3-(methoxymethoxy)-8-((triisopropylsilyl)ethynyl)naphthalen-1-yl)-2-(((1-(hydroxymethyl)cyclopropyl) methyl)thio)pyrido[4,3-*d*]pyrimidin-4-yl)-3,8-diazabicyclo[3.2.1]octane-8-carboxylate (280 mg, 0.325 mmol) in dichloromethane (5 mL). The mixture was stirred at room temperature for 2 h. After the reaction was completed, a sodium thiosulfate solution (5 mL) and a sodium bicarbonate solution (5 mL) were added to quench the reaction, and the mixture was extracted with dichloromethane (10 mL × 3). The organic phases were combined, dried over anhydrous sodium sulfate, and concentrated under reduced pressure. The concentrate was purified by silica gel column chromatography (PE:EA = 1:1) to give *tert*-butyl 3-(8-fluoro-7-(7-fluoro-3-(methoxymethoxy)-8-((triisopropylsilyl)ethynyl)naphthalen-1-yl)-2-(((1-formylcyclopropyl) methyl)thio)pyrido[4,3-*d*]pyrimidin-4-yl)-3,8-diazabicyclo[3.2.1]octane-8-carboxylate.

LC-MS: (ESI, m/z): [M+H]⁺ = 858.0.

### Step 8: preparation of tert-butyl 3-(2-(((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methyl)thio)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

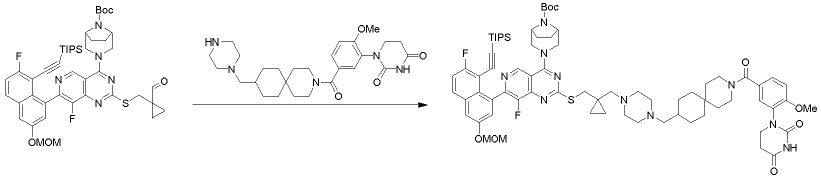

1-(2-Methoxy-5-(9-(piperazin-1-ylmethyl)-3-azaspiro[5.5]undecane-3-carbonyl)phenyl)dihydropyrimidine-2,4(1H,3H)-dione (65 mg, 0.13 mmol) and tetraisopropyl titanate (170 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-(((1-formylcyclopropyl)methyl)thio)pyrido[4,3-*d*]pyrimidin-4-yl)-3,8-diazabicyclo[3.2.1]octane-8-carboxylate (95 mg, 0.11 mmol) in dichloromethane (1.2 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM:MeOH = 20:1) to give *tert-butyl* 3-(2-(((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methyl)thio)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 1339.2.

### Step 9: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)thio)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

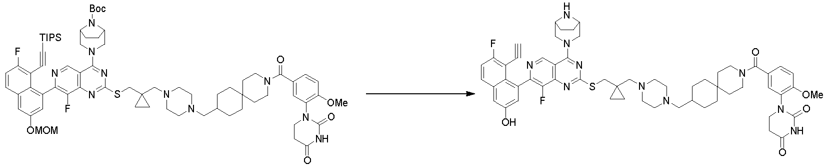

*tert*-Butyl 3-(2-(((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methyl)thio)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (70 mg, 0.052 mmol) was dissolved in DMF (2 mL), and CsF (102 mg, 0.67 mmol) was added. The mixture was reacted at room temperature for 1 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (10 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)thio)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 520.2.

¹H NMR (400 MHz, DMSO-*d*₆) δ 10.33 (s, 1H), 10.15 (s, 1H), 9.03 (s, 1H), 7.98 (dd, *J* = 9.2, 5.9 Hz, 1H), 7.51 - 7.42 (m, 1H), 7.42 - 7.35 (m, 2H), 7.32 (d, *J* = 2.1 Hz, 1H), 7.22 - 7.07 (m, 2H), 4.48 (d, *J* = 12.3 Hz, 1H), 4.25 (d, *J* = 12.0 Hz, 1H), 3.96 (s, 1H), 3.84 (s, 3H), 3.69 - 3.33 (m, 12H), 2.68 (t, *J* = 6.7 Hz, 2H), 2.48 - 2.13 (m, 11H), 2.05 (d, *J* = 6.8 Hz, 2H), 1.61 - 1.70 (m, 5H), 1.57 - 1.39 (m, 5H), 1.35 - 1.17 (m, 3H), 1.15 - 0.93 (m, 4H), 0.69 - 0.55 (m, 2H), 0.42 - 0.30 (m, 2H).

### Example 55: 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

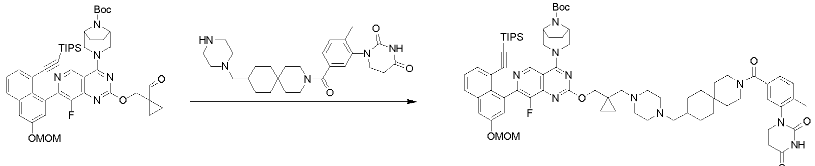

1-(2-Methyl-5-(9-(piperazin-1-ylmethyl)-3-azaspiro[5.5]undecane-3-carbonyl)phenyl)dihydropyrimidine-2,4(1*H*,3*H*)-dione (56 mg, 0.12 mmol) and tetraisopropyl titanate (170 mg, 0.6 mmol) were added to a solution of *tert*-butyl 3-(8-fluoro-2-((1-formylcyclopropyl)methoxy)-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (80 mg, 0.097 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 645.4.

### Step 2: preparation of 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

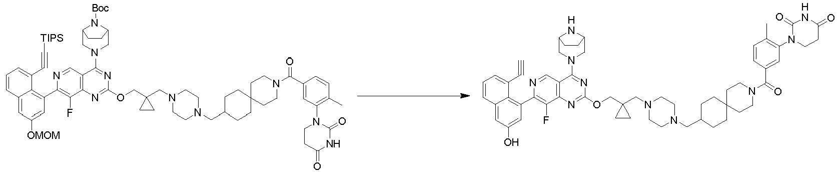

*tert*-Butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(3 (methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (60 mg, 0.047 mmol) was dissolved in DMF (2 mL), and CsF (71 mg, 0.47 mmol) was added. The mixture was reacted at room temperature for 1 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (10 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 495.2.

¹H NMR (400 MHz, DMSO-*d*₆) δ 10.37 (s, 1H), 10.13 (s, 1H), 9.01 (s, 1H), 7.94 - 7.84 (m, 1H), 7.48 - 7.33 (m, 2H), 7.31 - 7.28 (m, 3H), 7.24 (d, *J* = 7.7 Hz, 1H), 7.11 (d, *J* = 2.5 Hz, 1H), 4.49 (d, *J* = 10.9 Hz, 1H), 4.34 - 4.17 (m, 3H), 3.86 - 3.74 (m, 1H), 3.68 - 3.44 (m, 8H), 3.43 - 3.35 (m, 2H), 2.85 - 2.56 (m, 3H), 2.46 - 2.14 (m, 13H), 2.12 - 1.91 (m, 3H), 1.69 - 1.62 (m, 5H), 1.53 - 1.22 (m, 8H), 1.12 - 0.91 (m, 4H), 0.66- 0.54 (m, 2H), 0.42 - 0.32 (m, 2H).

### Example 56: 1-(5-(9-(2-(4-((3-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)bicyclo[1.1.1]pentan-1-yl)methyl)piperazin-1-yl)ethyl)-3-azaspiro[5.5]undecane-3-carbonyl)-2-chlorophenyl)dihydropyrimidine-2,4(1H,3H)-dione

### Step 1: preparation of methyl 3-(4-benzylpiperazine-1-carbonyl)bicyclo[1.1.1]pentane-1-carboxylate

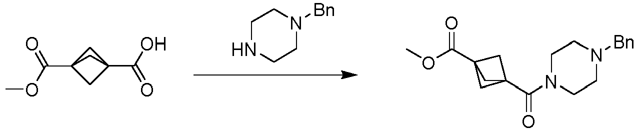

3-(Methoxycarbonyl)bicyclo[1.1.1]pentane-1-carboxylic acid (3.0 g, 17.6 mmol), 1-benzylpiperazine (2.7 g, 18.51 mmol), and DIEA (6.8 g, 52.9 mmol) were dissolved in DCM (80 mL), and EDCI (4.38 g, 22.9 mmol) and HOBT (3.1 g, 22.9 mmol) were added. The mixture was reacted at room temperature for 2 h. An aqueous ammonium chloride solution was added to quench the reaction, and the mixture was extracted with dichloromethane (30 mL × 3). The organic phases were combined, washed with saturated brine, dried over anhydrous sodium sulfate, and concentrated under reduced pressure. The concentrate was stirred with ethyl acetate (20 mL) and filtered to give methyl 3-(4-benzylpiperazine-1-carbonyl)bicyclo[1.1.1]pentane-1-carboxylate.

LC-MS: (ESI, m/z): [M+H]⁺ = 329.2.

### Step 2: preparation of (3-((4-benzylpiperazin-1-yl)methyl)bicyclo[1.1.1]pentan-1-yl)methanol

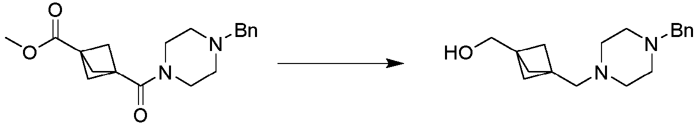

Methyl 3-(4-benzylpiperazine-1-carbonyl)bicyclo[1.1.1]pentane-1-carboxylate (2.2 g, 6.70 mmol) in THF (50 mL) was cooled to 0 °C. A 1 M solution of LiAlH₄ in THF (13.4 mL, 13.4 mmol) was added dropwise, and the mixture was reacted for 2 h. Sodium sulfate decahydrate was added to quench the reaction. The mixture was filtered, and the filtrate was concentrated to give (3-((4-benzylpiperazin-1-yl)methyl)bicyclo[1.1.1]pentan-1-yl)methanol.

LC-MS: (ESI, m/z): [M+H]⁺ = 287.3.

### Step 3: preparation of tert-butyl 3-(2-((3-((4-benzylpiperazin-1-yl)methyl)bicyclo[1.1.1]pentan-1-yl)methoxy)-7-chloro-8-fluoropyrido[4,3-d]pyrimidin-4-yl)-3,8-diazabicyclo[3.2.1]octane-8-carboxylate

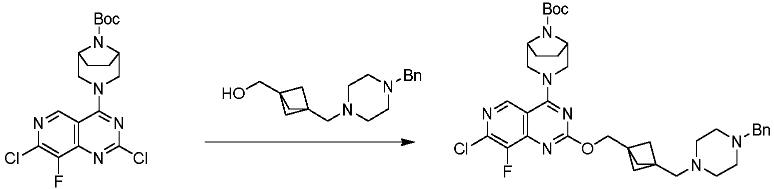

*tert*-Butyl 3-(2,7-dichloro-8-fluoropyrido[4,3-*d*]pyrimidin-4-yl)-3,8-diazabicyclo[3.2.1]octane-8-carboxylate (898 mg, 2.13 mmol) and (3-((4-benzylpiperazin-1-yl)methyl)bicyclo[1.1.1]pentan-1-yl)methanol (900 mg, 3.15 mmol) were added to THF (15 mL), and then cesium carbonate (1710 mg, 5.25 mmol) was added. The mixture was stirred at 90 °C for 16 h. After the reaction was completed, the mixture was cooled to room temperature. Water was added, and the mixture was extracted with ethyl acetate (30 mL × 3). The organic phases were combined, dried over anhydrous sodium sulfate, and concentrated under reduced pressure. The concentrate was purified by silica gel column chromatography (DCM:MeOH = 100:1) to give *tert*-butyl 3-(2-((3-((4-benzylpiperazin-1-yl)methyl)bicyclo[1.1.1]pentan-1-yl)methoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 678.0.

### Step 4: preparation of tert-butyl 3-(7-chloro-8-fluoro-2-((3-(piperazin-1-ylmethyl)bicyclo[1.1.1]pentan-1-yl)methoxy)pyrido[4,3-d]pyrimidin-4-yl)-3,8-diazabicyclo[3.2.1]octane-8-carboxylate

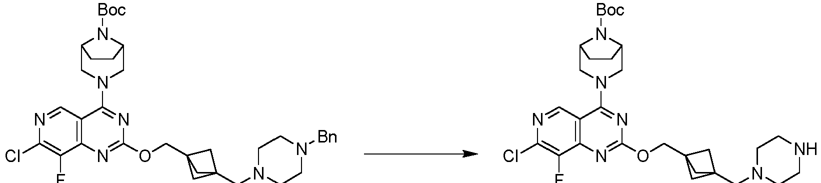

*tert-Butyl* 3-(2-((3-((4-benzylpiperazin-1-yl)methyl)bicyclo[1.1.1]pentan-1-yl)methoxy)-7-chloro-8-fluoropyrido[4,3-*d*]pyrimidin-4-yl)-3,8-diazabicyclo[3.2.1]octane-8-carboxylate (1.0 g, 1.47 mmol) was dissolved in DCM (15 mL), and 1-chloroethyl chloroformate (0.53 g, 3.68 mmol) was added. The mixture was reacted at room temperature for 1 h, and MeOH (10 mL) was added for dissolution. The resulting mixture was heated to 50 °C and reacted for 30 min. After the reaction was completed, the mixture was concentrated and purified by silica gel column chromatography (DCM:MeOH = 100:1 to 10:1) to give *tert-*butyl 3-(7-chloro-8-fluoro-2-((3-(piperazin-1-ylmethyl)bicyclo[1.1.1]pentan-1-yl)methoxy)pyrido[4,3-*d*]pyrimidin-4-yl)-3,8-diazabicyclo[3.2.1]octane-8-carboxylate.

LC-MS: (ESI, m/z): [M+H]⁺ = 588.2.

### Step 5: preparation of tert-butyl 3-(8-fluoro-7-(7-fluoro-3-(methoxymethoxy)-8-((triisopropylsilyl)ethynyl) naphthalen-1-yl)-2-((3-(piperazin-1-ylmethyl)bicyclo[1.1.1]pentan-1-yl)methoxy)pyrido[4,3-d]pyrimidin-4-yl)-3,8-diazabicyclo[3.2.1]octane-8-carboxylate

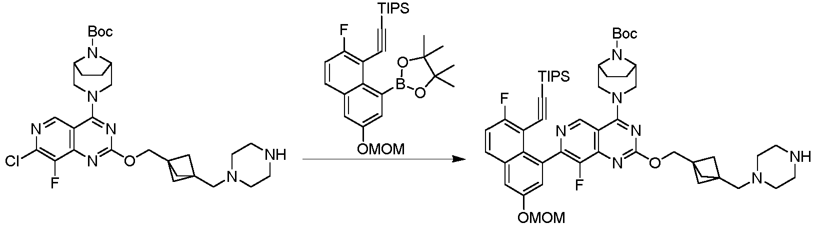

cataCXium A Pd G3 (41 mg, 0.056 mmol) and cesium carbonate (456 mg, 1.40 mmol) were added to a mixed solution of *tert*-butyl 3-(7-chloro-8-fluoro-2-((3-(piperazin-1-ylmethyl)bicyclo[1.1.1]pentan-1-yl)methoxy) pyrido[4,3-*d*]pyrimidin-4-yl)-3,8-diazabicyclo[3.2.1]octane-8-carboxylate (330 mg, 0.56 mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (374 mg, 0.73 mmol) in 1,4-dioxane (5 mL) and H₂O (1 mL). The mixture was reacted at 80 °C for 16 h under nitrogen atmosphere. Water (10 mL) was added, and the mixture was extracted with ethyl acetate (100 mL × 3). The organic phases were combined, dried over anhydrous sodium sulfate, and concentrated under reduced pressure. The concentrate was purified by silica gel column chromatography (DCM:MeOH = 100:1 to 7:1) to give *tert-butyl* 3-(8-fluoro-7-(7-fluoro-3-(methoxymethoxy)-8-((triisopropylsilyl)ethynyl)naphthalen-1-yl)-2-((3-(piperazin-1-ylmethyl)bicyclo[1.1.1]pentan-1-yl)methoxy)pyrido[4,3-*d*]pyrimidin-4-yl)-3,8-diazabicyclo[3.2.1]octane-8-carboxylate.

LC-MS: (ESI, m/z): [M/2+H]⁺ = 469.8.

### Step 6: preparation of tert-butyl 3-(2-((3-((4-(2-(3-(4-chloro-3-(2,4-dioxotetrahydropyrimidin-1(2H)-yl)benzoyl)-3-azaspiro[5.5]undecan-9-yl)ethyl)piperazin-1-yl)methyl)bicyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

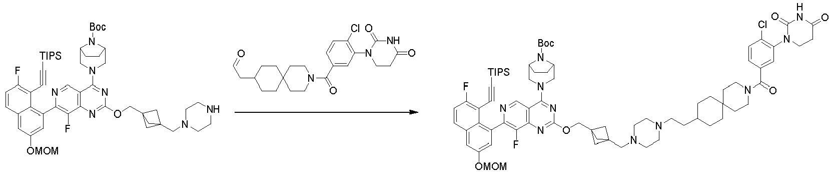

2-(3-(4-Chloro-3-(2,4-dioxotetrahydropyrimidin-1(2*H*)-yl)benzoyl)-3-azaspiro[5.5]undecan-9-yl)acetaldehyde (36 mg, 0.11 mmol) and tetraisopropyl titanate (170 mg, 0.6 mmol) were added to a solution of *tert-butyl* 3-(8-fluoro-7-(7-fluoro-3-(methoxymethoxy)-8-((triisopropylsilyl)ethynyl)naphthalen-1-yl)-2-((3-(piperazin-1-ylmethyl)bicyclo[1.1.1]pentan-1-yl)methoxy)pyrido[4,3-*d*]pyrimidin-4-yl)-3,8-diazabicyclo[3.2.1]octane-8-carboxylate (100 mg, 0.11 mmol) in dichloromethane (1.5 mL) and acetic acid (0.1 mL), and the mixture was stirred at 25 °C for 2 h. Then NaBH(OAc)₃ (70 mg, 0.33 mmol) was added at 0 °C, and the mixture was stirred at 25 °C for 1 h. After the reaction was completed, H₂O (5 mL) was added, and the mixture was extracted with ethyl acetate (15 mL × 3). The combined organic phase was dried over anhydrous sodium sulfate, filtered, and concentrated in vacuum. The crude product was purified by silica gel column chromatography (DCM:MeOH = 20:1) to give *tert-butyl* 3-(2-((3-((4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)methyl)bicyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 684.4.

### Step 7: preparation of 1-(5-(9-(2-(4-((3-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)bicyclo[1.1.1]pentan-1-yl) methyl)piperazin-1-yl)ethyl)-3-azaspiro[5.5]undecane-3-carbonyl)-2-chlorophenyl)dihydropyrimidine-2,4(1H,3H)-dione

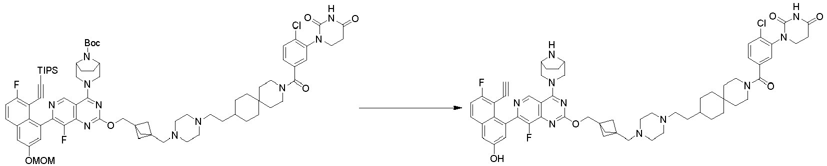

CsF (51 mg, 0.33 mmol) was added to a stirred solution of *tert-butyl* 3-(2-((3-((4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)methyl)bicyclo[1.1.1]pentan-1-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (65 mg, 0.048 mmol) in DMF (2.0 mL), and the mixture was reacted at room temperature for 1 h. Water (10 mL) was added, and the mixture was extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (2.0 mL), and HCl/1,4-dioxane (6 N, 1.0 mL) was added. The mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-(2-(4-((3-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)bicyclo[1.1.1]pentan-1-yl)methyl)piperazin-1-yl)ethyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 534.4.

¹H NMR (400 MHz, DMSO-*d*₆) δ 10.49 (s, 1H), 10.12 (s, 1H), 9.03 (s, 1H), 7.97 (dd, *J =* 9.2, 6.0 Hz, 1H), 7.63 (d, *J =* 8.2 Hz, 1H), 7.54 (d, *J =* 1.9 Hz, 1H), 7.49 - 7.43 (m, 1H), 7.38 (dd, *J =* 7.2, 2.2 Hz, 2H), 7.17 (d, *J* = 2.4 Hz, 1H), 4.46 - 4.27 (m, 4H), 3.90 (s, 1H), 3.79 - 3.70 (m, 1H), 3.66 - 3.49 (m, 7H), 3.28 - 3.21 (m, 2H), 2.77 - 2.70 (m, 2H), 2.44 - 2.09 (m, 13H), 1.72 - 1.61 (m, 12H), 1.53 - 1.40 (m, 4H), 1.33 - 1.21 (m, 5H), 1.12 - 0.98 (m, 4H).

### Example 57: 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro [5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

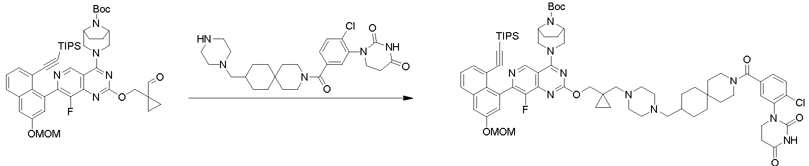

1-(2-Chloro-5-(9-(piperazin-1-ylmethyl)-3-azaspiro[5.5]undecane-3-carbonyl)phenyl)dihydropyrimidine-2,4(1*H*,3*H*)-dione (58 mg, 0.12 mmol) and tetraisopropyl titanate (170 mg, 0.6 mmol) were added to a solution of *tert-*butyl 3-(8-fluoro-2-((1-formylcyclopropyl)methoxy)-7-(3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate (80 mg, 0.097 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 655.3.

### Step 2: preparation of 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

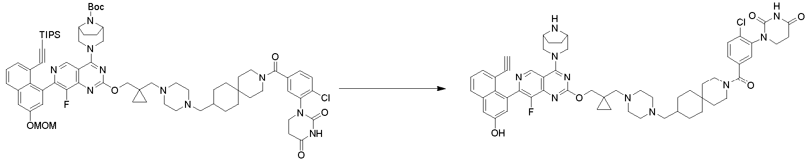

*tert-Butyl* 3-(2-((1-((4-((3-(4-chloro-3-(2,4-dioxotetrahydropyrimidin-1(2*H*)-yl)benzoyl)-3-azaspiro[5.5] undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (50 mg, 0.038 mmol) was dissolved in DMF (2 mL), and CsF (71 mg, 0.47 mmol) was added. The mixture was reacted at room temperature for 1 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (10 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M+H]⁺ = 1009.2.

¹H NMR (400 MHz, DMSO-*d*₆) δ 10.50 (s, 1H), 10.12 (s, 1H), 9.01 (s, 1H), 7.88 (dd, *J* = 8.0, 1.5 Hz, 1H), 7.63 (d, *J* = 8.2 Hz, 1H), 7.54 (d, *J =* 1.9 Hz, 1H), 7.47 - 7.31 (m, 4H), 7.11 (d, *J* = 2.5 Hz, 1H), 4.49 (d, *J* = 10.8 Hz, 1H), 4.30 - 4.15 (m, 3H), 3.87 - 3.68 (m, 1H), 3.67 - 3.34 (m, 8H), 2.86 - 2.59 (m, 3H), 2.44 - 2.13 (m, 11H), 2.15 - 1.93 (m, 3H), 1.69 - 1.58 (m, 5H), 1.56 - 1.19 (m, 8H), 1.12 - 0.90 (m, 4H), 0.66- 0.54 (m, 2H), 0.47 - 0.30 (m, 2H).

### Example 58: 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,8-diazabicyclo[3.2.1]octan-8-yl)methyl)-3-azaspiro[5.5]undecane-3-carbonyl)-2-chlorophenyl)dihydropyrimidine-2,4(1H,3H)-dione

### Step 1: preparation of tert-butyl 8-((3-(4-chloro-3-(2,4-dioxotetrahydropyrimidin-1(2H)-yl)benzoyl)-3-azaspiro[5.5]undecan-9-yl)methyl)-3,8-diazabicyclo[3.2.1]octane-3-carboxylate

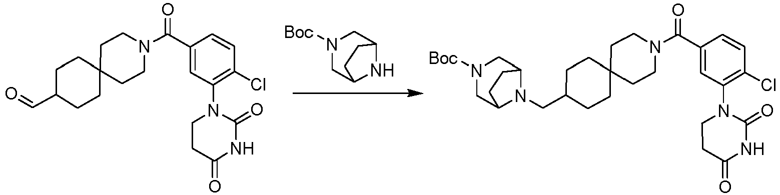

*tert*-Butyl 3,8-diazabicyclo[3.2.1]octane-3-carboxylate (30 mg, 138.91 µmol) was added to 3-(4-chloro-3-(2,4-dioxotetrahydropyrimidin-1(2*H*)-yl)benzoyl)-3 -azaspiro [5. 5]undecane-9-carbaldehyde (50 mg, 115.76 µmol) in 1,2-dichloroethane (5 mL) at room temperature, and the mixture was reacted at 25 °C for 0.5 h under nitrogen atmosphere. Then sodium triacetoxyborohydride (49 mg, 213.52 µmol) was added, and the mixture was reacted at 25 °C for another 3 h. Methanol (5 mL) was added to quench the reaction, and the mixture was concentrated under reduced pressure at 40 °C. The crude product was purified by a preparative thin-layer chromatography plate (dichloromethane:methanol = 10:1) to give *tert*-butyl 8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1]octane-3-carboxylate.

LC-MS: (ESI, m/z): [M+H]⁺ = 628.4.

### Step 2: preparation of 1-(5-(9-((3,8-diazabicyclo[3.2.1]octan-8-yl)methyl)-3-azaspiro[5.5]undecane-3-carbonyl)-2-chlorophenyl)dihydropyrimidine-2,4(1H,3H)-dione

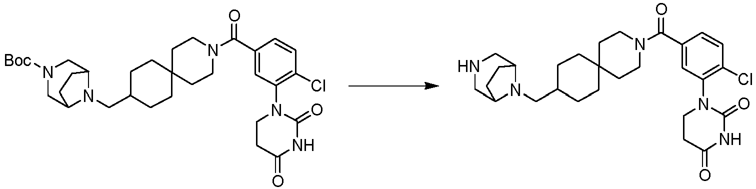

Trifluoroacetic acid (1.8 mL) was added to a solution of *tert-butyl* 8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1]octane-3-carboxylate (72.5 mg, 115.40 µmol) in dichloromethane (3 mL) at room temperature, and the mixture was reacted at room temperature for 1.5 h. After the reaction was completed, the reaction liquid was poured into a saturated aqueous sodium carbonate solution (50 mL), and the mixture was extracted with dichloromethane (50 mL × 2). The organic phases were combined, washed with saturated brine (50 mL), dried over anhydrous sodium sulfate, filtered, and concentrated under reduced pressure to give 1-(5-(9-((3,8-diazabicyclo[3.2.1]octan-8-yl)methyl)-3-azaspiro[5.5]undecane-3-carbonyl)-2-chlorophenyl)dihydropyrimidine-2,4(1*H*,*3H)*-dione.

LC-MS: (ESI, m/z): [M+H]⁺ = 528.3.

### Step 3: preparation of tert-butyl 3-(2-((1-((8-((3-(4-chloro-3-(2,4-dioxotetrahydropyrimidin-1(2H)-yl)benzoyl)-3-azaspiro[5.5]undecan-9-yl)methyl)-3,8-diazabicyclo[3.2.1]octan-3-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-d]pyrimidin-4-yl)-3,8-diazabicyclo[3.2.1]octane-8-carboxylate

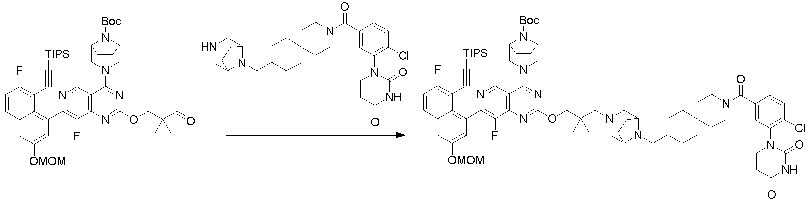

Tetraisopropyl titanate (115.2 mg, 405.55 µmol) and glacial acetic acid (48.7 mg, 811.10 µmol) were added to a stirred solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (68.3 mg, 81.11 µmol) and 1-(5-(9-((3,8-diazabicyclo[3.2.1]octan-8-yl)methyl)-3-azaspiro[5.5]undecane-3-carbonyl)-2-chlorophenyl)dihydropyrimidine-2,4(1*H*,3*H*)-dione (51.4 mg, 97.33 µmol) in DCM (6 mL) at room temperature, and the mixture was stirred at 25 °C for 2 h. Then sodium triacetoxyborohydride (51.6 mg, 243.33 µmol) was added, and the mixture was reacted at 25 °C for another 3 h. Methanol (5 mL) was added to quench the reaction, and the mixture was concentrated under reduced pressure at 40 °C. The crude product was purified by a preparative thin-layer chromatography plate (dichloromethane:methanol = 10:1) to give *tert*-butyl 3-(2-((1-((8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1] octan-3-yl)methyl)cyclopropyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 677.2.

### Step 4: preparation of tert-butyl 3-(2-((1-((8-((3-(4-chloro-3-(2,4-dioxotetrahydropyrimidin-1(2H)-yl)benzoyl)-3-azaspiro[5.5]undecan-9-yl)methyl)-3,8-diazabicyclo[3.2.1]octan-3-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

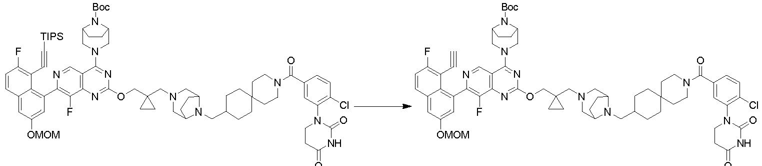

Cesium fluoride (63 mg, 412.80 µmol) was added to a solution of *tert-butyl* 3-(2-((1-((8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1]octan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (55.9 mg, 41.28 µmol) in *N,N*-dimethylformamide (5 mL) at room temperature, and the mixture was reacted at 25 °C for 1 h under nitrogen atmosphere. After the reaction was completed, the reaction liquid was poured into water (50 mL), and the mixture was extracted with ethyl acetate (20 mL × 3). The organic phases were combined, washed with saturated brine (50 mL), dried over anhydrous sodium sulfate, filtered, and concentrated under reduced pressure to give *tert*-butyl 3-(2-((1-((8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1] octan-3-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M+H]⁺ = 1197.2.

### Step 5: preparation of 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,8-diazabicyclo[3.2.1]octan-8-yl)methyl)-3-azaspiro[5.5]undecane-3-carbonyl)-2-chlorophenyl)dihydropyrimidine-2,4(1H,3H)-dione

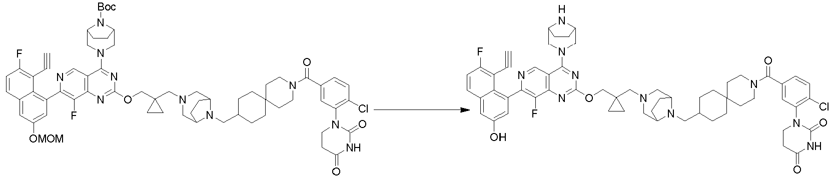

A hydrogen chloride/1,4-dioxane solution (4.0 M, 5 mL) was added to *tert*-butyl 3-(2-((1-((8-((3-(4-chloro-3-(2,4-dioxotetrahydropyrimidin-1(2*H*)-yl)benzoyl)-3-azaspiro[5.5]undecan-9-yl)methyl)-3,8-diazabicyclo[3.2.1]octan-3-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (40 mg, 41.28 µmol) at room temperature, and the mixture was reacted at room temperature for 1.5 h. After the reaction was completed, the mixture was concentrated to dryness under reduced pressure at 30 °C, and a solution of ammonia in methanol (1 mL) was added. Then the mixture was concentrated to dryness under reduced pressure at 30 °C. The crude product was purified by a preparative thin-layer chromatography plate (dichloromethane:methanol = 10:1, 0.1% NH₃) and then purified by preparative high-performance liquid chromatography to give 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-3,8-diazabicyclo[3.2.1]octan-8-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 527.2.

¹H NMR (400 MHz, DMSO-*d*₆) δ 10.50 (s, 1H), 10.15 (s, 1H), 9.02 (s, 1H), 7.97 (dd, *J =* 9.2, 5.9 Hz, 1H), 7.63 (d, *J =* 8.2 Hz, 1H), 7.54 (d, *J =* 1.9 Hz, 1H), 7.49 - 7.43 (t, *J =* 9.0 Hz, 1H), 7.38 (dd, *J =* 7.0, 2.2 Hz, 2H), 7.16 (d, *J =* 2.4 Hz, 1H), 4.48 (d, *J =* 11.6 Hz, 1H), 4.41 - 4.19 (m, 3H), 3.88 (s, 1H), 3.79 - 3.71 (m, 1H), 3.67 - 3.44 (m, 7H), 3.02 - 2.91 (m, 2H), 2.78 - 2.71 (m, 2H), 2.63 - 2.55 (m, 2H), 2.32 - 2.19 (m, 2H), 2.16 - 2.02 (m, 4H), 1.76 - 1.17 (m, 19H), 1.16 - 0.82 (m, 5H), 0.64 - 0.51(m, 2H), 0.40 - 0.27 (m, 2H).

### Example 59: 1-(5-(9-((5-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-2,5-diazabicyclo[2.2.1]heptan-2-yl)methyl)-3-azaspiro[5.5]undecane-3-carbonyl)-2-chlorophenyl)dihydropyrimidine-2,4(1H,3H)-dione

### Step 1: preparation of tert-butyl 5-((3-(4-chloro-3-(2,4-dioxotetrahydropyrimidin-1(2H)-yl)benzoyl)-3-azaspiro[5.5]undecan-9-yl)methyl)-2,5-diazabicyclo[2.2.1]heptane-2-carboxylate

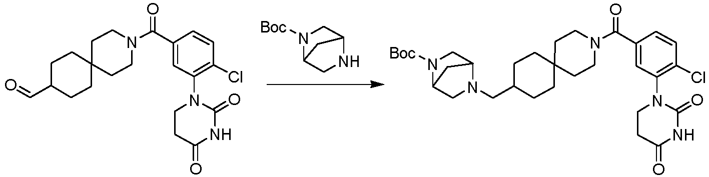

*tert*-Butyl 2,5-diazabicyclo[2.2.1]heptane-2-carboxylate (52 mg, 0.14 mmol) was added to 3-(4-chloro-3-(2,4-dioxotetrahydropyrimidin-1(2*H*)-yl)benzoyl)-3-azaspiro[5.5]undecane-9-carbaldehyde (100 mg, 231.52 µmol) in 1,2-dichloroethane (5 mL) at room temperature, and the mixture was reacted at 25 °C for 0.5 h under nitrogen atmosphere. Then sodium triacetoxyborohydride (98 mg, 0.46 mmol) was added, and the mixture was reacted at 25 °C for another 3 h. Methanol (5 mL) was added to quench the reaction, and the mixture was concentrated under reduced pressure at 40 °C. The crude product was purified by a preparative thin-layer chromatography plate (dichloromethane:methanol = 10:1) to give *tert*-butyl 5-((3-(4-chloro-3-(2,4-dioxotetrahydropyrimidin-1(2*H*)-yl)benzoyl)-3-azaspiro[5.5]undecan-9-yl)methyl)-2,5-diazabicyclo[2.2.1]heptane-2-carboxylate.

LC-MS: (ESI, m/z): [M+H]⁺ = 614.4.

### Step 2: preparation of 1-(5-(9-((2,5-diazabicyclo[2.2.1]heptan-2-yl)methyl)-3-azaspiro[5.5]undecane-3-carbonyl)-2-chlorophenyl)dihydropyrimidine-2,4(1H,3H)-dione

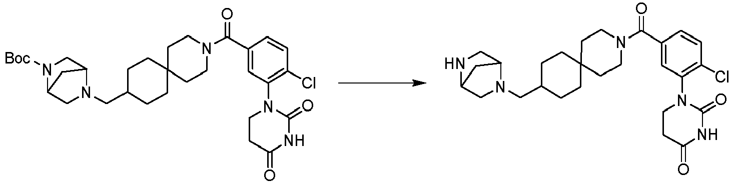

Trifluoroacetic acid (3 mL) was added to a solution of *tert-butyl* 5-((3-(4-chloro-3-(2,4-dioxotetrahydropyrimidin-1(2*H*)-yl)benzoyl)-3-azaspiro[5.5]undecan-9-yl)methyl)-2,5-diazabicyclo[2.2.1] heptane-2-carboxylate (128.5 mg, 209.22 µmol) in dichloromethane (6 mL) at room temperature, and the mixture was reacted at room temperature for 1.5 h. After the reaction was completed, the reaction liquid was poured into a saturated aqueous sodium carbonate solution (100 mL), and the mixture was extracted with dichloromethane (50 mL × 2). The organic phases were combined, washed with saturated brine (50 mL), dried over anhydrous sodium sulfate, filtered, and concentrated under reduced pressure to give 1-(5-(9-((2,5-diazabicyclo[2.2.1]heptan-2-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 514.3.

### Step 3: preparation of tert-butyl 3-(2-((1-((5-((3-(4-chloro-3-(2,4-dioxotetrahydropyrimidin-1(2H)-yl)benzoyl)-3-azaspiro[5.5]undecan-9-yl)methyl)-2,5-diazabicyclo[2.2.1]heptan-2-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

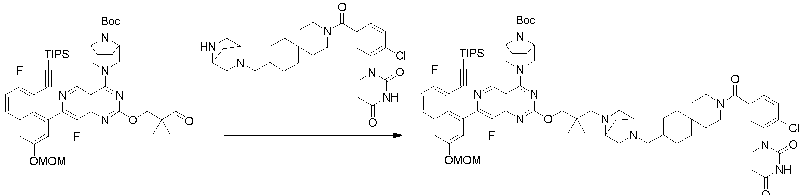

Tetraisopropyl titanate (253 mg, 0.89 mmol) and glacial acetic acid (107 mg, 0.18 mmol) were added to a stirred solution of *tert*-butyl 3-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-*d*]pyrimidin-4-yl)-3,8-diazabicyclo[3.2.1] octane-8-carboxylate (150 mg, 0.18 mmol) and 1-(5-(9-((2,5-diazabicyclo[2.2.1]heptan-2-yl)methyl)-3-azaspiro[5.5]undecane-3-carbonyl)-2-chlorophenyl)dihydropyrimidine-2,4(1H,3H)-dione (110 mg, 0.21 mmol) in DCM (15 mL) at room temperature, and the mixture was stirred at 25 °C for 2 h. Then sodium triacetoxyborohydride (113 mg, 0.53 mmol) was added, and the mixture was reacted at 25 °C for another 3 h. Methanol (5 mL) was added to quench the reaction, and the mixture was concentrated under reduced pressure at 40 °C. The crude product was purified by a preparative thin-layer chromatography plate (dichloromethane:methanol = 10:1) to give *tert*-butyl 3-(2-((1-((5-((3-(4-chloro-3-(2,4-dioxotetrahydropyrimidin-1(2*H*)-yl)benzoyl)-3-azaspiro[5.5]undecan-9-yl)methyl)-2,5-diazabicyclo[2.2.1] heptan-2-yl)methyl)cyclopropyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 670.3.

### Step 4: preparation of tert-butyl 3-(2-((1-((5-((3-(4-chloro-3-(2,4-dioxotetrahydropyrimidin-1(2H)-yl)benzoyl)-3-azaspiro[5.5]undecan-9-yl)methyl)-2,5-diazabicyclo[2.2.1]heptan-2-yl)methyl)cyclopropyl)methoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

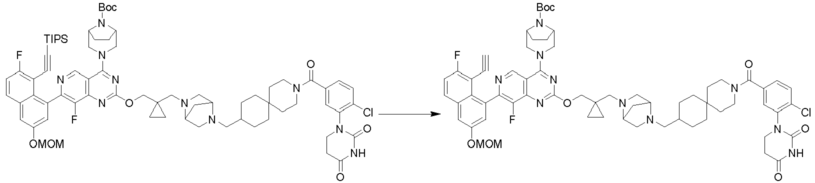

Cesium fluoride (142 mg, 935.00 µmol) was added to a solution of *tert*-butyl 3-(2-((1-((5-((3-(4-chloro-3-(2,4-dioxotetrahydropyrimidin-1(2*H*)-yl)benzoyl)-3-azaspiro[5.5]undecan-9-yl)methyl)-2,5-diazabicyclo[2.2.1] heptan-2-yl)methyl)cyclopropyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate (125.3 mg, 93.50 µmol) in *N*,*N*-dimethylformamide (12 mL) at room temperature, and the mixture was reacted at 25 °C for 1 h under nitrogen atmosphere. After the reaction was completed, the reaction liquid was poured into water (50 mL), and the mixture was extracted with ethyl acetate (20 mL × 3). The organic phases were combined, washed with saturated brine (50 mL), dried over anhydrous sodium sulfate, filtered, and concentrated under reduced pressure to give *tert*-butyl 3-(2-((1-((5-((3-(4-chloro-3-(2,4-dioxotetrahydropyrimidin-1(2*H*)-yl)benzoyl)-3-azaspiro[5.5]undecan-9-yl)methyl)-2,5-diazabicyclo [2.2.1]heptan-2-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 592.3.

### Step 5: preparation of 1-(5-(9-((5-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-2,5-diazabicyclo[2.2.1]heptan-2-yl)methyl)-3-azaspiro[5.5]undecane-3-carbonyl)-2-chlorophenyl)dihydropyrimidine-2,4(1H,3H)-dione

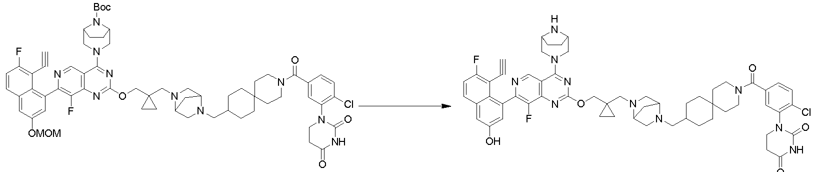

A hydrogen chloride/1,4-dioxane solution (4.0 M, 5 mL) was added to *tert*-butyl 3-(2-((1-((5-((3-(4-chloro-3-(2,4-dioxotetrahydropyrimidin-1(2*H*)-yl)benzoyl)-3-azaspiro[5.5]undecan-9-yl)methyl)-2,5-diazabicyclo[2.2.1]heptan-2-yl)methyl)cyclopropyl)methoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (112.8 mg, 93.50 µmol) at room temperature, and the mixture was reacted at room temperature for 1.5 h. After the reaction was completed, the mixture was concentrated to dryness under reduced pressure at 30 °C, and a solution of ammonia in methanol (1 mL) was added. Then the mixture was concentrated to dryness under reduced pressure at 30 °C. The crude product was purified by a preparative thin-layer chromatography plate (dichloromethane:methanol = 10:1, 0.1% NH₃) and then purified by preparative high-performance liquid chromatography to give 1-(5-(9-((5-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-2,5-diazabicyclo[2.2.1]heptan-2-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 520.7.

¹H NMR (400 MHz, DMSO-*d*₆) δ 10.50 (s, 1H), 10.14 (s, 1H), 9.02 (s, 1H), 7.97 (dd, *J* = 9.0, 6.0 Hz, 1H), 7.63 (d, *J* = 8.1 Hz, 1H), 7.54 (s, 1H), 7.49 - 7.43 (m, 1H), 7.38 (d, *J =* 7.4 Hz, 2H), 7.17 (s, 1H), 4.47 (d, *J =* 11.3 Hz, 1H), 4.37 - 4.20 (m, 3H), 3.91 (s, 1H), 3.80 - 3.71 (m, 1H), 3.68 - 3.45 (m, 7H), 3.29 - 3.21 (m, 3H), 3.16 - 3.06 (m, 1H), 2.77-2.71 (m, 2H), 2.64 - 2.53 (m, 4H), 2.49 - 2.41 (m, 3H), 2.34 - 2.17 (m, 2H), 1.72 - 1.59 (m, 6H), 1.55 - 1.21 (m, 9H), 1.11 - 0.92 (m, 4H), 0.61 - 0.50 (m, 2H), 0.48 - 0.36 (m, 2H).

### Example 60: 1-(5-(9-((4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-(2-(4-((3-(4-chloro-3-(2,4-dioxotetrahydropyrimidin-1(2H)-yl)benzoyl)-3-azaspiro[5.5]undecan-9-yl)methyl)piperazin-1-yl)ethoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

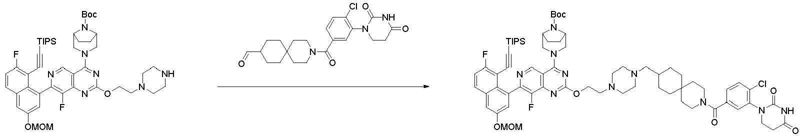

Tetraisopropyl titanate (81 mg, 286.65 µmol) and glacial acetic acid (5 mg, 83.33 µmol) were added to a stirred solution of *tert*-butyl 3-(8-fluoro-7-(7-fluoro-3-(methoxymethoxy)-8-((triisopropylsilyl)ethynyl)naphthalen-1-yl)-2-(2-(piperazin-1-yl)ethoxy)pyrido[4,3-*d*]pyrimidin-4-yl)-3,8-diazabicyclo[3.2.1]octane-8-carboxylate (50 mg, 57.33 µmol) and 3-(4-chloro-3-(2,4-dioxotetrahydropyrimidin-1(2*H*)-yl)benzoyl)-3-azaspiro[5.5]undecane-9-carbaldehyde (25 mg, 57.33 µmol) in DCM (5 mL) at room temperature, and the mixture was stirred at 25 °C for 2 h. Then sodium triacetoxyborohydride (36 mg, 172.00 µmol) was added, and the mixture was reacted at 25 °C for another 3 h. Methanol (5 mL) was added to quench the reaction, and the mixture was concentrated under reduced pressure at 40 °C. The resulting crude product was purified by a preparative thin-layer chromatography plate (dichloromethane:methanol = 10:1) to give *tert-butyl* 3-(2-(2-(4-((3-(4-chloro-3-(2,4-dioxotetrahydropyrimidin-1(2*H*)-yl)benzoyl)-3-azaspiro[5.5]undecan-9-yl)methyl)piperazin-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 644.4.

### Step 2: preparation of tert-butyl 3-(2-(2-(4-((3-(4-chloro-3-(2,4-dioxotetrahydropyrimidin-1(2H)-yl)benzoyl)-3-azaspiro[5.5]undecan-9-yl)methyl)piperazin-1-yl)ethoxy)-7-(8-ethynyl-7-fluoro-3-(methoxymethoxy) naphthalen-1-yl)-8-fluoropyrido[4,3-d]pyrimidin-4-yl)-3,8-diazabicyclo[3.2.1]octane-8-carboxylate

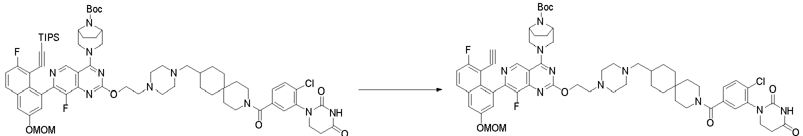

Cesium fluoride (69 mg, 451.84 µmol) was added to a solution of *tert-butyl* 3-(2-(2-(4-((3-(4-chloro-3-(2,4-dioxotetrahydropyrimidin-1(2*H*)-yl)benzoyl)-3-azaspiro[5.5]undecan-9-yl)methyl)piperazin-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (58 mg, 45.18 µmol) in *N,N-*dimethylformamide (5 mL) at room temperature, and the mixture was reacted at 25 °C for 1 h under nitrogen atmosphere. After the reaction was completed, the reaction liquid was poured into water (50 mL), and the mixture was extracted with ethyl acetate (20 mL × 3). The organic phases were combined, washed with saturated brine (50 mL), dried over anhydrous sodium sulfate, filtered, and concentrated under reduced pressure to give *tert*-butyl 3-(2-(2-(4-((3-(4-chloro-3-(2,4-dioxotetrahydropyrimidin-1(2*H*)-yl)benzoyl)-3-azaspiro[5.5]undecan-9-yl)methyl)piperazin-1-yl)ethoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 566.3.

### Step 3: preparation of 1-(5-(9-((4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)piperazin-1-yl)methyl)-3-azaspiro[5.5]undecane-3-carbonyl)-2-chlorophenyl)dihydropyrimidine-2,4(1H,3H)-dione

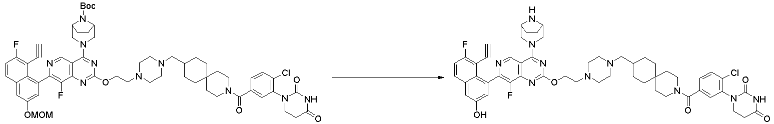

A hydrogen chloride/1,4-dioxane solution (4.0 M, 5 mL) was added to *tert*-butyl 3-(2-(2-(4-((3-(4-chloro-3-(2,4-dioxotetrahydropyrimidin-1(2*H*)-yl)benzoyl)-3-azaspiro[5.5]undecan-9-yl)methyl)piperazin-1-yl)ethoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (52 mg, 45.18 µmol) at room temperature, and the mixture was reacted at room temperature for 1.5 h. After the reaction was completed, the mixture was concentrated to dryness under reduced pressure at 30 °C, and a solution of ammonia in methanol (1 mL) was added. Then the mixture was concentrated to dryness under reduced pressure at 30 °C. The resulting crude product was purified by a preparative thin-layer chromatography plate (dichloromethane:methanol = 10:1, 0.1% NH₃) and then separated and purified by preparative high-performance liquid chromatography to give 1-(5-(9-((4-(2-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)ethyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M/2+H]⁺ = 494.2.

¹H NMR (400 MHz, DMSO-*d*₆) δ 10.50 (s, 1H), 10.12 (s, 1H), 9.03 (s, 1H), 7.97 (dd, *J =* 9.0, 6.1 Hz, 1H), 7.63 (d, *J* = 8.2 Hz, 1H), 7.54 (s, 1H), 7.49 - 7.43 (m, 1H), 7.38 (d, *J* = 7.8 Hz, 2H), 7.17 (s, 1H), 4.51 - 4.39 (m, 3H), 4.31 (d, *J* = 12.0 Hz, 1H), 3.92 (s, 1H), 3.79 - 3.53 (m, 8H), 2.80 - 2.67 (m, 4H), 2.37 - 2.25 (m, 4H), 2.25 - 1.87 (m, 4H), 1.72 - 1.60 (m, 6H), 1.60 - 1.18 (m, 11H), 1.17 - 0.83 (m, 5H).

### Example 61: 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)piperazin-1-yl)ethyl)-3-azaspiro[5.5]undecane-3-carbonyl)-2-chlorophenyl)dihydropyrimidine-2,4(1H,3H)-dione

### Step 1: preparation of tert-butyl 3-(2-(2-(4-(2-(3-(4-chloro-3-(2,4-dioxotetrahydropyrimidin-1(2H)-yl)benzoyl)-3-azaspiro[5.5]undecan-9-yl)ethyl)piperazin-1-yl)ethoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

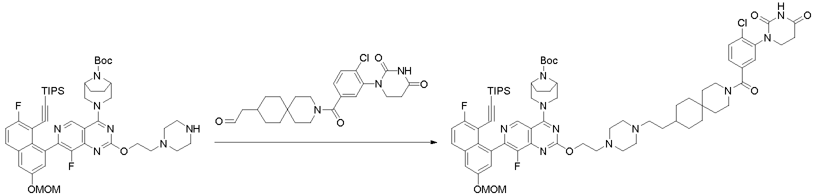

Tetraisopropyl titanate (163 mg, 573.30 µmol) and glacial acetic acid (10 mg, 166.66 µmol) were added to a stirred solution of *tert*-butyl 3-(8-fluoro-7-(7-fluoro-3-(methoxymethoxy)-8-((triisopropylsilyl)ethynyl)naphthalen-1-yl)-2-(2-(piperazin-1-yl)ethoxy)pyrido[4,3-*d*]pyrimidin-4-yl)-3,8-diazabicyclo[3.2.1]octane-8-carboxylate (100 mg, 114.66 µmol) and 2-(3-(4-chloro-3-(2,4-dioxotetrahydropyrimidin-1(2*H*)-yl)benzoyl)-3-azaspiro[5.5]undecan-9-yl)acetaldehyde (52 mg, 114.66 µmol) in DCM (10 mL) at room temperature, and the mixture was stirred at 25 °C for 2 h. Then sodium triacetoxyborohydride (72 mg, 344.00 µmol) was added, and the mixture was reacted at 25 °C for another 3 h. Methanol (10 mL) was added to quench the reaction, and the mixture was concentrated under reduced pressure at 40 °C. The resulting crude product was purified by a preparative thin-layer chromatography plate (dichloromethane:methanol = 10:1) to give *tert*-butyl 3-(2-(2-(4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 651.4.

### Step 2: preparation of tert-butyl 3-(2-(2-(4-(2-(3-(4-chloro-3-(2,4-dioxotetrahydropyrimidin-1(2H)-yl)benzoyl)-3-azaspiro[5.5]undecan-9-yl)ethyl)piperazin-1-yl)ethoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

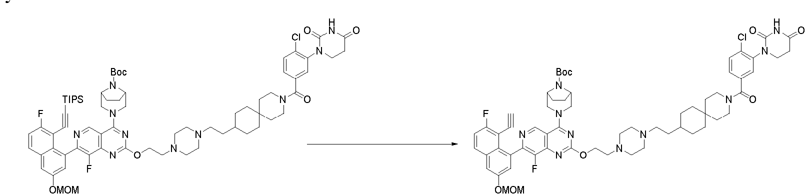

Cesium fluoride (115 mg, 759.55 µmol) was added to a solution of *tert*-butyl 3-(2-(2-(4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (99 mg, 75.95 µmol) in *N,N-*dimethylformamide (10 mL) at room temperature, and the mixture was reacted at 25 °C for 1 h under nitrogen atmosphere. After the reaction was completed, the reaction liquid was poured into water (50 mL), and the mixture was extracted with ethyl acetate (20 mL × 3). The organic phases were combined, washed with saturated brine (50 mL), dried over anhydrous sodium sulfate, filtered, and concentrated under reduced pressure to give *tert*-butyl 3-(2-(2-(4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)ethoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 573.3.

### Step 3: preparation of 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)piperazin-1-yl)ethyl)-3-azaspiro[5.5]undecane-3-carbonyl)-2-chlorophenyl)dihydropyrimidine-2,4(1H,3H)-dione

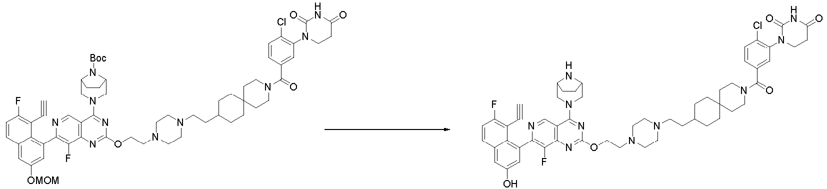

A hydrogen chloride/1,4-dioxane solution (4.0 M, 5 mL) was added to *tert*-butyl 3-(2-(2-(4-(2-(3-(4-chloro-3-(2,4-dioxotetrahydropyrimidin-1(2*H*)-yl)benzoyl)-3-azaspiro[5.5]undecan-9-yl)ethyl)piperazin-1-yl)ethoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (91 mg, 75.95 µmol) at room temperature, and the mixture was reacted at room temperature for 1.5 h. After the reaction was completed, the mixture was concentrated to dryness under reduced pressure at 30 °C, and a solution of ammonia in methanol (1 mL) was added. Then the mixture was concentrated to dryness under reduced pressure at 30 °C. The resulting crude product was purified by a preparative thin-layer chromatography plate (dichloromethane:methanol = 10:1, 0.1% NH₃) and then separated and purified by preparative high-performance liquid chromatography to give 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)ethyl)piperazin-1-yl)ethyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1001.1.

¹H NMR (400 MHz, DMSO-*d*₆) δ 10.50 (s, 1H), 10.14 (s, 1H), 9.03 (s, 1H), 7.97 (dd, *J =* 9.2, 6.0 Hz, 1H), 7.63 (d, *J =* 8.2 Hz, 1H), 7.54 (d, *J =* 1.9 Hz, 1H), 7.49 - 7.43 (m, 1H), 7.38 (dd, *J =* 7.4, 2.2 Hz, 2H), 7.17 (d, *J =* 2.4 Hz, 1H), 4.51 - 4.38 (m, 3H), 4.31 (d, *J =* 11.8 Hz, 1H), 3.92 (s, 1H), 3.80 - 3.69 (m, 1H), 3.66 - 3.48 (m, 7H), 3.29 - 3.21 (m, 2H), 2.80 - 2.64 (m, 5H), 2.49 - 2.40 (m, 4H), 2.37 - 2.16 (m, 6H), 1.68 - 1.62 (m, 5H), 1.53 - 1.40 (m, 4H), 1.36 - 1.19 (m, 6H), 1.13 - 0.99 (m, 4H).

### Example 62: 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(7-(8-ethyl-3-(methoxymethoxy)naphthalen-1-yl)-8-fluoro-2-((1-(hydroxymethyl)cyclopropyl)methoxy)quinazolin-4-yl)-3,8-diazabicyclo[3.2.1]octane-8-carboxylate

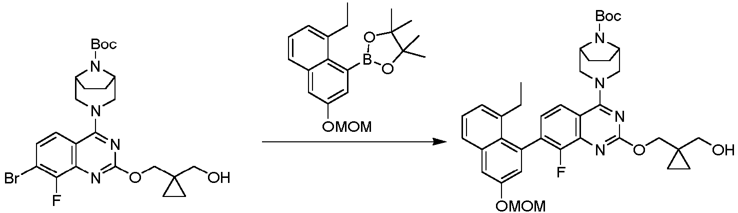

RuPhos Pd G3 (62 mg, 0.074 mmol) and cesium carbonate (726 mg, 2.22 mmol) were added to a mixed solution of *tert*-butyl 3-(7-bromo-8-fluoro-2-((1-(hydroxymethyl)cyclopropyl)methoxy)quinazolin-4-yl)-3,8-diazabicyclo[3.2.1]octane-8-carboxylate (400 mg, 0.74 mmol) and 2-(8-ethyl-3-(methoxymethoxy)naphthalen-1-yl)-4,4,5,5-tetramethyl-1,3,2-dioxaborolane (304 mg, 0.89 mmol) in 1,4-dioxane (6 mL) and H₂O (1.2 mL). The mixture was reacted at 85 °C for 16 h under nitrogen atmosphere. The reaction liquid was directly filtered and concentrated under reduced pressure. The concentrate was purified by silica gel column chromatography (DCM:MeOH = 100:1 to 30:1) to give *tert*-butyl 3-(7-(8-ethyl-3-(methoxymethoxy)naphthalen-1-yl)-8-fluoro-2-((1-(hydroxymethyl)cyclopropyl)methoxy)quinazolin-4-yl)-3,8-diazabicyclo[3.2.1]octane-8-carboxylate.

LC-MS: (ESI, m/z): [M+H]⁺ = 673.4.

### Step 2: preparation of tert-butyl 3-(7-(8-ethyl-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate

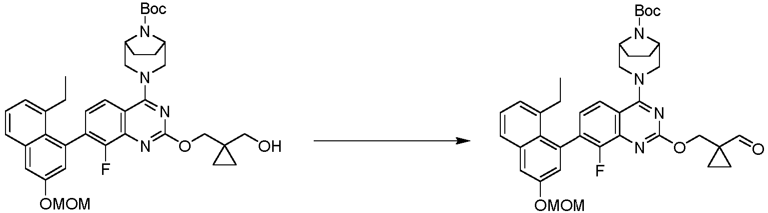

Dess-Martin reagent (153 mg, 0.36 mmol) was added to a solution of *tert*-butyl 3-(7-(8-ethyl-3-(methoxymethoxy)naphthalen-1-yl)-8-fluoro-2-((1-(hydroxymethyl)cyclopropyl)methoxy)quinazolin-4-yl)-3,8-diazabicyclo[3.2.1]octane-8-carboxylate (200 mg, 0.30 mmol) in dichloromethane (3 mL). The mixture was stirred at room temperature for 2 h. After the reaction was completed, a sodium thiosulfate solution (5 mL) and a sodium bicarbonate solution (5 mL) were added to quench the reaction, and the mixture was extracted with dichloromethane (10 mL × 3). The organic phases were combined, dried over anhydrous sodium sulfate, and concentrated under reduced pressure. The concentrate was purified by silica gel column chromatography (DCM:MeOH = 100:1 to 50:1) to give *tert*-butyl 3-(7-(8-ethyl-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate.

LC-MS: (ESI, m/z): [M+H]⁺ = 671.3.

### Step 3: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate

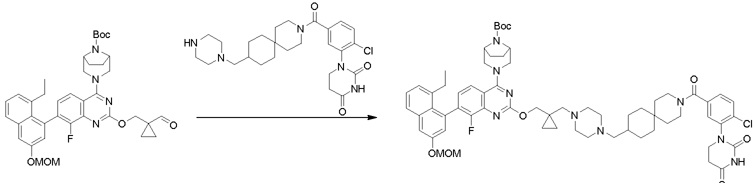

1-(2-Chloro-5-(9-(piperazin-1-ylmethyl)-3-azaspiro[5.5]undecane-3-carbonyl)phenyl)dihydropyrimidine-2,4(1*H*,3*H*)-dione (131 mg, 0.26 mmol) and tetraisopropyl titanate (185 mg, 0.65 mmol) were added to a solution of *tert*-butyl 3-(7-(8-ethyl-3-(methoxymethoxy)naphthalen-1-yl)-8-fluoro-2-((1-formylcyclopropyl) methoxy)quinazolin-4-yl)-3,8-diazabicyclo[3.2.1]octane-8-carboxylate (90 mg, 0.13 mmol) in dichloromethane (1 mL) and acetic acid (0.1 mL), and the mixture was stirred at 25 °C for 2 h. Then NaBH(OAc)₃ (83 mg, 0.39 mmol) was added at 0 °C, and the mixture was stirred at 25 °C for 1 h. After the reaction was completed, H₂O (5 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM/MeOH = 100:1 to 20:1) to give *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M+H]⁺ = 1156.6.

### Step 4: preparation of 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

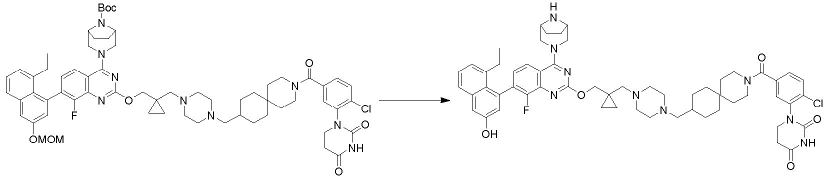

*tert*-Butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate (100 mg, 0.086 mmol) was dissolved in 1,4-dioxane (2 mL), and HCl/1,4-dioxane (6 M, 1 mL) was added. The mixture was stirred at room temperature for 0.5 h. After the reaction was completed, H₂O (5 mL) was added, and the mixture was adjusted to pH > 7 with an aqueous Na₂CO₃ solution and extracted with ethyl acetate (10 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione.

LC-MS: (ESI, m/z): [M/2+H]⁺ = 506.8.

¹H NMR (400 MHz, DMSO-*d*₆) δ 10.51 (s, 1H), 9.87 (s, 1H), 7.78 (d, *J* = 8.7 Hz, 1H), 7.69 - 7.59 (m, 2H), 7.54 (d, *J* = 1.8 Hz, 1H), 7.41 - 7.32 (m, 2H), 7.27 - 7.17 (m, 2H), 7.11 (d, *J =* 7.0 Hz, 1H), 6.88 (d, *J* = *2.5* Hz, 1H), 4.31 - 4.17 (m, 4H), 3.81 - 3.70 (m, 1H), 3.63 - 3.40 (m, 7H), 3.30 - 3.22 (m, 2H), 2.77 - 2.70 (m, 2H), 2.46 - 2.11 (m, 12H), 2.07 - 1.97 (m, 2H), 1.73 - 1.60 (m, 6H), 1.54 - 1.20 (m, 8H), 1.10 - 0.98 (m, 3H), 0.79 (t, *J* = 7.4 Hz, 3H), 0.65 - 0.57 (m, 2H), 0.42 - 0.33 (m, 2H).

### Example 63: 1-(5-(4-(2-((1-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-4-hydroxypiperidin-4-yl)methoxy)ethyl)piperidine-1-carbonyl)-2-chlorophenyl)dihydropyrimidine-2,4(1H,3H)-dione

### Step 1: preparation of benzyl 4-(2-(tosyloxy)ethyl)piperidine-1-carboxylate

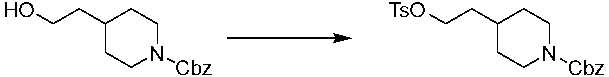

DMAP (23 mg, 0.19 mmol) and triethylamine (0.96 g, 5.7 mmol) were added to a solution of benzyl 4-(2-hydroxyethyl)piperidine-1-carboxylate (0.5 g, 1.9 mmol) in DCM (15 mL). The mixture was stirred at 0 °C for 5 min. Then TsCl (0.44 g, 2.25 mmol) was added, and the mixture was stirred at room temperature overnight. The reaction liquid was concentrated and purified by silica gel column chromatography (PE:EA = 100:1 to 1:1) to give benzyl 4-(2-(tosyloxy)ethyl)piperidine-1-carboxylate.

LC-MS: (ESI, m/z): [M+H]⁺ = 418.1.

### Step 2: preparation of tert-butyl 4-((2-(1-((benzyloxy)carbonyl)piperidin-4-yl)ethoxy)methyl)-4-hydroxypiperidine-1-carboxylate

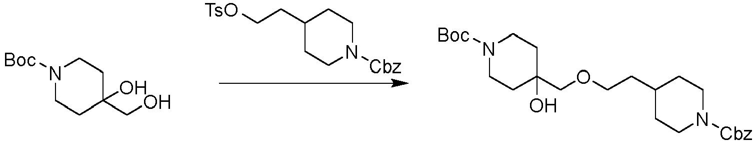

*tert*-Butyl 4-hydroxy-4-(hydroxymethyl)piperidine-1-carboxylate (0.3 g, 1.3 mmol) was dissolved in THF (20 mL), and NaH (62 mg, 1.56 mmol, 60%) was added. The mixture was stirred at the temperature of an ice-water bath to room temperature for 1 h. Benzyl 4-(2-(tosyloxy)ethyl)piperidine-1-carboxylate (0.6 g, 1.44 mmol) was added, and the mixture was stirred at 60 °C overnight. The reaction liquid was cooled to 0 °C. An aqueous NH₄Cl solution (100 mL) was added to quench the reaction, and the mixture was extracted with ethyl acetate (100 mL × 3). The combined organic phase was washed with brine (100 mL × 3), dried over anhydrous sodium sulfate, filtered, concentrated, and purified by silica gel column chromatography (PE:EA = 100:1 to 1:1) to give *tert-butyl* 4-((2-(1-((benzyloxy)carbonyl)piperidin-4-yl)ethoxy)methyl)-4-hydroxypiperidine-1-carboxylate.

LC-MS: (ESI, m/z): [M+H-100]⁺ = 377.3.

### Step 3: preparation of tert-butyl 4-hydroxy-4-((2-(piperidin-4-yl)ethoxy)methyl)piperidine-1-carboxylate

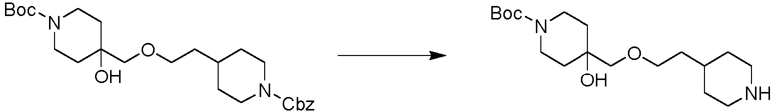

Pd/C (100 mg, 10%) was added to a solution oftert-butyl 4-((2-(1-((benzyloxy)carbonyl)piperidin-4-yl)ethoxy)methyl)-4-hydroxypiperidine-1-carboxylate (0.45 g, 0.94 mmol) in THF (50 mL). The mixture was reacted at room temperature for 16 h. The mixture was filtered, and the filtrate was concentrated to give *tert-butyl* 4-hydroxy-4-((2-(piperidin-4-yl)ethoxy)methyl)piperidine-1-carboxylate.

LC-MS: (ESI, m/z): [M+H]⁺ = 343.3.

### Step 4: preparation of tert-butyl 4-((2-(1-(4-chloro-3-(2,4-dioxotetrahydropyrimidin-1(2H)-yl)benzoyl)piperidin-4-yl)ethoxy)methyl)-4-hydroxypiperidine-1-carboxylate

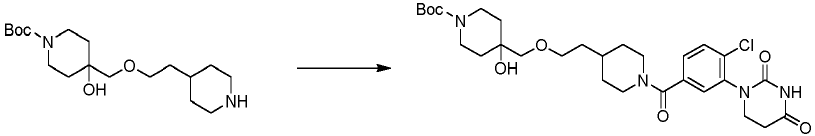

Pentafluorophenyl 4-chloro-3-(2,4-dioxotetrahydropyrimidin-1(2*H*)-yl)benzoate (291 mg, 0.67 mmol) and DIEA (248 mg, 1.92 mmol) were added to a solution of *tert*-butyl 4-hydroxy-4-((2-(piperidin-4-yl)ethoxy)methyl)piperidine-1-carboxylate (220 mg, 0.64 mmol) in DMF (10 mL), and the mixture was reacted at room temperature for 2 h. Water (30 mL) was added, and the mixture was extracted with ethyl acetate (30 mL × 2). The organic phase was washed with saturated brine, dried over anhydrous sodium sulfate, and filtered, and the filtrate was concentrated and purified by silica gel column chromatography (DCM:MeOH = 100:1 to 20:1) to give *tert*-butyl 4-((2-(1-(4-chloro-3-(2,4-dioxotetrahydropyrimidin-1(2*H*)-yl)benzoyl)piperidin-4-yl)ethoxy)methyl)-4-hydroxypiperidine-1-carboxylate.

LC-MS: (ESI, m/z): [M+H-100]⁺ = 493.2.

### Step 5: preparation of 1-(2-chloro-5-(4-(2-((4-hydroxypiperidin-4-yl)methoxy)ethyl)piperidine-1-carbonyl)phenyl)dihydropyrimidine-2,4(1H,3H)-dione

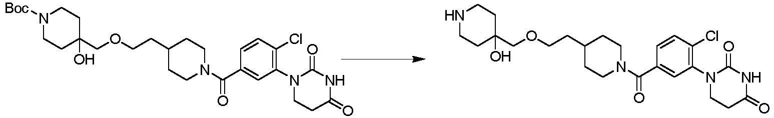

Hydrochloric acid/1,4-dioxane (1.5 mL) was added to a solution of *tert*-butyl 4-((2-(1-(4-chloro-3-(2,4-dioxotetrahydropyrimidin-1(2*H*)-yl)benzoyl)piperidin-4-yl)ethoxy)methyl)-4-hydroxypiperidine-1-carboxylate (190 mg, 0.32 mmol) in 1,4-dioxane (3 mL), and the mixture was stirred at room temperature for 1 h. The reaction liquid was directly concentrated under reduced pressure to give 1-(2-chloro-5-(4-(2-((4-hydroxypiperidin-4-yl)methoxy)ethyl)piperidine-1-carbonyl)phenyl)dihydropyrimidine-2,4(1*H*,3*H*)-dione.

LC-MS: (ESI, m/z): [M+H]⁺ = 493.2.

### Step 6: preparation of tert-butyl 3-(2-((1-((4-((2-(1-(4-chloro-3-(2,4-dioxotetrahydropyrimidin-1(2H)-yl)benzoyl)piperidin-4-yl)ethoxy)methyl)-4-hydroxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

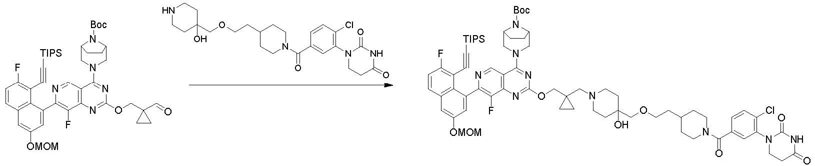

1-(2-Chloro-5-(4-(2-((4-hydroxypiperidin-4-yl)methoxy)ethyl)piperidine-1-carbonyl)phenyl)dihydropyrimidine-2,4(1*H*,3*H*)-dione (80 mg, 0.16 mmol) and tetraisopropyl titanate (500 mg, 1.8 mmol) were added to a solution of *tert-*butyl 3-(8-fluoro-7-(7-fluoro-3-(methoxymethoxy)-8-((triisopropylsilyl)ethynyl)naphthalen-1-y1)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-((1-((4-((2-(1-(4-chloro-3-(2,4-dioxotetrahydropyrimidin-1(2*H*)-yl)benzoyl)piperidin-4-yl)ethoxy)methyl)-4-hydroxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 660.0.

### Step 7: preparation of 1-(5-(4-(2-((1-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-4-hydroxypiperidin-4-yl)methoxy)ethyl)piperidine-1-carbonyl)-2-chlorophenyl)dihydropyrimidine-2,4(1H,3H)-dione

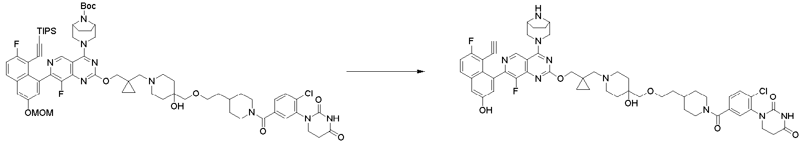

*tert*-Butyl 3-(2-((1-((4-((2-(1-(4-chloro-3-(2,4-dioxotetrahydropyrimidin-1(2*H*)-yl)benzoyl)piperidin-4-yl)ethoxy)methyl)-4-hydroxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (25 mg, 0.019 mmol) was dissolved in DMF (1 mL), and CsF (14 mg, 0.095 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(4-(2-((1-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-4-hydroxypiperidin-4-yl)methoxy)ethyl)piperidine-1-carbonyl)-2-chlorophenyl)dihydropyrimidine-2,4(1*H*,3*H*)-dione.

LC-MS: (ESI, m/z): [M/2+H]⁺ = 509.7.

¹H NMR (400 MHz, CD₃OD) δ 9.07 (s, 1H), 8.41 (br, 2H), 7.90 - 7.83 (m, 1H), 7.66 - 7.59 (m, 1H), 7.50 (s, 1H), 7.40 - 7.30 (m, 3H), 7.20 (d, *J =* 2.5 Hz, 1H), 4.73 - 4.65 (m, 4H), 4.60 - 4.51 (m, 4H), 4.46 - 4.37 (m, 2H), 3.96-3.89 (m, 2H), 3.84 - 3.76 (m, 4H), 3.66 - 3.58 (m, 2H), 3.49 - 3.43 (m, 2H), 3.37 (s, 1H), 3.23 - 3.11 (m, 4H), 2.89 - 2.82 (m, 2H), 2.08 - 1.89 (m, 6H), 1.81 - 1.59 (m, 5H), 1.49 - 1.43 (m, 2H), 1.16 - 1.05 (m, 2H), 0.99 - 0.93 (m, 2H), 0.90 - 0.82 (m, 2H).

### Example 64: 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)bicyclo[2.2.2]octan-1-yl)ethyl)-3,9-diazaspiro[5.5]undecane-3-carbonyl)-2-chlorophenyl)dihydropyrimidine-2,4(1H,3H)-dione

### Step 1: preparation of bicyclo[2.2.2]octane-1,4-diyldimethanol

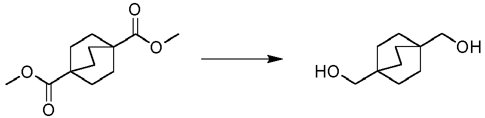

LAH (22 mL, 22.1 mmol, 1 M) was added to a solution of dimethyl bicyclo[2.2.2]octane-1,4-dicarboxylate (1 g, 4.4 mmol) in THF (10 mL) in an ice-water bath, and the mixture was reacted at room temperature for 2 h. Sodium sulfate decahydrate was added to the mixture. The resulting mixture was filtered and concentrated to give bicyclo[2.2.2]octane-1,4-diyldimethanol.

### Step 2: preparation of bicyclo[2.2.2]octane-1,4-dicarbaldehyde

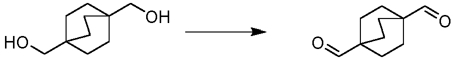

In a dry ice-acetone bath, bicyclo[2.2.2]octane-1,4-diyldimethanol (1 g, 5.87 mmol) was dissolved in dichloromethane (10 mL), and the mixture was added to a solution of oxalyl chloride (1.5 g, 11.75 mmol) and dimethyl sulfoxide (2.3 g, 29.35 mmol) in dichloromethane (20 mL). The mixture was reacted for 1 h, and triethylamine (5.9 g, 58.7 mmol) was then added. The mixture was stirred for another 15 min and then warmed to room temperature. Dilute hydrochloric acid (1 N, 50 mL) was added, and the mixture was extracted with dichloromethane. The organic phase was extracted with a saturated sodium bicarbonate solution. The organic phases were combined, dried over anhydrous sodium sulfate, filtered, and concentrated to give bicyclo[2.2.2]octane-1,4-dicarbaldehyde.

### Step 3: preparation of 1,4-bis(2-methoxyvinyl)bicyclo[2.2.2]octane

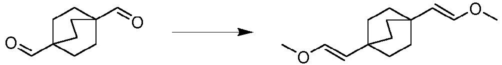

NaH (0.84 g, 21 mmol, 60%) was addded to a solution of (methoxymethyl)triphenylphosphonium chloride (7.2 g, 21 mmol) in DMF (50 mL) in an ice-water bath, and the mixture was reacted at room temperature for 0.5 h. Then a solution of bicyclo[2.2.2]octane-1,4-dicarbaldehyde (1 g, 6 mmol) in DMF (10 mL) was added dropwise, and the mixture was reacted at room temperature for 3 h. After the reaction was completed, H₂O (100 mL) was added, and the mixture was extracted with ethyl acetate (100 mL × 3). The combined organic phase was dried over anhydrous sodium sulfate, filtered, and concentrated in vacuum. The crude product was purified by silica gel column chromatography (PE:EA = 10:1) to give 1,4-bis(2-methoxyvinyl)bicyclo[2.2.2]octane.

LC-MS: (ESI, m/z): [M+H]⁺ = 223.3.

### Step 4: preparation of 2,2'-(bicyclo[2.2.2]octane-1,4-diyl)diacetaldehyde

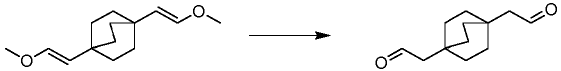

Dilute hydrochloric acid (3 mL, 1 N) was added to a solution of 1,4-bis(2-methoxyvinyl)bicyclo[2.2.2]octane (0.45 g, 2 mmol) in THF (6 mL), and the mixture was reacted at room temperature overnight. H₂O (20 mL) was added, and the mixture was extracted with ethyl acetate (20 mL × 3). The combined organic phase was dried over anhydrous sodium sulfate, filtered, and concentrated to give 2,2'-(bicyclo[2.2.2]octane-1,4-diyl)diacetaldehyde.

### Step 5: preparation of 2,2'-(bicyclo[2.2.2]octane-1,4-diyl)bis(ethan-1-ol)

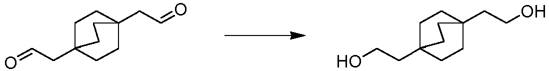

NaBH₄(378 mg, 10 mmol) was added to a solution of 2,2'-(bicyclo[2.2.2]octane-1,4-diyl)diacetaldehyde (0.4 g, 2 mmol) in methanol (20 mL), and the mixture was stirred at room temperature for 2 h. H₂O (20 mL) was added, and the mixture was extracted with ethyl acetate (20 mL × 3). The combined organic phase was dried over anhydrous sodium sulfate, filtered, and concentrated to give 2,2'-(bicyclo[2.2.2]octane-1,4-diyl)bis(ethan-1-ol).

### Step 6: preparation of tert-butyl 3-(7-chloro-8-fluoro-2-(2-(4-(2-hydroxyethyl)bicyclo[2.2.2]octan-1-yl)ethoxy)pyrido[4,3-d]pyrimidin-4-yl)-3,8-diazabicyclo[3.2.1]octane-8-carboxylate

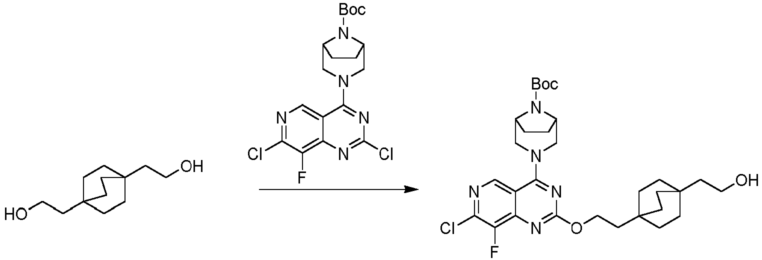

*tert*-Butyl 3-(2,7-dichloro-8-fluoropyrido[4,3-*d*]pyrimidin-4-yl)-3,8-diazabicyclo[3.2.1]octane-8-carboxylate (414 mg, 0.97 mmol) and 2,2'-(bicyclo[2.2.2]octane-1,4-diyl)bis(ethan-1-ol) (230 mg, 1.16 mmol) were added to THF (30 mL), and then cesium carbonate (0.94 g, 2.9 mmol) was added. The mixture was stirred at 80 °C for 16 h. After the reaction was completed, water (50 mL) was added, and the mixture was extracted with ethyl acetate (50 mL × 2). The organic phases were combined, dried over anhydrous sodium sulfate, and concentrated under reduced pressure. The concentrate was purified by silica gel column chromatography (PE:EA = 5:1) to give *tert*-butyl 3-(7-chloro-8-fluoro-2-(2-(4-(2-hydroxyethyl)bicyclo[2.2.2]octan-1-yl)ethoxy)pyrido[4,3-*d*]pyrimidin-4-yl)-3,8-diazabicyclo[3.2.1]octane-8-carboxylate.

LC-MS: (ESI, m/z): [M+H]⁺ = 590.3.

### Step 7: preparation of tert-butyl 3-(8-fluoro-7-(7-fluoro-3-(methoxymethoxy)-8-((triisopropylsilyl)ethynyl) naphthalen-1-yl)-2-(2-(4-(2-hydroxyethyl)bicyclo[2.2.2]octan-1-yl)ethoxy)pyrido[4,3-d]pyrimidin-4-yl)-3,8-diazabicyclo[3.2.1]octane-8-carboxylate

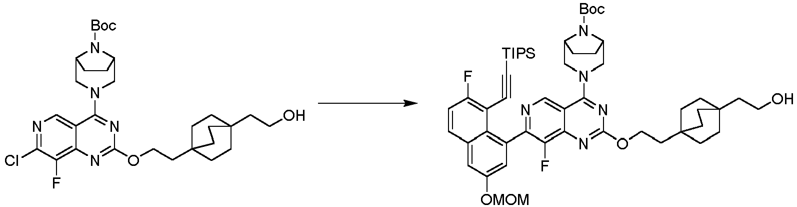

cataCXium A Pd G3 (26 mg, 0.035 mmol) and cesium carbonate (290 mg, 0.89 mmol) were added to a mixed solution of *tert*-butyl 3-(7-chloro-8-fluoro-2-(2-(4-(2-hydroxyethyl)bicyclo[2.2.2]octan-1-yl)ethoxy)pyrido[4,3-*d*]pyrimidin-4-yl)-3,8-diazabicyclo[3.2.1]octane-8-carboxylate (210 mg, 0.35 mmol) and (2-fluoro-6-(methoxymethoxy)-8-(4,4,5,5-tetramethyl-1,3,2-dioxaborolan-2-yl)naphthalen-1-yl)ethynyl)triisopropylsilane (274 mg, 0.53 mmol) in 1,4-dioxane (8 mL) and H₂O (2 mL). The mixture was reacted at 100 °C overnight under nitrogen atmosphere. The reaction liquid was directly filtered and concentrated under reduced pressure. The concentrate was purified by silica gel column chromatography (DCM:MeOH = 100:1) to give *tert*-butyl 3-(8-fluoro-7-(7-fluoro-3-(methoxymethoxy)-8-((triisopropylsilyl)ethynyl)naphthalen-1-yl)-2-(2-(4-(2-hydroxyethyl)bicyclo[2.2.2]octan-1-yl)ethoxy)pyrido[4,3-*d*]pyrimidin-4-yl)-3,8-diazabicyclo[3.2.1]octane-8-carboxylate.

LC-MS: (ESI, m/z): [M+H]⁺ = 940.9.

### Step 8: preparation of tert-butyl 3-(8-fluoro-7-(7-fluoro-3-(methoxymethoxy)-8-((triisopropylsilyl)ethynyl)naphthalen-1-yl)-2-(2-(4-(2-oxoethyl)bicyclo[2.2.2]octan-1-yl)ethoxy)pyrido[4,3-d]pyrimidin-4-yl)-3,8-diazabicyclo[3.2.1]octane-8-carboxylate

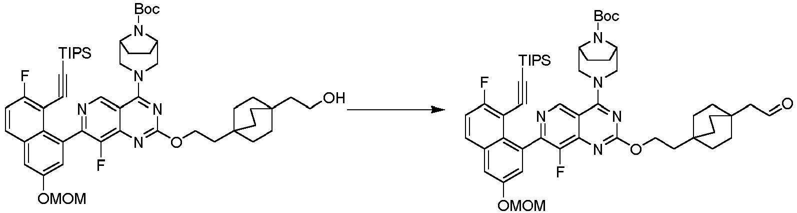

Dess-Martin reagent (108 mg, 0.26 mmol) was added to a solution of *tert*-butyl 3-(8-fluoro-7-(7-fluoro-3-(methoxymethoxy)-8-((triisopropylsilyl)ethynyl)naphthalen-1-yl)-2-(2-(4-(2-hydroxyethyl)bicyclo[2.2.2]octan-1-yl)ethoxy)pyrido[4,3-*d*]pyrimidin-4-yl)-3,8-diazabicyclo[3.2.1]octane-8-carboxylate (200 mg, 0.21 mmol) in dichloromethane (10 mL). The mixture was stirred at room temperature for 1 h. After the reaction was completed, a sodium thiosulfate solution (3 mL) and a sodium bicarbonate solution (3 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (PE:EA = 1:1) to give *tert*-butyl 3-(8-fluoro-7-(7-fluoro-3-(methoxymethoxy)-8-((triisopropylsilyl)ethynyl)naphthalen-1-yl)-2-(2-(4-(2-oxoethyl)bicyclo [2.2.2]octan-1-yl)ethoxy)pyrido[4,3-*d*]pyrimidin-4-yl)-3,8-diazabicyclo[3.2.1]octane-8-carboxylate.

LC-MS: (ESI, m/z): [M+H]⁺ = 938.9.

### Step 9: preparation of tert-butyl 3-(2-(2-(4-(2-(9-(4-chloro-3-(2,4-dioxotetrahydropyrimidin-1(2H)-yl)benzoyl)-3,9-diazaspiro[5.5]undecan-3-yl)ethyl)bicyclo[2.2.2]octan-1-yl)ethoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

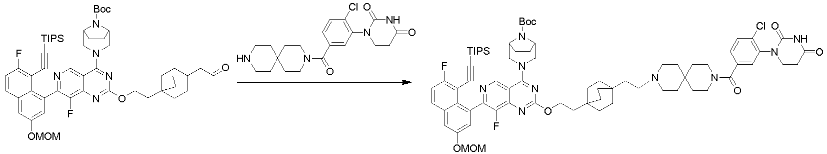

1-(2-Chloro-5-(3,9-diazaspiro[5.5]undecane-3-carbonyl)phenyl)dihydropyrimidine-2,4(1*H*,3*H*)-dione hydrochloride (62 mg, 0.15 mmol) and tetraisopropyl titanate (170 mg, 0.6 mmol) were added to a solution of *tert-*butyl 3-(8-fluoro-7-(7-fluoro-3-(methoxymethoxy)-8-((triisopropylsilyl)ethynyl)naphthalen-1-yl)-2-(2-(4-(2-oxoethyl)bicyclo [2.2.2]octan-1-yl)ethoxy)pyrido[4,3-*d*]pyrimidin-4-yl)-3,8-diazabicyclo[3.2.1]octane-8-carboxylate (120 mg, 0.13 mmol) in dichloromethane (5 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (25 mL × 3). The combined organic phase was dried over anhydrous sodium sulfate, filtered, and concentrated in vacuum. The crude product was purified by silica gel column chromatography (DCM:MeOH = 20:1) to give *tert*-butyl 3-(2-(2-(4-(2-(9-(4-chloro-3-(2,4-dioxotetrahydropyrimidin-1(2*H*)-yl)benzoyl)-3,9-diazaspiro[5.5]undecan-3-yl)ethyl)bicyclo[2.2.2]octan-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 664.0.

### Step 10: preparation of 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)ethyl)bicyclo[2.2.2]octan-1-yl)ethyl)-3,9-diazaspiro[5.5]undecane-3-carbonyl)-2-chlorophenyl)dihydropyrimidine-2,4(1H,3H)-dione

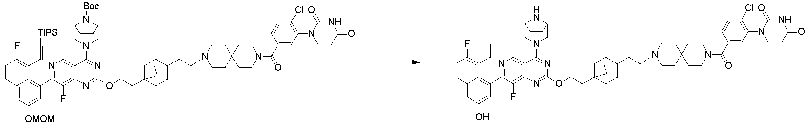

*tert*-Butyl 3-(2-(2-(4-(2-(9-(4-chloro-3-(2,4-dioxotetrahydropyrimidin-1(2*H*)-yl)benzoyl)-3,9-diazaspiro[5.5]undecan-3-yl)ethyl)bicyclo[2.2.2]octan-1-yl)ethoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (125 mg, 0.094 mmol) was dissolved in DMF (5 mL), and CsF (72 mg, 0.47 mmol) was added. The mixture was reacted at room temperature for 1 h. After the reaction was completed, the mixture was diluted with H₂O (10 mL) and extracted with ethyl acetate (10 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-(2-(4-(2-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)ethyl)bicyclo[2.2.2] octan-1-yl)ethyl)-3,9-diazaspiro[5.5]undecane-3-carbonyl)-2-chlorophenyl)dihydropyrimidine-2,4(1H,3H)-dione.

LC-MS: (ESI, m/z): [M/2+H]⁺ = 513.7.

¹H NMR (400 MHz, DMSO-*d*₆) δ 10.50 (s, 1H), 10.17 (br, 1H), 9.02 (s, 1H), 7.97 (dd, *J* = 9.1, 6.0 Hz, 1H), 7.63 (d, *J* = 8.2 Hz, 1H), 7.54 (d, *J =* 1.7 Hz, 1H), 7.46 (t, *J =* 9.0 Hz, 1H), 7.41 - 7.36 (m, 2H), 7.17 (d, *J=* 2.3 Hz, 1H), 4.53 - 4.22 (m, 4H), 3.92 (s, 1H), 3.82 - 3.71 (m, 1H), 3.69 - 3.43 (m, 8H), 2.78 - 2.70 (m, 2H), 2.35 - 2.15 (m, 6H), 1.71 - 1.60 (m, 4H), 1.55 (t, *J* = 7.2 Hz, 2H), 1.48 - 1.30 (m, 20H), 1.23 - 1.16 (m, 2H).

### Example 65: 1-(5-(4-((2-(1-((1-(((7-(6-amino-4-methyl-3-(trifluoromethyl)pyridin-2-yl)-4-(3,8-diazabicyclo[3.2.1]octan-3-yl)-6-chloro-8-fluoroquinazolin-2-yl)oxy)methyl)cyclopropyl)methyl)piperidin-4-yl)ethoxy)methyl)piperidine-1-carbonyl)-2-chlorophenyl)dihydropyrimidine-2,4(1H,3H)-dione

### Step 1: preparation of 6-bromo-N,N-bis(4-methoxybenzyl)-4-methylpyridin-2-amine

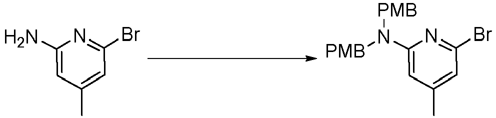

A solution of 6-bromo-4-methylpyridin-2-amine (25.00 g, 133.66 mmol) in *N,N-*dimethylformamide (10 mL) and tetrahydrofuran (100 mL) was slowly added to a suspension of sodium hydride (16.04 g, 400.98 mmol, 60%) in tetrahydrofuran (400 mL) at 0 °C under nitrogen atmosphere, and the mixture was reacted at 10 °C for 1 h under nitrogen atmosphere. 4-Methoxybenzyl chloride (62.80 g, 400.98 mmol) was then added at 0 °C, and the mixture was reacted at 25 °C for another 18 h. After the reaction was completed, the reaction liquid was poured into a saturated aqueous ammonium chloride solution (500 mL), and the mixture was extracted with ethyl acetate (200 mL × 3). The organic phases ere combined, washed with saturated brine (500 mL), dried over anhydrous sodium sulfate, filtered, and concentrated under reduced pressure to give 6-bromo-*N*,*N*-bis(4-methoxybenzyl)-4-methylpyridin-2-amine.

LC-MS: (ESI, m/z): [M+H]⁺ = 427.1.

### Step 2: preparation of N,N-bis(4-methoxybenzyl)-4-methyl-6-(tributylstannyl)pyridin-2-amine

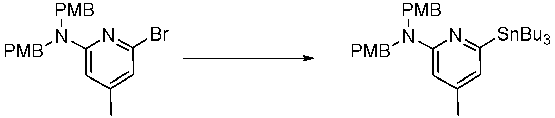

An n-butyllithium solution (1.6 M, 26.3 mL) was added dropwise to a solution of 6-bromo-*N*,*N-*bis(4-methoxybenzyl)-4-methylpyridin-2-amine (37.90 g, 23.40 mmol) in tetrahydrofuran (200 mL) at - 65 °C under nitrogen atmosphere, and the mixture was heated to -45 °C and stirred for 1 h. Then the mixture was cooled to -65 °C, and tri-n-butyltin chloridean (19.00 g, 58.50 mmol) was added dropwise. The mixture was slowly warmed to room temperature and stirred for 18 h. After the reaction was completed, the reaction liquid was poured into a saturated aqueous ammonium chloride solution (500 mL), and the mixture was extracted with ethyl acetate (150 mL × 2). The organic phases were combined, washed with saturated brine (200 mL), dried over anhydrous sodium sulfate, filtered, and concentrated under reduced pressure to give a crude product. The crude product was purified by silica gel column chromatography (petroleum ether:ethyl acetate = 20:1 to 10:1) to give *N*,*N*-bis(4-methoxybenzyl)-4-methyl-6-(tributylstannyl)pyridin-2-amine.

Step 3: preparation of *tert-*butyl 3-(7-bromo-2,6-dichloro-8-fluoroquinazolin-4-yl)-3,8-diazabicyclo[3.2.1]octane-8-carboxylate

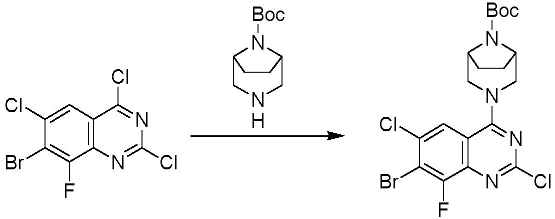

Triethylamine (3.212 g, 31.8 mmol) and *tert*-butyl 3,8-diazabicyclo[3.2.1]octane-8-carboxylate (2.03 g, 9.54 mmol) were added to a solution of 7-bromo-2,4,6-trichloro-8-fluoroquinazoline (3.5 g, 10.6 mmol) in anhydrous dichloromethane (40 mL) at -50 °C, and the mixture was stirred at -50 °C for 2 h. After the reaction was completed, saturated ammonium chloride (50 mL) was added, and the mixture was extracted with dichloromethane (50 mL × 3). The organic phases were combined, dried over anhydrous sodium sulfate, and concentrated under reduced pressure. The resulting crude product was purified by silica gel column chromatography (petroleum ether:ethyl acetate = 3:1) to give *tert*-butyl 3-(7-bromo-2,6-dichloro-8-fluoroquinazolin-4-yl)-3,8-diazabicyclo[3.2.1]octane-8-carboxylate.

LC-MS: (ESI, m/z): [M+H]⁺ = 506.9.

### Step 4: preparation of tert-butyl 3-(7-bromo-2-((1-(((tertbutyldimethylsilyl)oxy)methyl)cyclopropyl)methoxy)-6-chloro-8-fluoroquinazolin-4-yl)-3,8-diazabicyclo[3.2.1]octane-8-carboxylate

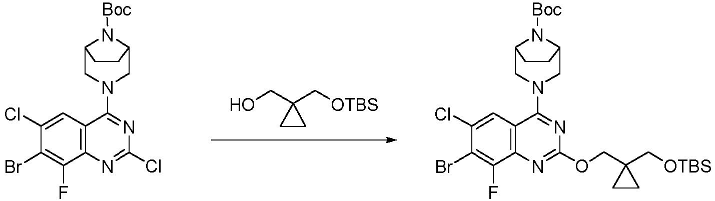

1,4-Diazabicyclo[2.2.2]octane (0.28 g, 2.47 mmol) and cesium carbonate (3.22 mg, 9.88 mmol) were added to a solution of *tert*-butyl 3-(7-bromo-2,6-dichloro-8-fluoroquinazolin-4-yl)-3,8-diazabicyclo[3.2.1]octane-8-carboxylate (2.50 g, 4.94 mmol) and (1-(((*tert*butyldimethylsilyl)oxy)methyl)cyclopropyl)methanol (2.14 g, 9.88 mmol) in anhydrous acetonitrile (25 mL) at room temperature, and the mixture was stirred at 25 °C for 18 h. After the reaction was completed, the reaction liquid was poured into ice water (100 mL), and the mixture was extracted with ethyl acetate (50 mL × 3). The organic phases were combined, washed with saturated brine (100 mL), dried over anhydrous sodium sulfate, filtered, and concentrated under reduced pressure to give a crude product. The crude product was purified by silica gel column chromatography (petroleum ether:tetrahydrofuran = 200:1 to 20:1) to give *tert-butyl* 3-(7-bromo-2-((1-(((*tert*-butyldimethylsilyl)oxy) methyl)cyclopropyl)methoxy)-6-chloro-8-fluoroquinazolin-4-yl)-3,8-diazabicyclo[3.2.1]octane-8-carboxylate.

LC-MS: (ESI, m/z): [M+H]⁺ = 685.0.

### Step 5: preparation of tert-butyl 3-(7-(6-(bis(4-methoxybenzyl)amino)-4-methylpyridin-2-yl)-2-((1-(((tert-butyldimethylsilyl)oxy)methyl)cyclopropyl)methoxy)-6-chloro-8-fluoroquinazolin-4-yl)-3,8-diazabicyclo[3.2.1]octane-8-carboxylate

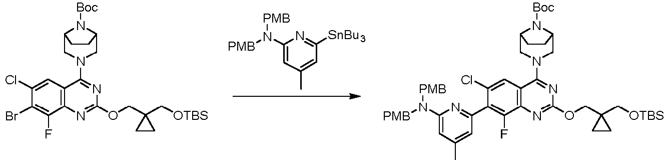

Tetrakis(triphenylphosphine)palladium(O) (0.34 g, 0.23 mmol) was added to a solution of *tert-*butyl 3-(7-bromo-2-((1-(((*tert*-butyldimethylsilyl)oxy)methyl)cyclopropyl)methoxy)-6-chloro-8-fluoroquinazolin-4-yl)-3,8-diazabicyclo[3.2.1]octane-8-carboxylate (2.01 g, 2.93 mmol) and *N,N*-bis(4-methoxybenzyl)-4-methyl-6-(tributylstannyl)pyridin-2-amine (5.61 g, 8.79 mmol) in 1,4-dioxane (80 mL) at room temperature, and the mixture was reacted at 110 °C for 18 h under nitrogen atmosphere. After the reaction was completed, the mixture was cooled to room temperature. A saturated aqueous potassium fluoride solution (100 mL) was added, and the mixture was stirred for 2 h and filtered through celite. The filtrate was extracted with ethyl acetate (50 mL × 3). The organic phases were combined, washed with saturated brine (100 mL), dried over anhydrous sodium sulfate, filtered, and concentrated under reduced pressure to give a crude product. The crude product was purified by silica gel column chromatography (petroleum ether:tetrahydrofuran = 30:1 to 10:1) to give *tert*-butyl 3-(7-(6-(bis(4-methoxybenzyl)amino)-4-methylpyridin-2-yl)-2-((1-(((tert-butyldimethylsilyl) oxy)methyl)cyclopropyl)methoxy)-6-chloro-8-fluoroquinazolin-4-yl)-3,8-diazabicyclo[3.2.1]octane-8-carboxylate.

LC-MS: (ESI, m/z): [M+H]⁺ = 952.8.

### Step 6: preparation of tert-butyl 3-(7-(6-(bis(4-methoxybenzyl)amino)-3-iodo-4-methylpyridin-2-yl)-2-((1-(((tert-butyldimethylsilyl)oxy)methyl)cyclopropyl)methoxy)-6-chloro-8-fluoroquinazolin-4-yl)-3,8-diazabicyclo[3.2.1]octane-8-carboxylate

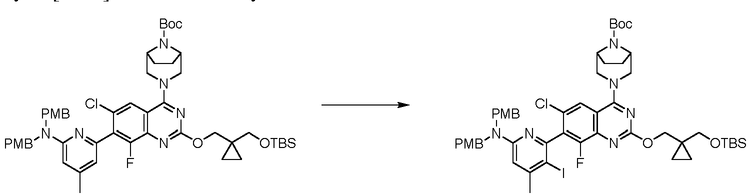

*N*-Iodosuccinimide (107 mg, 477.35 µmol) was added to a solution of *tert-butyl* 3-(7-(6-(bis(4-methoxybenzyl)amino)-4-methylpyridin-2-yl)-2-((1-(((*tert*-butyldimethylsilyl)oxy)methyl)cyclopropyl) methoxy)-6-chloro-8-fluoroquinazolin-4-yl)-3,8-diazabicyclo[3.2.1]octane-8-carboxylate (303 mg, 318.23 µmol) in glacial acetic acid (15 mL) at room temperature, and the mixture was reacted at 20 °C for 2 h under nitrogen atmosphere. After the reaction was completed, the reaction liquid was poured into a saturated aqueous sodium carbonate solution (200 mL), and the mixture was extracted with ethyl acetate (50 mL × 2). The organic phases were combined, washed with saturated brine (100 mL), dried over anhydrous sodium sulfate, filtered, and concentrated under reduced pressure to give a crude product. The crude product was purified by silica gel column chromatography (petroleum ether:tetrahydrofuran = 100:1 to 10:1) to give *tert-*butyl 3-(7-(6-(bis(4-methoxybenzyl)amino)-3-iodo-4-methylpyridin-2-yl)-2-((1-(((*tert*butyldimethylsilyl)oxy)methyl)cyclopropyl)methoxy)-6-chloro-8-fluoroquinazolin-4-yl)-3,8-diazabicyclo[3.2.1]octane-8-carboxylate.

LC-MS: (ESI, m/z): [M+H]⁺ = 1078.6.

### Step 7: preparation of tert-butyl 3-(7-(6-(bis(4-methoxybenzyl)amino)-4-methyl-3-(trifluoromethyl)pyridin-2-yl)-2-((1-(((tert-butyldimethylsilyl)oxy)methyl)cyclopropyl)methoxy)-6-chloro-8-fluoroquinazolin-4-yl)-3,8-diazabicyclo[3.2.1]octane-8-carboxylate

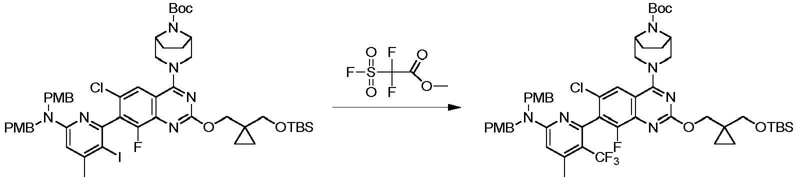

Cuprous iodide (579 mg, 3040.17 µmol) and methyl 2,2-difluoro-2-(fluorosulfonyl)acetate (1459 mg, 7595.50 µmol) were added to a solution of *ter*t*-*butyl 3-(7-(6-(bis(4-methoxybenzyl)amino)-3-iodo-4-methylpyridin-2-yl)-2-((1-(((*tert*-butyldimethylsilyl)oxy)methyl)cyclopropyl)methoxy)-6-chloro-8-fluoroquinazolin-4-yl)-3,8-diazabicyclo[3.2.1]octane-8-carboxylate (266 mg, 246.39 µmol) and hexamethylphosphoramide (544 mg, 3038.20 µmol) in *N*,*N*-dimethylacetamide (10 mL) at room temperature, and the mixture was reacted at 80 °C for 18 h under nitrogen atmosphere. After the reaction was completed, the mixture was diluted with ethyl acetate (100 mL), stirred for 15 min, and filtered through celite. The filtrate was washed with saturated brine (100 mL), dried over anhydrous sodium sulfate, filtered, and concentrated under reduced pressure to give a crude product. The crude product was purified by silica gel column chromatography (petroleum ether:tetrahydrofuran = 25:1 to 5:1) to give *tert-butyl* 3-(7-(6-(bis(4-methoxybenzyl)amino)-4-methyl-3-(trifluoromethyl)pyridin-2-yl)-2-((1-(((*tert-*butyldimethylsllyl)oxy)methyl)cyclopropyl)methoxy)-6-chloro-8-fluoroquinazolin-4-yl)-3,8-diazabicyclo[3.2.1]octane-8-carboxylate.

LC-MS: (ESI, m/z): [M+H]⁺ = 1020.8.

### Step 8: preparation of tert-butyl 3-(7-(6-(bis(4-methoxybenzyl)amino)-4-methyl-3-(trifluoromethyl)pyridin-2-yl)-6-chloro-8-fluoro-2-((1-(hydroxymethyl)cyclopropyl)methoxy)quinazolin-4-yl)-3,8-diazabicyclo[3.2.1]octane-8-carboxylate

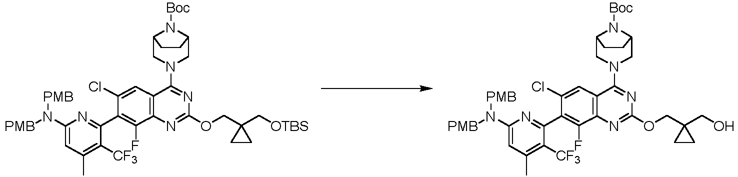

Cesium fluoride (176 mg, 1157.00 µmol) was added to a solution of *tert-*butyl 3-(7-(6-(bis(4-methoxybenzyl)amino)-4-methyl-3-(trifluoromethyl)pyridin-2-yl)-2-((1-(((*tert*-butyldimethylsilyl)oxy) methyl)cyclopropyl)methoxy)-6-chloro-8-fluoroquinazolin-4-yl)-3,8-diazabicyclo[3.2.1]octane-8-carboxylate (118 mg, 115.50 µmol) in *N*,*N*-dimethylformamide (10 mL) at room temperature, and the mixture was reacted at 60 °C for 18 h under nitrogen atmosphere. After the reaction was completed, the reaction liquid was poured into water (100 mL), and the mixture was extracted with ethyl acetate (20 mL × 3). The organic phases were combined, washed with saturated brine (100 mL), dried over anhydrous sodium sulfate, filtered, and concentrated under reduced pressure to give *tert-butyl* 3-(7-(6-(bis(4-methoxybenzyl)amino)-4-methyl-3-(trifluoromethyl)pyridin-2-yl)-6-chloro-8-fluoro-2-((1-(hydroxymethyl)cyclopropyl)methoxy)quinazolin-4-yl)-3,8-diazabicyclo[3.2.1]octane-8-carboxylate.

LC-MS: (ESI, m/z): [M+H]⁺ = 906.7.

### Step 9: preparation of tert-butyl 3-(7-(6-(bis(4-methoxybenzyl)amino)-4-methyl-3-(trifluoromethyl)pyridin-2-yl)-6-chloro-8-fluoro-2-((1-formylcyclopropyl)methoxy)quinazolin-4-yl)-3,8-diazabicyclo[3.2.1]octane-8-carboxylate

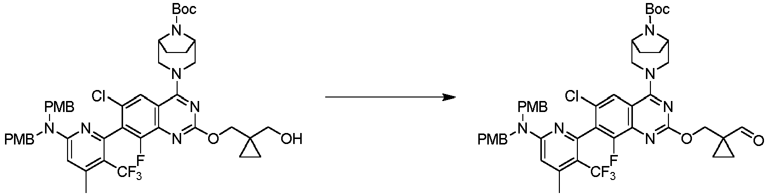

Dess-Martin oxidant (74 mg, 173.55 µmol) was added to a solution of *tert-butyl* 3-(7-(6-(bis(4-methoxybenzyl)amino)-4-methyl-3-(trifluoromethyl)pyridin-2-yl)-6-chloro-8-fluoro-2-((1-(hydroxymethyl) cyclopropyl)methoxy)quinazolin-4-yl)-3,8-diazabicyclo[3.2.1]octane-8-carboxylate (165 mg, 115.50 µmol) in dichloromethane (10 mL) at room temperature, and the mixture was reacted at 25 °C for 1 h under nitrogen atmosphere. After the reaction was completed, the reaction liquid was poured into a saturated aqueous sodium bicarbonate solution (50 mL), and the mixture was extracted with ethyl acetate (20 mL × 3). The organic phases were combined, washed with saturated brine (50 mL), dried over anhydrous sodium sulfate, filtered, concentrated under reduced pressure, and purified by a preparative thin-layer chromatography plate (dichloromethane:methanol (NH₃) = 10:1) to give *tert-*butyl 3-(7-(6-(bis(4-methoxybenzyl)amino)-4-methyl-3-(trifluoromethyl)pyridin-2-yl)-6-chloro-8-fluoro-2-((1-formylcyclopropyl)methoxy)quinazolin-4-yl)-3,8-diazabicyclo[3.2.1]octane-8-carboxylate.

LC-MS: (ESI, m/z): [M+H]⁺ = 904.7.

### Step 10: preparation of tert-butyl 3-(7-(6-(bis(4-methoxybenzyl)amino)-4-methyl-3-(trifluoromethyl)pyridin-2-yl)-6-chloro-2-((1-((4-(2-((1-(4-chloro-3-(2,4-dioxotetrahydropyrimidin-1(2H)-yl)benzoyl)piperidin-4-yl)methoxy)ethyl)piperidin-1-yl)methyl)cyclopropyl)methoxy)-8-fluoroquinazolin-4-yl)-3,8-diazabicyclo[3.2.1]octane-8-carboxylate

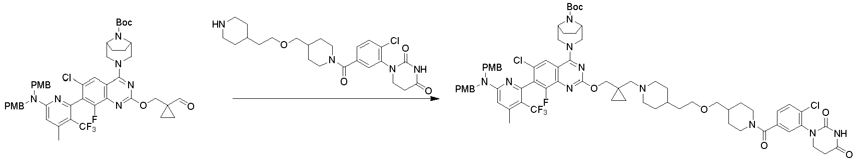

Tetraisopropyl titanate (147 mg, 517.20 mmol) and glacial acetic acid (0.1 mL) were added to a solution of *tert*-butyl 3-(7-(6-(bis(4-methoxybenzyl)amino)-4-methyl-3-(trifluoromethyl)pyridin-2-yl)-6-chloro-8-fluoro-2-((1-formylcyclopropyl)methoxy)quinazolin-4-yl)-3,8-diazabicyclo[3.2.1]octane-8-carboxylate (86 mg, 94.99 µmol) and 1-(2-chloro-5-(4-((2-(piperidin-4-yl)ethoxy)methyl)piperidine-1-carbonyl)phenyl)dihydropyrimidine-2,4(1*H*,3*H*)-dione (65 mg, 136.27 µmol) in dichloromethane (10 mL) at room temperature, and the mixture was reacted at 25 °C for 2 h under nitrogen atmosphere. Then sodium triacetoxyborohydride (62 mg, 292.54 mmol) was added, and the mixture was reacted at 25 °C for another 18 h. Methanol (5 mL) was added to quench the reaction, and the mixture was concentrated under reduced pressure at 40 °C to give a crude product of *tert*-butyl 3-(7-(6-(bis(4-methoxybenzyl)amino)-4-methyl-3-(trifluoromethyl)pyridin-2-yl)-6-chloro-2-((1-((4-(2-((1-(4-chloro-3-(2,4-dioxotetrahydropyrimidin-1(2*H*)-yl)benzoyl)piperidin-4-yl)methoxy)ethyl)piperidin-1-yl)methyl)cyclopropyl)methoxy)-8-fluoroquinazolin-4-yl)-3,8-diazabicyclo[3.2.1]octane-8-carboxylate.

LC-MS: (ESI, m/z): [M+H]⁺ = 1365.6.

### Step 11: preparation of 1-(5-(4-((2-(1-((1-(((7-(6-amino-4-methyl-3-(trifluoromethyl)pyridin-2-yl)-4-(3,8-diazabicyclo[3.2.1]octan-3-yl)-6-chloro-8-fluoroquinazolin-2-yl)oxy)methyl)cyclopropyl)methyl)piperidin-4-yl)ethoxy)methyl)piperidine-1-carbonyl)-2-chlorophenyl)dihydropyrimidine-2,4(1H,3H)-dione

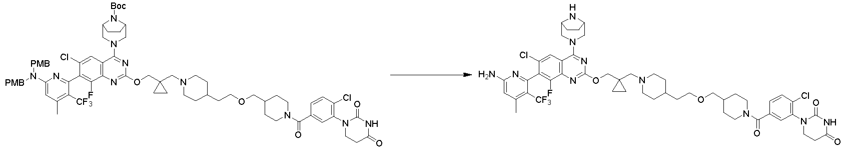

Trifluoroacetic acid (1 mL) and p-toluenesulfonic acid monohydrate (57 mg, 299.68 µmol) were added to a solution of *tert*-butyl 3-(7-(6-(bis(4-methoxybenzyl)amino)-4-methyl-3-(trifluoromethyl)pyridin-2-yl)-6-chloro-2-((1-((4-(2-((1-(4-chloro-3-(2,4-dioxotetrahydropyrimidin-1(2*H*)-yl)benzoyl)piperidin-4-yl)methoxy) ethyl)piperidin-1-yl)methyl)cyclopropyl)methoxy)-8-fluoroquinazolin-4-yl)-3,8-diazabicyclo[3.2.1]octane-8-carboxylate (41 mg, 30.00 µmol) in dichloromethane (2 mL) at room temperature, and the mixture was reacted at 25 °C for 18 h. After the reaction was completed, the mixture was concentrated to dryness under reduced pressure at 30 °C, and a solution of ammonia in methanol (1 mL) was added. Then the mixture was concentrated to dryness under reduced pressure at 30 °C. The resulting crude product was purified by a preparative thin-layer chromatography plate (dichloromethane:methanol = 10:1, a 0.1% solution of ammonia in methanol) and then purified by preparative high-performance liquid chromatography to give 1-(5-(4-((2-(1-((1-(((7-(6-amino-4-methyl-3-(trifluoromethyl)pyridin-2-yl)-4-(3,8-diazabicyclo[3.2.1]octan-3-yl)-6-chloro-8-fluoroquinazolin-2-yl)oxy)methyl)cyclopropyl)methyl)piperidin-4-yl)ethoxy)methyl)piperidine-1-carbonyl)-2-chlorophenyl)dihydropyrimidine-2,4(1*H*,3*H*)-dione.

LC-MS: (ESI, m/z): [M/2+H]⁺ = 512.9.

¹H NMR (400 MHz, CD₃OD) δ 7.82 (s, 1H), 7.63 (d, *J =* 8.3 Hz, 1H), 7.52 (d, *J =* 1.9 Hz, 1H), 7.41 (d, *J =* 8.1 Hz, 1H), 6.59 (s, 1H), 4.63 - 4.54 (m, 1H), 4.48 - 4.32 (m, 4H), 3.82 - 3.71 (m, 3H), 3.63 - 3.56 (m, 4H), 3.45 (t, *J* = 6.2 Hz, 2H), 3.28 - 3.25 (m, 1H), 3.18 - 3.05 (m, 3H), 2.91 - 2.77 (m, 3H), 2.55 - 2.45 (m, 2H), 2.44 (d, *J* = 1.3 Hz, 3H), 2.08 - 1.92 (m, 3H), 1.84 - 1.79 (m, 4H), 1.69 - 1.65 (m, 2H), 1.49 - 1.44 (m, 2H), 1.36 - 1.31 (m, 2H), 1.26 - 1.15 (m, 4H), 0.95 - 0.85 (m, 2H), 0.75 - 0.65 (m, 2H), 0.55 - 0.45 (m, 2H).

### Example 66: 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-4-methoxypiperidin-4-yl)methoxy)ethyl)piperidine-1-carbonyl)-2-chlorophenyl)dihydropyrimidine-2,4(1H,3H)-dione

### Step 1: preparation of benzyl 4-(2-(tosyloxy)ethyl)piperidine-1-carboxylate

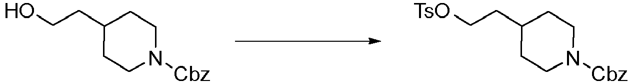

DMAP (230 mg, 1.9 mmol) and triethylamine (9.6 g, 57 mmol) were added to a solution of benzyl 4-(2-hydroxyethyl)piperidine-1-carboxylate (5 g, 19 mmol) in DCM (150 mL). The mixture was stirred at 0 °C for 15 min. Then TsCl (4.4 g, 22.5 mmol) was added, and the mixture was stirred at room temperature overnight. The reaction liquid was concentrated and purified by silica gel column chromatography (PE:EA = 100:1 to 1:1) to give benzyl 4-(2-(tosyloxy)ethyl)piperidine-1-carboxylate.

LC-MS: (ESI, m/z): [M+H]⁺ = 418.1.

### Step 2: preparation of tert-butyl 4-(hydroxymethyl)-4-methoxypiperidine-1-carboxylate

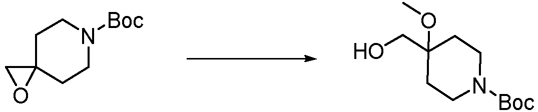

*tert*-Butyl 1-oxa-6-azaspiro[2.5]octane-6-carboxylate (1 g, 4.7 mmol) was dissolved in MeOH (15 mL), and *p*-toluenesulfonic acid (40 mg, 0.23 mmol) was added. The mixture was stirred at room temperature for 16 h. The reaction liquid was directly concentrated, and the resulting crude product was purified by silica gel column chromatography (PE:EA = 100:1 to 1:1) to give *tert*-buty*l* 4-(hydroxymethyl)-4-methoxypiperidine-1-carboxylate.

¹H NMR (400 MHz, CDCl₃) δ 3.50 - 3.90 (m, 2H), 3.51 (s, 2H), 3.24 (s, 3H), 3.14 (t, *J* = 11.6 Hz, 2H), 1.82 - 1.76 (m, 2H), 1.49 - 1.41 (m, 11H).

### Step 3: preparation of tert-butyl 4-((2-(1-((benzyloxy)carbonyl)piperidin-4-yl)ethoxy)methyl)-4-methoxypiperidine-1-carboxylate

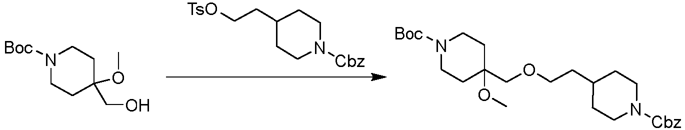

NaH (66 mg, 1.65 mmol, 60%) was added to a solution of *tert*-butyl 4-(hydroxymethyl)-4-methoxypiperidine-1-carboxylate (0.34 g, 1.38 mmol) in THF (10 mL), and the mixture was reacted at the temperature of an ice-water bath to room temperature for 1 h. Then benzyl 4-(2-(tosyloxy)ethyl)piperidine-1-carboxylate (637 mg, 1.52 mmol) was added, and the reaction liquid was stirred at 60 °C overnight. After the reaction was completed, the reaction liquid was directly concentrated, and the resulting crude product was purified by silica gel column chromatography (DCM:MeOH = 20:1) to give *tert*-butyl 4-((2-(1-((benzyloxy)carbonyl)piperidin-4-yl)ethoxy)methyl)-4-methoxypiperidine-1-carboxylate.

LC-MS: (ESI, m/z): [M-Boc+H]⁺ = 391.3.

### Step 4: preparation of tert-butyl 4-methoxy-4-((2-(piperidin-4-yl)ethoxy)methyl)piperidine-1-carboxylate

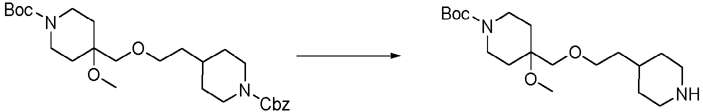

Pd/C (25 mg, 10%) was added to a solution of *tert*-butyl 4-((2-(1-((benzyloxy)carbonyl)piperidin-4-yl)ethoxy)methyl)-4-methoxypiperidine-1-carboxylate (230 mg, crude) in THF (10 mL), and the mixture was reacted at room temperature overnight. After the reaction was completed, the mixture was filtered, and the filtrate was concentrated to give *tert*-butyl 4-methoxy-4-((2-(piperidin-4-yl)ethoxy)methyl)piperidine-1-carboxylate.

### Step 5: preparation of tert-butyl 4-((2-(1-(4-chloro-3-(2,4-dioxotetrahydropyrimidin-1(2H)-yl)benzoyl)piperidin-4-yl)ethoxy)methyl)-4-methoxypiperidine-1-carboxylate

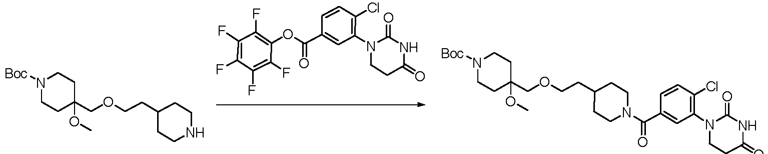

Pentafluorophenyl 4-chloro-3-(2,4-dioxotetrahydropyrimidin-1(2*H*)-yl)benzoate (205 mg, 0.47 mmol) and DIEA (151 mg, 1.17 mmol) were added to a solution of *tert-butyl* 4-methoxy-4-((2-(piperidin-4-yl)ethoxy)methyl)piperidine-1-carboxylate (140 mg, 0.39 mmol) in DMF (5 mL), and the mixture was reacted at room temperature for 2 h. Water (10 mL) was added to the reaction liquid, and the mixture was extracted with ethyl acetate (10 mL × 2). The organic phase was washed with saturated brine, dried over anhydrous sodium sulfate, and filtered, and the filtrate was concentrated and purified by silica gel column chromatography (DCM:MeOH = 100:1 to 20:1) to give *tert*-butyl 4-((2-(1-(4-chloro-3-(2,4-dioxotetrahydropyrimidin-1(2*H*)-yl)benzoyl)piperidin-4-yl)ethoxy)methyl)-4-methoxypiperidine-1-carboxylate.

LC-MS: (ESI, m/z): [M-Boc+H]⁺ = 507.0.

### Step 6: preparation of 1-(2-chloro-5-(4-(2-((4-methoxypiperidin-4-yl)methoxy)ethyl)piperidine-1-carbonyl)phenyl)dihydropyrimidine-2,4(1H,3H)-dione

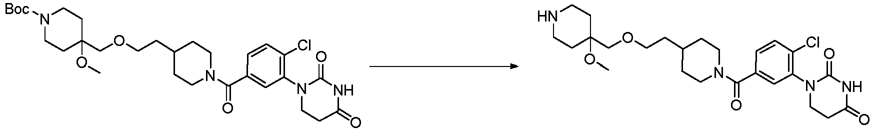

Trifluoroacetic acid (1 mL) was added to a solution of *tert*-butyl 4-((2-(1-(4-chloro-3-(2,4-dioxotetrahydropyrimidin-1(2*H*)-yl)benzoyl)piperidin-4-yl)ethoxy)methyl)-4-methoxypiperidine-1-carboxylate (130 mg, 0.21 mmol) in dichloromethane (3 mL), and the reaction liquid was stirred at 25 °C for 1 h. After the reaction was completed, the reaction liquid was directly concentrated. DIEA was added to the resulting crude product, and the mixture was then concentrated under reduced pressure to give 1-(2-chloro-5-(4-(2-((4-methoxypiperidin-4-yl)methoxy)ethyl)piperidine-1-carbonyl)phenyl)dihydropyrimidine-2,4(1H,3H)-dione.

LC-MS: (ESI, m/z): [M+H]⁺ = 507.0.

### Step 7: preparation of tert-butyl 3-(2-((1-((4-((2-(1-(4-chloro-3-(2,4-dioxotetrahydropyrimidin-1(2H)-yl)benzoyl)piperidin-4-yl)ethoxy)methyl)-4-methoxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

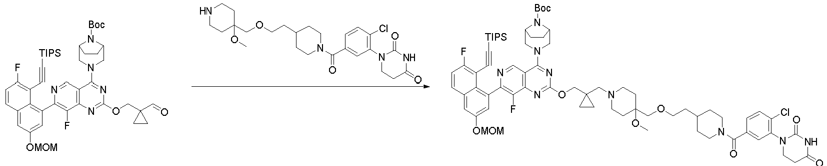

1-(2-Chloro-5-(4-(2-((4-methoxypiperidin-4-yl)methoxy)ethyl)piperidine-1-carbonyl)phenyl)dihydropyrimidine-2,4(1*H*,3*H*)-dione (60 mg, 0.12 mmol) and tetraisopropyl titanate (506 mg, 1.8 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then the reaction system was cooled to 0 °C, and NaBH(OAc)₃ (76 mg, 0.36 mmol) was added. The mixture was stirred at 25 °C for 1 h. After the reaction was completed, H₂O (5 mL) was added to the reaction liquid, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert*-butyl 3-(2-((1-((4-((2-(1-(4-chloro-3-(2,4-dioxotetrahydropyrimidin-1(2*H*)-yl)benzoyl)piperidin-4-yl)ethoxy)methyl)-4-methoxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 666.9.

### Step 8: preparation of 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-4-methoxypiperidin-4-yl)methoxy)ethyl)piperidine-1-carbonyl)-2-chlorophenyl)dihydropyrimidine-2,4(1H,3H)-dione

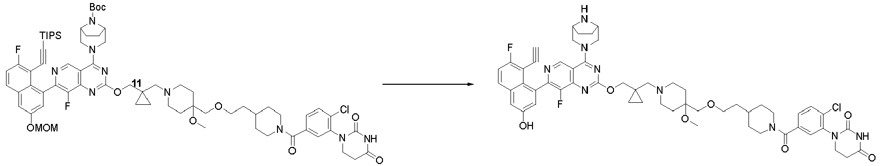

*tert*-Butyl 3-(2-((1-((4-((2-(1-(4-chloro-3-(2,4-dioxotetrahydropyrimidin-1(2*H*)-yl)benzoyl)piperidin-4-yl)ethoxy)methyl)-4-methoxypiperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (120 mg, 0.09 mmol) was dissolved in DMF (1 mL), and CsF (58 mg, 0.45 mmol) was then added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(4-(2-((1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-4-methoxypiperidin-4-yl)methoxy)ethyl)piperidine-1-carbonyl)-2-chlorophenyl)dihydropyrimidine-2,4(1*H*,3*H*)-dione.

LC-MS: (ESI, m/z): [M/2+H]⁺ = 516.7.

¹H NMR (400 MHz, CD₃OD) δ 8.99 (s, 1H), 7.85 (dd, *J* = 9.1, 5.7 Hz, 1H), 7.62 (d, *J =* 8.2 Hz, 1H), 7.53 - 7.48 (m, 1H), 7.41 - 7.28 (m, 3H), 7.20 (d, *J* = 2.3 Hz, 1H), 4.66 - 4.33 (m, 6H), 3.80 - 3.63 (m, 7H), 3.45 (t, *J* = 6.0 Hz, 2H), 3.36 (s, 1H), 3.20 (s, 3H), 2.89 - 2.72 (m, 5H), 2.56 - 2.42 (m, 2H), 2.35 - 2.25 (m, 2H), 1.89 - 1.69 (m, 8H), 1.62 - 1.47 (m, 5H), 1.35 - 1.27 (m, 2H), 1.23 - 1.05 (m, 2H), 0.76 - 0.68 (m, 2H), 0.56 - 0.48 (m, 2H).

### Example 67: 1-(5-(4-((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)ethoxy)methyl)-4-methoxypiperidine-1-carbonyl)-2-chlorophenyl)dihydropyrimidine-2,4(1H,3H)-dione

### Step 1: preparation of 2-(1-(4-methoxybenzyl)piperidin-4-yl)ethan-1-ol

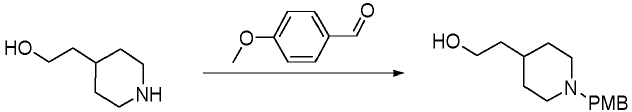

Acetic acid (0.5 mL) was added to a mixed solution of 2-(piperidin-4-yl)ethan-1-ol (1.5 g, 11.6 mmol) and 4-methoxybenzaldehyde (1.6 g, 11.6 mmol) in 1,2-dichloroethane (28 mL) and methanol (14 mL). The mixture was stirred at 30 °C for 1 h. NaBH₃CN (1.8 g, 29.0 mmol) was added at 0 °C, and the mixture was reacted at room temperature for 16 h. After the reaction was completed, H₂O (30 mL) was added, and the mixture was extracted with ethyl acetate (30 mL × 3). The combined organic phase was dried over anhydrous sodium sulfate, filtered, and concentrated in vacuum. The crude product was purified by column chromatography (DCM:MeOH = 10:1) to give 2-(1-(4-methoxybenzyl)piperidin-4-yl)ethan-1-ol.

LC-MS: (ESI, m/z): [M+H]⁺ = 250.2.

### Step 2: preparation of 2-(1-(4-methoxybenzyl)piperidin-4-yl)ethyl 4-methylbenzenesulfonate

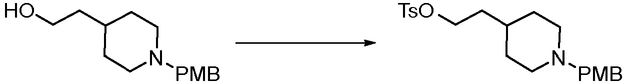

2-(1-(4-Methoxybenzyl)piperidin-4-yl)ethan-1-ol (1.9 g, 7.6 mmol) was dissolved in chloroform (60 mL), and pyridine (1.2 g, 15.2 mmol) and TsCl (2.2 g, 11.5 mmol) were added. The mixture was stirred at the temperature of an ice-water bath to room temperature for 12 h. After the reaction was completed, the mixture was concentrated and purified by silica gel column chromatography (DCM:MeOH = 20:1) to give 2-(1-(4-methoxybenzyl)piperidin-4-yl)ethyl 4-methylbenzenesulfonate.

LC-MS: (ESI, m/z): [M+H]⁺ = 404.1.

### Step 3: preparation of tert-butyl 4-methoxy-4-((2-(1-(4-methoxybenzyl)piperidin-4-yl)ethoxy)methyl)piperidine-1-carboxylate

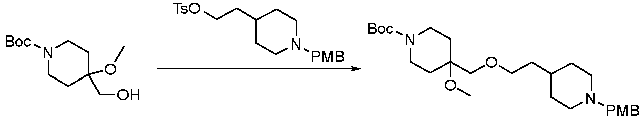

NaH (35 mg, 0.73 mmol, 60%) was added to a solution of *tert*-butyl 4-(hydroxymethyl)-4-methoxypiperidine-1-carboxylate (0.15 g, 0.61 mmol) in THF (5 mL), and the mixture was reacted at the temperature of an ice-water bath to room temperature for 1 h. Then 2-(1-(4-methoxybenzyl)piperidin-4-yl)ethyl 4-methylbenzenesulfonate (300 mg, 0.67 mmol) was added, and the reaction liquid was stirred at 60 °C overnight. After the reaction was completed, the reaction liquid was directly concentrated, and the resulting crude product was purified by silica gel column chromatography (DCM:MeOH = 20:1) to give *tert*-butyl 4-methoxy-4-((2-(1-(4-methoxybenzyl)piperidin-4-yl)ethoxy)methyl)piperidine-1-carboxylate.

LC-MS: (ESI, m/z): [M+H]⁺ = 477.3.

### Step 4: preparation of 1-(4-methoxybenzyl)-4-(2-((4-methoxypiperidin-4-yl)methoxy)ethyl)piperidine

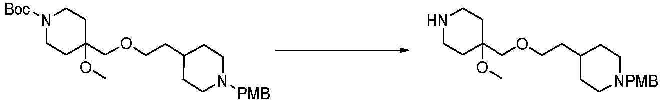

TFA (2 mL) was added to a solution of *tert*-butyl 4-methoxy-4-((2-(1-(4-methoxybenzyl)piperidin-4-yl)ethoxy)methyl)piperidine-1-carboxylate (160 mg, 0.33 mmol) in DCM (4 mL). The mixture was stirred at room temperature for 1 h, and the reaction liquid was directly concentrated to give 1-(4-methoxybenzyl)-4-(2-((4-methoxypiperidin-4-yl)methoxy)ethyl)piperidine, which was directly used in the next step without further purification.

### Step 5: preparation of 1-(2-chloro-5-(4-methoxy-4-((2-(1-(4-methoxybenzyl)piperidin-4-yl)ethoxy)methyl)piperidine-1-carbonyl)phenyl)dihydropyrimidine-2,4(1H,3H)-dione

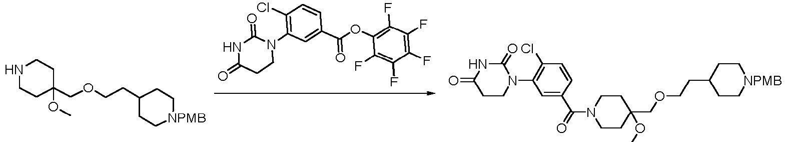

Pentafluorophenyl 4-chloro-3-(2,4-dioxotetrahydropyrimidin-1(2*H*)-yl)benzoate (160 mg, 0.37 mmol) and DIEA (130 mg, 0.96 mmol) were added to a solution of 1-(4-methoxybenzyl)-4-(2-((4-methoxypiperidin-4-yl)methoxy)ethyl)piperidine (150 mg, crude) in DMSO (3 mL), and the mixture was reacted at room temperature for 2 h. Water (10 mL) was added to the reaction liquid, and the mixture was extracted with ethyl acetate (10 mL × 2). The organic phase was washed with saturated brine, dried over anhydrous sodium sulfate, and filtered, and the filtrate was concentrated. The resulting crude product was purified by silica gel column chromatography (DCM:MeOH = 100:1 to 20:1) to give 1-(2-chloro-5-(4-methoxy-4-((2-(1-(4-methoxybenzyl)piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 627.2.

### Step 6: preparation of 1-(2-chloro-5-(4-methoxy-4-((2-(piperidin-4-yl)ethoxy)methyl)piperidine-1-carbonyl)phenyl)dihydropyrimidine-2,4(1H,3H)-dione

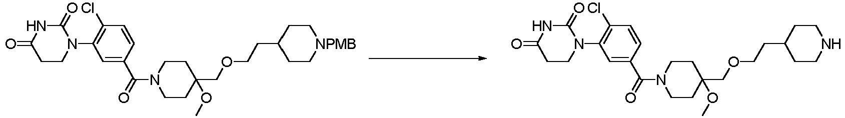

1-(2-Chloro-5-(4-methoxy-4-((2-(1-(4-methoxybenzyl)piperidin-4-yl)ethoxy)methyl)piperidine-1-carbonyl)phenyl)dihydropyrimidine-2,4(1*H*,3*H*)-dione (200 mg, 0.32 mmol) was dissolved in DCM (5 mL), and 1-chloroethyl chloroformate (92 mg, 0.64 mmol) and DIEA (82 mg, 0.64 mmol) were added. The mixture was reacted at room temperature for 30 min and concentrated under reduced pressure. The concentrate was dissolved in MeOH (5 mL), and the mixture was then reacted at 50 °C for 30 min. After the reaction was completed, the mixture was concentrated and purified by silica gel column chromatography (DCM:MeOH = 10:1) to give 1-(2-chloro-5-(4-methoxy-4-((2-(piperidin-4-yl)ethoxy)methyl)piperidine-1-carbonyl)phenyl)dihydropyrimidine-2,4(1*H*,3*H*)-dione.

LC-MS: (ESI, m/z): [M+H]⁺ = 507.1.

### Step 7: preparation of tert-butyl 3-(2-((1-((4-(2-((1-(4-chloro-3-(2,4-dioxotetrahydropyrimidin-1(2H)-yl)benzoyl)-4-methoxypiperidin-4-yl)methoxy)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

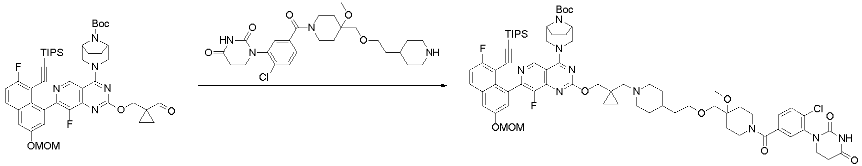

1-(2-Chloro-5-(4-methoxy-4-((2-(piperidin-4-yl)ethoxy)methyl)piperidine-1-carbonyl)phenyl)dihydropyrimidine-2,4(1*H*,3*H*)-dione (60 mg, 0.12 mmol) and tetraisopropyl titanate (506 mg, 1.8 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. Then NaBH(OAc)₃ (76 mg, 0.36 mmol) was added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert*-butyl 3-(2-((1-((4-(2-((1-(4-chloro-3-(2,4-dioxotetrahydropyrimidin-1(2*H*)-yl)benzoyl)-4-methoxypiperidin-4-yl)methoxy)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 666.9.

### Step 8: preparation of 1-(5-(4-((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)ethoxy)methyl)-4-methoxypiperidine-1-carbonyl)-2-chlorophenyl)dihydropyrimidine-2,4(1H,3H)-dione

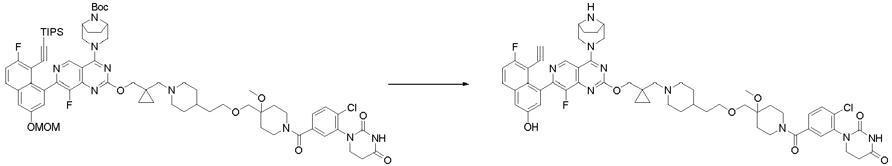

*tert*-Butyl 3-(2-((1-((4-(2-((1-(4-chloro-3-(2,4-dioxotetrahydropyrimidin-1(2*H*)-yl)benzoyl)-4-methoxypiperidin-4-yl)methoxy)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (100 mg, 0.075 mmol) was dissolved in DMF (1 mL), and CsF (57 mg, 0.375 mmol) was then added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(4-((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl) cyclopropyl)methyl)piperidin-4-yl)ethoxy)methyl)-4-methoxypiperidine-1-carbonyl)-2-chlorophenyl) dihydropyrimidine-2,4(1H,3H)-dione.

LC-MS: (ESI, m/z): [M+H]⁺ = 1032.1.

¹H NMR (400 MHz, CD₃OD) δ 8.99 (s, 1H), 7.85 (dd, *J =* 9.1, 5.7 Hz, 1H), 7.62 (d, *J =* 8.2 Hz, 1H), 7.53 (d, *J =* 1.8 Hz, 1H), 7.44 - 7.39 (m, 1H), 7.35 - 7.28 (m, 2H), 7.20 *(*d, *J =* 2.4 Hz, 1H), 4.66 - 4.26 (m, 6H), 3.80 - 3.64 (m, 6H), 3.54 - 3.44 (m, 3H), 3.38 - 3.36 (m, 2H), 3.34 (s, 1H), 3.25 (s, 3H), 3.16 - 3.05 (m, 3H), 2.89 - 2.80 (m, 2H), 2.54 - 2.41 (m, 2H), 2.05 - 1.95 (m, 2H), 1.89 - 1.66 (m, 8H), 1.53 - 1.40 (m, 4H), 1.34 - 1.22 (m, 3H), 0.77 - 0.69 (m, 2H), 0.55 - 0.48 (m, 2H).

Example 68: 1-(5-(9-(3-(4-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)piperidin-1-yl)propyl)-3-azaspiro[5.5]undecane-3-carbonyl)-2-chlorophenyl)dihydropyrimidine-2,4(1*H*,3*H*)-dione

### Step 1: preparation of tert-butyl 3-(2-((1-(3-(3-(4-chloro-3-(2,4-dioxotetrahydropyrimidin-1(2H)-yl)benzoyl)-3-azaspiro[5.5]undecan-9-yl)propyl)piperidin-4-yl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

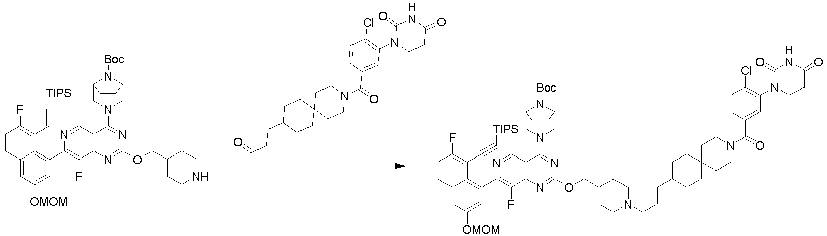

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-(piperidin-4-ylmethoxy)pyrido[4,3-*d*]pyrimidin-4-yl)-3,8-diazabicyclo[3.2.1]octane-8-carboxylate (100 mg, 0.120 mmol) and 3-(3-(4-chloro-3-(2,4-dioxotetrahydropyrimidin-1(2*H*)-yl)benzoyl)-3-azaspiro[5.5]undecan-9-yl)propanal (81 mg, 0.18 mmol) in DCM/AcOH (1.5 mL/0.1 mL), and the mixture was stirred at 30 °C for 2 h. NaBH(OAc)₃ (128 mg, 0.60 mmol) was added to the mixture in an ice bath, and the resulting mixture was stirred for 2 h. The reaction liquid was concentrated and purified by silica gel column chromatography (MeOH:DCM = (0-1):4) to give *tert*-butyl 3-(2-((1-(3-(3-(4-chloro-3-(2,4-dioxotetrahydropyrimidin-1(2*H*)-yl)benzoyl)-3-azaspiro[5.5] undecan-9-yl)propyl)piperidin-4-yl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 650.9.

### Step 2: preparation of 1-(5-(9-(3-(4-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)piperidin-1-yl)propyl)-3-azaspiro[5.5]undecane-3-carbonyl)-2-chlorophenyl)dihydropyrimidine-2,4(1H,3H)-dione

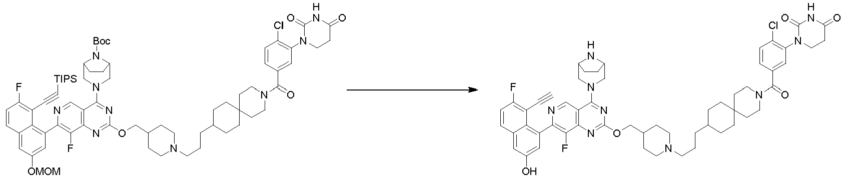

Cesium fluoride (42 mg, 0.28 mmol) was added to a solution of *tert*-butyl 3-(2-((1-(3-(3-(4-chloro-3-(2,4-dioxotetrahydropyrimidin-1(2*H*)-yl)benzoyl)-3-azaspiro[5.5]undecan-9-yl)propyl)piperidin-4-yl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (72 mg, 0.06 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. 1,4-Dioxane (0.5 mL) was added to the concentrate, and HCl/1,4-dioxane (6 N, 1.0 mL) was then added while stirring. The mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-(3-(4-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)piperidin-1-yl)propyl)-3-azaspiro[5.5]undecane-3-carbonyl)-2-chlorophenyl)dihydropyrimidine-2,4(1H,3H)-dione.

LC-MS: (ESI, m/z): [M/2+H]⁺ = 500.7.

¹H NMR (400 MHz, MeOD) δ 9.00 (s, 1H), 7.90 - 7.82 (m, 1H), 7.64 (d, *J* = 8.3 Hz, 1H), 7.53 (s, 1H), 7.42 (d, *J* = 8.3 Hz, 1H), 7.38 - 7.28 (m, 2H), 7.23 - 7.17 (m, 1H), 4.68 - 4.52 (m, 2H), 4.40 - 4.27 (m, 2H), 3.84 - 3.63 (m, 8H), 3.50 - 3.35 (m, 3H), 3.15 - 3.06 (m, 2H), 2.91 - 2.78 (m, 2H), 2.57 - 2.33 (m, 2H), 2.25 - 2.11 (m, 2H), 1.96 - 1.75 (m, 8H), 1.63 - 1.42 (m, 9H), 1.37 - 1.12 (m, 9H).

### Example 69: 1-(5-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazine-1-carbonyl)-2-chlorophenyl)dihydropyrimidine-2,4(1H,3H)-dione

### Step 1: preparation of tert-butyl 3-(2-((1-((4-(4-chloro-3-(2,4-dioxotetrahydropyrimidin-1(2H)-yl)benzoyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

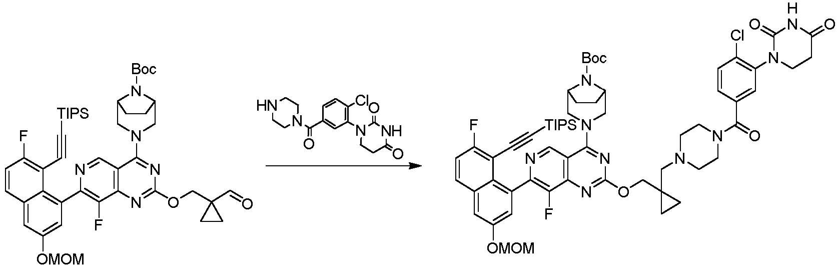

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido,3-*d*]pyrimidin-4-yl)-3,8-diazabicyclo[3.2.1]octane-8-carboxylate (150 mg, 0.18 mmol) and 1-(2-chloro-5-(piperazine-1-carbonyl)phenyl)dihydropyrimidine-2,4(1*H*,3*H*)-dione (90 mg, 0.27 mmol) in DCM/AcOH (1.5 mL/0.1 mL), and the mixture was stirred at 30 °C for 2 h. NaBH(OAc)₃ (113 mg, 0.53 mmol) was added to the mixture in an ice bath, and the resulting mixture was stirred for 2 h. The mixture was concentrated and purified by silica gel column chromatography (MeOH:DCM = (0-1):4) to give *tert*-butyl 3-(2-((1-((4-(4-chloro-3-(2,4-dioxotetrahydropyrimidin-1(2*H*)-yl)benzoyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 582.4.

### Step 2: preparation of 1-(5-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazine-1-carbonyl)-2-chlorophenyl)dihydropyrimidine-2,4(1H,3H)-dione

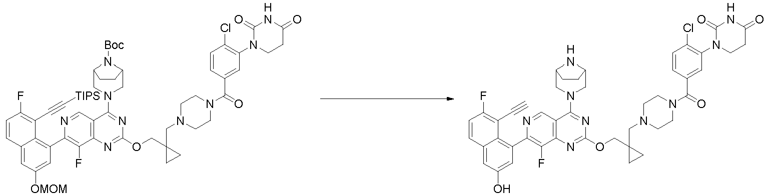

Cesium fluoride (108 mg, 0.71 mmol) was added to a solution of *tert*-butyl 3-(2-((1-((4-(4-chloro-3-(2,4-dioxotetrahydropyrimidin-1(2*H*)-yl)benzoyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (166 mg, 0.14 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. 1,4-Dioxane (0.5 mL) was added to the concentrate, and HCl/dioxane (6 N, 1.0 mL) was then added while stirring. The mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazine-1-carbonyl)-2-chlorophenyl)dihydropyrimidine-2,4(1*H*,3*H*)-dione.

LC-MS: (ESI, m/z): [M+H]⁺ = 862.1.
¹H NMR (400 MHz, DMSO-*d*₆) δ 10.50 (s, 1H), 10.14 (s, 1H), 9.03 (s, 1H), 8.01 - 7.94 (m, 1H), 7.63 (d, *J* = 8.2 Hz, 1H), 7.56 - 7.53 (m, 1H), 7.46 (t, *J =* 9.0 Hz, 1H), 7.40 - 7.35 (m, 2H), 7.18 - 7.15 (m, 1H), 4.47 (d, *J* = 11.6 Hz, 1H), 4.31 - 4.24 (m, 3H), 3.93 (s, 1H), 3.79 - 3.70 (m, 1H), 3.68 - 3.41 (m, 8H), 2.77 - 2.68 (m, 2H), 2.49 - 2.26 (m, 8H), 1.68 - 1.62 (m, 4H), 0.67 - 0.63 (m, 2H), 0.45 - 0.39 (m, 2H).

### Example 70: 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate

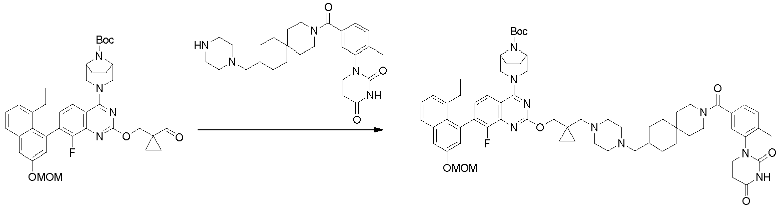

1-(2-Methyl-5-(9-(piperazin-1-ylmethyl)-3-azaspiro[5.5]undecane-3-carbonyl)phenyl)dihydropyrimidine-2,4(1*H*,3*H*)-dione (144 mg, 30 mmol) and tetraisopropyl titanate (213 mg, 0.75 mmol) were added to a solution of *tert*-butyl 3-(7-(8-ethyl-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (100 mg, 0.15 mmol) in dichloromethane (1 mL) and acetic acid (0.1 mL), and the mixture was stirred at 25 °C for 2 h. Then NaBH(OAc)₃ (95 mg, 0.45 mmol) was added at 0 °C, and the mixture was stirred at 25 °C for 1 h. After the reaction was completed, H₂O (5 mL) was added, and the mixture was extracted with ethyl acetate (10 mL × 3). The combined organic phase was dried over anhydrous sodium sulfate, filtered, and concentrated in vacuum. The crude product was purified by silica gel column chromatography (DCM:MeOH = 100:1 to 20:1) to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M+H]⁺ = 1136.8.

### Step 2: preparation of 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

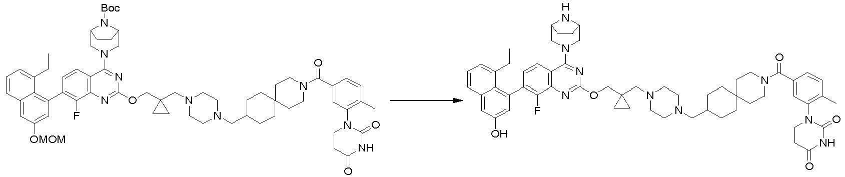

*tert-Butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate (90 mg, 0.076 mmol) was dissolved in 1,4-dioxane (1 mL), and HCl/1,4-dioxane (6 M, 0.5 mL) was added. The mixture was stirred at room temperature for 0.5 h. After the reaction was completed, H₂O (5 mL) was added, and the mixture was adjusted to pH > 7 with an aqueous Na₂CO₃ solution and extracted with ethyl acetate (10 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 496.7.

¹H NMR (400 MHz, DMSO-*d*₆) δ 10.36 (s, 1H), 9.85 (s, 1H), 7.78 (d, *J =* 8.5 Hz, 1H), 7.65 (d, *J* = 8.1 Hz, 1H), 7.38 - 7.29 (m, 3H), 7.25 - 7.17 (m, 3H), 7.14 - 7.08 (m, 1H), 6.87 - 6.90 (m, 1H), 4.19 - 4.29 (m, 4H), 3.86 - 3.74 (m, 1H), 3.61 - 3.39 (m, 8H), 2.80 - 2.64 (m, 3H), 2.41 - 2.24 (m, 10H), 2.36 - 2.16 (m, 4H), 2.13 - 1.94 (m, 2H), 1.71 - 1.62 (m, 6H), 1.57 - 1.36 (m, 6H), 1.34 - 1.18 (m, 3H), 1.13 - 0.94 (m, 4H), 0.80 (t, *J* = 7.4 Hz, 3H), 0.67 - 0.56 (m, 2H), 0.47 - 0.28 (m, 2H).

### Example 71: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

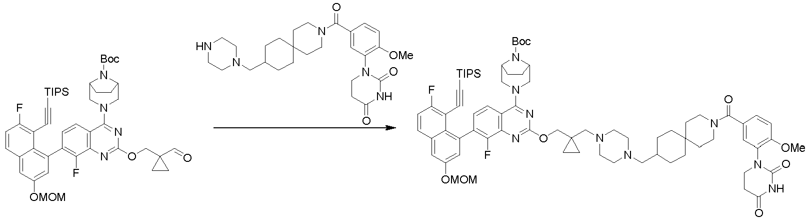

Tetraisopropyl titanate (170 mg, 0.60 mmol) was added to a solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate (100 mg, 0.12 mmol) and 1-(2-methoxy-5-(9-(piperazin-1-ylmethyl)-3-azaspiro[5.5]undecane-3-carbonyl)phenyl) dihydropyrimidine-2,4(1*H*,3*H*)-dione (70 mg, 0.14 mmol) in DCM/AcOH (1.0 mL/0.1 mL), and the mixed solution was stirred at 30 °C for 2 h. NaBH(OAc)₃ (76 mg, 0.36 mmol) was then added at room temperature, and the mixture was stirred for 2 h. The mixture was concentrated and purified by silica gel column chromatography (MeOH:DCM = (0-1):4) to give *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1322.7.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

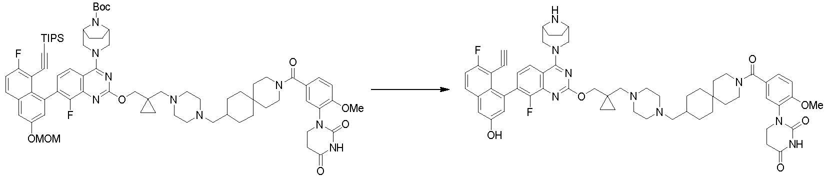

CsF (66 mg, 0.435 mmol) was added to a stirred solution of *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)quinazolin-4-yl)-3,8-diazabicyclo[3.2.1]octane-8-carboxylate (115 mg, 0.087 mmol) in DMF (2 mL), and the mixture was stirred at room temperature for 1 h. After the reaction was completed, the mixture was diluted with H₂O (5 mL) and extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1022.2.

¹H NMR (400 MHz, DMSO-*d*₆) δ 10.34 (s, 1H), 10.14 (s, 1H), 7.97 (dd, *J*₁ = 9.2 Hz, *J*₂ = 2.0 Hz, 1H), 7.73 (d, *J* = 8.6 Hz, 1H), 7.50 - 7.44 (m, 1H),7.40 - 7.35 (m, 2H), 7.34 - 7.31 (m, 1H), 7.20 - 7.14 (m, 2H), 7.07 (d, *J =* 2.0 Hz, 1H), 4.38 - 4.27 (m, 1H), 4.26 - 4.11 (m, 3H), 3.90 - 3.81 (m, 4H), 3.67 - 3.58 (m, 2H), 3.55 - 3.34 (m, 8H), 2.74 - 2.66 (m, 2H), 2.56 - 2.62 (m, 1H), 2.49 - 2.16 (m, 10H), 2.11 - 2.02 (m, 2H), 1.76 - 1.64 (m, 5H), 1.58 - 1.40 (m, 5H), 1.39 - 1.19 (m, 3H), 1.15 - 0.93 (m, 4H), 0.68 - 0.59 (m, 2H), 0.43 - 0.36 (m, 2H).

### Example 72: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

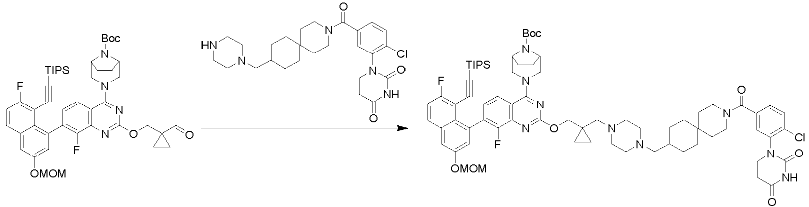

1-(2-Chloro-5-(9-(piperazin-1-ylmethyl)-3-azaspiro[5.5]undecane-3-carbonyl)phenyl)dihydropyrimidine-2,4(1*H*,3*H*)-dione (136 mg, 0.27 mmol) and AcOH (0.1 mL) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl) methoxy)quinazolin-4-yl)-3,8-diazabicyclo[3.2.1]octane-8-carboxylate (150 mg, 0.18 mmol) in MeOH (1.0 mL), and the mixed solution was stirred at 25 °C for 2 h. NaBH₃CN (34 mg, 0.54 mmol) was then added at 0 °C, and the mixture was stirred for 1 h. The mixture was concentrated and subjected to silica gel column chromatography (DCM:MeOH = 100:1 to 20:1) to give *tert-butyl* 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl) cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M/2+H]⁺ = 664.0.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

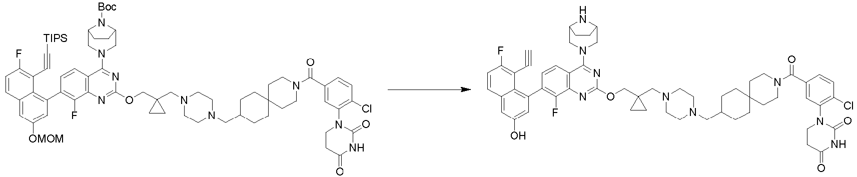

CsF (57 mg, 0.375 mmol) was added to a stirred solution of *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (100 mg, 0.075 mmol) in DMF (1 mL), and the mixture was stirred at room temperature for 1 h. After the reaction was completed, the mixture was diluted with H₂O (5 mL) and extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M/2+H]⁺ = 513.8.

¹H NMR (400 MHz, DMSO-*d*₆) δ 10.50 (s, 1H), 10.11 (s, 1H), 7.96 (dd, *J =* 9.2 Hz, 2.0 Hz, 1H), 7.72 (d, *J =* 8.7 Hz, 1H), 7.63 (d, *J =* 8.2 Hz, 1H), 7.55 - 7.53 (m, 1H), 7.48 - 7.42 (m, 1H), 7.39 - 7.34 (m, 2H), 7.18 - 7.13 (m, 1H), 7.05 (d, *J =* 2.0 Hz, 1H), 4.32 - 4.16 (m, 4H), 3.83 (s, 1H), 3.78 - 3.70 (m, 1H), 3.66 - 3.38 (m, 8H), 2.81 - 2.64 (m, 3H), 2.42 - 2.22 (m, 10H), 2.11 - 1.97 (m, 3H), 1.75 - 1.63 (m, 6H), 1.55 - 1.30 (m, 7H), 1.13 - 0.95 (m, 4H), 0.64 - 0.59 (m, 2H), 0.40 -0.35 (m, 2H).

### Example 73: 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1] octane-8-carboxylate

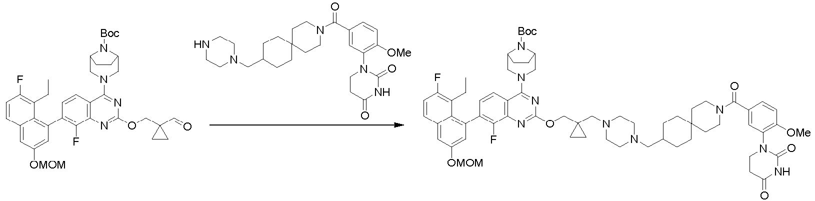

Tetraisopropyl titanate (270 mg, 0.95 mmol) was added to a solution of *tert*-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (130 mg, 0.19 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (114 mg, 0.23 mmol) in DCM/AcOH (1.5 mL/0.15 mL), and the mixed solution was stirred at 30 °C for 2 h. NaBH(OAc)₃ (121 mg, 0.57 mmol) was then added at room temperature, and the mixture was stirred for 1 h. The mixture was concentrated and purified by silica gel column chromatography (DCM:MeOH = 100:1 to 5:1) to give *tert*-butyl 3-(2-((1-((4-((3-(3-(2,4-dioxotetrahydropyrimidin-1(2*H*)-yl)-4-methoxybenzoyl)-3-azaspiro [5.5] undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M+H]⁺ = 1170.8.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

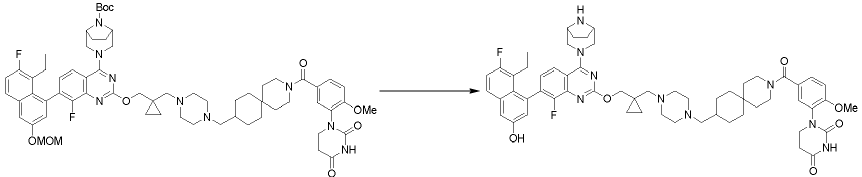

1,4-Dioxane (1 mL) and hydrochloric acid/1,4-dioxane (8 M, 1 mL) were added to *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate (160 mg, 0.14 mmol), and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 513.7.

¹H NMR (400 MHz, DMSO-*d*₆) δ 10.33 (s, 1H), 9.91 (s, 1H), 7.82 - 7.72 (m, 2H), 7.38 - 7.33 (m, 2H), 7.32 - 7.30 (m, 1H), 7.29 - 7.27 (m, 1H), 7.26 - 7.21 (m, 1H), 7.14 (d, *J* = 8.6 Hz, 1H), 6.92 (d,*J* = 2.0 Hz, 1H), 4.31 - 4.19 (m, 4H), 3.84 (s, 3H), 3.65 - 3.53 (m, 3H), 3.52 - 3.37 (m, 7H), 2.72 - 2.64 (m, 2H), 2.49 - 2.08 (m, 13H), 2.06 - 1.99 (m, 2H), 1.72 - 1.62 (m, 6H), 1.53 - 1.37 (m, 5H), 1.32 - 1.23 (m, 2H), 1.11 - 0.94 (m, 4H), 0.71 (t, *J =* 7.3 Hz, 3H), 0.64 - 0.59 (m, 2H), 0.40 -0.35 (m, 2H).

### Example 74: 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate

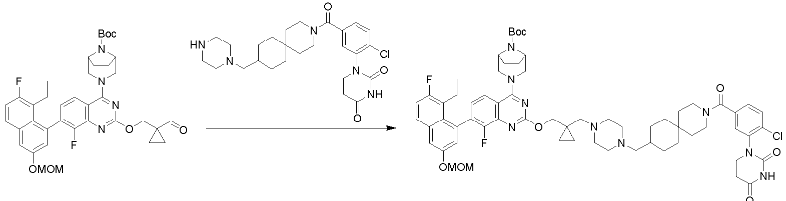

Tetraisopropyl titanate (270 mg, 0.95 mmol) was added to a solution of *tert*-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (130 mg, 0.19 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (115 mg, 0.23 mmol) in DCM/AcOH (1.5 mL/0.15 mL), and the mixed solution was stirred at 30 °C for 2 h. NaBH(OAc)₃ (121 mg, 0.57 mmol) was then added at room temperature, and the mixture was stirred for 1 h. The mixture was concentrated and purified by silica gel column chromatography (DCM:MeOH = 100:1 to 5:1) to give *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M/2+H]⁺ = 587.5.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

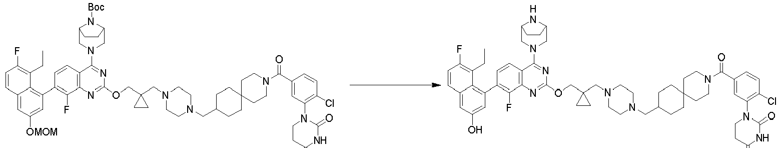

1,4-Dioxane (1 mL) and hydrochloric acid/1,4-dioxane (8 M, 1 mL) were added to *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate (170 mg, 0.14 mmol), and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1030.2.

¹H NMR (400 MHz, DMSO-*d*₆) δ 10.50 (s, 1H), 9.90 (s, 1H), 7.82 - 7.72 (m, 2H), 7.63 (d, *J =* 8.2 Hz, 1H), 7.54 (d, *J =* 2.0 Hz, 1H), 7.40 - 7.28 (m, 3H), 7.26 - 7.21 (m, 1H), 6.94 - 6.92 (d, *J* = 2.4 Hz, 1H), 4.30 - 4.19 (m, 4H), 3.80 - 3.71 (m, 1H), 3.67 - 3.38 (m, 8H), 2.81 - 2.62 (m, 3H), 2.45 - 2.15 (m, 12H), 2.17 - 1.93 (m, 3H), 1.72 - 1.61 (m, 6H), 1.54 - 1.38 (m, 5H), 1.34 - 1.22 (m, 2H), 1.14 - 0.93 (m, 4H), 0.74 - 0.68 (m, 3H), 0.64 - 0.58 (m, 2H), 0.40 - 0.34 (m, 2H).

### Example 75: 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1] octane-8-carboxylate

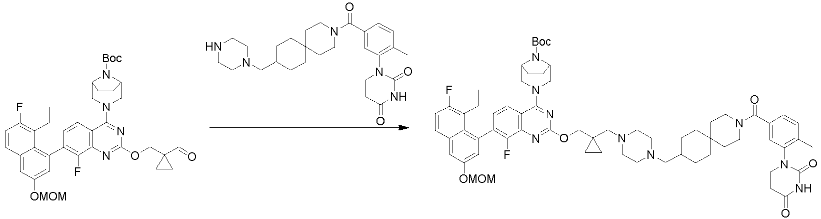

Tetraisopropyl titanate (270 mg, 0.95 mmol) was added to a solution of *tert*-butyl 3-(7-(8-ethyl-7-fluoro-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (130 mg, 0.19 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (111 mg, 0.23 mmol) in DCM/AcOH (1.5 mL/0.15 mL), and the mixed solution was stirred at 30 °C for 2 h. NaBH(OAc)₃ (121 mg, 0.57 mmol) was then added at room temperature, and the mixture was stirred for 1 h. The mixture was concentrated and purified by silica gel column chromatography (MeOH:DCM = (0-1):4) to give *tert-*butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M+H]⁺ = 1154.8.

### Step 2: preparation of 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

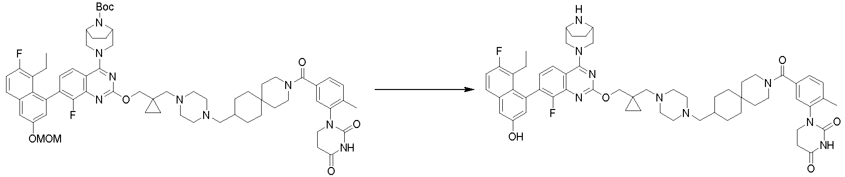

1,4-Dioxane (1 mL) and hydrochloric acid/1,4-dioxane (8 M, 1 mL) were added to *tert*-butyl 3-(2-((1-((4-((3-(3-(2,4-dioxotetrahydropyrimidin-1(2*H*)-yl)-4--methylbenzoyl)-3-azaspiro[5.5]undecan-9-yl)methyl)piperazin-1-yl)methyl)cyclopropyl)methoxy)-7-(8-ethyl-7-fluoro-3-(methoxymethoxy)naphthalen-1-yl)-8-fluoroquinazolin-4-yl)-3,8-diazabicyclo[3.2.1]octane-8-carboxylate (155 mg, 0.13 mmol), and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethyl-7-fluoro-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione.

LC-MS: (ESI, m/z): [M/2+H]⁺ = 505.8.

¹H NMR (400 MHz, DMSO-*d*₆) δ 10.36 (s, 1H), 9.90 (s, 1H), 7.83 - 7.69 (m, 2H), 7.37 - 7.28 (m, 4H), 7.26 - 7.20 (m, 2H), 6.92 (d, *J* = 2.4 Hz, 1H), 4.33 - 4.17 (m, 4H), 3.85 - 3.74 (m, 1H), 3.64 - 3.35 (m, 8H), 2.85 - 2.59 (m, 3H), 2.46 - 2.15 (m, 15H), 2.13 - 1.92 (m, 3H), 1.73 - 1.61 (m, 6H), 1.53 - 1.37 (m, 5H), 1.33 - 1.22 (m, 2H), 1.12 - 0.93 (m, 4H), 0.75 - 0.67 (m, 3H), 0.64 - 0.58 (m, 2H), 0.40 - 0.34 (m, 2H).

### Example 76: 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(8-fluoro-2-((1-(hydroxymethyl)cyclopropyl)methoxy)-7-(3-(methoxymethoxy)-8-((triisopropylsilyl)ethynyl)naphthalen-1-yl)quinazolin-4-yl)-3,8-diazabicyclo[3.2.1] octane-8-carboxylate

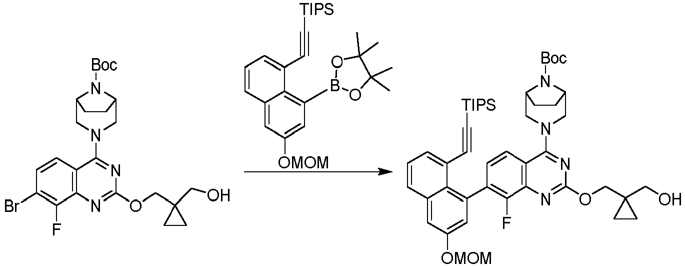

PdCl₂(dtbpf) (291 mg, 0.446 mmol) and cesium carbonate (2.19 g, 6.69 mmol) were added to a mixed solution of *tert*-butyl 3-(7-bromo-8-fluoro-2-((1-(hydroxymethyl)cyclopropyl)methoxy)quinazolin-4-yl)-3,8-diazabicyclo[3.2.1]octane-8-carboxylate (1.2 g, 2.23 mmol) and triisopropyl((6-(methoxymethoxy)-8-(4,4,5,5-tetramethyl-1,3,2-dioxaborolan-2-yl)naphthalen-1-yl)ethynyl)silane (1.3 g, 2.68 mmol) in 1,4-dioxane (12 mL) and H₂O (2.4 mL). The mixture was reacted at 85 °C for 16 h under nitrogen atmosphere. Water (20 mL) was added to quench the reaction, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 30:1) to give *tert-butyl* 3-(8-fluoro-2-((1-(hydroxymethyl)cyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 825.3.

### Step 2: preparation of tert-butyl 3-(8-fluoro-2-((1-formylcyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate

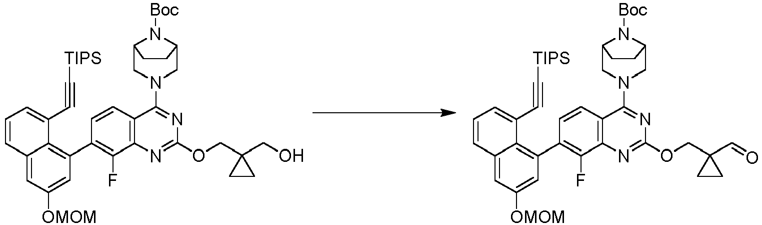

A solution of *tert*-butyl 3-(8-fluoro-2-((1-(hydroxymethyl)cyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate (1.5 g, 1.82 mmol) in DCM (10 mL) was cooled to 0 °C, and Dess-Martin oxidant (2.32 g, 5.46 mmol) was then added. The mixture was reacted at room temperature for 2 h. After the reaction was completed, a saturated sodium thiosulfate solution (10 mL) was added to quench the reaction, and the mixture was extracted with dichloromethane (30 mL × 3). The organic phases were combined, dried over anhydrous sodium sulfate, and concentrated under reduced pressure. The concentrate was purified by silica gel column chromatography (DCM:EtOAc = 10:1) to give *tert*-butyl 3-(8-fluoro-2-((1-formylcyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 823.4.

### Step 3: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo [3.2.1]octane-8-carboxylate

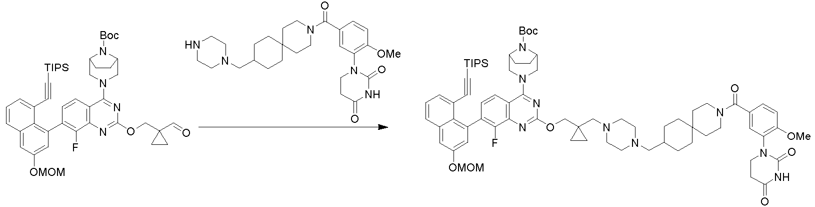

Tetraisopropyl titanate (256 mg, 0.90 mmol) was added to a solution of *tert-butyl* 3-(8-fluoro-2-((1-formylcyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate (150 mg, 0.18 mmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (109 mg, 0.22 mmol) in DCM/AcOH (1.5 mL/0.15 mL), and the mixed solution was stirred at 30 °C for 2 h. NaBH(OAc)₃ (114 mg, 0.54 mmol) was then added at room temperature, and the mixture was stirred for 1 h. The mixture was concentrated and purified by silica gel column chromatography (DCM:MeOH = 100:1 to 10:1) to give *tert*-butyl 3-(2-((1-((4-((3-(3-(2,4-dioxotetrahydropyrimidin-1(2*H*)-yl)-4-methoxybenzoyl)-3-azaspiro[5.5] undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1304.8.

### Step 4: preparation of 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

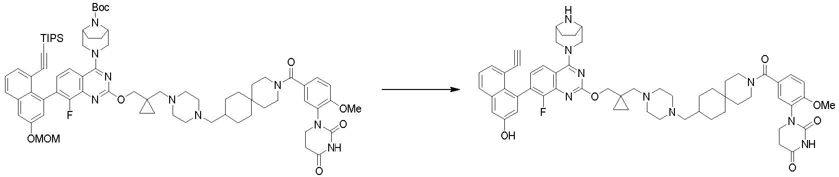

Cesium fluoride (106 mg, 0.70 mmol) was added to a stirred solution of *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (180 mg, 0.14 mmol) in DMF (2 mL), and the mixture was stirred at 30 °C for 1 h. The mixture was then diluted with H₂O (5 mL), and the resulting mixture was extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 502.7.

¹H NMR (400 MHz, DMSO-*d*₆) δ 10.32 (s, 1H), 10.09 (s, 1H), 7.87 (d, *J =* 7.3 Hz, 1H), 7.71 (d, *J* = 8.7 Hz, 1H), 7.47 - 7.38 (m, 2H), 7.37 - 7.33 (m, 1H), 7.32 - 7.28 (m, 2H), 7.18 - 7.12 (m, 2H), 7.00 (d, *J* = 2.8 Hz, 1H), 4.31 (d, *J* = 11.4 Hz, 1H), 4.25 - 4.16 (m, 3H), 3.84 (s, 3H), 3.62 - 3.51 (m, 5H), 3.49 - 3.40 (m, 5H), 2.70 - 2.65 (m, 2H), 2.42 - 2.19 (m, 11H), 2.12 - 1.95 (m, 3H), 1.76 - 1.64 (m, 6H), 1.55 - 1.40 (m, 5H), 1.31 - 1.23 (m, 2H), 1.11 - 0.96 (m, 4H), 0.64 - 0.58 (m, 2H), 0.40 - 0.34 (m, 2H).

### Example 77: 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate

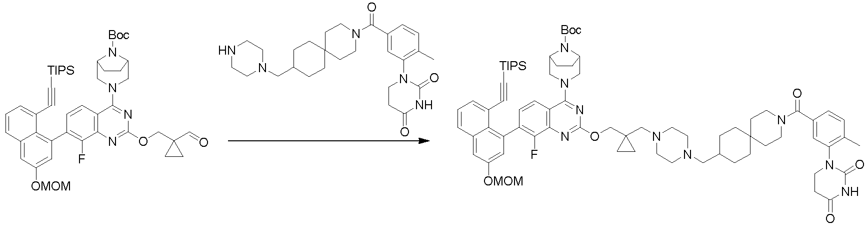

Tetraisopropyl titanate (256 mg, 0.90 mmol) was added to a solution of *tert*-butyl 3-(8-fluoro-2-((1-formylcyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate (150 mg, 0.18 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (106 mg, 0.22 mmol) in DCM/AcOH (1.5 mL/0.15 mL), and the mixed solution was stirred at 30 °C for 2 h. NaBH(OAc)₃ (114 mg, 0.54 mmol) was then added at room temperature, and the mixture was stirred for 1 h. The mixture was concentrated and purified by silica gel column chromatography (DCM:MeOH = 100:1 to 10:1) to give *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1288.8.

### Step 2: preparation of 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

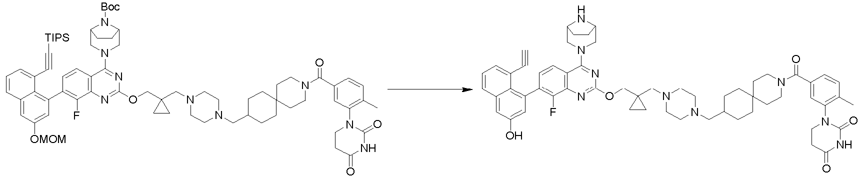

Cesium fluoride (106 mg, 0.70 mmol) was added to a stirred solution of *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate

(175 mg, 0.14 mmol) in DMF (2 mL), and the mixture was stirred at 30 °C for 1 h. The mixture was diluted with H₂O (5 mL), and the resulting mixture was extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione.

LC-MS: (ESI, m/z): [M/2+H]⁺ = 494.8.

¹H NMR (400 MHz, DMSO-*d*₆) δ 10.36 (s, 1H), 10.10 (s, 1H), 7.87 (d, *J* = 6.9 Hz, 1H), 7.71 (d, *J* = 8.6 Hz, 1H), 7.47 - 7.38 (m, 2H), 7.32 (dd, *J* = 12.1, 5.0 Hz, 3H), 7.24 (d, *J* = 7.6 Hz, 1H), 7.17 - 7.12 (m, 1H), 7.00 (d, *J* = 2.5 Hz, 1H), 4.34 - 4.15 (m, 4H), 3.86 - 3.73 (m, 1H), 3.58 - 3.38 (m, 8H), 3.30 - 3.20 (m, 2H), 2.80 - 2.66 (m, 2H), 2.47 - 2.22 (m, 10H), 2.21 (s, 3H), 2.12 - 1.96 (m, 3H), 1.76 - 1.63 (m, 6H), 1.54 - 1.38 (m, 5H), 1.33 - 1.21 (m, 2H), 1.11 - 0.94 (m, 4H), 0.64 - 0.58 (m, 2H), 0.40 - 0.34 (m, 2H).

### Example 78: 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((1-((4-((3-(4-chloro-3-(2,4-dioxotetrahydropyrimidin-1(2H)-yl)benzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate

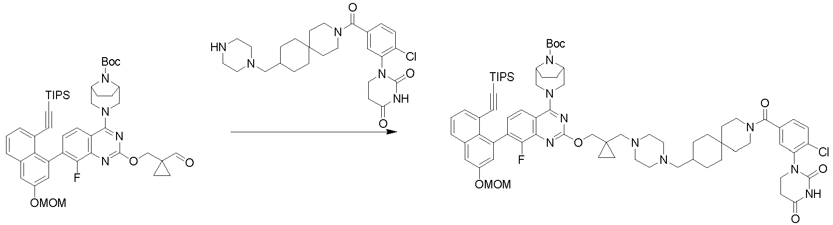

AcOH (0.15 mL) was added to a solution of *tert*-butyl 3-(8-fluoro-2-((1-formylcyclopropyl)methoxy)-7-(3-(methoxymethoxy)-8-((triisopropylsilyl)ethynyl)naphthalen-1-yl)quinazolin-4-yl)-3,8-diazabicyclo[3.2.1]octane-8-carboxylate (150 mg, 0.18 mmol) and 1-(2-chloro-5-(9-(piperazin-1-ylmethyl)-3-azaspiro[5.5]undecane-3-carbonyl)phenyl)dihydropyrimidine-2,4(1*H*,3*H*)-dione (136 mg, 0.27 mmol) in MeOH (1.5 mL), and the mixed solution was stirred at 30 °C for 2 h. NaBH₃CN (34 mg, 0.54 mmol) was then added at room temperature, and the mixture was stirred for 1 h. The mixture was concentrated and purified by silica gel column chromatography (DCM:MeOH = 100:1 to 10:1) to give *tert-*butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1308.8.

### Step 2: preparation of 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1H,3H)-dione

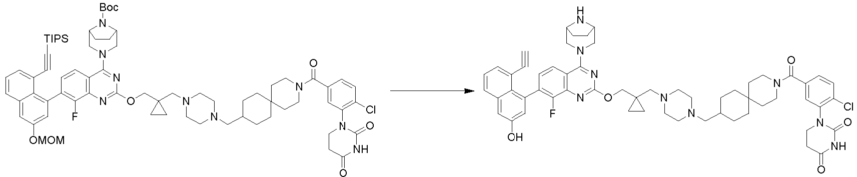

Cesium fluoride (87 mg, 0.575 mmol) was added to a stirred solution of *tert*-butyl 3-(2-((1-((4-((3-(4-chloro-3-(2,4-dioxotetrahydropyrimidin-1(2*H*)-yl)benzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)quinazolin-4-yl)-3,8-diazabicyclo[3.2.1]octane-8-carboxylate (150 mg, 0.115 mmol) in DMF (2 mL), and the mixture was stirred at 30 °C for 1 h. The mixture was diluted with H₂O (5 mL), and the resulting mixture was extracted with ethyl acetate (5 mL × 3). The combined organic phase was dried over Na₂SO₄, filtered, and concentrated under reduced pressure. 1,4-Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1008.2.

¹H NMR (400 MHz, DMSO-*d*₆) δ 10.50 (s, 1H), 10.09 (s, 1H), 7.87 (d, *J* = 8.0 Hz, 1H), 7.71 (d, *J* = 8.7 Hz, 1H), 7.63 (d, *J* = 8.2 Hz, 1H), 7.55 - 7.50 (m, 1H), 7.47 - 7.35 (m, 3H), 7.30 (d, *J* = 2.4 Hz, 1H), 7.18 - 7.11 (m, 1H), 7.00 (d, *J* = 2.4 Hz, 1H), 4.30 (d, *J* = 11.3 Hz, 1H), 4.25 - 4.13 (m, 3H), 3.80 - 3.69 (m, 1H), 3.64 - 3.39 (m, 8H), 3.29 - 3.25 (m, 2H), 2.82 - 2.62 (m, 3H), 2.44 - 2.18 (m, 10H), 2.04 (s, 2H), 1.76 - 1.62 (m, 6H), 1.55 - 1.38 (m, 5H), 1.34 - 1.22 (m, 2H), 1.11 - 0.92 (m, 4H), 0.64 - 0.58 (m, 2H), 0.40 - 0.34 (m, 2H).

Example 79: 1-(5-(4-(((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)ethyl)(methyl)amino)methyl)piperidine-1-carbonyl)-2-chlorophenyl)dihydropyrimidine-2,4(1*H*,3*H*)-dione

### Step 1: preparation of benzyl 4-(2-(methyl(piperidin-4-ylmethyl)amino)ethyl)piperidine-1-carboxylate

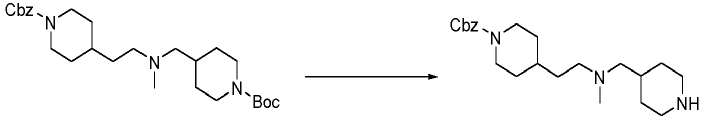

TFA (1 mL) was added to a solution of benzyl 4-(2-(((1-(*tert*-butoxycarbonyl)piperidin-4-yl)methyl)(methyl)amino)ethyl)piperidine-1-carboxylate (300 mg, 0.63 mmol) in DCM (1 mL), and the mixture was stirred at 25 °C for 2 h and concentrated under reduced pressure to give benzyl 4-(2-(methyl(piperidin-4-ylmethyl)amino)ethyl)piperidine-1-carboxylate, which was directly used in the next step without purification.

### Step 2: preparation of benzyl 4-(2-(((1-(4-chloro-3-(2,4-dioxotetrahydropyrimidin-1(2H)-yl)benzoyl)piperidin-4-yl)methyl)(methyl)amino)ethyl)piperidine-1-carboxylate

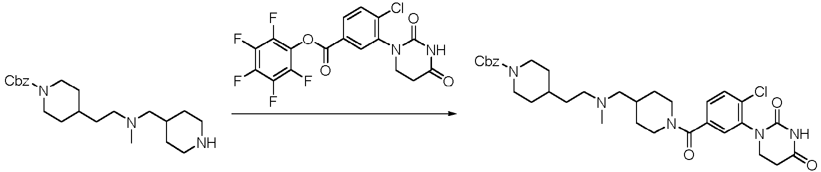

Pentafluorophenyl 4-chloro-3-(2,4-dioxotetrahydropyrimidin-1(2*H*)-yl)benzoate (274 mg, 0.63 mmol) and DIEA (407 mg, 3.15 mmol) were added to a solution of benzyl 4-(2-(methyl(piperidin-4-ylmethyl)amino)ethyl)piperidine-1-carboxylate (235 mg, crude) in DMF (3 mL). The mixture was stirred at room temperature for 2 h. After the reaction was completed, water (10 mL) was added to quench the reaction, and the mixture was extracted with ethyl acetate (10 mL × 3). The organic phase was dried, concentrated, and purified by silica gel column chromatography (DCM:MeOH = 20:1) to give benzyl 4-(2-(((1-(4-chloro-3-(2,4-dioxotetrahydropyrimidin-1(2*H*)-yl)benzoyl)piperidin-4-yl)methyl)(methyl)amino)ethyl)piperidine-1-carboxylate.

LC-MS: (ESI, m/z): [M+H]⁺ = 624.3.

### Step 3: preparation of 1-(2-chloro-5-(4-((methyl(2-(piperidin-4-yl)ethyl)amino)methyl)piperidine-1-carbonyl)phenyl)dihydropyrimidine-2,4(1H,3H)-dione

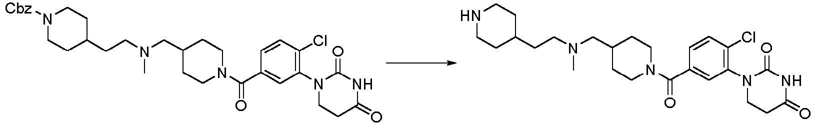

Benzyl 4-(2-(((1-(4-chloro-3-(2,4-dioxotetrahydropyrimidin-1(2*H*)-yl)benzoyl)piperidin-4-yl)methyl)(methyl)amino)ethyl)piperidine-1-carboxylate (350 mg, 0.56 mmol) was added to a TFA solution (3 mL). The mixture was stirred at 25 °C for 1 h and then concentrated under reduced pressure, and the crude product was purified by silica gel column chromatography (DCM:MeOH = 10:1) to give 1-(2-chloro-5-(4-((methyl(2-(piperidin-4-yl)ethyl)amino)methyl)piperidine-1-carbonyl)phenyl)dihydropyrimidine-2,4(1*H*,3*H*)-dione.

LC-MS: (ESI, m/z): [M+H]⁺= 489.9.

### Step 4: preparation of tert-butyl 3-(2-((1-((4-(2-(((1-(4-chloro-3-(2,4-dioxotetrahydropyrimidin-1(2H)-yl)benzoyl)piperidin-4-yl)methyl)(methyl)amino)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

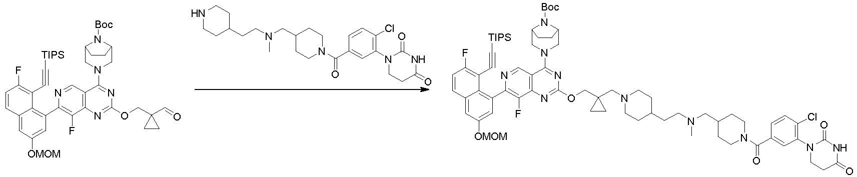

1-(2-Chloro-5-(4-((methyl(2-(piperidin-4-yl)ethyl)amino)methyl)piperidine-1-carbonyl)phenyl)dihydropyrimidine-2,4(1*H*,3*H*)-dione (132 mg, 0.27 mmol) and tetraisopropyl titanate (256 mg, 0.90 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (150 mg, 0.18 mmol) in tetrahydrofuran (1 mL), and the mixture was stirred at 70 °C for 2 h. Methanol (1 mL) and NaBH(OAc)₃ (114 mg, 0.54 mmol) were then added at 0 °C, and the mixture was stirred for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 15:1) to give *tert*-butyl 3-(2-((1-((4-(2-(((1-(4-chloro-3-(2,4-dioxotetrahydropyrimidin-1(2*H*)-yl)benzoyl)piperidin-4-yl)methyl)(methyl)amino)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺= 658.0.

### Step 5: preparation of 1-(5-(4-(((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)ethyl)(methyl)amino)methyl)piperidine-1-carbonyl)-2-chlorophenyl)dihydropyrimidine-2,4(1H,3H)-dione

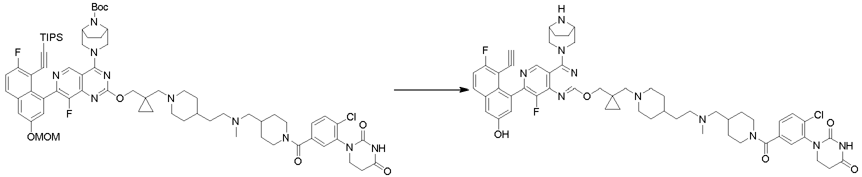

Cesium fluoride (91 mg, 0.60 mmol) was added to a stirred solution of *tert*-butyl 3-(2-((1-((4-(2-(((1-(4-chloro-3-(2,4-dioxotetrahydropyrimidin-1(2*H*)-yl)benzoyl)piperidin-4-yl)methyl)(methyl)amino)ethyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (160 mg, 0.12 mmol) in DMF (1 mL), and the mixture was stirred at 30 °C for 1 h. After the reaction was completed, the mixture was diluted with H₂O (5 mL), and the resulting mixture was extracted with ethyl acetate (5 mL × 3). The combined organic phase was dried over Na₂SO₄, filtered, and concentrated under reduced pressure. Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(4-(((2-(1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)ethyl)(methyl)amino)methyl)piperidine-1-carbonyl)-2-chlorophenyl)dihydropyrimidine-2,4(1*H*,3*H*)-dione.

LC-MS: (ESI, m/z): [M/2+H]⁺ = 508.8.

¹H NMR (400 MHz, DMSO-*d*₆) δ 10.50 (s, 1H), 10.13 (s, 1H), 9.02 (s, 1H), 7.97 (dd, *J* = 9.1, 6.0 Hz, 1H), 7.62 (d, *J* = 8.2 Hz, 1H), 7.55 - 7.51 (m, 1H), 7.49 - 7.42 (m, 1H), 7.40 - 7.34 (m, 2H), 7.16 (d,*J* = 2.4 Hz, 1H), 4.55 - 4.35 (m, 2H), 4.32 - 4.22 (m, 3H), 3.92 (s, 1H), 3.82 - 3.69 (m, 1H), 3.67 - 3.48 (m, 6H), 3.10 - 2.66 (m, 7H), 2.45 - 2.13 (m, 6H), 2.12 - 2.05 (m, 5H), 1.83 - 1.74 (m, 3H), 1.68 - 1.63 (m, 4H), 1.57 (d, *J=* 11.8 Hz, 2H), 1.31 - 1.20 (m, 3H), 1.11 - 0.98 (m, 4H), 0.66 - 0.58 (m, 2H), 0.42 - 0.35 (m, 2H).

Example 80: 1-(5-(4-(2-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)methyl)(methyl)amino)ethyl)piperidine-1-carbonyl)-2-chlorophenyl)dihydropyrimidine-2,4(1*H*,3*H*)-dione

### Step 1: preparation of tert-butyl 3-(2-((1-((4-(((2-(1-(4-chloro-3-(2,4-dioxotetrahydropyrimidin-1(2H)-yl)benzoyl)piperidin-4-yl)ethyl)(methyl)amino)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

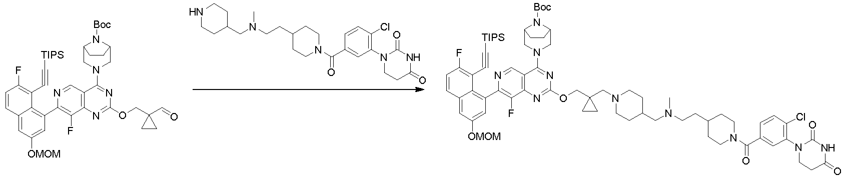

1-(2-Chloro-5-(4-(2-(methyl(piperidin-4-ylmethyl)amino)ethyl)piperidine-1-carbonyl)phenyl)dihydropyrimidine-2,4(1*H*,3*H*)-dione (98 mg, 0.20 mmol) and tetraisopropyl titanate (241 mg, 0.85 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (140 mg, 0.17 mmol) in tetrahydrofuran (1 mL), and the mixture was stirred at 70 °C for 2 h. Methanol (1 mL) and NaBH(OAc)₃ (108 mg, 0.51 mmol) were then added at 0 °C, and the mixture was stirred for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 15:1) to give *tert*-butyl 3-(2-((1-((4-(((2-(1-(4-chloro-3-(2,4-dioxotetrahydropyrimidin-1(2*H*)-yl)benzoyl)piperidin-4-yl)ethyl)(methyl)amino)methyl)piperidin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺= 658.0.

### Step 2: preparation of 1-(5-(4-(2-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperidin-4-yl)methyl)(methyl)amino)ethyl)piperidine-1-carbonyl)-2-chlorophenyl)dihydropyrimidine-2,4(1H,3H)-dione

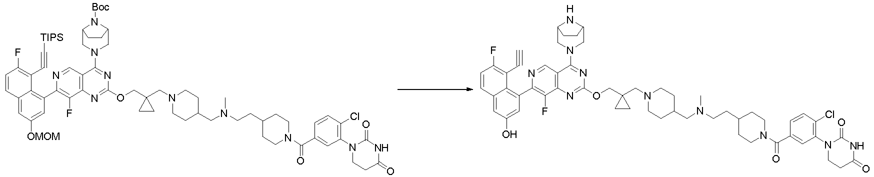

Cesium fluoride (76 mg, 0.50 mmol) was added to a stirred solution of *tert*-butyl 3-(2-((1-((4-(((2-(1-(4-chloro-3-(2,4-dioxotetrahydropyrimidin-1(2*H*)-yl)benzoyl)piperidin-4-yl)ethyl)(methyl)amino)methyl)piperidin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate (135 mg, 0.10 mmol) in DMF (1 mL), and the mixture was stirred at 30 °C for 1 h. After the reaction was completed, the mixture was diluted with H₂O (5 mL), and the resulting mixture was extracted with ethyl acetate (5 mL × 3). The combined organic phase was dried over Na₂SO₄, filtered, and concentrated under reduced pressure. Dioxane (1 mL) and hydrochloric acid/1,4-dioxane (8 M, 1 mL) were added to the crude product, and the mixture was stirred at room temperature for 0.5 h. An aqueous Na₂CO₃ solution was added to the reaction liquid, and the mixture was adjusted to pH > 7 and extracted with ethyl acetate (5 mL × 3). The combined organic phase was dried over Na₂SO₄, filtered, and concentrated under reduced pressure. The crude product was purified by preparative high-performance liquid chromatography to give 1-(5-(4-(2-(((1-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperidin-4-yl)methyl)(methyl)amino)ethyl)piperidine-1-carbonyl)-2-chlorophenyl)dihydropyrimidine-2,4(1*H*,3*H*)-dione.

LC-MS: (ESI, m/z): [M/2+H]⁺ = 508.2.

¹H NMR (400 MHz, CD₃OD) δ 9.08 (s, 1H), 8.53 (s, 1H), 7.90 (dd, *J* = 9.0, 5.8 Hz, 1H), 7.66 (d, *J* = 8.2 Hz, 1H), 7.54 (s, 1H), 7.45 - 7.33 (m, 3H), 7.26 - 7.20 (m, 1H), 4.72 - 4.54 (m, 4H), 4.40 (d, *J* = 11.7 Hz, 1H), 3.86 - 3.76 (m, 6H), 3.63 - 3.49 (m, 2H), 3.41 (s, 1H), 3.06 - 2.74 (m, 6H), 2.70 - 2.53 (m, 4H), 2.49 - 2.35 (m, 5H), 2.01 - 1.77 (m, 8H), 1.73 - 1.36 (m, 7H), 1.25 - 1.10 (m, 2H), 0.94 - 0.85 (m, 2H), 0.79 - 0.69 (m, 2H).

### Example 81: 1-(5-(9-((4-((1-(((4-(3,8-diazabicyclo[3.2.1]octan-3-yl)-7-(3-chloro-2-cyclopropyl-5-hydroxyphenyl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of 2-bromo-6-chloro-4-methoxyaniline

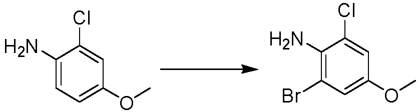

2-Chloro-4-methoxyaniline (50.0 g, 0.32 mol) was dissolved in a DCM solution (500 mL), and bromine (45.5 g, 0.29 mol) was added dropwise at 0 °C. The mixture was reacted at room temperature for 16 h. After the reaction was comuleted, an aaueous Na₂S₂O₃ solution (60 mL) was added to quench the reaction, and the mixture was extracted with DCM (60 mL × 3). The organic phases were combined, dried over anhydrous sodium sulfate, and concentrated under reduced pressure. The concentrate was purified by silica gel column chromatography (PE:EtOAc = 100:1) to give 2-bromo-6-chloro-4-methoxyaniline.

LC-MS: (ESI, m/z): [M+H]⁺ = 236.0.

### Step 2: preparation of 1-bromo-3-chloro-2-iodo-5-methoxybenzene

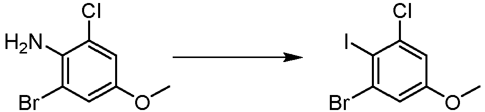

A solution of NaNO₂ (23.5 g, 0.34 mol) and KI (29.0 g, 0.42 mol) in H₂O (300 mL) was added to a solution of 2-bromo-6-chloro-4-methoxyaniline (40 g, 0.17 mol) and *p*-toluenesulfonic acid (92.9 g, 0.54 mol) in acetonitrile (2 L) at 0 °C, and the mixture was stirred at room temperature for 16 h. After the reaction was completed, a sodium bicarbonate solution (300 mL) and a sodium thiosulfate solution (300 mL) were added to quench the reaction. The mixture was stirred for 1 h and then extracted with ethyl acetate (500 mL × 3). The organic phases were combined, dried over anhydrous sodium sulfate, and concentrated under reduced pressure. The concentrate was purified by silica gel column chromatography (PE/EtOAc = 20/1) to give 1-bromo-3-chloro-2-iodo-5-methoxybenzene.

¹H NMR (400 MHz, CDCl₃) δ 7.12 (d, *J* = 2.4 Hz, 1H), 6.98 (d, *J* = 2.8 Hz, 1H), 3.78 (s, 3H).

### Step 3: preparation of 3-bromo-5-chloro-4-iodophenol

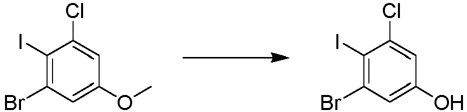

BBr₃ (1 M, 205 mL) was slowly added dropwise to a solution of 1-bromo-3-chloro-2-iodo-5-methoxybenzene (20 g, 0.057 mol) in DCM (200 mL) at 0 °C, and the mixture was stirred at room temperature for 4 h. After the reaction was completed, H₂O (100 mL) was added to quench the reaction. The pH was adjusted to 8-9 with a Na₂CO₃ solution, and the mixture was extracted with DCM (100 mL × 3). The organic phases were combined, dried over anhydrous sodium sulfate, and concentrated under reduced pressure. The concentrate was purified by silica gel column chromatography (PE:EtOAc = 10:1) to give 3-bromo-5-chloro-4-iodophenol.

LC-MS: (ESI, m/z): [M-H]⁻ = 330.7, 332.7.

### Step 4: preparation of 3-bromo-5-chloro-4-cyclopropylphenol

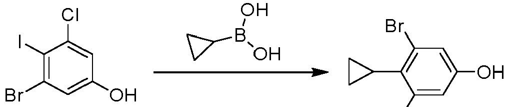

Cyclopropylboronic acid (6.4 g, 0.075 mol), Pd(dppf)Cl₂ (1.8 g, 0.0025 mol), and K₃PO₄ (38.2 g, 0.18 mol) were added to a solution of 3-bromo-5-chloro-4-iodophenol (16.5 g, 0.05 mol) in 1,4-dioxane (210 mL) and H₂O (70 mL), and the mixture was reacted at 110 °C for 16 h under N₂ atmosphere. After the reaction was completed, H₂O (200 mL) was added, and the mixture was extracted with EtOAc (60 mL × 3). The organic phases were combined, dried over anhydrous sodium sulfate, and concentrated under reduced pressure, and the mixture was purified by preparative high-performance liquid chromatography to give 3-bromo-5-chloro-4-cyclopropylphenol.

LC-MS: (ESI, m/z): [M-H]⁻ = 245.0, 247.0.

### Step 5: preparation of 3-chloro-4-cyclopropyl-5-(4,4,5,5-tetramethyl-1,3,2-dioxaborolan-2-yl)phenol

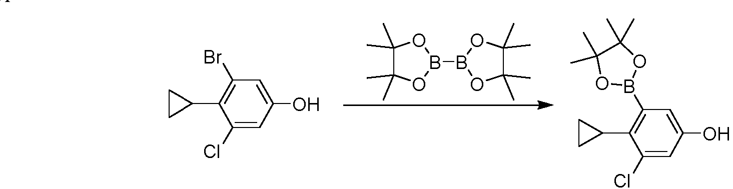

3-Bromo-5-chloro-4-cyclopropylphenol (3.2 g, 12.93 mmol), bis(pinacolato)diboron (8.2 g, 32.33 mmol), Pd(dppf)Cl₂ (0.95 g, 1.30 mmol), and AcOK (3.8 g, 38.79 mmol) were dissolved in 1,4-dioxane (30 mL), and the mixture was stirred at 110 °C for 16 h. H₂O (30 mL) was added, and the mixture was extracted with EtOAc (50 mL × 3). The organic phases were combined, dried over anhydrous sodium sulfate, and concentrated under reduced pressure to give 3-chloro-4-cyclopropyl-5-(4,4,5,5-tetramethyl-1,3,2-dioxaborolan-2-yl)phenol, which was directly used in the next step without purification.

### Step 6: preparation of tert-butyl 3-(7-(3-chloro-2-cyclopropyl-5-hydroxyphenyl)-8-fluoro-2-((1-(hydroxymethyl)cyclopropyl)methoxy)pyrido[4,3-d]pyrimidin-4-yl)-3,8-diazabicyclo[3.2.1]octane-8-carboxylate

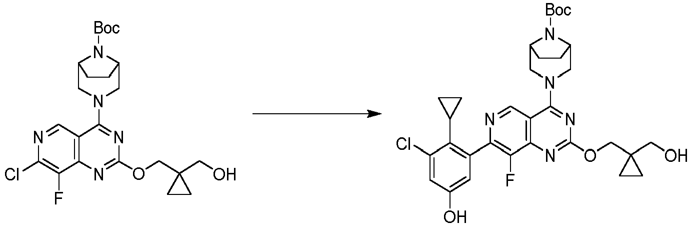

3-Chloro-4-cyclopropyl-5-(4,4,5,5-tetramethyl-1,3,2-dioxaborolan-2-yl)phenol (330 mg, 1.12 mmol), cataCXium A Pd G3 (109 mg, 0.15 mmol), and cesium carbonate (734 mg, 2.25 mmol) were added to a mixed solution of *tert*-butyl 3-(7-chloro-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (370 mg, 0.75 mmol) in 1,4-dioxane (6 mL) and H₂O (1 mL). The mixture was reacted at 85 °C for 16 h under nitrogen atmosphere. Water (10 mL) was added to quench the reaction, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 50:1 to give *tert*-butyl 3-(7-(3-chloro-2-cyclopropyl-5-hydroxyphenyl)-8-fluoro-2-((1-(hydroxymethyl)cyclopropyl)methoxy)pyrido [4,3-*d*]pyrimidin-4-yl)-3,8-diazabicyclo[3.2.1]octane-8-carboxylate.

LC-MS: (ESI, m/z): [M+H]⁺ = 626.2.

### Step 7: preparation of tert-butyl 3-(7-(3-chloro-2-cyclopropyl-5-hydroxyphenyl)-8-fluoro-2-((1-formylcyclopropyl)methoxy)pyrido[4,3-d]pyrimidin-4-yl)-3,8-diazabicyclo[3.2.1]octane-8-carboxylate

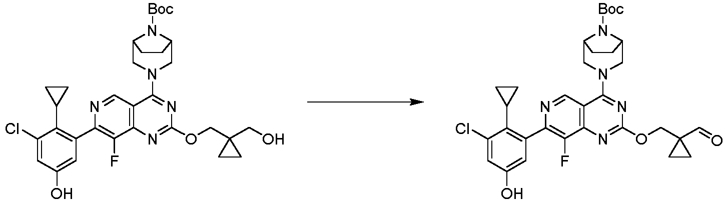

TPAP (5.6 mg, 0.016 mmol) and NMO (41 mg, 0.35 mmol) were added to a solution of *tert*-butyl 3-(7-(3-chloro-2-cyclopropyl-5-hydroxyphenyl)-8-fluoro-2-((1-(hydroxymethyl)cyclopropyl)methoxy)pyrido[4,3-*d*]pyrimidin-4-yl)-3,8-diazabicyclo[3.2.1]octane-8-carboxylate (100 mg, 0.16 mmol) in DCM (2 mL), and the mixture was stirred at room temperature for 2 h. After the reaction was completed, water (10 mL) was added, and the mixture was extracted with DCM (10 mL × 3). The organic phases were combined, dried over anhydrous sodium sulfate, and concentrated under reduced pressure. The concentrate was purified by silica gel column chromatography (DCM:MeOH = 50:1) to give *tert-butyl* 3-(7-(3-chloro-2-cyclopropyl-5-hydroxyphenyl)-8-fluoro-2-((1-formylcyclopropyl)methoxy)pyrido[4,3-*d*]pyrimidin-4-yl)-3,8-diazabicyclo[3.2.1]octane-8-carboxylate.

LC-MS: (ESI, m/z): [M+H]⁺ = 623.8.

### Step 8: preparation of tert-butyl 3-(7-(3-chloro-2-cyclopropyl-5-hydroxyphenyl)-2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoropyrido[4,3-d]pyrimidin-4-yl)-3,8-diazabicyclo[3.2.1]octane-8-carboxylate

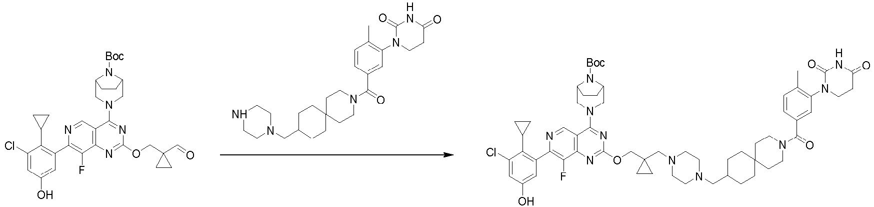

1-(2-Methyl-5-(9-(piperazin-1-ylmethyl)-3-azaspiro[5.5]undecane-3-carbonyl)phenyl)dihydropyrimidine-2,4(1*H*,3*H*)-dione (79 mg, 0.16 mmol), AcOH (0.1 mL), and tetraisopropyl titanate (156 mg, 0.55 mmol) were added to a solution of *tert*-butyl 3-(7-(3-chloro-2-cyclopropyl-5-hydroxyphenyl)-8-fluoro-2-((1-formylcyclopropyl)methoxy)pyrido[4,3-*d*]pyrimidin-4-yl)-3,8-diazabicyclo[3.2.1]octane-8-carboxylate (70 mg, 0.11 mmol) in DCM (1 mL), and the mixture was stirred at room temperature for 2 h. The reaction liquid was cooled to 0 °C, and NaBH(OAc)₃ (70 mg, 0.33 mmol) was added. The mixture was reacted at room temperature for another 4 h. The mixture was concentrated and purified by silica gel column chromatography (DCM:MeOH = 100:1 to 20:1) to give *tert-*butyl 3-(7-(3-chloro-2-cyclopropyl-5-hydroxyphenyl)-2-((1-((4-((3-(3-(2,4-dioxotetrahydropyrimidin-1(2*H*)-yl)-4-methylbenzoyl)-3-azaspiro[5.5]undecan-9-yl)methyl) piperazin-1-yl)methyl)cyclopropyl)methoxy)-8-fluoropyrido[4,3-*d*]pyrimidin-4-yl)-3,8-diazabicyclo[3.2.1] octane-8-carboxylate.

LC-MS: (ESI, m/z): [M+H]⁺ = 1089.3.

### Step 9: preparation of 1-(5-(9-((4-((1-(((4-(3,8-diazabicyclo[3.2.1]octan-3-yl)-7-(3-chloro-2-cyclopropyl-5-hydroxyphenyl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

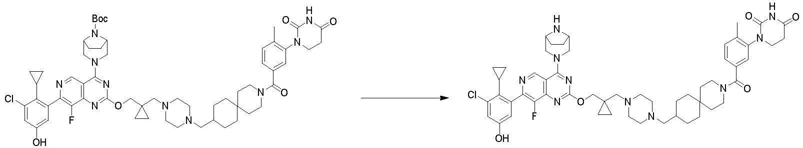

*tert*-Butyl 3-(7-(3-chloro-2-cyclopropyl-5-hydroxyphenyl)-2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-3,8-diazabicyclo[3.2.1]octane-8-carboxylate (70 mg, 0.06 mmol) was dissolved in 1,4-dioxane (2 mL), and HCl/1,4-dioxane (6 N, 1 mL) was added. The mixture was reacted at room temperature for 0.5 h. The reaction liquid was concentrated and purified by preparative high-performance liquid chromatography to give 1-(5-(9-((4-((1-(((4-(3,8-diazabicyclo[3.2.1]octan-3-yl)-7-(3-chloro-2-cyclopropyl-5-hydroxyphenyl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 989.2.

¹H NMR (400 MHz, CD₃OD) δ 9.03 (s, 1H), 7.40 (d, *J* = 7.8 Hz, 1H), 7.34 - 7.27 (m, 2H), 6.95 (d, *J* = 2.3 Hz, 1H), 6.78 (d, *J* = 2.5 Hz, 1H), 4.59 (d, *J* = 12.0 Hz, 2H), 4.42 (s, 2H), 3.90 - 3.80 (m, 1H), 3.73 - 3.58 (m, 7H), 3.49 - 3.36 (m, 2H), 2.90 - 2.77 (m, 2H), 2.67 - 2.35 (m, 9H), 2.31 (s, 3H), 2.19 - 2.10 (m, 2H), 1.89 - 1.81 (m, 3H), 1.81-1.70 (m, 4H), 1.63 - 1.56 (m, 3H), 1.53 - 1.40 (m, 3H), 1.34 - 1.26 (m, 3H), 1.21 - 1.10 (m, 3H), 0.74 - 0.68 (m, 2H), 0.67 - 0.56 (m, 2H), 0.53 - 0.47 (m, 2H), 0.13 - 0.01 (m, 2H).

### Example 82: 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

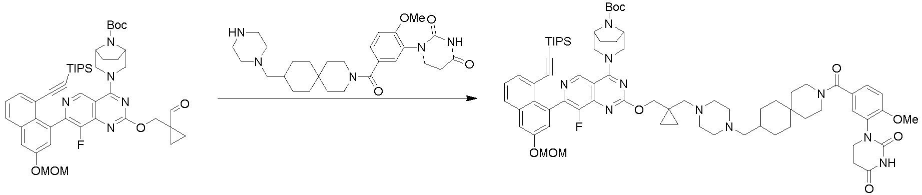

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (134 mg, 0.27 mmol), AcOH (0.1 mL), and tetraisopropyl titanate (68 mg, 0.90 mmol) were added to a solution of *tert*-butyl 3-(8-fluoro-2-((1-formylcyclopropyl)methoxy)-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (150 mg, 0.18 mmol) in DCM (1.5 mL), and the mixture was stirred at room temperature for 2 h. The reaction liquid was cooled to 0 °C, and NaBH(OAc)₃ (114 mg, 0.54 mmol) was added. The mixture was reacted at room temperature for another 4 h. The mixture was concentrated and purified by silica gel column chromatography (DCM:MeOH = 100:1 to 20:1) to give *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 1305.8.

### Step 2: preparation of 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

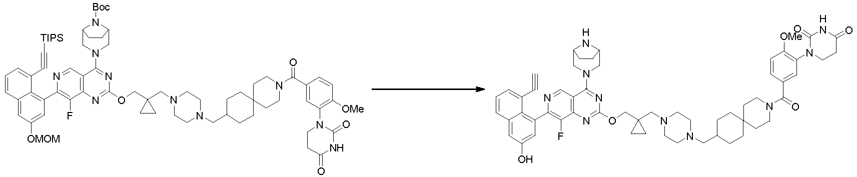

CsF (8.97 mg, 0.55 mmol) was added to a solution of *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoro-7-(3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (140 mg, 0.11 mmol) in DMF (2 mL), and the mixture was stirred at room temperature for half an hour. Water (10 mL) was added, and the mixture was extracted with ethyl acetate (10 mL × 3). The organic phases were combined, dried over anhydrous sodium sulfate, and concentrated under reduced pressure. The intermediate obtained by concentration was dissolved in dioxane (2 mL), and HCl/1,4-dioxane (6 N, 1 mL) was added. The mixture was reacted at room temperature for 0.5 h. An aqueous Na₂CO₃ solution was added to the reaction liquid, and the mixture was adjusted to pH > 7 and extracted with ethyl acetate (5 mL × 3). The combined organic layer was dried over Na₂SO₄, filtered, concentrated under reduced pressure, and purified by preparative high-performance liquid chromatography to give 1-(5-(9-((4-((1-(((4-(3,8-diazabicyclo[3.2.1]octan-3-yl)-7-(8-ethynyl-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 503.2.

¹H NMR(400 MHz, MeOD) δ: 8.98 (s, 1H), 7.81 (d, *J* = 8.0 Hz, 1H), 7.53 - 7.47 (m, 1H), 7.43 (dd, *J* = 8.5, 2.1 Hz, 1H), 7.41 - 7.35 (m, 2H), 7.31 (d, *J* = 2.4 Hz, 1H), 7.18 (d, *J* = 8.6 Hz, 1H), 7.15 (d, *J* = 2.6 Hz, 1H), 4.65 (d, *J* = 11.3 Hz, 1H), 4.58 - 4.45 (m, 2H), 4.35 (d, *J* = 11.1 Hz, 1H), 3.92 (s, 3H), 3.79 - 3.61(m, 8H), 3.54 -3.43 (m, 2H), 2.80 (t, *J* = 6.7 Hz, 2H), 2.69 - 2.36 (m, 9H), 2.20 -2.11 (m, 2H), 1.95 - 1.71 (m, 6H), 1.69 - 1.21 (m, 9H), 1.19 - 1.00 (m, 4H), 0.74 - 0.67 (m, 2H), 0.54 -0.42 (m, 2H).

### Example 83: 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate

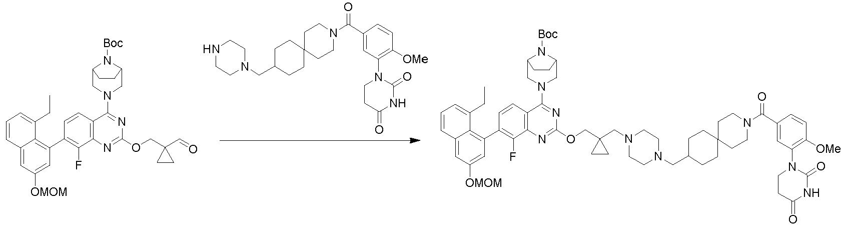

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (90 mg, 0.18 mmol), AcOH (0.1 mL), and tetraisopropyl titanate (170 mg, 0.60 mmol) were added to a solution of *tert*-butyl 3-(7-(8-ethyl-3-(methoxymethoxy)naphthalen-1-yl)-8-fluoro-2-((1-formylcyclopropyl)methoxy)quinazolin-4-yl)-3,8-diazabicyclo[3.2.1]octane-8-carboxylate (80 mg, 0.12 mmol) in DCM (1.0 mL), and the mixture was stirred at room temperature for 2 h. The reaction liquid was cooled to 0 °C, and NaBH(OAc)₃ (76 mg, 0.36 mmol) was added. The mixture was reacted at room temperature for another 4 h. The mixture was concentrated and purified by column chromatography (DCM:MeOH = 100:1 to 20:1) to give *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy) naphthalen-1-yl)-8-fluoroquinazolin-4-yl)-3,8-diazabicyclo[3.2.1]octane-8-carboxylate.

LC-MS: (ESI, m/z): [(M-Boc)/2+H]⁺ = 526.9.

### Step 2: preparation of 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

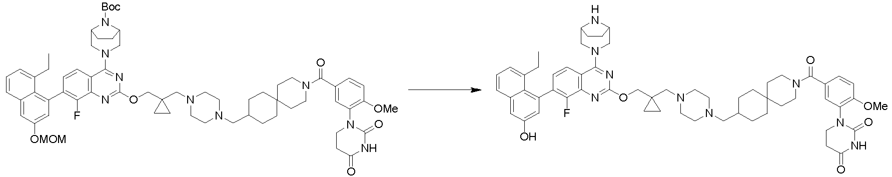

*tert*-Butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoroquinazolin-4-yl)-3,8-diazabicyclo[3.2.1]octane-8-carboxylate (40 mg, 0.035 mmol) was dissolved in 1,4-dioxane (2 mL), and HCl/1,4-dioxane (6 N, 1 mL) was added. The mixture was reacted at room temperature for 0.5 h. The reaction liquid was concentrated and purified by preparative high-performance liquid chromatography to give 1-(5-(9-((4-((1-(((4-(3,8-diazabicyclo[3.2.1]octan-3-yl)-7-(8-ethyl-3-hydroxynaphthalen-1-yl)-8-fluoroquinazolin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 504.7.

¹H NMR (400 MHz, CD₃OD) δ: 7.83 (d, *J* = 9.2 Hz, 1H), 7.60 *(d, J=* 8.0 Hz, 1H), 7.46 - 7.41 (m, 1H), 7.37 (d, *J* = 2.0 Hz, 1H), 7.34 (d, *J* = 8.4 Hz, 1H), 7.32 - 7.27 (m, 1H), 7.22 (d, *J* = 2.8 Hz, 1H), 7.18 (d, *J* = 8.4 Hz, 1H), 7.13 (d, *J* = 6.8 Hz, 1H), 6.90 (d, *J* = 2.8 Hz, 1H), 4.58 - 4.33 (m, 4H), 3.98 - 3.85 (m, 5H), 3.73 - 3.63 (m, 5H), 3.46-3.51(m, 2H), 2.86 - 2.79(m, 5H), 2.50 - 2.38 (m, 4H), 2.09 - 0.98 (m, 4H), 1.97 - 1.88 (m, 4H), 1.80 - 1.71(m, 2H), 1.66 - 1.53 (m, 4H), 1.53 - 1.45 (m, 2H), 1.35 - 1.24(m, 7H), 1.22 - 1.16 (m, 2H), 0.86 (t*, J* = 6.8 Hz, 3H), 0.81 - 0.73 (m, 2H), 0.64 - 0.52 (m, 2H).

### Example 84: 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of methyl 3-(4-benzylpiperazin-1-yl)-2-methylpropanoate

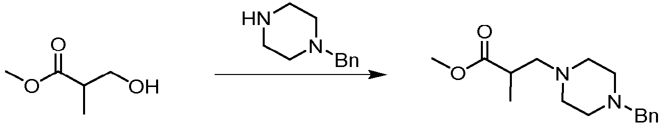

Methyl 3-hydroxy-2-methylpropanoate (3.60 g, 30.47 mmol) was dissolved in dichloromethane (35 mL), and triethylamine (9.25 g, 91.42 mmol) and methanesulfonyl chloride (5.24 g, 45.71 mmol) were added at 0 °C. The mixture was reacted for 2 h. After the reaction was completed, the mixture was warmed to room temperature. Water (50 mL) was added, and the mixture was extracted with ethyl acetate (50 mL × 3). The combined organic phase was washed with a saturated NaCl solution, dried over anhydrous Na₂SO₄, and filtered, and the filtrate was concentrated to give a crude product. The crude product was dissolved in DMF (50 mL), and potassium carbonate (12.64 g, 91.42 mmol) and 1-benzylpiperazine (10.74 g, 60.95 mmol) were added. The reaction liquid was heated to 85 °C and reacted for 16 h. After the reaction was completed, the mixture was cooled to room temperature. Water (200 mL) was added to the reaction liquid, and the mixture was extracted with ethyl acetate (100 mL × 3). The combined organic phase was washed with a saturated NaCl solution, dried over anhydrous Na₂SO₄, and filtered, and the filtrate was concentrated to give a crude product. The resulting crude product was purified by silica gel column chromatography (DCM:MeOH = 20:1) to give methyl 3-(4-benzylpiperazin-1-yl)-2-methylpropanoate.

LC-MS: (ESI, m/z): [M+H]⁺ = 277.3.

### Step 2: preparation of 3-(4-benzylpiperazin-1-yl)-2-methylpropan-1-ol

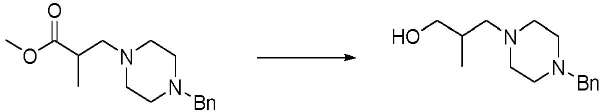

Methyl 3-(4-benzylpiperazin-1-yl)-2-methylpropanoate (1.17 g, 4.23 mmol) was dissolved in THF (10 mL), and DIBAL-H (12.69 mL, 12.69 mmol, 1 M) was added in an ice bath. The mixture was reacted for 3 h. After the reaction was completed, the mixture was warmed to room temperature. A saturated potassium sodium tartrate solution (100 mL) was added to the reaction liquid, and the mixture was extracted with ethyl acetate (50 mL × 3). The combined organic phase was washed with a saturated NaCl solution, dried over anhydrous Na₂SO₄, and filtered, and the filtrate was concentrated to give a crude product. The resulting crude product was purified by silica gel column chromatography (DCM:MeOH = 20:1) to give 3-(4-benzylpiperazin-1-yl)-2-methylpropan-1-ol.

LC-MS: (ESI, m/z): [M+H]⁺ = 249.1.

### Step 3: preparation of tert-butyl 3-(2-(3-(4-benzylpiperazin-1-yl)-2-methylpropoxy)-7-chloro-8-fluoropyrido[4,3-d]pyrimidin-4-yl)-3,8-diazabicyclo[3.2.1]octane-8-carboxylate

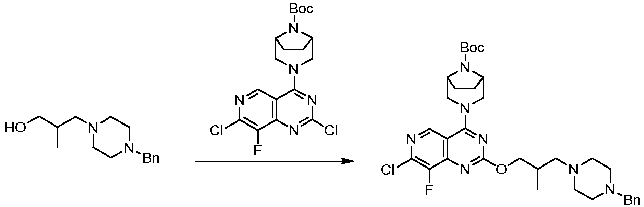

*tert*-Butyl 3-(2,7-dichloro-8-fluoropyrido[4,3-*d*]pyrimidin-4-yl)-3,8-diazabicyclo[3.2.1]octane-8-carboxylate (997 mg, 2.33 mmol) and 3-(4-benzylpiperazin-1-yl)-2-methylpropan-1-ol (867 mg, 3.49 mmol) were dissolved in THF (10 mL), and Cs₂CO₃ (1.14 g, 3.49 mmol) was added. The reaction liquid was heated to 90 °C and reacted for 4 d. After the reaction was completed, the mixture was cooled to room temperature. Water (30 mL) was added to the reaction liquid, and the mixture was extracted with ethyl acetate (30 mL × 3). The combined organic phase was washed with a saturated NaCl solution, dried over anhydrous Na₂SO₄, and filtered, and the filtrate was concentrated to give a crude product. The resulting crude product was purified by silica gel column chromatography (DCM:MeOH = 30:1) to give *tert*-butyl 3-(2-(3-(4-benzylpiperazin-1-yl)-2-methylpropoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 640.3.

### Step 4: preparation of tert-butyl 3-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

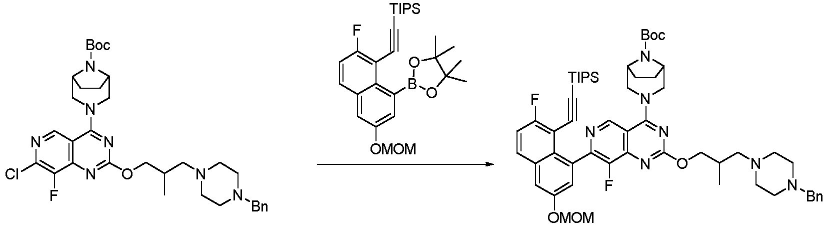

Ruphos Pd G3 (40 mg, 0.05 mmol) and potassium phosphate (298 mg, 1.41 mmol) were added to a mixed solution of *tert*-butyl 3-(2-(3-(4-benzylpiperazin-1-yl)-2-methylpropoxy)-7-chloro-8-fluoropyrido[4,3-*d*]pyrimidin-4-yl)-3,8-diazabicyclo[3.2.1]octane-8-carboxylate (300 mg, 0.47 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (288 mg, 0.56 mmol) in 1,4-dioxane (3 mL) and H₂O (0.6 mL). The mixture was reacted at 85 °C for 16 h under nitrogen atmosphere. Water (20 mL) was added to the reaction liquid to quench the reaction, and the mixture was extracted with ethyl acetate (30 mL × 3). The organic phases were combined, dried over anhydrous sodium sulfate, and concentrated under reduced pressure. The concentrate was purified by silica gel column chromatography (DCM:MeOH = 10:1) to give *tert*-butyl 3-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 495.6.

### Step 5: preparation of tert-butyl 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-d]pyrimidin-4-yl)-3,8-diazabicyclo[3.2.1]octane-8-carboxylate

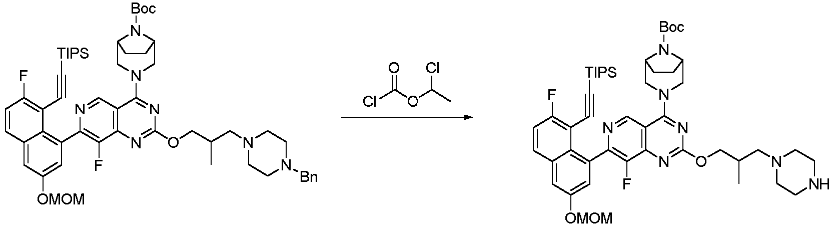

*tert*-Butyl 3-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (320 mg, 0.32 mmol) was dissolved in DCM (3 mL), and 1-chloroethyl chloroformate (139 mg, 0.97 mmol) and DIEA (125 mg, 0.97 mmol) were added. The mixture was reacted at room temperature for 1 h. The reaction liquid was directly concentrated under reduced pressure, and the concentrate was dissolved in MeOH (3 mL). Then the mixture was heated to 50 °C and reacted for 10 min. After the reaction was completed, the mixture was concentrated under reduced pressure. The concentrate was purified by silica gel column chromatography (DCM:MeOH (NH₃) = 30:1) to give *tert-*butyl 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-*d*]pyrimidin-4-yl)-3,8-diazabicyclo[3.2.1]octane-8-carboxylate.

LC-MS: (ESI, m/z): [M+H]⁺ = 899.9.

### Step 6: preparation of tert-butyl 3-(2-(3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

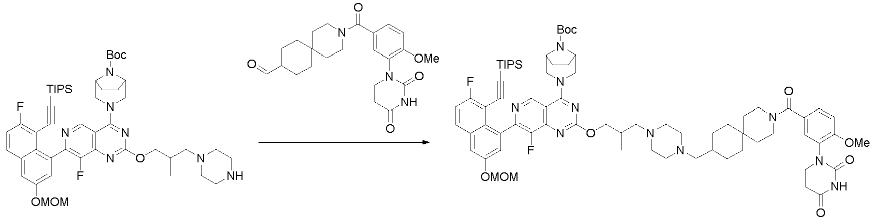

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-*d*]pyrimidin-4-yl)-3,8-diazabicyclo[3.2.1]octane-8-carboxylate (150 mg, 0.17 mmol) and 3-(3-(2,4-dioxotetrahydropyrimidin-1(2*H*)-yl)-4-methoxybenzoyl)-3-azaspiro[5.5]undecane-9-carbaldehyde (107 mg, 0.25 mmol) in DCM/AcOH (1.5 mL/0.1 mL), and the mixture was stirred at 30 °C for 2 h. NaBH(OAc)₃ (106 mg, 0.50 mmol) was added to the mixture in an ice bath, and the resulting mixture was stirred for 2 h. The mixture was concentrated and purified by silica gel column chromatography (DCM:MeOH = 100:1 to 10:1) to give *tert*-butyl 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 656.9.

### Step 7: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

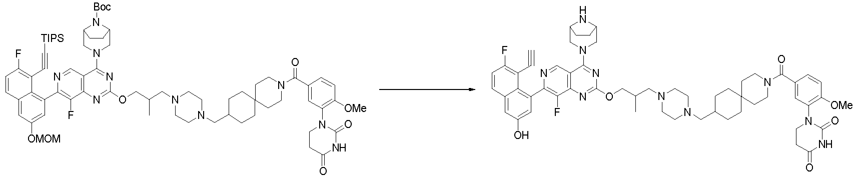

Cesium fluoride (60 mg, 0.40 mmol) was added to a stirred solution of *tert*-butyl 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (104 mg, 0.08 mmol) in DMF (1 mL), and the mixture was stirred at room temperature for 30 min. Water (10 mL) was added to the reaction liquid to quench the reaction, and the mixture was extracted with ethyl acetate (10 mL × 3). The combined organic phase was dried over anhydrous sodium sulfate and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 506.3.

¹H NMR (400 MHz, DMSO-*d*₆) δ 10.32 (s, 1H), 10.14 (s, 1H), 9.02 (s, 1H), 7.97 (dd, *J* = 9.2 Hz, 6.0 Hz, 1H), 7.47 - 7.36 (m, 1H), 7.40 - 7.34 (m, 2H), 7.33 - 7.30 (m, 1H), 7.19 - 7.11 (m, 2H), 4.51 - 4.40 (m, 2H), 4.35 - 4.26 (m, 1H), 4.12 - 4.01 (m, 1H), 3.93 (s, 1H), 3.84 (s, 3H), 3.63 - 3.44 (m, 8H), 2.68 (t, *J* = 6.4 Hz, 2H), 2.44 - 2.24 (m, 8H), 2.23 - 2.09 (m, 3H), 2.08 - 1.87 (m, 3H), 1.72 - 1.61 (m, 6H), 1.54 - 1.40 (m, 5H), 1.34 - 1.22 (m, 4H), 1.13 - 0.94 (m, 7H).

### Example 85: 1-(5-(9-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,9-diazaspiro[5.5]undecane-3-carbonyl)-2-chlorophenyl)dihydropyrimidine-2,4(1H,3H)-dione

### Step 1: preparation of tert-butyl 3-(2-((1-((9-(4-chloro-3-(2,4-dioxotetrahydropyrimidin-1(2H)-yl)benzoyl)-3,9-diazaspiro[5.5]undecan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

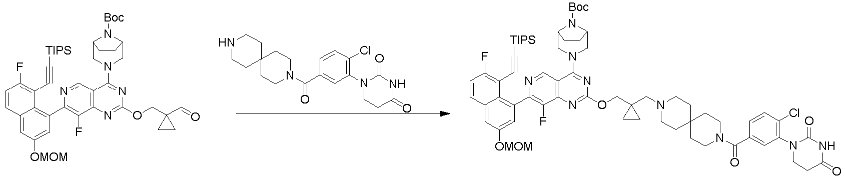

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (80 mg, 0.095 mmol) and 1-(2-chloro-5-(3,9-diazaspiro[5.5]undecane-3-carbonyl)phenyl)dihydropyrimidine-2,4(1*H*,3*H*)-dione (57.6 mg, 0.14 mmol) in DCM/AcOH (2 mL/0.1 mL), and the mixture was stirred at 30 °C for 2 h. NaBH(OAc)₃ (50 mg, 0.24 mmol) was added to the mixture in an ice bath, and the resulting mixture was stirred for 2 h. The mixture was concentrated and purified by silica gel column chromatography (DCM:MeOH = 100:1 to 10:1) to give *tert*-butyl 3-(2-((1-((9-(4-chloro-3-(2,4-dioxotetrahydropyrimidin-1(2*H*)-yl)benzoyl)-3,9-diazaspiro[5.5]undecan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 615.5.

### Step 2: preparation of 1-(5-(9-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,9-diazaspiro[5.5]undecane-3-carbonyl)-2-chlorophenyl)dihydropyrimidine-2,4(1H,3H)-dione

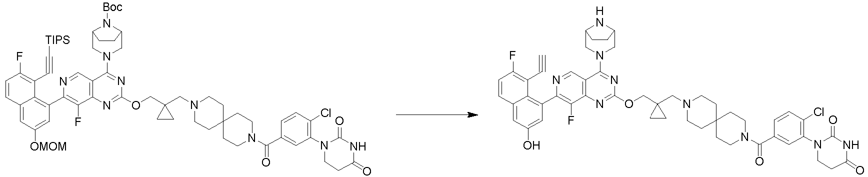

Cesium fluoride (83 mg, 0.55 mmol) was added to a stirred solution of *tert*-butyl 3-(2-((1-((9-(4-chloro-3-(2,4-dioxotetrahydropyrimidin-1(2*H*)-yl)benzoyl)-3,9-diazaspiro[5.5]undecan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (70 mg, 0.057 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (0.5 mL), and the mixture was reacted at room temperature for 0.5 h. The reaction liquid was adjusted to pH > 7 with an aqueous Na₂CO₃ solution and then extracted with EtOAc (10 mL × 2). The combined organic phase was washed with brine, dried over anhydrous Na₂SO₄, and concentrated. The crude product was purified by preparative high-performance liquid chromatography to give 1-(5-(9-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-3,9-diazaspiro[5.5]undecane-3-carbonyl)-2-chlorophenyl)dihydropyrimidine-2,4(1*H*,3*H*)-dione.

LC-MS: (ESI, m/z): [M/2+H]⁺ = 465.7.

¹H NMR (400 MHz, DMSO-*d*₆) δ 10.50 (s, 1H), 9.03 (s, 1H), 8.19 (s, 1H), 7.97 (dd, *J* = 9.1, 5.9 Hz, 1H), 7.63 (d, *J* = 8.2 Hz, 1H), 7.54 (d, *J* = 1.8 Hz, 1H), 7.49 - 7.42 (m, 1H), 7.41 - 7.33 (m, 2H), 7.16 (d, *J* = 2.3 Hz, 1H), 4.50 (d, *J* = 11.6 Hz, 1H), 4.34 - 4.20 (m, 3H), 3.92 (s, 1H), 3.78 - 3.41 (m, 11H), 2.78 - 2.68 (m, 2H), 2.43 - 2.29 (m, 6H), 1.78 - 1.60 (m, 4H), 1.54 - 1.29 (m, 8H), 0.70 - 0.55 (m, 2H), 0.48 - 0.33 (m, 2H).

### Example 86: 1-(5-(9-((4-((1-(((4-((1S,4S)-2,5-diazabicyclo[2.2.1]heptan-2-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl (1S,4S)-5-(2,7-dichloro-8-fluoropyrido[4,3-d]pyrimidin-4-yl)-2,5-diazabicyclo[2.2.1]heptane-2-carboxylate

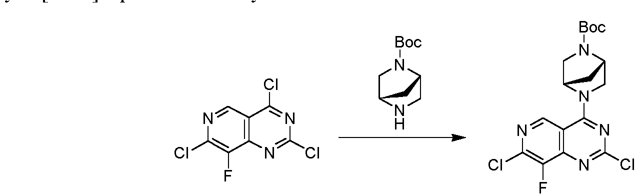

DIEA (2.04 g, 15.84 mmol) and *tert*-butyl (1*S*,4*S*)-2,5-diazabicyclo[2.2.1]heptane-2-carboxylate (0.67 g, 3.37 mmol) were added to a solution of 2,4,7-trichloro-8-fluoropyrido[4,3-*d*]pyrimidine (1 g, 3.96 mmol) in anhydrous tetrahydrofuran (20 mL) at -70 °C, and the mixture was stirred at -70 °C for 2 h. After the reaction was completed, a saturated aqueous ammonium chloride solution (30 mL) was added, and the mixture was extracted with ethyl acetate (50 mL × 2). The organic phases were combined, dried over anhydrous sodium sulfate, and concentrated under reduced pressure. The resulting crude product was purified by silica gel column chromatography (petroleum ether:ethyl acetate = 2:1) to give *tert*-butyl (1S,4S)-5-(2,7-dichloro-8-fluoropyrido[4,3-*d*]pyrimidin-4-yl)-2,5-diazabicyclo[2.2.1]heptane-2-carboxylate.

LC-MS: (ESI, m/z): [M+H]⁺ = 414.1.

### Step 2: preparation of tert-butyl (1S,4S)-5-(7-chloro-8-fluoro-2-((1-(hydroxymethyl)cyclopropyl)methoxy)pyrido[4,3-d]pyrimidin-4-yl)-2,5-diazabicyclo[2.2.1]heptane-2-carboxylate

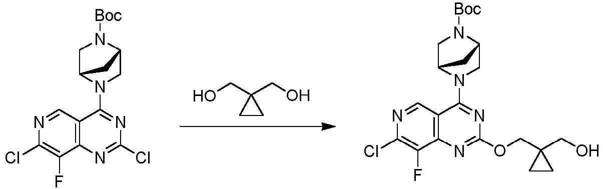

Anhydrous cesium carbonate (798 mg, 2.42 mmol) was added to a solution of *tert*-butyl (1*S*,4*S*)-5-(2,7-dichloro-8-fluoropyrido[4,3-*d*]pyrimidin-4-yl)-2,5-diazabicyclo[2.2.1]heptane-2-carboxylate (500 mg, 1.21 mmol) and cyclopropane-1,1-diyldimethanol (247 mg, 2.42 mmol) in anhydrous THF (10 mL), and the mixture was stirred at 25 °C for 16 h. After the reaction was completed, the mixture was poured into water (30 mL) and extracted with ethyl acetate (20 mL × 2). The organic phases were combined, washed with saturated sodium chloride (50 mL), dried over anhydrous sodium sulfate, and concentrated under reduced pressure. The resulting crude product was purified by silica gel column chromatography (dichloromethane:methanol = 20:1) to give *tert*-butyl (1*S*,4*S*)-5-(7-chloro-8-fluoro-2-((1-(hydroxymethyl)cyclopropyl)methoxy)pyrido[4,3-*d*]pyrimidin-4-yl)-2,5-diazabicyclo[2.2.1]heptane-2-carboxylate.

LC-MS: (ESI, m/z): [M+H]⁺ = 480.3.

### Step 3: preparation of tert-butyl (1S,4S)-5-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-d]pyrimidin-4-yl)-2,5-diazabicyclo[2.2.1]heptane-2-carboxylate

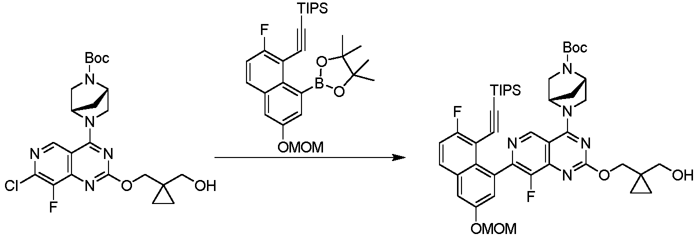

Anhydrous cesium carbonate (476 mg, 1.46 mmol) and cataCXium(R) A Pd G3 (53 mg, 0.073 mmol) were added to a mixed solution of *tert*-butyl (1*S*,4*S*)-5-(7-chloro-8-fluoro-2-((1-(hydroxymethyl)cyclopropyl)methoxy)pyrido[4,3-*d*]pyrimidin-4-yl)-2,5-diazabicyclo[2.2.1]heptane-2-carboxylate (350 mg, 0.73 mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (487 mg, 0.95 mmol) in 1,4-dioxane (8 mL) and water (1 mL) at room temperature. The mixture was stirred at 85 °C for 16 h under nitrogen atmosphere. After the reaction was completed, the mixture was concentrated under reduced pressure at 40 °C, and the resulting crude product was purified by silica gel thin-layer chromatography (dichloromethane:methanol = 100:1 to 10:1) to give *tert*-butyl (1*S*,4*S*)-5-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido[4,3-*d*]pyrimidin-4-yl)-2,5-diazabicyclo[2.2.1]heptane-2-carboxylate.

LC-MS: (ESI, m/z): [M+H]⁺ = 830.6.

### Step 4: preparation of tert-butyl (1S,4S)-5-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-d]pyrimidin-4-yl)-2,5-diazabicyclo [2.2.1]heptane-2-carboxylate

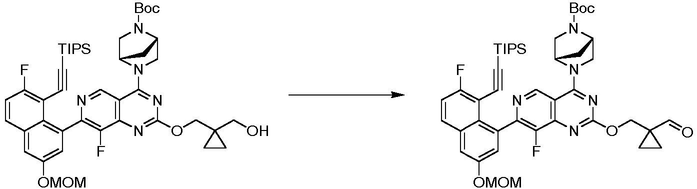

A solution of *tert*-butyl (1*S*,4*S*)-5-(8-fluoro-7-(7-fluoro-3-(methoxymethoxy)-8-((triisopropylsilyl)ethynyl)naphthalen-1-yl)-2-((1-(hydroxymethyl)cyclopropyl)methoxy)pyrido [4,3-*d*]pyrimidin-4-yl)-2,5-diazabicyclo[2.2.1]heptane-2-carboxylate (280 mg, 0.38 mmol) in dichloromethane

(5 mL) was cooled to 0 °C from room temperature, and Dess-Martin reagent (215 mg, 0.51 mmol) was added. The mixture was stirred at 15 °C for 2 h. After the reaction was completed, an aqueous sodium thiosulfate solution (20 mL) was added, and the mixture was extracted with dichloromethane (20 mL × 2). The organic phases were combined, washed with saturated sodium chloride (30 mL), dried over anhydrous sodium sulfate, and concentrated under reduced pressure. The resulting crude product was purified by silica gel thin-layer chromatography (dichloromethane:methanol = 100:1 to 20:1) to give *tert*-butyl (1S,4S)-5-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-2,5-diazabicyclo[2.2.1]heptane-2-carboxylate.

LC-MS: (ESI, m/z): [M+H]⁺ = 828.7.

### Step 5: preparation of tert-butyl (1S,4S)-5-(2-((1-((4-((3-(3-(2,4-dioxotetrahydropyrimidin-1(2H)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-2,5-diazabicyclo[2.2.1]heptane-2-carboxylate

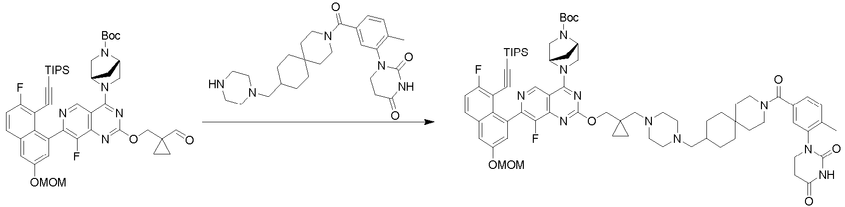

Tetraisopropyl titanate (156 mg, 0.55 mmol) was added to a solution of *tert*-butyl (1*S*,4*S*)-5-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-2,5-diazabicyclo[2.2.1]heptane-2-carboxylate (90 mg, 0.11 mmol) and 1-(2-methyl-5-(9-(piperazin-1-ylmethyl)-3-azaspiro[5.5]undecane-3-carbonyl)phenyl)dihydropyrimidine-2,4(1*H*,3*H*)-dione (105 mg, 0.22 mmol) in dichloromethane/AcOH (2.3 mL/0.2 mL) at room temperature, and the mixture was stirred at 25 °C for 2 h. Sodium triacetoxyborohydride (69 mg, 0.33 mmol) was added to the mixture at 0-5 °C. The reaction liquid was reacted at 25 °C for another 3 h, concentrated, and purified by silica gel column chromatography (dichloromethane:methanol = 100:1 to 10:1, 0.1% NH₃) to give *tert*-butyl (1*S*,4*S*)-5-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-2,5-diazabicyclo[2.2.1]heptane-2-carboxylate.

LC-MS: (ESI, m/z): [M/2+H]⁺ = 647.5.

### Step 6: preparation of 1-(5-(9-((4-((1-(((4-((1S,4S)-2,5-diazabicyclo[2.2.1]heptan-2-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1H,3H)-dione

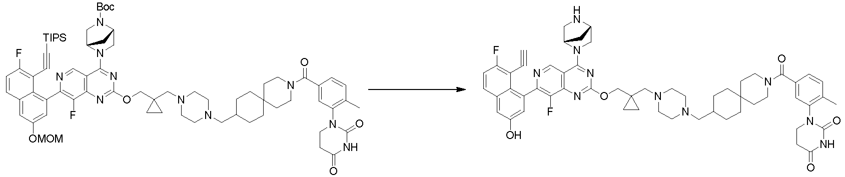

CsF (59 mg, 0.39 mmol) was added to a solution of *tert*-butyl (1*S*,4*S*)-5-(2-((1-((4-((3-(3-(2,4-dioxotetrahydropyrimidin-1(2*H*)-yl)-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-2,5-diazabicyclo[2.2.1]heptane-2-carboxylate (100 mg, 0.077 mmol) in DMF (3 mL), and the mixture was stirred at room temperature for 1 h. Water (10 mL) was added, and the mixture was extracted with ethyl acetate (10 mL × 3). The organic phases were combined, washed with a saturated aqueous sodium chloride solution, dried over anhydrous sodium sulfate, and concentrated under reduced pressure. The intermediate obtained by concentration was dissolved in 1,4-dioxane (2 mL), and HCl/1,4-dioxane (6 N, 1 mL) was added. The mixture was reacted at room temperature for 0.5 h. An aqueous Na₂CO₃ solution was added to the reaction liquid, and the mixture was adjusted to pH > 7 and extracted with ethyl acetate (10 mL × 3). The combined organic layer was dried over Na₂SO₄, filtered, concentrated under reduced pressure, and purified by preparative high-performance liquid chromatography to give 1-(5-(9-((4-((1-(((4-((1S,4S)-2,5-diazabicyclo[2.2.1]heptan-2-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 497.3.

¹H NMR (400 MHz, DMSO-*d*₆) δ 10.36 (s, 1H), 10.14 (s, 1H), 9.09 (d, *J* = 19.7 Hz, 1H), 7.97 (dd, *J* = 9.0, 6.1 Hz, 1H), 7.49 - 7.42 (m, 1H), 7.38 (d, *J* = 2.4 Hz, 1H), 7.33 (d, *J* = 8.0 Hz, 1H), 7.30 (s, 1H), 7.24 (d, *J* = 7.7 Hz, 1H), 7.19 - 7.10 (m, 1H), 5.18 (d*, J =* 21.9 Hz, 1H), 4.37 - 4.11 (m, 3H), 4.01 - 3.89 (m, 1H), 3.78 (s, 3H), 3.64 - 3.44 (m, 3H), 3.30 - 3.20 (m, 2H), 3.12 - 2.91 (m, 2H), 2.80 - 2.65 (m, 2H), 2.46 - 2.23 (m, 10H), 2.21 (s, 3H), 2.11 - 1.95 (m, 3H), 1.93 - 1.86 (m, 1H), 1.80 - 1.73 (m, 1H), 1.71 - 1.62 (m, 2H), 1.55 - 1.38 (m, 5H), 1.32 - 1.23 (m, 2H), 1.12 - 0.94 (m, 4H), 0.66 - 0.58 (m, 2H), 0.44 - 0.35 (m, 2H).

### Example 87: 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,6-diazabicyclo[3.1.1]heptan-6-yl)methyl)-3-azaspiro[5.5]undecane-3-carbonyl)-2-chlorophenyl)dihydropyrimidine-2,4(1H,3H)-dione

### Step 1: preparation of tert-butyl 6-((3-(4-chloro-3-(2,4-dioxotetrahydropyrimidin-1(2H)-yl)benzoyl)-3-azaspiro[5.5]undecan-9-yl)methyl)-3,6-diazabicyclo[3.1.1]heptane-3-carboxylate

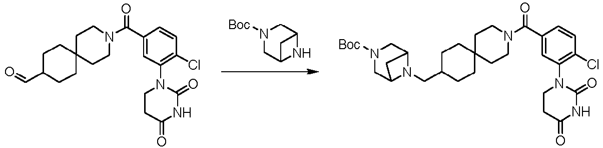

*tert*-Butyl 3,6-diazabicyclo[3.1.1]heptane-3-carboxylate (66 mg, 0.33 mmol) was added to a solution of 3-(4-chloro-3-(2,4-dioxotetrahydropyrimidin-1(2*H*)-yl)benzoyl)-3-azaspiro[5.5]undecane-9-carbaldehyde (120 mg, 0.28 mmol) in 1,2-dichloroethane (6 mL). The mixture was stirred at 25 °C for 1 h. NaBH₃CN (177 mg, 0.84 mmol) was added at 0 °C, and the mixture was reacted at room temperature for 2 h. After the reaction was completed, H₂O (15 mL) was added, and the mixture was extracted with ethyl acetate (15 mL × 3). The combined organic phase was dried over anhydrous sodium sulfate, filtered, and concentrated under reduced pressure. The resulting crude product was purified by silica gel column chromatography (DCM:MeOH = 10:1) to give *tert*-butyl 6-((3-(4-chloro-3-(2,4-dioxotetrahydropyrimidin-1(2*H*)-yl)benzoyl)-3-azaspiro[5.5]undecan-9-yl)methyl)-3,6-diazabicyclo[3.1.1]heptane-3-carboxylate.

MS (ESI, m/z): [M+H]⁺ = 614.3.

### Step 4: preparation of 1-(5-(9-((3,6-diazabicyclo[3.1.1]heptan-6-yl)methyl)-3-azaspiro[5.5]undecane-3-carbonyl)-2-chlorophenyl)dihydropyrimidine-2,4(1H,3H)-dione

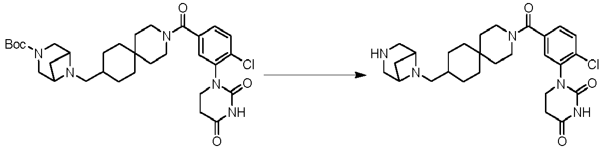

HCl/1,4-dioxane (1.5 mL) was added to a solution of *tert*-butyl 6-((3-(4-chloro-3-(2,4-dioxotetrahydropyrimidin-1(2*H*)-yl)benzoyl)-3-azaspiro[5.5]undecan-9-yl)methyl)-3,6-diazabicyclo[3.1.1]heptane-3-carboxylate (100 mg, 0.16 mmol) in 1,4-dioxane (3 mL), and the mixture was stirred at room temperature for 1 h. The reaction liquid was directly concentrated to give 1-(5-(9-((3,6-diazabicyclo[3.1.1]heptan-6-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺= 514.3.

### Step 5: preparation of tert-butyl 3-(2-((1-((6-((3-(4-chloro-3-(2,4-dioxotetrahydropyrimidin-1(2H)-yl)benzoyl)-3-azaspiro[5.5]undecan-9-yl)methyl)-3,6-diazabicyclo[3.1.1]heptan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

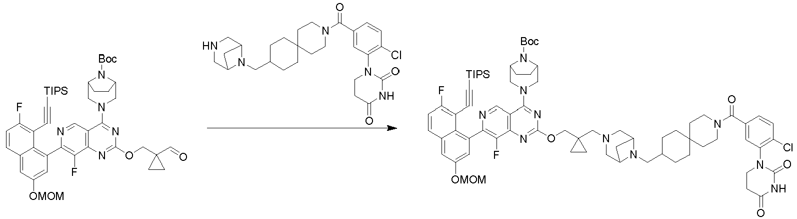

1-(5-(9-((3,6-Diazabicyclo[3.1.1]heptan-6-yl)methyl)-3-azaspiro[5.5]undecane-3-carbonyl)-2-chlorophenyl)dihydropyrimidine-2,4(1*H*,3*H*)-dione (73 mg, 0.14 mmol) and tetraisopropyl titanate (506 mg, 1.8 mmol) were added to a solution of *tert*-butyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (1 mL) and acetic acid (0.1 mL), and the mixture was stirred at 25 °C for 2 h. NaBH(OAc)₃ (76 mg, 0.36 mmol) was then added at 0 °C, and the mixture was stirred at 25 °C for 1 h. After the reaction was completed, H₂O (5 mL) was added, and the mixture was extracted with ethyl acetate (15 mL × 3). The combined organic phase was dried over anhydrous sodium sulfate, filtered, and concentrated under reduced pressure. The crude product was purified by silica gel column chromatography (DCM:MeOH = 10:1) to give *tert*-butyl 3-(2-((1-((6-((3-(4-chloro-3-(2,4-dioxotetrahydropyrimidin-1(2*H*)-yl)benzoyl)-3-azaspiro[5.5]undecan-9-yl)methyl)-3,6-diazabicyclo[3.1.1]heptan-3-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

MS (ESI): [M/2+H]⁺ = 670.5.

### Step 6: preparation of 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)-3,6-diazabicyclo[3.1.1]heptan-6-yl)methyl)-3-azaspiro[5.5]undecane-3-carbonyl)-2-chlorophenyl)dihydropyrimidine-2,4(1H,3H)-dione

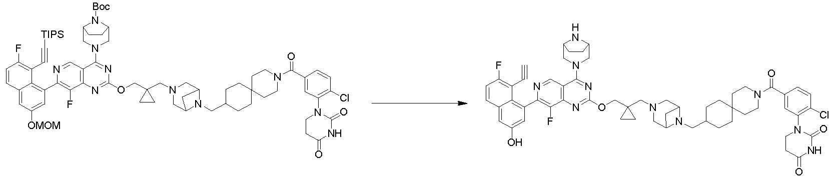

*tert*-Butyl 3-(2-((1-((6-((3-(4-chloro-3-(2,4-dioxotetrahydropyrimidin-1(2*H*)-yl)benzoyl)-3-azaspiro[5.5]undecan-9-yl)methyl)-3,6-diazabicyclo[3.1.1]heptan-3-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-3,8-diazabicyclo[3.2.1]octane-8-carboxylate (25 mg, 0.019 mmol) was dissolved in DMF (1 mL), and CsF (14 mg, 0.095 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrogen chloride/1,4-dioxane (6 M, 1 mL) was added to the concentrate, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((3-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)-3,6-diazabicyclo[3.1.1]heptan-6-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1039.1.

¹H NMR (400 MHz, CD₃OD) δ 9.00 (s, 1H), 7.89 - 7.82 (m, 1H), 7.67 - 7.59 (m, 1H), 7.51 (s, 1H), 7.44 - 7.28 (m, 3H), 7.20 (s, 1H), 4.63 - 4.47 (m, 4H), 4.34 - 4.27 (m, 1H), 3.81 - 3.65 (m, 8H), 3.35 (s, 1H), 3.19 - 3.11 (m, 4H), 2.89 - 2.67 (m, 5H), 2.19 (t, *J* = 7.5 Hz, 2H), 1.86 - 1.70 (m, 5H), 1.61 - 1.54 (m, 4H), 1.33 - 1.30 (m, 7H), 1.17 - 1.13 (m, 2H), 0.93 - 0.87 (m, 3H), 0.73 - 0.66 (m, 2H), 0.56 - 0.49 (m, 2H).

### Example 88: 1-(5-(9-((4-((1-(((4-(3,9-diazabicyclo[4.2.1]nonan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2,7-dichloro-8-fluoropyrido[4,3-d]pyrimidin-4-yl)-3,9-diazabicyclo[4.2.1]nonane-9-carboxylate

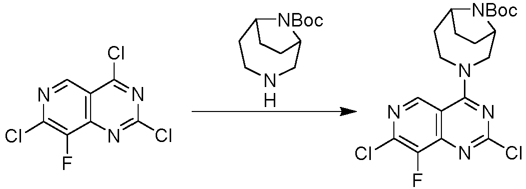

TEA (727 mg, 7.2 mmol) was added to a solution of 2,4,7-trichloro-8-fluoropyrido[4,3-*d*]pyrimidine (600 mg, 2.4 mmol) in THF (6 mL) at -78 °C, and the mixture was stirred for 30 min. *tert-*Butyl 3,9-diazabicyclo[4.2.1]nonane-9-carboxylate (434 mg, 1.9 mmol) was then added, and the mixture was stirred for 4 h. After the reaction was completed, the mixture was naturally warmed to room temperature, H₂O (10 mL) was added, and the mixture was extracted with EtOAc (10 mL × 3). The organic phases were combined, dried over anhydrous sodium sulfate, and concentrated under reduced pressure, and the concentrate was purified by silica gel column chromatography (PE:EtOAc = 5:1) to give *tert*-butyl 3-(2,7-dichloro-8-fluoropyrido[4,3-*d*]pyrimidin-4-yl)-3,9-diazabicyclo[4.2.1]nonane-9-carboxylate.

LC-MS: (ESI, m/z): [M+H]⁺ = 442.1.

### Step 2: preparation of tert-butyl 3-(7-chloro-8-fluoro-2-((1-(hydroxymethyl)cyclopropyl)methoxy)pyrido[4,3-d]pyrimidin-4-yl)-3,9-diazabicyclo[4.2.1]nonane-9-carboxylate

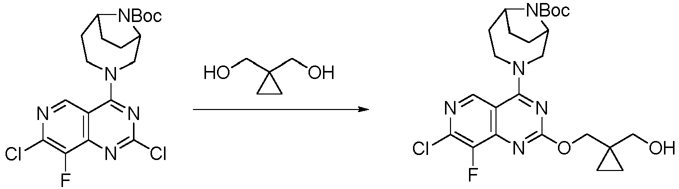

Cs₂CO₃ (782 mg, 2.4 mmol) and DABCO (44.8 mg, 0.4 mmol) were added to a solution of *tert-*butyl 3-(2,7-dichloro-8-fluoropyrido[4,3-*d*]pyrimidin-4-yl)-3,9-diazabicyclo[4.2.1]nonane-9-carboxylate (360 mg, 0.8 mmol) and cyclopropane-1,1-diyldimethanol (163 mg, 1.6 mmol) in acetonitrile (5 mL), and the mixture was stirred at room temperature for 30 min. After the reaction was completed, water (10 mL) was added, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give *tert*-butyl 3-(7-chloro-8-fluoro-2-((1-(hydroxymethyl)cyclopropyl)methoxy)pyrido[4,3-*d*]pyrimidin-4-yl)-3,9-diazabicyclo[4.2.1]nonane-9-carboxylate.

LC-MS: (ESI, m/z): [M+H]⁺ = 508.2.

### Step 3: preparation of tert-butyl 3-(7-(8-ethyl-3-(methoxymethoxy)naphthalen-1-yl)-8-fluoro-2-((1-(hydroxymethyl)cyclopropyl)methoxy)pyrido[4,3-d]pyrimidin-4-yl)-3,9-diazabicyclo[4.2.1]nonane-9-carboxylate

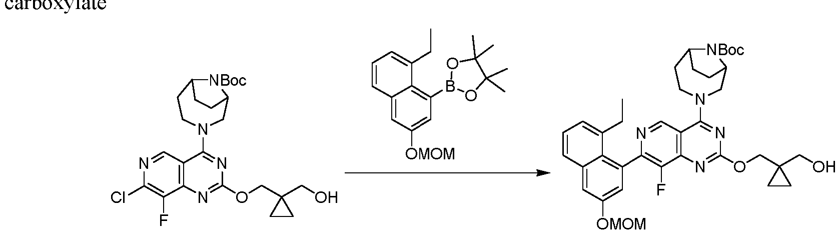

2-(8-Ethyl-3-(methoxymethoxy)naphthalen-1-yl)-4,4,5,5-tetramethyl-1,3,2-dioxaborolane (243 mg, 0.71 mmol), cataCXium A Pd G3 (87 mg, 0.12 mmol), and cesium carbonate (577 mg, 1.77 mmol) were added to a mixed solution of *tert*-butyl 3-(7-chloro-8-fluoro-2-((1-(hydroxymethyl)cyclopropyl)methoxy)pyrido[4,3-*d*]pyrimidin-4-yl)-3,9-diazabicyclo[4.2.1]nonane-9-carboxylate (300 mg, 0.59 mmol) in 1,4-dioxane (6 mL) and H₂O (1 mL). The mixture was reacted at 85 °C for 16 h under nitrogen atmosphere. After the reaction was completed, water (10 mL) was added, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 10:1) to give *tert*-butyl 3-(7-(8-ethyl-3-(methoxymethoxy)naphthalen-1-yl)-8-fluoro-2-((1-(hydroxymethyl)cyclopropyl)methoxy) pyrido[4,3-*d*]pyrimidin-4-yl)-3,9-diazabicyclo[4.2.1]nonane-9-carboxylate.

LC-MS: (ESI, m/z): [M+H]⁺ = 688.4.

### Step 4: preparation of tert-butyl 3-(7-(8-ethyl-3-(methoxymethoxy)naphthalen-1-yl)-8-fluoro-2-((1-formylcyclopropyl)methoxy)pyrido[4,3-d]pyrimidin-4-yl)-3,9-diazabicyclo[4.2.1]nonane-9-carboxylate

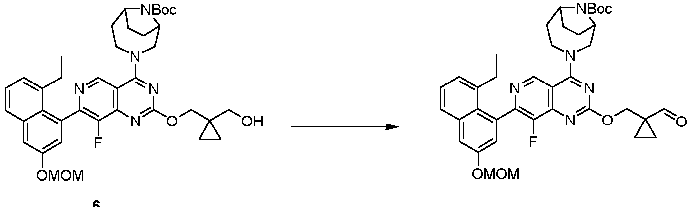

A solution of *tert*-butyl 3-(7-(8-ethyl-3-(methoxymethoxy)naphthalen-1-yl)-8-fluoro-2-((1-(hydroxymethyl)cyclopropyl)methoxy)pyrido[4,3-*d*]pyrimidin-4-yl)-3,9-diazabicyclo[4.2.1]nonane-9-carboxylate (135 mg, 0.20 mmol) in DCM (5 mL) was cooled to 0 °C, and Dess-Martin reagent (170 mg, 0.40 mmol) was added. The mixture was reacted at room temperature for 1 h. After the reaction was completed, a sodium thiosulfate solution (5 mL) and a sodium bicarbonate solution (5 mL) were added to quench the reaction, and the mixture was extracted with dichloromethane (5 mL × 3). The organic phases were combined, dried over anhydrous sodium sulfate, and concentrated under reduced pressure. The concentrate was purified by silica gel column chromatography (DCM:MeOH = 20:1) to give *tert*-butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,9-diazabicyclo[4.2.1]nonane-9-carboxylate.

LC-MS: (ESI, m/z): [M+H]⁺ = 686.4.

### Step 5: preparation of tert-butyl 3-(2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-d]pyrimidin-4-yl)-3,9-diazabicyclo[4.2.1]nonane-9-carboxylate

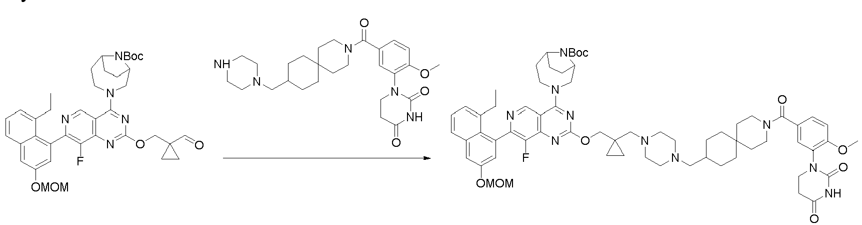

1-(2-Methoxy-5-(9-(piperazin-1-ylmethyl)-3-azaspiro[5.5]undecane-3-carbonyl)phenyl)dihydropyrimidine-2,4(1*H*,3*H*)-dione (100 mg, 0.20 mmol) and AcOH (0.1 mL) were added to a solution of *tert*-butyl 3-(7-(8-ethyl-3-(methoxymethoxy)naphthalen-1-yl)-8-fluoro-2-((1-formylcyclopropyl)methoxy)pyrido[4,3-*d*]pyrimidin-4-yl)-3,9-diazabicyclo[4.2.1]nonane-9-carboxylate (90 mg, 0.13 mmol) in DCM (2 mL), and the mixture was stirred at room temperature for 2 h. The reaction liquid was cooled to 0 °C, and NaBH(OAc)₃ (83 mg, 0.39 mmol) was added. The mixture was reacted at room temperature for another 4 h. The mixture was concentrated and purified by silica gel column chromatography (DCM:MeOH = 100:1 to 20:1) to give *tert-butyl* 3-(2-((1-((4-((3-(3-(2,4-dioxotetrahydropyrimidin-1(2*H*)-yl)-4-methoxybenzoyl)-3-azaspiro [5.5] undecan-9-yl)methyl)piperazin-1-yl)methyl)cyclopropyl)methoxy)-7-(8-ethyl-3-(methoxymethoxy) naphthalen-1-yl)-8-fluoropyrido[4,3-*d*]pyrimidin-4-yl)-3,9-diazabicyclo[4.2.1]nonane-9-carboxylate.

LC-MS: (ESI, m/z): [M+H]⁺ = 1167.8.

### Step 6: preparation of 1-(5-(9-((4-((1-(((4-(3,9-diazabicyclo[4.2.1]nonan-3-yl)-7-(8-ethyl-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

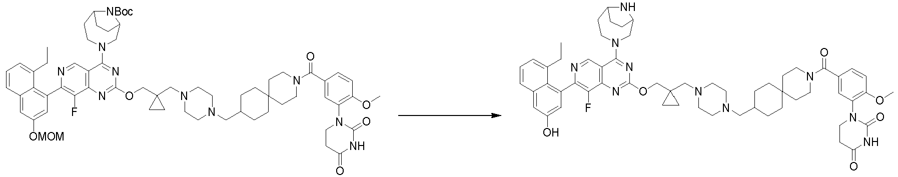

HCl/1,4-dioxane (6 N, 1 mL) was added to a solution of *tert*-butyl 3-(2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-7-(8-ethyl-3-(methoxymethoxy)naphthalen-1-yl)-8-fluoropyrido[4,3-*d*]pyrimidin-4-yl)-3,9-diazabicyclo[4.2.1]nonane-9-carboxylate (50 mg, 0.04 mmol) in 1,4-dioxane (2 mL), and the mixture was reacted at room temperature for 0.5 h. The reaction liquid was concentrated and purified by preparative high-performance liquid chromatography to give 1-(5-(9-((4-((1-(((4-(3,9-diazabicyclo [4.2.1]nonan-3-yl)-7-(8-ethyl-3-hydroxynaphthalen-1-yl)-8-fluoropyrido[4,3-*d*]pyrimidin-2-yl)oxy)methyl) cyclopropyl)methyl)piperazin-1-yl)methyl)-3-azaspiro[5.5]undecane-3-carbonyl)-2-methoxyphenyl) dihydropyrimidine-2,4(1*H*,3*H*)-dione.

LC-MS: (ESI, m/z): [M/2+H]⁺ = 512.3.

¹H NMR (400 MHz, CD₃OD) δ 9.09 (s, 1H), 7.62 (d, *J* = 8.4 Hz, 1H), 7.43 (d, *J* = 8.5 Hz, 1H), 7.39 - 7.32 (m, 2H), 7.28 (d, *J* = 2.8 Hz, 1H), 7.21 - 7.11 (m, 2H), 7.02 (dd, *J* = 2.8, 2.4 Hz, 1H), 4.69 (d, *J* = 14.4 Hz, 1H), 4.56-4.49 (m, 2H), 4.42 - 4.32 (m, 1H), 4.30 - 3.99 (m, 2H), 3.92 (s, 3H), 3.87-3.82 (m, 2H), 3.76 - 3.61 (m, 4H), 3.52 - 3.44 (m, 2H), 2.80 (t, *J =* 6.6 Hz, 2H), 2.53-2.34 (m, 10H), 2.24-2.11 (m, 7H), 1.84-1.74 (m, 3H), 1.67 - 1.56 (m, 4H), 1.55 - 1.47 (m, 2H), 1.42-1.28 (m, 3H), 1.21 - 1.06 (m, 4H), 0.93 - 0.86 (m, 3H), 0.78 - 0.65 (m, 2H), 0.55 - 0.48 (m, 2H).

### Example 89: 1-(5-(9-((4-((1-(((4-(diazabicyclo[3.2.1]octan-3-yl)-7-(3-chloro-2-cyclopropyl-5-hydroxyphenyl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(7-(3-chloro-2-cyclopropyl-5-hydroxyphenyl)-2-((1-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)cyclopropyl)methoxy)-8-fluoropyrido[4,3-d]pyrimidin-4-yl)-diazabicyclo[3.2.1]octane-8-carboxylate

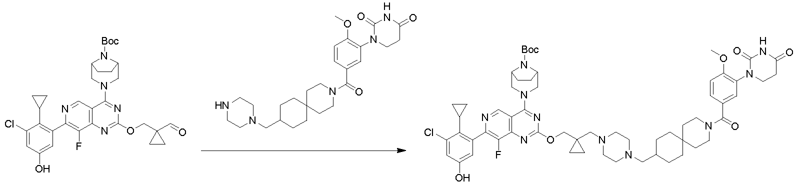

Tetraisopropyl titanate (205 mg, 721.05 µmol) and glacial acetic acid (0.1 mL) were added to a solution of *tert*-butyl 3-(7-(3-chloro-2-cyclopropyl-5-hydroxyphenyl)-8-fluoro-2-((1-formylcyclopropyl)methoxy) pyrido[4,3-*d*]pyrimidin-4-yl)-diazabicyclo[3.2.1]octane-8-carboxylate (90 mg, 144.21 µmol) and 1-(2-methoxy-5-(9-(piperazin-1-ylmethyl)-3-azaspiro[5.5]undecane-3-carbonyl)phenyl)dihydropyrimidine-2,4(1*H*,3*H*)-dione (108 mg, 216.31 µmol) in dichloromethane (10 mL) at room temperature, and the mixture was stirred at 25 °C for 3 h. Sodium triacetoxyborohydride (92 mg, 432.64 µmol) was added, and the mixture was reacted at 25 °C for another 18 h. Methanol (10 mL) was added to the reaction liquid to quench the reaction, and the mixture was concentrated under reduced pressure at 40 °C. The resulting crude product was purified by a preparative thin-layer chromatography plate (dichloromethane:methanol = 10:1) to give *tert-*butyl 3-(7-(3-chloro-2-cyclopropyl-5-hydroxyphenyl)-2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-diazabicyclo[3.2.1]octane-8-carboxylate.

LC-MS: (ESI, m/z): [M+H]⁺ = 1105.5.

### Step 2: preparation of 1-(5-(9-((4-((1-(((4-(diazabicyclo[3.2.1]octan-3-yl)-7-(3-chloro-2-cyclopropyl-5-hydroxyphenyl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-methoxyphenyl)dihydropyrimidine-2,4(1H,3H)-dione

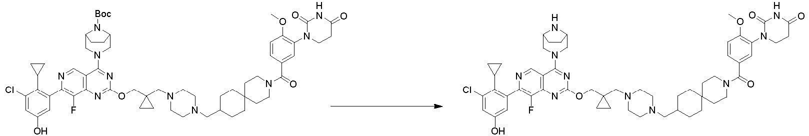

A hydrogen chloride/1,4-dioxane solution (4.0 M, 5 mL) was added to a solution of a crude product of *tert*-butyl 3-(7-(3-chloro-2-cyclopropyl-5-hydroxyphenyl)-2-((1-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)cyclopropyl)methoxy)-8-fluoropyrido[4,3-*d*]pyrimidin-4-yl)-diazabicyclo[3.2.1]octane-8-carboxylate (99.2 mg, 89.71 µmol) in 1,4-dioxane (2 mL) at room temperature, and the mixture was reacted at room temperature for 1 h. After the reaction was completed, the mixture was concentrated under reduced pressure at 30 °C, and methanol (5 mL) and a solution of ammonia in methanol (1 mL) were added to the residue for dissolution. The mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((1-(((4-(diazabicyclo[3.2.1]octan-3-yl)-7-(3-chloro-2-cyclopropyl-5-hydroxyphenyl)-8-fluoropyrido[4,3-*d*]pyrimidin-2-yl)oxy)methyl)cyclopropyl) methyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 503.4.

¹H NMR (400 MHz, DMSO-*d*₆) δ 10.32 (s, 1H), 9.97 (s, 1H), 9.07 (s, 1H), 7.36 (dd, *J* = 8.4 Hz, 2.0 Hz, 1H), 7.31 (d*, J* = 2.0 Hz, 1H), 7.14 (d*, J* = 8.8 Hz, 1H), 6.94 (d*, J* = 2.4 Hz, 1H), 6.77 (d*, J* = 2.4 Hz, 1H), 4.40-4.37 (m, 2H), 4.28 (s, 2H), 3.84 (s, 3H), 3.61-3.52 (m, 10H), 2.69-2.66 (m, 2H), 2.50-2.10 (m, 10H), 2.03-2.01 (m, 2H), 1.76-1.72 (m, 1H), 1.72-0.75 (m, 20H), 0.62-0.57 (m, 4H), 0.40-0.38 (m, 2H).

### Example 90: 1-(5-(9-((4-((R)-3-((4-(diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-diazabicyclo[3.2.1]octane-8-carboxylate

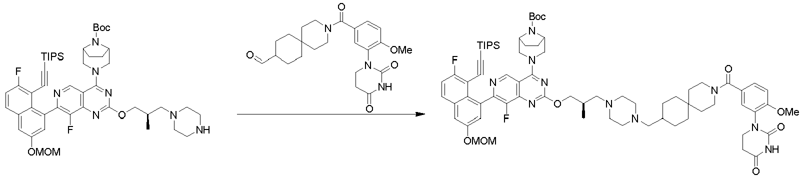

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate (140 mg, 0.14 mmol) and 3-(3-(2,4-dioxotetrahydropyrimidin-1(2*H*)-yl)-4-methoxybenzoyl)-3-azaspiro[5.5]undecane-9-carbaldehyde (91 mg, 0.21 mmol) in DCM/AcOH (1.5 mL/0.1 mL), and the mixture was stirred at 30 °C for 2 h. NaBH(OAc)₃ (90 mg, 0.42 mmol) was added to the above mixture in an ice bath, and the resulting mixture was stirred for 2 h. The mixture was concentrated and purified by silica gel column chromatography (DCM:MeOH = 100:1 to 10:1) to give *tert*-butyl 3-(2-((R)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido [4,3-*d*]pyrimidin-4-yl)-diazabicyclo[3.2.1]octane-8-carboxylate.

LC-MS: (ESI, m/z): [M/2+H]⁺ = 657.0.

### Step 2: preparation of 1-(5-(9-((4-((R)-3-((4-(diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

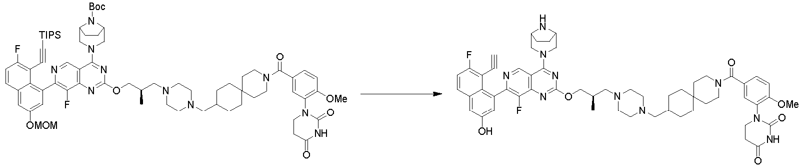

Cesium fluoride (70 mg, 0.46 mmol) was added to a stirred solution of *tert*-butyl 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-diazabicyclo[3.2.1]octane-8-carboxylate (120 mg, 0.092 mmol) in DMF (1 mL), and the mixture was stirred at room temperature for 30 min. Water (10 mL) was added to the reaction liquid, and the mixture was extracted with ethyl acetate (10 mL × 2). The organic phases were combined, washed with a saturated sodium chloride solution, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by high-performance liquid chromatography to give 1-(5-(9-((4-((*R*)-3-((4-(diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1011.5.

¹H NMR (400 MHz, DMSO-*d*₆) δ 10.32 (s, 1H), 10.14 (s, 1H), 9.03 (s, 1H), 7.97 (dd, *J* = 9.2, 5.9 Hz, 1H), 7.49 - 7.43 (m, 1H), 7.40 - 7.34 (m, 2H), 7.31 (d, *J* = 2.0 Hz, 1H), 7.19 - 7.12 (m, 2H), 4.51 - 4.40 (m, 2H), 4.35 - 4.26 (m, 1H), 4.12 - 4.01 (m, 1H), 3.93 (s, 1H), 3.84 (s, 3H), 3.66 - 3.37 (m, 10H), 2.68 (d, *J=* 6.4 Hz, 2H), 2.45 - 1.97 (m, 14H), 1.71 - 1.60 (m, 6H), 1.57 - 1.35 (m, 5H), 1.33 - 1.31 (m, 2H), 1.15 - 0.91 (m, 7H).

### Example 100: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

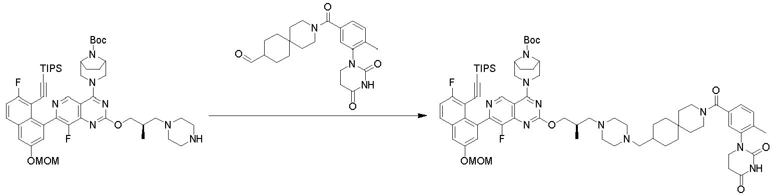

Tetraisopropyl titanate (189 mg, 666.50 µmol) and glacial acetic acid (0.2 mL) were added to a stirred solution of *tert*-butyl 3-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)pyrido[4,3-*d*]pyrimidin-4-yl)-3,8-diazabicyclo[3.2.1]octane-8-carboxylate (120 mg, 133.30 µmol) and 3-(3-(2,4-dioxotetrahydropyrimidin-1(2*H*)-yl)-4-methylbenzoyl)-3-azaspiro[5.5]undecane-9-carbaldehyde (110 mg, 266.61 µmol) in dichloromethane (10 mL) at room temperature, and the mixture was stirred at 25 °C for 2 h. Then sodium triacetoxyborohydride (85 mg, 399.90 µmol) was added, and the mixture was reacted at 25 °C for 16 h. Methanol (5 mL) was added to the reaction liquid, and the mixture was concentrated under reduced pressure at 40 °C. The resulting crude product was purified by a preparative thin-layer chromatography plate (dichloromethane:methanol = 10:1) to give *tert*-butyl 3-(2-((*R*)-3-(4-((3-(3-(2,4-dioxotetrahydropyrimidin-1(2*H*)-yl)-4-methylbenzoyl)-3-azaspiro[5.5] undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 648.4.

### Step 2: preparation of tert-butyl 3-(2-((R)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-d]pyrimidin-4-yl)-3,8-diazabicyclo [3.2.1]octane-8-carboxylate

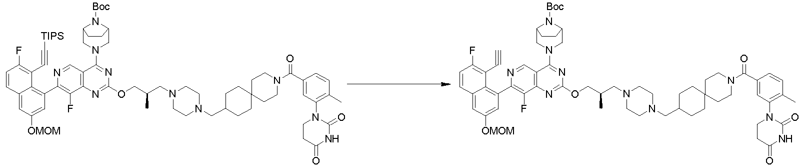

Cesium fluoride (127 mg, 833.50 µmol) was added to a solution of *tert*-butyl 3-(2-((R)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (108.0 mg, 83.35 µmol) in *N,N*-dimethylformamide (5 mL) at room temperature, and the mixture was reacted at 25 °C for 2 h under nitrogen atmosphere. After the reaction was completed, the reaction liquid was poured into water (60 mL), and the mixture was extracted with ethyl acetate (20 mL × 3). The organic phase was washed with water (60 mL) and saturated brine (60 mL), dried over anhydrous sodium sulfate, filtered, and concentrated under reduced pressure to give a crude product of *tert*-butyl 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-*d*]pyrimidin-4-yl)-3,8-diazabicyclo
[3.2.1]octane-8-carboxylate.

LC-MS: (ESI, m/z): [M+H]⁺ = 1139.5.

### Step 3: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

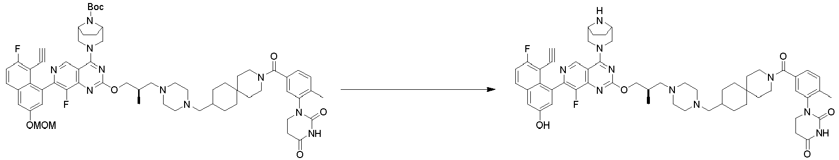

A hydrogen chloride/1,4-dioxane solution (4.0 M, 3 mL) was added to a solution of the crude product of *tert*-butyl 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (67.1 mg, 58.89 µmol) in 1,4-dioxane (2 mL) at room temperature, and the mixture was reacted at room temperature for 1.5 h. After the reaction was completed, the mixture was concentrated to dryness under reduced pressure at 30 °C, and a solution of dilute ammonia in methanol (5 mL) was added. Then the mixture was concentrated to dryness under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl) piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 995.5.

¹H NMR (400 MHz, DMSO-*d*₆) δ 10.36 (s, 1H), 9.02 (s, 1H), 7.97 (dd, *J* = 9.2 Hz, 6.0 Hz, 1H), 7.45 (t, *J* = 8.8 Hz, 1H), 7.38 (d, *J* = 2.4 Hz, 1H), 7.33 (d, *J* = 8.0 Hz, 1H), 7.30 (s, 1H), 7.23 (d, *J* = 8.4 Hz, 1H), 7.17 (d, *J* = 3.6 Hz, 1H), 4.47-4.43 (m, 2H), 4.32-4.29 (m, 1H), 4.10-4.03 (m, 1H), 3.92 (s, 1H), 3.83-3.76 (m, 2H), 3.53-3.50 (m, 6H), 2.81-2.65 (m, 4H), 2.33-2.29 (m, 6H), 2.21 (m, 3H), 2.16-1.99 (m, 6H), 1.70-1.60 (m, 6H), 1.55-1.35 (m, 6H), 1.35-1.20 (m, 4H), 1.20-1.10 (m, 4H), 0.97-0.96 (m, 3H).

### Example 102: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 3-(2-((R)-3-(4-((3-(4-chloro-3-(2,4-dioxotetrahydropyrimidin-1(2H)-yl)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

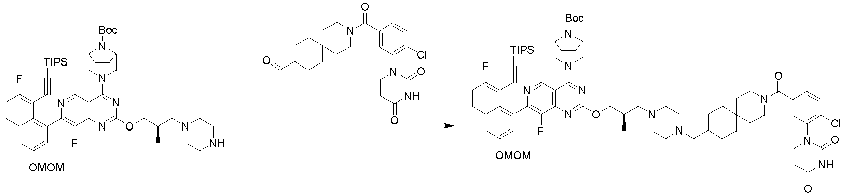

3-(4-Chloro-3-(2,4-dioxotetrahydropyrimidin-1(2*H*)-yl)benzoyl)-3-azaspiro[5.5]undecane-9-carbaldehyde (94 mg, 0.22 mmol) and tetraisopropyl titanate (205 mg, 0.72 mmol) were added to a mixed solution of *tert*-butyl 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate (130 mg, 0.14 mmol) in dichloromethane (2 mL) and acetic acid (0.1 mL), and the mixture was stirred at 25 °C for 2 h. NaBH(OAc)₃ (92 mg, 0.43 mmol) was then added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum under reduced pressure. The crude product was purified by silica gel column chromatography (DCM:MeOH = 10:1) to give *tert*-butyl 3-(2-((*R*)-3-(4-((3-(4-chloro-3-(2,4-dioxotetrahydropyrimidin-1(2*H*)-yl)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 658.4.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-chlorophenyl)dihydropyrimidine-2,4(1H,3H)-dione

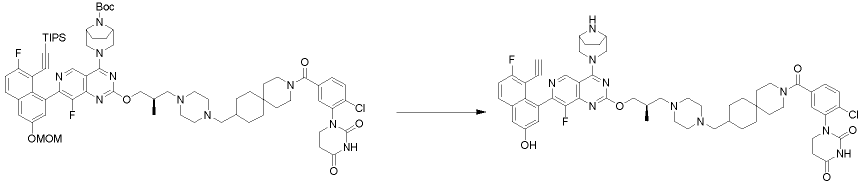

*tert*-Butyl 3-(2-((*R*)-3-(4-((3-(4-chloro-3-(2,4-dioxotetrahydropyrimidin-1(2*H*)-yl)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (160 mg, 0.12 mmol) was dissolved in DMF (2 mL), and CsF (92 mg, 0.61 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 2 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺ = 1015.4.

¹H NMR (400 MHz, DMSO-*d*₆) δ 10.50 (s, 1H), 10.15 (brs, 1H), 9.02 (s, 1H), 7.97 (dd, *J =* 9.2, 5.9 Hz, 1H), 7.63 (d, *J* = 8.2 Hz, 1H), 7.54 (d, *J* = 1.9 Hz, 1H), 7.46 (t, *J* = 9.0 Hz, 1H), 7.41 - 7.35 (m, 2H), 7.17 (d, *J* = 2.5 Hz, 1H), 4.53 - 4.36 (m, 2H), 4.36 - 4.23 (m, 1H), 4.14 - 3.99 (m, 1H), 3.92 (s, 1H), 3.83 - 3.69 (m, 1H), 3.66 - 3.49 (m, 7H), 2.77 - 2.70 (m, 2H), 2.41 - 1.96 (m, 14H), 1.72 - 1.61 (m, 6H), 1.54 - 1.21 (m, 8H), 1.10 - 0.90 (m, 7H).

### Example 103: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-(methyl-d3)phenyl)dihydropyrimidine-2,4(1H,3H)-dione

### Step 1: preparation of tert-butyl 3-(2-((1-((4-benzylpiperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

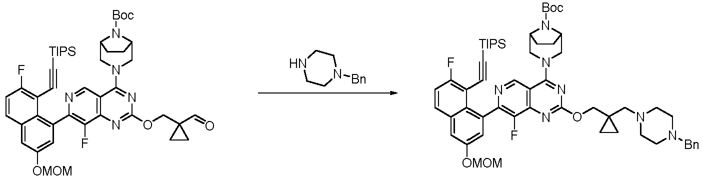

1-Benzylpiperazine (150 mg, 0.72 mmol) and tetraisopropyl titanate (506 mg, 1.8 mmol) were added to a mixed solution of *tert*-butyl 3-(8-fluoro-7-(7-fluoro-3-(methoxymethoxy)-8-((triisopropylsilyl)ethynyl) naphthalen-1-yl)-2-((1-formylcyclopropyl)methoxy)pyrido[4,3-*d*]pyrimidin-4-yl)-3,8-diazabicyclo[3.2.1]octane-8-carboxylate (300 mg, 0.36 mmol) in dichloromethane (6 mL), methanol (3 mL), and acetic acid (0.1 mL), and the mixture was stirred at 25 °C for 2 h. NaBH(OAc)₃ (227 mg, 1.07 mmol) was then added at 0 °C, and the mixture was stirred at 25 °C for 1 h. After the reaction was completed, H₂O (15 mL) was added, and the mixture was extracted with ethyl acetate (15 mL × 3). The combined organic phase was dried over anhydrous sodium sulfate, filtered, and concentrated in vacuum under reduced pressure. The crude product was purified by silica gel column chromatography (DCM:MeOH = 10:1) to give *tert-*butyl 3-(2-((1-((4-benzylpiperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 501.9.

### Step 2: preparation of tert-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate

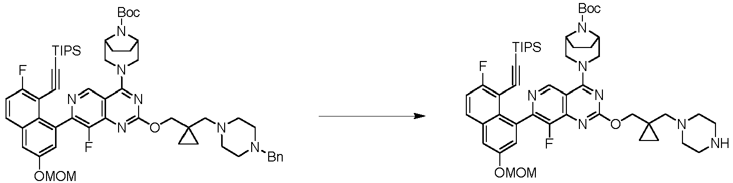

*tert*-Butyl 3-(2-((1-((4-benzylpiperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (180 mg, 0.18 mmol) was dissolved in DCM (5 mL), and then 1-chloroethyl chloroformate (29 mg, 0.36 mmol) and DIEA (26 mg, 0.36 mmol) were added. The mixture was reacted at room temperature for 30 min, and the reaction liquid was concentrated under reduced pressure. MeOH (5 mL) was added to the concentrate, and the mixture was heated to 50 °C for 30 min and then directly concentrated under reduced pressure. The resulting crude product was purified by silica gel column chromatography (DCM:MeOH = 200:1 to 20:1) to give *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-*d*]pyrimidin-4-yl)-3,8-diazabicyclo[3.2.1]octane-8-carboxylate.

### Step 3: preparation of tert-butyl 3-(2-((1-((4-((3-(3-(2,4-dioxotetrahydropyrimidin-1(2H)-yl)-4-(methyl-d3)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

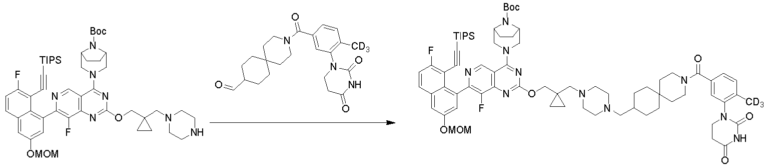

3-(3-(2,4-Dioxotetrahydropyrimidin-1(2*H*)-yl)-4-(methyl-d3)benzoyl)-3-azaspiro[5.5]undecane-9-carbaldehyde (47 mg, 0.11 mmol) and tetraisopropyl titanate (125 mg, 0.44 mmol) were added to a mixed solution of *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido [4,3-*d*]pyrimidin-4-yl)-3,8-diazabicyclo[3.2.1]octane-8-carboxylate (80 mg, 0.088 mmol) in dichloromethane (1 mL) and acetic acid (0.1 mL), and the mixture was stirred at 25 °C for 2 h. NaBH(OAc)₃ (56 mg, 0.26 mmol) was then added at 0 °C, and the mixture was stirred at 25 °C for 1 h. After the reaction was completed, H₂O (5 mL) was added to the reaction liquid, and the mixture was extracted with ethyl acetate (5 mL × 3). The combined organic phase was dried over anhydrous sodium sulfate, filtered, and concentrated in vacuum under reduced pressure. The crude product was purified by silica gel column chromatography (DCM:MeOH = 10:1) to give *tert*-butyl 3-(2-((1-((4-((3-(3-(2,4-dioxotetrahydropyrimidin-1(2*H*)-yl)-4-(methyl-d3)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3*-d*]pyrimidin-4-yl)-3,8-diazabicyclo[3.2.1]octane-8-carboxylate.

LC-MS: (ESI, m/z): [M/2+H]⁺ = 656.0.

### Step 4: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-(methyl-d3)phenyl)dihydropyrimidine-2,4(1H,3H)-dione

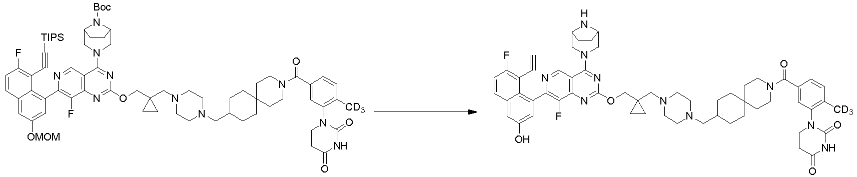

*tert*-Butyl 3-(2-((1-((4-((3-(3-(2,4-dioxotetrahydropyrimidin-1(2*H*)-yl)-4-(methyl-d3)benzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (52 mg, 0.04 mmol) was dissolved in DMF (1 mL), and CsF (30 mg, 0.2 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-(methyl-d3)phenyl)dihydropyrimidine-2,4(1*H*,3*H*)-dione.

LC-MS: (ESI, m/z): [M+H]⁺ = 1010.4.

¹H NMR (400 MHz, CD₃OD) δ 8.99 (s, 1H), 7.85 (dd, *J* = 9.1, 5.7 Hz, 1H), 7.40 (d, *J* = 7.8 Hz, 1H), 7.35 - 7.27 (m, 4H), 7.20 (d, *J* = 2.5 Hz, 1H), 4.67 - 4.30 (m, 7H), 3.90 - 3.80 (m, 1H), 3.72 - 3.64 (m, 6H), 3.46 - 3.36 (m, 2H), 3.35 (s, 1H), 2.92 - 2.80 (m, 2H), 2.58 - 2.36 (m, 8H), 2.18 - 2.10 (m, 2H), 1.90 - 1.72 (m, 6H), 1.61 - 1.29 (m, 8H), 1.18 - 1.07 (m, 3H), 0.75 - 0.65 (m, 2H), 0.53 - 0.45 (m, 2H).

Example 104: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-4-fluoro-2-methylphenyl)dihydropyrimidine-2,4(1*H*,3*H*)-dione

### Step 1: preparation of tert-butyl 3-(2-((1-((4-((3-(5-(2,4-dioxotetrahydropyrimidin-1(2H)-yl)-2-fluoro-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

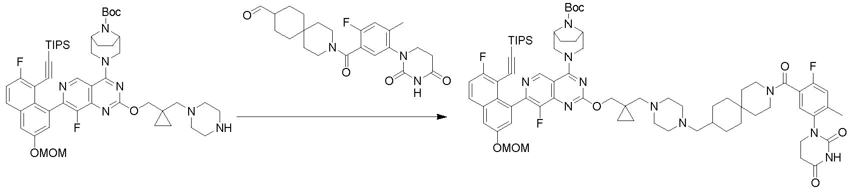

3-(5-(2,4-Dioxotetrahydropyrimidin-1(2*H*)-yl)-2-fluoro-4-methylbenzoyl)-3-azaspiro[5.5]undecane-9-carbaldehyde (39 mg, 0.09 mmol) and tetraisopropyl titanate (85 mg, 0.3 mmol) were added to a mixed solution of *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-*d*]pyrimidin-4-yl)-3,8-diazabicyclo[3.2.1]octane-8-carboxylate (55 mg, 0.06 mmol) in dichloromethane (2 mL) and acetic acid (0.1 mL), and the mixture was stirred at 25 °C for 2 h. NaBH(OAc)₃ (39 mg, 0.18 mmol) was then added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum under reduced pressure. The crude product was purified by silica gel column chromatography (DCM:MeOH = 10:1) to give *tert*-butyl 3-(2-((1-((4-((3-(5-(2,4-dioxotetrahydropyrimidin-1(2*H*)-yl)-2-fluoro-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 663.5.

### Step 2: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-4-fluoro-2-methylphenyl)dihydropyrimidine-2,4(1H,3H)-dione

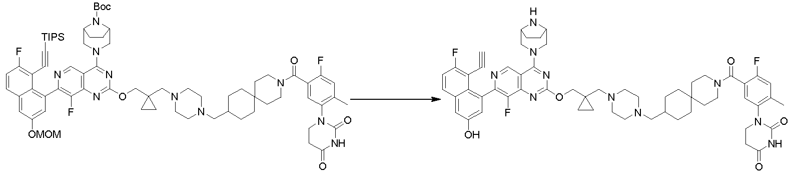

*tert*-Butyl 3-(2-((1-((4-((3-(5-(2,4-dioxotetrahydropyrimidin-1(2*H*)-yl)-2-fluoro-4-methylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (30 mg, 0.023 mmol) was dissolved in DMF (1 mL), and CsF (17 mg, 0.11 mmol) was added. The mixture was reacted at room temperature for 0.5 h. After the reaction was completed, the mixture was diluted with H₂O (5 mL) and extracted with ethyl acetate (5 mL × 3). The combined organic phase was dried over Na₂SO₄, filtered, and concentrated under reduced pressure. Hydrochloric acid/1,4-dioxane (8 M, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and then extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-4-fluoro-2-methylphenyl)dihydropyrimidine-2,4(1*H*,3*H*)-dione.

LC-MS: (ESI, m/z): [M+H]⁺ = 1025.5.

¹H NMR (400 MHz, CD₃OD) δ 8.99 (s, 1H), 7.88 - 7.82 (m, 1H), 7.36- 7.28 (m, 3H), 7.22 - 7.14 (m, 2H), 4.64 - 4.52 (m, 10H), 3.75 - 3.65 (m, 6H), 3.35 (s, 1H), 2.91 - 2.80 (m, 2H), 2.62 - 2.39 (m, 8H), 2.30 (s, 3H), 2.21 - 2.15 (m, 2H), 1.89 - 1.68 (m, 6H), 1.60 - 1.47 (m, 4H), 1.33 - 1.29 (m, 4H), 1.20 - 1.10 (m, 3H), 0.75 - 0.65 (m, 2H), 0.54 - 0.45 (m, 2H).

### Example 105: 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1H,3H)-dione

### Step 1: preparation of 1-(4-benzylpiperazin-1-yl)-3-((tert-butyldimethylsilyl)oxy)propan-2-ol

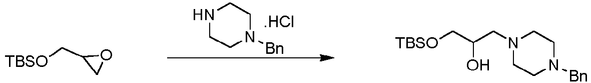

*tert*-Butyldimethyl(oxiran-2-ylmethoxy)silane (9 g, 47.9 mmol) was dissolved in ethanol (50 mL), and 1-benzylpiperazine hydrochloride (11.2 g, 52.7 mmol) and potassium carbonate (13.2 g, 95.8 mmol) were added. The mixture was reacted at 100 °C for 5 h. After the reaction was completed, the mixture was filtered, and the filtrate was concentrated and purified by silica gel column chromatography (PE:EtOAc = 5:1) to give 1-(4-benzylpiperazin-1-yl)-3-((*tert*-butyldimethylsilyl)oxy)propan-2-ol.

¹H NMR (400 MHz, CDCl₃) 7.27 - 7.23 (m, 4H), 7.23 - 7.26 (m, 1H), 3.79 - 3.71 (m, 1H), 3.64 - 3.57 (m, 2H), 3.53 - 3.47 (m, 2H), 3.27 - 3.17 (m, 1H), 2.67 - 2.41 (m, 8H), 0.89 (s, 9H), 0.06 (s, 6h).

LC-MS: (ESI, m/z): [M+H]⁺ = 365.2.

### Step 2: preparation of 1-benzyl-4-(3-((tert-butyldimethylsilyl)oxy)-2-methoxypropyl)piperazine

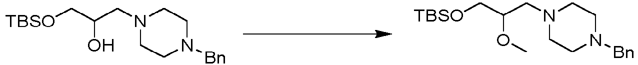

1-(4-Benzylpiperazin-1-yl)-3-((*tert*-butyldimethylsilyl)oxy)propan-2-ol (5.0 g, 13.7 mmol) was dissolved in THF (50 mL), and NaH (0.82 g, 20.6 mmol) was added at 0-5 °C. The mixture was reacted at room temperature for 30 min and then cooled to 0-5 °C. Iodomethane (2.44 g, 17.2 mmol) was added, and the mixture was reacted at room temperature for 5 h. After the reaction was completed, a saturated aqueous ammonium chloride solution was added to quench the reaction, and the mixture was extracted with ethyl acetate (50 mL × 2). The organic phase was washed with a saturated sodium chloride solution, dried over anhydrous sodium sulfate, and filtered, and the filtrate was concentrated and purified by silica gel column chromatography (PE:EtOAc = 8:1) to give 1-benzyl-4-(3-((*tert*-butyldimethylsilyl)oxy)-2-methoxypropyl)piperazine.

LC-MS: (ESI, m/z): [M+H]⁺ = 379.3.

### Step 3: preparation of 3-(4-benzylpiperazin-1-yl)-2-methoxypropan-1-ol

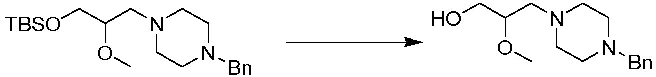

1-Benzyl-4-(3-((*tert*-butyldimethylsilyl)oxy)-2-methoxypropyl)piperazine (1.60 g, 4.23 mmol) was dissolved in methanol (10 mL), and concentrated hydrochloric acid (3 mL) was added at 0 °C in an ice bath. The mixture was reacted for 2 h. After the reaction was completed, a saturated sodium carbonate solution was added to adjust the pH > 7, and then the mixture was extracted with ethyl acetate (20 mL × 3). The organic phases were combined, washed with a saturated NaCl solution, dried over anhydrous Na₂SO₄, and filtered, and the filtrate was concentrated. The resulting crude product was purified by silica gel column chromatography (PE:EA = 4:1) to give 3-(4-benzylpiperazin-1-yl)-2-methoxypropan-1-ol.

LC-MS: (ESI, m/z): [M+H]⁺ = 265.2.

### Step 4: preparation of tert-butyl 3-(2-(3-(4-benzylpiperazin-1-yl)-2-methoxypropoxy)-7-chloro-8-fluoropyrido[4,3-d]pyrimidin-4-yl)-3,8-diazabicyclo[3.2.1]octane-8-carboxylate

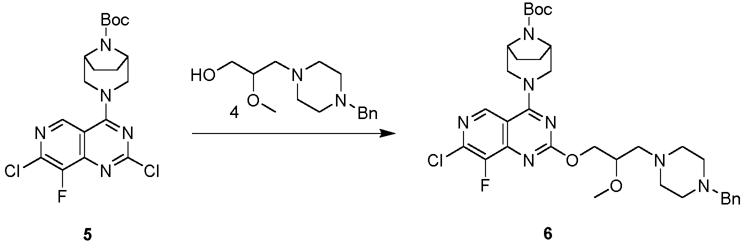

*tert*-Butyl 3-(2,7-dichloro-8-fluoropyrido[4,3-*d*]pyrimidin-4-yl)-3,8-diazabicyclo[3.2.1]octane-8-carboxylate (750 mg, 1.75 mmol) and 3-(4-benzylpiperazin-1-yl)-2-methoxypropan-1-ol (555 mg, 2.1 mmol) were dissolved in ACN (10 mL), and Cs₂CO₃ (5.25 g, 1.71 mmol) and DABCO (39 mg, 0.35 mmol) were added. The mixture was reacted at room temperature for 1 h. After the reaction was completed, the mixture was filtered, and the filter cake was washed twice with dichloromethane. The filtrate was concentrated, and the resulting concentrate was purified by silica gel column chromatography (PE:EA = 2:1) to give *tert*-butyl 3-(2-(3-(4-benzylpiperazin-1-yl)-2-methoxypropoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 656.3.

### Step 5: preparation of tert-butyl 3-(2-(3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

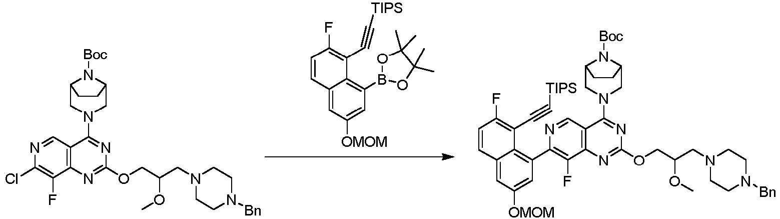

Cata CXium Pd G₃ (111 mg, 0.152 mmol) and cesium carbonate (743 mg, 2.28 mmol) were added to a mixed solution of *tert*-butyl 3-(2-(3-(4-benzylpiperazin-1-yl)-2-methoxypropoxy)-7-chloro-8-fluoropyrido[4,3-*d*]pyrimidin-4-yl)-3,8-diazabicyclo[3.2.1]octane-8-carboxylate (500 mg, 0.76 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (467 mg, 0.912 mmol) in 1,4-dioxane (6 mL) and H₂O (1 mL). The mixture was reacted at 85 °C for 16 h under nitrogen atmosphere. Water (20 mL) was added to the reaction liquid, and the mixture was extracted with ethyl acetate (30 mL × 3). The organic phases were combined, dried over anhydrous sodium sulfate, and concentrated under reduced pressure. The concentrate was purified by silica gel column chromatography (DCM:MeOH = 5%) to give *tert*-butyl 3-(2-(3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 1006.5.

### Step 6: preparation of tert-butyl 3-(8-fluoro-7-(7-fluoro-3-(methoxymethoxy)-8-((triisopropylsilyl)ethynyl)naphthalen-1-yl)-2-(2-methoxy-3-(piperazin-1-yl)propoxy)pyrido[4,3-d]pyrimidin-4-yl)-3,8-diazabicyclo[3.2.1]octane-8-carboxylate

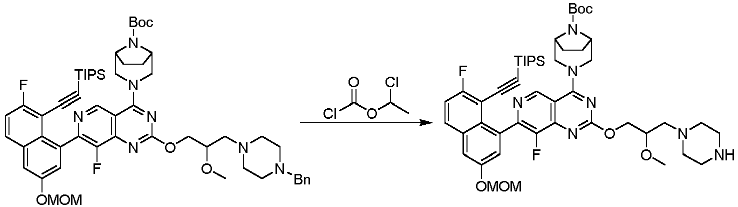

*tert*-Butyl 3-(2-(3-(4-benzylpiperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (500 mg, 0.5 mmol) was dissolved in DCM (5 mL), and 1-chloroethyl carbonochloridate (194 mg,1.5 mmol) and DIEA (129 mg,1.0 mmol) were added. The mixture was reacted at room temperature for 1 h. The reaction liquid was concentrated under reduced pressure, MeOH (5 mL) was added to the concentrate, and the mixture was heated to 50 °C and reacted for 50 min. After the reaction was completed, the mixture was concentrated under reduced pressure, and the concentrate was purified by silica gel column chromatography (DCM:MeOH (NH₃) = 15:1) to give *tert*-butyl 3-(8-fluoro-7-(7-fluoro-3-(methoxymethoxy)-8-((triisopropylsilyl)ethynyl)naphthalen-1-yl)-2-(2-methoxy-3-(piperazin-1-yl)propoxy)pyrido[4,3-*d*]pyrimidin-4-yl)-3,8-diazabicyclo[3.2.1]octane-8-carboxylate.

LC-MS: (ESI, m/z): [M/2+H]⁺ = 458.8.

### Step 7: preparation of tert-butyl 3-(2-(3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

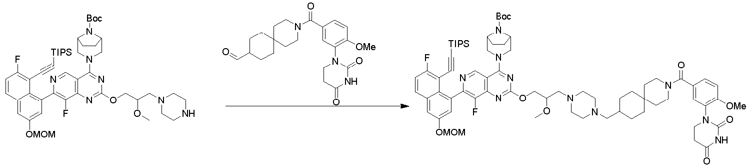

Tetraisopropyl titanate (0.5 mL) was added to a solution of *tert*-butyl 3-(8-fluoro-7-(7-fluoro-3-(methoxymethoxy)-8-((triisopropylsilyl)ethynyl)naphthalen-1-yl)-2-(2-methoxy-3-(piperazin-1-yl)propoxy)pyrido[4,3-*d*]pyrimidin-4-yl)-3,8-diazabicyclo[3.2.1]octane-8-carboxylate (150 mg, 0.164 mmol) and 3-(3-(2,4-dioxotetrahydropyrimidin-1(2*H*)-yl)-4-methoxybenzoyl)-3-azaspiro[5.5]undecane-9-carbaldehyde (84 mg, 0.197 mmol) in DCM/AcOH (2 mL/0.1 mL), and the mixture was stirred at 30 °C for 2 h. NaBH(OAc)₃ (109 mg, 0.492 mmol) was added to the mixture in an ice bath, and the resulting mixture was stirred for 2 h. The mixture was concentrated and purified by silica gel column chromatography (DCM:MeOH = 10:1) to give *tert*-butyl 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 664.4.

### Step 8: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1H,3H)-dione

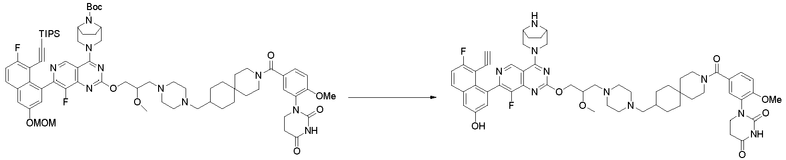

Cesium fluoride (92 mg, 0.60 mmol) was added to a solution of *tert*-butyl 3-(2-(3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (160 mg, 0.12 mmol) in DMF (2 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. An aqueous Na₂CO₃ solution was added to the reaction liquid, and the mixture was adjusted to pH > 7 and extracted with EtOAc (20 mL × 2). The organic phase was washed with brine, dried over anhydrous Na₂SO₄, and concentrated. The crude product was purified by preparative high-performance liquid chromatography to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1027.4.

¹H NMR (400 MHz, DMSO-*d*₆) δ 10.32 (s, 1H), 10.14 (s, 1H), 9.04 (s, 1H), 7.97 (dd, *J* = 9.2, 5.9 Hz, 1H), 7.46 (t, *J=* 9.0 Hz, 1H), 7.41 - 7.27(m, 3H), 7.21 - 7.10 (m, 2H), 4.59 - 4.41 (m, 2H), 4.36 - 4.26(m, 2H), 3.92 (s, 1H), 3.84 (s, 3H), 3.76 - 3.38 (m, 10H), 3.36 (d, *J* = 1.9 Hz, 3H), 2.77 - 2.60(m, 3H), 2.49 - 2.14 (m, 11H), 2.09 - 1.98 (m, 2H), 1.74 -1.58(s, 6H), 1.57 - 1.19 (m, 7H), 1.15 - 0.88 (m, 4H).

### Example 106: 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-2-oxa-9-azaspiro[5.5]undecane-9-carbonyl)-2-methylphenyl)dihydropyrimidine-2,4(1H,3H)-dione

### Step 1: preparation of tert-butyl 3-(hydroxymethyl)-2-oxa-9-azaspiro[5.5]undecane-9-carboxylate

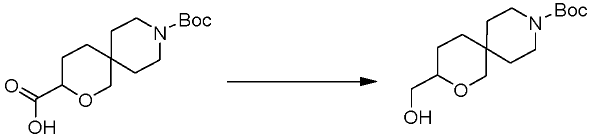

BH₃/THF (0.23 mL, 2.34 mmol, 10 M/DMSO) was added dropwise to a solution of 9-(*tert-*butoxycarbonyl)-2-oxa-9-azaspiro[5.5]undecane-3-carboxylic acid (350 mg, 1.17 mmol) in THF (4 mL) at 0 °C. The mixture was reacted at room temperature for 2 h. The reaction liquid was cooled to 0-5 °C, and methanol was added to quench the reaction. The reaction liquid was stirred for 10 min and concentrated to give *tert-butyl* 3-(hydroxymethyl)-2-oxa-9-azaspiro[5.5]undecane-9-carboxylate.

### Step 2: preparation of (2-oxa-9-azaspiro[5.5]undecan-3-yl)methanol

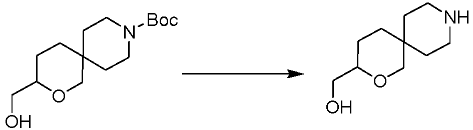

*tert*-Butyl 3-(hydroxymethyl)-2-oxa-9-azaspiro[5.5]undecane-9-carboxylate (300 mg, 1.05 mmol) was dissolved in DCM (3 mL), and TFA (1.5 mL) was added. The mixture was stirred at room temperature for 1 h and concentrated to give (2-oxa-9-azaspiro[5.5]undecan-3-yl)methanol. The crude product was directly used in the next step without further purification.

### Step 3: preparation of 1-(5-(3-(hydroxymethyl)-2-oxa-9-azaspiro[5.5]undecane-9-carbonyl)-2-methylphenyl)dihydropyrimidine-2,4(1H,3H)-dione

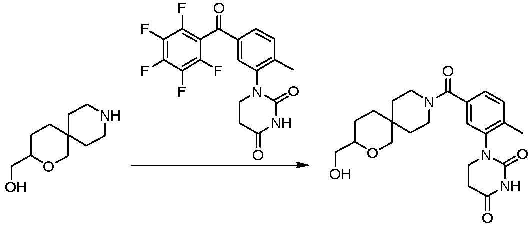

DIEA (1.08 g, 8.4 mmol) and 1-(2-methyl-5-(pentafluorobenzoyl)phenyl)dihydropyrimidine-2,4(1*H*,3*H*)-dione (436 mg, 1.05 mmol) were added to a solution of (2-oxa-9-azaspiro[5.5]undecan-3-yl)methanol (280 mg, crude) in DMF (5 mL), and the reaction liquid was stirred at room temperature for 1 h. Water was added, and the mixture was extracted with ethyl acetate. The organic phases were combined, dried over anhydrous sodium sulfate, and filtered, and the filtrate was concentrated and purified by silica gel column chromatography (DCM:MeOH = 100:1 to 5:1) to give 1-(5-(3-(hydroxymethyl)-2-oxa-9-azaspiro[5.5]undecane-9-carbonyl)-2-methylphenyl)dihydropyrimidine-2,4(1*H*,3*H*)-dione.

LC-MS: (ESI, m/z): [M+H]⁺ = 416.3.

### Step 4: preparation of 9-(3-(2,4-dioxotetrahydropyrimidin-1(2H)-yl)-4-methylbenzoyl)-2-oxa-9-azaspiro[5.5]undecane-3-carbaldehyde

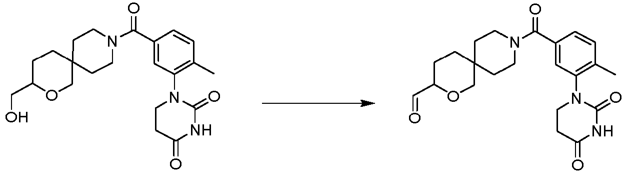

Dess-Martain oxidant (332 mg,0.783 mmol) was added to a solution of 1-(5-(3-(hydroxymethyl)-2-oxa-9-azaspiro[5.5]undecane-9-carbonyl)-2-methylphenyl)dihydropyrimidine-2,4(1*H*,3*H*)-dione (130 mg, 0.313 mmol) in DCM (3 mL), and the reaction liquid was stirred at room temperature for 1 h. After the reaction was completed, sodium thiosulfate was added to quench the reaction, and then the mixture was extracted with ethyl acetate. The organic phases were combined, dried over anhydrous sodium sulfate, and filtered, and the filtrate was concentrated and purified by silica gel column chromatography (DCM:MeOH = 100:1 to 5:1) to give 9-(3-(2,4-dioxotetrahydropyrimidin-1(2*H*)-yl)-4-methylbenzoyl)-2-oxa-9-azaspiro[5.5]undecane-3-carbaldehyde.

LC-MS: (ESI, m/z): [M+H]⁺ = 414.3.

### Step 5: preparation of tert-butyl 3-(2-((1-((4-((9-(3-(2,4-dioxotetrahydropyrimidin-1(2H)-yl)-4-methylbenzoyl)-2-oxa-9-azaspiro[5.5]undecan-3-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

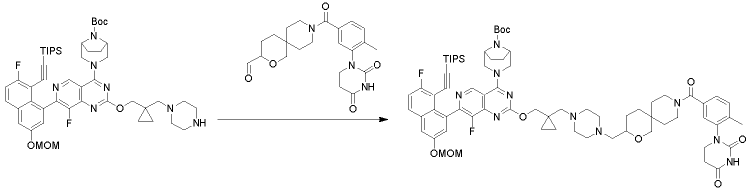

Tetraisopropyl titanate (0.3 mL) was added to a solution of *tert-butyl* 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-d]pyrimidin-4-yl)-3,8-diazabicyclo[3.2.1]octane-8-carboxylate (80 mg, 0.088 mmol) and 9-(3-(2,4-dioxotetrahydropyrimidin-1(2*H*)-yl)-4-methylbenzoyl)-2-oxa-9-azaspiro[5.5]undecane-3-carbaldehyde (76 mg, 0.17 mmol) in DCM/AcOH (2.0 mL/0.1 mL), and the mixture was stirred at room temperature for 2 h. NaBH(OAc)₃ (59 mg, 0.26 mmol) was added to the mixture at 0-5 °C, and the resulting mixture was reacted at room temperature for 2 h. After the reaction was completed, the mixture was concentrated and purified by silica gel column chromatography (MeOH:DCM = (0-1):4) to give *tert-butyl* 3-(2-((1-((4-((9-(3-(2,4-dioxotetrahydropyrimidin-1(2*H*)-yl)-4-methylbenzoyl)-2-oxa-9-azaspiro[5.5]undecan-3-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 655.4.

### Step 6: preparation of 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-2-oxa-9-azaspiro[5.5]undecane-9-carbonyl)-2-methylphenyl)dihydropyrimidine-2,4(1H,3H)-dione

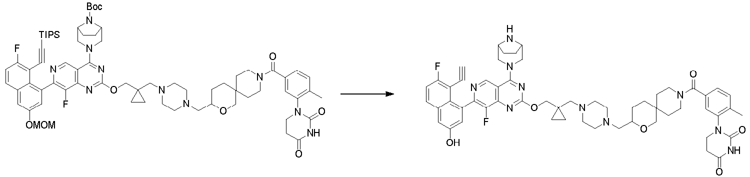

CsF (23 mg, 0.15 mmol) was added to a stirred solution of *tert-butyl* 3-(2-((1-((4-((9-(3-(2,4-dioxotetrahydropyrimidin-1(2*H*)-yl)-4-methylbenzoyl)-2-oxa-9-azaspiro[5.5]undecan-3-yl)methyl) piperazin-1-yl)methyl)cyclopropyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate (40 mg, 0.03 mmol) in DMF (2.0 mL), and the mixture was reacted at room temperature for 2 h. Water (10 mL) was added to the reaction liquid, and the mixture was extracted with ethyl acetate (10 mL × 2). The organic phases were combined, washed with saturated brine, dried over anhydrous sodium sulfate, and filtered, and the filtrate was concentrated to give a crude product of the intermediate, which was dissolved in 1,4-dioxane (1.0 mL), and HCl/1,4-dioxane (6 N, 0.5 mL) was added. The mixture was reacted at room temperature for 0.5 h. An aqueous Na₂CO₃ solution was added to the reaction liquid, and the mixture was adjusted to pH > 7, and extracted with ethyl acetate (10 mL × 2). The organic phase was washed with brine, dried over anhydrous Na₂SO₄, and concentrated. The crude product was purified by preparative high-performance liquid chromatography to give 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-2-oxa-9-azaspiro[5.5]undecane-9-carbonyl)-2-methylphenyl)dihydropyrimidine-2,4(1*H*,3*H*)-dione.

LC-MS: (ESI, m/z): [M+H]⁺ = 1009.4.

¹H NMR (400 MHz, DMSO-*d*₆) δ 9.03 (s, 1H), 8.44 - 8.40 (m, 2H), 7.90 - 7.83 (m, 1H), 7.43-7.39 (m, 1H), 7.37 - 7.28 (m, 4H), 7.20 (d, *J* = 2.8 Hz, 1H), 4.76 - 4.63 (m, 2H), 4.60 - 4.47 (m, 1H), 4.44-4.33 (m, 1H), 4.05 - 3.91 (m, 2H), 3.90 - 3.76 (m, 4H), 3.75 - 3.53 (m, 4H), 3.54 - 3.36 (m, 3H), 3.23 - 3.14 (m, 1H), 3.11 - 2.98 (m, 4H), 2.97 - 2.76 (m, 8H), 2.73 - 2.50 (m, 2H), 2.32 (s, 3H), 2.10 - 1.85 (m, 5H), 1.80 - 1.57 (m, 2H), 1.53 - 1.12 (m, 5H), 0.88 - 0.70 (m, 2H), 0.65 - 0.50 (m, 2H).

### Example 107: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl-d2)-3-azaspiro[5.5]undecane-3-carbonyl)-2-methylphenyl)dihydropyrimidine-2,4(1H,3H)-dione

### Step 1: preparation of 3-(tert-butoxycarbonyl)-3-azaspiro[5.5]undecane-9-carboxylic acid

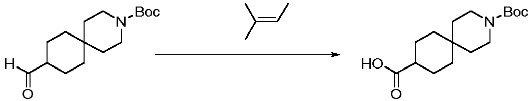

An aqueous solution (80 mL) of sodium chlorite (2.571 g, 28.429 mmol) and sodium dihydrogen phosphate (6.822 g, 56.856 mmol) was added to a solution of *tert*-butyl 9-formyl-3-azaspiro[5.5]undecane-3-carboxylate (2.000 g, 7.107 mmol) and 2-methylbut-2-ene (6 mL) in tetrahydrofuran (80 mL) and *tert-*butanol (20 mL) at room temperature, and the mixture was reacted at room temperature for 18 h. After the reaction was completed, the mixture was extracted with ethyl acetate (100 mL × 3). The organic phases were combined, washed with saturated sodium chloride (100 mL), dried over anhydrous sodium sulfate, and concentrated under reduced pressure. The resulting crude product was purified by silica gel column chromatography (petroleum ether:tetrahydrofuran = 10:1 to 5:1) to give 3-(*tert*-butoxycarbonyl)-3-azaspiro[5.5]undecane-9-carboxylic acid.

LC-MS: (ESI, m/z): [2M+Na]⁺ = 617.5.

### Step 2: preparation of tert-butyl 9-(4-((benzyloxy)carbonyl)piperazine-1-carbonyl)-3-azaspiro[5.5]undecane-3-carboxylate

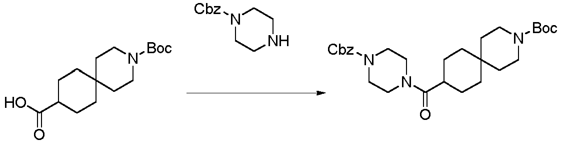

*N*,*N*-Diisopropylethylamine (391 mg, 3.026 mmol) and HATU (767 mg, 2.018 mmol) were added to a solution of 3-(*tert*-butoxycarbonyl)-3-azaspiro[5.5]undecane-9-carboxylic acid (300 mg, 1.009 mmol) and benzyl piperazine-1-carboxylate (222 mg, 1.009 mmol) in *N*,*N*-dimethylformamide (15 mL) at 0 °C, and the mixture was stirred at 25 °C for 3 h. After the reaction was completed, the mixture was poured into ice water (100 mL) and extracted with ethyl acetate (50 mL × 3). The organic phase was washed with water (100 mL) and saturated sodium chloride (100 mL), dried over anhydrous sodium sulfate, and concentrated under reduced pressure. The resulting crude product was purified by silica gel column chromatography (dichloromethane:methanol = 200:1 to 100:1) to give *tert-butyl* 9-(4-((benzyloxy)carbonyl)piperazine-1-carbonyl)-3-azaspiro[5.5]undecane-3-carboxylate.

LC-MS: (ESI, m/z): [M-tBu+H]⁺ = 444.3.

### Step 3: preparation of tert-butyl 9-((4-((benzyloxy)carbonyl)piperazin-1-yl)methyl-d2)-3-azaspiro[5.5]undecane-3-carboxylate

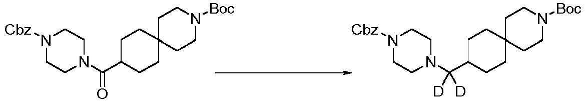

Deuterated lithium aluminum hydride (56.2 mg, 1339.24 µmol) was added to a solution of *tert-*butyl 9-(4-((benzyloxy)carbonyl)piperazine-1-carbonyl)-3-azaspiro[5.5]undecane-3-carboxylate (446.1 mg, 892.82 µmol) in tetrahydrofuran (20 mL) at 0 °C, and the mixture was stirred at 25 °C for 18 h. After the reaction was completed, acetone (1 mL) was added, and the mixture was stirred for 10 min and then concentrated under reduced pressure. The resulting crude product was purified by silica gel column chromatography (dichloromethane:methanol = 200:1 to 100:1) to give *tert-butyl 9-((4-*((benzyloxy)carbonyl)piperazin-1-yl)methyl-d2)-3-azaspiro[5.5]undecane-3-carboxylate.

LC-MS: (ESI, m/z): [M+H]⁺ = 488.4.

### Step 4: preparation of benzyl 4-((3-azaspiro[5.5]undecan-9-yl)methyl-d2)piperazine-1-carboxylate

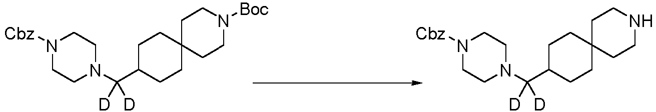

Trifluoroacetic acid (1.7 mL) was added to a solution of *tert-butyl 9-((4-*((benzyloxy)carbonyl)piperazin-1-yl)methyl-d2)-3-azaspiro[5.5]undecane-3-carboxylate (96.5 mg, 197.87 µmol) in dichloromethane (5 mL) at 0 °C, and the mixture was stirred at 25 °C for 1 h. After the reaction was completed, the mixture was concentrated under reduced pressure to give benzyl 4-((3-azaspiro[5.5]undecan-9-yl)methyl-d2)piperazine-1-carboxylate, which was directly used in the next step.

LC-MS: (ESI, m/z): [M+H]⁺ = 388.3.

### Step 5: preparation of benzyl 4-((3-(3-(2,4-dioxotetrahydropyrimidin-1(2H)-yl)-4-methylbenzoyl)-3-azaspiro[5.5]undecan-9-yl)methyl-d2)piperazine-1-carboxylate

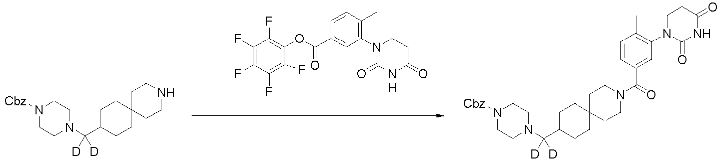

Pentafluorophenyl 3-(2,4-dioxotetrahydropyrimidin-1(2*H*)-yl)-4-methylbenzoate (90 mg, 217.66 µmol) was added to a solution of benzyl 4-((3-azaspiro[5.5]undecan-9-yl)methyl-d2)piperazine-1-carboxylate (80 mg, crude) and *N*,*N*-diisopropylethylamine (300 mg, 2321.26 µmol) in *N,N-*dimethylformamide (10 mL) at room temperature. After the reaction was completed, the mixture was poured into ice water (100 mL) and extracted with ethyl acetate (50 mL × 3). The organic phase was washed with water (100 mL) and saturated sodium chloride (100 mL), dried over anhydrous sodium sulfate, and concentrated under reduced pressure. The resulting crude product was purified by silica gel thin-layer chromatography (dichloromethane:methanol = 10:1) to give benzyl 4-((3-(3-(2,4-dioxotetrahydropyrimidin-1(2*H*)-yl)-4-methylbenzoyl)-3-azaspiro[5.5]undecan-9-yl)methyl-d2)piperazine-1-carboxylate.

LC-MS: (ESI, m/z): [M+H]⁺ = 618.4.

### Step 6: preparation of 1-(2-methyl-5-(9-(piperazin-1-ylmethyl-d2)-3-azaspiro[5.5]undecane-3-carbonyl)phenyl)dihydropyrimidine-2,4(1H,3H)-dione

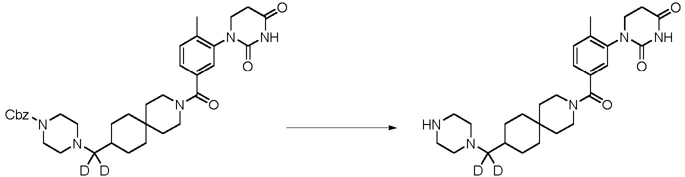

Palladium on carbon (10 mg) was added to a solution of benzyl 4-((3-(3-(2,4-dioxotetrahydropyrimidin-1(2*H*)-yl)-4-methylbenzoyl)-3-azaspiro[5.5]undecan-9-yl)methyl-d2)piperazine-1-carboxylate (74.5 mg, 120.59 µmol) in methanol (5 mL) at room temperature, and the mixture was purged three times with hydrogen and stirred at 25 °C for 18 h. After the reaction was completed, the mixture was concentrated under reduced pressure to give 1-(2-methyl-5-(9-(piperazin-1-ylmethyl-d2)-3-azaspiro[5.5]undecane-3-carbonyl)phenyl)dihydropyrimidine-2,4(1*H*,3*H*)-dione, which was directly used in the next step.

LC-MS: (ESI, m/z): [M+H]⁺ = 484.4.

### Step 7: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl-d2)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

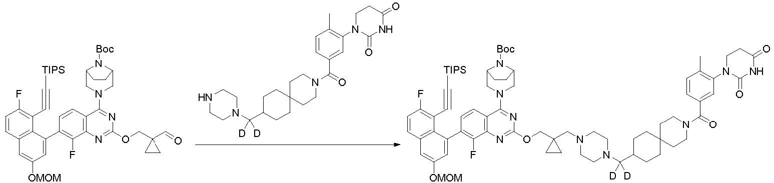

Tetraisopropyl titanate (286 mg, 1004.90 µmol) and glacial acetic acid (0.1 mL) were added to a stirred solution of *tert-butyl* 3-(8-fluoro-7-(7-fluoro-3-(methoxymethoxy)-8-((triisopropylsilyl)ethynyl)naphthalen-1-yl)-2-((1-formylcyclopropyl)methoxy)quinazolin-4-yl)-3,8-diazabicyclo[3.2.1]octane-8-carboxylate (85 mg, 100.49 µmol) and 1-(2-methyl-5-(9-(piperazin-1-ylmethyl-d2)-3-azaspiro[5.5]undecane-3-carbonyl)phenyl) dihydropyrimidine-2,4(1*H*,3*H*)-dione (58 mg, 120.59 µmol) in dichloromethane (10 mL) at room temperature, and the mixture was stirred at 25 °C for 2 h. Then sodium triacetoxyborohydride (72 mg, 344.00 µmol) was added, and the mixture was reacted at 25 °C for another 4 h. Methanol (5 mL) was added to quench the reaction, and the mixture was concentrated under reduced pressure at 40 °C. The resulting crude product was purified by a preparative thin-layer chromatography plate (dichloromethane:methanol = 10:1) to give *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl-d2)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1308.7.

### Step 8: preparation of tert-butyl 3-(2-((1-((4-((3-(3-(2,4-dioxotetrahydropyrimidin-1(2H)-yl)-4-methylbenzoyl)-3-azaspiro[5.5]undecan-9-yl)methyl-d2)piperazin-1-yl)methyl)cyclopropyl)methoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate

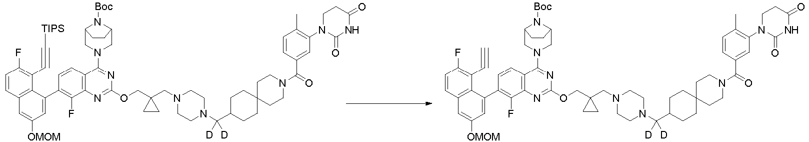

Cesium fluoride (67 mg, 442.40 µmol) was added to a solution of *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl-d2)piperazin-1-yl)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (57.9 mg, 44.24 µmol) in *N*,*N*-dimethylformamide (5 mL) at room temperature, and the mixture was reacted at 25 °C for 1 h under nitrogen atmosphere. After the reaction was completed, the reaction liquid was poured into water (100 mL), and the mixture was extracted with ethyl acetate (20 mL × 3). The organic phases were combined, washed with saturated brine (50 mL), dried over anhydrous sodium sulfate, filtered, and concentrated under reduced pressure to give *tert-butyl* 3-(2-((1-((4-((3-(3-(2,4-dioxotetrahydropyrimidin-1(2*H*)-yl)-4-methylbenzoyl)-3-azaspiro[5.5]undecan-9-yl)methyl-d2)piperazin-1-yl)methyl)cyclopropyl)methoxy)-7-(8-ethynyl-7-fluoro-3-(methoxymethoxy)naphthalen-1-yl)-8-fluoroquinazolin-4-yl)-3,8-diazabicyclo[3.2.1|octane-8-carboxylate.

LC-MS: (ESI, m/z): [M+H]⁺ = 1152.7.

### Step 9: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl-d2)-3-azaspiro[5.5]undecane-3-carbonyl)-2-methylphenyl)dihydropyrimidine-2,4(1H,3H)-dione

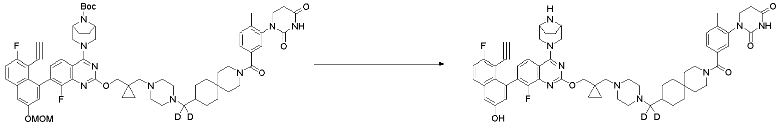

A hydrogen chloride/1,4-dioxane solution (4.0 M, 6 mL) was added to a solution of *tert*-butyl 3-(2-((1-((4-((3-(3-(2,4-dioxotetrahydropyrimidin-1(2*H*)-yl)-4-methylbenzoyl)-3-azaspiro[5.5]undecan-9-yl)methyl-d2)piperazin-1-yl)methyl)cyclopropyl)methoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate (37.0 mg, 32.11 µmol) in 1,4-dioxane (2 mL) at room temperature, and the mixture was reacted at room temperature for 1.5 h. After the reaction was completed, the mixture was concentrated to dryness under reduced pressure at 30 °C, and a solution of ammonia in methanol (1 mL) was added to the concentrate. Then the mixture was concentrated to dryness under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoroquinazolin-2-yl)oxy)methyl)cyclopropyl)methyl)piperazin-1-yl)methyl-d2)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 1008.2.

¹H NMR (400 MHz, CD₃OD) δ 7.83 - 7.79 (m, 1H), 7.73 (d, *J* = 8.4 Hz, 1H), 7.39 (d, *J* = 7.6 Hz, 1H), 7.32 - 7.21 (m, 4H), 7.19 - 7.17 (m, 1H), 7.08 (d, *J* = 2.4 Hz, 1H), 4.52 - 4.41 (m, 3H), 4.33 - 4.30 (m, 1H), 3.90 - 3.80 (m, 1H), 3.70 - 3.63 (m, 4H), 3.60- 3.54 (m, 2H), 3.50 - 3.35 (m, 2H), 3.19 (s, 1H), 2.87 - 2.75 (m, 2H), 2.60 - 2.50 (m, 2H), 2.50 - 2.30 (m, 4H), 2.31 (s, 3H), 1.95 - 1.80 (m, 4H), 1.80 - 1.70 (m, 2H), 1.70 - 1.53 (m, 4H), 1.53 - 1.40 (m, 4H), 1.40 - 1.25 (m, 4H), 1.20 - 1.00 (m, 4H), 0.75 - 0.60 (m, 2H), 0.53 - 0.43 (m, 2H).

### Example 108: 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-(3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

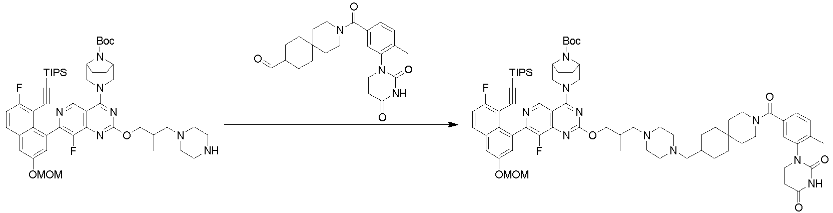

Tetraisopropyl titanate (375 mg, 1318.00 µmol) and glacial acetic acid (0.3 mL) were added to a stirred solution of *tert-butyl* 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)pyrido[4,3-*d*]pyrimidin-4-yl)-3,8-diazabicyclo[3.2.1]octane-8-carboxylate (237.3 mg, 263.60 µmol) and 3-(3-(2,4-dioxotetrahydropyrimidin-1(2*H*)-yl)-4-methylbenzoyl)-3-azaspiro[5.5]undecane-9-carbaldehyde (217.0 mg, 527.30 µmol) in dichloromethane (15 mL) at room temperature, and the mixture was stirred at 25 °C for 3 h. Then sodium triacetoxyborohydride (168 mg, 790.80 µmol) was added, and the mixture was reacted at 25 °C for another 16 h. Methanol (5 mL) was added to the reaction liquid to quench the reaction, and the mixture was concentrated under reduced pressure at 40 °C. The resulting crude product was purified by a preparative thin-layer chromatography plate (dichloromethane:methanol = 10:1) to give *tert*-butyl 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 648.5.

### Step 2: preparation of tert-butyl 3-(2-(3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-d]pyrimidin-4-yl)-3,8-diazabicyclo[3.2.1]octane-8-carboxylate

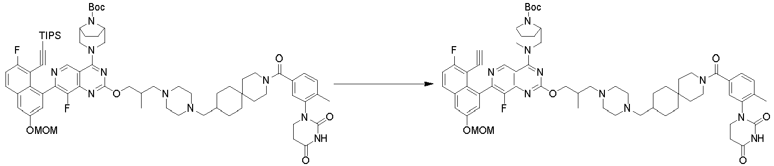

Cesium fluoride (257 mg, 1693.30 µmol) was added to a solution of *tert*-butyl 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (219.4 mg, 169.33 µmol) in *N*,*N*-dimethylformamide (10 mL) at room temperature, and the mixture was reacted at 25 °C for 2 h under nitrogen atmosphere. After the reaction was completed, the reaction liquid was poured into water (60 mL), and the mixture was extracted with ethyl acetate (20 mL × 3). The organic phase was washed with water (60 mL) and saturated brine (60 mL), dried over anhydrous sodium sulfate, filtered, and concentrated under reduced pressure to give *tert-butyl* 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate.

LC-MS: (ESI, m/z): [M/2+H]⁺ = 570.4.

### Step 3: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

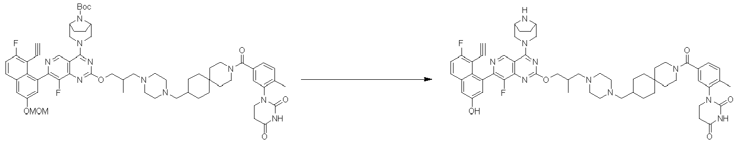

A hydrogen chloride/1,4-dioxane solution (4.0 M, 5 mL) was added to a solution of the crude product of *tert*-butyl 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoropyrido[4,3-*d*]pyrimidin-4-yl)-3,8-diazabicyclo[3.2.1]octane-8-carboxylate (198.2 mg, 169.33 µmol) in 1,4-dioxane (3 mL) 0 °C, and the mixture was reacted at room temperature for 2 h. After the reaction was completed, the mixture was concentrated under reduced pressure at 30 °C, and a solution of ammonia in methanol (1 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 995.4.

¹H NMR (400 MHz, DMSO-*d*₆) δ 10.37 (s, 1H), 10.15 (br, 1H), 9.03 (s, 1H), 7.97 (dd, *J* = 9.2 Hz, 6.0 Hz, 1H), 7.45 (t, *J* = 9.2 Hz, 1H), 7.38 (d, *J* = 2.8 Hz, 1H), 7.33 (d, *J* = 8.0 Hz, 1H), 7.31 (d, *J* = 1.2 Hz, 1H), 7.24 - 7.22 (m, 1H), 7.17 (d, *J =* 2.4 Hz, 1H), 4.50-4.37 (m, 2H), 4.35 - 4.25 (m, 1H), 4.15 - 4.00 (m, 1H), 3.93(s, 1H), 3.83 - 3.77 (m, 1H), 3.70 - 3.50 (m, 8H), 2.85 - 2.65 (m, 3H), 2.45 - 2.30 (m, 9H), 2.30 - 2.25 (m, 4H), 2.20 - 2.10 (m, 2H), 2.10 - 2.00 (m, 3H), 1.70 - 1.60 (m, 6H), 1.55 - 1.23 (m, 8H), 1.10 - 1.00 (m, 2H), 1.00 - 0.90 (m, 3H).

### Example 109: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-(methyl-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

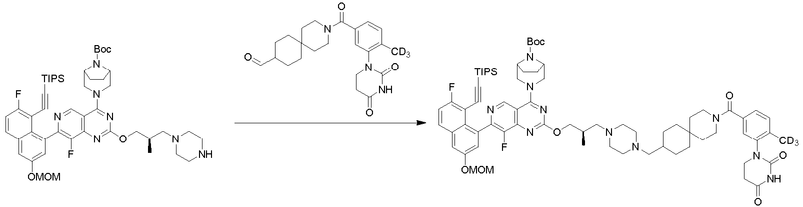

Tetraisopropyl titanate (0.5 mL) was added to a stirred solution of *tert-butyl* 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate (150 mg, 0.17 mmol) and 3-(3-(2,4-dioxotetrahydropyrimidin-1(2*H*)-yl)-4-(methyl-d3)benzoyl)-3-azaspiro[5.5]undecane-9-carbaldehyde (108 mg, 0.26 mmol) in DCM/AcOH (1.5 mL/0.15 mL), and the mixture was stirred at 30 °C for 2 h. NaBH(OAc)₃ (108 mg, 0.51 mmol) was added to the mixture in an ice bath, and the resulting mixture was stirred for 2 h. The mixture was concentrated, and the resulting crude product was purified by silica gel column chromatography (DCM:MeOH = 100:1 to 10:1) to give *tert-butyl* 3-(2-((*R*)-3-(4-((3-(3-(2,4-dioxotetrahydropyrimidin-1(2*H*)-yl)-4-(methyl-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 650.5.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1H,3H)-dione

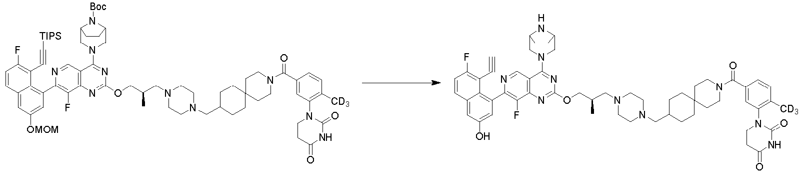

Cesium fluoride (184 mg, 1.20 mmol) was added to a stirred solution of *tert*-butyl 3-(2-((*R*)-3-(4-((3-(3-(2,4-dioxotetrahydropyrimidin-1(2*H*)-yl)-4-(methyl-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (157 mg, 0.12 mmol) in DMF (1 mL), and the mixture was stirred at room temperature for 30 min. Water (20 mL) was added to the reaction liquid to quench the reaction, and the mixture was extracted with ethyl acetate (20 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7 and then extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-(methyl-d3)phenyl)dihydropyrimidine-2,4(1*H*,3*H*)-dione.

LC-MS: (ESI, m/z): [M+H]⁺ = 998.5.

¹H NMR (400 MHz, DMSO-*d*₆) δ 10.36 (s, 1H), 10.14 (s, 1H), 9.02 (s, 1H), 7.97 (dd, *J* = 9.2, 5.9 Hz, 1H), 7.48 - 7.43 (m, 1H), 7.41 - 7.36 (m, 1H), 7.35 - 7.28 (m, 2H), 7.26 - 7.21 (m, 1H), 7.19 - 7.15 (m, 1H), 4.51 - 4.40 (m, 2H), 4.36 - 4.27 (m, 1H), 4.12 - 4.00 (m, 1H), 3.93 (s, 1H), 3.86 - 3.74 (m, 1H), 3.66 - 3.48 (m, 7H), 2.82 - 2.65 (m, 3H), 2.47 - 1.97 (m, 15H), 1.65 (s, 6H), 1.55 - 1.38 (m, 5H), 1.34 - 1.21 (m, 2H), 1.15 - 1.01 (m, 3H), 0.99 - 0.93 (m, 4H).

### Example 110: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-ethylphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-ethylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

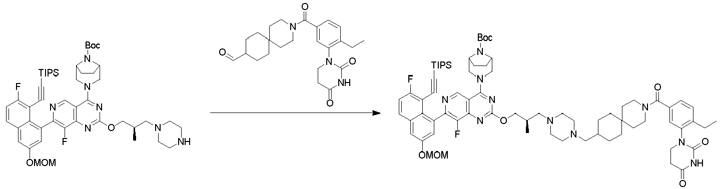

Tetraisopropyl titanate (0.5 mL) was added to a stirred solution of *tert-butyl* 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate (150 mg, 0.17 mmol) and 3-(3-(2,4-dioxotetrahydropyrimidin-1(2*H*)-yl)-4-ethylbenzoyl)-3-azaspiro[5.5]undecane-9-carbaldehyde (111 mg, 0.26 mmol) in DCM/AcOH (1.5 mL/0.15 mL), and the mixture was stirred at 30 °C for 2 h. NaBH(OAc)₃ (108 mg, 0.51 mmol) was added to the mixture in an ice bath, and the resulting mixture was stirred for 2 h. The mixture was concentrated, and the resulting crude product was purified by silica gel column chromatography (DCM:MeOH = 100:1 to 10:1) to give *tert-butyl* 3-(2-((*R*)-3-(4-((3-(3-(2,4-dioxotetrahydropyrimidin-1(2*H*)-yl)-4-ethylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 656.0.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-ethylphenyl)dihydropyrimidine-2,4(1H,3H)-dione

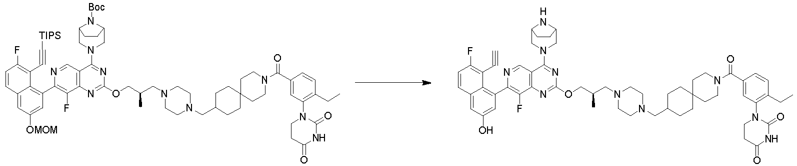

Cesium fluoride (182 mg, 1.20 mmol) was added to a stirred solution of *tert-butyl* 3-(2-((*R*)-3-(4-((3-(3-(2,4-dioxotetrahydropyrimidin-1(2*H*)-yl)-4-ethylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (150 mg, 0.12 mmol) in DMF (1 mL), and the mixture was stirred at room temperature for 30 min. Water (10 mL) was added to the reaction liquid to quench the reaction, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic layer was washed with brine, dried over anhydrous Na₂SO₄, and concentrated. The crude product was purified by high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*|pyrimidin-2-yl)oxy)-2-methylpropyl) piperazin-1-yl)methyl)-3-azaspiro[5.5]undecane-3-carbonyl)-2-ethylphenyl)dihydropyrimidine-2,4(1*H*,3*H*)-dione.

LC-MS: (ESI, m/z): [M+H]⁺ = 1009.4.

¹H NMR (400 MHz, DMSO-*d*₆) δ 10.36 (s, 1H), 10.14 (s, 1H), 9.02 (s, 1H), 7.97 (dd, *J=* 9.2, 6.0 Hz, 1H), 7.48 - 7.43 (m, 1H), 7.40 - 7.35 (m, 2H), 7.32 - 7.28 (m, 2H), 7.19 - 7.15 (m, 1H), 4.51 - 4.40 (m, 2H), 4.35 - 4.26 (m, 1H), 4.13 - 4.00 (m, 1H), 3.92 (s, 1H), 3.86 - 3.76 (m, 1H), 3.65 - 3.46 (m, 7H), 2.83 - 2.64 (m, 3H), 2.60 - 2.54 (m, 2H), 2.45 - 1.99 (m, 15H), 1.65 (s, 6H), 1.54 - 1.38 (m, 5H), 1.35 - 1.23 (m, 2H), 1.16 (t, *J* = 7.5 Hz, 3H), 1.09 - 0.93 (m, 7H).

### Example 151: 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-1-oxa-9-azaspiro[5.5]undecane-9-carbonyl)-2-methylphenyl)dihydropyrimidine-2,4(1H,3H)-dione

### Step 1: preparation of tert-butyl 4-allyl-4-((2-(methoxycarbonyl)allyl)oxy)piperidine-1-carboxylate

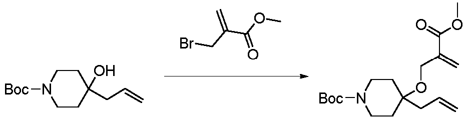

tert-Butyl 4-allyl-4-hydroxypiperidine-1-carboxylate (9.0 g, 37.29 mmol) was dissolved in DMF (200 mL), NaH (1.79 g, 44.75 mmol) was added at 0 °C, and the mixture was reacted at room temperature for 1 h and then cooled to 0 °C. Methyl 2-(bromomethyl)acrylate (6.68 g, 37.29 mmol) was added, and the mixture was reacted at room temperature for another 72 h. After the reaction was completed, NH₄Cl (200 mL) was added to the reaction liquid, and the mixture was extracted with ethyl acetate (200 mL × 3). The organic phases were combined, washed with a saturated NaCl solution, dried over anhydrous Na₂SO₄, and filtered, and the filtrate was concentrated. The resulting crude product was purified by silica gel column chromatography (PE:EA = 5:1) to give *tert-butyl* 4-allyl-4-((2-(methoxycarbonyl)allyl)oxy)piperidine-1-carboxylate.

LC-MS: (ESI, m/z): [M+Na]⁺ = 362.2.

### Step 2: preparation of 9-(tert-butyl) 3-methyl 1-oxa-9-azaspiro[5.5]undec-3-ene-3,9-dicarboxylate

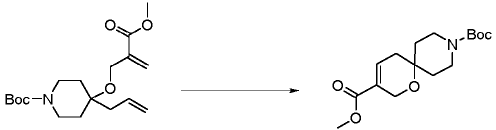

*tert-Butyl* 4-allyl-4-((2-(methoxycarbonyl)allyl)oxy)piperidine-1-carboxylate (3.0 g, 8.84 mmol) was dissolved in DCE (700 mL), Grubbs II (229 mg, 0.27 mmol) was added, and the mixture was reacted at 90 °C for 16 h. After the reaction was completed, the mixture was cooled to room temperature and filtered, and the filtrate was concentrated. The resulting crude product was purified by silica gel column chromatography (PE:EA = 5:1) to give 9-(*tert*-butyl) 3-methyl 1-oxa-9-azaspiro[5.5]undec-3-ene-3,9-dicarboxylate.

LC-MS: (ESI, m/z): [M+Na]⁺ = 334.2.

### Step 3: preparation of 9-(tert-butyl) 3-methyl 1-oxa-9-azaspiro[5.5]undecane-3,9-dicarboxylate

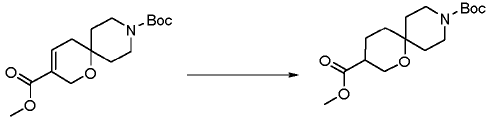

Pd(OH)₂ (586 mg, 4.17 mmol) was added to a solution of 9-(tert-butyl) 3-methyl 1-oxa-9-azaspiro[5.5]undec-3-ene-3,9-dicarboxylate (1.3 g, 4.17 mmol) in EtOH (20 mL), and the mixture was reacted at 50 °C for 16 h under H₂ atmosphere. After the reaction was completed, the mixture was cooled to room temperature and filtered, and the filtrate was concentrated. The resulting crude product was purified by silica gel column chromatography (PE:EA = 5:1) to give 9-(tert-butyl) 3-methyl 1-oxa-9-azaspiro[5.5]undecane-3,9-dicarboxylate.

LC-MS: (ESI, m/z): [M+Na]⁺ = 336.2.

### Step 4: preparation of tert-butyl 3-(hydroxymethyl)-1-oxa-9-azaspiro[5.5]undecane-9-carboxylate

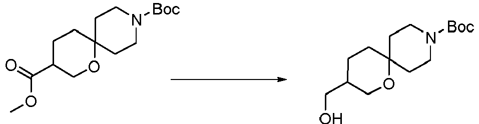

9-(tert-Butyl) 3-methyl 1-oxa-9-azaspiro[5.5]undecane-3,9-dicarboxylate (1.0 g, 2.19 mmol) was dissolved in THF (10 mL), DIBAL-H (9.57 mL, 9.57 mmol) was added in an ice-water bath, and the mixture was reacted for 3 h. After the reaction was completed, the mixture was warmed to room temperature. A saturated potassium sodium tartrate solution (100 mL) was added, and the mixture was extracted with ethyl acetate (50 mL × 3). The organic phases were combined, washed with a saturated NaCl solution, dried over anhydrous Na₂SO₄, and filtered, and the filtrate was concentrated. The resulting crude product was purified by silica gel column chromatography (PE:EA = 1:1) to give *tert-butyl* 3-(hydroxymethyl)-1-oxa-9-azaspiro [5 .5]undecane-9-carboxylate.

LC-MS: (ESI, m/z): [M+Na]⁺ = 308.3.

### Step 5: preparation of tert-butyl 3-formyl-1-oxa-9-azaspiro[5.5]undecane-9-carboxylate

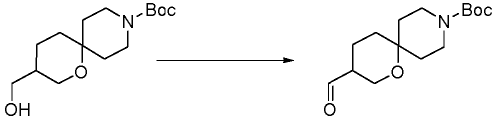

Dess-Martain (2.09 g, 4.92 mmol) was added to a solution of *tert*-butyl 3-(hydroxymethyl)-1-oxa-9-azaspiro[5.5]undecane-9-carboxylate (700 mg, 2,46 mmol) in DCM (10 ml), and the reaction liquid was stirred at room temperature for 1 h. After the reaction was completed, sodium thiosulfate was added to quench the reaction, and then the mixture was extracted with ethyl acetate. The organic phases were combined, dried over anhydrous sodium sulfate, and filtered, and the filtrate was concentrated. The resulting crude product was purified by silica gel column chromatography (PE:EA = 1:1) to give *tert-butyl* 3-formyl-1-oxa-9-azaspiro[5.5]undecane-9-carboxylate.

LC-MS: (ESI, m/z): [M-tBu+H]⁺ = 228.2.

### Step 6: preparation of 1-oxa-9-azaspiro[5.5]undecane-3-carbaldehyde

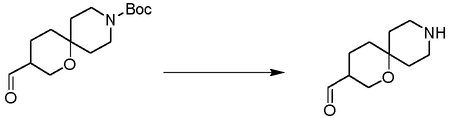

*tert-Butyl* 3-formyl-1-oxa-9-azaspiro[5.5]undecane-9-carboxylate (200 mg, 0.71 mmol) was dissolved in DCM (1.5 mL), and then TFA (1.5 mL) was added. The mixture was stirred at room temperature for 1 h and concentrated to give 1-oxa-9-azaspiro[5.5]undecane-3-carbaldehyde. The crude product was directly used in the next step without further purification.

### Step 7: preparation of 9-(3-(2,4-dioxotetrahydropyrimidin-1(2H)-yl)-4-methylbenzoyl)-1-oxa-9-azaspiro[5.5]undecane-3-carbaldehyde

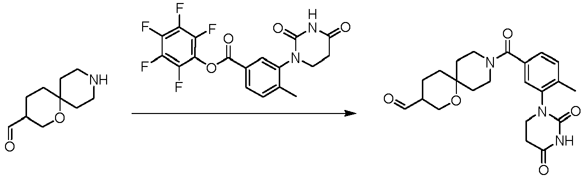

DIEA (459 mg, 3.55 mmol) and pentafluorophenyl 3-(2,4-dioxotetrahydropyrimidin-1(2*H*)-yl)-4-methylbenzoate (294 mg, 0.71 mmol) were added to a solution of 1-oxa-9-azaspiro[5.5]undecane-3-carbaldehyde (230 mg, crude) in DMF (3 mL), and the reaction liquid was stirred at room temperature for 1 h. Water was added to the reaction liquid, and then the mixture was extracted with ethyl acetate. The organic phases were combined, dried over anhydrous sodium sulfate, and filtered, and the filtrate was concentrated. The resulting crude product was purified by silica gel column chromatography (DCM:MeOH = 20:1) to give 9-(3-(2,4-dioxotetrahydropyrimidin-1(2*H*)-yl)-4-methylbenzoyl)-1-oxa-9-azaspiro[5.5]undecane-3-carbaldehyde.

LC-MS: (ESI, m/z): [M+H]⁺ = 414.3.

### Step 8: preparation of tert-butyl 3-(2-((1-((4-((9-(3-(2,4-dioxotetrahydropyrimidin-1(2H)-yl)-4-methylbenzoyl)-1-oxa-9-azaspiro[5.5]undecan-3-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

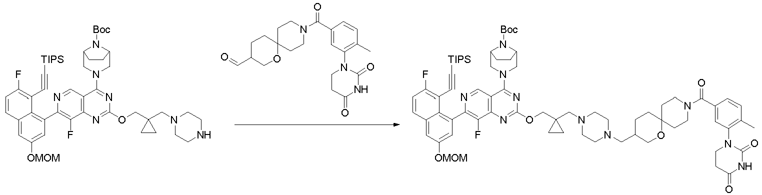

Tetraisopropyl titanate (0.5 mL) was added to a solution of *tert*-butyl 3-(8-fluoro-7-(7-fluoro-3-(methoxymethoxy)-8-((triisopropylsilyl)ethynyl)naphthalen-1-yl)-2-((1-(piperazin-1-ylmethyl)cyclopropyl)methoxy)pyrido[4,3-*d*]pyrimidin-4-yl)-3,8-diazabicyclo[3.2.1]octane-8-carboxylate (150 mg, 0.16 mmol) and 9-(3-(2,4-dioxotetrahydropyrimidin-1(2*H*)-yl)-4-methylbenzoyl)-1-oxa-9-azaspiro[5.5]undecane-3-carbaldehyde (99 mg, 0.24 mmol) in DCM/AcOH (2.0 mL/0.2 mL), and the mixture was stirred at room temperature for 2 h. NaBH(OAc)₃ (153 mg, 0.72 mmol) was added to the above mixture at 0-5 °C, and then the resulting mixture was reacted at room temperature for another 2 h. After the reaction was completed, the mixture was directly concentrated under reduced pressure, and the resulting crude product was purified by silica gel column chromatography (DCM:MeOH =1:0 to 4:1) to give *tert*-butyl 3-(2-((1-((4-((9-(3-(2,4-dioxotetrahydropyrimidin-1(2*H*)-yl)-4-methylbenzoyl)-1-oxa-9-azaspiro[5.5]undecan-3-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 655.4

### Step 9: preparation of 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-1-oxa-9-azaspiro[5.5]undecane-9-carbonyl)-2-methylphenyl)dihydropyrimidine-2,4(1H,3H)-dione

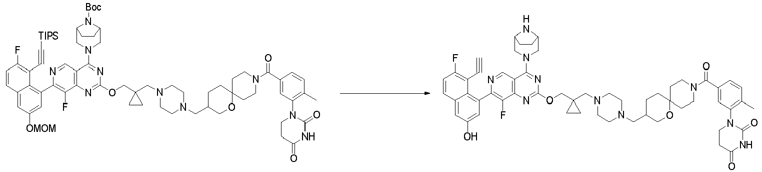

Cesium fluoride (150 mg, 0.99 mmol) was added to a solution of *tert*-butyl 3-(2-((1-((4-((9-(3-(2,4-dioxotetrahydropyrimidin-1(2*H*)-yl)-4-methylbenzoyl)-1-oxa-9-azaspiro[5.5]undecan-3-yl)methyl) piperazin-1-yl)methyl)cyclopropyl)methoxy)-8-fluoro-7-(7-fluoro-3-(methoxymethoxy)-8-((triisopropylsilyl) ethynyl)naphthalen-1-yl)pyrido[4,3-*d*]pyrimidin-4-yl)-3,8-diazabicyclo[3.2.1]octane-8-carboxylate (130 mg, 0.099 mmol) in DMF (1 mL), and the mixture was stirred at room temperature for 1 h. Water (10 mL) was added to the reaction liquid, and the mixture was extracted with ethyl acetate (10 mL × 3). The organic phases were washed with brine, combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The combined organic phase was dried over anhydrous Na₂SO₄ and concentrated. The crude product was purified by preparative high-performance liquid chromatography to give 1-(5-(3-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-1-oxa-9-azaspiro[5.5]undecane-9-carbonyl)-2-methylphenyl)dihydropyrimidine-2,4(1*H*,3*H*)-dione.

LC-MS: (ESI, m/z): [M+H]⁺ = 1009.4

¹H NMR (400 MHz, DMSO-*d*₆) δ 10.36 (s, 1H), 10.13 (s, 1H), 9.02 (s, 1H), 7.97 (dd, *J=* 9.2, 5.9 Hz, 1H), 7.46 (t, *J* = 9.0 Hz, 1H), 7.40 - 7.36 (m, 1H), 7.35 - 7.29 (m, 2H), 7.28 - 7.22 (m, 1H), 7.19 - 7.14 (m, 1H), 4.55 - 4.40 (m, 1H), 4.34 - 4.20 (m, 3H), 4.15 - 4.00 (m, 1H), 3.92 (s, 1H), 3.85 - 3.74 (m, 1H), 3.69 - 3.58 (m, 2H), 3.57 - 3.44 (m, 4H), 3.23 - 3.05 (m, 3H), 2.79 - 2.65 (m, 2H), 2.41 - 1.98 (m, 17H), 1.72 - 1.60 (m, 5H), 1.59 - 1.42 (m, 4H), 1.38 - 1.19 (m, 4H), 0.65 - 0.57 (m, 2H), 0.44 - 0.32 (m, 2H).

### Example 155: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

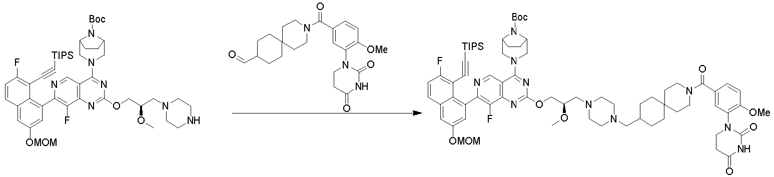

Tetraisopropyl titanate (0.5 mL) was added to a solution of *tert*-butyl 3-(8-fluoro-7-(7-fluoro-3-(methoxymethoxy)-8-((triisopropylsilyl)ethynyl)naphthalen-1-yl)-2-((*R*)-2-methoxy-3-(piperazin-1-yl)propoxy)pyrido[4,3-*d*]pyrimidin-4-yl)-3,8-diazabicyclo[3.2.1]octane-8-carboxylate (150 mg, 0.16 mmol) and 3-(3-(2,4-dioxotetrahydropyrimidin-1(2*H*)-yl)-4-methoxybenzoyl)-3-azaspiro[5.5]undecane-9-carbaldehyde (103 mg, 0.24 mmol) in DCM/AcOH (1.5 mL/0.15 mL), and the mixture was stirred at 30 °C for 2 h. NaBH(OAc)₃ (102 mg, 0.48 mmol) was added to the mixture in an ice bath, and the resulting mixture was stirred for 2 h. The reaction mixture was directly concentrated, and the resulting crude product was purified by silica gel column chromatography (DCM:MeOH = 10:1) to give *tert*-butyl 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 664.5.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1H,3H)-dione

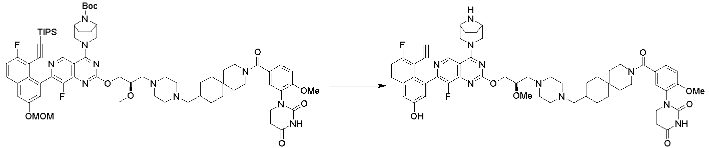

Cesium fluoride (152 mg, 1.0 mmol) was added to a solution of *tert*-butyl 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (137 mg, 0.10 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. An aqueous Na₂CO₃ solution was added to the reaction liquid, and the mixture was adjusted to pH > 7 and extracted with EtOAc (20 mL × 2). The organic phase was washed with brine, dried over anhydrous Na₂SO₄, and concentrated. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methoxypropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1027.1.

¹H NMR (400 MHz, DMSO-*d*₆) δ 10.32 (s, 1H), 10.14 (s, 1H), 9.04 (s, 1H), 7.97 (dd, *J =* 9.2, 6.0 Hz, 1H), 7.46 (t, *J* = 9.0 Hz, 1H), 7.41 - 7.30 (m, 3H), 7.19 - 7.12 (m, 2H), 4.59 - 4.41 (m, 2H), 4.36 - 4.24 (m, 2H), 3.92 (s, 1H), 3.84 (s, 3H), 3.79 - 3.42 (m, 10H), 3.36 (d, *J* = 1.9 Hz, 3H), 2.76 - 2.60 (m, 3H), 2.48 - 1.96 (m, 13H), 1.73 - 1.58 (m, 6H), 1.55 - 1.38 (m, 5H), 1.34 - 1.25 (m, 2H), 1.13 - 0.94 (m, 4H).

### Example 172: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

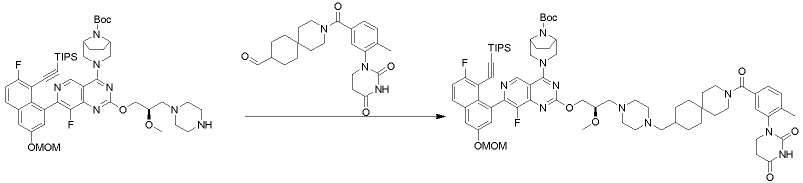

Tetraisopropyl titanate (0.5 mL) was added to a solution of *tert-butyl* 3-(8-fluoro-7-(7-fluoro-3-(methoxymethoxy)-8-((triisopropylsilyl)ethynyl)naphthalen-1-yl)-2-((*R*)-2-methoxy-3-(piperazin-1-yl)propoxy)pyrido[4,3-*d*]pyrimidin-4-yl)-3,8-diazabicyclo[3.2.1]octane-8-carboxylate (150 mg, 0.16 mmol) and 3-(3-(2,4-dioxotetrahydropyrimidin-1(2*H*)-yl)-4-methylbenzoyl)-3-azaspiro[5.5]undecane-9-carbaldehyde (99 mg, 0.24 mmol) in DCM/AcOH (1.5 mL/0.15 mL), and the mixture was stirred at 30 °C for 2 h. NaBH(OAc)₃ (102 mg, 0.48 mmol) was added to the mixture in an ice bath, and the resulting mixture was stirred for 2 h. The reaction mixture was directly concentrated, and the resulting crude product was purified by silica gel column chromatography (DCM:MeOH = 10:1) to give *tert-butyl* 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 656.5.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1H,3H)-dione

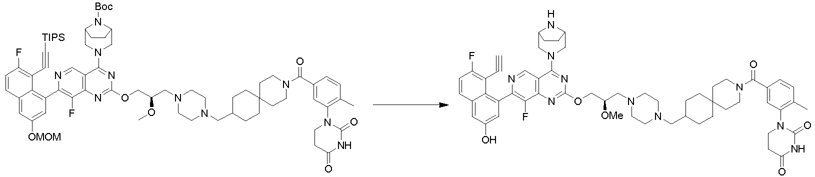

Cesium fluoride (167 mg, 1.10 mmol) was added to a solution of *tert-butyl* 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methoxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (141 mg, 0.11 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. An aqueous Na₂CO₃ solution was added to the reaction liquid, and the mixture was adjusted to pH > 7 and extracted with EtOAc (20 mL × 2). The organic phase was washed with brine, dried over anhydrous Na₂SO₄, and concentrated. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methoxypropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 506.2.

¹H NMR (400 MHz, DMSO-*d*₆) δ 10.36 (s, 1H), 10.14 (s, 1H), 9.04 (s, 1H), 7.97 (dd, *J* = 9.1, 6.1 Hz, 1H), 7.46 (t, *J* = 9.0 Hz, 1H), 7.40 - 7.29 (m, 3H), 7.26 - 7.21 (m, 1H), 7.18 (s, 1H), 4.59 - 4.43 (m, 2H), 4.37 - 4.26 (m, 2H), 3.92 (s, 1H), 3.84 - 3.73 (m, 1H), 3.69 - 3.51 (m, 7H), 3.35 (s, 3H), 2.82 - 2.63 (m, 3H), 2.46 - 2.19 (m, 13H), 2.09 - 1.96 (m, 3H), 1.70 - 1.61 (m, 6H), 1.55 - 1.39 (m, 5H), 1.31 - 1.22 (m, 4H), 1.10 - 0.90 (m, 4H).

### Example 173: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1H,3H)-dione

### Step 1: preparation of tert-butyl 3-(2-((R)-3-(4-((3-(5-(2,4-dioxotetrahydropyrimidin-1(2H)-yl)-2-fluoro-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

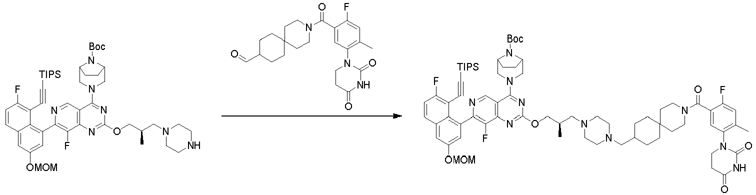

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)pyrido[4,3-*d*]pyrimidin-4-yl)-3,8-diazabicyclo[3.2.1]octane-8-carboxylate (150 mg, 0.17 mmol) and 3-(5-(2,4-dioxotetrahydropyrimidin-1(2*H*)-yl)-2-fluoro-4-methylbenzoyl)-3-azaspiro[5.5]undecane-9-carbaldehyde (112 mg, 0.26 mmol) in DCM/AcOH (1.5 mL/0.15 mL), and the mixture was stirred at 30 °C for 2 h. NaBH(OAc)₃ (108 mg, 0.51 mmol) was added to the mixture in an ice bath, and the resulting mixture was stirred for 2 h. The reaction mixture was directly concentrated, and the resulting crude product was purified by silica gel column chromatography (DCM:MeOH = 100:1 to 10:1) to give *tert-butyl* 3-(2-((R)-3-(4-((3-(5-(2,4-dioxotetrahydropyrimidin-1(2*H*)-yl)-2-fluoro-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 657.5.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1H,3H)-dione

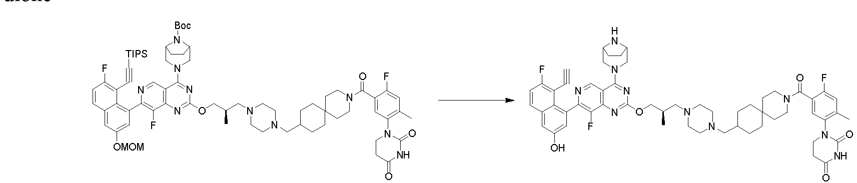

Cesium fluoride (144 mg, 0.95 mmol) was added to a stirred solution of *tert-butyl* 3-(2-((R)-3-(4-((3-(5-(2,4-dioxotetrahydropyrimidin-1(2*H*)-yl)-2-fluoro-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (125 mg, 0.095 mmol) in DMF (1 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. An aqueous Na₂CO₃ solution was added to the reaction liquid, and the mixture was adjusted to pH > 7 and extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-4-fluoro-2-methylphenyl)dihydropyrimidine-2,4(1*H*,3*H*)-dione.

LC-MS: (ESI, m/z): [M+H]⁺ = 1013.5.

¹H NMR (400 MHz, DMSO-*d*₆) δ 10.38 (s, 1H), 10.14 (s, 1H), 9.03 (s, 1H), 8.01 - 7.93 (m, 1H), 7.46 (t, *J=* 9.0 Hz, 1H), 7.40 - 7.37 (m, 1H), 7.35 - 7.31 (m, 1H), 7.26 - 7.20 (m, 1H), 7.18 - 7.15 (m, 1H), 4.52 - 4.42 (m, 2H), 4.35 - 4.26 (m, 1H), 4.12 - 4.00 (m, 1H), 3.93 (s, 1H), 3.80 - 3.72 (m, 1H), 3.65 - 3.50 (m, 7H), 3.23 - 3.15 (m, 2H), 2.78 - 2.66 (m, 2H), 2.39 - 2.02 (m, 17H), 1.73 - 1.59 (m, 6H), 1.54 - 1.38 (m, 5H), 1.34 - 1.22 (m, 2H), 1.13 - 1.01 (m, 3H), 1.00 - 0.94 (m, 4H).

### Example 174: 1-(5-(9-((4-(3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

### Step 1: preparation of tert-butyl 3-(2-(3-(4-benzylpiperazin-1-yl)-2-methylpropoxy)-7-bromo-8-fluoroquinazolin-4-yl)-3,8-diazabicyclo[3.2.1]octane-8-carboxylate

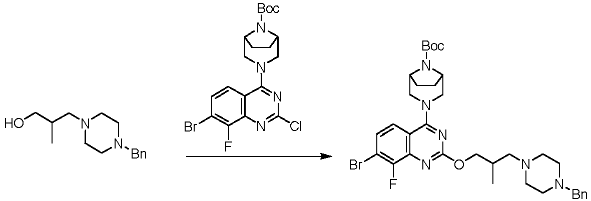

*tert-Butyl* 3-(7-bromo-2-chloro-8-fluoroquinazolin-4-yl)-3,8-diazabicyclo[3.2.1]octane-8-carboxylate (500 mg, 1.06 mmol) and 3-(4-benzylpiperazin-1-yl)-2-methylpropan-1-ol (342 mg, 1.38 mmol) were dissolved in ACN (10 mL), and Cs₂CO₃ (1.04 g, 3.18 mmol) and DABCO (24 mg,0.212 mmol) were added. The reaction liquid was reacted at room temperature for 2 h. After the reaction was completed, the mixture was filtered to remove the solid, and the filtrate was concentrated to give a crude product, which was purified by silica gel column chromatography (PE:EA = 1:1) to give *tert*-butyl 3-(2-(3-(4-benzylpiperazin-1-yl)-2-methylpropoxy)-7-bromo-8-fluoroquinazolin-4-yl)-3,8-diazabicyclo[3.2.1]octane-8-carboxylate.

LC-MS: (ESI, m/z): [M+H]⁺ = 683.3.

### Step 2: preparation of tert-butyl 3-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

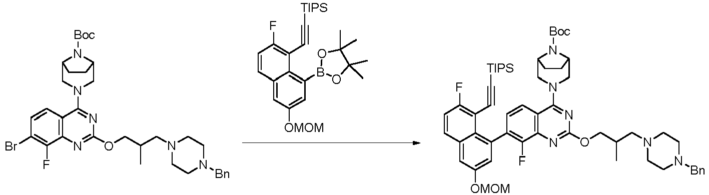

Cata CXium Pd G3 (105 mg, 0.16 mmol) and cesium carbonate (786 mg, 2.41 mmol) were added to a mixed solution of *tert-butyl* 3-(2-(3-(4-benzylpiperazin-1-yl)-2-methylpropoxy)-7-bromo-8-fluoroquinazolin-4-yl)-3,8-diazabicyclo[3.2.1]octane-8-carboxylate (550 mg, 0.804 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (495 mg, 0.97 mmol) in 1,4-dioxane (6 mL) and H₂O (1 mL). The mixture was reacted at 85 °C for 16 h under nitrogen atmosphere. Water (20 mL) was added to the reaction liquid, and then the mixture was extracted with ethyl acetate (30 mL × 3). The organic phases were combined, dried over anhydrous sodium sulfate, and concentrated under reduced pressure. The concentrate was purified by silica gel column chromatography (PE:EA = 1:1) to give *tert*-butyl 3-(2-(3-(4-benzylpiperazin-1-yl)-2-methylpropoxy)-8-fluoro-7-(7-fluoro-3-(methoxymethoxy)-8-((triisopropylsilyl) ethynyl)naphthalen-1-yl)quinazolin-4-yl)-3,8-diazabicyclo[3.2.1]octane-8-carboxylate.

LC-MS: (ESI, m/z): [M+H]⁺ = 989.6.

### Step 3: preparation of tert-butyl 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)quinazolin-4-yl)-3,8-diazabicyclo[3.2.1]octane-8-carboxylate

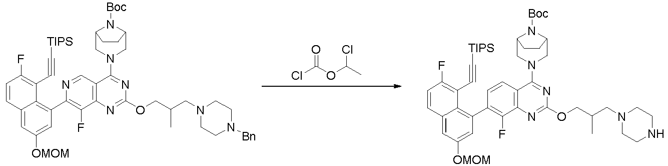

*tert-Butyl* 3-(2-(3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (600 mg, 0.61 mmol) was dissolved in DCM (6 mL), and 1-chloroethyl carbonochloridate (262 mg, 1.83 mmol) and DIEA (157 mg, 1.22 mmol) were added. The mixture was reacted at room temperature for 1 h and concentrated under reduced pressure. The concentrate was dissolved in MeOH (3 mL), and the mixture was heated to 50 °C, and reacted for 30 min. After the reaction was completed, the mixture was concentrated under reduced pressure. The concentrate was purified by silica gel column chromatography (DCM:MeOH (NH₃) = 15:1) to give *tert*-butyl 3-(8-fluoro-7-(7-fluoro-3-(methoxymethoxy)-8-((triisopropylsilyl)ethynyl) naphthalen-1-yl)-2-(2-methyl-3-(piperazin-1-yl)propoxy)quinazolin-4-yl)-3,8-diazabicyclo[3.2.1]octane-8-carboxylate.

LC-MS: (ESI, m/z): [M+H]⁺ = 899.5.

### Step 4: preparation of tert-butyl 3-(2-(3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

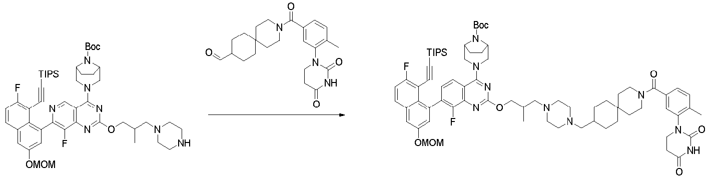

Tetraisopropyl titanate (0.5 mL) was added to a stirred solution of *tert*-butyl 3-(8-fluoro-7-(7-fluoro-3-(methoxymethoxy)-8-((triisopropylsilyl)ethynyl)naphthalen-1-yl)-2-(2-methyl-3-(piperazin-1-yl)propoxy)quinazolin-4-yl)-3,8-diazabicyclo[3.2.1]octane-8-carboxylate (150 mg, 0.17 mmol) and 3-(3-(2,4-dioxotetrahydropyrimidin-1(2*H*)-yl)-4-methylbenzoyl)-3-azaspiro[5.5]undecane-9-carbaldehyde (103 mg, 0.25 mmol) in DCM/AcOH (3 mL/0.1 mL), and the mixture was stirred at 30 °C for 2 h. NaBH(OAc)₃ (237 mg, 0.84 mmol) was added to the mixture in an ice bath, and the resulting mixture was stirred for 2 h. The mixture was concentrated, and the resulting concentrate was purified by silica gel column chromatography (DCM:MeOH = 1:0 to 4:1) to give *tert-butyl* 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M/2+H]⁺ = 647.9.

### Step 5: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

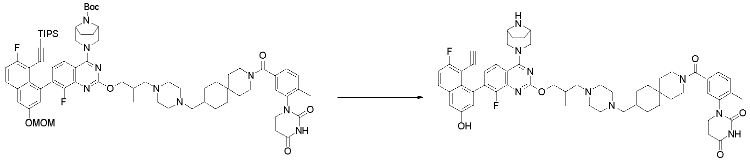

Cesium fluoride (74 mg, 0.49 mmol) was added to a solution of *tert*-butyl 3-(2-(3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (125 mg, 0.097 mmol)) in DMF (2 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M/2+H]⁺ = 497.7.

¹H NMR (400 MHz, DMSO-*d*₆) δ 10.37 (s, 1H), 10.12 (s, 1H), 7.96 (dd, *J* = 9.2 Hz, 6.0 Hz, 1H), 7.72 (d, *J* = 8.4 Hz, 1H), 7.45 (t, *J* = 8.8 Hz, 1H), 7.36 - 7.21 (m, 3H), 7.24 (d, *J* = 8 Hz, 1H),7.18 - 7.10 (m, 1H), 7.06 (d, *J =* 2.8 Hz, 1H), 4.07 - 3.95 (m, 1H), 3.85 - 3.71 (m, 1H), 3.63 - 3.38 (m, 7H), 2.82 - 2.64 (m, 2H), 2.47 - 2.23 (m, 9H), 2.22 - 1.98 (m, 9H), 1.77 - 1.61 (m, 6H), 1.54 - 1.22 (m, 8H), 1.12 - 0.89 (m, 7H).

### Example 191: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-hydroxypropyl)piperazin-1-yl)methyl)-3-azaspiro[5.5]undecane-3-carbonyl)-2-methoxyphenyl)dihydropyrimidine-2,4(1H,3H)-dione

### Step 1: preparation of (R)-3-(4-benzylpiperazin-1-yl)propane-1,2-diol

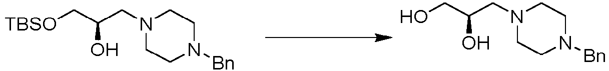

(*R*)-1-(4-Benzylpiperazin-1-yl)-3-((*tert*-butyldimethylsilyl)oxy)propan-2-ol (5.00 g, 13.70 mmol) was dissolved in ethanol (500 mL), and concentrated hydrochloric acid (10 mL) was added in an ice bath. The mixture was reacted for 2 h. After the reaction was completed, the mixture was concentrated to remove ethanol, and water (50 mL) was added. The mixture was extracted with methyl *tert*-butyl ether (50 mL). A saturated sodium carbonate solution was added to the aqueous phase to adjust the pH > 7, and then the mixture was extracted with ethyl acetate/isopropanol (30 mL × 3). The EA phases were combined, washed with a saturated NaCl solution, dried over anhydrous Na₂SO₄, and filtered, and the filtrate was concentrated. The resulting crude product was purified by silica gel column chromatography (PE:EA = 4:1) to give (*R*)-3-(4-benzylpiperazin-1-yl)propane-1,2-diol.

LC-MS: (ESI, m/z): [M+H]⁺ = 251.2.

### Step 2: preparation of tert-butyl 3-(2-((R)-3-(4-benzylpiperazin-1-yl)-2-hydroxypropoxy)-7-chloro-8-fluoropyrido[4,3-d]pyrimidin-4-yl)-3,8-diazabicyclo[3.2.1]octane-8-carboxylate

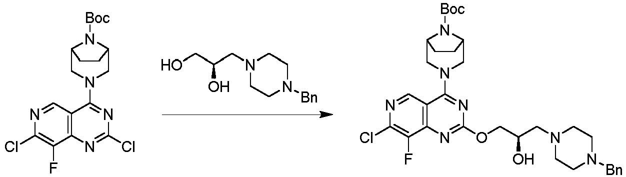

*tert*-Butyl 3-(2,7-dichloro-8-fluoropyrido[4,3-*d*]pyrimidin-4-yl)-3,8-diazabicyclo[3.2.1]octane-8-carboxylate (1.50 g, 3.50 mmol) and (*R*)-3-(4-benzylpiperazin-1-yl)propane-1,2-diol (1.75 g, 7.00 mmol) were dissolved in acetonitrile (20 mL) and added to a sealed tube reactor, and Cs₂CO₃ (2.28 g, 7.00 mmol) and DABCO (39 mg, 0.35 mmol) were added. The mixture was reacted at 30 °C for 3 h. After the reaction was completed, the mixture was filtered, and the filtrate was concentrated. The resulting crude product was purified by silica gel column chromatography (PE:EtOAc = 10:1 to 1:2) to give *tert-butyl* 3-(2-((*R*)-3-(4-benzylpiperazin-1-yl)-2-hydroxypropoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 642.3.

### Step 3: preparation of tert-butyl 3-(2-((R)-3-(4-benzylpiperazin-1-yl)-2-hydroxypropoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

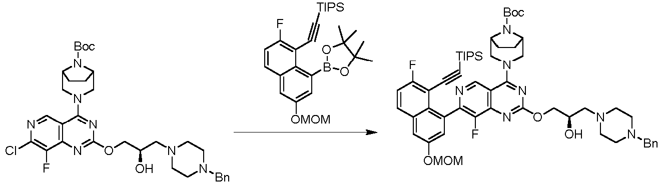

CataCXium A Pd G3 (114 mg, 0.16 mmol) and cesium carbonate (1.02 g, 3.12 mmol) were added to a mixed solution of *tert-butyl* 3-(2-((*R*)-3-(4-benzylpiperazin-1-yl)-2-hydroxypropoxy)-7-chloro-8-fluoropyrido[4,3-*d*]pyrimidin-4-yl)-3,8-diazabicyclo[3.2.1]octane-8-carboxylate (1.00 g, 1.56 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (1.04 g, 2.03 mmol) in 1,4-dioxane (10 mL) and H₂O (2 mL). The mixture was reacted at 85 °C for 16 h under nitrogen atmosphere. Water (10 mL) was added to the reaction liquid, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 20:1) to give *tert*-butyl 3-(2-((*R*)-3-(4-benzylpiperazin-1-yl)-2-hydroxypropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 496.8.

### Step 4: preparation of tert-butyl 3-(2-((R)-3-(4-benzylpiperazin-1-yl)-2-((tertbutyldimethylsilyl)oxy)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

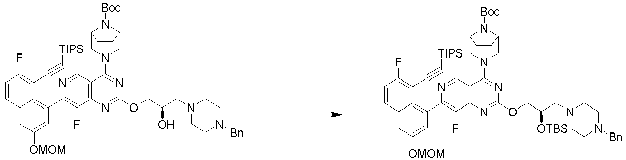

*tert-Butyl* 3-(2-((*R*)-3-(4-benzylpiperazin-1-yl)-2-hydroxypropoxy)-8-fluoro-7-(7-fluoro-3-(methoxymethoxy)-8-((triisopropylsilyl)ethynyl )naphthalen-1-yl)pyrido[4,3-*d*]pyrimidin-4-y1)-3,8-diazabicyclo[3.2.1]octane-8-carboxylate (800 mg, 0.81 mmol) and imidazole (109 mg, 1.60 mmol) were dissolved in dichloromethane, and TBSCl (159 mg, 1.05 mmol) was added. The mixture was reacted at 25 °C for 5 h. The reaction liquid was concentrated, and the resulting crude product was purified by silica gel column chromatography (DCM:MeOH = 100:1 to 20:1) to give *tert*-butyl 3-(2-((*R*)-3-(4-benzylpiperazin-1-yl)-2-((*tert*-butyldimethylsilyl)oxy)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 553.9.

### Step 5: preparation of tert-butyl 3-(2-((R)-2-((tert-butyldimethylsilyl)oxy)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

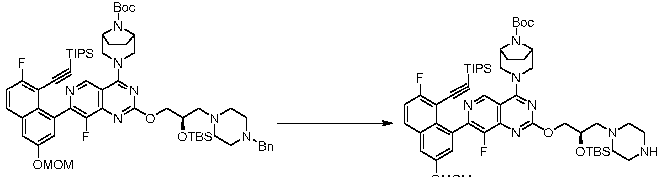

*tert-Butyl* 3-(2-((*R*)-3-(4-benzylpiperazin-1-yl)-2-((*tert*-butyldimethylsilyl)oxy)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (200 mg, 0.18 mmol) was dissolved in DCM (2 mL), and DIEA (46 mg, 0.36 mmol) and 1-chloroethyl chloroformate (39 mg, 0.27 mmol) were added. The mixture was reacted at room temperature for 1 h and concentrated under reduced pressure. MeOH (3 mL) was added to the concentrate, and the mixture was heated to 50 °C and reacted for 1 h. After the reaction was completed, the mixture was concentrated under reduced pressure, and the concentrate was purified by silica gel column chromatography (DCM:MeOH (NH₃) = 20:1) to give *tert*-butyl 3-(2-((R)-2-((*tert*butyldimethylsilyl)oxy)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 508.9.

### Step 6: preparation of tert-butyl 3-(2-((R)-2-((tert-butyldimethylsilyl)oxy)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

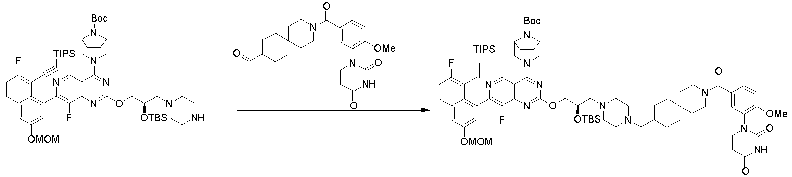

Tetraisopropyl titanate (0.3 mL) was added to a solution of *tert*-butyl 3-(2-((*R*)-2-((*tert*butyldimethylsilyl)oxy)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (90 mg, 0.089 mmol) and 3-(3-(2,4-dioxotetrahydropyrimidin-1(2*H*)-yl)-4-methoxybenzoyl)-3-azaspiro[5.5]undecane-9-carbaldehyde (76 mg, 0.18 mmol) in DCM/AcOH (1 mL/0.1 mL), and the mixture was stirred at 30 °C for 2 h. NaBH(OAc)₃ (57 mg, 0.26 mmol) was added to the mixture in an ice bath, and the resulting mixture was stirred for 2 h. The mixture was concentrated, and the resulting crude product was purified by silica gel column chromatography (DCM:MeOH = 100:1 to 10:1) to give *tert-butyl* 3-(2-((*R*)-2-((*tert*-butyldimethylsilyl)oxy)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 714.5.

### Step 7: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-hydroxypropyl)piperazin-1-yl)methyl)-3-azaspiro[5.5]undecane-3-carbonyl)-2-methoxyphenyl)dihydropyrimidine-2,4(1H,3H)-dione

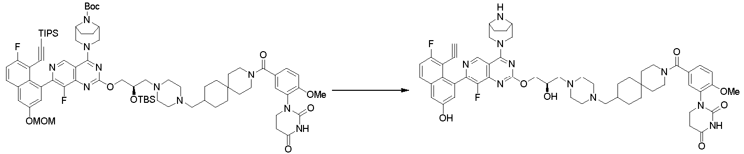

Cesium fluoride (62 mg, 0.41 mmol) was added to a stirred solution of *tert*-butyl 3-(2-((*R*)-2-((*tert*butyldimethylsilyl)oxy)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (75 mg, 0.0525 mmol) in DMF (1 mL), and the mixture was stirred at room temperature for 2 h. Water (10 mL) was added to the reaction liquid, and the mixture was extracted with ethyl acetate (10 mL × 2). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-hydroxypropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 507.2.

¹H NMR (400 MHz, DMSO-*d*₆) δ 10.33 (s, 1H), 10.15 (s, 1H), 9.03 (s, 1H), 7.97 (dd, *J* = 9.3, 5.9 Hz, 1H), 7.46 (t, *J=* 9.0 Hz, 1H), 7.41 - 7.33 (m, 2H), 7.32 (d, *J=* 2.0 Hz, 1H), 7.18 - 7.12 (m, 2H), 4.88 (t, *J= 4.6* Hz, 1H), 4.51 - 4.39 (m, 2H), 4.36 - 4.25 (m, 1H), 4.23 - 4.10 (m, 1H), 4.06 - 3.96 (m, 1H), 3.95 - 3.88 (m, 1H), 3.84 (s, 3H), 3.68 - 3.34 (m, 9H), 2.68 (t, *J=* 6.5 Hz, 2H), 2.46 - 2.12 (m, 11H), 2.10 - 1.91 (m, 3H), 1.68 - 1.62 (m, 5H), 1.54 - 1.39 (m, 5H), 1.31 - 1.22 (m, 3H), 1.10 - 0.94 (m, 4H).

### Example 207: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5] undecane-3-carbonyl)-2-methoxyphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methoxybenzoyl)-3-azaspiro[5.5 Jundecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

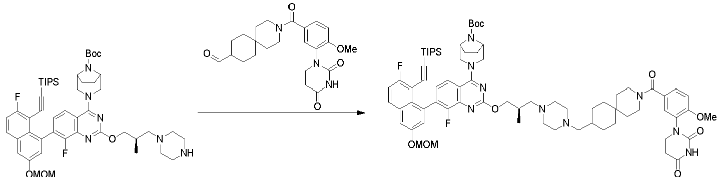

Tetraisopropyl titanate (237 mg, 834.05 µmol) and glacial acetic acid (0.3 mL) were added to a stirred solution of *tert-butyl* 3-(8-fluoro-7-(7-fluoro-3-(methoxymethoxy)-8-((triisopropylsilyl)ethynyl)naphthalen-1-yl)-2-((*R*)-2-methyl-3-(piperazin-1-yl)propoxy)quinazolin-4-yl)-3,8-diazabicyclo[3.2.1]octane-8-carboxylate (150 mg, 166.81 µmol) and 3-(3-(2,4-dioxotetrahydropyrimidin-1(2*H*)-yl)-4-methoxybenzoyl)-3-azaspiro[5.5]undecane-9-carbaldehyde (128 mg, 300.36 µmol) in dichloromethane (10 mL) at room temperature, and the mixture was stirred at 25 °C for 2 h. Then sodium triacetoxyborohydride (106 mg, 500.43 µmol) was added, and the mixture was reacted at 25 °C for another 16 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, a 1% solution of ammonia in methanol) to give *tert-butyl* 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1310.6.

### Step 2: preparation of tert-butyl 3-(2-((R)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate

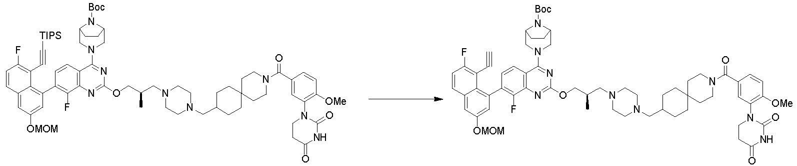

Cesium fluoride (133 mg, 877.39 µmol) was added to a solution of *tert-butyl* 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate (115.0 mg, 87.74 µmol) in *N*,*N*-dimethylformamide (8 mL) at room temperature, and the mixture was reacted at 25 °C for 2 h under nitrogen atmosphere. After the reaction was completed, the reaction liquid was poured into water (60 mL), and the mixture was extracted with ethyl acetate (20 mL × 3). The organic phases were washed with water (60 mL) and saturated brine (60 mL), combined, dried over anhydrous sodium sulfate, filtered, and concentrated under reduced pressure to give *tert-butyl* 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate.

MS(ESI)*m*/*z*: [M+H]⁺ = 1154.5.

### Step 3: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

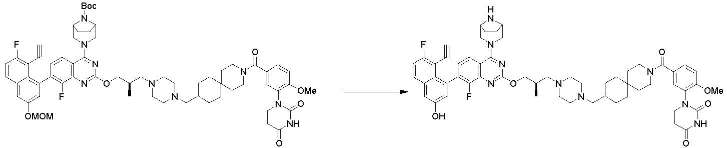

A hydrogen chloride/1,4-dioxane solution (4.0 M, 5 mL) was added to a solution of the crude product of *tert-butyl* 3-(2-((*R*)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate (104.2 mg, 87.74 µmol) in 1,4-dioxane (2.5 mL) at 0 °C, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro [5.5]undecane-3-carbonyl)-2-methoxyphenyl)dihydropyrimidine-2,4(1*H*,3*H*)-dione.

LC-MS: (ESI, m/z): [M+H]⁺ = 1010.4.

¹H NMR (400 MHz, DMSO-*d*₆) δ 10.33 (s, 1H), 7.98 - 7.94 (m, 1H), 7.72 (d, *J =* 8.4 Hz, 1H), 7.46 (t, *J=* 8.8 Hz, 1H), 7.38 - 7.32 (m, 2H), 7.32 (d, *J=* 1.6 Hz, 1H), 7.18 - 7.14 (m, 2H), 7.07 (d, *J =* 2.0 Hz, 1H), 4.45 - 4.38 (m, 1H), 4.30 - 4.20 (m, 2H), 4.05 - 3.99 (m, 1H), 3.90 - 3.80 (m, 4H), 3.61 - 3.58 (m, 2H), 3.50 - 3.46 (m, 3H), 2.70 - 2.67 (m, 3H), 2.45 - 2.05 (m, 12H), 2.05 - 1.95 (m, 3H), 1.75 - 1.60 (m, 6H), 1.60 - 1.35 (m, 6H), 1.35 - 1.20 (m, 4H), 1.20 - 0.85 (m, 8H).

### Example 243: 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-ethylphenyl)dihydropyrimidine-2,4(1H,3H)-dione

### Step 1: preparation of benzyl 4-((3-(3-(2,4-dioxotetrahydropyrimidin-1(2H)-yl)-4-ethylbenzoyl)-3-azaspiro[5 .5]undecan-9-yl)methyl)piperazine-1-carboxylate

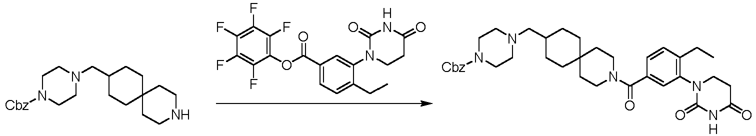

Pentafluorophenyl 3-(2,4-dioxotetrahydropyrimidin-1(2*H*)-yl)-4-ethylbenzoate (1.3 g, 3.12 mmol) and *N*,*N*-diisopropylethylamine (3.3 g, 26.0 mmol) were added to a solution of benzyl 4-((3-azaspiro[5.5]undecan-9-yl)methyl)piperazine-1-carboxylate (1 g, 2.60 mmol) in *N*,*N*-dimethylformamide (10 mL), and the mixture was stirred at room temperature for 2 h. After the reaction was completed, water (20 mL) was added, and the mixture was extracted with ethyl acetate (20 mL × 2). The organic phases were combined, washed with saturated sodium chloride (20 mL × 3), dried over anhydrous sodium sulfate, and concentrated under reduced pressure. The resulting crude product was purified by silica gel column chromatography (dichloromethane:methanol = 20:1) to give benzyl 4-((3-(3-(2,4-dioxotetrahydropyrimidin-1(2*H*)-yl)-4-ethylbenzoyl)-3-azaspiro[5.5]undecan-9-yl)methyl)piperazine-1-carboxylate.

LC-MS: (ESI, m/z): [M+H]⁺ = 630.4.

### Step 2: preparation of 1-(2-ethyl-5-(9-(piperazin-1-ylmethyl)-3-azaspiro[5.5]undecane-3-carbonyl)phenyl)dihydropyrimidine-2,4(1H,3H)-dione

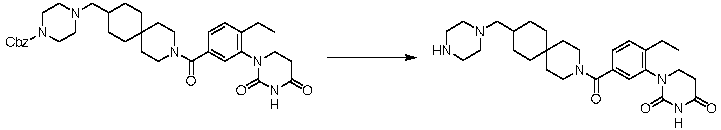

Pd/C (123 mg) was added to a solution of benzyl 4-((3-(3-(2,4-dioxotetrahydropyrimidin-1(2*H*)-yl)-4-ethylbenzoyl)-3-azaspiro[5.5]undecan-9-yl)methyl)piperazine-1-carboxylate (730 mg, 1.16 mmol) in methanol (5 mL), and the mixture was stirred under hydrogen at 25 °C for 1 h. After the reaction was completed, the mixture was filtered and concentrated under reduced pressure, and the resulting crude product was purified by silica gel column chromatography (dichloromethane:methanol = 20:1) to give 1-(2-ethyl-5-(9-(piperazin-1-ylmethyl)-3-azaspiro[5.5]undecane-3-carbonyl)phenyl)dihydropyrimidine-2,4(1*H*,3*H*)-dione.

LC-MS: (ESI, m/z): [M+H]⁺ = 496.4.

### Step 3: preparation of tert-butyl 3-(2-((1-((4-((3-(3-(2,4-dioxotetrahydropyrimidin-1(2H)-yl)-4-ethylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

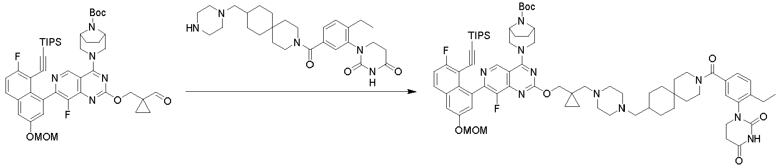

Tetraisopropyl titanate (170 mg, 0.60 mmol) was added to a solution of 1-(2-ethyl-5-(9-(piperazin-1-ylmethyl)-3-azaspiro[5.5]undecane-3-carbonyl)phenyl)dihydropyrimidine-2,4(1H,3H)-dione (90 mg, 0.18 mmol) and benzyl 3-(8-fluoro-7-(7-fluoro-3-(methoxymethoxy)-8-((triisopropylsilyl)ethynyl)naphthalen-1-yl)-2-((1-formylcyclopropyl)methoxy)pyrido[4,3-*d*]pyrimidin-4-yl)-3,8-diazabicyclo[3.2.1]octane-8-carboxylate (100 mg, 0.12 mmol) in dichloromethane (2 mL) at room temperature, and the mixture was stirred at 25 °C for 16 h. Sodium triacetoxyborohydride (85 mg, 0.40 mmol) was added to the mixture at room temperature, and the mixture was reacted at 25 °C for another 3 h. The reaction liquid was concentrated under reduced pressure and the resulting crude product was purified by a preparative thin-layer chromatography plate (dichloromethane:methanol = 10:1, 0.1% NH₃) to give *tert-*butyl 3-(2-((1-((4-((3-(3-(2,4-dioxotetrahydropyrimidin-1(2*H*)-yl)-4-ethylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 1321.8.

### Step 4: preparation of 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)cyclopropyl)methyl)piperazin-1-yl)methyl)-3-azaspiro[5.5]undecane-3-carbonyl)-2-ethylphenyl)dihydropyrimidine-2,4(1H,3H)-dione

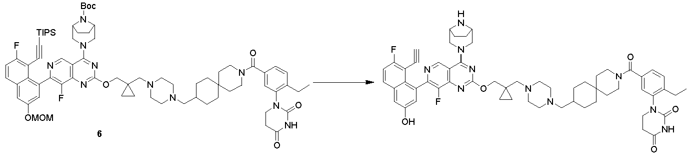

CsF (64 mg, 0.42 mmol) was added to a solution of *tert-butyl* 3-(2-((1-((4-((3-(3-(2,4-dioxotetrahydropyrimidin-1(2*H*)-yl)-4-ethylbenzoyl)-3-azaspiro[5.5]undecan-9-yl)methyl)piperazin-1-yl)methyl)cyclopropyl)methoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (112 mg, 0.085 mmol) in DMF (1 mL), and the mixture was stirred at room temperature for half an hour. Water (10 mL) was added, and the mixture was extracted with ethyl acetate (10 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, and concentrated under reduced pressure, and the resulting crude product was purified by preparative high-performance liquid chromatography to give 1-(5-(9-((4-((1-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)cyclopropyl)methyl)piperazin-1-yl)methyl)-3-azaspiro[5.5]undecane-3-carbonyl)-2-ethylphenyl)dihydropyrimidine-2,4(1*H*,3*H*)-dione.

LC-MS: (ESI, m/z): [M+H]⁺ = 1021.5.

¹H NMR (400 MHz, DMSO-*d*₆) δ 10.37 (s, 1H), 10.14 (s, 1H), 9.02 (s, 1H), 7.97 (dd, *J =* 9.2, 6.1 Hz, 1H), 7.46 (t, *J =* 9.0 Hz, 1H), 7.40 - 7.34 (m, 2H), 7.32 - 7.27 (m, 2H), 7.16 (d, *J =* 2.4 Hz, 1H), 4.48 (d, *J =* 11.2 Hz, 1H), 4.31 - 4.18 (m, 3H), 3.92 (s, 1H), 3.87 - 3.72 (m, 1H), 3.68 - 3.39 (m, 7H), 2.82 - 2.65 (m, 2H), 2.60 - 2.53 (m, 2H), 2.44 - 1.93 (m, 15H), 1.72 - 1.62 (m, 6H), 1.53 - 1.26 (m, 7H), 1.16 (t, *J=* 7.5 Hz, 3H), 1.11 - 0.94 (m, 4H), 0.69 - 0.58 (m, 2H), 0.45 - 0.34 (m, 2H).

### Example 249: 1-(5-(9-((4-((R)-3-((4-(3,8-diazabicyclo[32.1]octan-3-yl)-7-(8-ethynyl-7-fluoro-3-hydroxynaphthalen-1-yl)-8-fluoropyrido[4,3-d]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl (S)-(1-(4-benzylpiperazin-1-yl)-3-hydroxy-1-oxopropan-2-yl)carboxylate

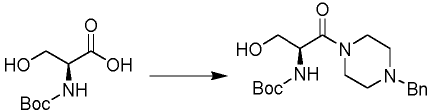

PyBOP (2.7 g, 5.2 mmol) and DIEA (3.3 mL, 18.8 mmol) were added to a solution of (*tert-*butoxycarbonyl)-*L*-serine (1.16 g, 5.64 mmol) in DCM (20 mL). The mixture was stirred at 0 °C to room temperature for 2 h. The reaction liquid was concentrated and purified by silica gel column chromatography (PE:EA = 100:1 to 1:1) to give *tert-butyl* (S)-(1-(4-benzylpiperazin-1-yl)-3-hydroxy-1-oxopropan-2-yl)carboxylate.

LC-MS: (ESI, m/z): [M+H]⁺ = 364.3.

### Step 2: preparation of tert-butyl (R)-(1-(4-benzylpiperazin-1-yl)-3-hydroxypropan-2-yl)carboxylate

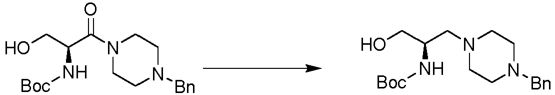

*tert*-Butyl (*S*)-(1-(4-benzylpiperazin-1-yl)-3-hydroxy-1-oxopropan-2-yl)carboxylate (2 g, 5.5 mmol) was dissolved in THF (40 mL), and DIBAL-H (27.5 mL, 27.5 mmol) was added. The mixture was stirred in an ice-water bath at room temperature overnight. After the reaction was completed, the mixture was quenched with a potassium sodium tartrate solution and extracted with ethyl acetate (50 mL × 2). The organic phase was washed with saturated brine, dried over anhydrous sodium sulfate, and filtered, and the filtrate was concentrated and purified by silica gel column chromatography (DCM:MeOH = 100:1 to 10:1) to give *tert-butyl* (*R*)-(1-(4-benzylpiperazin-1-yl)-3-hydroxypropan-2-yl)carboxylate.

LC-MS: (ESI, m/z): [M+H]⁺ = 350.3.

### Step 3: preparation of tert-butyl 3-(2-((R)-3-(4-benzylpiperazin-1-yl)-2-((tert-butoxycarbonyl)amino)propoxy)-7-chloro-8-fluoropyrido[4,3-d]pyrimidin-4-yl)-3,8-diazabicyclo[3.2.1]octane-8-carboxylate

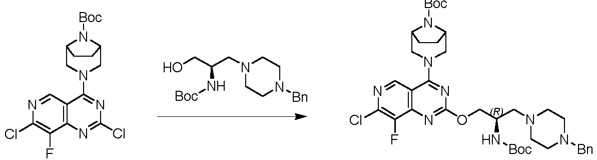

*tert*-Butyl 3-(2,7-dichloro-8-fluoropyrido[4,3-*d*]pyrimidin-4-yl)-3,8-diazabicyclo[3.2.1]octane-8-carboxylate (735 mg, 1.72 mmol), DABCO (96 mg, 0.86 mmol), and cesium carbonate (1.4 g, 4.2 mmol) were added to a solution of *tert*-butyl (*R*)-(1-(4-benzylpiperazin-1-yl)-3-hydroxypropan-2-yl)carboxylate (0.9 g, 2.57 mmol) in acetonitrile (20 mL), and the mixture was stirred at room temperature for 1 h. After the reaction was completed, the mixture was concentrated and purified by silica gel column chromatography (DCM:MeOH = 20:1) to give *tert-butyl* 3-(2-((*R*)-3-(4-benzylpiperazin-1-yl)-2-((*tert-*butoxycarbonyl)amino)propoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 741.4.

### Step 4: preparation of tert-butyl 3-(2-((R)-3-(4-benzylpiperazin-1-yl)-2-((tert-butoxycarbonyl)amino)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

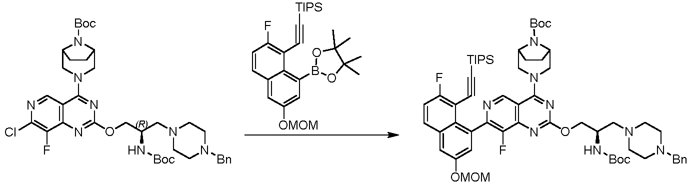

Ruphos Pd G3 (36 mg, 0.043 mmol) and potassium phosphate (228 mg, 1.08 mmol) were added to a mixed solution of *tert*-butyl 3-(2-((*R*)-3-(4-benzylpiperazin-1-yl)-2-((*tert-*butoxycarbonyl)amino)propoxy)-7-chloro-8-fluoropyrido[4,3-*d*]pyrimidin-4-yl)-3,8-diazabicyclo[3.2.1]octane-8-carboxylate (320 mg, 0.43 mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (332 mg, 0.65 mmol) in 1,4-dioxane (5 mL) and H₂O (1 mL). The mixture was reacted at 100 °C for 16 h under nitrogen atmosphere. Water (20 mL) was added to the reaction liquid, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 20:1) to give *tert*-butyl 3-(2-((*R*)-3-(4-benzylpiperazin-1-yl)-2-((*tert-*butoxycarbonyl)amino)propoxy)-8-fluoro-7-(7-fluoro-3-(methoxymethoxy)-8-((triisopropylsilyl)ethynyl) naphthalen-1-yl)pyrido[4,3-*d*]pyrimidin-4-yl)-3,8-diazabicyclo[3.2.1]octane-8-carboxylate.

LC-MS: (ESI, m/z): [M+H]⁺ = 1091.6.

### Step 5: preparation of tert-butyl 3-(2-((R)-2-((tert-butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

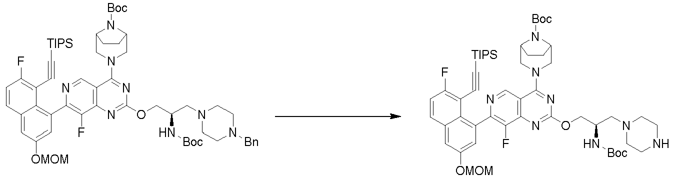

*tert*-Butyl 3-(2-((*R*)-3-(4-benzylpiperazin-1-yl)-2-((*tert*-butoxycarbonyl)amino)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (320 mg, 29 mmol) was dissolved in DCM (15 mL), and 1-chloroethyl carbonochloridate (84 mg, 0.58 mmol) and DIEA (76 mg, 0.58 mmol) were added. The mixture was reacted at room temperature for 1 h and concentrated under reduced pressure. MeOH (15 mL) was added to the concentrate for dissolution, and the mixture was heated to 50 °C and reacted for 1 h. After the reaction was completed, the mixture was concentrated under reduced pressure, and the concentrate was purified by silica gel column chromatography (DCM:MeOH (NH₃) = 15:1) to give *tert-*butyl 3-(2-((R)-2-((*tert*-butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 501.4.

### Step 6: preparation of tert-butyl 3-(2-((R)-2-((tert-butoxycarbonyl)amino)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

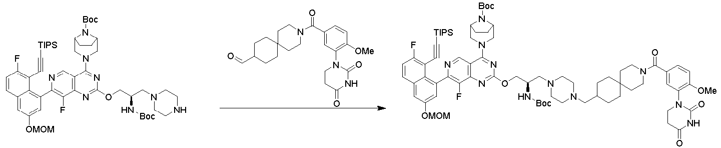

3-(3-(2,4-Dioxotetrahydropyrimidin-1(2*H*)-yl)-4-methoxybenzoyl)-3 -azaspiro [5 .5]undecane-9-carbaldehyde (155 mg, 0.36 mmol) and tetraisopropyl titanate (340 mg, 1.2 mmol) were added to a solution of *tert*-butyl 3-(2-((R)-*2*-((*tert*-butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (240 mg, 0.24 mmol) in dichloromethane (15 mL) and acetic acid (0.5 mL), and the mixture was stirred at 25 °C for 1 h. NaBH(OAc)₃ (155 mg, 0.72 mmol) was then added at 0 °C, and the mixture was stirred at 25 °C for 1 h. After the reaction was completed, H₂O (15 mL) was added, and the mixture was extracted with ethyl acetate (15 mL × 3). The combined organic phase was dried over anhydrous sodium sulfate, filtered, and concentrated in vacuum. The crude product was purified by silica gel column chromatography (DCM:MeOH = 10:1) to give *tert*-butyl 3-(2-((*R*)-2-((*tert-*butoxycarbonyl)amino)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 707.0.

### Step 7: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1H,3H)-dione

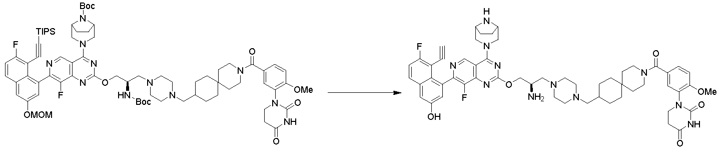

*tert*-Butyl 3-(2-((*R*)-2-((*tert*-butoxycarbonyl)amino)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo
[3.2.1]octane-8-carboxylate (300 mg, 0.21 mmol) was dissolved in DMF (5 mL), and CsF (167 mg, 1.1 mmol) was added. The mixture was reacted at room temperature for 0.5 h. After the reaction was completed, the reaction liquid was diluted with H₂O (15 mL) and extracted with ethyl acetate (15 mL × 3). The combined organic phase was dried over Na₂SO₄, filtered, and concentrated under reduced pressure. HCl/1,4-dioxane (8 N, 5 mL) was added to the crude product, and the mixture was stirred at room temperature for 0.5 h. After the reaction was completed, H₂O (15 mL) was added, and the mixture was adjusted to pH > 7 with an aqueous Na₂CO₃ solution and extracted with ethyl acetate (15 mL × 3). The combined organic phase was dried over Na₂SO₄, filtered, and concentrated under reduced pressure. The crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1012.5.

¹H NMR (400 MHz, DMSO-*d*₆) δ 10.32 (s, 1H), 10.13 (br, 1H), 8.75 (s, 1H), 7.95 (dd, *J* = 8.6, 6.0 Hz, 1H), 7.44 (td, *J =* 9.0, 2.2 Hz, 1H), 7.40 - 7.27 (m, 3H), 7.17 - 7.11 (m, 2H), 4.95 - 4.59 (m, 1H), 4.56 - 4.07 (m, 3H), 3.94 (s, 1H), 3.84 (s, 3H), 3.69 - 3.46 (m, 11H), 2.68 (t, *J* = 6.4 Hz, 2H), 2.48 - 2.37 (m, 6H), 2.35 - 2.18 (m, 4H), 2.11 - 1.99 (m, 2H), 1.76 - 1.58 (m, 6H), 1.57 - 1.37 (m, 5H), 1.34 - 1.23 (m, 2H), 1.14 - 0.91 (m, 4H).

### Example 250: 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl (1-(4-benzylpiperazin-1-yl)-3-hydroxy-1-oxopropan-2-yl)carboxylate

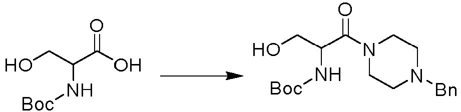

PyBOP (2.7 g, 5.2 mmol) and DIEA (3.3 mL, 18.8 mmol) were added to a solution of (*tert-*butoxycarbonyl)serine (1.16 g, 5.64 mmol) in DCM (20 mL). The mixture was stirred at 0 °C to room temperature for 2 h. The reaction liquid was concentrated and purified by silica gel column chromatography (PE:EA = 100:1 to 1:1) to give *tert*-butyl (1-(4-benzylpiperazin-1-yl)-3-hydroxy-1-oxopropan-2-yl)carboxylate.

LC-MS: (ESI, m/z): [M+H]⁺ = 364.3.

### Step 2: preparation of tert-butyl (1-(4-benzylpiperazin-1-yl)-3-hydroxypropan-2-yl)carboxylate

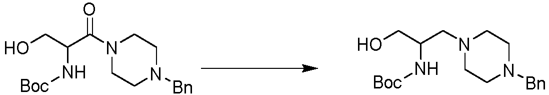

*tert-Butyl* (1-(4-benzylpiperazin-1-yl)-3-hydroxy-1-oxopropan-2-yl)carboxylate (0.7 g, 1.9 mmol) was dissolved in THF (20 mL), and DIBAL-H (9.6 mL, 9.6 mmol) was added. The mixture was stirred in an ice-water bath at room temperature overnight. After the reaction was completed, the mixture was quenched with a potassium sodium tartrate solution and extracted with ethyl acetate (50 mL × 2). The organic phase was washed with saturated brine, dried over anhydrous sodium sulfate, and filtered, and the filtrate was concentrated and purified by silica gel column chromatography (DCM:MeOH = 100:1 to 10:1) to give *tert*-butyl (1-(4-benzylpiperazin-1-yl)-3-hydroxypropan-2-yl)carboxylate.

LC-MS: (ESI, m/z): [M+H]⁺ = 350.3.

### Step 3: preparation of tert-butyl 3-(2-(3-(4-benzylpiperazin-1-yl)-2-((tert-butoxycarbonyl)amino)propoxy)-7-chloro-8-fluoropyrido[4,3-d]pyrimidin-4-yl)-3,8-diazabicyclo[3.2.1]octane-8-carboxylate

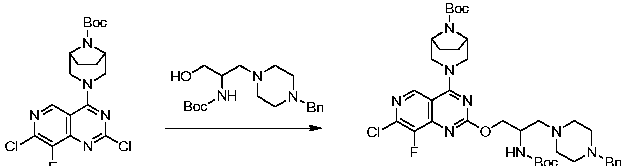

*tert*-Butyl 3-(2,7-dichloro-8-fluoropyrido[4,3-*d*]pyrimidin-4-yl)-3,8-diazabicyclo[3.2.1]octane-8-carboxylate (240 mg, 0.56 mmol), DABCO (31.4 mg, 0.28 mmol), and cesium carbonate (456 mg, 1.4 mmol) were added to a solution of *tert*-butyl (1-(4-benzylpiperazin-1-yl)-3-hydroxypropan-2-yl)carboxylate (0.39 g, 1.12 mmol) in acetonitrile (10 mL). The mixture was stirred at room temperature for 1 h. After the reaction was completed, the mixture was concentrated and purified by silica gel column chromatography (DCM:MeOH = 20:1) to give *tert*-butyl 3-(2-(3-(4-benzylpiperazin-1-yl)-2-((*tert-*butoxycarbonyl)amino)propoxy)-7-chloro-8-fluoropyrido [4,3-*d*]pyrimidin-4-yl)-3,8-diazabicyclo[3.2.1]octane-8-carboxylate.

LC-MS: (ESI, m/z): [M+H]⁺ = 741.4.

### Step 4: preparation of tert-butyl 3-(2-(3-(4-benzylpiperazin-1-yl)-2-((tert-butoxycarbonyl)amino)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

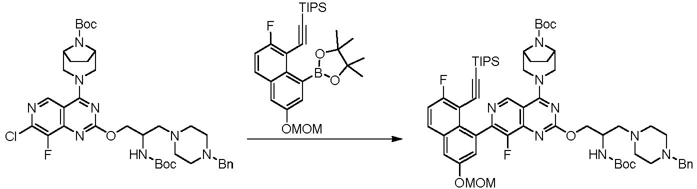

Ruphos Pd G3 (15 mg, 0.018 mmol) and potassium phosphate (93 mg, 0.44 mmol) were added to a mixed solution of *tert-butyl* 3-(2-(3-(4-benzylpiperazin-1-yl)-2-((*tert*-butoxycarbonyl)amino)propoxy)-7-chloro-8-fluoropyrido[4,3-*d*]pyrimidin-4-yl)-3,8-diazabicyclo[3.2.1]octane-8-carboxylate (130 mg, 0.175 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (135 mg, 0.263 mmol) in 1,4-dioxane (5 mL) and H₂O (1 mL). The mixture was reacted at 100 °C for 16 h under nitrogen atmosphere. Water (10 mL) was added to quench the reaction, and the mixture was extracted with ethyl acetate (10 mL × 3). The organic phases were combined, dried over anhydrous sodium sulfate, and concentrated under reduced pressure. The concentrate was purified by silica gel column chromatography (DCM:MeOH = 100:1 to 20:1) to give *tert*-butyl 3-(2-(3-(4-benzylpiperazin-1-yl)-2-((*tert*-butoxycarbonyl)amino)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 546.4.

### Step 5: preparation of tert-butyl 3-(2-(2-((tert-butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

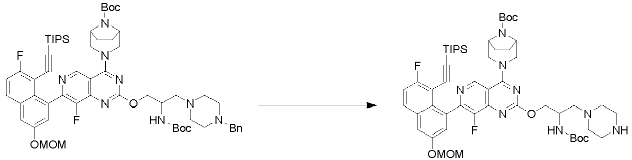

*tert-Butyl* 3-(2-(3-(4-benzylpiperazin-1-yl)-2-((*tert*-butoxycarbonyl)amino)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (110 mg, 0.1 mmol) was dissolved in DCM (5 mL), and 1-chloroethyl carbonochloridate (29 mg, 0.2 mmol) and DIEA (26 mg, 0.2 mmol) were added. The mixture was reacted at room temperature for 1 h and concentrated under reduced pressure. MeOH (5 mL) was added to the concentrate for dissolution, and the mixture was heated to 50 °C and reacted for 1 h. After the reaction was completed, the mixture was concentrated under reduced pressure, and the concentrate was purified by silica gel column chromatography (DCM:MeOH (NH₃) = 15:1) to give *tert*-butyl 3-(2-(2-((*tert-*butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 501.9.

### Step 6: preparation of tert-butyl 3-(2-(2-((tert-butoxycarbonyl)amino)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

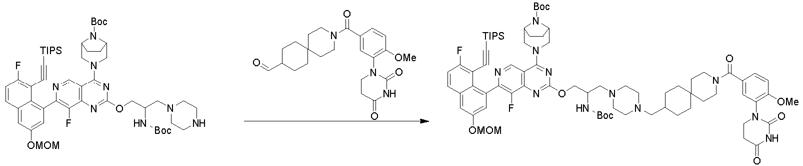

3-(3-(2,4-Dioxotetrahydropyrimidin-1(2*H*)-yl)-4-methoxybenzoyl)-3-azaspiro [5.5]undecane-9-carbaldehyde (55 mg, 0.13 mmol) and tetraisopropyl titanate (121 mg, 0.43 mmol) were added to a solution of *tert-butyl* 3-(2-(2-((*tert*-butoxycarbonyl)amino)-3-(piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (86 mg, 0.086 mmol) in dichloromethane (1 mL) and acetic acid (0.1 mL), and the mixture was stirred at 25 °C for 1 h. NaBH(OAc)₃ (55 mg, 0.26 mmol) was then added at 0 °C, and the mixture was stirred at 25 °C for 1 h. After the reaction was completed, H₂O (5 mL) was added, and the mixture was extracted with ethyl acetate (5 mL × 3). The combined organic phase was dried over anhydrous sodium sulfate, filtered, and concentrated in vacuum. The crude product was purified by silica gel column chromatography (DCM:MeOH = 10:1) to give *tert*-butyl 3-(2-(2-((*tert*-butoxycarbonyl)amino)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 707.0.

### Step 7: preparation of 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1H,3H)-dione

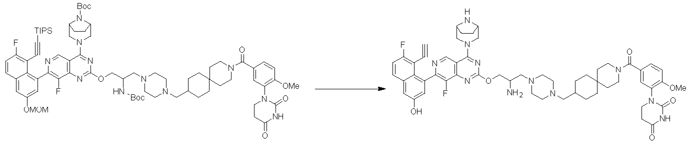

*tert*-Butyl 3-(2-(2-((*tert*-butoxycarbonyl)amino)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)propoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (70 mg, 0.05 mmol) was dissolved in DMF (1 mL), and CsF (38 mg, 0.25 mmol) was added. The mixture was reacted at room temperature for 0.5 h. After the reaction was completed, the reaction liquid was diluted with H₂O (5 mL) and extracted with ethyl acetate (5 mL × 3). The combined organic phase was dried over Na₂SO₄, filtered, and concentrated under reduced pressure. HCl/1,4-dioxane (8 N, 1 mL) was added to the crude product, and the mixture was stirred at room temperature for 0.5 h. After the reaction was completed, H₂O (5 mL) was added, and the mixture was adjusted to pH > 7 with an aqueous Na₂CO₃ solution and extracted with ethyl acetate (5 mL × 3). The combined organic phase was dried over Na₂SO₄, filtered, and concentrated under reduced pressure. The crude product was purified by preparative high-performance liquid chromatography to give 1-(5-(9-((4-(3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-aminopropyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M+H]⁺ = 1012.5.

¹H NMR (400 MHz, CD₃OD) δ 8.78 (s, 1H), 8.52 (s, 1H), 7.93 - 7.79 (m, 1H), 7.43 (d, *J* = 8.6 Hz, 1H), 7.38 (s, 1H), 7.35 - 7.28 (m, 2H), 7.18 (d, *J* = 8.4 Hz, 2H), 4.70 - 4.33 (m, 4H), 3.99 - 3.82 (m, 5H), 3.79 - 3.44 (m, 11H), 3.41 (s, 1H), 2.92 - 2.62 (m, 10H), 2.39 (s, 2H), 2.09 - 1.89 (m, 4H), 1.81 - 1.49 (m, 7H), 1.42 - 1.05 (m, 6H).

### Example 251: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5] undecane-3-carbonyl)-2-methylphenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

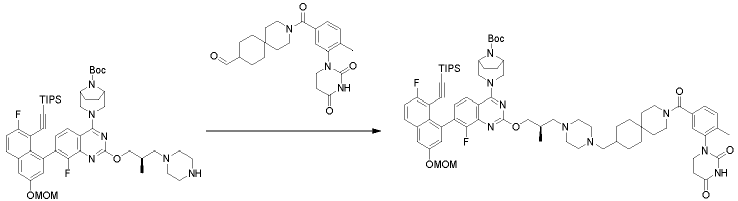

Tetraisopropyl titanate (237 mg, 834.05 µmol) and glacial acetic acid (0.3 mL) were added to a stirred solution of *tert-butyl* 3-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)quinazolin-4-yl)-3,8-diazabicyclo[3.2.1]octane-8-carboxylate (150 mg, 166.81 µmol) and 3-(3-(2,4-dioxotetrahydropyrimidin-1(2*H*)-yl)-4-methylbenzoyl)-3-azaspiro[5.5]undecane-9-carbaldehyde (124 mg, 300.26 µmol) in dichloromethane (10 mL) at room temperature, and the mixture was stirred at 25 °C for 2 h. Then sodium triacetoxyborohydride (106 mg, 500.43 µmol) was added, and the mixture was reacted at 25 °C for another 16 h. Methanol (5 mL) was added to quench the reaction, and the mixture was concentrated under reduced pressure at 40 °C. The resulting crude product was purified by a preparative thin-layer chromatography plate (dichloromethane:methanol = 10:1, a 1% solution of ammonia in methanol) to give *tert-butyl* 3-(2-((*R*)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate.

LC-MS: (ESI, m/z): [M+H]⁺ = 1294.6.

### Step 2: preparation of tert-butyl 3-(2-((R)-3-(4-((3-(3-(2,4-dioxotetrahydropyrimidin-1(2H)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate

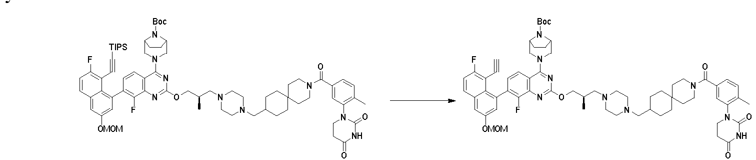

Cesium fluoride (197 mg, 1296.81 µmol) was added to a solution of *tert-butyl* 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)quinazolin-4-yl)-3,8-diazabicyclo[3.2.1]octane-8-carboxylate

(169.7 mg, 129.68 µmol) in *N*,*N*-dimethylformamide (8 mL) at room temperature, and the mixture was reacted at 25 °C for 2 h under nitrogen atmosphere. After the reaction was completed, the reaction liquid was poured into water (60 mL), and the mixture was extracted with ethyl acetate (20 mL × 3). The organic phases were washed with water (60 mL) and saturated brine (60 mL), and the combined organic phase was dried over anhydrous sodium sulfate, filtered, and concentrated under reduced pressure to give *tert*-butyl 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate.

LC-MS: (ESI, m/z): [M+H]⁺ = 1138.5.

### Step 3: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methylphenyl)dihydropyrimidine-2,4(1H,3H)-dione

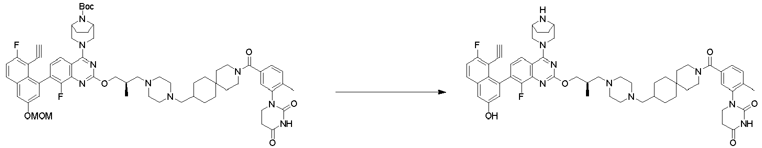

A hydrogen chloride/1,4-dioxane solution (4.0 M, 5 mL) was added to a solution of the crude product of *tert*-butyl 3-(2-((*R*)-3-(4-((3-(3-(2,4-dioxotetrahydropyrimidin-1(2*H*)-yl)-4-methylbenzoyl)-3-azaspiro[5.5]undecan-9-yl)methyl)piperazin-1-yl)-2-methylpropoxy)-7-(8-ethynyl-7-fluoro-3-(methoxymethoxy)naphthalen-1-yl)-8-fluoroquinazolin-4-yl)-3,8-diazabicyclo[3.2.1]octane-8-carboxylate (150.9 mg, 129.68 µmol) in 1,4-dioxane (2.5 mL) at 0 °C, and the mixture was reacted at room temperature for 1 h. After the reaction was completed, the mixture was concentrated under reduced pressure at 30 °C, and a solution of ammonia in methanol (5 mL) was added to the concentrate. Then the mixture was concentrated under reduced pressure at 30 °C. The resulting crude product was purified by a preparative thin-layer chromatography plate (dichloromethane:methanol = 10:1, 0.1% NH₃) and then purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoroquinazolin-2-yl)oxy)-2-methylpropyl)piperazin-1-yl)methyl)-3-azaspiro[5.5]undecane-3-carbonyl)-2-methylphenyl)dihydropyrimidine-2,4(1*H*,3*H*)-dione.

LC-MS: (ESI, m/z): [M+H]⁺ = 994.1.

¹H NMR (400 MHz, DMSO-*d*₆) δ 10.37 (s, 1H), 7.98 - 7.94 (m, 1H), 7.72 (d, *J =* 8.8 Hz, 1H), 7.46 (t, *J* = 8.0 Hz, 1H), 7.36 - 7.33 (m, 2H), 7.31 (s, 1H), 7.24 (d, *J* = 7.2 Hz, 1H), 7.18 - 7.14 (m, 1H), 7.07 (d, *J =* 2.4 Hz, 1H), 4.46 - 4.38 (m, 1H), 4.30 - 4.20 (m, 2H), 4.00 - 3.99 (m, 1H), 3.94 - 3.82 (m, 1H), 3.82 - 3.77 (m, 1H), 3.56 - 3.43 (m, 4H), 2.83 - 2.66 (m, 3H), 2.33 - 2.22 (m, 9H), 2.21 (s, 3H), 2.15 - 1.96 (m, 6H), 1.73 - 1.66 (m, 6H), 1.60 - 1.35 (m, 6H), 1.35 - 1.00 (m, 8H), 1.00 - 0.95 (m, 3H), 0.95 - 0.83 (m, 1H).

### Example 252: 1-(5-(9-((4-((R)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)-3-methylbutyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((R)-2-((4-benzylpiperazin-1-yl)methyl)-3-methylbutoxy)-7-chloro-8-fluoropyrido[4,3-d]pyrimidin-4-yl)-3,8-diazabicyclo[3.2.1]octane-8-carboxylate

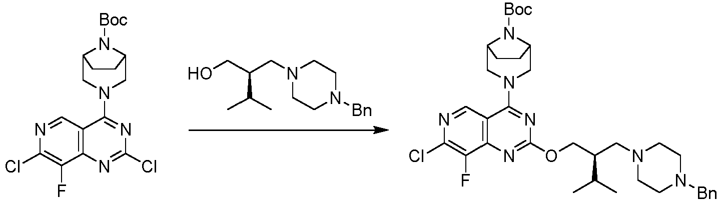

*tert*-Butyl 3-(2,7-dichloro-8-fluoropyrido[4,3-*d*]pyrimidin-4-yl)-3,8-diazabicyclo[3.2.1]octane-8-carboxylate (1.52 g, 3.03 mmol) was dissolved in THF (15 mL), and Cs₂CO₃ (2.28 g, 6.99 mmol) and (*R*)-2-((4-benzylpiperazin-1-yl)methyl)-3-methylbutan-1-ol () were added. The reaction liquid was heated to 90 °C and reacted for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated. The resulting crude product was purified by silica gel column chromatography (PE:EtOAc = 2:1) to give *tert*-butyl 3-(2-((*R*)-2-((4-benzylpiperazin-1-yl)methyl)-3-methylbutoxy)-7-chloro-8-fluoropyrido[4,3-*d*]pyrimidin-4-yl)-3,8-diazabicyclo[3.2.1]octane-8-carboxylate.

LC-MS: (ESI, m/z): [M+H]⁺ = 668.4.

### Step 2: preparation of tert-butyl 3-(2-((R)-2-((4-benzylpiperazin-1-yl)methyl)-3-methylbutoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

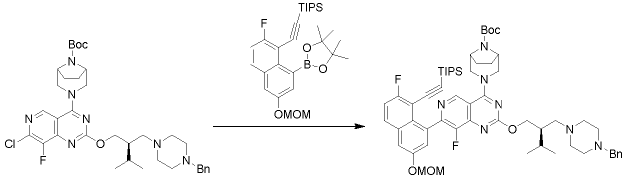

Cata CXium Pd G₃ (197 mg, 0.27 mmol) and cesium carbonate (1.58 g, 4.86 mmol) were added to a mixed solution of *tert*-butyl 3-(2-((*R*)-2-((4-benzylpiperazin-1-yl)methyl)-3-methylbutoxy)-7-chloro-8-fluoropyrido[4,3-*d*]pyrimidin-4-yl)-3,8-diazabicyclo[3.2.1]octane-8-carboxylate (900 mg, 1.35 mmol) and ((2-fluoro-6-(methoxymethoxy)-8-(4,4,5,5-tetramethyl-1,3,2-dioxaborolan-2-yl)naphthalen-1-yl)ethynyl) triisopropylsilane (830 mg, 1.62 mmol) in 1,4-dioxane (10 mL) and H₂O (2 mL). The mixture was reacted at 85 °C for 16 h under nitrogen atmosphere. Water (20 mL) was added to quench the reaction, and the mixture was extracted with ethyl acetate (30 mL × 3). The organic phases were combined, dried over anhydrous sodium sulfate, and concentrated under reduced pressure. The concentrate was purified by silica gel column chromatography (PE:EA = 1:1) to give *tert*-butyl 3-(2-((*R*)-2-((4-benzylpiperazin-1-yl)methyl)-3-methylbutoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 509.9.

### Step 3: preparation of tert-butyl 3-(8-fluoro-7-(7-fluoro-3-(methoxymethoxy)-8-((triisopropylsilyl)ethynyl)naphthalen-1-yl)-2-((R)-3-methyl-2-(piperazin-1-ylmethyl)butoxy)pyrido [4,3-d]pyrimidin-4-yl)-3,8-diazabicyclo[3.2.1]octane-8-carboxylate

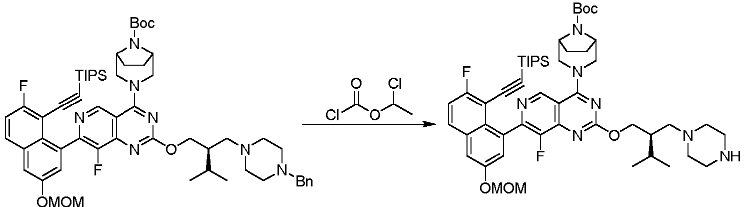

*tert*-Butyl 3-(2-((*R*)-2-((4-benzylpiperazin-1-yl)methyl)-3-methylbutoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (900 mg, 0.88 mmol) was dissolved in DCM (10 mL), and 1-chloroethyl carbonochloridate (378 mg,2.64 mmol) and DIEA (227 mg,1.0 mmol) were added. The mixture was reacted at room temperature for 1 h and concentrated under reduced pressure. MeOH (10 mL) was added to the concentrate for dissolution, and the mixture was heated to 50 °C and reacted for 50 min. After the reaction was completed, the mixture was concentrated under reduced pressure, and the concentrate was purified by silica gel column chromatography (DCM:MeOH (NH₃) = 15:1) to give *tert*-butyl 3-(8-fluoro-7-(7-fluoro-3-(methoxymethoxy)-8-((triisopropylsilyl)ethynyl)naphthalen-1-yl)-2-((R)-3-methyl-2-(piperazin-1-ylmethyl)butoxy)pyrido[4,3-*d*]pyrimidin-4-yl)-3,8-diazabicyclo[3.2.1]octane-8-carboxylate.

LC-MS: (ESI, m/z): [M+H]⁺ = 928.6.

### Step 4: preparation of tert-butyl 3-(2-((R)-2-((4-((3-(3-(2,4-dioxotetrahydropyrimidin-1(2H)-yl)-4-methoxybenzoyl)-3-azaspiro[5.5]undecan-9-yl)methyl)piperazin-1-yl)methyl)-3-methylbutoxy)-8-fluoro-7-(7-fluoro-3-(methoxymethoxy)-8-((triisopropylsilyl)ethynyl)naphthalen-1-yl)pyrido [4,3-d]pyrimidin-4-yl)-3,8-diazabicyclo[3.2.1]octane-8-carboxylate

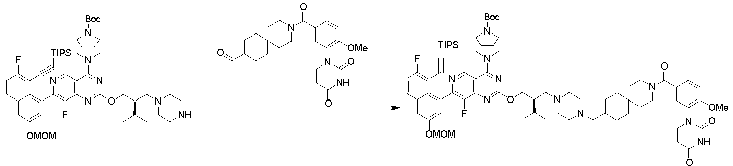

Tetraisopropyl titanate (0.5 mL) was added to a solution of *tert*-butyl 3-(8-fluoro-7-(7-fluoro-3-(methoxymethoxy)-8-((triisopropylsilyl)ethynyl)naphthalen-1-yl)-2-((*R*)-3-methyl-2-(piperazin-1-ylmethyl)butoxy)pyrido[4,3-*d*]pyrimidin-4-yl)-3,8-diazabicyclo[3.2.1]octane-8-carboxylate (150 mg, 0.162 mmol) and 3-(3-(2,4-dioxotetrahydropyrimidin-1(2*H*)-yl)-4-methoxybenzoyl)-3-azaspiro[5.5]undecane-9-carbaldehyde (104 mg, 0.243 mmol) in DCM/AcOH (2 mL/0.1 mL), and the mixture was stirred at 30 °C for 2 h. NaBH(OAc)₃ (108 mg, 0.486 mmol) was added to the mixture in an ice bath, and the resulting mixture was stirred for 2 h. The mixture was concentrated and purified by silica gel column chromatography (DCM:MeOH = 10:1) to give *tert*-butyl 3-(2-((*R*)-2-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)-3-methylbutoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 670.5.

### Step 5: preparation of 1-(5-(9-((4-((R)-2-(((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)methyl)-3-methylbutyl)piperazin-1-yl)methyl)-3-azaspiro[5.5]undecane-3-carbonyl)-2-methoxyphenyl)dihydropyrimidine-2,4(1H,3H)-dione

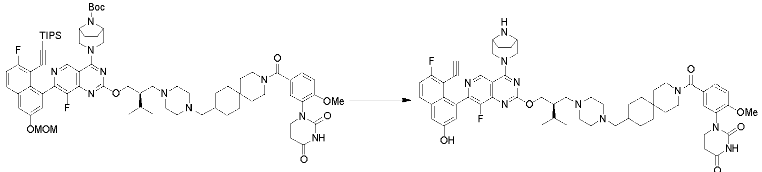

Cesium fluoride (84 mg, 0.55 mmol) was added to a solution of *tert*-butyl 3-(2-((*R*)-2-((4-((3-(3-(2,4-dioxotetrahydropyrimidin-1(2*H*)-yl)-4-methoxybenzoyl)-3-azaspiro[5.5]undecan-9-yl)methyl)piperazin-1-yl)methyl)-3-methylbutoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (145 mg, 0.11 mmol) in DMF (2 mL), and the mixture was stirred at room temperature for 30 min. Water (10 mL) was added to the reaction liquid, and the mixture was extracted with ethyl acetate (10 mL × 3). The organic phases were combined, dried over anhydrous sodium sulfate, and concentrated under reduced pressure. HCl/1,4-dioxane (6 N, 1.0 mL) was added to a stirred solution of the concentrate in 1,4-dioxane (2 mL), and the mixture was reacted at room temperature for 0.5 h. The reaction liquid was adjusted to pH > 7 with an aqueous Na₂CO₃ solution, and the mixture was extracted with EtOAc (20 mL × 2). The organic phase was washed with brine, dried over anhydrous Na₂SO₄, and concentrated. The crude product was purified by preparative high-performance liquid chromatography to give 1-(5-(9-((4-((*R*)-2-(((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)methyl)-3-methylbutyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, m/z): [M/2+H]⁺ = 520.2.

¹H NMR (400 MHz, DMSO-*d*₆) δ 10.32 (s, 1H), 10.14 (s, 1H), 9.02 (s, 1H), 8.04 - 7.89 (m, 1H), 7.46 (t, *J =* 9.0 Hz, 1H), 7.40 - 7.34 (m, 2H), 7.33 - 7.30 (m, 1H), 7.19 - 7.12 (m, 2H), 4.52 - 4.39 (m, 2H), 4.37 - 4.22 (m, 2H), 3.92 (d, *J =* 1.6 Hz, 1H), 3.84 (s, 3H), 3.65 - 3.37 (m, 9H), 2.70 - 2.65 (m, 2H), 2.46 - 2.13 (m 10H), 2.07 - 2.00 (m, 2H), 1.96 - 1.86 (m, 2H), 1.73 - 1.59 (m, 6H), 1.55 - 1.21(m, 8H), 1.16 - 0.82 (m, 11H).

### Example 253: 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1H,3H)-dione

### Step 1: preparation of tert-butyl 3-(2-((R)-3-(4-((3-(3-(2,4-dioxotetrahydropyrimidin-1(2H)-yl)-4-(methoxy-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazabicyclo[3.2.1]octane-8-carboxylate

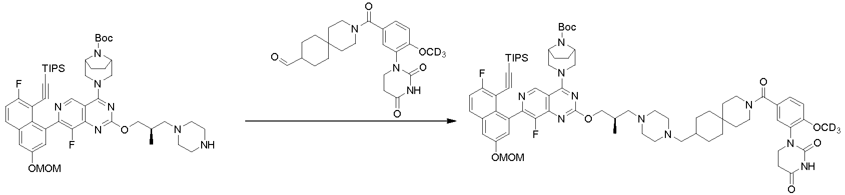

Tetraisopropyl titanate (158 mg, 0.555 mmol) was added to a solution of *tert*-butyl 3-(8-fluoro-7-(7-fluoro-3-(methoxymethoxy)-8-((triisopropylsilyl)ethynyl)naphthalen-1-yl)-2-((R)-2-methyl-3-(piperazin-1-yl)propoxy)pyrido[4,3-*d*]pyrimidin-4-yl)-3,8-diazabicyclo[3.2.1]octane-8-carboxylate (100 mg, 0.111 mmol) and 3-(3-(2,4-dioxotetrahydropyrimidin-1(2*H*)-yl)-4-(methoxy-d3)benzoyl)-3-azaspiro[5.5]undecane-9-carbaldehyde (57 mg, 0.133 mmol) in dichloromethane (2 mL) at room temperature, and the mixture was stirred at 25 °C for 16 h. Sodium triacetoxyborohydride (71 mg, 0.333 mmol) was added to the mixture at 0 °C, and the mixture was reacted at 25 °C for another 3 h. Methanol (5 mL) was added, and the mixture was concentrated under reduced pressure at 40 °C. The resulting crude product was purified by a preparative thin-layer chromatography plate (dichloromethane:methanol = 10:1, 0.1% NH₃) to give *tert*-butyl 3-(2-((*R*)-3-(4-((3-(3-(2,4-dioxotetrahydropyrimidin-1(2*H*)-yl)-4-(methoxy-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)
naphthalen-1-yl)pyrido[4,3-*d*]pyrimidin-4-yl)-3,8-diazabicyclo[3.2.1]octane-8-carboxylate.

LC-MS: (ESI, m/z): [M/2+H]⁺ = 658.0.

### Step 2: preparation of 1-(5-(9-((4-((R)-3-((4-(3,8-diazabicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1H,3H)-dione

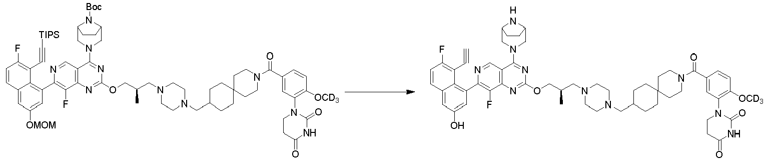

CsF (52 mg, 0.343 mmol) was added to a solution of *tert-butyl* 3-(2-((R)-3-(4-((3-(3-(2,4-dioxotetrahydropyrimidin-1(2*H*)-yl)-4-(methoxy-d3)benzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazabicyclo[3.2.1]octane-8-carboxylate (90 mg, 0.0685 mmol) in DMF (2 mL), and the mixture was stirred at room temperature for half an hour. Water (2 mL) was added, and the mixture was extracted with ethyl acetate (10 mL × 3). The organic phases were combined, dried over anhydrous sodium sulfate, and concentrated under reduced pressure. The intermediate obtained by concentration was dissolved in 1,4-dioxane (2 mL), and HCl/1,4-dioxane (6 N, 1 mL) was added. The mixture was reacted at room temperature for 0.5 h. The reaction liquid was adjusted to pH > 7 with an aqueous Na₂CO₃ solution and extracted with ethyl acetate (5 mL × 3). The combined organic phase was dried over Na₂SO₄, filtered, and concentrated under reduced pressure, and the resulting crude product was purified by preparative high-performance liquid chromatography to give 1-(5-(9-((4-((*R*)-3-((4-(3,8-diazabicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-(methoxy-d3)phenyl)dihydropyrimidine-2,4(1*H*,3*H*)-dione.

LC-MS: (ESI, m/z): [M/2+H]⁺ = 507.6.

¹H NMR (400 MHz, DMSO-*d*₆) δ 10.33 (s, 1H), 10.15 (s, 1H), 9.03 (s, 1H), 7.97 (dd, *J* = 9.2, 5.9 Hz, 1H), 7.46 (t, *J* = 9.0 Hz, 1H), 7.40 - 7.33 (m, 2H), 7.32 (d, *J* = 1.9 Hz, 1H), 7.17 (d, *J* = 2.4 Hz, 1H), 7.14 (d, *J* = 8.4 Hz, 1H), 4.53 - 4.40 (m, 2H), 4.36 - 4.23 (m, 1H), 4.14 - 4.00 (m, 1H), 3.94 (s, 1H), 3.66 - 3.41 (m, 8H), 2.68 (t, *J* = 6.7 Hz, 2H), 2.44 - 1.97 (m, 15H), 1.72 - 1.61 (m, 6H), 1.54 - 1.39 (m, 5H), 1.33 - 1.20 (m, 4H), 1.10 - 1.02 (m, 2H), 0.99 - 0.92 (m, 4H).

### Example 254: 1-(5-(9-((4-((S)-3-((4-(3,8-diazaspirobicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

### Step 1: preparation of tert-butyl 3-(2-((S)-3-(4-benzylpiperazin-1-yl)-2-methylpropoxy)-7-chloro-8-fluoropyrido[4,3-djpyrimidin-4-yl)-3,8-diazaspirobicyclo[3.2.1]octane-8-carboxylate

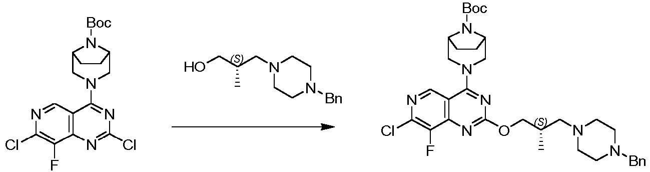

*tert*-Butyl 3-(2,7-dichloro-8-fluoropyrido[4,3-*d*]pyrimidin-4-yl)-3,8-diazaspirobicyclo[3.2.1]octane-8-carboxylate (1.50 g, 3.50 mmol) and (S)-3-(4-benzylpiperazin-1-yl)-2-methylpropan-1-ol (1.30 g, 5.26 mmol) were dissolved in THF (20 mL) in a sealed tube reactor, and Cs₂CO₃ (2.28 g, 7.0 mmol) was added. The reaction liquid was heated to 90 °C and reacted for 16 h. After the reaction was completed, the mixture was cooled to room temperature. Water (30 mL) was added, and the mixture was extracted with ethyl acetate (30 mL × 3). The organic phases were combined, washed with a saturated NaCl solution, dried over anhydrous Na₂SO₄, and filtered, and the filtrate was concentrated to give a crude product, which was purified by silica gel column chromatography (DCM:MeOH = 20:1) to give *tert*-butyl 3-(2-((*S*)-3-(4-benzylpiperazin-1-yl)-2-methylpropoxy)-7-chloro-8-fluoropyrido[4,3-*d*]pyrimidin-4-yl)-3,8-diazaspirobicyclo[3.2.1]octane-8-carboxylate.

LC-MS: (ESI, m/z): [M+H]⁺ = 640.4.

### Step 2: preparation of tert-butyl 3-(2-((S)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazaspirobicyclo[3.2.1]octane-8-carboxylate

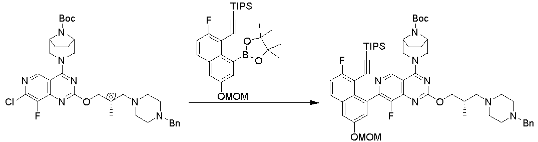

Ruphos Pd G3 (105 mg, 0.125 mmol) and potassium phosphate (796 mg, 3.75 mmol) were added to a mixed solution of *tert*-butyl 3-(2-((*S*)-3-(4-benzylpiperazin-1-yl)-2-methylpropoxy)-7-chloro-8-fluoropyrido[4,3-*d*]pyrimidin-4-yl)-3,8-diazaspirobicyclo[3.2.1]octane-8-carboxylate (800 mg, 1.25 mmol) and ((2-fluoro-6-(methoxymethoxy)-8-(4,4,5,5-tetramethyl-1,3,2-dioxaborolan-2-yl)naphthalen-1-yl)ethynyl)triisopropylsilane (769 mg, 1.50 mmol) in 1,4-dioxane/water (5.0 mL/1.0 mL). The mixture was reacted at 85 °C for 16 h under nitrogen atmosphere. Water (20 mL) was added, and the mixture was extracted with ethyl acetate (20 mL × 3). The organic phases were combined, dried over anhydrous sodium sulfate, and concentrated under reduced pressure. The concentrate was purified by silica gel column chromatography (DCM:MeOH = 20:1) to give *tert*-butyl 3-(2-((*S*)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazaspirobicyclo[3.2.1]octane-8-carboxylate.

LC-MS: (ESI, m/z): [M+H]⁺ = 990.6.

### Step 3: preparation of tert-butyl 3-(8-fluoro-7-(7-fluoro-3-(methoxymethoxy)-8-((triisopropylsilyl)ethynyl)naphthalen-1-yl)-2-((S)-2-methyl-3-(piperazin-1-yl)propoxy)pyrido[4,3-d]pyrimidin-4-yl)-3,8-diazaspirobicyclo[3.2.1]octane-8-carboxylate

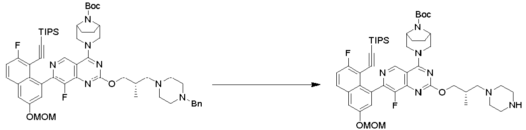

*tert-*Butyl 3-(2-((S)-3-(4-benzylpiperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazaspirobicyclo[3.2.1]octane-8-carboxylate (850 mg, 0.86 mmol) was dissolved in DCM (4 mL), and 1-chloroethyl chloroformate (369 mg, 2.58 mmol) and DIEA (333 mg, 2.58 mmol) were added. The mixture was reacted at room temperature for 1 h and then concentrated under reduced pressure. MeOH (5 mL) was added to the concentrate for dissolution, and the mixture was heated to 50 °C and reacted for 1 h. After the reaction was completed, the mixture was concentrated under reduced pressure. The concentrate was purified by silica gel column chromatography (DCM:MeOH (NH₃) = 20:1) to give *tert*-butyl 3-(8-fluoro-7-(7-fluoro-3-(methoxymethoxy)-8-((triisopropylsilyl)ethynyl)naphthalen-1-yl)-2-((*S*)-2-methyl-3-(piperazin-1-yl)propoxy)pyrido[4,3-*d*]pyrimidin-4-yl)-3,8-diazaspirobicyclo[3.2.1]octane-8-carboxylate.

LC-MS: (ESI, m/z): [M+H]⁺ = 900.5.

### Step 4: preparation of tert-butyl 3-(2-((S)-3-(4-((3-(3-(2,4-dioxotetrahydropyrimidin-1(2H)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-d]pyrimidin-4-yl)-3,8-diazaspirobicyclo[3.2.1]octane-8-carboxylate

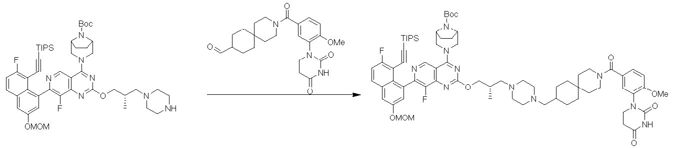

Tetraisopropyl titanate (0.5 mL) was added to a stirred solution of *tert-*butyl 3-(8-fluoro-7-(7-fluoro-3-(methoxymethoxy)-8-((triisopropylsilyl)ethynyl)naphthalen-1-yl)-2-((*S*)-2-methyl-3-(piperazin-1-yl)propoxy)pyrido[4,3-*d*]pyrimidin-4-yl)-3,8-diazaspirobicyclo[3.2.1]octane-8-carboxylate (150 mg, 0.167 mmol) and 3-(3-(2,4-dioxotetrahydropyrimidin-1(2*H*)-yl)-4-methoxybenzoyl)-3-azaspiro[5.5]undecane-9-carbaldehyde (107 mg, 0.251 mmol) in DCM/AcOH (1.5 mL/0.1 mL), and the mixture was stirred at 30 °C for 2 h. NaBH(OAc)₃ (106 mg, 0.501 mmol) was added to the mixture in an ice bath, and the resulting mixture was stirred for 2 h. The mixture was concentrated and purified by silica gel column chromatography (DCM:MeOH = 100:1 to 10:1) to give *tert*-butyl 3-(2-((S)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazaspirobicyclo[3.2.1]octane-8-carboxylate.

LC-MS: (ESI, m/z): [M/2+H]⁺ = 657.0.

### Step 5: preparation of 1-(5-(9-((4-((S)-3-((4-(3,8-diazaspirobicyclo[3.2.1]octan-3-yl)-7-(8-ethynyl-7-fluoro-3-hydroxynaphthalen-1-yl)-8-fluoropyrido[4,3-d]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione

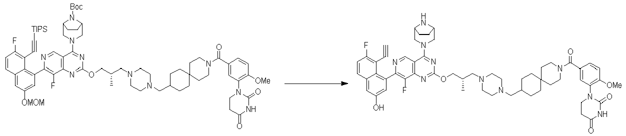

Cesium fluoride (191 mg, 1.26 mmol) was added to a stirred solution of *tert*-butyl 3-(2-((S)-3-(4-((3-(3-(2,4-dioxotetrahydropyrimidin-1(2*H*)-yl)-4-methoxybenzoyl)-3-azaspiro[5.5]undecan-9-yl)methyl)piperazin-1-yl)-2-methylpropoxy)-8-fluoro-7-(7-fluoro-3-(methoxymethoxy)-8-((triisopropylsilyl)ethynyl)naphthalen-1-yl)pyrido[4,3-*d*]pyrimidin-4-yl)-3,8-diazaspirobicyclo[3.2.1]octane-8-carboxylate (165 mg, 0.126 mmol) in DMF (1 mL), and the mixture was stirred at room temperature for 30 min. Water (10 mL) was added to the reaction liquid, and the mixture was extracted with ethyl acetate (10 mL × 2). The organic phases were combined, washed with a saturated sodium chloride solution, dried over anhydrous sodium sulfate, and concentrated under reduced pressure. HCl/1,4-dioxane (6 N, 1.0 mL) was added to a stirred solution of the concentrate in 1,4-dioxane (1.0 mL), and the mixture was reacted at room temperature for 0.5 h. The reaction liquid was adjusted to pH > 7 with an aqueous Na₂CO₃ solution, and the mixture was extracted with EtOAc (10 mL × 2). The organic phase was washed with brine, dried over anhydrous Na₂SO₄, and concentrated. The crude product was purified by preparative high-performance liquid chromatography to give 1-(5-(9-((4-((*S*)-3-((4-(3,8-diazaspirobicyclo[3.2.1]octan-3-yl)-7-(8-ethynyl-7-fluoro-3-hydroxynaphthalen-1-yl)-8-fluoropyrido[4,3-*d*]pyrimidin-2-yl)oxy)-2-methylpropyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, m/z): [M+H]⁺ = 1011.5.

¹H NMR (400 MHz, DMSO-*d*₆) δ 10.32 (s, 1H), 10.14 (s, 1H), 9.03 (s, 1H), 7.97 (dd, *J =* 9.2, 5.9 Hz, 1H), 7.49 - 7.43 (m, 1H), 7.40 - 7.34 (m, 2H), 7.31 (d, *J=* 2.0 Hz, 1H), 7.19 - 7.12 (m, 2H), 4.51 - 4.40 (m, 2H), 4.35 - 4.26 (m, 1H), 4.12 - 4.01 (m, 1H), 3.93 (s, 1H), 3.84 (s, 3H), 3.66 - 3.37 (m, 10H), 2.68 *(t, J* = 6.4 Hz, 2H), 2.45 - 1.97 (m, 14H), 1.71 - 1.60 (m, 6H), 1.57 - 1.35 (m, 5H), 1.33 - 1.31 (m, 2H), 1.15 - 0.91 (m, 7H).

The compounds in Table 1 were synthesized using methods similar to those shown in the preceding examples

**Table 1**

| Example | Compound structure | LC-MS: (ESI, m/z): [M+H]⁺ |
|---|---|---|
| 91 | 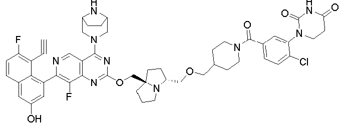 | 960 |
| 92 | 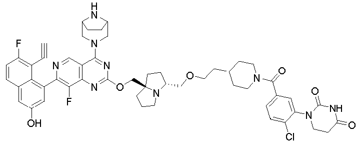 | 974 |
| 93 | 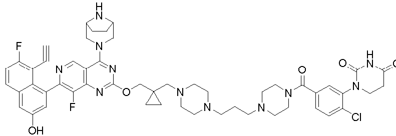 | 988 |
| 94 | 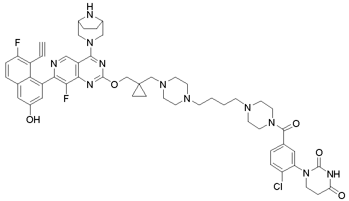 | 1002 |
| 95 | 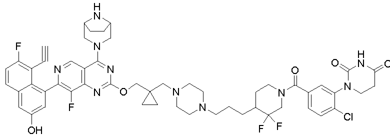 | 1023 |
| 96 | 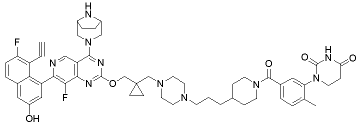 | 967 |
| 97 | 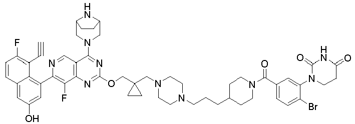 | 1031 |
| 98 | 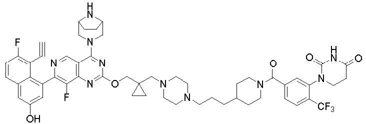 | 1021 |
| 99 | 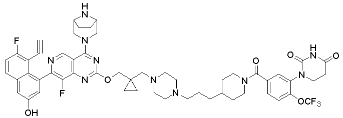 | 1037 |
| 101 | 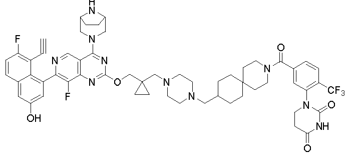 | 1061 |
| 111 | 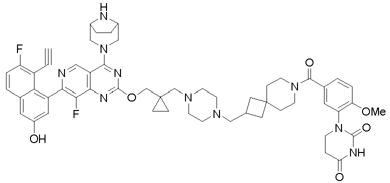 | 995 |
| 112 | 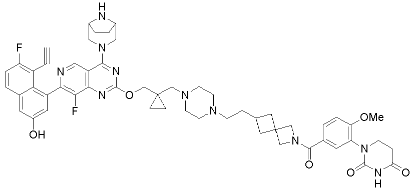 | 981 |
| 113 | 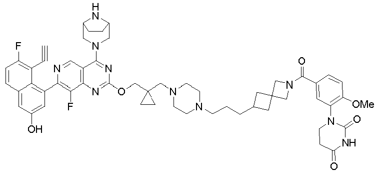 | 995 |
| 114 | 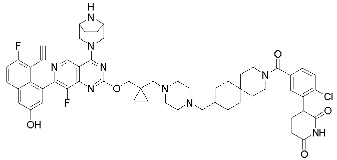 | 1026 |
| 115 | 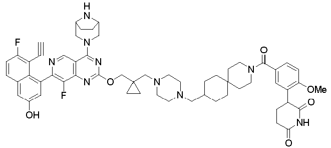 | 1022 |
| 116 | 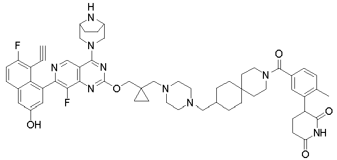 | 1006 |
| 117 | 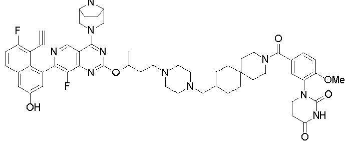 | 1011 |
| 118 | 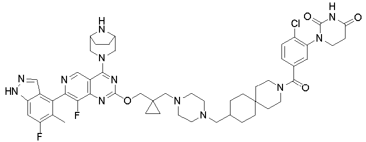 | 991 |
| 119 | 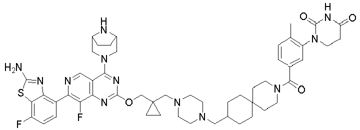 | 989 |
| 120 | 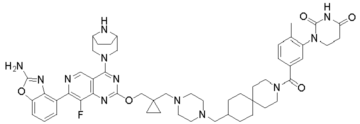 | 955 |
| 121 | 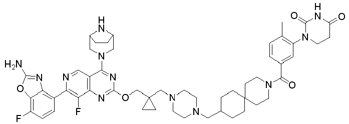 | 973 |
| 122 | 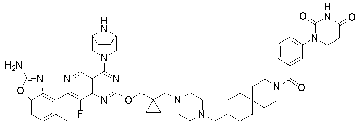 | 969 |
| 123 | 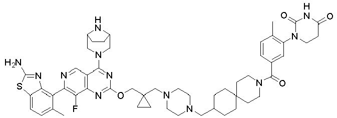 | 985 |
| 124 | 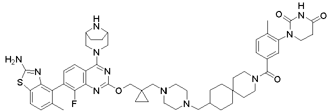 | 984 |
| 125 | 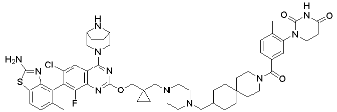 | 1018 |
| 126 | 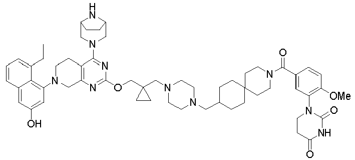 | 995 |
| 127 | 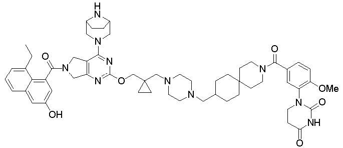 | 1009 |
| 128 | 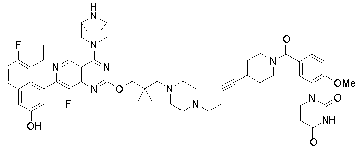 | 997 |
| 129 | 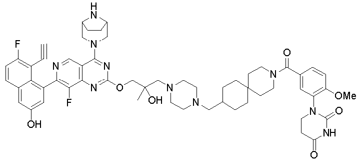 | 1027 |
| 130 | 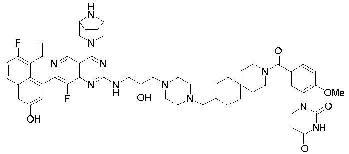 | 1012 |
| 131 | 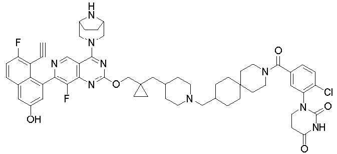 | 1026 |
| 132 | 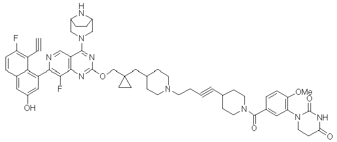 | 992 |
| 133 | 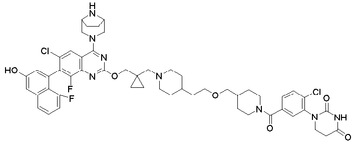 | 1011 |
| 134 | 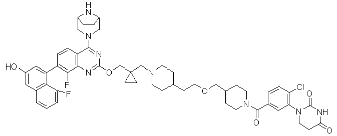 | 977 |
| 135 | 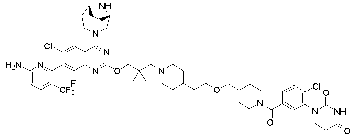 | 1039 |
| 136 | 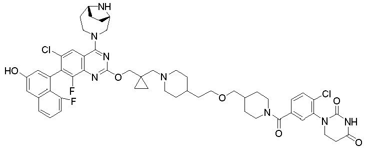 | 1025 |
| 137 | 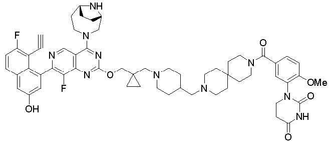 | 1037 |
| 138 | 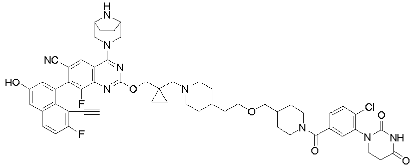 | 1026 |
| 139 | 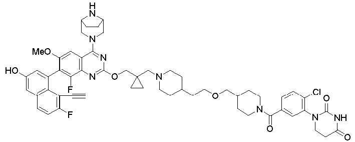 | 1031 |
| 140 | 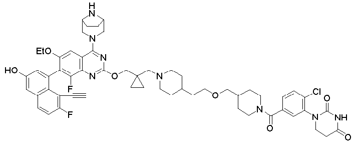 | 1045 |
| 141 | 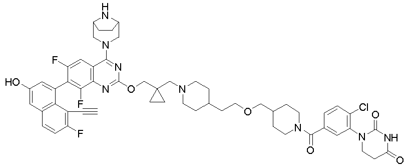 | 1019 |
| 142 | 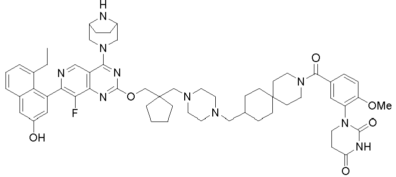 | 1037 |
| 143 | 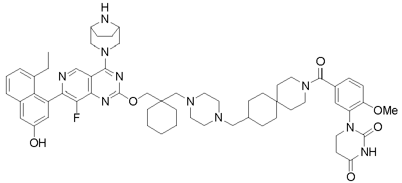 | 1051 |
| 144 | 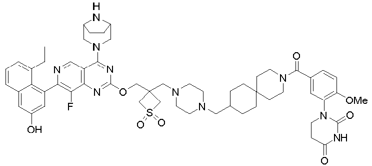 | 1073 |
| 145 | 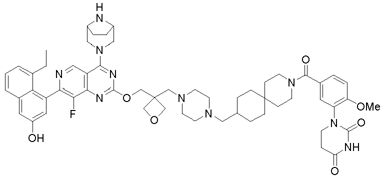 | 1025 |
| 146 | 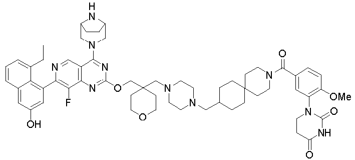 | 1053 |
| 147 | 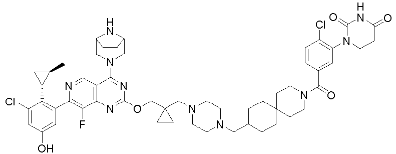 | 1023 |
| 148 | 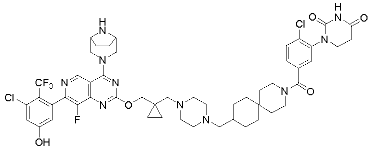 | 1037 |
| 149 | 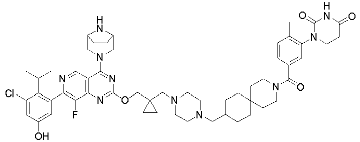 | 991 |
| 150 | 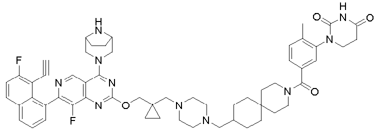 | 991 |
| 152 | 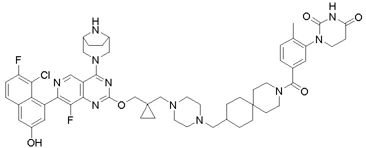 | 1017 |
| 153 | 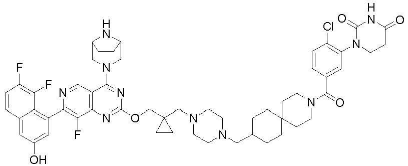 | 1021 |
| 154 | 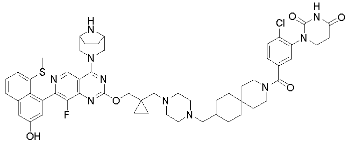 | 1031 |
| 156 | 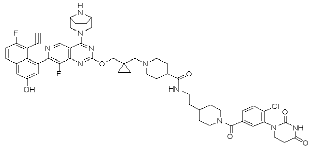 | 1015 |
| 157 | 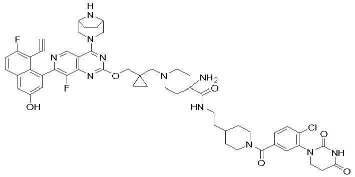 | 1030 |
| 158 | 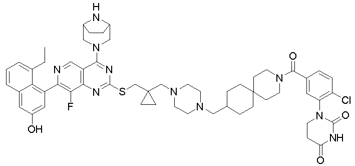 | 1029 |
| 159 | 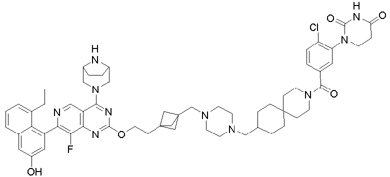 | 1053 |
| 160 | 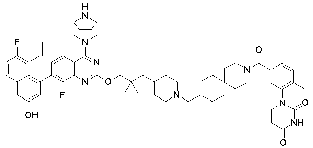 | 1005 |
| 161 | 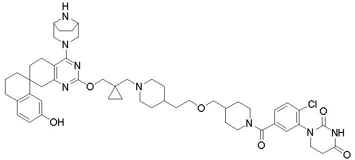 | 935 |
| 162 | 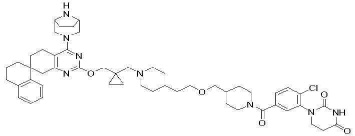 | 919 |
| 163 | 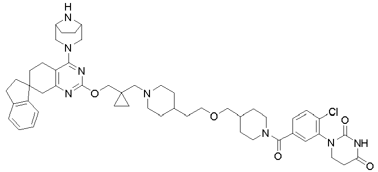 | 905 |
| 164 | 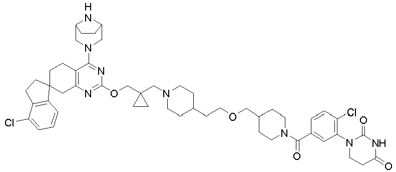 | 939 |
| 165 | 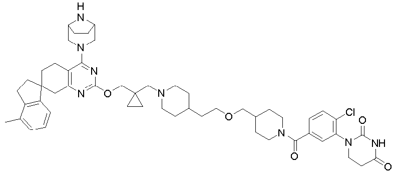 | 919 |
| 166 | 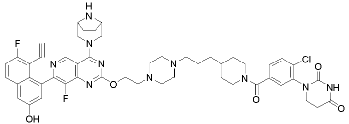 | 947 |
| 167 | 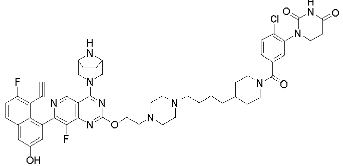 | 961 |
| 168 | 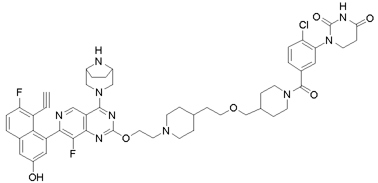 | 962 |
| 169 | 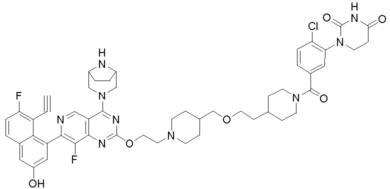 | 962 |
| 170 | 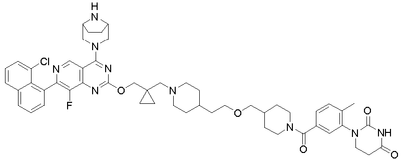 | 958 |
| 171 | 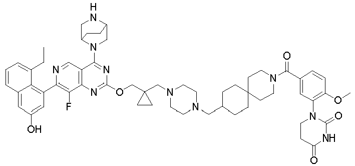 | 1009 |
| 175 | 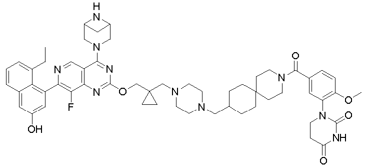 | 995 |
| 176 | 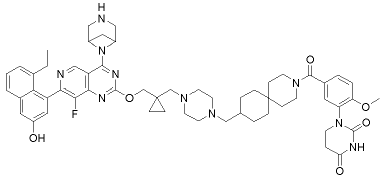 | 995 |
| 177 | 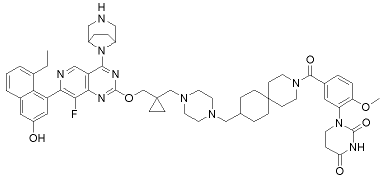 | 1009 |
| 178 | 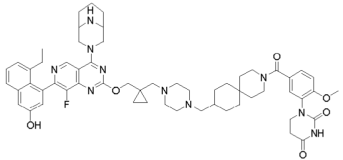 | 1038 |
| 179 | 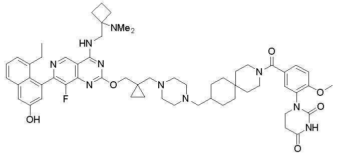 | 1025 |
| 180 | 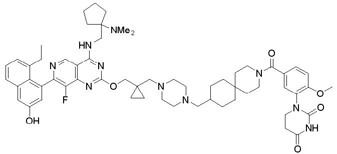 | 1039 |
| 181 | 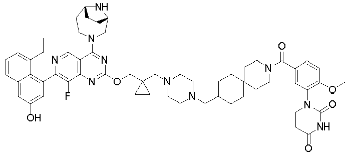 | 1023 |
| 182 | 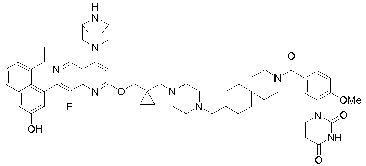 | 1008 |
| 183 | 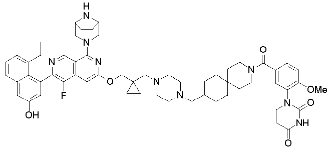 | 1008 |
| 184 | 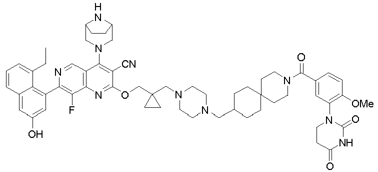 | 1033 |
| 185 | 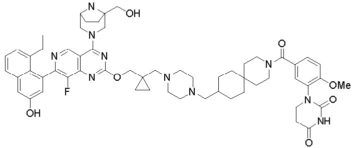 | 1039 |
| 186 | 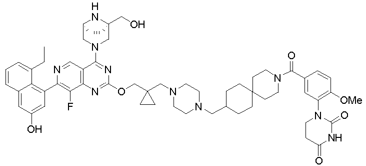 | 1039 |
| 187 | 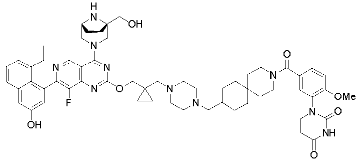 | 1039 |
| 188 | 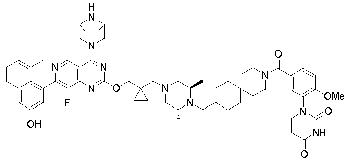 | 1037 |
| 189 | 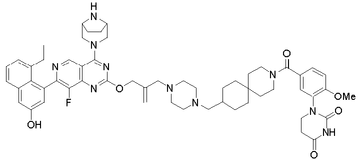 | 995 |
| 190 | 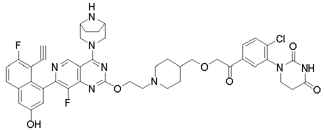 | 865 |
| 192 | 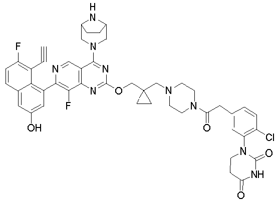 | 878 |
| 193 | 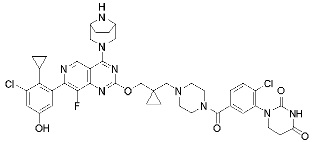 | 844 |
| 194 | 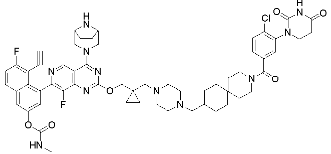 | 1084 |
| 195 | 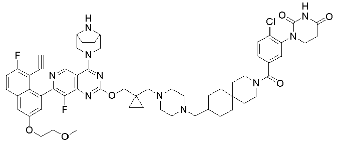 | 1085 |
| 196 | 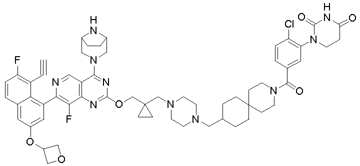 | 1083 |
| 197 | 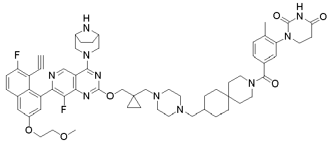 | 1065 |
| 198 | 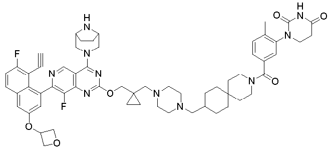 | 1063 |
| 199 | 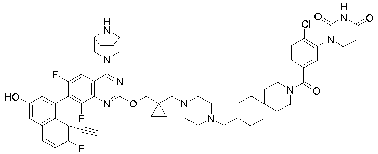 | 1044 |
| 200 | 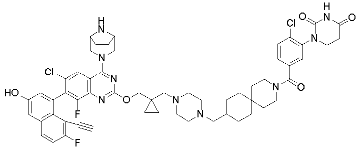 | 1060 |
| 201 | 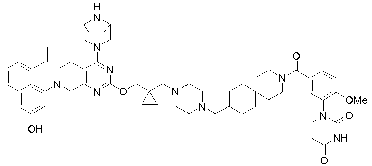 | 991 |
| 202 | 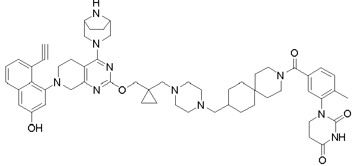 | 975 |
| 203 | 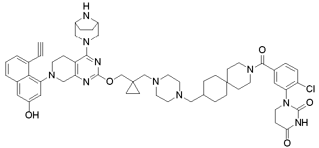 | 995 |
| 204 | 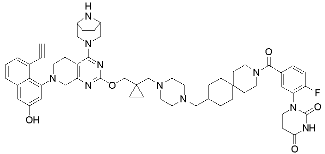 | 979 |
| 205 | 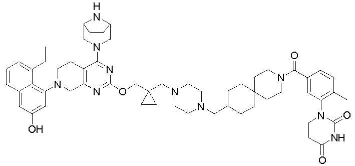 | 979 |
| 206 | 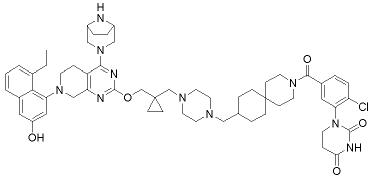 | 999 |
| 208 | 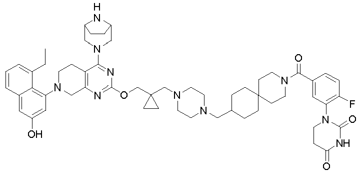 | 983 |
| 209 | 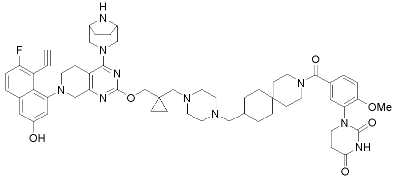 | 1009 |
| 210 | 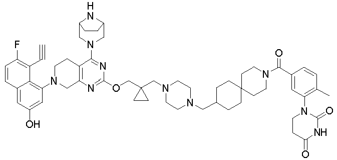 | 993 |
| 211 | 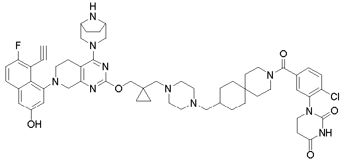 | 1013 |
| 212 | 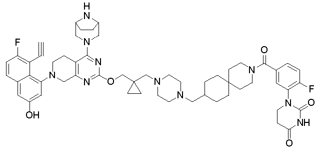 | 997 |
| 213 | 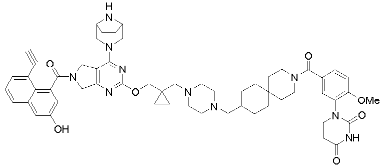 | 1005 |
| 214 | 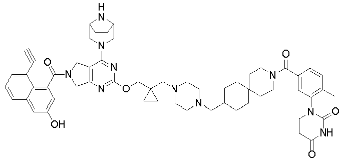 | 989 |
| 215 | 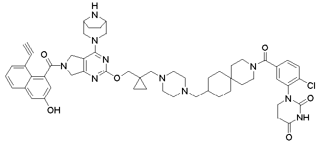 | 1009 |
| 216 | 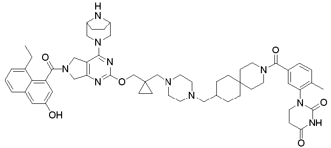 | 993 |
| 217 | 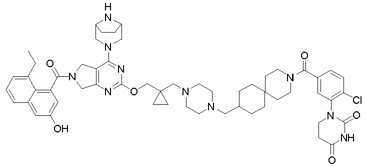 | 1013 |
| 218 | 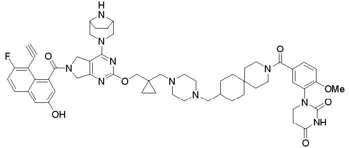 | 1023 |
| 219 | 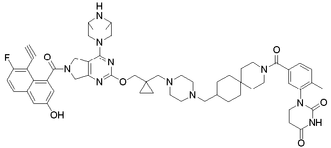 | 1007 |
| 220 | 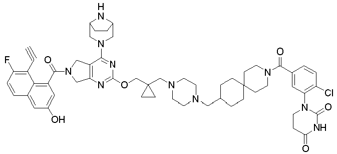 | 1027 |
| 221 | 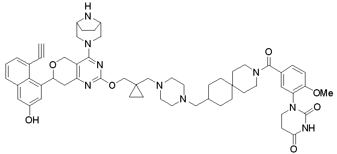 | 992 |
| 222 | 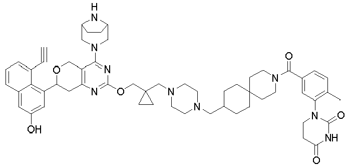 | 976 |
| 223 | 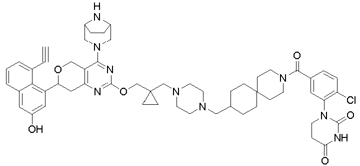 | 996 |
| 224 | 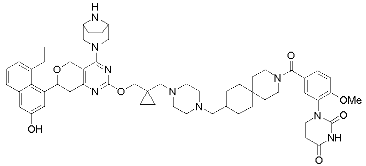 | 996 |
| 225 | 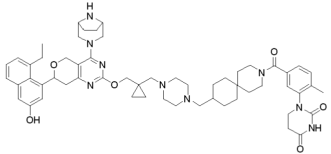 | 980 |
| 226 | 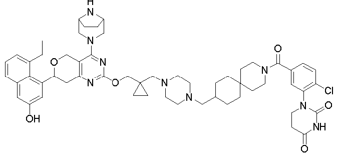 | 1000 |
| 227 | 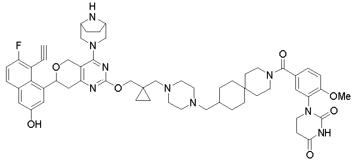 | 1010 |
| 228 | 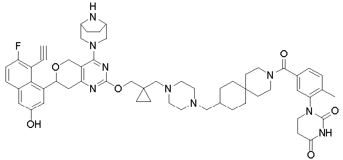 | 994 |
| 229 | 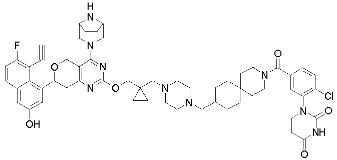 | 1014 |
| 230 | 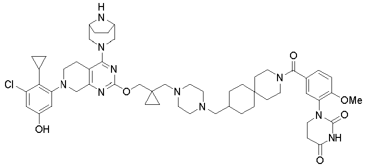 | 991 |
| 231 | 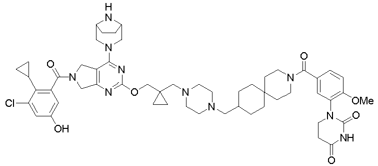 | 1005 |
| 232 | 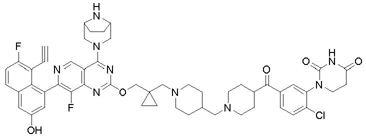 | 958 |
| 233 | 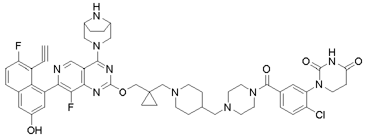 | 959 |
| 234 | 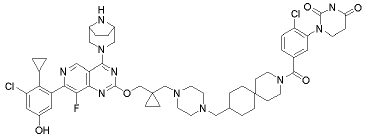 | 1009 |
| 235 | 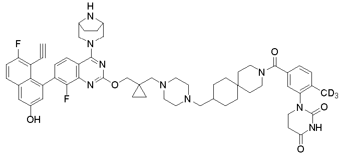 | 1009 |
| 236 | 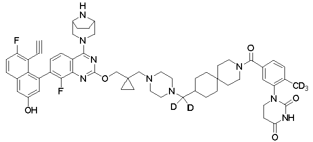 | 1011 |
| 237 | 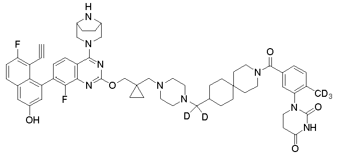 | 994 |
| 238 | 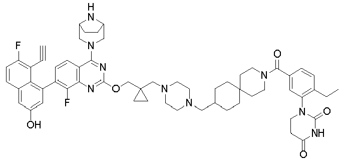 | 1020 |
| 239 | 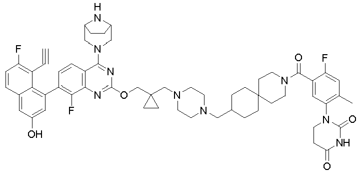 | 1024 |
| 240 | 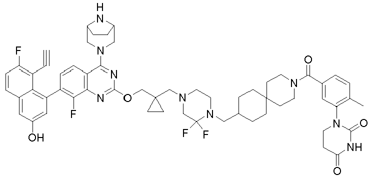 | 1042 |
| 241 | 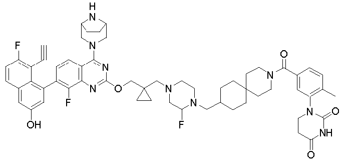 | 1024 |
| 242 | 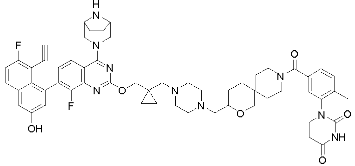 | 1008 |
| 244 | 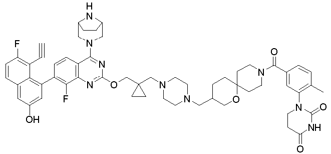 | 1008 |
| 245 | 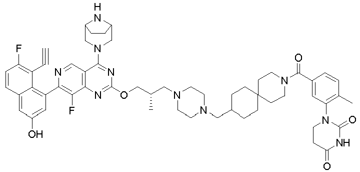 | 995 |
| 246 | 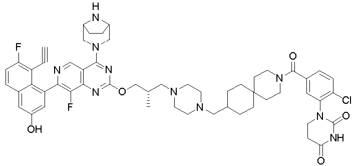 | 1015 |
| 247 | 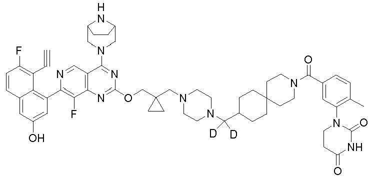 | 1009 |
| 248 | 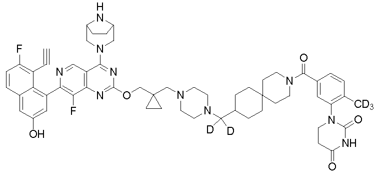 | 1012 |

### II. Biological Activity Test Examples

### Test Example 1: KRAS-G12D Binding Assay

In this method, the inhibitory effects of the compounds on the binding of KRAS-G12D to GTP were assayed by a homogeneous time-resolved fluorescence (HTRF) technique using a KRAS-G12D/GTP binding assay kit (PerkinElmer).

The experimental procedures were as follows (refer to the kit instructions for detailed test methods): the compounds of the present disclosure were each firstly dissolved in DMSO at a concentration of 20 mM, and then diluted in an equal gradient with a buffer in the kit, so that the final concentrations of the test compounds in the reaction system were in a range of 0.02-5000 nM, and the final concentration of DMSO was 0.5%. 5 µL of each compound was incubated with 5 µL of 1× Human KRAS G12D 6His-tagged (PerkinElmer), 5 µL of 1× 6His Eu Cryptate Antibody (PerkinElmer), and 5 µL of 1× GTP Red reagent (PerkinElmer) in a 384-well plate (Greiner) at 25 °C for 1 h. After the incubation was completed, the fluorescence intensity of each well was measured at the excitation wavelength of 337 nm and the emission wavelengths of 620 nm and 665 nm with an EnVision microplate reader (PerkinElmer) in the HTRF mode, and Ratio values were calculated using the formula: Ratio = (665 nm/620 nm) × 10⁴. The inhibition rates of the compounds at each concentration were calculated by comparing the fluorescence intensity ratio with that of the control group. Then non-linear curve fittings for logarithmic concentration-inhibition rate were performed using the GraphPad Prism 8 analysis software, and the IC₅₀ values of the compounds were obtained (Table 2).

### Test Example 2: KRAS-G12D Degradation Assay

The degradation effects of the compounds on KRAS-G12D were investigated on KRAS G12D mutant human lung cancer cell A-427 (ATCC), KRAS G12D mutant human gastric cancer cell AGS, KRAS G12D mutant human metastatic pancreatic cancer cell AsPc-1, and KRAS G12D mutant human pancreatic adenocarcinoma cell Panc 04.03. The specific procedures were as follows: 0.95 mL of cells were seeded into each well of a 24-well cell culture plate to achieve a cell density of 5 × 10⁵ cells/well; and the cell plate was incubated in an incubator at 37 °C with 5% CO₂ overnight. Then 50 µL of the compound solution after dilution was added to each of the wells with the corresponding cells, so that the final concentration of the compound was in a range of 0.03-3000 nM. The final concentration of DMSO was 0.25%. After the compounds were added, the cell plate was incubated in the incubator at 37 °C with 5% CO₂ for 24 h. The culture medium in the 24-well cell culture plate was removed, and the plate was washed twice with 1× PBS (KeyGEN). 120 µL of RIPA (strong) lysis buffer (Beyotime) supplemented with 1 mM phenylmethylsulfonyl fluoride, a protease inhibitor mixture (Beyotime), and a protease phosphatase inhibitor mixture (Beyotime) was added to lyse adherentA-427 cells at the bottom of the cell culture plate. After the plate was left to stand on ice for 30 min, the protein lysate in each well was transferred to a 1.5 mL centrifuge tube and centrifuged at 15000 g for 20 min at 4 °C. The cell lysate supernatants after centrifugation were cryopreserved in a refrigerator at -80 °C for later use.

After the samples of the cell lysate supernatants that had been prepared were thawed from the refrigerator at -80 °C, the total protein concentrations of the cell lysates were determined using a BCA protein quantification kit (Tiangen). The total protein concentrations of the samples were then adjusted to 0.5 µg/µL with PBS and 5× SDS-PAGE protein loading buffer (Beyotime), and the samples were incubated in a water bath at 100 °C for 15 min. The samples were then incubated in an ice bath for 5 min and centrifuged at 14000 g for 1 min at 4 °C, and the supernatants were mixed well as loading samples for the Western Blot assay. The samples were loaded into wells of a 10% precast gel (KeyGEN) with a loading volume of 10 µL (total protein: 5 µg), a sufficient Tris-MOPS-SDS running buffer (Adamas) was added, and then electrophoresis was performed at a constant voltage of 120 V for 55 min. After the electrophoresis, the proteins on the gel were transferred to PVDF membranes at a constant current of 250 mA for 55 min. After the membrane transfer, the PVDF membranes were incubated in 1× Quick Block blocking buffer (Beyotime) at room temperature for 30 min. After the blocking, the PVDF membranes were separately incubated at 4 °C overnight with the KRAS antibody (Abcam) diluted in a ratio of 1:1000 and the β-actin antibody (Abcam) diluted in a ratio of 1:2000 in 5% BSA as the diluent, and then washed with 1× TBST buffer (2.4 g of Tris, 8.8 g of NaCl, 1.5 mL of Tween 20, with pH adjusted to 7.4, with the volume brought to 1 L) 3 times for 10 min/time. The membranes were incubated with a secondary antibody (Abcam) diluted in 5% BSA at room temperature for 2 h, and then washed with 1× TBST buffer 3 times for 10 min/time. Finally, the membranes were incubated with Clarity Western ECL Substrate (BIO-RAD) for 5 min for luminescence and color development, and a ChemiScope 6200 Touch chemiluminescence imaging system (Clinx) was used for color development and protein mapping. Gray value analysis of the protein maps was performed using the chemiluminescence analysis software (Clinx). The gray correction value for each sample was calculated using the formula: gray correction value = (gray value of target protein/gray value of corresponding internal reference) × 10³. Then degradation rates were calculated by comparison with the gray correction value of the control group. Then non-linear curve fittings for logarithmic concentration-inhibition rate were performed using the GraphPad Prism 8 analysis software, and the DC₅₀ and Dₘₐₓ values of the compounds were obtained (Tables 3 and 4).

### Test Example 3: 3D Cell Proliferation Inhibition Assay

The inhibitory effects of the compounds on 3D cell proliferation were investigated on KRAS G12D mutant human lung cancer cell A427, KRAS G12D mutant human gastric cancer cell AGS, KRAS G12D mutant human metastatic pancreatic cancer cell AsPc-1, KRAS G12D mutant human pancreatic cancer cell Panc 04.03, KRAS G12D mutant human pancreatic cancer cell HPAC, KRAS G12V mutant human lung squamous cell carcinoma cell SW900, and KRAS WT human gastric cancer cell MKN1. The specific procedures were as follows:

10 µL of each compound solution diluted in a gradient was added to corresponding wells of a 96-well low-adsorption microplate (PerkinElmer), so that the final concentrations of the test compounds in the reaction system were in a range of 10000-0.026 nM. The final concentration of DMSO was 0.2%. Then 90 µL of cells were correspondingly seeded into each well to achieve a cell density of 5 × 10² cells/well (the cell density of SW900 was 5 × 10³ cells/well, and the cell density of MKN1 was 1 × 10³ cells/well). In addition to test wells for the test compounds, both a DMSO control well and a medium control well were set, with the DMSO control well containing DMSO and cells and the medium well containing only a medium. After the sample loading, the 96-well low-adsorption microplate was incubated in an incubator at 37 °C with 5% carbon dioxide for 7 days. After 7 days, the microplate was taken out, and 100 µL of CTG reagent (Promega) was added to each well. The plate was incubated at room temperature for 60 min and read on a microplate reader EnVision by the chemiluminescence program. The inhibition rates of the compounds at each concentration were calculated using the formula for the percentage inhibition of cell proliferation: inhibition rate % = (mean value of DMSO control group - single concentration reading value of compound)/(mean value of DMSO control group - mean value of medium control group) × 100. Then non-linear curve fittings for logarithmic concentration-inhibition rate were performed by GraphPad Prism 8, and the IC₅₀ values of the compounds were obtained (Table 5).

Test Example 4: KRAS G12D-Mediated Phosphorylated ERK Inhibition Assay

The relative amounts of phosphorylated ERK and total ERK in the samples were separately determined according to the test methods provided in the instructions of the AlphaLISA SureFire Ultra pERK 1/2 (Thr202/Tyr204) kit (PerkinElmer) and the AlphaLISA SureFire Ultra TotalERK 1/2 kit (PerkinElmer).

The experimental procedures for the assay of the phosphorylated ERK and total ERK were as follows: 10 µL of each sample was added to a 384-well plate (Greiner), and 5 µL of the prepared Acceptor Beads (PerkinElmer) mixed solution (containing 50× Acceptor Beads 615, Activation Buffer, and equal volumes of Reaction Buffer 1 and Reaction Buffer 2) was added to each well; and the plate was incubated at room temperature for 2 h. Then 5 µL of 1× Donor Beads (PerkinElmer) diluted with Dilution Buffer was added to each well, and the plate was incubated at room temperature overnight. After the incubation was completed, AlphaLISA signal values for the phosphorylated ERK and total ERK were separately measured on an EnVision microplate reader (PerkinElmer) in the AlphaScreen standard mode. The following formula was adopted: phosphorylated ERK correction value = phosphorylated ERK AlphaLISA signal value/total ERK AlphaLISA signal value. The relative ratio of the phosphorylated ERK for each sample was calculated. The percentage inhibition rates of the compounds on the phosphorylated ERK at each concentration were calculated by comparing the relative value of the phosphorylated ERK with that of the DMSO control well. Then non-linear curve fittings for logarithmic concentration-inhibition rate were performed by the GraphPad Prism 8 analysis software, and the IC₅₀ values of the compounds on the inhibition of the phosphorylated ERK were obtained (Table 2).

**Table 2**

| Example | KRAS G12D:GTP / IC₅₀ (nM) | pERK (A427) / IC₅₀ (nM) |
|---|---|---|
| Example 1 | C | - |
| Example 2 | C | - |
| Example 3 | A | B |
| Example 4 | A | A |
| Example 5 | A | A |
| Example 6 | A | A |
| Example 7 | A | A |
| Example 8 | A | A |
| Example 9 | B | - |
| Example 10 | A | A |
| Example 11 | A | - |
| Example 12 | C | - |
| Example 13 | A | A |
| Example 14 | A | A |
| Example 15 | A | A |
| Example 16 | E | E |
| Example 17 | A | A |
| Example 18 | A | A |
| Example 19 | A | A |
| Example 20 | A | A |
| Example 21 | A | A |
| Example 22 | A | A |
| Example 23 | A | C |
| Example 24 | A | A |
| Example 25 | A | C |
| Example 26 | B | A |
| Example 27 | A | - |
| Example 28 | A | - |
| Example 29 | A | A |
| Example 30 | B | A |
| Example 31 | A | A |
| Example 32 | D | E |
| Example 33 | C | A |
| Example 34 | C | - |
| Example 35 | A | - |
| Example 36 | A | A |
| Example 38 | A | - |
| Example 40 | A | - |
| Example 42 | A | - |
| Example 45 | A | - |
| Example 52 | C | - |
| Example 55 | A | - |
| Example 56 | B | - |
| Example 57 | A | - |
| Example 58 | D | - |
| Example 59 | A | - |
| Example 61 | 21.13 | - |
| Example 63 | 1.15 | - |
| Example 69 | 2.4 | - |
| Example 84 | 2.47 | - |
| Example 85 | 1.34 | - |
| Example 108 | 3.22 | - |
| Example 174 | 5.19 | - |

Table 2: "-" represents undetectable
when IC₅₀ ≤ 50 nM, the level was A; when 50 nM < IC₅₀ ≤ 100 nM, the level was B; when 100 nM < IC₅₀ ≤ 500 nM, the level was C; when 500 nM < IC₅₀ ≤ 1000 nM, the level was D; when 1000 nM < IC₅₀ ≤ 10 µM, the level was E.

**Table 3**

| Example | A427 (KRAS G12D) | | AGS (KRAS G12D) | |
|---|---|---|---|---|
| | DC₅₀ (nM) | Dmax (%) | DC₅₀ (nM) | Dmax (%) |
| Example 1 | E | C | - | - |
| Example 2 | E | C | - | - |
| Example 3 | A | B | - | - |
| Example 4 | A | A | - | - |
| Example 5 | A | A | - | - |
| Example 6 | A | A | 7.91 | 100 |
| Example 7 | A | A | - | - |
| Example 8 | A | B | - | - |
| Example 9 | E | C | - | - |
| Example 10 | A | A | - | - |
| Example 11 | E | B | - | - |
| Example 12 | E | B | - | - |
| Example 13 | B | B | - | - |
| Example 14 | A | A | - | - |
| Example 15 | A | A | 2.17 | 99.6 |
| Example 16 | E | C | - | - |
| Example 17 | E | D | - | - |
| Example 18 | A | A | - | - |
| Example 19 | B | A | - | - |
| Example 20 | A | A | 19.88 | 100 |
| Example 21 | A | A | - | - |
| Example 22 | A | B | - | - |
| Example 23 | E | C | - | - |
| Example 24 | A | A | - | - |
| Example 25 | A | B | - | - |
| Example 26 | E | C | - | - |
| Example 27 | E | C | - | - |
| Example 28 | E | D | - | - |
| Example 29 | A | A | - | - |
| Example 30 | A | B | - | - |
| Example 31 | A | A | - | - |
| Example 32 | E | D | - | - |
| Example 33 | A | A | - | - |
| Example 34 | E | D | - | - |
| Example 35 | E | D | - | - |
| Example 36 | A | A | - | - |
| Example 37 | A | A | - | - |
| Example 38 | A | A | - | - |
| Example 39 | A | B | - | - |
| Example 40 | E | C | - | - |
| Example 41 | E | - | - | - |
| Example 42 | A | A | 11.8 | 77.43 |
| Example 43 | A | B | - | - |
| Example 44 | A | A | - | - |
| Example 45 | E | D | - | - |
| Example 46 | A | A | - | - |
| Example 47 | C | B | - | - |
| Example 48 | A | A | - | - |
| Example 50 | A | A | 1.73 | 97.3 |
| Example 51 | A | A | 9.87 | 97.23 |
| Example 52 | C | B | - | - |
| Example 55 | A | A | 1.91 | 99.17 |
| Example 56 | A | B | - | - |
| Example 57 | A | A | 0.69 | 94.24 |
| Example 58 | A | A | - | - |
| Example 59 | E | D | - | - |
| Example 62 | 15.75 | 94.8 | - | - |
| Example 70 | 14.64 | 87.3 | - | - |
| Example 71 | 2.1 | 95.02 | 7.02 | 99.62 |
| Example 72 | 101 | 82.64 | | |
| Example 73 | 19.84 | 87.25 | 16.03 | 85.65 |
| Example 74 | 38.12 | 76.95 | - | - |
| Example 75 | 4.13 | 83.79 | 57.81 | 89.25 |
| Example 76 | 1.74 | 89.31 | - | - |
| Example 77 | 1.76 | 94.49 | 1.47 | 100 |
| Example 78 | 10.08 | 70.25 | 28.86 | 92.62 |
| Example 81 | 16.83 | 76.5 | 2.99 | 80.33 |
| Example 82 | 1.21 | 98.96 | 3.84 | 97.74 |
| Example 83 | 33.87 | 87.25 | - | - |
| Example 84 | 4.67 | 86.33 | 3.82 | 92.15 |
| Example 88 | 0.65 | 89.47 | - | - |
| Example 90 | 0.86 | 95.58 | 10.62 | 98.9 |
| Example 107 | 4.98 | 94.74 | - | - |
| Example 109 | - | - | 4.8 | 94.46 |
| Example 243 | 1.66 | 92.88 | - | - |

**Table 4**

| Example | ASPC-1 (KRAS G12D) | | Panc 04.03 (KRAS G12D) | |
|---|---|---|---|---|
| | DC₅₀ (nM) | Dmax (%) | DC₅₀ (nM) | Dmax (%) |
| Example 6 | 2.9 | 100 | 21.71 | 99.87 |
| Example 15 | 12.97 | 99.3 | 16.58 | 98.91 |
| Example 20 | 0.57 | 100 | 15.15 | 100 |
| Example 42 | 0.22 | 100 | 7.46 | 100 |
| Example 50 | 33.13 | 100 | 31.91 | 99.26 |
| Example 51 | 4.79 | 99.41 | 23.01 | 93.8 |
| Example 55 | 11.07 | 95.17 | 4.08 | 98.62 |
| Example 57 | 15.03 | 99.48 | 8.95 | 97.31 |
| Example 71 | 4.76 | 98.61 | 11.85 | 98.85 |
| Example 73 | 35.67 | 100 | 27.1 | 95.64 |
| Example 75 | 8.79 | 99.46 | 9.38 | 92.04 |
| Example 77 | 13.15 | 98.18 | 26.23 | 98.51 |
| Example 78 | 23.74 | 100 | 16.26 | 100 |
| Example 81 | 17.77 | 98.2 | - | - |
| Example 82 | 12.56 | 93.67 | 6.74 | 100 |
| Example 84 | 0.71 | 99.59 | 1.99 | 98.61 |
| Example 89 | 94.69 | 69 | - | - |
| Example 90 | 1.87 | 100 | - | - |
| Example 100 | 2.54 | 100 | - | - |
| Example 102 | 8.93 | 100 | - | - |
| Example 106 | 10.47 | 99.46 | - | - |
| Example 107 | 13.71 | 97.54 | - | - |
| Example 109 | 0.64 | 98.77 | - | - |
| Example 110 | 2.56 | 99.23 | - | - |
| Example 172 | 113.41 | 55.05 | - | - |
| Example 173 | 2.42 | 98.98 | - | - |
| Example 174 | 13.65 | 100 | - | - |
| Example 191 | 149.35 | 81.92 | | |
| Example 207 | 15.07 | 99.7 | - | - |
| Example 251 | 8.72 | 99.13 | - | - |
| Example 252 | 42.29 | 100 | - | - |
| Example 253 | 1.39 | 100 | - | - |
| Example 254 | 32.5 | 83.42 | - | - |

In Tables 3 and 4: "-" represents undetectable
when DC₅₀ ≤ 50 nM, the level was A; when 50 nM < DC₅₀ ≤ 100 nM, the level was B; when 100 nM < DC₅₀ ≤ 500 nM, the level was C; when 500 nM < DC₅₀ ≤ 1000 nM, the level was D; when 1000 nM < DC₅₀ ≤ 10 µM, the level was E;
when 80 ≤ Dₘₐₓ (%) ≤ 100, the level was A; when 50 ≤ Dₘₐₓ (%) < 80, the level was B; when 30 ≤ Dₘₐₓ (%) < 50, the level was C; when Dₘₐₓ (%) < 30, the level was D.

**Table 5**

| Example | 3D Cell proliferation IC₅₀ (nM) | | | | |
|---|---|---|---|---|---|
| | A427 (KRAS G12D) | AGS (KRAS G12D) | AsPc-1 (KRAS G12D) | Panc 04.03 (KRAS G12D) | HPAC (KRAS G12D) |
| Example 3 | E | A | - | - | - |
| Example 4 | C | B | - | - | - |
| Example 5 | B | A | - | - | - |
| Example 6 | A | A | - | - | 5.35 |
| Example 7 | A | A | - | - | - |
| Example 8 | B | A | - | - | - |
| Example 10 | C | A | - | - | - |
| Example 11 | - | A | - | - | - |
| Example 13 | B | A | - | - | - |
| Example 14 | A | A | - | - | - |
| Example 15 | A | A | A | A | 4.06 |
| Example 16 | C | E | - | - | - |
| Example 17 | B | B | - | - | - |
| Example 18 | A | A | - | - | - |
| Example 19 | C | A | - | - | - |
| Example 20 | A | A | 3.27 | 10.49 | - |
| Example 21 | C | A | - | - | - |
| Example 22 | B | A | - | - | - |
| Example 23 | C | B | - | - | - |
| Example 24 | C | B | - | - | - |
| Example 25 | C | - | - | - | - |
| Example 26 | B | B | - | - | - |
| Example 27 | - | A | - | - | - |
| Example 29 | A | A | - | - | - |
| Example 30 | A | A | - | - | - |
| Example 31 | A | A | 5.62 | 22.24 | - |
| Example 32 | E | E | - | - | - |
| Example 33 | C | A | - | - | - |
| Example 36 | A | A | - | - | 1.19 |
| Example 37 | C | A | - | - | - |
| Example 38 | A | A | - | - | - |
| Example 39 | C | A | - | - | - |
| Example 40 | E | C | - | - | - |
| Example 42 | A | A | 1.75 | 8.41 | 2.96 |
| Example 44 | C | A | - | - | - |
| Example 45 | B | B | - | - | - |
| Example 47 | B | B | - | - | - |
| Example 48 | A | A | - | - | - |
| Example 50 | A | A | A | A | 11.58 |
| Example 51 | A | A | A | A | 4.55 |
| Example 54 | A | A | - | - | - |
| Example 55 | A | A | - | - | 4.28 |
| Example 56 | B | A | - | - | - |
| Example 57 | A | A | A | A | 9.88 |
| Example 59 | C | A | - | - | - |
| Example 60 | C | A | - | - | - |
| Example 61 | C | A | - | - | - |
| Example 62 | 13.07 | 4.98 | - | - | - |
| Example 67 | 36.51 | 33.65 | - | - | - |
| Example 68 | - | 24.78 | - | - | - |
| Example 70 | - | 15.51 | - | - | - |
| Example 71 | 46.81 | 4.17 | 5.28 | 16.7 | 9.31 |
| Example 72 | - | 8.64 | - | - | - |
| Example 73 | 3.54 | 21.05 | 14.93 | 64.81 | 14.73 |
| Example 74 | 31.58 | 15.74 | - | - | - |
| Example 75 | 88.07 | 9.10 | 14.85 | 83.87 | 11.33 |
| Example 76 | 74.76 | 9.53 | - | - | - |
| Example 77 | 17.75 | 8.9 | 6.75 | 31.47 | 9.54 |
| Example 78 | 16.26 | 9.25 | 17.03 | 37.93 | 18.71 |
| Example 81 | 67.08 | 5.52 | 12.96 | 27.77 | 4.37 |
| Example 82 | 21.83 | 5.78 | 4.95 | 14.13 | 4.15 |
| Example 84 | 6.5 | 1.05 | 2.58 | 17.01 | 1.30 |
| Example 85 | - | 50.22 | - | - | - |
| Example 86 | - | 97.52 | - | - | - |
| Example 87 | - | 605.7 | - | - | - |
| Example 89 | - | 8.65 | 11.46 | - | 12.85 |
| Example 90 | 28.27 | 0.65 | 1.19 | 8.82 | 2.44 |
| Example 100 | - | 3.63 | 2.02 | 23.79 | 2.43 |
| Example 102 | - | 6.56 | 3.69 | 15.17 | 13.49 |
| Example 103 | - | 4.35 | 1.41 | 16.79 | 1.58 |
| Example 104 | - | 2.49 | 1.83 | 31.28 | 5.32 |
| Example 105 | - | - | 93.5 | - | 12.69 |
| Example 106 | - | 13.37 | 30.78 | 63.45 | 151.5 |
| Example 107 | - | - | 27.46 | - | 10.79 |
| Example 108 | - | - | 4.53 | - | 6.87 |
| Example 109 | - | 3.98 | 1.42 | 6.73 | 1.62 |
| Example 110 | - | 5.69 | 4.47 | 12.53 | 18.64 |
| Example 151 | - | 6.82 | 3.86 | 23.11 | 6.92 |
| Example 173 | - | 4.22 | 1.51 | 5.88 | 3.8 |
| Example 174 | 64.2 | 19.41 | 62.48 | 31.28 | 1.78 |
| Example 207 | - | 11.21 | 16.15 | 34.46 | - |
| Example 243 | 121.2 | 2.16 | 2.34 | 21.73 | 0.61 |
| Example 251 | - | - | 14.33 | 22.43 | 40.22 |
| Example 252 | - | 31.74 | 21.03 | 51.02 | 18.88 |
| Example 253 | - | 3.92 | 1.59 | 18.81 | 10.61 |
| Example 254 | 74.67 | 12.98 | 7.85 | - | 14.3 |

In Table 5: "-" represents undetectable
when IC₅₀ ≤ 50 nM, the level was A; when 50 nM < IC₅₀ ≤ 100 nM, the level was B; when 100 nM < IC₅₀ ≤ 500 nM, the level was C; when 500 nM < IC₅₀ ≤ 1000 nM, the level was D; when 1000 nM < IC₅₀ ≤ 10 µM, the level was E.

Experimental conclusion: the compounds of the present disclosure are able to effectively bind to the KRAS G12D target protein or have an inhibitory effect, and the compounds of the present disclosure are able to effectively and specifically degrade the KRAS G12D protein. The compounds of the present disclosure are able to effectively inhibit pERK phosphorylation, and effectively inhibit 3D cell proliferation.

## Claims

1. A compound of formula I or I', and/or a stereoisomer, an enantiomer, a diastereomer, an atropisomer, a deuteride, a hydrate, a solvate or a prodrug thereof and/or a pharmaceutically acceptable salt thereof:
G-L-E I
G'-L-E I'
wherein:
G is G1: 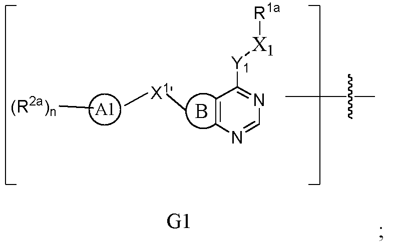
G' is G1': 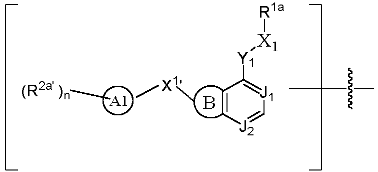
each Y₁ is independently a bond, O, C(R^{3a})₂, or NR^{3a};
each X₁ is independently a bond or -(CH₂)ₘ-;
each X¹' is independently a bond or -C(O)-;
each R^{1a} is independently -OH, -N(R^{3a})₂, C₃-C₁₂ cycloalkyl, or 3- to 12-membered heterocycloalkyl, wherein
the C₃-C₁₂ cycloalkyl or 3- to 12-membered heterocycloalkyl may be optionally substituted with 1 or more R^{a};
each R^{2a} is independently halogen, deuterium, cyano, amino, hydroxy, C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, triazolyl, -O-C₁-C₆ alkyl, -O-C₃-C₈ cycloalkyl, -CH₂C(=O)N(R^{3a})₂, N(R^{3a})₂, C₁-C₃ alkyl-O-C₁-C₃ alkyl-, HC(=O)-, - CO₂R^{3a}, -CON(R^{3a})₂, or 5- to 6-membered heteroaryl, wherein the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, O-C₁-C₆ alkyl, -O-C₃-C₈ cycloalkyl, or 5- to 6-membered heteroaryl is optionally substituted with 1 or more deuterium, halogen, hydroxy, cyano, amino, nitro, C₁-C₃ alkoxy, or C₁-C₃ alkyl;
each R^{3a} is independently H, C₁-C₆ alkyl, C₃-C₈ cycloalkyl, C₃-C₆ alkenyl, or C₃-C₆ alkynyl;
or two R^{3a}, together with the atom linked thereto, form 4- to 12-membered heterocycloalkyl, wherein the 4-to 12-membered heterocycloalkyl is optionally substituted with 1 or more deuterium, halogen, hydroxy, cyano, amino, C₁-C₃ alkoxy, or C₁-C₃ alkyl;
or R^{1a} and R^{3a}, together with the atoms to which they are each linked, form 4- to 12-membered heterocycloalkyl, wherein the 4- to 12-membered heterocycloalkyl contains at least one heteroatom selected from N, O, and S, and is optionally substituted with 1 or more R^{b};
each ring A1 is independently absent, -L'-(6- to 15-membered aryl), -L'-(5- to 15-membered heteroaryl), or -L'-(5- to 15-membered heterocycloalkyl);
each ring B is independently 6- to 15-membered aryl, 5- to 15-membered heteroaryl, C₅-C₁₅ cycloalkyl, or 5- to 16-membered heterocycloalkyl, wherein the 6- to 15-membered aryl, 5- to 15-membered heteroaryl, C₅-C₁₅ cycloalkyl, or 5- to 16-membered heterocycloalkyl is optionally substituted with 1 or more R^{a};
each L' is independently a bond or C₁-C₄ alkylene, wherein the C₁-C₄ alkylene is optionally substituted with 1 or more deuterium, hydroxy, C₁-C₄ hydroxyalkyl, or 5- to 10-membered heteroaryl;
each R^{a} and each R^{b} are independently hydrogen, hydroxy, halogen, cyano, -N(R^{3a})₂, -CH₂N(R^{3a})₂, 3- to 8-membered heterocycloalkyl, C₁-C₆ alkyl, HC(=O)-, -CO₂R^{4a}, -CO₂N(R^{4a})₂, C₁-C₃ alkoxy, (C₁-C₃ alkoxy)-C₁-C₃ alkyl-, C₁-C₃ alkyl-N(R^{4a})₂, C₂-C₄ alkenyl, C₃-C₈ cycloalkyl, 3- to 8-membered heterocycloalkyl, or 5- to 6-membered heteroaryl, wherein the C₁-C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more deuterium, halogen, cyano, hydroxy, amino, nitro, C₁-C₃ alkyl, or C₁-C₃ alkoxy;
each R^{4a} is independently hydrogen, C₁-C₃ alkyl, or C₁-C₃ hydroxyalkyl;
or two R^{4a}, together with the atom linked thereto, form heterocycloalkyl, wherein the heterocycloalkyl is optionally substituted with 1 or more deuterium, -OH, -NH₂, C₁-C₃ alkyl, or C₁-C₃ alkoxy;
m is 0, 1, 2, 3, or 4;
n is 0, 1, 2, 3, or 4;
J₁ and J₂ are independently CR' or N, provided that J₁ and J₂ are not both CR'; R' is hydrogen, halogen, deuterium, cyano, amino, hydroxy, or C₁-C₆ alkyl;
each R^{2a'} is independently halogen, deuterium, cyano, amino, hydroxy, C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, triazolyl, -O-C₁-C₆ alkyl, -O-C₃-C₈ cycloalkyl, -CH₂C(=O)N(R^{3a})₂, N(R^{3a})₂, C₁-C₃ alkyl-O-C₁-C₃ alkyl-, HC(=O)-, - CO₂R^{3a}, -CON(R^{3a})₂, 5- to 6-membered heteroaryl, or -O-(3- to 8-membered heterocycloalkyl), wherein the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, O-C₁-C₆ alkyl, -O-C₃-C₈ cycloalkyl, 5- to 6-membered heteroaryl, or -O-(3- to 8-membered heterocycloalkyl) is optionally substituted with 1 or more deuterium, halogen, hydroxy, cyano, amino, nitro, C₁-C₃ alkoxy, or C₁-C₃ alkyl;
when J₁ and J₂ are both N, at least one R^{2a'} is -O-(3- to 8-membered heterocycloalkyl);
L is a linking chain, which links G and E by means of covalent bonds;
L is a linking chain, which links G' and E by means of covalent bonds;
each E is independently E1, E2, or E3: 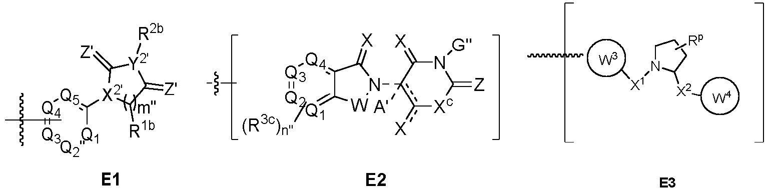
wherein in E1:
Z' is O, S, or CH₂;
X²' is CH or N;
Y²' is CH, N, O, or S;
Q₁, Q₂, Q₃, Q₄, and Q₅ are each independently CR^{3b} or N;
each R^{3b} is independently hydrogen, deuterium, hydroxy, amino, cyano, halogen, nitro, sulfhydryl, C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, 6- to 10-membered aryl, 5- to 10-membered heteroaryl, -O-(C₁-C₆ alkyl), -O-(C₁-C₆ heteroalkyl), -O-(C₃-C₈ cycloalkyl), -O-(3- to 8-membered heterocycloalkyl), -S-(C₁-C₆ alkyl), -S-(C₁-C₆ heteroalkyl), -S-(C₃-C₈ cycloalkyl), -S-(3- to 8-membered heterocycloalkyl), -N(C₁-C₆ alkyl)₁₋₂, -N(C₁-C₆ heteroalkyl)₁₋₂, -N(C₃-C₈ cycloalkyl)₁₋₂, -N(3- to 8-membered heterocycloalkyl)₁₋₂, -O-(C₆-C₁₀ aryl), or -O-(5- to 10-membered heteroaryl), wherein the C₁-C₆ alkyl, C₁-C₆ heteroalkyl, C₂-C₆ alkenyl, C₂-C₆ alkynyl, C₃-C₈ cycloalkyl, 3- to 8-membered heterocycloalkyl, C₆-C₁₀ aryl, or 5- to 10-membered heteroaryl is optionally substituted with 1-3 groups independently selected from deuterium, hydroxy, halogen, cyano, amino, O(C₁-C₆ alkyl), O-(C₃-C₈ cycloalkyl), -O-(3- to 8-membered heterocycloalkyl), N(C₁-C₆ alkyl)₁₋₂, - NH(C₃-C₈ cycloalkyl), -NH(3- to 8-membered heterocycloalkyl), -O-(C₆-C₁₀ aryl), and -O-(5- to 10-membered heteroaryl); or two adjacent R^{3b}, together with the atoms linked thereto, form cycloalkyl, heterocycloalkyl, heteroaryl, or aryl;
m" is 1, 2, or 3;
each R^{1b} is independently hydrogen, deuterium, hydroxy, amino, cyano, halogen, C₁-C₆ alkyl, C₁-C₆ heteroalkyl, C₃-C₈ cycloalkyl, 3- to 8-membered heterocycloalkyl, C₆-C₁₀ aryl, 5- to 10-membered heteroaryl, -O(C₁-C₆ alkyl), -O-(C₃-C₈ cycloalkyl), -O-(3- to 8-membered heterocycloalkyl), -N(C₁-C₆ alkyl)₁₋₂, -NH(C₃-C₈ cycloalkyl), -NH(3- to 8-membered heterocycloalkyl), -O-(C₆-C₁₀ aryl), or -O-(5- to 10-membered heteroaryl), wherein the alkyl, cycloalkyl, heterocycloalkyl, aryl, or heteroaryl is optionally substituted with 1-3 groups independently selected from hydroxy, halogen, cyano, hydroxy, and amino; R^{2b} is absent, hydrogen, deuterium, C₁-C₆ alkyl, or C₃-C₆ cycloalkyl, wherein the C₁-C₆ alkyl and C₃-C₆ cycloalkyl are optionally substituted with 1-3 groups independently selected from hydroxy, halogen, cyano, hydroxy, amino, and -OC(O)(C₁-C₆ alkyl); 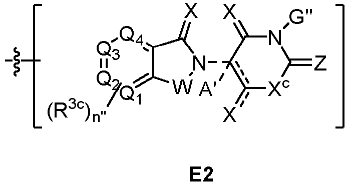
wherein in E2:
Q₁, Q₂, Q₃, and Q₄ are each independently CR^{3b} or N;
W is CR^{1c}R^{2c}, C(S), C(O), SO₂, -OC=R^{4c}-, -SC=R^{4c}-, *-*C=R^{4c}NR^{5c}-, or -N=CA'-;
X is CH₂, O, or S;
X^{c} is -CH₂- or -NG'-;
Z is CH₂, O, or S;
G' and G" are each independently hydrogen, deuterium, C₁-C₆ alkyl, OH, C₃-C₆ cycloalkyl, -CH₂- heterocycloalkyl, or -CH₂-phenyl, wherein the C₁-C₆ alkyl, C₃-C₆ cycloalkyl, -CH₂-heterocycloalkyl, or - CH₂-phenyl is optionally substituted with 1 or more hydroxy, halogen, cyano, and amino;
A' is hydrogen, deuterium, C₁-C₆ alkyl, C₃-C₈ cycloalkyl, or halogen;
R^{1c}, R^{2c}, and R^{3c} are each independently hydrogen, deuterium, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, -CR_{'}R_{"}, - CR_{'}NR_{'}R_{"}, C₆-C₁₀ aryl, 5- to 10-membered heteroaryl, C₃-C₈ cycloalkyl, 3- to 8-membered heterocycloalkyl, -P(O)(OR_{'})R_{"}, -P(O)R_{'}R_{"}, -OP(O)(OR_{'})R_{"}, CN, -NR_{'}SO₂NR_{'}R_{"}, -NR_{'}C(O)NR_{'}R_{"}, -C(O)NR_{'}C(O)R_{"}, - NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, -SO₂NR_{'}COR_{"}, -NO₂, - COR_{'}, -C(C=N-OR_{'})R_{"}, -CR_{'}=CR_{'}R_{"}, -CCR_{'}, -S(C=O)(C=N-R_{'})R_{"}, -SF₅, or -OCF₃; R^{4c} is O or S;
R^{5c} is H, C₁-C₆ alkyl, C₆-C₁₀ aryl, 5- to 12-membered heteroaryl, C₃-C₈ cycloalkyl, or 3- to 8-membered heterocycloalkyl;
R_{'} and R_{"} are each independently a bond, hydrogen, deuterium, C₁-C₆ alkyl, C₃-C₈ cycloalkyl, C₆-C₁₀ aryl, 5- to 10-membered heteroaryl, or 3- to 8-membered heterocycloalkyl;
n" is 0, 1, 2, 3, or 4;
-̅ -̅ -̅ is a single bond or a double bond;
----- is a bond, which may be an R stereoisomer, an S stereoisomer, or a non-stereoisomer; 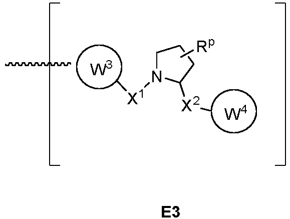
wherein in E3:
X¹ and X² are each independently a bond, O, C(O), C(S), NR^{1d}, or CR^{1d}R^{2d};
R^{1d} and R^{2d} are each independently H, deuterium, or C₁-C₆ alkyl, wherein the C₁-C₆ alkyl is optionally substituted with 1 or more halogen or C₁-C₆ alkoxy;
R^{P} is independently H, deuterium, halogen, -OH, or C₁-C₃ alkyl, wherein the C₁-C₃ alkyl is optionally substituted with 1 or more halogen, hydroxy, or C₁-C₃ alkoxy;
W³ is C₁-C₆ alkyl, -T-N(R^{3d}R^{4d}), -T-N(R^{3d}R^{4d})X³, -T-C₆-C₁₀ aryl, -T-(5- to 10-membered heteroaryl), -T-(3-to 8-membered heterocyclyl), -NR^{5d}-T-C₆-C₁₀ aryl, -NR^{5d}-T-(5- to 10-membered heteroaryl), or -NR^{5d}-T-(3-to 8-membered heterocyclyl), wherein the C₁-C₆ alkyl, -T-N(R^{3d}R^{4d}), -T-N(R^{3d}R^{4d})X³, -T-C₆-C₁₀ aryl, -T-(5-to 10-membered heteroaryl), -T-(3- to 8-membered heterocyclyl), -NR^{5d}-T-C₆-C₁₀ aryl, -NR^{5d}-T-(5- to 10-membered heteroaryl), or -NR^{5d}-T-(3- to 8-membered heterocyclyl) is optionally substituted;
X³ is C(O), R^{3d}, R^{4d}, or R^{5d};
R^{3d}, R^{4d}, or R^{5d} is each independently selected from H, deuterium, and C₁-C₆ alkyl, wherein the C₁-C₆ alkyl is optionally substituted with 1 or more halogen, -OH, R^{1d}C(O), R^{1d}C(S), R^{1d}SO, R^{1d}SO₂, NR^{1d}R^{2d}C(O), NR^{1d}R^{2d}C(S), NR^{1d}R^{2d}SO, or NR^{1d}R^{2d}SO₂;
T is C₁-C₆ alkyl or -(CH₂)ₙ-, wherein 1 or more methylene groups in the -(CH₂)ₙ- are optionally substituted with groups selected from deuterium, halogen, and C₁-C₆ alkyl, and the C₁-C₆ alkyl is optionally substituted with halogen, -OH, or amino;
n is 0, 1, 2, 3, 4, 5, or 6;
W⁴ is 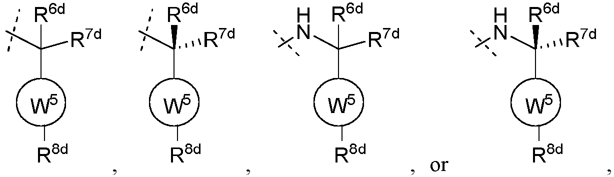 wherein the 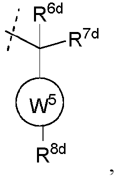 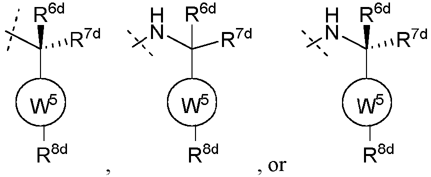 is optionally substituted;
R^{6d} and R^{7d} are each independently H, deuterium, C₃-C₈ cycloalkyl, or C₁-C₆ alkyl, wherein the C₃-C₈ cycloalkyl or C₁-C₆ alkyl is optionally substituted with halogen, -OH, CN, NO₂, or amino;
W⁵ is 6- to 10-membered aryl or 5- to 10-membered heteroaryl;
R^{8d} is H, deuterium, halogen, CN, OH, NO₂, NR^{6d}R^{7d}, OR^{6d}, COR^{6d}R^{7d}, NR^{6d}COR^{7d}, SO₂R^{6d}R^{7d}, R^{6d}SO₂R^{7d}, C₁-C₆ alkyl, C₁-C₆ alkoxy, C₆-C₁₀ aryl, 5- to 10-membered heteroaryl, C₃-C₈ cycloalkyl, or 3- to 8-membered heterocycloalkyl, wherein the C₁-C₆ alkyl or C₁-C₆ alkoxy is optionally substituted with deuterium, halogen, -OH, CN, NO₂, or amino.

2. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1, wherein
in formula I or I', the definitions of one or more of the groups R^{2a}, R^{2a'}, R^{3a}, C₁-C₄ alkylene in L', R^{a}, and R^{b} are also replaced by the following definitions, provided that L is linked to E via -C(O)-;
R^{2a} and R^{2a'} are independently -O-C₁-C₆ heteroalkyl, -NH(C₁-C₆ heteroalkyl), -N(C₁-C₆ heteroalkyl)(C₁-C₆ heteroalkyl), or -C₃-C₄ alkynyl-N(R^{5a'})₂;
in R^{2a} and R^{2a'}, the hydroxy is independently optionally substituted with 1 or more deuterium, halogen, hydroxy, cyano, amino, nitro, C₁-C₃ alkoxy, C₁-C₃ alkyl, C₃-C₈ cycloalkyl, -Si-C₁-C₃ alkyl, 3- to 8-membered heterocycloalkyl, or -CON(R^{3a})₂;
in R^{2a} and R^{2a'}, the C₁-C₆ alkyl, C₁-C₆ heteroalkyl, C₃-C₈ cycloalkyl, 3- to 8-membered heterocycloalkyl, -S-C₁-C₆ alkyl, C₂-C₆ alkenyl, C₂-C₆ alkynyl, -O-C₁-C₆ alkyl, -O-C₃-C₈ cycloalkyl, and 5- to 6-membered heteroaryl are independently optionally substituted with 1 or more C₃-C₈ cycloalkyl, -Si-C₁-C₃ alkyl, 3- to 8-membered heterocycloalkyl, or -CON(R^{3a})₂;
each R^{3a} is deuterium;
C₁-C₄ alkylene in L' is also optionally substituted with 1 or more halogen;
R^{a} and R^{b} are independently a bond, deuterium, oxo, cyanomethyl, -N(R^{3a})₂, -CH₂N(R^{3a})₋₂, -O(C₁-C₆ alkyl), -O(C₃-C₈ cycloalkyl), -O(C₁-C₆ heteroalkyl), -O(3- to 8-membered heterocycloalkyl), -S(C₁-C₆ alkyl), - S(C₃-C₈ cycloalkyl), -O-phenyl, -O-pyridyl, C₂-C₄ alkynyl, triazolyl, -CH₂C(=O)N(R^{3a})₂, -C₃-C₄ alkynyl-N(R^{3a})₂, or -(3- to 8-membered heterocycloalkyl);
or two adjacent R^{a}, together with the atoms linked thereto, form 6- to 10-membered aryl, 5- to 10-membered heteroaryl, 5- to 8-membered cycloalkyl, or 5- to 8-membered heterocycloalkyl, wherein the 6- to 10-membered aryl, 5- to 10-membered heteroaryl, 5- to 8-membered cycloalkyl, or 5- to 8-membered heterocycloalkyl is optionally substituted with 1 or more hydroxy, amino, halogen, cyano, or nitro;
in R^{a} and R^{b}, the C₁-C₆ alkyl, C₁-C₃ alkoxy, C₂-C₄ alkenyl, C₃-C₈ cycloalkyl, 3- to 8-membered heterocycloalkyl, or 5- to 6-membered heteroaryl is optionally substituted with 1 or more C₁-C₃ haloalkyl; in R^{a} and R^{b}, the C₁-C₆ heteroalkyl, phenyl, pyridyl, C₂-C₄ alkynyl, triazolyl, -CH₂C(=O)N(R^{3a})₁₋₂, or -C₃-C₄ alkynyl-N(R^{3a})₂ is optionally substituted with 1 or more deuterium, halogen, cyano, hydroxy, amino, nitro, C₁-C₃ haloalkyl, C₁-C₃ alkyl, or C₁-C₃ alkoxy;
each R^{4a} is independently C₄-C₆ alkyl.

3. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein 1 or more of the following conditions are met:
(1) 1, 2, 3, or 4 methylene groups in each C₁-C₆ heteroalkyl are replaced by a heteroatom and/or a heteroatom group, wherein the heteroatom is one or more of O, S, and N; and the heteroatom group is one or more of - C(O)-, -S(O)-, -S(O)₂-,-C(O)O-, -OC(O)-, -C(O)NH-, and -NHC(O)-;
(2) the heteroatoms or heteroatom groups in each 3- to 8-membered heterocycloalkyl, each 4- to 12-membered heterocycloalkyl, each 5- to 16-membered heterocycloalkyl, each heterocycloalkyl, each 5- to 6-membered heteroaryl, each 5- to 10-membered heteroaryl, each 5- to 12-membered heteroaryl, each 5- to 15-membered heteroaryl, and each heteroaryl are each independently one or more of O, S, -S(O)-, -S(O)₂-, and N, the number of the heteroatoms or heteroatom groups being 1, 2, 3, 4, or 5;
(3) in each R^{1a}, the C₃-C₁₂ cycloalkyl is cyclopropyl, cyclobutyl, cyclopentyl, or cyclohexyl;
(4) in each R^{1a}, the 3- to 12-membered heterocycloalkyl is 5- to 6-membered monocyclic saturated heterocycloalkyl or 7-, 8-, or 9-membered bridged heterocycloalkyl containing 1 or 2 heteroatoms of N, such as piperidinyl, 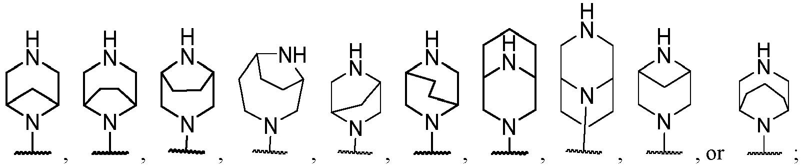
(5) in each R^{2a} and each R^{2a'}, the halogen is F, Cl, Br, or I, such as F or Cl;
(6) in each R^{2a} and each R^{2a'}, the C₁-C₆ alkyl, C₁-C₆ alkyl in the -S-C₁-C₆ alkyl, and C₁-C₆ alkyl in the -O-C₁-C₆ alkyl are independently C₁-C₄ alkyl, and may also be C₁-C₃ alkyl, such as methyl, ethyl, n-propyl, or isopropyl;
(7) in each R^{2a} and each R^{2a'}, the C₁-C₆ heteroalkyl is C₃ heteroalkyl, wherein in the heteroalkyl, 2 methylene groups are replaced by O and -C(O)NH-, such as 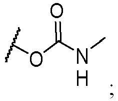
(8) in each R^{2a} and each R^{2a'}, the C₃-C₈ cycloalkyl is C₃-C₆ cycloalkyl, such as cyclopropyl, cyclobutyl, cyclopentyl, or cyclohexyl;
(9) in each R^{2a'}, the -O-(3- to 8-membered heterocycloalkyl) is -O-(3-, 4-, 5-, or 6-membered monocyclic saturated heterocycloalkyl) containing 1, 2, or 3 heteroatoms being one or more of N, S, and O, such as 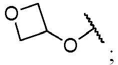
(10) in each R^{2a} and each R^{2a'}, the 3- to 8-membered heterocycloalkyl is 3- to 6-membered heterocycloalkyl containing 1, 2, or 3 heteroatoms being one or more of N, S, and O;
(11) in each R^{2a} and each R^{2a'}, the C₂-C₆ alkynyl is C₂-C₄ alkynyl, such as ethynyl;
(12) each R^{2a} and each R^{2a'} are optionally substituted with substituents, wherein in the substituents, the halogen is F, Cl, Br, or I, such as F;
(13) each R^{2a} and each R^{2a'} are optionally substituted with substituents, wherein in the substituents, the C₁-C₃ alkoxy is methoxy, ethoxy, n-propoxy, or isopropoxy, such as methoxy;
(14) each R^{2a} and each R^{2a'} are optionally substituted with substituents, wherein in the substituents, the C₁-C₃ alkyl is methyl, ethyl, n-propyl, or isopropyl, such as methyl;
(15) in each R^{3a}, the C₁-C₆ alkyl, C₁-C₆ alkyl in the -S-C₁-C₆ alkyl, and C₁-C₆ alkyl in the -O-C₁-C₆ alkyl are independently C₁-C₄ alkyl, and may also be C₁-C₃ alkyl, such as methyl, ethyl, n-propyl, or isopropyl;
(16) in each ring A1, 6- to 15-membered aryl in the -L'-(6- to 15-membered aryl) is 6- to 10-membered aryl, such as phenyl or naphthyl;
(17) in each ring A1, 5- to 15-membered heteroaryl in the -L'-(5- to 15-membered heteroaryl) is 5- to 10-membered heteroaryl, and may also be 6-, 7-, 8-, or 9-membered monocyclic or bicyclic heteroaryl, containing 1 or 2 heteroatoms being one or more of N, S, and O, such as pyridyl, 1*H*-indazolyl, phenyl[*d*]thiazole, or oxazolophenyl;
(18) in each ring A1, 5- to 15-membered heteroaryl in the -L'-(5- to 15-membered heteroaryl) is 5- to 10-membered heteroaryl, and may also be 6-, 7-, 8-, or 9-membered monocyclic or bicyclic heteroaryl, containing 1 or 2 heteroatoms being one or more of N, S, and O, such as pyridyl, 1*H*-indazolyl, phenyl[*d*]thiazole, or oxazolophenyl;
(19) in each ring A1, 5- to 15-membered heterocycloalkyl in the -L'-(5- to 15-membered heterocycloalkyl) is 5- to 10-membered heterocycloalkyl containing 1 or 2 heteroatoms being one or more of N, S, and O;
(20) in each ring B, the 6- to 15-membered aryl is 6- to 10-membered aryl, such as phenyl or naphthyl;
(21) in each ring B, the 5- to 15-membered heteroaryl is 5- to 10-membered heteroaryl, and may also be 5-to 6-membered heteroaryl, containing 1 or 2 heteroatoms of N, such as pyridyl;
(22) in each ring B, the C₅-C₁₅ cycloalkyl is C₅-C₇ monocyclic saturated cycloalkyl, C₅-C₇ cycloalkenyl, or C₈-C₁₅ tricyclic cycloalkyl, such as 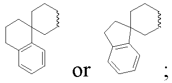
(23) in each ring B, the 5- to 16-membered heterocycloalkyl is 5- to 7-membered monocyclic heterocycloalkyl containing 1 or 2 heteroatoms of N and/or O, such as pyrrolidinyl, piperidinyl, or tetrahydro-2H-pyranyl;
(24) in each R^{a} and each R^{b}, the halogen is F, Cl, Br, or I, such as F or Cl;
(25) in each R^{a} and each R^{b}, the C₁-C₆ alkyl is C₁-C₃ alkyl, such as methyl, ethyl, n-propyl, or isopropyl; and
(26) in each R^{a} and each R^{b}, the C₁-C₃ alkoxy and C₁-C₃ alkoxy in the (C₁-C₃ alkoxy)-C₁-C₃ alkyl- are independently methoxy, ethoxy, n-propoxy, or isopropoxy.

4. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein 1 or more of the following conditions are met:
(1) each ring B is independently 6- to 15-membered aryl, 5- to 15-membered heteroaryl, or 5- to 16-membered heterocycloalkyl, and each R^{a} is independently halogen, hydroxy, amino, cyano, -C₁-C₆ alkyl, - C₁-C₆ heteroalkyl, -C₃-C₈ cycloalkyl, -C₂-C₆ alkenyl, -C₂-C₆ alkynyl, -C₃-C₈ heterocycloalkyl (e.g., 3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂ substitution;
preferably, each ring B is independently 6- to 15-membered aryl, 5- to 15-membered heteroaryl, or 5- to 16-membered heterocycloalkyl, and each R^{a} is independently halogen, hydroxy, amino, cyano, -C₁-C₆ alkyl, - C₁-C₆ heteroalkyl, -C₃-C₆ alkenyl, -C₃-C₆ alkynyl, -C₃-C₈ heterocycloalkyl (e.g., 3- to 8-membered heterocycloalkyl), -O(C₁-C₆ alkyl), -S(C₁-C₆ alkyl), or -N(C₁-C₆ alkyl)₁₋₂ substitution;
more preferably, R^{a} is F, Cl, hydroxy, cyano, or methyl; and
(2) each ring A1 is C₆-C₁₅ aryl or 5- to 15-membered heteroaryl; R^{2a} and R^{2a'} are independently hydroxy, amino, halogen, C₁-C₆ alkyl, C₁-C₆ heteroalkyl, -S-C₁-C₆ alkyl, -O-C₁-C₆ alkyl, C₃-C₈ cyclopropyl, C₂-C₆ alkynyl, or -O-(3- to 8-membered heterocycloalkyl), wherein the C₁-C₆ alkyl, -S-C₁-C₆ alkyl, and C₃-C₈ cyclopropyl are optionally substituted with halogen, CN, -OH, -NH₂, or C₁-C₃ alkyl;
preferably, R^{2a} and R^{2a'} are independently hydroxy, amino, halogen, C₁-C₆ alkyl, C₁-C₆ heteroalkyl, -S-C₁-C₆ alkyl, -O-C₁-C₆ alkyl, C₃-C₈ cyclopropyl, or C₂-C₆ alkynyl, wherein the C₁-C₆ alkyl, -S-C₁-C₆ alkyl, and C₃-C₈ cyclopropyl are optionally substituted with halogen, CN, -OH, -NH₂, or C₁-C₃ alkyl;
more preferably, R^{2a} and R^{2a'} are independently -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 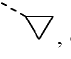 or -SCH₃ substitution, wherein the -CH₃, -CH₂CH₃, - CH₂CH₂CH₃, -CH(CH₃)₂-, 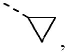 or -SCH₃ is optionally substituted with halogen, CN, -OH, -NH₂, or C₁-C₃ alkyl;
further preferably, R^{2a} and R^{2a'} are independently hydroxy, amino, F, Cl, methyl, ethyl, isopropyl, trifluoromethyl, 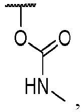 -SCH₃, 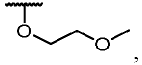 cyclopropyl, or

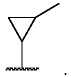

5. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 4, wherein 1 or more of the following conditions are met:
(1) the structural unit 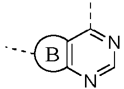 is 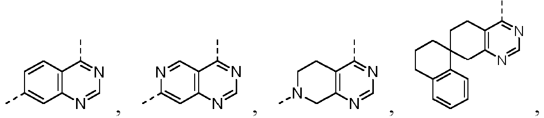 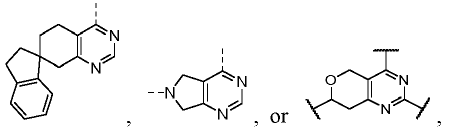 wherein the 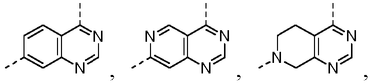 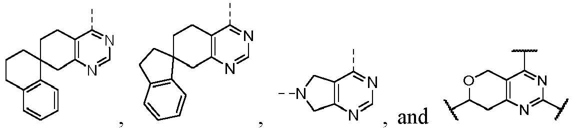 are independently optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂; the structural unit 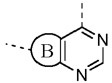 may be 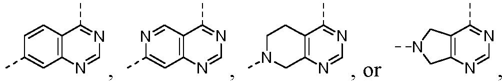 wherein the 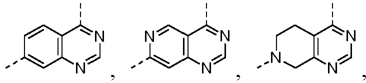 and 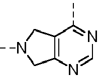 are independently optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -C₃-C₈ heterocycloalkyl, -O(C₁-C₆ alkyl), -O(C₃-C₈ cycloalkyl), -S(C₁-C₆ alkyl), -S(C₃-C₈ cycloalkyl), -N(C₁-C₆ alkyl)₁₋₂, or -N(C₃-C₈ cycloalkyl)₁₋₂; the structural unit 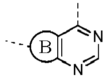 may also be 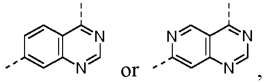 wherein the 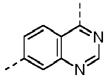 and 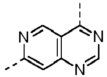 are optionally substituted with F, Cl, Br, I, hydroxy, amino, cyano, -C₁-C₆ alkyl, -C₁-C₆ heteroalkyl, -C₃-C₆ alkenyl, -C₃-C₆ alkynyl, -C₃-C₈ heterocycloalkyl (e.g., 3- to 8-membered heterocycloalkyl), -O(C₁-C₆ alkyl), -S(C₁-C₆ alkyl), or -N(C₁-C₆ alkyl)₁₋₂; the structural unit 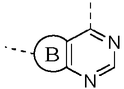 may further be 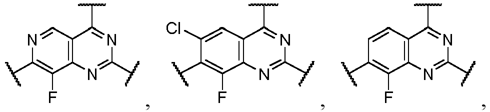 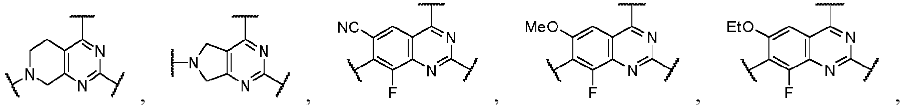 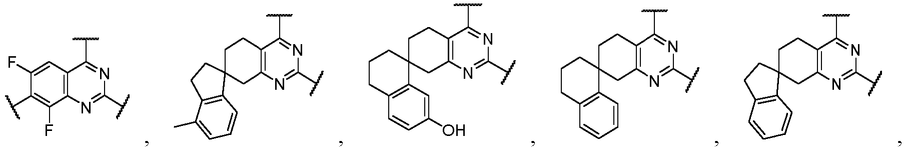 or 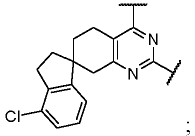
(2) the structural unit 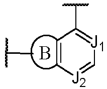 is 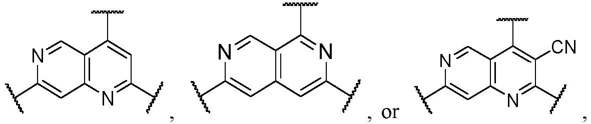 and the structural unit 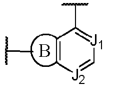 is optionally substituted with F, Cl, Br, I, hydroxy, amino, cyano, -C₁-C₆ alkyl, -C₁-C₆ heteroalkyl, -C₃-C₈ cycloalkyl, -C₂-C₆ alkenyl, -C₂-C₆ alkynyl, -(3- to 8-membered heterocycloalkyl), -O(C₁-C₆ alkyl), -O(C₃-C₈ cycloalkyl), -S(C₁-C₆ alkyl), -S(C₃-C₈ cycloalkyl), -NH(C₁-C₆ alkyl), -N(C₁-C₆ alkyl)₂, -NH(C₃-C₈ cycloalkyl), or -N(C₃-C₈ cycloalkyl)₂; the structural unit 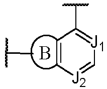 may further be 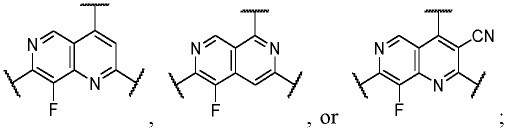 and
(3) ring A1 is phenyl, pyridyl, naphthyl, 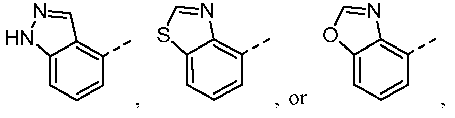 wherein the phenyl, pyridyl, naphthyl, 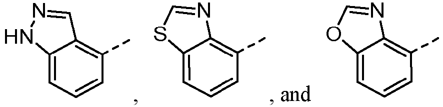 are independently optionally substituted with -OH, F, Cl, Br, I, CN, -NH₂, -CH₃, -CH₂CH₃, -CH₂CH₂CH₃, -CH(CH₃)₂-, -OCH₃, OCH₂CH₃, -C≡CH, 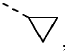 or -SCH₃, the -CH₃, -CH₂CH₃, -CH₂CH₂CH₃, -CH(CH₃)₂-, 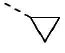 , or -SCH₃ being optionally substituted with halogen, CN, -OH, -NH₂, or C₁-C₃ alkyl.

6. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 5, wherein 1 or more of the following conditions are met:
(1) 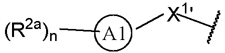 is absent, 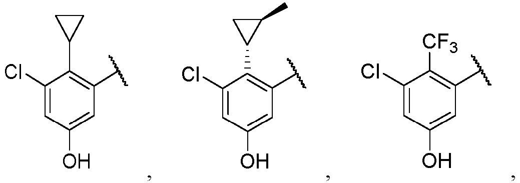 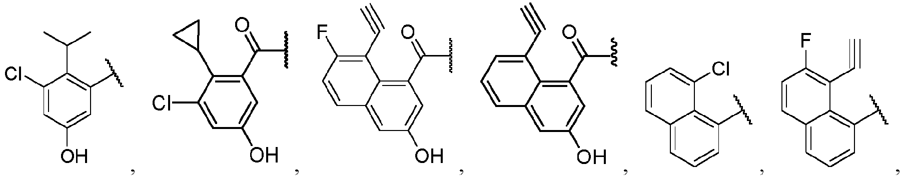 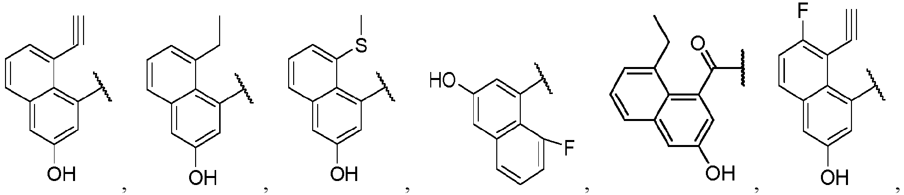 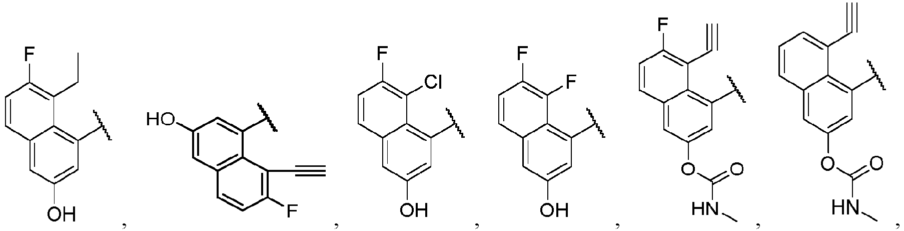 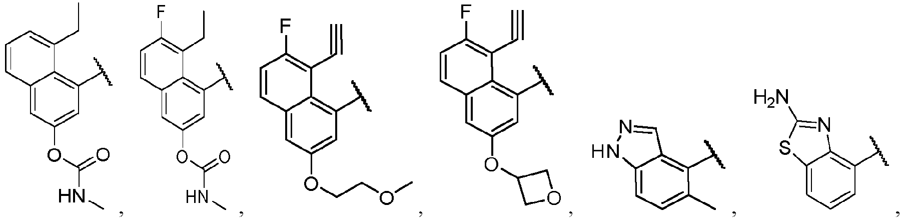 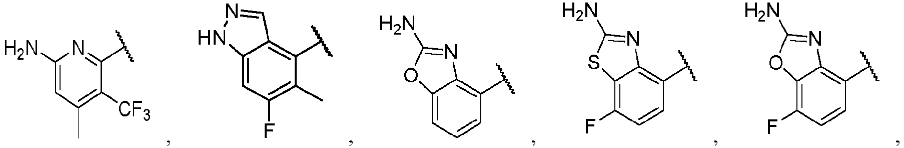 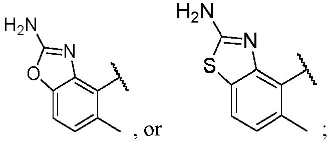
(2) the structural unit 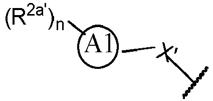 is 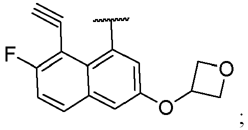
(3) the structural unit 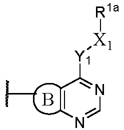 is 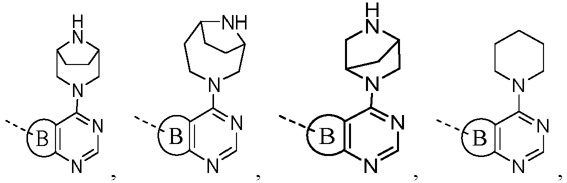 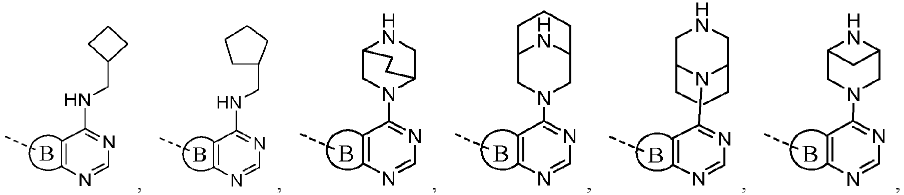 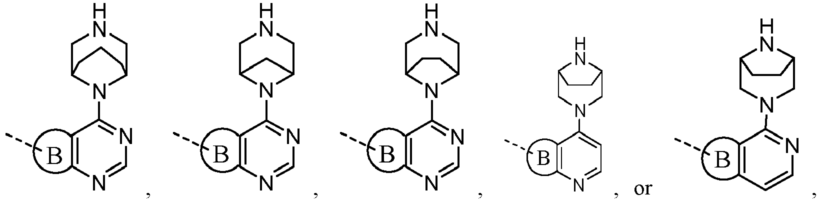 wherein the 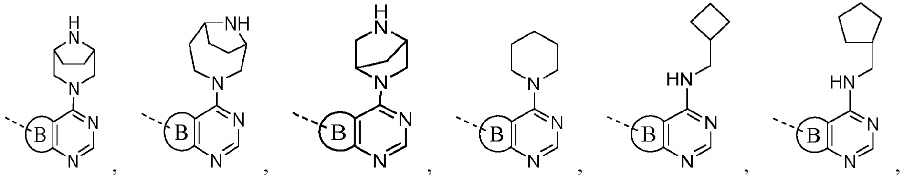 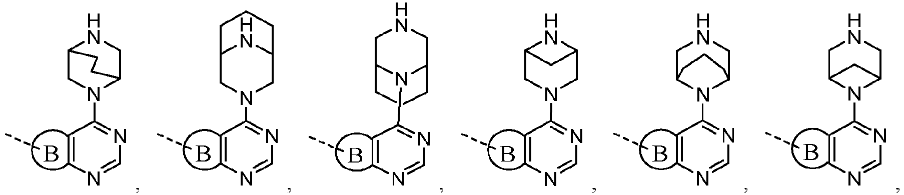 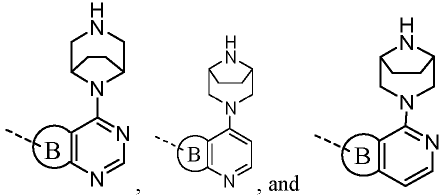 are independently optionally substituted with 1, 2, or 3 R^{a}; the structural unit 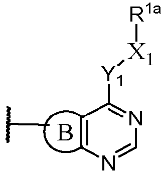 may be 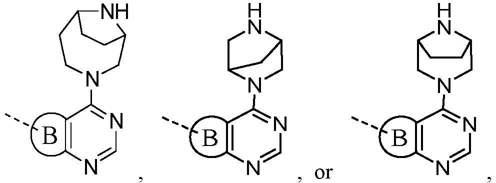 wherein the 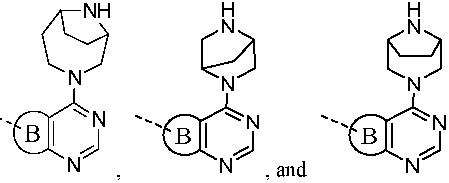 with 1 or 2 R^{a}; are independently optionally substituted the structural unit 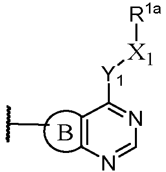 may further be 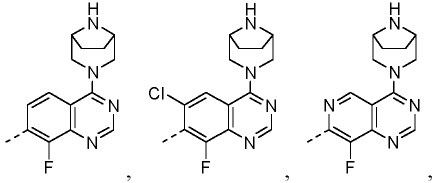 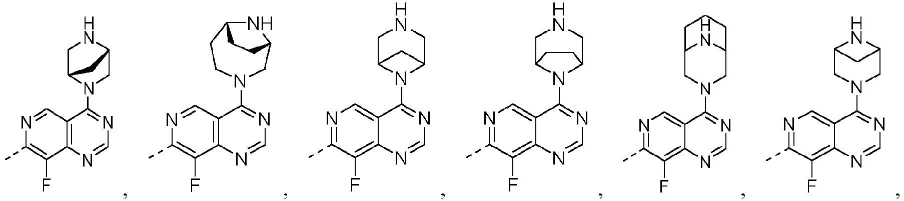 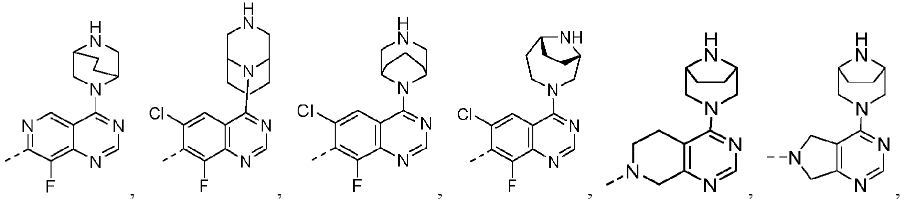 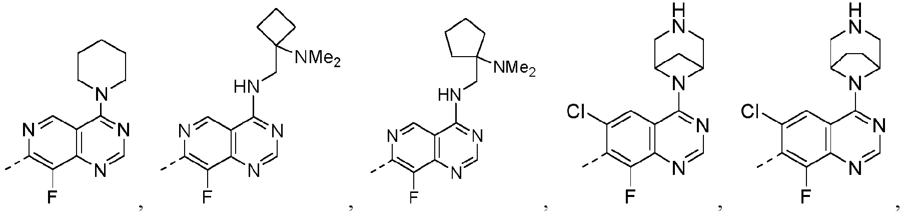 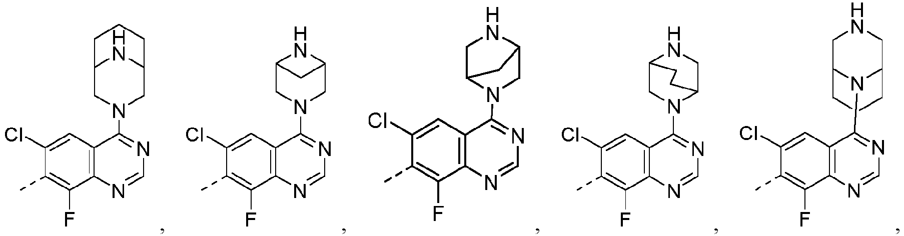 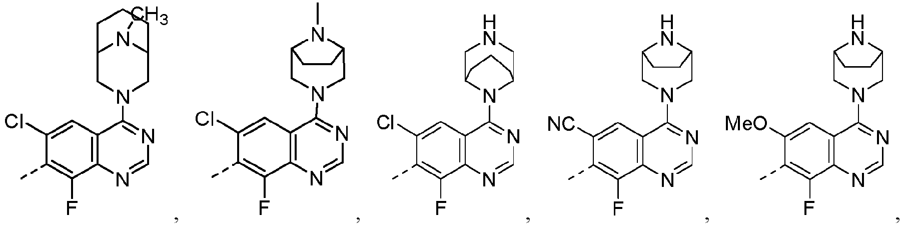 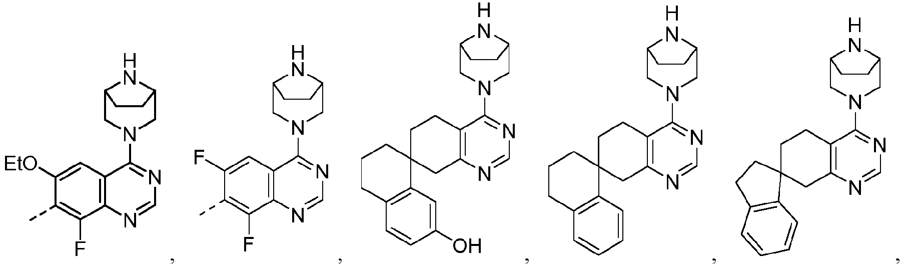 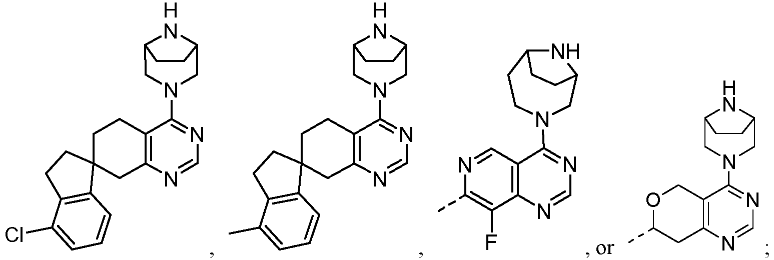 for example, the structural unit 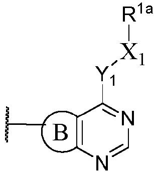 is 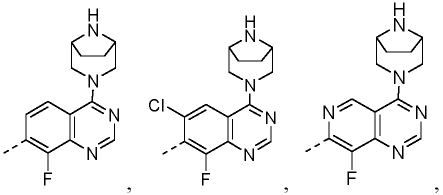 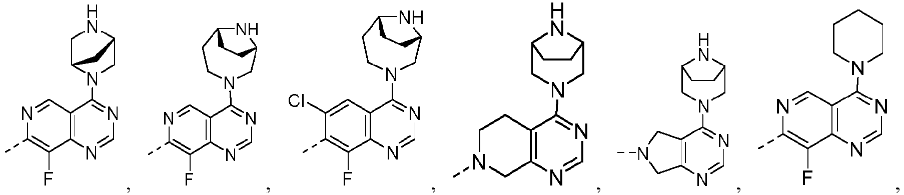 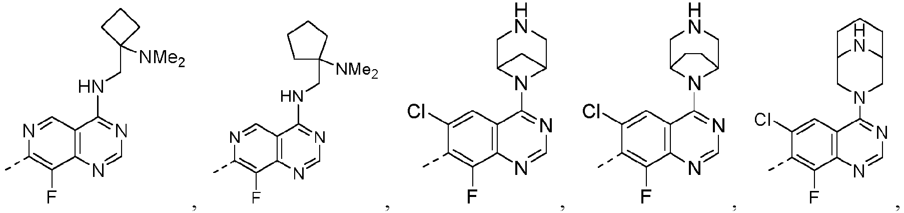 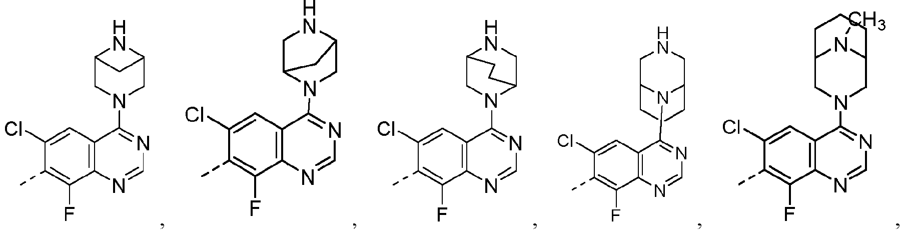 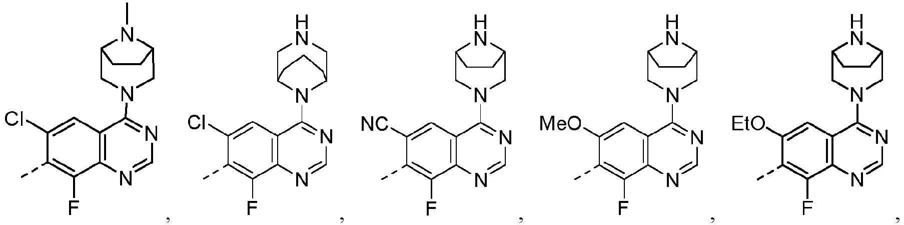 or 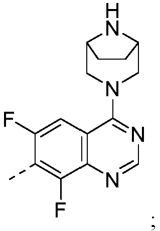 for another example, the structural unit 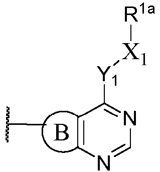 is 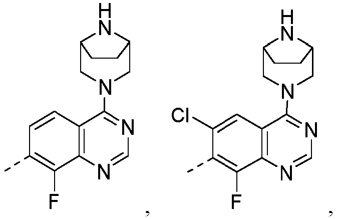 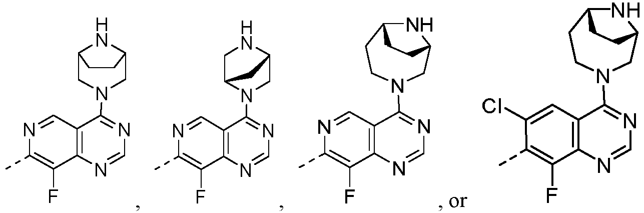 ; and
(4) the structural unit 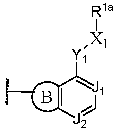 is 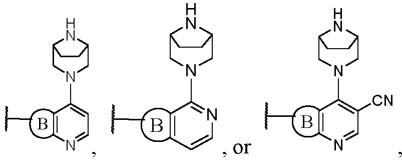 and the structural unit 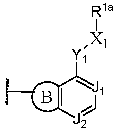 is optionally substituted with 1, 2, or 3 R^{a}; the structural unit 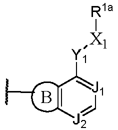 may further be

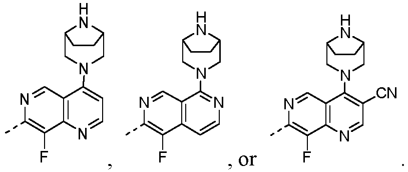

7. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein 1 or more of the following conditions are met:
(1) the structural unit 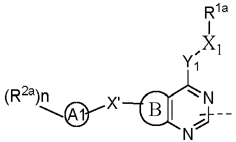 is: 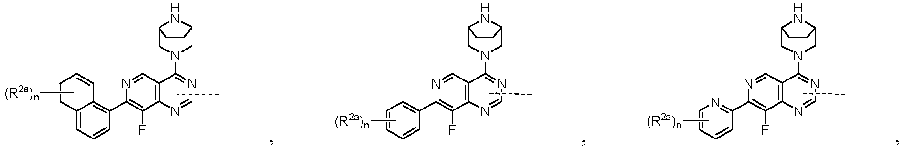 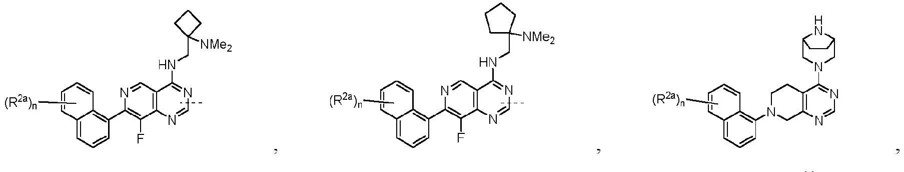 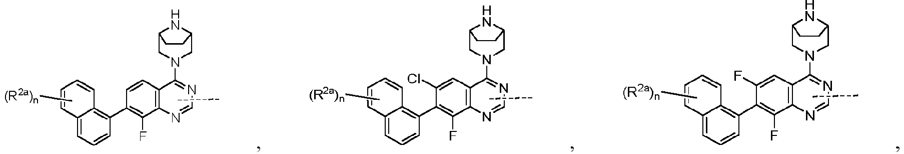 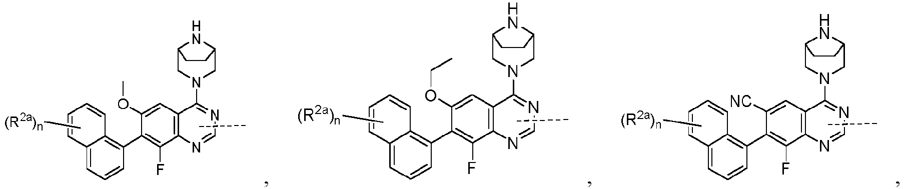 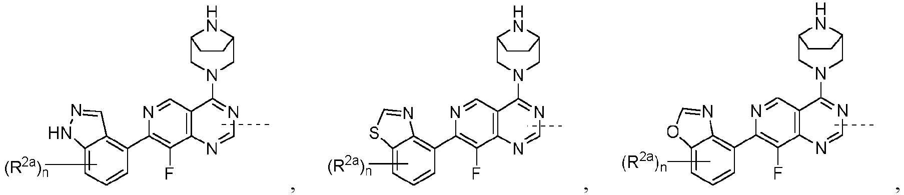 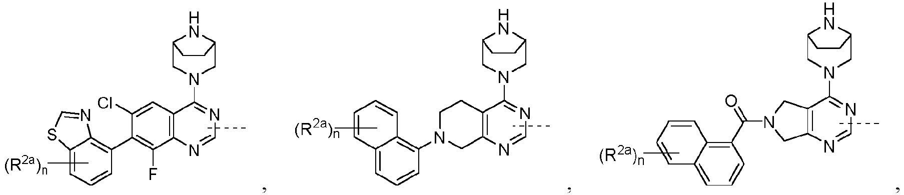 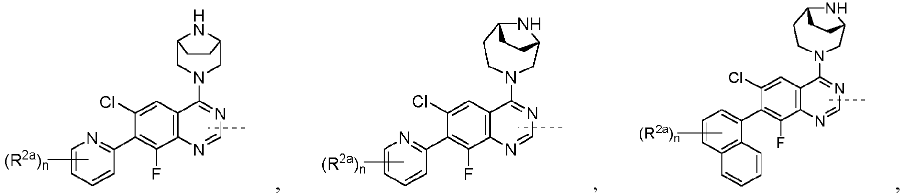 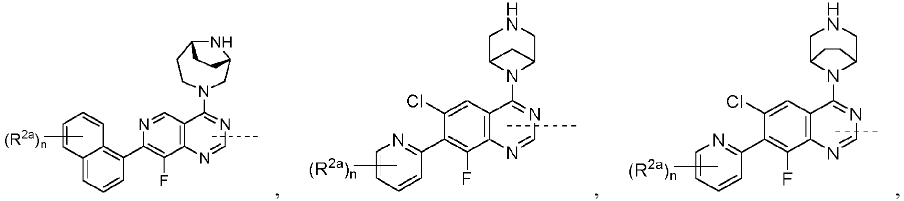 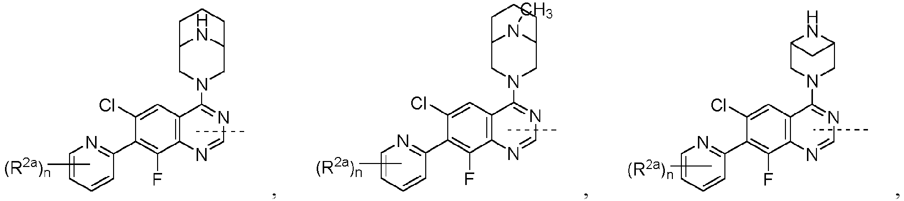 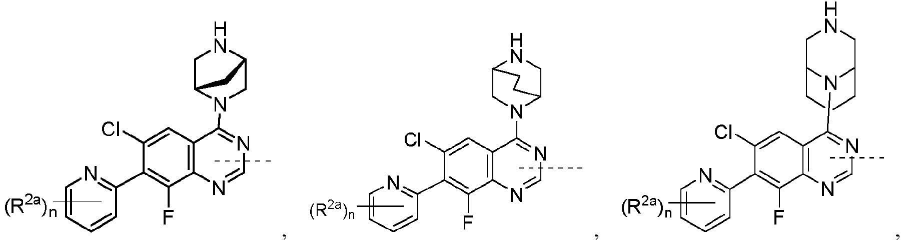 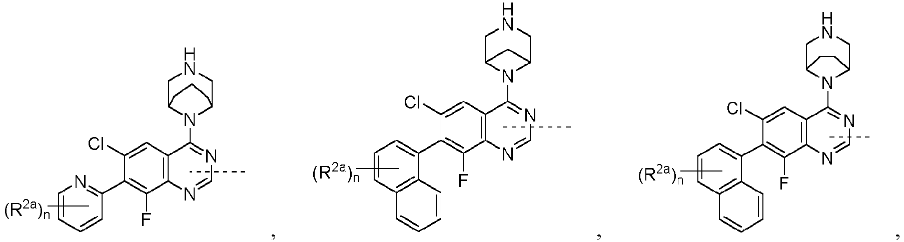 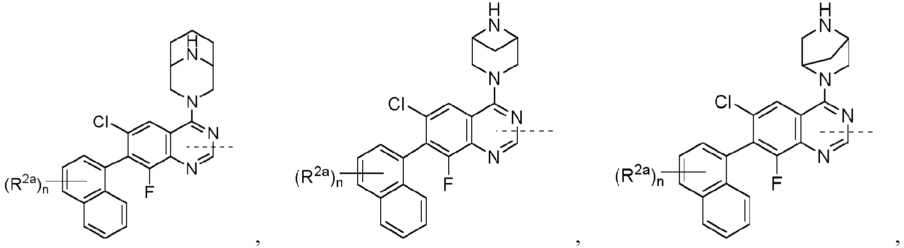 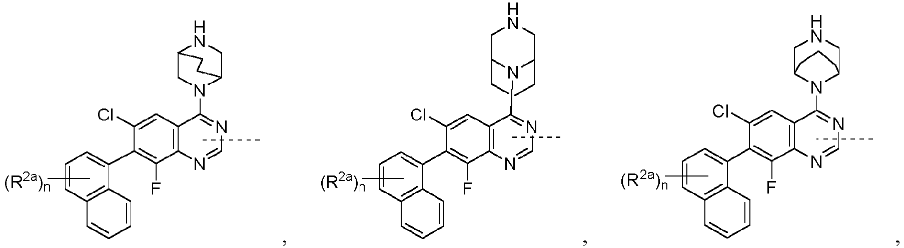 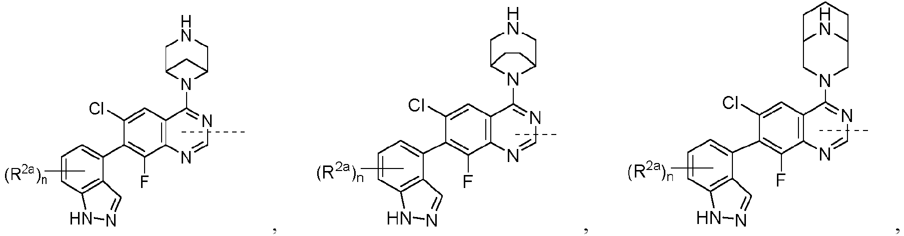 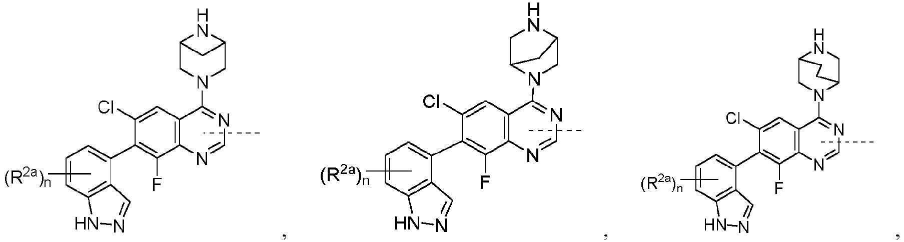 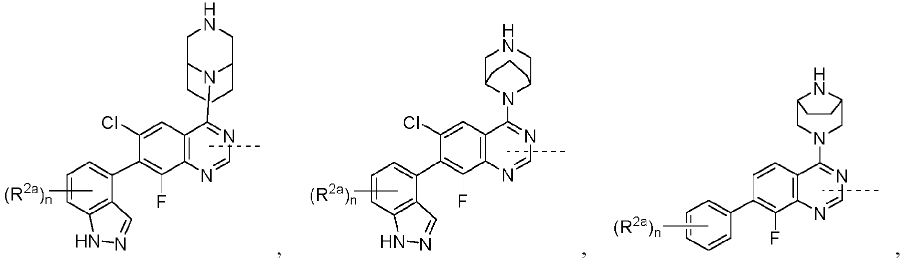 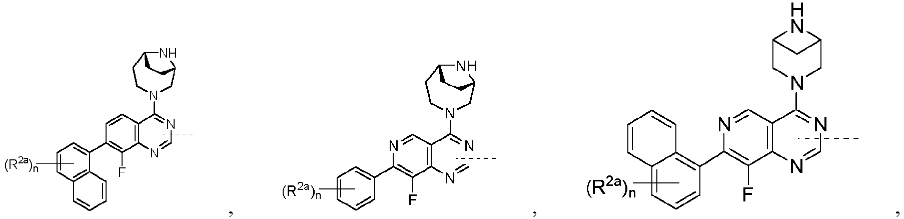 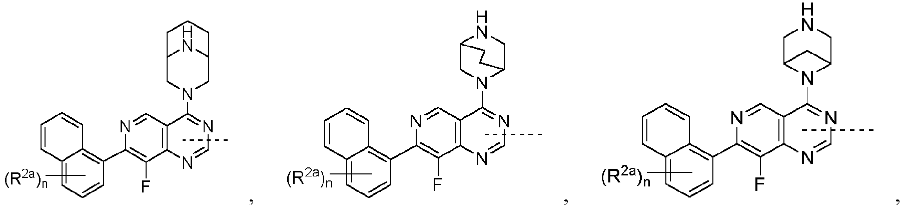 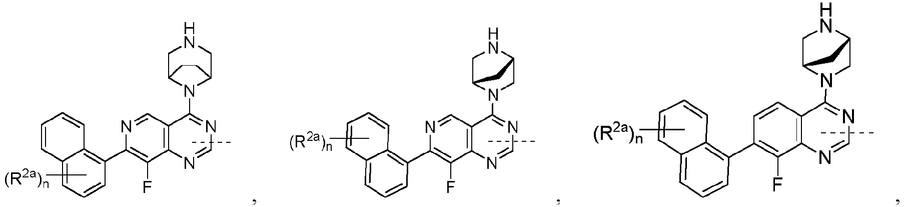 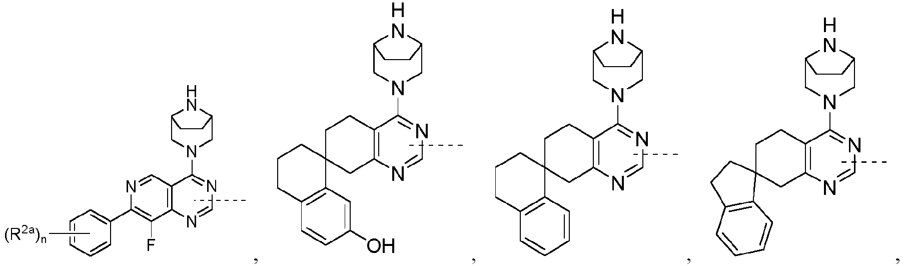 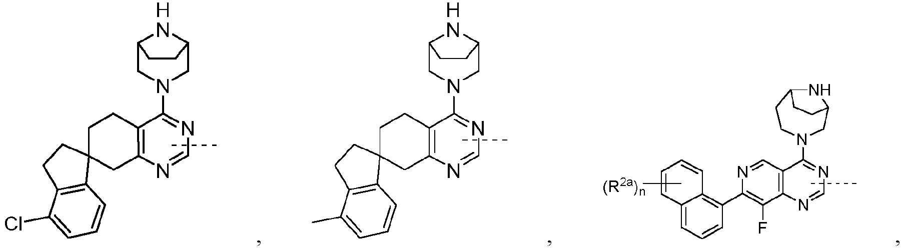 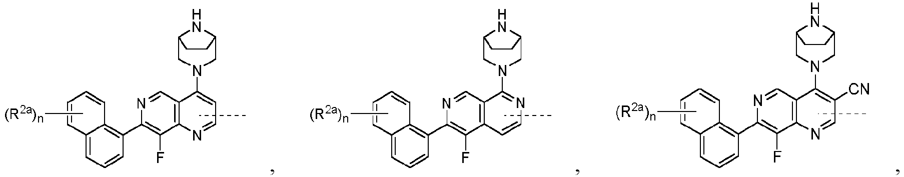 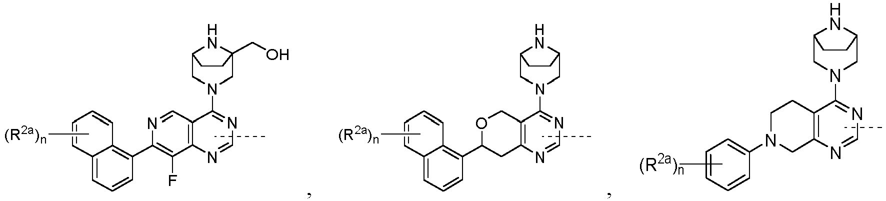 , or 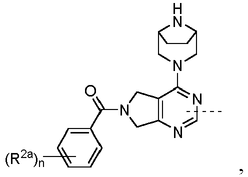 such as 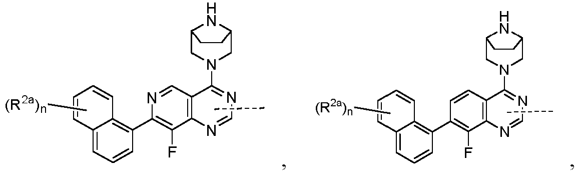 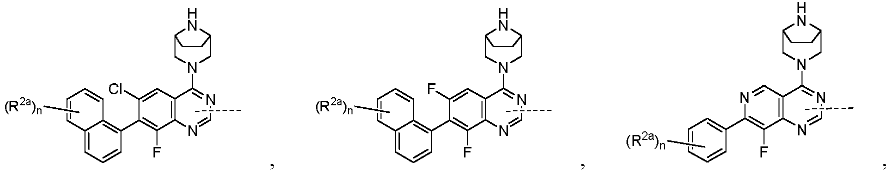 or 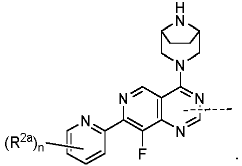 the structural unit 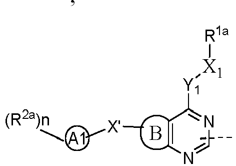 may be: 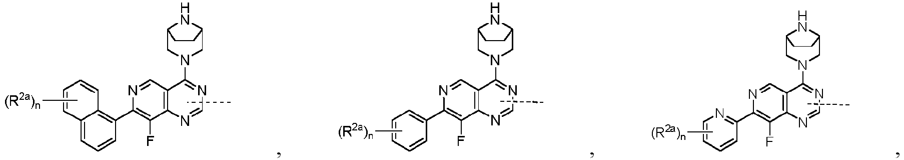 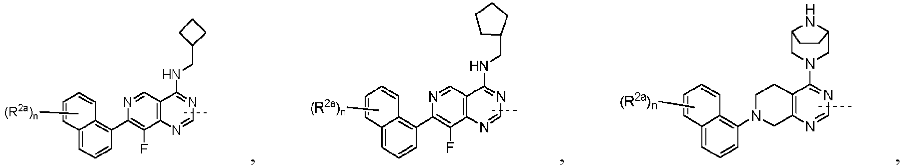 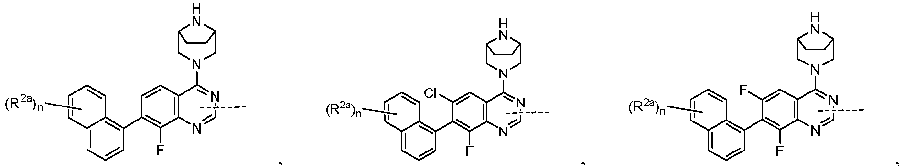 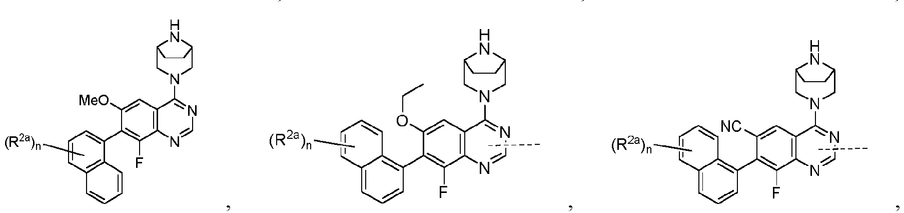 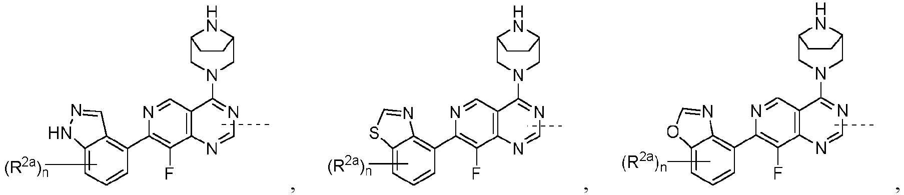 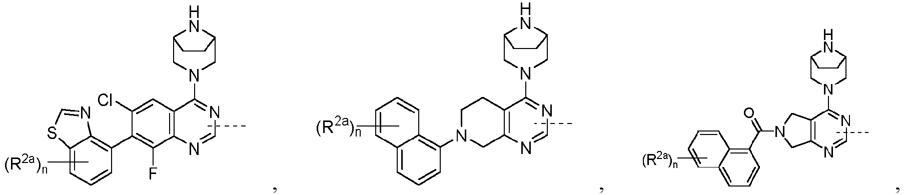 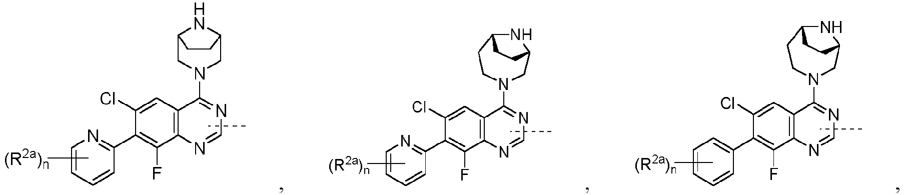 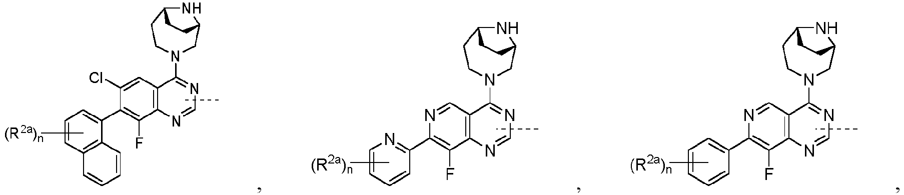 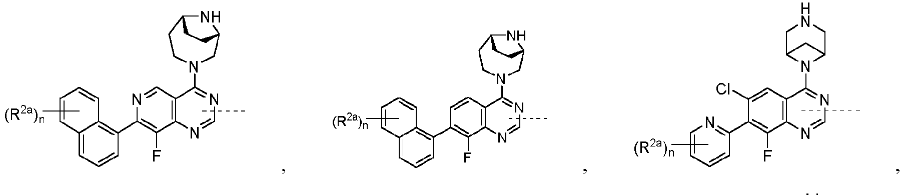 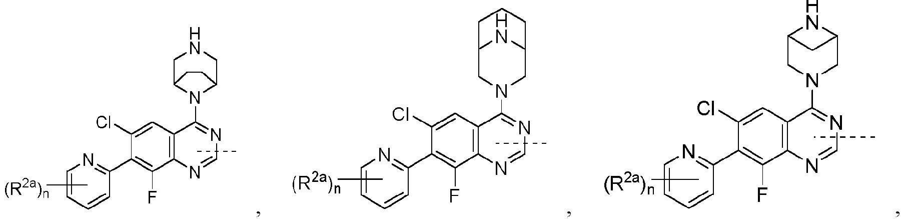 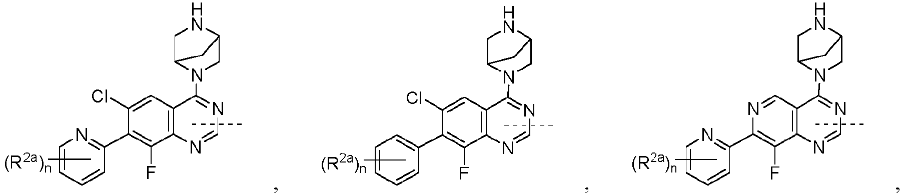 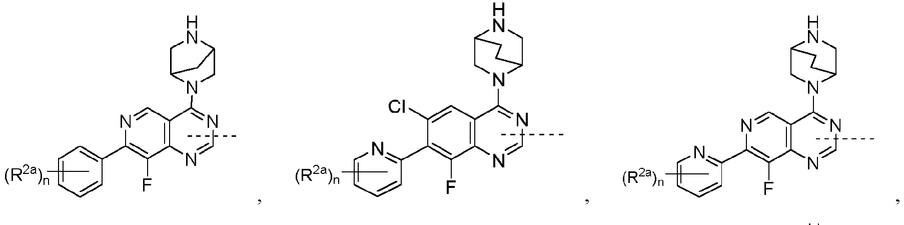 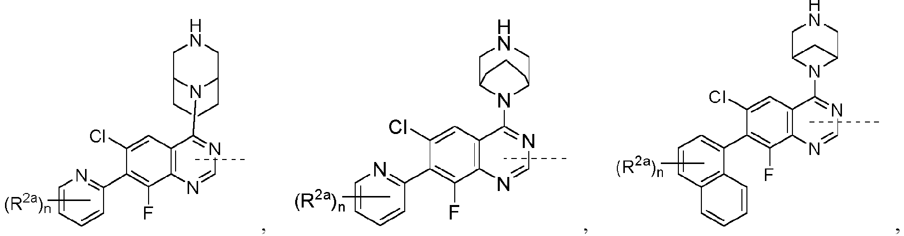 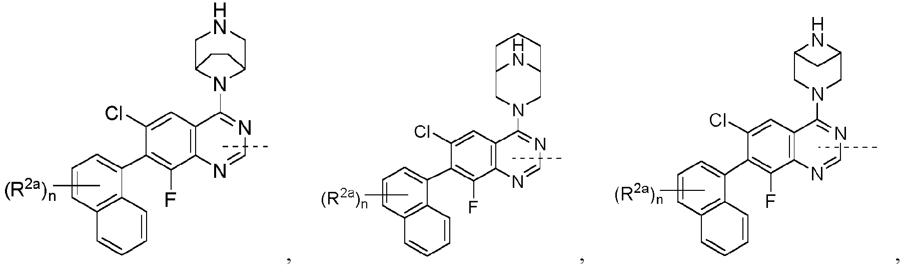 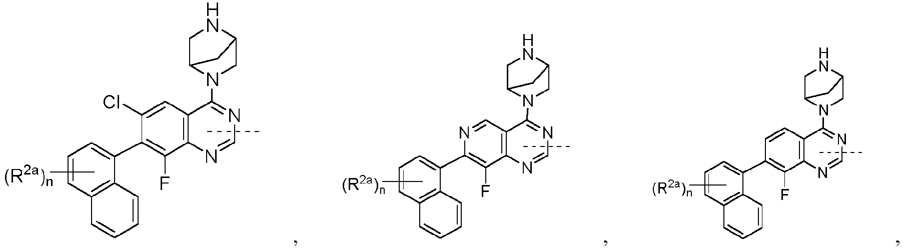 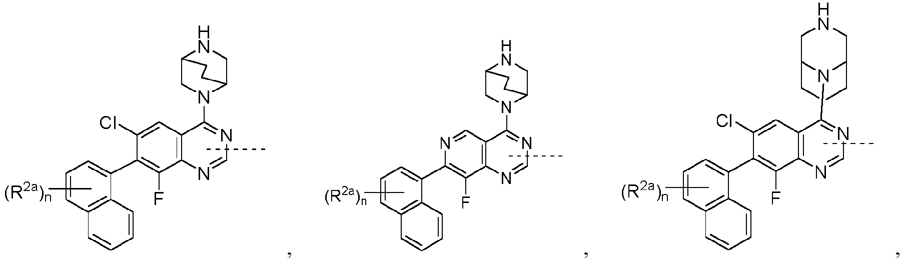 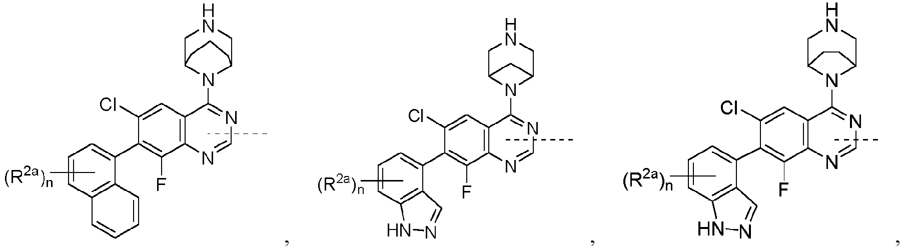 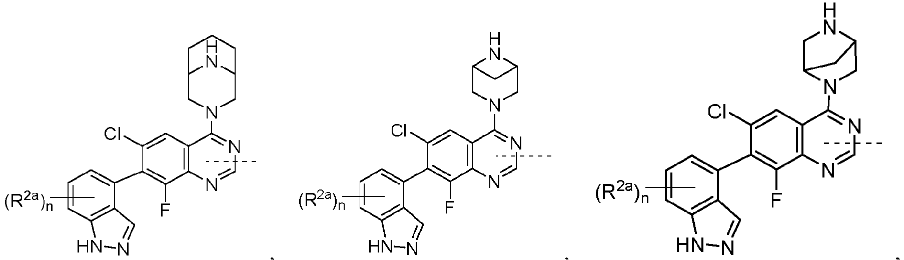 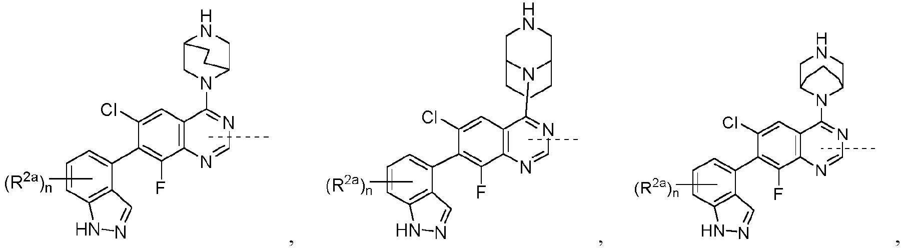 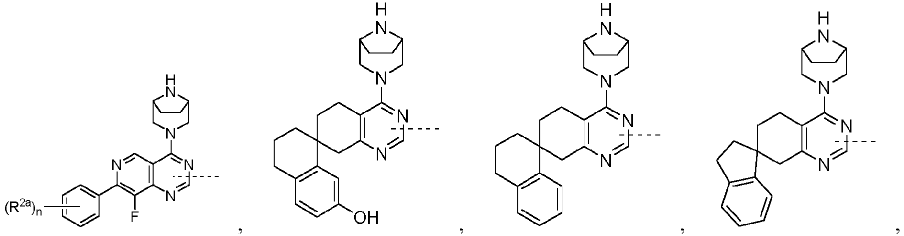 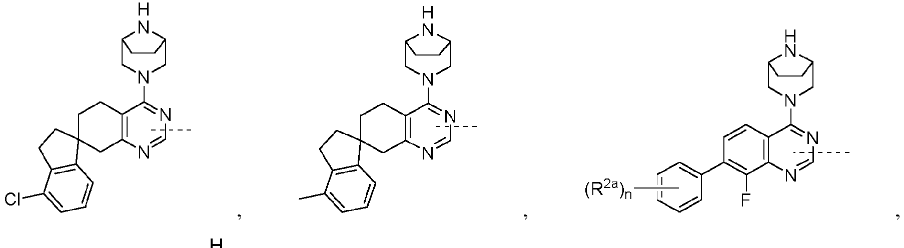 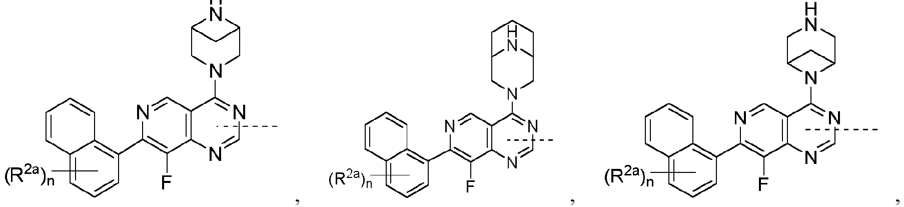 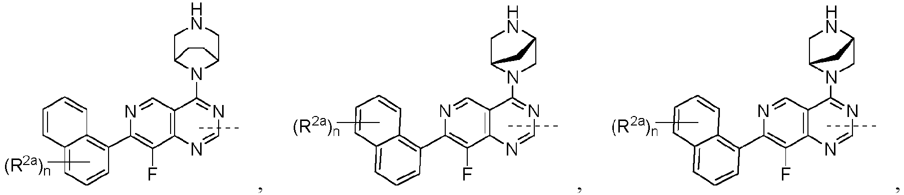 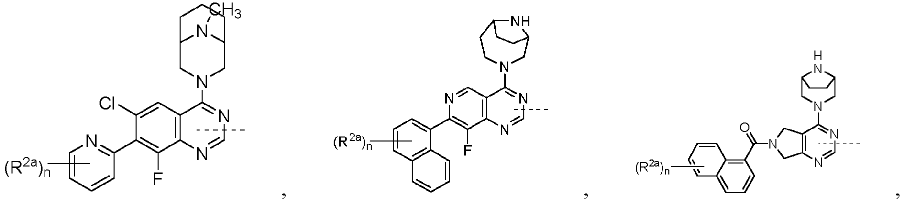 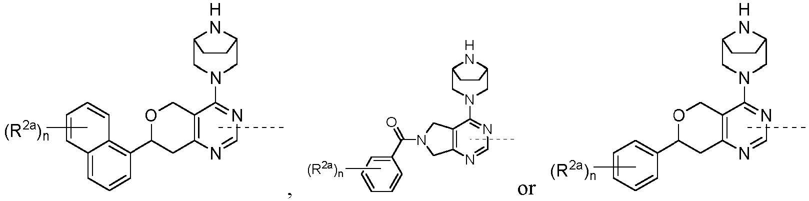 , wherein the 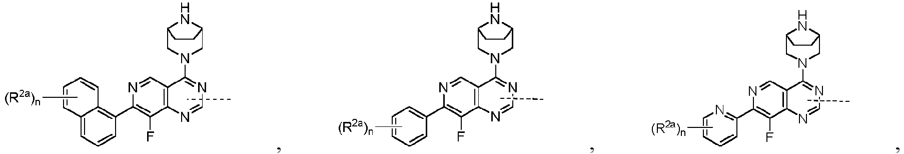 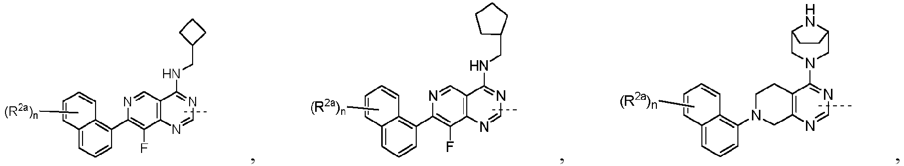 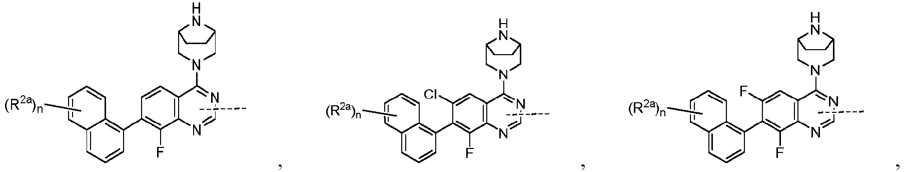 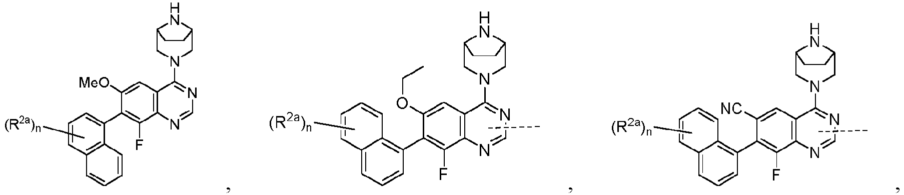 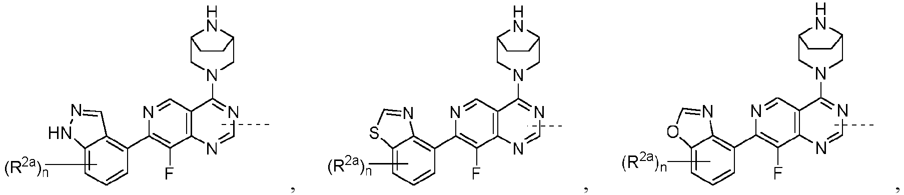 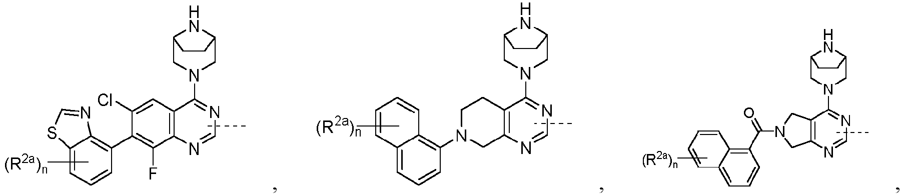 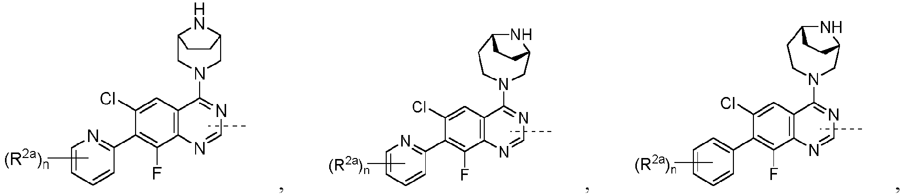 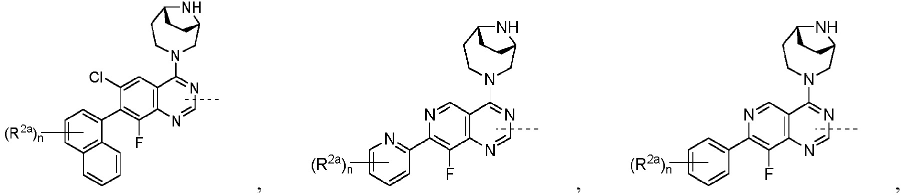 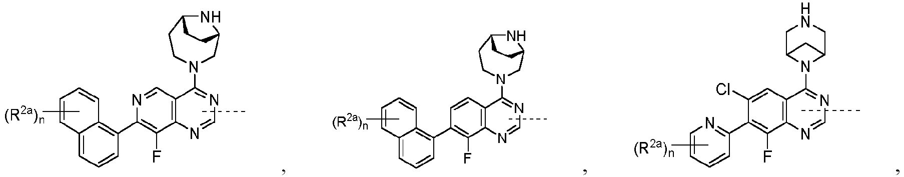 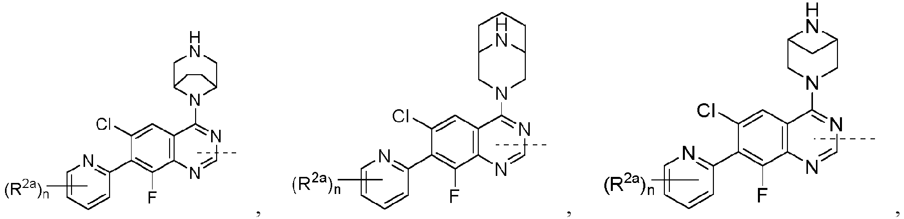 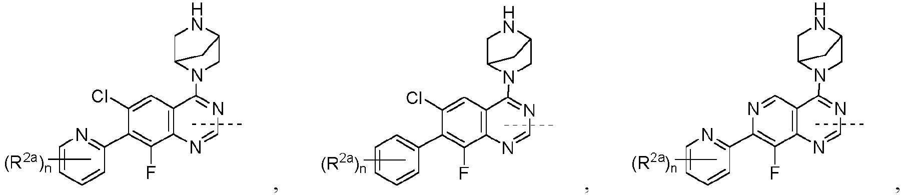 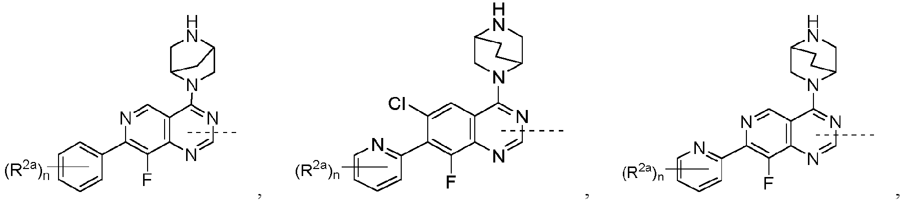 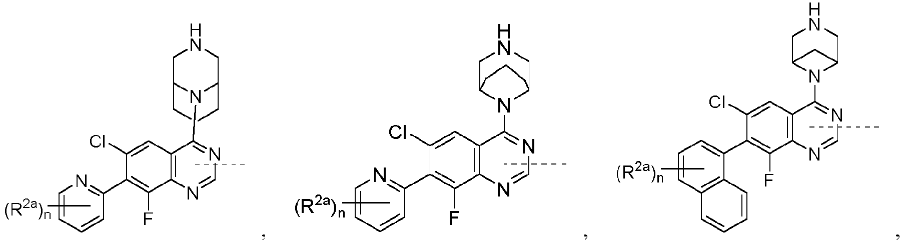 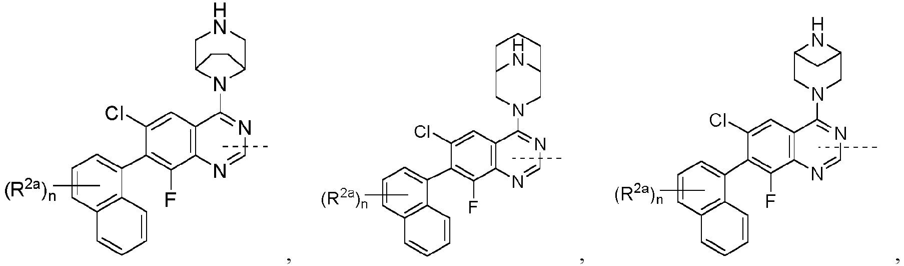 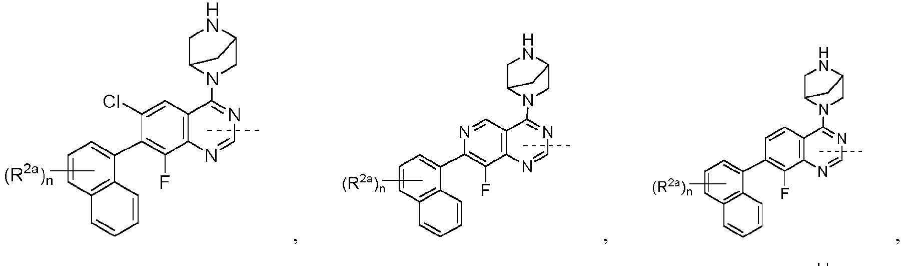 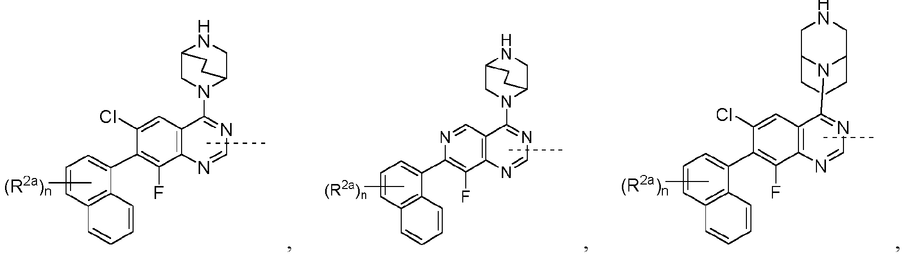 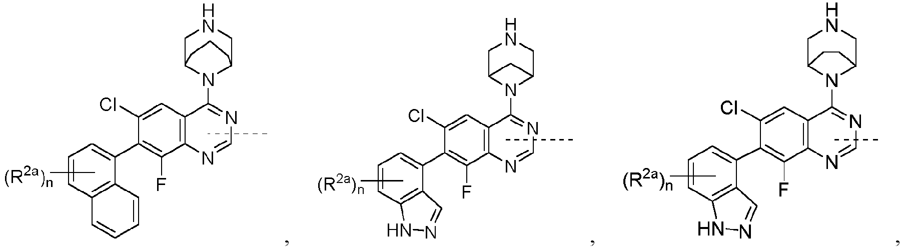 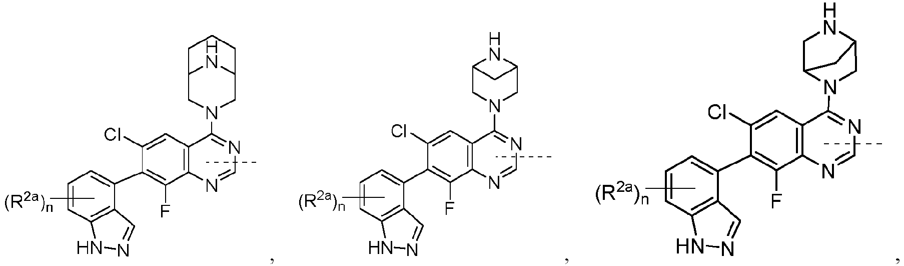 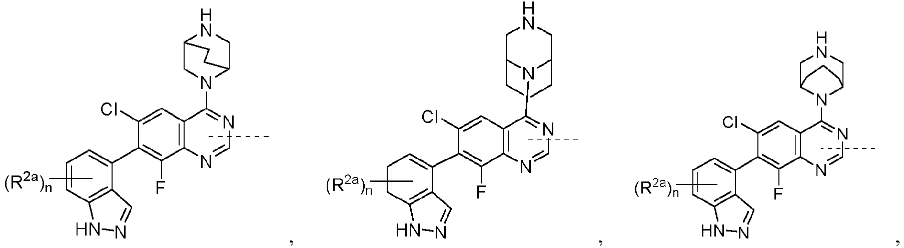 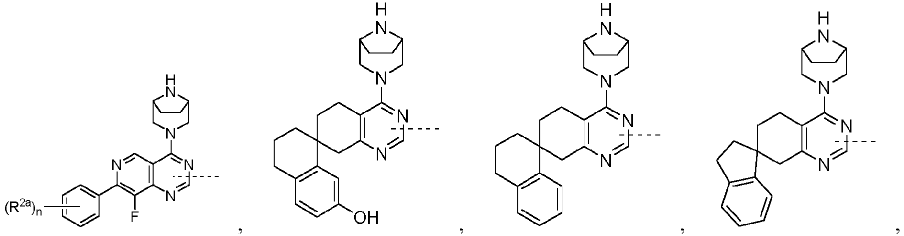 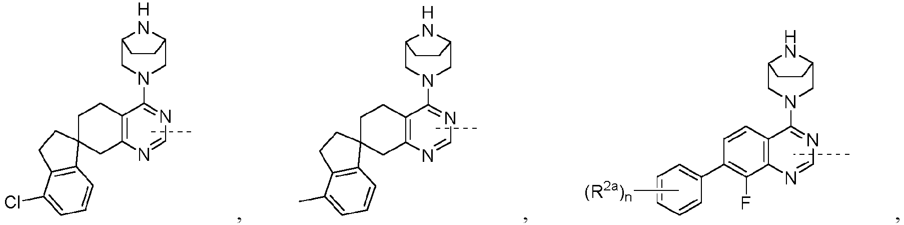 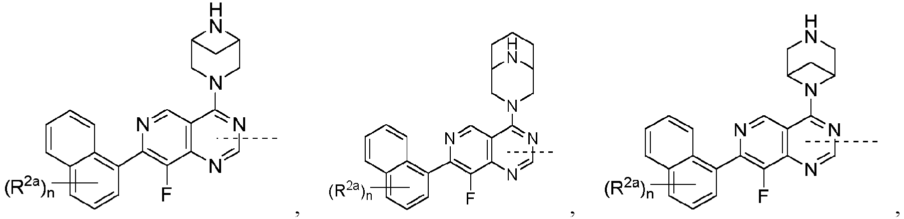 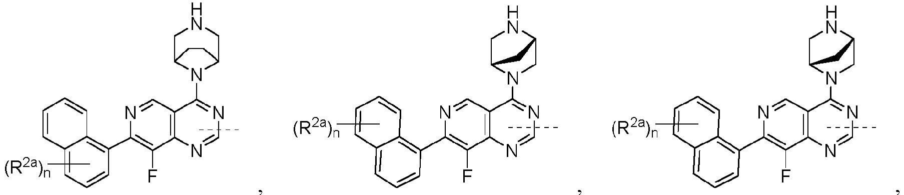 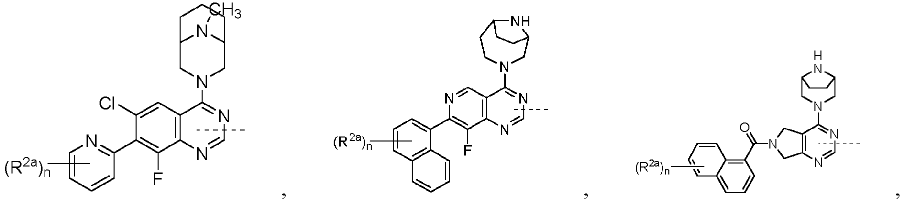 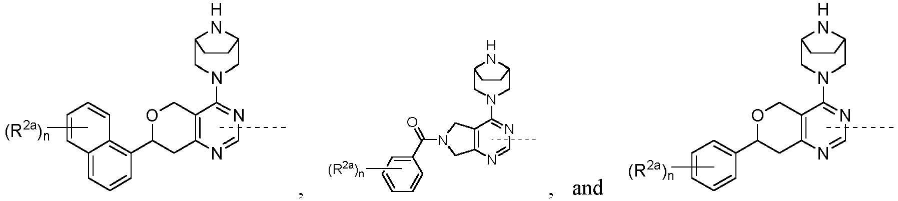 are independently optionally substituted with 1, 2, or 3 R^{a}; the structural unit 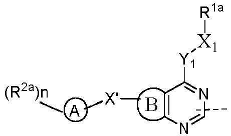 may also be: 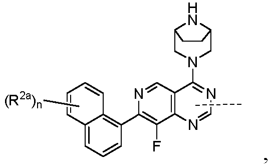 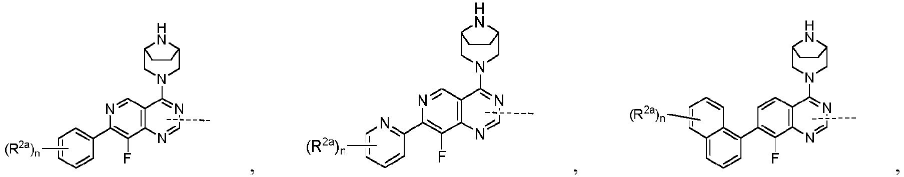 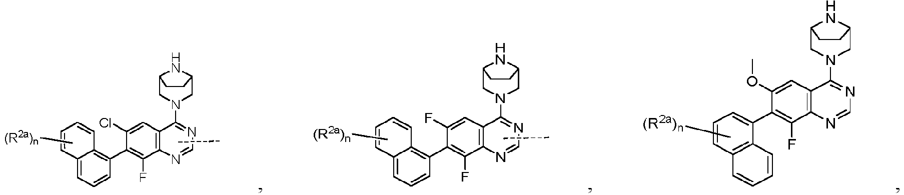 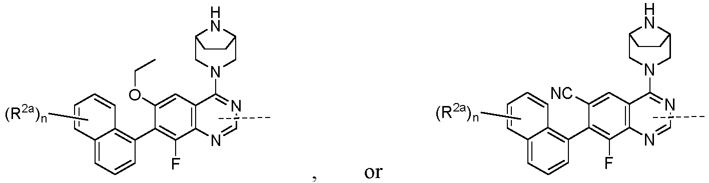 , wherein the 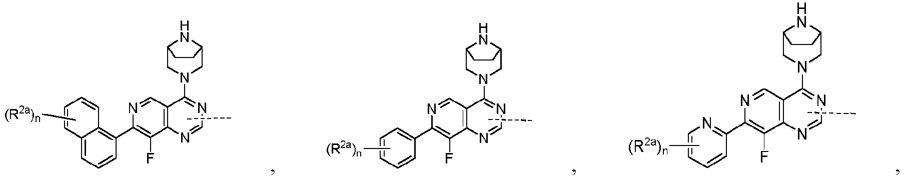 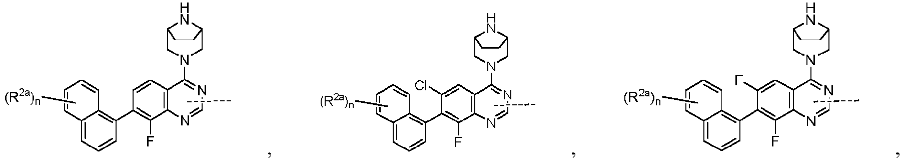 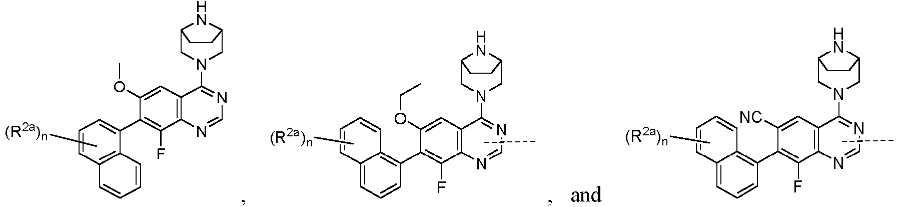 are independently optionally substituted with 1 or 2 R^{a}; preferably, R^{a} is C₁-C₆ alkyl optionally substituted with 1 or more hydroxy, such as -CH₂OH; the structural unit 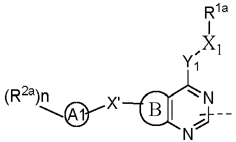 may further be: 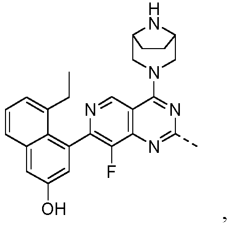 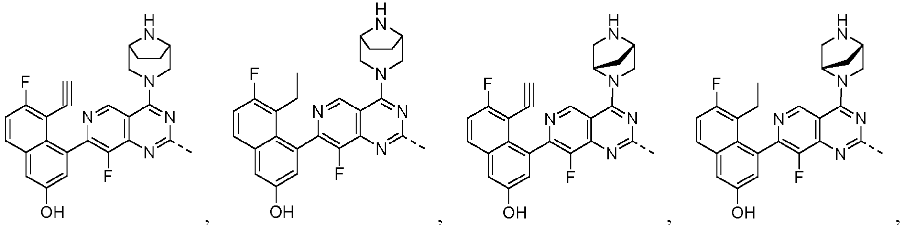 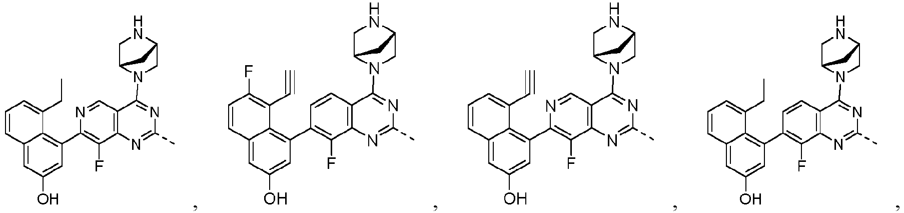 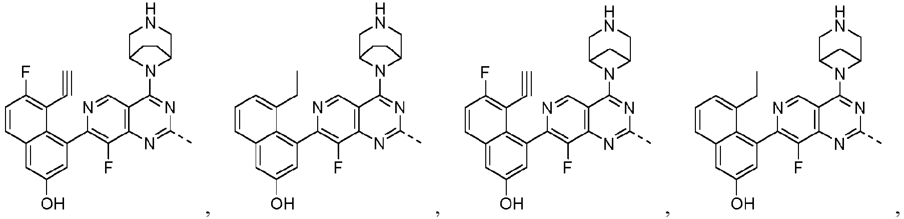 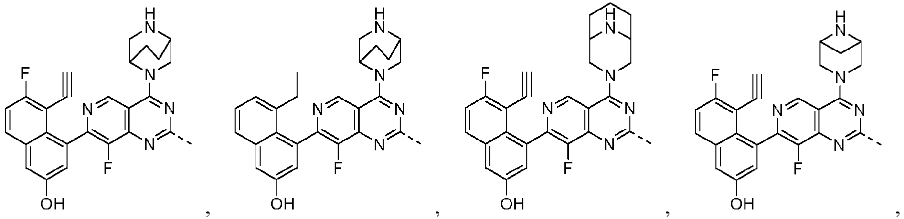 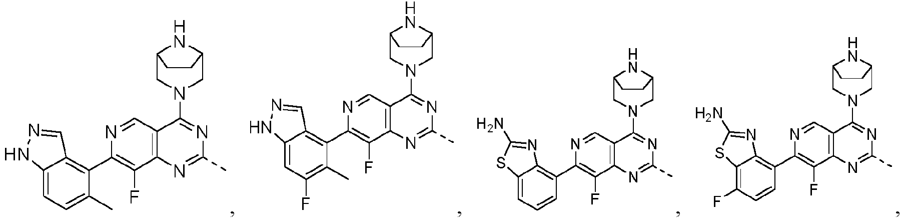 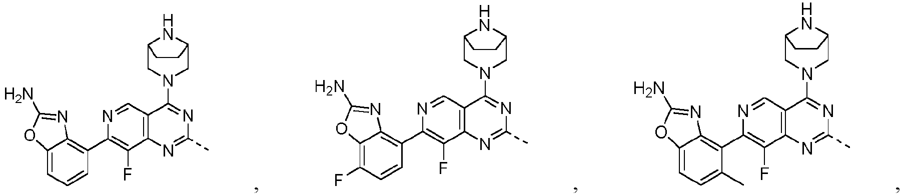 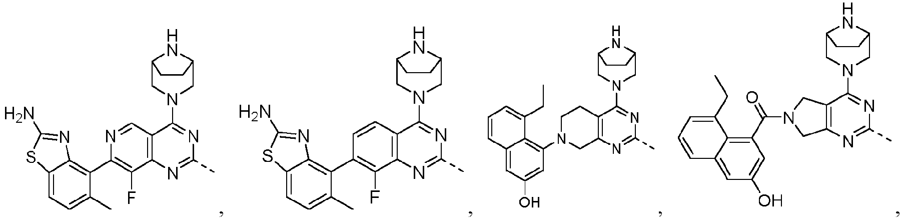 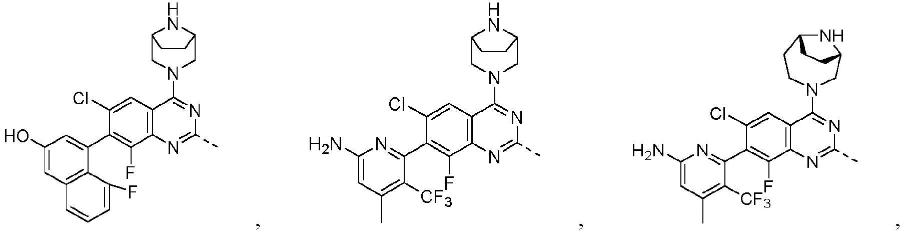 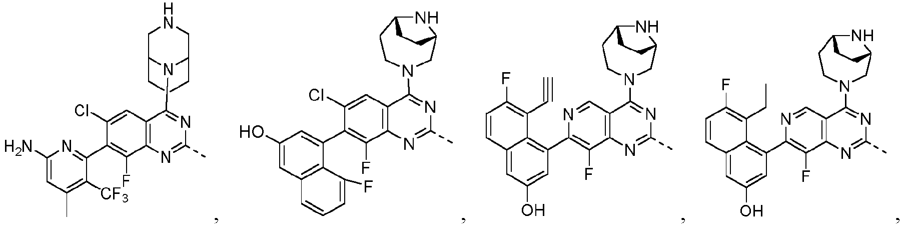 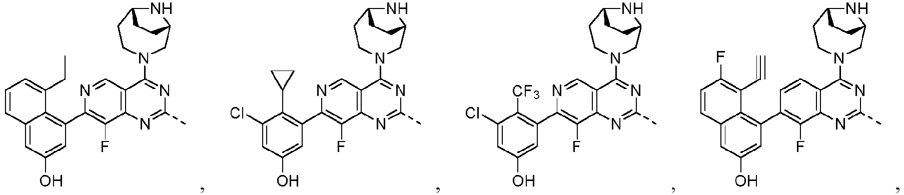 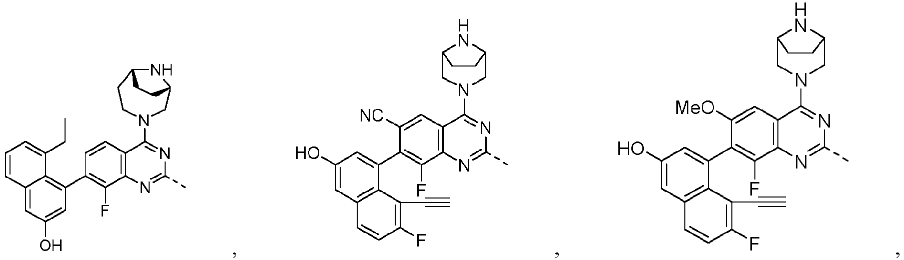 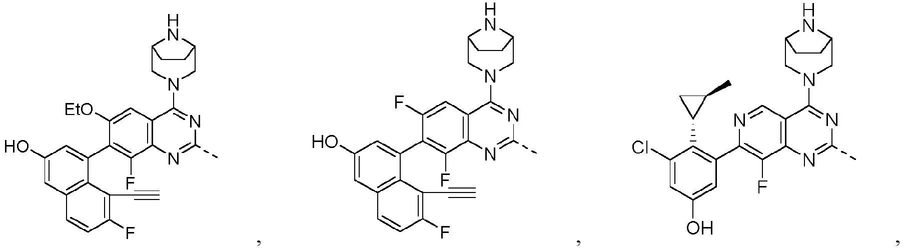 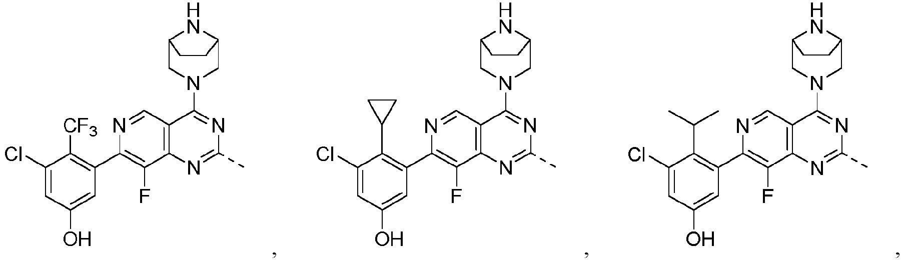 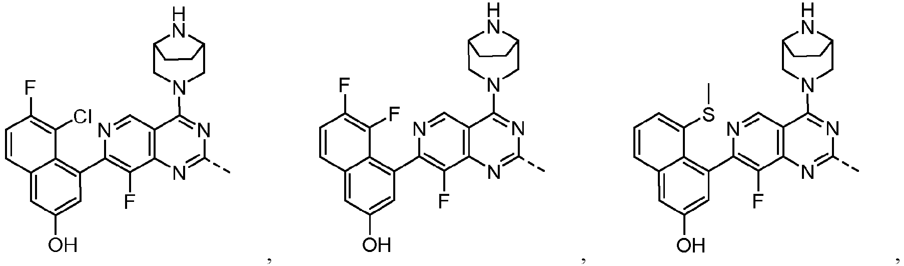 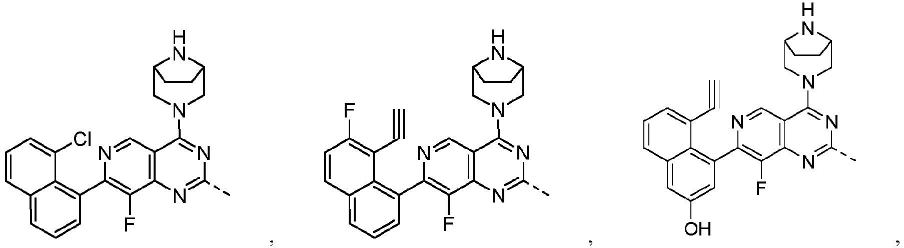 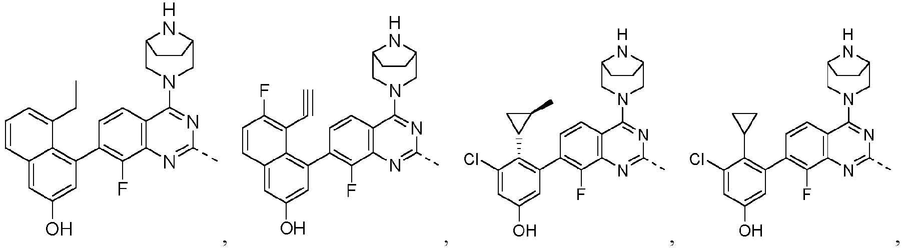 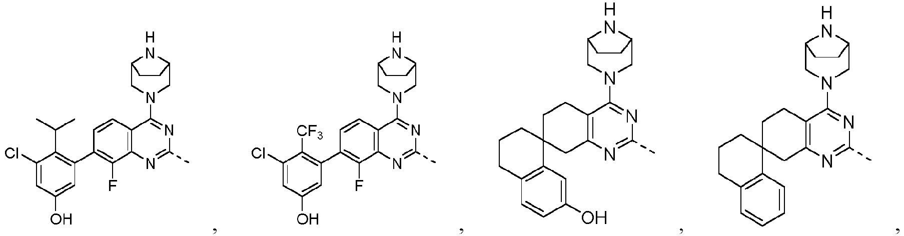 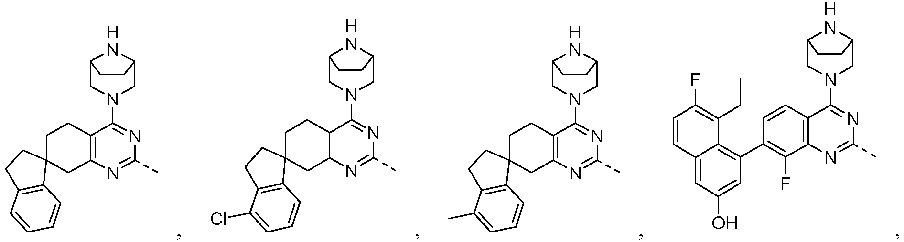 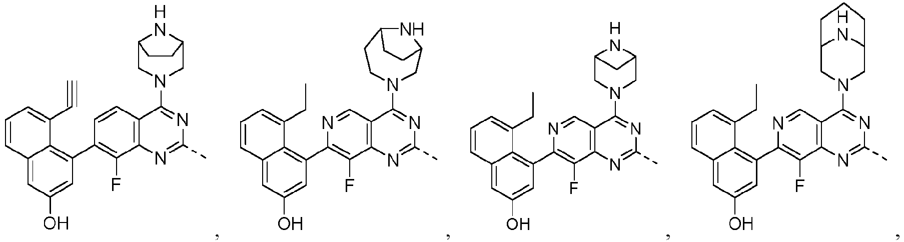 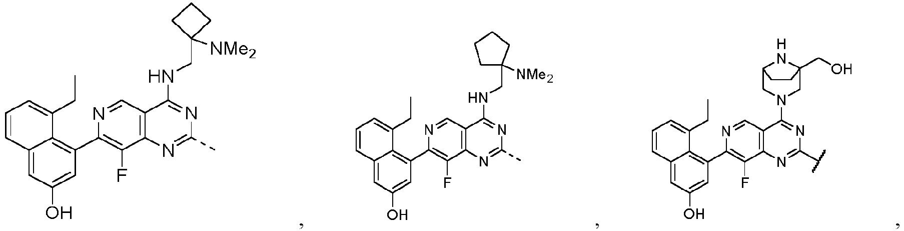 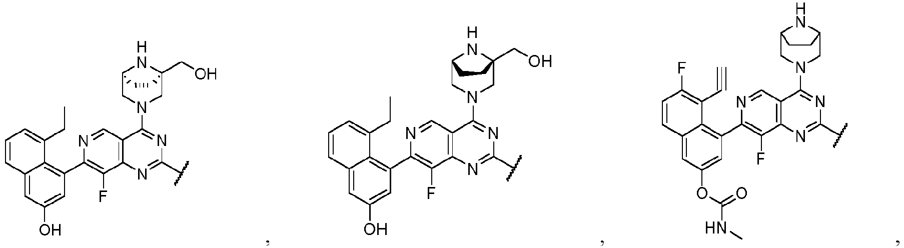 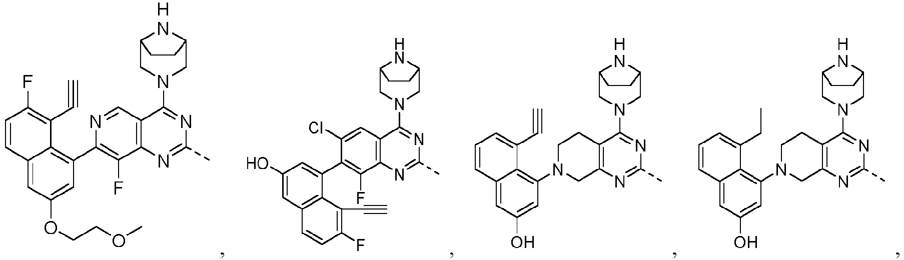 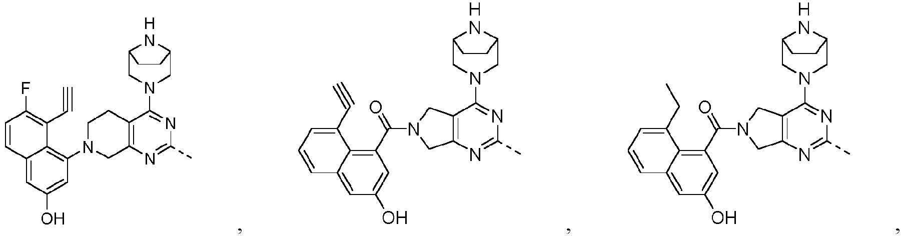 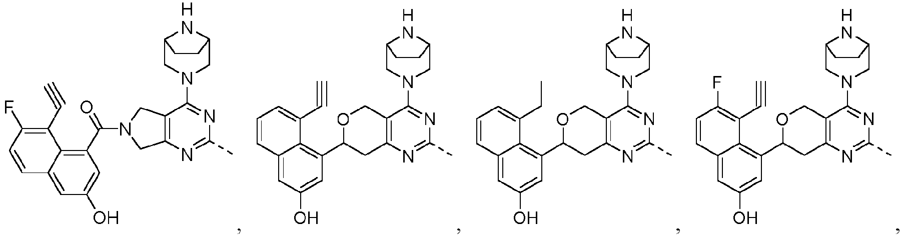 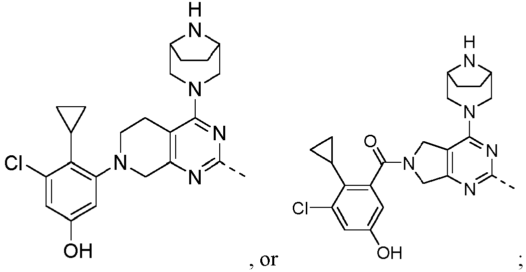 and
(2) the structural unit 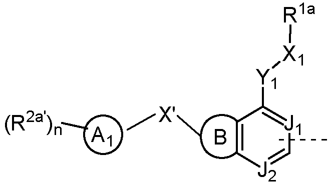 is 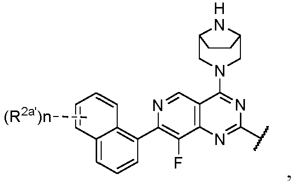 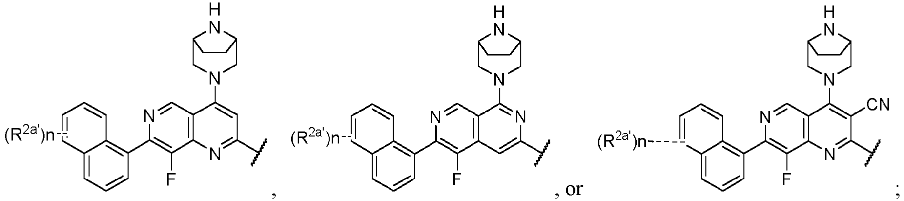 the structural unit may further be

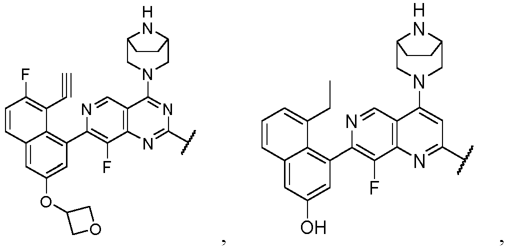

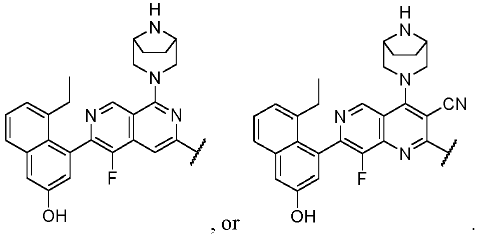

8. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein 1 or more of the following conditions are met:
(1) G1 is G1-I or G1-II: 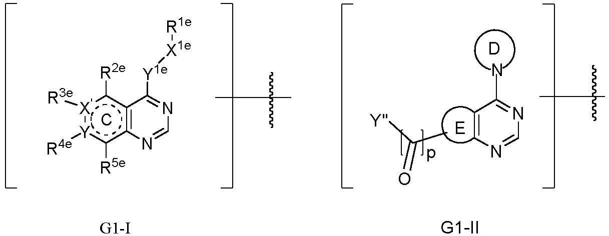 wherein in G1-I:
R^{1e} is hydroxy, -N(R^{a"})₂, C₃-C₁₂ cycloalkyl, or 3- to 12-membered heterocycloalkyl, wherein the C₃-C₁₂ cycloalkyl or 3- to 12-membered heterocycloalkyl is optionally substituted with one or more R^{x};
X^{1e} is a bond or C₁-C₄ alkylene;
Y^{1e} is a bond, O, or NR^{a"};
ring C is C₆-C₁₀ aryl, 5- to 10-membered heteroaryl, or 5- to 10-membered heterocycloalkyl;
X' is a bond, C, or N;
Y' is C or N; 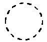
is one or more double bonds that are optionally present;
R^{2e} is H, deuterium, halogen, hydroxy, amino, C₁-C₆ alkyl, C₃-C₈ cycloalkyl, -O(C₁-C₆ alkyl), or -O(C₃-C₈ cycloalkyl);
R^{3e} is a bond, H, deuterium, halogen, cyano, oxo, -O(C₁-C₆ alkyl), -O(C₁-C₆ heteroalkyl), -O(C₃-C₈ cycloalkyl), -O(3- to 8-membered heterocycloalkyl), -S(C₁-C₆ alkyl), -S(C₃-C₈ cycloalkyl), -O-phenyl, or - O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is -L₁-(6- to 10-membered aryl) or -L₁-(5- to 10-membered heteroaryl), wherein the 6- to 10-membered aryl or 5- to 10-membered heteroaryl is optionally substituted with 1 or more R^{a'};
or R^{4e}, together with the carbon atom linked thereto, forms C₈-C₁₀ aryl or 8- to 10-membered heteroaryl, wherein the C₈-C₁₀ aryl or 8- to 10-membered heteroaryl is optionally substituted with halogen, hydroxy, cyano, -O(C₁-C₆ alkyl), -S(C₁-C₆ alkyl), and C₁-C₆ alkyl;
R^{5e} is hydrogen, deuterium, halogen, cyano, C₁-C₆ alkyl, C₃-C₈ cycloalkyl, -O(C₁-C₆ alkyl), or -O(C₃-C₈ cycloalkyl);
L₁ is a bond or C₁-C₄ alkylene, wherein the C₁-C₄ alkylene is optionally substituted with halogen, deuterium, hydroxy, C₁-C₄ hydroxyalkyl, or 5- to 10-membered heteroaryl;
each R^{a"} is independently hydrogen, deuterium, or C₁-C₆ alkyl;
each R^{x} is independently H, deuterium, -OH, halogen, C₁-C₃ alkyl, -N(R^{a"})₁₋₂, -CH₂N(R^{a"})₁₋₂, C₁-C₃ alkoxy,
(C₁-C₃ alkoxy)-C₁-C₃ alkyl, C₁-C₃ alkyl-N(R^{a"})₁₋₂, cyano, C₂-C₄ alkenyl, HC(=O), -CO₂R^{a"}, -CON(R^{a"})₂, 3-to 8-membered heterocycloalkyl, or 5- to 6-membered heteroaryl, wherein the C₁-C₃ alkyl, C₁-C₃ alkoxy, or C₂-C₄ alkenyl is optionally substituted with deuterium, halogen, cyano, hydroxy, nitro, or amino;
R^{a'} is independently halogen, cyano, hydroxy, C₁-C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -N(C₁-C₆ alkyl)₁₋₂, -N(C₁-C₆ heteroalkyl)₁₋₂, - CH₂C(=O)N(R^{5a'})₂, -C₃-C₄ alkynyl(NR^{5a'})₁₋₂, -N(R^{5a'})₁₋₂, (C₁-C₃ alkoxy)C₁-C₃ alkyl-, C₃-C₆ cycloalkyl, or 3-to 6-membered heterocycloalkyl, wherein the C₁-C₆ alkyl, C₁-C₆ heteroalkyl, -S-C₁-C₆ alkyl, C₂-C₆ alkenyl,
C₂-C₆ alkynyl, -O-C₁-C₆ alkyl, -O-C₁-C₆ heteroalkyl, -N(C₁-C₆ alkyl)₁₋₂, -N(C₁-C₆ heteroalkyl)₁₋₂, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-C₁-C₃;
each R^{5a'} is independently H, deuterium, or C₁-C₆ alkyl; 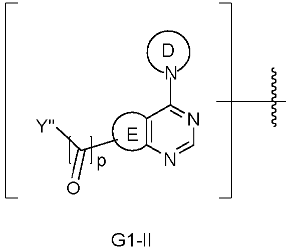
wherein in G1-II:
ring D is 6- to 10-membered heterocycloalkyl, wherein the 6- to 10-membered heterocycloalkyl is optionally substituted with R^{b'}, and the 6- to 10-membered heterocycloalkyl contains at least one heteroatom selected from N, S, and O;
ring E is C₅-C₇ cycloalkyl, 5- to 7-membered heterocycloalkyl, C₆-C₁₀ aryl, 5- to 10-membered heteroaryl, C₅-C₇ cycloalkenyl, or a 8- to 10-membered spiro ring, and a fused ring is formed between ring E and a pyrimidine ring, wherein the C₅-C₇ cycloalkyl, 5- to 7-membered heterocycloalkyl, C₆-C₁₀ aryl, 5- to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
Y" is 6- to 10-membered heterocycloalkyl, 6- to 10-membered aryl, or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
R^{b'} is C₁-C₃ alkyl, hydroxy, -O(C₁-C₃ alkyl), cyano, halogen, -N(R^{x'})₁₋₂, -CH₂N(R^{x'})₁₋₂, cyanomethyl, or 3- to 8-membered heterocycloalkyl;
R^{b"} is halogen, deuterium, cyano, hydroxy, C₁-C₄ alkyl, -S-C₁-C₃ alkyl, C₂-C₄ alkenyl, C₂-C₄ alkynyl, triazolyl, -O-C₁-C₃ alkyl, -CH₂C(=O)N(R^{x'})₁₋₂, -C₃-C₄ alkynyl(NR^{x'})₁₋₂, -N(R^{x'})₁₋₂, or C₁-C₃ alkoxy-C₁-C₃ alkyl-, wherein the C₁-C₄ alkyl, -S-C₁-C₃ alkyl, C₂-C₄ alkenyl, C₂-C₄ alkynyl, or C₁-C₃ alkoxy is optionally substituted with deuterium, halogen, hydroxy, cyano, amino, nitro, C₁-C₃ alkoxy, or C₁-C₃ alkyl;
R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁-C₆ alkyl is optionally substituted with halogen, hydroxy, cyano, or amino;
each R^{x'} is independently H or C₁-C₃ alkyl;
p is 0 or 1; and
(2) G1' is G1'-I or G1'-II: 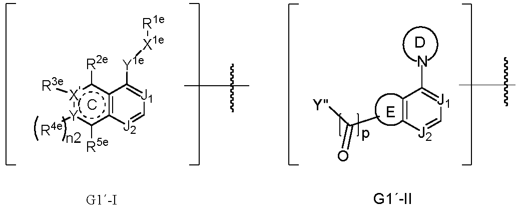 wherein in G1'-I:
n2 is 1 or 2;
J₁ and J₂ are independently selected from CR' and N, and J₁ and J₂ are not both CR';
R' is hydrogen, halogen, deuterium, cyano, amino, hydroxy, or C₁-C₆ alkyl;
R^{1e} is hydroxy, -N(R^{a"})₂, C₃-C₁₂ cycloalkyl, or 3- to 12-membered heterocycloalkyl, wherein the C₃-C₁₂ cycloalkyl or 3- to 12-membered heterocycloalkyl is optionally substituted with one or more R^{x};
X^{1e} is a bond or C₁-C₄ alkylene;
Y^{1e} is a bond, O, or NR^{a"};
ring C is C₆-C₁₀ aryl, 5- to 10-membered heteroaryl, or 5- to 10-membered heterocycloalkyl;
X' is a bond, O, C, or N;
Y' is C or N; 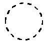
is one or more double bonds that are optionally present;
R^{2e} is H, deuterium, halogen, hydroxy, amino, C₁-C₆ alkyl, C₃-C₈ cycloalkyl, -O(C₁-C₆ alkyl), or -O(C₃-C₈ cycloalkyl);
R^{3e} is H, deuterium, halogen, cyano, oxo, -O(C₁-C₆ alkyl), -O(C₁-C₆ heteroalkyl), -O(C₃-C₈ cycloalkyl), - O(3- to 8-membered heterocycloalkyl), -S(C₁-C₆ alkyl), -S(C₃-C₈ cycloalkyl), -O-phenyl, or -O-pyridyl, wherein the phenyl or pyridyl is optionally substituted with 1 or more hydroxy, halogen, cyano, amino, C₁-C₃ haloalkyl, C₁-C₃ alkyl, or C₁-C₃ alkoxy;
R^{4e} is -L₁-(6- to 10-membered aryl) or -L₁-(5- to 10-membered heteroaryl), wherein the 6- to 10-membered aryl or 5- to 10-membered heteroaryl is optionally substituted with 1 or more R^{a'};
or when n2 is 2, two R^{4e}, together with the carbon atom linked thereto, form C₈-C₁₀ aryl or 8- to 10-membered heteroaryl, wherein the C₈-C₁₀ aryl or 8- to 10-membered heteroaryl is optionally substituted with halogen, hydroxy, cyano, -O(C₁-C₆ alkyl), -S(C₁-C₆ alkyl), or C₁-C₆ alkyl;
R^{5e} is hydrogen, deuterium, halogen, cyano, Cₗ-C₆ alkyl, C₃-C₈ cycloalkyl, -O(C₁-C₆ alkyl), or -O(C₃-C₈ cycloalkyl);
L₁ is a bond or C₁-C₄ alkylene, wherein the C₁-C₄ alkylene is optionally substituted with halogen, deuterium, hydroxy, C₁-C₄ hydroxyalkyl, or 5- to 10-membered heteroaryl;
each R^{a"} is independently hydrogen, deuterium, or C₁-C₆ alkyl;
each R^{x} is independently H, deuterium, -OH, halogen, cyano, C₁-C₆ alkyl, -N(R^{a"})₁₋₂, -CH₂N(R^{a"})₁₋₂, C₁-C₃ alkoxy, (C₁-C₃ alkoxy)-C₁-C₃ alkyl, C₁-C₃ alkyl-N(R^{a"})₁₋₂, cyano, C₂-C₄ alkenyl, HC(=O), -CO₂R^{a"}, - CON(R^{a"})₂, C₃-C₈ cycloalkyl, 3- to 8-membered heterocycloalkyl, or 5- to 6-membered heteroaryl, wherein
the C₁-C₆ alkyl, C₃-C₈ cycloalkyl, C₁-C₃ alkoxy, or C₂-C₄ alkenyl is optionally substituted with deuterium, halogen, cyano, hydroxy, nitro, or amino;
R^{a'} is independently halogen, deuterium, cyano, hydroxy, C₁-C₆ alkyl, C₁-C₆ heteroalkyl, -S-C₁-C₆ alkyl, C₂-C₆ alkenyl, C₂-C₆ alkynyl, triazolyl, -O-C₁-C₆ alkyl, -O-C₁-C₆ heteroalkyl, -NH(C₁-C₆ heteroalkyl), -N(C₁-C₆ heteroalkyl)₂, -CH₂C(=O)N(R^{5a'})₂, -C₃-C₄ alkynyl(NR^{5a'})₂, -N(R^{5a'})₂ (e.g., -N(C₁-C₆ alkyl)₂, -NH(C₁-C₆ heteroalkyl)), (C₁-C₃ alkoxy)C₁-C₃ alkyl-, C₃-C₆ cycloalkyl, or 3- to 6-membered heterocycloalkyl, wherein the C₁-C₆ alkyl, C₁-C₆ heteroalkyl, -S-C₁-C₆ alkyl, C₂-C₆ alkenyl, C₂-C₆ alkynyl, -O-C₁-C₆ alkyl, -O-C₁-C₆ heteroalkyl, -NH(C₁-C₆ alkyl), -N(C₁-C₆ alkyl)₂, -NH(C₁-C₆ heteroalkyl), -N(C₁-C₆ heteroalkyl)₂, -, C₃-C₆ cycloalkyl, or 3- to 6-membered heterocycloalkyl is optionally substituted with deuterium, halogen, cyano, hydroxy, nitro, amino, C₁-C₃ alkyl, -O-C₁-C₃ alkyl, or -Si-C₁-C₃ alkyl;
each R^{5a'} is independently H, deuterium, or C₁-C₆ alkyl; 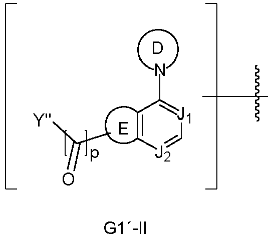
wherein in G1'-II:
J₁ and J₂ are independently CR' or N, and J₁ and J₂ are not both CR';
R' is hydrogen, halogen, deuterium, cyano, amino, hydroxy, or C₁-C₆ alkyl;
ring D is 6- to 10-membered heterocycloalkyl, wherein the 6- to 10-membered heterocycloalkyl is optionally substituted with R^{b'}, and the 6- to 10-membered heterocycloalkyl contains at least one heteroatom selected from N, S, and O;
ring E is C₅-C₁₅ cycloalkyl, 5- to 7-membered heterocycloalkyl, C₆-C₁₀ aryl, 5- to 10-membered heteroaryl, C₅-C₇ cycloalkenyl 8- to 10-membered spiro ring, wherein the C₅-C₇ cycloalkyl, 5- to 7-membered heterocycloalkyl, C₆-C₁₀ aryl, 5- to 10-membered heteroaryl, C₅-C₇ cycloalkenyl, or 8- to 10-membered spiro ring is optionally substituted with R^{b"}, and the heterocycloalkyl contains at least one heteroatom selected from N, S, and O;
Y" is 6- to 10-membered heterocycloalkyl, 6- to 10-membered aryl, or 5- to 10-membered heteroaryl, wherein the 6- to 10-membered heterocycloalkyl or 5- to 10-membered heteroaryl contains at least one heteroatom selected from N, S, and O, and is optionally substituted with R^{b‴};
R^{b'} is hydrogen, C₁-C₆ alkyl, hydroxy, -O(C₁-C₃ alkyl), cyano, halogen, -N(R^{x'})₂, -CH₂N(R^{x'})₂, -CO₂R^{x'}, - CO₂N(R^{x'})₂ cyanomethyl 5- to 6-membered heteroaryl, or 3- to 8-membered heterocycloalkyl, wherein the C₁-C₆ alkyl or C₃-C₈ cycloalkyl is optionally substituted with halogen, hydroxy, cyano, or amino;
R^{b"} is halogen, deuterium, cyano, hydroxy, C₁-C₄ alkyl, -S-C₁-C₃ alkyl, C₂-C₄ alkenyl, C₂-C₄ alkynyl, triazolyl, -O-C₁-C₃ alkyl, -CH₂C(=O)N(R^{x'})₋₂, -C₃-C₄ alkynyl(NR^{x'})₂, -N(R^{x'})₂, or C₁-C₃ alkoxy-C₁-C₃ alkyl-, wherein the C₁-C₄ alkyl, -S-C₁-C₃ alkyl, C₂-C₄ alkenyl, C₂-C₄ alkynyl, or C₁-C₃ alkoxy is optionally substituted with deuterium, halogen, hydroxy, cyano, amino, nitro, C₁-C₃ alkoxy, or C₁-C₃ alkyl;
R^{b‴} is hydrogen, hydroxy, halogen, cyano, C₁-C₆ alkyl, C₂-C₄ alkynyl, C₂-C₄ alkenyl, O(C₁-C₆ alkyl), HC(=O)-, -CO₂R^{x'}, -CO₂N(R^{x'})₂, or 5- to 6-membered heteroaryl, wherein the C₁-C₆ alkyl is optionally substituted with halogen, hydroxy, cyano, or amino;
each R^{x'} is independently H or C₁-C₆ alkyl;
p is 0 or 1.

9. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 8, wherein
G1 is G1-I-a, G1-I-b, or G1-II-a: 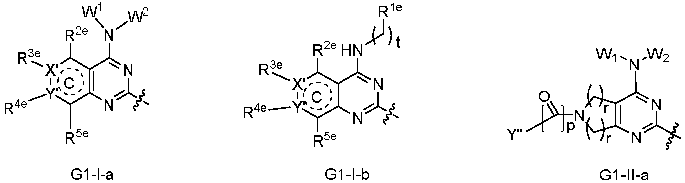
wherein:
r is 1 or 2; t is 1, 2, or 3;
W¹ and W², together with the N atom linked thereto, form 6- to 10-membered heterocycloalkyl, wherein the 6- to 10-membered heterocycloalkyl contains at least one heteroatom selected from N, S, and O;
ring C, R^{1e}, R^{2e}, R^{3e}, R^{4e}, R^{5e}, X', Y', Y", and p in G1-I-a, G1-I-b, or G1-II-a are as defined in claim 8; preferably, G1 is G1-I-a1, G1-I-a1', G1-I-a1", G1-I-a1‴, G1-I-a2, G1-I-a2', G1-I-b1, G1-I-b2, or G1-II-a1: 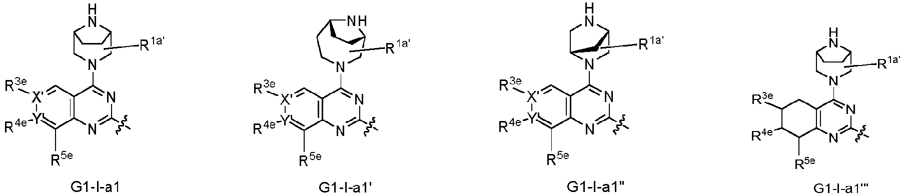 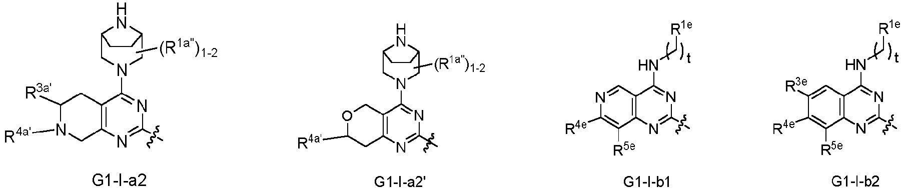 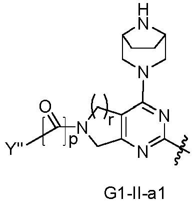
wherein in G1-I-a1, G1-I-a1', G1-I-a1", and G1-I-a1‴:
R^{1a'} is hydrogen, hydroxy, halogen, cyano, C₁-C₆ alkyl, C₃-C₈ cycloalkyl, HC(=O)-, -CO₂R^{5a'}, -CO₂N(R^{5a'})₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl; R^{3e}, R^{4e}, R^{5e}*,* X', and Y' as defined in G1-I-a are as described in claim 8; 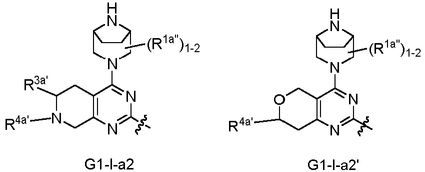
wherein in G1-I-a2 and G1-I-a2':
each R^{1a"} is independently hydrogen, hydroxy, halogen, C₁-C₃ alkyl, C₁-C₃ alkoxy, (C₁-C₃ alkoxy)-C₁-C₃ alkyl-, C₁-C₃ alkyl-N(R^{5a'})₁₋₂, cyano, C₂-C₄ alkenyl, HC(=O)-, -CO₂R^{5a'}, or -CO₂N(R^{5a'})₂, wherein the C₁-C₃ alkyl or C₂-C₄ alkenyl is optionally substituted with halogen, hydroxy, cyano, or amino;
each R^{4a'} is -L₂-(6- to 10-membered aryl), 6- to 10-membered aryl, -L₂-(5- to 10-membered heteroaryl), or 5- to 10-membered heteroaryl, wherein the 6- to 10-membered aryl or 5- to 10-membered heteroaryl may be optionally substituted with one or more R^{8a'};
each L₂ is C₁-C₄ alkylene, wherein the C₁-C₄ alkylene is optionally substituted with hydroxy, halogen, cyano, amino, C₁-C₄ hydroxyalkyl, or 5- to 10-membered heteroaryl;
each R^{5a'} is independently H or C₁-C₃ alkyl;
each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, N(R^{5a'})₁₋₂, or -O-C₁-C₆ alkyl, wherein the C₁-C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
R^{3a'} is hydrogen or oxo; 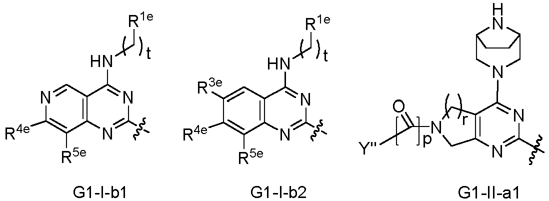
in G1-I-b1, G1-I-b2, and G1-II-a1:
R^{1e}, R^{3e}, R^{4e}, R^{5e}*,* t, r, Y", and p are as defined in claim 8;
G1' is G1'-I-a, G1'-I-b, or G1'-II-a: 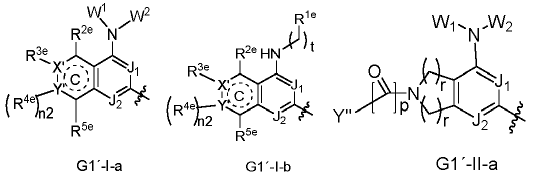
wherein:
r is 1 or 2;
t is 1, 2, or 3;
W¹ and W², together with the N atom linked thereto, form 6- to 10-membered heterocycloalkyl, wherein the 6- to 10-membered heterocycloalkyl contains at least one heteroatom selected from N, S, and O;
ring C, J₁, J₂, R^{1e}, R^{2e}, R^{3e}, R^{4e}, R^{5e}*,* X', Y', Y", and p in G1'-I-a, G1'-I-b, or G1'-II-a are as defined in claim 8;
preferably, G1' is G1'-I-a1, G1'-I-a1', G1'-I-a1", G1'-I-a1‴, G1'-I-a2, G1'-I-b1, G1'-I-b2, or G1'-II-a1: 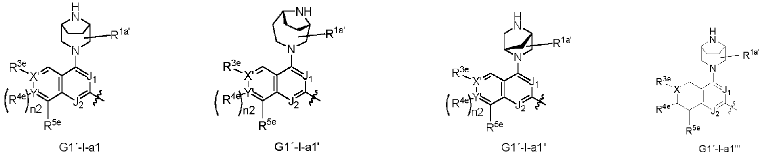 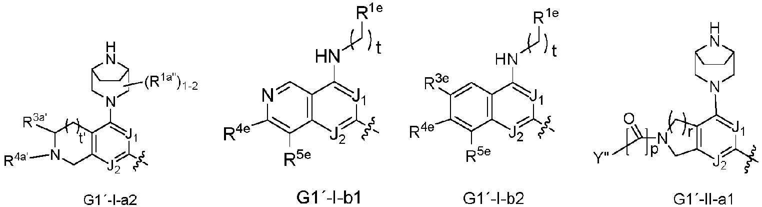
wherein in G1'-I-a1, G1'-I-a1', G1'-I-a1", and G1'-I-a1‴:
R^{1a'} is hydrogen, hydroxy, halogen, cyano, C₁-C₆ alkyl, C₃-C₈ cycloalkyl, HC(=O)-, -CO₂R^{5a'}, -CO₂N(R^{5a'})₂, or 5- to 6-membered heteroaryl, wherein the C₁-C₆ alkyl or C₃-C₈ cycloalkyl is optionally substituted with halogen, hydroxy, cyano, or amino;
each R^{5a'} is independently hydrogen or C₁-C₆ alkyl;
J₁, J₂, R^{3e}, R^{4e}, R^{5e}*,* X', and Y' are as defined in claim 8; 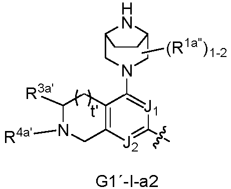
wherein in G1'-I-a2:
J₁ and J₂ are independently CR' or N, and J₁ and J₂ are not both CR';
R' is hydrogen, halogen, deuterium, cyano, amino, hydroxy, or C₁-C₆ alkyl;
t' is 0 or 1;
each R^{1a"} is independently hydrogen, hydroxy, halogen, C₁-C₃ alkyl, C₁-C₃ alkoxy, -(C₁-C₃ alkoxy)-C₁-C₃ alkyl-, -C₁-C₃ alkyl-N(R^{5a'})₋₂, cyano, C₂-C₄ alkenyl, HC(=O)-, -CO₂R^{5a'}, or -CO₂N(R^{5a'})₂, wherein the C₁-C₃ alkyl or C₂-C₄ alkenyl is optionally substituted with halogen, hydroxy, cyano, or amino;
R^{4a'} is -L₂-(6- to 10-membered aryl), 6- to 10-membered aryl, -L₂-(5- to 10-membered heteroaryl), or 5- to 10-membered heteroaryl, wherein the 6- to 10-membered aryl or 5- to 10-membered heteroaryl may be optionally substituted with one or more R^{8a'};
L₂ is C₁-C₄ alkylene, wherein the C₁-C₄ alkylene is optionally substituted with hydroxy, halogen, cyano, amino, C₁-C₄ hydroxyalkyl, or 5- to 10-membered heteroaryl;
R^{5a'} is independently H or C₁-C₃ alkyl;
each R^{8a'} is independently halogen, deuterium, cyano, hydroxy, amino, C₁-C₆ alkyl, C₁-C₆ heteroalkyl, C₃-C₆ cycloalkyl, -S-C₁-C₃ alkyl, C₂-C₄ alkenyl, C₂-C₄ alkynyl, triazolyl, -O-C₁-C₆ alkyl, N(R^{5a'})₁₋₂, or -O-C₁-C₆ alkyl, wherein the C₁-C₆ alkyl, C₁-C₆ heteroalkyl, C₃-C₆ cycloalkyl, -S-C₁-C₃ alkyl, C₂-C₄ alkenyl, C₂-C₄ alkynyl, or -O-C₁-C₆ alkyl is optionally substituted with halogen, hydroxy, cyano, or amino;
R^{3a'} is hydrogen or oxo; 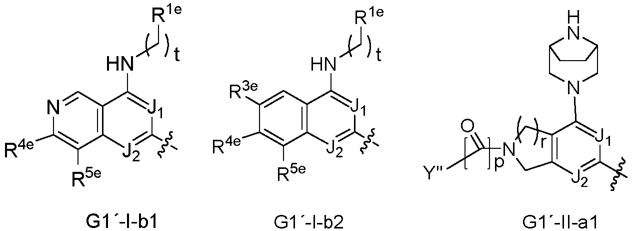
in G1'-I-b1, G1'-I-b2, and G1'-II-a1:
J₁, J₂, R^{1e}, R^{3e}, R^{4e}, R^{5e}*,* t, r, Y", and p are as defined in claim 8.

10. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, wherein
L is -(CH₂)ⱼ- or LA 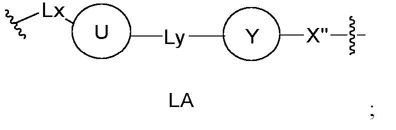
1 or more methylene groups in the -(CH₂)ⱼ- are optionally replaced by a group selected from -NR^{3'}-, -O-, - CR^{1'}R^{2'}-, -C(O)-, -S(O)-, -S(O)₂-, -C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8- to 10-membered bicyclic arylene, saturated or partially unsaturated 3- to 7-membered cycloalkylene, saturated or partially unsaturated 5- to 11-membered spirocycloalkylene, saturated or partially unsaturated 5- to 11-membered fused cycloalkylene, saturated or partially unsaturated 8- to 10-membered bicyclic cycloalkylene, saturated or partially unsaturated 4- to 7-membered heterocycloalkylene having 1-2 heteroatoms independently selected from nitrogen, oxygen, and sulfur, saturated or partially unsaturated 5- to 11-membered spiroheterocycloalkylene having 1-2 heteroatoms independently selected from nitrogen, oxygen, and sulfur, saturated or partially unsaturated 5- to 11-membered fused heterocycloalkylene having 1-2 heteroatoms independently selected from nitrogen, oxygen, and sulfur, saturated or partially unsaturated 8- to 10-membered bicyclic heterocycloalkylene having 1-2 heteroatoms independently selected from nitrogen, oxygen, and sulfur, 5- to 6-membered heteroarylene having 1-4 heteroatoms independently selected from nitrogen, oxygen, and sulfur, and 8- to 10-membered bicyclic heteroaryl having 1-5 heteroatoms selected from nitrogen, oxygen, and sulfur, wherein the ethenylene, ethynylene, cycloalkylene, heterocycloalkylene, phenyl, spiroheterocycloalkylene, fused heterocycloalkylene, spirocycloalkylene, fused cycloalkylene, and heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl, and C₃-C₁₀ heterocycloalkyl, the alkyl, cycloalkyl, or heterocycloalkyl being optionally substituted with 1 or more substituents selected from halogen, -OH, -NH₂, -CN, C₁-C₄ alkyl, and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl), or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl; j is 2, 3, 4, 5, 6, 7, 8, 9, 10, 11, or 12;
LA is as defined in case I or case II:
case I:
ring U is C₃-C₁₂ cycloalkylene or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
ring Y is a bond, C₃-C₁₂ cycloalkylene, or 3- to 12-membered heterocycloalkylene containing 1-2 heteroatoms selected from N, O, and S, wherein the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl, and -O-(C₁-C₆ alkyl);
X" is a bond, -C(O)-, -NH-, -NCH₃-, -O-, -C(CH₃)₂-, -S-, -C=C-, -C≡C-, -CHF-, -CHCF₃-, -(CH₂)_{q}C(O)-, - S(O)-, -S(O)₂-, -C(O)O-, -OC(O)-, -(CH₂)_{q}C(O)NH-, -C(O)NCH₃-, -NHC(O)-, -NCH₃C(O)-, or - C(O)CH₂O-;
q is 1 or 2;
Lx is -(CH₂)ᵥ-, wherein one or two methylene groups in Lx are optionally replaced by a group selected from -O-, -S-, -NH-, -C≡C-, -N(C₁-C₆ alkyl)-, -N(C₁-C₆ haloalkyl)-, -C(O)-, -N(C₁-C₆ hydroxyalkyl)-, -N(C₃-C₈ cycloalkyl)-, and -CR_{d}Rₑ-; v is 1, 2, 3, 4, 5, 6, or 7;
R_{d} and Rₑ are each independently H, -OH, C₁-C₆ alkyl, or C₁-C₆ alkoxy;
or R_{d} and Rₑ, together with the C atom linked thereto, form C₃-C₈ cycloalkyl or 3- to 8-membered heterocycloalkyl;
Ly is -(CH₂)ₖ-, wherein one or two methylene groups in Ly are optionally replaced by a group selected from -O-, -NH-, -C≡C-, -N(C₁-C₆ alkyl)-, -N(C₁-C₆ haloalkyl)-, -C(O)-, -N(C₁-C₆ hydroxyalkyl)-, and -N(C₃-C₈ cycloalkyl)-; k is 1, 2, 3, 4, 5, 6, 7, or 8;
case II:
ring U is 3- to 12-membered heterocycloalkylene containing 1-2 heteroatoms selected from N, O, and S, wherein the 3- to 12-membered heterocycloalkylene is optionally substituted with the following substituent: C₁-C₆ alkyl substituted with 1, 2, or 3 hydroxy;
ring Y is 3- to 12-membered heterocycloalkylene containing 1-2 heteroatoms selected from N, O, and S; X" is -C(O)-;
Lx is -(CH₂)ᵥ-, wherein 1 or two methylene groups in Lx are optionally replaced by a group selected from - O- and -CR_{d}Rₑ-; v is 1, 2, 3, 4, 5, 6, or 7; R_{d} and Rₑ are each independently H, NH₂, or C₁-C₆ alkyl substituted with 1, 2, or 3 halogen, or R_{d} and Rₑ, together with the C atom linked thereto, form C₃-C₈ cycloalkyl;
Ly is -(CH₂)ₖ-, wherein 1 or more hydrogens on the methylene group in Ly are optionally substituted with deuterium; each k is independently 1, 2, 3, 4, 5, 6, 7, or 8.

11. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 10, wherein 1 or more of the following conditions are met:
in L, the methylene group in -(CH₂)ⱼ- is substituted with a group selected from -O-, C(O)-, ethenylene, saturated or partially unsaturated 4- to 7-membered heterocycloalkylene having 1-2 heteroatoms independently selected from nitrogen, oxygen, and sulfur, and saturated or partially unsaturated 5- to 11-membered spiroheterocycloalkylene having 1-2 heteroatoms independently selected from nitrogen, oxygen, and sulfur; j is 8;
preferably, -(CH₂)ⱼ- is -O-CH₂-ethenylene-CH₂-(saturated or partially unsaturated 4- to 7-membered heterocycloalkylene having 1-2 heteroatoms independently selected from nitrogen, oxygen, and sulfur)-CH₂-(saturated or partially unsaturated 5- to 11-membered spiroheterocycloalkylene having 1-2 heteroatoms independently selected from nitrogen, oxygen, and sulfur)-C(O)-;
LA is scheme 1 or 2 below;
scheme 1:
ring U in LA is 4- to 8-membered saturated monocyclic heterocycloalkylene, 6- to 10-membered fused heterocycloalkylene, 6- to 10-membered bridged heterocycloalkylene, or C₆-C₁₀ bridged cycloalkylene, containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; Lx is -(CH₂)ᵥ-, and v is 1, 2, 3, 4, or 5; Ly is -(CH₂)ₖ-, and k is 1, 2, 3, 4, or 5; or in LA, ring Y may also be replaced by 7-, 8-, 9-, 10-, or 11-membered spiroheterocycloalkylene containing 1 or 2 heteroatoms of N and/or O;
preferably, ring U in LA is 4- to 8-membered saturated monocyclic heterocycloalkylene or 6- to 10-membered fused heterocycloalkylene, containing 1 or 2 nitrogen heteroatoms; ring Y is 6- to 8-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; Lx is -(CH₂)ᵥ-, and v is 1, 2, 3, 4, or 5; Ly is -(CH₂)ₖ-, and k is 1, 2, 3, 4, or 5; or in LA, ring Y may also be replaced by 7-, 8-, 9-, 10-, or 11-membered spiroheterocycloalkylene containing 1 or 2 heteroatoms of N and/or O;
scheme 2:
LA is LA-1 or LA-2: 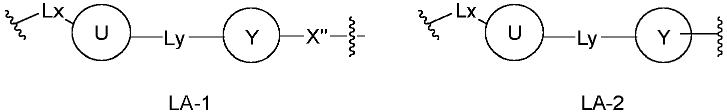
wherein X" is -C(O)- or -C(O)NH-;
in LA-1 and LA-2, ring U, ring Y, Lx, Ly, and q are as defined in claim 10; preferably, LA is LA-3: 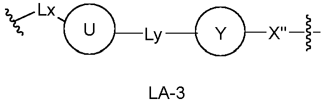
wherein LA-3 is as defined in any one of cases 1-5 below;
case 1:
Lx is -(CH₂)ᵥ-, wherein one or two CH₂ contained in Lx are each independently optionally replaced by -O-, -S-, -NH-, -NMe-, or -CR_{d}Rₑ-; -CR_{d}Rₑ- is 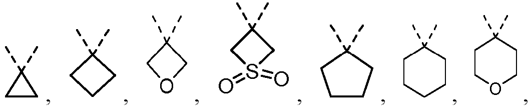 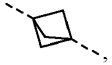 -C(CH₃)₂-, -CH(CH₃)-, -CH(OH)-, -CH(OCH₃)-, C(CH₃)(OH)-, or -C=CH₂; v is 1, 2, 3, 4, 5, or 6;
ring U is 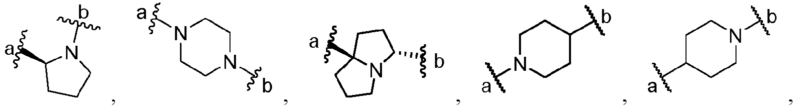 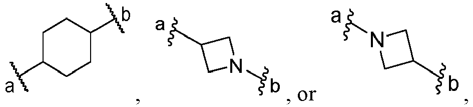 wherein end a is linked to Lx, and end b is linked to Ly; 1, 2, 3, or 4 hydrogen atoms in ring U are optionally substituted with F, -OH, -OCH₃, or -NH₂;
ring Y is a bond, 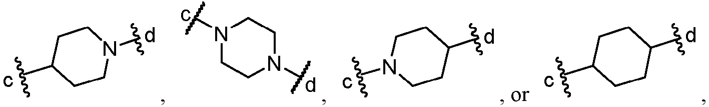 wherein end c is linked to Ly, and end d is linked to X"; 1, 2, 3, or 4 hydrogen atoms in ring Y are optionally substituted with F, -OH, or -OCH₃;
Ly is -(CH₂)ₖ-, wherein one or two CH₂ contained in Ly are each independently optionally replaced by -O-, -C≡C-, -C(O)-, or -N(C₁-C₆ alkyl)-; k is 1, 2, 3, 4, 5, or 6;
X" is a bond, -C(O)-, -(CH₂)₁₋₂C(O)-, or -(CH₂)₁₋₂C(O)NH-;
case 2:
Lx is -(CH₂)ᵥ-, wherein one or two CH₂ contained in Lx are each independently optionally replaced by -O-, -S-, -NH-, or -CR_{d}Rₑ-; -CR_{d}Rₑ- is 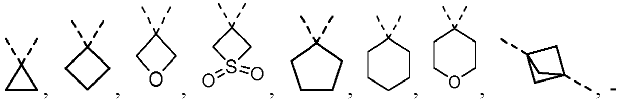 C(CH₃)₂-, -CH(CH₃)-, -CH(CH₃)-, -CH(OCH₃)-, -CH(OH)-, -C(CH₃)(OH)-, or -C=CH₂; v is 1, 2, 3, 4, 5, or 6;
ring U is 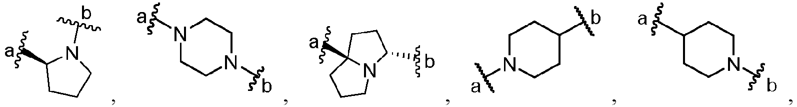 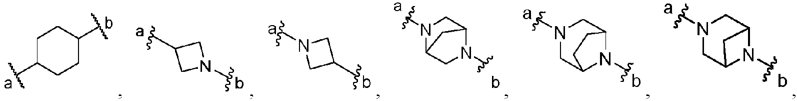 or 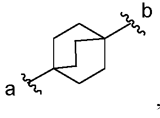 wherein end a is linked to Lx, and end b is linked to Ly; 1, 2, 3, or 4 hydrogen atoms in ring U are optionally substituted with F;
ring Y is 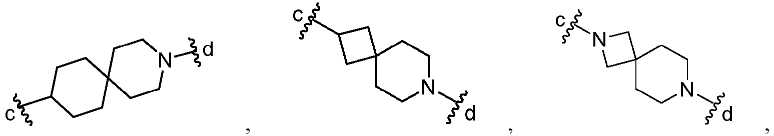 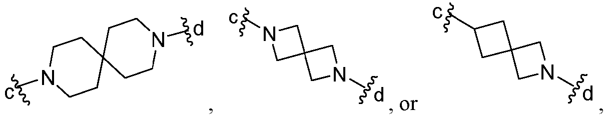 wherein end c is linked to Ly, and end d is linked to X"; 1, 2, 3, or 4 hydrogen atoms in ring Y are optionally substituted with F;
Ly is -(CH₂)ₖ-, wherein one or two CH₂ contained in Ly are each independently optionally replaced by -O- or -N(C₁-C₆ alkyl)-; k is 1, 2, 3, 4, 5, or 6;
X" is -C(O)-;
or ring Y in case 2 is also replaced by 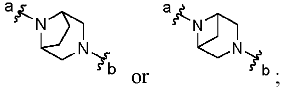
case 3:
Lx is -(CH₂)ᵥ-, wherein one or two CH₂ contained in Lx are each independently optionally replaced by -O-, -S-, -NH-, or -CR_{d}Rₑ-; -CR_{d}Rₑ- is 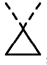 , -CH(NH₂)-, -CH(CH₂CHF₂)-, -CH(CH₂CH₂CF₃)-, or -CH(CH₃)₂-;
v is 1, 2, 3, 4, 5, or 6;
ring U is 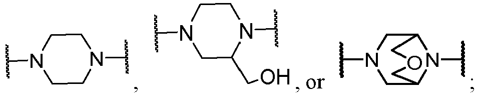
ring Y is 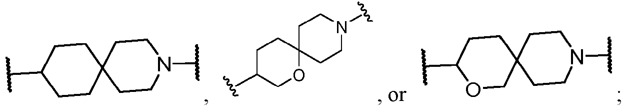
Ly is -(CH₂)ₖ-, wherein 1 or more hydrogens on the methylene group in Ly are optionally substituted with deuterium; k is 1, 2, 3, 4, 5, or 6;
X" is -C(O)-;
case 4:
Lx is -(CH₂)ᵥ-, wherein one or two CH₂ contained in Lx are each independently optionally replaced by -O-;
v is 1, 2, 3, or 4;
ring U is 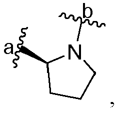 wherein end a is linked to Lx, and end b is linked to Ly; 1, 2, 3, or 4 hydrogen atoms in ring U are optionally substituted with F;
ring Y is a bond;
Ly is -(CH₂)ₖ-, wherein one or two CH₂ contained in Ly are each independently optionally replaced by -O-, -C(O)-, or -NH-; k is 1, 2, 3, 4, 5, 6, 7, or 8;
X" is -C(O)NH-;
case 5:
Lx is -(CH₂)ᵥ-, wherein one or two CH₂ contained in Lx are each independently optionally replaced by -O-
or -CR_{d}Rₑ-; -CR_{d}Rₑ- is 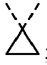 ; v is 1, 2, 3, or 4;
ring U is 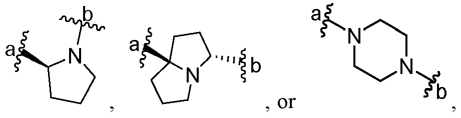 wherein end a is linked to Lx, and end b is linked to Ly;
ring Y is 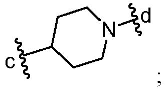
Ly is -(CH₂)ₖ-, wherein one or two CH₂ contained in Ly are each independently optionally replaced by -O-, -C(O)-, or -NH-; k is 1, 2, 3, 4, 5, or 6;
X" is a bond.

12. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 10, wherein 1 or more of the following conditions are met:
(1) in ring U of LA, the C₃-C₁₂ cycloalkylene is C₃-C₆ monocyclic saturated cycloalkylene or C₆-C₁₀ bridged cycloalkylene, such as cyclohexylene or bicyclo[2.2.2]octanyl;
(2) in ring U of LA, the 3- to 12-membered heterocycloalkylene is 3- to 8-membered saturated monocyclic heterocycloalkylene, 6- to 10-membered fused heterocycloalkylene, or 6- to 10-membered bridged heterocycloalkylene, containing 1 or 2 heteroatoms of N; or the 3- to 12-membered heterocycloalkylene is 8-, 9-, or 10-membered bridged heterocycloalkylene containing 1, 2, or 3 heteroatoms of N and/or O, preferably azetidinylene, pyrrolidinylene, piperidinylidene, piperazinylidene, hexahydro-1H-pyrrolizinylene, 2,5-diazabicyclo[2.2.1]heptanylene, 3,8-diazabicyclo[3.2.1]octanylene, 3,6-diazabicyclo[3.1.1]heptanylene, or 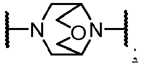
(3) in the substituents of ring U in LA, the halogen is F, Cl, Br, or I;
(4) in the substituents of ring U in LA, the C₁-C₆ alkyl and C₁-C₆ alkyl in the -O-(C₁-C₆ alkyl) are independently methyl, ethyl, n-propyl, isopropyl, n-butyl, isobutyl, sec-butyl, or *tert*-butyl;
(5) in LA, 1, 2, 3, or 4 hydrogen atoms on ring U are optionally substituted with substituents selected from halogen, amino, hydroxy, -O-(C₁-C₆ alkyl), and C₁-C₆ alkyl, or 1, 2, 3, or 4 hydrogen atoms on ring U may also be substituted with C₁-C₆ alkyl that is substituted with 1, 2, or 3 hydroxy;
preferably, 1, 2, 3, or 4 hydrogen atoms in ring U are optionally substituted with groups selected from F, - OH, -OCH₃, -NH₂, and methyl; or 1, 2, 3, or 4 hydrogen atoms on ring U are substituted with -CH₂OH;
(6) in ring Y of LA, the C₃-C₁₂ cycloalkylene is C₃-C₆ monocyclic cycloalkylene, such as cyclohexylene;
(7) in ring Y of LA, the 3- to 12-membered heterocycloalkylene is 6- to 8-membered monocyclic heterocycloalkylene, 7- to 11-membered spiroheterocycloalkylene, or 7- to 11-membered fused heterocycloalkylene, containing 1 or 2 heteroatoms of N; or the 3- to 12-membered heterocycloalkylene is 7-, 8-, 9-, 10-, or 11-membered spiroheterocycloalkylene containing 1 or 2 heteroatoms of N and/or O, preferably piperidinylidene, piperazinylidene, 3-azaspiro[5.5]undecylene, 7-azaspiro[3.5]nonanylene, 2,7-diazaspiro[3.5]nonanylene, 3,9-diazaspiro[5.5]undecylene, 2,6-diazaspiro[3.3]heptanylene, 2-azaspiro[3.3]heptanylene, or 2-oxa-9-azaspiro[5.5]undecylene; and
(8) in the substituents of ring Y in LA, C₁-C₆ alkyl in the -O-(C₁-C₆ alkyl) is independently methyl, ethyl, n-propyl, isopropyl, n-butyl, isobutyl, sec-butyl, or *tert*-butyl.

13. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 12, wherein LA is of the following structure: 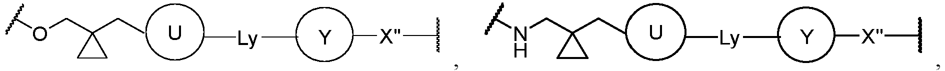 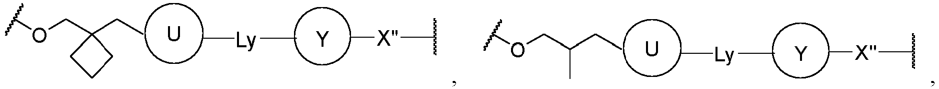 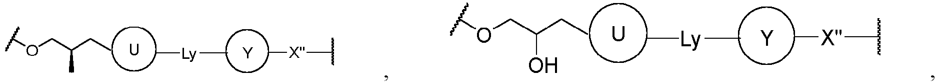 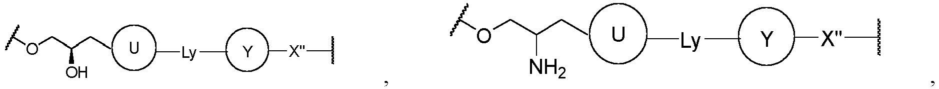 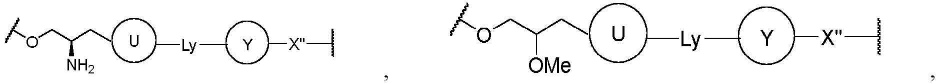 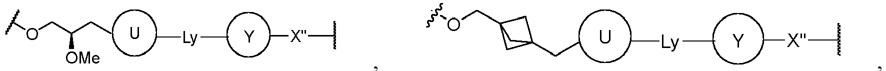 or 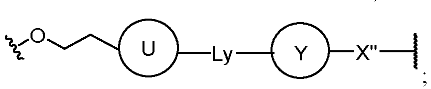
preferably, each ring U is independently 4- to 8-membered saturated monocyclic heterocycloalkylene or 6-to 10-membered bridged heterocycloalkylene, containing 1 or 2 nitrogen heteroatoms; each ring Y is independently 3- to 12-membered heterocycloalkylene containing 1-2 heteroatoms selected from N, O, and S, wherein the heterocycloalkyl is monocyclic heterocycloalkylene or bispiroheterocycloalkylene; X" is - C(O)-;
more preferably, LA is of any one of the following structures: 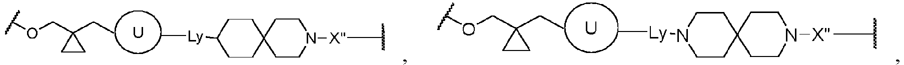 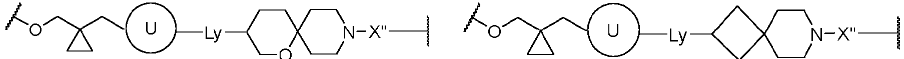 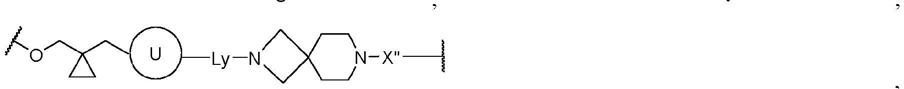 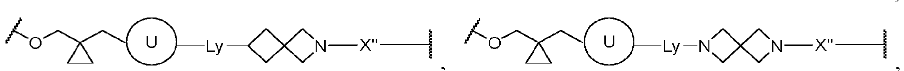 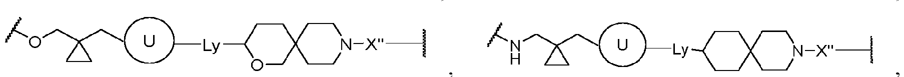 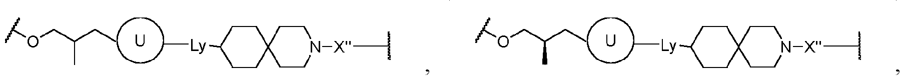 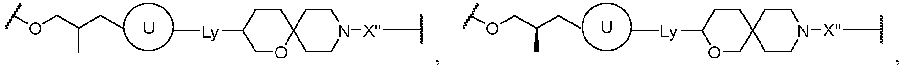 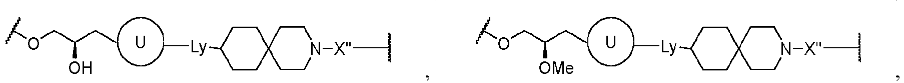 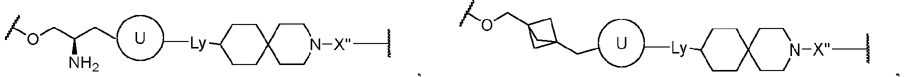 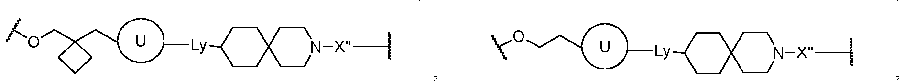 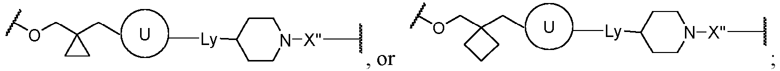
most preferably, ring U in LA is

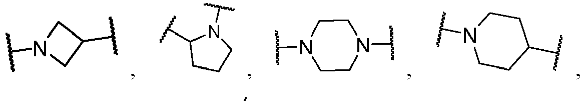

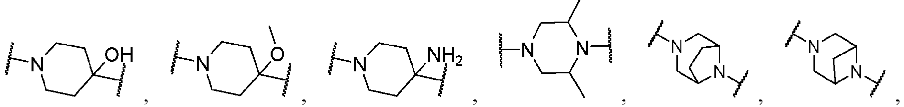

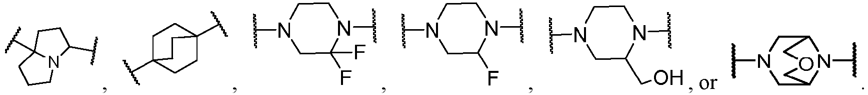

14. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 10, wherein 1 or more of the following conditions are met:
(1) in L, -(CH₂)ⱼ- is: 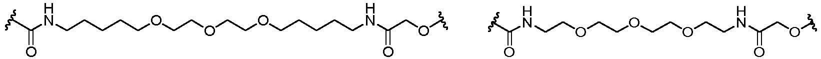 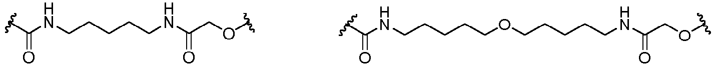 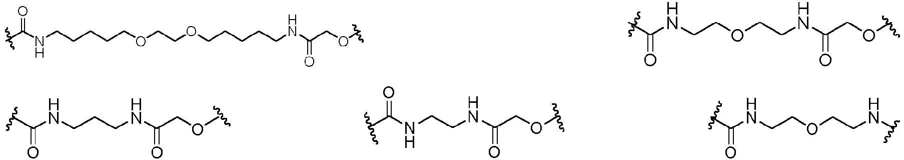 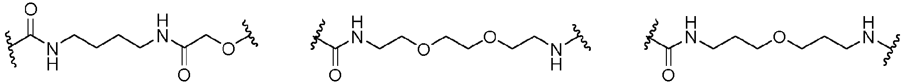 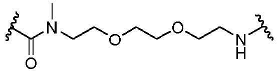 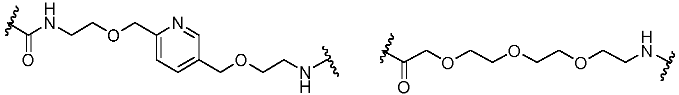 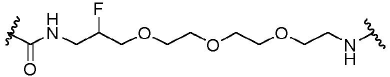 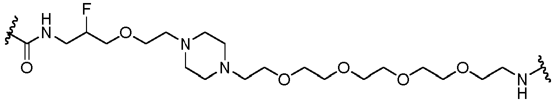 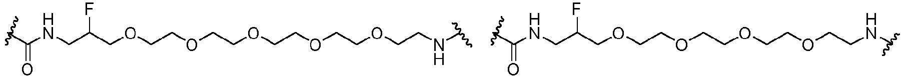 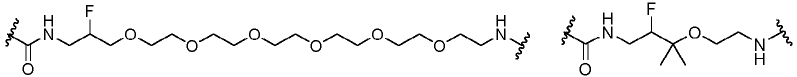 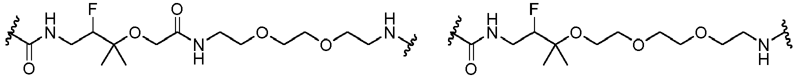 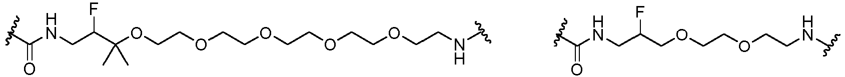 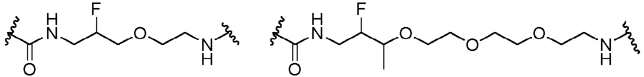 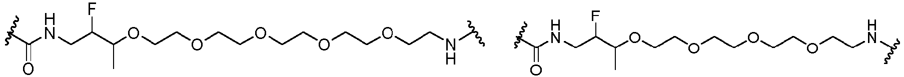 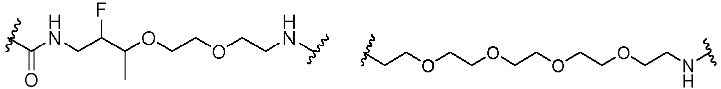 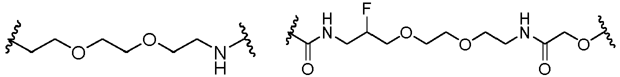 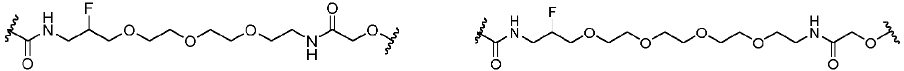 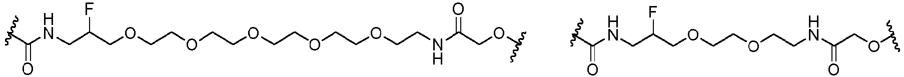 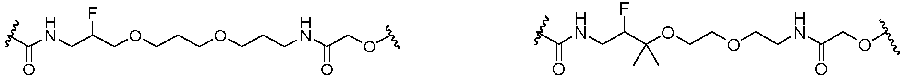 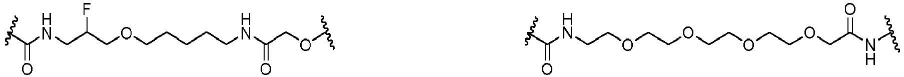 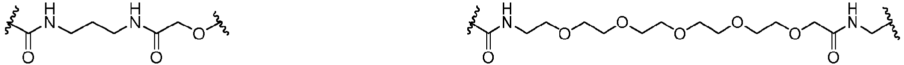 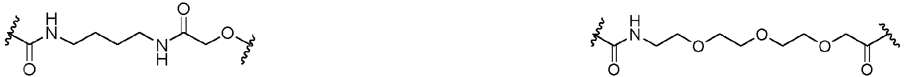 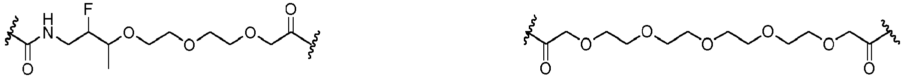 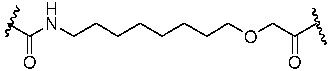 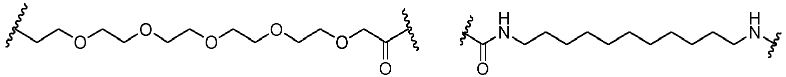 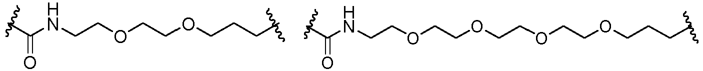 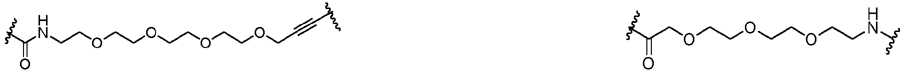 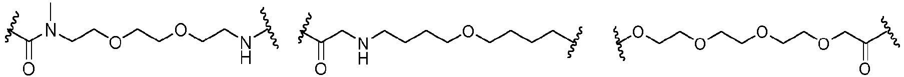 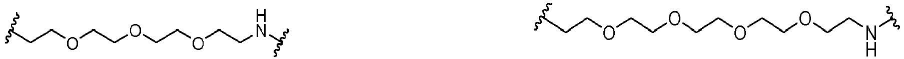 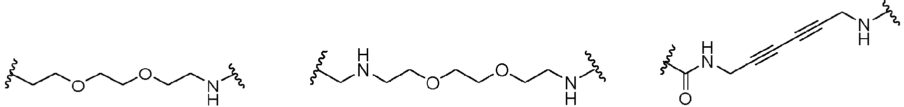 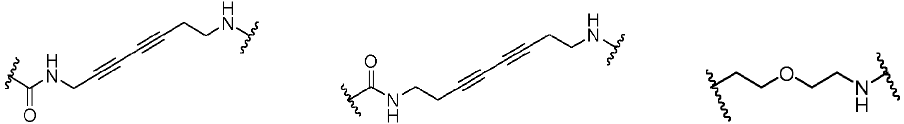 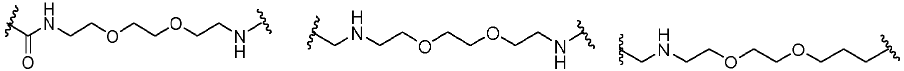 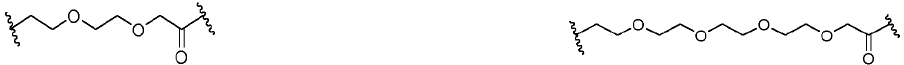 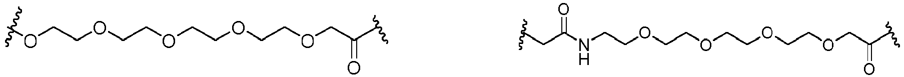 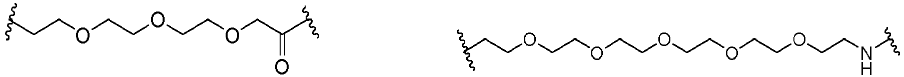 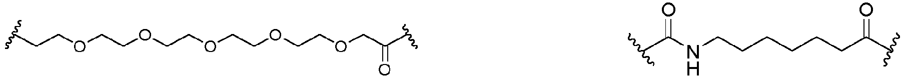 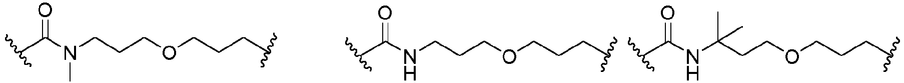 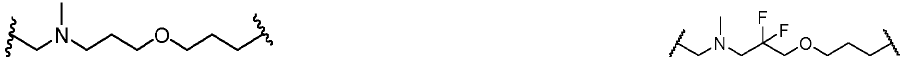 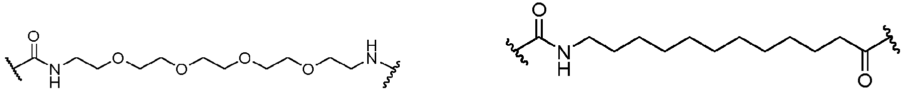 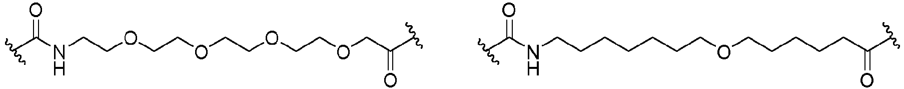 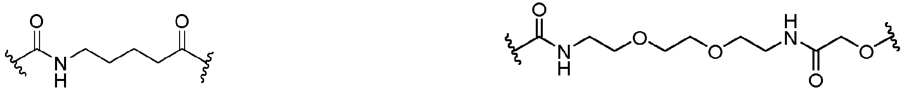 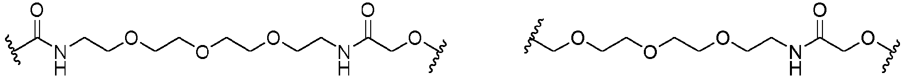 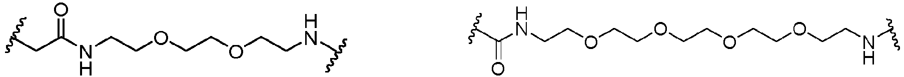 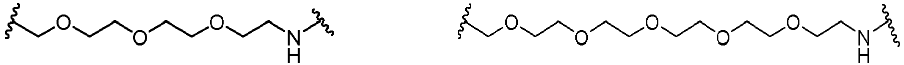 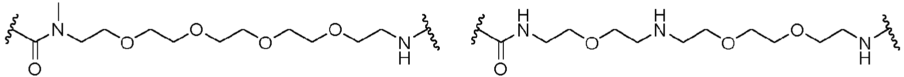 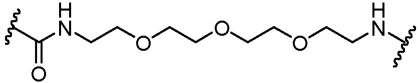 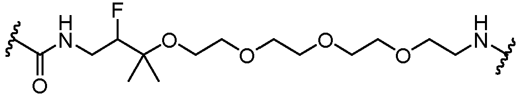 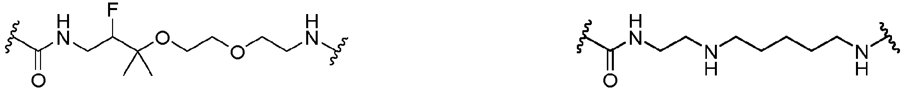 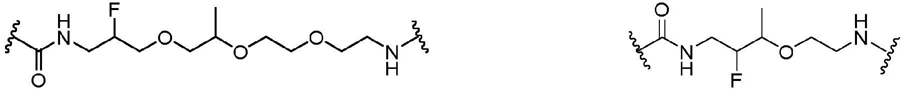 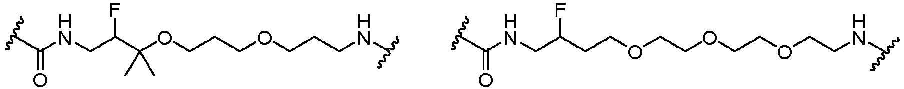 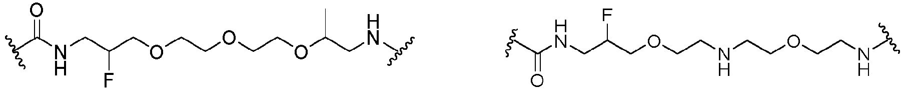 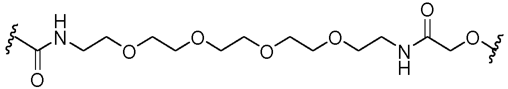 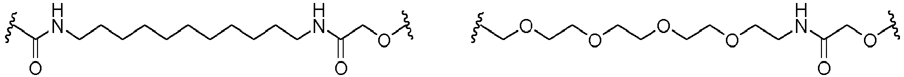 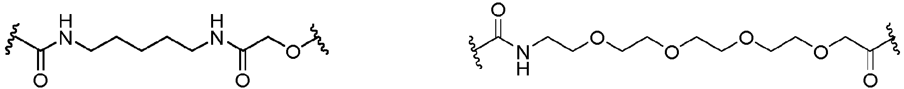 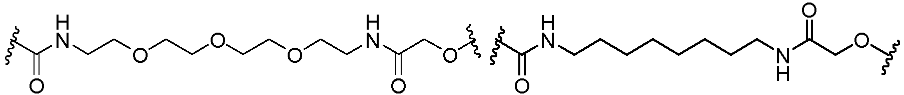 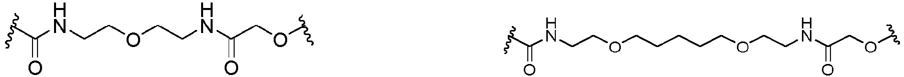 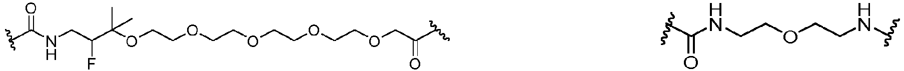 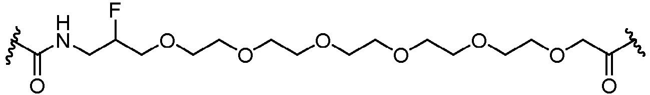 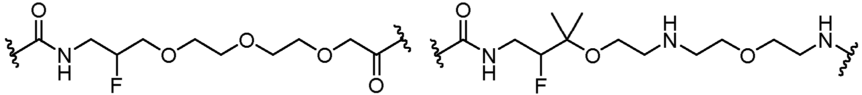 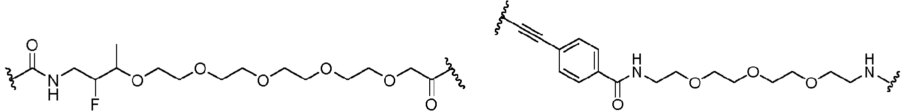 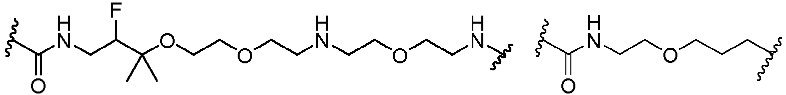 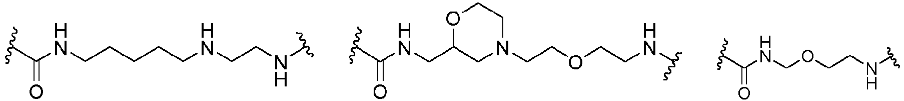 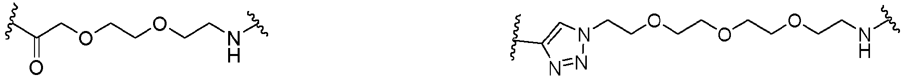 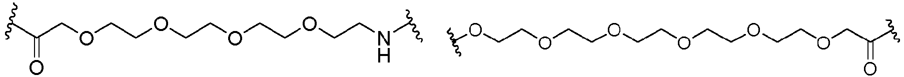 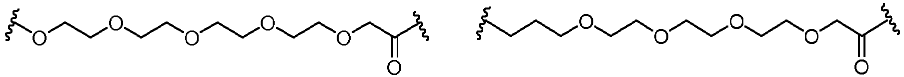 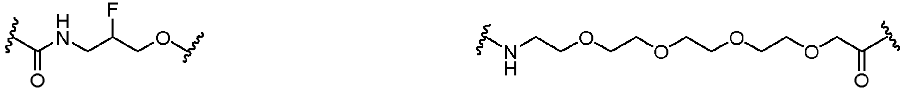 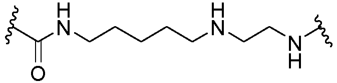 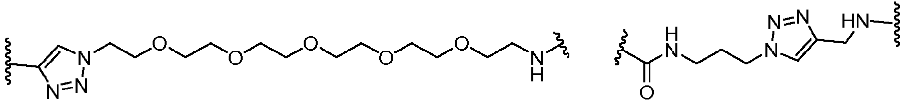 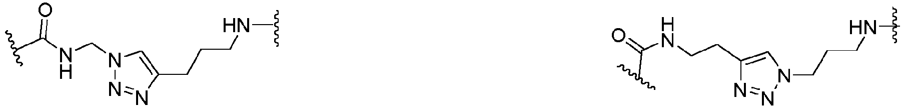 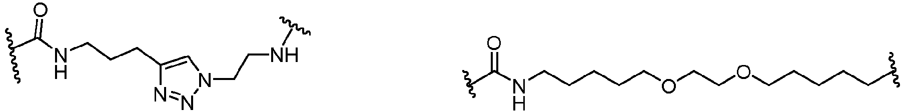 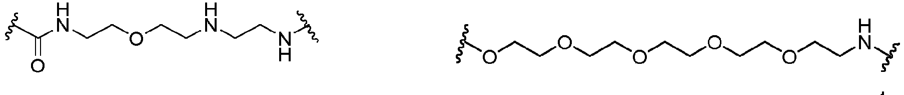 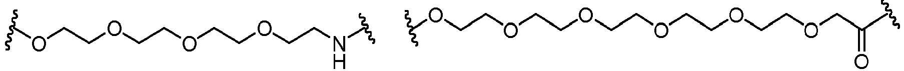 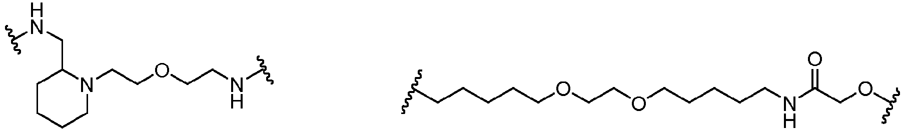 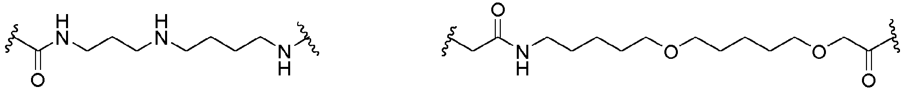 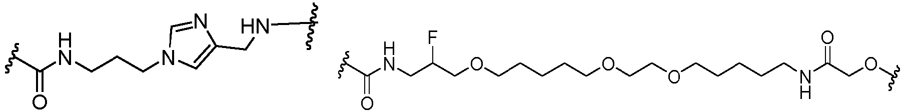 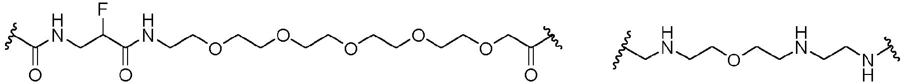 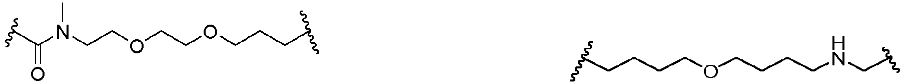 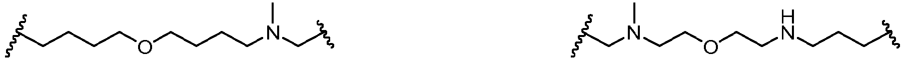 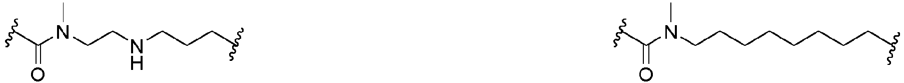 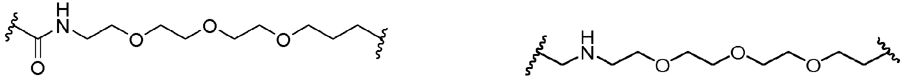 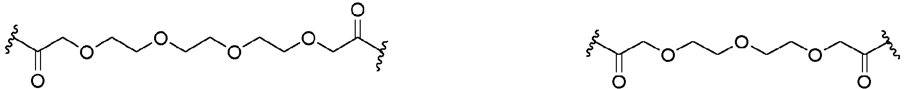 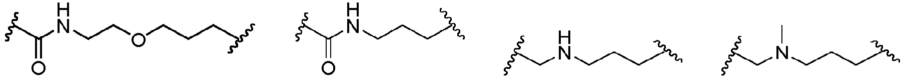 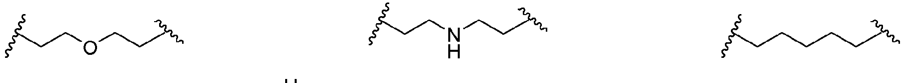 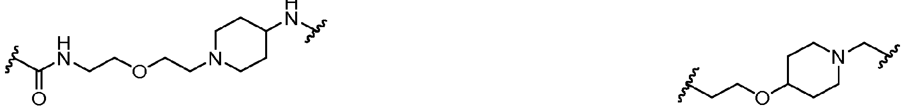 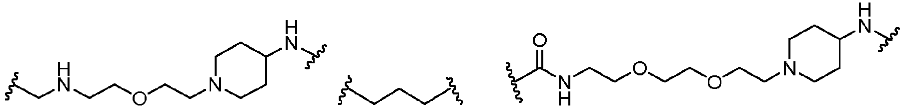 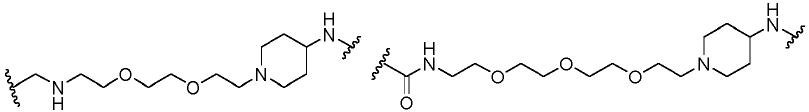 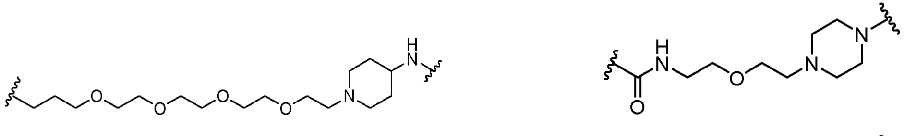 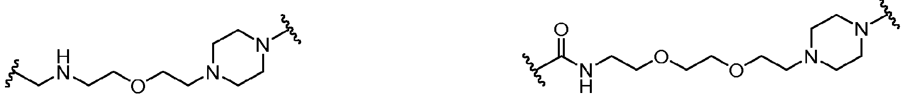 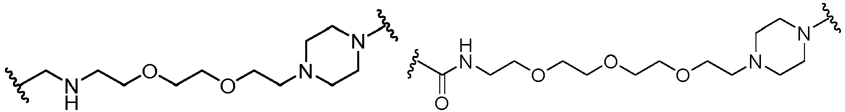 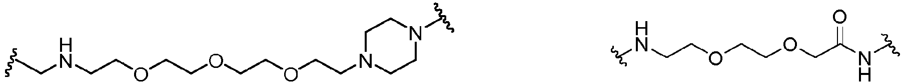 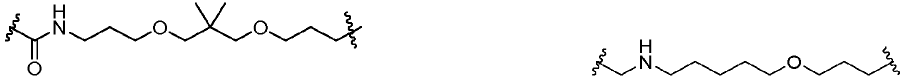 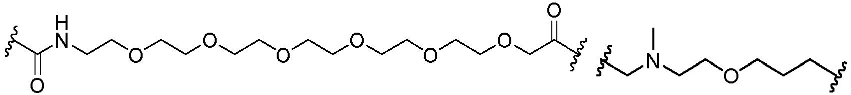 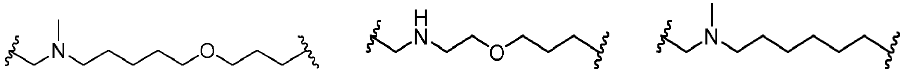 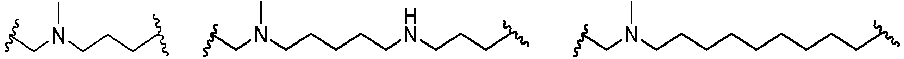 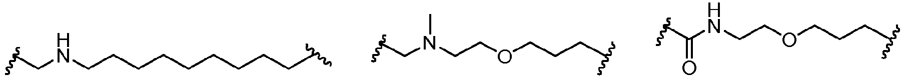 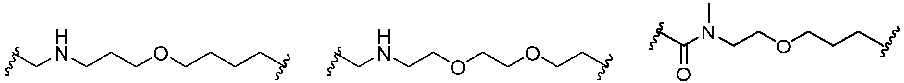 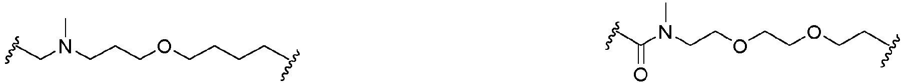 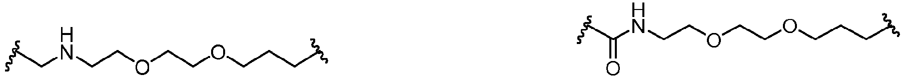 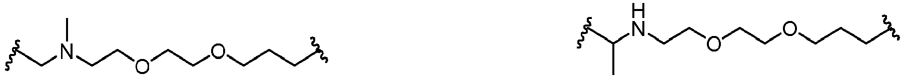 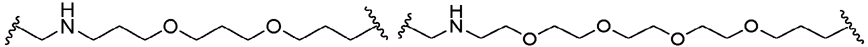 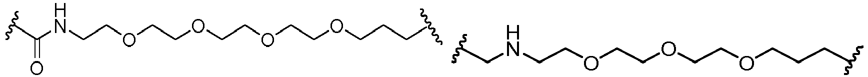 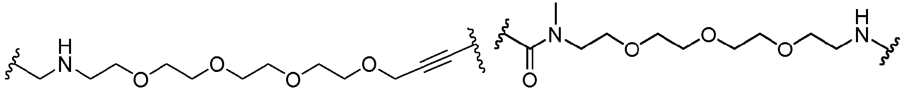 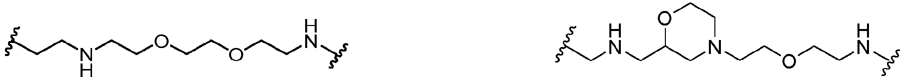 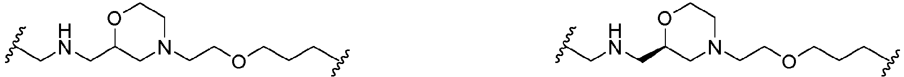 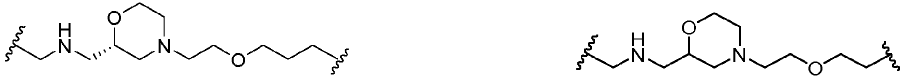 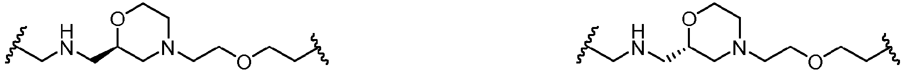 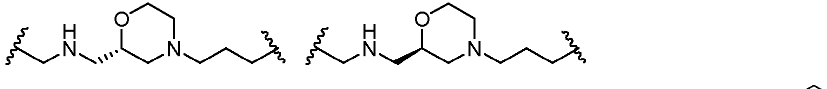 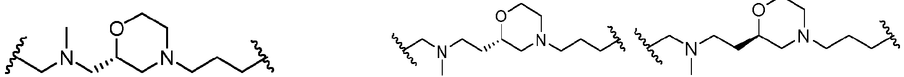 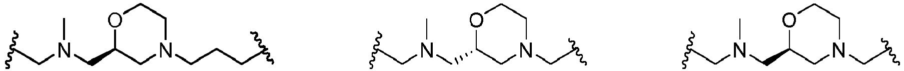 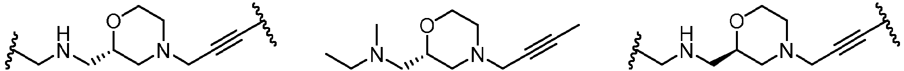 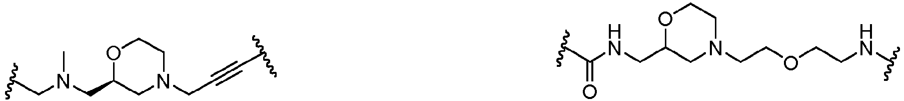 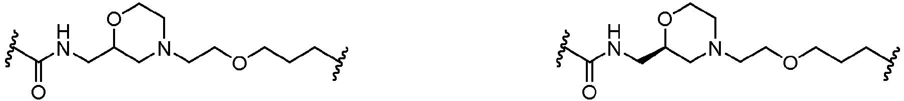 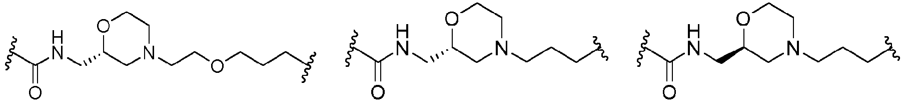 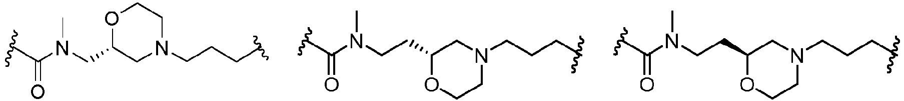 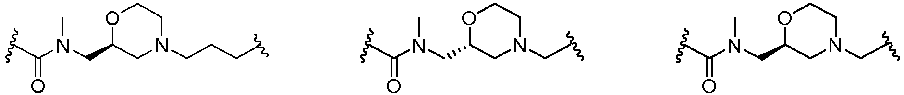 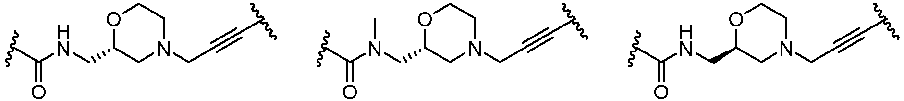 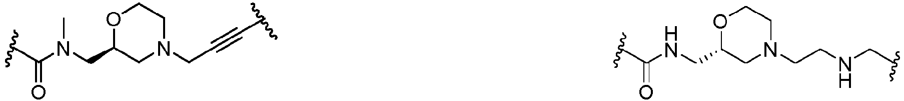 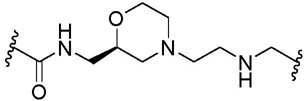 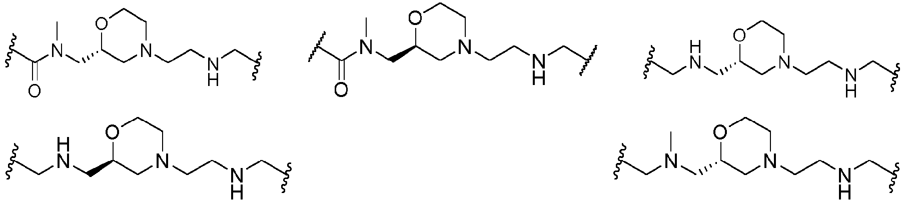 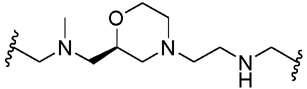 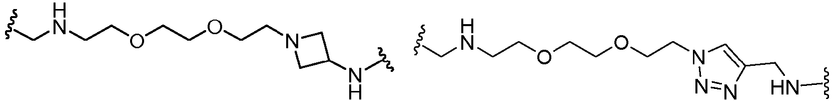 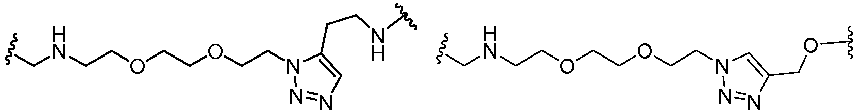 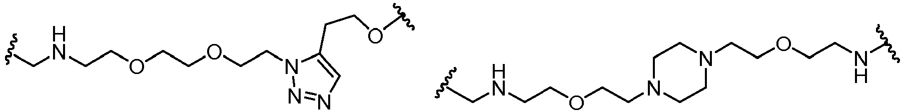 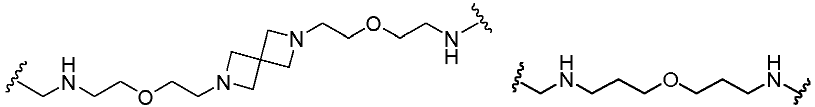 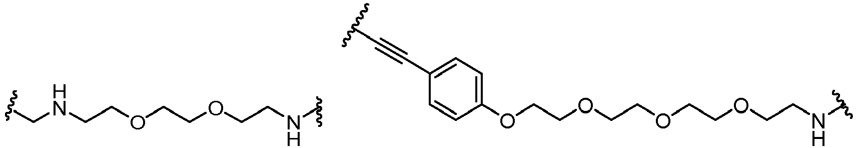 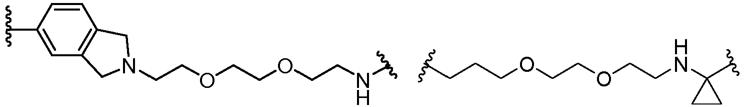 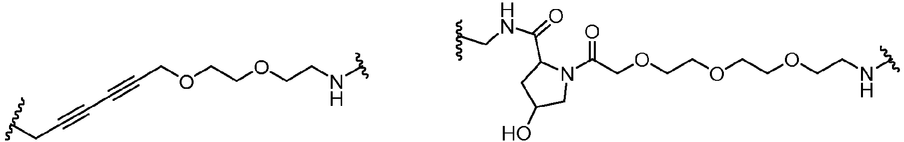 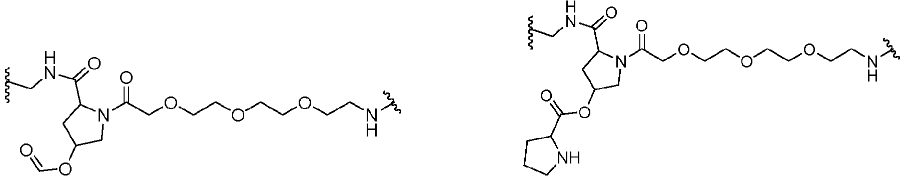 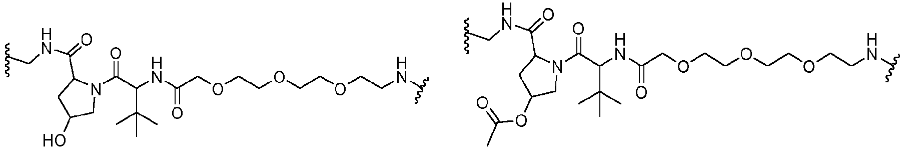 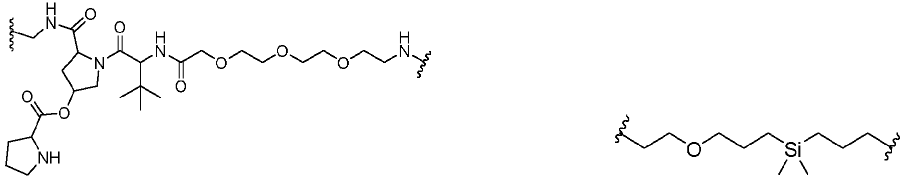 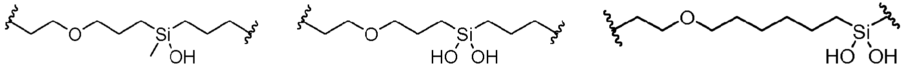 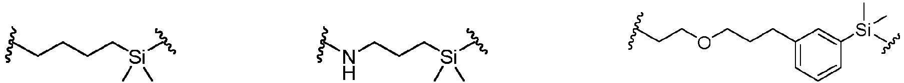 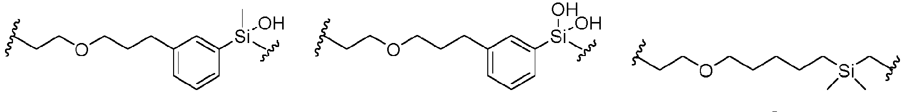 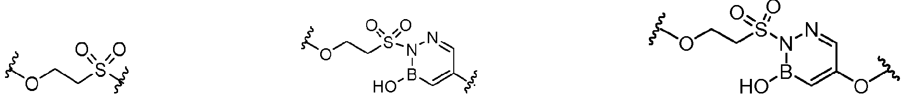 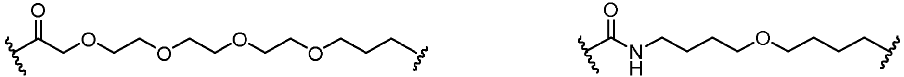 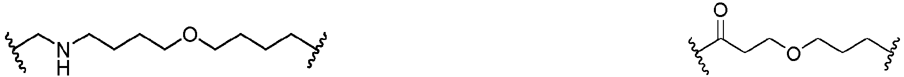 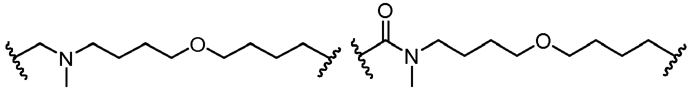 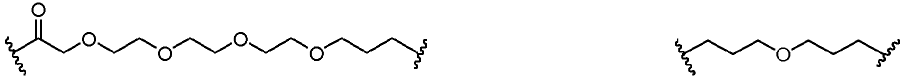 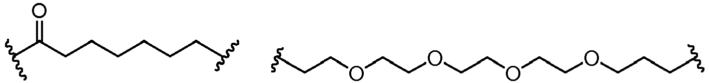 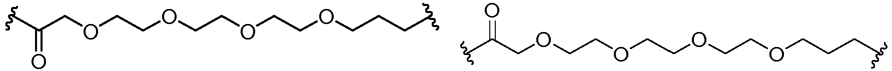 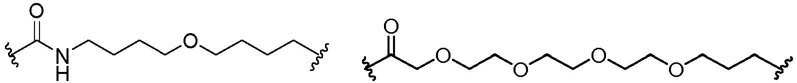 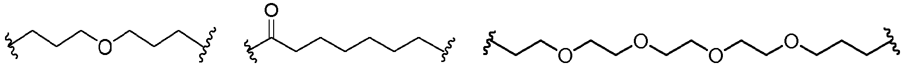 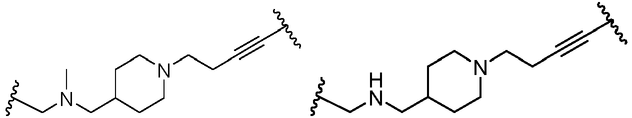 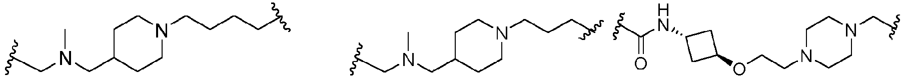 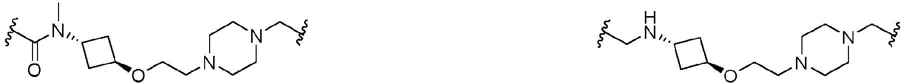 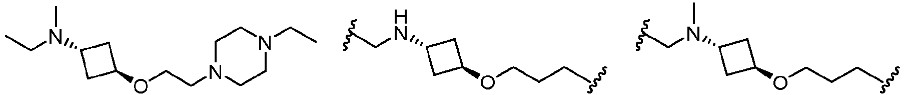 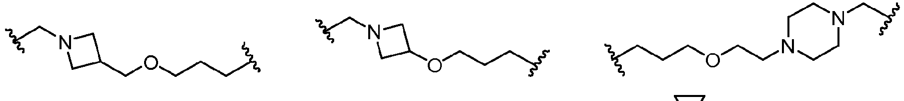 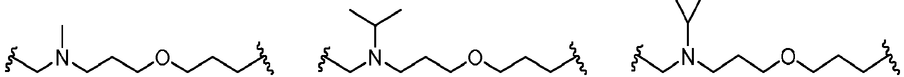 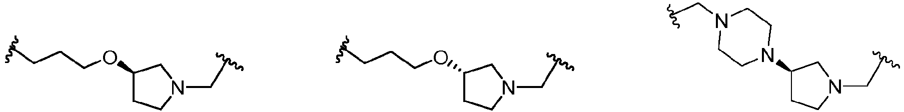 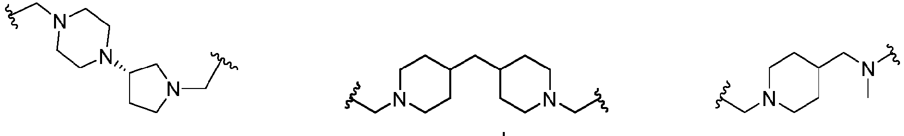 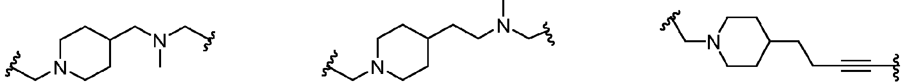 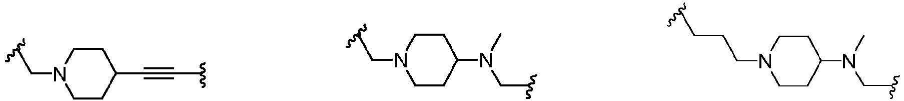 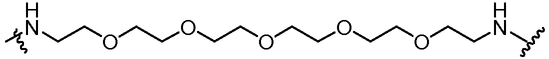 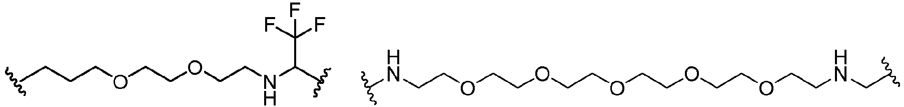 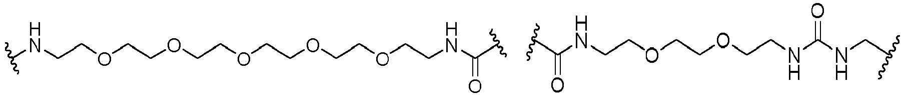 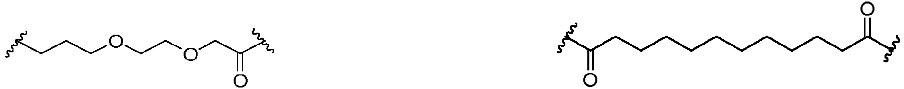 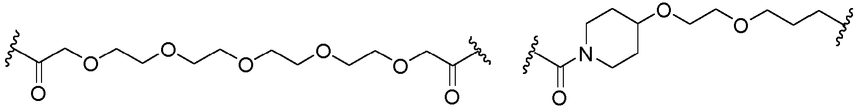 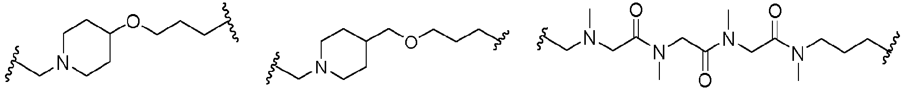 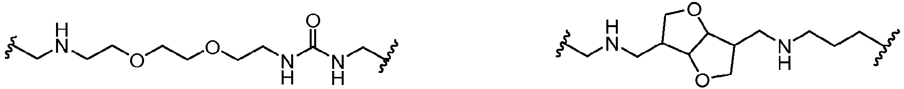 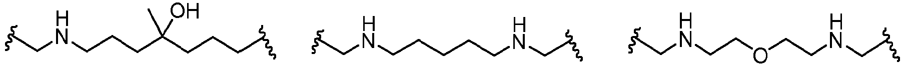 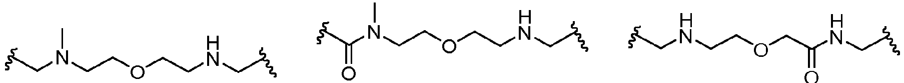 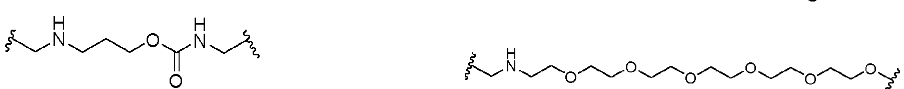 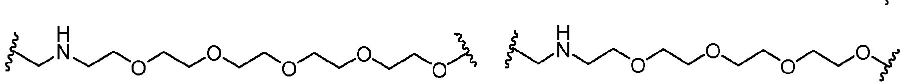 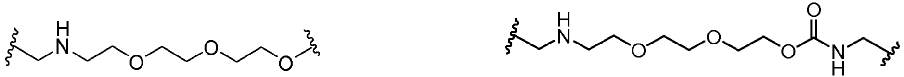 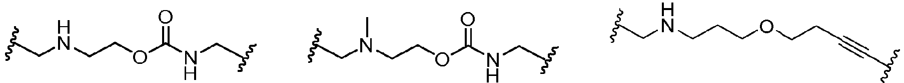 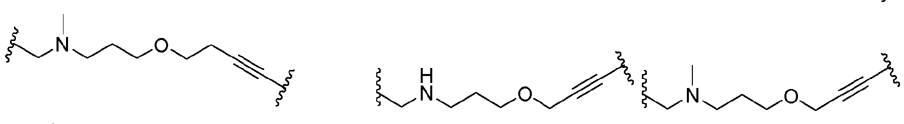 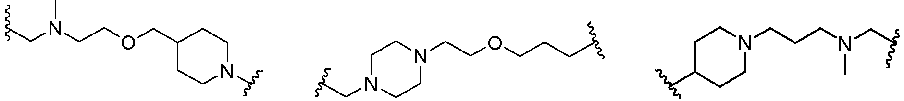 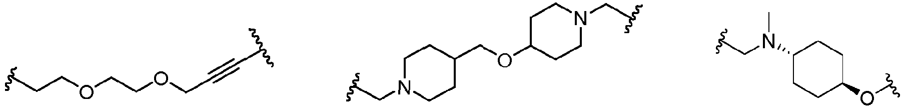 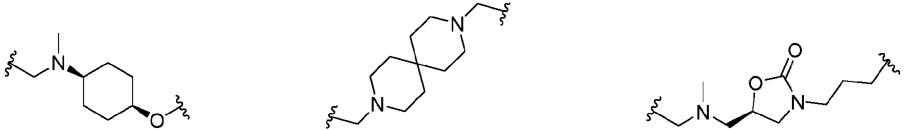 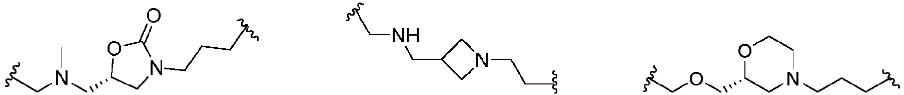 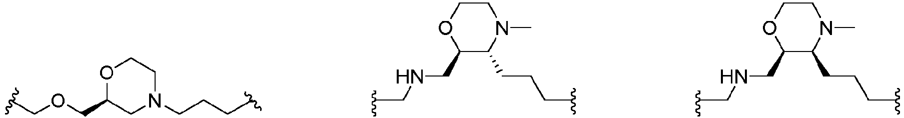 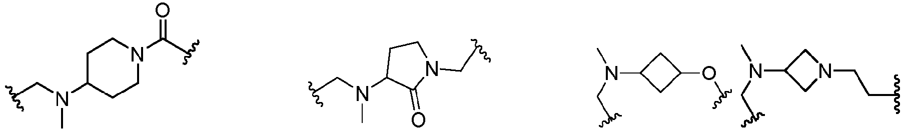 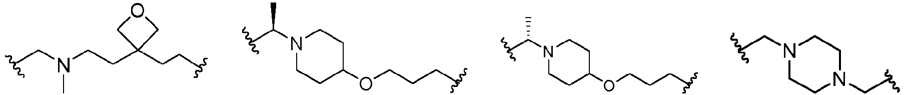 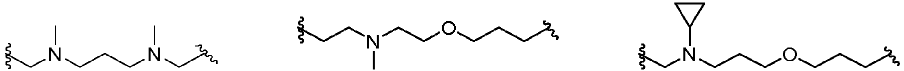 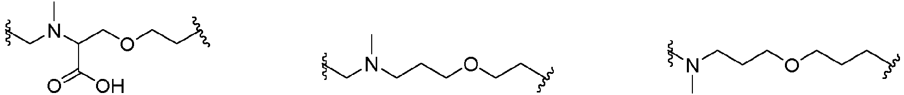 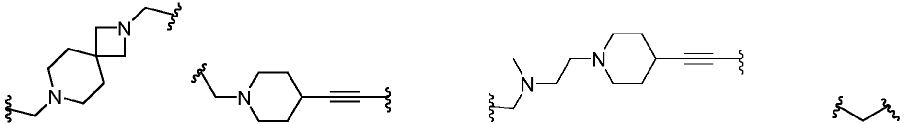 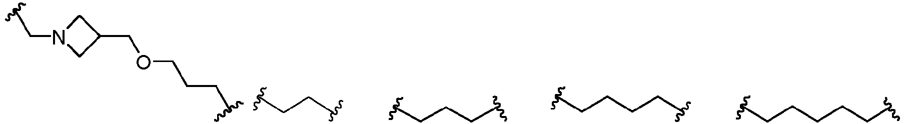 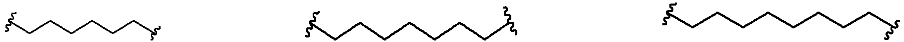 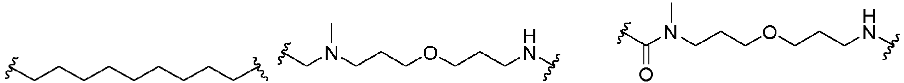 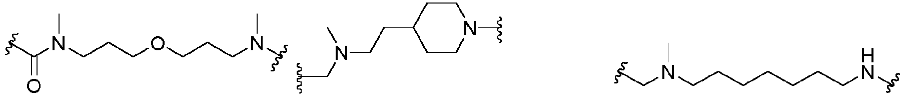 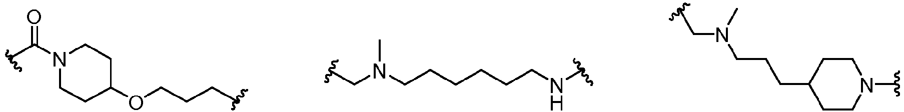 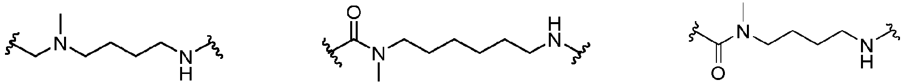 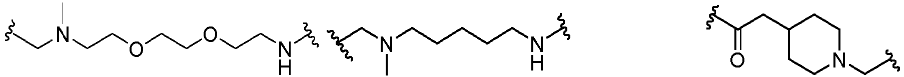 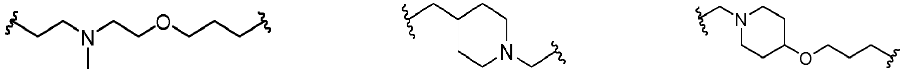 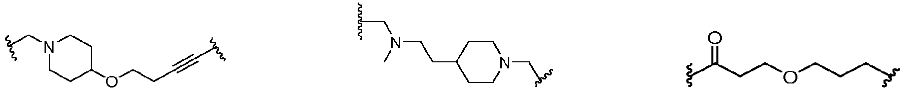 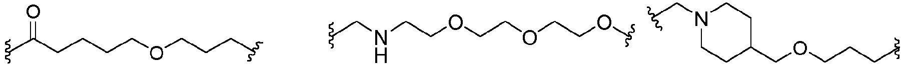 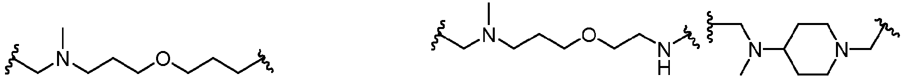 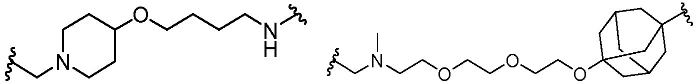 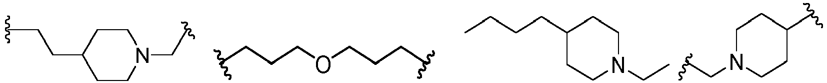 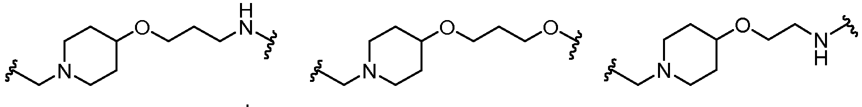 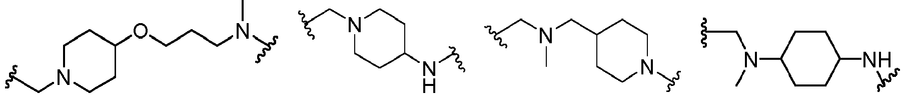 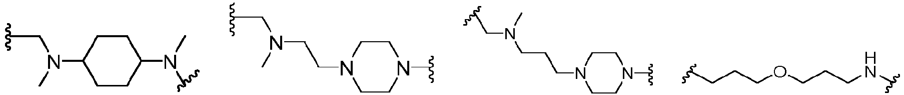 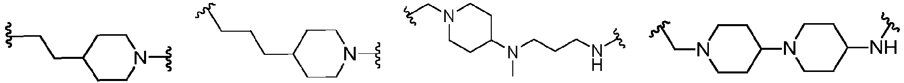 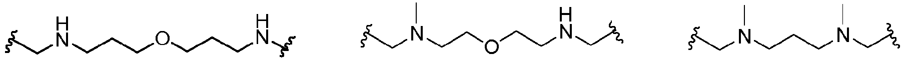 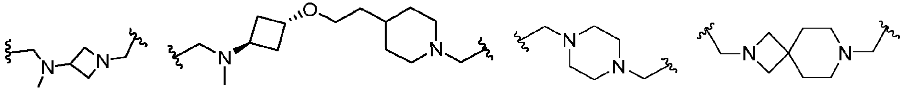 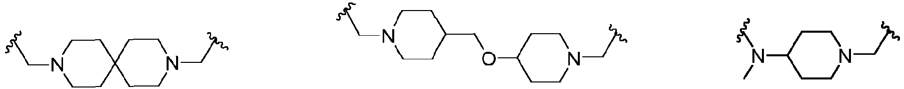 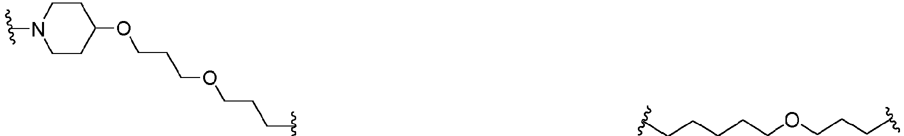 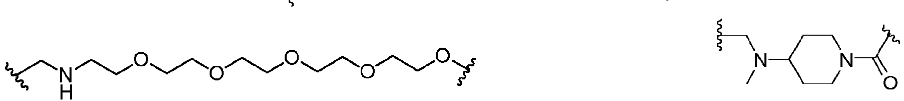 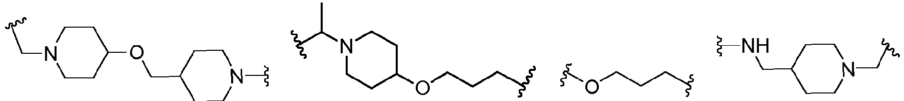 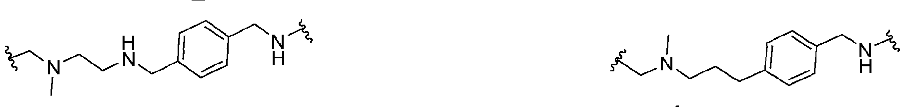 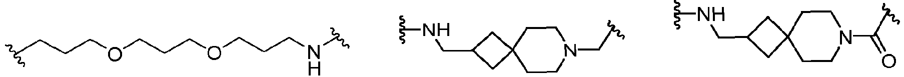 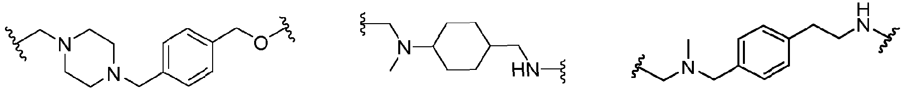 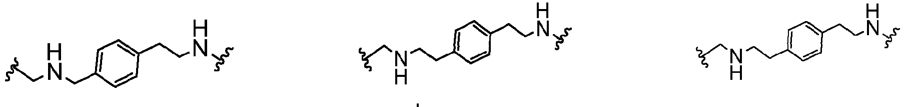 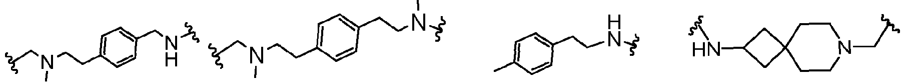 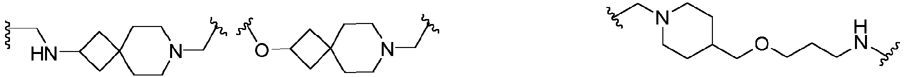 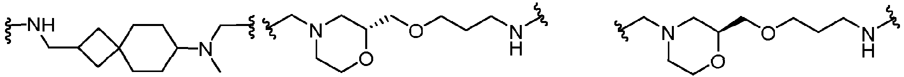 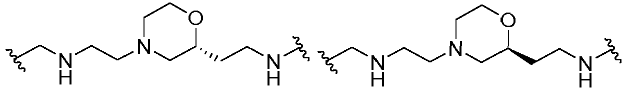 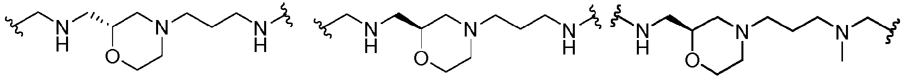 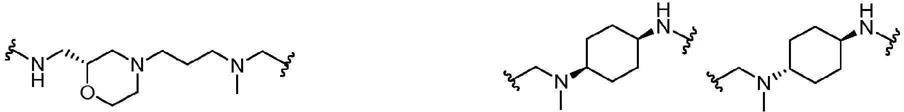 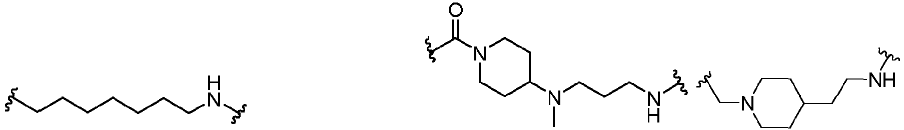 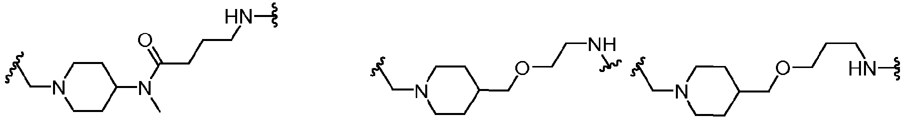 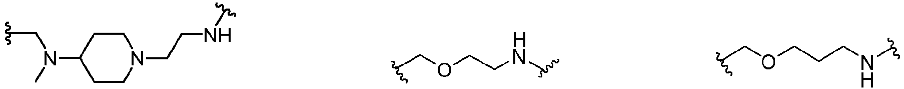 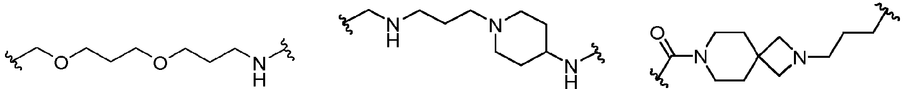 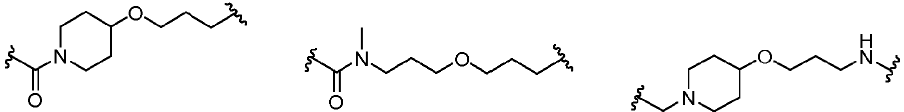 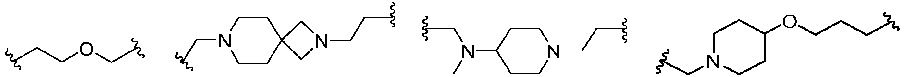 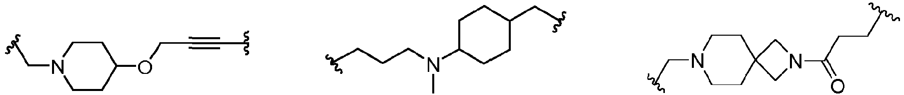 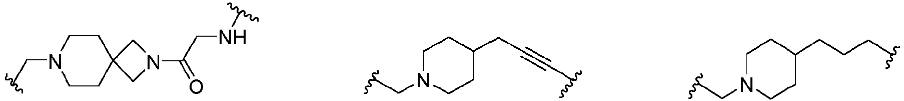 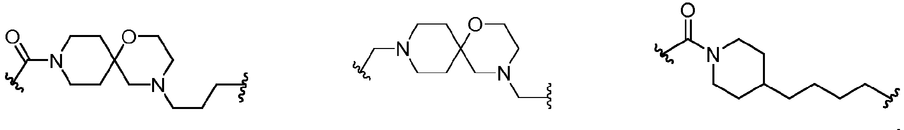 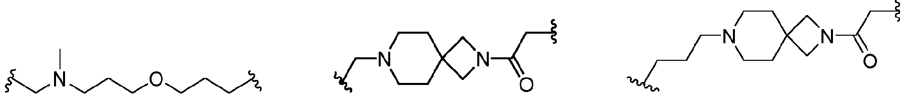 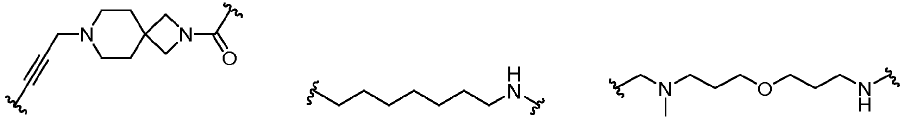 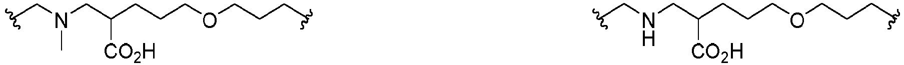 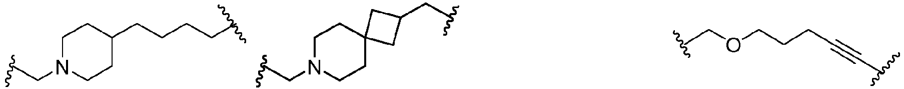 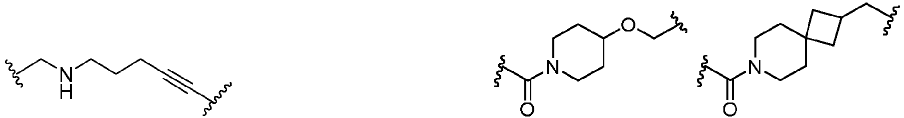 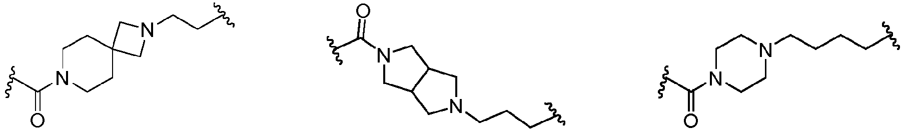 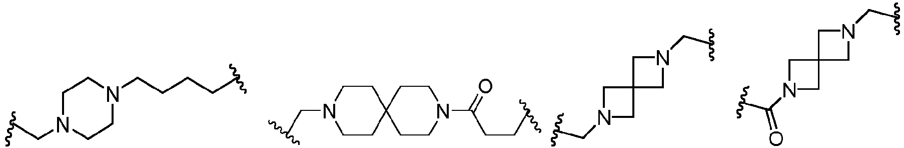 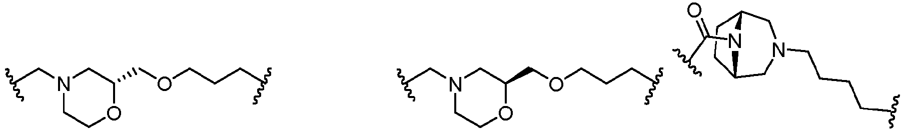 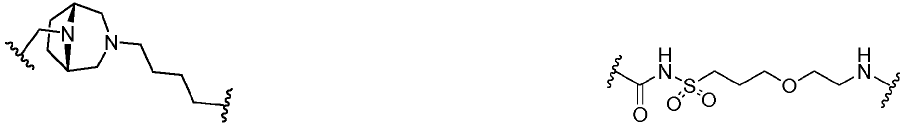 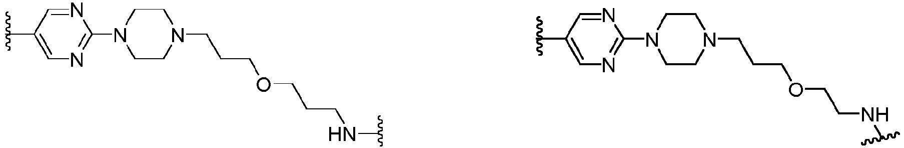 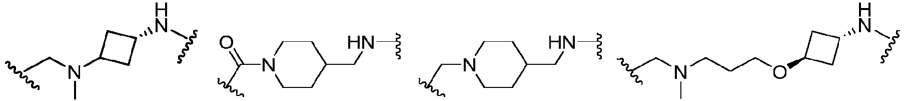 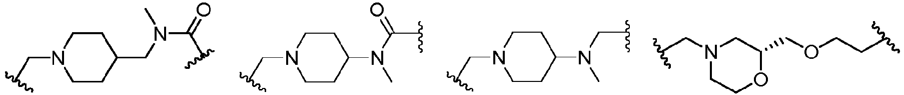 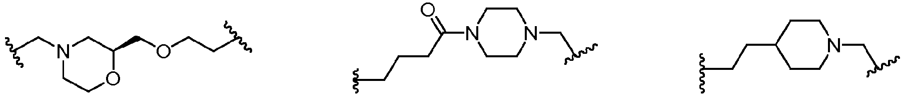 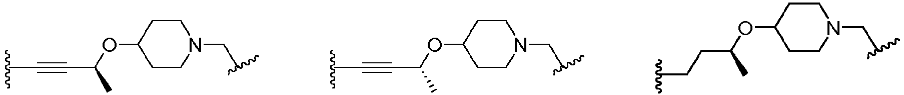 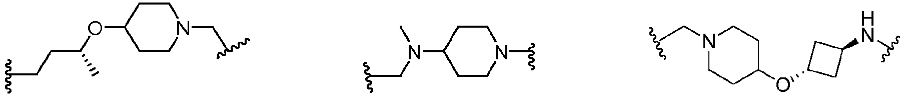 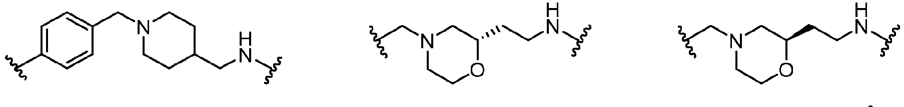 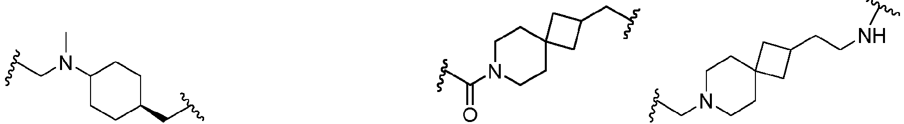 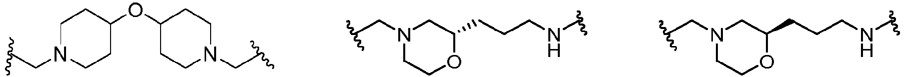 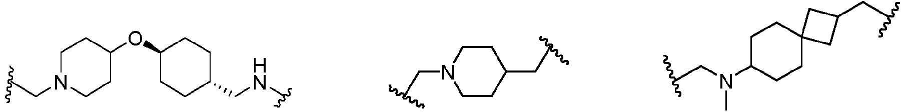 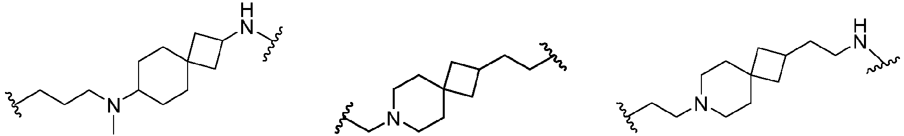 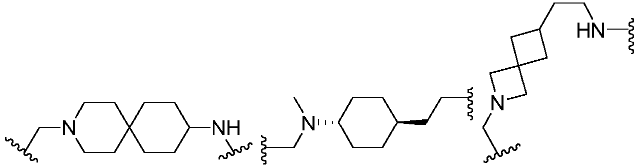 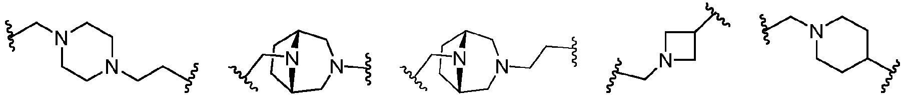 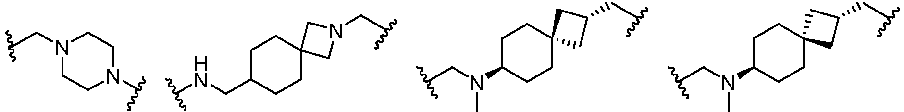 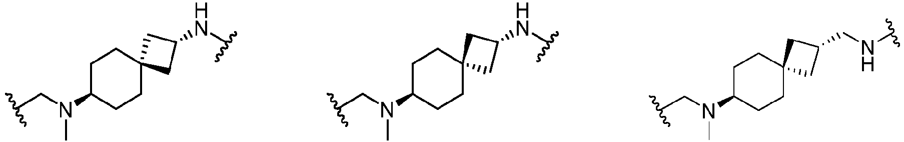 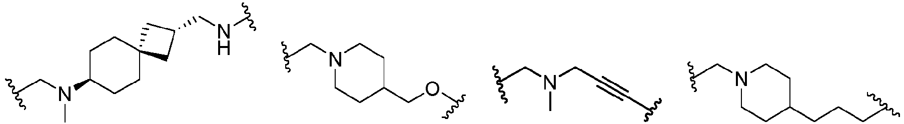 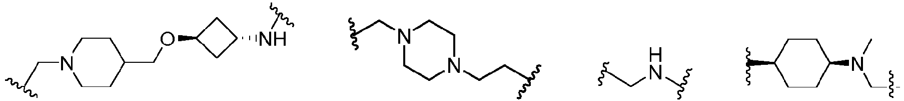 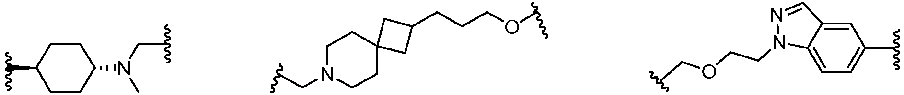 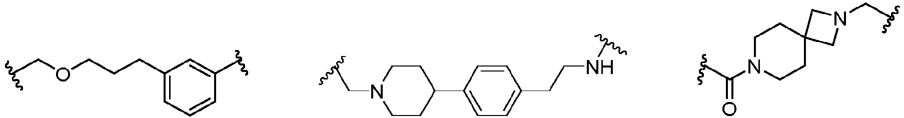 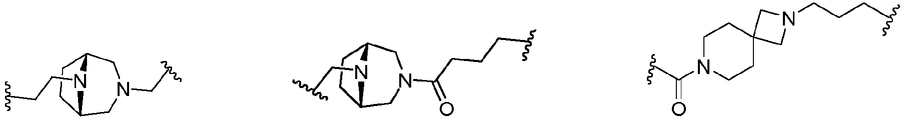 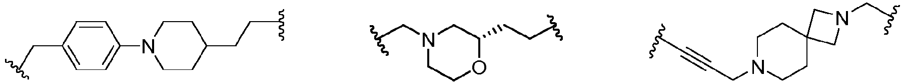 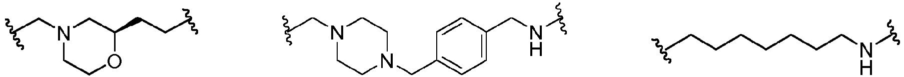 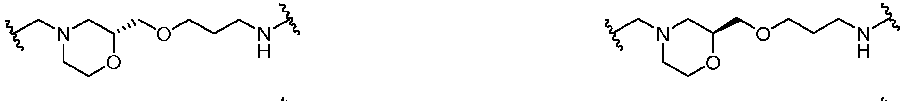 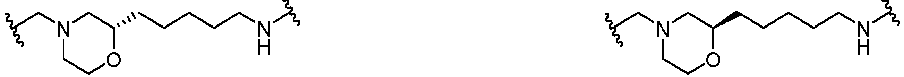 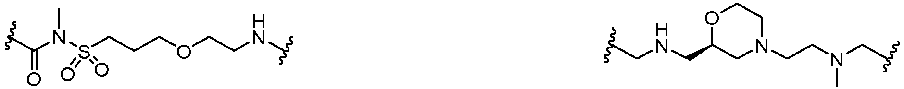 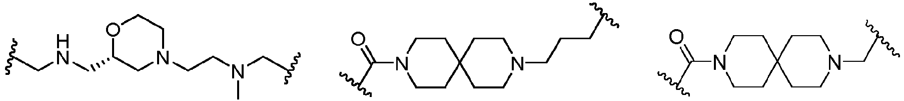 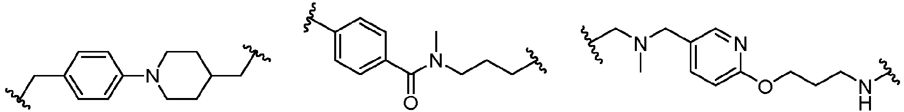 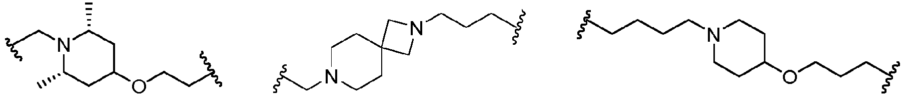 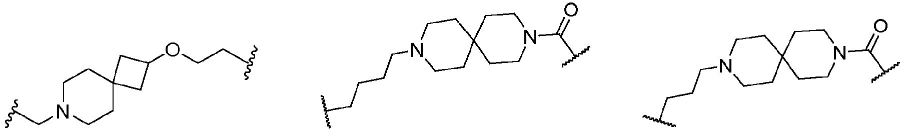 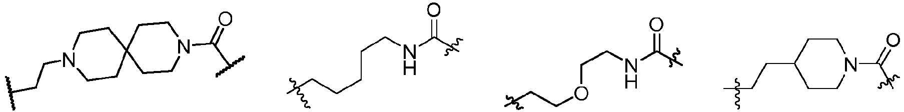 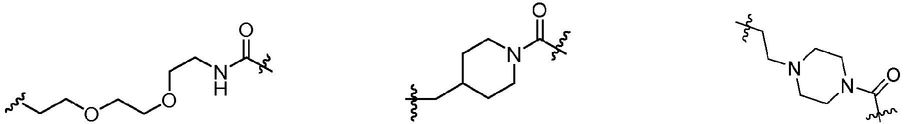 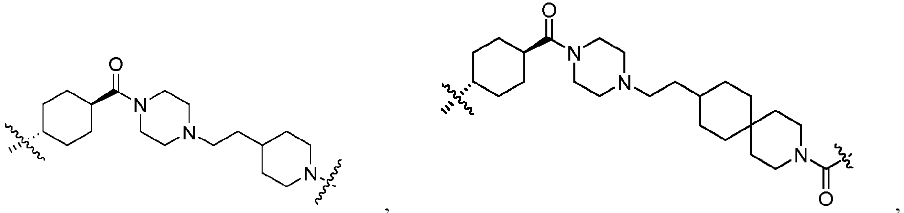 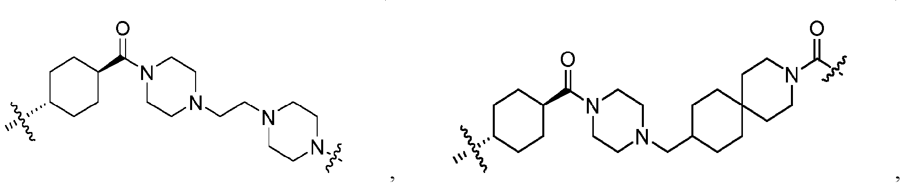 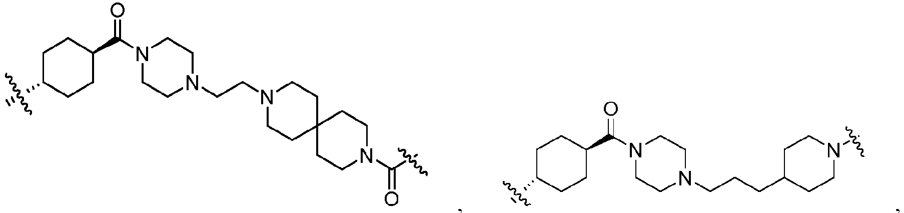 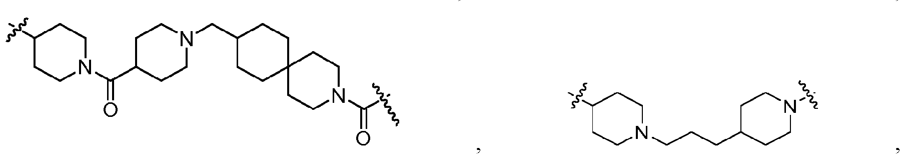 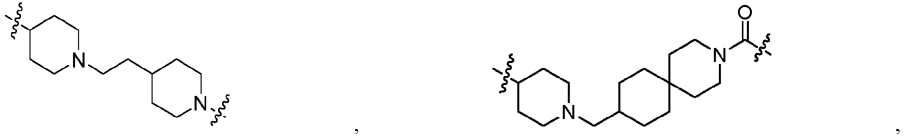 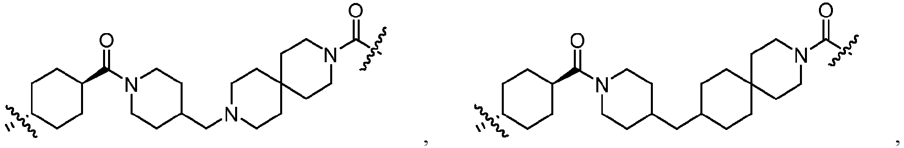 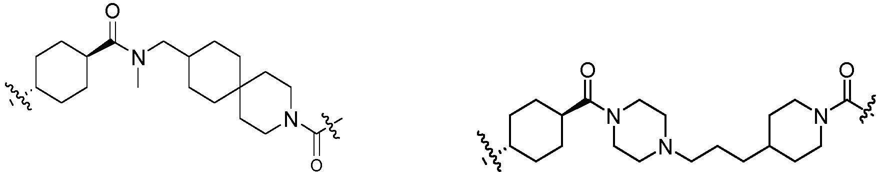 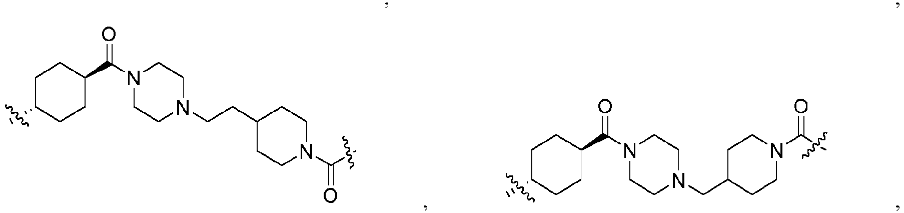 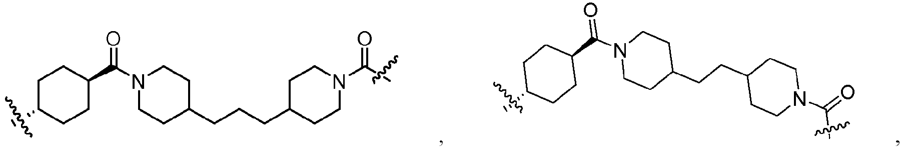 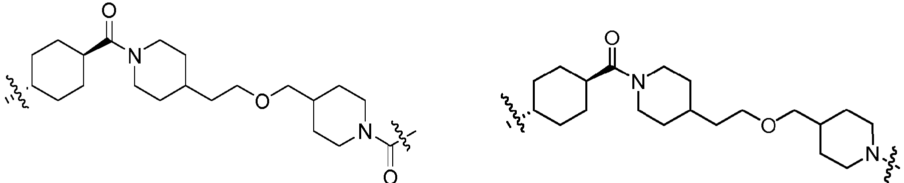 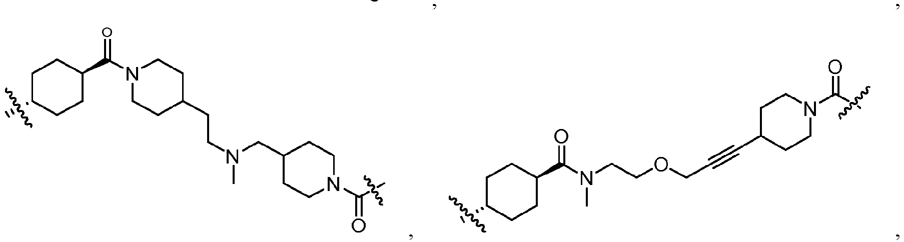 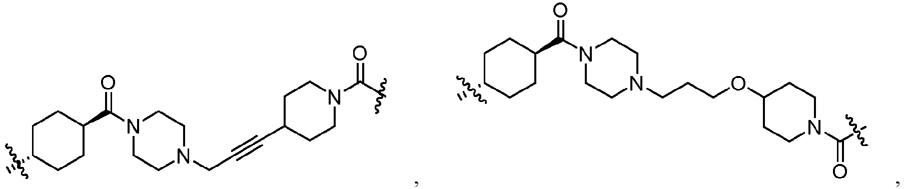 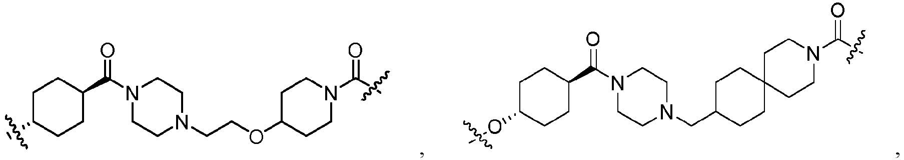 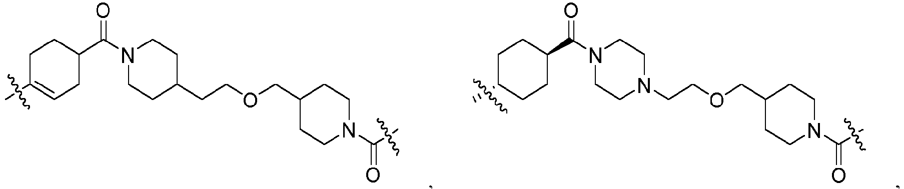 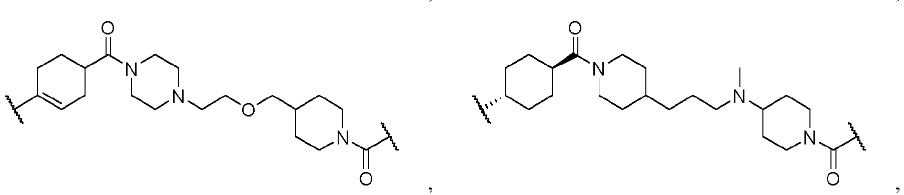 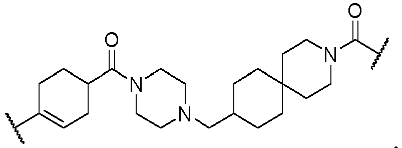 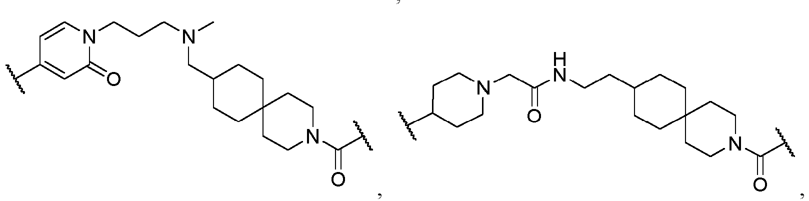 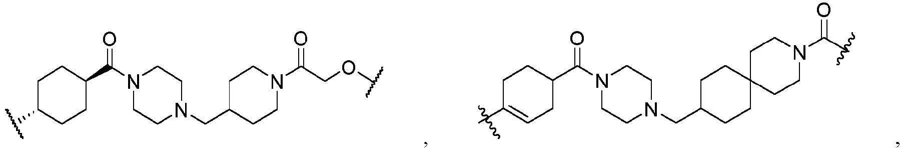 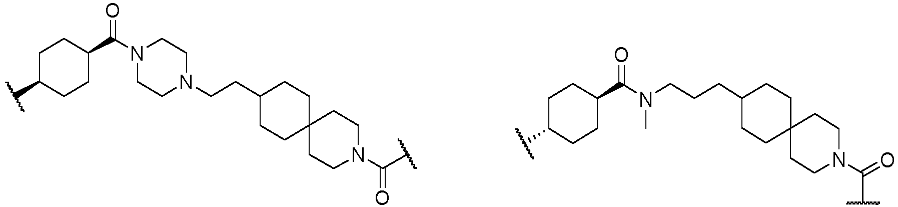 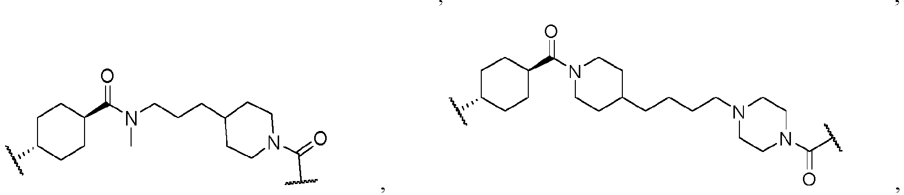 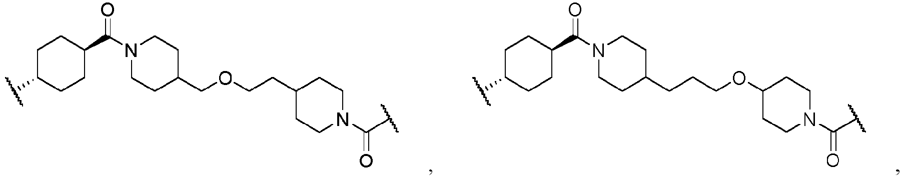 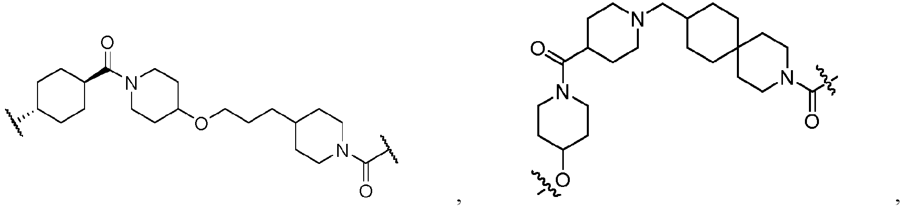 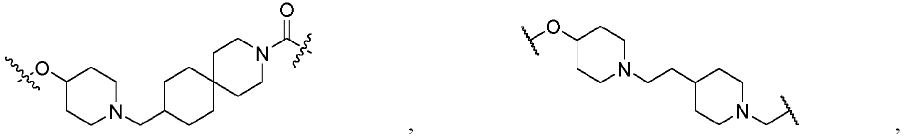 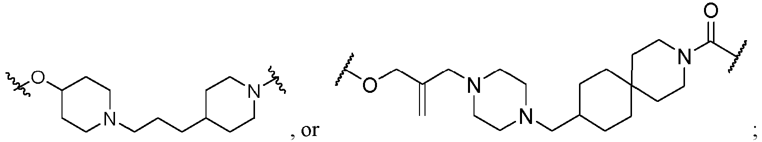
(2) in L, LA is of any one of the following structures:

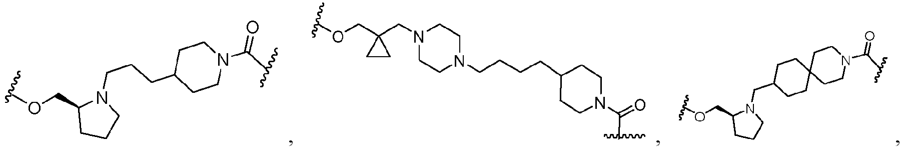

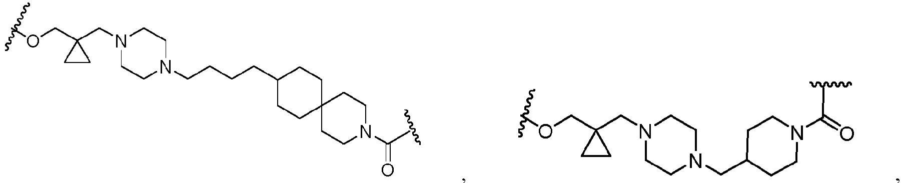

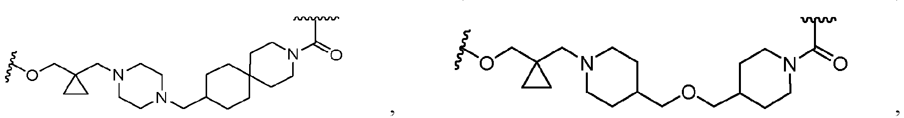

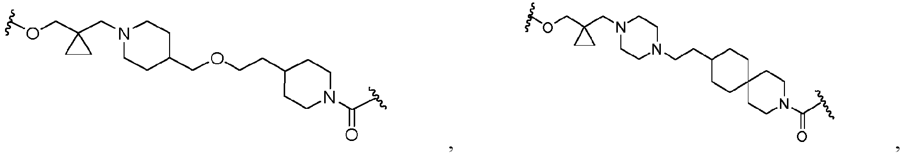

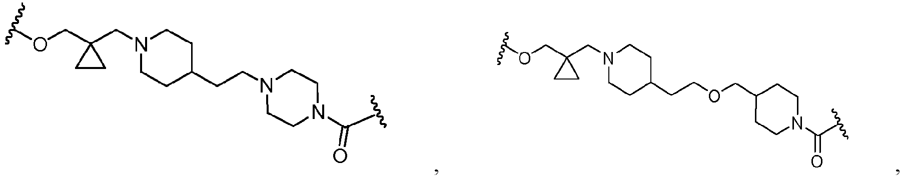

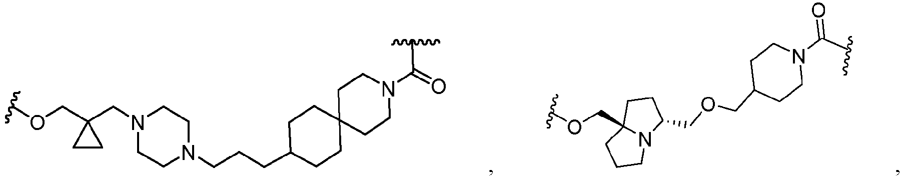

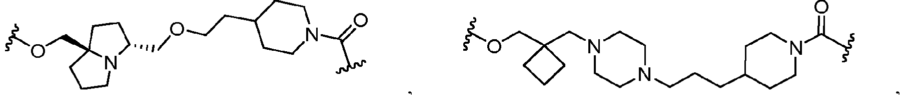

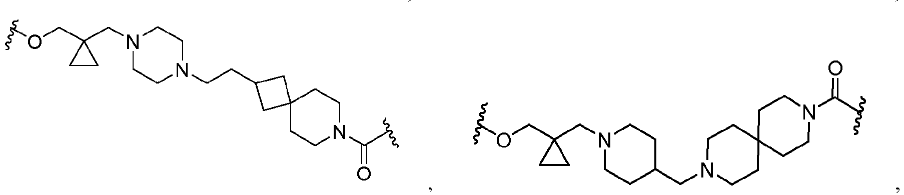

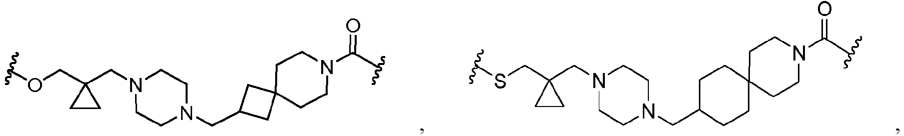

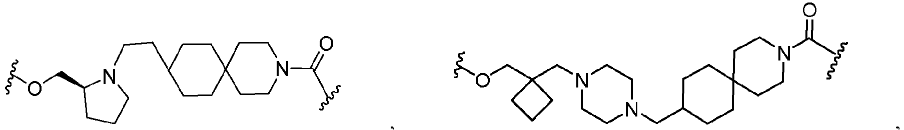

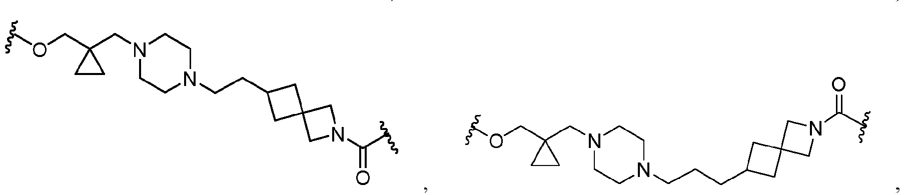

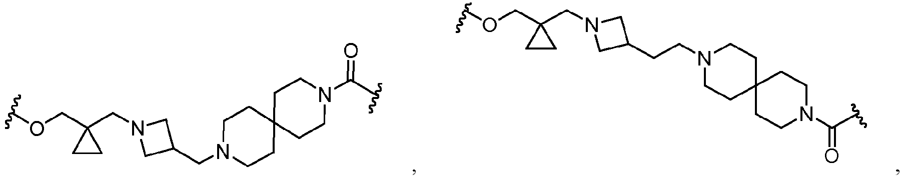

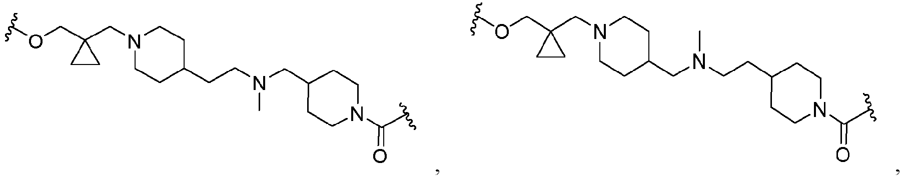

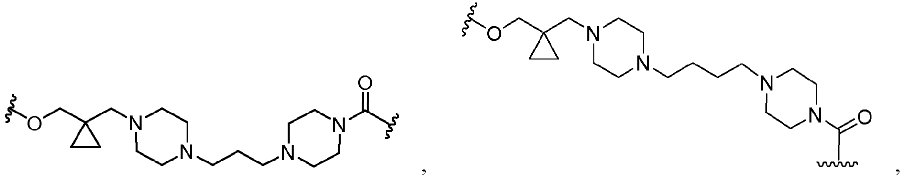

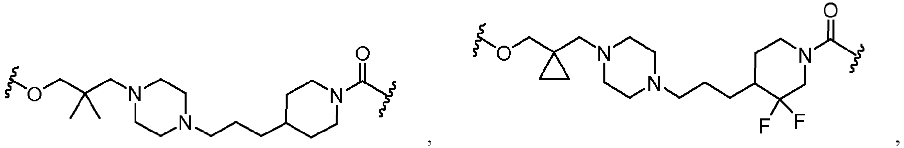

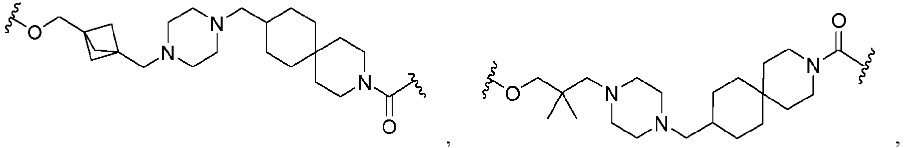

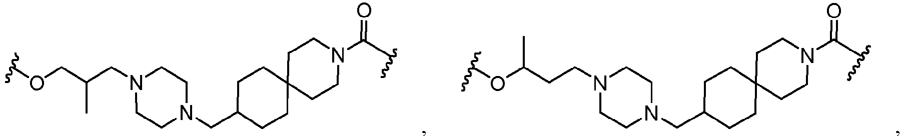

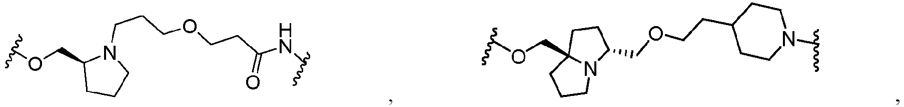

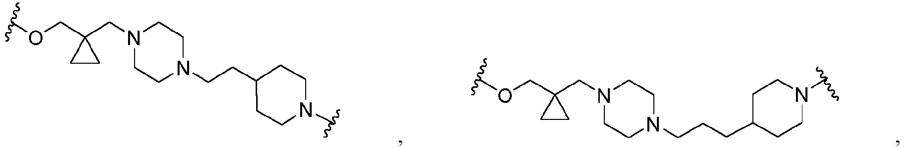

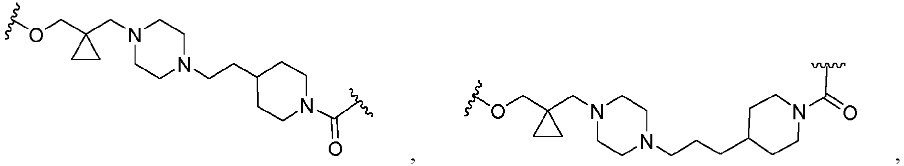

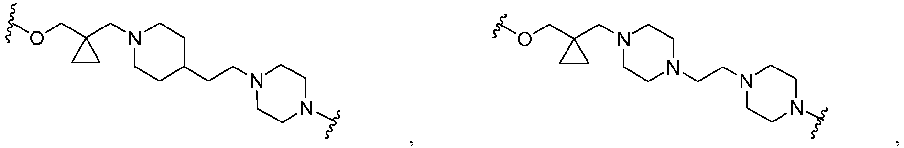

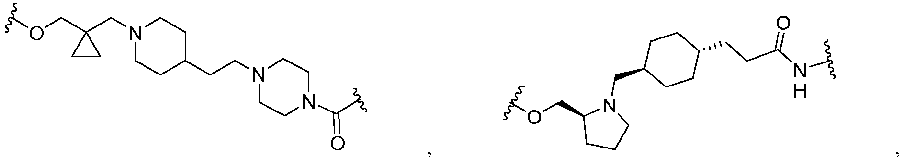

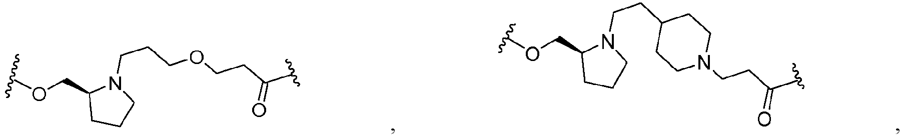

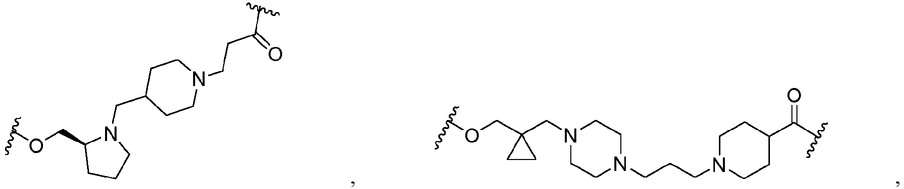

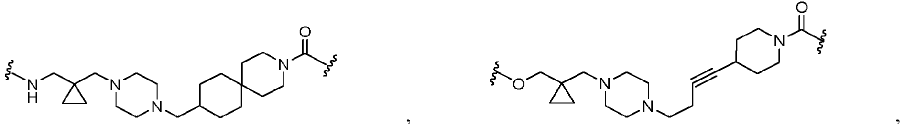

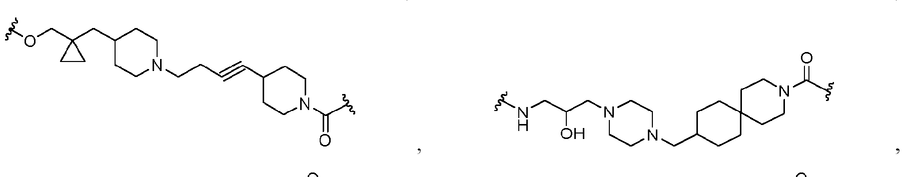

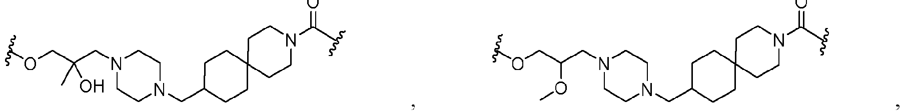

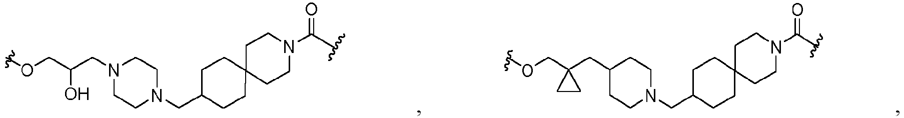

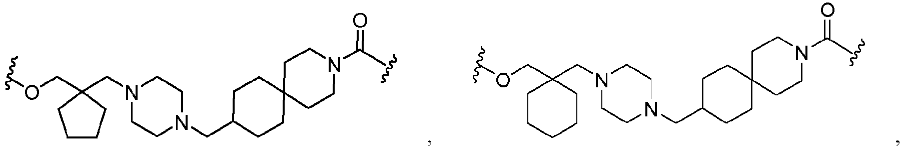

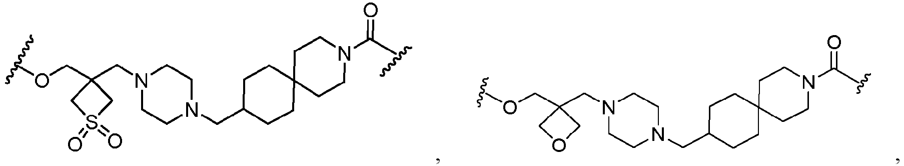

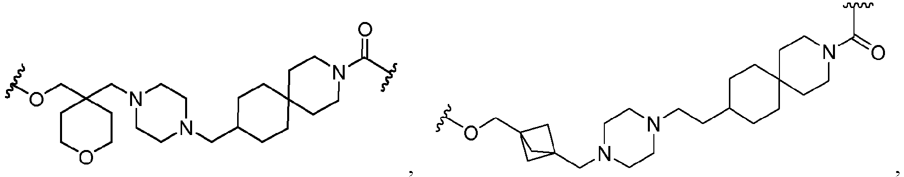

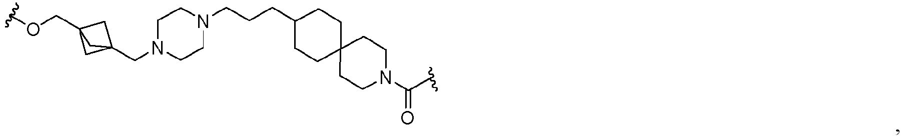

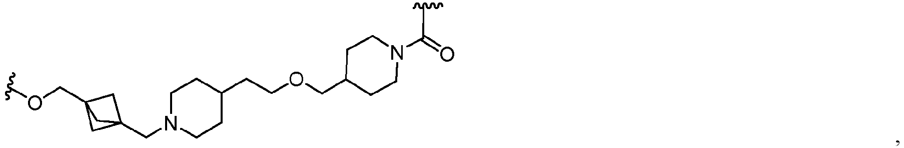

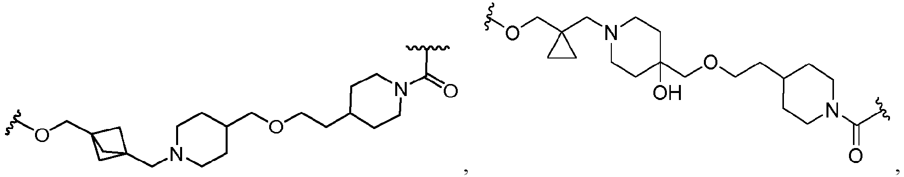

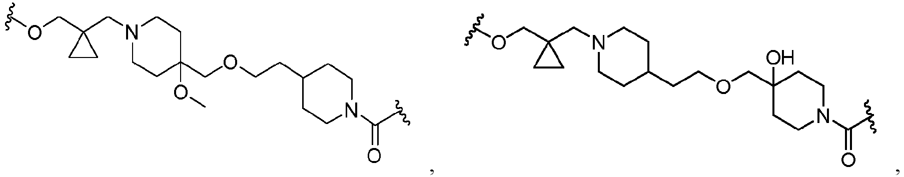

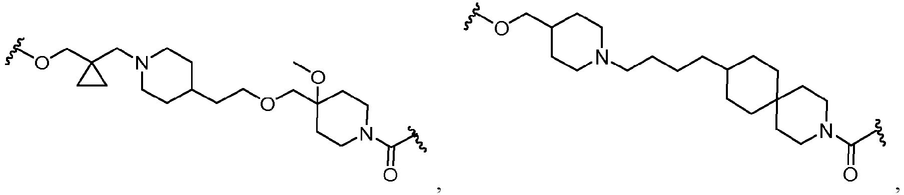

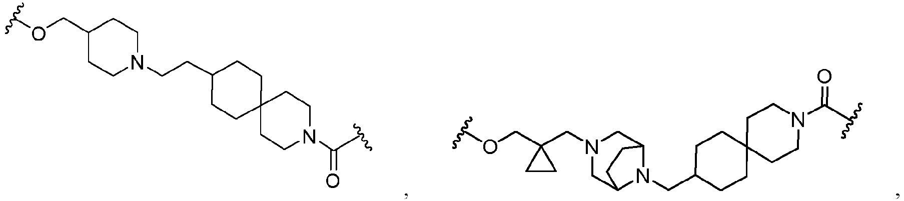

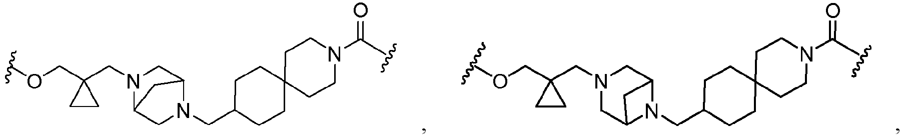

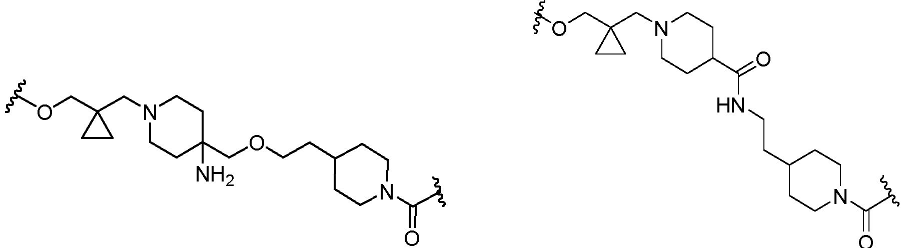

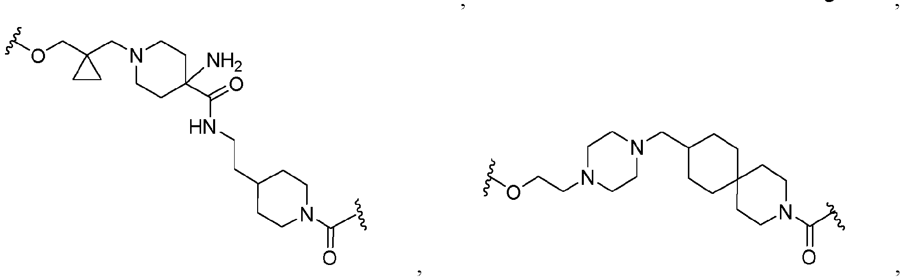

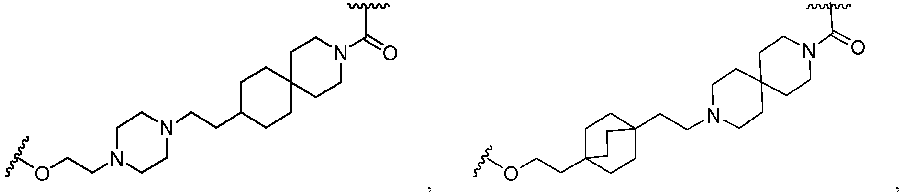

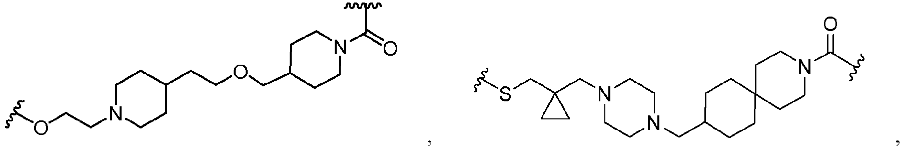

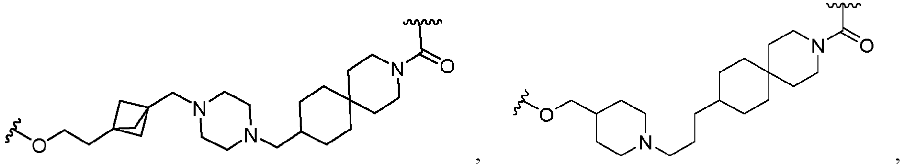

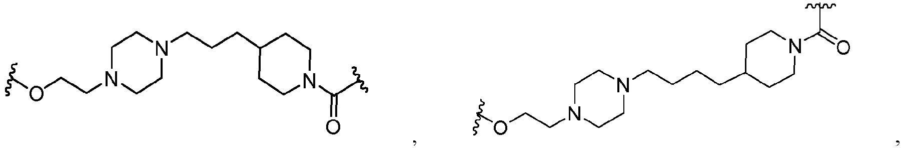

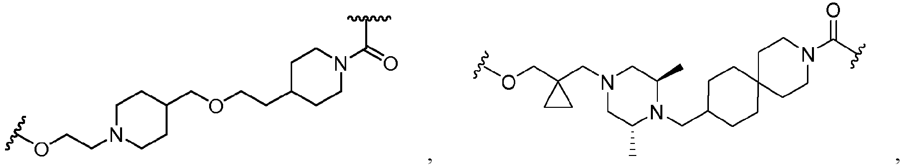

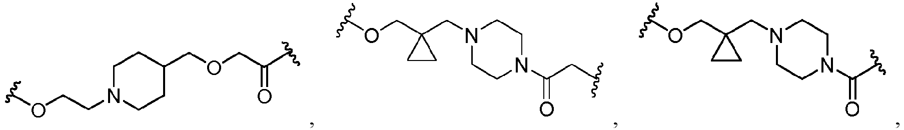

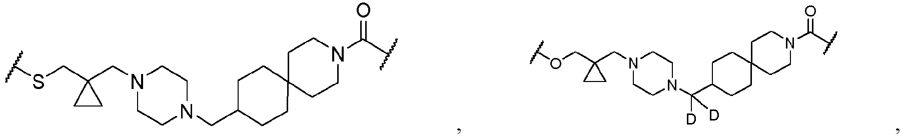

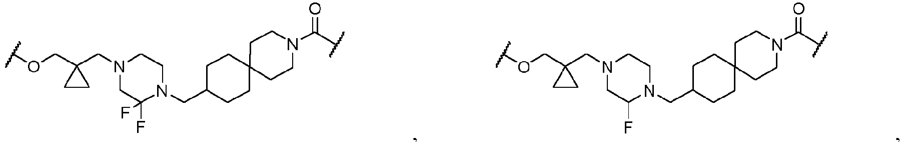

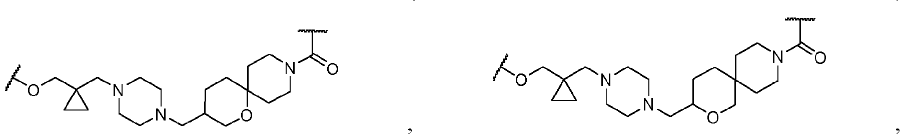

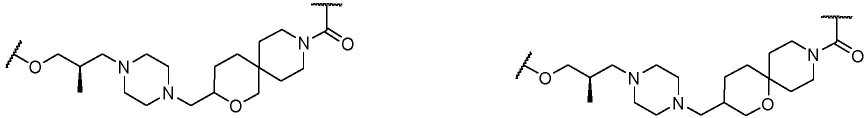

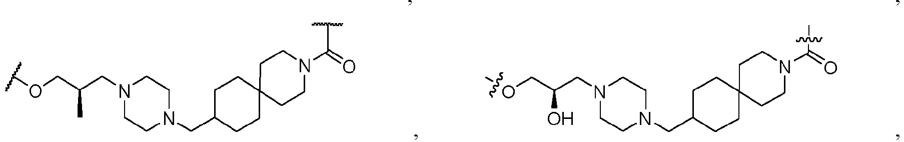

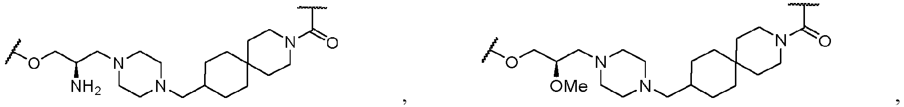

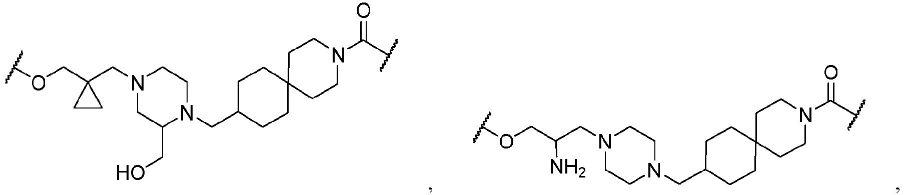

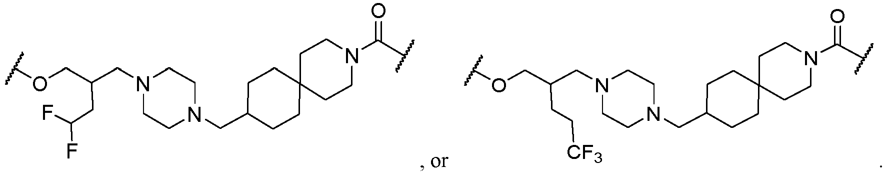

15. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1, wherein 1 or more of the following conditions are met:
(1) E1 has a structure of formula E1-1a, E1-1b, E1-1c, E1-1d, E1-1e, E1-1f, E1-1g, E1-1aa, E E1-1h, 1-1bb, E1-1cc, E1-1dd, E1-1ee, E1-1ff, E1-1gg, or E1-1hh: 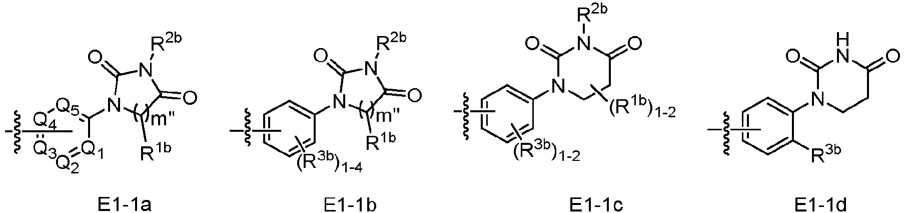 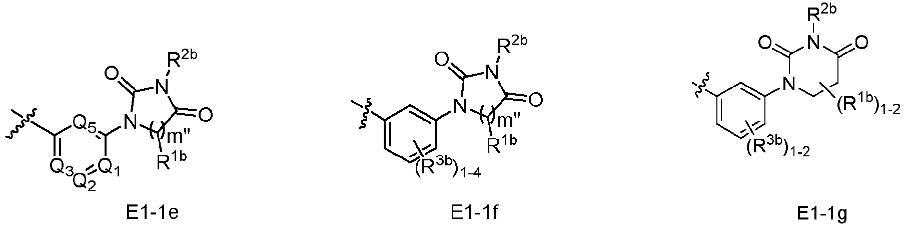 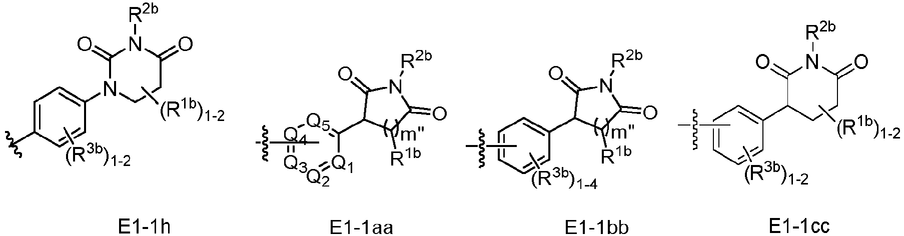 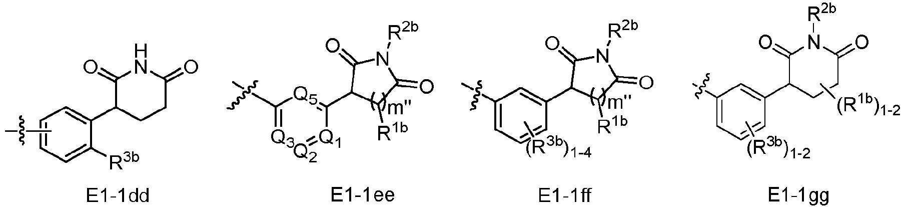 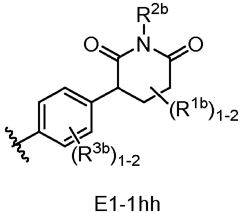 Q₁, Q₂, Q₃, Q₄, Q₅, R₁", R₂", R₃", and m" are as defined in claim 1 or 2;
(2) E2 has a structure of formula E2-1a, E2-1b, E2-1c, E2-1d, E2-1e, or E2-1f: 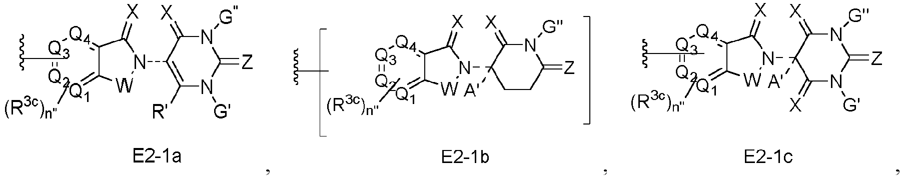 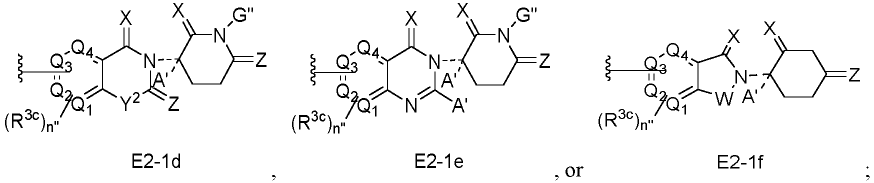 wherein in E2-1a, E2-1b, E2-1c, E2-1d, E2-1e, and E2-1f:
W is CR^{1c}R^{2c}, C(S), C(O), or SO₂;
X is CH₂, O, or S;
Y² is NH, -N-C₁-C₆ alkyl, -N-C₆-C₁₀ aryl, -N-(5- to 10-membered heteroaryl), -N-C₃-C₈ cycloalkyl, -N-(3-to 8-membered heterocycloalkyl), O, or S;
Z is CH₂, O, or S;
G" and G' are each independently selected from hydrogen, deuterium, C₁-C₆ alkyl, OH, C₃-C₆ cycloalkyl, - CH₂-heterocycloalkyl, and -CH₂-phenyl, wherein the C₁-C₆ alkyl, C₃-C₆ cycloalkyl, -CH₂-heterocycloalkyl,
or -CH₂-phenyl is optionally substituted with 1-3 groups selected from hydroxy, halogen, cyano, and amino;
Q₁, Q₂, Q₃, and Q₄ are each independently CR^{3b} or N; R^{3b} is as defined in claim 1 or 2;
A' is hydrogen, deuterium, C₁-C₆ alkyl, C₃-C₆ cycloalkyl, or halogen;
R^{1c}, R^{2c}, and R^{3c} are each independently selected from hydrogen, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"}, -OR_{'}, -NR_{'}R_{"}, -SR_{'}, -SO₂R_{'}, -SO₂NR_{'}R_{"}, -CR_{'}R_{"}, - CR_{'}NR_{'}R_{"}, aryl, heteroaryl, C₃-C₈ cycloalkyl, 3- to 8-membered heterocycloalkyl, -P(O)(OR_{'})R_{"}, -P(PO)R_{'}R_{"}, -OP(O)(OR_{'})R_{"}, -Cl, -F, -Br, -I, -CF₃, -CN, -NR_{'}SO₂NR_{'}R_{"}, -NR_{'}C(O)NR_{'}R_{"}, -C(O)NR_{'}C(O)R_{"}, -NR_{'}C(=N-CN)NR_{'}R_{"}, -C(=N-CN)NR_{'}R_{"}, -NR_{'}C(=N-CN)R_{"}, -NR_{'}C(=C-NO₂)NR_{'}R_{"}, -SO₂NR_{'}COR_{"}, -NO₂, -COR_{'}, - C(C=N-OR_{'})R_{"}, -CR_{'}=CR_{'}R_{"}, -CCR_{'}, -S(C=O)(C=N-R_{'})R_{"}, -SF₅, and -OCF₃;
R_{'} and R_{"} are each independently a bond, hydrogen, deuterium, C₁-C₆ alkyl, C₃-C₈ cycloalkyl, C₆-C₁₀ aryl, 5- to 10-membered heteroaryl, or 3- to 8-membered heterocycloalkyl;
n^{"} is 0, 1, 2, or 3;
----- is a bond, which may be an *R* stereoisomer, an *S* stereoisomer, or a non-stereoisomer;
(3) E3 has a structure of formula E3-1: 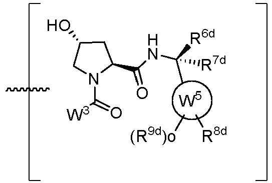
W³ is C₆-C₁₀ aryl, 5- to 10-membered heteroaryl, or 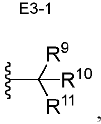 wherein the aryl or heteroaryl is optionally substituted;
R⁹ and R¹⁰ are each independently selected from hydrogen, C₁-C₆ alkyl, C₃-C₈ cycloalkyl, and 5- to 10-membered heteroaryl, wherein the C₁-C₆ alkyl, C₃-C₈ cycloalkyl, or 5- to 10-membered heteroaryl is optionally substituted with 1 or more -OH, halogen, or -NH₂;
or R⁹ and R¹⁰, together with the carbon atom linked thereto, form C₃-C₈ cycloalkyl, wherein the C₃-C₈ cycloalkyl is optionally substituted with -OH, halogen, -NH₂, or C₁-C₃ alkyl;
R¹¹ is selected from C₁-C₆ alkyl, C₁-C₆ alkoxy, 3- to 8-membered heterocycloalkyl, C₆-C₁₀ aryl, 5- to 10-membered heteroaryl, and 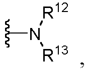 wherein the C₁-C₆ alkyl, C₁-C₆ alkoxy, 3- to 8-membered heterocycloalkyl, C₆-C₁₀ aryl, or 5- to 10-membered heteroaryl is optionally substituted with 1 or more -OH, halogen, or -NH₂;
R¹² is selected from H, C(O), and substituted alkyl;
R¹³ is selected from H, C₁-C₆ alkyl, -alkyl CO-, -(cycloalkyl)alkyl CO-, -aralkyl CO-, -aryl CO-, - (heterocycloalkyl)CO-, and arylalkyl, wherein the alkyl, -alkyl CO-, -(cycloalkyl)alkyl CO-, -aralkyl CO-, - aryl CO-, -(heterocycloalkyl)CO-, or arylalkyl is optionally substituted;
R¹⁶ is H, halogen, -OH, C₁-C₆ alkyl, or C₁-C₆ alkoxy, wherein the C₁-C₆ alkyl or C₁-C₆ alkoxy is optionally substituted with halogen;
o is 1, 2, 3, or 4;
R^{8d}, R^{14a}, R^{14b}, and R¹⁵ are as defined in claim 1 or 2.

16. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 15, wherein 1 or more of the following conditions are met:
(1) E1 has a structure of formula E1-1h", E1-1i', E1-1j', or E1-1h"h": 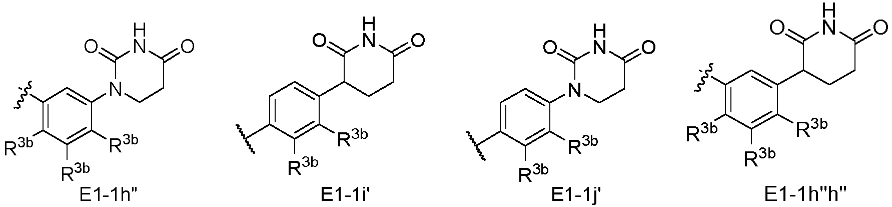
wherein R^{3b} is as defined in claim 1 or 2;
preferably, R^{3b} is independently hydrogen, halogen, cyano, -OH, -NH₂, C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl);
the C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, or -S(C₁-C₆ alkyl) is optionally substituted with 1-3 halogen, cyano, -OH, or -NH₂;
or the C₁-C₆ alkyl, C₃-C₈ cycloalkyl, 3- to 8-membered heterocycloalkyl, -O(C₁-C₆ alkyl), -O(C₃-C₈ cycloalkyl), -O(3- to 8-membered heterocycloalkyl), -N(C₁-C₆ alkyl)₁₋₂, -N(C₃-C₈ cycloalkyl)₁₋₂, and -S(C₁-C₆ alkyl) are also optionally substituted with 1, 2, or 3 deuterium;
(2) E2 has a structure of formula E2-1bb, E2-1aa, or E2-1cc: 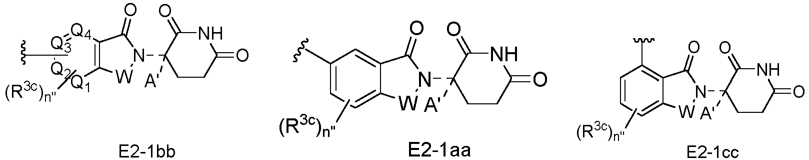 wherein in E2-1bb:
Q₁, Q₂, Q₃, and Q₄ are each independently CR^{3b} or N;
W is C(O) or CH₂;
A' is hydrogen, deuterium, C₁-C₆ alkyl, or halogen;
R^{3c} is hydrogen, deuterium, hydroxy, halogen, -NH₂, -N(C₁-C₆ alkyl)₁₋₂, C₁-C₆ alkyl, C₁-C₆ alkoxy, or C₁-C₆ haloalkyl;
n^{"} is 0, 1, 2, or 3;
----- is a bond, which may be an R stereoisomer, an S stereoisomer, or a non-stereoisomer;
wherein R^{3b} is as defined in claim 1 or 2; 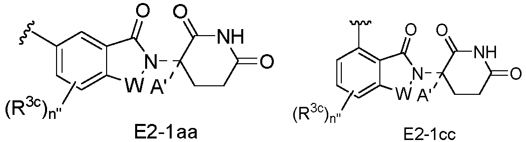
wherein in E2-1aa or E2-1cc:
W is CH₂ or C(O);
A' is hydrogen, methyl, Cl, or F;
each R^{3c} is independently hydrogen, halogen, cyano, hydroxy, NH₂, C₁-C₆ alkyl, or C₁-C₆ alkoxy;
n^{"} is 0, 1, 2, or 3;
----- is a bond, which may be an R stereoisomer, an S stereoisomer, or a non-stereoisomer;
(3) E3 has a structure of formula E3-1a, E3-1b, or E3-1c: 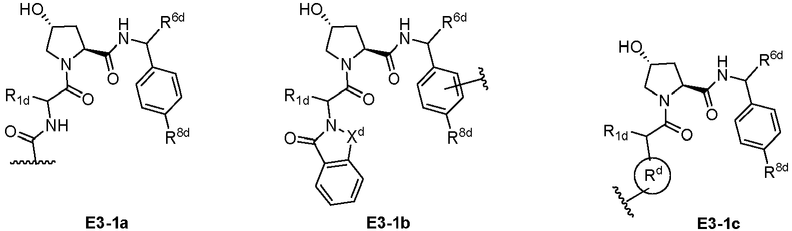 wherein,
R_{1d} is H, ethyl, isopropyl, *tert*-butyl, sec-butyl, cyclopropyl, cyclobutyl, cyclopentyl, cyclohexyl, alkyl, hydroxyalkyl, heteroaryl, or haloalkyl, wherein the alkyl, hydroxyalkyl, or heteroaryl is optionally substituted;
R^{6d} is H, -CH₃, -CH₂F, -CH₂OH, ethyl, isopropyl, or cyclopropyl;
R^{8d} is H, halogen, CN, OH, NO₂, C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl, wherein the C₆-C₁₀ aryl, 5- to 10-membered heteroaryl, C₁-C₆ alkyl, C₁-C₆ alkoxy, C₃-C₈ cycloalkyl, or 3- to 8-membered heterocycloalkyl is optionally substituted with halogen, -OH, CN, NO₂, or amino;
X^{d} is CH₂ or C(O);
R^{d} is 5- to 6-membered heteroaryl, wherein the 5- to 6-membered heteroaryl is optionally substituted; preferably, E3 has a structure of formula E3-1aa: 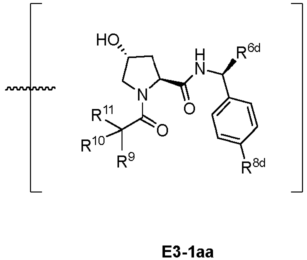
wherein,
R^{6d} is H, -CH₃, -CH₂F, -CH₂OH, ethyl, isopropyl, or cyclopropyl;
R⁹ is H;
R¹⁰ is H, ethyl, isopropyl, *tert*-butyl, sec-butyl, cyclopropyl, cyclobutyl, cyclopentyl, or cyclohexyl;
R¹¹ is 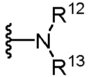
or 5- to 10-membered heteroaryl, wherein the 5- to 10-membered heteroaryl is optionally substituted with 1 or more -OH, halogen, or -NH₂;
R¹² is H or C(O);
R¹³ is H, alkyl, -alkyl CO-, -(cycloalkyl)alkyl CO-, -aralkyl CO-, -aryl CO-, -(heterocycloalkyl)CO-, or arylalkyl, wherein the alkyl, -alkyl CO-, -(cycloalkyl)alkyl CO-, -aralkyl CO-, -aryl CO-, - (heterocycloalkyl)CO-, or arylalkyl is optionally substituted;
R^{8d} is H, halogen, CN, OH, NO₂, nn, 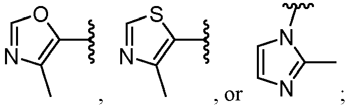
More preferably, formula E3-1aa is linked to L via R¹².

17. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 16, wherein 1 or more of the following conditions are met:
(1) E1 is: 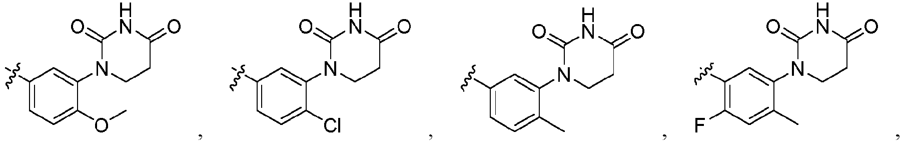 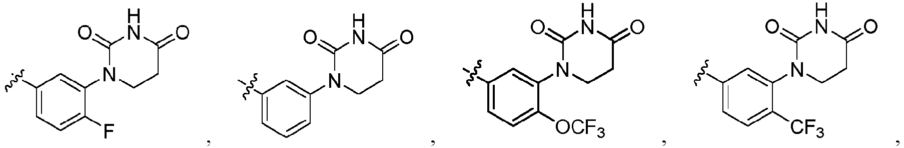 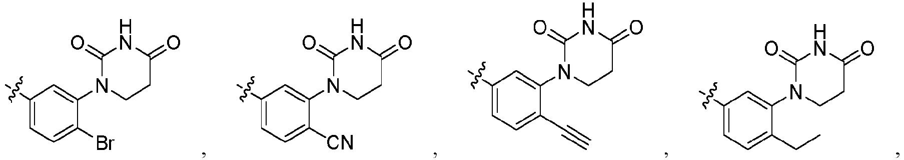 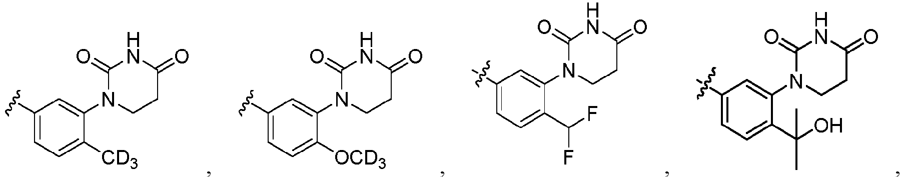 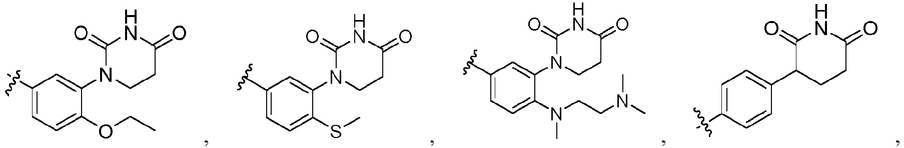 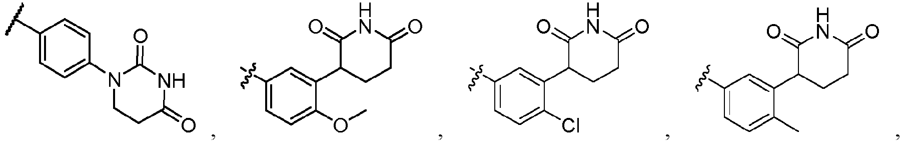 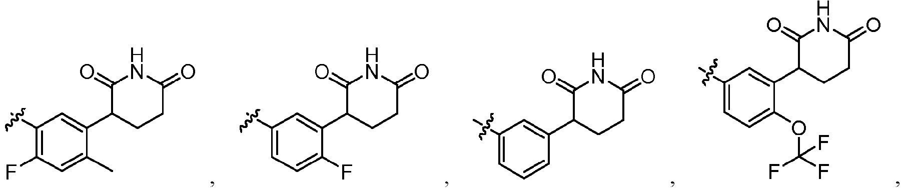 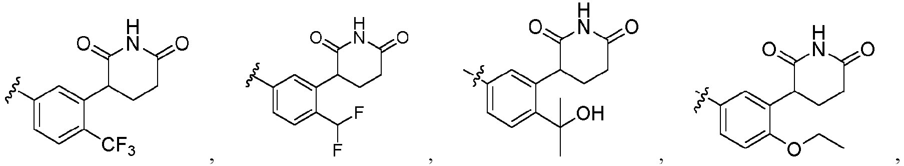 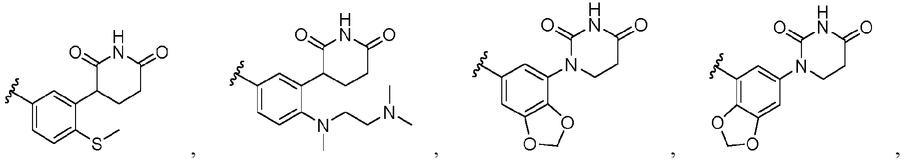 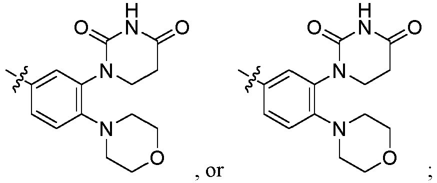
(2) in E, E2 is: 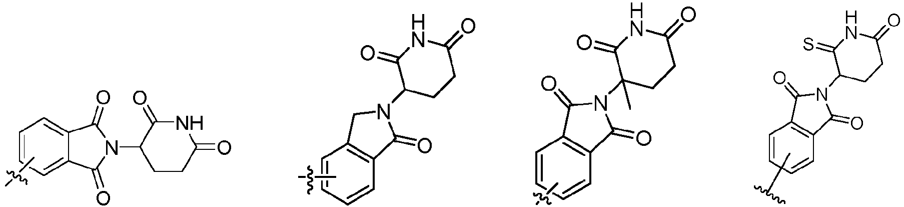 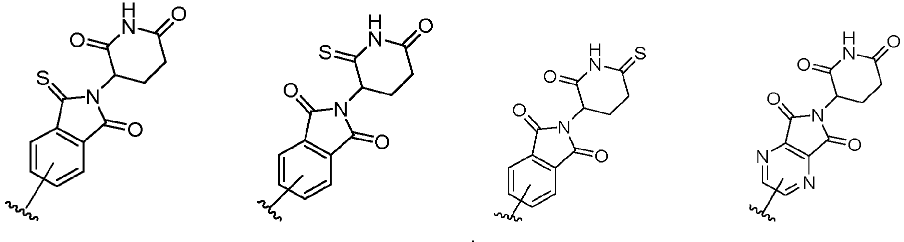 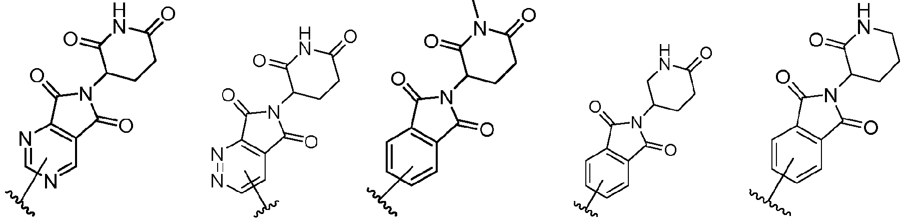 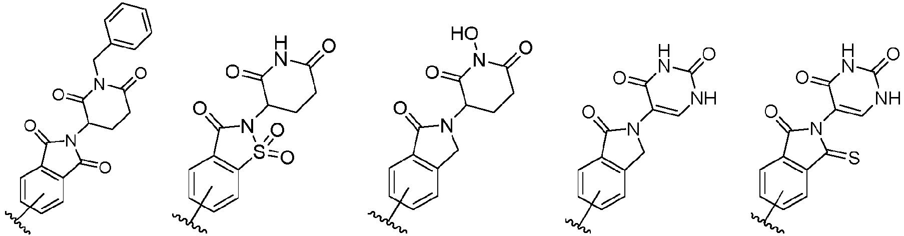 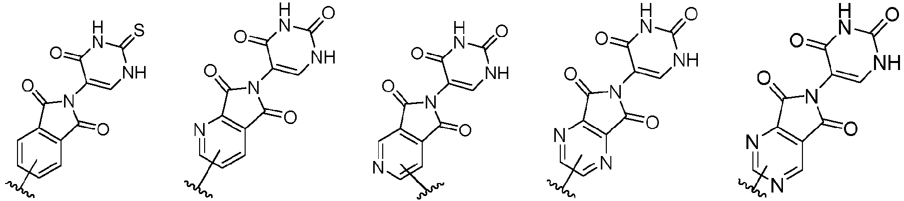 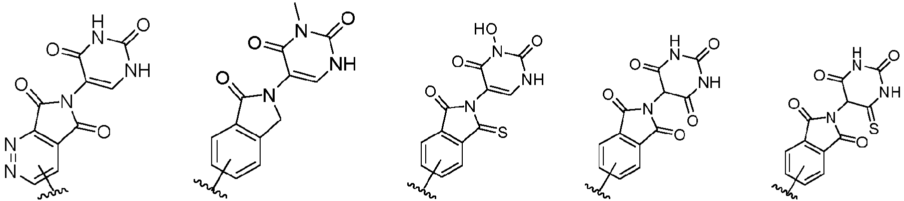 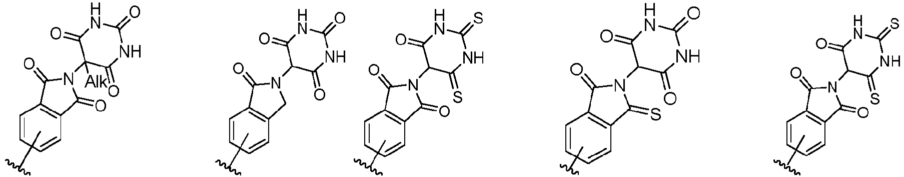 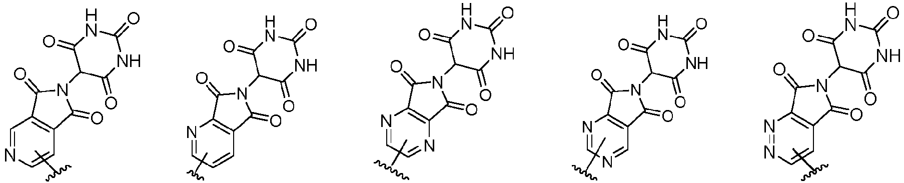 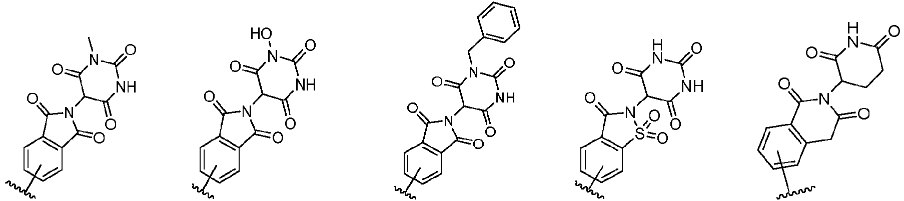 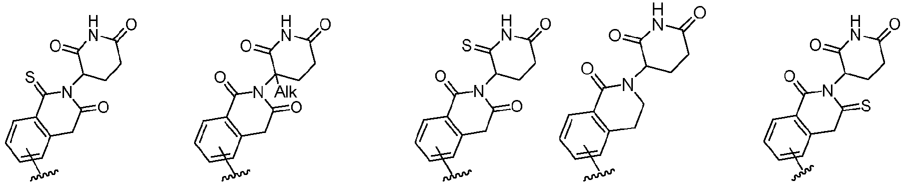 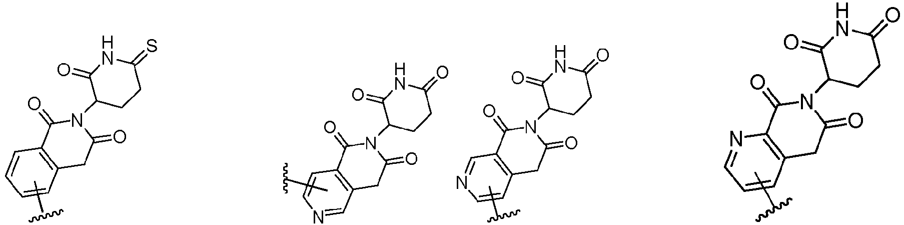 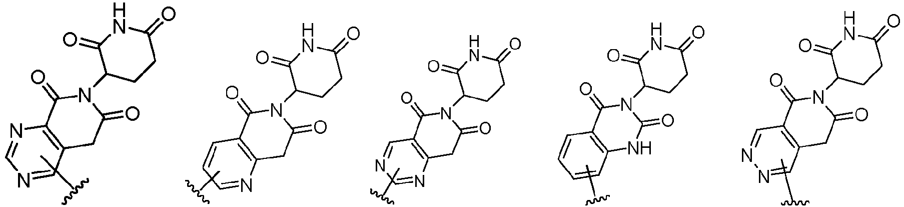 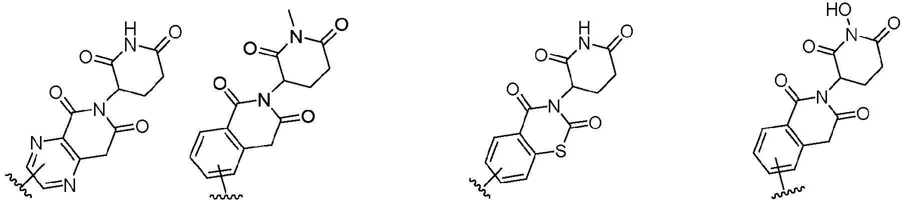 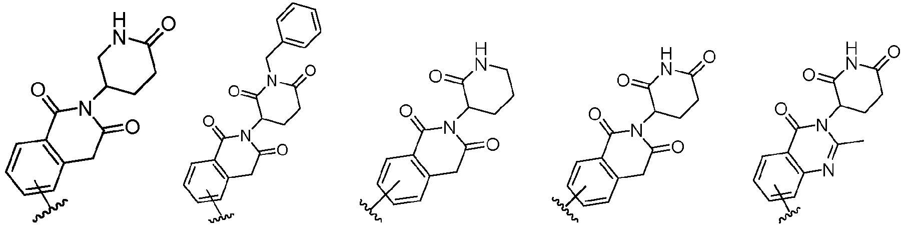 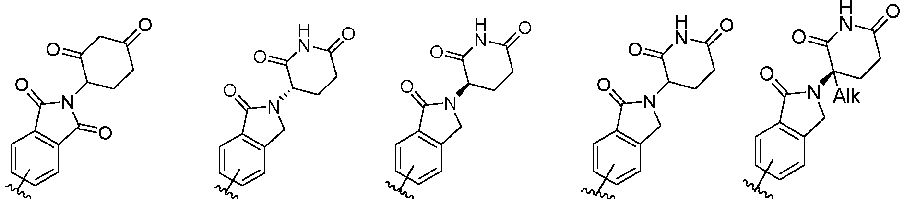 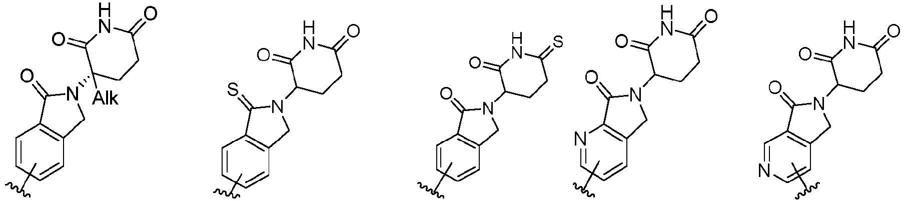 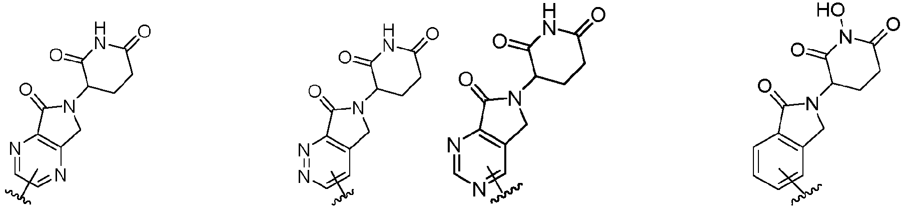 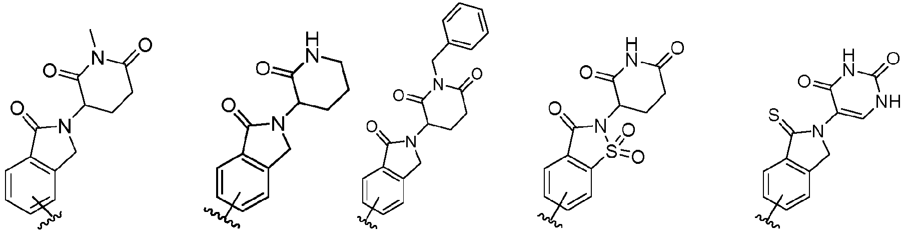 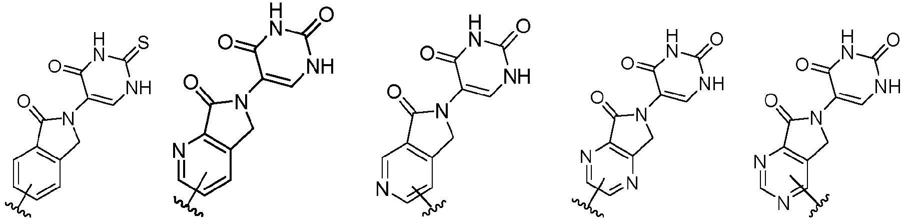 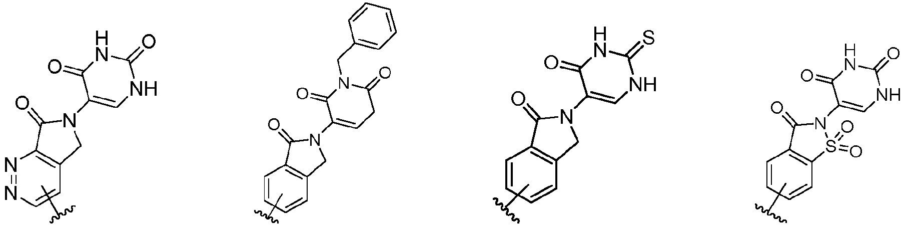 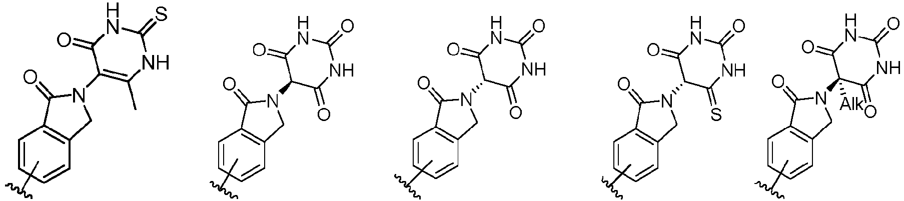 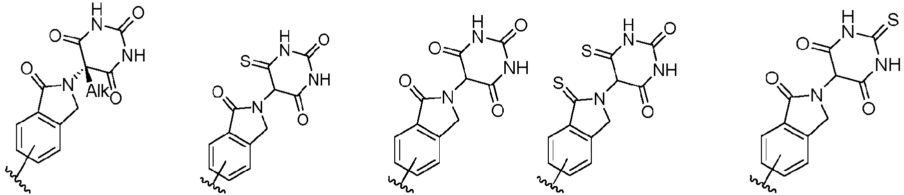 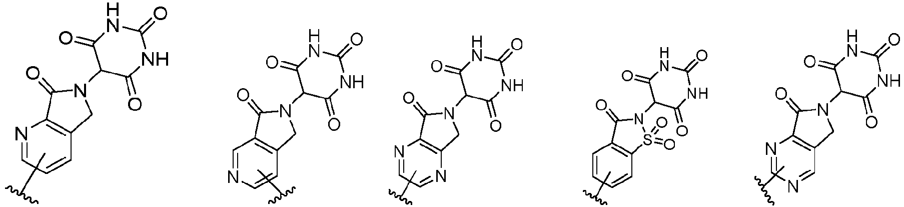 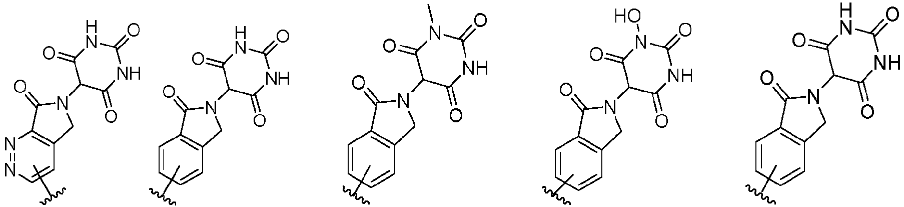 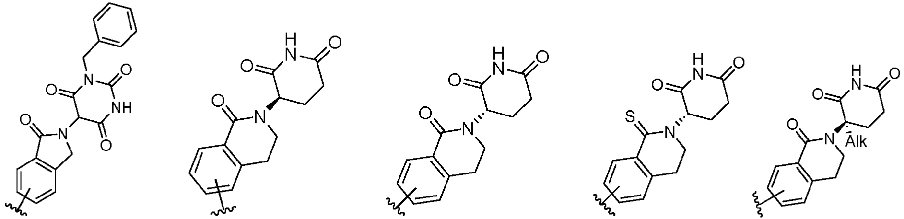 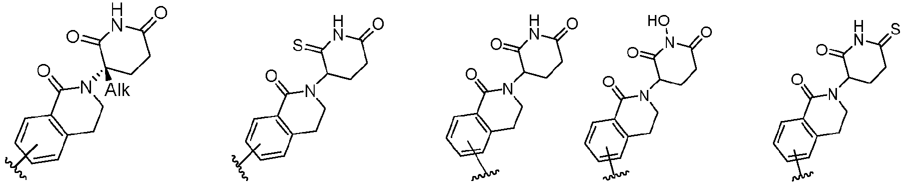 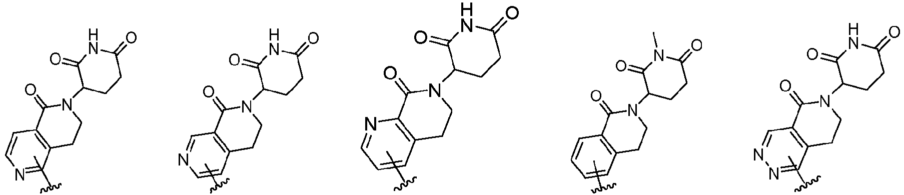 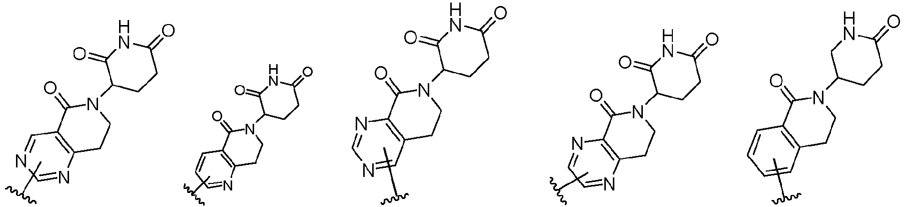 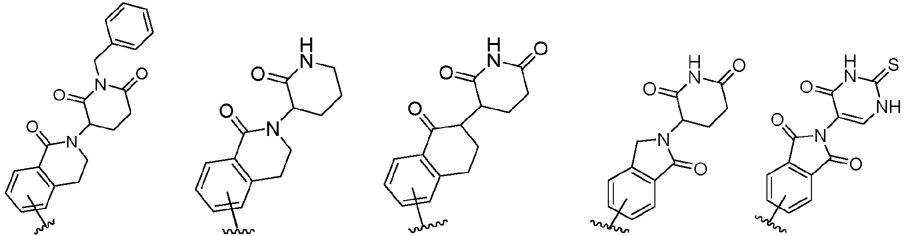 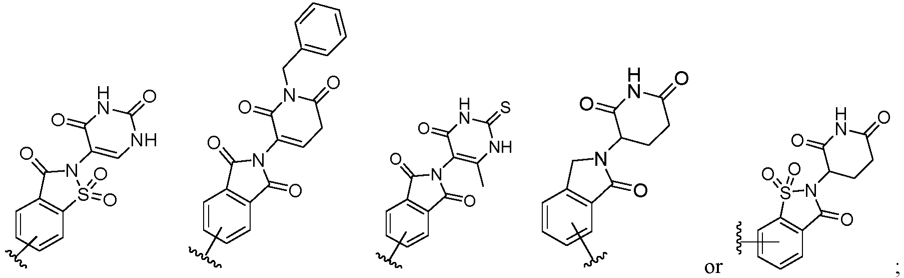
(3) E3 is: 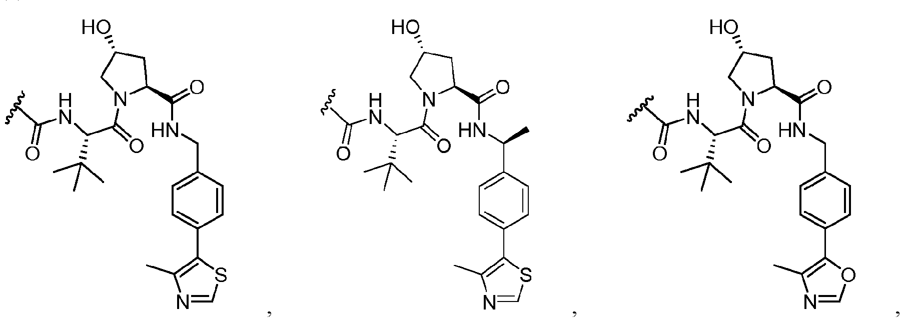 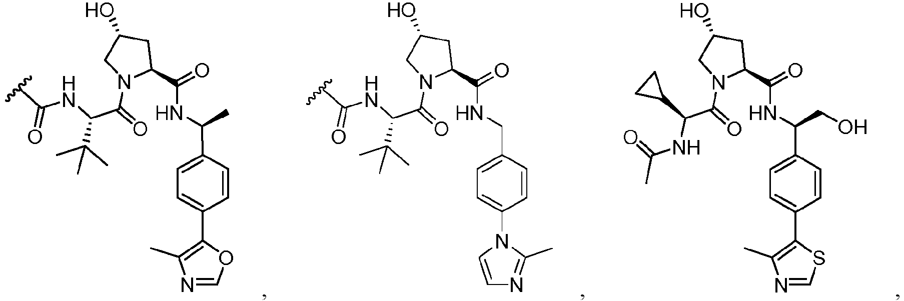 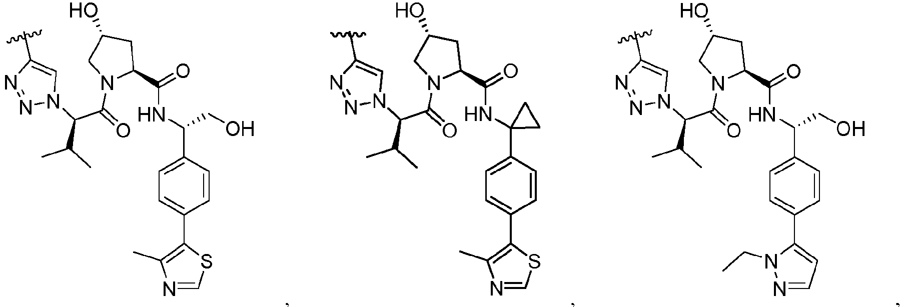 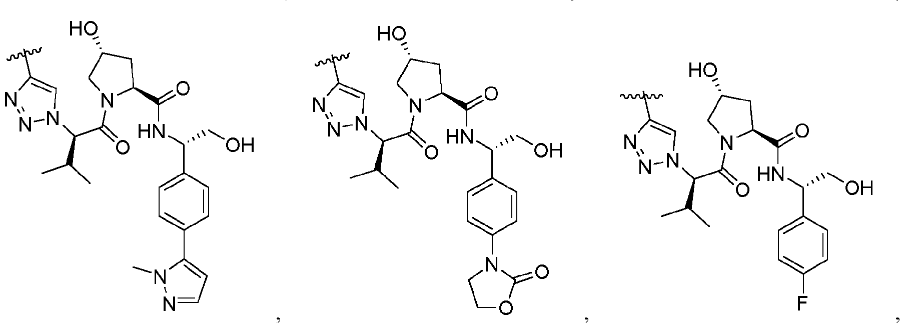 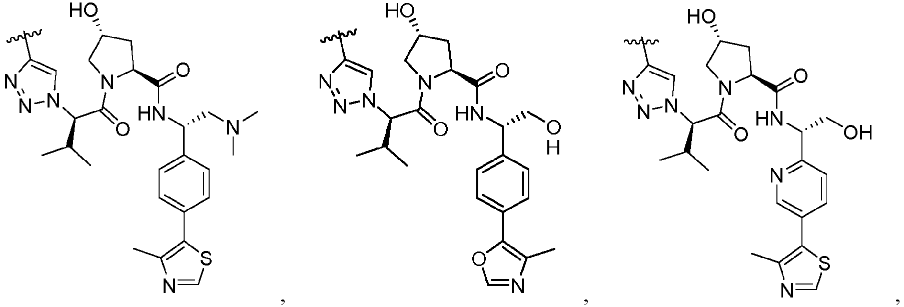 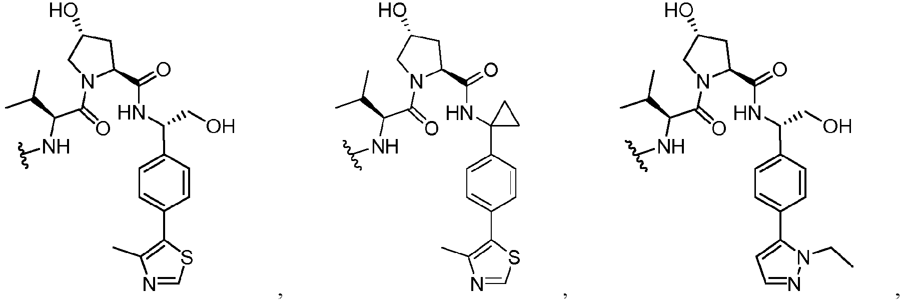 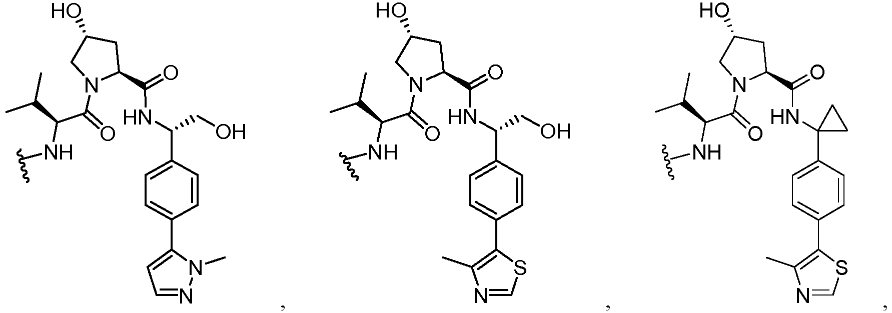 or

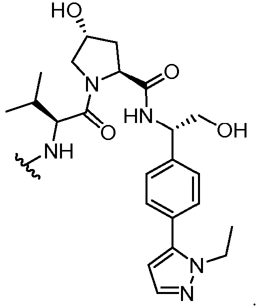

18. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, being scheme a, b, or c:
scheme a:
in the compound of formula I or I', G1 is 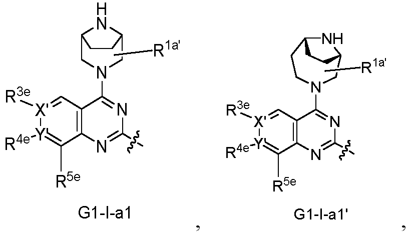 or 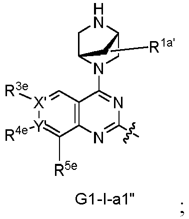
G1' 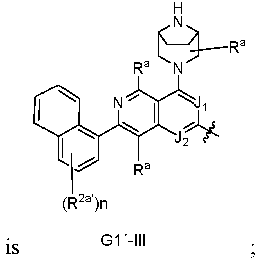 L is 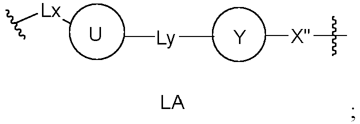
E is independently E1: 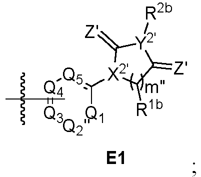
R^{1a}', R^{3e}, R^{4e}, R^{5e}*,* X', and Y' are as defined in claim 8 or 9; each R^{a}, R^{2a}', J₁, and J₂ are as defined in any one of claims 1-9; Lx, Ly, ring U, and ring Y are as defined in any one of claims 10-14; X", Q₁, Q₂, Q₃, Q₄, Q₅,
X^{2'}, Y^{2'} , R^{1b}, R^{2b}, Z', and m" are as defined in any one of claims 1-9;
scheme b:
in the compound of formula II or I', G1 is 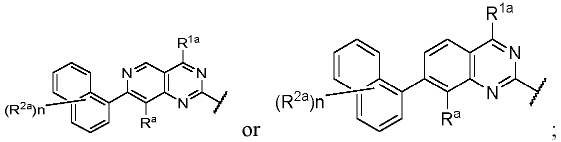
G1' is 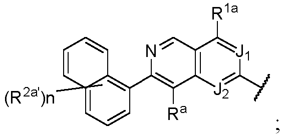
R^{1a} is 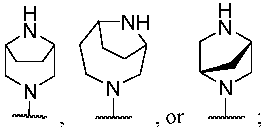
L is 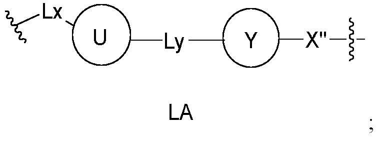
Lx is -(CH₂)ᵥ-, wherein two methylene groups in Lx are replaced by a group selected from -O- and -CR_{d}Rₑ-;
R_{d} and Rₑ are each independently H, C₁-C₆ alkyl, C₁-C₆ alkoxy, NH₂, or C₁-C₆ alkyl substituted with 1, 2, or
3 halogen, or R_{d} and Rₑ, together with the C atom linked thereto, form C₃-C₈ cycloalkyl;
v is 1, 2, 3, 4, 5, 6, or 7;
ring U is 3- to 12-membered heterocycloalkylene containing 1-2 N heteroatoms;
Ly is -(CH₂)ₖ-, wherein one or two methylene groups in Ly are optionally replaced by -O-; k is 1, 2, 3, 4, 5, 6, 7, or 8;
ring Y is 3- to 12-membered heterocycloalkylene containing 1, 2, or 3 heteroatoms selected from N and O;
X" is -C(O)-;
E is 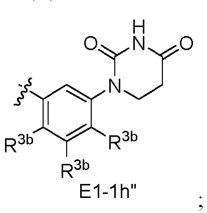
each R^{a}, R^{2a}, R^{2a}', J₁, J₂, and n are as defined in any one of claims 1-9; each R^{3b} is as defined in any one of claims 1-17;
preferably,
R^{2a} and R^{2a'} are each independently halogen, hydroxy, C₁-C₆ alkyl, or C₂-C₆ alkynyl; R^{a} is halogen;
L is of any one of the following structures: 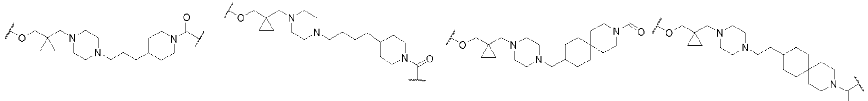 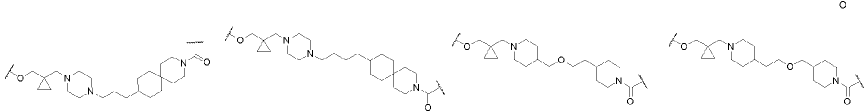 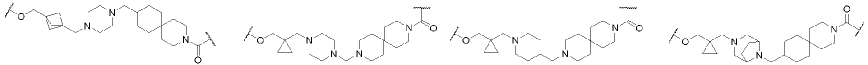 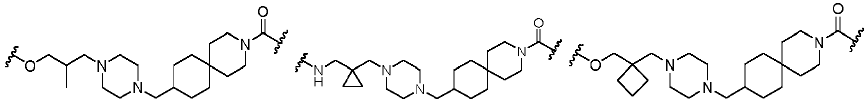 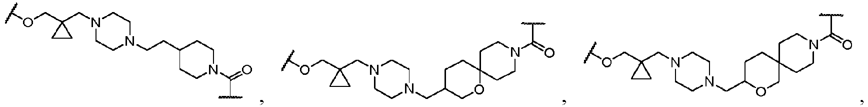 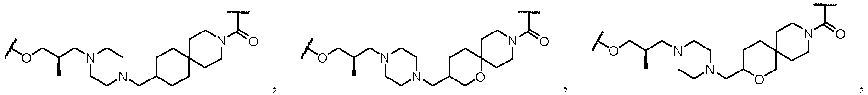 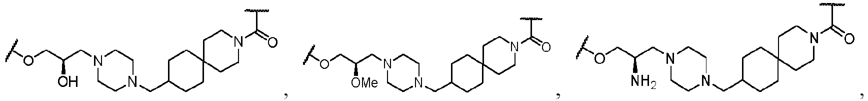 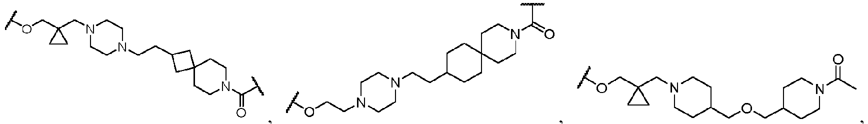 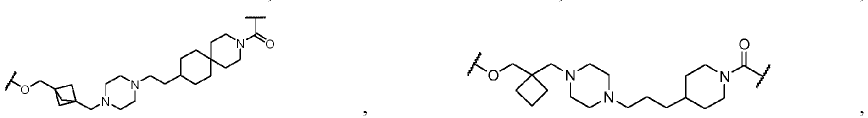 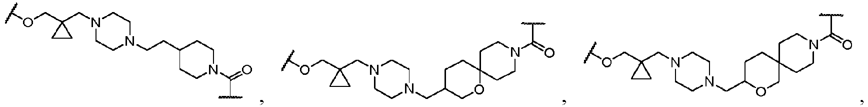 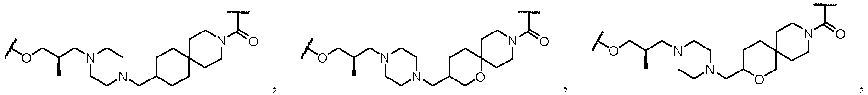 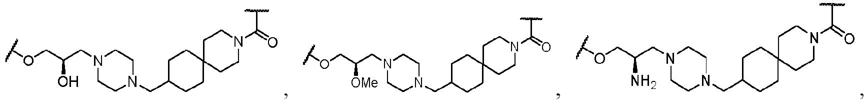 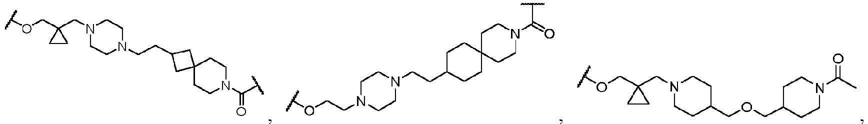 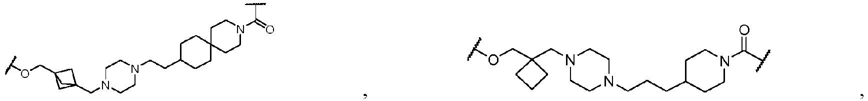 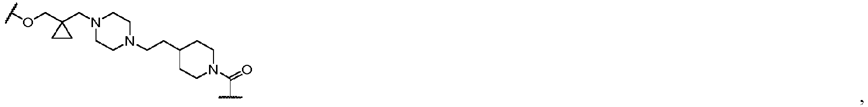 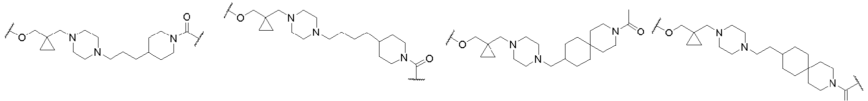 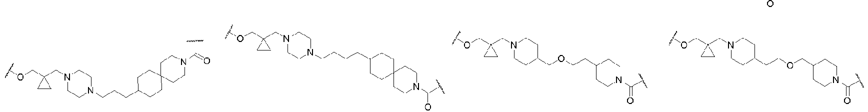 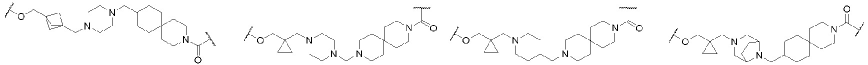 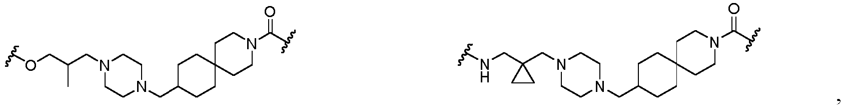 or 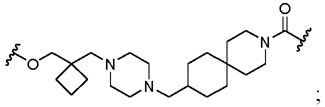
R^{3b} is halogen, C₁-C₆ alkyl, or -O-(C₁-C₆ alkyl), wherein the C₁-C₆ alkyl or -O-(C₁-C₆ alkyl) is optionally substituted with 1-3 groups independently selected from deuterium and halogen;
scheme c:
in the compound of formula II or I', G1 is 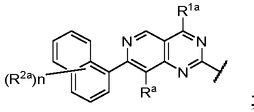 G1' is 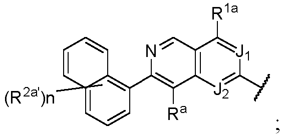
R^{1a} is 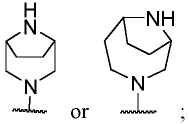
L is 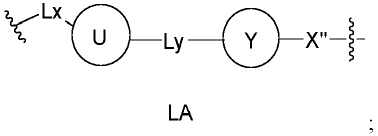
Lx is -(CH₂)ᵥ-, wherein two methylene groups in Lx are replaced by a group selected from -O- and -CR_{d}Rₑ-;
R_{d} and Rₑ are each independently C₁-C₆ alkyl, or R_{d} and Rₑ, together with the C atom linked thereto, form C₃-C₈ cycloalkyl;
v is 1, 2, 3, 4, 5, 6, or 7;
ring U is 3- to 12-membered heterocycloalkylene containing 1-2 N heteroatoms;
Ly is -(CH₂)ₖ-, wherein one or two methylene groups in Ly are optionally replaced by -O-; k is 1, 2, 3, 4, 5, 6, 7, or 8;
ring Y is 3- to 12-membered heterocycloalkylene containing 1-2 heteroatoms selected from N;
X" is -C(O)-;
E is 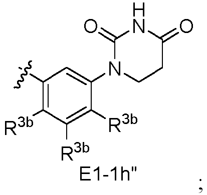
each R^{a}, R^{2a}, R^{2a}', J₁, J₂, and n are as defined in any one of claims 1-9; each R^{3b} is as defined in any one of claims 1-17;
preferably,
R^{2a} and R^{2a'} are each independently halogen, hydroxy, C₁-C₆ alkyl, or C₂-C₆ alkynyl; R^{a} is halogen;
L is of any one of the following structures: 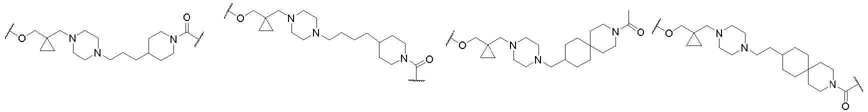 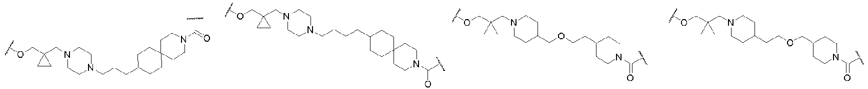 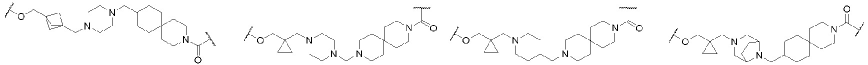 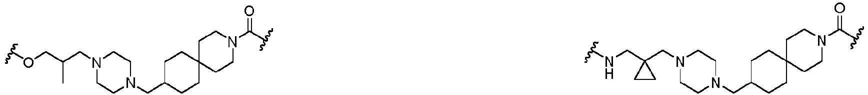 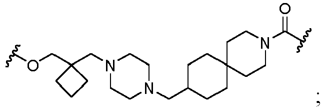
each R^{3b} is independently hydrogen, C₁-C₆ alkyl, or -O-(C₁-C₆ alkyl).

19. The compound of formula I or I', and/or the stereoisomer, the enantiomer, the diastereomer, the atropisomer, the deuteride, the hydrate, the solvate or the prodrug thereof and/or the pharmaceutically acceptable salt thereof according to claim 1 or 2, being any one of the following compounds:

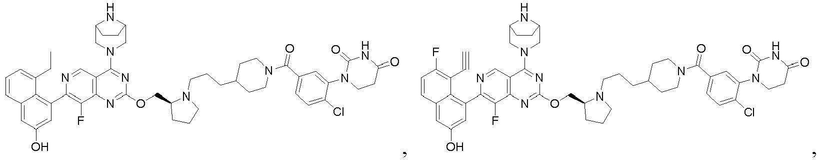

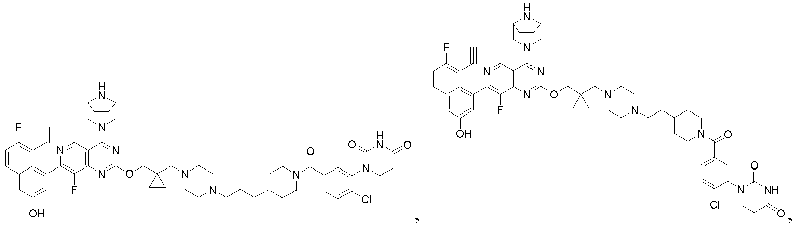

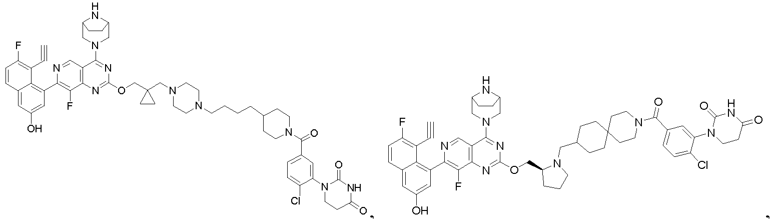

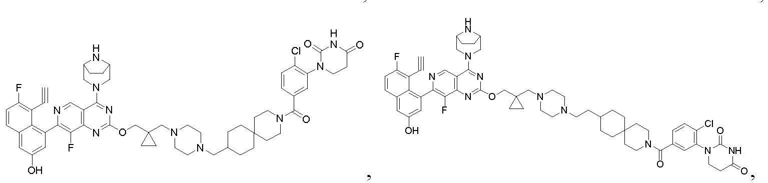

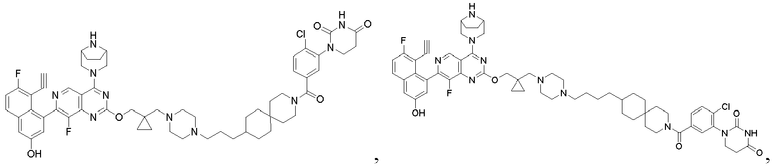

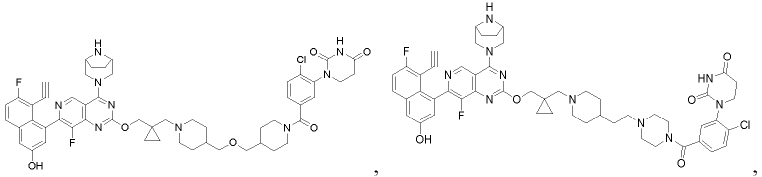

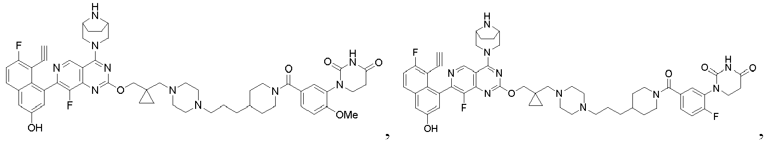

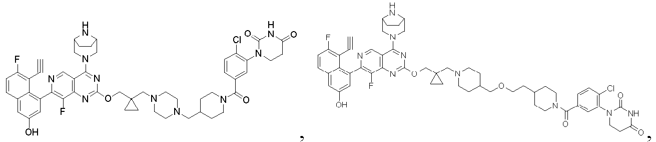

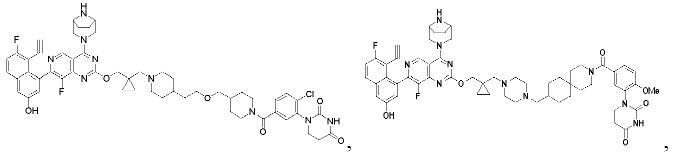

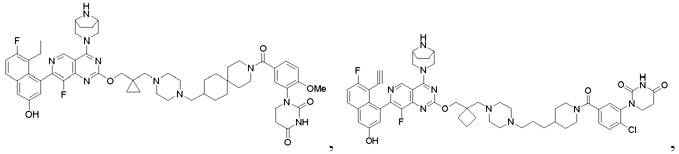

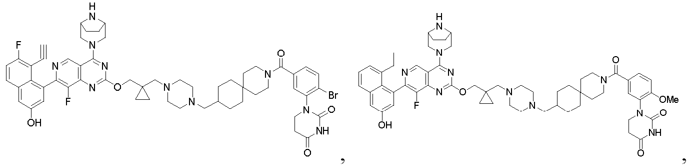

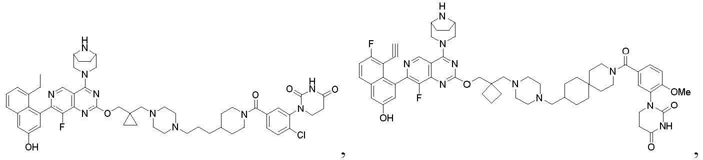

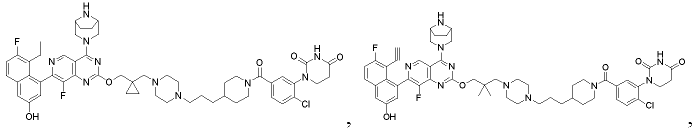

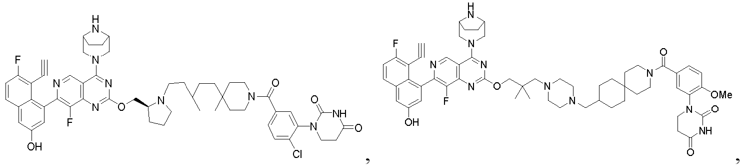

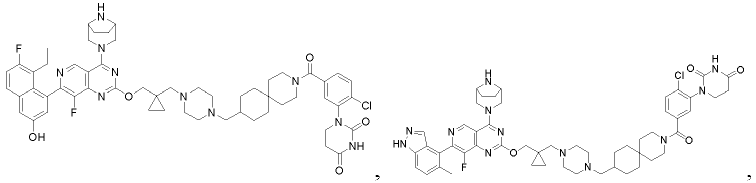

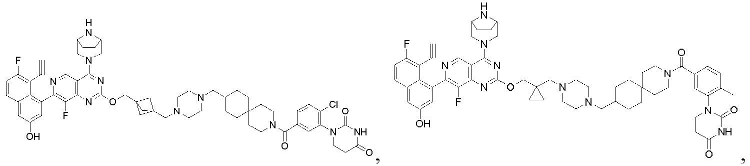

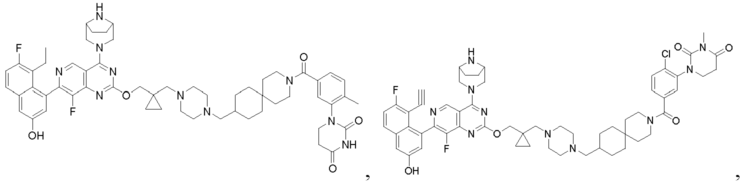

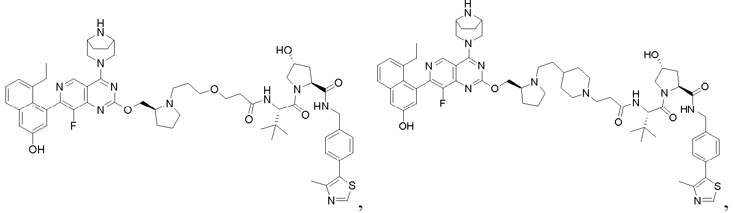

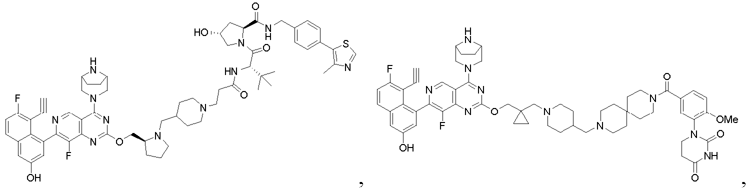

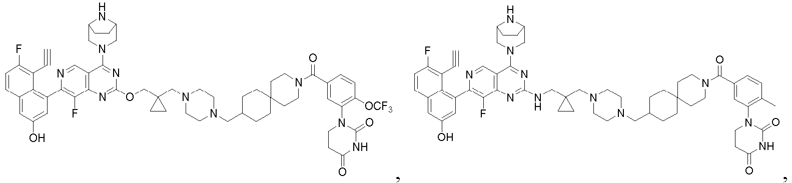

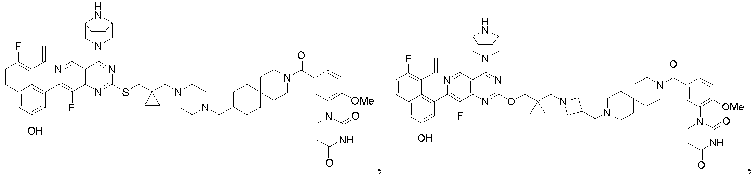

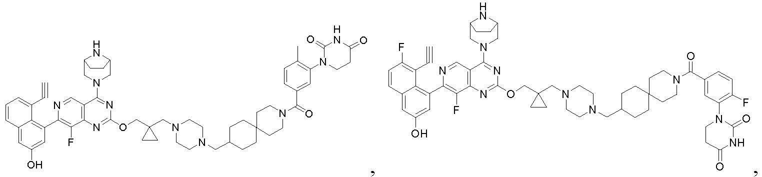

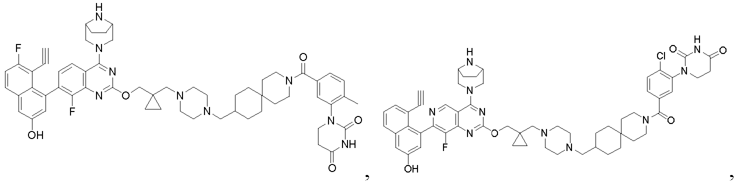

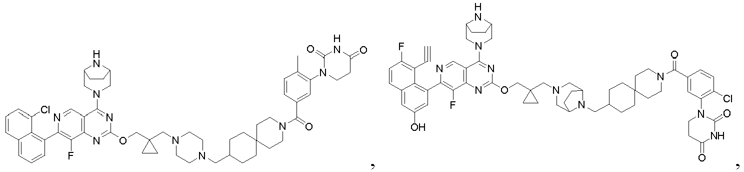

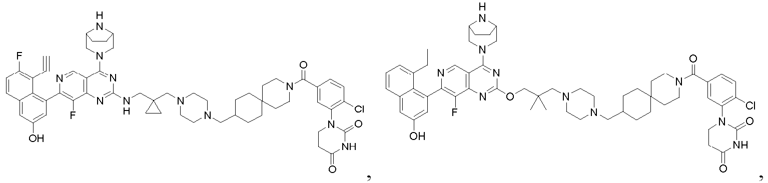

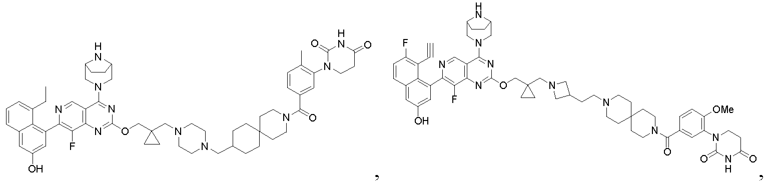

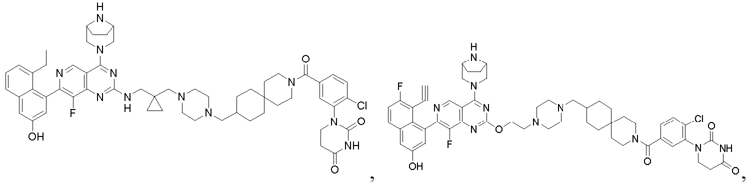

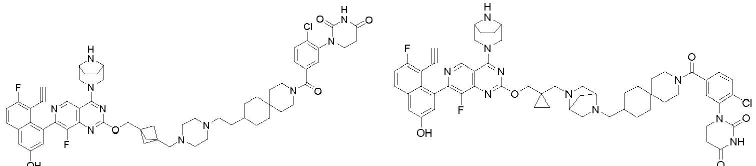

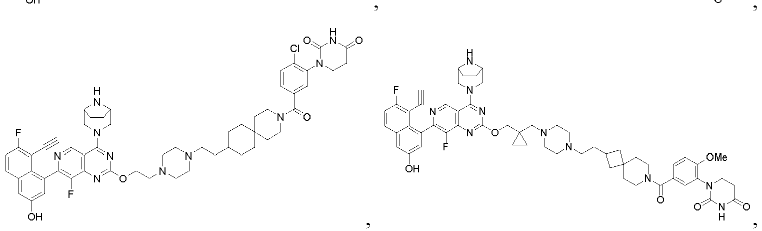

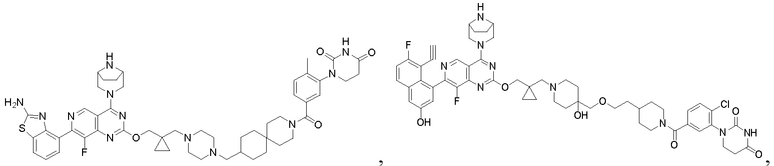

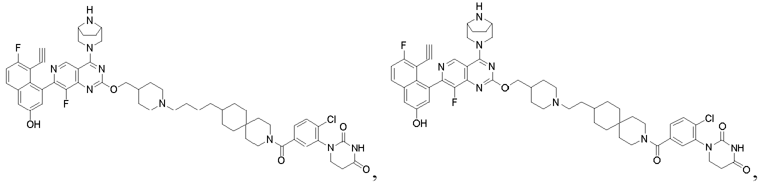

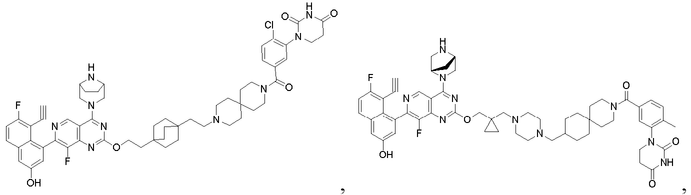

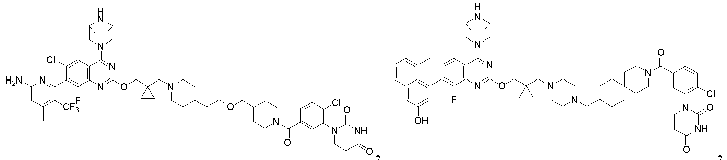

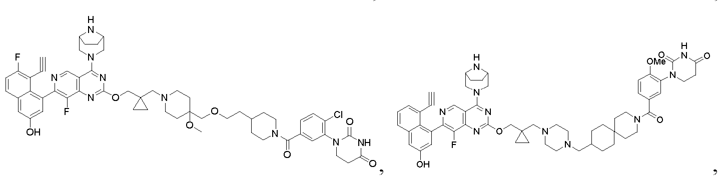

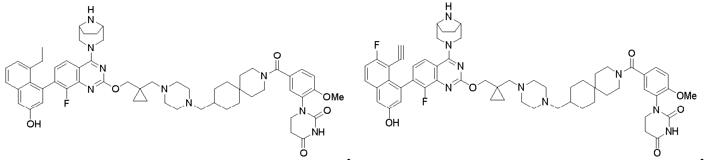

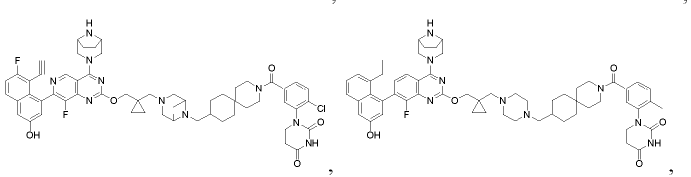

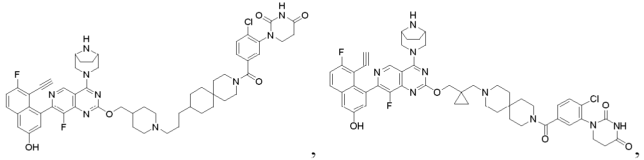

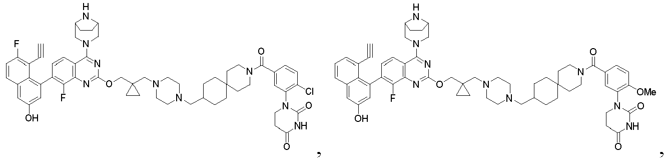

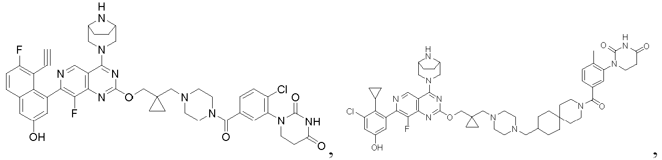

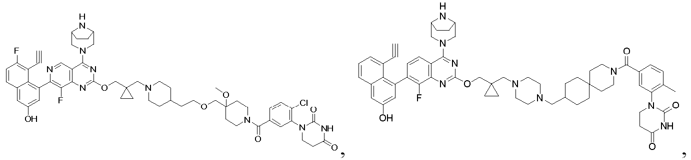

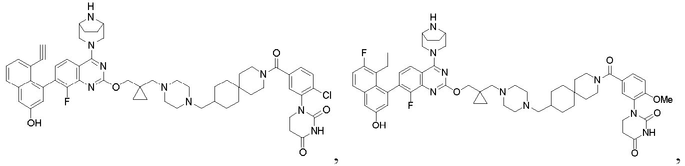

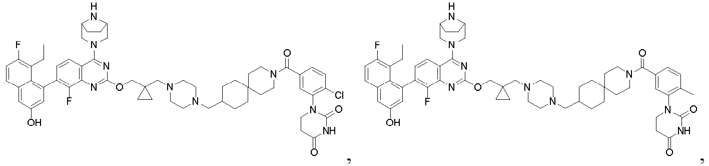

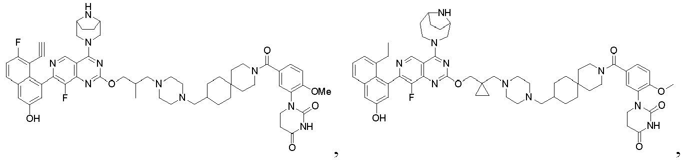

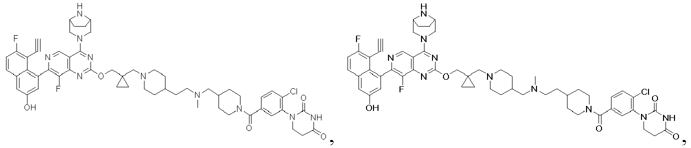

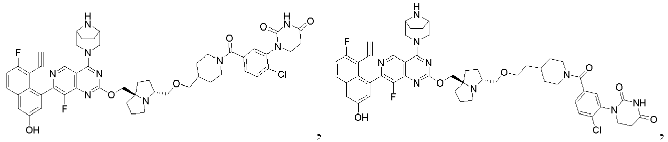

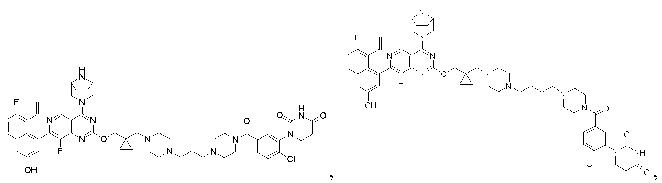

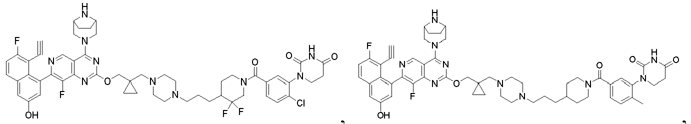

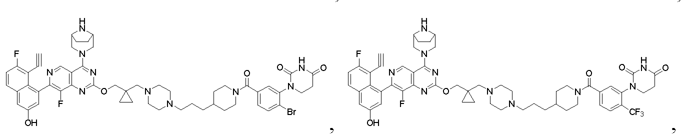

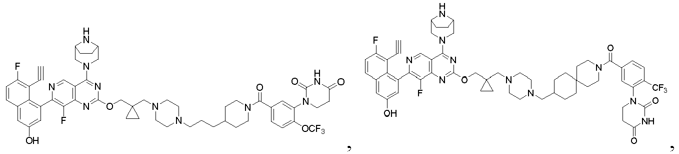

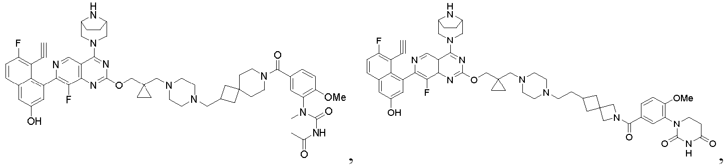

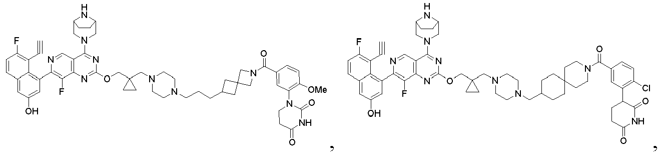

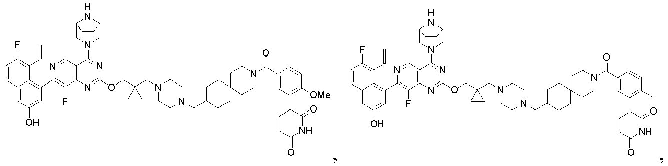

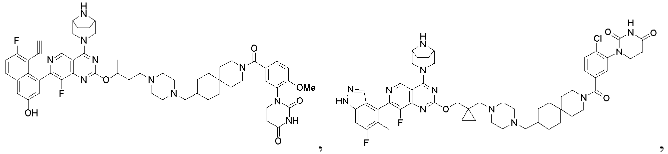

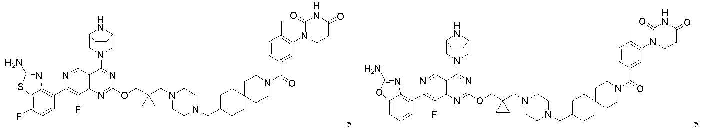

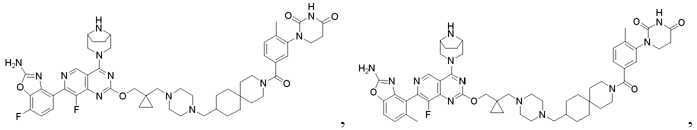

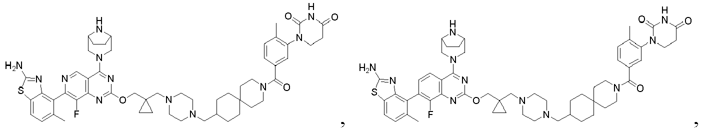

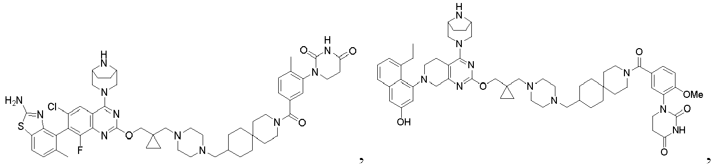

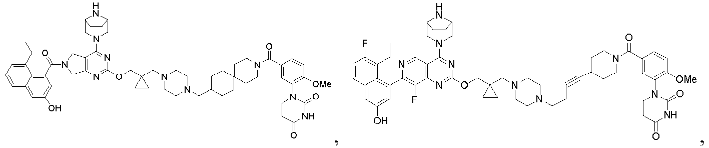

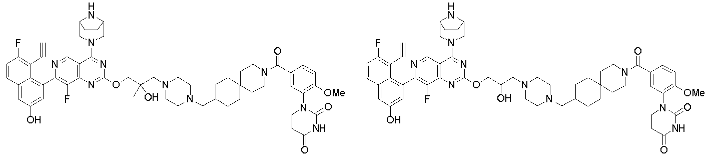

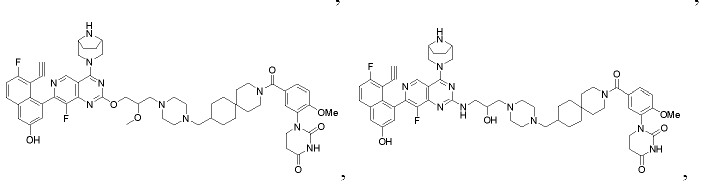

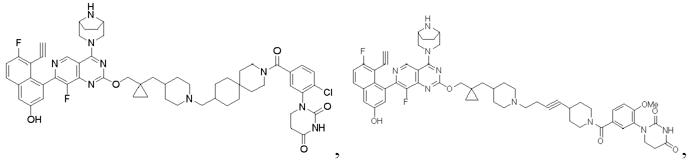

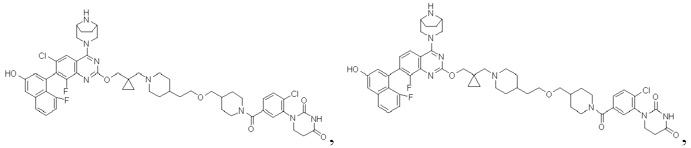

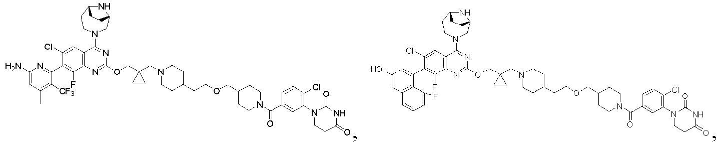

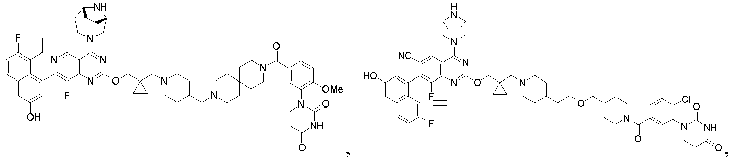

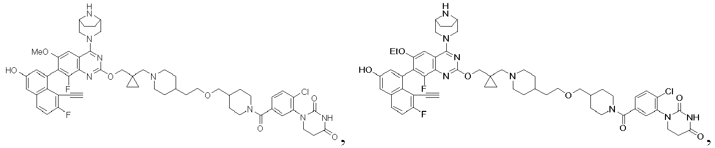

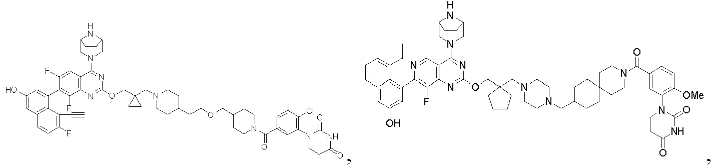

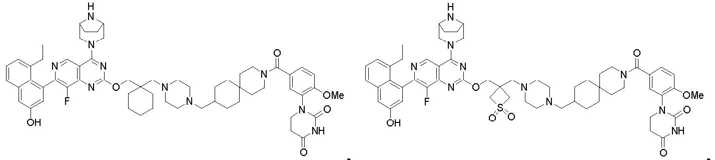

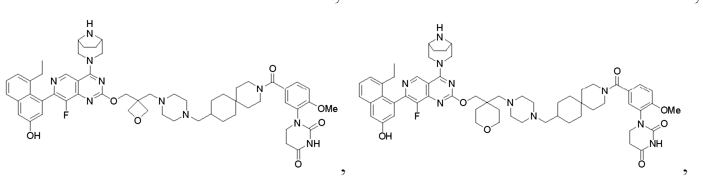

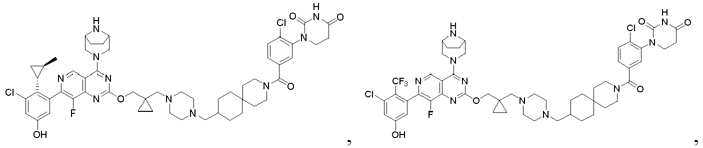

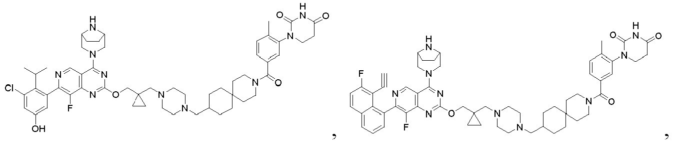

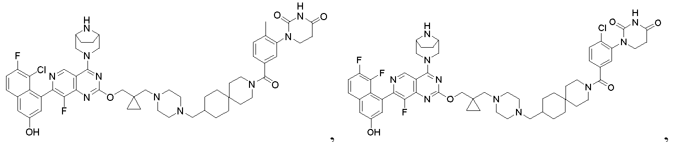

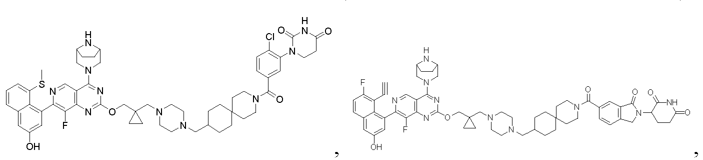

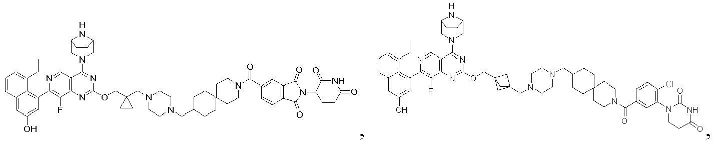

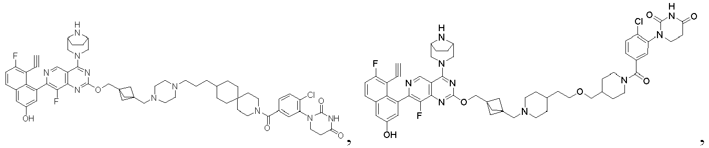

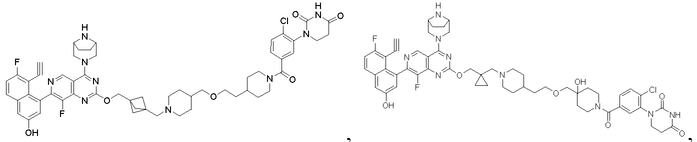

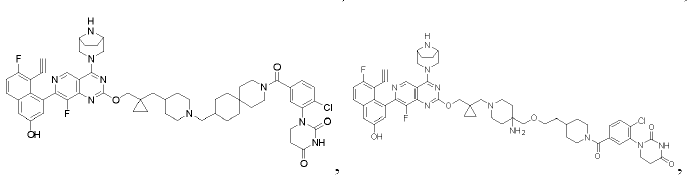

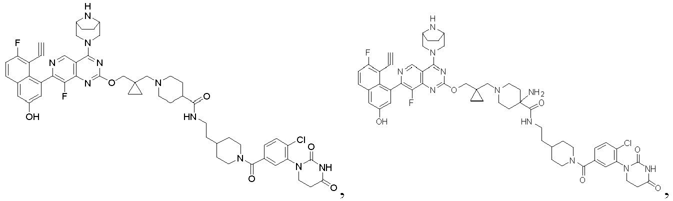

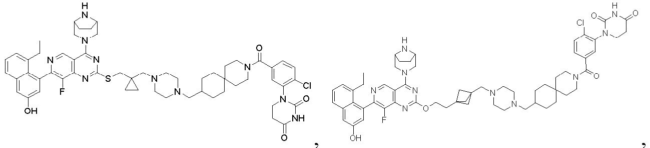

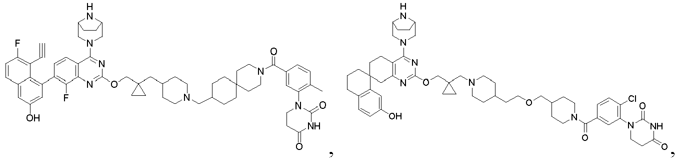

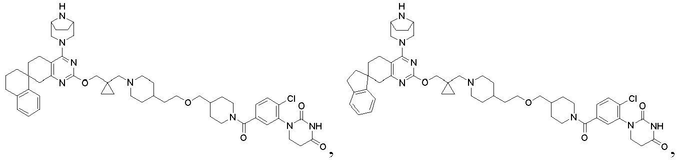

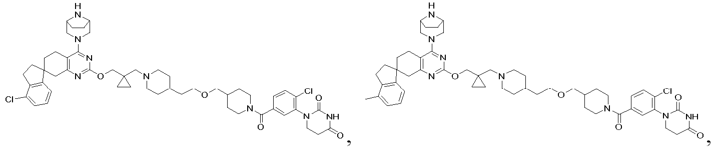

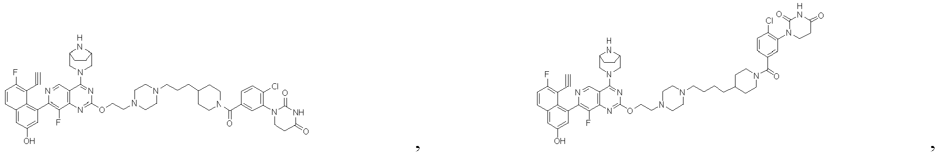

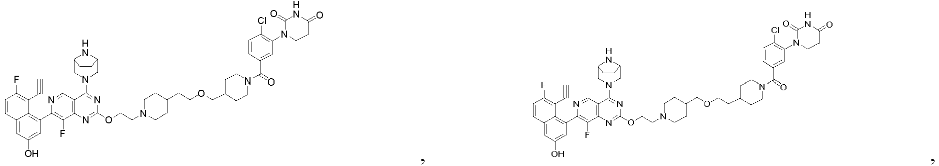

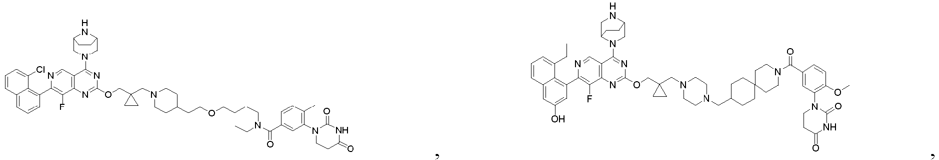

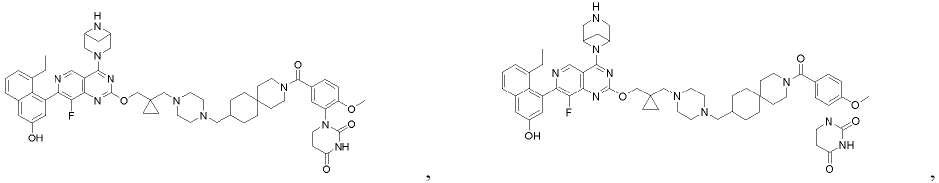

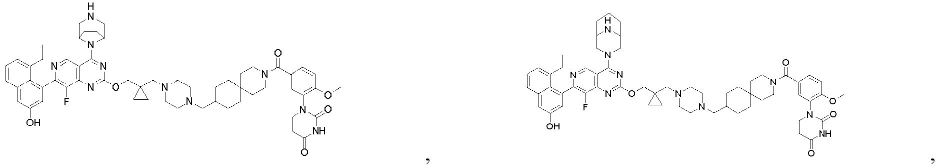

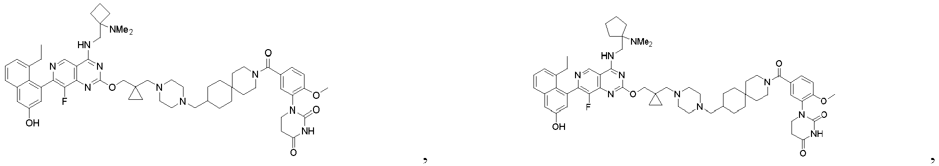

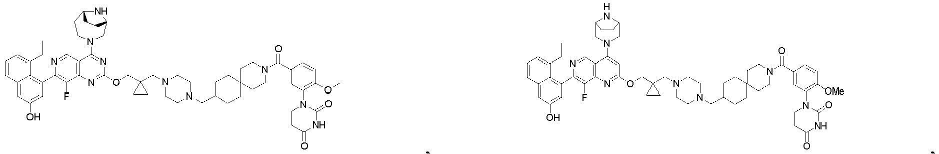

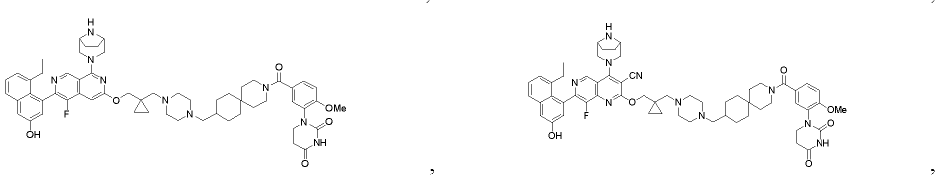

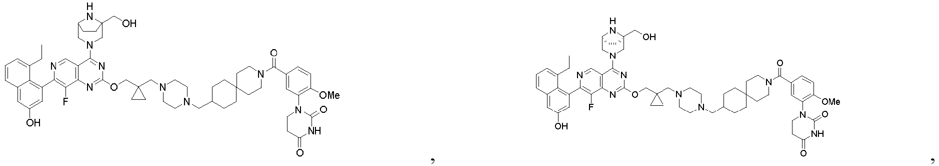

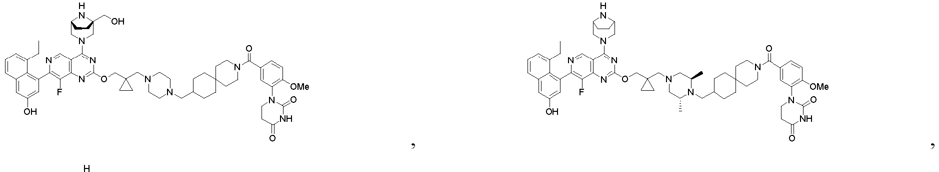

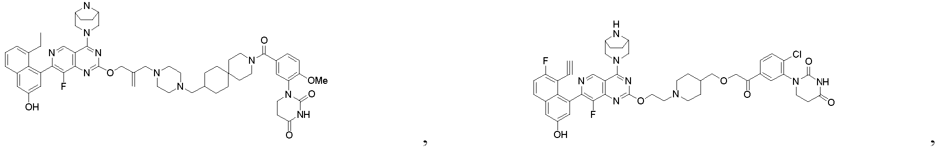

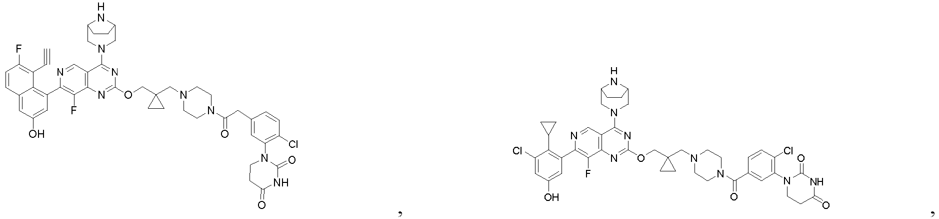

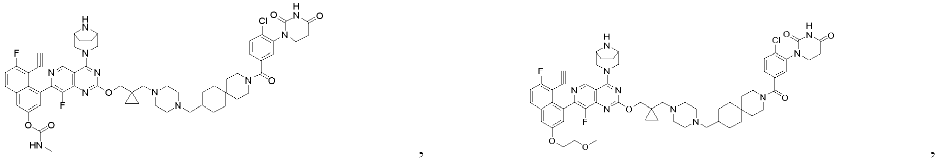

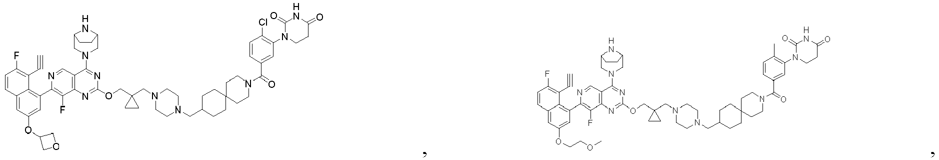

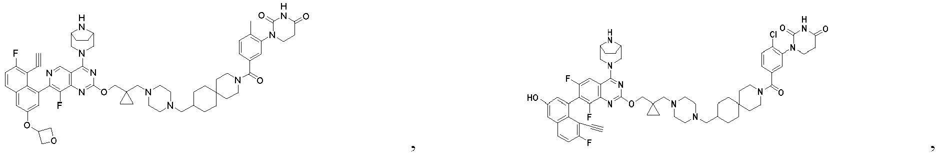

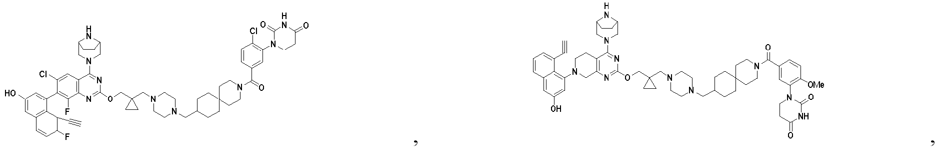

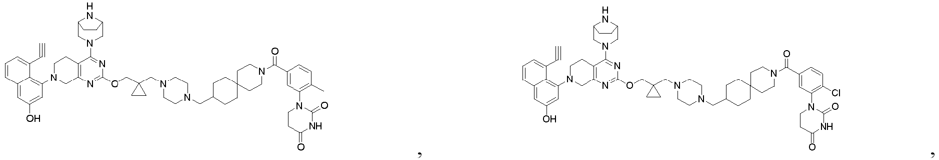

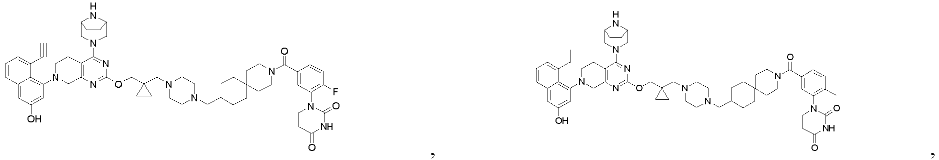

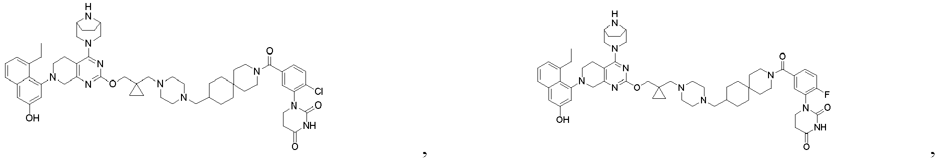

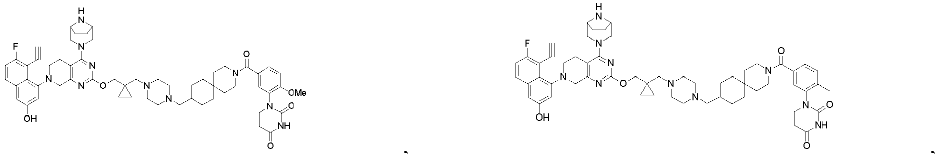

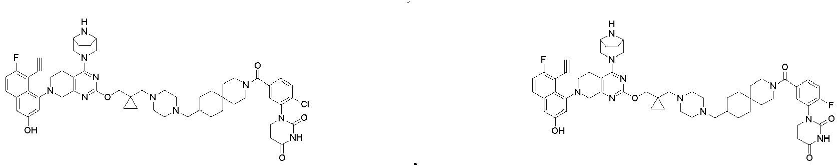

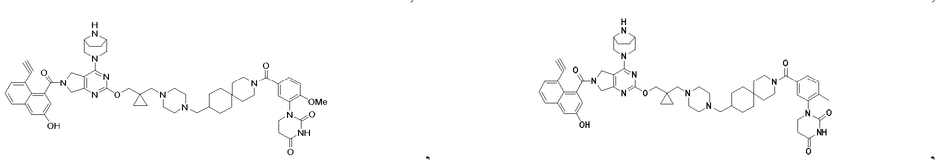

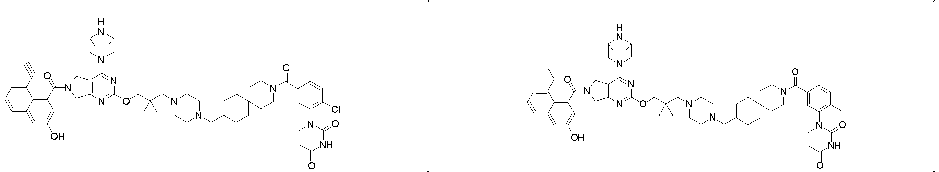

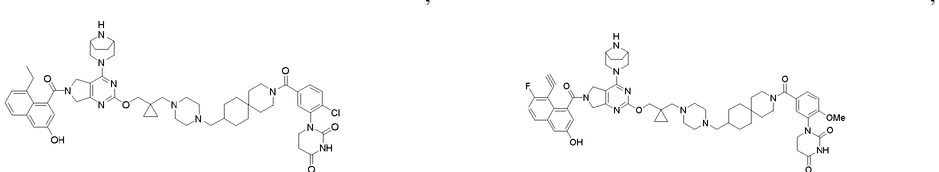

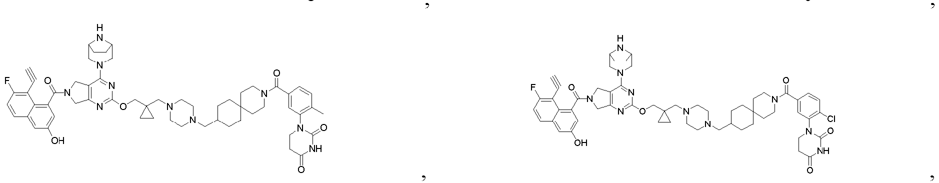

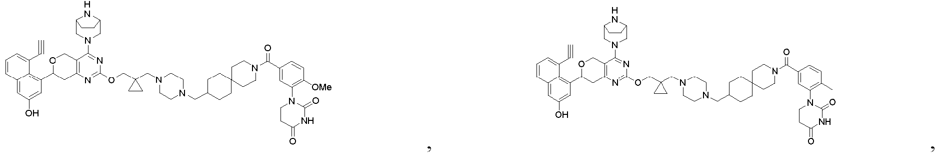

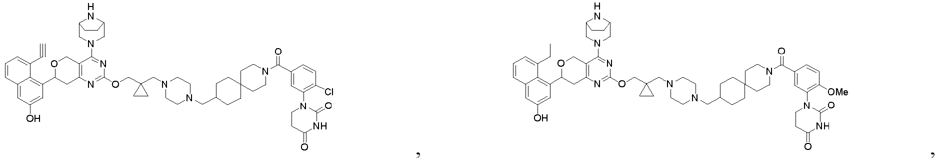

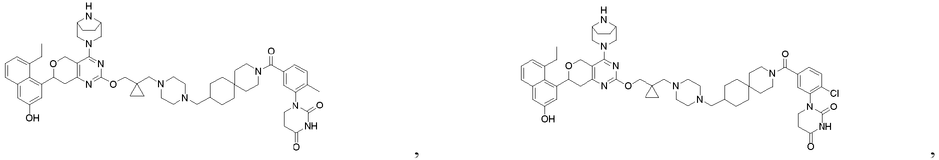

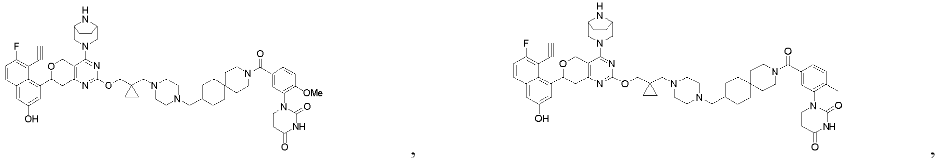

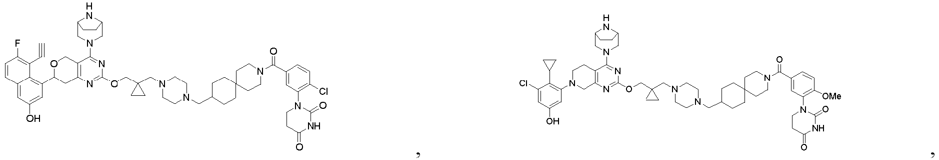

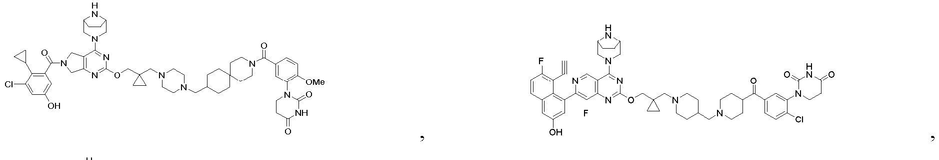

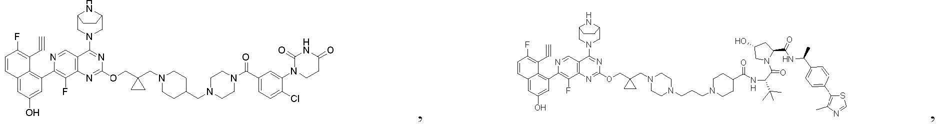

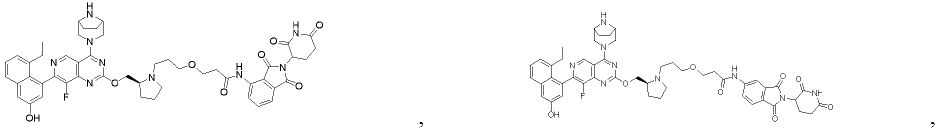

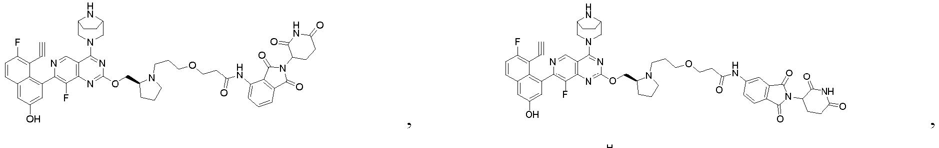

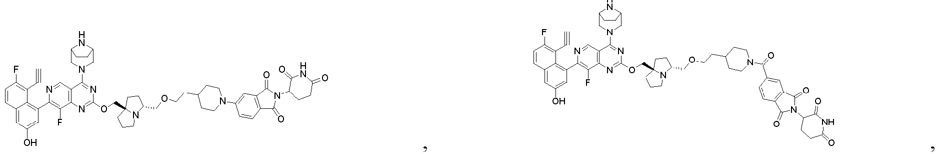

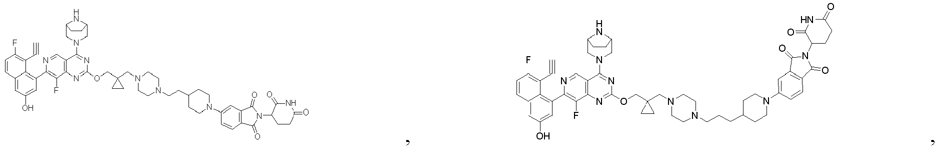

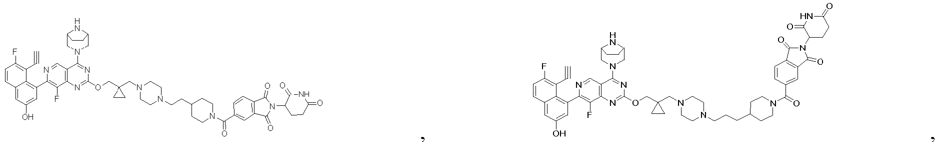

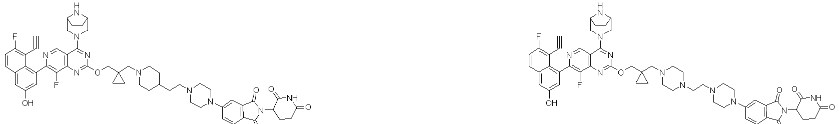

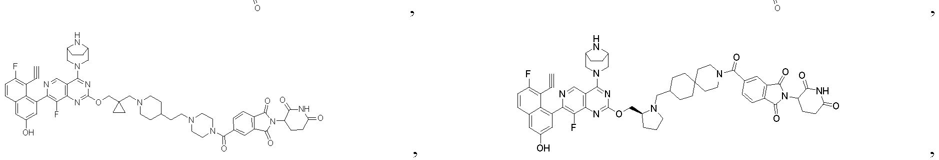

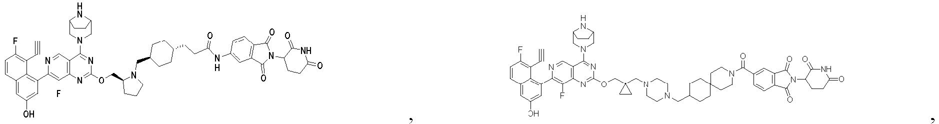

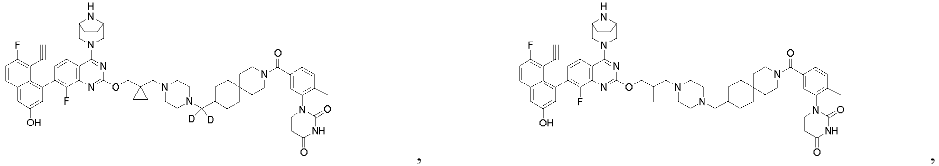

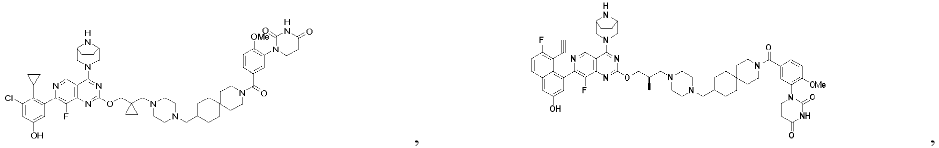

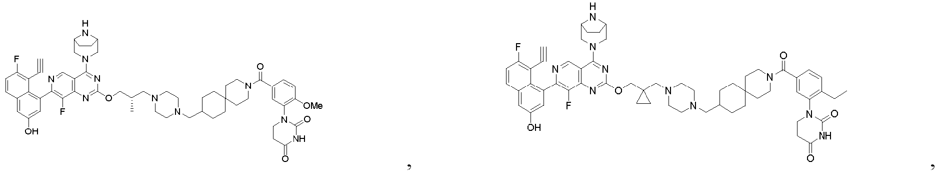

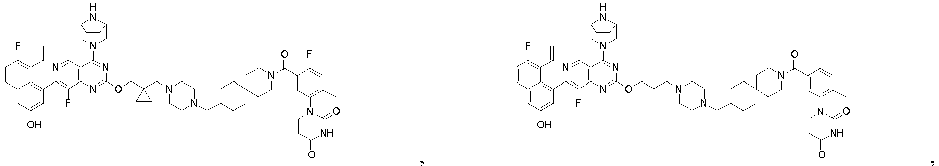

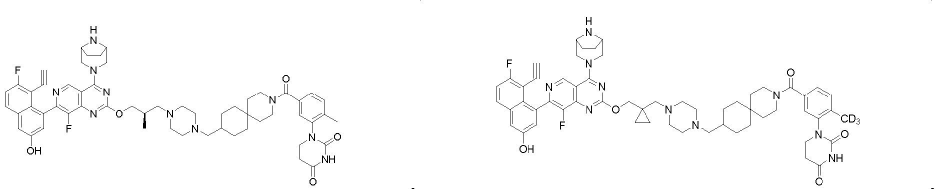

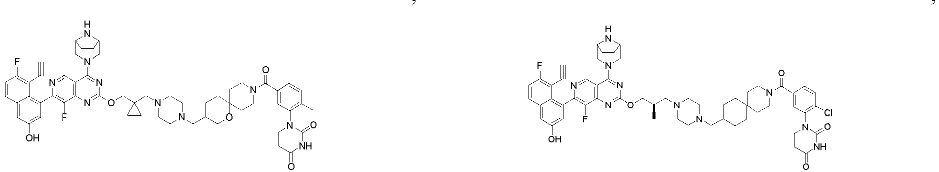

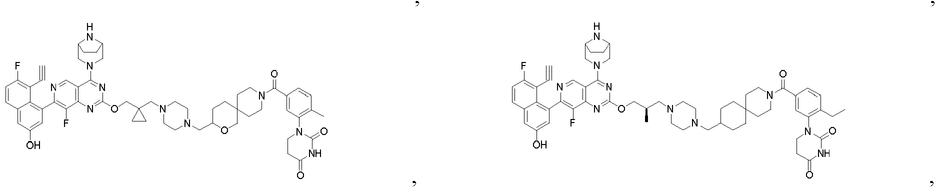

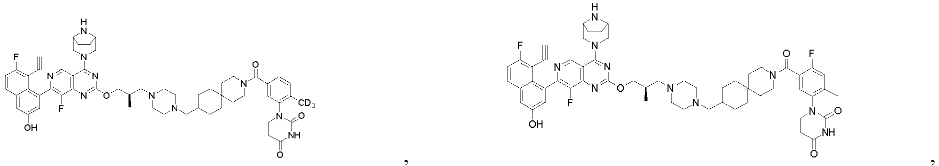

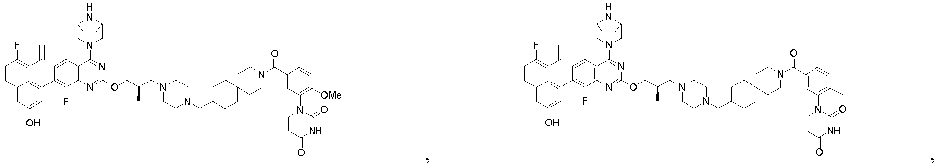

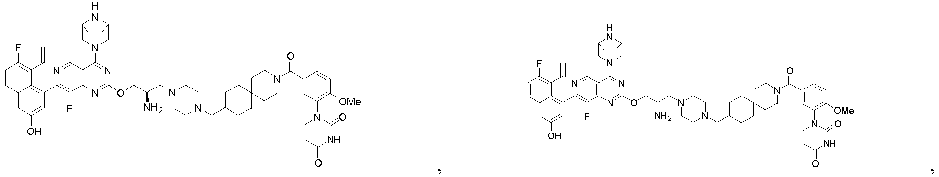

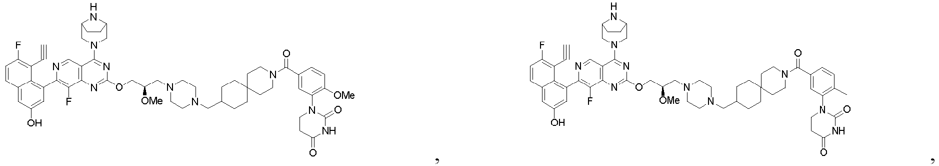

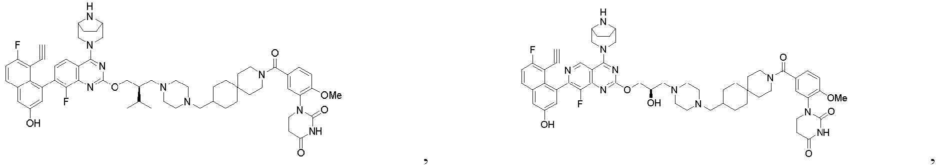

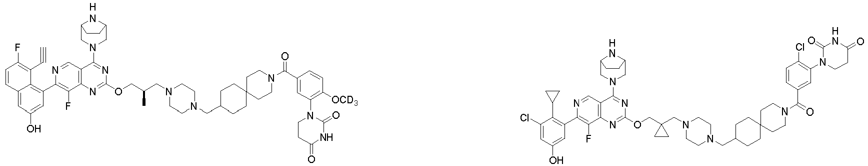

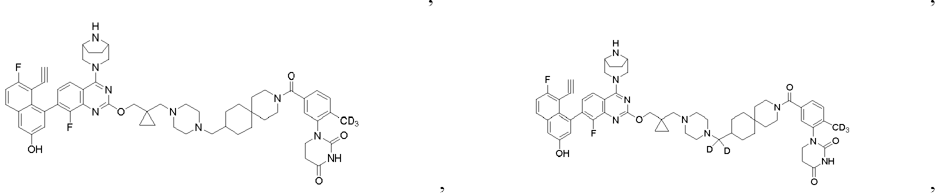

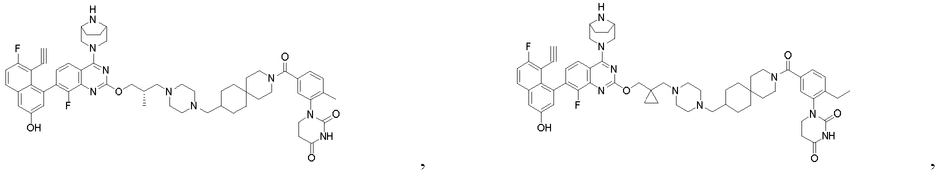

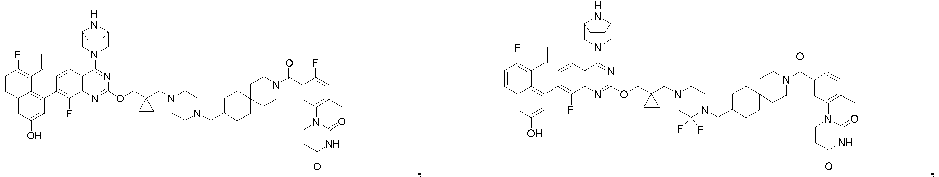

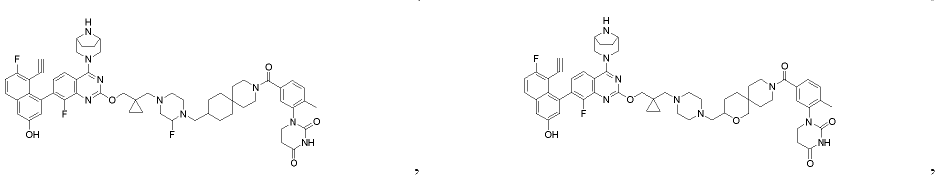

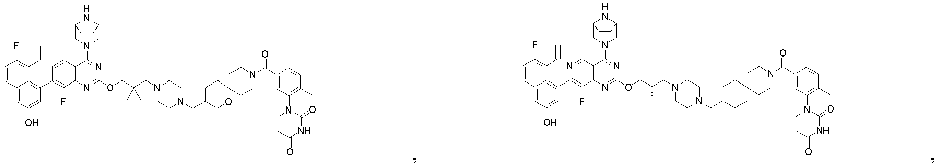

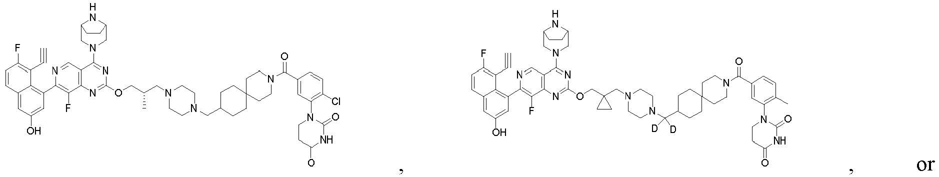

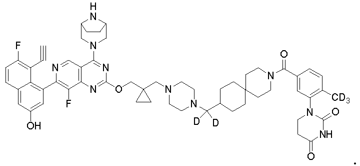

20. A pharmaceutical composition comprising a therapeutically effective amount of the compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1-19, and a pharmaceutically acceptable carrier, diluent, or excipient.

21. Use of substance **X** in preparing a medicament, wherein substance **X** is the compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1-19, or the pharmaceutical composition according to claim 20; the medicament is for use as a medicament for the treatment or prevention of a disease or disorder mediated by a KRAS mutation, or for use as a medicament for the treatment or prevention of cancer;
preferably, the disease or disorder mediated by the KRAS mutation is a disease or disorder caused by the interaction of KRAS G12D with SOS1 or SHP2 protein;
the cancer is selected from:
heart: sarcoma (angiosarcoma, fibrosarcoma, rhabdomyosarcoma and liposarcoma), myxoma, rhabdomyoma, fibroma, lipoma and teratoma;
lung: bronchial cancer (squamous cell carcinoma, undifferentiated small cell carcinoma, undifferentiated large cell carcinoma and adenocarcinoma), bronchioloalveolar carcinoma (bronchiolar carcinoma), bronchial adenoma, sarcoma, lymphoma, chondroma, hamartoma and mesothelioma;
gastrointestinal tract: esophagus cancer (squamous cell carcinoma, adenocarcinoma, leiomyosarcoma, and lymphoma), stomach (cancer, lymphoma, and leiomyosarcoma), pancreas (ductal adenocarcinoma, insulinoma, glucagonoma, gastrinoma, carcinoid tumor, and vipoma), small intestine (adenocarcinoma, lymphoma, carcinoid tumor, Kaposi's sarcoma, leiomyoma, hemangioma, lipoma, neurofibroma, and fibroma), and large intestine (adenocarcinoma, tubular adenoma, villous adenoma, hamartoma, and leiomyoma);
genitourinary system: kidney (adenocarcinoma, embryonal carcinosarcoma (Wilms' tumor), lymphoma, and leukemia), bladder and urinary tract (squamous cell carcinoma, transitional cell carcinoma, and adenocarcinoma), prostate (adenocarcinoma and sarcoma), and testis (seminoma, teratoma, embryonal carcinoma, teratoma, choriocarcinoma, sarcoma, interstitial cell carcinoma, fibroma, fibroadenoma, adenomatoid tumor, and lipoma);
liver: liver cancer (hepatocellular carcinoma), intrahepatic cholangiocarcinoma, hepatoblastoma, malignant hemangioendothelioma, hepatocellular adenoma, and hemangioma;
biliary tract: cholangiocarcinoma, gallbladder cancer, ampullary cancer, and intrahepatic cholangiocarcinoma;
bone: osteogenic sarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma, and giant cell tumor;
nervous system: skull (osteoma, hemangioma, granuloma, xanthoma, and osteitis deformans), meninges (meningioma, meningosarcoma, and glioma), brain (astrocytoma, medulloblastoma, glioma, ependymoma, germinal cell tumor (pinealoma), glioblastoma multiforme, oligodendroglioma, neurilemmoma, eye cancer, and congenital tumor), spinal neurofibroma, meningioma, glioma, and sarcoma);
gynaecology and obstetrics: uterus (endometrial cancer (serous cystadenocarcinoma, mucinous cystadenocarcinoma, and unclassified cancer), granulosa theca cell tumor, ovarian Sertoli-Leydig cell tumor, dysgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, botulinum sarcoma (embryonal rhabdomyosarcoma), and fallopian tube (cancer);
hematology: blood (myeloid leukemia (acute and chronic), acute lymphoblastic leukemia, chronic lymphocytic leukemia, myeloproliferative disease, multiple myeloma, and myelodysplastic syndrome), Hodgkin's disease, and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid, and psoriasis; and
adrenal gland: neuroblastoma;
for example, the cancer is selected from:
pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer, and sarcoma.

22. A preparation method for the compound of formula I or I', and/or the stereoisomer, the enantiomer, the diastereomer, the atropisomer, the deuteride, the hydrate, the solvate, the metabolite, or the prodrug thereof and/or the pharmaceutically acceptable salt thereof according to any one of claims 1-19, being the following method:
when G is 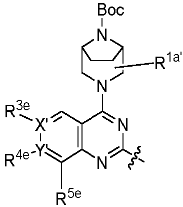 , L is LA 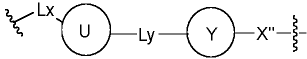 , and ring U contains an NH group, the preparation method is: 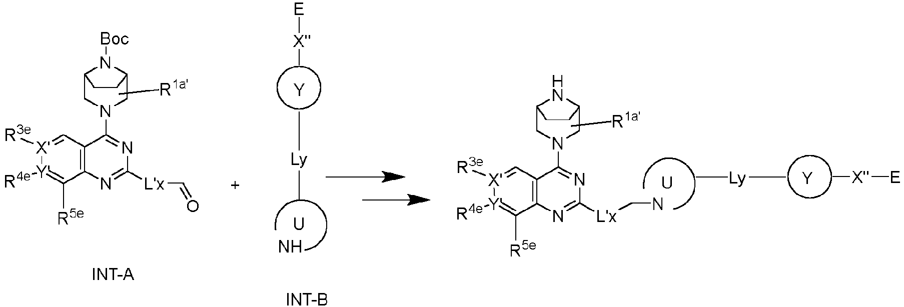
when G1' is 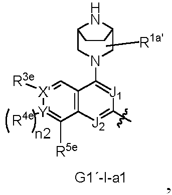 L is LA 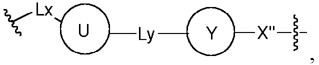 and ring U contains an NH group, the preparation method is: 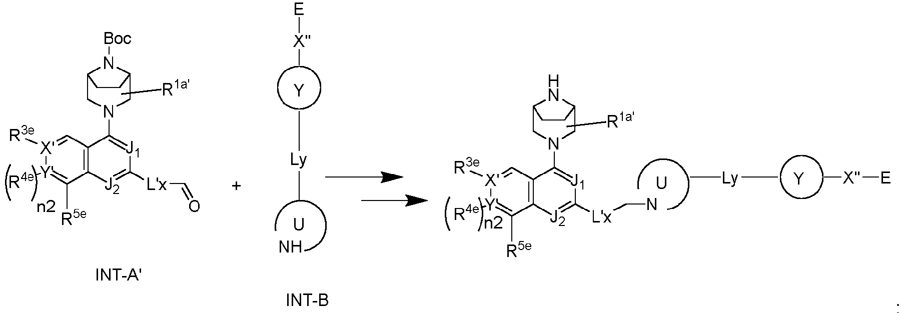
wherein 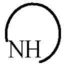 refers to ring U containing the NH group;
R^{1a'} is as defined in any one of claims 1-10; R^{3e}, R^{4e}, R^{5e}, X', Y', and R^{1a'} are as defined in any one of claims 8 and 9; 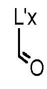
refers to Lx containing an aldehyde group; Lx is as defined in any one of claims 10-14;
ring U, ring Y, X", Lx, and Ly are as defined in any one of claims 10-14;
E is as defined in any one of claims 1-19;
when G is 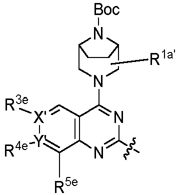 , L is LA 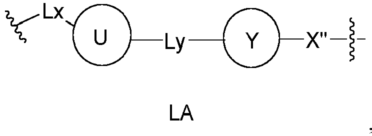 ring Y contains an NH group, and X" is C(O), the preparation method is: 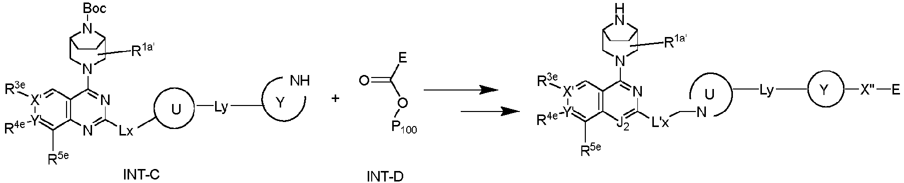
when G1' is 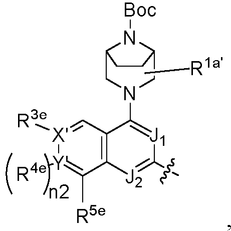 L is LA 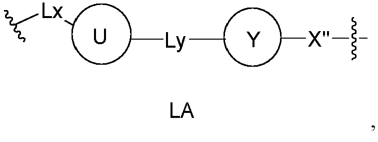 ring Y contains an NH group, and X" is C(O), the preparation method is: 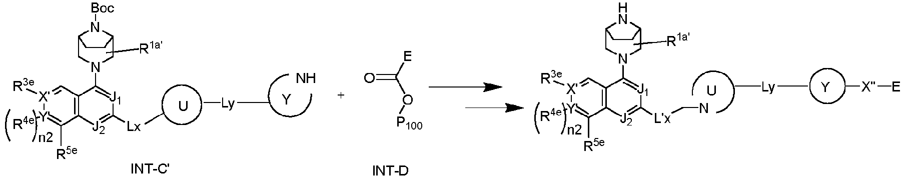
wherein 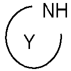 refers to ring Y containing the NH group;
P₁₀₀ is pentafluorophenyl or*p*-nitrophenyl;
R^{1a'} is as defined in any one of claims 1-10; R^{3e}, R^{4e}, R^{5e}, X', Y', and R^{1a'} are as defined in claim 8 or 9;
ring U, ring Y, X", Lx, and Ly are as defined in LA according to any one of claims 10-14;
E is as defined in any one of claims 1-18.

23. A compound of formula W-1, W-2, W-3, W-4, W-5, II-1, II-2, or II-3: 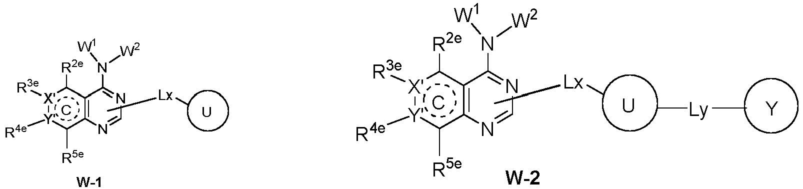 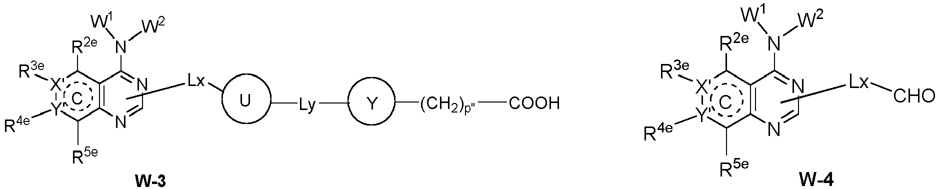 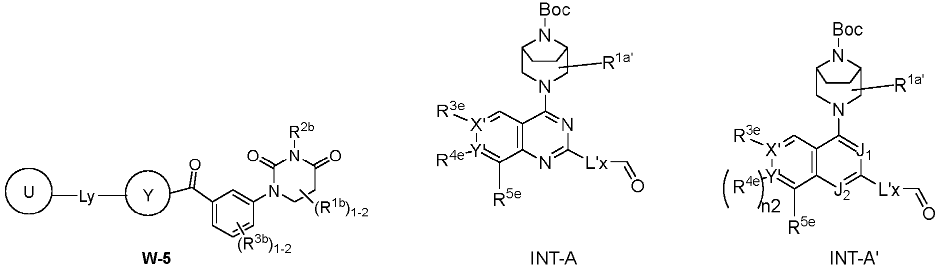 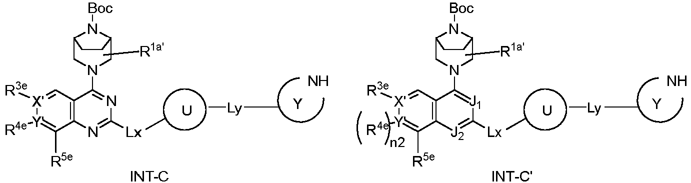 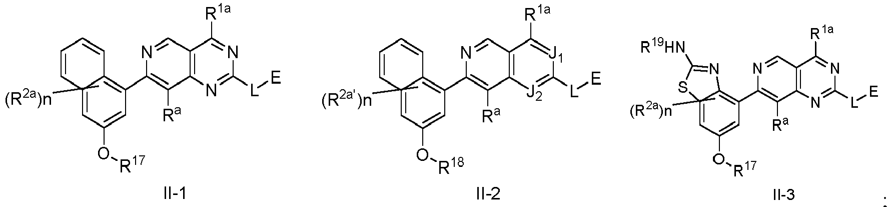 wherein each R^{1a} is independently 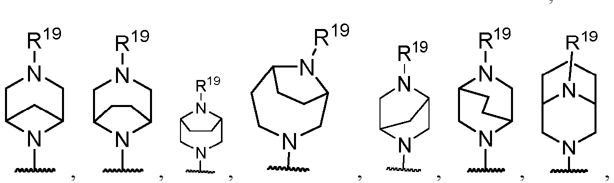 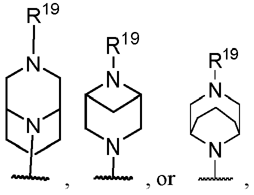 and each R¹⁹ is independently an amino-protecting group; R¹⁷ and R¹⁸ are each independently a hydroxy-protecting group;
J1, J2, L, and E are as defined in any one of claims 1-19;
ring C, R^{2e}, R^{3e}, R^{4e}, R^{5e}, R^{1b}, R^{2b}, R^{3b}, W¹, W², X', and Y' are as defined in any one of claims 8-10;
ring U, ring Y, X", Lx, and Ly are as defined in any one of claims 10-13;
p' is 1, 2, 3, or 4;
preferably, formula W-1 is formula W-1-1, formula W-2 is formula W-2-1, formula W-3 is formula W-3-1, 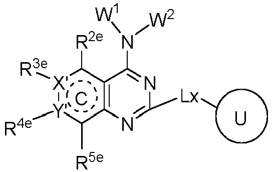
formula W-4 is formula W-4-1, or formula W-5 is formula W-5-1: W-1-1 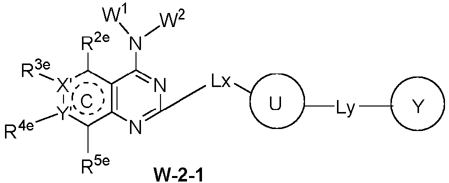 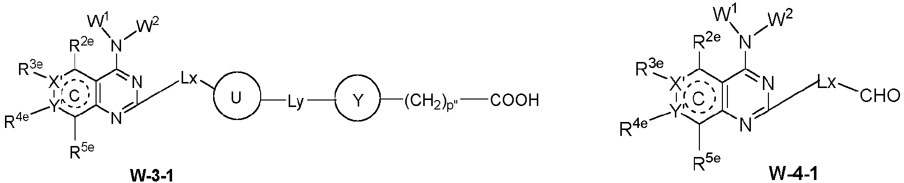 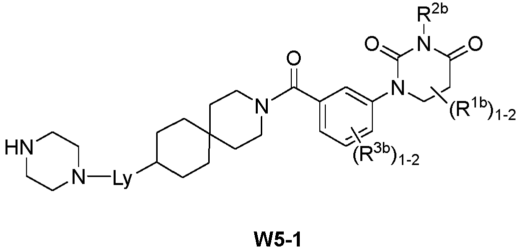
wherein ring C, R^{2e}, R^{3e}, R^{4e}, R^{5e}, W¹, and W² are as defined in any one of claims 8-10; R^{1b}, R^{2b}, and R^{3b} are as defined in any one of claims 1-16;
Lx, ring U, Ly, ring Y, and p" are as defined in any one of claims 10-14;
more preferably, the compound of formula W-1, W-2, W-3, W-4, or W-5 is selected from: 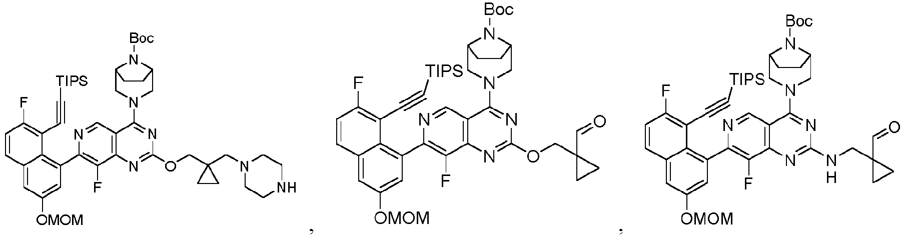 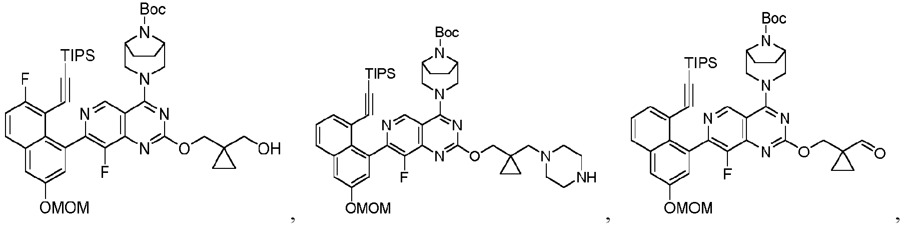 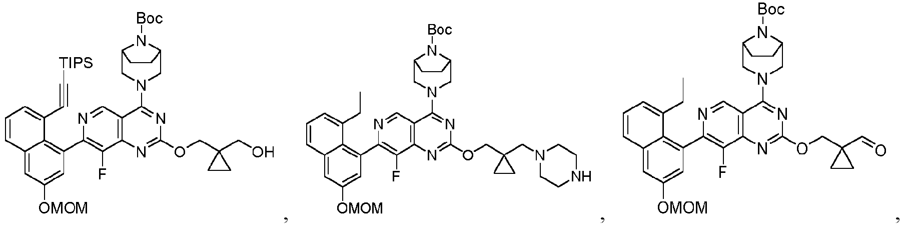 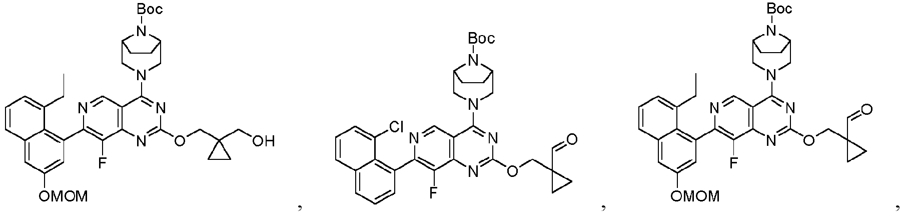 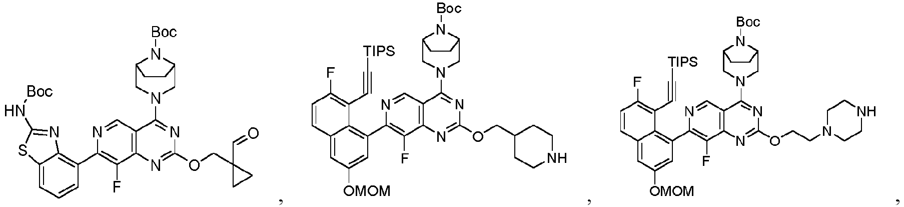 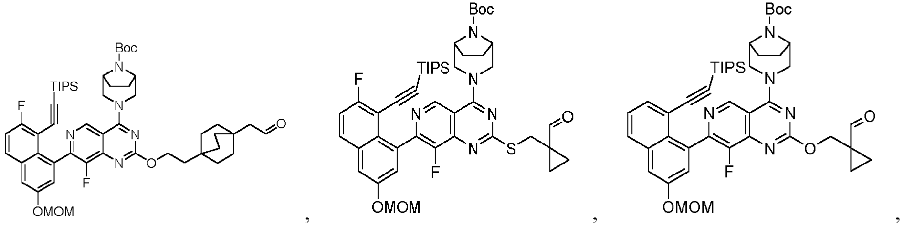 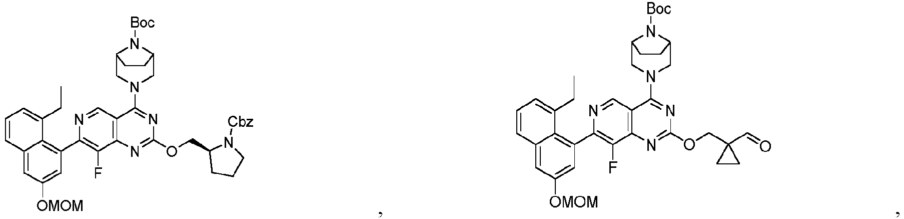 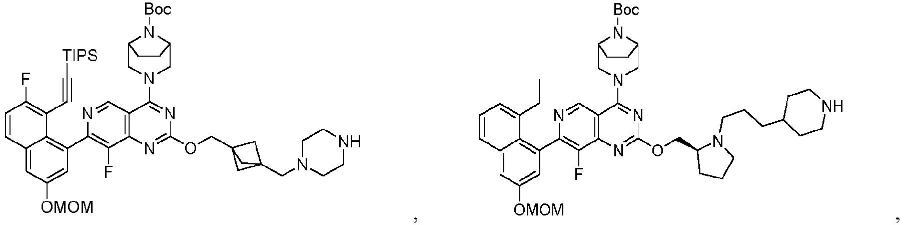 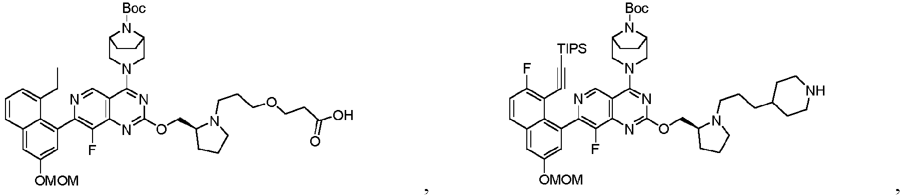 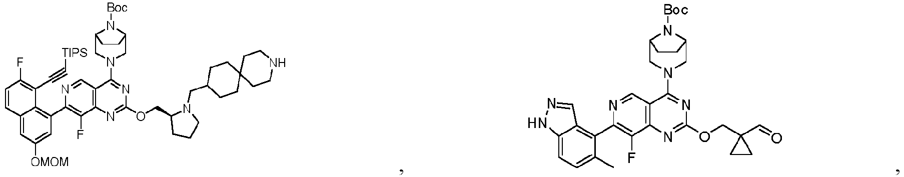 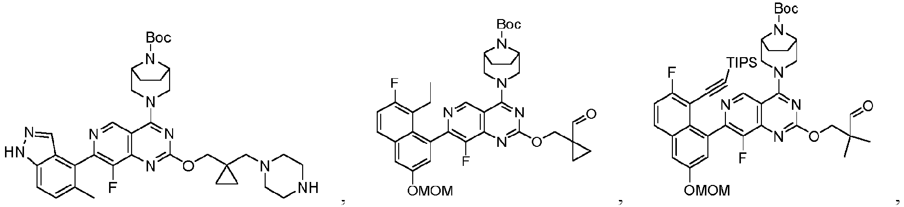 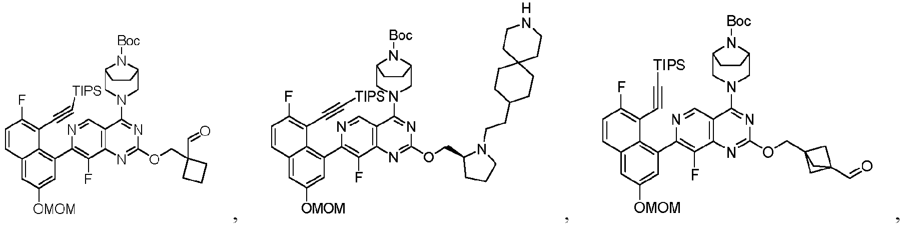 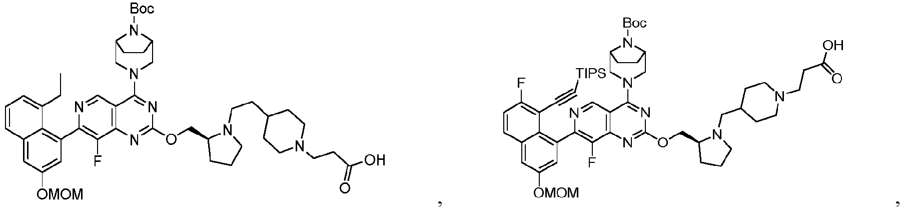 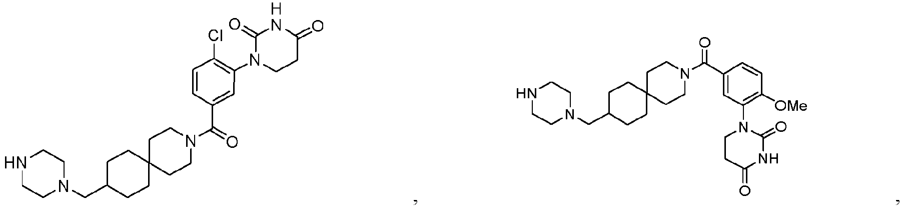 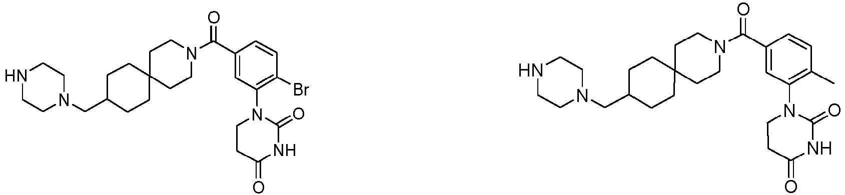 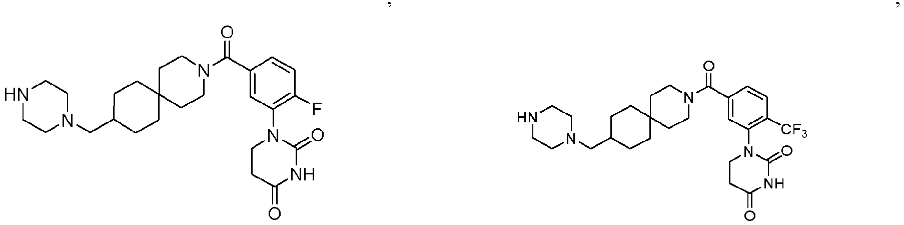 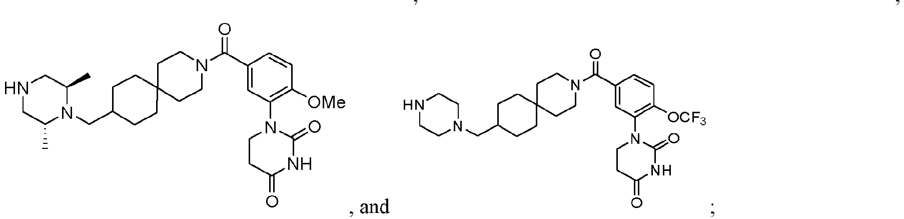 the compound of formula II-1, formula II-2, or formula II-3 is preferably any one of the following compounds: 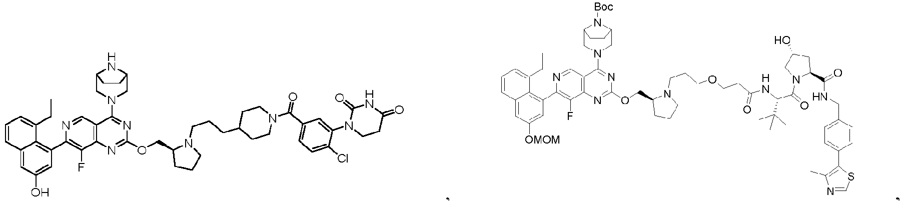 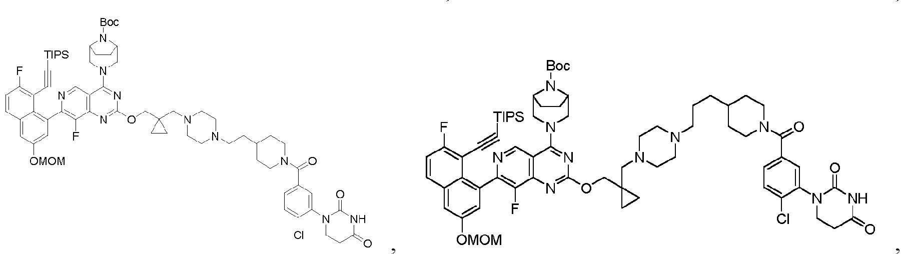 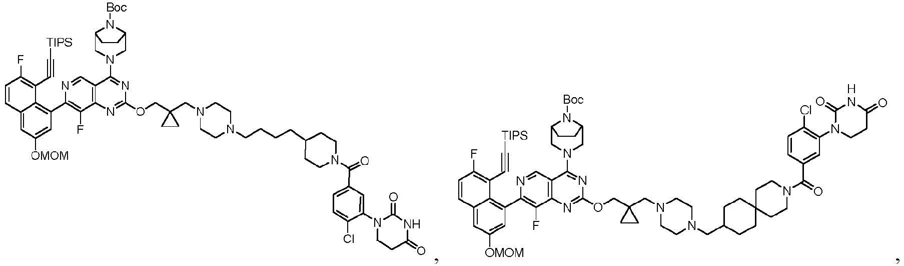 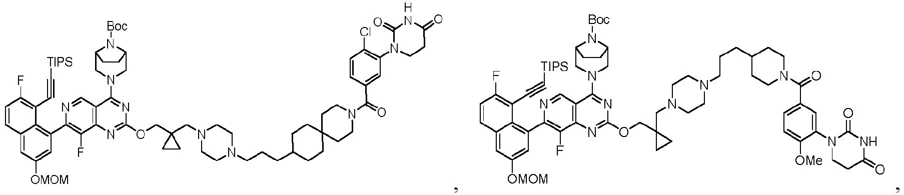 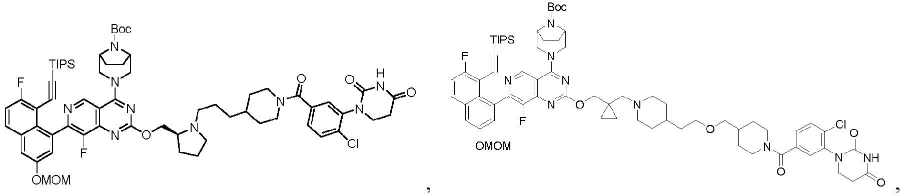 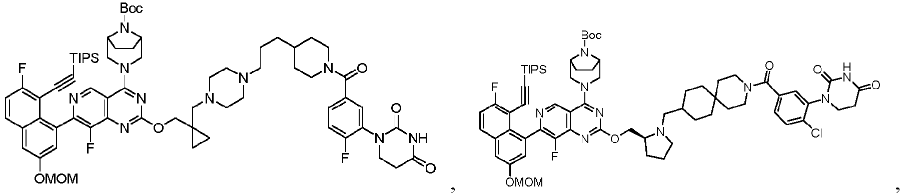 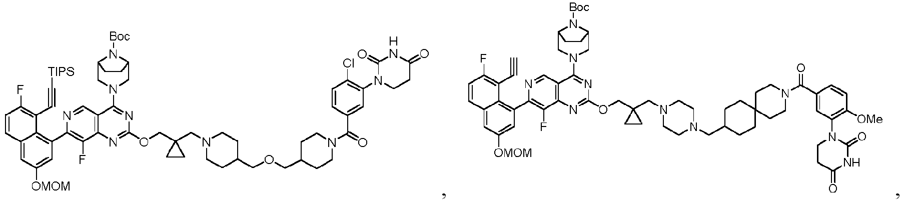 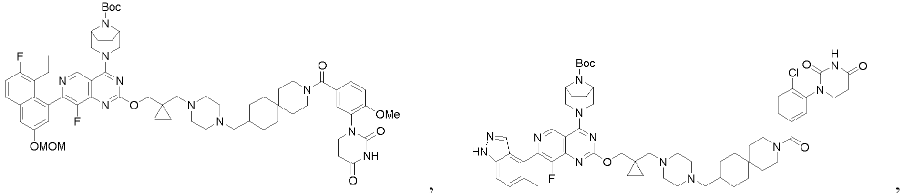 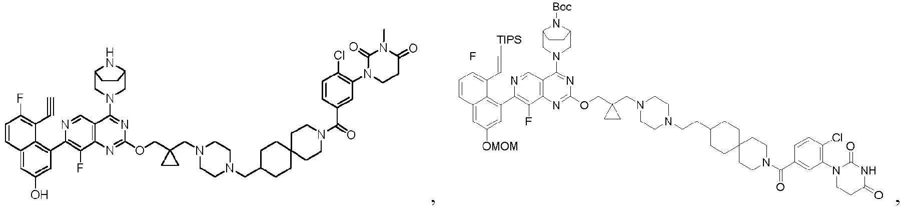 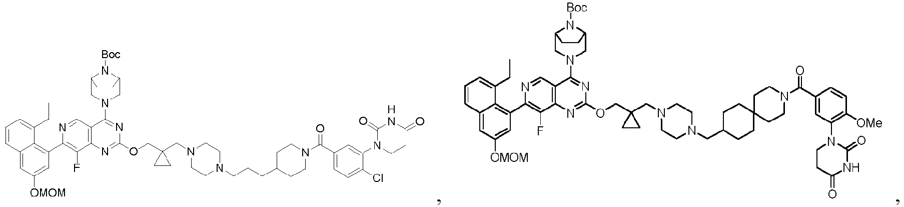 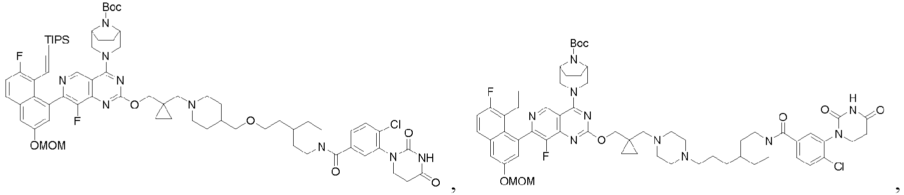 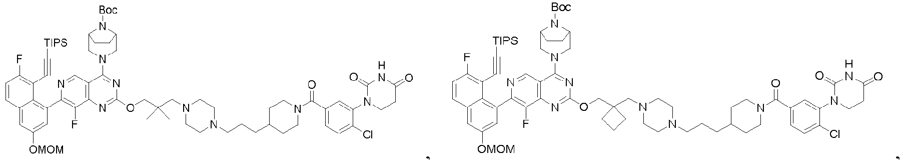 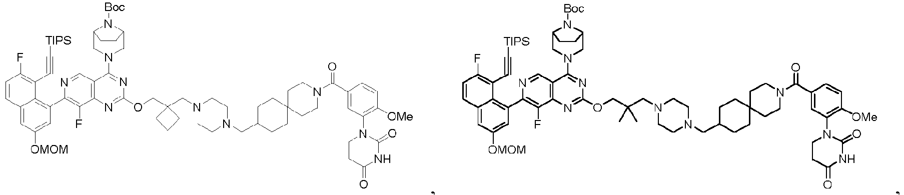 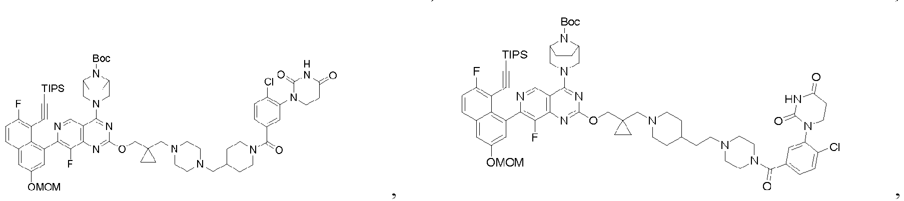 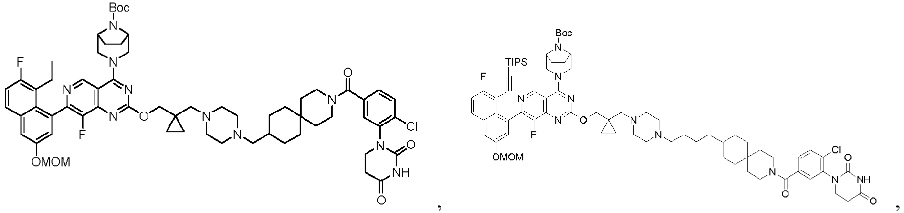 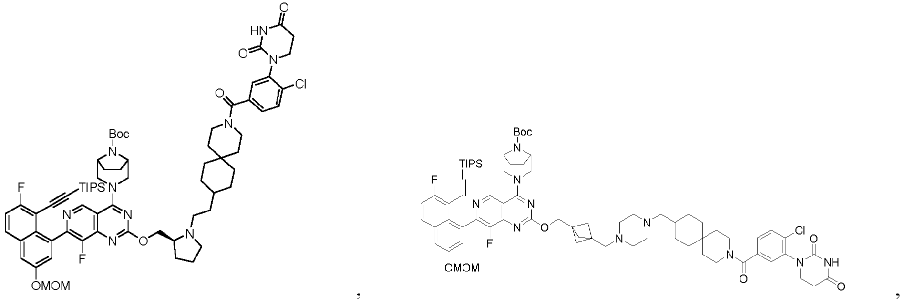 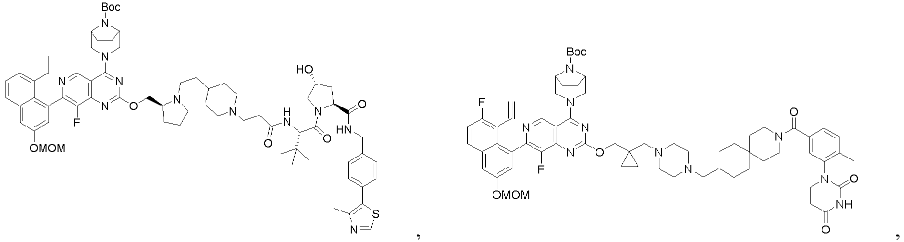 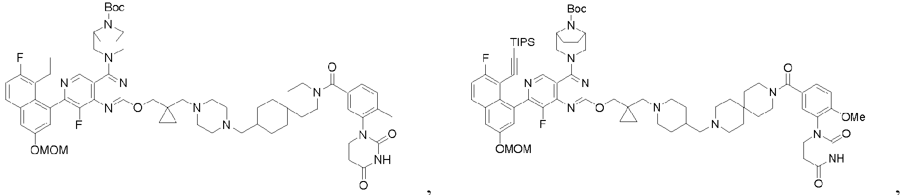 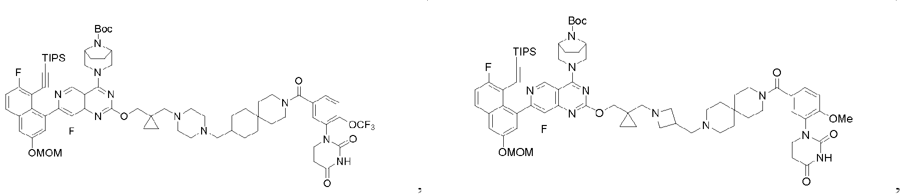 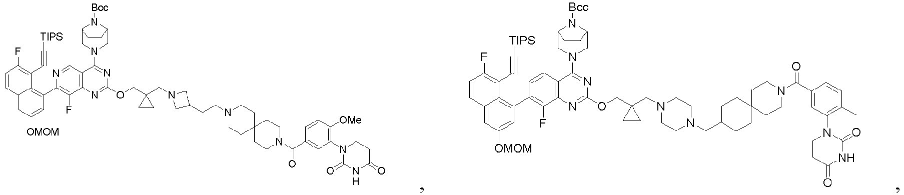 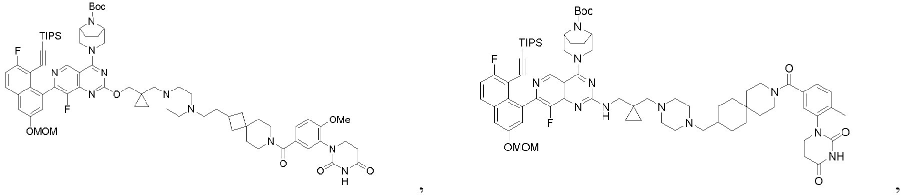 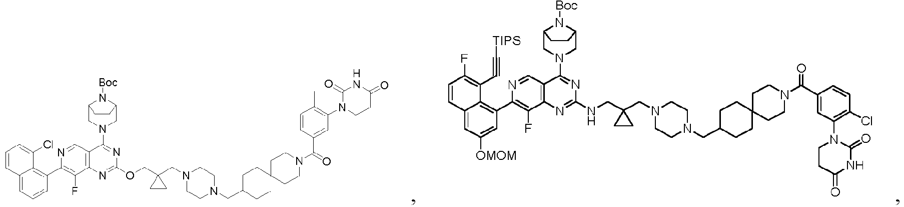 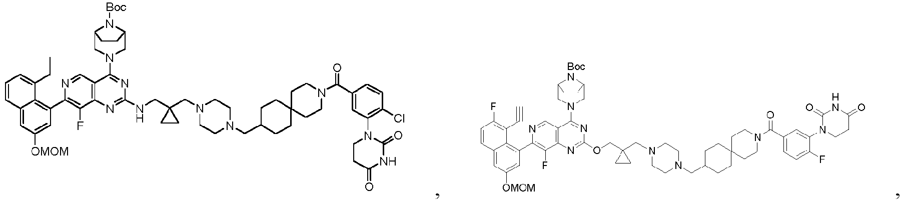 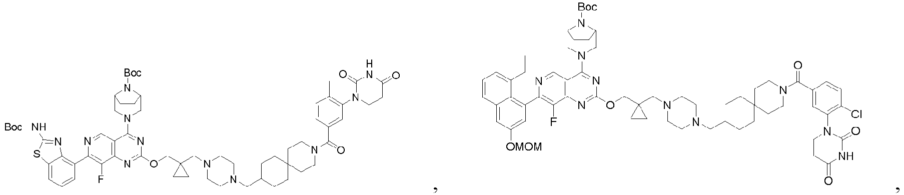 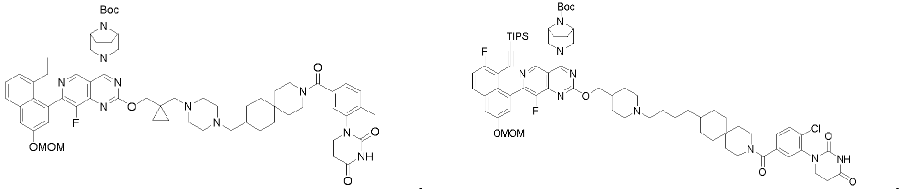 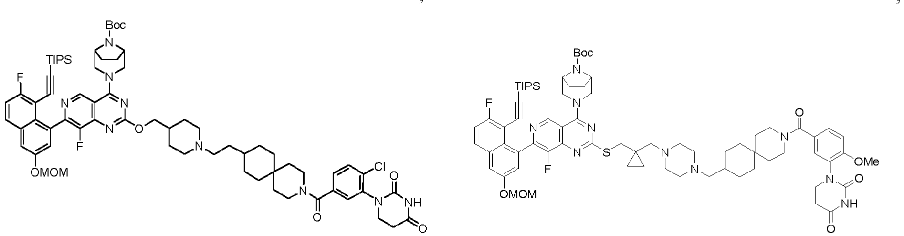 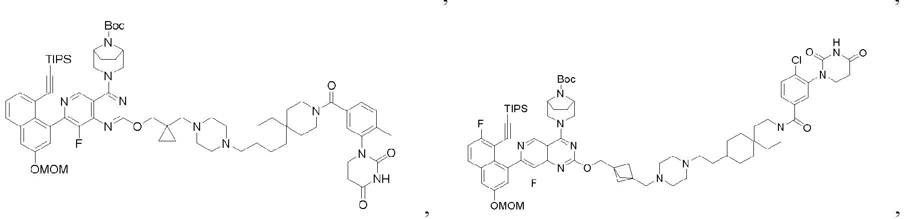 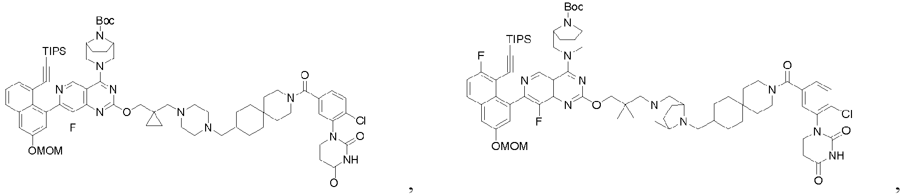 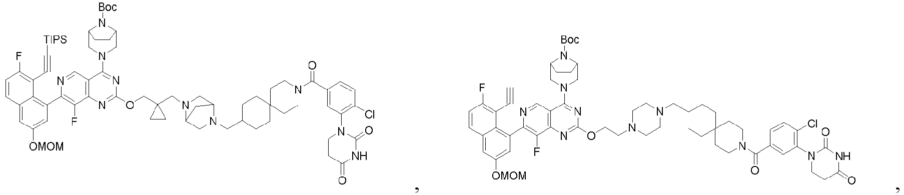 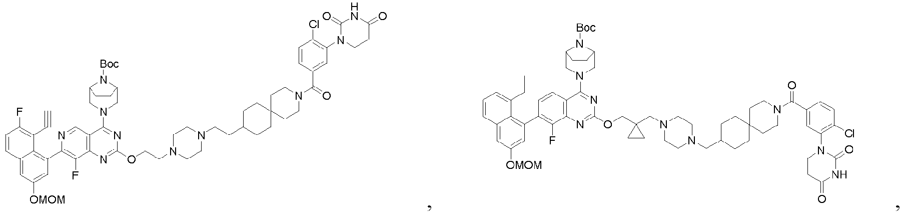 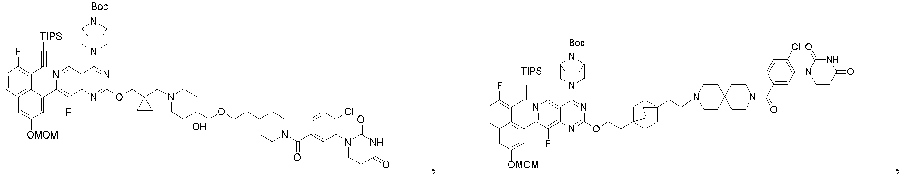 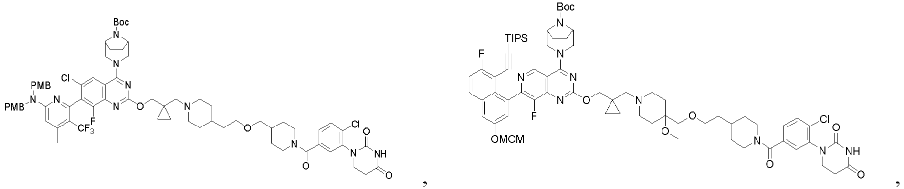 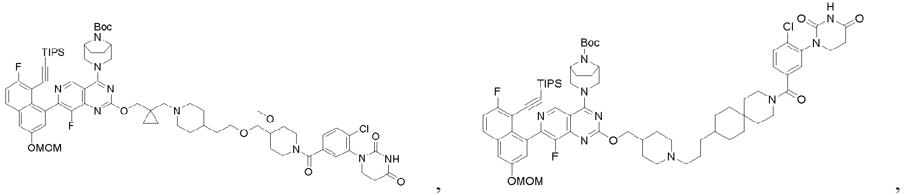 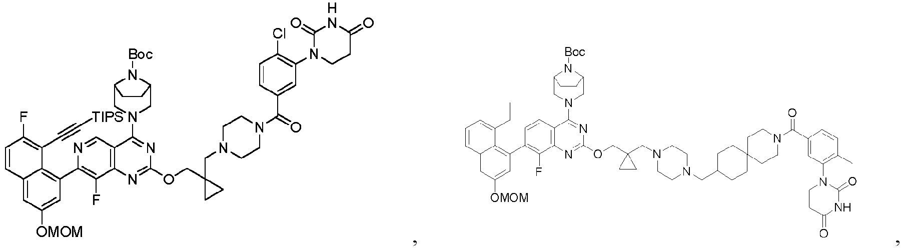 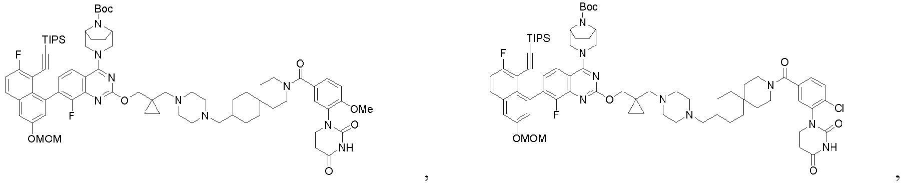 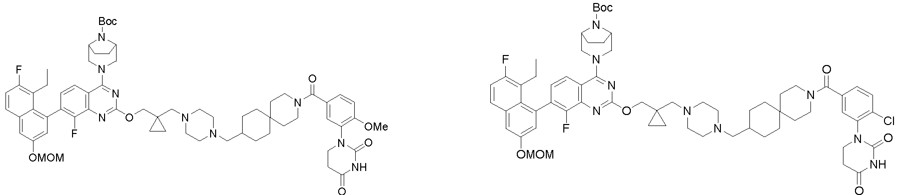 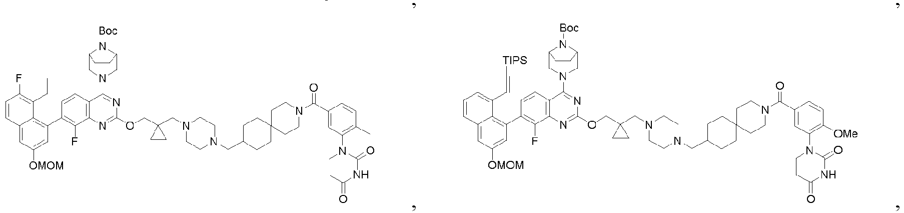 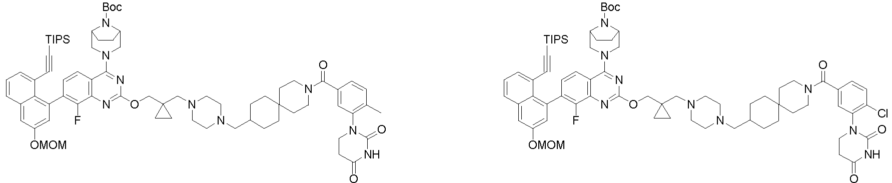 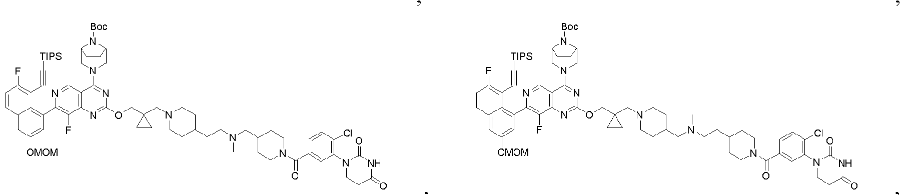 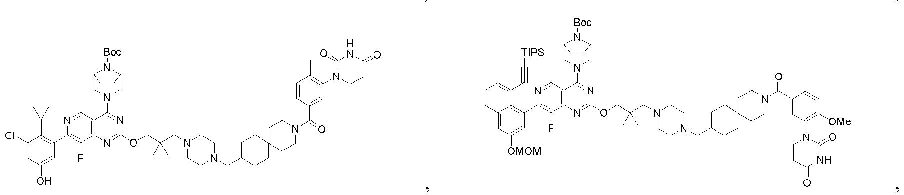 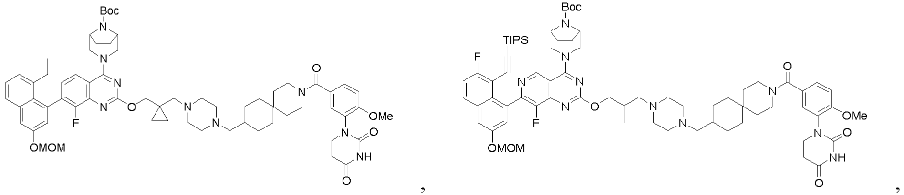 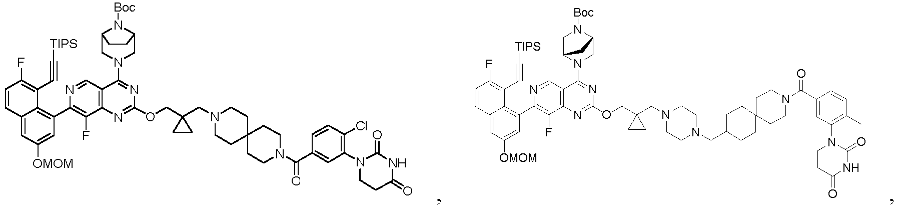
or

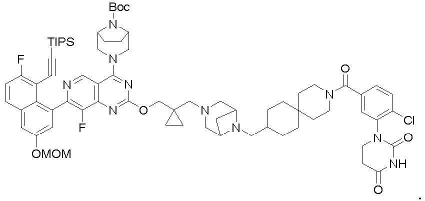

24. A method for treating or preventing a KRAS G12D-mediated disease or disorder, comprising administering to a patient in need thereof a therapeutically effective amount of substance **X,** wherein substance **X** is the compound of formula I or I', and/or the stereoisomer, the enantiomer, the diastereomer, the atropisomer, the deuteride, the hydrate, the solvate or the prodrug thereof and/or the pharmaceutically acceptable salt thereof according to any one of claims 1-19, or the pharmaceutical composition according to claim 20;
preferably, the KRAS G12D-mediated disease or disorder is as described in claim 21.
